# EUROPEAN PATENT APPLICATION

(11) **EP 4 520 393 A2**
(43) Date of publication of application: **12.03.2025**
(21) Application number: 24218624.5
(22) Date of filing: 19.04.2022
(51) Int. Cl.: A61P 3/10

(54) **CARBOXY-BENZIMIDAZOLE GLP-1R MODULATING COMPOUNDS**

(30) Priority: 21.04.2021 US 202163177775 P; 02.12.2021 US 202163285410 P
(62) Divisional of application: 22723850.8
(71) Applicant: Gilead Sciences, Inc., Foster City, CA 94404 (US)
(72) Inventor: ARMSTRONG, Megan K., Foster City, California 94404 (US); BRIZGYS, Gediminas J., Foster City, California 94404 (US); CASSIDY, James S., San Francisco, California 94108 (US); CHIN, Elbert, Foster City, California 94404 (US); CHOU, Chienhung, Foster City, California 94404 (US); HUNG, Chao-I, Foster City, California 94404 (US); LIN, David W., Foster City, California 94404 (US); MITCHELL, Michael L., Foster City, California 94404 (US); ROBERTS, Ezra, San Francisco, California 94110 (US); SCHROEDER, Scott D., Foster City, California 94404 (US); TAYLOR, James G., Foster City, California 94404 (US); THOMAS-TRAN, Rhiannon, Foster City, California 94404 (US); WRIGHT, Nathan E., San Diego, California 92131 (US); YANG, Zheng-Yu, Foster City, California 94404 (US)
(74) Representative: Marks & Clerk LLP

(57) **Abstract**

The present disclosure provides GLP-1R agonists, and compositions, methods, and kits thereof. Such compounds are generally useful for treating a GLP-1R mediated disease or condition in a human.

## Description

### CROSS-REFERENCE TO RELATED APPLICATIONS

This application claims the benefit under 35 U.S.C. § 119(e) of U.S. Provisional Application No. 63/177,775, filed on April 21, 2021 and U.S. Provisional Application No. 63/285,410, filed on December 2, 2021, which are hereby incorporated herein by reference in their entireties for all purposes.

### FIELD

The present disclosure relates to compounds that bind to and act as agonists or modulators of the glucagon-like peptide-1 receptor (GLP-1R) and act as agonists or modulators of GLP-1R. The disclosure further relates to the use of the compounds for the treatment and/or prevention of diseases and/or conditions by said compounds.

### BACKGROUND

Glucagon-like peptide-1 (GLP-1) is a peptide hormone that is secreted from the enteroendocrine cells in the gut in response to a meal. GLP-1 is believed to play a role in regulation of post-prandial glycemia, via directly augmenting meal-induced insulin secretion from the pancreatic beta-cells, as well as in promoting satiety by delaying the transit of food through the gut. GLP-1 mediates intracellular signaling via the GLP-1 receptor (GLP-1R) which belongs to a family of G-protein coupled receptors that are present on the cell membrane and can result in accumulation of the secondary messenger cyclic adenosine monophosphate (cAMP) upon activation. Non-alcoholic steatohepatitis (NASH) can be associated with features of metabolic syndrome, including obesity, type 2 diabetes, insulin resistance and cardiovascular disease.

GLP-1R agonists are currently being investigated in connection with diabetes, obesity, and NASH. GLP-1R agonists include peptides, such as exenatide, liraglutide, and dulaglutide, that have been approved for the management of type 2 diabetes. Such peptides are predominantly administered by subcutaneous injection. Oral GLP-1 agonists are also under investigation for treatment of type 2 diabetes. Some GLP-1R agonists, such as liraglutide, dulaglutide, and exenatide, are resistant to rapid degradation by dipeptidyl peptidase 4, resulting in longer half-lives than endogenous GLP-1.

There remains a need for compounds, such as agonists of GLP-1R, with desirable therapeutic properties, metabolic properties, and/or easy administration in the treatment of metabolic diseases and related diseases, including but not limited to NASH, obesity, and Type 2 diabetes.

### SUMMARY

In one embodiment, the present disclosure provides a compound of Formula (I): 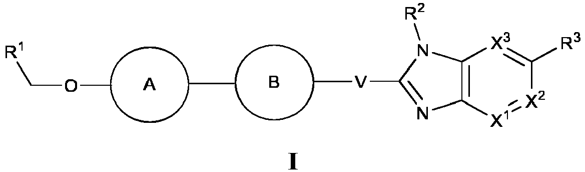 or a pharmaceutically acceptable salt thereof, wherein
R¹ is
   i) 5-membered heteroaryl, optionally substituted with one to four R⁴;
   ii) 5-membered heteroaryl fused to a heteroaryl or aryl to form a heteroaryl ring system, wherein the heteroaryl ring system is optionally substituted with one to four R⁴;
   iii) 5-membered heteroaryl substituted with two R⁴ groups attached to adjacent ring atoms and optionally substituted with one or two additional R⁴, wherein the two R⁴ groups attached to adjacent ring atoms are combined with the atoms to which they are attached to form a C₅₋₁₀ cycloalkyl or heterocyclyl, which is each optionally substituted with one to four R⁶;
   iv) 6-membered heteroaryl or phenyl substituted with C₂-alkynyl, wherein the 6-membered heteroaryl or phenyl is optionally substituted with one to three R⁴, wherein the C₂-alkynyl is substituted with -C(CH₃)₂SO₂CH₃, aryl, heteroaryl, or heterocyclyl, wherein the aryl, heteroaryl or heterocyclyl is optionally substituted with one to four R⁵;
   v) 6-membered heteroaryl or phenyl substituted with -C(O)NR^{a}R^{b}, wherein the heteroaryl or phenyl is optionally substituted with one to four R⁴; or
   vi) 6- membered heteroaryl or phenyl, wherein the 6-membered heteroaryl or phenyl is substituted with heteroaryl, C₃₋₁₀ cycloalkyl, or heterocycle and optionally substituted with one to three R⁴, wherein the heteroaryl, C₃₋₁₀ cycloalkyl, or heterocycle is each substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl, wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶;
X¹, X², and X³ are each independently -C(H)=, or-C(R⁸)=;
ring A is 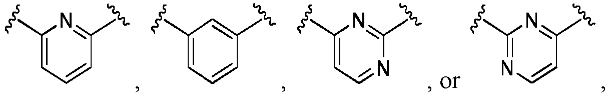 each optionally substituted with one to three Z^{1a} groups, each independently C₁₋₆ alkyl, C₁₋₆ haloalkyl, C₁₋₆ alkoxy, C₁₋₆ haloalkoxy, halogen, -OH, or -CN;
ring B is C₆₋₁₀ aryl or heteroaryl, which is each optionally substituted with one to four R⁴; V is -C(O)-, -O-, -N(R^{6a})-, or -C(R^{6b})(R^{6c})-;
R² is H, C₁₋₆ alkyl, C₂₋₆ alkynyl, C₁₋₆ haloalkyl, C₃₋₁₀ cycloalkyl, or heterocyclyl,
   wherein the alkyl, alkynyl, cycloalkyl, or heterocyclyl is each optionally substituted with one to four Z¹, wherein each Z¹ is independently C₁₋₆ alkyl, C₁₋₆ alkoxy, C₁₋₆ hydroxyalkyl, C₂₋₆ alkoxyalkyl, halogen, C₁₋₆ haloalkyl, C₁₋₆ haloalkoxy, -OH, -CN, C₁₋₆ alkyl-CN, -O-C₃₋₆ cycloalkyl, heterocyclyl, C₃₋₁₀ cycloalkyl, or heteroaryl optionally substituted with 1 to 4 groups each independently C₁₋₆ alkyl, C₁₋₆ alkoxy, halogen, -CN, C₁₋₆ haloalkyl, or C₁₋₆ haloalkoxy;
R³ is -C(O)OR^{3a};
R^{3a} is H, C₁₋₄alkyl-N(R^{9a})(R^{9b}), ^{_}C₁₋₄alkyl-N(R^{9a})C(O)-O-C₁₋₄ alkyl-OP(O)(OR^{9c})₂, C₁₋₄ alkyl-C(O)N(R^{9a})(R^{9b}), -C₁₋₄alkyl-O-C(O)-C₁₋₄alkyl, -C₁₋₄alkyl-O-C(O)-O-C₁₋₄alkyl, -C₁₋₄alkyl-O-C(O)-C₁₋₄alkyl-N(R^{9a})(R^{9b}), -C₁₋₄alkyl-O-C(O)-C₁₋₄alkyl-OP(O)(OR^{9c})₂, -CH₂CH(N(R^{9a})₂)C(O)OR^{9b}, -P(O)(OR^{9c})₂, -OP(O)(OR^{9c})₂, - CH₂P(O)(OR^{9c})₂, -CH₂OP(O)(OR^{9c})₂, -OCH₂P(O)(OR^{9c})₂, C(O)OCH₂P(O)(OR^{9c})₂, -P(O)(R^{9c})(OR^{9d}), -OP(O)(R^{9c})(OR^{9d}), - CH₂P(O)(R^{9c})(OR^{9d}), -OCH₂P(O)(R^{9c})(OR^{9d}), -C(O)OCH₂P(O)(R^{9c})(OR^{9d}), - P(O)(N(R^{9c})₂)₂, -OP(O)(N(R^{9c})₂)₂, -CH₂P(O)(N(R^{9c})₂)₂, -OCH₂P(O)(N(R^{9c})₂)₂, - C(O)OCH₂P(O)(N(R^{9c})₂)₂, -P(O)(N(R^{9c})₂)(OR^{9d}), -OP(O)(N(R^{9c})₂)(OR^{9d}), - CH₂P(O)(N(R^{9c})₂)(OR^{9d}), -OCH₂P(O)(N(R^{9c})₂)(OR^{9d}), - C(O)OCH₂P(O)(N(R^{9c})₂)(OR^{9d}), -P(O)(R^{9c})(N(R^{9d})₂), -OP(O)(R^{9c})(N(R^{9d})₂), - CH₂P(O)(R^{9c})(N(R^{9d})₂), -OCH₂P(O)(R^{9c})(N(R^{9d})₂), - C(O)OCH₂P(O)(R^{9c})(N(R^{9d})₂), or C₁₋₆ alkyl-heterocyclyl,
   wherein the alkyl or heterocyclyl is each optionally substituted with one to four halogens;
each R⁴ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₅ cycloalkyl, heterocyclyl, C₆₋₁₀ aryl, heteroaryl, oxo, -NO₂, -N₃, -CN, -OR^{10a}, -C(O)R^{10a}, -C(O)O-R^{10a}, -C(O)-N(R^{10a})(R^{10b}), -N(R^{10a})(R^{10b}), -N(R^{10a})₂(R^{10b})⁺, -N(R^{10a})C(O)-R^{10b}, -N(R^{10a})C(O)OR^{10b}, -N(R^{10a})C(O)N(R^{10b})(R^{10c}), -N(R^{10a})S(O)2(R^{10b}), -N R^{10a}S(O)2N(R^{10b})(R^{10c}), -NR^{10a}S(O)2O(R^{10b}), -OC(O)R^{10a}, -OC(O)O R^{10a}, -OC(O)-N(R^{10a})(R^{10b}), -S-R^{10a}, -S(O) R^{10a}, -S(O)(NH)R^{10a}, - S(O)₂R^{10a}, -S(O)₂N(R^{10a})(R^{10b}), -S(O)(NR^{10a}) R^{10b}, or -Si(R^{10a})₃;
   wherein each alkyl, haloalkyl, alkenyl, alkynyl, cycloalkyl, heterocyclyl, aryl, or heteroaryl is optionally substituted with one to four R⁵;
each R⁵ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, C₃₋₁₅ cycloalkyl, halogen, C₃₋₁₅ cycloalkyl, heterocyclyl, C₆₋₁₀ aryl, heteroaryl, oxo, -NO₂, -N₃, -CN, -O- R^{10a}, -C(O)-R^{10a}, - C(O)OR^{10a}, -C(O)N(R^{10a})(R^{10b}), - N(R^{10a})(R^{10b}), -N(R^{10a})C(O)R^{10b}, -N(R^{10a})C(O)OR^{10b}, -N(R^{10a})C(O)N(R^{10b})(R^{10c}), -N(R^{10a})S(O)2(R^{10b}), -NR^{10a}S(O)2N(R^{10b})(R^{10c}), -NR^{10a}S(O)2O(R^{10b}), -OC(O)R^{10a}, -OC(O)OR^{10a}, -OC(O)-N(R^{10a})(R^{10b}), -S-R^{10a}, -S(O)R^{10a}, -S(O)(NH)R^{10a}, -S(O)₂R^{10a}, -S(O)₂N(R^{10a})(R^{10b}), -S(O)(NR^{10a})R^{10b}, or -Si(R^{10a})₃,
   wherein each alkyl, haloalkyl, alkenyl, alkynyl, cycloalkyl, heterocyclyl, aryl, or heteroaryl is optionally substituted with one to four R⁶;
each R⁶ and R⁸ is independently C₁₋₉ alkyl, C₁₋₈ haloalkyl, C₂₋₆ alkenyl, C₂₋₆ alkynyl, halogen, C₃₋₁₅ cycloalkyl, heterocyclyl, C₆₋₁₀ aryl, heteroaryl, oxo, -OH, -CN, - NO₂, -NH₂, -N₃, -SH, -O(C₁₋₉ alkyl), -O(C₁₋₈ haloalkyl), -O(C₂₋₆ alkenyl), -O(C₂₋₆ alkynyl), -O(C₃₋₁₅ cycloalkyl), -O(heterocyclyl), -O(C₆₋₁₀ aryl), - O(heteroaryl), -NH(C₁₋₉ alkyl), -NH(C₁₋₈ haloalkyl), -NH(C₂₋₆ alkenyl), -NH(C₂₋₆ alkynyl), -NH(C₃₋₁₅ cycloalkyl), -NH(heterocyclyl), -NH(C₆₋₁₀ aryl), - NH(heteroaryl), -N(C₁₋₉ alkyl)₂, -N(C₁₋₈ haloalkyl)₂, -N(C₂₋₆ alkenyl)₂, -N(C₂₋₆ alkynyl)₂, -N(C₃₋₁₅ cycloalkyl)₂, -N(heterocyclyl)₂, -N(C₆₋₁₀ aryl)₂, - N(heteroaryl)₂, -N(C₁₋₉ alkyl)(C₁₋₈ haloalkyl), -N(C₁₋₉ alkyl)(C₂₋₆ alkenyl), -N(C₁₋₉ alkyl)(C₂₋₆ alkynyl), -N(C₁₋₉ alkyl)(C₃₋₁₅ cycloalkyl), -N(C₁₋₉ alkyl)(heterocyclyl), -N(C₁₋₉ alkyl)(C₆₋₁₀ aryl), -N(C₁₋₉ alkyl)(heteroaryl), -C(O)(C₁₋₉ alkyl), -C(O)(C₁₋₈ haloalkyl), -C(O)(C₂₋₆ alkenyl), - C(O)(C₂₋₆ alkynyl), -C(O)(C₃₋₁₅ cycloalkyl), -C(O)(heterocyclyl), -C(O)(C₆₋₁₀ aryl), -C(O)(heteroaryl), -C(O)O(C₁₋₉ alkyl), -C(O)O(C₁₋₈ haloalkyl), -C(O)O(C₂₋₆ alkenyl), -C(O)O(C₂₋₆ alkynyl), -C(O)O(C₃₋₁₅ cycloalkyl), - C(O)O(heterocyclyl), -C(O)O(C₆₋₁₀ aryl), -C(O)O(heteroaryl), -C(O)NH₂, -C(O)NH(C₁₋₉ alkyl), -C(O)NH(C₁₋₈ haloalkyl), -C(O)NH(C₂₋₆ alkenyl), -C(O)NH(C₂₋₆ alkynyl), -C(O)NH(C₃₋₁₅ cycloalkyl), -C(O)NH(heterocyclyl), -C(O)NH(C₆₋₁₀ aryl), -C(O)NH(heteroaryl), -C(O)N(C₁₋₉ alkyl)₂, -C(O)N(C₁₋₈ haloalkyl)₂, -C(O)N(C₂₋₆ alkenyl)₂, -C(O)N(C₂₋₆ alkynyl)₂, -C(O)N(C₃₋₁₅ cycloalkyl)₂, -C(O)N(heterocyclyl)₂, -C(O)N(C₆₋₁₀ aryl)₂, -C(O)N(heteroaryl)₂, -NHC(O)(C₁₋₉ alkyl), -NHC(O)(C₁₋₈ haloalkyl), -NHC(O)(C₂₋₆ alkenyl), -NHC(O)(C₂₋₆ alkynyl), -NHC(O)(C₃₋₁₅ cycloalkyl), -NHC(O)(heterocyclyl), -NHC(O)(C₆₋₁₀ aryl), -NHC(O)(heteroaryl), -NHC(O)O(C₁₋₉ alkyl), -NHC(O)O(C₁₋₈ haloalkyl), -NHC(O)O(C₂₋₆ alkenyl), -NHC(O)O(C₂₋₆ alkynyl), -NHC(O)O(C₃₋₁₅ cycloalkyl), -NHC(O)O(heterocyclyl),-NHC(O)O(C₆₋₁₀ aryl), -NHC(O)O(heteroaryl), -NHC(O)NH(C₁₋₉ alkyl), -NHC(O)NH(C₁₋₈ haloalkyl), -NHC(O)NH(C₂₋₆ alkenyl), -NHC(O)NH(C₂₋₆ alkynyl), -NHC(O)NH(C₃₋₁₅ cycloalkyl), -NHC(O)NH(heterocyclyl), -NHC(O)NH(C₆₋₁₀ aryl), -NHC(O)NH(heteroaryl), -NHS(O)(C₁₋₉ alkyl), -N(C₁₋₉ alkyl)(S(O)(C₁₋₉ alkyl), - S(C₁₋₉ alkyl), -S(C₁₋₈ haloalkyl), -S(C₂₋₆ alkenyl), -S(C₂₋₆ alkynyl), -S(C₃₋₁₅ cycloalkyl), -S(heterocyclyl), -S(C₆₋₁₀ aryl), -S(heteroaryl), -S(O)N(C₁₋₉ alkyl)₂, -S(O)(C₁₋₉ alkyl), -S(O)(C₁₋₈ haloalkyl), -S(O)(C₂₋₆ alkenyl), -S(O)(C₂₋₆ alkynyl), -S(O)(C₃₋₁₅ cycloalkyl), -S(O)(heterocyclyl), -S(O)(C₆₋₁₀ aryl), -S(O)(heteroaryl), -S(O)₂(C₁₋₉ alkyl), -S(O)₂(C₁₋₈ haloalkyl), - S(O)₂(C₂₋₆ alkenyl), -S(O)₂(C₂₋₆ alkynyl), -S(O)₂(C₃₋₁₅ cycloalkyl), -S(O)₂(heterocyclyl), -S(O)₂(C₆₋₁₀ aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
   wherein each alkyl, cycloalkyl, heterocyclyl, aryl, or heteroaryl is optionally substituted with 1 to 3 C₁₋₉ alkyl, C₁₋₈ haloalkyl, halogen, -OH, -NH₂, CO₂H,-O(C₁₋₉ alkyl), -O(C1-₈ haloalkyl), -O(C₃₋₁₅ cycloalkyl), - O(heterocyclyl), -O(aryl), -O(heteroaryl), -NH(C₁₋₉ alkyl), -NH(C₁₋₈ haloalkyl), -NH(C₃₋₁₅ cycloalkyl), -NH(heterocyclyl), -NH(aryl), - NH(heteroaryl), -N(C₁₋₉ alkyl)₂, -N(C₃₋₁₅ cycloalkyl)₂, -NHC(O)(C₁₋₈ haloalkyl), -NHC(O)(C₃₋₁₅ cycloalkyl), -NHC(O)(heterocyclyl), - NHC(O)(aryl), -NHC(O)(heteroaryl), -NHC(O)O(C₁₋₉ alkyl), -NHC(O)O(C₁₋₈ haloalkyl), -NHC(O)O(C₂₋₆ alkynyl), -NHC(O)O(C₃₋₁₅ cycloalkyl), -NHC(O)O(heterocyclyl), - NHC(O)O(aryl), -NHC(O)O(heteroaryl), -NHC(O)NH(C₁₋₉ alkyl), S(O)₂(C₁₋₉ alkyl), -S(O)₂(C₁₋₈ haloalkyl), -S(O)₂(C₃₋₁₅ cycloalkyl), - S(O)₂(heterocyclyl), -S(O)₂(aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
   wherein the alkyl or heterocyclyl is each optionally substituted with one to four halogens;
R^{6a} is H, C₁₋₆ alkyl, C₃₋₁₀ cycloalkyl, heterocyclyl, -S(O)₂R^{6a1}, or -S(O)₂N(R^{6a1})(NR^{6a2}), wherein the cycloalkyl or heterocyclyl is each optionally substituted with C₁₋₆ alkyl, F, or -CN;
each R^{6b} and R^{6c} is independently H, C₁₋₆ alkyl, C₁₋₆ haloalkyl, C₂₋₆ alkoxyalkyl, halogen, C₃₋₁₀ cycloalkyl, heterocyclyl, -C₁₋₆ alkyl-N(R^{9a})(R^{9b}), -CN, -OR^{6c1}, or - N(R^{6c2})(R^{6c3}),
   wherein the alkyl, cycloalkyl, or heterocyclyl is each optionally substituted with one to four R^{6b1}; or
R^{6b} and R^{6c} are combined with the atom to which they are attached to form a C₃₋₁₀ cycloalkyl or heterocyclyl, which is each optionally substituted with one to four R^{6b1} ; or
R^{6a} or R^{6c} is combined with one R⁴ group and the atoms to which they are attached to form a C₅₋₁₀ cycloalkyl or heterocyclyl, which is each optionally substituted with one to four R¹⁰;
each R^{6b1} and R¹⁰ is independently C₁₋₆ alkyl, C₁₋₆ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₀ cycloalkyl, heterocyclyl, C₆₋₁₀ aryl, heteroaryl, oxo, -OH, -CN, CO₂R^{3e}, -NO₂, or - C(O)N(R^{2a})(R^{2b}),
   wherein the heterocyclyl or heteroaryl is optionally substituted with C₁₋₆ alkyl, C₁₋₆ haloalkyl, or C₁₋₆ haloalkoxy;
each R^{6a1}, R^{6a2}, R^{6c1}, R^{6c2}, and R^{6c3} is independently H, C₁₋₆ alkyl or C₃₋₁₀ cycloalkyl;
each R^{9a} and R^{9b} is independently H, C₁₋₆ alkyl, or C₁₋₆ haloalkyl, or R^{9a} and R^{9b} together form a 6-membered heterocyclyl;
each R^{9c}, R^{9d}, R^{10a}, R^{10b}, and R^{10c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl,
   wherein the alkyl, alkenyl, alkynyl, cycloalkyl, heterocyclyl, aryl, or heteroaryl is each optionally substituted with one to four R⁶;
R^{a} and R^{b} are independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶;
optionally R^{a} and R^{b} are combined with the atom to which they are attached to form a 5- or 6-membered heterocyclyl, which is optionally substituted with one to four R⁶;
each R^{2a}, R^{2b}, R^{12a}, R^{12b}, and R^{12c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl
   wherein the alkyl, alkenyl, alkynyl, cycloalkyl, heterocyclyl, aryl, or heteroaryl is each optionally substituted with one to four R⁶;
each R^{3e} is independently H, C₁₋₆ alkyl, C₁₋₆ haloalkyl, -C₁₋₄alkyl-N(R^{9a})(R^{9b}), -C₁₋₄ alkyl-C(O)N(R^{9a})(R^{9b}), -C₁₋₄alkyl-O-C(O)-C₁₋₄alkyl, -C₁₋₄alkyl-O-C(O)-O-C₁₋₄alkyl, -C₁₋₄alkyl-O-C(O)-C₁₋₄alkyl-N(R^{9a})(R^{9b}), -C₁₋₄alkyl-C₃₋₈ cycloalkyl, -C₁₋₄ alkyl-heterocyclyl, C₃₋₁₀ cycloalkyl, heterocyclyl, C₆₋₁₀ aryl, heteroaryl, - P(O)(OR^{9c})₂, -CH2P(O)(OR^{9c})₂, -OCH₂P(O)(OR^{9c})₂, -C(O)OCH₂P(O)(OR^{9c})₂, - P(O)(R^{9c})(OR^{9d}), -OP(O)(R^{9c})(OR^{9d}), -CH₂P(O)(R^{9c})(OR^{9d}), - C(O)OCH₂P(O)(R^{9c})(OR^{9d}), -P(O)(N(R^{9c})₂)₂, -CH₂P(O)(N(R^{9c})₂)₂, - C(O)OCH₂P(O)(N(R^{9c})₂)₂, -P(O)(N(R^{9c})₂)(OR^{9d}), -CH₂P(O)(N(R^{9c})₂)(OR^{9d}), - C(O)OCH₂P(O)(N(R^{9c})₂)(OR^{9d}), -P(O)(R^{9c})(N(R^{9d})₂), -CH₂P(O)(R^{9c})(N(R^{9d} )₂), or -C(O)OCH₂P(O)(R^{9c})(N(R^{9d})₂);
   wherein the alkyl, alkenyl, cycloalkyl, heterocyclyl, aryl, or heteroaryl is each optionally substituted with one to four R⁶; wherein each heteroaryl has 5 to 12 ring members and has one to four heteroatoms each independently N, O, or S;
wherein each heterocyclyl has three to twelve ring members and has one to four heteroatoms, each independently N, O, or S; and
wherein each heteroaryl has five to twelve ring members and one to four heteroatoms, each independently N, O, or S.

In another embodiment, a pharmaceutical composition is provided comprising a therapeutically effective amount of a compound of Formula (I), or a pharmaceutically acceptable salt thereof, and a pharmaceutically acceptable excipient.

In some embodiments of the compound of Formula (I), or pharmaceutically acceptable salt thereof, having the structure of a compound in Table 2.

Also disclosed herein are the in vivo metabolic products of the compounds described herein, to the extent such products are novel and unobvious over the prior art. Such products may result for example from the oxidation, reduction, hydrolysis, amidation, esterification and the like of the administered compound, primarily due to enzymatic processes. Accordingly, included are novel and unobvious compounds produced by a process comprising contacting a compound with a mammal for a period of time sufficient to yield a metabolic product thereof. Such products typically are identified by preparing a radiolabeled (e.g. 14C or 3H) compound, administering it parenterally in a detectable dose (e.g. greater than about 0. 5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products can be easily isolated since they are labeled (others are isolated by the use of antibodies capable of binding epitopes surviving in the metabolite). The metabolite structures are determined in conventional fashion, e.g. by MS or NMR analysis. In general, analysis of metabolites can be done in the same way as conventional drug metabolism studies well-known to those skilled in the art. The conversion products, so long as they are not otherwise found in vivo, can be useful in diagnostic assays for therapeutic dosing of the compounds even if they possess no GLP-1R activity of their own.

Recipes and methods for determining stability of compounds in surrogate gastrointestinal secretions are known. Compounds are defined herein as stable in the gastrointestinal tract where less than about 50 mole percent of the protected groups are deprotected in surrogate intestinal or gastric juice upon incubation for 1 hour at 37 °C. Simply because the compounds are stable to the gastrointestinal tract does not mean that they cannot be hydrolyzed in vivo. The prodrugs typically will be stable in the digestive system but may be substantially hydrolyzed to the parental drug in the digestive lumen, liver, lung or other metabolic organ, or within cells in general. As used herein, a prodrug is understood to be a compound that is chemically designed to efficiently liberate the parent drug after overcoming biological barriers to oral delivery.

### BRIEF DESCRIPTION OF THE DRAWINGS

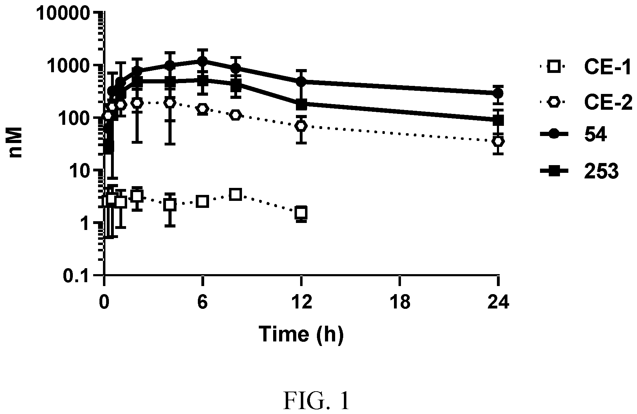

FIG. 1: Graph depicting plasma concentration as a function of time in cynomolgus monkey.

### DETAILED DESCRIPTION

### I. DEFINITIONS

Unless defined otherwise, all technical and scientific terms used herein have the same meaning as commonly understood by one of ordinary skill in the art. A dash at the front or end of a chemical group is a matter of convenience to indicate the point of attachment to a parent moiety; chemical groups may be depicted with or without one or more dashes without losing their ordinary meaning. A prefix such as "Cᵤ₋ᵥ" or "Cᵤ-Cᵥ" indicates that the following group has from u to v carbon atoms, where u and v are integers. For example, "C₁₋₆ alkyl" or "C₁-C₆ alkyl" indicates that the alkyl group has from 1 to 6 carbon atoms.

"Alkyl" is a monovalent or divalent linear or branched saturated hydrocarbon radical. For example, an alkyl group can have 1 to 10 carbon atoms (i.e. , C₁₋₁₀ alkyl) or 1 to 8 carbon atoms (i.e. , C₁₋₈ alkyl) or 1 to 6 carbon atoms (i.e. , C₁₋₆ alkyl) or 1 to 4 carbon atoms (i.e. , C₁₋₄ alkyl). Examples of alkyl groups include, but are not limited to, methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, and octyl (-(CH₂)₇CH₃). Alkyl groups can be unsubstituted or substituted.

"Alkoxy" refers to the group -O-alkyl, where alkyl is as defined above. For example, C₁₋₄ alkoxy refers to an -O-alkyl group having 1 to 4 carbons. Alkoxy groups can be unsubstituted or substituted.

"Alkoxyalkyl" is an alkoxy group attached to an alkyl as defined above, such that the alkyl is divalent. For example, C₂₋₆ alkoxyalkyl includes -CH₂-OMe, -CH₂-O-iPr, -CH₂-CH₂-OMe, -CH₂-CH₂-O-CH₂-CH₃, and -CH₂-CH₂-O-tBu. Alkoxyalkyl groups can be unsubstituted or substituted.

"Hydroxyalkyl" is a hydroxy group attached to an alkyl as defined above, such that the alkyl is divalent. For example, C₁₋₆ hydroxyalkyl includes -CH₂-OH, and -CH₂-CH₂-OH. Hydroxyalkyl groups can be unsubstituted or substituted.

"Alkenyl" is a monovalent or divalent linear or branched hydrocarbon radical with at least one carbon-carbon double bond. For example, an alkenyl group can have 2 to 8 carbon atoms (i.e. , C₂₋₈ alkenyl) or 2 to 6 carbon atoms (i.e. , C₂₋₆ alkenyl) or 2 to 4 carbon atoms (i.e. , C₂₋₄ alkenyl). Examples of alkenyl groups include, but are not limited to, ethenyl (-CH=CH₂), allyl (-CH₂CH=CH₂), and -CH₂-CH=CH-CH₃. Alkenyl groups can be unsubstituted or substituted.

"Alkynyl" is a monovalent or divalent linear or branched hydrocarbon radical with at least one carbon-carbon triple bond. For example, an alkynyl group can have 2 to 8 carbon atoms (i.e., C₂₋₈ alkynyl) or 2 to 6 carbon atoms (i.e., C₂₋₆ alkynyl) or 2 to 4 carbon atoms (i.e. , C₂₋₄ alkynyl). Examples of alkynyl groups include, but are not limited to, acetylenyl (-C=CH), propargyl (-CH₂C≡CH), and -CH₂-C≡C-CH₃. Alkynyl groups can be unsubstituted or substituted.

"Halogen" refers to fluoro (-F), chloro (-Cl), bromo (-Br) and iodo (-I).

"Haloalkyl" is an alkyl as defined herein, wherein one or more hydrogen atoms of the alkyl are independently replaced by a halogen, which may be the same or different, such that the alkyl is divalent. The alkyl group and the halogen can be any of those described above. In some embodiments, the haloalkyl defines the number of carbon atoms in the alkyl portion, e.g., C₁₋₄ haloalkyl includes CF₃, CH₂F, CHF₂, CH₂CF₃, CH₂CH₂CF₃, CCl₂CH₂CH₂CH₃, and C(CH₃)₂(CF₂H). Haloalkyl groups can be unsubstituted or substituted.

"Haloalkoxy" is an alkoxy as defined herein, wherein one or more hydrogen atoms of the alkyl in the alkyoxy are independently replaced by a halogen, which may be the same or different, such that the alkyl is divalent. The alkoxy group and the halogen can be any of those described above. In some embodiments, the haloalkoxy defines the number of carbon atoms in the alkyl portion, *e.g.,* C₁₋₄ haloalkoxy includes OCF₃, OCH₂F, OCH₂CF₃, OCH₂CH₂CF₃, OCCl₂CH₂CH₂CH₃, and OC(CH₃)₂(CF₂H). Haloalkoxy groups can be unsubstituted or substituted.

"Cycloalkyl" is a monovalent or divalent single all carbon ring or a multiple condensed all carbon ring system wherein the ring in each instance is a non-aromatic saturated or unsaturated ring. For example, in some embodiments, a cycloalkyl group has 3 to 12 carbon atoms, 3 to 10 carbon atoms, 3 to 8 carbon atoms, 3 to 6 carbon atoms, 3 to 5 carbon atoms, or 3 to 4 carbon atoms. Exemplary single ring cycloalkyl groups include cyclopropyl, cyclobutyl, cyclopentyl, cyclohexyl, cyclohexenyl, cycloheptyl, cycloheptenyl, and cyclooctyl. Cycloalkyl also includes multiple condensed ring systems (e.g., ring systems comprising 2 rings) having about 7 to 12 carbon atoms. The rings of the multiple condensed ring system can be connected to each other via fused, spiro, or bridged bonds when allowed by valency requirements. Exemplary multiple ring cycloalkyl groups include octahydropentalene, bicyclo[2. 2. 1]heptane, bicyclo[2. 2. 2]octane, bicyclo[2. 2. 2]oct-2-ene, and spiro[2. 5]octane. Cycloalkyl groups can be unsubstituted or substituted.

"Alkylcycloalkyl" refers to an alkyl as defined herein, wherein one or more hydrogen atoms of the alkyl are independently replaced by a cycloalkyl group, which may be the same or different. The alkyl group and the cycloalkyl group can be any of those described above. In some embodiments, the number of carbon atoms in the alkyl and cycloalkyl portion can be designated separately, e.g., C₁₋₆ alkyl-C₃₋₁₂ cycloalkyl. Alkylcycloalkyl groups can be unsubstituted or substituted.

"Aryl" as used herein refers to a monovalent or divalent single all carbon aromatic ring or a multiple condensed all carbon ring system wherein the ring is aromatic. For example, in some embodiments, an aryl group has 6 to 20 carbon atoms, 6 to 14 carbon atoms, 6 to 12 carbon atoms, or 6 to 10 carbon atoms. Aryl includes a phenyl radical. Aryl also includes multiple condensed ring systems (e.g., ring systems comprising 2, 3 or 4 rings) having about 9 to 20 carbon atoms in which multiple rings are aromatic. The rings of the multiple condensed ring system can be connected to each other via fused, spiro, or bridged bonds when allowed by valency requirements. It is also understood that when reference is made to a certain atom-range membered aryl (e.g., 6-10 membered aryl), the atom range is for the total ring atoms of the aryl. For example, a 6-membered aryl would include phenyl and a 10-membered aryl would include naphthyl. Non-limiting examples of aryl groups include, but are not limited to, phenyl, naphthyl, anthracenyl, and the like. Aryl groups can be unsubstituted or substituted.

"Alkylaryl" refers to an alkyl as defined herein, wherein one or more hydrogen atoms of the alkyl are independently replaced by an aryl group, which may be the same or different. The alkyl group and the aryl group can be any of those described above, such that the alkyl is divalent. In some embodiments, an alkylaryl group has 7 to 24 carbon atoms, 7 to 16 carbon atoms, 7 to 13 carbon atoms, or 7 to 11 carbon atoms. An alkylaryl group defined by the number of carbon atoms refers to the total number of carbon atoms present in the constitutive alkyl and aryl groups combined. For example, C₇ alkylaryl refers to benzyl, while C₁₁ alkylaryl includes 1-methylnaphthyl and n-pentylphenyl. In some embodiments the number of carbon atoms in the alkyl and aryl portion can be designated separately, *e.g*., C₁₋₆ alkyl-C₆₋₁₀ aryl. Non-limiting examples of alkylaryl groups include, but are not limited to, benzyl, 2,2-dimethylphenyl, n-pentylphenyl, 1-methylnaphthyl, 2-ethylnaphthyl, and the like. Alkylaryl groups can be unsubstituted or substituted.

"Heterocyclyl" or "heterocycle" or "heterocycloalkyl" as used herein refers to a single saturated or partially unsaturated non-aromatic ring or a non-aromatic multiple ring system that has at least one heteroatom in the ring (i.e. , at least one annular (i.e., ring-shaped) heteroatom selected from oxygen, nitrogen, and sulfur). Unless otherwise specified, a heterocyclyl group has from 3 to about 20 annular atoms, for example from 3 to 12 annular atoms, for example from 4 to 12 annular atoms, 4 to 10 annular atoms, or 3 to 8 annular atoms, or 3 to 6 annular atoms, or 3 to 5 annular atoms, or 4 to 6 annular atoms, or 4 to 5 annular atoms. Thus, the term includes single saturated or partially unsaturated rings (*e.g.,* 3, 4, 5, 6 or 7-membered rings) having from about 1 to 6 annular carbon atoms and from about 1 to 3 annular heteroatoms selected from the group consisting of oxygen, nitrogen and sulfur in the ring. The rings of the multiple condensed ring (*e.g*. bicyclic heterocyclyl) system can be connected to each other via fused, spiro and bridged bonds when allowed by valency requirements. Heterocycles include, but are not limited to, azetidine, aziridine, imidazolidine, morpholine, oxirane (epoxide), oxetane, thietane, piperazine, piperidine, pyrazolidine, piperidine, pyrrolidine, pyrrolidinone, tetrahydrofuran, tetrahydrothiophene, dihydropyridine, tetrahydropyridine, quinuclidine, 2-oxa-6-azaspiro[3. 3]heptan-6-yl, 6-oxa-1-azaspiro[3. 3]heptan-1-yl, 2-thia-6-azaspiro[3. 3]heptan-6-yl, 2,6-diazaspiro[3. 3]heptan-2-yl, 2-azabicyclo[3. 1. 0]hexan-2-yl, 3-azabicyclo[3. 1. 0]hexanyl, 2-azabicyclo[2. 1. 1]hexanyl, 2-azabicyclo[2. 2. 1]heptan-2-yl, 4-azaspiro[2. 4]heptanyl, 5-azaspiro[2. 4]heptanyl, and the like. Heterocyclyl groups can be unsubstituted or substituted.

"Alkylheterocyclyl" refers to an alkyl as defined herein, wherein one or more hydrogen atoms of the alkyl are independently replaced by a heterocyclyl group, which may be the same or different. The alkyl group and the heterocyclyl group can be any of those described above, such that the alkyl is divalent. In some embodiments, the number of atoms in the alkyl and heterocyclyl portion can be designated separately, *e.g*., C₁₋₆ alkyl-3 to 12 membered heterocyclyl having one to three heteroatoms each independently N, O, or S. Alkylheterocyclyl groups can be unsubstituted or substituted.

"Heteroaryl" refers to a single aromatic ring that has at least one atom other than carbon in the ring, wherein the atom is selected from the group consisting of oxygen, nitrogen and sulfur; "heteroaryl" also includes multiple condensed ring systems that have at least one such aromatic ring, which multiple condensed ring systems are further described below. Thus, "heteroaryl" includes single aromatic rings of from about 1 to 6 carbon atoms and about 1-4 heteroatoms selected from the group consisting of oxygen, nitrogen and sulfur. The sulfur and nitrogen atoms may also be present in an oxidized form provided the ring is aromatic. Exemplary heteroaryl ring systems include but are not limited to pyridyl, pyrimidinyl, oxazolyl or furyl. "Heteroaryl" also includes multiple condensed ring systems (*e.g*., ring systems comprising 2, 3 or 4 rings) wherein a heteroaryl group, as defined above, is condensed with one or more rings selected from heteroaryls (to form for example 1,8-naphthyridinyl) and aryls (to form, for example, benzimidazolyl or indazolyl) to form the multiple condensed ring system. Thus, a heteroaryl (a single aromatic ring or multiple condensed ring system) can have about 1-20 carbon atoms and about 1-6 heteroatoms within the heteroaryl ring. For example, tetrazolyl has 1 carbon atom and 4 nitrogen heteroatoms within the ring. The rings of the multiple condensed ring system can be connected to each other via fused, spiro, or bridged bonds when allowed by valency requirements. It is to be understood that the individual rings of the multiple condensed ring system may be connected in any order relative to one another. It is to be understood that the point of attachment for a heteroaryl or heteroaryl multiple condensed ring system can be at any suitable atom of the heteroaryl or heteroaryl multiple condensed ring system including a carbon atom and a heteroatom (*e.g*., a nitrogen). It also to be understood that when a reference is made to a certain atom-range membered heteroaryl (*e.g*., a 5 to 10 membered heteroaryl), the atom range is for the total ring atoms of the heteroaryl and includes carbon atoms and heteroatoms. It is also to be understood that the rings of the multiple condensed ring system may include an aryl ring fused to a heterocyclic ring with saturated or partially unsaturated bonds (*e.g.,* 3, 4, 5, 6 or 7-membered rings) having from about 1 to 6 annular carbon atoms and from about 1 to 3 annular heteroatoms selected from the group consisting of oxygen, nitrogen and sulfur in the ring. For example, a 5-membered heteroaryl includes thiazolyl and a 10-membered heteroaryl includes quinolinyl. Exemplary heteroaryls include but are not limited to pyridyl, pyrrolyl, pyrazinyl, pyrimidinyl, pyridazinyl, pyrazolyl, thienyl, indolyl, imidazolyl, oxazolyl, isoxazolyl, thiazolyl, furyl, oxadiazolyl, thiadiazolyl, quinolyl, isoquinolyl, benzothiazolyl, benzoxazolyl, indazolyl, quinoxalyl, quinazolyl, benzofuranyl, benzimidazolyl, thianaphthenyl, pyrrolo[2,3-b]pyridinyl, quinazolinyl-4(3H)-one, triazolyl, and tetrazolyl. Heteroaryl groups can be unsubstituted or substituted.

"Alkylheteroaryl" refers to an alkyl as defined herein, wherein one or more hydrogen atoms of the alkyl are independently replaced by a heteroaryl group, which may be the same or different, such that the alkyl is divalent. The alkyl group and the heteroaryl group can be any of those described above. In some embodiments, the number of atoms in the alkyl and heteroaryl portion are designated separately, *e.g*., C₁₋₆ alkyl-5 to 10 membered heteroaryl having one to four heteroatoms each independently N, O, or S. Alkylheteroaryl groups can be unsubstituted or substituted.

"Oxo" as used herein refers to =O.

"Substituted" as used herein refers to wherein one or more hydrogen atoms of the group are independently replaced by one or more substituents (e.g., 1, 2, 3, or 4 or more) as indicated.

A "compound of the present disclosure" includes compounds disclosed herein, for example a compound of the present disclosure includes compounds of Formula (I), including the compounds of the Examples. In some embodiments, a "compound of the present disclosure" includes compounds of Formula (I).

"Pharmaceutically acceptable excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Therapeutically effective amount" or "effective amount" as used herein refers to an amount that is effective to elicit the desired biological or medical response, including the amount of a compound that, when administered to a subject for treating a disease, is sufficient to affect such treatment for the disease. The effective amount will vary depending on the compound, the disease, and its severity and the age, weight, etc., of the subject to be treated. The effective amount can include a range of amounts. As is understood in the art, an effective amount may be in one or more doses, i.e., a single dose or multiple doses may be required to achieve the desired treatment endpoint. An effective amount may be considered in the context of administering one or more therapeutic agents, and a single agent may be considered to be given in an effective amount if, in conjunction with one or more other agents, a desirable or beneficial result may be or is achieved. Suitable doses of any co-administered compounds may optionally be lowered due to the combined action (e.g., additive or synergistic effects) of the compounds.

"Co-administration" as used herein refers to administration of unit dosages of the compounds disclosed herein before or after administration of unit dosages of one or more additional therapeutic agents, for example, administration of the compound disclosed herein within seconds, minutes, or hours of the administration of one or more additional therapeutic agents. For example, in some embodiments, a unit dose of a compound of the present disclosure is administered first, followed within seconds or minutes by administration of a unit dose of one or more additional therapeutic agents. Alternatively, in other embodiments, a unit dose of one or more additional therapeutic agents is administered first, followed by administration of a unit dose of a compound of the present disclosure within seconds or minutes. In some embodiments, a unit dose of a compound of the present disclosure is administered first, followed, after a period of hours (e.g., 1-12 hours), by administration of a unit dose of one or more additional therapeutic agents. In other embodiments, a unit dose of one or more additional therapeutic agents is administered first, followed, after a period of hours (e.g., 1-12 hours), by administration of a unit dose of a compound of the present disclosure. Co-administration of a compound disclosed herein with one or more additional therapeutic agents generally refers to simultaneous or sequential administration of a compound disclosed herein and one or more additional therapeutic agents, such that therapeutically effective amounts of each agent are present in the body of the subj ect.

Provided are also pharmaceutically acceptable salts, hydrates, solvates, tautomeric forms, polymorphs, and prodrugs of the compounds described herein. "Pharmaceutically acceptable" or "physiologically acceptable" refer to compounds, salts, compositions, dosage forms and other materials which are useful in preparing a pharmaceutical composition that is suitable for veterinary or human pharmaceutical use.

The compounds described herein may be prepared and/or formulated as pharmaceutically acceptable salts or when appropriate as a free base. Pharmaceutically acceptable salts are nontoxic salts of a free base form of a compound that possesses the desired pharmacological activity of the free base. These salts may be derived from inorganic or organic acids or bases. For example, a compound that contains a basic nitrogen may be prepared as a pharmaceutically acceptable salt by contacting the compound with an inorganic or organic acid. Non-limiting examples of pharmaceutically acceptable salts include sulfates, pyrosulfates, bisulfates, sulfites, bisulfites, phosphates, monohydrogen-phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, methylsulfonates, propylsulfonates, besylates, xylenesulfonates, naphthalene-1-sulfonates, naphthalene-2-sulfonates, phenylacetates, phenylpropionates, phenylbutyrates, citrates, lactates, γ-hydroxybutyrates, glycolates, tartrates, and mandelates. Lists of other suitable pharmaceutically acceptable salts are found in Remington: The Science and Practice of Pharmacy, 21 st Edition, Lippincott Wiliams and Wilkins, Philadelphia, Pa., 2006.

Examples of "pharmaceutically acceptable salts" of the compounds disclosed herein also include salts derived from an appropriate base, such as an alkali metal (for example, sodium, potassium), an alkaline earth metal (for example, magnesium), ammonium and N(C₁≡C₄ alkyl)₄₊. Also included are base addition salts, such as sodium or potassium salts.

Provided are also compounds described herein or pharmaceutically acceptable salts, isomers, or a mixture thereof, in which from 1 to n hydrogen atoms attached to a carbon atom may be replaced by a deuterium atom or D, in which n is the number of hydrogen atoms in the molecule. As known in the art, the deuterium atom is a non-radioactive isotope of the hydrogen atom. Such compounds may increase resistance to metabolism, and thus may be useful for increasing the half-life of the compounds described herein or pharmaceutically acceptable salts, isomer, or a mixture thereof when administered to a mammal. See, e.g., Foster, "Deuterium Isotope Effects in Studies of Drug Metabolism", Trends Pharmacol. Sci., 5(12):524-527 (1984). Such compounds are synthesized by means well known in the art, for example by employing starting materials in which one or more hydrogen atoms have been replaced by deuterium.

Examples of isotopes that can be incorporated into the disclosed compounds also include isotopes of hydrogen, carbon, nitrogen, oxygen, phosphorous, fluorine, chlorine, and iodine, such as ²H , ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of Formula (I) can generally be prepared by conventional techniques known to those skilled in the art or by processes analogous to those described in the Examples as set out below using an appropriate isotopically-labeled reagent in place of the non-labeled reagent previously employed.

The compounds of the embodiments disclosed herein, or their pharmaceutically acceptable salts may contain one or more asymmetric centers and may thus give rise to enantiomers, diastereomers, and other stereoisomeric forms that may be defined, in terms of absolute stereochemistry, as (*R*)- or (*S*)- or, as (D)- or (L)- for amino acids. The present disclosure is meant to include all such possible isomers, as well as their racemic and optically pure forms. Optically active (+) and (-), (R)- and (5)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centers of geometric asymmetry, and unless specified otherwise, it is intended that the compounds include both E and Z geometric isomers. Likewise, all tautomeric forms are also intended to be included. Where compounds are represented in their chiral form, it is understood that the embodiment encompasses, but is not limited to, the specific diastereomerically or enantiomerically enriched form. Where chirality is not specified but is present, it is understood that the embodiment is directed to either the specific diastereomerically or enantiomerically enriched form; or a racemic or scalemic mixture of such compound(s). As used herein, "scalemic mixture" is a mixture of stereoisomers at a ratio other than 1: 1.

"Stereoisomer" as used herein refers to a compound made up of the same atoms bonded by the same bonds but having different three-dimensional structures, which are not interchangeable. The present disclosure contemplates various stereoisomers and mixtures thereof and includes "enantiomers", which refers to two stereoisomers whose molecules are non-superimposable mirror images of one another.

"Tautomer" as used herein refers to a proton shift from one atom of a molecule to another atom of the same molecule. In some embodiments, the present disclosure includes tautomers of said compounds.

"Solvate" as used herein refers to the result of the interaction of a solvent and a compound. Solvates of salts of the compounds described herein are also provided. Hydrates of the compounds described herein are also provided.

"Hydrate" as used herein refers to a compound of the disclosure that is chemically associated with one or more molecules of water.

"Prevention" or "preventing" means any treatment of a disease or condition that causes the clinical symptoms of the disease or condition not to develop. Compounds may, in some embodiments, be administered to a subject (including a human) who is at risk or has a family history of the disease or condition.

"Prodrug" as used herein refers to a derivative of a drug that upon administration to the human body is converted to the parent drug according to some chemical or enzymatic pathway. In some embodiments, a prodrug is a biologically inactive derivative of a drug that upon administration to the human body is converted to the biologically active parent drug according to some chemical or enzymatic pathway.

"Treatment" or "treat" or "treating" as used herein refers to an approach for obtaining beneficial or desired results. For purposes of the present disclosure, beneficial or desired results include, but are not limited to, alleviation of a symptom and/or diminishment of the extent of a symptom and/or preventing a worsening of a symptom associated with a disease or condition. In one embodiment, "treatment" or "treating" includes one or more of the following: a) inhibiting the disease or condition (e.g., decreasing one or more symptoms resulting from the disease or condition, and/or diminishing the extent of the disease or condition); b) slowing or arresting the development of one or more symptoms associated with the disease or condition (e.g., stabilizing the disease or condition, delaying the worsening or progression of the disease or condition); and c) relieving the disease or condition, e.g., causing the regression of clinical symptoms, ameliorating the disease state, delaying the progression of the disease, increasing the quality of life, and/or prolonging survival. "At risk individual" as used herein refers to an individual who is at risk of developing a condition to be treated. An individual "at risk" may or may not have detectable disease or condition and may or may not have displayed detectable disease prior to the treatment of methods described herein. "At risk" denotes that an individual has one or more so-called risk factors, which are measurable parameters that correlate with development of a disease or condition and are known in the art. An individual having one or more of these risk factors has a higher probability of developing the disease or condition than an individual without these risk factor(s).

### II. COMPOUNDS

In some embodiments the compound of the present disclosure is a compound of Formula (I): 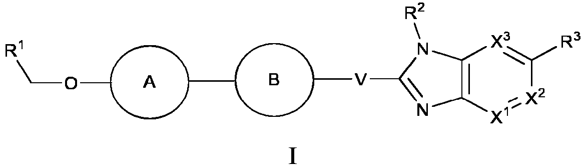 or a pharmaceutically acceptable salt thereof, wherein
R¹ is
   i) 5-membered heteroaryl, optionally substituted with one to four R⁴;
   ii) 5-membered heteroaryl fused to a heteroaryl or aryl to form a heteroaryl ring system, wherein the heteroaryl ring system is optionally substituted with one to four R⁴;
   iii) 5-membered heteroaryl substituted with two R⁴ groups attached to adjacent ring atoms and optionally substituted with one or two additional R⁴, wherein the two R⁴ groups attached to adjacent ring atoms are combined with the atoms to which they are attached to form a C₅₋₁₀ cycloalkyl or heterocyclyl, which is each optionally substituted with one to four R⁶;
   iv) 6-membered heteroaryl or phenyl substituted with C₂-alkynyl, wherein the 6-membered heteroaryl or phenyl is optionally substituted with one to three R⁴, wherein the C₂-alkynyl is substituted with -C(CH₃)₂SO₂CH₃, aryl, heteroaryl, or heterocyclyl, wherein the aryl, heteroaryl or heterocyclyl is optionally substituted with one to four R⁵;
   v) 6-membered heteroaryl or phenyl substituted with -C(O)NR^{a}R^{b}, wherein the heteroaryl or phenyl is optionally substituted with one to four R⁴; or
   vi) 6- membered heteroaryl or phenyl, wherein the 6-membered heteroaryl or phenyl is substituted with heteroaryl, C₃₋₁₀ cycloalkyl, or heterocycle and optionally substituted with one to three R⁴, wherein the heteroaryl, C₃₋₁₀ cycloalkyl, or heterocycle is each substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl, wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶;
X¹, X², and X³ are each independently -C(H)=, or -C(R⁸)=;
ring A is 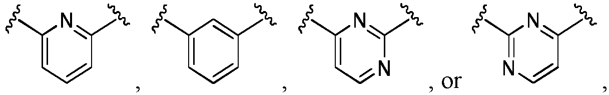 each optionally substituted with one to three Z^{1a} groups, each independently C₁₋₆ alkyl, C₁₋₆ haloalkyl, C₁₋₆ alkoxy, C₁₋₆ haloalkoxy, halogen, -OH, or -CN;
ring B is C₆₋₁₀ aryl or heteroaryl, which is each optionally substituted with one to four R⁴; V is -C(O)-, -O-, -N(R^{6a})-, or -C(R^{6b})(R^{6c})-;
R² is H, C₁₋₆ alkyl, C₂₋₆ alkynyl, C₁₋₆ haloalkyl, C₃₋₁₀ cycloalkyl, or heterocyclyl,
   wherein the alkyl, alkynyl, cycloalkyl, or heterocyclyl is each optionally substituted with one to four Z¹, wherein each Z¹ is independently C₁₋₆ alkyl, C₁₋₆ alkoxy, C₁₋₆ hydroxyalkyl, C₂₋₆ alkoxyalkyl, halogen, C₁₋₆ haloalkyl, C₁₋₆ haloalkoxy, -OH, -CN, C₁₋₆ alkyl-CN, -O-C₃₋₆ cycloalkyl, heterocyclyl, C₃₋₁₀ cycloalkyl, or heteroaryl optionally substituted with 1 to 4 groups each independently C₁₋₆ alkyl, C₁₋₆ alkoxy, halogen, -CN, C₁₋₆ haloalkyl, or C₁₋₆ haloalkoxy;
R³ is -C(O)OR^{3a};
R^{3a} is H, C₁₋₄alkyl-N(R^{9a})(R^{9b}), -C₁₋₄alkyl-N(R^{9a})C(O)-O-C₁₋₄alkyl-OP(O)(OR^{9c})₂, C₁₋₄ alkyl-C(O)N(R^{9a})(R^{9b}), -C₁₋₄alkyl-O-C(O)-C₁₋₄alkyl, -C₁₋₄alkyl-O-C(O)-O-C₁₋₄alkyl, -C₁₋₄alkyl-O-C(O)-C₁₋₄alkyl-N(R^{9a})(R^{9b}), -C₁₋₄alkyl-O-C(O)-C₁₋₄alkyl-OP(O)(OR^{9c})₂, -CH₂CH(N(R^{9a})₂)C(O)OR^{9b}, -P(O)(OR^{9c})₂, -OP(O)(OR^{9c})₂, - CH₂P(O)(OR^{9c})₂, -CH₂OP(O)(OR^{9c})₂, -OCH₂P(O)(OR^{9c})₂, C(O)OCH₂P(O)(OR^{9c})₂, -P(O)(R^{9c})(OR^{9d}), -OP(O)(R^{9c})(OR^{9d}), - CH₂P(O)(R^{9c})(OR^{9d}), -OCH₂P(O)(R^{9c})(OR^{9d}), -C(O)OCH₂P(O)(R^{9c})(OR^{9d}), - P(O)(N(R^{9c})₂)₂, -OP(O)(N(R^{9c})₂)₂, -CH₂P(O)(N(R^{9c})₂)₂, -OCH₂P(O)(N(R^{9c})₂)₂, - C(O)OCH₂P(O)(N(R^{9c})₂)₂, -P(O)(N(R^{9c})₂)(OR^{9d}), -OP(O)(N(R^{9c})₂)(OR^{9d}), - CH₂P(O)(N(R^{9c})₂)(OR^{9d}), -OCH₂P(O)(N(R^{9c})₂)(OR^{9d}), - C(O)OCH₂P(O)(N(R^{9c})₂)(OR^{9d}), -P(O)(R^{9c})(N(R^{9d})₂), -OP(O)(R^{9c})(N(R^{9d})₂), - CH₂P(O)(R^{9c})(N(R^{9d})₂), -OCH₂P(O)(R^{9c})(N(R^{9d})₂), - C(O)OCH₂P(O)(R^{9c})(N(R^{9d})₂), or C₁₋₆ alkyl-heterocyclyl,
   wherein the alkyl or heterocyclyl is each optionally substituted with one to four halogens;
each R⁴ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₅ cycloalkyl, heterocyclyl, C₆₋₁₀ aryl, heteroaryl, oxo, -NO₂, -N₃, -CN, -OR^{10a}, -C(O)R^{10a}, -C(O)O-R^{10a}, -C(O)-N(R^{10a})(R^{10b}), -N(R^{10a})(R^{10b}), -N(R^{10a})₂(R^{10b})⁺, -N(R^{10a})C(O)-R^{10b}, -N(R^{10a})C(O)OR^{10b}, -N(R^{10a})C(O)N(R^{10b})(R^{10c}), -N(R^{10a})S(O)2(R^{10b}), -N R^{10a}S(O)2N(R^{10b})(R^{10c}), -NR^{10a}S(O)2O(R^{10b}), -OC(O)R^{10a}, -OC(O)O R^{10a}, -OC(O)-N(R^{10a})(R^{10b}), -S-R^{10a}, -S(O) R^{10a}, -S(O)(NH)R^{10a}, - S(O)₂R^{10a}, -S(O)₂N(R^{10a})(R^{10b}), -S(O)(NR^{10a}) R^{10b}, or -Si(R^{10a})₃;
   wherein each alkyl, haloalkyl, alkenyl, alkynyl, cycloalkyl, heterocyclyl, aryl, or heteroaryl is optionally substituted with one to four R⁵;
each R⁵ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, C₃₋₁₅ cycloalkyl, halogen, C₃₋₁₅ cycloalkyl, heterocyclyl, C₆₋₁₀ aryl, heteroaryl, oxo, -NO₂, -N₃, -CN, -O- R^{10a}, -C(O)-R^{10a}, - C(O)OR^{10a}, -C(O)N(R^{10a})(R^{10b}), - N(R^{10a})(R^{10b}), -N(R^{10a})C(O)R^{10b}, -N(R^{10a})C(O)OR^{10b}, -N(R^{10a})C(O)N(R^{10b})(R^{10c}), -N(R^{10a})S(O)2(R^{10b}), -NR^{10a}S(O)2N(R^{10b})(R^{10c}), -NR^{10a}S(O)2O(R^{10b}), -OC(O)R^{10a}, -OC(O)OR^{10a}, -OC(O)-N(R^{10a})(R^{10b}), -S-R^{10a}, -S(O)R^{10a}, -S(O)(NH)R^{10a}, -S(O)₂R^{10a}, -S(O)₂N(R^{10a})(R^{10b}), -S(O)(NR^{10a})R^{10b}, or -Si(R^{10a})₃,
   wherein each alkyl, haloalkyl, alkenyl, alkynyl, cycloalkyl, heterocyclyl, aryl, or heteroaryl is optionally substituted with one to four R⁶;
each R⁶ and R⁸ is independently C₁₋₉ alkyl, C₁₋₈ haloalkyl, C₂₋₆ alkenyl, C₂₋₆ alkynyl, halogen, C₃₋₁₅ cycloalkyl, heterocyclyl, C₆₋₁₀ aryl, heteroaryl, oxo, -OH, -CN, - NO₂, -NH₂, -N₃, -SH, -O(C₁₋₉ alkyl), -O(C₁₋₈ haloalkyl), -O(C₂₋₆ alkenyl), -O(C₂₋₆ alkynyl), -O(C₃₋₁₅ cycloalkyl), -O(heterocyclyl), -O(C₆₋₁₀ aryl), - O(heteroaryl), -NH(C₁₋₉ alkyl), -NH(C₁₋₈ haloalkyl), -NH(C₂₋₆ alkenyl), -NH(C₂₋₆ alkynyl), -NH(C₃₋₁₅ cycloalkyl), -NH(heterocyclyl), -NH(C₆₋₁₀ aryl), - NH(heteroaryl), -N(C₁₋₉ alkyl)₂, -N(C₁₋₈ haloalkyl)₂, -N(C₂₋₆ alkenyl)₂, -N(C₂₋₆ alkynyl)₂, -N(C₃₋₁₅ cycloalkyl)₂, -N(heterocyclyl)₂, -N(C₆₋₁₀ aryl)₂, - N(heteroaryl)₂, -N(C₁₋₉ alkyl)(C₁₋₈ haloalkyl), -N(C₁₋₉ alkyl)(C₂₋₆ alkenyl), -N(C₁₋₉ alkyl)(C₂₋₆ alkynyl), -N(C₁₋₉ alkyl)(C₃₋₁₅ cycloalkyl), -N(C₁₋₉ alkyl)(heterocyclyl), -N(C₁₋₉ alkyl)(C₆₋₁₀ aryl), -N(C₁₋₉ alkyl)(heteroaryl), -C(O)(C₁₋₉ alkyl), -C(O)(C₁₋₈ haloalkyl), -C(O)(C₂₋₆ alkenyl), - C(O)(C₂₋₆ alkynyl), -C(O)(C₃₋₁₅ cycloalkyl), -C(O)(heterocyclyl), -C(O)(C₆₋₁₀ aryl), -C(O)(heteroaryl), -C(O)O(C₁₋₉ alkyl), -C(O)O(C₁₋₈ haloalkyl), -C(O)O(C₂₋₆ alkenyl), -C(O)O(C₂₋₆ alkynyl), -C(O)O(C₃₋₁₅ cycloalkyl), - C(O)O(heterocyclyl), -C(O)O(C₆₋₁₀ aryl), -C(O)O(heteroaryl), -C(O)NH₂, -C(O)NH(C₁₋₉ alkyl), -C(O)NH(C₁₋₈ haloalkyl), -C(O)NH(C₂₋₆ alkenyl), -C(O)NH(C₂₋₆ alkynyl), -C(O)NH(C₃₋₁₅ cycloalkyl), -C(O)NH(heterocyclyl), -C(O)NH(C₆₋₁₀ aryl), -C(O)NH(heteroaryl), -C(O)N(C₁₋₉ alkyl)₂, -C(O)N(C₁₋₈ haloalkyl)₂, -C(O)N(C₂₋₆ alkenyl)₂, -C(O)N(C₂₋₆ alkynyl)₂, -C(O)N(C₃₋₁₅ cycloalkyl)₂, -C(O)N(heterocyclyl)₂, -C(O)N(C₆₋₁₀ aryl)₂, -C(O)N(heteroaryl)₂, -NHC(O)(C₁₋₉ alkyl), -NHC(O)(C₁₋₈ haloalkyl), -NHC(O)(C₂₋₆ alkenyl), -NHC(O)(C₂₋₆ alkynyl), -NHC(O)(C₃₋₁₅ cycloalkyl), -NHC(O)(heterocyclyl), -NHC(O)(C₆₋₁₀ aryl), -NHC(O)(heteroaryl), -NHC(O)O(C₁₋₉ alkyl), -NHC(O)O(C₁₋₈ haloalkyl), -NHC(O)O(C₂₋₆ alkenyl), -NHC(O)O(C₂₋₆ alkynyl), -NHC(O)O(C₃₋₁₅ cycloalkyl), -NHC(O)O(heterocyclyl),-NHC(O)O(C₆₋₁₀ aryl), -NHC(O)O(heteroaryl), -NHC(O)NH(C₁₋₉ alkyl), -NHC(O)NH(C₁₋₈ haloalkyl), -NHC(O)NH(C₂₋₆ alkenyl), -NHC(O)NH(C₂₋₆ alkynyl), -NHC(O)NH(C₃₋₁₅ cycloalkyl), -NHC(O)NH(heterocyclyl), -NHC(O)NH(C₆₋₁₀ aryl), -NHC(O)NH(heteroaryl), -NHS(O)(C₁₋₉ alkyl), -N(C₁₋₉ alkyl)(S(O)(C₁₋₉ alkyl), - S(C₁₋₉ alkyl), -S(C₁₋₈ haloalkyl), -S(C₂₋₆ alkenyl), -S(C₂₋₆ alkynyl), -S(C₃₋₁₅ cycloalkyl), -S(heterocyclyl), -S(C₆₋₁₀ aryl), -S(heteroaryl), -S(O)N(C₁₋₉ alkyl)₂, -S(O)(C₁₋₉ alkyl), -S(O)(C₁₋₈ haloalkyl), -S(O)(C₂₋₆ alkenyl), -S(O)(C₂₋₆ alkynyl), -S(O)(C₃₋₁₅ cycloalkyl), -S(O)(heterocyclyl), -S(O)(C₆₋₁₀ aryl), -S(O)(heteroaryl), -S(O)₂(C₁₋₉ alkyl), -S(O)₂(C₁₋₈ haloalkyl), - S(O)₂(C₂₋₆ alkenyl), -S(O)₂(C₂₋₆ alkynyl), -S(O)₂(C₃₋₁₅ cycloalkyl), -S(O)₂(heterocyclyl), -S(O)₂(C₆₋₁₀ aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
   wherein each alkyl, cycloalkyl, heterocyclyl, aryl, or heteroaryl is optionally substituted with 1 to 3 C₁₋₉ alkyl, C₁₋₈ haloalkyl, halogen, -OH, -NH₂, CO₂H,-O(C₁₋₉ alkyl), -O(C₁₋₈ haloalkyl), -O(C₃₋₁₅ cycloalkyl), - O(heterocyclyl), -O(aryl), -O(heteroaryl), -NH(C₁₋₉ alkyl), -NH(C₁₋₈ haloalkyl), -NH(C₃₋₁₅ cycloalkyl), -NH(heterocyclyl), -NH(aryl), - NH(heteroaryl), -N(C₁₋₉ alkyl)₂, -N(C₃₋₁₅ cycloalkyl)₂, -NHC(O)(C₁₋₈ haloalkyl), -NHC(O)(C₃₋₁₅ cycloalkyl), -NHC(O)(heterocyclyl), - NHC(O)(aryl), -NHC(O)(heteroaryl), -NHC(O)O(C₁₋₉ alkyl), -NHC(O)O(C₁₋₈ haloalkyl), -NHC(O)O(C₂₋₆ alkynyl), -NHC(O)O(C₃₋₁₅ cycloalkyl), -NHC(O)O(heterocyclyl), - NHC(O)O(aryl), -NHC(O)O(heteroaryl), -NHC(O)NH(C₁₋₉ alkyl), S(O)₂(C₁₋₉ alkyl), -S(O)₂(C₁₋₈ haloalkyl), -S(O)₂(C₃₋₁₅ cycloalkyl), - S(O)₂(heterocyclyl), -S(O)₂(aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
   wherein the alkyl or heterocyclyl is each optionally substituted with one to four halogens;
R^{6a} is H, C₁₋₆ alkyl, C₃₋₁₀ cycloalkyl, heterocyclyl, -S(O)₂R^{6a1}, or -S(O)₂N(R^{6a1})(NR^{6a2}), wherein the cycloalkyl or heterocyclyl is each optionally substituted with C₁₋₆ alkyl, F, or -CN;
each R^{6b} and R^{6c} is independently H, C₁₋₆ alkyl, C₁₋₆ haloalkyl, C₂₋₆ alkoxyalkyl, halogen, C₃₋₁₀ cycloalkyl, heterocyclyl, -C₁₋₆ alkyl-N(R^{9a})(R^{9b}), -CN, -OR^{6c1}, or - N(R^{6c2})(R^{6c3}),
   wherein the alkyl, cycloalkyl, or heterocyclyl is each optionally substituted with one to four R^{6b1}; or
R^{6b} and R^{6c} are combined with the atom to which they are attached to form a C₃₋₁₀ cycloalkyl or heterocyclyl, which is each optionally substituted with one to four R^{6b1} ; or
R^{6a} or R^{6c} is combined with one R⁴ group and the atoms to which they are attached to form a C₅₋₁₀ cycloalkyl or heterocyclyl, which is each optionally substituted with one to four R¹⁰;
each R^{6b1} and R¹⁰ is independently C₁₋₆ alkyl, C₁₋₆ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₀ cycloalkyl, heterocyclyl, C₆₋₁₀ aryl, heteroaryl, oxo, -OH, -CN, CO₂R^{3e}, -NO₂, or - C(O)N(R^{2a})(R^{2b}),
   wherein the heterocyclyl or heteroaryl is optionally substituted with C₁₋₆ alkyl, C₁₋₆ haloalkyl, or C₁₋₆ haloalkoxy;
each R^{6a1}, R^{6a2}, R^{6c1}, R^{6c2}, and R^{6c3} is independently H, C₁₋₆ alkyl or C₃₋₁₀ cycloalkyl;
each R^{9a} and R^{9b} is independently H, C₁₋₆ alkyl, or C₁₋₆ haloalkyl, or R^{9a} and R^{9b} together form a 6-membered heterocyclyl;
each R^{9c}, R^{9d}, R^{10a}, R^{10b}, and R^{10c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl,
   wherein the alkyl, alkenyl, alkynyl, cycloalkyl, heterocyclyl, aryl, or heteroaryl is each optionally substituted with one to four R⁶;
R^{a} and R^{b} are independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶;
optionally R^{a} and R^{b} are combined with the atom to which they are attached to form a 5- or 6-membered heterocyclyl, which is optionally substituted with one to four R⁶;
each R^{2a}, R^{2b}, R^{12a}, R^{12b}, and R^{12c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl
   wherein the alkyl, alkenyl, alkynyl, cycloalkyl, heterocyclyl, aryl, or heteroaryl is each optionally substituted with one to four R⁶;
each R^{3e} is independently H, C₁₋₆ alkyl, C₁₋₆ haloalkyl, -C₁₋₄alkyl-N(R^{9a})(R^{9b}), -C₁₋₄ alkyl-C(O)N(R^{9a})(R^{9b}), -C₁₋₄alkyl-O-C(O)-C₁₋₄alkyl, -C₁₋₄alkyl-O-C(O)-O-C₁₋₄alkyl, -C₁₋₄alkyl-O-C(O)-C₁₋₄alkyl-N(R^{9a})(R^{9b}), -C₁₋₄alkyl-C₃₋₈ cycloalkyl, -C₁₋₄ alkyl-heterocyclyl, C₃₋₁₀ cycloalkyl, heterocyclyl, C₆₋₁₀ aryl, heteroaryl, - P(O)(OR^{9c})₂, -CH2P(O)(OR^{9c})₂, -OCH₂P(O)(OR^{9c})₂, -C(O)OCH₂P(O)(OR^{9c})₂, - P(O)(R^{9c})(OR^{9d}), -OP(O)(R^{9c})(OR^{9d}), -CH₂P(O)(R^{9c})(OR^{9d}), - C(O)OCH₂P(O)(R^{9c})(OR^{9d}), -P(O)(N(R^{9c})₂)₂, -CH₂P(O)(N(R^{9c})₂)₂, - C(O)OCH₂P(O)(N(R^{9c})₂)₂, -P(O)(N(R^{9c})₂)(OR^{9d}), -CH₂P(O)(N(R^{9c})₂)(OR^{9d}), - C(O)OCH₂P(O)(N(R^{9c})₂)(OR^{9d}), -P(O)(R^{9c})(N(R^{9d})₂), -CH₂P(O)(R^{9c})(N(R^{9d})₂), or -C(O)OCH₂P(O)(R^{9c})(N(R^{9d})₂);
   wherein the alkyl, alkenyl, cycloalkyl, heterocyclyl, aryl, or heteroaryl is each optionally substituted with one to four R⁶;
   wherein each heteroaryl has 5 to 12 ring members and has one to four heteroatoms each independently N, O, or S;

wherein each heterocyclyl has three to twelve ring members and has one to four heteroatoms, each independently N, O, or S; and
wherein each heteroaryl has five to twelve ring members and one to four heteroatoms, each independently N, O, or S.

In some embodiments of the compound of Formula (**I**), or a pharmaceutically acceptable salt thereof, R¹ is
i) 5-membered heteroaryl, optionally substituted with one to four R⁴;
ii) 5-membered heteroaryl fused to a heteroaryl or aryl to form a heteroaryl ring system, wherein the heteroaryl ring system is optionally substituted with one to four R⁴; or
iii) 5-membered heteroaryl substituted with two R⁴ groups attached to adjacent ring atoms and optionally substituted with one or two additional R⁴, wherein the two R⁴ groups attached to adjacent ring atoms are combined with the atoms to which they are attached to form a C₅₋₁₀ cycloalkyl or heterocyclyl, which is each optionally substituted with one to four R⁶.

In some embodiments, R¹ is pyrrolyl, pyrazolyl, thienyl, indolyl, imidazolyl, oxazolyl, isoxazolyl, thiazolyl, furyl, oxadiazolyl, thiadiazolyl, benzothiazolyl, benzoxazolyl, indazolyl, benzofuranyl, benzimidazolyl, pyrrolo[2,3-*b*]pyridinyl, triazolyl, tetrazolyl, thiazolo[5,4-*b*]pyridinyl, pyrazolo[1,5-*a*]pyridinyl, imidazo[1,2-*a*]pyrimidinyl, imidazo[1,2-*a*]pyridinyl, imidazo[1,2-*a*]pyrazinyl, or 1λ²-thieno[3,2-*d*]pyrazolyl, optionally substituted with one to four R⁴; or 5-membered heteroaryl substituted with two to four R⁴, wherein two R⁴ groups attached to adjacent ring atoms are combined with the atoms to which they are attached to form a C₅₋₆ cycloalkyl or heterocyclyl, which is each optionally substituted with one to four R⁶. In some embodiments, R¹ is pyrazolyl, thienyl, imidazolyl, oxazolyl, isoxazolyl, thiazolyl, furyl, oxadiazolyl, thiadiazolyl, benzimidazolyl, triazolyl, thiazolo[5,4-*b*]pyridinyl, pyrazolo[1,5-*a*]pyridinyl, imidazo[1,2-*a*]pyrimidinyl, imidazo[1,2-*a*]pyridinyl, imidazo[1,2-*a*]pyrazinyl, or 1λ²-thieno[3,2-*d*]pyrazolyl, optionally substituted with one to four R⁴; or pyrazolyl substituted with two to four R⁴, wherein two R⁴ groups attached to adjacent pyrazolyl atoms are combined with the atoms to which they are attached to form a C₅₋₆ cycloalkyl or heterocyclyl, which is each optionally substituted with one to four R⁶.

In some embodiments of the compound of Formula (I), or a pharmaceutically acceptable salt thereof, R¹ is 5-membered heteroaryl, optionally substituted with one to four R⁴. In some embodiments, R¹ is pyrrolyl, pyrazolyl, thienyl, indolyl, imidazolyl, oxazolyl, isoxazolyl, thiazolyl, furyl, oxadiazolyl, thiadiazolyl, triazolyl, or tetrazolyl, optionally substituted with one to four R⁴. In some embodiments R¹ is thiazolyl, pyrrolyl, imidazolyl, oxazolyl, or triazolyl optionally substituted with one to four R⁴. In some embodiments R¹ is pyrrolyl optionally substituted with one to four R⁴. In some embodiments R¹ is imidazolyl optionally substituted with one to four R⁴. In some embodiments R¹ is oxazolyl optionally substituted with one to four R⁴. In some embodiments R¹ is triazolyl optionally substituted with one to four R⁴. In some embodiments R¹ is thiazolyl optionally substituted with one to four R⁴. In some embodiments, R¹ is optionally substituted with one or two R⁴. In some embodiments, R¹ is substituted with one or two R⁴. In some embodiments, R¹ is optionally substituted with one R⁴. In some embodiments, R¹ is substituted with one R⁴. In some embodiments, R¹ is unsubstituted.

In some embodiments, R¹ is 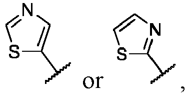 which is each substituted with one R⁴.

In some embodiments, R¹ is

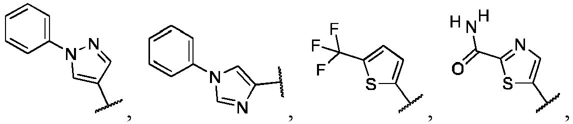

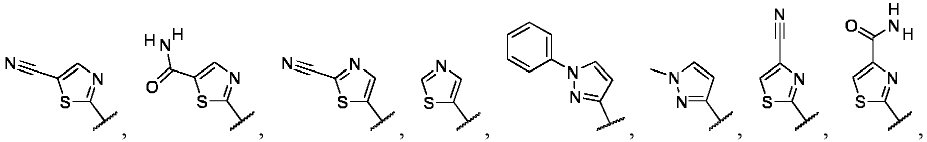

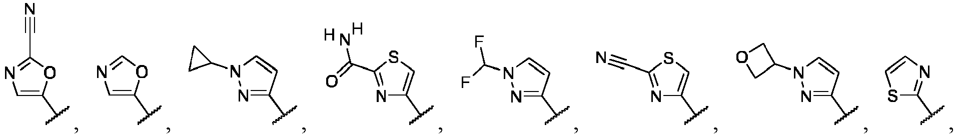

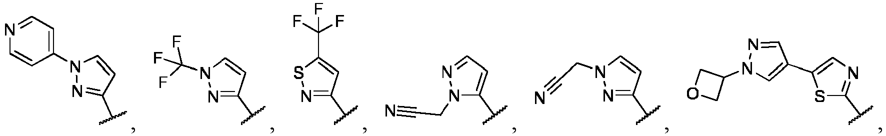

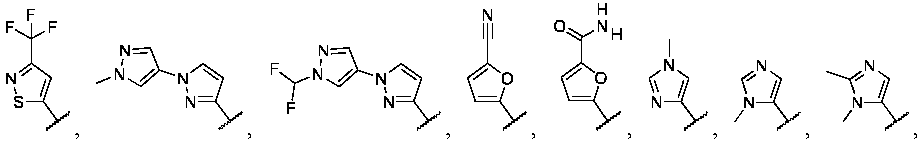

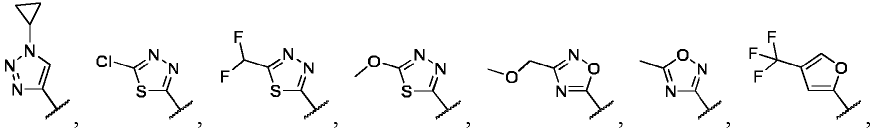

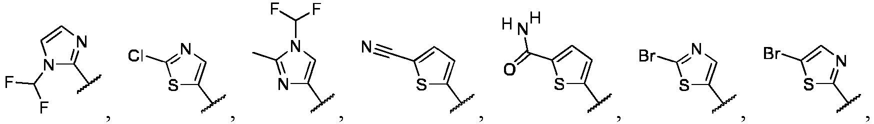

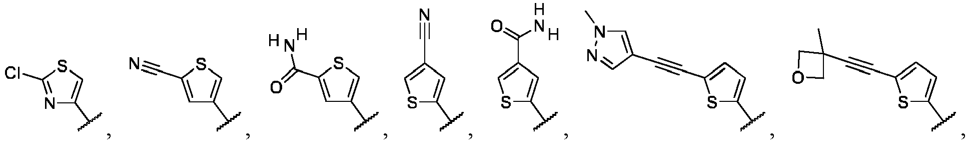

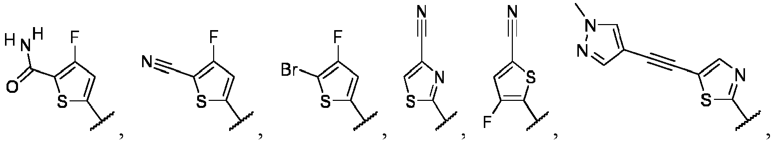

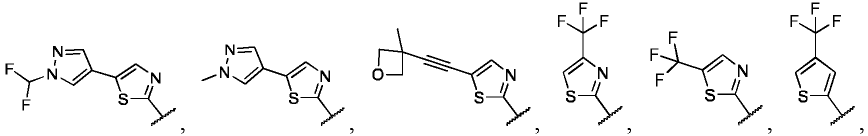

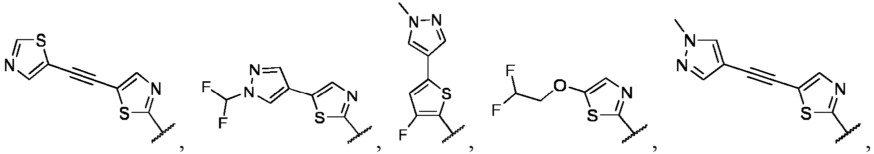

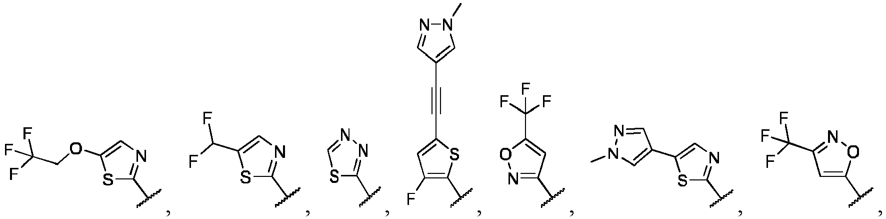

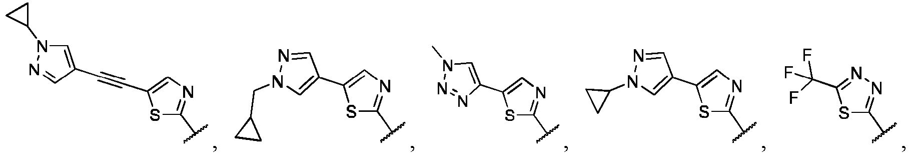

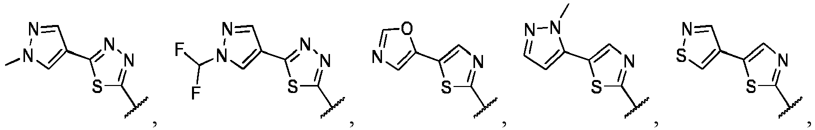

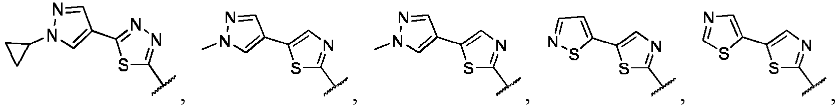

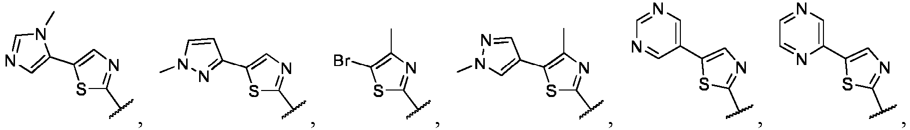

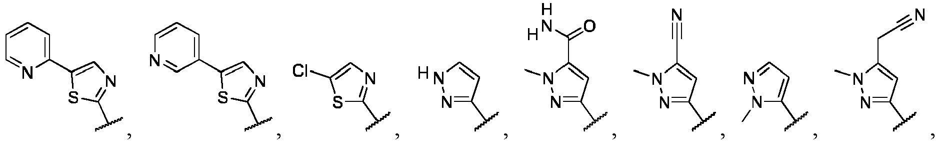

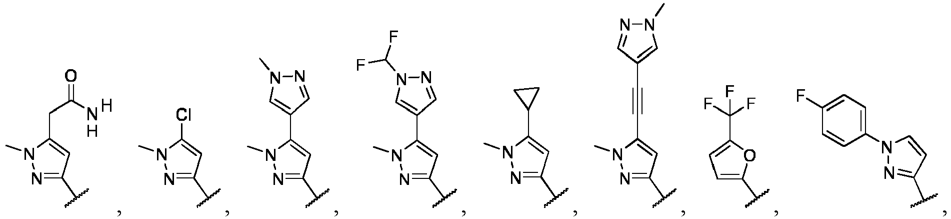

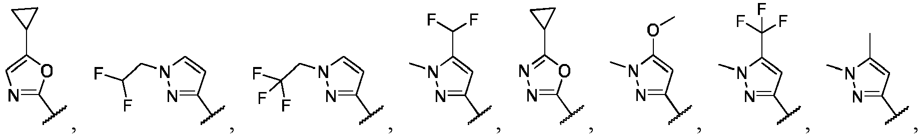

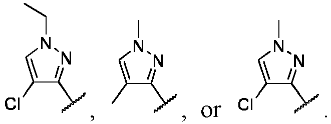

In some embodiments of the compound of Formula (I), or a pharmaceutically acceptable salt thereof, R¹ is

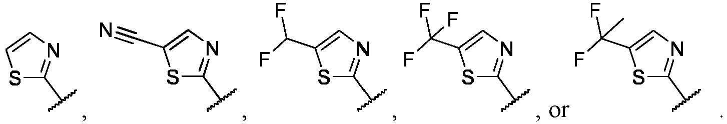

In some embodiments R¹ is

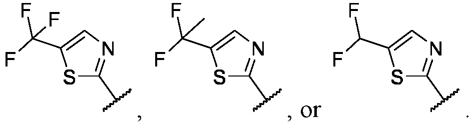

In some embodiments R¹ is

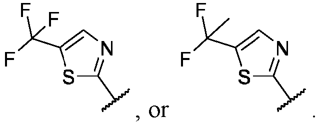

In some embodiments R¹ is

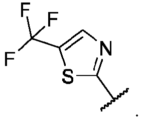

In some embodiments of the compound of Formula (I), or a pharmaceutically acceptable salt thereof, R¹ is 5-membered heteroaryl fused to a heteroaryl or aryl to form a heteroaryl ring system, wherein the heteroaryl ring system is optionally substituted with one to four R⁴. In some embodiments, R¹ is benzothiazolyl, benzoxazolyl, indazolyl, benzofuranyl, benzimidazolyl, pyrrolo[2,3-*b*]pyridinyl, thiazolo[5,4-*b*]pyridinyl, pyrazolo[1,5-*a*]pyridinyl, imidazo[1,2-*a*]pyrimidinyl, imidazo[1,2-*a*]pyridinyl, imidazo[1,2-*a*]pyrazinyl, or 1λ²-thieno[3,2-*d*]pyrazolyl, optionally substituted with one to four R⁴. In some embodiments, R¹ is optionally substituted with one or two R⁴. In some embodiments, R¹ is optionally substituted with one R⁴. In some embodiments, R¹ is substituted with one R⁴. In some embodiments, R¹ is unsubstituted. In some embodiments, R¹ is

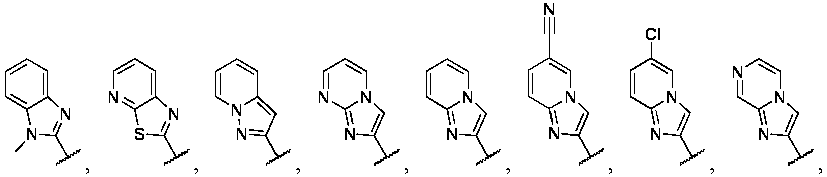

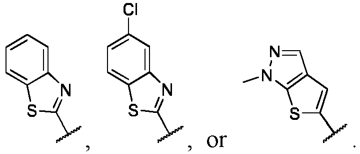

In some embodiments of the compound of Formula (I), or a pharmaceutically acceptable salt thereof, R¹ is 5-membered heteroaryl substituted with two R⁴ groups attached to adjacent ring atoms and optionally substituted with one or two additional R⁴, wherein the two R⁴ groups attached to adjacent ring atoms are combined with the atoms to which they are attached to form a C₅₋₁₀ cycloalkyl or heterocyclyl, which is each optionally substituted with one to four R⁶. In some embodiments, R¹ is 5-membered heteroaryl substituted with two R⁴ groups attached to adjacent ring atoms and optionally substituted with one or two additional R⁴, wherein the two R⁴ groups attached to adjacent ring atoms are combined with the atoms to which they are attached to form a C₅ or C₆ cycloalkyl or heterocyclyl, which is each optionally substituted with one to four R⁶. In some embodiments, R¹ is 5-membered heteroaryl substituted with two R⁴ groups attached to adjacent ring atoms and optionally substituted with one or two additional R⁴, wherein the two R⁴ groups attached to adjacent ring atoms are combined with the atoms to which they are attached to form a C₅ or C₆ cycloalkyl or heterocyclyl, which is each optionally substituted with one or two R⁶. In some embodiments, R¹ is

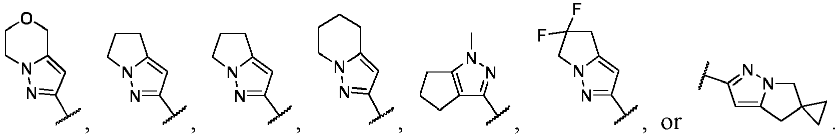

In some embodiments of the compound of Formula (I), or a pharmaceutically acceptable salt thereof, R¹ is 6-membered heteroaryl or phenyl substituted with C₂-alkynyl, wherein the 6-membered heteroaryl or phenyl is optionally substituted with one to three R⁴, wherein the C₂-alkynyl is substituted with -C(CH₃)₂SO₂CH₃, aryl, heteroaryl, or heterocyclyl, wherein the aryl, heteroaryl or heterocyclyl is optionally substituted with one to four R⁵. In some embodiments, R¹ is pyridyl, pyrazinyl, pyrimidinyl, or phenyl substituted with C₂-alkynyl, wherein the pyridyl, pyrazinyl, pyrimidinyl, or phenyl is optionally substituted with one to three R⁴, wherein the C₂-alkynyl is substituted with -C(CH₃)₂SO₂CH₃, aryl, heteroaryl, or heterocyclyl, wherein the aryl, heteroaryl or heterocyclyl is optionally substituted with one to four R⁵. In some embodiments, the pyridyl, pyrazinyl, pyrimidinyl, or phenyl is optionally substituted with one or two R⁴. In some embodiments, the pyridyl, pyrazinyl, pyrimidinyl, or phenyl is optionally substituted with one R⁴. In some embodiments, the pyridyl, pyrazinyl, pyrimidinyl, or phenyl is substituted with one R⁴. In some embodiments, the pyridyl, pyrazinyl, pyrimidinyl, or phenyl is unsubstituted. In some embodiments, R¹ is

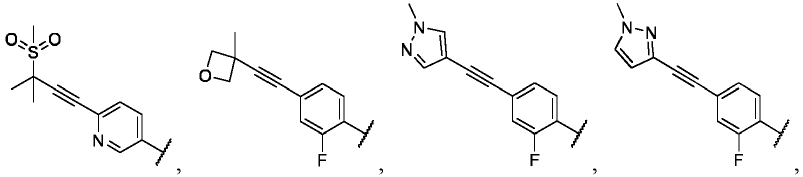

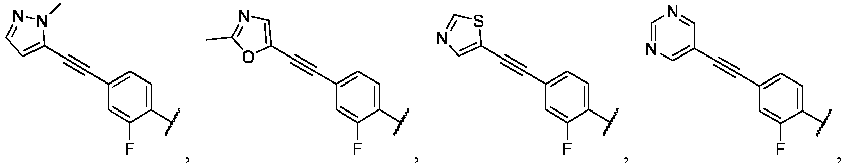

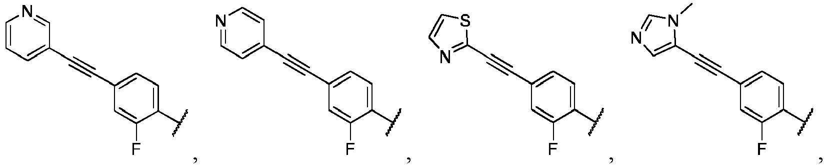

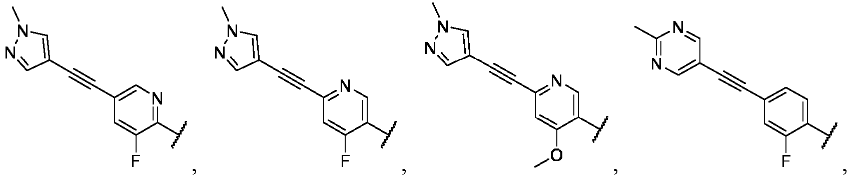

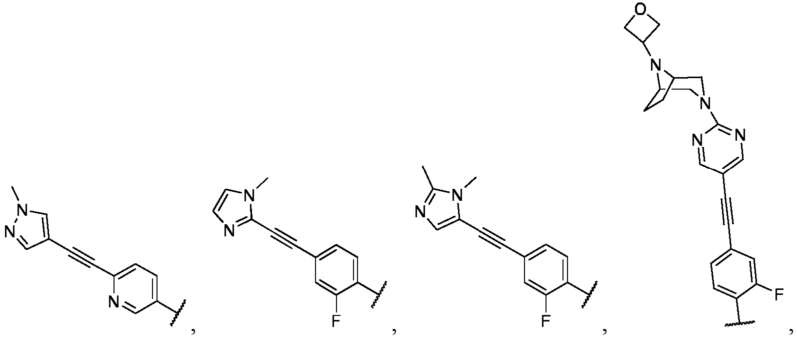

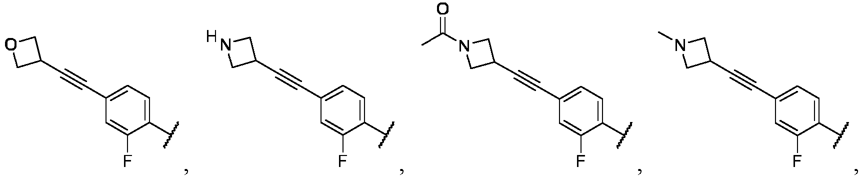

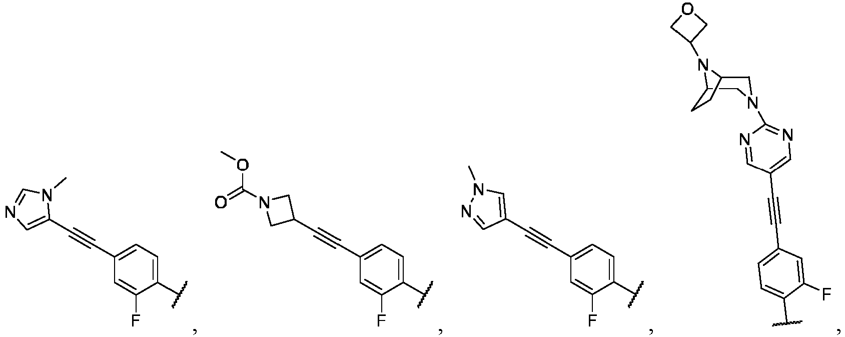

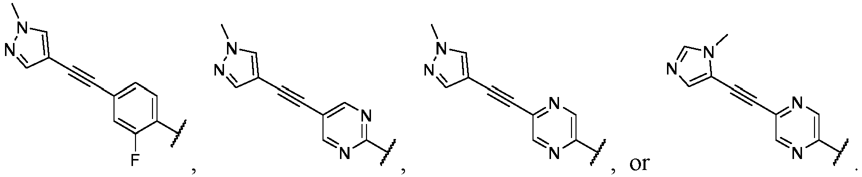

In some embodiments of the compound of Formula (I), or a pharmaceutically acceptable salt thereof, R¹ is 6-membered heteroaryl or phenyl substituted with -C(O)NR^{a}R^{b}, wherein the heteroaryl or phenyl is optionally substituted with one to four R⁴. In some embodiments, R¹ is pyridyl, pyrazinyl, pyrimidinyl, or phenyl substituted with -C(O)NR^{a}R^{b}, wherein the pyridyl, pyrazinyl, pyrimidinyl, or phenyl is optionally substituted with one to four R⁴. In some embodiments, R¹ is pyridyl, pyrazinyl, or phenyl substituted with -C(O)NR^{a}R^{b}, wherein the pyridyl, pyrazinyl, or phenyl is optionally substituted with one to four R⁴. In some embodiments, the pyridyl, pyrazinyl, or phenyl is optionally substituted with one or two R⁴. In some embodiments, the pyridyl, pyrazinyl, or phenyl is optionally substituted with one R⁴. In some embodiments, the pyridyl, pyrazinyl, or phenyl is substituted with one R⁴. In some embodiments, the pyridyl, pyrazinyl, or phenyl is unsubstituted. In some embodiments, R¹ is 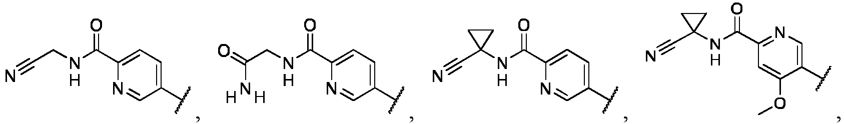 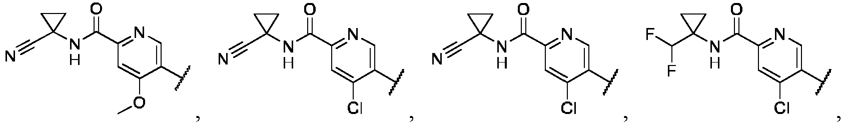 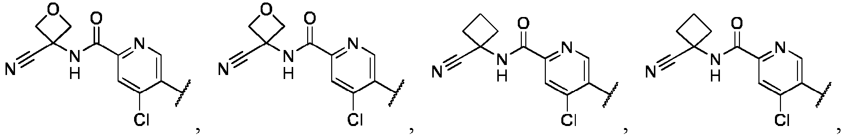 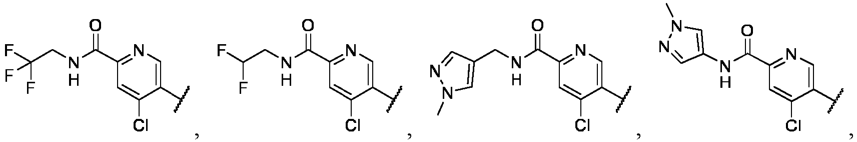 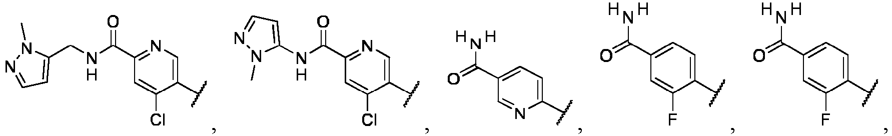 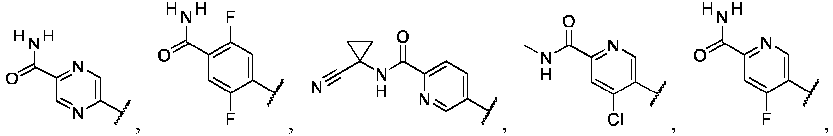 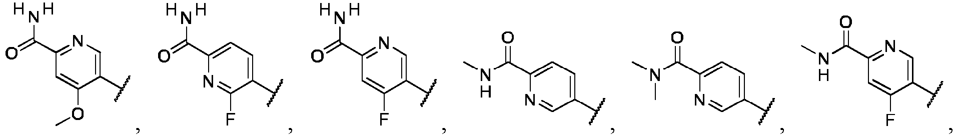 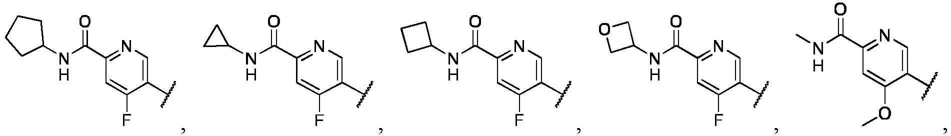 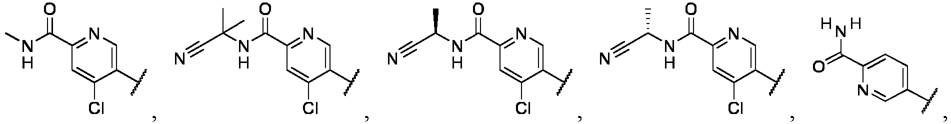 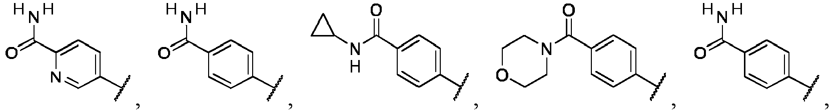 or

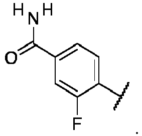

In some embodiments of the compound of Formula (**I**), or a pharmaceutically acceptable salt thereof, R¹ is 6-membered heteroaryl or phenyl, wherein the 6-membered heteroaryl or phenyl is substituted with heteroaryl, C₃₋₁₀ cycloalkyl, or heterocycle and optionally substituted with one to three R⁴, wherein the heteroaryl, C₃₋₁₀ cycloalkyl, or heterocycle is each substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl, wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶.

In some embodiments, R¹ is pyridyl, pyrazinyl, pyrimidinyl, or phenyl, wherein the pyridyl, pyrazinyl, pyrimidinyl, or phenyl is substituted with heteroaryl, C₃₋₁₀ cycloalkyl, or heterocycle and optionally substituted with one to three R⁴, wherein the heteroaryl, C₃₋₁₀ cycloalkyl, or heterocycle is each substituted with C₁₋₆ alkyl, C₃₋₁₀ cycloalkyl or heterocyclyl, wherein the C₁₋₆ alkyl, C₃₋₁₀ cycloalkyl or heterocyclyl, is optionally substituted with one to four R⁶. In some embodiments, R¹ is pyridyl or phenyl, wherein the pyridyl or phenyl is substituted with heteroaryl, C₃₋₁₀ cycloalkyl, or heterocycle and optionally substituted with one to three R⁴, wherein the heteroaryl, C₃₋₁₀ cycloalkyl, or heterocycle is each substituted with C₁₋₆ alkyl, C₃₋₁₀ cycloalkyl or heterocyclyl, wherein the C₁₋₆ alkyl, C₃₋₁₀ cycloalkyl or heterocyclyl, is optionally substituted with one to four R⁶. In some embodiments, R¹ is pyridyl, pyrazinyl, pyrimidinyl, or phenyl, wherein the pyridyl, pyrazinyl, pyrimidinyl, or phenyl is substituted with heteroaryl, and optionally substituted with one to three R⁴, wherein the heteroaryl, is substituted with C₁₋₆ alkyl, C₃₋₁₀ cycloalkyl or heterocyclyl, wherein the C₁₋₆ alkyl, C₃₋₁₀ cycloalkyl or heterocyclyl, is optionally substituted with one to four R⁶. In some embodiments, R¹ is

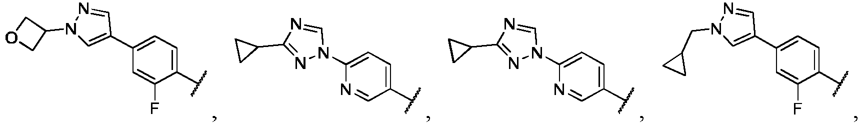

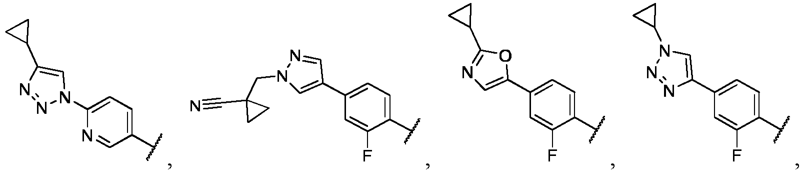

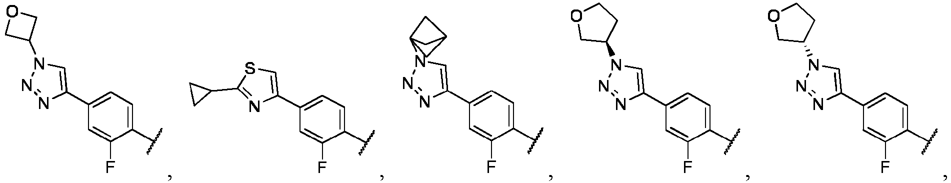

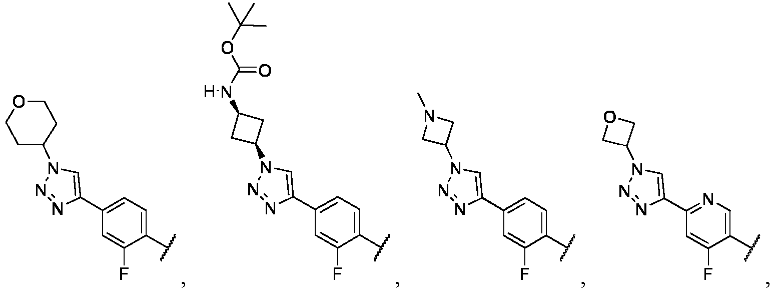

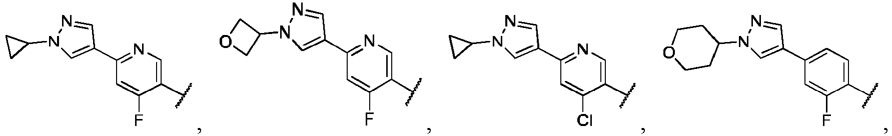

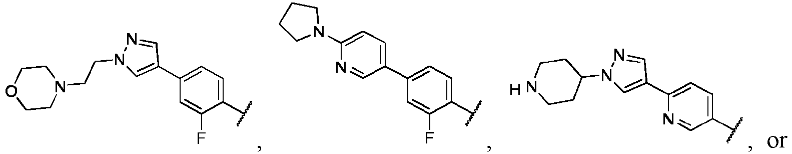

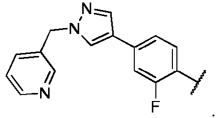

In some embodiments of the compound of Formula (**I**), or a pharmaceutically acceptable salt thereof, X¹, X², and X³ are each independently -CH=, -C(F)=, -C(Cl)=, -C(Br)=, or - C(CN)=. In some embodiments, X¹, X², and X³ are each independently -CH= or -C(F)=. In some embodiments, two of X¹, X², and X³ are -CH= and one is -C(F)=. In some embodiments X¹ is -C(F)=, and X², and X³ are each -CH=. In some embodiments X² is -C(F)=, and X¹, and X³ are each -CH=. In some embodiments X¹, and X² are each -CH=, and X³ is -C(F)=. In some embodiments, X¹, X², and X³ is each -CH=.

In some embodiments of the compound of Formula (**I**), or a pharmaceutically acceptable salt thereof, ring A is 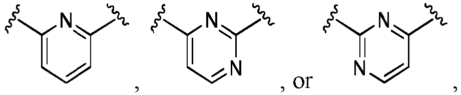 each optionally substituted with one to three Z^{1a} groups. In some embodiments, ring A is 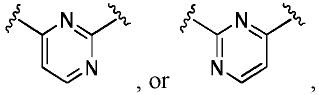 each optionally substituted with one to three Z^{1a} groups.

In some embodiments, ring A is 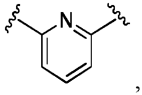 optionally substituted with one to three Z^{1a} groups.

In some embodiments, ring A is 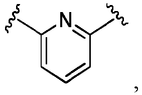 optionally substituted with one halogen. In some embodiments, ring A is 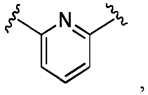 substituted with one halogen. In some embodiments, ring A is 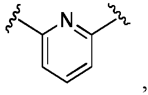 substituted with F or Cl. In some embodiments, ring A is 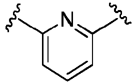 substituted with F. In some embodiments, ring A is 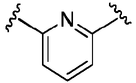 substituted with Cl. In some embodiments, ring A is unsubstituted

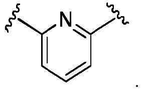

In some embodiments, ring A is 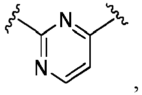 optionally substituted with one Z^{1a} group. In some embodiments, ring A is 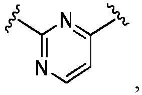 optionally substituted with one halogen. In some embodiments, ring A is unsub stituted

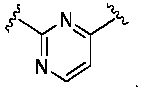

In some embodiments, ring A is optionally substituted with one Z^{1a} group. In some embodiments, ring A is optionally substituted with one halogen. In some embodiments of the compound of Formula (**I**), or a pharmaceutically acceptable salt thereof, ring B is C₆₋₁₀ aryl or 5-to 10-membered heteroaryl having one to three heteroatoms each independently N, O or S, wherein the aryl or heteroaryl is optionally substituted with one to four R⁴. In some embodiments of the compound of Formula (I), or a pharmaceutically acceptable salt thereof, ring B is C₆₋₁₀ aryl having one to three heteroatoms each independently N, O or S, wherein the aryl is optionally substituted with one to four R⁴. In some embodiments, ring B is phenyl or 5- to 6-membered heteroaryl having one to three heteroatoms each independently N, O or S. In some embodiments, ring B is 5- to 6-membered heteroaryl having one to three heteroatoms each independently N, O or S. In some embodiments, ring B is phenyl. In some embodiments, ring B is phenyl optionally substituted with one to three R⁴. In some embodiments, ring B is phenyl optionally substituted with one or two R⁴. In some embodiments, ring B is phenyl optionally substituted with three R⁴. In some embodiments, ring B is phenyl optionally substituted with two R⁴. In some embodiments, ring B is phenyl optionally substituted with one R⁴. In some embodiments, ring B is phenyl substituted with two R⁴. In some embodiments, ring B is substituted with two R⁴.

In some embodiments of the compound of Formula (**I**), or a pharmaceutically acceptable salt thereof, V is -O- or -C(R^{6b})(R^{6c})-. In some embodiments, V is -O-. In some embodiments, V is -C(R^{6b})(R^{6c})-. In some embodiments, V is -CH₂-.

In some embodiments of the compound of Formula (**I**), or a pharmaceutically acceptable salt thereof, R² is C₁₋₆ alkyl or heterocyclyl, wherein the alkyl or heterocyclyl is each optionally substituted with one to four Z¹, wherein each Z¹ is independently C₁₋₆ alkyl, C₁₋₆ alkoxy, C₁₋₆ hydroxyalkyl, C₂₋₆ alkoxyalkyl, halogen, C₁₋₆ haloalkyl, C₁₋₆ haloalkoxy, -OH, -CN, C₁₋₆ alkyl-CN, -O-C₃₋₆ cycloalkyl, heterocyclyl, C₃₋₁₀ cycloalkyl, or heteroaryl optionally substituted with 1 to 4 groups each independently C₁₋₆ alkyl, C₁₋₆ alkoxy, halogen, -CN, C₁₋₆ haloalkyl, or C₁₋₆ haloalkoxy. In some embodiments, R² is C₁₋₆ alkyl or heterocyclyl, wherein the alkyl or heterocyclyl is each optionally substituted with one to four Z¹, wherein each Z¹ is independently C₁₋₆ alkyl, C₁₋₆ alkoxy, or heterocyclyl In some embodiments, each Z¹ is independently C₁₋₆ alkyl, C₁₋₆ alkoxy, heterocyclyl, or C₃₋₁₀ cycloalkyl. In some embodiments, R² is

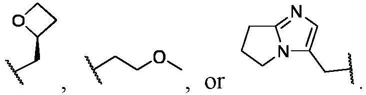

In some embodiments, R² is

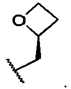

In some embodiments, R² is

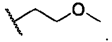

In some embodiments of the compound of Formula (**I**), or a pharmaceutically acceptable salt thereof, R³ is -C(O)OH.

In some embodiments of the compound of Formula (I), or a pharmaceutically acceptable salt thereof, each R⁴ is independently C₁₋₉ alkyl or halogen. In some embodiments, each R⁴ is independently halogen. In some embodiments, each R⁴ is independently methyl or F. In some embodiments, each R⁴ is F.

In some embodiments of the compound of Formula (I), or a pharmaceutically acceptable salt thereof, each R⁵ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, C₃₋₁₅ cycloalkyl, halogen, C₃₋₁₅ cycloalkyl, heterocyclyl, C₆₋₁₀ aryl, heteroaryl, oxo, -NO₂, -N₃, -CN, -O- R^{10a}, -C(O)-R^{10a}, -C(O)OR^{10a}, -C(O)N(R^{10a})(R^{10b}), -N(R^{10a})(R^{10b}), -N(R^{10a})C(O)R^{10b}, -N(R^{10a})C(O)OR^{10b}, -N(R^{10a})C(O)N(R^{10b})(R^{10c}), - N(R^{10a})S(O)₂(R^{10b}), -NR^{10a}S(O)₂N(R^{10b})(R^{10c}), -NR^{10a}S(O)₂O(R^{10b}), -OC(O)R^{10a}, - OC(O)OR^{10a}, -OC(O)-N(R^{10a})(R^{10b}), -S-R^{10a}, -S(O)R^{10a}, -S(O)(NH)R^{10a}, - S(O)₂R^{10a}, -S(O)₂N(R^{10a})(R^{10b}), -S(O)(NR^{10a})R^{10b}, or -Si(R^{10a})₃, wherein each alkyl, haloalkyl, alkenyl, alkynyl, cycloalkyl, heterocyclyl, aryl, or heteroaryl is optionally substituted with one to four R⁶.

In some embodiments of the compound of Formula (I), or a pharmaceutically acceptable salt thereof, each R⁶ or R⁸ is independently C₁₋₉ alkyl, C₁₋₈ haloalkyl, C₂₋₆ alkenyl, C₂₋₆ alkynyl, halogen, C₃₋₁₅ cycloalkyl, heterocyclyl, C₆₋₁₀ aryl, heteroaryl, oxo, -OH, -CN, -NO₂, -NH₂, -N₃, - SH, -O(C₁₋₉ alkyl), -O(C₁₋₈ haloalkyl), -O(C₂₋₆ alkenyl), -O(C₂₋₆ alkynyl), -O(C₃₋₁₅ cycloalkyl), - O(heterocyclyl), -O(C₆₋₁₀ aryl), -O(heteroaryl), -NH(C₁₋₉ alkyl), -NH(C₁₋₈ haloalkyl), -NH(C₂₋₆ alkenyl), -NH(C₂₋₆ alkynyl), -NH(C₃₋₁₅ cycloalkyl), -NH(heterocyclyl), -NH(C₆₋₁₀ aryl), - NH(heteroaryl), -N(C₁₋₉ alkyl)₂, -N(C₁₋₈ haloalkyl)₂, -N(C₂₋₆ alkenyl)₂, -N(C₂₋₆ alkynyl)₂, -N(C₃₋₁₅ cycloalkyl)₂, -N(heterocyclyl)₂, -N(C₆₋₁₀ aryl)₂, -N(heteroaryl)₂, -N(C₁₋₉ alkyl)(C₁₋₈ haloalkyl), -N(C₁₋₉ alkyl)(C₂₋₆ alkenyl), -N(C₁₋₉ alkyl)(C₂₋₆ alkynyl), -N(C₁₋₉ alkyl)(C₃₋₁₅ cycloalkyl), -N(C₁₋₉ alkyl)(heterocyclyl), -N(C₁₋₉ alkyl)(C₆₋₁₀ aryl), -N(C₁₋₉ alkyl)(heteroaryl), -C(O)(C₁₋₉ alkyl), -C(O)(C₁₋₈ haloalkyl), -C(O)(C₂₋₆ alkenyl), -C(O)(C₂₋₆ alkynyl), -C(O)(C₃₋₁₅ cycloalkyl), -C(O)(heterocyclyl), -C(O)(C₆₋₁₀ aryl), -C(O)(heteroaryl), - C(O)O(C₁₋₉ alkyl), -C(O)O(C₁₋₈ haloalkyl), -C(O)O(C₂₋₆ alkenyl), -C(O)O(C₂₋₆ alkynyl), -C(O)O(C₃₋₁₅ cycloalkyl), -C(O)O(heterocyclyl), -C(O)O(C₆₋₁₀ aryl), - C(O)O(heteroaryl), -C(O)NH₂, -C(O)NH(C₁₋₉ alkyl), -C(O)NH(C₁₋₈ haloalkyl), -C(O)NH(C₂₋₆ alkenyl), -C(O)NH(C₂₋₆ alkynyl), -C(O)NH(C₃₋₁₅ cycloalkyl), -C(O)NH(heterocyclyl), - C(O)NH(C₆₋₁₀ aryl), -C(O)NH(heteroaryl), -C(O)N(C₁₋₉ alkyl)₂, -C(O)N(C₁₋₈ haloalkyl)₂, -C(O)N(C₂₋₆ alkenyl)₂, -C(O)N(C₂₋₆ alkynyl)₂, -C(O)N(C₃₋₁₅ cycloalkyl)₂, - C(O)N(heterocyclyl)₂, -C(O)N(C₆₋₁₀ aryl)₂, -C(O)N(heteroaryl)₂, -NHC(O)(C₁₋₉ alkyl), -NHC(O)(C₁₋₈ haloalkyl), -NHC(O)(C₂₋₆ alkenyl), -NHC(O)(C₂₋₆ alkynyl), -NHC(O)(C₃₋₁₅ cycloalkyl), -NHC(O)(heterocyclyl), -NHC(O)(C₆₋₁₀ aryl), -NHC(O)(heteroaryl), - NHC(O)O(C₁₋₉ alkyl), -NHC(O)O(C₁₋₈ haloalkyl), -NHC(O)O(C₂₋₆ alkenyl), -NHC(O)O(C₂₋₆ alkynyl), -NHC(O)O(C₃₋₁₅ cycloalkyl), -NHC(O)O(heterocyclyl),-NHC(O)O(C₆₋₁₀ aryl), - NHC(O)O(heteroaryl), -NHC(O)NH(C₁₋₉ alkyl), -NHC(O)NH(C₁₋₈ haloalkyl), -NHC(O)NH(C₂₋₆ alkenyl), -NHC(O)NH(C₂₋₆ alkynyl), -NHC(O)NH(C₃₋₁₅ cycloalkyl), -NHC(O)NH(heterocyclyl), -NHC(O)NH(C₆₋₁₀ aryl), -NHC(O)NH(heteroaryl), - NHS(O)(C₁₋₉ alkyl), -N(C₁₋₉ alkyl)(S(O)(C₁₋₉ alkyl), -S(C₁₋₉ alkyl), -S(C₁₋₈ haloalkyl), -S(C₂₋₆ alkenyl), -S(C₂₋₆ alkynyl), -S(C₃₋₁₅ cycloalkyl), -S(heterocyclyl), -S(C₆₋₁₀ aryl), -S(heteroaryl), - S(O)N(C₁₋₉ alkyl)₂, -S(O)(C₁₋₉ alkyl), -S(O)(C₁₋₈ haloalkyl), -S(O)(C₂₋₆ alkenyl), -S(O)(C₂₋₆ alkynyl), -S(O)(C₃₋₁₅ cycloalkyl), -S(O)(heterocyclyl), -S(O)(C₆₋₁₀ aryl), -S(O)(heteroaryl), - S(O)₂(C₁₋₉ alkyl), -S(O)₂(C₁₋₈ haloalkyl), -S(O)₂(C₂₋₆ alkenyl), -S(O)₂(C₂₋₆ alkynyl), -S(O)₂(C₃₋₁₅ cycloalkyl), -S(O)₂(heterocyclyl), -S(O)₂(C₆₋₁₀ aryl), -S(O)₂(heteroaryl), -S(O)(NH)(C₁₋₉ alkyl), - S(O)₂NH(C₁₋₉ alkyl), or -S(O)₂N(C₁₋₉ alkyl)₂, wherein each alkyl, cycloalkyl, heterocyclyl, aryl, or heteroaryl is optionally substituted with 1 to 3 C₁₋₉ alkyl, C₁₋₈ haloalkyl, halogen, -OH, -NH₂, CO₂H,-O(C₁₋₉ alkyl), -O(C₁₋₈ haloalkyl), -O(C₃₋₁₅ cycloalkyl), -O(heterocyclyl), -O(aryl), - O(heteroaryl), -NH(C₁₋₉ alkyl), -NH(C₁₋₈ haloalkyl), -NH(C₃₋₁₅ cycloalkyl), -NH(heterocyclyl), - NH(aryl), -NH(heteroaryl), -N(C₁₋₉ alkyl)₂, -N(C₃₋₁₅ cycloalkyl)₂, -NHC(O)(C₁₋₈ haloalkyl), -NHC(O)(C₃₋₁₅ cycloalkyl), -NHC(O)(heterocyclyl), -NHC(O)(aryl), - NHC(O)(heteroaryl), -NHC(O)O(C₁₋₉ alkyl), -NHC(O)O(C₁₋₈ haloalkyl), -NHC(O)O(C₂₋₆ alkynyl), -NHC(O)O(C₃₋₁₅ cycloalkyl), -NHC(O)O(heterocyclyl), -NHC(O)O(aryl), - NHC(O)O(heteroaryl), -NHC(O)NH(C₁₋₉ alkyl), S(O)₂(C₁₋₉ alkyl), -S(O)₂(C₁₋₈ haloalkyl), -S(O)₂(C₃₋₁₅ cycloalkyl), -S(O)₂(heterocyclyl), -S(O)₂(aryl), -S(O)₂(heteroaryl), - S(O)(NH)(C₁₋₉ alkyl), -S(O)₂NH(C₁₋₉ alkyl), or -S(O)₂N(C₁₋₉ alkyl)₂, wherein the alkyl or heterocyclyl is each optionally substituted with one to four halogens.

In some embodiments of the compound of Formula (I), or a pharmaceutically acceptable salt thereof, R^{6a} is H, C₁₋₆ alkyl, C₃₋₁₀ cycloalkyl, heterocyclyl, -S(O)₂R^{6a1}, or - S(O)₂N(R^{6a1})(NR^{6a2}), wherein the cycloalkyl or heterocyclyl is each optionally substituted with C₁₋₆ alkyl, F, or -CN.

In some embodiments of the compound of Formula (I), or a pharmaceutically acceptable salt thereof, each R^{6b} and R^{6c} is independently H, C₁₋₆ alkyl, C₁₋₆ haloalkyl, C₂₋₆ alkoxyalkyl, halogen, C₃₋₁₀ cycloalkyl, heterocyclyl, -C₁₋₆ alkyl-N(R^{9a})(R^{9b}), -CN, -OR^{6c1}, or -N(R^{6c2})(R^{6c3}), wherein the alkyl, cycloalkyl, or heterocyclyl is each optionally substituted with one to four R^{6b1}.

In some embodiments of the compound of Formula (I), or a pharmaceutically acceptable salt thereof, R^{6b} and R^{6c} are combined with the atom to which they are attached to form a C₃₋₁₀ cycloalkyl or heterocyclyl, which is each optionally substituted with one to four R^{6b1}.

In some embodiments of the compound of Formula (I), or a pharmaceutically acceptable salt thereof, R^{6a} or R^{6c} is combined with one R⁴ group and the atoms to which they are attached to form a C₅₋₁₀ cycloalkyl or heterocyclyl, which is each optionally substituted with one to four R¹⁰.

In some embodiments of the compound of Formula (I), or a pharmaceutically acceptable salt thereof, each R^{6b1} and R¹⁰ is independently C₁₋₆ alkyl, C₁₋₆ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₀ cycloalkyl, heterocyclyl, C₆₋₁₀ aryl, heteroaryl, oxo, -OH, -CN, CO₂R^{3e}, -NO₂, or -C(O)N(R^{2a})(R^{2b}), wherein the heterocyclyl or heteroaryl is optionally substituted with C₁₋₆ alkyl, C₁₋₆ haloalkyl, or C₁₋₆ haloalkoxy.

In some embodiments of the compound of Formula (I), or a pharmaceutically acceptable salt thereof, each R^{6a1}, R^{6a2}, R^{6c1}, R^{6c2}, and R^{6c3} is independently H, C₁₋₆ alkyl or C₃₋₁₀ cycloalkyl.

In some embodiments of the compound of Formula (I), or a pharmaceutically acceptable salt thereof, each R^{9a} and R^{9b} is independently H, C₁₋₆ alkyl, or C₁₋₆ haloalkyl, or R^{9a} and R^{9b} together form a 6-membered heterocyclyl.

In some embodiments of the compound of Formula (I), or a pharmaceutically acceptable salt thereof, each R^{9c}, R^{9d}, R^{10a}, R^{10b}, and R^{10c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶.

In some embodiments of the compound of Formula (I), or a pharmaceutically acceptable salt thereof, R^{a} and R^{b} are independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶.

In some embodiments of the compound of Formula (I), or a pharmaceutically acceptable salt thereof, optionally R^{a} and R^{b} are combined with the atom to which they are attached to form a 5- or 6-membered heterocyclyl, which is optionally substituted with one to four R⁶.

In some embodiments of the compound of Formula (I), or a pharmaceutically acceptable salt thereof, each R^{2a}, R^{2b}, R^{12a}, R^{12b}, and R^{12c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶.

In some embodiments of the compound of Formula (I), or a pharmaceutically acceptable salt thereof, each R^{3e} is independently H, C₁₋₆ alkyl, C₁₋₆ haloalkyl, -C₁₋₄ alkyl-N(R^{9a})(R^{9b}), -C₁₋₄ alkyl-C(O)N(R^{9a})(R^{9b}), -C₁₋₄ alkyl-O-C(O)-C₁₋₄ alkyl, -C₁₋₄ alkyl-O-C(O)-O-C₁₋₄alkyl, -C₁₋₄ alkyl-O-C(O)-C₁₋₄ alkyl-N(R^{9a})(R^{9b}), -C₁₋₄ alkyl-C₃₋₈ cycloalkyl, -C₁₋₄ alkyl-heterocyclyl, C₃₋₁₀ cycloalkyl, heterocyclyl, C₆₋₁₀ aryl, heteroaryl, -P(O)(OR^{9c})₂, -CH2P(O)(OR^{9c})₂, - OCH₂P(O)(OR^{9c})₂, -C(O)OCH₂P(O)(OR^{9c})₂, -P(O)(R^{9c})(OR^{9d}), -OP(O)(R^{9c})(OR^{9d}), - CH₂P(O)(R^{9c})(OR^{9d}), -C(O)OCH₂P(O)(R^{9c})(OR^{9d}), -P(O)(N(R^{9c})₂)₂, -CH₂P(O)(N(R^{9c})₂)₂, - C(O)OCH₂P(O)(N(R^{9c})₂)₂, -P(O)(N(R^{9c})₂)(OR^{9d}), -CH₂P(O)(N(R^{9c})₂)(OR^{9d}), - C(O)OCH₂P(O)(N(R^{9c})₂)(OR^{9d}), -P(O)(R^{9c})(N(R^{9d})₂), -CH₂P(O)(R^{9c})(N(R^{9d})₂), or - C(O)OCH₂P(O)(R^{9c})(N(R^{9d})₂); wherein the alkyl, alkenyl, cycloalkyl, heterocyclyl, aryl, or heteroaryl is each optionally substituted with one to four R⁶;wherein each heteroaryl has 5 to 12 ring members and has one to four heteroatoms each independently N, O, or S; wherein each heterocyclyl has three to twelve ring members and has one to four heteroatoms, each independently N, O, or S.

In some embodiments of the compound of Formula (I), or a pharmaceutically acceptable salt thereof, each heteroaryl has five to twelve ring members and one to four heteroatoms, each independently N, O, or S.

In some embodiments, a compound of Formula (II) is provided: 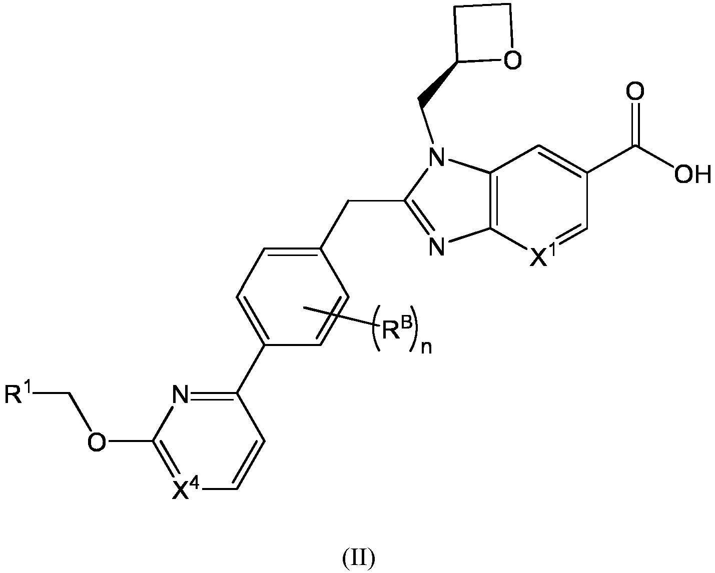 or a pharmaceutically acceptable salt thereof, wherein
R¹ is thiazolyl optionally substituted with R⁴;
X¹ is -C(H)= or -C(R⁸)=;
X⁴ is -C(H)=, -C(R⁸)= or N;
each R^{B} is independently C₁₋₉ alkyl, C₁₋₈ haloalkyl, or halogen;
each R⁸ is independently halogen; and
n is 0, 1, 2, or 3.

In some embodiments, a compound of Formula (I) is a compound according to Formula (II).

In some embodiments of a compound of Formula (II), or a pharmaceutically acceptable salt thereof, R¹ is 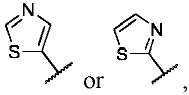 which is each substituted with C₁₋₈ haloalkyl.

In some embodiments of a compound of Formula (II), or a pharmaceutically acceptable salt thereof, R¹ is

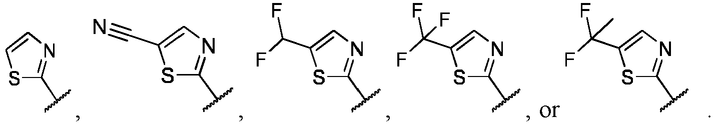

In some embodiments of a compound of Formula (II), or a pharmaceutically acceptable salt thereof, R⁸ is F or Cl

In some embodiments of the compound of Formula (I) or Formula (II), or a pharmaceutically acceptable salt thereof, has the structure of a compound in Table 2.

Also disclosed herein are the in vivo metabolic products of the compounds described herein, to the extent such products are novel and unobvious over the prior art. Such products may result for example from the oxidation, reduction, hydrolysis, amidation, esterification and the like of the administered compound, primarily due to enzymatic processes. Accordingly, included are novel and unobvious compounds produced by a process comprising contacting a compound with a mammal for a period of time sufficient to yield a metabolic product thereof. Such products typically are identified by preparing a radiolabeled (e.g. 14C or 3H) compound, administering it parenterally in a detectable dose (e.g. greater than about 0. 5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products can be easily isolated since they are labeled (others are isolated by the use of antibodies capable of binding epitopes surviving in the metabolite). The metabolite structures are determined in conventional fashion, e.g. by MS or NMR analysis. In general, analysis of metabolites can be done in the same way as conventional drug metabolism studies well-known to those skilled in the art. The conversion products, so long as they are not otherwise found in vivo, can be useful in diagnostic assays for therapeutic dosing of the compounds even if they possess no GLP-1R activity of their own.

Recipes and methods for determining stability of compounds in surrogate gastrointestinal secretions are known. Compounds are defined herein as stable in the gastrointestinal tract where less than about 50 mole percent of the protected groups are deprotected in surrogate intestinal or gastric juice upon incubation for 1 hour at 37 °C. Simply because the compounds are stable to the gastrointestinal tract does not mean that they cannot be hydrolyzed in vivo. The prodrugs typically will be stable in the digestive system but may be substantially hydrolyzed to the parental drug in the digestive lumen, liver, lung or other metabolic organ, or within cells in general. As used herein, a prodrug is understood to be a compound that is chemically designed to efficiently liberate the parent drug after overcoming biological barriers to oral delivery.

### III. METHODS OF PREPARING COMPOUNDS

The compounds of the present disclosure can be prepared by any method known in the art. The following exemplary general methods illustrate routes that may be used to obtain a compound of the present disclosure.

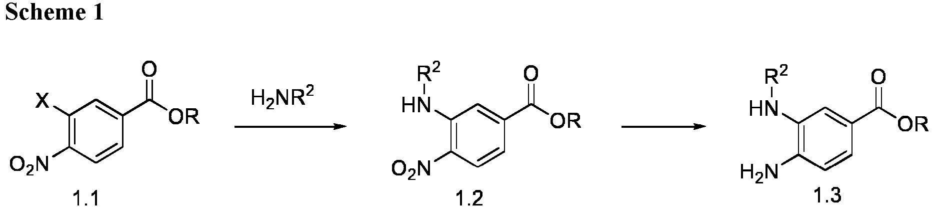

Intermediate 1.3 may be assembled by reacting an amine with Intermediate 1.1, wherein X is a halogen, and R is an alkyl, alkylaryl, or aryl, in the presence of a suitable base (*e.g*. DIPEA, KOtBu, etc.) to give Intermediate 1.2. Intermediate 1.2 can be converted to Intermediate 1. 3 using suitable reducing conditions (e.g. H₂ and Pd/C, Fe and HCl, etc.).

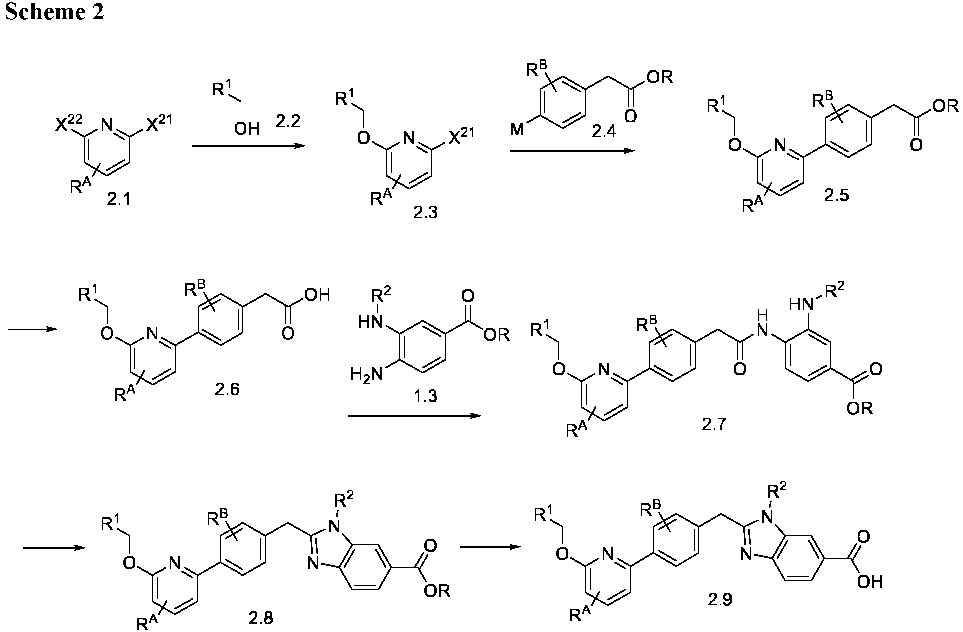

Compounds of Formula (I) having the structure of a compound of Formula 2.9 can be assembled by first coupling Intermediate 2.1, wherein X²¹ and X²² is each a leaving group, *e.g.,* a halogen such as Cl or Br, with a heteroatom containing Intermediate 2.2 (where Y = O, NH, or S) using either a suitable base (*e.g*., DIPEA, KOtBu, etc.) or through metal mediated cross-coupling using a suitable palladium catalyst to give Intermediate 2.3 (Scheme 2). Intermediate 2. 4 (where M = Li, MgBr, MgCl, or MgI, purchased commercially or obtained through metalation of a corresponding halide) can be combined with Intermediate 2.3 using a suitable palladium catalyst to deliver Intermediate 2.5. Following conversion to the acid Intermediate 2.6 using standard conditions (*e.g*. LiOH, LiI and pyridine, etc.), the Intermediate 1.3 can be added using standard amide bond forming conditions (*e.g*. DIPEA with HATU, etc.) to give Intermediate 2.7, which can, in turn, be converted to the corresponding benzimidazole Intermediate 2.8 under the influence of an acid catalyst (*e.g.* HCl, AcOH, etc.) This intermediate can be converted to the compound of Formula (I) using standard ester hydrolysis conditions (*e.g*., LiOH, LiI and pyridine, etc.).

While the above Scheme 2 is illustrated using Intermediate 2.1 as a dihalopyridine, any dihalogenated A-ring starting material can be used to obtain the analogous compound of Formula (I).

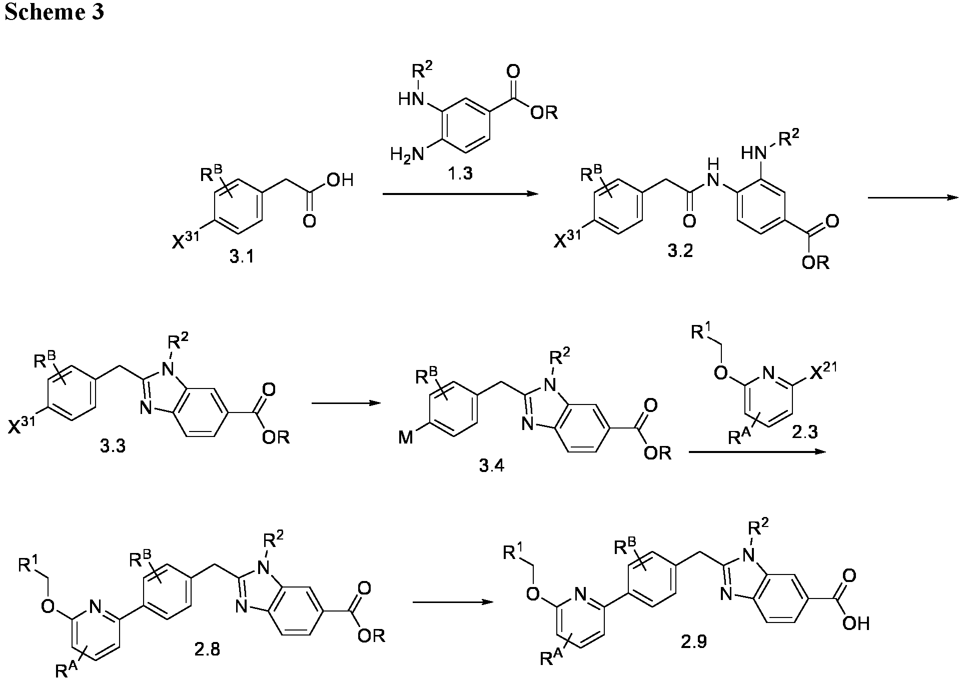

In some embodiments, a compound of Formula (I) having the structure of a compound of Formula 2.9 can be assembled first by the combination of Intermediate 3.1 (wherein X³¹ is Cl, Br, or I) with Intermediate 1.3 (wherein R = alkyl, alkylaryl, or aryl) under standard amide bond forming conditions, *e.g*. DIPEA with HATU, etc. (Scheme 3). Treatment with a suitable acid catalyst (*e.g.* HCl, AcOH, etc.) can deliver Intermediate 3.3. Halogen metal exchange of -X³¹ to -M can be achieved using a suitable reagent (*e.g*. iPrMgBr, etc.) or transition metal coupling using a suitable palladium catalyst and metal source (*e.g*. B₂Pin₂, Bu₆Sn₂, etc.) to give Intermediate 2.8 which can be converted to the compound of Formula (I) using standard ester hydrolysis conditions (*e.g*. LiOH, LiI and pyridine, etc.).

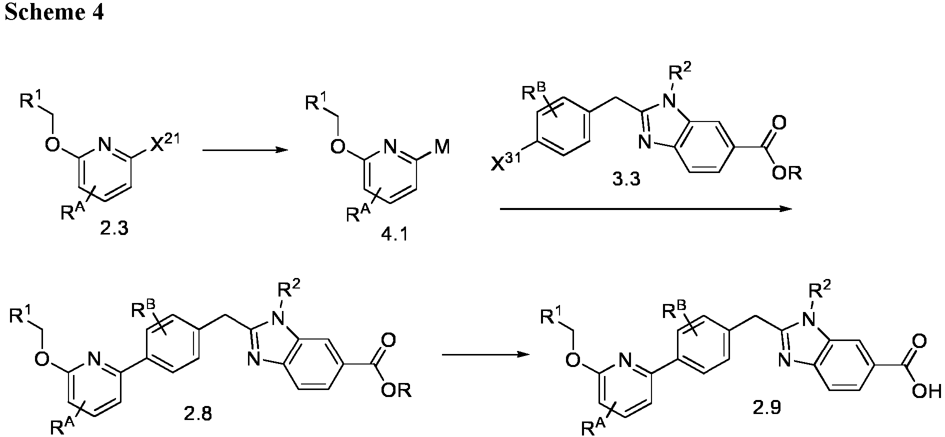

In some embodiments, a compound of Formula 2.9 can be formed by first conversion of Intermediate 2.3 to the metallated variant Intermediate 4.1 using a suitable palladium catalyst and metal source, *e.g*. B₂Pin₂, Bu₆Sn₂, etc. (Scheme 4). Intermediate 4.1 can be coupled to Intermediate 3.3 using a suitable palladium catalyst to deliver Intermediate 2.8 which can then be converted to the compound of Formula (I) using standard ester hydrolysis conditions, e.g. LiOH, LiI and pyridine, etc.

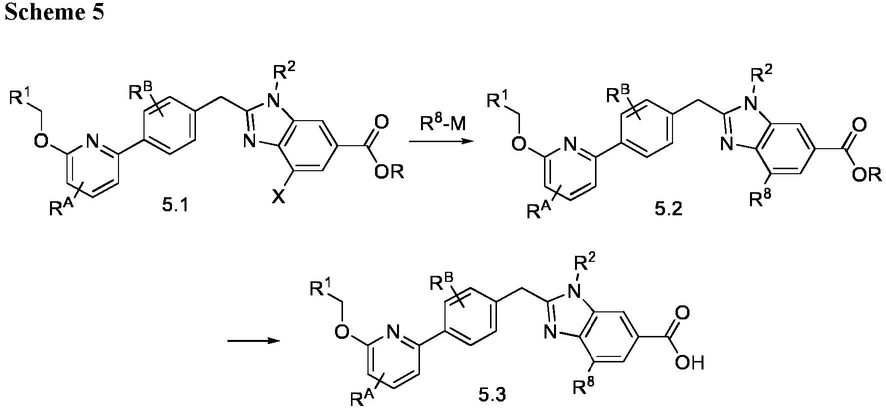

A compound of Formula (I-A-1) and/or Formula (I) having the structure of a compound of Formula 5.3 can be assembled via first coupling to the halogen -X (wherein X is Cl, Br, or I) of Intermediate 5.1 using a suitable coupling partner and palladium catalyst to deliver Intermediate 5.2 which can be converted to a compound of Formula 5.3 using standard ester hydrolysis conditions, *e.g*. LiOH, LiI and pyridine, etc. (Scheme 5).

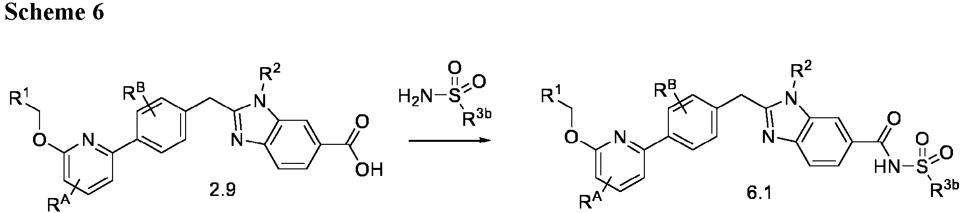

A compound of Formula (I) having the structure of a compound of Formula 6.1 can be obtained through the reaction of Intermediate 2.9 with a sulfonamide under suitable coupling conditions (*e.g*. EDCI and DMAP, etc.) (Scheme 6).

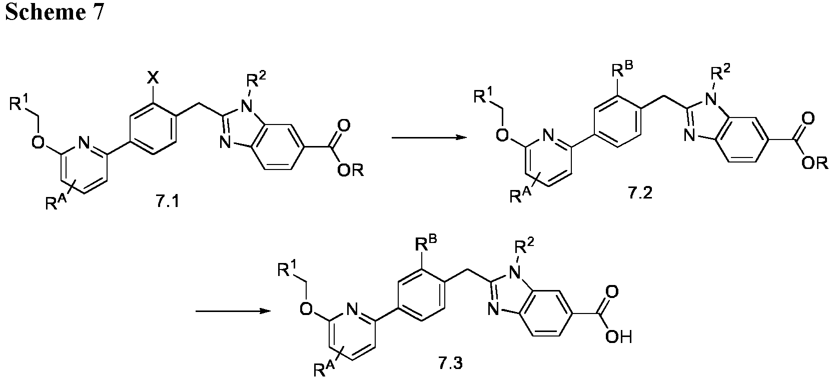

A compound of Formula (I) having the structure of a compound of Formula 7.3 can be assembled via first coupling to the halogen -X of Intermediate 7.1 using a suitable coupling partner and palladium catalyst to deliver Intermediate 7.2, which can be converted to a compound of Formula 7.3 using standard ester hydrolysis conditions (*e.g*. LiOH, LiI and pyridine, etc.) (Scheme 7).

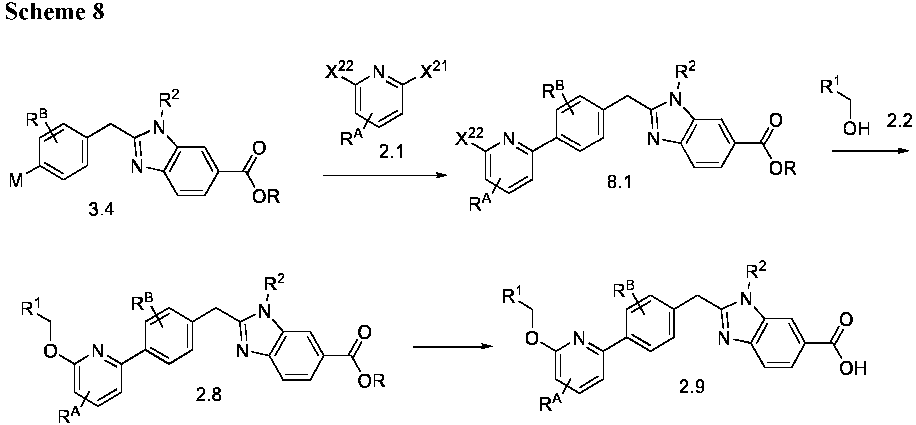

A compound of Formula (I) having the structure of a compound of Formula 2.9 can be assembled through first cross-coupling of an Intermediate 3.4 with Intermediate 2.1 using a suitable transition metal catalyst (e.g. palladium, etc.) (Scheme 8). This can then be coupled with a heteroatom containing Intermediate 2.2 (where Y = O, N or S) using either a suitable base (*e.g.* DIPEA, KOtBu, etc.) or through metal mediated cross-coupling using a suitable palladium catalyst to give Intermediate 2.8. Intermediate 2.8 can be converted to the compound of Formula (I) having the structure of a compound of Formula 2.9 using standard ester hydrolysis conditions (*e.g.* LiOH, LiI and pyridine, etc.).

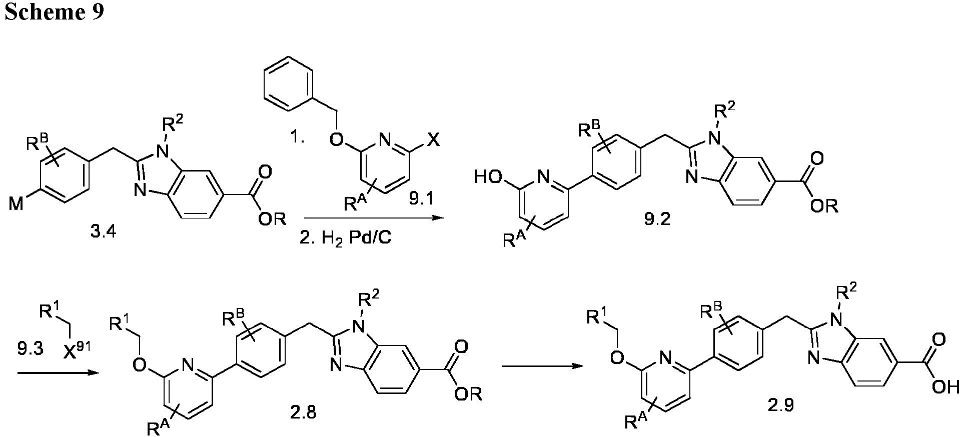

A compound of Formula (I) having the structure of a compound of Formula 2.9 can be assembled through first cross-coupling of an Intermediate 3.4 with Intermediate 9.1 using a suitable transition metal catalyst (*e.g*. palladium, etc.) (Scheme 9). The benzyl ether can then be removed through reduction using H₂ and a suitable catalyst (Pd/C, etc.) to yield intermediate 9.2. Intermediate 9.2 can then be alkylated using a suitable base (K₂CO₃, Cs₂CO₃, Ag₂CO₃, etc.) a suitable electrophile represented by intermediate 9.3 where X⁹¹ can be -Cl, -Br, I, or -OTs. Intermediate 2.8 can be converted to the compound of Formula (I) having the structure of a compound of Formula 2.9 using standard ester hydrolysis conditions (*e.g*. LiOH, LiI and pyridine, etc.).

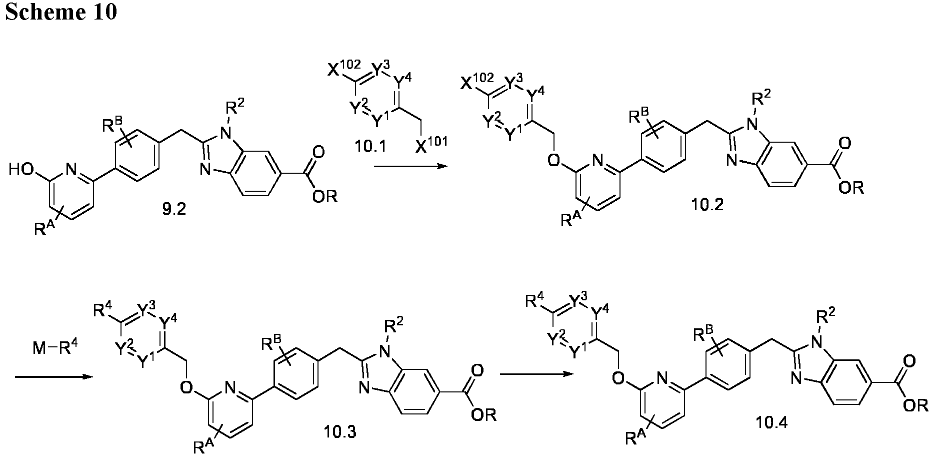

A compound of Formula (I) having the structure of a compound of Formula 10.4 can be assembled through first alkylation if intermediate 9.2 with an intermediate of the type 10.1 using a suitable base (K₂CO₃, Cs₂CO₃, Ag₂CO₃, etc.) where X¹⁰¹ and X¹⁰² are each independently -Cl, -Br, -I, -OTs, or -OTf and Y¹, Y², Y³, and Y⁴ are each independently -CH= or -N=. Intermediate 10.2 is then converted to intermediate 10.3 using a suitable transition metal catalyst (*e.g*. palladium, etc.). Intermediate 10.3 can be converted to the compound of Formula (I) having the structure of a compound of Formula 10.4 using standard ester hydrolysis conditions (*e.g*. LiOH, LiI and pyridine, etc.).

### IV. PHARMACEUTICAL FORMULATIONS

In some embodiments, the present disclosure provides a pharmaceutical composition comprising a compound of the present disclosure (*e.g*. a compound of Formula (I)), or a pharmaceutically acceptable salt thereof, and a pharmaceutically acceptable carrier or excipient.

In some embodiments of the disclosure, the pharmaceutical composition comprises a compound of Formula (I), or a pharmaceutically acceptable salt thereof, and one or more additional therapeutic agents, as more fully set forth below.

Pharmaceutical compositions comprising the compounds disclosed herein, or pharmaceutically acceptable salts thereof, may be prepared with one or more pharmaceutically acceptable excipients which may be selected in accord with ordinary practice. Tablets may contain excipients including glidants, fillers, binders and the like. Aqueous compositions may be prepared in sterile form, and when intended for delivery by other than oral administration generally may be isotonic. In some embodiments, compositions may contain excipients such as those set forth in the Rowe et al, Handbook of Pharmaceutical Excipients, 6th edition, American Pharmacists Association, 2009. Excipients can include ascorbic acid and other antioxidants, chelating agents such as EDTA, carbohydrates such as dextrin, hydroxyalkylcellulose, hydroxyalkylmethylcellulose, stearic acid and the like. In some embodiments, the composition is provided as a solid dosage form, including a solid oral dosage form.

The compositions include those suitable for various administration routes, including oral administration. The compositions may be presented in unit dosage form and may be prepared by any of the methods well known in the art of pharmacy. Such methods include the step of bringing into association the active ingredient (e.g., a compound of the present disclosure or a pharmaceutical salt thereof) with one or more pharmaceutically acceptable excipients. The compositions may be prepared by uniformly and intimately bringing into association the active ingredient with liquid excipients or finely divided solid excipients or both, and then, if desired, shaping the product. Techniques and formulations generally are found in Remington: The Science and Practice of Pharmacy, 21 st Edition, Lippincott Wiliams and Wilkins, Philadelphia, Pa., 2006.

Compositions described herein that are suitable for oral administration may be presented as discrete units (a unit dosage form) including but not limited to capsules, sachets or tablets each containing a predetermined amount of the active ingredient. In one embodiment, the pharmaceutical composition of the disclosure is a tablet.

Pharmaceutical compositions disclosed herein comprise one or more compounds disclosed herein, or a pharmaceutically acceptable salt thereof, together with a pharmaceutically acceptable excipient and optionally other therapeutic agents. Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more excipients including sweetening agents, flavoring agents, coloring agents and preserving agents, in order to provide a palatable preparation. Tablets containing the active ingredient in admixture with non-toxic pharmaceutically acceptable excipients which are suitable for manufacture of tablets are acceptable. These excipients may be, for example, inert diluents, such as calcium or sodium carbonate, lactose, lactose monohydrate, croscarmellose sodium, povidone, calcium or sodium phosphate; granulating and disintegrating agents, such as maize starch, or alginic acid; binding agents, such as cellulose, microcrystalline cellulose, starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

The amount of active ingredient that may be combined with the inactive ingredients to produce a dosage form may vary depending upon the intended treatment subject and the mode of administration. For example, in some embodiments, a dosage form for oral administration to humans may contain approximately 1 to 1000 mg of active material formulated with an appropriate and convenient amount of a pharmaceutically acceptable excipient. In some embodiments, the pharmaceutically acceptable excipient varies from about 5 to about 95% of the total compositions (weight:weight).

In some embodiments, a composition comprising a compound of the present disclosure, or a pharmaceutically acceptable salt thereof in one variation does not contain an agent that affects the rate at which the active ingredient is metabolized. Thus, it is understood that compositions comprising a compound of the present disclosure in one aspect do not comprise an agent that would affect (e.g., slow, hinder or retard) the metabolism of a compound of the present disclosure or any other active ingredient administered separately, sequentially or simultaneously with a compound of the present disclosure. It is also understood that any of the methods, kits, articles of manufacture and the like detailed herein in one aspect do not comprise an agent that would affect (e.g., slow, hinder or retard) the metabolism of a compound of the present disclosure or any other active ingredient administered separately, sequentially or simultaneously with a compound of the present disclosure.

In some embodiments, the pharmaceutical compositions described above are for use in a human or an animal.

The disclosure further includes a compound of the present disclosure for administration as a single active ingredient of a pharmaceutically acceptable composition which can be prepared by conventional methods known in the art, for example by binding the active ingredient to a pharmaceutically acceptable, therapeutically inert organic and/or inorganic carrier or excipient, or by mixing therewith.

In one aspect, provided herein is the use of a compound of the present disclosure as a second or other active ingredient having a synergistic effect with other active ingredients in known drugs, or administration of the compound of the present disclosure together with such drugs.

The compound of the present disclosure may also be used in the form of a prodrug or other suitably modified form which releases the active ingredient in vivo.

### V. ROUTES OF ADMINISTRATION

The compounds of the present disclosure (also referred to herein as the active ingredients), can be administered by any route appropriate to the condition to be treated. Suitable routes include oral, rectal, nasal, topical (including buccal and sublingual), transdermal, vaginal and parenteral (including subcutaneous, intramuscular, intravenous, intradermal, intratumoral, intrathecal and epidural), and the like. It will be appreciated that the preferred route may vary with for example the condition of the recipient. An advantage of certain compounds disclosed herein is that they are orally bioavailable and can be dosed orally.

A compound of the present disclosure may be administered to an individual in accordance with an effective dosing regimen for a desired period of time or duration, such as at least about one month, at least about 2 months, at least about 3 months, at least about 6 months, or at least about 12 months or longer. In one variation, the compound is administered on a daily or intermittent schedule for the duration of the individual's life.

The dosage or dosing frequency of a compound of the present disclosure may be adjusted over the course of the treatment, based on the judgment of the administering physician.

The compound may be administered to an individual (e.g., a human) in an effective amount. In some embodiments, the compound is administered once daily.

The compound can be administered by any useful route and means, such as by oral or parenteral (e.g., intravenous) administration. Therapeutically effective amounts of the compound may include from about 0. 00001 mg/kg body weight per day to about 10 mg/kg body weight per day, such as from about 0. 0001 mg/kg body weight per day to about 10 mg/kg body weight per day, or such as from about 0. 001 mg/kg body weight per day to about 1 mg/kg body weight per day, or such as from about 0. 01 mg/kg body weight per day to about 1 mg/kg body weight per day, or such as from about 0. 05 mg/kg body weight per day to about 0. 5 mg/kg body weight per day, or such as from about 0. 3 mg to about 30 mg per day, or such as from about 30 mg to about 300 mg per day.

A compound of the present disclosure may be combined with one or more additional therapeutic agents in any dosage amount of the compound of the present disclosure (e.g., from 1 mg to 1000 mg of compound). Therapeutically effective amounts may include from about 1 mg per dose to about 1000 mg per dose, such as from about 50 mg per dose to about 500 mg per dose, or such as from about 100 mg per dose to about 400 mg per dose, or such as from about 150 mg per dose to about 350 mg per dose, or such as from about 200 mg per dose to about 300 mg per dose. Other therapeutically effective amounts of the compound of the present disclosure are about 100, 125, 150, 175, 200, 225, 250, 275, 300, 325, 350, 375, 400, 425, 450, 475, or about 500 mg per dose. Other therapeutically effective amounts of the compound of the present disclosure are about 100 mg per dose, or about 125, 150, 175, 200, 225, 250, 275, 300, 350, 400, 450, or about 500 mg per dose. A single dose can be administered hourly, daily, or weekly. For example, a single dose can be administered once every 1 hour, 2, 3, 4, 6, 8, 12, 16 or once every 24 hours. A single dose can also be administered once every 1 day, 2, 3, 4, 5, 6, or once every 7 days. A single dose can also be administered once every 1 week, 2, 3, or once every 4 weeks. In some embodiments, a single dose can be administered once every week. A single dose can also be administered once every month.

Kits that comprise a compound of the present disclosure, or an enantiomer, or pharmaceutically acceptable salt thereof, or a pharmaceutical composition containing any of the above, are also included in the present disclosure. In one embodiment, a kit further includes instructions for use. In one aspect, a kit includes a compound of the disclosure, or a pharmaceutically acceptable salt, tautomer, stereoisomer, mixture of stereoisomers, prodrug, or deuterated analog thereof, and a label and/or instructions for use of the compounds in the treatment of the indications, such as the diseases or conditions, described herein. In one embodiment, kits comprising a compound of the present disclosure, or a pharmaceutically acceptable salt thereof, in combination with one or more (e.g., one, two, three, four, one or two, or one to three, or one to four) additional therapeutic agents are provided.

Provided herein are also articles of manufacture that include a compound of the present disclosure or a pharmaceutically acceptable salt, tautomer, stereoisomer, mixture of stereoisomers, prodrug, or deuterated analog thereof in a suitable container. The container may be a vial, jar, ampoule, preloaded syringe, and intravenous bag.

### VI. COMBINATION THERAPY

In some embodiments, a compound of the present disclosure, or a pharmaceutically acceptable salt thereof, can be combined with a therapeutically effective amount of one or more (*e.g.,* one, two, three, four, one or two, one to three, or one to four) additional therapeutic agents. In some embodiments, the additional therapeutic agent comprises an apoptotic signal-regulating kinase (ASK-1) inhibitor, a farnesoid X receptor (FXR) agonist, a peroxisome proliferator-activated receptor alpha (PPARα) agonist, fish oil, an acetyl-coA carboxylase (ACC) inhibitor, a TGFβ antagonist, a LPAR antagonist, a SGLT2 inhibitor, a Tpl2 inhibitor, or a GLP-1 agonist combination thereof.

The benefit of combination may be increased efficacy and/or reduced side effects for a component as the dose of that component may be adjusted down to reduce its side effects while benefiting from its efficacy augmented by the efficacy of the compound of the present disclosure.

In some embodiments, the therapeutic agent, or combination of therapeutic agents, are a(n) ACE inhibitor, 2-Acylglycerol O-acyltransferase 2 (DGAT2) inhibitor, Acetaldehyde dehydrogenase inhibitor, Acetyl CoA carboxylase inhibitor, Adrenergic receptor agonist, Alstrom syndrome protein 1(ALMS1)/PKC alpha protein interaction inhibitor, Apelin receptor agonist, Diacylglycerol O acyltransferase 2 inhibitor, Adenosine A3 receptor agonist, Adenosine A3 receptor antagonist, Adiponectin receptor agonist, Aldehyde dehydrogenase 2 stimulator, AKT protein kinase inhibitor, AMP-activated protein kinases (AMPK), AMP kinase activator, ATP citrate lyase inhibitor, AMP activated protein kinase stimulator, Endothelial nitric oxide synthase stimulator, NAD-dependent deacetylase sirtuin-1 stimulator, Adrenergic receptor antagonist, Androgen receptor agonist, Amylin receptor agonist, Angiotensin II AT-1 receptor antagonist, Apical sodium-dependent bile acid transport inhibitor, Autophagy protein modulator, Autotaxin inhibitors, Axl tyrosine kinase receptor inhibitor, Bax protein stimulator, Beta-catenin inhibitor, Bioactive lipid, Calcitonin agonist, Cannabinoid receptor modulator, Caspase inhibitor, Caspase-3 stimulator, Cathepsin inhibitor, Caveolin 1 inhibitor, CCK receptor antagonist, CCL26 gene inhibitor, CCR2 chemokine antagonist, CCR2 chemokine antagonist, Angiotensin II AT-1 receptor antagonist, CCR3 chemokine antagonist, CCR5 chemokine antagonist, CD3 antagonist, CDGSH iron sulfur domain protein modulator, chitinase inhibitor, Chloride channel stimulator, Chitotriosidase 1 inhibitor, CNR1 inhibitor, Connective tissue growth factor ligand inhibitor, COT protein kinase inhibitor, Cyclin D1 inhibitor, Cytochrome P450 7A1 inhibitor, Cytochrome P450 reductase inhibitors, DGAT1/2 inhibitor, Diacylglycerol O acyltransferase 1 inhibitor (DGAT1), Cytochrome P450 2E1 inhibitor (CYP2E1), CXCR3 chemokine antagonist, CXCR4 chemokine antagonist, Dihydroceramide delta 4 desaturase inhibitor, Dihydroorotate dehydrogenase inhibitor, Dipeptidyl peptidase IV inhibitor, Endosialin modulator, Eotaxin ligand inhibitor, Extracellular matrix protein modulator, Farnesoid X receptor agonist, Fatty acid synthase inhibitors, FGF1 receptor agonist, Fibroblast growth factor (FGF-15, FGF-19, FGF-21) ligands, fibroblast activation protein inhibitor, Free fatty acid receptor 1 agonist, Galectin-3 inhibitor, GDNF family receptor alpha like agonist, Glucagon receptor agonist, Glucagon-like peptide 1 agonist, Glucocorticoid receptor antagonist, Glucose 6-phosphate 1-dehydrogenase inhibitor, G-protein coupled bile acid receptor 1 agonist, G-protein coupled receptor-119 agonist, G-protein coupled receptor 84 antagonist, Hedgehog (Hh) modulator, Hepatitis C virus NS3 protease inhibitor, Hepatocyte nuclear factor 4 alpha modulator (HNF4A), Hepatocyte growth factor modulator, Histone deacetylase inhibitor, STAT-3 modulator, HMG CoA reductase inhibitor, HSD17B13 gene inhibitor, 5-HT 2a receptor antagonist, Hydrolase inhibitor, Hypoxia inducible factor-2 alpha inhibitor, IL-10 agonist, IL-17 antagonist, IL-22 agonist, Ileal sodium bile acid cotransporter inhibitor, Insulin sensitizer, Insulin ligand agonist, Insulin receptor agonist, integrin modulator, Integrin Antagonist, Integrin alpha-V/beta-1 antagonist, Integrin alpha-V/beta-6 antagonist, intereukin-1 receptor-associated kinase 4 (IRAK4) inhibitor, IL-6 receptor agonist, interleukin 17 ligand inhibitor, Jak2 tyrosine kinase inhibitor, Jun N terminal kinase-1 inhibitor, Kelch like ECH associated protein 1 modulator, Ketohexokinase (KHK) inhibitor, Klotho beta stimulator, Leukotriene A4 hydrolase inhibitor, 5-Lipoxygenase inhibitor, Lipoprotein lipase inhibitor, Liver X receptor, LPL gene stimulator, Lysophosphatidate-1 receptor antagonist, Lysyl oxidase homolog 2 inhibitor, LXR inverse agonists, Macrophage mannose receptor 1 modulator, Matrix metalloproteinases (MMPs) inhibitor, MEKK-5 protein kinase inhibitor, MCH receptor-1 antagonist, Membrane copper amine oxidase (VAP-1) inhibitor, Methionine aminopeptidase-2 inhibitor, Methyl CpG binding protein 2 modulator, MicroRNA-132 (miR-132) antagonist, MicroRNA-21(miR-21) inhibitor, Mitochondrial uncoupler, Mixed lineage kinase-3 inhibitor, Motile sperm domain protein 2 inhibitor, Myelin basic protein stimulator, NACHT LRR PYD domain protein 3 (NLRP3) inhibitor, NAD-dependent deacetylase sirtuin stimulator, NADPH oxidase inhibitor (NOX), NFE2L2 gene inhibitor, Nicotinic acid receptor 1 agonist, Opioid receptor mu antagonist, P2Y13 purinoceptor stimulator, Nuclear erythroid 2-related factor 2 stimulator, Nuclear receptor modulators, Nuclear transport of transcription factor modulator, P2X7 purinoceptor modulator, PACAP type I receptor agonist, PDE 3 inhibitor, PDE 4 inhibitor, PDE 5 inhibitor, PDGF receptor beta modulator, Phenylalanine hydroxylase stimulator, Phospholipase C inhibitor, Phosphoric diester hydrolase inhibitor, PPAR alpha agonist, PPAR delta agonist, PPAR gamma agonist, Peptidyl-prolyl cis-trans isomerase A inhibitor, PNPLA3 gene inhibitor, PPAR gamma modulator, Protease-activated receptor-2 antagonist, Protein kinase modulator, Protein NOV homolog modulator, PTGS2 gene inhibitor, renin inhibitor, Resistin/CAP1 (adenylyl cyclase associated protein 1) interaction inhibitor, Rho associated protein kinase inhibitor, RNA polymerase inhibitors, S-nitrosoglutathione reductase (GSNOR) enzyme inhibitor, Sodium glucose transporter-2 inhibitor, Sphingolipid delta 4 desaturase DES1 inhibitor, SREBP transcription factor inhibitor, STAT-1 inhibitor, Stearoyl CoA desaturase-1 inhibitor, STK25 inhibitor, Suppressor of cytokine signalling-1 stimulator, Suppressor of cytokine signalling-3 stimulator, Taste receptor type 2 agonist, Telomerase stimulator, TERT gene modulator, TGF beta (TGFB1) ligand inhibitor, TNF antagonist, Transforming growth factor β (TGF-β), Transforming growth factor β activated Kinase 1 (TAK1), Thyroid hormone receptor beta agonist, TLR-4 antagonist, Transglutaminase inhibitor, Tyrosine kinase receptor modulator, GPCR modulator, nuclear hormone receptor modulator, TLR-9 antagonist, VDR agonist, Vitamin D3 receptor modulators, WNT modulators, YAP/TAZ modulator or a Zonulin inhibitor, and combinations thereof.

Non-limiting examples of the one or more additional therapeutic agents include:
- ACE inhibitors, such as enalapril;
- Acetaldehyde dehydrogenase inhibitors, such as ADX-629;
- Acetyl CoA carboxylase (ACC) inhibitors, such as NDI-010976 (firsocostat), DRM-01, gemcabene, GS-834356, PF-05175157, QLT-091382, PF-05221304;
- Acetyl CoA carboxylase/Diacylglycerol O acyltransferase 2 inhibitors, such as PF-07055341;
- Adenosine receptor agonists, such as CF-102 (namodenoson), CF-101 (piclidenoson), CF-502, CGS21680;
- Adenosine A3 receptor antagonist, such as FM-101;
- Adiponectin receptor agonists, such as ADP-355, ADP-399, ALY668-SR;
- Adrenergic receptor antagonist, such as bromocriptine, phentermine, VI-0521;
- Aldehyde dehydrogenase 2 stimulators, such as FP-045;
- Amylin/calcitonin receptor agonists, such as KBP-042, KBP-089;
- AMP activated protein kinase stimulators, such as C-455, PXL-770, O-304;
- AMP kinase activators/ATP citrate lyase inhibitors, such as bempedoic acid (ETC-1002, ESP-55016);
- AMP activated protein kinase/Endothelial nitric oxide synthase/NAD-dependent deacetylase sirtuin-1 stimulators, such as NS-0200 (leucine + metformin + sildenafil);
- Androgen receptor agonists, such as LPCN-1144, LPCN-1148, testosterone prodrug;
- Angiotensin II AT-1 receptor antagonists, such as irbesartan; Angiopoietin-related protein-3 inhibitors, such as vupanorsen (IONIS-ANGPTL3-LRx);
- Apelin receptor agonist, such as CB-5064, MBT-2;
- Apical sodium-dependent bile acid transport inhibitors, such as A-3907
- Autophagy protein modulators, such as A-2906, GM-90194;
- Autotaxin (ectonucleotide pyrophosphatase/phosphodiesterase 2 (NPP2 or ENPP2)) inhibitors, such as FP10.47, PAT-505, PAT-048, GLPG-1690, X-165, PF-8380, TJC-0265, TJC-0316, AM-063, BBT-877;
- Axl tyrosine kinase receptor inhibitors, such as bemcentinib (BGB-324, R-428);
- Bax protein stimulators, such as CBL-514;
- Bioactive lipids, such as DS-102;
- Cannabinoid receptor modulators, such as namacizumab (nimacimab), GWP-42004, REV-200, CRB-4001, INV-101, SCN-002;
- Caspase inhibitors, such as emricasan;
- Pan cathepsin B inhibitors, such as VBY-376;
- Pan cathepsin inhibitors, such as VBY-825;
- CCK receptor antagonist, such as proglumide;
- CCL26 gene inhibitor, such as mosedipimod, KDDF-201410-10;
- CCR2/CCR5 chemokine antagonists, such as BMS-687681, cenicriviroc, maraviroc, CCX-872, leronlimab, WXSH-0213;
- CCR2/CCR5 chemokine antagonists and FXR agonists, such as LJC-242 (tropifexor + cenivriviroc);
- CCR2 chemokine antagonists, such as propagermanium;
- CCR2 chemokine/Angiotensin II AT-1 receptor antagonists, such as DMX-200, DMX-250;
- CCR3 chemokine antagonists, such as bertilimumab;
- CD3 antagonists, such as NI-0401 (foralumab);
- CDGSH iron sulfur domain protein modulators, such as EYP-002;
- Chitinase inhibitor, such as OATD-01;
- Chitotriosidase 1 inhibitors, such as OAT-2068;
- Chloride channel stimulators, such as cobiprostone, and lubiprostone;
- Casein kinase-1 (CK1) delta/epsilon inhibitors, such as PF-05006739;
- Connective tissue growth factor ligand inhibitor, such as PBI-4050;
- COT protein kinase inhibitors, such as GS-4875, GS-5290;
- CXCR4 chemokine antagonists, such as AD-214;
- Cytochrome P450 reductase inhibitors, such as SNP-630;
- Diglyceride acyltransferase 2 (DGAT2) inhibitors, such as IONIS-DGAT2Rx, PF-06865571;
- Diglyceride acyltransferase 1 (DGAT1) inhibitors, such as GSK-3008356;
- Diacylglycerol O acyltransferase 1 (DGAT1)/ Cytochrome P450 2E1 inhibitors (CYP2E1), such as SNP-610;
- Dihydroorotate dehydrogenase inhibitor, such as vidofludimus;
- Dipeptidyl peptidase IV inhibitors, such as linagliptin, evogliptin;
- Eotaxin ligand inhibitors, such as bertilimumab, CM-101;
- Extracellular matrix protein modulators, such as CNX-024;
- Farnesoid X receptor (FXR) agonists, such as AGN-242266, AGN-242256, ASC-42, EDP-297 (EP-024297), RDX-023, BWL-200, AKN-083, EDP-305, GNF-5120, cilofexor tromethamine (GS-9674), HPG-1860, IOT-022, LMB-763, obeticholic acid, Px-102, Px-103, M790, M780, M450, M-480, MET-409, MET-642, PX20606, SYHA-1805, vonafexor (EYP-001), TERN-101, TC-100, INT-2228, TQA-3526, ZG-5266, HPD-001, alendronate;
- Farnesoid X receptor (FXR)/ G-protein coupled bile acid receptor 1(TGR5) agonists, such as INT-767;
- Fatty acid synthase inhibitors, such as TVB-2640, FT-8225;
- Fibroblast growth factor 19 (rhFGF19)/cytochrome P450 (CYP) 7A1 inhibitors, such as aldafermin (NGM-282);
- Fibroblast growth factor 21(FGF-21) ligand modulators, such as AP-025, BMS-986171, B-1654, BIO89-100, BOS-580, Pegbelfermin (BMS-986036), B-1344, NN-9499;
- Fibroblast growth factor 21 (FGF-21)/glucagon like peptide 1 (GLP-1) agonists, such as YH-25723 (YH-25724; YH-22241), efruxifermin (AKR-001);
- FGF receptor agonists/Klotho beta stimulators, such as BFKB-8488A (RG-7992);
- Free fatty acid receptor 1 agonist, such as SCO-267;
- Galectin-3 inhibitors, such as belapectin (GR-MD-02), GB-1107 (Gal-300), GB-1211 (Gal-400), IMT-001;
- GDNF family receptor alpha like agonist, such as NGM-395;
- Glucagon-like peptide 1 (GLP1R) agonists, such as ALT-801, AC-3174, liraglutide, cotadutide (MEDI-0382), SAR-425899, LY-3305677, HM-15211, YH-25723, YH-GLP1, RPC-8844, PB-718, PF-06882961, semaglutide;
- Glucagon-like peptide 1 receptor agonist; Oxyntomodulin ligand; Glucagon receptor agonist, such as efinopegdutide;
- Gastric inhibitory polypeptide/Glucagon-like peptide-1 (GIP/GLP-1) receptor co-agonist, such as tirzepatide (LY-3298176);
- PEGylated long-acting glucagon-like peptide-1/glucagon (GLP-1R/GCGR) receptor dual agonist, such as DD-01;
- Glucagon/GLP1-receptor agonist, such as BI-456906, NN-6177;
- Glucocorticoid receptor antagonists, such as CORT-118335 (miricorilant);
- Glucose 6-phosphate 1-dehydrogenase inhibitors, such as ST001;
- Glucokinase stimulator, such as dorzagliatin, sinogliatin (RO-5305552);
- G-protein coupled bile acid receptor 1(TGR5) agonists, such as RDX-009, INT-777, HY-209;
- G-protein coupled receptor 84 antagonist, such as PBI-4547;
- G-protein coupled receptor-119 agonist, such as DA-1241;
- Heat shock protein 47 (HSP47) inhibitors, such as ND-L02-s0201;
- Hedgehog protein TGF beta ligand inhibitors, such as Oxy-210;
- Histone deacetylase inhibitors/ STAT-3 modulators, such as SFX-01;
- HMG CoA reductase inhibitors, such as atorvastatin, fluvastatin, pitavastatin, pravastatin, rosuvastatin, simvastatin;
- HSD17B13 gene inhibitor, such as ALN-HSD, ARO-HSD;
- Hydrolase inhibitor, such as ABD-X;
- Hypoxia inducible factor-2 alpha inhibitors, such as PT-2567;
- IL-10 agonists, such as peg-ilodecakin;
- Ileal sodium bile acid cotransporter inhibitors, such as odevixibat (A-4250), volixibat potassium ethanolate hydrate (SHP-262), GSK2330672, CJ-14199, elobixibat (A-3309);
- Insulin sensitizers, such as, KBP-042, azemiglitazone potassium (MSDC-0602K), ION-224, MSDC-5514, Px-102, RG-125 (AZD4076), Tolimidone, VVP-100X, CB-4211, ETI-101;
- Insulin ligand/dsInsulin receptor agonists, such as ORMD-0801;
- Integrin antagonists, such as IDL-2965;
- IL-6 receptor agonists, such as KM-2702;
- Integrin alpha-V/beta-6 and alpha-V/beta-1 dual inhibitor; such as PLN-74809;
- Interleukin 17 ligand inhibitor, such as netakimab;
- Jak1/2 tyrosine kinase inhibitor, such as baricitinib;
- Jun N terminal kinase-1 inhibitor, such as CC-90001;
- Kelch like ECH associated protein 1 modulator, such as alpha-cyclodextrin-stabilized sulforaphane;
- Ketohexokinase (KHK) inhibitors, such as PF-06835919, LY-3478045, LY-3522348;
- beta Klotho (KLB)- FGFIc agonists, such as MK-3655 (NGM-313);
- Leukotriene A4 hydrolase inhibitor, such as LYS-006;
- 5-Lipoxygenase inhibitors, such as tipelukast (MN-001), epeleuton (DS-102, AF-102);
- Lipoprotein lipase inhibitors, such as CAT-2003;
- LPL gene stimulators, such as alipogene tiparvovec;
- Liver X receptor (LXR) inhibitors, such as PX-665, PX-L603, PX-L493, BMS-852927, T-0901317, GW-3965, SR-9238;
- Lysophosphatidate-1 receptor antagonists, such as BMT-053011, UD-009 (CP-2090), AR-479, ITMN-10534, BMS-986020, KI-16198;
- Lysyl oxidase homolog 2 inhibitors, such as simtuzumab, PXS-5382A (PXS-5338);
- Macrophage mannose receptor 1 modulators, such as tilmanocept-Cy3 (technetium Tc 99m tilmanocept);
- Matrix metalloprotease inhibitors, such as ALS-L1023;
- Membrane copper amine oxidase (VAP-1) inhibitors, such as TERN-201, TT-01025;
- MEKK-5 protein kinase (ASK-1) inhibitors, such as CJ-16871, CS-17919, selonsertib (GS-4997), SRT-015, GS-444217, GST-HG-151, TERN-301;
- MCH receptor-1 antagonists, such as CSTI-100 (ALB-127158);
- Semicarbazide-Sensitive Amine Oxidase/Vascular Adhesion Protein-1 (SSAO/VAP-1) Inhibitors, such as PXS-4728A (BI-1467335);
- Methionine aminopeptidase-2 inhibitors, such as ZGN-1061, ZGN-839, ZN-1345;
- Methyl CpG binding protein 2 modulators, such as mercaptamine;
- Mineralocorticoid receptor antagonists (MCRA), such as MT-3995 (apararenone);
- Mitochondrial uncouplers, such as 2,4-dinitrophenol, HU6, Mito-99-0053;
- Mixed lineage kinase-3 inhibitors, such as URMC-099-C;
- Motile sperm domain protein 2 inhibitors, such as VB-601;
- Myelin basic protein stimulators, such as olesoxime;
- Myeloperoxidase inhibitors, such as PF-06667272, AZM-198;
- NADPH oxidase inhibitors, such as GKT-831, GenKyoTex, APX-311, setanaxib;
- Nicotinic acid receptor 1 agonists, such as ARI-3037MO;
- NACHT LRR PYD domain protein 3 (NLRP3) inhibitors, such as KDDF-201406-03, NBC-6, IFM-514, JT-194 (JT-349);
- NFE2L2 gene inhibitor, such as GeRP-amiR-144;
- Nuclear transport of transcription factor modulators, such as AMTX-100;
- Nuclear receptor modulators, such as DUR-928 (DV-928);
- Opioid receptor mu antagonists, such as methylnaltrexone;
- P2X7 purinoceptor modulators, such as SGM-1019;
- P2Y13 purinoceptor stimulators, such as CER-209;
- PDE 3/4 inhibitors, such as tipelukast (MN-001);
- PDE 5 inhibitors, such as sildenafil, MSTM-102;
- PDGF receptor beta modulators, such as BOT-191, BOT-509;
- Peptidyl-prolyl cis-trans isomerase inhibitors, such as CRV-431 (CPI-432-32), NVP-018, NV-556 (NVP-025);
- Phenylalanine hydroxylase stimulators, such as HepaStem;
- Phosphoric diester hydrolase inhibitor, such as ZSP-1601;
- PNPLA3 gene inhibitor, such as AZD-2693;
- PPAR agonists, such as Chiglitazar, elafibranor (GFT-505), seladelpar lysine (MBX-8025), deuterated pioglitazone R-enantiomer, pioglitazone, PXL-065 (DRX-065), saroglitazar, lanifibranor (IVA-337), CHS-131, pemafibrate (K-877), ZG-0588, ZSP-0678; ZSYM-008;
- Protease-activated receptor-2 antagonists, such as PZ-235;
- Protein kinase modulators, such as CNX-014;
- Protein NOV homolog modulators, such as BLR-200;
- PTGS2 gene inhibitors, such as STP-705, STP-707;
- Renin inhibitors, such as PRO-20;
- Resistin/CAP1 (adenylyl cyclase associated protein 1) interaction inhibitors, such as DWJ-211;
- Rev protein modulator, such as ABX-464;
- Rho associated protein kinase (ROCK) inhibitors, such as REDX-10178 (REDX-10325), KD-025, RXC-007, TDI-01;
- RNA polymerase inhibitors, such as rifaximin;
- Snitrosoglutathione reductase (GSNOR) enzyme inhibitors, such as SL-891;
- Sodium glucose transporter-2 (SGLT2) inhibitors, such as ipragliflozin, remogliflozin etabonate, ertugliflozin, dapagliflozin, tofogliflozin, sotagliflozin;
- Sodium glucose transporter-1/2 (SGLT 1/2) inhibitors, such as licogliflozin bis(prolinate) (LIK-066);
- SREBP transcription factor inhibitors, such as CAT-2003, HPN-01, MDV-4463;
- Stearoyl CoA desaturase-1 inhibitors, such as aramchol;
- Taste receptor type 2 agonists, such as ARD-101;
- Thyroid hormone receptor beta agonists, such as ALG-009, ASC-41, CNPT-101101; CNPT-101207, CS-27186, KY-41111, resmetirom (MGL-3196), MGL-3745, TERN-501, VK-2809, HP-515;
- TLR-2/TLR-4 antagonists, such as VB-201 (CI-201);
- TLR-4 antagonists, such as JKB-121, JKB-122, naltrexone;
- Tyrosine kinase receptor modulators, such as CNX-025, GFE-2137 (repurposed nitazoxanide);
- TLR-9 antagonist, such as GNKS-356, AVO-101;
- TNF antagonist, such as ALF-421;
- GPCR modulators, such as CNX-023;
- Nuclear hormone receptor modulators, such as Px-102;
- VDR agonist, such as CK-15;
- Xanthine oxidase inhibitors, such as ACQT-1127;
- Xanthine oxidase/Urate anion exchanger 1 (URAT1) inhibitors, such as RLBN-1001, RLBN-1127; or
- Zonulin Inhibitors, such as larazotide acetate (INN-202).

In certain specific embodiments, the one or more additional therapeutic agents are selected from A-4250, AC-3174, acetylsalicylic acid, AK-20, alipogene tiparvovec, AMX-342, AN-3015, anti-CXCR3 antibodies, anti-TAGE antibody, aramchol, ARI-3037MO, ASP-8232, AXA-1125, bertilimumab, Betaine anhydrous, BI-1467335, BMS-986036, BMS-986171, BMT-053011, BOT-191, BTT-1023, budesonide, BX-003, CAT-2003, cenicriviroc, CBW-511, CER-209, CF-102, CGS21680, CNX-014, CNX-023, CNX-024, CNX-025, cobiprostone, colesevelam, dabigatran etexilate mesylate, dapagliflozin, DCR-LIV1, deuterated pioglitazone R-enantiomer, 2,4-dinitrophenol, DRX-065, DS-102, DUR-928, edaravone (TTYP-01), EDP-305, elafibranor (GFT-505), emricasan, enalapril, ertugliflozin, evogliptin, F-351, fluasterone (ST-002), FT-4101, GDD-3898, GH-509, GKT-831, GNF-5120, GRI-0621, GR-MD-02, GS-300, GS-4997, GS-9674, GS-4875, GS-5290, HEC-96719, HTD-1801, HS-10356, HSG-4112, HST-202, HST-201, HU-6, hydrochlorothiazide, icosabutate (PRC-4016), icosapent ethyl ester, IMM-124-E, INT-767, INV-240, ION-455, IONIS-DGAT2Rx, ipragliflozin, Irbesarta, propagermanium, IVA-337, J2H-1702, JKB-121, KB-GE-001, KBLP-004, KBLP-009, KBP-042, KD-025, M790, M780, M450, metformin, sildenafil, LB-700, LC-280126, linagliptin, liraglutide, (LJN-452) tropifexor, LM-011, LM-002 (CVI-LM-002), LMB-763, LYN-100, MB-N-008, MBX-8025, MDV-4463, mercaptamine, MGL-3196, MGL-3745, MP-301, MSDC-0602K, namacizumab, NC-101, NDI-010976, ND-L02-s0201 (BMS-986263), NGM-282, NGM-313, NGM-386, NGM-395, NP-011, NP-135, NP-160, norursodeoxycholic acid, NV-422, NVP-022, O-304, obeticholic acid (OCA), 25HC3S, olesoxime, PAT-505, PAT-048, peg-ilodecakin, pioglitazone, pirfenidone, PRI-724, PX20606, Px-102, PX-L603, PX-L493, PXS-4728A, PZ-235, PZH-2109, RCYM-001, RDX-009, remogliflozin etabonate, RG-125 (AZD4076), RP-005, RPI-500, S-723595, saroglitazar, SBP-301, semaglutide, SH-2442, SHC-028, SHC-023, simtuzumab, solithromycin, sotagliflozin, statins (atorvastatin, fluvastatin, pitavastatin, pravastatin, rosuvastatin, simvastatin), TCM-606F, TEV-45478, TQA-3526, TQA-3563, tipelukast (MN-001), TLY-012, TRX-318, TVB-2640, TXR-611, TXR-612, TS-20004, UD-009, UN-03, ursodeoxycholic acid, VBY-376, VBY-825, VK-2809, vismo°Cib, volixibat potassium ethanolate hydrate (SHP-626), VVP-100X, WAV-301, WNT-974, WXSH-0038, WXSH-0078, XEN-103, XRx-117, XTYW-003, XW-003, XW-004, XZP-5610, ZGN-839, ZG-5216, ZSYM-008, or ZYSM-007.

In some embodiments, the compound of the present disclosure is combined with one or more thereapeutic agents selected from an anti-obesity agent including but not limited to peptide YY or an analogue thereof, a neuropeptide Y receptor type 2 (NPYR2) agonist, a NPYR1 agonist, an NPYR5 antagonist, a cannabinoid receptor type 1 (CB 1 R) antagonist, a lipase inhibitor (*e.g*., orlistat), a human proislet peptide (HIP), a melanocortin receptor 4 agonist (*e.g*., setmelanotide), a melanin concentrating hormone receptor 1 antagonist, a famesoid X receptor (FXR) agonist (*e.g*. obeticholic acid), apoptotic signal-regulating kinase (ASK-1) inhibitor, zonisamide, phentermine (alone or in combination with topiramate), a norepinephrine/dopamine reuptake inhibitor (*e.g*., buproprion), an opioid receptor antagonist (*e.g*., naltrexone), a combination of norepinephrine/dopamine reuptake inhibitor and opioid receptor antagonist (*e.g*., a combination of bupropion and naltrexone), a GDF-15 analog, sibutramine, a cholecystokinin agonist, amylin and analogues thereof (*e.g.*, pramlintide), leptin and analogues thereof (*e.g.,* metroleptin), a serotonergic agent (*e.g*., lorcaserin), a methionine aminopeptidase 2 (MetAP2) inhibitor (*e.g*., beloranib or ZGN-1061), phendimetrazine, diethylpropion, benzphetamine, an SGLT2 inhibitor (*e.g*., empagliflozin, canagliflozin, dapagliflozin, ipragliflozin, tofogliflozin, sergliflozin etabonate, remogliflozin etabonate, or ertugliflozin), an SGLTL1 inhibitor, a dual SGLT2/SGLT1 inhibitor, a fibroblast growth factor receptor (FGFR) modulator, an AMP-activated protein kinase (AMPK) activator, biotin, a MAS receptor modulator, or a glucagon receptor agonist (alone or in combination with another GLP-1 R agonist, *e.g*., liraglutide, exenatide, dulaglutide, albiglutide, lixisenatide, or semaglutide), an insulin sensitizer such as thiazolidinediones (TZDs), a peroxisome proliferator-activated receptor alpha (PPARα) agonist, fish oil, an acetyl-coA carboxylase (ACC) inhibitor, a transforming growth factor beta (TGFβ) antagonist, a GDNF family receptor alpha like (GFRAL) agonist, a melanocortin-4 receptor (MC4R) agonist, including the pharmaceutically acceptable salts of the specifically named agents and the pharmaceutically acceptable solvates of said agents and salts.

### VII. METHODS OF TREATMENT

In some embodiments, compounds of Formula (I), or pharmaceutically acceptable salt thereof, are useful in a method of treating and/or preventing a GLP-1R mediated disease or condition. In some embodiments, a method for treating and/or preventing a GLP-1R mediated disease or condition includes administering to a subject in need thereof a pharmaceutically effective amount of a compound of the present disclosure or pharmaceutically acceptable salt thereof. In some embodiments, compounds of the present disclosure have desirable properties, including for example advantageous pharmacokinetic properties, physicochemical properties such as hepatic uptake properties, and/or bile salt export pump (BSEP) inhibition characteristics. In one embodiment, compounds of the present disclosure have desirable pharmacokinetic properties, such as prolonged exposures and/or higher oral bioavailability. In one embodiment, compounds of the present disclosure have desirable hepatic uptake properties, such as reduced transporter-mediated hepatic uptake. In one embodiment, compounds of the present disclosure demonstrate desirable BSEP inhibition.

In some embodiments, the disease or condition comprises a liver disease or related diseases or conditions, e.g., liver fibrosis, non-alcoholic fatty liver disease (NAFLD), non-alcoholic steatohepatitis (NASH), liver cirrhosis, compensated liver fibrosis, decompensated liver fibrosis, hepatocellular carcinoma, Primary Biliary Cirrhosis (PBC), or Primary Sclerosing Cholangitis (PSC). In some embodiments, the disease or condition comprises a metabolic disease or related diseases or conditions, such as diabetes mellitus, obesity, or cardiometabolic diseases.

GLP-1R agonists are currently being investigated in connection with certain disorders and conditions, including for example diabetes. GLP-1 analogs that are DPP4 resistant and have longer half-lives than endogenous GLP-1 have been reported to be associated with weight loss and improved insulin action. Liraglutide, a peptide GLP-1R agonist approved in connection with treatment of diabetes, has been reported to show favorable improvements in outcomes in NASH subj ects.

In some embodiments, the present disclosure relates to the use of compounds of Formula (I), or a pharmaceutically acceptable salt thereof in the preparation of a medicament for the prevention and/or treatment of a disease or condition mediated by GLP-1R, such as a liver disease or metabolic disease. In some embodiments, the present disclosure relates to the use of compounds of Formula (I), or a pharmaceutically acceptable salt thereof in the preparation of a medicament for the prevention and/or treatment of a disease or condition mediated by GLP-1R, such as a liver disease or metabolic disease. For example, some embodiments provide a compound of Formula (I), or a pharmaceutically acceptable salt thereof, or a use thereof, for treatment and/or prevention of chronic intrahepatic or some forms of extrahepatic cholestatic conditions, of liver fibrosis, of acute intrahepatic cholestatic conditions, of obstructive or chronic inflammatory disorders that arise out of improper bile composition, of gastrointestinal conditions with a reduced uptake of dietary fat and fat-soluble dietary vitamins, of inflammatory bowel diseases, of lipid and lipoprotein disorders, of type II diabetes and clinical complications of type I and type II diabetes, of conditions and diseases which result from chronic fatty and fibrotic degeneration of organs due to enforced lipid and specifically triglyceride accumulation and subsequent activation of profibrotic pathways, of obesity and metabolic syndrome (combined conditions of dyslipidemia, diabetes and abnormally high body-mass index), of acute myocardial infarction, of acute stroke, of thrombosis which occurs as an endpoint of chronic obstructive atherosclerosis, of persistent infections by intracellular bacteria or parasitic protozoae, of non-malignant hyperproliferative disorders, of malignant hyperproliferative disorders, of colon adenocarcinoma and hepatocellular carcinoma for instance, of liver steatosis and associated syndromes, of liver failure or liver malfunction as an outcome of chronic liver diseases or of surgical liver resection, of Hepatitis B infection, of Hepatitis C infection and/or of cholestatic and fibrotic effects that are associated with alcohol-induced cirrhosis or with viral-borne forms of hepatitis, of type I diabetes, pre-diabetes, idiopathic type 1 diabetes, latent autoimmune diabetes, maturity onset diabetes of the young, early onset diabetes, malnutrition-related diabetes, gestational diabetes, hyperglycemia, insulin resistance, hepatic insulin resistance, impaired glucose tolerance, diabetic neuropathy, diabetic nephropathy, kidney disease, diabetic retinopathy, adipocyte dysfunction, visceral adipose deposition, obesity, eating disorders, sleep apnea, weight gain, sugar craving, dyslipidemia, hyperinsulinemia, congestive heart failure, myocardial infarction, stroke, hemorrhagic stroke, ischemic stroke, traumatic brain injury, pulmonary hypertension, restenosis after angioplasty, intermittent claudication, postprandial lipemia, metabolic acidosis, ketosis, arthritis, left ventricular hypertrophy, Parkinson's Disease, peripheral arterial disease, macular degeneration, cataract, glomerulosclerosis, chronic renal failure, metabolic syndrome, angina pectoris, premenstrual syndrome, thrombosis, atherosclerosis, impaired glucose metabolism, or vascular restenosis.

In some embodiments, a method of treating and/or preventing a non-alcoholic fatty liver disease (NAFLD), comprises administering to a subject in need thereof a compound of the present disclosure or a pharmaceutically acceptable salt thereof.

The disclosure also relates to a compound according to Formula (I) or a pharmaceutical composition comprising said compound for preventive and posttraumatic treatment of a cardiovascular disorder, such as acute myocardial infarction, acute stroke, or thrombosis which occur as an endpoint of chronic obstructive atherosclerosis. In some embodiments, a method for treating and/or preventing cardiovascular disorder comprises administering a compounds of Formula (I) to a subject in need thereof.

The disclosure further relates to a compound or pharmaceutical composition for the treatment and/or prevention of obesity and associated disorders such as metabolic syndrome (combined conditions of dyslipidemias, diabetes and abnormally high body-mass index) which can be overcome by GLP1R-mediated lowering of serum triglycerides, blood glucose and increased insulin sensitivity and GLP1R-mediated weight loss. In some embodiments, a method for treating and/or preventing a metabolic disease comprises administering a compounds of Formula (I) to a subject in need thereof. In some embodiments, a method for treating and/or preventing a metabolic disease comprises administering a compounds of Formula (I), to a subject in need thereof.

In a further embodiment, the compounds or pharmaceutical composition of the present disclosure are useful in preventing and/or treating clinical complications of Type I and Type II Diabetes. Examples of such complications include diabetic nephropathy, diabetic retinopathy, diabetic neuropathies, or Peripheral Arterial Occlusive Disease (PAOD). Other clinical complications of diabetes are also encompassed by the present disclosure. In some embodiments, a method for treating and/or preventing complications of Type I and Type II Diabetes comprises administering a compounds of Formula (I) to a subject in need thereof. In some embodiments, a method for treating and/or preventing complications of Type I and Type II Diabetes comprises administering a compounds of Formula (I) to a subject in need thereof.

Furthermore, conditions and diseases which result from chronic fatty and fibrotic degeneration of organs due to enforced lipid and/or triglyceride accumulation and subsequent activation of profibrotic pathways may also be prevented and/or treated by administering the compounds or pharmaceutical composition of the present disclosure. Such conditions and diseases can include NASH and chronic cholestatic conditions in the liver, Glomerulosclerosis and Diabetic Nephropathy in the kidney, Macular degeneration and Diabetic Retinopathy in the eye and neurodegenerative diseases, such as Alzheimer's Disease in the brain, or Diabetic Neuropathies in the peripheral nervous system. In some embodiments, a method for treating and/or preventing conditions and diseases which result from chronic fatty and fibrotic degeneration of organs due to enforced lipid and/or triglyceride accumulation and subsequent activation of profibrotic pathways comprises administering a compounds of Formula (I) to a subject in need thereof. In some embodiments, a method for treating and/or preventing conditions and diseases which result from chronic fatty and fibrotic degeneration of organs due to enforced lipid and/or triglyceride accumulation and subsequent activation of profibrotic pathways comprises administering a compounds of Formula (I) to a subject in need thereof. In some embodiments, a method for treating and/or preventing NASH comprises administering a compounds of Formula (I) to a subject in need thereof. In some embodiments, a method for treating and/or preventing NASH comprises administering a compounds of Formula (I) to a subject in need thereof.

Further provided herein is a pharmaceutical composition for use in treating a GLP-1R mediated disease or condition described herein, comprising a compound of the present disclosure or a pharmaceutically acceptable salt thereof.

The present disclosure also describes a use for the manufacture of a medicament in treating a GLP-1R mediated disease or condition comprising a compound of the present disclosure or a pharmaceutically acceptable salt thereof. Medicaments as referred to herein may be prepared by conventional processes, including the combination of a compound according to the present disclosure and a pharmaceutically acceptable carrier.

Also disclosed is a compound of the present disclosure or a pharmaceutically acceptable salt thereof for the treatment of a GLP-1R mediated disease or condition. Also disclosed is a compound of the present disclosure or a pharmaceutically acceptable salt thereof for the prevention of a GLP-1R mediated disease or condition.

### VIII. EXAMPLES

Many general references providing commonly known chemical synthetic schemes and conditions useful for synthesizing the disclosed compounds are available (see, *e.g.,* Smith, March's Advanced Organic Chemistry: Reactions, Mechanisms, and Structure, 7th edition, Wiley-Interscience, 2013.).

Compounds as described herein can be purified by any of the means known in the art, including chromatographic means, such as high-performance liquid chromatography (HPLC), preparative thin layer chromatography, flash column chromatography and ion exchange chromatography. Any suitable stationary phase can be used, including normal and reversed phases as well as ionic resins. For example, the disclosed compounds can be purified via silica gel and/or alumina chromatography. See, e.g., Introduction to Modern Liquid Chromatography, 2nd ed., ed. L. R. Snyder and J. J. Kirkland, John Wiley and Sons, 1979; and Thin Layer Chromatography, E. Stahl (ed.), Springer-Verlag, New York, 1969.

During any of the processes for preparation of the subject compounds, it may be desirable to protect sensitive or reactive groups on any of the molecules concerned. This may be achieved by means of conventional protecting groups as described in standard works, such as T. W. Greene and P. G. M. Wuts, "Protective Groups in Organic Synthesis," 4th ed., Wiley, New York 2006. The protecting groups may be removed at a convenient subsequent stage using methods known from the art.

Exemplary chemical entities useful in methods of the embodiments will now be described by reference to illustrative synthetic schemes for their general preparation herein and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be desirable to employ, in the place of the ultimately desired substituent, a suitable group that may be carried through the reaction scheme and replaced as appropriate with the desired substituent. Furthermore, one of skill in the art will recognize that the transformations shown in the schemes below may be performed in any order that is compatible with the functionality of the pendant groups. Each of the reactions depicted in the general schemes can be run at a temperature from about 0 °C to the reflux temperature of the organic solvent used.

The Examples provided herein describe the synthesis of compounds disclosed herein as well as intermediates used to prepare the compounds. It is to be understood that individual steps described herein may be combined. It is also to be understood that separate batches of a compound may be combined and then carried forth in the next synthetic step.

In the following description of the Examples, specific embodiments are described. These embodiments are described in sufficient detail to enable those skilled in the art to practice certain embodiments of the present disclosure. Other embodiments may be utilized, and logical and other changes may be made without departing from the scope of the disclosure. The embodiments are also directed to processes and intermediates useful for preparing the subject compounds or pharmaceutically acceptable salts thereof. The following description is, therefore, not intended to limit the scope of the present disclosure.

In some embodiments, the present disclosure generally provides a specific enantiomer or diastereomer as the desired product, although the stereochemistry of the enantiomer or diastereomer was not determined in all cases. When the stereochemistry of the specific stereocenter in the enantiomer or diastereomer is not determined, the compound is drawn without showing any stereochemistry at that specific stereocenter even though the compound can be substantially enantiomerically or disatereomerically pure.

Representative syntheses of compounds of the present disclosure are described in schemes below, and the examples that follow.

The compounds detailed in the Examples were synthesized according to the general synthetic methods described below. Compounds were named using ChemDraw version 18. 1. 0. 535 (PerkinElmer Informatics, Inc.) unless otherwise indicated.

### Abbreviations

Certain abbreviations and acronyms are used in describing the experimental details. Although most of these would be understood by one skilled in the art, Table 1 contains a list of many of these abbreviations and acronyms.

**Table 1. List of Abbreviations and Acronyms**

| **Abbreviation** | **Meaning** |
|---|---|
| Ac | acetate |
| AcOH | Acetic acid |
| ACN | acetonitrile |
| AmPhos | di-tert-butyl(4-dimethylaminophenyl)phosphine |
| Aq. | aqueous |
| Bn | benzyl |
| Bpin | (pinacolato)boron |
| B₂Pin₂ | bis(pinacolato)diboron |
| Bu | butyl |
| Bz | benzoyl |
| BzCl | benzoyl chloride |
| cataCXium^{®} A Pd G3 | Mesylate[(di(1-adamantyl)-n-butylphosphine)-2-(2'-amino-1,1'-biphenyl)]palladium(II) |
| CAN | Cerium Ammonium Nitrate |
| CDI | 1,1'-carbonyldiimidazole |
| DBA | dibenzalacetone |
| DBU | 1,8-Diazabicyclo[5. 4. 0]undec-7-ene |
| DCM | dichloromethane |
| DCE | dichlorethane |
| DEA | diethylamine |
| Deoxofluor | Bis(2-methoxyethyl)aminosulfur trifluoride |
| DIPEA | diisopropylethylamine |
| DMAP | 4-dimethylaminopyridine |
| DME | dimethoxyethane |
| DMEM | Dulbecco's Modified Eagle Medium |
| DMF | dimethylformamide |
| DMSO | dimethylsulfoxide |
| dppf | 1,1'-Ferrocenediyl-bis(diphenylphosphine) |
| EDCI | *N*-(3-dimethylaminopropyl)-*N*'-ethylcarbodiimide hydrochloride |
| ES/MS | electron spray mass spectrometry |
| Et | ethyl |
| FBS | fetal bovine serum |
| HATU | 1-[Bis(dimethylamino)methylene]-1*H*-1,2,3-triazolo[4,5-*b*]pyridinium 3-oxide hexafluorophosphate |
| IPA | isopropanol |
| JohnPhos | (2-Biphenyl)di-*tert*-butylphosphine |
| KOtBu | potassium tert-butoxide |
| LC | liquid chromatography |
| LCMS | liquid chromatography / mass spectrometry |
| MCPBA | meta-chloroperbenzoic acid |
| Me | methyl |
| m/z | mass to charge ratio |
| MS or ms | mass spectrum |
| NMP | *N*-methyl-2-pyrrolidone |
| OTs | Tosylate, *p*-toluenesulfonate |
| OTf | Triflate, trifluoromethanesulfonate |
| Pd Rockphos G3 | [(2-Di-*tert*-butylphosphino-3-methoxy-6-methyl-2',4',6'-triisopropyl-1,1'-biphenyl)-2-(2-aminobiphenyl)]palladium(II) methanesulfonate |
| Ph | phenyl |
| Ph₃P | triphenylphosphine |
| pin | pinacol |
| PO | By mouth/orally |
| Pyr | pyridine |
| RBF | round bottom flask |
| RP-HPLC | reverse phase high performance liquid chromatography |
| RT/rt | room temperature |
| SFC | supercritical fluid chromatography |
| tBuXPhos Pd G3 | [(2-Di-*tert*-butylphosphino-2',4',6'-triisopropyl-1,1'-biphenyl)-2-(2'-amino-1,1'-biphenyl)] palladium(II) methanesulfonate |
| TEA | triethylamine |
| TFA | trifluoroacetic acid |
| THF | tetrahydrofuran |
| TLC | Thin layer chromatography |
| Ts | 4-toluenesulfonyl |
| XPhos Pd G2 | Chloro(2-dicyclohexylphosphino-2',4',6'-triisopropyl-1,1'-biphenyl)[2-(2'-amino-1,1'-biphenyl)]palladium(II) |
| δ | parts per million referenced to residual solvent peak |
| δ | parts per million referenced to residual solvent peak |
| | |
| | |

### A. SYNTHESIS OF INTERMEDIATES

### Preparation of Intermediate I-1

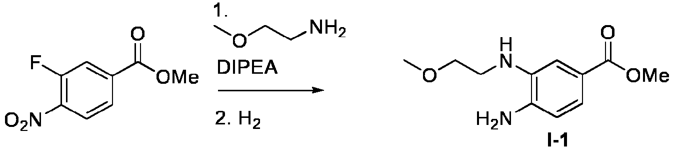

**Methyl 4-amino-3-(2-methoxyethylamino)benzoate (I-1):** To a solution of methyl 3-fluoro-4-nitro-benzoate (50. 0 g, 251 mmol) in THF (400 mL) was added diisopropylethylamine (70. 0 mL, 402 mmol) and 2-Methoxyethylamine (34. 9 mL, 402 mmol). The resulting solution was heated to 55 °C for 6 hrs. Upon completion the solvent was removed, and the resulting residue taken up in EtOAc (150 mL), washed with brine (30 mL), concentrated and carried forward without further purification. Methyl 3-(2-methoxyethylamino)-4-nitro-benzoate (20. 0 g, 78. 7 mmol) was then dissolved in EtOAc:EtOH (1:1, 140 mL) after which 10% palladium on carbon (5. 02 g, 4. 72 mmol) was then added. The resulting suspension was stirred under a hydrogen balloon at room temperature for 16 hrs. The reaction mixture was filtered through Celite washing with EtOAc (100 mL) and concentrated to give the desired compound without further purification: ES/MS: 225. 2 (M+H⁺).

### Preparation of Intermediate I-2

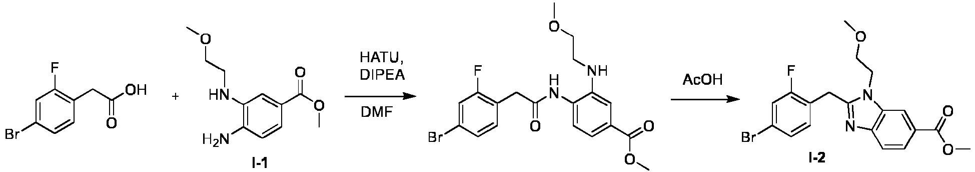

**Methyl 4-{[2-(4-bromo-2-fluoro-phenyl)acetyl] amino}-3-(2-methoxyethylamino)benzoate:** To a solution of 2-(4-bromo-2-fluoro-phenyl)acetic acid (1. 00 g, 4. 29 mmol) in DMF (20. 0 mL) was added methyl 4-amino-3-(2-methoxyethylamino)benzoate (1. 18 g, 5. 28 mmol) and *O*-(7-Azabenzotriazol-1-yl)-*N,N,N*',*N'-*tetramethyluronium hexafluorophosphate (1. 96 g, 5. 15 mmol) followed by *N,N-*diisopropylethylamine (3. 74 mL, 21. 5 mmol) and the reaction mixture was stirred for 2 h at room temperature. The mixture was concentrated *in vacuo,* the residue was taken up in EtOAc and washed with water (1x) and brine (1x). The organic layer was dried over sodium sulfate, filtered and concentrated *in vacuo.* The crude residue was taken forward without further purification, assuming full conversion: ES/MS m/z: 441.2 (M+H⁺).

**Methyl 2-[(4-bromo-2-fluoro-phenyl)methyl]-3-(2-methoxyethyl)benzimidazole-5-carboxylate (I-2):** The crude product from the previous step, methyl 4-{[2-(4-bromo-2-fluorophenyl)acetyl]amino}-3-(2-methoxyethylamino)benzoate (1. 89 g, 4. 29 mmol) was dissolved in AcOH (40. 0 mL) and the reaction mixture was heated to 60 °C for 2 h. The reaction mixture was concentrated *in vacuo* and the crude residue was taken up in DCM and washed with saturated aqueous sodium bicarbonate. The layers were separated, and the aqueous layer was extracted with DCM (2x). The combined organic extracts were dried over sodium sulfate, filtered and the filtrate was concentrated *in vacuo.* The crude residue was purified by column chromatography (0-100% EtOAc in hexane) to give the title compound: ES/MS m/z: 421. 9 (M+H⁺).

### Preparation of Intermediate I-3

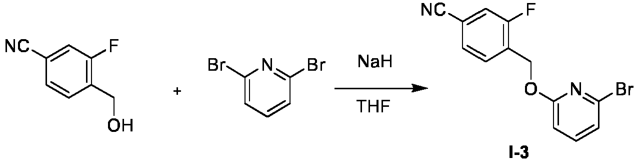

**4-[(6-bromo-2-pyridyl)oxymethyl]-3-fluoro-benzonitrile (I-3):** To a dried 100 mL RBF was added 3-fluoro-4-(hydroxymethyl)benzonitrile (2 g, 13. 2 mmol). The material was dissolved in dry THF (20 mL) under a nitrogen atmosphere at 0 °C. Sodium hydride (60% dispersion in mineral oil, 0. 507 g, 13.2 mmol) was added in one portion, and the mixture was stirred for 30 mins at 0 °C under N₂. Subsequently, 2,6-dibromopyridine (3. 13g, 13. 2 mmol) was added, and the reaction mixture was stirred room temperature overnight. The mixture was diluted with EtOAc (100 mL) and water (20 mL). The layers were separated, and the aqueous layer was extracted with ethyl acetate (2x 30 mL). The combined organic layers were dried over MgSO₄, filtered, and concentrated under reduced pressure. The crude material was purified by silica gel column chromatography (eluent: EtOAc/hexanes) to afford the Intermediate 1-6: ES/MS: 307. 058 (M+H⁺); ¹H NMR (400 MHz, Chloroform-*d*) δ 7. 72 - 7. 63 (m, 1H), 7. 52 - 7. 46 (m, 2H), 7. 41 (dd, *J =* 9. 2, 1. 5 Hz, 1H), 7. 14 (dd, *J =* 7. 5, 0. 7 Hz, 1H), 6. 79 (dd, *J =* 8. 2, 0. 7 Hz, 1H), 5. 50 (t, *J* = 0. 9 Hz, 2H).

### Preparation of Intermediate I-4

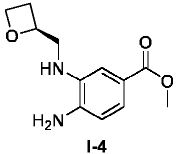

**Methyl (S)-4-amino-3-((oxetan-2-ylmethyl)amino)benzoate (I-4):** Methyl (S)-4-amino-3-((oxetan-2-ylmethyl)amino)benzoate was prepared following procedure Intermediate **I-1** substituting (S)-oxetan-2-ylmethanamine for 2-methoxyethylamine. ES/MS: 237.2 (M+H⁺).

### Preparation of Intermediate I-5

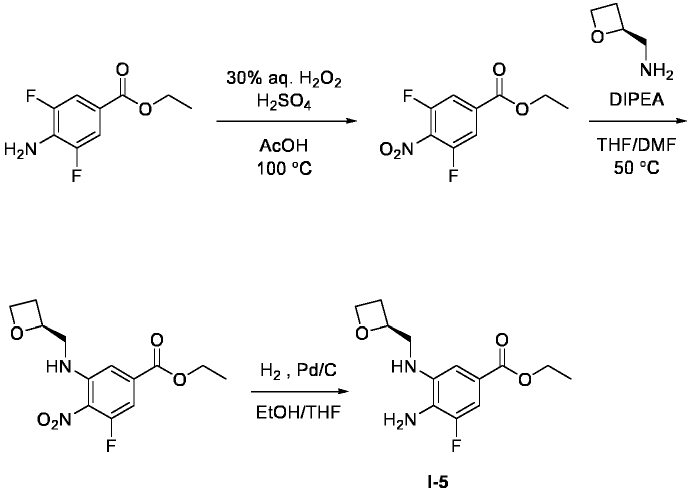

**Ethyl 3,5-difluoro-4-nitrobenzoate:** Ethyl 4-amino-3,5-difluorobenzoate (5.00 g, 24.9 mmol) was taken up in acetic acid (50.0 mL) and sulfuric acid (12.1 M, 2.05 mL, 24.9 mmol) and hydrogen peroxide (30% aqueous solution, 46.7 mL, 74.6 mmol) were added sequentially. The mixture was heated to 100 °C for 1 hour. The mixture was then cooled to room temperature and then slowly poured into 300 mL of ice water while swirling. The mixture was then diluted with EtOAc (200 mL), transferred to a separatory funnel, and the organic phase collected. The aqueous phase was extracted with EtOAc (2 x 100 mL) and the combined organics were dried over MgSO₄ and concentrated *in vacuo.* The residue was purified by column chromatography (eluent: EtOAc/Hexanes gradient) to afford the product.

**Ethyl (S)-3-fluoro-4-nitro-5-((oxetan-2-ylmethyl)amino)benzoate:** Ethyl 3,5-difluoro-4-nitro-benzoate (2.50 g, 10.8 mmol) and (S)-oxetan-2-ylmethanamine (989 mg, 11.4 mol) were taken up in tetrahydrofuran (12.0 mL) and N,N-dimethylformamide (6.0 mL), and N,N-diisopropylethylamine (9.42 mL, 54.1 mmol) was added. The mixture was heated to 50 °C for 16 hours. Following this time, the mixture was concentrated *in vacuo* and the residue purified by column chromatography (eluent: 0 - 25% EtOAc/Hexanes) to afford the product. ES/MS: 299.2 (M+H⁺).

**Ethyl (S)-4-amino-3-fluoro-5-((oxetan-2-ylmethyl)amino)benzoate (I-5):** Ethyl (S)-3-fluoro-4-nitro-5-((oxetan-2-ylmethyl)amino)benzoate (2.20 g, 7.38 mmol) was taken up in ethanol (10 mL) and tetrahydrofuran (5 mL) and the mixture sparged with nitrogen for 5 minutes. Palladium on carbon (10 wt. % loading, 785 mg, 0.74 mmol) was then added and sparging continued for 5 minutes. Hydrogen was then bubbled through the solution for one minute and then the mixture was set up under balloon hydrogen atmosphere for 21 hours. Following this time, the reaction was stopped, and the mixture was filtered through Celite. The filter was washed with EtOAc (2 × 20 mL) and methanol (2 × 10 mL) and the filtrate concentrated *in vacuo* to afford ethyl (S)-4-amino-3-fluoro-5-((oxetan-2-ylmethyl)amino)benzoate **(I-5).** ES/MS: 269.2 (M+H⁺); ¹H NMR (400 MHz, chloroform) δ 7.44 - 7.30 (m, 2H), 5.13 (qd, J = 7.1, 3.4 Hz, 1H), 4.72 (ddd, J = 8.7, 7.4, 6.0 Hz, 1H), 4.62 (dt, J = 9.1, 6.1 Hz, 1H), 4.33 (q, J = 7.1 Hz, 2H), 3.58 - 3.30 (m, 2H), 2.76 (dtd, J = 11.4, 8.0, 6.1 Hz, 1H), 2.56 (ddt, J = 11.3, 9.0, 7.1 Hz, 1H), 1.37 (t, J = 7.1 Hz, 3H).

### Preparation of Intermediate I-6

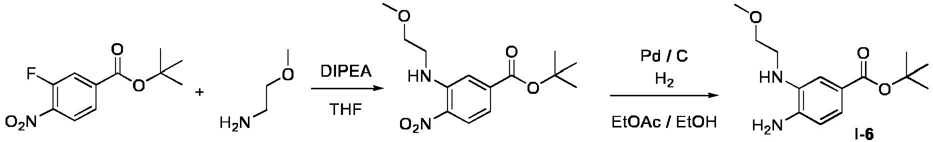

**Tert-butyl 3-((2-methoxyethyl)amino)-4-nitrobenzoate:** To a 500 mL RBF was added tert-butyl 3-fluoro-4-nitrobenzoate (10 g, 41.5 mmol). The material was dissolved in THF (150 mL), and 2-methoxyethanamine (7.2 mL, 82.9 mmol) and N,N-diisopropylethylamine (21.7 mL, 124 mmol) were added. The mixture was stirred at 50 °C overnight. Afterward, the mixture was concentrated to remove most of the THF, and the crude material was dissolved in EtOAc (400 mL). The organics were washed with 50% NH₄Cl (2x 100 mL) and with brine (1x 50 mL). The organics were subsequently dried over MgSO₄, filtered, and concentrated under reduced pressure. The crude material was carried forward without further purification: ES/MS: 297.1 (M+H⁺); ¹H NMR (400 MHz, Chloroform-d) δ 8.21 (d, J = 8.9 Hz, 1H), 7.55 (d, J = 1.7 Hz, 1H), 7.20 (dd, J = 8.9, 1.7 Hz, 1H), 3.72 (dd, J = 5.8, 4.8 Hz, 2H), 3.57 (q, J = 5.2 Hz, 2H), 3.46 (s, 3H), 1.62 (s, 9H).

**Tert-butyl 4-amino-3-((2-methoxyethyl)amino)benzoate (I-6):** To a 1L RBF was added tert-butyl 3-((2-methoxyethyl)amino)-4-nitrobenzoate (13 g, 43.9 mmol), ethanol (100 mL), and EtOAc (50 mL). The mixture was stirred and sonicated until all material was dissolved. Nitrogen was bubbled through the mixture for 5 minutes, and then palladium on carbon (10% wt, 2.33 g, 2.19 mmol) was added. Hydrogen was bubbled through the mixture for 5 minutes, and the mixture was stirred overnight under a hydrogen balloon. Nitrogen was subsequently bubbled through the flask for 10 minutes, and then the mixture was filtered through Celite to remove the catalyst. The filtrate was concentrated under reduced pressure and was used without further purification: ES/MS: 267.2 (M+H⁺).

### Preparation of Intermediate I-7

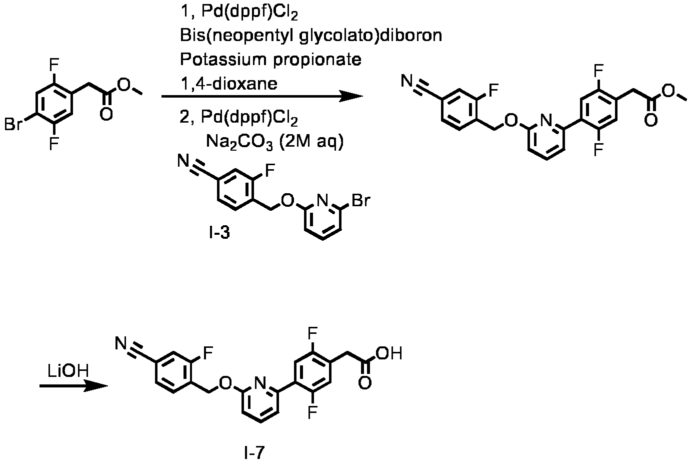

**Methyl 2-(4-(6-((4-cyano-2-fluorobenzyl)oxy)pyridin-2-yl)-2,5-difluorophenyl)acetate:** A suspension of methyl 2-(4-bromo-2,5-difluorophenyl)acetate (10.5 g, 39.6 mmol), Bis(neopentyl glycolato)diboron (17.9 g, 79.2 mmol), [1,1'-Bis(diphenylphosphino)ferrocene] dichloropalladium(II) ;PdCl₂(dppf) (2.94 g, 3.96 mmol), and potassium propionate (15.6 g, 139 mmol) in dioxane (50 mL) was degassed with Ar for 20 min. The mixture was sealed and heated at 100 °C for 2 hours. Sodium carbonate (2.0 M, 39.6 mL, 79.2 mmol) was added and the mixture was stirred at RT for 10 min. [1,1'-Bis(diphenylphosphino)ferrocene] dichloropalladium(II) ;PdCl₂(dppf) (1.47 g, 1.98 mmol) and **I-3** (14 g, 45.6 mmol) were added, the mixture was degassed for 10 min with Ar, then sealed and heated at 100 °C for 1 hour. The mixture was diluted with EtOAc and washed with brine. The organic extract was dried over sodium sulfate and chromatographed (eluent: EtOAc/hexanes) to give the title product: ES/MS: 413.2 (M+H⁺).

**2-(4-(6-((4-cyano-2-fluorobenzyl)oxy)pyridin-2-yl)-2,5-difluorophenyl)acetic acid (I-7).** A solution of methyl 2-(4-(6-((4-cyano-2-fluorobenzyl)oxy)pyridin-2-yl)-2,5-difluorophenyl)acetate (12.5 g, 30.3 mmol) and lithium hydroxide (0.2 M, 19.7 mL, 39.4 mmol) in CH₃CN (50 mL) was heated at 50 °C for 2 hours. The mixture was acidified with 1 N of hydrochloride to pH= 6-7. The material crashed out and was filtered by filter funnel. The solid was washed with water and dried overnight to yield the product: ES/MS: 399.2 (M+H⁺); ¹H NMR (400 MHz, Methanol-d4) δ 7.83 - 7.77 (m, 1H), 7.78 - 7.65 (m, 2H), 7.64 - 7.59 (m, 2H), 7.58 - 7.51 (m, 1H), 7.26 - 7.14 (m, 1H), 6.91 (d, J = 8.2 Hz, 1H), 5.63 (s, 2H), 3.73 (d, J = 1.2 Hz, 2H).

### Preparation of Intermediate I-8

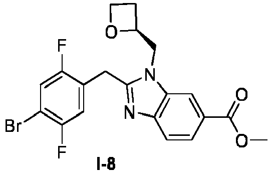

**Methyl (S)-2-(4-bromo-2,5-difluorobenzyl)-1-(oxetan-2-ylmethyl)-1H-benzo[d]imidazole-6-carboxylate (I-8):** Methyl (S)-2-(4-bromo-2,5-difluorobenzyl)-1-(oxetan-2-ylmethyl)-1H-benzo[d]imidazole-6-carboxylate was prepared following procedure Intermediate **I-2** substituting **I-4** for **I-1** and 2-(4-bromo-2,5-difluorophenyl)acetic acid for 2-(4-bromo-2-fluoro-phenyl)acetic acid. ES/MS: 451.0, 453.0 (M+H⁺).

### Preparation of Intermediate I-9

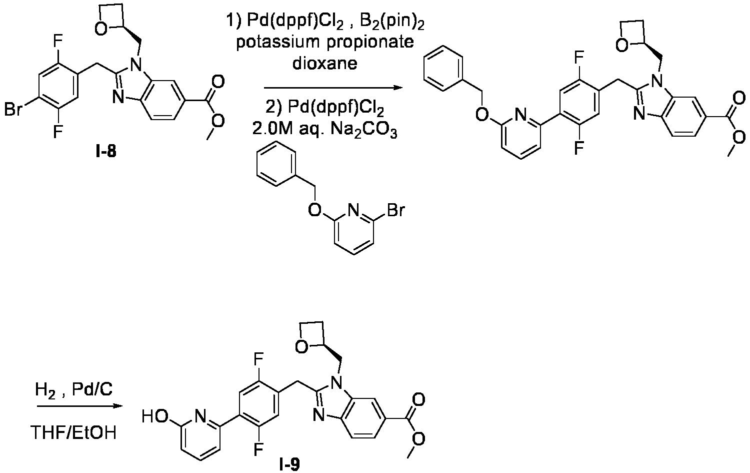

**Methyl (S)-2-(4-(6-(benzyloxy)pyridin-2-yl)-2,5-difluorobenzyl)-1-(oxetan-2-ylmethyl)-1H-benzo[d]imidazole-6-carboxylate:** Methyl (S)-2-(4-bromo-2,5-difluorobenzyl)-1-(oxetan-2-ylmethyl)-1H-benzo[d]imidazole-6-carboxylate **(I-8)** (450 mg, 0.997 mmol), Pd(dppf)Cl₂ (74.0 mg, 0.100 mmol), potassium propionate (336 mg, 2.99 mmol), and bis(pinacolato)diboron (304 mg, 2.99 mmol) were taken up in 1,4-dioxane (4.00 mL) and the mixture sparged with argon for 5 minutes. The mixture was then heated to 110 °C for one hour. Following this time, complete conversion to the intermediate boronate ester was observed. The mixture was cooled to r.t. and aqueous sodium carbonate (2.0 M, 0.997 mL, 1.99 mmol) was added. The mixture was stirred for 5 minutes, then 2-(benzyloxy)-6-bromopyridine (290 mg, 1.10 mmol) and Pd(dppf)Cl₂ (37.0 mg, 0.050 mmol) were added and the mixture heated to 90 °C for 1 hour. The mixture was then loaded directly onto SiO₂ for purification with normal phase column chromatography (eluent: EtOAc/CH₂Cl₂ gradient) which afforded the desired product. ES/MS: 556.2 (M+H⁺).

**Methyl (S)-2-(2,5-difluoro-4-(6-hydroxypyridin-2-yl)benzyl)-1-(oxetan-2-ylmethyl)-1H-benzo[d]imidazole-6-carboxylate (I-9):** Methyl (S)-2-(4-(6-(benzyloxy)pyridin-2-yl)-2,5-difluorobenzyl)-1-(oxetan-2-ylmethyl)-1H-benzo[d]imidazole-6-carboxylate (426.0 mg, 0.767 mmol) was taken up in ethanol (6.0 mL) and tetrahydrofuran (3.0 mL) and the solution sparged with nitrogen for 5 minutes. Pd/C (408 mg, 0.383 mmol) was added and nitrogen bubbled through the suspension for an additional 5 minutes. Hydrogen was then bubbled through the solution for 5 minutes before the reaction was set up under balloon hydrogen atmosphere. The mixture was stirred at RT for 30 minutes. Following this time, the suspension was filtered through celite, washed with EtOAc (3 × 10 mL). The filtrate was concentrated in vacuo to afford the methyl (S)-2-(2,5-difluoro-4-(6-hydroxypyridin-2-yl)benzyl)-1-(oxetan-2-ylmethyl)-1H-benzo[d]imidazole-6-carboxylate **(I-9).** ES/MS: 466.2 (M+H⁺).

### Preparation of Intermediate I-10

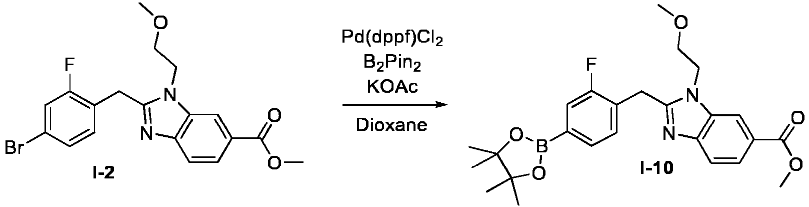

**Methyl 2-[[2-fluoro-4-(4,4,5,5-tetramethyl-1,3,2-dioxaborolan-2-yl)phenyl]methyl]**- **3-(2-methoxyethyl)benzimidazole-5-carboxylate (I-10):** To a vial, methyl 2-[(4-bromo-2-fluoro-phenyl)methyl]-3-(2-methoxyethyl)benzimidazole-5-carboxylate **(I-2)** (200 mg, 0.475 mmol), 4,4,5,5-tetramethyl-2-(4,4,5,5-tetramethyl-1,3,2-dioxaborolan-2-yl)-1,3,2-dioxaborolane (145 mg, 0.570 mmol), (1,1'-bis(diphenylphosphino)ferrocene)-dichloropalladium(II) (33.6 mg, 0.0475 mmol) and potassium acetate (0.140 g, 1.42 mmol) was added. Next, 1,4-dioxane (4.80 mL) was added and the mixture was heated to 100 °C for 24 hr. The reaction mixture was filtered through celite, eluting with DCM and the filtrate was concentrated *in vacuo.* The crude residue was purified by column chromatography (0-100% EtOAc in hexane) to give methyl 2-[[2-fluoro-4-(4,4,5,5-tetramethyl-1,3,2-dioxaborolan-2-yl)phenyl]methyl]-3-(2-methoxyethyl)benzimidazole-5-carboxylate (I-10). ES/MS m/z: 469.4 (M+H⁺).

### Preparation of Intermediate I-11

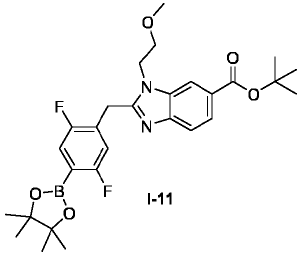

**Tert-butyl 2-(2,5-difluoro-4-(4,4,5,5-tetramethyl-1,3,2-dioxaborolan-2-yl)benzyl)-1-(2-methoxyethyl)-1H-benzo[d]imidazole-6-carboxylate (I-11):** *tert*-butyl 2-(2,5-difluoro-4-(4,4,5,5-tetramethyl-1,3,2-dioxaborolan-2-yl)benzyl)-1-(2-methoxyethyl)-1H-benzo[d]imidazole-6-carboxylate was prepared in a manner as described for Intermediate **I-10** substituting 2-(4-bromo-2,5-difluorophenyl)acetic acid for 2-(4-bromo-2-fluorophenyl)acetic acid and tert-butyl 4-amino-3-((2-methoxyethyl)amino)benzoate **(I-6)** for methyl 4-amino-3-(2-methoxyethylamino)benzoate. ES/MS: 529.3 (M+H⁺); ¹H NMR (400 MHz, DMSO-*d*⁶) δ 8.13 (s, 1H), 7.74 (dd, *J =* 8.4, 1.6 Hz, 1H), 7.56 (d, *J =* 8.4 Hz, 1H), 7.33 (dd, *J =* 9.3, 4.6 Hz, 1H), 7.17 (dd, *J =* 9.1, 5.5 Hz, 1H), 4.54 (t, *J =* 5.1 Hz, 2H), 4.39 (s, 2H), 3.65 (t, *J =* 5.1 Hz, 2H), 3.20 (s, 3H), 1.57 (s, 9H), 1.31 (s, 12H).

### Preparation of Intermediate I-12

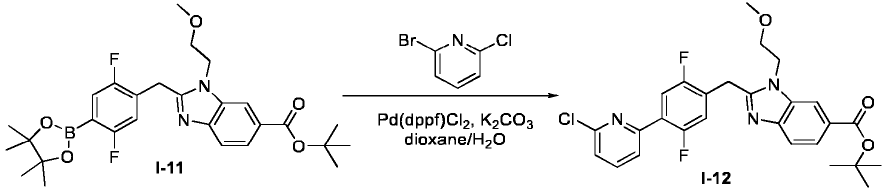

**Tert-butyl 2-[[4-(6-chloropyridin-2-yl)-2,5-difluorophenyl]methyl] -3-(2-methoxyethyl)-1,3-benzodiazole-5-carboxylate (I-12):** *tert*-butyl 2-[[2,5-difluoro-4-(4,4,5,5-tetramethyl-1,3,2-dioxaborolan-2-yl)phenyl]methyl]-3-(2-methoxyethyl)-1,3-benzodiazole-5-carboxylate **(I-11)** (30.0 g, 56.7 mmol, 1.00 equivalent) and 2-bromo-6-chloropyridine (14.2 g, 73.8 mmol, 1.30 equivalent) were dissolved in 1,4-dioxane (600 mL) and H₂O (60 mL). To the solution Pd(dppf)Cl₂ (4.15 g, 5.68 mmol, 0.1 equivalent) and K₂CO₃ (15.7 g, 114 mmol, 2.0 equivalent) were added. The resulting solution was heated to 90 °C overnight under nitrogen atmosphere. The resulting mixture was concentrated under reduced pressure. The residue was purified by silica gel column chromatography, eluted with petroleum ether /EtOAc (2/1) to afford tert-butyl 2-[[4-(6-chloropyridin-2-yl)-2,5-difluorophenyl]methyl]-3-(2-methoxyethyl)-1,3-benzodiazole-5-carboxylate **(I-12).** ES/MS: 513.8 (M+H⁺); ¹H NMR (400 MHz, DMSO-d⁶) δ 8.14 (s, 1H), 8.02 (t, J = 7.9 Hz, 1H), 7.87 (d, J = 7.7 Hz, 1H), 7.78 -7.69 (m, 2H), 7.59 (d, J = 8.2 Hz, 2H), 7.41 (dd, J = 11.4, 6.0 Hz, 1H), 4.58 (t, J = 5.1 Hz, 2H), 4.44 (s, 2H), 3.68 (t, J = 5.1 Hz, 2H), 3.22 (s, 3H), 1.58 (s, 9H).

### Preparation of Intermediate I-13

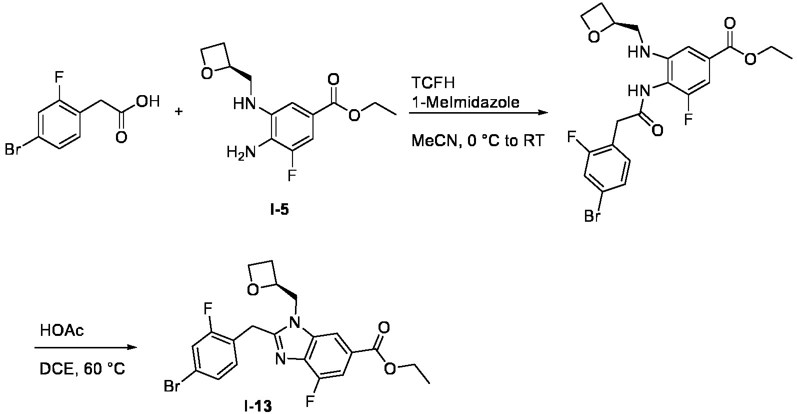

**Ethyl (S)-4-(2-(4-bromo-2-fluorophenyl)acetamido)-3-fluoro-5-((oxetan-2-ylmethyl)amino)benzoate:** A solution of **I-5** (500 mg, 1.86 mmol) and 2-(4-bromo-2-fluorophenyl)acetic acid (521 mg, 2.24 mmol) in MeCN (9.0 mL) was cooled to 0 °C and 1-methylimidazole (765 mg, 0.74 mL, 9.32 mmol) was added followed by *N,N,N',N'-*Tetramethylchloroformamidinium Hexafluorophosphate (732 mg, 2.61 mmol). The mixture was warmed to RT and stirred for 30 minutes. The crude mixture was concentrated *in vacuo,* then partitioned between water and EtOAc. The organic layer was isolated and washed with an additional portion of water and then brine. The isolated organic layer was dried over sodium sulfate, isolated by vacuum filtration, concentrated *in vacuo,* and purified by silica gel column chromatography (eluent: EtOAc/hexanes) to provide ethyl (S)-4-(2-(4-bromo-2-fluorophenyl)acetamido)-3-fluoro-5-((oxetan-2-ylmethyl)amino)benzoate. ES/MS: 483.0, 485.0 [M+H]⁺.

**Ethyl (S)-2-(4-bromo-2-fluorobenzyl)-4-fluoro-1-(oxetan-2-ylmethyl)-1H-benzo[d]imidazole-6-carboxylate (I-13):** To a solution of ethyl (S)-4-(2-(4-bromo-2-fluorophenyl)acetamido)-3-fluoro-5-((oxetan-2-ylmethyl)amino)benzoate (530 mg, 1.10 mmol) in DCE (12.0 mL) was added acetic acid (1.88 mL, 32.9 mmol). The mixture was heated to 60 °C for 12 hours. The mixture was concentrated and partitioned between EtOAc and saturated aqueous sodium bicarbonate. The organic layer was isolated and dried over sodium sulfate, isolated by vacuum filtration, concentrated *in vacuo,* and purified by silica gel column chromatography (eluent: EtOAc/hexanes) to provide ethyl (S)-2-(4-bromo-2-fluorobenzyl)-4-fluoro-1-(oxetan-2-ylmethyl)-1H-benzo[d]imidazole-6-carboxylate **(I-13).** ES/MS: 465.0, 467.0 [M+H]⁺.

### Preparation of Intermediate I-14

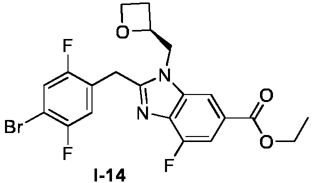

**Ethyl (S)-2-(4-bromo-2,5-difluorobenzyl)-4-fluoro-1-(oxetan-2-ylmethyl)-1H-benzo[d]imidazole-6-carboxylate (I-14):** Ethyl (S)-2-(4-bromo-2,5-difluorobenzyl)-4-fluoro-1-(oxetan-2-ylmethyl)-1H-benzo[d]imidazole-6-carboxylate was prepared in a manner as described for Intermediate **I-13** substituting 2-(4-bromo-2,5-difluorophenyl)acetic acid for 2-(4-bromo-2-fluorophenyl)acetic acid. ES/MS: 483.0, 485.0 (M+H⁺).

### Preparation of Intermediate I-15

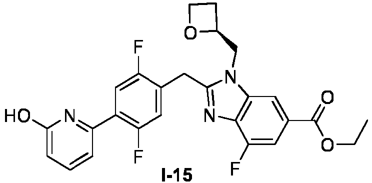

**Ethyl (S)-2-(2,5-difluoro-4-(6-hydroxypyridin-2-yl)benzyl)-4-fluoro-1-(oxetan-2-ylmethyl)-1H-benzo[d]imidazole-6-carboxylate (I-15):** Ethyl (S)-2-(2,5-difluoro-4-(6-hydroxypyridin-2-yl)benzyl)-4-fluoro-1-(oxetan-2-ylmethyl)-1H-benzo[d]imidazole-6-carboxylate was prepared in a manner as described for Intermediate **I-9** substituting **I-14** for **I-8.** ES/MS: 498.2 (M+H⁺).

### Preparation of Intermediate I-16

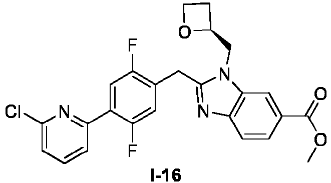

**Methyl (S)-2-(4-(6-chloropyridin-2-yl)-2,5-difluorobenzyl)-1-(oxetan-2-ylmethyl)-1H-benzo[d]imidazole-6-carboxylate (I-16):** Methyl (S)-2-(4-(6-chloropyridin-2-yl)-2,5-difluorobenzyl)-1-(oxetan-2-ylmethyl)-1H-benzo[d]imidazole-6-carboxylate was prepared in a manner a described for Intermediate **I-12** substituting **I-4** for **I-6.** ES/MS: 484.0 (M+H⁺).

### Preparation of Intermediate 17

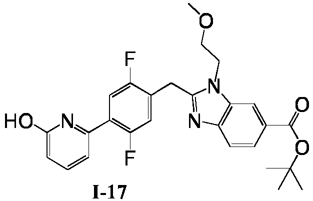

**Tert-butyl 2-(2,5-difluoro-4-(6-hydroxypyridin-2-yl)benzyl)-1-(2-methoxyethyl)-1H-benzo[d]imidazole-6-carboxylate (I-17):** Tert-butyl 2-(2,5-difluoro-4-(6-hydroxypyridin-2-yl)benzyl)-1-(2-methoxyethyl)-1H-benzo[d]imidazole-6-carboxylate was prepared in a manner as described for Intermediate **I-9** substituting tert-butyl 2-(4-bromo-2,5-difluorobenzyl)-1-(2-methoxyethyl)-1H-benzo[d]imidazole-6-carboxylate (prepared in a manner as described for intermediate **I-2** substituting 2-(4-bromo-2,5-difluorophenyl)acetic acid for 2-(4-bromo-2-fluorophenyl)acetic acid) and **I-6** for **I-1.** ES/MS: 496.9 (M+H⁺).

### Preparation of Intermediate I-18

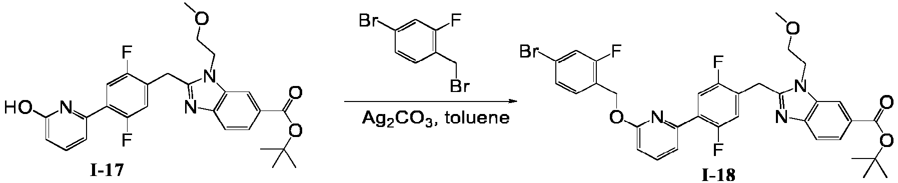

**Tert-butyl 2-(4-(6-((4-bromo-2-fluorobenzyl)oxy)pyridin-2-yl)-2,5-difluorobenzyl)-1-(2-methoxyethyl)-1H-benzo[d]imidazole-6-carboxylate (I-18):** To a solution of *tert*-butyl 2-[[2,5-difluoro-4-(6-hydroxy-2-pyridyl)phenyl]methyl]-3-(2-methoxyethyl)benzimidazole-5-carboxylate **(I-17)** (500 mg, 1.0 mmol) in toluene (5 ml), 4-bromo-1-(bromomethyl)-2-fluorobenzene (406 mg, 1.5 mmol) and Silver carbonate (835 mg, 3 mmol) were added. The solution was stirred at 70 °C for 8 hrs., cooled and filtered. The solution was concentrated and purified by silica gel column chromatography (eluent: EtOAc/hexanes) to provide *tert*-butyl 2-(4-(6-((4-bromo-2-fluorobenzyl)oxy)pyridin-2-yl)-2, 5-difluorobenzyl)-1-(2-methoxyethyl)-1H-benzo[d]imidazole-6-carboxylate **(I-18):.** ES/MS: 683.2, 684.1 (M+H⁺).

### Preparation of Intermediate I-19

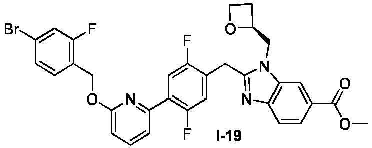

**Methyl (S)-2-(4-(6-((4-bromo-2-fluorobenzyl)oxy)pyridin-2-yl)-2,5-difluorobenzyl)-1-(oxetan-2-ylmethyl)-1H-benzo[d]imidazole-6-carboxylate (I-19):** Methyl (S)-2-(4-(6-((4-bromo-2-fluorobenzyl)oxy)pyridin-2-yl)-2,5-difluorobenzyl)-1-(oxetan-2-ylmethyl)-1H-benzo[d]imidazole-6-carboxylate was prepared in a manner as described for Intermediate **I-18** substituting **I-9** for **I-17.** ES/MS: 652.3 (M+H⁺).

### Preparation of Intermediate I-20

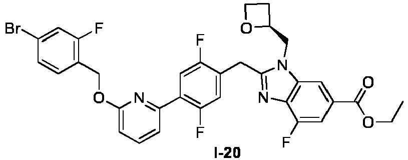

**Ethyl (S)-2-(4-(6-((4-bromo-2-fluorobenzyl)oxy)pyridin-2-yl)-2,5-difluorobenzyl)-4-fluoro-1-(oxetan-2-ylmethyl)-1H-benzo[d]imidazole-6-carboxylate (I-20):** Ethyl (S)-2-(4-(6-((4-bromo-2-fluorobenzyl)oxy)pyridin-2-yl)-2, 5-difluorobenzyl)-4-fluoro-1-(oxetan-2-ylmethyl)-1H-benzo[d]imidazole-6-carboxylate was prepared in a manner as described for Intermediate **I-18** substituting **I-15** for **I-17.** ES/MS: 684.2 (M+H⁺).

### Preparation of Intermediate I-21

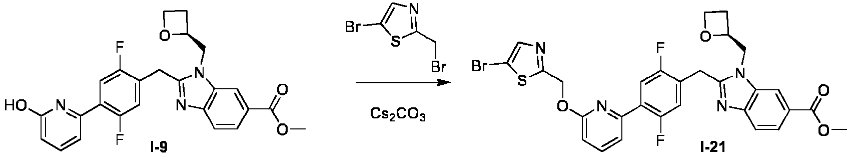

**Methyl (S)-2-(4-(6-((5-bromothiazol-2-yl)methoxy)pyridin-2-yl)-2,5-difluorobenzyl)-1-(oxetan-2-ylmethyl)-1H-benzo[d]imidazole-6-carboxylate (I-21):** To a solution of methyl (S)-2-(2,5-difluoro-4-(6-hydroxypyridin-2-yl)benzyl)-1-(oxetan-2-ylmethyl)-1H-benzo[d]imidazole-6-carboxylate **(I-9)** (500 mg, 1.07 mmol) in acetonitrile (15 mL) was added cesium carbonate (560 mg, 1.72 mmol) and 5-bromo-2-(bromomethyl)thiazole (290 mg, 1.13 mmol) and the resulting mixture stirred for 1 hr at 50 °C. Upon completion the crude reaction mixture was filtered through celite, rinsing with DCM. The filtrate was concentrated, and the crude residue purified by silica gel column chromatography (eluent: EtOAc/hexanes) to provide methyl (S)-2-(4-(6-((5-bromothiazol-2-yl)methoxy)pyridin-2-yl)-2,5-difluorobenzyl)-1-(oxetan-2-ylmethyl)-1H-benzo[d]imidazole-6-carboxylate **(I-21).** ES/MS: 643.0 (M+H⁺).

### Preparation of Intermediate I-22

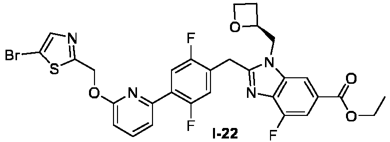

**Ethyl (S)-2-(4-(6-((5-bromothiazol-2-yl)methoxy)pyridin-2-yl)-2,5-difluorobenzyl)-4-fluoro-1-(oxetan-2-ylmethyl)-1H-benzo[d]imidazole-6-carboxylate (I-22):** Ethyl (S)-2-(4-(6-((5-bromothiazol-2-yl)methoxy)pyridin-2-yl)-2,5-difluorobenzyl)-4-fluoro-1-(oxetan-2-ylmethyl)-1H-benzo[d]imidazole-6-carboxylate was prepared in a manner as described for Intermediate **I-21** substituting **I-15** for **I-17.** ES/MS: 673.0 (M+H⁺).

### Preparation of Intermediate I-23

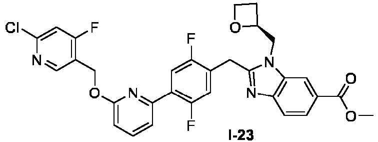

**Methyl (S)-2-(4-(6-((6-chloro-4-fluoropyridin-3-yl)methoxy)pyridin-2-yl)-2,5-difluorobenzyl)-1-(oxetan-2-ylmethyl)-1H-benzo[d]imidazole-6-carboxylate (I-23):** Methyl (S)-2-(4-(6-((6-chloro-4-fluoropyridin-3-yl)methoxy)pyridin-2-yl)-2, 5-difluorobenzyl)-1-(oxetan-2-ylmethyl)-1H-benzo[d]imidazole-6-carboxylate was prepared in a manner as described for Intermediate **I-19** substituting 5-(bromomethyl)-2-chloro-4-fluoropyridine for 4-bromo-1-(bromomethyl)-2-fluoro-benzene ES/MS: 609.2 (M+H⁺).

### Preparation of Intermediate I-24

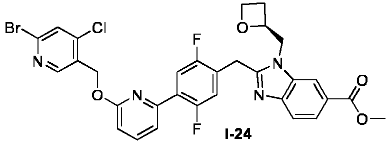

**Methyl (S)-2-(4-(6-((6-bromo-4-chloropyridin-3-yl)methoxy)pyridin-2-yl)-2,5-difluorobenzyl)-1-(oxetan-2-ylmethyl)-1H-benzo[d]imidazole-6-carboxylate (I-24):** Methyl (S)-2-(4-(6-((6-bromo-4-chloropyridin-3-yl)methoxy)pyridin-2-yl)-2, 5-difluorobenzyl)-1-(oxetan-2-ylmethyl)-1H-benzo[d]imidazole-6-carboxylate was prepared in a manner as described for Intermediate **I-19** substituting 2-bromo-5-(bromomethyl)-4-chloropyridine for 4-bromo-1-(bromomethyl)-2-fluoro-benzene ES/MS: 654.0, 656.0 (M+H⁺).

### Preparation of Intermediate I-25

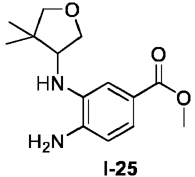

**Methyl 4-amino-3-((4,4-dimethyltetrahydrofuran-3-yl)amino)benzoate (I-25):** Methyl 4-amino-3-((4,4-dimethyltetrahydrofuran-3-yl)amino)benzoate was prepared in a manner as described for Intermediate **I-1** substituting (±)-4,4-dimethyltetrahydrofuran-3-amine for 2-methoxyethylamine, with the following modifications: to a solution of methyl 3-fluoro-4-nitro-benzoate (3.94 g, 19.8 mmol) and 4,4-dimethyltetrahydrofuran-3-amine hydrochloride (3.00 g, 19.8 mmol) in 2-methyltetrahydrofuran (40 mL) under argon was added DIPEA (17.2 mL, 98.9 mmol). The resulting solution was refluxed at 80 °C for 3 days. The mixture was concentrated in vacuo and partitioned between EtOAc and water. The aqueous phase was extracted with additional EtOAc. The combined organic phase was washed with brine, dried over MgSO₄, filtered and concentrated in vacuo. The resulting residue was redissolved in EtOH (52 ml) and tetrahydrofuran (26 mL) under argon, then 10% palladium on carbon (2.1 g, 1.98 mmol). The mixture was cycled between argon and vacuum, then placed under hydrogen atmosphere and stirred at rt for 18 hr. The mixture was filtered through Celite and concentrated in vacuo. The crude was purified by silica gel flash column chromatography (EtOAc/hexane gradient) to yield methyl 4-amino-3-((4,4-dimethyltetrahydrofuran-3-yl)amino)benzoate (Intermediate **I-25).** ES/MS: 265.2 (M+H⁺).

### Preparation of Intermediate I-26

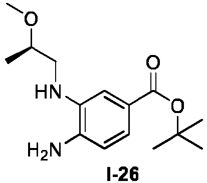

**Tert-butyl (R)-4-amino-3-((2-methoxypropyl)amino)benzoate (I-26):** Tert-butyl (R)-4-amino-3-((2-methoxypropyl)amino)benzoate was prepared in a manner as described for Intermediate **I-6** substituting (R)-2-methoxypropan-1-amine for 2-methoxyethylamine. ES/MS: 281.1 (M+H⁺).

### Preparation of Intermediate I-27

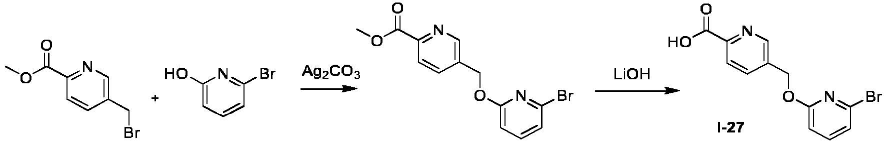

**Methyl 5-(((6-bromopyridin-2-yl)oxy)methyl)picolinate:** To a solution of 6-bromopyridin-2-ol (3.00 g, 17 mmol) in acetonitrile (100 mL) was added silver carbonate (10.2 g, 37 mmol) and methyl 5-(bromomethyl)pyridine-2-carboxylate (5.00g, 22 mmol) and the resultant mixture heated to 60 °C for 3 hours. Upon completion the reaction mixture was filtered through celite, concentrated and purified by silica gel column chromatography (eluent: EtOAc/hexanes) to provide the desired product. ES/MS: 325.2 (M+H⁺).

**5-(((6-bromopyridin-2-yl)oxy)methyl)picolinic acid (I-27):** To a solution of methyl 5-(((6-bromopyridin-2-yl)oxy)methyl)picolinate (5.57 g, 17 mmol) in acetonitrile (75mL) was added lithium hydroxide (2.17 g, 51.7 mmol) as an solution in water (25 mL) and mixture stirred at RT temperature for 1 hr. pH was adjusted to ~6 with 1N HCl, after which the reaction mixture was diluted with EtOAc (200 mL) and the layers separated. The organic layer was washed with brine, dried over MgSO₄, filtered and concentrated to give **5-(((6-bromopyridin-2-yl)oxy)methyl)picolinic acid (I-27**)without further purification. ES/MS: 309.1 (M+H⁺).

### Preparation of Intermediate I-28

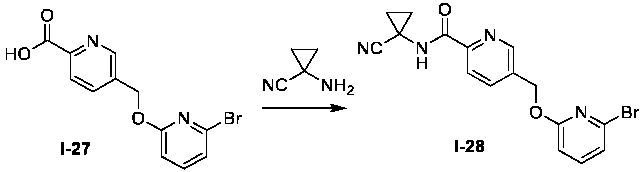

**5-(((6-bromopyridin-2-yl)oxy)methyl)-N-(1-cyanocyclopropyl)picolinamide (I-28):** To a solution of 5-(((6-bromopyridin-2-yl)oxy)methyl)picolinic acid **(I-27)** (5.32 g, 17 mmol) in DMF (80 mL), 1-aminocyclopropanecarbonitrile hydrochloride (3.14 g, 26 mmol), HATU (9.62 g, 25 mmol), and diisopropylethylamine (12 mL, 69 mmol) was added sequentially. The resultant solution was stirred at RT temperature for 30 minutes. Upon completion the mixture contents were diluted with EtOAc (250 mL, wash with water (2 x 50 mL), brine (1 x 40 mL), dried over MgSO₄, filtered and concentrated to give 5-(((6-bromopyridin-2-yl)oxy)methyl)-N-(1-cyanocyclopropyl)picolinamide **(I-28)** without further purification. ES/MS: 375.1 (M+H⁺).

### The following intermediates were synthesized in an analogous manner as described for Intermediate I-28

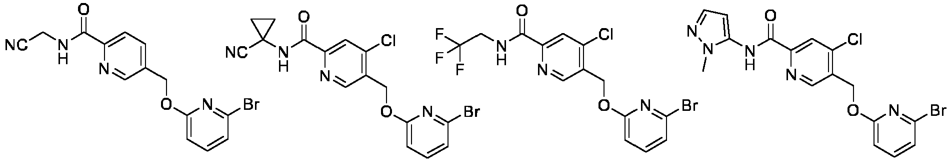

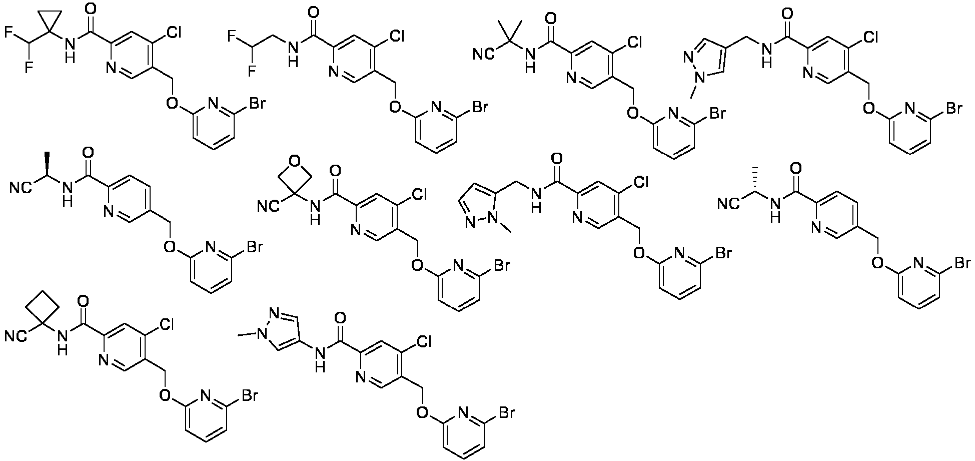

### Preparation of Intermediates I-29 and I-30

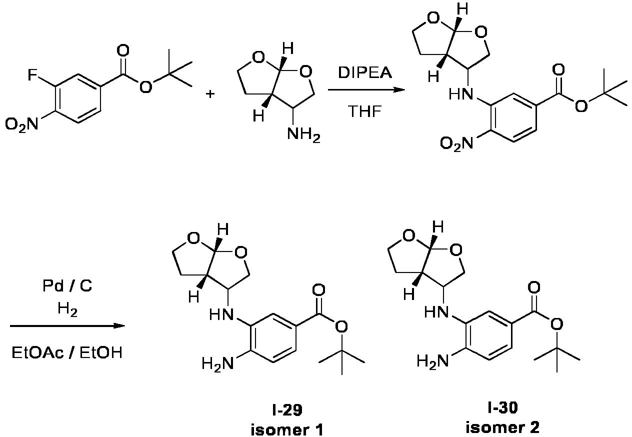

### Tert-butyl 4-amino-3-(((3aS,6aR)-hexahydrofuro[2,3-b]furan-3-yl)amino)benzoate (I-29 and I-30):

**Tert-butyl 3-(((3aS,6aR)-hexahydrofuro[2,3-b]furan-3-yl)amino)-4-nitrobenzoate:** A solution of *tert*-butyl 3-fluoro-4-nitro-benzoate (0.150 g, 0.622 mmol), (3aS,6aR)-2,3,3a,4,5,6a-hexahydrofuro[2,3-b]furan-4-amine (0.0984 g, 0.762 mmol), and N-ethyl-N-isopropyl-propan-2-amine (0.325 mL, 1.87 mmol) in NMP (4 mL) was heated at 90 C overnight. The mixture was diluted with EtOAc and washed with 5% LiCl and brine. The organic extract was dried over sodium sulfate and purified by flash chromatography (eluent: EtOAc/hexanes) to give tert-butyl 3-(((3aS,6aR)-hexahydrofuro[2,3-b]furan-3-yl)amino)-4-nitrobenzoate as an un-separable mixture of 2 compounds. ES/MS: 351.0 (M+H⁺).

***Tert*-butyl 4-amino-3-(((3aS,6aR)-hexahydrofuro[2,3-b]furan-3-yl)amino)benzoate (I-29 and I-30):** A solution of tert-butyl 3-[[(3aS,6aR)-2,3,3a,4,5,6a-hexahydrofuro[2,3-b]furan-4-yl]amino]-4-nitro-benzoate (156 mg, 0.445 mmol) in EtOH (15 mL) was degassedby cycling the mixture between argon and vacuum 3x. Pd/C (10.0 %, 47.4 mg, 0.0445 mmol) was added to the solution and then the solution was degassed 1x by cycling the mixture between argon and vacuum and stirred at RT with a balloon of hydrogen overnight. The reaction mixture was filtered over a Celite plug and rinsed with EtOAc. Concentrated and purified by flash chromatography (eluent: 30 to 40% EtOAc/hexanes) to give two distinct isomers of *tert*-butyl 4-amino-3-(((3aS,6aR)-hexahydrofuro[2,3-b]furan-3-yl)amino)benzoate **(I-29** and **I-30).**

### Isomer 1 (less polar, eluted first), I-29

ES/MS: 321.0 (M+H⁺). 1HNMR (400 MHz, Chloroform-d) δ 7.45 (dd, J = 8.1, 1.8 Hz, 1H), 7.37 (d, J = 1.8 Hz, 1H), 6.74 (d, J = 8.1 Hz, 1H), 5.87 (d, J = 5.1 Hz, 1H), 4.32 - 4.06 (m, 2H), 4.05 - 3.81 (m, 2H), 3.66 (t, J = 8.7 Hz, 1H), 3.24 (tt, J = 8.7, 4.2 Hz, 1H), 2.03 - 1.90 (m, 1H), 1.90 - 1.79 (m, 1H), 1.60 (s, 9H).

### Isomer 2 (more polar, eluted second), I-30

ES/MS: 321.0 (M+H⁺). 1HNMR (400 MHz, Chloroform-d) δ 7.45 (dd, J = 8.1, 1.8 Hz, 1H), 7.38 (d, J = 1.8 Hz, 1H), 6.75 (d, J = 8.0 Hz, 1H), 5.87 (d, J = 5.1 Hz, 1H), 4.34 - 4.05 (m, 2H), 4.02 - 3.82 (m, 2H), 3.67 (t, J = 8.6 Hz, 1H), 3.24 (tt, J = 8.6, 4.2 Hz, 1H), 2.02 - 1.81 (m, 2H), 1.60 (s, 9H).

### Preparation of Intermediate I-31

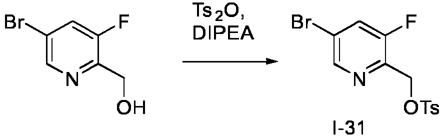

**(5-bromo-3-fluoropyridin-2-yl)methyl 4-methylbenzenesulfonate (I-31):** (5-bromo-3-fluoro-2-pyridyl)methanol (200 mg, 0.97 mmol), p-Toluenesulfonic anhydride (350 mg, 1.1 mmol), diisopropylethylamine (0.34 mL, 1.9 mmol), and DCM (10 mL were combined and stirred at ambient temperature for 16 hours. Upon completion the reaction mixture was washed with saturated aqueous NaHCO₃ (5 mL) and brine (5 mL), dried over MgSO₄, filtered and concentrated to give (5-bromo-3-fluoropyridin-2-yl)methyl 4-methylbenzenesulfonate **(I-31),** which was used without further purification.

The following intermediate was prepared in a manner as described for Intermediate 1-31:

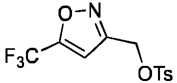

### Preparation of Intermediate I-32

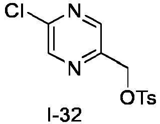

**(5-chloropyrazin-2-yl)methyl 4-methylbenzenesulfonate (I-32):** (5-chloropyrazin-2-yl)methyl 4-methylbenzenesulfonate was prepared in a manner as described for Intermediate **I-31** substituting (5-chloropyrazin-2-yl)methanol for (5-bromo-3-fluoro-2-pyridyl)methanol.

### Preparation of Intermediate I-33

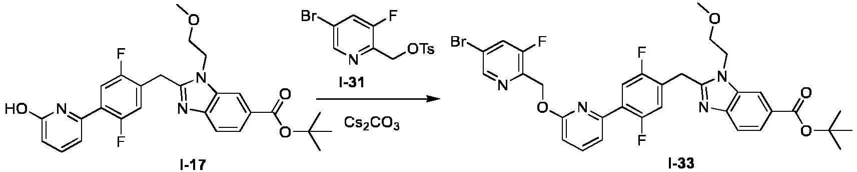

***Tert*-butyl 2-[[4-[6-[(5-bromo-3-fluoro-2-pyridyl)methoxy]-2-pyridyl]-2,5-difluorophenyl]methyl]-3-(2-methoxyethyl)benzimidazole-5-carboxylate (I-33):** To a solution of *tert-*butyl 2-[[2,5-difluoro-4-(6-hydroxy-2-pyridyl)phenyl]methyl]-3-(2-methoxyethyl)benzimidazole-5-carboxylate **(I-17)** (400 mg, 0.80 mmol) and (5-bromo-3-fluoro-2-pyridyl)methyl 4-methylbenzenesulfonate **(I-31)** (350 mg, 0.97 mmol) in 15 mL of acetonitrile was added Cs₂CO₃ (400 mg, 1.20 mmol). The solution was then heated to 50 °C for 30 minutes. The solution was cooled to RT, filtered, and then concentrated. The crude material was purified by silica gel column chromatography (eluent: EtOAc/hexanes) to provide *tert*-butyl 2-[[4-[6-[(5-bromo-3-fluoro-2-pyridyl)methoxy]-2-pyridyl]-2,5-difluoro-phenyl]methyl]-3-(2-methoxyethyl)benzimidazole-5-carboxylate **(I-33):** product. ES/MS: 686.0 (M+H⁺).

### Preparation of Intermediate I-34

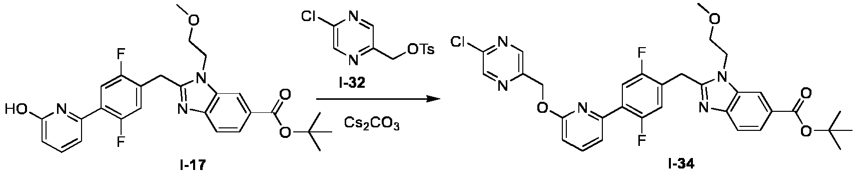

***Tert*-butyl 2-[[4-[6-[(5-chloropyrazin-2-yl)methoxy]-2-pyridyl]-2,5-difluorophenyl]methyl]-3-(2-methoxyethyl)benzimidazole-5-carboxylate(I-34):** *tert*-butyl 2-[[4-[6-[(5-chloropyrazin-2-yl)methoxy]-2-pyridyl]-2, 5-difluoro-phenyl]methyl]-3-(2-methoxyethyl)benzimidazole-5-carboxylate was prepared in a manner as described for Intermediate **I-33** substituting **I-31** for **I-32.** ES/MS: 566.0 (M+H⁺).

### Preparation of Intermediate I-35

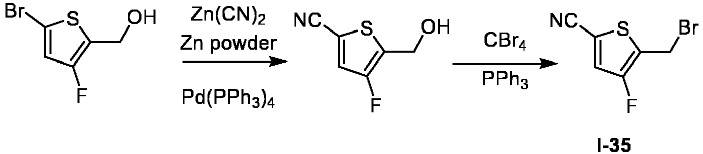

**4-fluoro-5-(hydroxymethyl)thiophene-2-carbonitrile:** (5-bromo-3-fluoro-2-thienyl)methanol (220 mg, 1.04 mmol), zinc cyanide (182 mg, 1.55 mmol), zinc powder (3 mg, 0.05 mmol), and Pd(PPh₃)₄ (300 mg, 0.26 mmol) in DMF (10 mL) was degassed by bubbling argon for 1 minute, sealed and heated to 100 °C for 20 hours. Upon completion, the mixture contents were poured into H₂O (10 mL) and extracted with EtOAc (2 × 20 mL). The organic layers were combined, washed with brine (5 mL), dried over MgSO₄, filtered, concentrated, and purified by flash chromatography (Eluent: EtOAc/hexane) to give 4-fluoro-5-(hydroxymethyl)thiophene-2-carbonitrile. ES/MS: 158.2 (M+1).

**5-(bromomethyl)-4-fluoro-thiophene-2-carbonitrile (I-35):** Carbon tetrabromide (111 mg, 0.34 mmol was added to a solution comprising 4-fluoro-5-(hydroxymethyl)thiophene-2-carbonitrile (48 mg, 0.31 mmol), triphenylphosphine (88 mg, 0.34 mmol) in DCM (3 mL) at RT. The mixture was stirred for 30 min at RT. Upon completion the mixture was concentrated and purified by flash chromatography (Eluent: EtOAc/hexane) to give 5-(bromomethyl)-4-fluoro-thiophene-2-carbonitrile **(I-35).** ES/MS: 221.2 (M+1).

### Preparation of Intermediate I-36

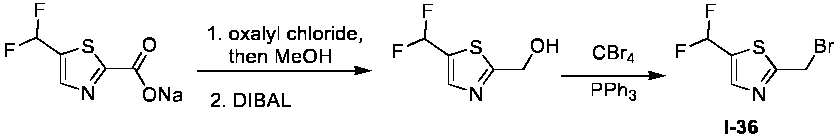

**[5-(difluoromethyl)thiazol-2-yl]methanol:** To a solution of [5-(difluoromethyl)thiazole-2-carbonyl]oxysodium (200 mg, 1.08 mmol) in DCM (5 mL), at RT, was added oxalyl chloride (2.0 M in DCM, 0.65 mL, 1.3 mmol). After stirring for 1 hour at RT, MeOH (1 mL) was added and the mixture was stirred for additional 30 minutes before pouring into H₂O (10 mL) and extracted with EtOAc (2 × 20 mL). The organic layers were combined, washed with brine (5 mL), dried over MgSO₄, filtered, and concentrated. The residue was re-dissolved in THF (5 mL) and cooled to 0 °C. Diisobutylaluminium hydride (1.0 M in DCM, 3.3 mL, 3.3 mmol) was added, and the mixture was warmed to RT and stirred for 1 hour. Upon completion, the reaction was quenched with 2M NaOH (0.4 mL), H₂O (0.4 mL), and diluted with EtOAc (10 mL). The mixture was then filtered through a plug of Celite. The organic layers were combined, washed with brine (5 mL), dried over MgSO₄, filtered, concentrated, and purified by flash chromatography (Eluent: EtOAc/hexane) to give the desired product. ES/MS: 166.2 (M+1).

**2-(bromomethyl)-5-(difluoromethyl)thiazole (I-36):** [5-(difluoromethyl)thiazol-2-yl]methanol (88 mg, 0.53 mmol), triphenylphosphine (150 mg, 0.56 mmol) in DCM (3 mL) was added carbon tetrabromide (190 mg, 0.56 mmol) at rt. The mixture was stirred at rt for 30 min. Upon completion the mixture was concentrated and purified by flash chromatography (Eluent: EtOAc/hexane) to give **I-36**. ES/MS: 229.2 (M+1).

### Preparation of Intermediate I-37

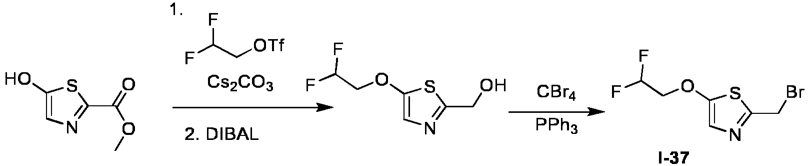

**[5-(2,2-difluoroethoxy)thiazol-2-yl]methanol:** A suspension of methyl 5-hydroxythiazole-2-carboxylate (200 mg, 1.3 mmol), 2,2-difluoroethyl trifluoromethanesulfonate (300 mg, 1.4 mmol), and cesium carbonate (610 mg, 1.9 mmol) in MeCN (5 mL) was stirred at RT for 16 h. Upon completion, the mixture was filtered through a plug of Celite and concentrated. The residue was re-dissolved in THF (5 mL) and cooled to 0 °C. Diisobutylaluminium hydride (1.0 M in DCM, 2.8 mL, 2.8 mmol) was added, and the mixture was warmed to RT and stirred for 1 hour. Upon completion, the reaction was quenched with 2 M NaOH (0.4 mL), H₂O (0.4 mL) and diluted with EtOAc (10 mL). The mixture was then filtered through a plug of Celite. The organic layers were combined, washed with brine (5 mL), dried over MgSO₄, filtered, concentrated, and purified by flash chromatography (Eluent: EtOAc/hexane) to give the desired product.

**2-(bromomethyl)-5-(2,2-difluoroethoxy)thiazole (I-37):** [5-(2,2-difluoroethoxy)thiazol-2-yl]methanol (91 mg, 0.47 mmol), triphenylphosphine (125 mg, 0.48 mmol) in DCM (5 mL) was added carbon tetrabromide (160 mg, 0.48 mmol) at RT. The mixture was stirred for 30 min at RT. Upon completion the mixture was concentrated and purified by flash chromatography (Eluent: EtOAc/hexane) to give **I-37**. ES/MS: 258.2 (M+1).

### Preparation of Intermediate I-38

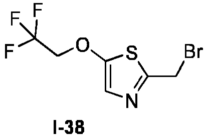

**2-(bromomethyl)-5-(2,2,2-trifluoroethoxy)thiazole (I-38):** 2-(bromomethyl)-5-(2,2,2-trifluoroethoxy)thiazole was prepared in a manner as described for Intermediate **I-37** substituting 2,2-difluoroethyl trifluoromethanesulfonate for 2,2,2-trifluoroethyl trifluoromethanesulfonate. ES/MS: 277.2 (M+1).

### Preparation of Intermediate I-39

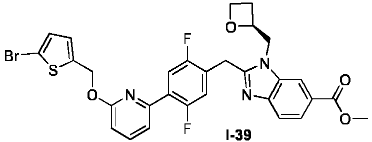

**Methyl (S)-2-(4-(6-((5-bromothiophen-2-yl)methoxy)pyridin-2-yl)-2,5-difluorobenzyl)-1-(oxetan-2-ylmethyl)-1H-benzo[d]imidazole-6-carboxylate (I-39):** Methyl (S)-2-(4-(6-((5-bromothiophen-2-yl)methoxy)pyridin-2-yl)-2, 5-difluorobenzyl)-1-(oxetan-2-ylmethyl)-1H-benzo[d]imidazole-6-carboxylate was prepared in a manner as described for Intermediate **I-19** substituting 5-(bromomethyl)-2-chloro-4-fluoropyridine for 2-bromo-5-(bromomethyl)thiophene ES/MS: 642.0 (M+H⁺).

### Preparation of Intermediate I-40

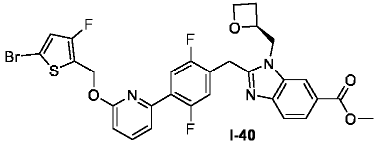

**Methyl (S)-2-(4-(6-((5-bromo-3-fluorothiophen-2-yl)methoxy)pyridin-2-yl)-2,5-difluorobenzyl)-1-(oxetan-2-ylmethyl)-1H-benzo[d]imidazole-6-carboxylate (I-40):** Methyl (S)-2-(4-(6-((5-bromo-3-fluorothiophen-2-yl)methoxy)pyridin-2-yl)-2, 5-difluorobenzyl)-1-(oxetan-2-ylmethyl)-1H-benzo[d]imidazole-6-carboxylate was prepared in a manner as described for Intermediate **I-19** substituting 5-(bromomethyl)-2-chloro-4-fluoropyridine for 5-bromo-2-(bromomethyl)-3-fluorothiophene ES/MS: 660.0 (M+H⁺).

### Preparation of Intermediate I-41

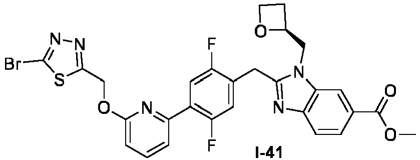

**Methyl 2-[[4-[6-[(5-bromo-1,3,4-thiadiazol-2-yl)methoxy] -2-pyridyl] -2,5-difluorophenyl]methyl]-3-[[(2S)-oxetan-2-yl]methyl]benzimidazole-5-carboxylate (I-41):** methyl 2-[[4-[6-[(5-bromo-1,3,4-thiadiazol-2-yl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-19** substituting 5-(bromomethyl)-2-chloro-4-fluoropyridine for 2-bromo-5-(bromomethyl)-1,3,4-thiadiazole ES/MS: 660.0 (M+H⁺).

### Preparation of Intermediate I-42

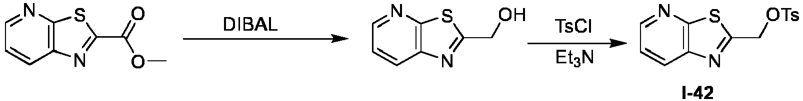

**Thiazolo[5,4-b]pyridin-2-ylmethanol:** methyl thiazolo[5,4-b]pyridine-2-carboxylate (100 mg, 0.52 mmol) in THF (5 mL) was cooled to 0 °C. Diisobutylaluminium hydride (1.0 M in DCM, 1.5 mL, 1.5 mmol) was added, and the mixture was warmed to rt and stirred for 1 hour. Upon completion, the reaction was quenched with 2 M NaOH (0.4 mL), H₂O (0.4 mL), and diluted with EtOAc (10 mL). The mixture was then filtered through a plug of Celite. The organic layers were combined, washed with brine (5 mL), dried over MgSO₄, filtered, concentrated, and purified by flash chromatography (Eluent: EtOAc/hexane) to give the titled product. ES/MS: 167.2 (M+1).

**Thiazolo[5,4-b]pyridin-2-ylmethyl 4-methylbenzenesulfonate (I-42):** To a solution of thiazolo[5,4-b]pyridin-2-ylmethanol (40 mg, 0.24 mmol), triethylamine (0.07 mL, 0.5 mmol) in DCM (5 mL) was added toluene-4-sulfonyl chloride (46 mg, 0.24 mmol) at rt. The mixture was stirred at rt for 20 hours. Upon completion the mixture was concentrated and purified by flash chromatography (Eluent: EtOAc/hexane) to give the title product. ES/MS: 321.2 (M+1).

### Preparation of Intermediate I-43

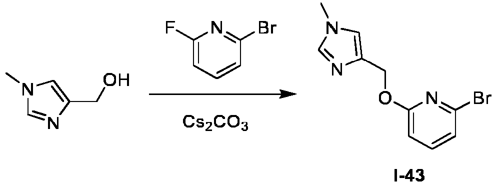

**2-bromo-6-[(1-methylimidazol-4-yl)methoxy]pyridine (I-43):** To a solution of 2-bromo-6-fluoro-pyridine (90.7 mg, 0.52 mmol) and (1-methylimidazol-4-yl)methanol (75.1 mg, 0.67 mmol) in 2.0 mL of acetonitrile was added Cs₂CO₃ (338 mg, 1.04 mmol). The solution was then heated to 50 °C for 30 minutes. The solution was cooled to rt, filtered, then concentrated. The crude material was purified by normal phase chromatography 1-12 % DCM/MeOH. The product containing fractions were combined and concentrated to give the title product. ES/MS m/z: 268.0, 270.2 (M+H⁺).
The following intermediates were synthesized in a manner as described for intermediate 1-43:

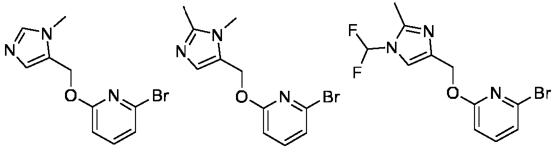

### Preparation of Intermediate I-44

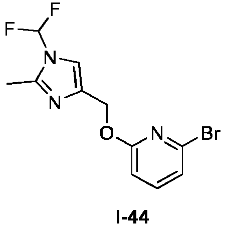

**2-bromo-6-[[1-(difluoromethyl)-2-methyl-imidazol-4-yl]methoxy]pyridine (I**-**44):** This intermediate was prepared in a manner as described for Intermediate **I-43** substituting [1-(difluoromethyl)-2-methyl-imidazol-4-yl]methanol for (1-methylimidazol-4-yl)methanol. ES/MS m/z: 318.2 (M+H⁺).

### Preparation of Intermediate I-45

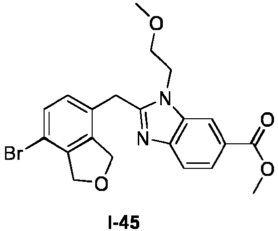

**Methyl 2-[(7-bromo-1,3-dihydroisobenzofuran-4-yl)methyl]-3-(2-methoxyethyl)benzimidazole-5-carboxylate (I-45):** This intermediate was prepared in a manner as described for Intermediate **I-13** substituting 2-(7-bromo-1,3-dihydroisobenzofuran-4-yl)acetic acid for (2-(4-bromo-2-fluorophenyl)acetic acid. ES/MS m/z: 447.1 (M+H⁺).

### Preparation of Intermediate I-46

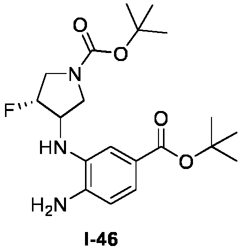

**Tert-butyl (3R,4R)-3-(2-amino-5-methoxycarbonyl-anilino)-4-fluoro-pyrrolidine-1-carboxylate (I-46):** This intermediate was prepared in a manner as described for Intermediate **I-6** substituting tert-butyl (3R,4R)-3-amino-4-fluoro-pyrrolidine-1-carboxylate for 2-methoxyethanamine. ES/MS m/z: 418.4 (M+Na⁺).

### Preparation of Intermediate I-47

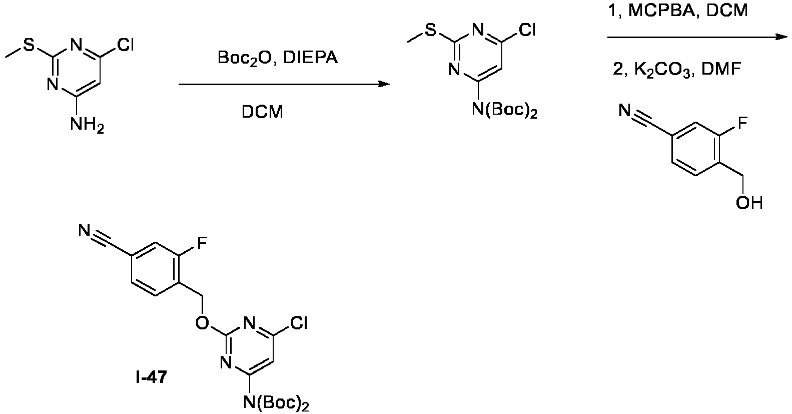

**Tert-butyl N-tert-butoxycarbonyl-N-(6-chloro-2-methylsulfanyl-pyrimidin-4-yl)carbamate:** A solution of methylsulfanyl-pyrimidin-4-amine (1 g, 5.7 mmol), *tert-*butoxycarbonyl tert-butyl carbonate (2.61 g, 12 mmol), ethyldiisopropylamine (3 mL, 17.1 mmol) and 4-dimethylaminopyridine (140 mg, 1.14 mmol) in 20 mL of DCM, was stirred overnight. The mixture was washed with H₂O and brine. The combined organic extracts were dried over sodium sulfate, filtered and the filtrate was concentrated in vacuo. The crude residue was purified by column chromatography (0-50% EtOAc in hexane) to give the title compound: ES/MS m/z: 375.2 (M+H+).

**Tert-butyl N-tert-butoxycarbonyl-N-[6-chloro-2-[(4-cyano-2-fluorophenyl)methoxy]pyrimidin-4-yl]carbamate (I-47):** A solution of *tert*-butyl *N-tert-*butoxycarbonyl-N-(6-chloro-2-methylsulfanyl-pyrimidin-4-yl)carbamate (0.5 g, 1.33 mmol) and 3-chloroperoxybenzoic acid (0.6 g, 2.7 mmol, 77%) in 5 mL of DCM, was stirred for overnight. The mixture was washed with H₂O and brine. The solvent was removed, and the resulting residue was dried in vacuo. The crude product was dissolved in 3 mL of DMF; to this solution 3-fluoro-4-(hydroxymethyl)benzonitrile (220 mg, 1.46 mmol) and potassium carbonate (366 mg, 2.65 mmol) was added and let sit at rt for 2 hr. After the 2 hours, the resulting solution was diluted with EtOAc (50 mL) and washed with H₂O. The combined organic extracts were dried over sodium sulfate, filtered and the filtrate was concentrated in vacuo. The crude residue was purified by column chromatography (0-50% EtOAc in hexane) to give the title compound: ES/MS m/z: 479.1(M+H+).

### Preparation of Intermediate I-48

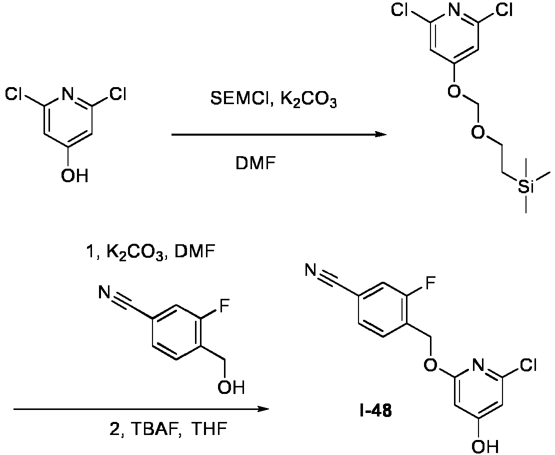

**2-[(2,6-dichloro-4-pyridyl)oxymethoxy]ethyl-trimethyl-silane:** A solution of 2,6-dichloropyridin-4-ol (1 g, 6.1 mmol) and 2-(chloromethoxy)ethyl-trimethyl-silane (1.07 g, 6.4 mmol) in 10 mL of THF, lithium bis(trimethylsilyl)amide (6.4 mL, 6,4 mmol) was stirred overnight. The solution was washed with H₂O and brine. The combined organic extracts were dried over sodium sulfate, filtered and the filtrate was concentrated in vacuo. The crude residue was purified by column chromatography (0-50% EtOAc in hexane) to give the title compound: ES/MS m/z: 294.1 (M+H+).

**4-[(6-chloro-4-hydroxy-2-pyridyl)oxymethyl]-3-fluoro-benzonitrile (I-48):** To a solution of 2-[(2,6-dichloro-4-pyridyl)oxymethoxy]ethyl-trimethyl-silane (1.0 g, 3.4 mmol) was dissolved in 5 mL of DMF, 3-fluoro-4-(hydroxymethyl)benzonitrile (0.77 g, 5.1 mmol) and potassium carbonate (0.94 g, 6.8 mmol) was added. The solution was heated to 120 °C for 6 hours. Upon completion, the solution was diluted with EtOAc (50 mL) and washed with H₂O. The combined organic extracts were dried over sodium sulfate, filtered and the filtrate was concentrated in vacuo. The crude product was dissolved in 2 mL of THF. To the resulting solution tetrabutylammonium fluoride (4.0 mL, 4 mmol) was added. The mixture was stirred for 3 hr. The solvent was removed, and the resulting residue was purified by column chromatography (0-80% EtOAc in hexane) to give the title compound: ES/MS m/z: 279.2 (M+H+).

### Preparation of Intermediate I-49

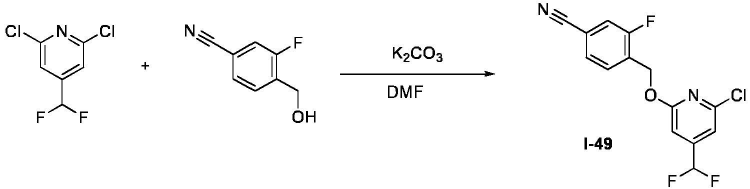

**4-[[6-chloro-4-(difluoromethyl)-2-pyridyl]oxymethyl]-3-fluoro-benzonitrile (I-49):** To a solution of 2,6-dichloro-4-(difluoromethyl)pyridine (927 mg, 4.68 mmol) was dissolved in 10 mL of DMF, 3-fluoro-4-(hydroxymethyl)benzonitrile (708 mg, 4.68 mmol) and potassium carbonate (971 mg, 7 mmol) was added to the solution. It was heated to 60 °C overnight and diluted with EtOAc (50 mL) and washed with water. The combined organic extracts were dried over sodium sulfate, filtered and the filtrate was concentrated in vacuo. The solvent was removed, and the resulting residue was purified by column chromatography (0-60% EtOAc in hexane) to give the title compound: ES/MS m/z: 313.1 (M+H+).

### Preparation of Intermediate I-50

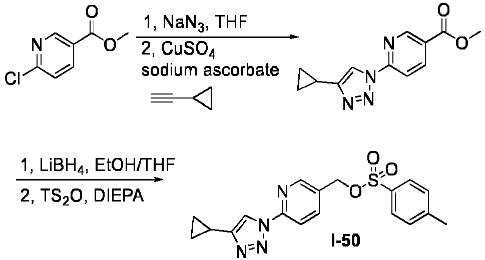

**Methyl 6-(4-cyclopropyltriazol-1-yl)pyridine-3-carboxylate:** A suspension of methyl 6-chloropyridine-3-carboxylate (300 mg, 1.75 mmol) and sodium azide (227 mg, 3.5 mmol) in THF, was heated to 60 °C for 5 hours. Upon completion, the mixture was diluted with EtOAc and washed with saturated solution of sodium bicarbonate (20 mL) and brine. The combined organic extracts were dried over sodium sulfate, filtered and the filtrate was concentrated in vacuo. To the crude residue, ethynylcyclopropane (145 mg, 2.2 mmol) in tert-butanol (5 mL) was added. To the resulting mixtures, solutions of sodium ascorbate (0.19 mmol, 38 mg) in water (2.5 mL) and copper sulfate pentahydrate (0.19 mmol, 48 mg) in H₂O (2.5 mL) were sequentially added. The reaction mixture was stirred at rt conditions for 18 hr. Upon completion the mixture was diluted with 5 mL 1M aq. NH₄OH and extracted with EtOAc (2x30 mL). The combined organic extracts were washed with brine, dried over Na₂SO₄ and concentrated under reduced pressure. Crude product was purified by flash chromatography on silica gel (0-100% EtOAc in hexane) to give the title compound: ES/MS m/z: 245.2 (M+H+).

**[6-(4-cyclopropyltriazol-1-yl)-3-pyridyl]methyl 4-methylbenzenesulfonate (I-50):** To a solution of methyl 6-(4-cyclopropyltriazol-1-yl)pyridine-3-carboxylate (300 mg, 1.23 mmol) in 2 mL of THF, 0.92 mL of 2 N of lithium borohydride in THF was added. The resulting solution was stirred for 8 hours and then diluted with 50 mL of EtOAc and washed with H₂O and brine. The combined organic extracts were washed with brine, dried over Na₂SO₄ and concentrated under reduced pressure. The resulting crude product was dissolved in 5 mL of DCM. Next, p-tolylsulfonyl 4-methylbenzenesulfonate (365 mg, 1.12 mmol) and ethyldiisopropylamine (0.37 mL, 2.13 mmol) was added to the solution. The mixture was stirred for overnight. Upon completion the resulting solution was diluted with 20 mL of DCM and washed with H₂O and brine. The organic layer was dried over Na₂SO₄ and concentrated under reduced pressure. The crude product was purified by flash chromatography on silica gel (0-100% EtOAc in hexane) to give the title compound: ES/MS m/z: 371.2 (M+H+).

### Preparation of Intermediate I-51

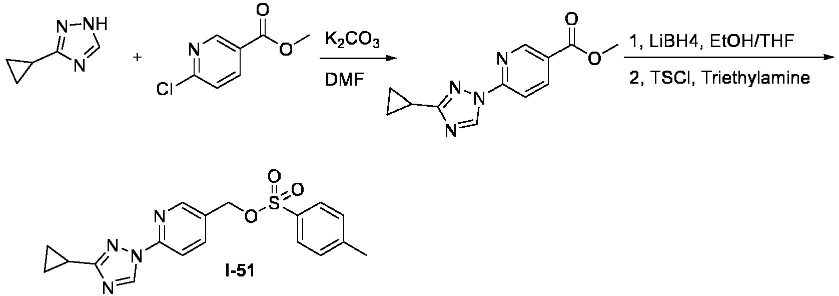

**Methyl 6-(3-cyclopropyl-1,2,4-triazol-1-yl)pyridine-3-carboxylate:** A suspension of methyl 6-chloropyridine-3-carboxylate (300 mg, 1.75 mmol), 3-cyclopropyl-1H-1,2,4-triazole (191 mg, 1.75 mmol) and potassium carbonate (483 mg, 3.5 mmol) in THF, was heated to reflux for 8 hours. Following this time, the solution was diluted with EtOAc and washed with saturated solution of sodium bicarbonate (20 mL) and brine. The combined organic extracts were dried over sodium sulfate, filtered and the filtrate was concentrated in vacuo. The crude residue was purified by flash chromatography on silica gel (0-100% EtOAc in hexane) to give the title compound: ES/MS m/z: 245.2 (M+H+).

**[6-(3-cyclopropyl-1,2,4-triazol-1-yl)-3-pyridyl]methyl 4-methylbenzenesulfonate (I-51):** To a solution of methyl 6-(3-cyclopropyl-1,2,4-triazol-1-yl)pyridine-3-carboxylate (300 mg, 1.23 mmol) in 2 mL of THF, 0.92 mL of 2 N of lithium borohydride in THF was added. The solution was stirred overnight and diluted with 50 mL of EtOAc and washed with water and brine. The combined organic extracts were washed with brine, dried over Na₂SO₄ and concentrated under reduced pressure. The crude product was dissolved in 5 mL of DCM, 4-methylbenzenesulfonyl chloride (241 mg, 1.26 mmol) and triethylamine (0.34 mL, 2.4 mmol) was added to the solution. The mixture was stirred for overnight and diluted with 20 mL of DCM and then washed with water and brine. The organic layer was dried over sodium sulfate, filtered and the filtrate was concentrated in vacuo. The crude residue was purified by flash chromatography on silica gel (0-100% EtOAc in hexane) to give **I-51:** ES/MS m/z: 371.1 (M+H+).

### Preparation of Intermediate I-52

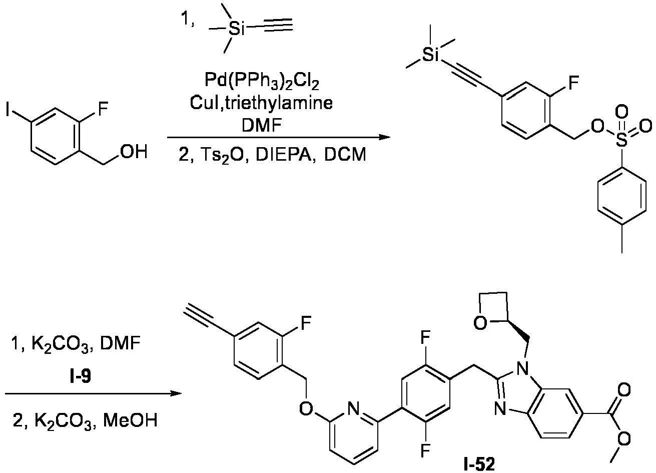

**[2-fluoro-4-(2-trimethylsilylethynyl)phenyl]methyl 4-methylbenzenesulfonate:** (2-fluoro-4-iodo-phenyl)methanol (2 g, 7.94 mmol), ethynyl(trimethyl)silane (1.17 g, 11.9 mmol), copper iodide (75.6 mg, 0.4 mmol), bis(triphenylphosphine)palladium chloride (280 mg, 0.4 mmole) and triethylamine (3.3 mL, 23.8 mmol) was suspended in THF (15 mL). The mixture was degassed with nitrogen and stirred for 16 hours at ambient temperature. Following this, the mixture was filtered with celite and washed with 20 mL of DCM three times. The solvent was removed, and the resulting crude product was dried in vacuo. Next, the crude product was dissolved in 20 mL of DCM, followed by the addition of p-tolylsulfonyl 4-methylbenzenesulfonate (2.58 g, 7.92 mmol) and ethyldiisopropylamine (2.76 mL, 15.8 mmol) to the solution. The mixture was stirred for 5 hours and checked via LC/MS. Then the mixture was diluted with 20 mL of DCM and washed with water and brine. The organic layer was dried over sodium sulfate, filtered and the filtrate was concentrated in vacuo. The crude residue was purified by flash chromatography on silica gel (0-50% EtOAc in hexane) to give the title compound: ES/MS m/z: 377.1 (M+H+).

**Methyl 2-[[4-[6-[(4-ethynyl-2-fluoro-phenyl)methoxy] -2-pyridyl] -2,5-difluorophenyl]methyl]-3-[[(2S)-oxetan-2-yl]methyl]benzimidazole-5-carboxylate (I-52):** Potassium carbonate (742 mg, 5.37 mmol) was added to a solution of methyl 2-[[2,5-difluoro-4-(6-hydroxy-2-pyridyl)phenyl]methyl]-3-[[(2S)-oxetan-2-yl]methyl]benzimidazole-5-carboxylate (500 mg, 1.07 mmol) and [2-fluoro-4-(2-trimethylsilylethynyl)phenyl]methyl 4-methylbenzenesulfonate (420 mg, 1.12 mmol) in 5 mL of DMF, and stirred for 3 hours. Following this time, 50 mL of water was added to the solution and the aqueous phase was extracted 2X EtOAc. The combined organic phase was dried over sodium sulfate, filtered and the filtrate was concentrated in vacuo. The crude product was dissolved in MeOH (20 mL) and potassium carbonate (51.6 mg, 0.37 mmol) was added to the solution. After 2 hours, the solution was filtered, and the filtrate was concentrated in vacuo. The crude residue was purified by flash chromatography on silica gel (0-80% EtOAc in hexane) to give I-52. ES/MS m/z: 598.2 (M+H+).

### Preparation of Intermediate I-53

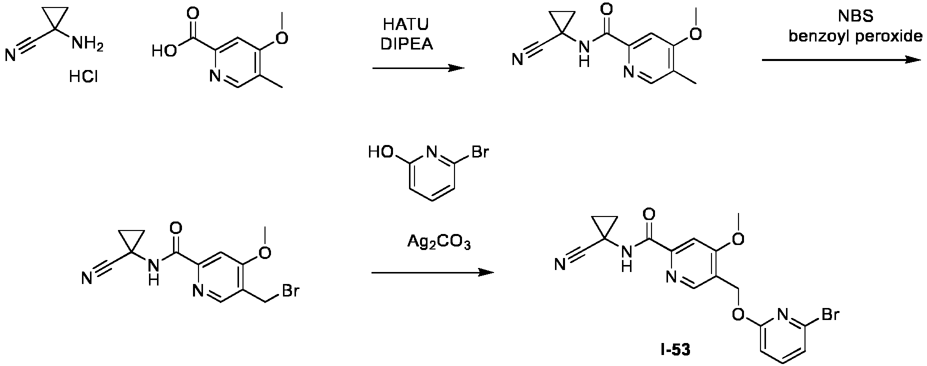

**N-(1-cyanocyclopropyl)-4-methoxy-5-methyl-pyridine-2-carboxamide:** N,N-Diisopropylethylamine (2.14 mL, 12.3 mmol) was added to a solution of 4-methoxy-5-methylpyridine-2-carboxylic acid;hydrochloride (500 mg, 2.46 mmol), 1-aminocyclopropanecarbonitrile;hydrochloride (349 mg, 2.95 mmol), and O-(7-Azabenzotriazol-1-yl)-N,N,N',N'-tetramethyluronium hexafluorophosphate (1373 mg, 3.61 mmol) in DMF (10 mL). The mixture was stirred at rt overnight. Following this time, the mixture was diluted with EtOAc and washed with 5%LiCl, saturated NaHCO₃, and brine. The organic extract was dried over sodium sulfate and purified by flash chromatography (eluent: EtOAc/hexanes) to give the title compound.

**5-(bromomethyl)-N-(1-cyanocyclopropyl)-4-methoxy-pyridine-2-carboxamide:** To a suspension of N-(1-cyanocyclopropyl)-4-methoxy-5-methyl-pyridine-2-carboxamide (400 mg, 1.73 mmol) in CCl4 (10 mL), was added N-Bromosuccinimide (403 mg, 2.26 mmol) followed by benzoyl peroxide (45.1 mg, 0.186 mmol). The resulting solution was heated at 90 °C for 1 hr. Upon completion the mixture was cooled to rt, diluted with 5 mL hexanes and the suspension was filtered. The filtrate was concentrated and purified by flash chromatography (eluent: EtOAc/hexanes) to give the title compound. ES/MS: 310, 312 (M+H⁺).

**5-[(6-bromo-2-pyridyl)oxymethyl]-N-(1-cyanocyclopropyl)-4-methoxy-pyridine-2-carboxamide (Intermediate I-53):** A suspension of 5-(bromomethyl)-N-(1-cyanocyclopropyl)-4-methoxy-pyridine-2-carboxamide (112 mg, 0.36 mmol), 6-bromopyridin-2-ol (50 mg, 0.29 mmol), and silver carbonate (169 mg, 0.61 mmol) in CH₃CN (5 mL) was heated at 50 °C overnight. Upon completion, the mixture was diluted with EtOAc and brine filtered over Celite frit. The reaction mixture was partitioned, and the organic phase was washed one more time with brine. The crude produced was dried over sodium sulfate, concentrated, and purified by flash chromatography (eluent: EtOAc/hexanes) to give **I-53.** ES/MS: 403, 405 (M+H⁺).

### Preparation of Intermediate I-54

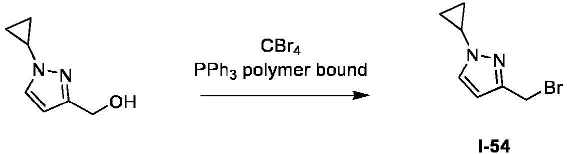

**3-(bromomethyl)-1-cyclopropyl-pyrazole (I-54):** Carbon tetrabromide (0.541 g, 0.00163 mol) was added to a solution of (1-cyclopropylpyrazol-3-yl)methanol (0.188 g, 1.36 mmol) and (4-diphenylphosphanylphenyl polymer bound) (78.7 %, 0.542 g, 0.00163 mol) in DCM (10 mL) at 0 °C. The mixture was gradually warmed to rt and stirred overnight. The resulting suspension was filtered, and the filtrate was diluted with DCM and washed with brine. The organic extract was dried over sodium sulfate, concentrated, and purified by flash chromatography (eluent: EtOAc/hexanes) to give **I-54.** ES/MS: 201.2, 203.2 (M+H⁺).

### The following intermediates were prepared in a manner as described for intermediate I-54

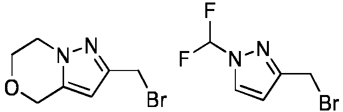

### Preparation of Intermediate I-55

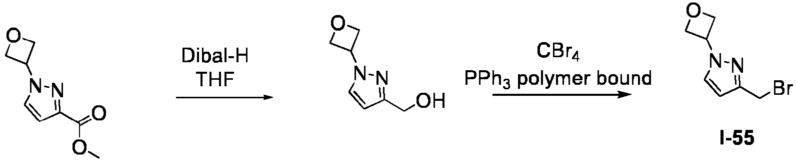

**[1-(oxetan-3-yl)pyrazol-3-yl]methanol:** Diisobutylaluminium hydride (1000 mmol/L in DCM, 2.40 mL, 2.40 mmol) was added to a solution of methyl 1-(oxetan-3-yl)pyrazole-3-carboxylate (175 mg, 0.961 mmol) in THF (5 mL) at 0 °C and stirred for 1 hr. Following this time, the mixture was diluted with 5 mL Et₂O and cooled to 0 °C. Upon completion of the time, 0.100 mL water, 0.100 mL 15% NaOH, and 0.240 mL water was added. The solution was warmed to rt and stirred for 15 min. Following this time, MgSO₄ was added and the solution was stirred for an additional 15 min, then filtered to give title product that was carried onto the next step without further purification. ES/MS: 155.2 (M+H⁺).

**3-(bromomethyl)-1-(oxetan-3-yl)pyrazole (I-55):** Carbon tetrabromide (0.281 g, 0.000848 mol) was added to a solution of [1-(oxetan-3-yl)pyrazol-3-yl]methanol (0.109 g, 0.707 mmol) and (4-diphenylphosphanylphenyl polymer bound) (78.7 %, 0.282 g, 0.000848 mol) in DCM (10 mL) at 0 °C. The mixture was gradually warmed to rt and stirred overnight. The resulting suspension was filtered, and the filtrate was diluted with DCM and washed with brine. The organic extract was dried over sodium sulfate, concentrated, and purified by flash chromatography (eluent: Et₂O/hexanes) to give **I-55.** ES/MS: 217.2, 219.2 (M+H⁺).

### Preparation of Intermediate I-56

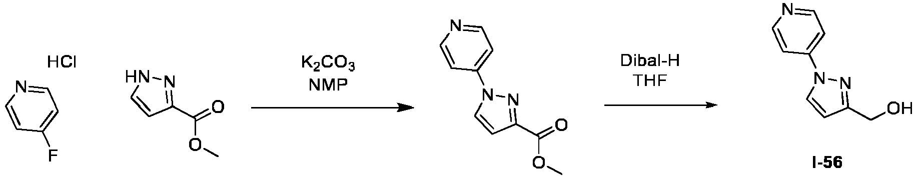

**Methyl 1-(4-pyridyl)pyrazole-3-carboxylate:** In a 40 mL reaction vial, a mixture of methyl 1H-pyrazole-3-carboxylate (472 mg, 3.74 mmol), 4-fluoropyridine;hydrochloride (500 mg, 3.74 mmol), and potassium carbonate (1358 mg, 9.83 mmol) in NMP (10 mL) was heated at 120 °C for 48 hr. Following this time, the mixture was diluted with EtOAc and washed with LiCl 5% 2x and brine. The organic extract was dried over sodium sulfate, concentrated, and purified by flash chromatography (eluent: EtOAc/hexanes) to give the title compound. ES/MS: 204.2 (M+H⁺).

**[1-(4-pyridyl)pyrazol-3-yl]methanol (I-56):** To a solution of methyl 1-(4-pyridyl)pyrazole-3-carboxylate (106 mg, 0.520 mmol) in THF (5 mL) at 0 °C, was added diisobutylaluminium hydride (1.0 M in DCM, 1.30 mL, 1.30 mmol). The solution was stirred for 1 hr. Following this time, the mixture was diluted with 5 mL Et₂O and cooled to 0 °C. Upon completion of the cooling, 0.05 mL water, 0.05 mL 15% NaOH, and 0.130 mL water was added to the solution. The solution was then warmed to rt and stirred for 15 min, followed by the addition of MgSO₄. The solution was stirred and additional 15 min, and then filtered. The crude product was purified by flash chromatography (eluent: EtOAc/hexanes) to give **I-56.** ES/MS: 176.2 (M+H⁺).

### Preparation of Intermediate I-57

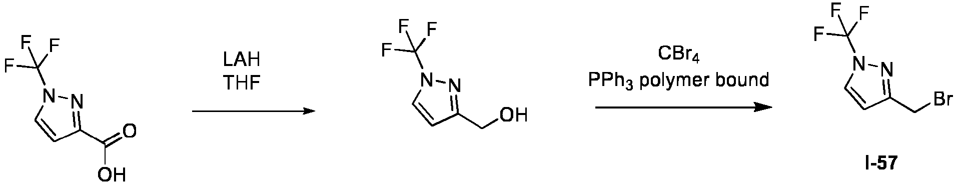

**[1-(trifluoromethyl)pyrazol-3-yl]methanol:** To a solution of 1-(trifluoromethyl)pyrazole-3-carboxylic acid (321 mg, 1.78 mmol) in THF (10 mL) at 0 °C, was added lithium aluminum hydride (2.0M in THF) (2.00 mmol/L, 980 mL, 1.96 mmol). The solution was gradually warmed to rt and stirred for 1 hr. Following this time, the solution was diluted with Et₂O, and cooled to 0 °C. Upon completion of the cooling, 0.075 mL water, 0.075 mL 15% aqueous NaOH, and 0.225 mL water was added to the solution, which was then warmed to rt and stirred for an additional 15 min. Following the additional 15 min. MgSO₄, was added and the solution was stirred another 15 min, then filtered to give title product that was carried onto the next step without further purification. ES/MS: 167.2 (M+H⁺).

**3-(bromomethyl)-1-(trifluoromethyl)pyrazole (I-57):** Carbon tetrabromide (0.465 g, 0.00140 mol) was added to a solution of [1-(trifluoromethyl)pyrazol-3-yl]methanol (0.194 g, 1.17 mmol) and (4-diphenylphosphanylphenyl polymer bound) (78.7 %, 0.465 g, 0.00140 mol) in DCM (10 mL) at 0 °C. The mixture was gradually warmed to rt and stirred overnight. The resulting suspension was filtered, and the filtrate was diluted with DCM and washed with brine. The organic extract was dried over sodium sulfate, concentrated, and purified by flash chromatography (eluent: Et₂O/hexanes) to give **I-57.** ES/MS: 230.2 (M+H⁺).

### Preparation of Intermediate I-58

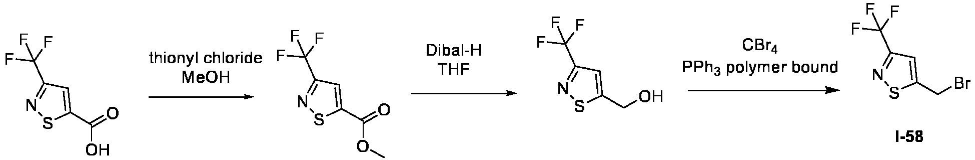

**Methyl 3-(trifluoromethyl)isothiazole-5-carboxylate:** To a solution of 3-(trifluoromethyl)isothiazole-5-carboxylic acid (303 mg, 1.54 mmol) in MeOH (3 mL) at 0 °C, was added thionyl chloride (0.125 mL, 1.69 mmol). The resulting solution was gradually warmed to rt and stirred overnight. Following this time, more thionyl chloride (0.125 mL, 1.69 mmol) was added and stirred for 9 hr. Upon completion of this time, the mixture was concentrated and purified by flash chromatography (eluent: EtOAc/hexanes) to give the title compound. 1H NMR (400 MHz, Chloroform-d) δ 8.01 (s, 1H), 4.01 (s, 3H).

**[3-(trifluoromethyl)isothiazol-5-yl] methanol:** To a solution of methyl 3-(trifluoromethyl)isothiazole-5-carboxylate (136 mg, 0.644 mmol) in THF (5 mL) at 0 °C, was added diisobutylaluminium hydride (1.0 M in DCM, 1.61 mL, 1.61 mmol). The resulting solution was stirred for 3 hr. Upon completion of this time the mixture was diluted with 5 mL Et₂O and cooled to 0 °C. Once the mixture was cooled, 0.064 mL water, 0.064 mL 15% NaOH, and 0.161 mL water, was added and the solution was warmed to rt and stirred for 15 min. Following this time, MgSO₄ was added and the solution was, stirred an additional 15 min, then filtered. The crude product was purified by flash chromatography (eluent: EtOAc/hexanes) to give the title compound. ES/MS: 184.2 (M+H⁺).

**5-(bromomethyl)-3-(trifluoromethyl)isothiazole (I-58):** To a solution of [3-(trifluoromethyl)isothiazol-5-yl]methanol (85 mg, 0.464 mmol) and (4-diphenylphosphanylphenyl polymer bound) (78.7 %, 185 mg, 0.557 mol) in DCM (10 mL) at 0 °C, was added carbon tetrabromide (185 mg, 0.557 mmol). The mixture was gradually warmed to rt and stirred overnight. The resulting suspension was filtered, and the filtrate was diluted with DCM and washed with brine. The organic extract was dried over sodium sulfate, concentrated, and purified by flash chromatography (eluent: Et2O/hexanes) to give **I-58.** 1H NMR (400 MHz, Chloroform-d) δ 7.49 (d, J = 0.8 Hz, 1H), 4.72 (d, J = 0.8 Hz, 2H).

### Preparation of Intermediate I-59

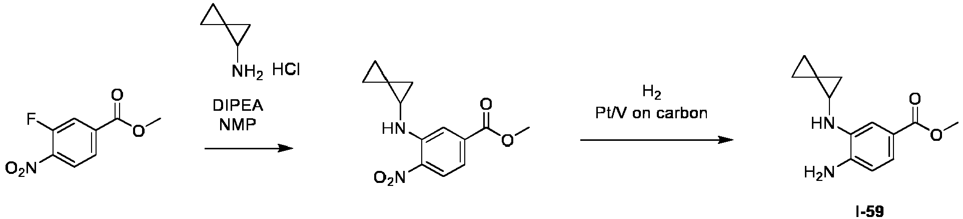

**Methyl 4-nitro-3-(spiro[2.2]pentan-2-ylamino)benzoate:** A solution of methyl 3-fluoro-4-nitro-benzoate (0.205 g, 1.03 mmol), spiro[2.2]pentan-2-amine;hydrochloride (0.151 g, 1.26 mmol) and N,N-Diisopropylethylamine (0.538 mL, 3.09 mmol) in NMP (3 mL) was heated at 90 °C for 12 hr. Following this time, the mixture was diluted with EtOAc, washed with 5%LiCl, brine and water. The organic extract was dried over sodium sulfate, concentrated, and purified by flash chromatography (eluent: EtOAc/hexanes) to give the title compound. ES/MS: 263.2 (M+H⁺); 1H NMR (400 MHz, Chloroform-d) δ 8.22 (d, J = 8.8 Hz, 1H), 8.06 (s, 1H), 7.79 (d, J = 1.8 Hz, 1H), 7.29 (dd, J = 8.9, 1.8 Hz, 1H), 3.98 (s, 3H), 3.01 (dd, J = 6.3, 3.1 Hz, 1H), 1.54 - 1.42 (m, 1H), 1.14 (ddd, J = 9.0, 5.5, 4.1 Hz, 1H), 1.06 - 0.98 (m, 2H), 0.95 (td, J = 8.5, 4.7 Hz, 2H).

**Methyl 4-amino-3-(spiro[2.2]pentan-2-ylamino)benzoate (I-59):** A solution of methyl 4-nitro-3-(spiro[2.2]pentan-2-ylamino)benzoate (101 mg, 0.4385mmol) in EtOAc (8 mL) was degassed by cycling the mixture between argon and vacuum 3x. To the mixture was added platinum (1%), vanadium (2%) on carbon (50-70% wetted) and **I-59** was carried onto the next step without further purification. ES/MS: 233.2 (M+H⁺).

### Preparation of Intermediates I-60 and I-61

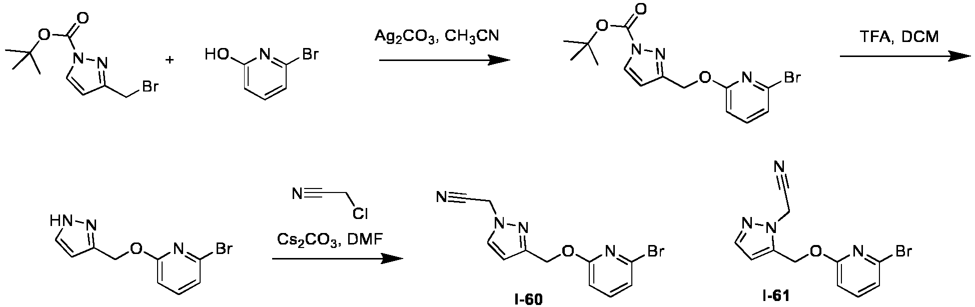

**Tert-butyl 3-[(6-bromo-2-pyridyl)oxymethyl]pyrazole-1-carboxylate:** A suspension of *tert*-butyl 3-(bromomethyl)pyrazole-1-carboxylate (946 mg, 3.6 mmol), 6-bromopyridin-2-ol (500 mg, 2.9 mmol), and silver carbonate (1694 mg, 6.1 mmol) in CH₃CN (15 mL) was heated at 50 °C for 15 hr. Following this time, 335 mg more of 6-bromopyridin-2-ol and 5 mL CH₃CN was added and the solution was heated at 50 °C for 5 hr. Upon completion 500 mg of 6-bromopyridin-2-ol was added and heating was resumed for 2 hr. Following this time, the mixture was diluted with EtOAc and brine. The mixture was filtered over a plug of Celite. The reaction mixture was partitioned, and the organic phase was washed with brine. The organic extract was dried over sodium sulfate, concentrated, and purified by flash chromatography (eluent: EtOAc/hexanes) to give the title compound. ES/MS: 298, 300 (M+H⁺).

**2-bromo-6-(1H-pyrazol-3-ylmethoxy)pyridine:** A solution of *tert*-butyl 3-[(6-bromo-2-pyridyl)oxymethyl]pyrazole-1-carboxylate (456 mg, 1.3 mmol) and TFA (0.49 mL, 6.4 mmol) in DCM (5 mL) was stirred at rt overnight. Following this time, the solution was diluted with DCM and washed with saturated sodium bicarbonate solution, dried over sodium sulfate, concentrated, and carried onto the next step without further purification. ES/MS: 254, 256 (M+H⁺).

**2-[3-[(6-bromo-2-pyridyl)oxymethyl]pyrazol-1-yl]acetonitrile (I-60) and 2-[5-[(6-bromo-2-pyridyl)oxymethyl]pyrazol-1-yl]acetonitrile (I-61):** To a suspension of 2-bromo-6-(1H-pyrazol-3-ylmethoxy)pyridine (0.115 g, 0.453 mmol) and cesium carbonate (0.177 g, 0.543 mmol) in DMF (3 mL), was added 2-chloroacetonitrile (0.0314 mL, 0.498 mmol). The solution was stirred at rt overnight. Following this time, the solution was then warmed to 40 °C for 2 hr., then diluted with EtOAc and washed with 5% LiCl 2x and brine. The organic extract was dried over sodium sulfate, concentrated, and purified by flash chromatography (eluent: EtOAc/hexanes) to give the 1-60 and 1-61.

**2-[3-[(6-bromo-2-pyridyl)oxymethyl]pyrazol-1-yl]acetonitrile (I-60):** ES/MS: 293.2, 295.1 (M+H⁺); 1H NMR (400 MHz, Chloroform-d) δ 7.55 (d, J = 2.4 Hz, 1H), 7.45 (dd, J = 8.2, 7.5 Hz, 1H), 7.11 (dd, J = 7.5, 0.7 Hz, 1H), 6.76 (dd, J = 8.2, 0.7 Hz, 1H), 6.52 (d, J = 2.4 Hz, 1H), 5.39 (s, 2H), 5.10 (s, 2H).

**2-[5-[(6-bromo-2-pyridyl)oxymethyl]pyrazol-1-yl]acetonitrile (I-61):** ES/MS: 293.2, 295.0 (M+H⁺); 1HNMR (400 MHz, Chloroform-d) δ 7.57 (d, J = 1.9 Hz, 1H), 7.50 (dd, J = 8.2, 7.5 Hz, 1H), 7.15 (d, J = 7.4 Hz, 1H), 6.80 (d, J = 8.1 Hz, 1H), 6.48 (d, J = 1.9 Hz, 1H), 5.47 (s, 2H), 5.35 (s, 2H).

### Preparation of Intermediate I-62

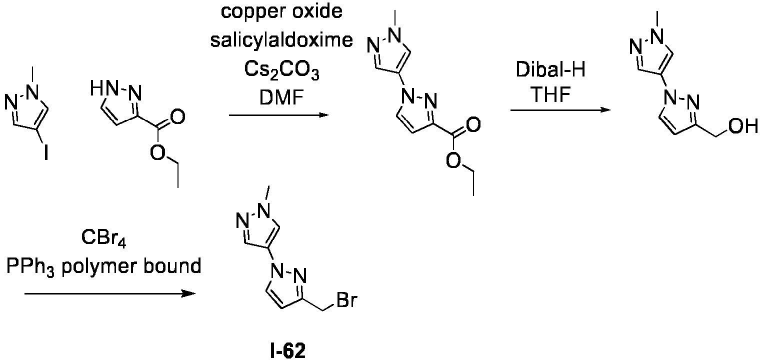

**Ethyl 1-(1-methylpyrazol-4-yl)pyrazole-3-carboxylate:** In a 40 mL reaction vial, a mixture of ethyl 1H-pyrazole-3-carboxylate (1000 mg, 7.14 mmol), 4-iodo-1-methyl-pyrazole (1484 mg, 7.14 mmol), cesium carbonate (5812 mg, 17.8 mmol), copper(I) oxide (60.0 mg, 0.419 mmol), and salicylaldoxime (120 mg, 0.875 mmol) in DMF (20 mL) was heated at 110 °C for 48 hr. Following this time, the mixture was diluted with EtOAc and washed with 5% LiCl, saturated sodium bicarbonate, and brine. The organic extract was dried over sodium sulfate and purified by flash chromatography (eluent: EtOAc/hexanes) to give the title compound. ES/MS: 221.2 (M+H⁺).

**[1-(1-methylpyrazol-4-yl)pyrazol-3-yl]methanol:** To a solution of ethyl 1-(1-methylpyrazol-4-yl)pyrazole-3-carboxylate (287 mg, 1.30 mmol) in THF (6 mL) at 0 °C, was added diisobutylaluminium hydride (1.0 M in DCM, 3.26 mL, 3.26 mmol). The solution was stirred for 1 hr. while gradually warming to rt. Following this time, the solution was diluted with Et₂O and cooled to 0 °C. Upon completion of the cooling 0.130 mL water, 0.130 mL 15% aqueous NaOH, and 0.326 mL water was added to the solution and then the resulting solution was warmed to rt. Following the warming, the solution was stirred for 15 min, then MgSO₄ was added and the solution was stirred for an additional 15 min, then filtered. The filtrate was concentrated and purified by flash chromatography (eluent: EtOAc/hexanes) to give the title compound. ES/MS: 179.2 (M+H⁺).

**3-(bromomethyl)-1-(1-methylpyrazol-4-yl)pyrazole (I-62):** To a solution of [1-(1-methylpyrazol-4-yl)pyrazol-3-yl]methanol (136 mg, 0.762 mmol) and (4-diphenylphosphanylphenyl polymer bound) (78.7 %, 303 mg, 0.914 mmol) in DCM (10 mL) at 0 °C, was added carbon tetrabromide (303 mg, 0.914 mmol). The mixture was gradually warmed to rt and stirred overnight. The resulting suspension was filtered, and the filtrate was diluted with DCM and washed with brine. The organic extract was dried over sodium sulfate, concentrated, and purified by flash chromatography (eluent: Et2O/hexanes) to give **I-62.** ES/MS: 241.2, 243.2 (M+H⁺); ¹H NMR (400 MHz, Chloroform-d) δ 7.71 (s, 1H), 7.69 (d, J = 0.8 Hz, 1H), 7.61 (d, J = 2.4 Hz, 1H), 6.47 (d, J = 2.4 Hz, 1H), 4.56 (s, 2H), 3.96 (s, 3H).

### The following intermediate was prepared in a manner as described for intermediate I-62

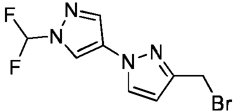

### Preparation of Intermediate I-63

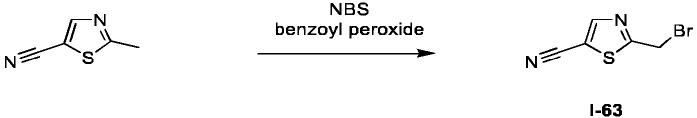

**2-(bromomethyl)thiazole-5-carbonitrile (I-63):** To a solution of 2-methylthiazole-5-carbonitrile (200 mg, 1.61 mmol) in CCl₄ (8 mL), was added N-Bromosuccinimide (375 mg, 2.11 mmol), followed by benzoyl peroxide (42.0 mg, 0.173 mmol). The solution was heated at 90 °C for 9 hrs. Following this time, the mixture was cooled to rt and 5 mL hexanes was added. The suspension was filtered, and the filtrate was concentrated, and purified by flash chromatography (eluent: EtOAc/hexanes) to give **I-63.** ES/MS: 203.0, 205.2 (M+H⁺); 1HNMR (400 MHz, Chloroform-d) δ 8.23 (s, 1H), 4.74 (s, 2H).

### The following intermediates were prepared in a manner as described for intermediate I-63

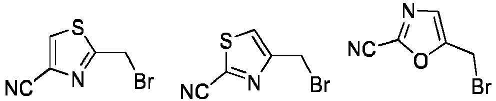

### Preparation of Intermediate I-64

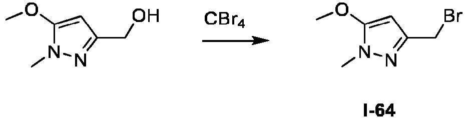

**3-(bromomethyl)-5-methoxy-1-methyl-1H-pyrazole (I-64):** To a stirring solution of (5-methoxy-1-methyl-1H-pyrazol-3-yl)methanol (1.42 g, 10 mmol, 1.0 eq) in DCM (15 mL),was added at rt, CBr₄ (7.2 g, 21mmol, 2.0 eq) and triphenylphosphine (5.7 g, 21 mmol, 2.0 eq). The mixture was stirred for an additional 16 h. Upon completion, the reaction mixture was diluted with water (150 mL), extracted with DCM (3×150 mL), washed with brine (50 mL), dried over Na₂SO₄ and concentrated to get the crude product which was purified by column chromatography (0 to 2% MeOH-DCM) to afford 3-(bromomethyl)-5-methoxy-1-methyl-1H-pyrazole **(I-64).**

### The following intermediates were prepared in a manner as describe for intermediate I-64

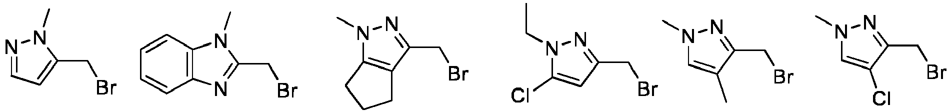

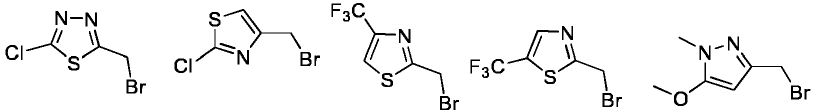

### Preparation of Intermediate I-65

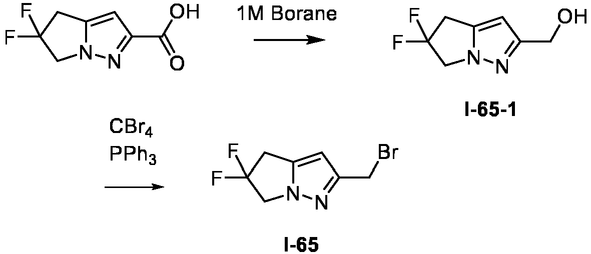

**(5,5-difluoro-5,6-dihydro-4H-pyrrolo[1,2-b]pyrazol-2-yl)methanol (I-65-1):** 5,5-difluoro-5,6-dihydro-4H-pyrrolo[1,2-b]pyrazole-2-carboxylic acid (105 mg, 0.56 mmol) was dissolved in THF (3 mL) and stirred at 0° C for 5 min. Next, 1M Borane (3.4 mL) solution was added dropwise to the reaction mixture at 0 °C over a period of 30 min. The ice bath was removed and stirring continued at rt for 7 hours. Following this time, the reaction mixture was cooled in an ice bath and treated with 3M HCl (5 mL). The solution was heated for 1 h at 50 °C. Upon completion of the time, the solution was washed with EtOAc (2x) and the aqueous layer was cooled in an ice bath and neutralized with 3M NaOH. The solution was extracted with EtOAc (3x), the combined organic layers were washed with brine, dried (Na₂SO₄) and concentrated in vacuo to obtain (5,5-difluoro-5,6-dihydro-4H-pyrrolo[1,2-b]pyrazol-2-yl)methanol **(I-65-1).**
**2-(bromomethyl)-5,5-difluoro-5,6-dihydro-4H-pyrrolo[1,2-b]pyrazole (I-65):** The title compound was prepared in a manner described for Intermediate **I-64,** using **Intermediate I-65-1.** ES/MS m/z 237 (M+H⁺).

### Preparation of Intermediate I-66

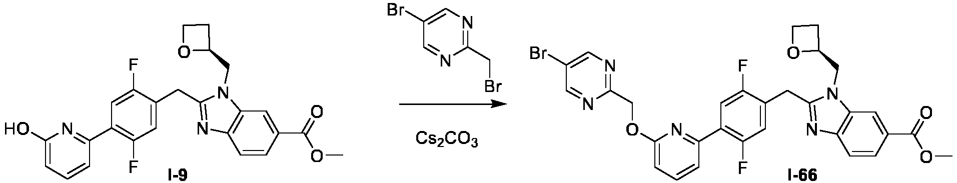

**Methyl 2-[[4-[6-[(5-bromopyrimidin-2-yl)methoxy]-2-pyridyl]-2,5-difluorophenyl]methyl]-3-[[(2S)-oxetan-2-yl]methyl]benzimidazole-5-carboxylate (I-66):** Methyl 2-[[4-[6-[(5-bromopyrimidin-2-yl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** to provide **I-66.** ES/MS: 637.4 (M+H+).

### Preparation of Intermediate I-67

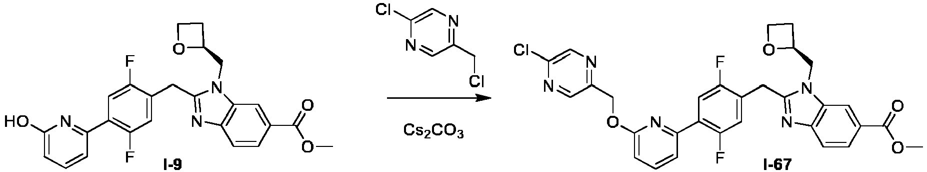

**Methyl 2-[[4-[6-[(5-chloropyrazin-2-yl)methoxy]-2-pyridyl]-2,5-difluorophenyl]methyl]-3-[[(2S)-oxetan-2-yl]methyl]benzimidazole-5-carboxylate (I-67):** Methyl 2-[[4-[6-[(5-chloropyrazin-2-yl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** to provide **I-67.** ES/MS: 593 (M+H+).

### Preparation of Intermediate I-68

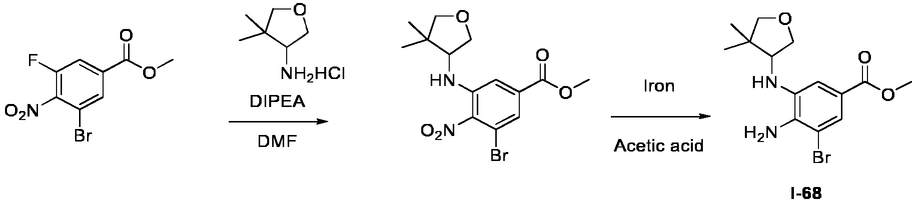

**Methyl 3-bromo-5-((4,4-dimethyltetrahydrofuran-3-yl) amino-4-nitrobenzoate:** Methyl 3-bromo-5-fluoro-4-nitrobenzoate (0.5 g, 1.8 mmol) was dissolved in a 100 mL round bottom flask containing DMF (10mL), Next, 4,4-dimethyltetrahydrofuran-3-amine hydrochloride (0.46 g, 3 mmol) and N,N-diisopropylethylamine (0.63 mL, 3.6mmol) was added to the solution. The mixture was stirred at 50 °C overnight. Afterward, the mixture was concentrated to remove most of the THF, and the crude material was dissolved in EtOAc (40 mL). The organics were washed with 50% NH₄Cl (2x 10 mL) and with brine (1x 50mL). The organics were subsequently dried over MgSO₄, filtered, and concentrated under reduced pressure. The crude material was carried forward without further purification: ES/MS: 374.2 (M+H+).

**Methyl 4-amino-3-bromo-5-[(4,4-dimethyltetrahydrofuran-3-yl)amino]benzoate (I-68):** To a 100 mL round bottom flask, methyl 3-bromo-5-((4,4-dimethyltetrahydrofuran-3-yl) amino-4-nitrobenzoate (0.58 g, 1.6 mmol), Iron (0.43 g, 7.8 mmol), and Acetic acid (10 mL) were added. The mixture was stirred and heated at 100 °C for 1h Following this time, the mixture was filtered through Celite to remove the catalyst. The filtrate was concentrated under reduced pressure to give **I-68** which was used without further purification: ES/MS: 344.2 (M+H+).

### Preparation of Intermediate I-69

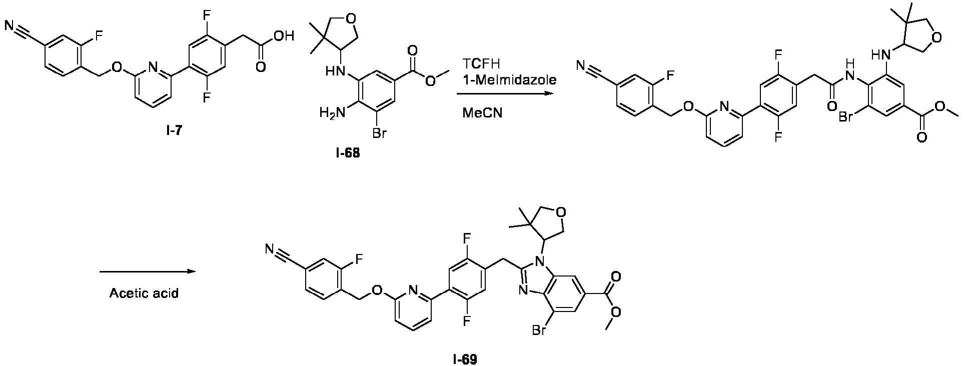

**Methyl 3-bromo-4-[[2-[4-[6-[(4-cyano-2-fluoro-phenyl)methoxy]-2-pyridyl]-2,5-difluoro-phenyl] acetyl] amino] -5- [(4,4-dimethyltetrahydrofuran-3-yl)amino]benzoate:** To a solution of **I-68** (200 mg, 0.58 mmol) and **I-7** (180 mg, 0.45 mmol) in MeCN (5 mL) and cooled to 0 °C was added 1-methylimidazole (239 mg, 0.23 mL, 2.9 mmol) followed by N,N,N',N'-Tetramethylchloroformamidinium Hexafluorophosphate (204 mg, 0.73 mmol). The reaction mixture was warmed to rt and stirred for 30 min. Upon completion of the 30 min, the crude mixture was concentrated in vacuo, then partitioned between water and EtOAc. The organic layer was isolated and washed with an additional portion of water and then brine. The isolated organic layer was dried over sodium sulfate, isolated by vacuum filtration, concentrated in vacuo, and purified by silica gel column chromatography (eluent: EtOAc/hexanes) to provide the desired product. ES/MS: 724.4 [M+H]+.

**Methyl 7-bromo-2-[[4-[6-[(4-cyano-2-fluoro-phenyl)methoxy]-2-pyridyl]-2,5-difluoro-phenyl]methyl]-3-(4,4-dimethyltetrahydrofuran-3-yl)benzimidazole-5-carboxylate (I-69):** A solution of methyl 3-bromo-4-[[2-[4-[6-[(4-cyano-2-fluoro-phenyl)methoxy]-2-pyridyl]-2, 5-difluoro-phenyl]acetyl] amino]-5-[(4,4-dimethyltetrahydrofuran-3-yl)amino]benzoate (100mg, 0.14 mmol) in acetic acid (2 mL) was heated to 80 °C for 5 days. The mixture was concentrated and partitioned between EtOAc and saturated aqueous sodium bicarbonate. The organic layer was isolated and dried over sodium sulfate, isolated by vacuum filtration, concentrated in vacuo, and purified by silica gel column chromatography (eluent: EtOAc/hexanes) to provide **I-69.** ES/MS: 706.5 [M+H]⁺.

### Preparation of Intermediate I-70

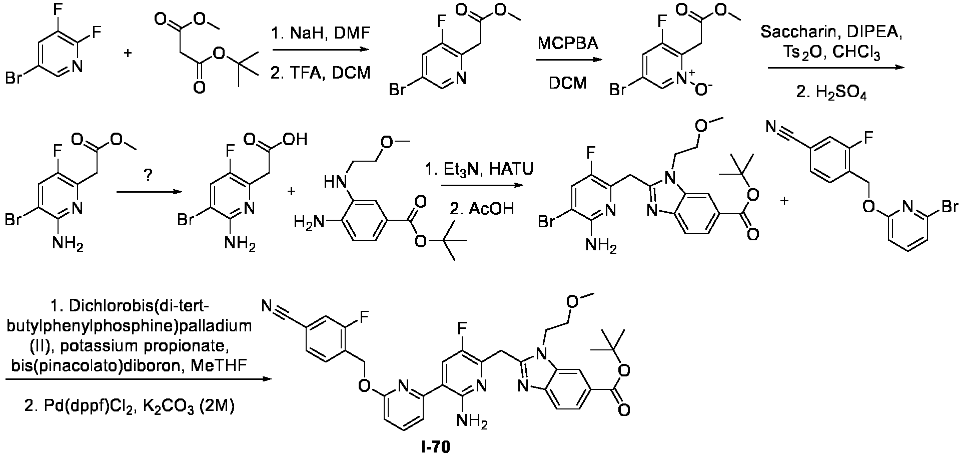

**Methyl 2-(5-bromo-3-fluoropyridin-2-yl)acetate:** Tert-butyl methyl malonate was added dropwise to a suspension of NaH (60% in mineral oil, 1.3g, 34 mmol) in DMF (20 mL) at 5 °C, and the suspension was stirred for 5 min. Next, 5-bromo-2,3-difluoropyridine was added dropwise, and the resulting suspension was warmed to 60 °C and stirred at that temperature overnight. Following this time, NH₄Cl was added, and the mixture was extracted with ether. The organic phase was rinsed with brine, and concentrated. The residue was redissolved in DCM (10 mL). Next, TFA (10 mL) was added, and the resulting solution was warmed to 40 °C and stirred for 5 hours. Upon completion of time, the mixture was concentrated and purified by flash chromatography (EtOAc/hexanes) to give title product: ES/MS: 248.2 (M+H⁺).

**5-bromo-3-fluoro-2-(2-methoxy-2-oxoethyl)pyridine 1-oxide:** MCPBA (3.51 g, 16 mmol) was added to a solution of methyl 2-(5-bromo-3-fluoropyridin-2-yl)acetate (2.68 g, 11 mmol) in DCM (30 mL) at 0 °C, and the resulting solution was allowed to warm to rt and stirred overnight. Next, the mixture was diluted with hexanes (20 mL) and filtered. The filtrate was concentrated and purified by flash chromatography (EtOAc/hexanes) to give title product: ES/MS: 265.2 (M+H⁺).

**Methyl 2-(6-amino-5-bromo-3-fluoropyridin-2-yl)acetate:** P-toluenesulfonic anhydride (1.5 g) was added over the course of 1.5 hours to a solution of 5-bromo-3-fluoro-2-(2-methoxy-2-oxoethyl)pyridine 1-oxide (1.24 g, 4.7 mmol), saccharin (6.3g, 34 mmol), and DIPEA (6.5 mL, 38 mmol) in chloroform (5 mL). The resulting solution was stirred at rt for 2 hours. Following this time, an additional amount of p-toluenesulfonic anhydride (1.3 g) was added and stirred for 1 hr. After the one hour, a further additional amount of p-toluenesulfonic anhydride (2.0 g) was added and stirred over the weekend. The reaction was quenched with Na₂CO₃ and filtered. The phases were separated, and the aqueous phase was extracted with DCM. The combined organics were washed with 10% citric acid, dried, filtered, concentrated, and purified by flash chromatography (EtOAc/hexanes). The resulting product was suspended in 2M H₂SO₄ (4 mL), and stirred at 90 °C for 18 hours, warmed to reflux, and stirred for 24 hours. The mixture was filtered, and filtrate washed with CHCl₃. The aqueous phase was basified with NaOH to pH ~ 7 and filtered. Filtrate was acidified to pH ~5 and extracted with CHCl₃ (3x). Organics were dried and concentrated to give title product: ES/MS: 249.0 (M+H⁺). ¹H NMR (400 MHz, Chloroform-d) δ 7.59 (d, J = 7.7 Hz, 1H), 5.05 (s, 2H), 3.80 (d, J = 2.2 Hz, 2H). ¹⁹F NMR (376 MHz, Chloroform-d) δ -137.76 (d, J = 7.5 Hz).

**2-(6-amino-5-bromo-3-fluoropyridin-2-yl)acetic acid:** The title intermediate was prepared in a manner as described for intermediate **I-7** (step 2) substituting methyl 2-(6-amino-5-bromo-3-fluoropyridin-2-yl)acetate for methyl 2-(4-(6-((4-cyano-2-fluorobenzyl)oxy)pyridin-2-yl)-2,5-difluorophenyl)acetate.

**Tert-butyl 2-((6-amino-5-bromo-3-fluoropyridin-2-yl)methyl)-1-(2-methoxyethyl)-1H-benzo[d]imidazole-6-carboxylate:** The title compound was prepared in a manner as described for Intermediate **I-2**, using 2-(6-amino-5-bromo-3-fluoropyridin-2-yl)acetic acid in place of 2-(4-bromo-2-fluoro-phenyl)acetic acid, and tert-butyl 4-amino-3-((2-methoxyethyl)amino)benzoate in place of methyl 4-amino-3-(2-methoxyethylamino)benzoate. ES/MS: 481.1 (M+H⁺). ¹H NMR (400 MHz, Chloroform-d) δ 8.07 (d, J = 1.5 Hz, 1H), 7.94 (d, J = 8.6 Hz, 1H), 7.78 (d, J = 8.5 Hz, 1H), 7.51 (d, J = 7.8 Hz, 1H), 4.79 (s, 2H), 4.57 - 4.39 (m, 4H), 3.70 (t, J = 5.3 Hz, 2H), 3.30 (s, 3H), 1.65 (s, 9H).

**Tert-butyl 2-((2'-amino-6-((4-cyano-2-fluorobenzyl)oxy)-5'-fluoro-[2,3'-bipyridin]-6'-yl)methyl)-1-(2-methoxyethyl)-1H-benzo[d]imidazole-6-carboxylate (I-70): I-70** was prepared in a manner as described for Intermediate **I-7,** using dichlorobis(di-tert-butylphenylphosphine)palladium(II) in place of Pd(dppf)Cl₂ in step 1, and tert-butyl 2-((6-amino-5-bromo-3-fluoropyridin-2-yl)methyl)-1-(2-methoxyethyl)-1H-benzo[d]imidazole-6-carboxylate in place of methyl 2-(4-bromo-2,5-difluorophenyl)acetate. ES/MS: 627.5 (M+H⁺).

### Preparation of Intermediate I-71

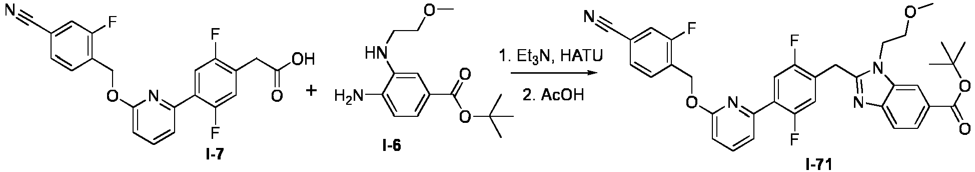

**Tert-butyl 2-(4-(6-((4-cyano-2-fluorobenzyl)oxy)pyridin-2-yl)-2,5-difluorobenzyl)-1-(2-methoxyethyl)-1H-benzo[d]imidazole-6-carboxylate (I-71):** The title compound was prepared in a manner as described for Intermediate **I-2,** using Intermediate **I-6** and Intermediate **I-7.** ES/MS: 629.5 (M+H⁺).

### Preparation of Intermediate I-72

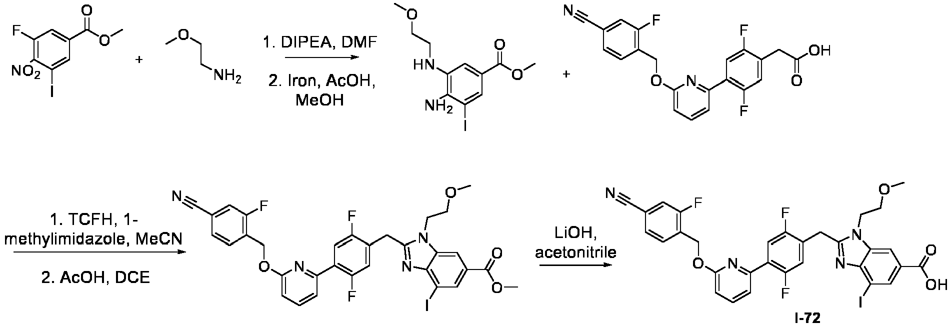

**Methyl 4-amino-3-iodo-5-((2-methoxyethyl)amino)benzoate:** The title compound was prepared in a manner as described for intermediate **I-1.** Reduction was executed by stirring Iron (603 mg, 10.8 mmol), acetic acid (12.0 mL, 1.8 mmol), and crude methyl 3-iodo-5-(2-methoxyethylamino)-4-nitro-benzoate (821 mg, 2.16 mmol) in methanol (5.0 mL) at reflux for 1 hour. The mixture was diluted with DCM, filtered, and organics were dried, filtered, concentrated, and carried on crude.

**Methyl 2-(4-(6-((4-cyano-2-fluorobenzyl)oxy)pyridin-2-yl)-2,5-difluorobenzyl)-4-iodo-1-(2-methoxyethyl)-1H-benzo[d]imidazole-6-carboxylate:** The title compound was prepared in a manner as described for Intermediate **I-13,** using methyl 4-amino-3-iodo-5-((2-methoxyethyl)amino)benzoate and Intermediate **I-7.**

**2-[[4-[6-[(4-cyano-2-fluoro-phenyl)methoxy]-2-pyridyl]-2,5-difluorophenyl]methyl]-7-iodo-3-(2-methoxyethyl)benzimidazole-5-carboxylic acid (I-72):** In an 8 mL reaction vial, a solution of methyl 2-[[4-[6-[(4-cyano-2-fluoro-phenyl)methoxy]-2-pyridyl]-2,5-difluoro-phenyl]methyl]-7-iodo-3-(2-methoxyethyl)benzimidazole-5-carboxylate (105 mg, 0.15 mmol) and lithium hydroxide, monohydrate (25 mg, 0.59 mmol) in THF/water (2:1, 3 mL) was heated at 70 °C until completion (15 min). Following completion of the reaction, trifluoroacetic anhydride (0.3 mL) was added and the solution purified directly by RP-HPLC (eluent: MeCN/H₂O) to give **I-72.** ES/MS: 699.1.

### Preparation of Intermediate I-73

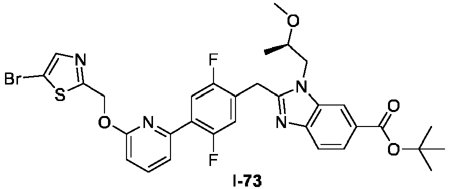

**Tert-butyl (R)-2-(4-(6-((5-bromothiazol-2-yl)methoxy)pyridin-2-yl)-2,5-difluorobenzyl)-1-(2-methoxypropyl)-1H-benzo[d]imidazole-6-carboxylate (I-73):** tert-butyl (R)-2-(4-(6-((5-bromothiazol-2-yl)methoxy)pyridin-2-yl)-2, 5-difluorobenzyl)-1-(2-methoxypropyl)-1H-benzo[d]imidazole-6-carboxylate was prepared in a manner as described for Intermediate **I-21** substituting **I-26** for **I-4.** ES/MS: 686.8 (M+H⁺).

### Preparation of Intermediate I-74:

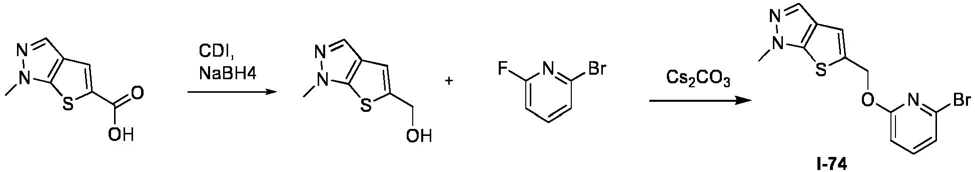

**(1-methylthieno[2,3-c]pyrazol-5-yl)methanol:** To a solution of 1-methylthieno[2,3-c]pyrazole-5-carboxylic acid (140 mg, 0.77 mmol) in THF (6 mL) was added CDI (249 mg, 1.54 mmol) and the resultant slurry stirred for 2 hours at ambient temperature. Following this time, NaBH₄ (145 mg, 3.84 mmol) was added portion-wise and the mixture stirred overnight. Upon completion MeOH was added (2 mL) and the crude mixture concentrated directly. The crude residue purified by silica gel column chromatography (eluent: EtOAc/hexanes) to provide desired product. ES/MS: 169.1(M+H⁺).

**5-[(6-bromo-2-pyridyl)oxymethyl]-1-methyl-thieno[2,3-c]pyrazole (I-74):** To a solution of 2-bromo-6-fluoro-pyridine (122 mg, 0.69 mmol) in acetonitrile (2 mL) was added (1-methylthieno[2,3-c]pyrazol-5-yl)methanol (106 mg, 0.63 mmol) and cesium carbonate (411 mg, 1.26 mmol) and the resultant mixture stirred for 2 hours at 80 °C. Upon completion the reaction mixture was diluted with EtOAc (25 mL), washed with water (5 mL) and brine (5 mL). The organic layer was dried over MgSO₄, filtered, concentrated and purified by silica gel column chromatography (eluent: EtOAc/hexanes) to provide **I-74**. ES/MS: 326.1 (M+H⁺).

### Preparation of Intermediate I-75

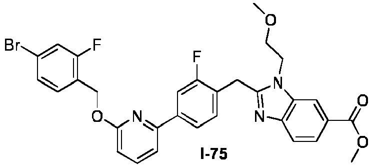

**Methyl 2-(4-(6-((4-bromo-2-fluorobenzyl)oxy)pyridin-2-yl)-2-fluorobenzyl)-1-(2-methoxyethyl)-1H-benzo[d]imidazole-6-carboxylate (I-75):** Methyl 2-(4-(6-((4-bromo-2-fluorobenzyl)oxy)pyridin-2-yl)-2-fluorobenzyl)-1-(2-methoxyethyl)-1H-benzo[d]imidazole-6-carboxylate was prepared in a manner as described for Intermediate **I-19** substituting **I-2** for **I-**96. ES/MS: 623.3 (M+H⁺).

### Preparation of Intermediate I-76

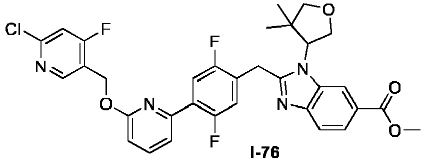

**Methyl 2-(4-(6-((6-chloro-4-fluoropyridin-3-yl)methoxy)pyridin-2-yl)-2,5-difluorobenzyl)-1-(4,4-dimethyltetrahydrofuran-3-yl)-1H-benzo[d]imidazole-6-carboxylate (I-76):** Methyl 2-(4-(6-((6-chloro-4-fluoropyridin-3-yl)methoxy)pyridin-2-yl)-2,5-difluorobenzyl)-1-(4,4-dimethyltetrahydrofuran-3-yl)-1H-benzo[d]imidazole-6-carboxylate was prepared in a manner as described for Intermediate **I-23** substituting **I-25** for **I-4.** ES/MS:638.0 (M+H⁺).

### Preparation of Intermediate I-77

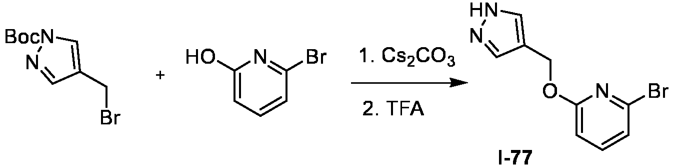

**2-bromo-6-(1H-pyrazol-3-ylmethoxy)pyridine (I-77):** To a solution of 6-bromopyridin-2-ol (180 mg, 1.0 mmol) in acetonitrile (4 mL) was added cesium carbonate (482 mg, 1.5 mmol) and tert-butyl 3-(bromomethyl)pyrazole-1-carboxylate (378 mg, 1.4 mmol) after which the reaction mixture was heated to 65 °C for 30 minutes. Upon completion the reaction mixture was filtered through celite, concentrated and purified by silica gel column chromatography (eluent: EtOAc/hexanes) to provide desired product. The so obtained tert-butyl 3-[(6-bromo-2-pyridyl)oxymethyl]pyrazole-1-carboxylate (343 mg, 0.97 mmol) was dissolved in DCM (4.4 mL) and TFA (1.1 mL) and stirred at ambient temperature for 2 hours. Upon completion the reaction mixture was diluted with EtOAc (25 mL) washed with saturated aqueous NaHCO₃ until gas evolution ceased, dried over MgSO₄, filtered and concentrated to give **I-77** which was used without further purification. ES/MS: 254.2, 256.2 (M+H⁺).

### Preparation of Intermediate I-78

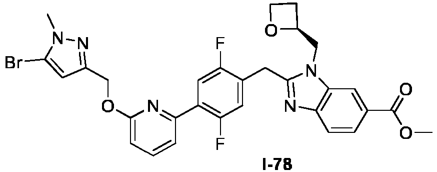

**Methyl (S)-2-(4-(6-((5-bromo-1-methyl-1H-pyrazol-3-yl)methoxy)pyridin-2-yl)-2,5-difluorobenzyl)-1-(oxetan-2-ylmethyl)-1H-benzo[d]imidazole-6-carboxylate (I-78):** Methyl (S)-2-(4-(6-((5-bromo-1-methyl-1H-pyrazol-3-yl)methoxy)pyridin-2-yl)-2,5-difluorobenzyl)-1-(oxetan-2-ylmethyl)-1H-benzo[d]imidazole-6-carboxylate was prepared in a manner as described for Intermediate **I-21** substituting 5-bromo-3-(bromomethyl)-1-methyl-1H-pyrazole for 5-bromo-2-(bromomethyl)thiazole. ES/MS: 638.0, 640.0 (M+H⁺).

### Preparation of Intermediate I-79

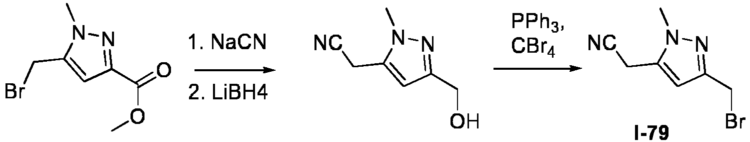

**2-[5-(hydroxymethyl)-2-methyl-pyrazol-3-yl]acetonitrile:** To a solution of methyl 5-(bromomethyl)-1-methyl-pyrazole-3-carboxylate (750 mg, 3.22 mmol) in DMF (9.5 mL) and water (1.2 mL), was added sodium cyanide (241 mg, 4.83 mmol) and the resultant mixture stirred at rt for 3.5 hours. Upon completion the reaction mixture was diluted with EtOAc (50 mL), washed with water (10 mL) and brine (10 mL). The organic layer was dried over MgSO₄, filtered, concentrated and purified by silica gel column chromatography (eluent: EtOAc/hexanes) to provide desired product. The so obtained methyl 5-(cyanomethyl)-1-methylpyrazole-3-carboxylate (328 mg, 1.83 mmol), was dissolved in THF (10 mL) and lithium borohydride (2.0M in THF, 1.83 mL, 3.66 mmol) was added at 0 °C. The reaction mixture was allowed to warm to rt and stir for 6 hr at which point additional lithium borohydride (2.0M in THF, 1.83 mL, 3.66 mmol) was added and the mixture stirred for 2 hr. Upon completion the reaction was quenched by the addition of water (5 mL), diluted with EtOAc (50 mL) and the layers separated. The organic layer was dried over MgSO₄, filtered, concentrated and purified by silica gel column chromatography (eluent: EtOAc/hexanes) to provide desired product.

**2-[5-(bromomethyl)-2-methyl-pyrazol-3-yl]acetonitrile (I-79):** 2-[5-(Hydroxymethyl)-2-methyl-pyrazol-3-yl]acetonitrile (100 mg, 0.662 mmol) was taken up in dichloromethane (2.65 mL) and triphenylphosphine (0.208 g, 0.794 mmol) was added followed by the addition of carbontetrabromide (0.263 g, 0.794 mmol). The reaction mixture was left to stir at rt for 5 minutes at which point the reaction was quenched by the addition of water (5 mL), diluted with EtOAc (25 mL) and the layers separated. The organic layer was dried over MgSO₄, filtered, concentrated and purified by silica gel column chromatography (eluent: EtOAc/hexanes) to provide **I-79.** ES/MS: 214.0, 216.0 (M+H⁺).

### Preparation of Intermediates I-80 and I-81 (Method 1)

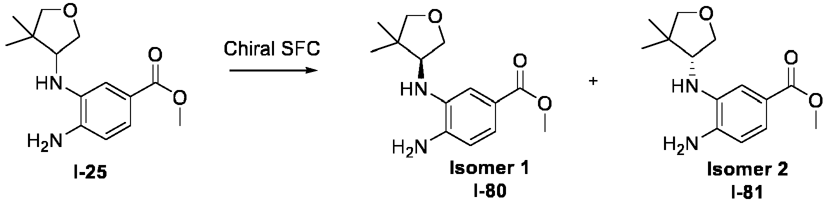

**Methyl 4-amino-3-((4,4-dimethyltetrahydrofuran-3-yl)amino)benzoate (I-80, I-81):** Methyl 4-amino-3-((4,4-dimethyltetrahydrofuran-3-yl)amino)benzoate as a mixture of 2 stereoisomers were separated by chiral SFC (SFC IB column with EtOH cosolvent) to give two distinct stereoisomers.

**Methyl (S)-4-amino-3-((4,4-dimethyltetrahydrofuran-3-yl)amino)benzoate isomer 1 (I-80):** Isolated as the earlier eluting of two isomers by chiral SFC (4.6 × 100 mm 5µm IB column, 10% EtOH in CO₂). ES/MS: 265.2 (M+H⁺).

**Methyl (R)-4-amino-3-((4,4-dimethyltetrahydrofuran-3-yl)amino)benzoate, isomer 2 (I-81):** Isolated as the later eluting of two isomers by chiral SFC (4.6 × 100 mm 5µm IB column, 10% EtOHin CO₂). ES/MS: 265.2 (M+H⁺).

### Preparation of Intermediate I-80 (Method 2)

**Methyl 4-amino-3-[[(3S)-4,4-dimethyltetrahydrofuran-3-yl]amino]benzoate isomer 2 (I-80):** A solution of methyl 3-[[(3S)-4,4-dimethyltetrahydrofuran-3-yl]amino]-4-nitrobenzoate (Intermediate **I-100,** 17.8 g, 60.5 mmol) in EtOAc (380 mL) was degassed with argon then vacuum 3x. Palladium on carbon (10.0 %, 6.07 g, 5.70 mmol) was added. The mixture was degassed with argon then vacuum 3 times and stirred at rt under an atmosphere of hydrogen until completion. The suspension was filtered over a Celite plug and rinsed with EtOAc. The mixture was concentrated to yield methyl 4-amino-3-[[(3S)-4,4-dimethyltetrahydrofuran-3-yl]amino]benzoate, which was carried forward to subsequent steps without further purification. ES/MS: 265.0 (M+H+). 1H NMR (400 MHz, Chloroform-d) δ 7.49 (dd, J = 8.0, 1.8 Hz, 1H), 7.35 (d, J = 1.8 Hz, 1H), 6.73 (d, J = 8.1 Hz, 1H), 4.36 (dd, J = 9.1, 6.4 Hz, 1H), 3.89 (s, 3H), 3.76 (t, J = 5.9 Hz, 1H), 3.72 - 3.63 (m, 2H), 3.63 - 3.59 (m, 1H), 1.22 (s, 3H), 1.15 (s, 3H).

### Preparation of Intermediates I-82 and I-83 (Method 1)

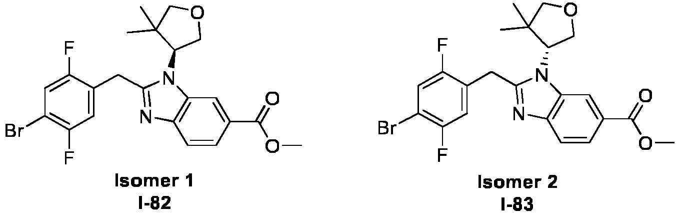

**Methyl 2-(4-bromo-2,5-difluorobenzyl)-1-(4,4-dimethyltetrahydrofuran-3-yl)-1H-benzo[d]imidazole-6-carboxylate (I-82, I-83): I-82** and **I-83** were prepared separately in a manner as described for Intermediate **I-8** substituting **I-80** (for Intermediate **I-82)** and **I-81** (for Intermediate **I-83)** for **I-4.**

**Methyl (S)-2-(4-bromo-2,5-difluorobenzyl)-1-(4,4-dimethyltetrahydrofuran-3-yl)-1H-benzo[d]imidazole-6-carboxylate isomer 1 (I-82):** ES/MS: 479.0, 481.0 (M+H⁺).

**Methyl (R)-2-(4-bromo-2,5-difluorobenzyl)-1-(4,4-dimethyltetrahydrofuran-3-yl)-1H-benzo[d]imidazole-6-carboxylate isomer 2 (I-83):** ES/MS: 479.0, 481.0 (M+H⁺).

### Preparation of Intermediate I-82 (Method 2):

**Methyl 2-[(4-bromo-2,5-difluoro-phenyl)methyl]-3-[(3S)-4,4-dimethyltetrahydrofuran-3-yl]benzimidazole-5-carboxylate (Intermediate I-82):** To a solution of 2-(4-bromo-2,5-difluorophenyl)acetic acid (3301 mg, 13.2 mmol), methyl 4-amino-3-[[(3S)-4,4-dimethyltetrahydrofuran-3-yl]amino]benzoate (Intermediate **I-80**, 3.16 g, 12.0 mmol),and o-(7-Azabenzotriazol-1-yl)-N,N,N',N'-tetramethyluronium hexafluorophosphate (6360 mg, 16.7 mmol) in DMF (20 mL) and CH3CN (20 mL), was added N,N-Diisopropylethylamine (10.2 mL, 58.4 mmol). The solution was stirred at rt overnight. Then to the mixture was added 0.2 eq of 2-(4-bromo-2,5-difluoro-phenyl)acetic acid (600 mg, 2.39 mmol) and o-(7-Azabenzotriazol-1-yl)-N,N,N',N'-tetramethyluronium hexafluorophosphate (930 mg, 2.39 mmol) and continued stirring until complete conversion to product. The crude reaction mixture was diluted with 200 mL EtOAc and washed with saturated NH₄Cl (200 mL), 5% LiCl (100 mL), saturated NaHCO₃ (100 mL), and brine (100 mL). The organic extract was dried over sodium sulfate to give methyl 4-[[2-(4-bromo-2,5-difluoro-phenyl)acetyl]amino]-3-[[(3S)-4,4-dimethyltetrahydrofuran-3-yl]amino]benzoate.

A solution of methyl 4-[[2-(4-bromo-2,5-difluoro-phenyl)acetyl]amino]-3-[[(3S)-4,4-dimethyltetrahydrofuran-3-yl]amino]benzoate (5.96 g, 12.0 mmol) in AcOH (60 mL) was heated to 180 °C for 90 minutes in a microwave reactor. The mixture was concentrated, then diluted with EtOAc and washed with saturated NaHCO₃, and brine. The mixture was dried over sodium sulfate and purified by silica gel column chromatography (eluent: EtOAc/hexanes) to afford methyl 2-[(4-bromo-2,5-difluoro-phenyl)methyl]-3-[(3S)-4,4-dimethyltetrahydrofuran-3-yl]benzimidazole-5-carboxylate (Intermediate **I-82**). ES/MS: 478.6, 480.6 (M+H⁺). 1H NMR (400 MHz, Chloroform-d) δ 8.55 (s, 1H), 8.02 (dd, J = 8.5, 1.5 Hz, 1H), 7.78 (d, J = 8.5 Hz, 1H), 7.37 (dd, J = 8.7, 5.6 Hz, 1H), 7.12 (dd, J = 8.4, 6.3 Hz, 1H), 4.59 (d, J = 10.5 Hz, 2H), 4.40 (dd, J = 11.1, 7.3 Hz, 1H), 4.31 (s, 2H), 3.97 (s, 4H), 3.80 (d, J = 8.8 Hz, 1H), 1.35 (s, 3H), 0.67 (s, 3H).

### Preparation of Intermediate I-84

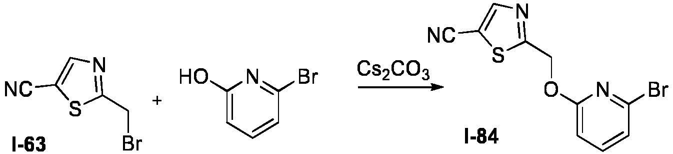

**2-[(6-bromo-2-pyridyl)oxymethyl]thiazole-5-carbonitrile (I-84):** To a solution of 6-bromopyridin-2-ol (500 mg, 2.9 mmol) in acetonitrile (10 mL) was added 2-(bromomethyl)thiazole-5-carbonitrile **(I-63)** (584 mg, 2.9 mmol) and cesium carbonate (1.34 g, 4.1 mmol) and the resultant mixture stirred at 65 °C for 1 hour. Upon completion the reaction mixture was filtered through celite, concentrated and purified by silica gel column chromatography (eluent: EtOAc/hexanes) to provide **I-84**. ES/MS: 296.0, 298.0 (M+H⁺).

### Preparation of Intermediate I-85

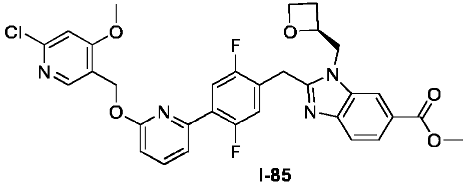

**Methyl 2-[[4-[6-[(6-chloro-4-methoxy-3-pyridyl)methoxy]-2-pyridyl]-2,5-difluorophenyl]methyl]-3-[[(2S)-oxetan-2-yl]methyl]benzimidazole-5-carboxylate (I-85):** Methyl 2-[[4-[6-[(6-chloro-4-methoxy-3-pyridyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-23** substituting 5-(bromomethyl)-2-chloro-4-methoxypyridine for 5-(bromomethyl)-2-chloro-4-fluoropyridine. ES/MS: 621.2 (M+H⁺).

### Preparation of Intermediate I-86

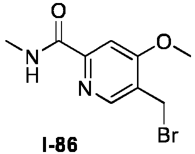

**5-(bromomethyl)-4-methoxy-N-methylpicolinamide (I-86):** 5-(bromomethyl)-4-methoxy-N-methylpicolinamide was prepared in a manner as described for Intermediate **I-53** substituting methylamine HCl for 1-aminocyclopropane-1-carbonitrile. ES/MS: 259.2, 261.2 (M+H⁺).

### Preparation of Intermediate I-87

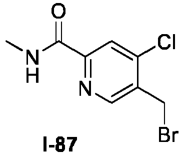

**5-(bromomethyl)-4-chloro-N-methylpicolinamide (I-87):** 5-(bromomethyl)-4-chloro-N-methylpicolinamide was prepared in a manner as described for Intermediate **I-53** substituting methylamine HCl for 1-aminocyclopropane-1-carbonitrile and 4-chloro-5-methylpicolinic acid for 4-methoxy-5-methylpicolinic acid. ES/MS: 263.0, 265.0 (M+H⁺).

### Preparation of Intermediate I-88

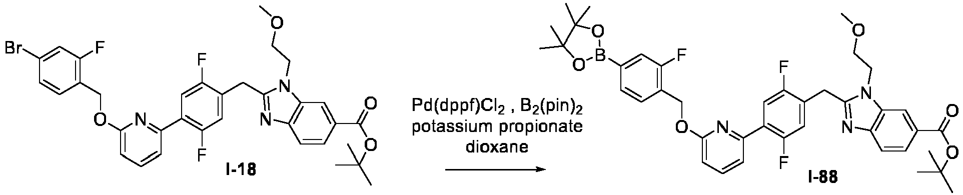

**Tert-butyl 2-[[2,5-difluoro-4-[6-[[2-fluoro-4-(4,4,5,5-tetramethyl-1,3,2-dioxaborolan-2-yl)phenyl]methoxy]-2-pyridyl]phenyl]methyl]-3-(2-methoxyethyl)benzimidazole-5-carboxylate (I-88):** To a solution of tert-butyl 2-[[4-[6-[(4-bromo-2-fluoro-phenyl)methoxy]-2-pyridyl]-2, 5-difluoro-phenyl]methyl]-3-(2-methoxyethyl)benzimidazole-5-carboxylate (300 mg, 0.44 mmol) in 1,4-dioxane (20 mL) was added Bis(pinacolato)diboron (179 mg, 0.70 mmol), Pd(dppf)Cl₂ (33 mg, 0.044 mmol), and potassium propionate (148 mg, 1.3 mmol). Argon was bubbled through the reaction mixture for 1 minute after which the reaction vessel was sealed and heated to 110 °C for 45 minutes in a microwave reactor. Upon completion the reaction mixture was concentrated directly and purified by silica gel column chromatography (eluent: EtOAc/hexanes) to provide **I-88**. ES/MS: 730.8 (M+H⁺).

### Preparation of Intermediate I-89

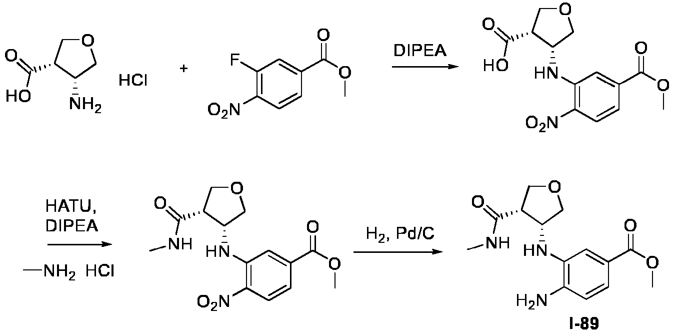

**(3S,4R)-4-(5-methoxycarbonyl-2-nitro-anilino)tetrahydrofuran-3-carboxylic acid:** To a solution of methyl 3-fluoro-4-nitro-benzoate (700 mg, 3.52 mmol) and (3S,4R)-4-aminotetrahydrofuran-3-carboxylic acid;hydrochloride (648 mg, 17.6 mmol) in DMF (2.5 mL) and THF (5 mL) was added diisopropylethylamine (3.1 mL, 17.6 mmol) and the resultant solution heated to 70 °C for 3 days. Upon completion, the reaction mixture was diluted with EtOAc (50 mL), washed with water (10 mL), brine (10 mL), dried over MgSO₄, filtered, concentrated to give the desired product which was used without further purification. ES/MS: 311.2 (M+H⁺).

**Methyl 3-[[(3R,4S)-4-(methylcarbamoyl)tetrahydrofuran-3-yl]amino]-4-nitrobenzoate:** (3 S,4R)-4-(5-methoxycarbonyl-2-nitro-anilino)tetrahydrofuran-3-carboxylic acid (370 mg, 0.00119 mol), 1-hydroxybenzotriazole hydrate (0.192 g, 0.00125 mol), and methylamine (2000 mmol/L in THF, 1.19 mL, 0.00239 mol) were taken up in tetrahydrofuran (8.00 mL) and triethylamine (0.151 g, 0.00149 mol) was added followed by 1-(3-dimethylaminopropyl)-3-ethylcarbodiimide hydrochloride (0.251 g, 0.00131 mol). The mixture was stirred at rt overnight. Upon completion the mixture was diluted with EtOAc (50 mL), washed with water (10 mL), brine (10 mL), dried over MgSO₄, filtered, and concentrated to give the desired product which was used without further purification. ES/MS: 324.2 (M+H⁺).

**Methyl 4-amino-3-[[(3R,4S)-4-(methylcarbamoyl)tetrahydrofuran-3-yl]amino]benzoate (I-89):** To a solution of methyl 3-[[(3R,4S)-4-(methylcarbamoyl)tetrahydrofuran-3-yl]amino]-4-nitro-benzoate (168 mg, 0.52 mmol) in EtOH (2 mL) and THF (1 mL) was added palladium on carbon (10%, 111 mg, 0.10 mmol). The reaction mixture was then purged with H₂ gas and stirred under 1 atm of H₂ for 1 hr. Upon completion the reaction mixture was filtered through celite, concentrated and the crude residue, **I-89**, was used without further purification. ES/MS: 294.2 (M+H⁺).

### Preparation of Intermediate I-90

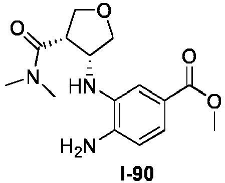

**Methyl 4-amino-3-(((3R,4S)-4-(dimethylcarbamoyl)tetrahydrofuran-3-yl)amino)benzoate (I-90):** Methyl 4-amino-3-(((3R,4S)-4-(dimethylcarbamoyl)tetrahydrofuran-3-yl)amino)benzoate was prepared in a manner as described for Intermediate **I-89** substituting dimethylamine for methylamine. ES/MS: 308.2 (M+H⁺).

### Preparation of Intermediate I-91

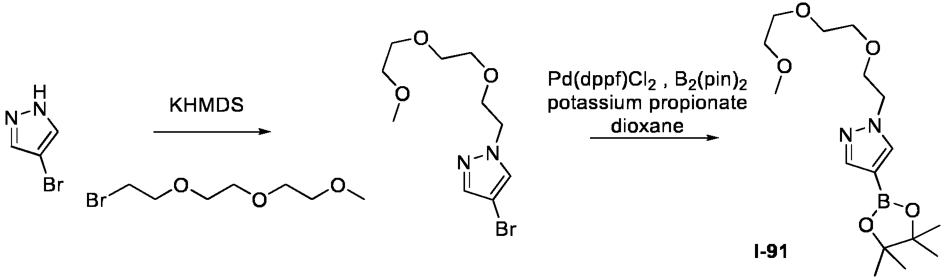

**4-bromo-1-[2-[2-(2-methoxyethoxy)ethoxy]ethyl]pyrazole:** To a solution of 4-bromo-1H-pyrazole (100 mg, 0.68 mmol) in 2-Me tetrahydrofuran (2 mL) was added Potassium Bis(trimethylsilyl)amide (204 mg, 1.0 mmol) and 1-[2-(2-bromoethoxy)ethoxy]-2-methoxyethane (309 mg, 1.4 mmol) and heated to 50 °C for 2 hours. Upon completion, the reaction mixture was diluted with EtOAc (25 mL), washed with water (5 mL), brine (5 mL), dried over MgSO₄, filtered and concentrated to give the desired product which was used without further purification. ES/MS: 293.3 (M+H⁺).

**1-[2-[2-(2-methoxyethoxy)ethoxy]ethyl]-4-(4,4,5,5-tetramethyl-1,3,2-dioxaborolan-2-yl)pyrazole (I-91):** 4-bromo-1-[2-[2-(2-methoxyethoxy)ethoxy]ethyl]pyrazole was converted to 1-[2-[2-(2-methoxyethoxy)ethoxy]ethyl]-4-(4,4,5,5-tetramethyl-1,3,2-dioxaborolan-2-yl)pyrazole **(I-91)** in a manner as described for intermediate **I-88**. ES/MS: 341.1 (M+H⁺).

### Preparation of Intermediate I-92

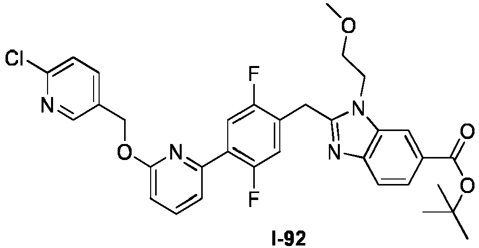

**Tert-butyl 2-(4-(6-((6-chloropyridin-3-yl)methoxy)pyridin-2-yl)-2,5-difluorobenzyl)-1-(2-methoxyethyl)-1H-benzo[d]imidazole-6- (I-92):** Tert-butyl 2-(4-(6-((6-chloropyridin-3-yl)methoxy)pyridin-2-yl)-2,5-difluorobenzyl)-1-(2-methoxyethyl)-1H-benzo[d]imidazole-6- was prepared in manner as described for Intermediate **I-18** substituting 5-(bromomethyl)-2-chloropyridine for 4-bromo-1-(bromomethyl)-2-fluoro-benzene. ES/MS: 621.2 (M+H⁺).

### Preparation of Intermediate I-93

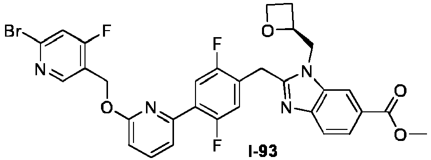

**Methyl (S)-2-(4-(6-((6-bromo-4-fluoropyridin-3-yl)methoxy)pyridin-2-yl)-2,5-difluorobenzyl)-1-(oxetan-2-ylmethyl)-1H-benzo[d]imidazole-6-carboxylate (I-93):** Methyl (S)-2-(4-(6-((6-bromo-4-fluoropyridin-3-yl)methoxy)pyridin-2-yl)-2,5-difluorobenzyl)-1-(oxetan-2-ylmethyl)-1H-benzo[d]imidazole-6-carboxylate was prepared in manner as described for Intermediate **I-19** substituting 2-bromo-5-(bromomethyl)-4-fluoropyridine for 4-bromo-1-(bromomethyl)-2-fluoro-benzene ES/MS: 654.0, 656.0 (M+H⁺).

### Preparation of Intermediate I-94

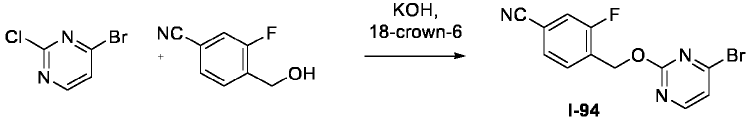

**4-[(4-bromopyrimidin-2-yl)oxymethyl]-3-fluoro-benzonitrile (I-94):** 4-bromo-2-chloro-pyrimidine (1.7 g, 8.8 mmol), 3-fluoro-4-(hydroxymethyl)benzonitrile (1.46 g, 9.7 mmol), potassium hydroxide (542 mg, 9.7 mmol), 18-crown-6 (116 mg, 0.44 mmol), and toluene (20 mL) were combined and heated to 110 °C for 2 hours. Upon completion the reaction mixture was diluted with EtOAc (100 mL) washed with water (25 mL), washed with brine (25 mL), dried over MgSO₄, filtered, concentrated and purified by silica gel chromatography (eluent: EtOAc/hexanes) to give **I-94.** ES/MS: 309.2 (M+H⁺).

### Preparation of Intermediate I-95

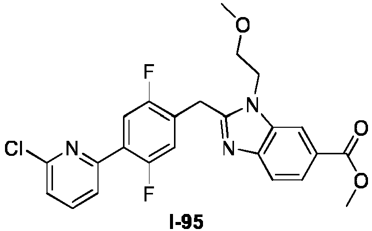

**Methyl 2-(4-(6-chloropyridin-2-yl)-2,5-difluorobenzyl)-1-(2-methoxyethyl)-1H-benzo[d]imidazole-6-carboxylate (I-95):** Methyl (S)-2-(4-(6-chloropyridin-2-yl)-2,5-difluorobenzyl)-1-(oxetan-2-ylmethyl)-1H-benzo[d]imidazole-6-carboxylate was prepared in a manner as described for Intermediate **I-12** substituting **I-1** for **I-6**. ES/MS: 472.8 (M+H⁺).

### Preparation of Intermediate I-96

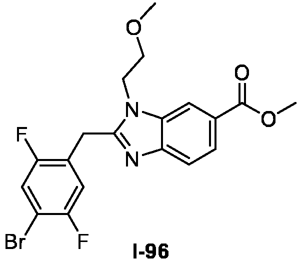

**Methyl 2-(4-bromo-2,5-difluorobenzyl)-1-(2-methoxyethyl)-1H-benzo[d]imidazole-6-carboxylate (I-96).** Methyl 2-(2,5-difluoro-4-(4,4,5,5-tetramethyl-1,3,2-dioxaborolan-2-yl)benzyl)-1-(2-methoxyethyl)-1H-benzo[d]imidazole-6-carboxylate was prepared in a manner as described for Intermediate **I-2** substituting 2-(4-bromo-2,5-difluorophenyl)acetic acid for 2-(4-bromo-2-fluorophenyl)acetic acid. ES/MS: 439.8 (M+H⁺).

### Preparation of Intermediate I-97:

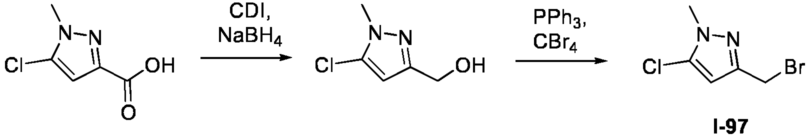

**(5-chloro-1-methyl-pyrazol-3-yl)methanol:** 5-chloro-1-methyl-pyrazole-3-carboxylic acid (700 mg, 4.36 mmol) was taken up in THF (20.0 mL) and 1,1'-Carbonyldiimidazole (1.41 g, 8.72 mmol) was added. The mixture was stirred at rt for 2 hours. Upon completion, the mixture was cooled to 0 °C and a solution of sodium borohydride (0.825 g, 21.8 mmol) in water (3.30 mL) was added slowly, after which the mixture was allowed to warm to rt over 40 minutes. Upon completion methanol (5 mL) was added, the reaction mixture was concentrated directly and purified by flash chromatography (Eluent: EtOAc/hexane) to give the desired product.

**3-(bromomethyl)-5-chloro-1-methyl-pyrazole (I-97):** (5-chloro-1-methyl-pyrazol-3-yl)methanol (550 mg, 3.75 mmol) was taken up in DCM (25.0 mL) and (4-diphenylphosphanylphenyl) polymer bound (78.7 %, 1.50 g, 0.00450 mol) was added. The reaction mixture was cooled to 0 °C and carbon tetrabromide (1.49 g, 0.00450 mol) was added as a single portion. The mixture was stirred for 16hr at rt. Upon completion mixture was filtered, concentrated, and purified by flash chromatography (Eluent: EtOAc/hexane) to give **I-97.** ES/MS: 209.0, 211.0 (M+H⁺).

### The following intermediates were prepared in a manner as described for intermediate I-97

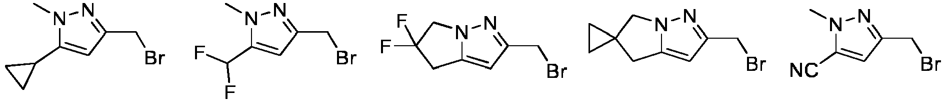

### Preparation of Intermediate I-98

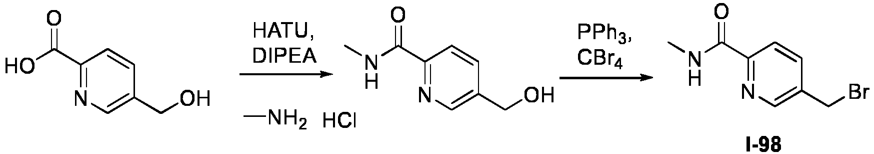

**5-(hydroxymethyl)-N-methyl-pyridine-2-carboxamide:** 5-(hydroxymethyl)pyridine-2-carboxylic acid (400 mg, 2.61 mmol), methanamine hydrochloride (194 mg, 2.87 mmol), and o-(7-Azabenzotriazol-1-yl)-N,N,N',N'-tetramethyluronium hexafluorophosphate (1192 mg, 3.14 mmol) were taken up in DMF (5.00 mL), N,N-Diisopropylethylamine (2.27 mL, 13.1 mmol) was added after which the reaction mixture was stirred for 30 min. Upon completion the reaction mixture was diluted with EtOAc (50 mL) washed with water (10 mL), washed with brine (10 mL), dried over MgSO₄, filtered, concentrated and purified by silica gel chromatography (eluent: MeOH/EtOAc/hexanes) to give desired product. ES/MS: 167.2 (M+H⁺).
**5-(bromomethyl)-N-methyl-pyridine-2-carboxamide (I-98):** 5-(hydroxymethyl)-N-methylpyridine-2-carboxamide (106 mg, 0.638 mmol) and triphenylphosphine (0.167 g, 0.638 mmol) were taken up in dichloromethane (2.60 mL) and carbon tetrabromide (0.212 g, 0.638 mmol). was added. The reaction mixture was stirred for 15 min. Upon completion the reaction mixture was filtered, concentrated, and purified by flash chromatography (Eluent: EtOAc/hexane) to give **I-98.** ES/MS: 229.0 (M+H⁺).

### The following intermediate was prepared in a manner as described for intermediate I-98

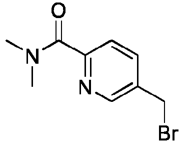

### Preparation of Intermediate I-99

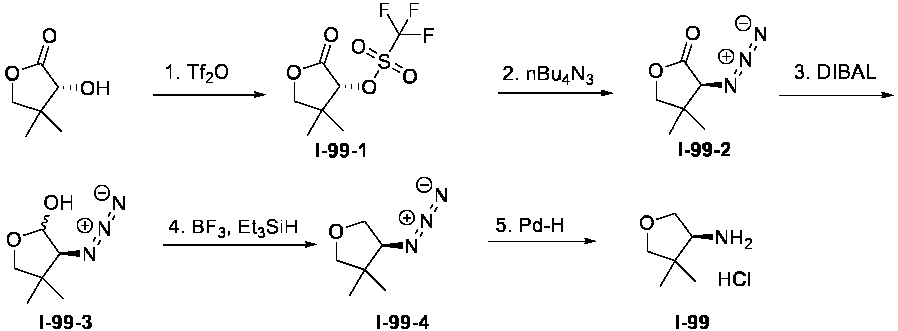

**(R)-4,4-dimethyl-2-oxotetrahydrofuran-3-yl trifluoromethanesulfonate (I-99-1):** A round-bottom flask was charged with (D)-(-)-pantolactone (4.20 g, 32.3 mmol, 1.0 equivalent). Anhydrous dichloromethane (20 mL) and pyridine (3.39 mL, 42.0 mmol, 1.30 equivalent) were added, and the resulting solution was cooled to -78 °C. A solution of trifluoromethanesulfonic anhydride (5.96 mL, 35.4 mmol, 1.10 equivalent) in dichloromethane (100 mL) was added slowly to the reaction mixture via an addition funnel while stirring at -78 °C. Following the addition, the mixture was maintained with stirring at -78 °C for 30 minutes before the cooling bath was removed. The mixture was maintained with stirring at rt for an additional 3 hours. The solvent was removed in vacuo, and the residue was dissolved in ethyl ether (200 mL) and washed with 10 % aqueous sodium bicarbonate (100 mL), and brine (100 mL). The organic layer was dried over anhydrous sodium sulfate, filtered, and concentrated in vacuo to afford (R)-4,4-dimethyl-2-oxotetrahydrofuran-3-yl trifluoromethanesulfonate. 1H NMR (500 MHz, CDCl3) δ = 5.10 (s, 1 H), 4.13 (d, J = 9.5 Hz, 1 H), 4.07 (d, J = 9.5 Hz, 1 H), 1.28 (s, 3 H), 1.18 (s, 3 H).

**(S)-3-azido-4,4-dimethyldihydrofuran-2(3H)-one (I-99-2):** A round-bottom flask was charged with tetrabutylammonium azide (10.5 g, 36.8 mmol, 1.15 equivalent). Anhydrous toluene (50 mL) was added, and the resulting solution was cooled to 0 °C. A solution of (R)-4,4-dimethyl-2-oxotetrahydrofuran-3-yl trifluoromethanesulfonate (8.40 g, 32 mmol, 1.0 equivalent) in toluene (50 mL) was added slowly via an addition funnel at 0 °C. The mixture was maintained at 0 °C for 30 minutes before the cooling bath was removed and the mixture was stirred at rt for 4 hours. The reaction mixture was diluted with ethyl ether (200 mL) and washed with 10 % aqueous sodium bicarbonate (200 mL), and brine (100 mL). The organic layer was dried over anhydrous sodium sulfate, filtered, and concentrated in vacuo to afford (S)-3-azido-4,4-dimethyldihydrofuran-2(3H)-one. The crude material was carried forward immediately to the next step without purification.

**3-azido-4,4-dimethyltetrahydrofuran-2-ol (I-99-3):** 3-azido-4,4-dimethyldihydrofuran-2(3H)-one (4.16 g, 26.8 mmol) was taken in dichloromethane (40 mL), cooled to -78 °C then diisobutylaluminum hydride (1.0 M in toluene) (32.2 mL, 32.2 mmol, 1.2 equivalent) was added slowly followed at same temperature. The mixture was stirred at -78 °C for 2 hrs. until no starting material remained. The reaction was quenched by adding saturated solution of potassium sodium tartarate (100 mL). The mixture was extracted with dichloromethane (3 X 50 mL). The organic extract was dried with anhydrous Na₂SO₄. and concentrated under reduced pressure. The crude compound was purified by column chromatography (silica gel) to yield 3-azido-4,4-dimethyltetrahydrofuran-2-ol. 1H NMR (400 MHz, CDCl₃) *δ* = 5.58-5.29 (m, 1 H), 3.93-3.76 (m, 2 H), 3.66-3.50 (m, 2 H), 1.19-1.08 (m, 6 H)

**4-azido-3,3-dimethyltetrahydrofuran (I-99-4):_**3-azido-4,4-dimethy)tetrahydrofuran-2-ol was taken in dichloromethane (80 mL), cooled to -78 °C then BF₃·Et₂O (3.6 mL, 28.7 mmol, 1.5 equivalent) was added slowly followed by addition of triethylsilane (6.1 mL, 2.0 mmol) at same temperature. The mixture was stirred at 0 °C for 4 h until no starting material remained. Then water (100 mL) was added to reaction mixture. The resulting phases were separated, then the aqueous phase was extracted with dichloromethane (2 X 50 mL). The organic extract was dried with anhydrous Na₂SO₄ and concentrated under reduced pressure. The crude compound was purified by column chromatography (silica gel) to yield (S)-4-azido-3,3-dimethyltetrahydrofuran. 1HNMR (400 MHz, CDCl3) = 4.17 (dd, J = 6.1, 9.8 Hz, 1 H), 3.77 (dd, J = 3.9, 9.8 Hz, 1 H), 3.65 (dd, J = 3.9, 6.1 Hz, 1 H), 3.58-3.50 (m, 2 H), 1.13 (d, J = 6.1 Hz, 6 H).

**4,4-dimethyltetrahydrofuran-3-amine (I-99):** 4-azido-3,3-dimethyltetrahydrofuran (1.89 g, 13.4 mmol) dissolved in ethyl acetate (50 mL) was added 10% palladium on carbon (2.14 g, 2.0 mmol, 0.15 equivalent). The mixture was stirred at rt for 16 h under 1 atm of hydrogen before filtering through a plug of celite. The solution was acidified 4 M HCl in methanol (5.0 mL) before concentrating in vacuo to afford (S)-4,4-dimethyltetrahydrofuran-3-amine as the hydrochloride salt. 1H NMR (400 MHz, CDCl3) = 4.09-4.05 (m, 1H), 3.72-3.68 (m, 1H), 3.59-3.56 (m, 1H), 3.44-3.42 (m, 1H), 3.35 (m, 1H), 1.07 (s, 6H).

### Preparation of Intermediate I-100

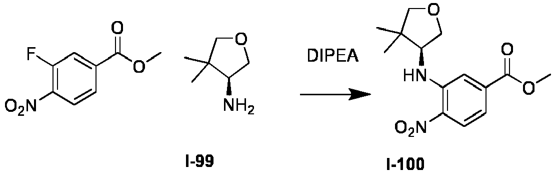

**Methyl 3-[[(3S)-4,4-dimethyltetrahydrofuran-3-yl]amino]-4-nitrobenzoate (I-100):** To a suspension of methyl 3-fluoro-4-nitrobenzoate (15.0 g, 75.3 mmol) and (3S)-4,4-dimethyltetrahydrofuran-3-amine hydrochloride (Intermediate **I-99**, 12.6 g, 82.9 mmol) in THF (100 mL) and DMF (50 mL), was added N,N-diisopropylethylamine (65.6 mL, 377 mmol). The resulting solution was heated at 80 °C overnight. The crude reaction mixture was diluted with EtOAc (300 mL), washed with 5% LiCl (250 mL) and brine (250 mL). The organic extract was dried over sodium sulfate and purified by silica gel column chromatography (eluent: EtOAc/hexanes) to afford methyl 3-[[(3S)-4,4-dimethyltetrahydrofuran-3-yl]amino]-4-nitrobenzoate. 1H NMR (400 MHz, Chloroform-d) δ 8.24 (d, J = 8.8 Hz, 2H), 7.57 (d, J = 1.6 Hz, 1H), 7.27 (dd, J = 8.9, 1.7 Hz, 1H), 4.41 (dd, J = 9.2, 6.8 Hz, 1H), 4.02 (s, 1H), 3.97 (s, 3H), 3.76 - 3.64 (m, 3H), 1.26 (s, 3H), 1.18 (s, 3H). ES/MS: 295.0 (M+H+).

### Preparation of Intermediate I-101

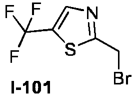

**2-(bromomethyl)-5-(trifluoromethyl)thiazole (I-101).** To [5-(trifluoromethyl)thiazol-2-yl]methanol (100 mg, 0.546 mmol) under argon was added DCM (5.5 mL). The mixture was cooled to 0 °C. Then triphenylphosphine (150 mg, 0.573 mmol) was added, followed by carbon tetrabromide (190 mg, 0.573 mmol). The cooling bath was removed, and the mixture was stirred at 20 °C for 30 min. The mixture was purified directly by silica gel flash column chromatography (EtOAc in hexane gradient) to yield 2-(bromomethyl)-5-(trifluoromethyl)thiazole (Intermediate **I-101**). 1H NMR (400 MHz, Chloroform-d) δ 8.04 (d, J = 1.2 Hz, 1H), 4.72 (s, 2H).

### Preparation of Intermediate I-102

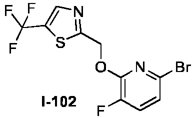

**2-[(6-bromo-3-fluoro-2-pyridyl)oxymethyl]-5-(trifluoromethyl)thiazole (Intermediate I-102).** 6-bromo-2-chloro-3-fluoro-pyridine (3.00 g, 14.3 mmol) was taken up in acetonitrile (20.0 mL), then [5-(trifluoromethyl)thiazol-2-yl]methanol (1.52 mL, 14.7 mmol) and cesium carbonate (9.29 g, 28.5 mmol) were added. The mixture was heated to 60 °C until all starting material was consumed as determined by LCMS. The mixture was filtered to remove Cs₂CO₃, washing with EtOAc, and concentrated in vacuo. Silica gel flash column chromatography (EtOAc in hexane gradient) yielded 2-[(6-bromo-3-fluoro-2-pyridyl)oxymethyl]-5-(trifluoromethyl)thiazole (Intermediate **I-102).** ES/MS m/z: 358 (M+H⁺).

### Preparation of Intermediate I-103

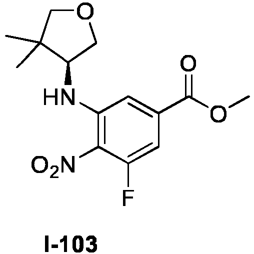

**Methyl (S)-3-((4,4-dimethyltetrahydrofuran-3-yl)amino)-5-fluoro-4-nitrobenzoate (Intermediate I-103):** To a solution of methyl 3,5-difluoro-4-nitro-benzoate (2.21 g, 10.2 mmol) and (S)-4,4-dimethyltetrahydrofuran-3-amine hydrochloride (Intermediate **I-99**, 1.70 g, 11.2 mmol) in tetrahydrofuran (12.5 mL) and N,N-dimethylformamide (6.0 mL) was added N,N-diisopropylethylamine (8.86 mL, 50.9 mmol). The mixture was stirred at 70 °C overnight before being cooled to rt. The mixture was diluted with water, extracted with EtOAc, washed with brine, dried over sodium sulfate, filtered, and concentrated to yield the title compound, which was carried forward to subsequent steps without further purification. ES/MS m/z: 313.3 (M+H+).

### Preparation of Intermediate I-104

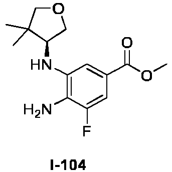

**Methyl (S)-4-amino-3-((4,4-dimethyltetrahydrofuran-3-yl)amino)-5-fluorobenzoate (Intermediate I-104):** Methyl (S)-3-((4,4-dimethyltetrahydrofuran-3-yl)amino)-5-fluoro-4-nitrobenzoate (Intermediate **I-103**, 2.88 g, 10.2 mmol) was dissolved in EtOAc, and put under argon. To this mixture was added 10% palladium on carbon (1.08 g, 1.02 mmol), and then the mixture was placed under hydrogen gas. The mixture was stirred overnight, then the mixture filtered through celite, and concentrated in vacuo. Purification by silica gel flash column chromatography (EtOAc / Hexane gradient) yielded methyl (S)-4-amino-3-((4,4-dimethyltetrahydrofuran-3-yl)amino)-5-fluorobenzoate (Intermediate **I-104).** ES/MS m/z: 283.2 (M + H)⁺.

### Preparation of Intermediate I-105

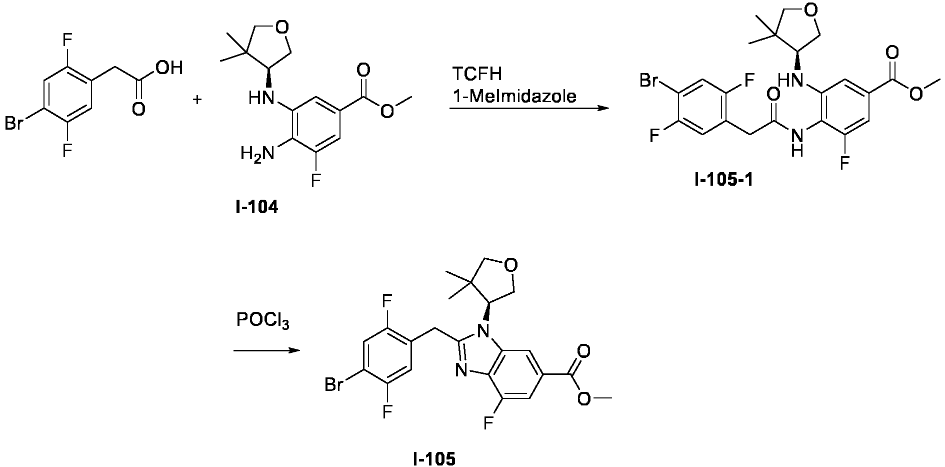

**Methyl (S)-4-(2-(4-bromo-2,5-difluorophenyl)acetamido)-3-((4,4-dimethyltetrahydrofuran-3-yl)amino)-5-fluorobenzoate (I-105-1)**: To a solution of methyl (S)-4-amino-3-((4,4-dimethyltetrahydrofuran-3-yl)amino)-5-fluorobenzoate (Intermediate **I-104**, 2000 mg, 7.08 mmol) and 2-(4-bromo-2,5-difluoro-phenyl)acetic acid (1867 mg, 7.44 mmol) in MeCN (10.0 mL) and cooled to 0 °C was added 1-methylimidazole (2.91 g, 2.82 mL, 35.4 mmol) followed by *N,N,N',N'*-Tetramethylchloroformamidinium Hexafluorophosphate (2.39 g, 8.50 mmol). The reaction mixture was warmed to rt and stirred for 30 minutes. The mixture was concentrated *in vacuo* to yield methyl (S)-4-(2-(4-bromo-2,5-difluorophenyl)acetamido)-3-((4,4-dimethyltetrahydrofuran-3-yl)amino)-5-fluorobenzoate **(I-105-1),** which was carried forward to the next step without further purification.

**Methyl 2- [(4-bromo-2,5-difluoro-phenyl)methyl] -3- [(3S)-4,4-dimethyltetrahydrofuran-3-yl]-7-fluorobenzimidazole-5-carboxylate (Intermediate I-105):** To methyl (S)-4-(2-(4-bromo-2,5-difluorophenyl)acetamido)-3-((4,4-dimethyltetrahydrofuran-3-yl)amino)-5-fluorobenzoate (**I-105-1**, 2.2 g, 4.3 mmol) in 21 mL of 1,2-dichloroethane was added phosphoryl trichloride (2.6 g, 17 mmol, 1.6 mL). The solution was heated to 80 °C for 24 h, then cooled to rt. An aliquot of 20 mL of water was added and stirring for 1 h, then aqueous sodium hydroxide (26 mL, 51 mmol, 2 M) was added. The mixture was diluted with DCM, layers separated, and the organic phase washed with brine, dried with MgSO₄, filtered, and concentrated. Purification by silica chromatography (EtOAc/hexane gradient) yielded methyl 2-[(4-bromo-2,5-difluorophenyl)methyl]-3-[(3S)-4,4-dimethyltetrahydrofuran-3-yl]-7-fluorobenzimidazole-5-carboxylate (Intermediate **I-105**). ES/MS m/z: 497.0 (M+H)⁺.

### Preparation of Intermediate I-106.

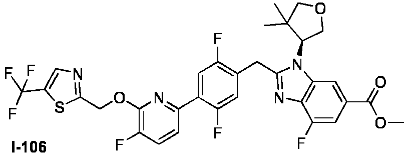

**Methyl 2-[[2,5-difluoro-4-[5-fluoro-6-[[5-(trifluoromethyl)thiazol-2-yl]methoxy]-2-pyridyl]phenyl]methyl]-3-[(3S)-4,4-dimethyltetrahydrofuran-3-yl]-7-fluoro-benzimidazole-5-carboxylate (Intermediate I-106).** To a vial was added methyl (S)-2-(4-bromo-2,5-difluorobenzyl)-1-(4,4-dimethyltetrahydrofuran-3-yl)-1H-benzo[d]imidazole-6-carboxylate (Intermediate **I-105,** 50 mg, 0.101 mmol), [1,1'-Bis(diphenylphosphino)ferrocene] dichloropalladium(II) (7.6 mg, 0.010 mmol), Bis(pinacolato)diboron (28 mg, 0.111 mmol) and potassium propionate (34 mg, 0.302 mmol) followed by 1,4-Dioxane (1.5 mL). Argon was bubbled through the solution for 3 min then the mixture was heated to 120 °C for 60 min. To the mixture was added aqueous sodium carbonate (2.00 M, 0.10 mL, 0.20 mmol), [1,1'-Bis(diphenylphosphino)ferrocene] dichloropalladium(II) (3.8 mg, 0.050 mmol), and 2-[(6-bromo-3-fluoro-2-pyridyl)oxymethyl]-5-(trifluoromethyl)thiazole (Intermediate **I-102**, 40 mg, 0.111 mmol). Argon was bubbled through the solution for 3 min then the mixture was heated to 110 °C for 60 min. The reaction mixture was filtered through celite, eluting with DCM and the filtrate was concentrated in vacuo. The crude residue was purified by silica gel flash column chromatography (EtOAc / Hex gradient) to give methyl 2-[[2,5-difluoro-4-[5-fluoro-6-[[5-(trifluoromethyl)thiazol-2-yl]methoxy]-2-pyridyl]phenyl]methyl]-3-[(3S)-4,4-dimethyltetrahydrofuran-3-yl]-7-fluoro-benzimidazole-5-carboxylate **(Intermediate I-106)**. ES/MS m/z: 694.2 (M+H⁺).

### Preparation of Intermediate I-107

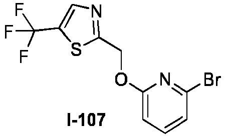

**2-[(6-bromo-2-pyridyl)oxymethyl]-5-(trifluoromethyl)thiazole (I-107):** A round-bottom flask was charged with [5-(trifluoromethyl)thiazol-2-yl]methanol (570 mg, 3.1 mmol, 1.1 equivalent), 2-bromo-6-fluoropyridine (500 mg, 2.8 mol, 1.0 equivalent), and cesium carbonate (1.3 g, 4.0 mmol, 1.4 equivalent). Anhydrous acetonitrile (10 mL) was added, and the resulting mixture was heated to reflux and stirred for 12 h. After cooling down to rt, the mixture was filtered through a plug of Celite and the and concentrated in vacuo. The residue was purified by silica gel flash column chromatography (EtOAc/hexane gradient) to yield the title compound. ES/MS m/z: 339.8 (M+H)⁺.

### Preparation of Intermediate I-108

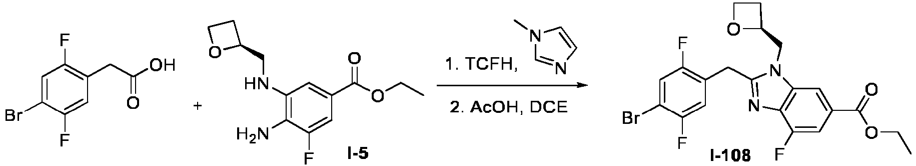

**Ethyl (S)-2-(4-bromo-2,5-difluorobenzyl)-4-fluoro-1-(oxetan-2-ylmethyl)-1H-benzo[d]imidazole-6-carboxylate (Intermediate I-108)**. A round-bottom flask was charged with 2-(4-bromo-2,5-difluoro-phenyl)acetic acid (3.37 g, 13.4 mmol, 1.2 equivalent), ethyl 4-amino-3-fluoro-5-[[(2S)-oxetan-2-yl]methylamino]benzoate (Intermediate **I-5**, 3.00 g, 11.2 mmol, 1.0 equivalent), and 1-methylimidazole (2.7 mL, 33 mmol, 3.0 equivalent). Anhydrous acetonitrile (40 mL) was added and the mixture was cooled to 0 °C. TCFH (3.76 g, 13.4 mmol, 1.2 equivalent) was added slowly, and the mixture was warmed to rt and stirred for 2 h before diluting with EtOAc (100 mL) and water (100 mL). The layers were separated, and the combined organic extracts were washed with saturated aqueous NH₄Cl, (100 mL), saturated aqueous NaHCO₃ (100 mL), and brine (50 mL), respectively. The resulting solution was dried over MgSO₄, filtered, and concentrated in vacuo. The resulting crude residue was dissolved in dichloroethane (60 mL) followed by acetic acid (10 mL). The mixture was heated to 60 °C and stirred for 48 h. After cooling to rt, the solution was concentrated and purified by silica gel column chromatography (EtOAc / hexane gradient) to yield ethyl (S)-2-(4-bromo-2,5-difluorobenzyl)-4-fluoro-1-(oxetan-2-ylmethyl)-1H-benzo[d]imidazole-6-carboxylate (Intermediate **I-108).** ES/MS m/z: 483.6 (M+H)⁺.

### Preparation of Intermediate I-109

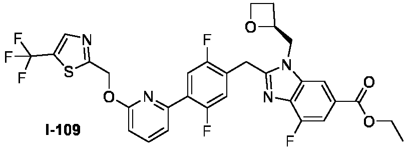

**Ethyl (S)-2-(2,5-difluoro-4-(6-((5-(trifluoromethyl)thiazol-2-yl)methoxy)pyridin-2-yl)benzyl)-4-fluoro-1-(oxetan-2-ylmethyl)-1H-benzo[d]imidazole-6-carboxylate (Intermediate I-109):** To a 25-mL microwave vial was charged with methyl 2-[(4-bromo-2,5-difluoro-phenyl)methyl]-3-[(3S)-4,4-dimethyltetrahydrofuran-3-yl]benzimidazole-5-carboxylate (Intermediate **I-108**, 1.3 g, 2.70 mmol, 1.0 equivalent), PdCl₂(dppf)₂ (300 mg, 0.400 mmol, 0.15 equivalent), potassium propionate (900 mg, 8.07 mmol, 3.0 equivalent), and B₂Pin₂ (820 mg, 3.23 mol, 1.2 equivalent). Anhydrous 1,4-dioxane (15 mL) was added and the resulting mixture was purged with argon for 2 minutes. The mixture was sealed and heated to 120 °C by microwave and stirred for 1 h. After cooling down to rt, 2-[(6-bromo-2-pyridyl)oxymethyl]-5-(trifluoromethyl)thiazole (Intermediate **I-107**, 1100 mg, 3.23 mmol, 1.2 equivalent), PdCl₂(dppf)₂ (100 mg, 0.134 mmol, 0.05 equivalent), 2 M aqueous Na₂CO₃ (3.4 mL, 6.72 mmol, 2.5 equivalent) were added, respectively. The resulting mixture was heated to 100°C under argon and stirred for 3 h before cooling to rt and filtered through a plug of Celite and MgSO₄. The filtrate was concentrated and purified by silica gel column chromatography (EtOAc/hexane gradient) to yield ethyl (S)-2-(2,5-difluoro-4-(6-((5-(trifluoromethyl)thiazol-2-yl)methoxy)pyridin-2-yl)benzyl)-4-fluoro-1-(oxetan-2-ylmethyl)-1H-benzo[d]imidazole-6-carboxylate (Intermediate **I-109).** ES/MS m/z: 662.6 (M+H)⁺.

### Preparation of Intermediate I-109a

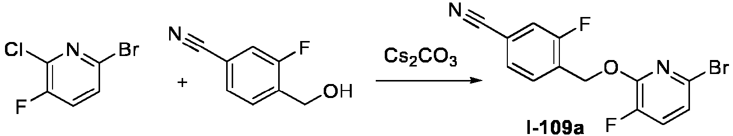

**4-[(6-bromo-3-fluoro-2-pyridyl)oxymethyl]-3-fluoro-benzonitrile (Intermediate** I-**109a):** To a solution of 6-bromo-2-chloro-3-fluoro-pyridine ( 4.90 g, 23.3 mmol) and 3-fluoro-4-(hydroxymethyl)benzonitrile (3.87 g, 25.6 mmol) in acetonitrile (40 mL) was added cesium carbonate ( 15.2 g, 25.6 mmol). The mixture was stirred at 60 °C overnight before being cooled to rt. The mixture was diluted with water, extracted three times with EtOAc, washed with brine, dried over sodium sulfate, filtered, and concentrated. Purification by silica gel flash column chromatography (EtOAc in Hexane gradient) yielded 4-[(6-bromo-3-fluoro-2-pyridyl)oxymethyl]-3-fluoro-benzonitrile (Intermediate **I-109a).** ES/MS m/z: 326.1 (M + H)⁺.

### Preparation of Intermidate I-1001

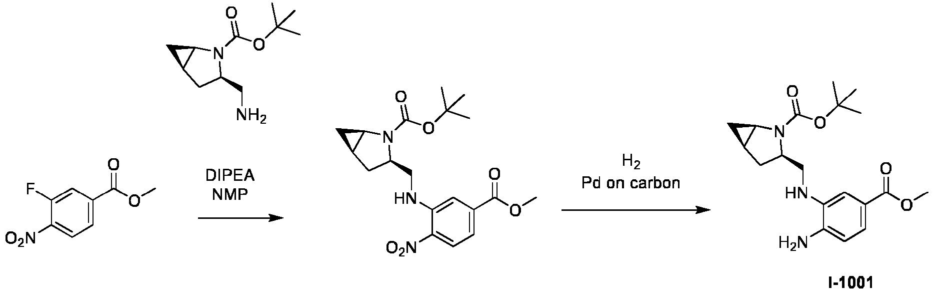

**tert-Butyl (1R,3R,SR)-3-[(2-amino-5-methoxycarbonyl-anilino)methyl]-2-azabicyclo[3.1.0]hexane-2-carboxylate** was prepared in a manner as described for Intermediate **I-1** substituting tert-butyl (1R,3R,SR)-3-(aminomethyl)-2-azabicyclo[3.1.0]hexane-2-carboxylate for 2-methoxyethylamine. ES/MS: 362.2 (M+H+). 1H NMR (400 MHz, Chloroform-d) δ 7.39 (d, J = 8.1 Hz, 1H), 7.15 (s, 1H), 6.63 (d, J = 8.1 Hz, 1H), 4.58 (t, J= 11.0 Hz, 1H), 3.87 (s, 3H), 3.64 (d, J = 7.3 Hz, 1H), 3.27 - 3.09 (m, 1H), 3.00 (t, J = 11.0 Hz, 1H), 2.56 (q, J = 11.7 Hz, 1H), 1.95 - 1.70 (m, 1H), 1.53 (s, 10H), 0.81 (q, J = 7.1 Hz, 1H), 0.57 (d, J = 5.7 Hz, 1H).

### Preparation of Intermediate I-1010

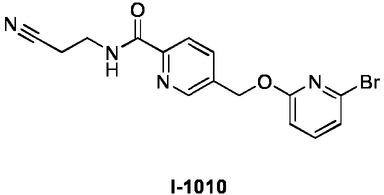

**5-[(6-bromo-2-pyridyl)oxymethyl]-N-(2-cyanoethyl)pyridine-2-carboxamide was** prepared in a manner as described for Intermediate **I-28** substituting 3-aminopropanenitrile for 1-aminocyclopropanecarbonitrile hydrochloride. ES/MS: 361.0, 363.0 (M+H+). 1H NMR (400 MHz, Chloroform-d) δ 8.68 (d, J = 2.0 Hz, 1H), 8.46 (d, J = 6.7 Hz, 1H), 8.20 (d, J = 8.0 Hz, 1H), 7.98 (dd, J = 8.0, 2.1 Hz, 1H), 7.48 (t, J = 7.8 Hz, 1H), 7.13 (d, J = 7.5 Hz, 1H), 6.79 (d, J = 8.1 Hz, 1H), 5.48 (s, 2H), 3.78 (q, J = 6.5 Hz, 2H), 2.77 (t, J = 6.5 Hz, 2H).

### Preparation of Intermediate I-1011

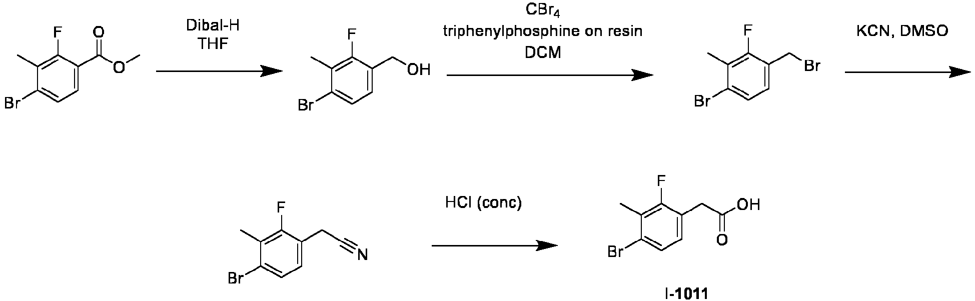

**(4-bromo-2-fluoro-3-methyl-phenyl)methanol: To a solution of methyl 4-bromo-2-fluoro-3-methyl-benzoate** (500 mg, 2.02 mmol) in THF (10 mL) at 0 °, was added diisobutylaluminum hydride (1000 mmol/L, 5.06 mL, 5.06 mmol). The mixture was stirred for 1 hr. The reaction was diluted with 25 mL Et₂O and cooled to 0 °C. 0.200 mL water and 0.200 mL 15% NaOH were added, and an additional 0.500 mL water was added. The mixture was warmed to rt and stirred for 15 min. To this was added MgSO₄ and the mixture was stirred 15 min, then filtered and rinsed with Et₂O to give crude residue which was purified by chromatography (eluent: EtOAc/hexanes) to give desired product. 1H NMR (400 MHz, Chloroform-d) δ 7.37 (dd, J = 8.2, 1.3 Hz, 1H), 7.16 (t, J = 7.9 Hz, 1H), 4.73 (s, 2H), 2.36 (d, J = 2.5 Hz, 3H).

**1-bromo-4-(bromomethyl)-3-fluoro-2-methyl-benzene:** To a solution of (4-bromo-2-fluoro-3-methyl-phenyl)methanol (358 mg, 1.63 mmol) and (4-diphenylphosphanylphenyl) polymer bound (78.7 %, 651 mg, 1.966 mmol), in DCM (10 mL) at 0 °, was added carbon tetrabromide (650 mg, 1.96 mmol). The mixture was gradually warmed to rt overnight. The suspension was filtered, diluted with DCM and washed with brine. The organic extract was dried over sodium sulfate, concentrated and purified by chromatography (eluent: EtOAc/hexanes) to give desired product. 1H NMR (400 MHz, Chloroform-d) δ 7.35 (dd, J = 8.3, 1.3 Hz, 1H), 7.12 (t, J = 8.0 Hz, 1H), 4.49 (d, J = 1.2 Hz, 2H), 2.37 (d, J = 2.5 Hz, 3H).

**2-(4-bromo-2-fluoro-3-methyl-phenyl)acetonitrile:** To a solution of 1-bromo-4-(bromomethyl)-3-fluoro-2-methyl-benzene (299 mg, 1.06 mmol) in DMSO (6 ml) at rt, was added potassium cyanide (138 mg, 2.12 mmol). The mixture was stirred at rt overnight. The reaction was diluted with EtOAc and washed with 10% Na₂CO₃. The organic layer was then washed with brine. The mixture was dried over sodium sulfate to give desired product. 1H NMR (400 MHz, Chloroform-d) δ 7.41 (dd, J = 8.3, 1.4 Hz, 1H), 7.17 (t, J = 8.0 Hz, 1H), 3.73 (d, J = 1.0 Hz, 2H), 2.37 (d, J = 2.6 Hz, 3H).

**2-(4-bromo-2-fluoro-3-methyl-phenyl)acetic acid (I-1011):** A solution of 2-(4-bromo-2-fluoro-3-methyl-phenyl)acetonitrile (164 mg, 0.717 mmol) in concentrated HCl (2 mL) was heated at 100 overnight. The reaction was cooled to rt, diluted with EtOAc and neutralized carefully with 6 NaOH (4 mL). The organic extract was dried organic extract over sodium sulfate, filtered and concentrated to give desired product. 1H NMR (400 MHz, Methanol-d4) δ 7.35 (dd, J = 8.3, 1.3 Hz, 1H), 7.08 (t, J = 8.0 Hz, 1H), 3.64 (d, J = 1.8 Hz, 2H), 2.35 (d, J = 2.5 Hz, 3H).

### Preparation of Intermediate I-1012

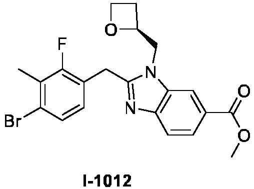

**Methyl 2-[(4-bromo-2-fluoro-3-methyl-phenyl)methyl]-3-[[(2S)-oxetan-2-yl]methyl]benzimidazole-5-carboxylate** was prepared in a manner as described for Intermediate **I-2** substituting **I-4** for **I-1** and 2-(4-bromo-2-fluoro-3-methyl-phenyl)acetic acid **(I-1011)** for 2-(4-bromo-2-fluoro-phenyl)acetic acid. ES/MS: 447.0, 449.0 (M+H+). 1H NMR (400 MHz, Chloroform-d) δ 8.11 (d, J = 1.7 Hz, 1H), 7.98 (dd, J = 8.5, 1.6 Hz, 1H), 7.75 (d, J = 8.5 Hz, 1H), 7.29 (dd, J = 8.3, 1.3 Hz, 1H), 6.99 (t, J = 8.0 Hz, 1H), 5.14 (qd, J = 6.8, 2.9 Hz, 1H), 4.72 - 4.51 (m, 1H), 4.51 - 4.27 (m, 5H), 3.96 (s, 3H), 2.73 (dtd, J = 11.4, 8.1, 6.1 Hz, 1H), 2.51 - 2.38 (m, 1H), 2.35 (d, J = 2.6 Hz, 3H).

### Preparation of Intermediate I-1017

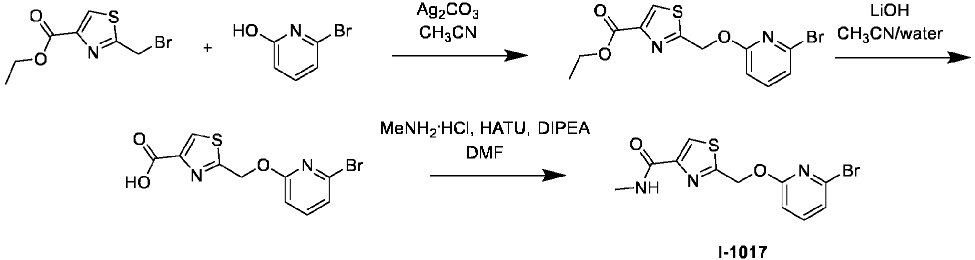

**Ethyl 2-[(6-bromo-2-pyridyl)oxymethyl]thiazole-4-carboxylate:** A suspension of ethyl 2-(bromomethyl)thiazole-4-carboxylate (500 mg, 2.0 mmol), 6-bromopyridin-2-ol (350 mg, 2.0 mmol), and silver carbonate (1186 mg, 4.3 mmol) in CH₃CN (15 mL) was heated at 50 ° C overnight. The reaction was diluted with EtOAc and brine. The mixture was filtered over Celite frit. The layers were partitioned, and the organic layer was washed with brine once more. The mixture was dried over sodium sulfate, concentrated, and purified by chromatography (eluent: EtOAc/hexanes) to give desired product.

ES/MS: 343, 345 (M+H+). 1HNMR (400 MHz, Chloroform-d) δ 8.22 (s, 1H), 7.51 (dd, J = 8.2, 7.5 Hz, 1H), 7.17 (dd, J = 7.5, 0.7 Hz, 1H), 6.82 (dd, J = 8.2, 0.7 Hz, 1H), 5.73 (s, 2H), 4.46 (q, J = 7.1 Hz, 2H), 1.43 (t, J = 7.1 Hz, 3H).

**2-[(6-bromo-2-pyridyl)oxymethyl]thiazole-4-carboxylic acid:** A solution of ethyl 2-[(6-bromo-2-pyridyl)oxymethyl]thiazole-4-carboxylate (114 mg, 0.332 mmol) and Lithium hydroxide, monohydrate (43.3 mg, 1.03 mmol) in CH₃CN (3 mL) and water (1 mL) was prepared. The mixture was stirred at rt for 2 hr. The reaction mixture was diluted with EtOAc. The mixture was adjusted to pH~6 with 1N HCl (1.05 mL). The organic layer was dried over sodium sulfate to give desired product. ES/MS: 315, 317 (M+H+). 1H NMR (400 MHz, Methanol-d4) δ 8.40 (d, J = 1.0 Hz, 1H), 7.71 - 7.57 (m, 1H), 7.25 (d, J = 7.5 Hz, 1H), 6.93 (d, J = 8.1 Hz, 1H), 5.68 (d, J = 1.0 Hz, 2H).

**2-[(6-bromo-2-pyridyl)oxymethyl]-N-methyl-thiazole-4-carboxamide (I-1017):** To a solution of 2-[(6-bromo-2-pyridyl)oxymethyl]thiazole-4-carboxylic acid (47.7 mg, 0.151 mmol), methanamine;hydrochloride (26.0 mg, 0.385 mmol), and o-(7-Azabenzotriazol-1-yl)-N,N,N',N'-tetramethyluronium hexafluorophosphate (84.6 mg, 0.223 mmol) in DMF (3 mL), was added N,N-diisopropylethylamine (0.105 mL, 0.605 mmol). The mixture was stirred at rt overnight. The reaction was diluted with EtOAc, washed with 5%LiCl, saturated NaHCO₃, and brine. The mixture was dried over sodium sulfate, concentrated, and purified by chromatography (eluent: EtOAc/hexanes) to give desired product. MR (400 MHz, Chloroform-d) δ 8.13 (s, 1H), 7.51 (t, J = 7.8 Hz, 1H), 7.34 (s, 1H), 7.18 (d, J = 7.5 Hz, 1H), 6.83 (d, J = 8.2 Hz, 1H), 5.64 (s, 2H), 3.04 (d, J = 5.0 Hz, 3H).

### Preparation of Intermediate I-1018

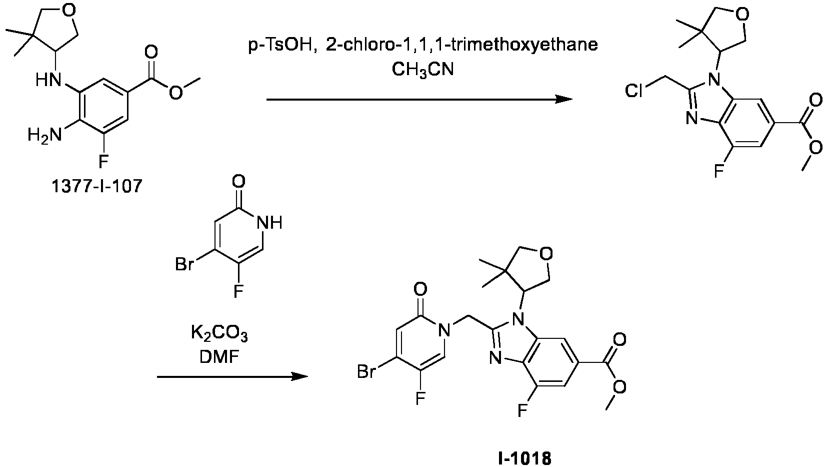

**Methyl 2-(chloromethyl)-3-(4,4-dimethyltetrahydrofuran-3-yl)-7-fluoro-benzimidazole-5-carboxylate:** To a solution of methyl 4-amino-3-[(4,4-dimethyltetrahydrofuran-3-yl)amino]-5-fluoro-benzoate (I-104 ) (1.13 g, 4.00 mmol) in CH₃CN (20 mL), was added p-Toluenesulfonic Acid, monohydrate (0.0345 g, 0.200 mmol), and 2-Chloro-1,1,1-trimethoxyethane (0.547 mL, 5.20 mmol). The mixture was heated at 60 °C overnight. To this was added 2-Chloro-1,1,1-trimethoxyethane (0.547 mL, 5.20 mmol) and p-Toluenesulfonic Acid monohydrate (0.0345 g, 0.200 mmol) and the mixture was then heated at 60 °C for 3 hr. The reaction was diluted with EtOAc washed with brine, dried over sodium sulfate, concentrated, and purified by chromatography (eluent: EtOAc/hexanes) to give desired product. ES/MS: 341.2 (M+H+). 1HNMR (400 MHz, Chloroform-d) δ 8.41 (s, 1H), 7.71 (dd, J = 10.6, 1.4 Hz, 1H), 4.96 (d, J = 12.6 Hz, 1H), 4.90 - 4.76 (m, 2H), 4.61 (dd, J = 11.2, 1.9 Hz, 1H), 4.50 (dd, J = 11.2, 7.0 Hz, 1H), 3.97 (d, J = 1.2 Hz, 4H), 3.83 (d, J = 9.3 Hz, 1H), 1.43 (s, 3H), 0.73 (s, 3H).

**Methyl 2-[(4-bromo-5-fluoro-2-oxo-1-pyridyl)methyl]-3-(4,4-dimethyltetrahydrofuran-3-yl)-7-fluoro-benzimidazole-5-carboxylate (I-1018):** A suspension of 4-bromo-5-fluoro-1H-pyridin-2-one (115 mg, 0.599 mmol), methyl 2-(chloromethyl)-3-(4,4-dimethyltetrahydrofuran-3-yl)-7-fluoro-benzimidazole-5-carboxylate (205 mg, 0.602 mmol), and Potassium carbonate (414 mg, 3.00 mmol) in DMF (3 mL) was heated at 60 °C for 24 hr. The mixture was cooled to rt, diluted with EtOAc, filtered, and concentrated. The mixture was diluted with EtOAc and washed with 5% LiCl and brine. The mixture was dried over sodium sulfate, concentrated, and purified by chromatography (eluent: EtOAc/hexanes) to give desired product. Submitted for chiral-SFC separation (SFC IG column with EtOH cosolvent) gave 2 distinct stereoisomers. ES/MS: 497, 499 (M+H+). 1HNMR (400 MHz, Chloroform-d) δ 8.45 (s, 1H), 7.82 (d, J = 3.9 Hz, 1H), 7.72 (dd, J = 10.8, 1.2 Hz, 1H), 6.93 (d, J = 6.4 Hz, 1H), 5.73 (d, J = 14.8 Hz, 1H), 5.28 (d, J = 6.6 Hz, 1H), 5.11 (d, J = 14.8 Hz, 1H), 4.69 - 4.36 (m, 2H), 3.97 (s, 3H), 3.90 - 3.72 (m, 2H), 1.43 (s, 3H), 0.61 (s, 3H).

### Preparation of Intermediate's I-1019 and I-1020

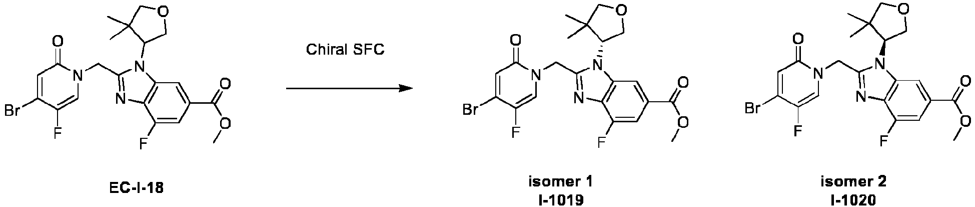

**Methyl 2-[(4-bromo-5-fluoro-2-oxo-1-pyridyl)methyl]-3-(4,4-dimethyltetrahydrofuran-3-yl)-7-fluoro-benzimidazole-5-carboxylate** was subjected to chiral-SFC separation (SFC IG column with EtOH cosolvent) and gave two distinct stereoisomers.

**Methyl 2-[(4-bromo-5-fluoro-2-oxo-1-pyridyl)methyl]-3-(4,4-dimethyltetrahydrofuran-3-yl)-7-fluoro-benzimidazole-5-carboxylate (I-1019 isomer 1):** RT 2.46 min. ES/MS: 496, 498 (M+H+). 1HNMR (400 MHz, Chloroform-d) δ 8.46 (s, 1H), 7.82 (d, J = 3.9 Hz, 1H), 7.73 (dd, J = 10.8, 1.2 Hz, 1H), 6.94 (d, J = 6.4 Hz, 1H), 5.74 (d, J = 14.8 Hz, 1H), 5.29 (d, J = 6.5 Hz, 1H), 5.10 (d, J = 14.8 Hz, 1H), 4.68 - 4.45 (m, 2H), 3.97 (s, 3H), 3.93 - 3.77 (m, 2H), 1.44 (s, 3H), 0.62 (s, 3H).

**Methyl 2-[(4-bromo-5-fluoro-2-oxo-1-pyridyl)methyl]-3-(4,4-dimethyltetrahydrofuran-3-yl)-7-fluoro-benzimidazole-5-carboxylate (I-1020 isomer 2):** RT 5.88 min. ES/MS: 496, 498 (M+H+). 1H NMR (400 MHz, Chloroform-d) δ 8.46 (s, 1H), 7.82 (d, J = 3.9 Hz, 1H), 7.73 (dd, J = 10.8, 1.2 Hz, 1H), 6.94 (d, J = 6.4 Hz, 1H), 5.74 (d, J = 14.8 Hz, 1H), 5.29 (d, J = 6.5 Hz, 1H), 5.10 (d, J = 14.8 Hz, 1H), 4.68 - 4.45 (m, 2H), 3.97 (s, 3H), 3.93 - 3.77 (m, 2H), 1.44 (s, 3H), 0.62 (s, 3H).

### Preparation of Intermediate I-1023

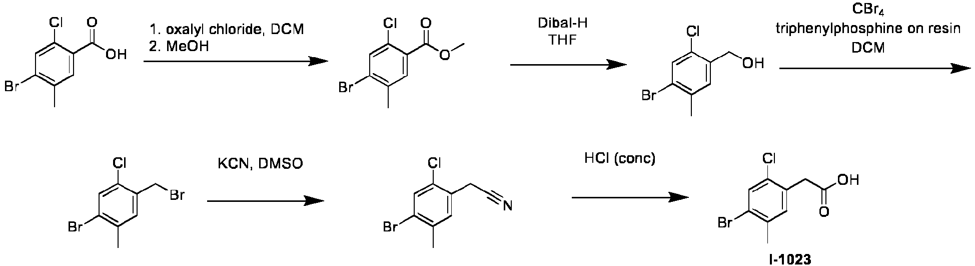

To a solution of 4-bromo-2-chloro-5-methyl-benzoic acid (996 mg, 3.99 mmol) in DCM (10 mL) at 0 °C, was added oxalyl dichloride (2000 mmol/L, 2.40 mL, 4.79 mmol) and 5 drops of DMF. The mixture was stirred for 1 hr. Upon completion of time, 10 mL MeOH was added and the resulting mixture stirred overnight at rt. Next, the mixture was diluted with DCM, washed with saturated NaHCO₃ dried over sodium sulfate, concentrated, and purified by chromatography (eluent: EtOAc/hexanes) to give desired product. 1HNMR (400 MHz, Chloroform-d) δ 7.74 (s, 1H), 7.68 (d, J = 1.1 Hz, 1H), 3.94 (d, J = 1.2 Hz, 3H), 2.42 (s, 3H).

**(4-bromo-2-chloro-5-methyl-phenyl)methanol:** To a solution of methyl 4-bromo-2-chloro-5-methyl-benzoate (931 mg, 3.53 mmol) in THF (15 mL) at 0 °C, diisobutylaluminium hydride (1000 mmol/L, 8.83 mL, 8.83 mmol) was added and the resulting mixture was stirred for 1 hr. Upon completion of time, the mixture was diluted with 25 mL Et₂O and cooled to 0 °C. Then 0.350 mL water, 0.350 mL 15% NaOH was added, then and additional 0.900 mL water was added. The resulting mixture was warmed to rt and stirred for 15 min. Next, MgSO₄ was added and stirred for 15 min. The mixture was then filtered and rinsed with Et₂O to give crude residue which was purified by chromatography (eluent: EtOAc/hexanes) to give desired product.1H MR (400 MHz, Chloroform-d) δ 7.56 (d, J = 1.4 Hz, 1H), 7.38 (s, 1H), 4.73 (s, 2H), 2.41 (d, J = 1.3 Hz, 4H).

**1-bromo-4-(bromomethyl)-5-chloro-2-methyl-benzene:** To a solution of (4-bromo-2-chloro-5-methyl-phenyl)methanol (639 mg, 2.71 mmol) and (4-diphenylphosphanylphenyl) polymer bound (78.7 %, 1081 mg, 3.26 mmol), in DCM (10 mL) at 0 °C, was added carbon tetrabromide (1080 mg, 3.26 mmol). The mixture was gradually warmed to rt overnight. The suspension was filtered, diluted with DCM and washed with brine. The organic extract was dried over sodium sulfate, concentrated and purified by chromatography (eluent: EtOAc/hexanes) to give desired product. 1H NMR (400 MHz, Chloroform-d) δ 7.60 (s, 1H), 7.32 (s, 1H), 4.53 (s, 2H), 2.39 (s, 3H).

**2-(4-bromo-2-chloro-5-methyl-phenyl)acetonitrile:** To a solution of 1-bromo-4-(bromomethyl)-5-chloro-2-methyl-benzene (721 mg, 2.42 mmol) in DMSO (12 ml) at rt, was added potassium cyanide (315 mg, 4.83 mmol). The resulting mixture was stirred at rt overnight. The mixture was diluted with EtOAc and washed with 10% Na₂CO₃. The organic extract was washed once more with brine, then dried over sodium sulfate to give desired product. 1H NMR (400 MHz, Chloroform-d) δ 7.62 (s, 1H), 7.52 - 7.33 (m, 1H), 3.78 (s, 2H), 2.42 (s, 3H).

**2-(4-bromo-2-chloro-5-methyl-phenyl)acetic acid (I-1023):** A solution of 2-(4-bromo-2-chloro-5-methyl-phenyl)acetonitrile (179 mg, 0.732 mmol) in conc HCl (5 mL) was heated at 100 °C overnight. The mixture was cooled to rt, then diluted with EtOAc, and neutralized carefully with 6 N NaOH (10 mL). The organic extract was dried over sodium sulfate to give desired product. 1HNMR (400 MHz, Methanol-d4) δ 7.59 (s, 1H), 7.29 (s, 1H), 3.71 (s, 2H), 2.37 (s, 3H).

### Preparation of Intermediate I-1024

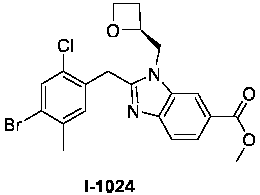

**Methyl 2-[(4-bromo-2-chloro-5-methyl-phenyl)methyl]-3-[[(2S)-oxetan-2-yl]methyl]benzimidazole-5-carboxylate (I-1024):** Methyl 2-[(4-bromo-2-chloro-5-methylphenyl)methyl]-3-[[(2S)-oxetan-2-yl]methyl]benzimidazole-5-carboxylate was prepared in a manner as described for Intermediate **I-2** substituting **I-4** for **I-1** and 2-(4-bromo-2-chloro-5-methyl-phenyl)acetic acid **(I-1023)** for 2-(4-bromo-2-fluoro-phenyl)acetic acid. ES/MS: 463, 465 (M+H+). 1H NMR (400 MHz, Chloroform-d) δ 8.12 (d, J = 1.6 Hz, 1H), 8.00 (dd, J = 8.5, 1.4 Hz, 1H), 7.78 (d, J = 8.5 Hz, 1H), 7.62 (s, 1H), 7.13 (s, 1H), 5.13 (dd, J = 7.1, 2.9 Hz, 1H), 4.77 - 4.59 (m, 1H), 4.59 - 4.23 (m, 5H), 3.97 (d, J = 1.0 Hz, 3H), 2.74 (ddt, J = 14.1, 11.7, 6.6 Hz, 1H), 2.52 - 2.35 (m, 1H), 2.29 (s, 3H).

### Preparation of Intermediate I-1026

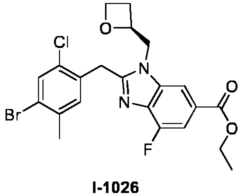

**Ethyl 4-[[2-(4-bromo-2-chloro-5-methyl-phenyl)acetyl]amino]-3-fluoro-5-[[(2S)-oxetan-2-yl]methylamino]benzoate:** To a solution of 2-(4-bromo-2-chloro-5-methylphenyl)acetic acid (501 mg, 1.90 mmol) and ethyl 4-amino-3-fluoro-5-[[(2S)-oxetan-2-yl]methylamino]benzoate (510 mg, 1.90 mmol) in CH₃CN (19 mL) at 0 °C, was added 1-Methylimidazole (0.758 mL, 9.50 mmol), followed by TCFH (640 mg, 2.28 mmol). The mixture was stirred at rt overnight. The mixture was diluted with EtOAc, washed with saturated NH₄Cl, 1N NaOH, and brine and subsequently dried over sodium sulfate, concentrated, and carried onto next step below. ES/MS: 513, 515 (M+H+).

**Ethyl 2-[(4-bromo-2-chloro-5-methyl-phenyl)methyl]-7-fluoro-3-[[(2S)-oxetan-2-yl]methyl]benzimidazole-5-carboxylate (I-1026):** A solution of ethyl 4-[[2-(4-bromo-2-chloro-5-methyl-phenyl)acetyl]amino]-3-fluoro-5-[[(2S)-oxetan-2-yl]methylamino]benzoate (977 mg, 1.90 mmol) and acetic acid (3426 mg, 57.0 mmol) in DCE (15 mL) was heated at 60 °C overnight. The mixture was cooled to rt, diluted with EtOAc and carefully neutralized with NaHCO₃ (4.78 g). Washed organic layer with brine and dried over sodium sulfate and concentrated. Purification by chromatography (eluent: EtOAc/hexanes) gave desired product. ES/MS: 495, 497 (M+H+). 1HNMR (400 MHz, Chloroform-d) δ 7.94 (d, J = 1.3 Hz, 1H), 7.69 (dd, J = 11.0, 1.3 Hz, 1H), 7.62 (s, 1H), 7.12 (s, 1H), 5.08 (qd, J = 6.9, 2.8 Hz, 1H), 4.74 - 4.59 (m, 1H), 4.59 - 4.24 (m, 8H), 2.73 (dtd, J = 11.4, 8.1, 6.1 Hz, 1H), 2.39 (ddt, J = 11.5, 9.0, 7.2 Hz, 1H), 2.29 (s, 3H), 1.44 (t, J = 7.2 Hz, 3H).

### Preparation of Intermediate I-1030

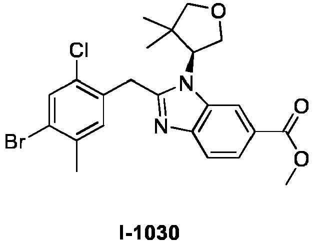

**Methyl 2-[(4-bromo-2-chloro-5-methyl-phenyl)methyl]-3-[(3S)-4,4-dimethyltetrahydrofuran-3-yl]benzimidazole-5-carboxylate (I-1030):** Methyl 2-[(4-bromo-2-chloro-5-methyl-phenyl)methyl]-3-[(3S)-4,4-dimethyltetrahydrofuran-3-yl]benzimidazole-5-carboxylate was prepared in a manner as described for Intermediate **I-2** substituting **I-80** for **I-1** and 2-(4-bromo-2-chloro-5-methyl-phenyl)acetic acid **(I-1023)** for 2-(4-bromo-2-fluorophenyl)acetic acid. ES/MS: 490.6, 492.6 (M+H+). 1HNMR (400 MHz, Chloroform-d) δ 8.56 (s, 1H), 8.02 (dd, J = 8.5, 1.4 Hz, 1H), 7.80 (d, J = 8.5 Hz, 1H), 7.64 (s, 1H), 7.06 (s, 1H), 4.60 - 4.47 (m, 2H), 4.45 - 4.26 (m, 3H), 3.95 (d, J = 10.4 Hz, 5H), 3.78 (d, J = 8.8 Hz, 1H), 2.28 (s, 3H), 2.07 (s, 1H), 1.31 (s, 3H), 0.67 (s, 3H).

### Preparation of Intermediate I-1031

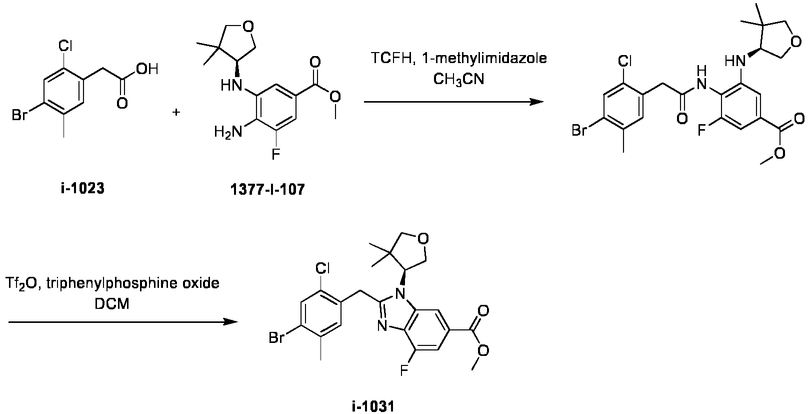

**methyl 4-[[2-(4-bromo-2-chloro-5-methyl-phenyl)acetyl]amino]-3-[[(3S)-4,4-dimethyltetrahydrofuran-3-yl]amino]-5-fluoro-benzoate:** To a solution of 2-(4-bromo-2-chloro-5-methyl-phenyl)acetic acid (399 mg, 1.51 mmol) and methyl 4-amino-3-[[(3S)-4,4-dimethyltetrahydrofuran-3-yl]amino]-5-fluoro-benzoate (427 mg, 1.51 mmol) in CH₃CN (7 mL) at 0 °C, 1-Methylimidazole (0.603 mL, 7.56 mmol) was added, followed by the addition of TCFH (509 mg, 1.82 mmol). The resulting mixture was stirred at rt overnight. The mixture was then diluted with EtOAc and washed with saturated NH₄Cl, 1N NaOH, and brine. Next, the mixture was dried over sodium sulfate, concentrated and carried onto next step below without purification. ES/MS: 527, 529 (M+H+).

**Methyl 2-[(4-bromo-2-chloro-5-methyl-phenyl)methyl]-3-[(3S)-4,4-dimethyltetrahydrofuran-3-yl]-7-fluoro-benzimidazole-5-carboxylate (I-1031):** To a solution of methyl 4-[[2-(4-bromo-2-chloro-5-methyl-phenyl)acetyl]amino]-3-[[(3S)-4,4-dimethyltetrahydrofuran-3-yl]amino]-5-fluoro-benzoate (798 mg, 1.51 mmol) and Triphenylphosphine oxide (1262 mg, 4.54 mmol) in DCM (15 mL) at 0 °C, Triflic anhydride (1000 mmol/L, 2.27 mL, 2.27 mmol) was added. The mixture was gradually warmed to rt and stirred for 1 hr. Upon completion, the mixture was diluted with DCM and washed with saturated NaHCO₃ solution and brine. Next, the mixture was dried over sodium sulfate, concentrated and purified by chromatography (eluent: EtOAc/hexanes) to give desired product. ES/MS: 508.6, 510.6 (M+H+). 1HNMR (400 MHz, Chloroform-d) δ 8.38 (s, 1H), 7.71 (dd, J = 10.8, 1.2 Hz, 1H), 7.64 (s, 1H), 7.06 (s, 1H), 4.55 - 4.42 (m, 2H), 4.41 (d, J = 2.8 Hz, 2H), 4.32 (dd, J = 11.0, 7.0 Hz, 1H), 3.97 (s, 3H), 3.92 (d, J = 8.8 Hz, 1H), 3.77 (d, J = 8.8 Hz, 1H), 2.27 (s, 3H), 1.29 (s, 3H), 0.64 (s, 3H).

### Preparation of Intermediate I-1032

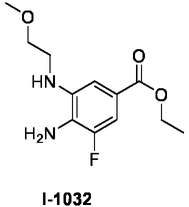

**Ethyl (S)-4-amino-3-fluoro-5-((oxetan-2-ylmethyl)amino)benzoate (I-1032):** Ethyl (S)-4-amino-3-fluoro-5-((oxetan-2-ylmethyl)amino)benzoate was prepared in a manner as described for Intermediate **I-1** substituting ethyl 3,5-difluoro-4-nitrobenzoate for methyl 3-fluoro-4-nitro-benzoate. ES/MS: 257.2 (M+H+); 'H NMR (400 MHz, CDCl3) δ 7.32 (dd, J = 10.8, 1.7 Hz, 1H), 7.20 (t, J = 1.4 Hz, 1H), 4.35 (q, J = 7.1 Hz, 2H), 3.78 (s, 3H), 3.68 (dd, J = 5.6, 4.5 Hz, 2H), 3.43 (s, 3H), 3.35 (dd, J = 5.6, 4.5 Hz, 2H), 1.39 (t, J = 7.1 Hz, 3H).

### Preparation of Intermediate I-1033

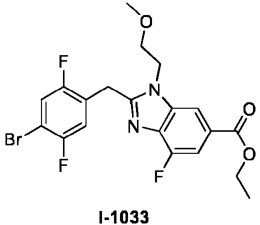

**Ethyl 2-(4-bromo-2,5-difluorobenzyl)-4-fluoro-1-(2-methoxyethyl)-1H-benzo[d]imidazole-6-carboxylate (I-1033):** Ethyl 2-(4-bromo-2,5-difluorobenzyl)-4-fluoro-1-(2-methoxyethyl)-lH-benzo[d]imidazole-6-carboxylate was prepared in a manner as described for Intermediate **I-2** substituting ethyl 4-amino-3-fluoro-5-((2-methoxyethyl)amino)benzoate (**I-1032)** for **I-1** and 2-(4-bromo-2,5-difluorophenyl)acetic acid for 2-(4-bromo-2-fluorophenyl)acetic acid. ES/MS: 471.0, 473.0 (M+H+). 1H NMR (400 MHz, CDCl3) δ 7.91 (d, J = 1.3 Hz, 1H), 7.70 (dd, J = 10.8, 1.3 Hz, 1H), 7.34 (dd, J = 8.7, 5.5 Hz, 1H), 7.12 (dd, J = 8.6, 6.3 Hz, 1H), 4.44 (q, J = 7.1 Hz, 2H), 4.39 (s, 2H), 4.37 - 4.29 (m, 2H), 3.66 (t, J = 5.1 Hz, 2H), 3.25 (s, 3H), 1.45 (t, J = 7.1 Hz, 3H).

### Preparation of Intermediate I-1037

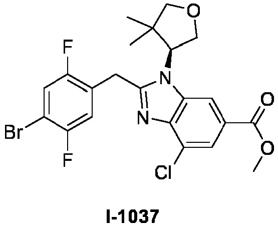

**Methyl 2-[(4-bromo-2,5-difluoro-phenyl)methyl]-7-chloro-3-[(3S)-4,4-dimethyltetrahydrofuran-3-yl]benzimidazole-5-carboxylate (I-1037):** Methyl 2-[(4-bromo-2,5-difluoro-phenyl)methyl]-7-chloro-3-[(3S)-4,4-dimethyltetrahydrofuran-3-yl]benzimidazole-5-carboxylate was prepared in a manner as described for Intermediate **I-1031** substituting 2-(4-bromo-2,5-difluoro-phenyl)acetic acid for 2-(4-bromo-2-chloro-5-methyl-phenyl)acetic acid and methyl 4-amino-3-chloro-5-[[(3S)-4,4-dimethyltetrahydrofuran-3-yl]amino]benzoate **I-1038** for methyl 4-amino-3-[[(3S)-4,4-dimethyltetrahydrofuran-3-yl]amino]-5-fluoro-benzoate. ES/MS: 513.0, 514.9 (M+H+). 1H NMR (400 MHz, Chloroform-d) δ 8.50 (s, 1H), 8.07 (d, J = 1.3 Hz, 1H), 7.38 (dd, J = 8.7, 5.6 Hz, 1H), 7.23 - 7.07 (m, 1H), 4.61 - 4.40 (m, 3H), 4.40 - 4.29 (m, 2H), 3.97 (s, 3H), 3.92 (d, J = 8.9 Hz, 1H), 3.78 (d, J = 8.9 Hz, 1H), 1.32 (s, 3H), 0.63 (s, 3H).

### Preparation of Intermediate I-1038

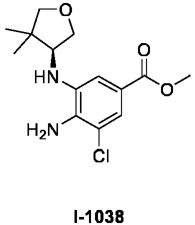

**Methyl 4-amino-3-chloro-5-[[(3S)-4,4-dimethyltetrahydrofuran-3-yl]amino]benzoate (I-1038):** Methyl 4-amino-3-chloro-5-[[(3S)-4,4-dimethyltetrahydrofuran-3-yl]amino]benzoate was prepared in a manner as described for Intermediate **I-68** substituting methyl 3-chloro-5-fluoro-4-nitro-benzoate for methyl 3-bromo-5-fluoro-4-nitro-benzoate. ES/MS: 299.2 (M+).

### Preparation of Intermediate I-1039

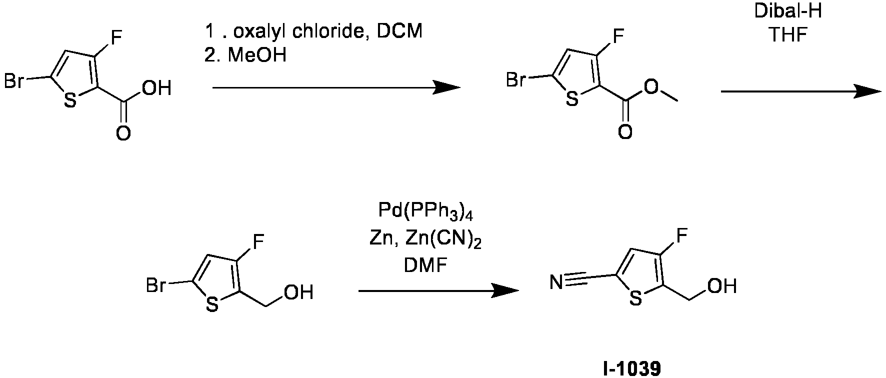

**Methyl 5-bromo-3-fluoro-thiophene-2-carboxylate:** To a suspension of 5-bromo-3-fluoro-thiophene-2-carboxylic acid (1000 mg, 4.44 mmol) in DCM (20 mL) at 0 °C, oxalyl dichloride (2000 mmol/L, 2.67 mL, 5.33 mmol) and 10 drops of DMF were added. the mixture was then stirred for 1 hr. Upon completion, 10 mL MeOH was added and the resulting mixture was stirred overnight at rt. Next, the mixture was diluted with DCM, washed with saturated NaHCO₃ and dried over sodium sulfate, concentrated to dryness, and purified by chromatography (eluent: EtOAc/hexanes) to give desired product. 1H NMR (400 MHz, Chloroform-d) δ 6.91 (s, 1H), 3.89 (s, 3H).

**(5-bromo-3-fluoro-2-thienyl)methanol:** To a solution of methyl 5-bromo-3-fluoro-thiophene-2-carboxylate (907 mg, 3.79 mmol) in THF (15 mL) at 0 °C, was added diisobutylaluminium hydride (1000 mmol/L, 9.48 mL, 9.48 mmol). The mixture was gradually warmed to rt and stirred for 1 hr. Next, the mixture was diluted with 25 mL Et₂O and cooled to 0 °C. Then 0.380 mL water, 0.380 mL 15% NaOH were added and an additional 0.948 mL water was further added. The resulting mixture was warmed to rt and stirred for 15 min. Next, MgSO₄ was added and resulting mixture was stirred for 15 min. Upon completion of time, the mixture was then filtered and rinsed with Et₂O to give crude residue which was purified by chromatography (eluent: EtOAc/hexanes) to give desired product. 1H NMR (400 MHz, Chloroform-d) δ 6.81 (s, 1H), 4.77 - 4.55 (m, 2H).

**4-fluoro-5-(hydroxymethyl)thiophene-2-carbonitrile (I-1039):** A suspension of (5-bromo-3-fluoro-2-thienyl)methanol (300 mg, 1.42 mmol), tetrakis(triphenylphosphine)palladium(O) (411 mg, 0.355 mmol), Zinc cyanide (334 mg, 2.84 mmol) and Zinc powder (4.65 mg, 0.0711 mmol) in DMF (3.5 mL) was degassed with argon, then heated at 100 °C for 16 hr. The mixture was concentrated to dryness and purified by chromatography (eluent: EtOAc/hexanes) to give desired product. ¹H NMR (400 MHz, CDCl₃) δ 7.36 - 7.31 (m, 1H), 4.89 (s, 2H). ¹⁹F NMR (376 MHz, CDCl₃) δ -129.96.

### Preparation of Intermediate I-1040

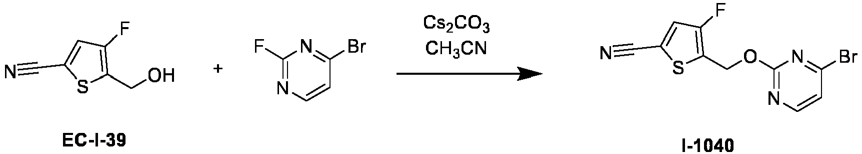

**5-[(4-bromopyrimidin-2-yl)oxymethyl]-4-fluoro-thiophene-2-carbonitrile (I-1040):** A solution of 4-bromo-2-fluoro-pyrimidine (40.0 mg, 0.226 mmol), 4-fluoro-5-(hydroxymethyl)thiophene-2-carbonitrile (37.3 mg, 0.237 mmol), and cesium carbonate (147 mg, 0.452 mmol) in CH₃CN (5 mL) was heated at 50 °C overnight. The mixture was diluted with EtOAc and washed with brine, then dried over sodium sulfate, concentrated to dryness, and purified by chromatography ES/MS: 314, 316 (M+H⁺). 1HNMR (400 MHz, Chloroform-d) δ 8.35 (d, J = 5.2 Hz, 1H), 7.30 - 7.22 (m, 2H), 5.57 (d, J = 1.0 Hz, 2H). 19F NMR (377 MHz, Chloroform-d) δ -127.05.

### Preparation of Intermediate I-1041

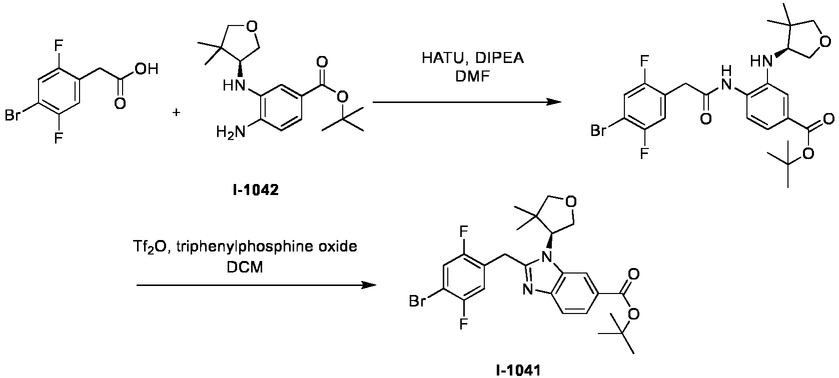

**tert-butyl 4-[[2-(4-bromo-2,5-difluoro-phenyl)acetyl]amino]-3-[[(3S)-4,4-dimethyltetrahydrofuran-3-yl]amino]benzoate:** To a solution of 2-(4-bromo-2,5-difluorophenyl)acetic acid (575 mg, 2.29 mmol), tert-butyl 4-amino-3-[[(3S)-4,4-dimethyltetrahydrofuran-3-yl]amino]benzoate (696 mg, 2.27 mmol), and o-(7-Azabenzotriazol-1-yl)-N,N,N',N'-tetramethyluronium hexafluorophosphate (1292 mg, 3.40 mmol) in DMF (10 mL), was added N,N-diisopropylethylamine (1.22 mL, 6.98 mmol). The mixture was stirred at rt overnight. Next, the mixture was diluted with EtOAc and washed with 5% LiCl, saturated NaHCO₃, and brine, dried over sodium sulfate, concentrated and carried onto next step below without purification. ES/MS: 539, 541 (M+H+).

**tert-butyl 2-[(4-bromo-2,5-difluoro-phenyl)methyl]-3-[(3S)-4,4-dimethyltetrahydrofuran-3-yl]benzimidazole-5-carboxylate (I-1041):** To a solution of tert-butyl 4-[[2-(4-bromo-2,5-difluoro-phenyl)acetyl]amino]-3-[[(3S)-4,4-dimethyltetrahydrofuran-3-yl]amino]-5-fluorobenzoate (1236 mg, 2.22 mmol) and Triphenylphosphine oxide, 99% (1851 mg, 6.65 mmol) in DCM (20 mL) at 0 °C, Triflic anhydride (0.559 mL, 3.33 mmol) was added. the mixture was then gradually warmed to rt and stirred for 1 hr. Upon completion the mixture was diluted with DCM and washed with saturated NaHCO₃ solution and brine. Next, the mixture was dried over sodium sulfate, concentrated and purified by chromatography (eluent: EtOAc/hexanes) to give desired product. ES/MS: 521, 523 (M+H+). 1H NMR (400 MHz, Chloroform-d) δ 8.55 (s, 1H), 7.97 (dd, J = 8.5, 1.5 Hz, 1H), 7.77 (d, J = 8.5 Hz, 1H), 7.37 (dd, J = 8.7, 5.5 Hz, 1H), 7.15 (t, J = 7.4 Hz, 1H), 4.59 (t, J = 8.9 Hz, 2H), 4.47 - 4.25 (m, 3H), 3.96 (d, J = 8.8 Hz, 1H), 3.81 (d, J = 8.8 Hz, 1H), 1.34 (s, 3H), 0.66 (s, 3H).

### Preparation of Intermediate I-1042

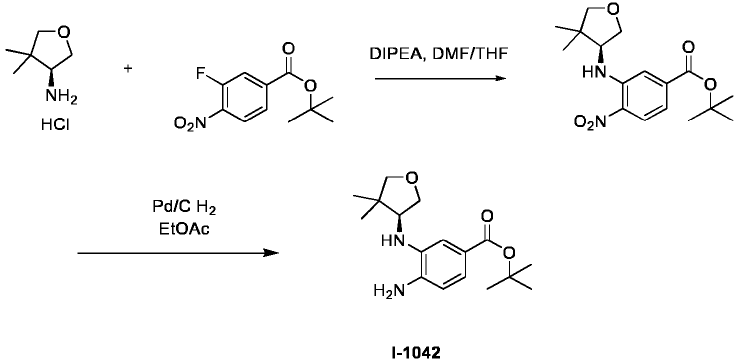

**tert-butyl 3-[[(3S)-4,4-dimethyltetrahydrofuran-3-yl]amino]-4-nitro-benzoate:** To a suspension of tert-butyl 3-fluoro-4-nitro-benzoate (3.50 g, 14.5 mmol), (3S)-4,4-dimethyltetrahydrofuran-3-amine;hydrochloride (2.50 g, 16.5 mmol) and (3S)-4,4-dimethyltetrahydrofuran-3-amine;hydrochloride (2.50 g, 16.5 mmol) in THF (30 mL) and DMF (15 mL), N,N-diisopropylethylamine (12.6 mL, 72.5 mmol) was added. The solution was heated at 80 °C for 18 hr. The crude reaction mixture was diluted with EtOAc (300 mL), washed with 5% LiCl (250 mL) and brine (250 mL). The organic extract was dried over sodium sulfate and purified by silica gel column chromatography (eluent: EtOAc/hexanes) to afford desired product. ES/MS: 337.2 (M+H⁺); 1H NMR (400 MHz, Chloroform-d) δ 8.21 (t, J = 7.8 Hz, 2H), 7.54 (d, J = 1.6 Hz, 1H), 7.22 (dd, J = 8.9, 1.7 Hz, 1H), 4.41 (dd, J = 9.2, 6.8 Hz, 1H), 4.00 (q, J = 7.0 Hz, 1H), 3.81 - 3.59 (m, 3H), 1.63 (s, 9H), 1.26 (s, 3H), 1.18 (s, 3H).

**tert-butyl 4-amino-3-[[(3S)-4,4-dimethyltetrahydrofuran-3-yl]amino]benzoate (I-1042):** A solution of tert-butyl 3-[[(3S)-4,4-dimethyltetrahydrofuran-3-yl]amino]-4-nitro-benzoate (4.35 g, 12.9 mmol) in EtOAc (86 mL) was degassed by cycling the mixture between argon and vacuum 3x. Next, palladium on carbon (10.0 %, 1.38 g, 1.29 mmol) was added followed by degassing by cycling the mixture between argon and vacuum. Next, the mixture was stirred at rt with a balloon of hydrogen for 24 hr. The mixture was filtered through Celite and concentrated in vacuo to give desired product. ES/MS m/z: 307.2 (M + H)⁺. 1H NMR (400 MHz, Chloroform-d) δ 7.40 (d, J = 16.5 Hz, 2H), 6.81 (s, 1H), 4.33 (d, J = 8.6 Hz, 1H), 3.82 - 3.54 (m, 4H), 1.60 (s, 9H), 1.23 (s, 3H), 1.17 (s, 2H).

### Preparation of Intermediate I-1043

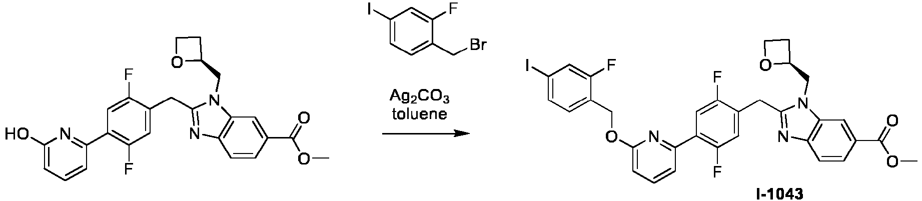

**Methyl (S)-2-(2,5-difluoro-4-(6-((2-fluoro-4-iodobenzyl)oxy)pyridin-2-yl)benzyl)-1-(oxetan-2-ylmethyl)-1H-benzo[d]imidazole-6-carboxylate (Intermediate I-1043):** A slurry of methyl (S)-2-(2,5-difluoro-4-(6-hydroxypyridin-2-yl)benzyl)-1-(oxetan-2-ylmethyl)-1H-benzo[d]imidazole-6-carboxylate (250 mg, 0.54 mmol), 1-(bromomethyl)-2-fluoro-4-iodobenzene (203 mg, 0.65 mmol), and silver carbonate (370 mg, 1.3 mmol) in toluene (5 mL) was heated at 70 °C for 1 hr. The mixture was filtered through celite, washing with EtOAc, concentrated, and purified (24g GOLD, 0-100% EtOAc in Hex). ES/MS: 700.1 (M+H⁺).

### Preparation of Intermediate I-1044

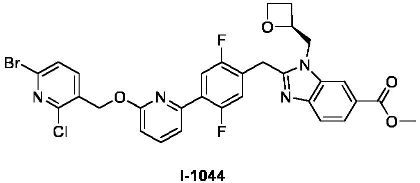

**Methyl (S)-2-(4-(6-((6-bromo-2-chloropyridin-3-yl)methoxy)pyridin-2-yl)-2,5-difluorobenzyl)-1-(oxetan-2-ylmethyl)-1H-benzo[d]imidazole-6-carboxylate (I-1044):** Methyl (S)-2-(4-(6-((6-bromo-2-chloropyridin-3-yl)methoxy)pyridin-2-yl)-2,5-difluorobenzyl)-1-(oxetan-2-ylmethyl)-1H-benzo[d]imidazole-6-carboxylate was prepared in a manner as described for Intermediate **I-18** substituting 6-bromo-3-(bromomethyl)-2-chloropyridine for 4-bromo-1-(bromomethyl)-2-fluorobenzene and methyl (S)-2-(2,5-difluoro-4-(6-hydroxypyridin-2-yl)benzyl)-1-(oxetan-2-ylmethyl)-1H-benzo[d]imidazole-6-carboxylate **(I-9)** for tert-butyl 2-[[2,5-difluoro-4-(6-hydroxy-2-pyridyl)phenyl]methyl]-3-(2-methoxyethyl)benzimidazole-5-carboxylate **(I-17).** ES/MS: 671.1 (M+H⁺).

### Preparation of Intermediate I-1045

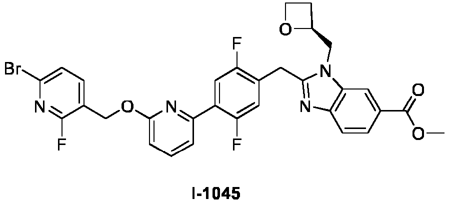

**Methyl (S)-2-(4-(6-((6-bromo-2-fluoropyridin-3-yl)methoxy)pyridin-2-yl)-2,5-difluorobenzyl)-1-(oxetan-2-ylmethyl)-1H-benzo[d]imidazole-6-carboxylate (I-1045):** Methyl (S)-2-(4-(6-((6-bromo-2-fluoropyridin-3-yl)methoxy)pyridin-2-yl)-2,5-difluorobenzyl)-1-(oxetan-2-ylmethyl)-1H-benzo[d]imidazole-6-carboxylate was prepared in a manner as described for Intermediate **I-18** substituting 6-bromo-3-(bromomethyl)-2-fluoropyridine for 4-bromo-1-(bromomethyl)-2-fluorobenzene and methyl (S)-2-(2,5-difluoro-4-(6-hydroxypyridin-2-yl)benzyl)-1-(oxetan-2-ylmethyl)-1H-benzo[d]imidazole-6-carboxylate **(I-9)** for tert-butyl 2-[[2,5-difluoro-4-(6-hydroxy-2-pyridyl)phenyl]methyl]-3-(2-methoxyethyl)benzimidazole-5-carboxylate **(I-17).** ES/MS: 653.2 (M+H⁺).

### Preparation of Intermediate I-1046

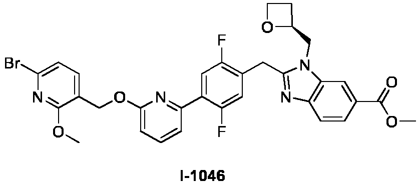

**Methyl (S)-2-(4-(6-((6-bromo-2-methoxypyridin-3-yl)methoxy)pyridin-2-yl)-2,5-difluorobenzyl)-1-(oxetan-2-ylmethyl)-1H-benzo[d]imidazole-6-carboxylate (I-1046):** Methyl (S)-2-(4-(6-((6-bromo-2-methoxypyridin-3-yl)methoxy)pyridin-2-yl)-2,5-difluorobenzyl)-1-(oxetan-2-ylmethyl)-1H-benzo[d]imidazole-6-carboxylate was prepared in a manner as described for Intermediate **I-18** substituting 6-bromo-3-(chloromethyl)-2-methoxypyridine for 4-bromo-1-(bromomethyl)-2-fluorobenzene and methyl (S)-2-(2,5-difluoro-4-(6-hydroxypyridin-2-yl)benzyl)-1-(oxetan-2-ylmethyl)-1H-benzo[d]imidazole-6-carboxylate **(I-9)** for tert-butyl 2-[[2,5-difluoro-4-(6-hydroxy-2-pyridyl)phenyl]methyl]-3-(2-methoxyethyl)benzimidazole-5-carboxylate **(I-17).** ES/MS: 665.2 (M+H⁺).

### Preparation of Intermediate I-1047

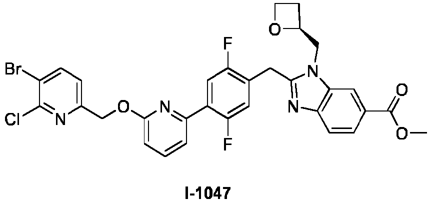

**Methyl (S)-2-(4-(6-((5-bromo-6-chloropyridin-2-yl)methoxy)pyridin-2-yl)-2,5-difluorobenzyl)-1-(oxetan-2-ylmethyl)-1H-benzo[d]imidazole-6-carboxylate (I-1047):** Methyl (S)-2-(4-(6-((6-bromo-2-methoxypyridin-3-yl)methoxy)pyridin-2-yl)-2,5-difluorobenzyl)-1-(oxetan-2-ylmethyl)-1H-benzo[d]imidazole-6-carboxylate was prepared in a manner as described for Intermediate **I-21** substituting 3-bromo-2-chloro-6-(chloromethyl)pyridine for 5-bromo-2-(bromomethyl)thiazole. ES/MS: 671.1 (M+H⁺).

### Preparation of Intermediate I-1048

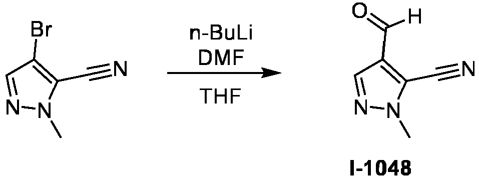

**4-formyl-1-methyl-1H-pyrazole-5-carbonitrile (I-1048):** n-BuLi (2.5M, 0.645 mL, 1.61 mmol) was added dropwise to a solution of 4-bromo-2-methyl-pyrazole-3-carbonitrile (250 mg, 1.34 mmol) in THF (12 mL) at -78 °C. Next the solution was stirred for 15 min. Next, DMF (0.21 mL, 2.69 mmol) was added dropwise, followed by stirring for 20 min. Upon completion of time, the mixture was quenched with saturated NH₄Cl and water, and warmed to rt. The desired product was extracted with EtOAc (2x) and organic was washed with brine, dried over MgSO₄, filtered, concentrated, and purified (ISCO 12g, 0-80% EtOAc in Hex).

### Preparation of Intermediate I-1054

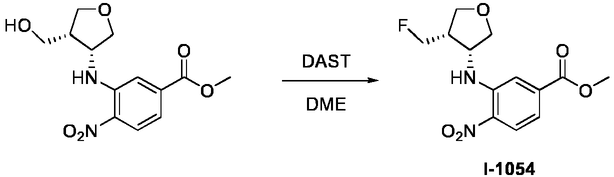

**Methyl 3-((cis-4-(fluoromethyl)tetrahydrofuran-3-yl)amino)-4-nitrobenzoate:** To a solution of methyl 3-((cis-4-(hydroxymethyl)tetrahydrofuran-3-yl)amino)-4-nitrobenzoate (100 mg, 0.338 mmol) in 1,2-dimethoxyethane (4 mL) at -50° C, DAST (0.054 mL, 0.41 mmol) in 1,2-dimethoxyethane (2 mL) was added. The reaction was stirred for 10 min at -50 °C, then warmed up to rt and stirred for an additional 4 hr. Next, cold saturated NaHCOs (20 mL) was added to the mixture. The product was extracted with EtOAc (3x). The combined organic layers were washed with brine, dried over MgSO₄, filtered, concentrated, redissolved in DMSO, and purified (RP HPLC, 0-100% ACN in H₂O). ES/MS: 299.0 (M+H⁺).

### Preparation of Intermediate I-1055

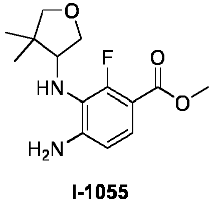

**Methyl 4-amino-3-((4,4-dimethyltetrahydrofuran-3-yl)amino)-2-fluorobenzoate:** Methyl 4-amino-3-((4,4-dimethyltetrahydrofuran-3-yl)amino)-2-fluorobenzoate **(I-1055)** was prepared in a manner as described for Intermediate **I-1** substituting methyl 2,3-difluoro-4-nitrobenzoate for methyl 3-fluoro-4-nitro-benzoate and 4,4-dimethyltetrahydrofuran-3-amine hydrochloride for 2-methoxyethylamine. ES/MS: 283.0 (M+H⁺).

### Preparation of Intermediate I-1056 (Peak 1) and I-1057 (Peak 2)

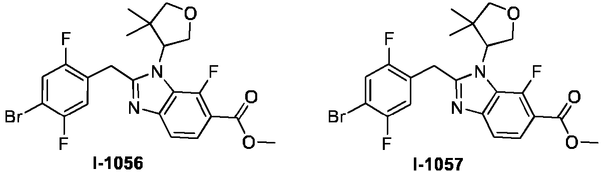

**Methyl 2-(4-bromo-2,5-difluorobenzyl)-1-(4,4-dimethyltetrahydrofuran-3-yl)-7-fluoro-1H-benzo[d]imidazole-6-carboxylate:** Methyl 2-(4-bromo-2,5-difluorobenzyl)-1-(4,4-dimethyltetrahydrofuran-3-yl)-7-fluoro-1H-benzo[d]imidazole-6-carboxylate was prepared in a manner as described for Intermediate **I-13** substituting methyl 4-amino-3-((4,4-dimethyltetrahydrofuran-3-yl)amino)-2-fluorobenzoate for ethyl (S)-4-amino-3-fluoro-5-((oxetan-2-ylmethyl)amino)benzoate and 2-(4-bromo-2,5-difluoro-phenyl)acetic acid for 2-(4-bromo-2-fluorophenyl)acetic acid, separating the two enantiomers (Peak 1, 1-1056) and (Peak 2, I-1057) by chiral SFC. ES/MS: 497.0 (M+H⁺).

### Preparation of Intermediate I-1059

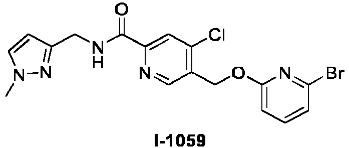

**5-(((6-bromopyridin-2-yl)oxy)methyl)-4-chloro-N-((1-methyl-1H-pyrazol-3-yl)methyl)picolinamide:** 5-(((6-bromopyridin-2-yl)oxy)methyl)-4-chloro-N-((1-methyl-1H-pyrazol-3-yl)methyl)picolinamide was prepared in a manner as described for Intermediate **I-1273** substituting (1-methylpyrazol-3-yl)methanamine for methylamine hydrochloride. ES/MS: 437.9 (M+H⁺).

### Preparation of Intermediate I-1065

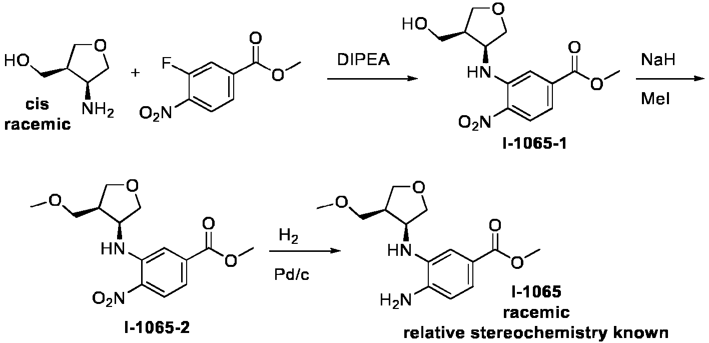

**Methyl 4-amino-3-(((1-(fluoromethyl)cyclopropyl)methyl)amino)benzoate (I-1065-1-1):** To a suspension of methyl 3-fluoro-4-nitro-benzoate (120mg, 0.603 mmol), racemic cis 4-aminotetrahydrofuran-3-yl]methanol (77.7 mg, 0.66 mmol) in THF (4 mL) and DMF (2 mL), N,N-diisopropylethylamine (0.525 mL, 3.01 mmol) was added. The solution was heated at 80 °C for 18 hr. Upon completion of time the crude mixture was diluted with EtOAc, washed with 5% LiCl and brine. The organic extract was dried over sodium sulfate and purified by silica gel column chromatography (eluent: EtOAc/hexanes) to afford the title compound. ES/MS: 297.2 (M+H⁺).

**Methyl 3-[[-4-(methoxymethyl)tetrahydrofuran-3-yl]amino]-4-nitro-benzoate(I-1065-2):** To a solution of methyl 3-[[4-(hydroxymethyl)tetrahydrofuran-3-yl]amino]-4-nitro-benzoate(75 mg, 0.253 mmol) in DMF (4 mL) was added NaH (24.2 mg, 0.633 mmol, 60% dispersion) at 0 °C. To this solution methyl iodide (35.9 mg, 0.253 mmol) was added and the resulting solution stirred for1 hour at rt. Next, the solution was then split between saturated aqueous ammonium chloride and EtOAc, the aqueous layer was extracted with EtOAc five times and washed with brine. The organic extract was dried over sodium sulfate and purified by silica gel column chromatography (eluent: EtOAc/hexanes) to afford the title compound. ES/MS: 311.2 (M+H⁺)

**Methyl 4-amino-3-[[-4-(methoxymethyl)tetrahydrofuran-3-yl]amino]benzoate(I-1065, racemic, relative stereochemistry known):** A solution of methyl 3-[[-4-(methoxymethyl)tetrahydrofuran-3-yl]amino]-4-nitro-benzoate (80 mg, 0.258 mmol) in EtOAc (5 mL) was degassed by cycling between argon and vacuum 3x. Then palladium on carbon (10.0 %, 27.4 mg, 0.258 mmol) was added to the mixture and the mixture was degassed with Ar/vac and stirred at rt with a balloon of hydrogen for 24 hr. The mixture was filtered through Celite and concentrated in vacuo to give the title compound. ES/MS: 281.2 (M+H⁺)

### Preparation of Intermediate I-1066

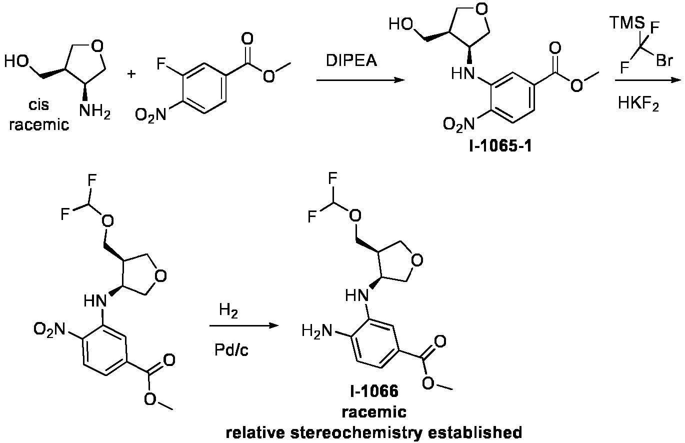

**Methyl 4-amino-3-(((1-(fluoromethyl)cyclopropyl)methyl)amino)benzoate (cis, relative stereochemistry known):** To a suspension of methyl 3-fluoro-4-nitro-benzoate (120mg, 0.603 mmol), 4-aminotetrahydrofuran-3-yl]methanol (77.7 mg, 0.66 mmol) in THF (4 mL) and DMF (2 mL), N,N-diisopropylethylamine (0.525 mL, 3.01 mmol) was added. The resulting solution was heated at 80 °C for 18 hr. The crude mixture was diluted with EtOAc, washed with 5% LiCl and brine. The organic extract was dried over sodium sulfate and purified by silica gel column chromatography (eluent: EtOAc/hexanes) to afford desired product. ES/MS: 297.2 (M+H⁺)

**Methyl 3-[[-4-(difluoromethoxymethyl)tetrahydrofuran-3-yl]amino]-4-nitro-benzoate:** To a solution of methyl 3-[[-4-(hydroxymethyl)tetrahydrofuran-3-yl]amino]-4-nitro-benzoate(75 mg, 0.253 mmol) in DCM (2 mL) and water (2 mL), trimethyl(bromodifluoromethyl)silane (308 mg, 1.52 mmol, 0.237 mL) and potassium hydrogen fluoride (356, 4.56 mmol) were added. The solution was stirred vigorously overnight at rt. The solution was then split between water and DCM, washed with brine, and dried over sodium sulfate and purified by silica gel column chromatography (eluent: EtOAc/hexanes) to afford desired product. ES/MS: 347.6 (M+H⁺)

**Methyl 4-amino-3-[[-4-(difluoromethoxymethyl)tetrahydrofuran-3-yl]amino]benzoate (I-1066):** A solution of methyl 3-[[-4-(difluoromethoxymethyl)tetrahydrofuran-3-yl]amino]-4-nitro-benzoate (88 mg, 0.254 mmol) in EtOAc (5 mL) was degassed by cycling the mixture between argon and vacuum 3x. Next, palladium on carbon (10.0 %, 27.0 mg, 0.254 mmol) was added and then the mixture was degassed by cycling the mixture between argon and vacuum and stirred at rt with a balloon of hydrogen for 24 hr. The mixture was filtered through Celite and concentrated in vacuo to give desired product. ES/MS: 317.2 (M+H⁺)

### Preparation of Intermediate I-1067

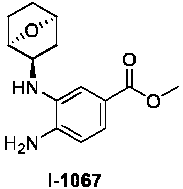

**Methyl 4-amino-3-[[(1R,2R,4S)-7-oxabicyclo[2.2.1]heptan-2-yl]amino]benzoate (I-1067):** Methyl 4-amino-3-[[(1R,2R,4S)-7-oxabicyclo[2.2.1]heptan-2-yl]amino]benzoate **(I-1067)** was prepared in a manner as described for Intermediate **I-1** substituting (1R,2R,4S)-7-oxabicyclo[2.2.1]heptan-2-amine for 2-methoxyethylamine. ES/MS: 263.5 (M+H⁺).

### Preparation of Intermediate I-1068

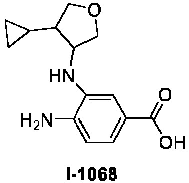

**2-[[4-[6-[(4-cyano-2-fluoro-phenyl)methoxy]-2-pyridyl]-2,5-difluorophenyl]methyl]-3-(4-cyclopropyltetrahydrofuran-3-yl)benzimidazole-5-carboxylic acid (I-1068):** Methyl 4-amino-3-[(4-cyclopropyltetrahydrofuran-3-yl)amino]benzoate (I-1068) as a mixture of four different stereoisomers was prepared in a manner as described for Intermediate **I-72** substituting 4-cyclopropyltetrahydrofuran-3-amine hydrochloride for 2-methoxyethylamine and methyl 3-fluoro-4-nitro-benzoate for methyl 3-fluoro-5-iodo-4-nitrobenzoate. ES/MS: 624.6 (M+H⁺).

### Preparation of Intermediate I-1069

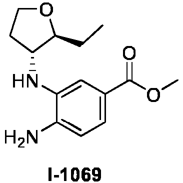

**Methyl 4-amino-3-[[(2S,3R)-2-ethyltetrahydrofuran-3-yl]amino]benzoate (I-1069):** methyl 4-amino-3-[[(2S,3R)-2-ethyltetrahydrofuran-3-yl]amino]benzoate (I-1069) was prepared in a manner as described for Intermediate **I-1** substituting (2S,3R)-2-ethyltetrahydrofuran-3-amine for 2-methoxyethylamine. ES/MS: 265.2 (M+H⁺).

### Preparation of Intermediate I-1070

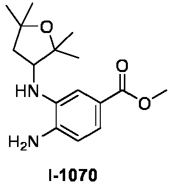

**Methyl 4-amino-3-[(2,2,5,5-tetramethyltetrahydrofuran-3-yl)amino]benzoate (I-1070):** Methyl 4-amino-3-[(2,2,5,5-tetramethyltetrahydrofuran-3-yl)amino]benzoate (I-1070) was prepared in a manner as described for Intermediate **I-1** substituting 2,2,5,5-tetramethyltetrahydrofuran-3-amine for 2-methoxyethylamine. ES/MS: 293.2 (M+H⁺).

### Preparation of Intermediate I-1071

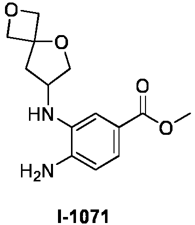

**Methyl 4-amino-3-(2,5-dioxaspiro[3.4]octan-7-ylamino)benzoate (I-1071):** Methyl 4-amino-3-(2,5-dioxaspiro[3.4]octan-7-ylamino)benzoate **(I-1071)** was prepared in a manner as described for Intermediate **I-1** substituting 2,5-dioxaspiro[3.4]octan-7-amine for 2-methoxyethylamine. ES/MS: 279.2 (M+H⁺).

### Preparation of Intermediate I-1072

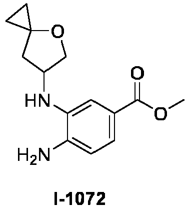

**Methyl 4-amino-3-(4-oxaspiro[2.4]heptan-6-ylamino)benzoate (I-1072):** Methyl 4-amino-3-(4-oxaspiro[2.4]heptan-6-ylamino)benzoate **(I-1072)** was prepared in a manner as described for Intermediate **I-1** substituting 4-oxaspiro[2.4]heptan-6-amine for 2-methoxyethylamine. ES/MS: 263.2 (M+H⁺).

### Preparation of Intermediate I-1075

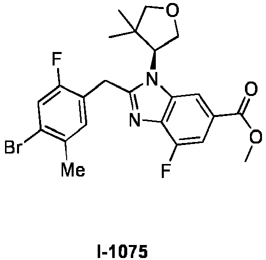

**Methyl 2-[(4-bromo-2-fluoro-5-methyl-phenyl)methyl]-3-[(3S)-4,4-dimethyltetrahydrofuran-3-yl]-7-fluoro-benzimidazole-5-carboxylate(I-1075):** This compound was prepared in a manner as described for **I-103**, substituting 2-(4-bromo-2-chloro-5-methyl-phenyl)acetic acid for 2-(4-bromo-2-fluoro-5-methyl-phenyl)acetic acid. ES/MS: 493.1, 495.0 (M+H+).

### Preparation of Intermediate I-1076

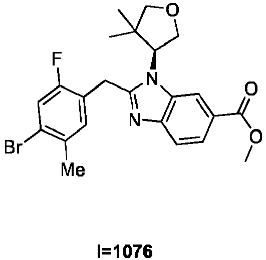

**Methyl 2-[(4-bromo-2-fluoro-5-methyl-phenyl)methyl]-3-[(3S)-4,4-dimethyltetrahydrofuran-3-yl]benzimidazole-5-carboxylate (I-1076):** This compound was in a manner as described for **I-2** substituting 2-(4-bromo-2-fluoro-phenyl)acetic acid with 2-(4-bromo-2-fluoro-5-methyl-phenyl)acetic acid and **I-1** with **I-80**. ES/MS: 492.6, 494.6 (M+H+).

### Preparation of Intermediate I-1078

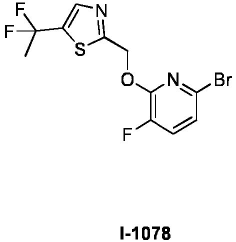

**2-[(6-bromo-3-fluoro-2-pyridyl)oxymethyl]-5-(1,1-difluoroethyl)thiazole (I-1078):** 2-[(6-bromo-3-fluoro-2-pyridyl)oxymethyl]-5-(1,1-difluoroethyl)thiazole **(I-1078)** was prepared in a manner as described for intermediate **I-1034** substituting 2-(bromomethyl)-5-(1,1-difluoroethyl)thiazole for 6-(bromomethyl)-1-methyl-benzotriazole and 6-bromo-3-fluoro-pyridin-2-ol for 6-bromopyridin-2-ol. ES/MS: 351.2, 353.2 (M+H+).

### Preparation of Intermediate I-1079

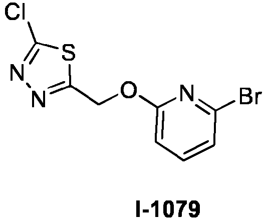

**2-[(6-bromo-2-pyridyl)oxymethyl]-5-chloro-1,3,4-thiadiazole (I-1079):** 2-[(6-bromo-2-pyridyl)oxymethyl]-5-chloro-1,3,4-thiadiazole **(I-1079)** was prepared in a manner as described for intermediate **I-1040** substituting (5-chloro-1,3,4-thiadiazol-2-yl)methanol for 6-(bromomethyl)-1-methyl-benzotriazole and 2-bromo-6-fluoro-pyridine for 4-bromo-2-fluoro-pyrimidine. ES/MS: 308.0, 310.0 (M+H+).

### Preparation of Intermediate I-1080

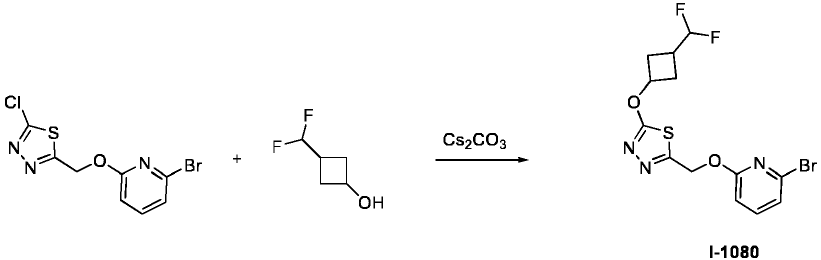

**2- [(6-bromo-2-pyridyl)oxymethyl]-5-[3-(difluoromethyl)cyclobutoxy]-1,3,4-thiadiazole (I-1080):** 2-[(6-bromo-2-pyridyl)oxymethyl]-5-chloro-1,3,4-thiadiazole (45.0 mg, 0.15 mmol), 3-(difluoromethyl)cyclobutanol(27.0 mg, 0.22 mmol), and cesium carbonate (95.7 mg, 0.294 mmol) were added to 4 mL of acetonitrile and the resulting mixture was heated to 50 °C Celsius for 4 hours. The mixture was cooled to rt, filtered, concentrated, and purified by column chromatography to give the product. ES/MS: 392.0, 394.0 (M+H+).

### Preparation of Intermediate I-1081

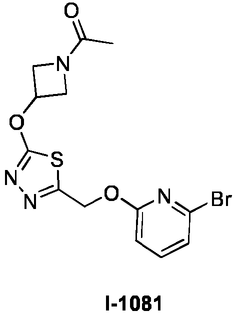

**1-[3-[[5-[(6-bromo-2-pyridyl)oxymethyl]-1,3,4-thiadiazol-2-yl]oxy]azetidin-1-yl]ethenone (I-1081):** 1-[3-[[5-[(6-bromo-2-pyridyl)oxymethyl]-1,3,4-thiadiazol-2-yl]oxy]azetidin-1-yl]ethenone **(I-1081)** was made in a manner as describe for **I-1080** substituting 3-(difluoromethyl)cyclobutanol with 1-(3-hydroxyazetidin-1-yl)ethenone. ES/MS: 385.0, 387.0 (M+H+).

### Preparation of Intermediate I-1082

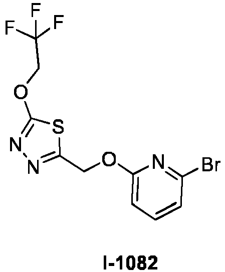

**2- [(6-bromo-2-pyridyl)oxymethyl] -5-(2,2,2-trifluoroethoxy)-1,3,4-thiadiazole (I-1082):** 2-[(6-bromo-2-pyridyl)oxymethyl]-5-(2,2,2-trifluoroethoxy)-1,3,4-thiadiazole **(I-1082)** was prepared in a manner as described for **I-1080** substituting 3-(difluoromethyl)cyclobutanol with 2,2,2-trifluoroethanol. ES/MS: 370.0, 372.0 (M+H+).

### Preparation of Intermediate I-1083

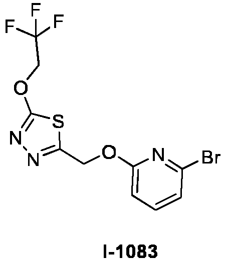

**2-[(6-bromo-2-pyridyl)oxymethyl]-5-(2,2-difluoroethoxy)-1,3,4-thiadiazole(I-1083):** 2-[(6-bromo-2-pyridyl)oxymethyl]-5-(2,2-difluoroethoxy)-1,3,4-thiadiazole **(I-1083)** was prepared in a manner as described for **I-1080** substituting 3-(difluoromethyl)cyclobutanol with 2,2-difluoroethanol. ES/MS: 352.0, 354.0 (M+H+).

### Preparation of Intermediate I-1084

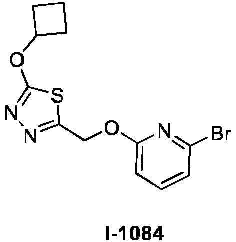

**2-[(6-bromo-2-pyridyl)oxymethyl]-5-(cyclobutoxy)-1,3,4-thiadiazole** (I-1084): 2-[(6-bromo-2-pyridyl)oxymethyl]-5-(cyclobutoxy)-1,3,4-thiadiazole **(I-1084)** was prepared in a manner as described for **I-1080** substituting 3-(difluoromethyl)cyclobutanol with cyclobutanol. ES/MS: 344.0, 346.0 (M+H+).

### Preparation of Intermediate I-1085

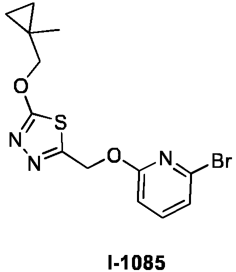

**2-[(6-bromo-2-pyridyl)oxymethyl]-5-[(1-methylcyclopropyl)methoxy]-1,3,4-thiadiazole (I-1085):** 2-[(6-bromo-2-pyridyl)oxymethyl]-5-[(1-methylcyclopropyl)methoxy]-1,3,4-thiadiazole **(I-1085)** was prepared in a manner as described for **I-1080** substituting 3-(difluoromethyl)cyclobutanol with (1-methylcyclopropyl)methanol. ES/MS: 356.0, 358.0 (M+H+).

### Preparation of Intermediate I-1089

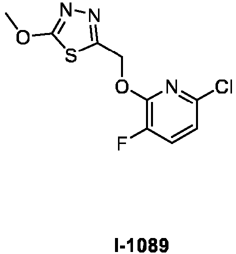

**2-(((6-chloro-3-fluoropyridin-2-yl)oxy)methyl)-5-methoxy-1,3,4-thiadiazole (I-1089):** 2-(((6-chloro-3-fluoropyridin-2-yl)oxy)methyl)-5-methoxy-1,3,4-thiadiazole **(I-1089)** was prepared in a manner as described for intermediate **I1034** substituting 2-(chloromethyl)-5-methoxy-1,3,4-thiadiazole for 6-(bromomethyl)-1-methyl-benzotriazol and 6-bromo-3-fluoro-pyridin-2-ol for 6-bromopyridin-2-ol. ES/MS: 275.2, 277.2 (M+H+).

### Preparation of Intermediate I1090

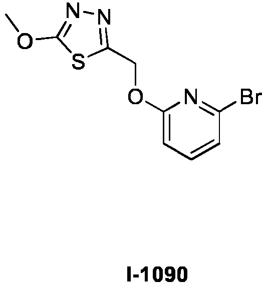

**2-(((6-bromopyridin-2-yl)oxy)methyl)-5-methoxy-1,3,4-thiadiazole (I-1090):** 2-(((6-bromopyridin-2-yl)oxy)methyl)-5-methoxy-1,3,4-thiadiazole **(I-1090)** was prepared in a manner as described for intermediate **I-1034** substituting 2-(chloromethyl)-5-methoxy-1,3,4-thiadiazole for 6-(bromomethyl)-1-methyl-benzotriazole. ES/MS: 302.2, 304.2 (M+H+).

### Preparation of Intermediate I-1093

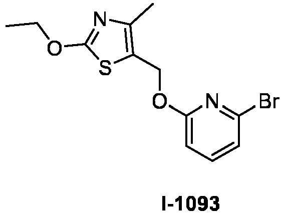

**5-[(6-bromo-2-pyridyl)oxymethyl]-2-ethoxy-4-methyl-thiazole (I-1093):** 5-[(6-bromo-2-pyridyl)oxymethyl]-2-ethoxy-4-methyl-thiazole **(I-1093)** was prepared in a manner as described for intermediate **I-1040** substituting (2-ethoxy-4-methyl-thiazol-5-yl)methanol for 6-(bromomethyl)-1-methyl-benzotriazole and 2-bromo-6-fluoro-pyridine for 4-bromo-2-fluoro-pyrimidine. ES/MS: 327.0, 329.0 (M+H+).

### Preparation of Intermediate I-1094

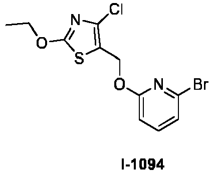

**5-[(6-bromo-2-pyridyl)oxymethyl]-4-chloro-2-ethoxy-thiazole (I-1094):** 5-[(6-bromo-2-pyridyl)oxymethyl]-4-chloro-2-ethoxy-thiazole **(I-1094)** was prepared in a manner as described for intermediate **I-1040** substituting (4-chloro-2-ethoxy-thiazol-5-yl)methanol for 6-(bromomethyl)-1-methyl-benzotriazole and 2-bromo-6-fluoro-pyridine for 4-bromo-2-fluoro-pyrimidine. ES/MS: 349.0, 351.0 (M+H+).

### Preparation of Intermediate I-1095

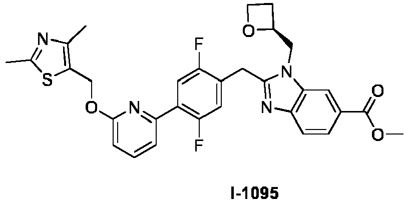

**Methyl 2-[[4-[6-[(2,4-dimethylthiazol-5-yl)methoxy]-2-pyridyl]-2,5-difluorophenyl]methyl]-3-[[(2S)-oxetan-2-yl]methyl]benzimidazole-5-carboxylate (I-1095):** methyl 2-[[4-[6-[(2,4-dimethylthiazol-5-yl)methoxy]-2-pyridyl]-2,5-difluoro-phenyl]methyl]-3-[[(2S)-oxetan-2-yl]methyl]benzimidazole-5-carboxylate **(I-1095)** was prepared in a manner as described for intermediate **I-1269** substituting methyl 2-[[2,5-difluoro-4-(6-hydroxy-2-pyridyl)phenyl]methyl]-3-[[(2S)-oxetan-2-yl]methyl]benzimidazole-5-carboxylate for ethyl 2-[[2,5-difluoro-4-(6-hydroxy-2-pyridyl)phenyl]methyl]-7-fluoro-3-[[(2S)-oxetan-2-yl]methyl]benzimidazole-5-carboxylate and 5-(chloromethyl)-2,4-dimethyl-thiazole for 5-bromo-2-(chloromethyl)-3-fluoro-pyridine. ES/MS: 591.2 (M+H+).

### Preparation of Intermediate I-1096

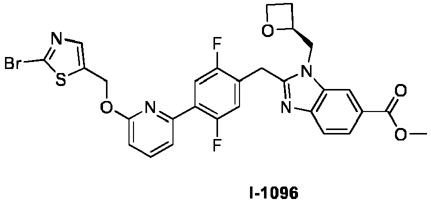

**Methyl 2-[[4-[6-[(2-bromothiazol-5-yl)methoxy]-2-pyridyl]-2,5-difluorophenyl]methyl]-3-[[(2S)-oxetan-2-yl]methyl]benzimidazole-5-carbox**ylate **(I-1096):** methyl 2-[[4-[6-[(2-bromothiazol-5-yl)methoxy]-2-pyridyl]-2,5-difluoro-phenyl]methyl]-3-[[(2S)-oxetan-2-yl]methyl]benzimidazole-5-carboxylate **(I-1096)** was prepared in a manner as described for intermediate **I-21** substituting 2-bromo-5-(bromomethyl)thiazole for 5-bromo-2-(bromomethyl)thiazole. ES/MS: 641.0, 643.0 (M+H+).

### Preparation of Intermediate I-1097

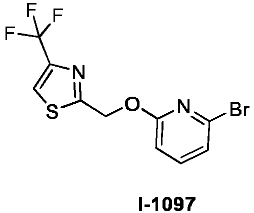

**2-(((6-bromopyridin-2-yl)oxy)methyl)-4-(trifluoromethyl)thiazole (I-1097):** 2-(((6-bromopyridin-2-yl)oxy)methyl)-4-(trifluoromethyl)thiazole **(I-1097)** was prepared in a manner as described for intermediate **I-1040** substituting (4-(trifluoromethyl)thiazol-2-yl)methanol for 6-(bromomethyl)-1-methyl-benzotriazole and 2-bromo-6-fluoro-pyridine for 4-bromo-2-fluoro-pyrimidine. ES/MS: 337.0, 340.0 (M+H+).

### Preparation of Intermediate I-1101

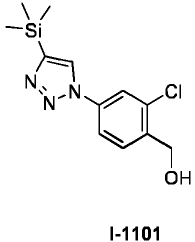

**(2-chloro-4-(4-(trimethylsilyl)-1H-1,2,3-triazol-1-yl)phenyl)methanol (I-1101):** (2-chloro-4-(4-(trimethylsilyl)-1H-1,2,3-triazol-1-yl)phenyl)methanol **(I-1101)** was prepared in a manner as described for **I-50** substituting methyl 6-chloropyridine-3-carboxylate with methyl 2-chloro-4-fluoro-benzoate and ethynylcyclopropane with ethynyl(trimethyl)silane. ES/MS: 282.0 (M+H+).

### Preparation of Intermediate I-1103

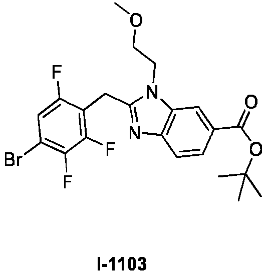

**tert-butyl 2-[(4-bromo-2,3,6-trifluoro-phenyl)methyl]-3-(2-methoxyethyl)benzimidazole-5-carboxylate (I-1103):** tert-butyl 2-[(4-bromo-2,3,6-trifluorophenyl)methyl]-3-(2-methoxyethyl)benzimidazole-5-carboxylate **(I-1103)** was prepared in a manner as described for **I-2** substituting 2-(4-bromo-2-fluoro-phenyl)acetic acid with 2-(4-bromo-2,3,6-trifluorophenyl)acetic acid and **I-1** with **I-6**. ES/MS: 500.0, 502.0 (M+H+).

### Preparation of Intermediate I-1104

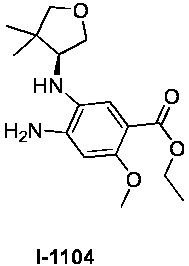

**Ethyl 4-amino-5-[[(3S)-4,4-dimethyltetrahydrofuran-3-yl]amino]-2-methoxybenzoate (I-1104):** ethyl 4-amino-5-[[(3S)-4,4-dimethyltetrahydrofuran-3-yl]amino]-2-methoxy-benzoate **(I-1104)** was prepared in a manner as described for **I-1** substituting methyl 3-fluoro-4-nitro-benzoate with methyl 5-fluoro-2-methoxy-4-nitro-benzoate and 2-Methoxyethylamine with (3S)-4,4-dimethyltetrahydrofuran-3-amine;hydrochloride. ES/MS: 309.0 (M+H+).

### Preparation of Intermediate I-1105

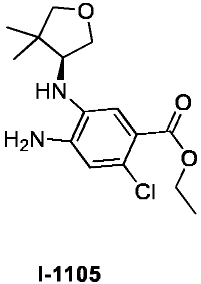

**Ethyl 4-amino-2-chloro-5-[[(3S)-4,4-dimethyltetrahydrofuran-3-yl] amino] benzoate (I-1105):** ethyl 4-amino-2-chloro-5-[[(3S)-4,4-dimethyltetrahydrofuran-3-yl]amino]benzoate **(I-1105)** was prepared in a manner as described for **I-1** substituting methyl 3-fluoro-4-nitrobenzoate with ethyl 2-chloro-5-fluoro-4-nitro-benzoate and 2-Methoxyethylamine with (3S)-4,4-dimethyltetrahydrofuran-3-amine;hydrochloride. ES/MS: 313.0 (M+H+).

### Preparation of Intermediate I-1106

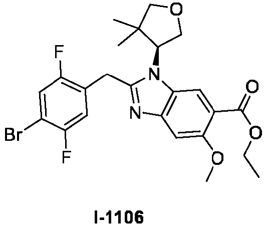

**Methyl 2-[(4-bromo-2,5-difluoro-phenyl)methyl]-3-[(3S)-4,4-dimethyltetrahydrofuran-3-yl]-6-methoxy-benzimidazole-5-carboxylate (I-1106):** methyl 2-[(4-bromo-2,5-difluoro-phenyl)methyl]-3-[(3S)-4,4-dimethyltetrahydrofuran-3-yl]-6-methoxy-benzimidazole-5-carboxylate **(I-1106)** was prepared in a manner as described for **I-2** substituting 2-(4-bromo-2-fluoro-phenyl)acetic acid with 2-(4-bromo-2,5-difluoro-phenyl)acetic acid and **I-1** with **I-1104**. ES/MS: 510.0 (M+H+).

### Preparation of Intermediate I-1107

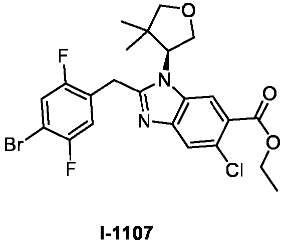

**Ethyl 2-[(4-bromo-2,5-difluoro-phenyl)methyl]-6-chloro-3-[(3S)-4,4-dimethyltetrahydrofuran-3-yl]benzimidazole-5-carboxylate (I-1107):** ethyl 2-[(4-bromo-2,5-difluoro-phenyl)methyl]-6-chloro-3-[(3S)-4,4-dimethyltetrahydrofuran-3-yl]benzimidazole-5-carboxylate **(I-1107)** was prepared in a manner as described for **I-2** substituting 2-(4-bromo-2-fluoro-phenyl)acetic acid with 2-(4-bromo-2,5-difluoro-phenyl)acetic acid and **I-1** with **I-1105.** ES/MS: 528.0 (M+H+).

### Preparation of Intermediate I-1110

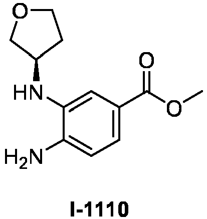

**Methyl (R)-4-amino-3-((tetrahydrofuran-3-yl)amino)benzoate (I-1110):** methyl (R)-4-amino-3-((tetrahydrofuran-3-yl)amino)benzoate (I-1110) was prepared in a manner as described for Intermediate **I-1** substituting 4-methylbenzenesulfonic acid;(3R)-tetrahydrofuran-3-amine for 2-methoxyethylamine. ES/MS: 237.2 (M+H+).

### Preparation of Intermediate I-1111

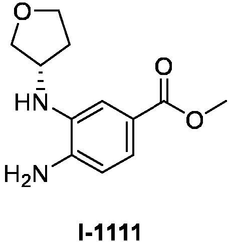

**Methyl (S)-4-amino-3-((tetrahydrofuran-3-yl)amino)benzoate (I-1111):** methyl (S)-4-amino-3-((tetrahydrofuran-3-yl)amino)benzoate (I-1111) was prepared in a manner as described for Intermediate **I-1** substituting 4-methylbenzenesulfonic acid;(3S)-tetrahydrofuran-3-amine for 2-methoxyethylamine. ES/MS: 237.1 (M+H+).

### Preparation of Intermediate I-1112

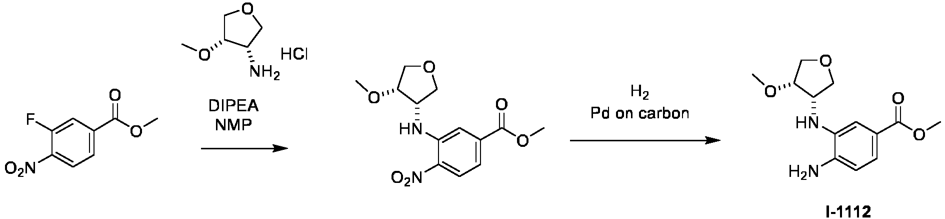

**Methyl 4-amino-3-((cis-4-methoxytetrahydrofuran-3-yl)amino)benzoate (I-1112):** methyl 4-amino-3-((cis-4-methoxytetrahydrofuran-3-yl)amino)benzoate was prepared in a manner as described for Intermediate **I-1** substituting cis-4-methoxytetrahydrofuran-3-amine hydrochloride for 2-methoxyethylamine. ES/MS: 367.2 (M+H+)

### Preparation of Intermediate I-1113

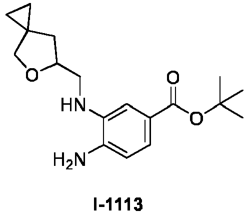

**tert-butyl 3-(((5-oxaspiro[2.4]heptan-6-yl)methyl)amino)-4-aminobenzoate (I-1113):** tert-butyl 3-(((5-oxaspiro[2.4]heptan-6-yl)methyl)amino)-4-aminobenzoate was prepared in a manner as described for Intermediate **I-6** substituting (5-oxaspiro[2.4]heptan-6-yl)methanamine hydrochloride for 2-methoxyethylamine. ES/MS: 319.0 (M+H+)

### Preparation of Intermediate I-1114

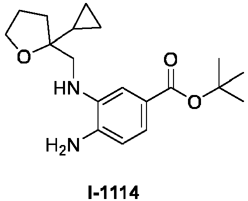

**tert-butyl 4-amino-3-(((2-cyclopropyltetrahydrofuran-2-yl)methyl)amino)benzoate (I-1114):** tert-butyl 3-(((5-oxaspiro[2.4]heptan-6-yl)methyl)amino)-4-aminobenzoate was prepared in a manner as described for Intermediate **I-6** substituting (2-cyclopropyltetrahydrofuran-2-yl)methanamine for 2-methoxyethylamine. ES/MS: 333.3 (M+H+)

### Preparation of Intermediate I-1115

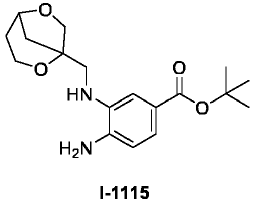

**tert-butyl 3-(((2,6-dioxabicyclo[3.2.1]octan-1-yl)methyl)amino)-4-aminobenzoate (I-1115):** tert-butyl 3-(((2,6-dioxabicyclo[3.2.1]octan-1-yl)methyl)amino)-4-aminobenzoate was prepared in a manner as described for Intermediate **I-6** substituting 2,6-dioxabicyclo[3.2.1]octan-1-ylmethanamine hydrochloride for 2-methoxyethylamine. ES/MS: 335.2 (M+H+)

### Preparation of Intermediate I-1116

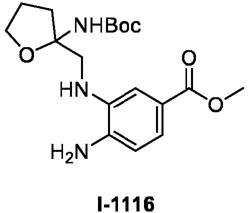

**methyl 4-amino-3-(((2-((tert-butoxycarbonyl)amino)tetrahydrofuran-2-yl)methyl)amino)benzoate (I-1116):** methyl 4-amino-3-(((2-((tert-butoxycarbonyl)amino)tetrahydrofuran-2-yl)methyl)amino)benzoate was prepared in a manner as described for Intermediate **I-1** substituting tert-butyl (2-(aminomethyl)tetrahydrofuran-2-yl)carbamate hydrochloride for 2-methoxyethylamine. ES/MS: 333.3 (M+H+)

### Preparation of Intermediate I-1117

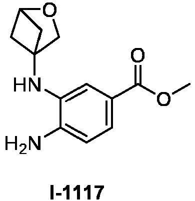

**Methyl 4-amino-3-(2-oxabicyclo[2.1.1]hexan-4-ylamino)benzoate (I-1117):** methyl 4-amino-3-(2-oxabicyclo[2.1.1]hexan-4-ylamino)benzoate was prepared in a manner as described for Intermediate **I-1** substituting 2-oxabicyclo[2.1.1]hexan-4-amine hydrochloride for 2-methoxyethylamine. ES/MS: 249.2 (M+H+)

### Preparation of Intermediate I-1118

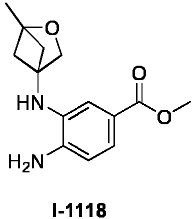

**methyl 4-amino-3-[(1-methyl-2-oxabicyclo[2.1.1]hexan-4-yl)amino]benzoate (I-1118):** methyl 4-amino-3-[(1-methyl-2-oxabicyclo[2.1.1]hexan-4-yl)amino]benzoate was prepared in a manner as described for Intermediate **I-1** substituting 1-methyl-2-oxabicyclo[2.1.1]hexan-4-amine hydrochloride for 2-methoxyethylamine. ES/MS: 249.2 (M+H+)

### Preparation of Intermediate I-1119

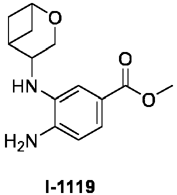

**Methyl 4-amino-3-(2-oxabicyclo[3.1.1]heptan-4-ylamino)benzoate (I-1119):** methyl 4-amino-3-(2-oxabicyclo[3.1.1]heptan-4-ylamino)benzoate was prepared in a manner as described for Intermediate **I-1** substituting 2-oxabicyclo[3.1.1]heptan-4-amine for 2-methoxyethylamine. ES/MS: 263.2 (M+H+)

### Preparation of Intermediate I-1120

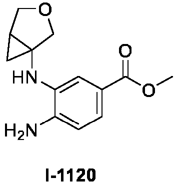

**Methyl 4-amino-3-(3-oxabicyclo[3.1.0]hexan-1-ylamino)benzoate (I-1120):** methyl 4-amino-3-(3-oxabicyclo[3.1.0]hexan-1-ylamino)benzoate was prepared in a manner as described for Intermediate **I-1** substituting 3-oxabicyclo[3.1.0]hexan-1-amine hydrochloride for 2-methoxyethylamine. ES/MS: 249.2 (M+H+)

### Preparation of Intermediate I-1121

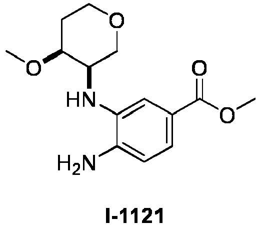

**Methyl 4-amino-3-[[cis-4-methoxytetrahydropyran-3-yl]amino]benzoate (I-1121):** methyl 4-amino-3-[[cis-4-methoxytetrahydropyran-3-yl]amino]benzoate was prepared in a manner as described for Intermediate **I-1** substituting cis-4-methoxytetrahydropyran-3-amine hydrochloride for 2-methoxyethylamine. ES/MS: 281.1 (M+H+)

### Preparation of Intermediate I-1122

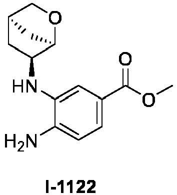

**Methyl 4-amino-3-[[(1r,4r,6s)-2-oxabicyclo[2.2.1]heptan-6-yl]amino]benzoate (I-1122):** methyl 4-amino-3-[[(1r,4r,6s)-2-oxabicyclo[2.2.1]heptan-6-yl]amino]benzoate was prepared in a manner as described for Intermediate **I-1** substituting (1r,4r,6s)-2-oxabicyclo[2.2.1]heptan-6-amine hydrochloride for 2-methoxyethylamine. ES/MS: 263.2 (M+H+)

### Preparation of Intermediate I-1123

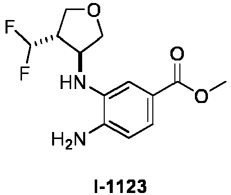

**Methyl 4-amino-3-[[trans-4-(difluoromethyl)tetrahydrofuran-3-yl]amino]benzoate (I-1123):** methyl 4-amino-3-[[trans-4-(difluoromethyl)tetrahydrofuran-3-yl]amino]benzoate was prepared in a manner as described for Intermediate **I-1** substituting trans-4-(difluoromethyl)tetrahydrofuran-3-amine hydrochloride for 2-methoxyethylamine. ES/MS: 287.2 (M+H+)

### Preparation of Intermediate I-1124

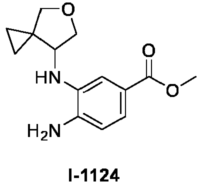

**Methyl 4-amino-3-(5-oxaspiro[2.4]heptan-7-ylamino)benzoate (I-1124):** methyl 4-amino-3-(5-oxaspiro[2.4]heptan-7-ylamino)benzoate was prepared in a manner as described for Intermediate **I-69** substituting 5-oxaspiro[2.4]heptan-7-amine hydrochloride for 4,4-dimethyltetrahydrofuran-3-amine hydrochloride. ES/MS: 263.2 (M+H+)

### Preparation of Intermediate I-1125

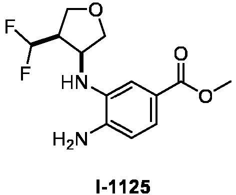

**Methyl 4-amino-3-[[cis-4-(difluoromethyl)tetrahydrofuran-3-yl]amino]benzoate (I-1125):** methyl 4-amino-3-[[trans-4-(difluoromethyl)tetrahydrofuran-3-yl]amino]benzoate was prepared in a manner as described for Intermediate **I-1** substituting cis-4-(difluoromethyl)tetrahydrofuran-3-amine hydrochloride for 2-methoxyethylamine. ES/MS: 287.2 (M+H+)

### Preparation of Intermediate I-1126

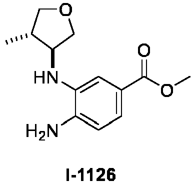

**Methyl 4-amino-3-[[trans-4-methyltetrahydrofuran-3-yl]amino]benzoate (I-1126):** methyl 4-amino-3-[[trans-4-methyltetrahydrofuran-3-yl]amino]benzoate was prepared in a manner as described for Intermediate **I-1** substituting trans-4-methyltetrahydrofuran-3-amine hydrochloride for 2-methoxyethylamine. ES/MS: 251.2 (M+H+)

### Preparation of Intermediate I-1127

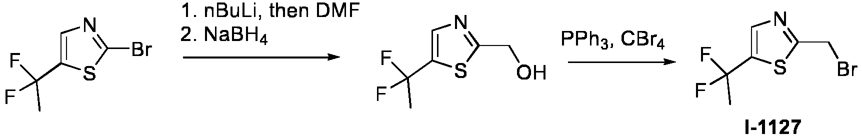

**[5-(1,1-difluoroethyl)thiazol-2-yl]methanol:** 2-bromo-5-(1,1-difluoroethyl)thiazole (1.0 g, 4.4 mmol) in THF (15 mL) was cooled to -78 °C, and nBuLi (2.2 M, 2.5 mL, 5.5 mmol) was added dropwise. The mixture was stirred at -78 °C for 30 min before addition of DMF (1 mL) and the resulting mixture was slowly warmed to rt. The mixture was quenched with saturated aqueous NH₄Cl and diluted with EtOAc, and the layers were separated. The combined organic extracts were washed with brine, dried over MgSO₄, filtered, and concentrated. The residue was dissolved in DCM/MeOH (1:1, 15 mL) and NaBH₄ (0.17 mg, 4.4 mmol) was added in small portions at rt. The mixture was stirred at rt for 1 h before quenching with saturated aqueous and diluting with DCM. The layers were separated, and the combined organic extracts were washed with brine, dried over MgSO₄, filtered, and concentrated. The concentrate was then purification by flash chromatography (eluent: EtOAc/hexanes) yielded the desired product. ES/MS: 180.2 (M+H+)

**2-(bromomethyl)-5-(1,1-difluoroethyl)thiazole (I-1127):** [5-(1,1-difluoroethyl)thiazol-2-yl]methanol (540 mg, 3.0 mmol) in DCM (10 mL) was cooled to 0 °C, and triphenylphosphine (870 mg, 3.33 mmol) and CBr₄ (1.1 g, 3.33 mmol) were added, respectively. The mixture was warmed to rt and stirred for 2 h before concentrating *in vacuo.* Purification by flash chromatography (eluent: EtOAc/hexanes) yielded the desired product. ES/MS: 244.0 (M+H+)

### Preparation of Intermediate I-1130

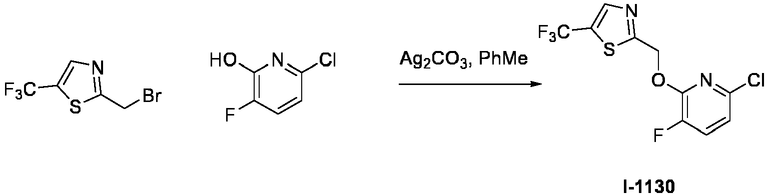

**2-[(6-chloro-3-fluoro-2-pyridyl)oxymethyl]-5-(trifluoromethyl)thiazole: 2-[(6-chloro-3-fluoro-2-pyridyl)oxymethyl]-5-(trifluoromethyl)thiazole (I-1130):** 2-[(6-chloro-3-fluoro-2-pyridyl)oxymethyl]-5-(trifluoromethyl)thiazole was prepared in a manner as described for Intermediate **I-1129** substituting 2-(bromomethyl)-5-(trifluoromethyl)thiazole for 2-(bromomethyl)-3-fluoro-5-(trifluoromethyl)pyridine and 6-chloro-3-fluoro-pyridin-2-ol for 6-bromopyridin-2-ol. ES/MS: 313.1 (M+H+)

### Preparation of Intermediate I-1131

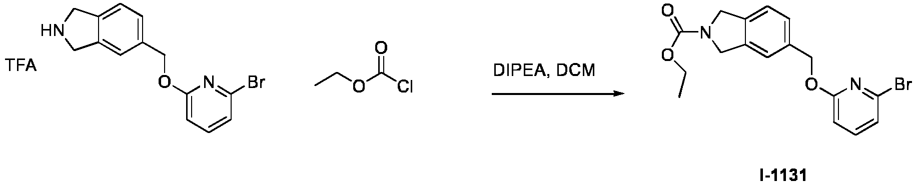

**Ethyl 5-[(6-bromo-2-pyridyl)oxymethyl]isoindoline-2-carboxylate (I-1131)**: 5-[(6-bromo-2-pyridyl)oxymethyl]isoindoline;2,2,2-trifluoroacetic acid (60 mg, 0.14 mmol) in DCM was added DIPEA (0.075 mL, 0.43 mmol) and ethyl chloroformate (0.016 mL, 0.17 mmol) at rt, respectively. The mixture was stirred at rt for 2 h before concentrating *in vacuo.* Purification by flash chromatography (eluent: EtOAc/hexanes) yielded the desired product. ES/MS: 379.0 (M+H+)

### Preparation of Intermediate I-1132

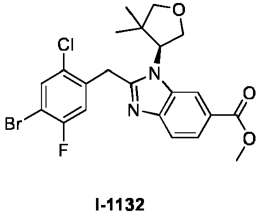

**Methyl 2-[(4-bromo-2-chloro-5-fluoro-phenyl)methyl]-3-[(3S)-4,4-dimethyltetrahydrofuran-3-yl]benzimidazole-5-carboxylate (I-1132):** Methyl 2-[(4-bromo-2-chloro-5-fluoro-phenyl)methyl]-3-[(3S)-4,4-dimethyltetrahydrofuran-3-yl]benzimidazole-5-carboxylate was prepared in a manner as described for Intermediate **I-2** substituting methyl 4-amino-3-((4,4-dimethyltetrahydrofuran-3-yl)amino)benzoate **I-80** for methyl 4-amino-3-(2-methoxyethylamino)benzoate **I-1** and 2-(4-bromo-2-chloro-5-fluoro-phenyl)acetic acid for 2-(4-bromo-2-fluoro-phenyl)acetic acid. ES/MS: 497.3 (M+H+).

### Preparation of Intermediate I-1133

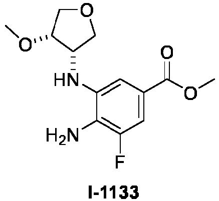

**Methyl 4-amino-3-fluoro-5-((cis-4-methoxytetrahydrofuran-3-yl)amino)benzoate (I-1133):** methyl 4-amino-3-fluoro-5-((cis-4-methoxytetrahydrofuran-3-yl)amino)benzoate was prepared in a manner as described for Intermediate **I-1** substituting trans-4-methoxytetrahydrofuran-3-amine hydrochloride for 2-methoxyethylamine and methyl 3,5-difluoro-4-nitro-benzoate for methyl 3-fluoro-4-nitro-benzoate. ES/MS: 285.2 (M+H+)

### Preparation of Intermediate I-1134

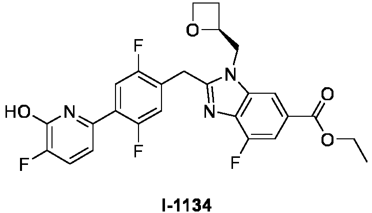

**Ethyl 2-[[2,5-difluoro-4-(5-fluoro-6-hydroxy-2-pyridyl)phenyl]methyl]-7-fluoro-3-[[(2S)-oxetan-2-yl]methyl]benzimidazole-5-carboxylate (I-1134):** ethyl 2-[[2,5-difluoro-4-(5-fluoro-6-hydroxy-2-pyridyl)phenyl]methyl]-7-fluoro-3-[[(2S)-oxetan-2-yl]methyl]benzimidazole-5-carboxylate was prepared in a manner as described for Intermediate **I-9** substituting **I-14** for **I-8** and 2-benzyloxy-6-bromo-3-fluoro-pyridine for 2-benzyloxy-6-bromopyridine. ES/MS: 516.1 (M+H+).

### Preparation of Intermediate I-1135

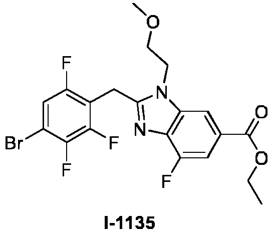

**Ethyl 2-(4-bromo-2,3,6-trifluorobenzyl)-4-fluoro-1-(2-methoxyethyl)-1H-benzo[d]imidazole-6-carboxylate (I-1135):** ethyl 2-(4-bromo-2,3,6-trifluorobenzyl)-4-fluoro-1-(2-methoxyethyl)-1H-benzo[d]imidazole-6-carboxylate was prepared in a manner as described for **I-2** substituting ethyl 4-amino-3-fluoro-5-((2-methoxyethyl)amino)benzoate **I-1032** for methyl 4-amino-3-(2 methoxyethylamino)benzoate **I-1** and 2-(4-bromo-2,5,6-trifluorophenyl)acetic acid for 2-(4-bromo-2,5-difluorophenyl)acetic acid. ES/MS: 490.2. M+H+).

### Preparation of Intermediate I-1136

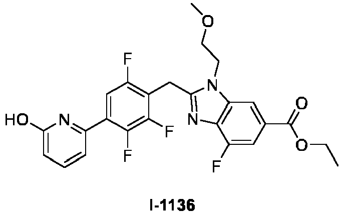

**Ethyl 7-fluoro-3-(2-methoxyethyl)-2-[[2,3,6-trifluoro-4-(6-hydroxy-2-pyridyl)phenyl]methyl]benzimidazole-5-carboxylate (I-1136):** ethyl 7-fluoro-3-(2-methoxyethyl)-2-[[2,3,6-trifluoro-4-(6-hydroxy-2-pyridyl)phenyl]methyl]benzimidazole-5-carboxylate was prepared in a manner as described for Intermediate **I-9** substituting ethyl 2-(4-bromo-2,3,6-trifluorobenzyl)-4-fluoro-1-(2-methoxyethyl)-1H-benzo[d]imidazole-6-carboxylate **I-1135** for **I-8.** ES/MS: 504.1 (M+H+).

### Preparation of Intermediate I-1137

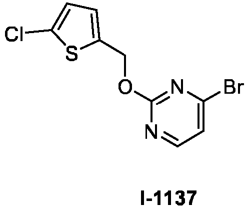

**4-bromo-2-[(5-chloro-2-thienyl)methoxy]pyrimidine (I-1137):** 4-bromo-2-[(5-chloro-2-thienyl)methoxy]pyrimidine was prepared in a manner as described for Intermediate **I-43** substituting (5-chloro-2-thienyl)methanol for (1-methylimidazol-4-yl)methanol and 4-bromo-2-fluoro-pyrimidine for 2-bromo-6-fluoropyridine. ES/MS: 307.0 (M+H+)

### Preparation of Intermediate I-1138

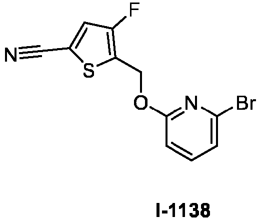

**5-[(6-bromo-2-pyridyl)oxymethyl]-4-fluoro-thiophene-2-carbonitrile (I-1138):** 5-[(6-bromo-2-pyridyl)oxymethyl]-4-fluoro-thiophene-2-carbonitrile was prepared in a manner as described for Intermediate **I-43** substituting 4-fluoro-5-(hydroxymethyl)thiophene-2-carbonitrile for (1-methylimidazol-4-yl)methanol. ES/MS: 313.1 (M+H+)

### Preparation of Intermediate I-1139

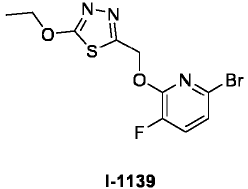

**2-[(6-bromo-3-fluoro-2-pyridyl)oxymethyl]-5-ethoxy-1,3,4-thiadiazole (I-1139):** 2-[(6-bromo-3-fluoro-2-pyridyl)oxymethyl]-5-ethoxy-1,3,4-thiadiazole was prepared in a manner as described for Intermediate **I-1129** substituting 2-(chloromethyl)-5-ethoxy-1,3,4-thiadiazole for 2-(bromomethyl)-3-fluoro-5-(trifluoromethyl)pyridine and 6-bromo-3-fluoro-pyridin-2-ol for 6-bromopyridin-2-ol. ES/MS: 335.0 (M+H+)

### Preparation of Intermediate I-1140

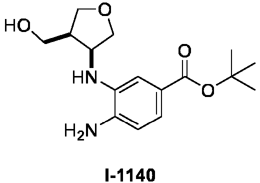

**tert-butyl 4-amino-3-((cis-4-(hydroxymethyl)tetrahydrofuran-3-yl)amino)benzoate** (I-1140): tert-butyl 4-amino-3-((cis-4-(hydroxymethyl)tetrahydrofuran-3-yl)amino)benzoate was prepared in a manner as described for Intermediate **I-6** substituting (cis-4-aminotetrahydrofuran-3-yl)methanol for 2-methoxyethylamine. ES/MS: 339.2 (M+H+)

### Preparation of Intermediate I-1141

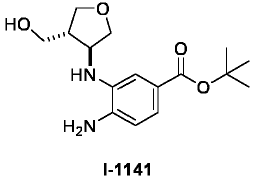

**tert-butyl 4-amino-3-((trans-4-(hydroxymethyl)tetrahydrofuran-3-yl)amino)benzoate (I-1141):** tert-butyl 4-amino-3-((trans-4-(hydroxymethyl)tetrahydrofuran-3-yl)amino)benzoate was prepared in a manner as described for Intermediate **I-6** substituting (trans-4-aminotetrahydrofuran-3-yl)methanol for 2-methoxyethylamine. ES/MS: 339.2 (M+H+)

### Preparation of Intermediate I-1142

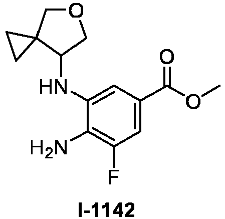

**Methyl 3-((5-oxaspiro[2.4]heptan-7-yl)amino)-4-amino-5-fluorobenzoate (I-1142):** methyl 3-((5-oxaspiro[2.4]heptan-7-yl)amino)-4-amino-5-fluorobenzoate was prepared in a manner as described for Intermediate **I-1** substituting 5-oxaspiro[2.4]heptan-7-amine hydrochloride for 2-methoxyethylamine and methyl 3,5-difluoro-4-nitro-benzoate for methyl 3-fluoro-4-nitro-benzoate. ES/MS: 281.2 (M+H+)

### Preparation of Intermediate I-1144

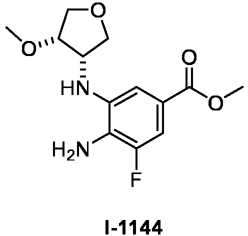

**Methyl 4-amino-3-fluoro-5-((cis-4-methoxytetrahydrofuran-3-yl)amino)benzoate** (I-1144): methyl 4-amino-3-fluoro-5-((cis-4-methoxytetrahydrofuran-3-yl)amino)benzoate was prepared in a manner as described for Intermediate **I-1** substituting 5-oxaspiro[2.4]heptan-7-amine hydrochloride for 2-methoxyethylamine and methyl 3,5-difluoro-4-nitro-benzoate for methyl 3-fluoro-4-nitro-benzoate. ES/MS: 281.2 (M+H+)

### Preparation of Intermediate I-1145

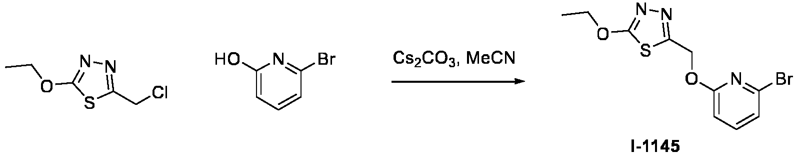

**2- [(6-bromo-2-pyridyl)oxymethyl] -5-ethoxy-1,3,4-thiadiazole (I-1145):** 2-(chloromethyl)-5-ethoxy-1,3,4-thiadiazole (113 mg, 0.63 mmol), 6-bromopyridin-2-ol (100 mg, 0.58 mmol), and cesium carbonate (300 mg, 0.92 mmol) in MeCN (7 mL) was stirred at 60 °C for 2 h. The mixture was cooled to rt and filtered through a Celite plug. The resulting solution was concentrated and purified by flash chromatography (eluent: EtOAc/hexanes) to yield the desired product. ES/MS: 316.8 (M+H+).

### Preparation of Intermediate I-1146

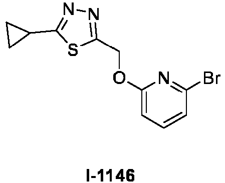

**2-[(6-bromo-2-pyridyl)oxymethyl]-5-cyclopropyl-1,3,4-thiadiazole (I-1146):** 2-[(6-bromo-2-pyridyl)oxymethyl]-5-cyclopropyl-1,3,4-thiadiazole was prepared in a manner as described for Intermediate **I-1145** substituting 2-(chloromethyl)-5-cyclopropyl-1,3,4-thiadiazole for 2-(chloromethyl)-5-ethoxy-1,3,4-thiadiazole. ES/MS: 312.8 (M+H+)

### Preparation of Intermediate I-1147

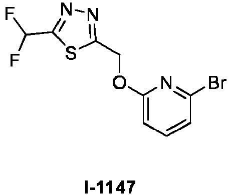

**2-[(6-bromo-2-pyridyl)oxymethyl]-5-(difluoromethyl)-1,3,4-thiadiazole (I-1147):** 2-[(6-bromo-2-pyridyl)oxymethyl]-5-(difluoromethyl)-1,3,4-thiadiazole was prepared in a manner as described for Intermediate **I-1145** substituting 2-(chloromethyl)-5-(difluoromethyl)-1,3,4-thiadiazole for 2-(chloromethyl)-5-ethoxy-1,3,4-thiadiazole. ES/MS: 322.8 (M+H+)

### Preparation of Intermediate I-1148

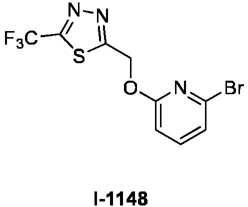

**2-[(6-bromo-2-pyridyl)oxymethyl]-5-(trifluoromethyl)-1,3,4-thiadiazole (I-1148):** 2-[(6-bromo-2-pyridyl)oxymethyl]-5-(difluoromethyl)-1,3,4-thiadiazole was prepared in a manner as described for Intermediate **I-1145** substituting 2-(chloromethyl)-5-(trifluoromethyl)-1,3,4-thiadiazole for 2-(chloromethyl)-5-ethoxy-1,3,4-thiadiazole. ES/MS: 341.8 (M+H+)

### Preparation of Intermediate I-1149

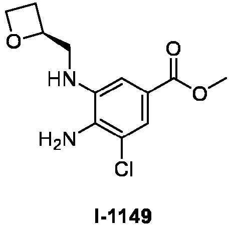

**Methyl (S)-4-amino-3-chloro-5-((oxetan-2-ylmethyl)amino)benzoate (I-1149):** methyl (S)-4-amino-3-chloro-5-((oxetan-2-ylmethyl)amino)benzoate was prepared in a manner as described for Intermediate **1378-I-68** substituting (S)-oxetan-2-ylmethanamine for 2-methoxyethylamine and methyl 3-chloro-5-fluoro-4-nitrobenzoate for methyl 3-fluoro-4-nitrobenzoate. ES/MS: 271.0 (M+H+).

### Preparation of Intermediate I-1150

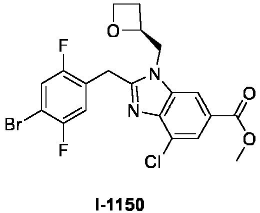

**Methyl (S)-2-(4-bromo-2,5-difluorobenzyl)-4-chloro-1-(oxetan-2-ylmethyl)-1H-benzo[d]imidazole-6-carboxylate (I-1150):** Methyl (S)-2-(4-bromo-2,5-difluorobenzyl)-4-chloro-1-(oxetan-2-ylmethyl)-1H-benzo[d]imidazole-6-carboxylate was prepared in a manner as described for Intermediate **I-2** substituting methyl (S)-4-amino-3-chloro-5-((oxetan-2-ylmethyl)amino)benzoate **I-1149** for methyl 4-amino-3-(2-methoxyethylamino)benzoate-**I-1** and 2-(4-bromo-2,5-difluoro-phenyl)acetic acid for 2-(4-bromo-2-fluoro-phenyl)acetic acid. ES/MS: 504.9 (M+H+).

### Preparation of Intermediate I-1151

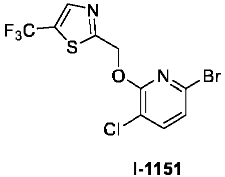

**2-[(6-bromo-3-chloro-2-pyridyl)oxymethyl]-5-(trifluoromethyl)thiazole (I-1151):** 2-[(6-bromo-3-chloro-2-pyridyl)oxymethyl]-5-(trifluoromethyl)thiazole was prepared in a manner as described for Intermediate **I-1129** substituting 2-(bromomethyl)-5-(trifluoromethyl)thiazole for 2-(bromomethyl)-3-fluoro-5-(trifluoromethyl)pyridine and 6-bromo-3-chloro-pyridin-2-ol for 6-bromopyridin-2-ol. ES/MS: 374.8 (M+H+)

### Preparation of Intermediate I-1152

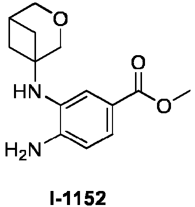

**Methyl 4-nitro-3-(3-oxabicyclo[3.1.1]heptan-1-ylamino)benzoate (I-1152):** Methyl 4-nitro-3-(3-oxabicyclo[3.1.1]heptan-1-ylamino)benzoate was prepared in a manner as described for Intermediate **I-1** substituting 3-oxabicyclo[3.1.1]heptan-1-amine hydrochloride for 2-methoxyethylamine. ES/MS: 293.0 (M+H+).

### Preparation of Intermediate I-1153

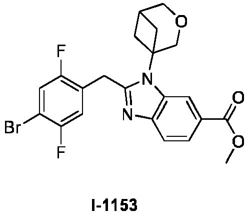

**Methyl 2-[(4-bromo-2,5-difluoro-phenyl)methyl] -3-(3-oxabicyclo [3.1.1]heptan-1-yl)benzimidazole-5-carboxylate (I-1153):** Methyl 2-[(4-bromo-2,5-difluoro-phenyl)methyl]-3-(3-oxabicyclo[3.1.1]heptan-1-yl)benzimidazole-5-carboxylate was prepared in a manner as described for Intermediate **I-2** substituting methyl 4-nitro-3-(3-oxabicyclo[3.1.1]heptan-1-ylamino)benzoate **I-1152** for methyl 4-amino-3-(2-methoxyethylamino)benzoate **I-1** and 2-(4-bromo-2,5-difluoro-phenyl)acetic acid for 2-(4-bromo-2-fluoro-phenyl)acetic acid. ES/MS: 477.0 (M+H+).

### Preparation of Intermediate I-1155

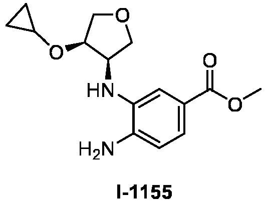

**Methyl 4-amino-3-[[cis-4-(cyclopropoxy)tetrahydrofuran-3-yl]amino]benzoate** (I-1155): Methyl 4-amino-3-[[cis-4-(cyclopropoxy)tetrahydrofuran-3-yl]amino]benzoate was prepared in a manner as described for Intermediate **I-68** substituting cis-4-(cyclopropoxy)tetrahydrofuran-3-amine hydrochloride for 2-methoxyethylamine. ES/MS: 293.0 (M+H+)

### Preparation of Intermediate I-1156

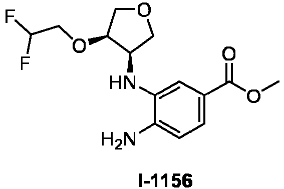

**Methyl 3-[[cis-4-(2,2-difluoroethoxy)tetrahydrofuran-3-yl]amino]-4-nitro-benzoate (I-1156):** Methyl 3-[[cis-4-(2,2-difluoroethoxy)tetrahydrofuran-3-yl]amino]-4-nitro-benzoate was prepared in a manner as described for Intermediate **I-1** substituting cis-4-(2,2-difluoroethoxy)tetrahydrofuran-3-amine hydrochloride for 2-methoxyethylamine. ES/MS: 317.2 (M+H+).

### Preparation of Intermediate I-1159

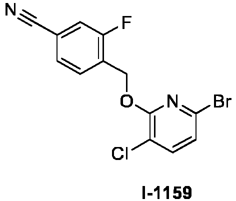

**4-[(6-bromo-3-chloro-2-pyridyl)oxymethyl]-3-fluoro-benzonitrile (I-1159):** 4-[(6-bromo-3-chloro-2-pyridyl)oxymethyl]-3-fluoro-benzonitrile was prepared in a manner as described for Intermediate **I-1129** substituting 4-(bromomethyl)-3-fluorobenzonitrile for 2-(bromomethyl)-3-fluoro-5-(trifluoromethyl)pyridine and 6-bromo-3-chloro-pyridin-2-ol for 6-bromopyridin-2-ol. ES/MS: 342.0 (M+H+)

### Preparation of Intermediate I-1160

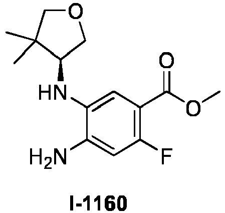

**methyl (S)-4-amino-5-((4,4-dimethyltetrahydrofuran-3-yl)amino)-2-fluorobenzoate** (I-1160): Methyl (S)-4-amino-5-((4,4-dimethyltetrahydrofuran-3-yl)amino)-2-fluorobenzoate was prepared in a manner as described for Intermediate **I-1** substituting (S)-4,4-dimethyltetrahydrofuran-3-amine hydrochloride for 2-methoxyethylamine and methyl 2,5-difluoro-4-nitrobenzoate for methyl 3-fluoro-4-nitrobenzoate. ES/MS: 283.2 (M+H+)

### Preparation of Intermediate I-1161

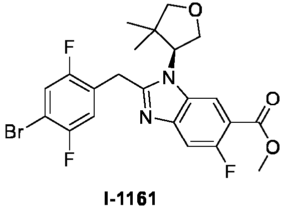

**Methyl 2-[(4-bromo-2,5-difluoro-phenyl)methyl]-3-[(3S)-4,4-dimethyltetrahydrofuran-3-yl]-6-fluoro-benzimidazole-5-carboxylate (I-1161):** Methyl 2-[(4-bromo-2,5-difluoro-phenyl)methyl]-3-[(3S)-4,4-dimethyltetrahydrofuran-3-yl]-6-fluoro-benzimidazole-5-carboxylate was prepared in a manner as described for Intermediate **I-2** substituting methyl (S)-4-amino-5-((4,4-dimethyltetrahydrofuran-3-yl)amino)-2-fluorobenzoate **I-1160** for methyl 4-amino-3-(2-methoxyethylamino)benzoate **I-1** and 2-(4-bromo-2,5-difluorophenyl)acetic acid for 2-(4-bromo-2-fluoro-phenyl)acetic acid. ES/MS: 498.0 (M+H+).

### Preparation of Intermediate I-1162

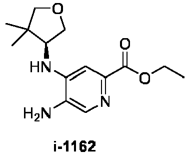

**Ethyl (S)-5-amino-4-((4,4-dimethyltetrahydrofuran-3-yl)amino)picolinate (I-1162):** ethyl (S)-5-amino-4-((4,4-dimethyltetrahydrofuran-3-yl)amino)picolinate was prepared in a manner as described for Intermediate **I-1** substituting (S)-4,4-dimethyltetrahydrofuran-3-amine hydrochloride for 2-methoxyethylamine and ethyl 4-chloro-5-nitropicolinate for methyl 3-fluoro-4-nitrobenzoate. ES/MS: 280.0 (M+H+)

### Preparation of Intermediate I-1163

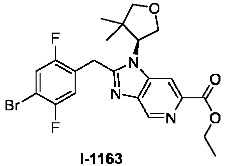

**Ethyl 2-[(4-bromo-2,5-difluoro-phenyl)methyl]-1-[(3S)-4,4-dimethyltetrahydrofuran-3-yl]imidazo[4,5-c]pyridine-6-carboxylate (I-1163):** Ethyl 2-[(4-bromo-2,5-difluoro-phenyl)methyl]-1-[(3S)-4,4-dimethyltetrahydrofuran-3-yl]imidazo[4,5-c]pyridine-6-carboxylate was prepared in a manner as described for Intermediate **I-2** substituting ethyl (S)-5-amino-4-((4,4-dimethyltetrahydrofuran-3-yl)amino)picolinate **I-1162** for methyl 4-amino-3-(2-methoxyethylamino)benzoate **I-1** and 2-(4-bromo-2,5-difluoro-phenyl)acetic acid for 2-(4-bromo-2-fluoro-phenyl)acetic acid. ES/MS: 495.0 (M+H+).

### Preparation of Intermediate I-1166

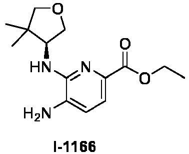

**Ethyl (S)-5-amino-6-((4,4-dimethyltetrahydrofuran-3-yl)amino)picolinate (I-1166):** Ethyl (S)-5-amino-6-((4,4-dimethyltetrahydrofuran-3-yl)amino)picolinate was prepared in a manner as described for Intermediate **I-1** substituting (S)-4,4-dimethyltetrahydrofuran-3-amine hydrochloride for 2-methoxyethylamine and ethyl 6-chloro-5-nitropicolinate for methyl 3-fluoro-4-nitrobenzoate. ES/MS: 280.0 (M+H+)

### Preparation of Intermediate I-1167

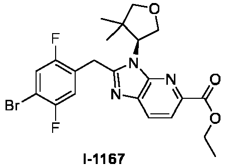

**Ethyl 2-[(4-bromo-2,5-difluoro-phenyl)methyl]-3-[(3S)-4,4-dimethyltetrahydrofuran-3-yl]imidazo[4,5-b]pyridine-5-carboxylate (I-1167):** Ethyl 2-[(4-bromo-2,5-difluoro-phenyl)methyl]-3-[(3S)-4,4-dimethyltetrahydrofuran-3-yl]imidazo[4,5-b]pyridine-5-carboxylate was prepared in a manner as described for Intermediate **I-2** substituting ethyl (S)-5-amino-6-((4,4-dimethyltetrahydrofuran-3-yl)amino)picolinate **I-1166** for methyl 4-amino-3-(2-methoxyethylamino)benzoate **I-1** and 2-(4-bromo-2,5-difluoro-phenyl)acetic acid for 2-(4-bromo-2-fluoro-phenyl)acetic acid. ES/MS: 495.0 (M+H+).

### Preparation of Intermediate I-1173

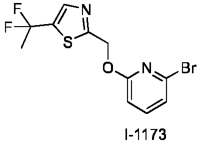

**2-(((6-bromopyridin-2-yl)oxy)methyl)-5-(1,1-difluoroethyl)thiazole (I-1173):** 2-(((6-bromopyridin-2-yl)oxy)methyl)-5-(1,1-difluoroethyl)thiazole was prepared in a manner as described for Intermediate **I-1129** substituting 2-(bromomethyl)-5-(1,1-difluoroethyl)thiazole for 2-(bromomethyl)-3-fluoro-5-(trifluoromethyl)pyridine. ES/MS: 335.0 (M+H+)

### Preparation of Intermediate I-1176

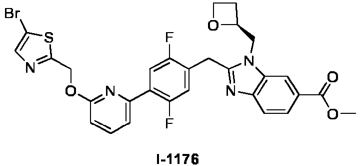

**Methyl (S)-2-(4-(6-((5-bromothiazol-2-yl)methoxy)pyridin-2-yl)-2,5-difluorobenzyl)-1-(oxetan-2-ylmethyl)-1H-benzo[d]imidazole-6-carboxylate (I-1176):** Methyl (S)-2-(4-(6-((5-bromothiazol-2-yl)methoxy)pyridin-2-yl)-2,5-difluorobenzyl)-1-(oxetan-2-ylmethyl)-1H-benzo[d]imidazole-6-carboxylate was prepared in a manner as described for Intermediate **I-1096** substituting 5-bromo-2-(bromomethyl)thiazole for 2-bromo-5-(bromomethyl)thiazole. ES/MS: 641.2 (M+H+)

### Preparation of Intermediate I-1177

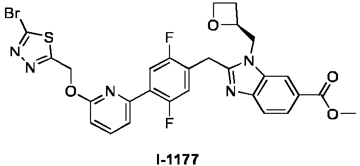

**Methyl (S)-2-(4-(6-((5-bromo-1,3,4-thiadiazol-2-yl)methoxy)pyridin-2-yl)-2,5-difluorobenzyl)-1-(oxetan-2-ylmethyl)-1H-benzo[d]imidazole-6-carboxylate (I-1177):** Methyl (S)-2-(4-(6-((5-bromo-1,3,4-thiadiazol-2-yl)methoxy)pyridin-2-yl)-2,5-difluorobenzyl)-1-(oxetan-2-ylmethyl)-1H-benzo[d]imidazole-6-carboxylate was prepared in a manner as described for Intermediate **I-1096** substituting 2-bromo-5-(chloromethyl)-1,3,4-thiadiazole for 2-bromo-5-(bromomethyl)thiazole. ES/MS: 642.1 (M+H+)

### Preparation of Intermediate I-1178

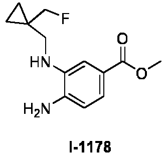

**Methyl 4-amino-3-(((1-(fluoromethyl)cyclopropyl)methyl)amino)benzoate (I-1178):** Methyl 4-amino-3-(((1-(fluoromethyl)cyclopropyl)methyl)amino)benzoate was prepared in a manner as described for Intermediate **I-1** substituting (1-(fluoromethyl)cyclopropyl)methanamine for 2-methoxyethylamine. ES/MS: 253.2 (M+H+).

### Preparation of Intermediate I-1179

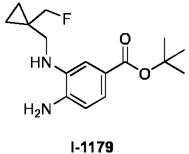

**tert-butyl 4-amino-3-(((1-(fluoromethyl)cyclopropyl)methyl)amino)benzoate** (I-1179): tert-butyl 4-amino-3-(((1-(fluoromethyl)cyclopropyl)methyl)amino)benzoate was prepared in a manner as described for Intermediate **I-6** substituting (1-(fluoromethyl)cyclopropyl)methanamine for 2-methoxyethylamine. ES/MS: 295.2 (M+H+).

### Preparation of Intermediate I-1180

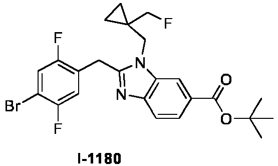

**tert-butyl 2-[(4-bromo-2,5-difluoro-phenyl)methyl]-3-[[1-(fluoromethyl)cyclopropyl]methyl]benzimidazole-5-carboxylate (I-1180):** tert-butyl 2-[(4-bromo-2,5-difluoro-phenyl)methyl]-3-[[1-(fluoromethyl)cyclopropyl]methyl]benzimidazole-5-carboxylate was prepared in a manner as described for Intermediate **I-2** substituting **I-1179** for **I-1** and 2-(4-bromo-2,5-difluorophenyl)acetic acid for 2-(4-bromo-2-fluoro-phenyl)acetic acid. ES/MS: 509, 511 (M+H+).

### Preparation of Intermediate I-1076

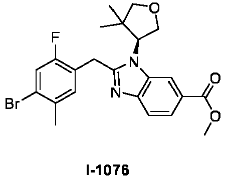

**Methyl 2-[(4-bromo-2-fluoro-5-methyl-phenyl)methyl]-3-[(3S)-4,4-dimethyltetrahydrofuran-3-yl]benzimidazole-5-carboxylate (I-1076):** Methyl 2-[(4-bromo-2-fluoro-5-methyl-phenyl)methyl]-3-[(3 S)-4,4-dimethyltetrahydrofuran-3-yl]benzimidazole-5-carboxylate was prepared in a manner as described for Intermediate **I-2** substituting **I-80** for **I-1** and 2-(4-bromo-2-fluoro-5-methyl-phenyl)acetic acid **I-1277** for 2-(4-bromo-2-fluorophenyl)acetic acid. ES/MS: 475, 477 (M+H+).

### Preparation of Intermediate I-1182

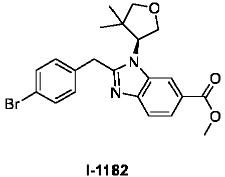

**Methyl 2-[(4-bromophenyl)methyl]-3-[(3S)-4,4-dimethyltetrahydrofuran-3-yl]benzimidazole-5-carboxylate (I-1182):** Methyl 2-[(4-bromophenyl)methyl]-3-[(3S)-4,4-dimethyltetrahydrofuran-3-yl]benzimidazole-5-carboxylate was prepared in a manner as described for Intermediate **I-2** substituting **I-80** for **I-1** and 2-(4-bromophenyl)acetic acid for 2-(4-bromo-2-fluoro-phenyl)acetic acid. ES/MS: 443, 445 (M+H+).

### Preparation of Intermediate I-1183

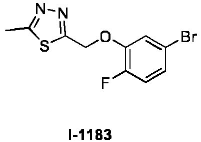

**2-[(5-bromo-2-fluoro-phenoxy)methyl]-5-ethoxy-1,3,4-thiadiazole (I-1183):** 2-[(5-bromo-2-fluoro-phenoxy)methyl]-5-ethoxy-1,3,4-thiadiazole was prepared in a manner as described for Intermediate **I-84** substituting 2-(chloromethyl)-5-ethoxy-1,3,4-thiadiazole for 2-(bromomethyl)thiazole-5-carbonitrile **(I-63)** and 5-bromo-2-fluoro-phenol for 6-bromopyridin-2-ol. ES/MS: 333, 335 (M+H+).

### Preparation of Intermediate I-1184

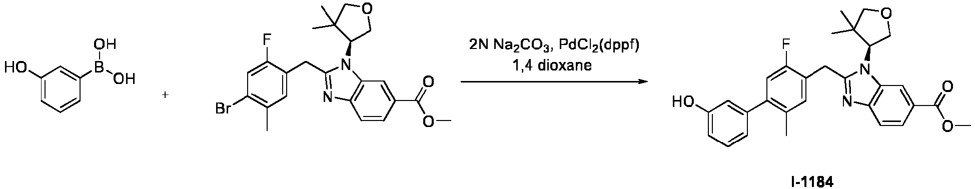

**Methyl 3-[(3S)-4,4-dimethyltetrahydrofuran-3-yl]-2-[[2-fluoro-4-(3-hydroxyphenyl)-5-methyl-phenyl]methyl]benzimidazole-5-carboxylate (I-1184):** A suspension of methyl 2-[(4-bromo-2-fluoro-5-methyl-phenyl)methyl]-3-[(3S)-4,4-dimethyltetrahydrofuran-3-yl]benzimidazole-5-carboxylate (130 mg, 0.273 mmol) **(I-1076),** (3-hydroxyphenyl)boronic acid (75.4 mg, 0.547 mmol), [1,1'-Bis(diphenylphosphino)ferrocene] dichloropalladium(II) ;PdCl₂(dppf) (20.3 mg, 0.0273 mmol, sodium carbonate (2000 mmol/L, 0.273 mL, 0.547 mmol) was degassed. The mixture was heated at 100 °C for 1 hr. Next, the mixture was diluted with EtOAc and water. The organic extract was dried over magnesium sulfate, filtered and concentrated. The crude residue was purified by flash chromatography (eluent: EtOAc/hexanes) to yield desired product. ES/MS: 489.1 (M+H+)

### Preparation of Intermediate I-1185

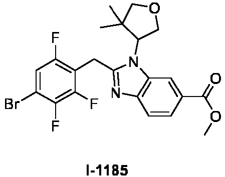

**Methyl 2-(4-bromo-2,3,6-trifluorobenzyl)-1-(4,4-dimethyltetrahydrofuran-3-yl)-1H-benzo[d]imidazole-6-carboxylate (I-1185):** Methyl 2-(4-bromo-2,3,6-trifluorobenzyl)-1-(4,4-dimethyltetrahydrofuran-3-yl)-1H-benzo[d]imidazole-6-carboxylate was prepared in a manner as described for Intermediate **I-2** substituting **I-25** for **I-1** and 2-(4-bromo-2,3,6-trifluorophenyl)acetic acid for 2-(4-bromo-2-fluoro-phenyl)acetic acid. ES/MS: 497, 499 (M+H+).

### Preparation of Intermediate I-1186

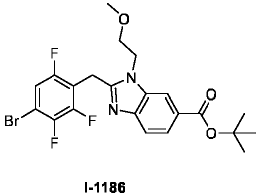

**tert-butyl 2-(4-bromo-2,3,6-trifluorobenzyl)-1-(2-methoxyethyl)-1H-benzo[d]imidazole-6-carboxylate (I-1186):** tert-butyl 2-(4-bromo-2,3,6-trifluorobenzyl)-1-(2-methoxyethyl)-1H-benzo[d]imidazole-6-carboxylate was prepared in a manner as described for Intermediate **I-2** substituting **I-6** for **I-1** and 2-(4-bromo-2,3,6-trifluorophenyl)acetic acid for 2-(4-bromo-2-fluoro-phenyl)acetic acid. ES/MS: 499, 501 (M+H+).

### Preparation of Intermediate I-1187

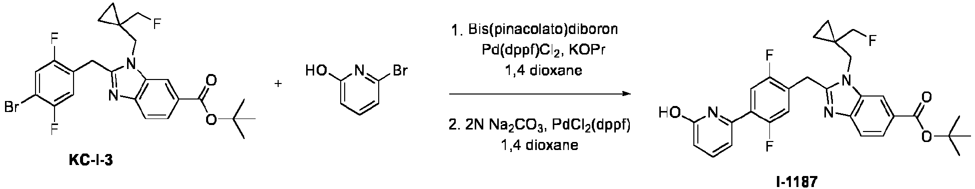

**Tert-butyl 2-(2,5-difluoro-4-(6-hydroxypyridin-2-yl)benzyl)-1-((1-(fluoromethyl)cyclopropyl)methyl)-1H-benzo[d]imidazole-6-carboxylate (I-1187):** A suspension of tert-butyl 2-[(4-bromo-2,5-difluoro-phenyl)methyl]-3-[[1-(fluoromethyl)cyclopropyl]methyl]benzimidazole-5-carboxylate (500 mg, 0.98 mmol), [1,1'-Bis(diphenylphosphino)ferrocene] dichloropalladium(II) ;PdCl₂(dppf) (72.8 mg, 0.098 mmol), potassium propionate (330 mg, 2.94 mmol), and Bis(pinacolato)diboron (324 mg, 1.28 mmol) was degassed and then heated at 110 °C for 2hr. Upon completion of time, sodium carbonate (2000 mmol/L, 0.98 mL, 1.96 mmol), 6-bromopyridin-2-ol 256 mg, 1.47 mmol), and [1,1'-Bis(diphenylphosphino)ferrocene] dichloropalladium(II) ;PdCl₂(dppf) (30.4 mg, 0.04 mmol) were added and resulting mixture was degassed. The mixture was then heated at 100 °C for 1 hr. Next, the mixture was diluted with EtOAc and water. The organic extract was dried over magnesium sulfate, filtered and concentrated. The crude residue was purified by flash chromatography (eluent: EtOAc/hexanes) to yield desired product. ES/MS: 523.7 (M+H+)

### Preparation of Intermediate I-1188

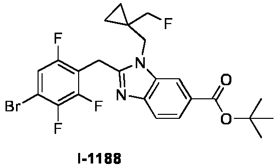

**Tert-butyl 2-[(4-bromo-2,3,6-trifluoro-phenyl)methyl]-3-[[1-(fluoromethyl)cyclopropyl]methyl]benzimidazole-5-carboxylate (I-1188):** tert-butyl 2-[(4-bromo-2,3,6-trifluoro-phenyl)methyl]-3-[[1-(fluoromethyl)cyclopropyl]methyl]benzimidazole-5-carboxylate was prepared in a manner as described for Intermediate **I-2** substituting **I-1179** for **I-1** and 2-(4-bromo-2,3,6-trifluorophenyl)acetic acid for 2-(4-bromo-2-fluoro-phenyl)acetic acid. ES/MS: 527, 529 (M+H+).

### Preparation of Intermediate I-1189

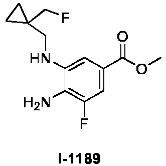

**Methyl 4-amino-3-fluoro-5-[[1-(fluoromethyl)cyclopropyl]methylamino]benzoate (I-1189):** Methyl 4-amino-3-fluoro-5-[[1-(fluoromethyl)cyclopropyl]methylamino]benzoate was prepared in a manner as described for Intermediate **I-1** substituting (1-(fluoromethyl)cyclopropyl)methanamine for 2-methoxyethylamine and methyl 3,5-difluoro-4-nitrobenzoate for methyl 3-fluoro-4-nitrobenzoate.ES/MS: 270.2 (M+H+).

### Preparation of Intermediate I-1190

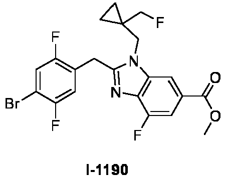

**Methyl 2-[(4-bromo-2,5-difluoro-phenyl)methyl]-7-fluoro-3-[[1-(fluoromethyl)cyclopropyl]methyl]benzimidazole-5-carboxylate (I-1190):** Methyl 2-[(4-bromo-2,5-difluoro-phenyl)methyl]-7-fluoro-3-[[1-(fluoromethyl)cyclopropyl]methyl]benzimidazole-5-carboxylate was prepared in a manner as described for Intermediate **I-13** substituting **I-1189** for **I-5** and 2-(4-bromo-2,5-difluorophenyl)acetic acid for 2-(4-bromo-2-fluoro-phenyl)acetic acid. ES/MS: 485, 487 (M+H+).

### Preparation of Intermediate I-1191

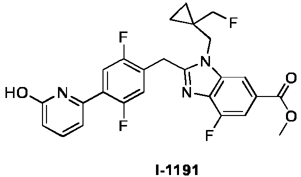

**Methyl 2-[[2,5-difluoro-4-(6-hydroxy-2-pyridyl)phenyl]methyl]-7-fluoro-3-[[1-(fluoromethyl)cyclopropyl]methyl]benzimidazole-5-carboxylate (I-1191):** Methyl 2-[[2,5-difluoro-4-(6-hydroxy-2-pyridyl)phenyl]methyl]-7-fluoro-3-[[1-(fluoromethyl)cyclopropyl]methyl]benzimidazole-5-carboxylate was prepared in a manner as described for Intermediate **I-1187** substituting **I-1190** for **I-1180**. ES/MS: 499, 501 (M+H+).

### Preparation of Intermediate I-1192

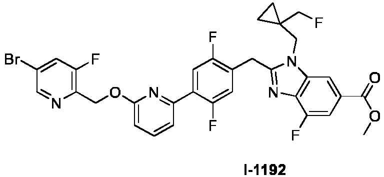

**Methyl 2-[[4-[6-[(5-bromo-3-fluoro-2-pyridyl)methoxy]-2-pyridyl]-2,5-difluoro-phenyl]methyl]-7-fluoro-3-[[1-(fluoromethyl)cyclopropyl]methyl]benzimidazole-5-carboxylate (I-1192):** Methyl 2-[[4-[6-[(5-bromo-3-fluoro-2-pyridyl)methoxy]-2-pyridyl]-2,5-difluoro-phenyl]methyl]-7-fluoro-3-[[1-(fluoromethyl)cyclopropyl]methyl]benzimidazole-5-carboxylate was prepared in a manner as described for Intermediate **I-21** substituting **I-1191** for **I-9** and 5-bromo-2-(chloromethyl)-3-fluoro-pyridine for 5-bromo-2-(bromomethyl)thiazole. ES/MS: 687, 689 (M+H+).

### Preparation of Intermediate I-1193

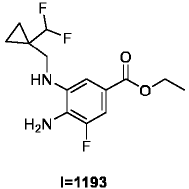

**Ethyl 3-[[1-(difluoromethyl)cyclopropyl]methylamino]-5-fluoro-4-nitro-benzoate (I-1193):** Ethyl 3-[[1-(difluoromethyl)cyclopropyl]methylamino]-5-fluoro-4-nitro-benzoate was prepared in a manner as described for Intermediate **I-1** substituting [1-(difluoromethyl)cyclopropyl]methanamine for 2-methoxyethylamine and ethyl 3,5-difluoro-4-nitrobenzoate for methyl 3-fluoro-4-nitrobenzoate.ES/MS: 302.2 (M+H+).

### Preparation of Intermediate I-1194

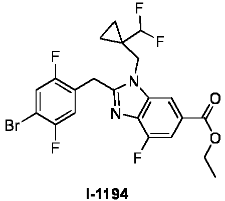

**Ethyl 2-[(4-bromo-2,5-difluoro-phenyl)methyl]-3-[[1-(difluoromethyl)cyclopropyl]methyl]-7-fluoro-benzimidazole-5-carboxylate (I-1194):** Ethyl 2-[(4-bromo-2,5-difluoro-phenyl)methyl]-3-[[1-(difluoromethyl)cyclopropyl]methyl]-7-fluoro-benzimidazole-5-carboxylate was prepared in a manner as described for Intermediate **I-13** substituting **I-1193** for **I-5** and 2-(4-bromo-2,5-difluorophenyl)acetic acid for 2-(4-bromo-2-fluoro-phenyl)acetic acid. ES/MS: 517.1, 519 (M+H+).

### Preparation of Intermediate I-1195

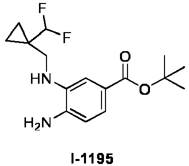

**tert-butyl 4-amino-3-(((1-(difluoromethyl)cyclopropyl)methyl)amino)benzoate (I-1195):** tert-butyl 4-amino-3-(((1-(difluoromethyl)cyclopropyl)methyl)amino)benzoate was prepared in a manner as described for Intermediate **I-6** substituting (1-(difluoromethyl)cyclopropyl)methanamine for 2-methoxyethylamine. ES/MS: 295.2 (M+H+).

### Preparation of Intermediate I-1196

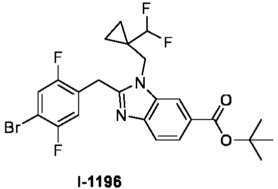

**tert-butyl 2-[(4-bromo-2,5-difluoro-phenyl)methyl]-3-[[1-(difluoromethyl)cyclopropyl]methyl]benzimidazole-5-carboxylate (I-1196):** tert-butyl 2-[(4-bromo-2,5-difluoro-phenyl)methyl]-3-[[1-(difluoromethyl)cyclopropyl]methyl]benzimidazole-5-carboxylate was prepared in a manner as described for Intermediate **I-2** substituting **I-1195** for **I-1** and 2-(4-bromo-2,5-difluorophenyl)acetic acid for 2-(4-bromo-2-fluoro-phenyl)acetic acid. ES/MS: 527, 529 (M+H+).

### Preparation of Intermediate I-1197

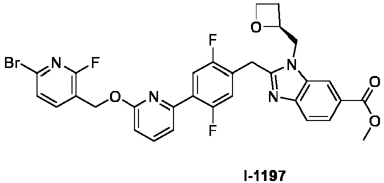

**Methyl 2-[[4-[6-[(6-bromo-2-fluoro-3-pyridyl)methoxy]-2-pyridyl]-2,5-difluoro-phenyl]methyl]-3-[[(2S)-oxetan-2-yl]methyl]benzimidazole-5-carboxylate (I-1197):** Methyl 2-[[4-[6-[(6-bromo-2-fluoro-3-pyridyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** substituting 6-bromo-3-(bromomethyl)-2-fluoropyridine for 5-bromo-2-(bromomethyl)thiazole. ES/MS: 653, 655 (M+H+).

### Preparation of Intermediate I-1198

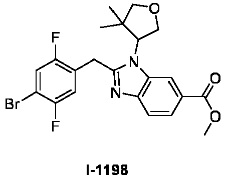

**Methyl 2- [(4-bromo-2,5-difluoro-phenyl)methyl] -3-(4,4-dimethyltetrahydrofuran-3-yl)benzimidazole-5-carboxylate (I-1198):** Methyl 2-[(4-bromo-2,5-difluoro-phenyl)methyl]-3-(4,4-dimethyltetrahydrofuran-3-yl)benzimidazole-5-carboxylate was prepared in a manner as described for Intermediate **I-2** substituting **I-25** for **I-1** and 2-(4-bromo-2,5-difluorophenyl)acetic acid for 2-(4-bromo-2-fluoro-phenyl)acetic acid. ES/MS: 479, 481 (M+H+).

### Preparation of Intermediate I-1199

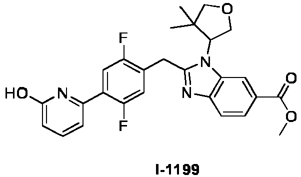

**Methyl 2-[[2,5-difluoro-4-(6-hydroxy-2-pyridyl)phenyl]methyl]-3-(4,4-dimethyltetrahydrofuran-3-yl)benzimidazole-5-carboxylate (I-1199):** methyl 2-[[2,5-difluoro-4-(6-hydroxy-2-pyridyl)phenyl]methyl]-3-(4,4-dimethyltetrahydrofuran-3-yl)benzimidazole-5-carboxylate was prepared in a manner as described for Intermediate **I-1187** substituting **I-1198** for **I-1180**. ES/MS: 493, 495 (M+H+).

### Preparation of Intermediate I-1200

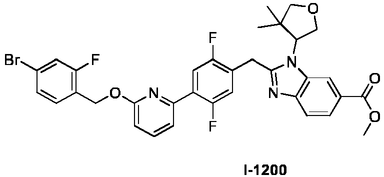

**Methyl 2-[[4-[6-[(4-bromo-2-fluoro-phenyl)methoxy]-2-pyridyl]-2,5-difluorophenyl]methyl]-3-(4,4-dimethyltetrahydrofuran-3-yl)benzimidazole-5-carboxylate (I-1200):** Methyl 2-[[4-[6-[(4-bromo-2-fluoro-phenyl)methoxy]-2-pyridyl]-2,5-difluorophenyl]methyl]-3-(4,4-dimethyltetrahydrofuran-3-yl)benzimidazole-5-carboxylate was prepared in a manner as described for Intermediate **I-21** substituting **I-1199** for **I-9** and 4-bromo-1-(bromomethyl)-2-fluoro-benzene for 5-bromo-2-(bromomethyl)thiazole. ES/MS: 680, 682 (M+H+).

### Preparation of Intermediate I-1201

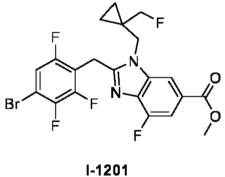

**Methyl 2-(4-bromo-2,3,6-trifluorobenzyl)-4-fluoro-1-((1-(fluoromethyl)cyclopropyl)methyl)-1H-benzo[d]imidazole-6-carboxylate (I-1201):** Methyl 2-(4-bromo-2,3,6-trifluorobenzyl)-4-fluoro-1-((1-(fluoromethyl)cyclopropyl)methyl)-1H-benzo[d]imidazole-6-carboxylate was prepared in a manner as described for Intermediate **I-13** substituting **I-1189** for **I-5** and 2-(4-bromo-2,3,6-trifluorophenyl)acetic acid for 2-(4-bromo-2-fluoro-phenyl)acetic acid. ES/MS: 485, 487 (M+H+).

### Preparation of Intermediate I-1202

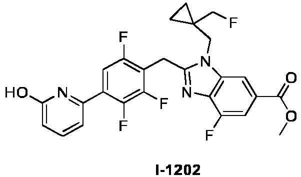

**Methyl 7-fluoro-3-[[1-(fluoromethyl)cyclopropyl]methyl]-2-[[2,3,6-trifluoro-4-(6-hydroxy-2-pyridyl)phenyl]methyl]benzimidazole-5-carboxylate (I-1202):** Methyl 7-fluoro-3-[[1-(fluoromethyl)cyclopropyl]methyl]-2-[[2,3,6-trifluoro-4-(6-hydroxy-2-pyridyl)phenyl]methyl]benzimidazole-5-carboxylate was prepared in a manner as described for Intermediate **I-1187** substituting **I-1201** for **I-1180**. ES/MS: 517, 519 (M+H+).

### Preparation of Intermediate I-1203

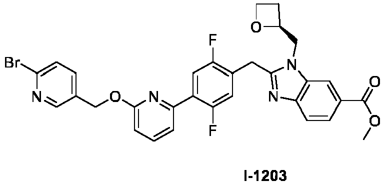

**Methyl 2-[[4-[6-[(6-bromo-3-pyridyl)methoxy]-2-pyridyl]-2,5-difluorophenyl]methyl]-3-[[(2S)-oxetan-2-yl]methyl]benzimidazole-5-carboxylate** (I-1203): Methyl 2-[[4-[6-[(6-bromo-3-pyridyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** substituting 2-bromo-5-(bromomethyl)pyridine for 5-bromo-2-(bromomethyl)thiazole. ES/MS: 635, 637 (M+H+).

### Preparation of Intermediate I-1204

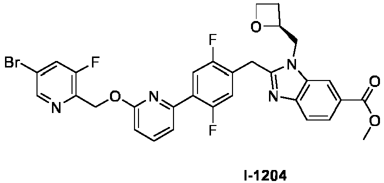

**Methyl 2-[[4-[6-[(6-bromo-3-pyridyl)methoxy]-2-pyridyl]-2,5-difluorophenyl]methyl]-3-[[(2S)-oxetan-2-yl]methyl]benzimidazole-5-carboxylate** (I-1204): Methyl 2-[[4-[6-[(6-bromo-3-pyridyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** substituting **I-31** for 5-bromo-2-(bromomethyl)thiazole. ES/MS: 653, 655 (M+H+).

### Preparation of Intermediate I-1205

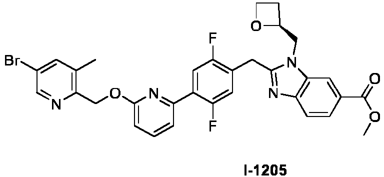

**Methyl 2-[[4-[6-[(5-bromo-3-methyl-2-pyridyl)methoxy]-2-pyridyl]-2,5-difluorophenyl]methyl]-3-[[(2S)-oxetan-2-yl]methyl]benzimidazole-5-carboxylate (I-1205):** Methyl 2-[[4-[6-[(5-bromo-3-methyl-2-pyridyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** substituting 5-bromo-2-(chloromethyl)-3-methyl-pyridine for 5-bromo-2-(bromomethyl)thiazole. ES/MS: 649, 651 (M+H+).

### Preparation of Intermediate I-1206

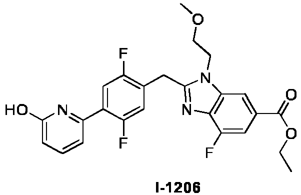

**Ethyl 2-[[2,5-difluoro-4-(6-hydroxy-2-pyridyl)phenyl]methyl]-7-fluoro-3-(2-methoxyethyl)benzimidazole-5-carboxylate (I-1206):** Ethyl 2-[[2,5-difluoro-4-(6-hydroxy-2-pyridyl)phenyl]methyl]-7-fluoro-3-(2-methoxyethyl)benzimidazole-5-carboxylate was prepared in a manner as described for Intermediate **I-1187** substituting **I-1033** for **I-1180.** ES/MS: 485, 487 (M+H+).

### Preparation of Intermediate I-1207

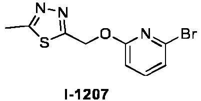

**2-[(6-bromo-2-pyridyl)oxymethyl]-5-methyl-1,3,4-thiadiazole (I-1207):** 2-[(6-bromo-2-pyridyl)oxymethyl]-5-methyl-1,3,4-thiadiazole was prepared in a manner as described for Intermediate **I-1034** substituting 2-(chloromethyl)-5-methyl-1,3,4-thiadiazole for 6-(bromomethyl)-1-methyl-benzotriazole. ES/MS: 286, 288 (M+H+).

### Preparation of Intermediate I-1208

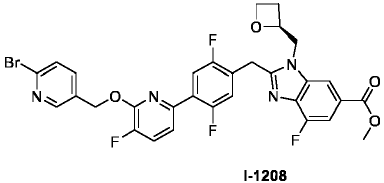

**Ethyl 2-[[4-[6-[(6-bromo-3-pyridyl)methoxy]-5-fluoro-2-pyridyl]-2,5-difluorophenyl]methyl]-7-fluoro-3-[[(2S)-oxetan-2-yl]methyl]benzimidazole-5-carboxylate (I-1208):** Ethyl 2-[[4-[6-[(6-bromo-3-pyridyl)methoxy]-5-fluoro-2-pyridyl]-2,5-difluoro-phenyl]methyl]-7-fluoro-3-[[(2S)-oxetan-2-yl]methyl]benzimidazole-5-carboxylate was prepared in a manner as described for Intermediate **I-21** substituting ethyl 2-[[2,5-difluoro-4-(5-fluoro-6-hydroxy-2-pyridyl)phenyl]methyl]-7-fluoro-3-[[(2S)-oxetan-2-yl]methyl]benzimidazole-5-carboxylate for 1-9 and 2-bromo-5-(bromomethyl)pyridine for 5-bromo-2-(bromomethyl)thiazole. ES/MS: 686, 687 (M+H+).

### Preparation of Intermediate I-1209

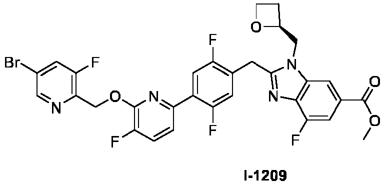

**Ethyl 2-[**[4-**[6-[(5-bromo-3-fluoro-2-pyridyl)methoxy]-5-fluoro-2-pyridyl]-2,5-difluoro-phenyl]methyl]-7-fluoro-3-[[(2S)-oxetan-2-yl]methyl]benzimidazole-5-carboxylate (I-1209):** Ethyl 2-[[4-[6-[(5-bromo-3-fluoro-2-pyridyl)methoxy]-5-fluoro-2-pyridyl]-2,5-difluoro-phenyl]methyl]-7-fluoro-3-[[(2S)-oxetan-2-yl]methyl]benzimidazole-5-carboxylate was prepared in a manner as described for Intermediate **I-21** substituting ethyl 2-[[2,5-difluoro-4-(5-fluoro-6-hydroxy-2-pyridyl)phenyl]methyl]-7-fluoro-3-[[(2S)-oxetan-2-yl]methyl]benzimidazole-5-carboxylate for **I-9** and 5-bromo-2-(chloromethyl)-3-fluoro-pyridine for 5-bromo-2-(bromomethyl)thiazole. ES/MS: 703, 705 (M+H+).

### Preparation of Intermediate I-1210

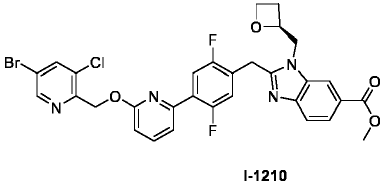

**Methyl 2-[[4-[6-[(5-bromo-3-chloro-2-pyridyl)methoxy]-2-pyridyl]-2,5-difluorophenyl]methyl]-3-[[(2S)-oxetan-2-yl]methyl]benzimidazole-5-carboxylate (I-1210):** Methyl 2-[[4-[6-[(5-bromo-3-chloro-2-pyridyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** substituting 5-bromo-3-chloro-2-(chloromethyl)pyridine for 5-bromo-2-(bromomethyl)thiazole. ES/MS: 670, 672 (M+H+).

### Preparation of Intermediate I-1211

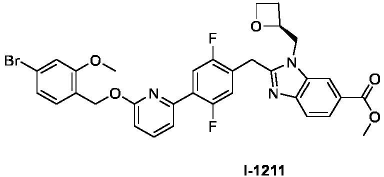

**Methyl 2-[[4-[6-[(4-bromo-2-methoxy-phenyl)methoxy]-2-pyridyl]-2,5-difluorophenyl]methyl]-3-[[(2S)-oxetan-2-yl]methyl]benzimidazole-5-carboxylate (I-1211):** Methyl 2-[[4-[6-[(4-bromo-2-methoxy-phenyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** substituting 4-bromo-1-(bromomethyl)-2-methoxy-benzene for 5-bromo-2-(bromomethyl)thiazole. ES/MS: 664, 666 (M+H+).

### Preparation of Intermediate I-1212

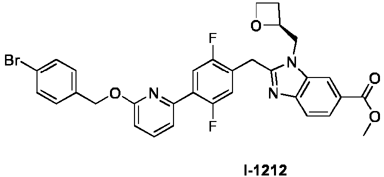

**Methyl 2-[[4-[6-[(4-bromophenyl)methoxy]-2-pyridyl]-2,5-difluoro-phenyl]methyl]-3-[[(2S)-oxetan-2-yl]methyl]benzimidazole-5-carboxylate (I-1212):** Methyl 2-[[4-[6-[(4-bromophenyl)methoxy]-2-pyridyl]-2,5-difluoro-phenyl]methyl]-3-[[(2S)-oxetan-2-yl]methyl]benzimidazole-5-carboxylate was prepared in a manner as described for Intermediate **I-21** substituting 1-bromo-4-(bromomethyl)benzene for 5-bromo-2-(bromomethyl)thiazole. ES/MS: 634, 636 (M+H+).

### Preparation of Intermediate I-1219

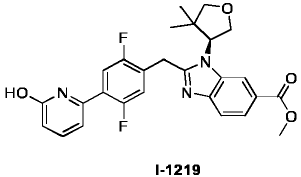

**Methyl (S)-2-(2,5-difluoro-4-(6-hydroxypyridin-2-yl)benzyl)-1-(4,4-dimethyltetrahydrofuran-3-yl)-1H-benzo[d]imidazole-6-carboxylate (I-1219):** Methyl (S)-2-(2,5-difluoro-4-(6-hydroxypyridin-2-yl)benzyl)-1-(4,4-dimethyltetrahydrofuran-3-yl)-1H-benzo[d]imidazole-6-carboxylate was prepared in a manner as described for Intermediate **I-1187** substituting **I-82** for **I-1180**. ES/MS: 493, 495 (M+H+).

### Preparation of Intermediate I-1220

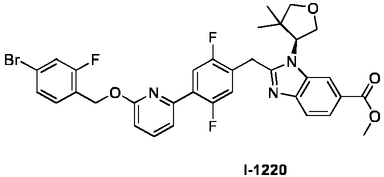

**Methyl (S)-2-(4-(6-((4-bromo-2-fluorobenzyl)oxy)pyridin-2-yl)-2,5-difluorobenzyl)-1-(4,4-dimethyltetrahydrofuran-3-yl)-1H-benzo[d]imidazole-6-carboxylate (I-1220):** Methyl (S)-2-(4-(6-((4-bromo-2-fluorobenzyl)oxy)pyridin-2-yl)-2,5-difluorobenzyl)-1-(4,4-dimethyltetrahydrofuran-3-yl)-1H-benzo[d]imidazole-6-carboxylate was prepared in a manner as described for Intermediate **I-21** substituting **I-1219** for **I-9** and 4-bromo-1-(bromomethyl)-2-fluoro-benzene for 5-bromo-2-(bromomethyl)thiazole. ES/MS: 680, 682 (M+H+).

### Preparation of Intermediate I-1223

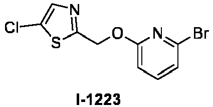

**2-[(6-bromo-2-pyridyl)oxymethyl]-5-chloro-thiazole (I-1223):** 2-[(6-bromo-2-pyridyl)oxymethyl]-5-chloro-thiazole was prepared in a manner as described for Intermediate **I-84** substituting 5-chloro-2-(chloromethyl)thiazole for 2-(bromomethyl)thiazole-5-carbonitrile. ES/MS: 305 (M+H+).

### Preparation of Intermediate I-1224

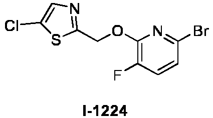

**2-[(6-bromo-3-fluoro-2-pyridyl)oxymethyl]-5-chloro-thiazole (I-1224):** 2-[(6-bromo-3-fluoro-2-pyridyl)oxymethyl]-5-chloro-thiazole was prepared in a manner as described for Intermediate **I-84** substituting 5-chloro-2-(chloromethyl)thiazole for 2-(bromomethyl)thiazole-5-carbonitrile and 6-bromo-3-fluoro-pyridin-2-ol for 6-bromopyridin-2-ol. ES/MS: 323 (M+H+).

### Preparation of Intermediate I-1226

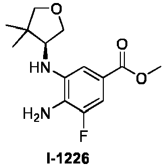

**Methyl (S)-4-amino-3-((4,4-dimethyltetrahydrofuran-3-yl)amino)-5-fluorobenzoate:** Methyl (S)-4-amino-3-((4,4-dimethyltetrahydrofuran-3-yl)amino)-5-fluorobenzoate was prepared in a manner as described for **I-5** substituting (3S)-4,4'-dimethyltetrahydrofuran-3-amine hydrochloride for (S)-oxetan-2-ylmethanamine. ES/MS: 338.5 (M+Na⁺):

### Preparation of Intermediate I-1229

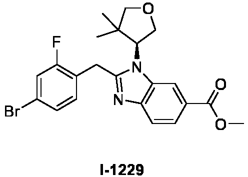

**Methyl (S)-2-(4-bromo-2-fluorobenzyl)-1-(4,4-dimethyltetrahydrofuran-3-yl)-1H-benzo[d]imidazole-6-carboxylate (I-1229):** Methyl (S)-2-(4-bromo-2-fluorobenzyl)-1-(4,4-dimethyltetrahydrofuran-3-yl)-1H-benzo[d]imidazole-6-carboxylate was prepared in a manner as described for **I-1041** substituting Intermediate **I-80** for **I-1042** and 2-(4-bromo-2-fluorophenyl)acetic acid for 2-(4-bromo-2,5-difluoro-phenyl)acetic acid. ES/MS: 469.8 (M+H⁺).

### Preparation of Intermediate I-1230

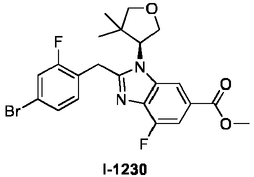

**Methyl (S)-2-(4-bromo-2-fluorobenzyl)-1-(4,4-dimethyltetrahydrofuran-3-yl)-4-fluoro-1H-benzo[d]imidazole-6-carboxylate (I-1230):** Methyl (S)-2-(4-bromo-2-fluorobenzyl)-1-(4,4-dimethyltetrahydrofuran-3-yl)-4-fluoro-1H-benzo[d]imidazole-6-carboxylate was prepared in a manner as described for **I-2** substituting **I-1226** for **I-1042** and 2-(4-bromo-2-fluoro-phenyl)acetic acid for 2-(4-bromo-2,5-di-fluoro-phenyl)acetic acid. ES/MS: 479.6 (M+H⁺);

### Preparation of Intermediate I-1231

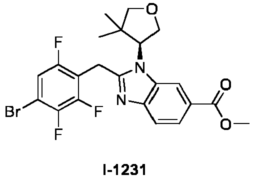

**Methyl (S)-2-(4-bromo-2,3,6-trifluorobenzyl)-1-(4,4-dimethyltetrahydrofuran-3-yl)-1H-benzo[d]imidazole-6-carboxylate:** Methyl (S)-2-(4-bromo-2,3,6-trifluorobenzyl)-1-(4,4-dimethyltetrahydrofuran-3-yl)-1H-benzo[d]imidazole-6-carboxylate was prepared in a manner as described for **I-1041** substituting **I-80** for **I-1042** and 2-(4-bromo-2,3,6-trifluorophenyl)acetic acid for 2-(4-bromo-2,5-difluorophenyl)acetic acid. ES/MS: 498.0 (M+H⁺);

### Preparation of Intermediate I-1232

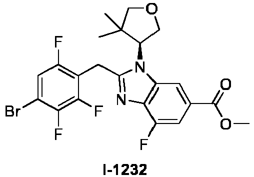

**Methyl (S)-2-(4-bromo-2,3,6-trifluorobenzyl)-1-(4,4-dimethyltetrahydrofuran-3-yl)-4-fluoro-1H-benzo[d]imidazole-6-carboxylate:** Methyl (S)-2-(4-bromo-2,3,6-trifluorobenzyl)-1-(4,4-dimethyltetrahydrofuran-3-yl)-4-fluoro-1H-benzo[d]imidazole-6-carboxylate was prepared in a manner as described for **I-2** substituting **I-1226** for **I-1042** and 2-(4-bromo-2,3,6-trifluorophenyl)acetic acid for 2-(4-bromo-2,5-difluorophenyl)acetic acid. ES/MS: 515.7 (M+H⁺);

### Preparation of Intermediate I-1233

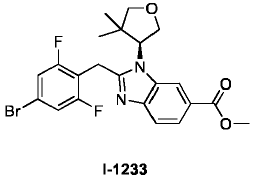

**Methyl (S)-2-(4-bromo-2,6-difluorobenzyl)-1-(4,4-dimethyltetrahydrofuran-3-yl)-1H-benzo[d]imidazole-6-carboxylate:** Methyl (S)-2-(4-bromo-2,6-difluorobenzyl)-1-(4,4-dimethyltetrahydrofuran-3-yl)-1H-benzo[d]imidazole-6-carboxylate was prepared in a manner as described for **I-1041** substituting **I-80** for **I-1042** and 2-(4-bromo-2,6-difluorophenyl)acetic acid for 2-(4-bromo-2,5-difluorophenyl)acetic acid. ES/MS: 479.9 (M+H⁺);

### Preparation of Intermediate I-1234

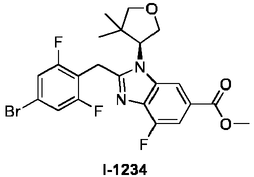

**Methyl (S)-2-(4-bromo-2,6-difluorobenzyl)-1-(4,4-dimethyltetrahydrofuran-3-yl)-4-fluoro-1H-benzo[d]imidazole-6-carboxylate:** Methyl (S)-2-(4-bromo-2,6-difluorobenzyl)-1-(4,4-dimethyltetrahydrofuran-3-yl)-4-fluoro-1H-benzo[d]imidazole-6-carboxylate was prepared in a manner as described for **I-2** substituting **I-1226** for **I-1042** and 2-(4-bromo-2,6-trifluorophenyl)acetic acid for 2-(4-bromo-2,6-difluorophenyl)acetic acid. ES/MS: 497.6 (M+H⁺);

### Preparation of Intermediate I-1235

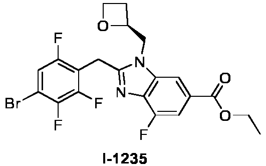

**Ethyl (S)-2-(4-bromo-2,3,6-trifluorobenzyl)-4-fluoro-1-(oxetan-2-ylmethyl)-1H-benzo[d]imidazole-6-carboxylate:** Ethyl (S)-2-(4-bromo-2,3,6-trifluorobenzyl)-4-fluoro-1-(oxetan-2-ylmethyl)-1H-benzo[d]imidazole-6-carboxylate was in a manner as described for **I-2** substituting **I-5** for **I-1** and 2-(4-bromo-2,3,6-trifluorophenyl)acetic acid for 2-(4-bromo-2-fluoro-phenyl)acetic acid. ES/MS: 501.3 (M+H⁺);

### Preparation of Intermediate I-1237

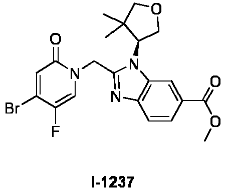

**Methyl (S)-2-((4-bromo-5-fluoro-2-oxopyridin-1(2H)-yl)methyl)-1-(4,4-dimethyltetrahydrofuran-3-yl)-1H-benzo[d]imidazole-6-carboxylate:** Methyl (S)-2-((4-bromo-5-fluoro-2-oxopyridin-1(2H)-yl)methyl)-1-(4,4-dimethyltetrahydrofuran-3-yl)-1H-benzo[d]imidazole-6-carboxylate was prepared in a manner as described for **I-1018** substituting **I-80** for **I-104** . ES/MS: 322.5 (M+H+).

### Preparation of Intermediate I-1238

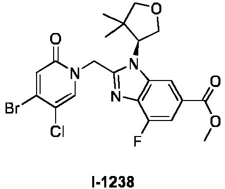

**Methyl (S)-2-((4-bromo-5-chloro-2-oxopyridin-1(2H)-yl)methyl)-1-(4,4-dimethyltetrahydrofuran-3-yl)-4-fluoro-1H-benzo[d]imidazole-6-carboxylate:** Methyl (S)-2-((4-bromo-5-chloro-2-oxopyridin-1(2H)-yl)methyl)-1-(4,4-dimethyltetrahydrofuran-3-yl)-4-fluoro-1H-benzo[d]imidazole-6-carboxylate was prepared in a manner as described for **I-1018** substituting **I-1226** for **I-104** and 4-chloro-5-fluoro-1H-pyridin-2-one for 4-bromo-S-fluoro-1H-pyridin-2-one . ES/MS: 514.4 (M+H+).

### Preparation of Intermediate I-1239

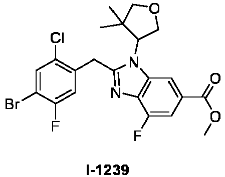

**Methyl 2-(4-bromo-2-chloro-5-fluorobenzyl)-1-(4,4-dimethyltetrahydrofuran-3-yl)-4-fluoro-1H-benzo[d]imidazole-6-carboxylate:** Methyl 2-(4-bromo-2-chloro-5-fluorobenzyl)-1-(4,4-dimethyltetrahydrofuran-3-yl)-4-fluoro-1H-benzo[d]imidazole-6-carboxylate was prepared in a manner as described for **I-1031** substituting methyl 4-amino-3-[(4,4-dimethyltetrahydrofuran-3-yl)amino]-5-fluoro-benzoate **I-104** for ethyl 4-amino-3-fluoro-5-[[(2S)-oxetan-2-yl]methylamino]benzoate and 2-(4-bromo-2-chloro-5-fluoro-phenyl)acetic acid for 2-(4-bromo-2-chloro-5-methyl-phenyl)acetic acid. ES/MS: 513.8 (M+H+).

### Preparation of Intermediate I-1240

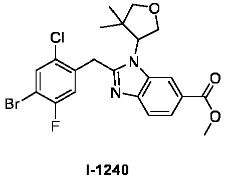

**Methyl 2-(4-bromo-2-chloro-5-fluorobenzyl)-1-(4,4-dimethyltetrahydrofuran-3-yl)-1H-benzo[d]imidazole-6-carboxylate:** Methyl 2-(4-bromo-2-chloro-5-fluorobenzyl)-1-(4,4-dimethyltetrahydrofuran-3-yl)-1H-benzo[d]imidazole-6-carboxylate was prepared in a manner as described for **I-1031** substituting methyl 4-amino-3-[(4,4-dimethyltetrahydrofuran-3-yl)amino]-benzoate **I-104** for ethyl 4-amino-3-fluoro-5-[[(2S)-oxetan-2-yl]methylamino]benzoate and 2-(4-bromo-2-chloro-5-fluoro-phenyl)acetic acid for 2-(4-bromo-2-chloro-5-methyl-phenyl)acetic acid. ES/MS: 494.6 (M+H+).

### Preparation of Intermediate I-1244

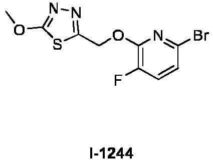

**2-(((6-bromo-3-fluoropyridin-2-yl)oxy)methyl)-5-methoxy-1,3,4-thiadiazole:** 2-(((6-bromo-3-fluoropyridin-2-yl)oxy)methyl)-5-methoxy-1,3,4-thiadiazole was prepared in a manner as described for Intermediate **I-1034** substituting 2-(chloromethyl)-5-methoxy-1,3,4-thiadiazole for 6-(bromomethyl)-1-methyl-benzotriazole and 6-bromo-3-fluoro-pyridin-2-ol for 6-bromopyridin-2-ol. ES/MS: 320.7 (M+H+).

### Preparation of Intermediate I-1245

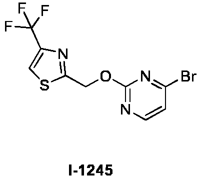

**2-(((4-bromopyrimidin-2-yl)oxy)methyl)-4-(trifluoromethyl)thiazole:** 2-(((4-bromopyrimidin-2-yl)oxy)methyl)-4-(trifluoromethyl)thiazole was prepared in a manner as described for Intermediate **I-1034** substituting 2-(bromomethyl)-4-(trifluoromethyl)thiazole for 6-(bromomethyl)-1-methyl-benzotriazole and 6-bromo-3-fluoro-pyrimidin-2-ol for 6-bromopyridin-2-ol. ES/MS: 340.1 (M+H+).

### Preparation of Intermediate I-1250

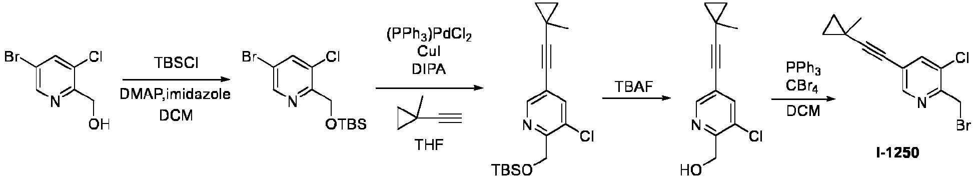

**5-bromo-2-(((tert-butyldimethylsilyl)oxy)methyl)-3-chloropyridine:** A suspension of 5-bromo-3-chloropyridin-2-yl)methanol (500 mg, 2.25 mmol), tert-Butylchlorodimethylsilane (508 mg, 3.37 mmol), Imidazole (459 mg, 6.74 mmol) and 4-(Dimethylamino)pyridine (55 mg, 0.45 mmol) ) in DCM (15 mL) was stirred at 25 °C for 16h. Then the mixture was diluted with DCM and washed with brine. Next, the mixture was dried over sodium sulfate, concentrated, and purified by chromatography (eluent: EtOAc/hexanes) to give desired product. ES/MZ: 336.7 (M+H+).

**2-(((tert-butyldimethylsilyl)oxy)methyl)-3-chloro-5-((1-methylcyclopropyl)ethynyl)pyridine:** A suspension of 5-bromo-2-(((tertbutyldimethylsilyl)oxy)methyl)-3-chloropyridine (250 mg, 0.742 mmol), 1-ethynyl-1-methyl-cyclopropane (178 mg, 2.23 mmol), Bis(triphenylphosphine)palladium Chloride (104 mg, 0.15 mmol), Cuprous Iodide (28 mg, 0.15 mmol) and Diisopropylamine (2.15 ml, 14.8 mmol) in THF (2 mL) was sparged with Argon for 5 min then heated at 80 °Cs for 2 h. Next, the mixture was diluted with EtOAC and washed with brine. The mixture was then dried over sodium sulfate, concentrated, and purified by chromatography (eluent: EtOAc/hexanes) to give desired product. ES/MZ: 336.7 (M+H+).

**(3-chloro-5-((1-methylcyclopropyl)ethynyl)pyridin-2-yl)methanol:** A suspension of 2-(((tertbutyldimethylsilyl)oxy)methyl)-3-chloro-5-((1-methylcyclopropyl)ethynyl)pyridine in THF (2 mL) and Tetrabutylammonium fluoride (1.11 mL, 1.11 mmol) was stirred at 25 °C for 16 h. The mixture was then diluted with EtOAC and washed with brine. Next, the mixture was dried over sodium sulfate, concentrated, and purified by chromatography (eluent: EtOAc/hexanes) to give desired product. ES/MZ: 222.6 (M+H+).

**2-(bromomethyl)-3-chloro-5-((1-methylcyclopropyl)ethynyl)pyridine (I-1250):** A suspension of (3-chloro-5-((1-methylcyclopropyl)ethynyl)pyridin-2-yl)methanol (165 mg, 0.742 mmol), carbon tetrabromide (295 mg, 0.90 mmol) and triphenylphosphine oxide (234 mg, 0.90 mmol) in DCM (3 ml) was stirred at 25 °C for 16 h. Then the mixture was diluted with DCM and washed with brine. Then the mixture was dried over sodium sulfate, concentrated, and purified by chromatography (eluent: EtOAc/hexanes) to give desired product. ES/MZ: 285.9 (M+H+).

### Preparation of Intermediate I-1251

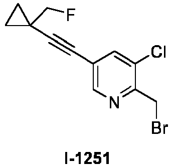

**2-(bromomethyl)-3-chloro-5-((1-(fluoromethyl)cyclopropyl)ethynyl)pyridine:** 2-(bromomethyl)-3-chloro-5-((1-(fluoromethyl)cyclopropyl)ethynyl)pyridine was prepared in a manner as described for the intermediate 1-1250 substituting 1-ethynyl-1-(fluoromethyl)cyclopropane for 1-ethynyl-1methyl-cyclopropane. ES/MZ: 302.6 (M+H+).

### Preparation of Intermediate I-1252

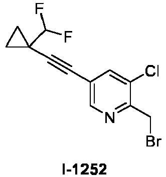

**2-(bromomethyl)-3-chloro-5-((1-(difluoromethyl)cyclopropyl)ethynyl)pyridine:** 2-(bromomethyl)-3-chloro-5-((1-(difluoromethyl)cyclopropyl)ethynyl)pyridine was prepared in a manner as described for the intermediate **I-1250** substituting 1-ethynyl-1-(difluoromethyl)cyclopropane for 1-ethynyl-1methyl-cyclopropane. ES/MZ: 320.6 (M+H+).

### Preparation of Intermediate I-1253 cis-isomer 1 and I-1253 trans-isomer 2

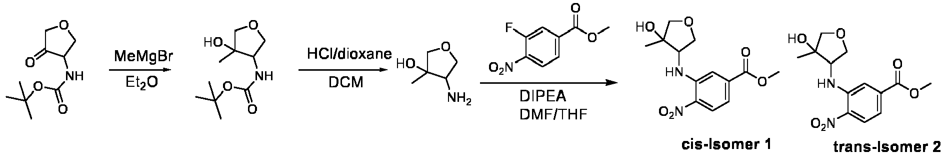

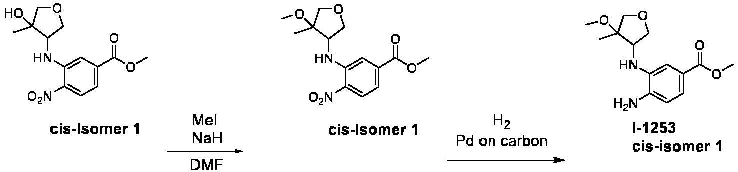

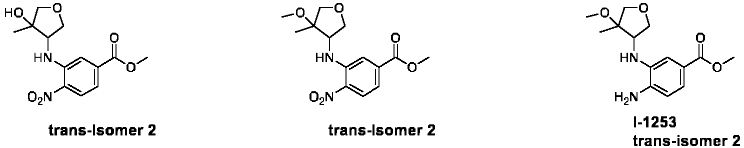

**tert-butyl (4-hydroxy-4-methyltetrahydrofuran-3-yl)carbamate:** A suspension of tert-butyl (4-oxotetrahydrofuran-3-yl)carbamate (500 mg, 2.5 mmol) in diethyl ether (212.5 mL) was cooled to 0 °C, then methylmagnesium bromide (2.48 mL, 3.0 Min diethyl ether, 7.45 mmol) was added slowly. The mixture was allowed to warm to 25 °C and then stirred for 16 h. Next, the mixture was diluted with diethyl ether and washed with brine. Then the mixture was dried over sodium sulfate, concentrated, and used without further purification. 1H NMR (400 MHz, CDCl3) δ 5.02 (s, 1H), 4.68 (d, *J =* 8.1 Hz, 1H), 4.32 - 4.24 (m, 1H), 4.21 - 4.13 (m, 1H), 4.06 - 3.91 (m, 1H), 3.85 - 3.72 (m, 2H), 3.62 - 3.46 (m, 1H), 1.47 (d, *J =* 3.5 Hz, 12H), 1.38 (s, 2H).

**4-amino-3-methyltetrahydrofuran-3-ol:** A suspension of tert-butyl (4-hydroxy-4-methyltetrahydrofuran-3-yl)carbamate (491 mg, 2.26 mmol) and hydrochloric acid (1.25 mL, 4.0M in dioxane, 5.00 mmol) in DCM (5.00 mL) was stirred overnight. The mixture was diluted in EtOAc and washed with brine, dried over sodium sulfate, concentrated and used without further purification. 1H NMR (400 MHz, CDCl3) δ 3.95 (ddt, J = 7.8, 3.8, 2.0 Hz, 1H), 3.77 (tq, J = 7.2, 5.3, 3.7 Hz, 2H), 3.72 - 3.60 (m, 2H), 3.55 (dd, J = 9.4, 2.0 Hz, 2H), 3.52 - 3.41 (m, 2H), 3.38 (t, J = 1.8 Hz, 0H), 3.33 - 3.23 (m, 2H), 3.09 (t, J = 6.2 Hz, 2H), 1.20 (t, J = 1.8 Hz, 5H), 1.17 (d, J = 2.0 Hz, 4H), 1.02 - 0.92 (m, 6H).

**Methyl 3-((4-hydroxy-4-methyltetrahydrofuran-3-yl)amino)-4-nitrobenzoate:** A suspension of 4-amino-3-methyltetrahydrofuran-3-ol (424 mg, 2.76 mmol), methyl 3-fluoro-4-nitrobenzoate (500 mg, 2.51 mmol) and N,N-Diisopropylethylamine (2.19 mL, 1.26 mmol) in THF (4 mL) and DMF (2 mL) was stirred at 82 °C for 16 h. The mixture was then diluted in EtOAc and washed with brine, dried over sodium sulfate, concentrated, and purified by chromatography (eluent: EtOAc/hexanes) to give desired product. ES/MZ: 297.1 (M+H+). Isomer 1: 1HNMR (400 MHz, CDCl3) δ 3.95 (ddt, J = 7.8, 3.8, 2.0 Hz, 1H), 3.77 (tq, J = 7.2, 5.3, 3.7 Hz, 2H), 3.72 - 3.60 (m, 2H), 3.55 (dd, J = 9.4, 2.0 Hz, 2H), 3.52 - 3.41 (m, 2H), 3.38 (t, J = 1.8 Hz, 0H), 3.33 - 3.23 (m, 2H), 3.09 (t, J = 6.2 Hz, 2H), 1.20 (t, J = 1.8 Hz, 5H), 1.17 (d, J = 2.0 Hz, 4H), 1.02-0.92 (m, 6H). Isomer 2: 1H NMR (400 MHz, CDCl3) δ 8.27 (d, J = 8.9 Hz, 1H), 8.11 (d, J = 7.7 Hz, 1H), 7.75 (d, J = 1.7 Hz, 1H), 7.33 (dd, J = 8.9, 1.7 Hz, 1H), 4.53 (dd, J = 9.6, 6.3 Hz, 1H), 4.24 (q, J = 7.0, 6.4 Hz, 1H), 3.98 (s, 3H), 3.91 (d, J = 9.8 Hz, 1H), 3.80 (d, J = 9.8 Hz, 1H), 3.73 (dd, J = 9.6, 4.6 Hz, 1H), 1.42 (s, 3H).

**Methyl 3-((4-methoxy-4-methyltetrahydrofuran-3-yl)amino)-4-nitrobenzoate:** A suspension of methyl 3-((4-hydroxy-4-methyltetrahydrofuran-3-yl)amino)-4-nitrobenzoate (144 mg, 0.49 mmol), sodium hydride (20.5 mg, 0.54 mmol) and iodomethane (33 uL, 0.54 mmol) in DMF (1.00 mL) was stirred at 0 °C for 15 min then warmed to 25 °C and stirred for 16h. The mixture was then diluted in EtOAc and washed with brine, dried over sodium sulfate, concentrated, and purified by chromatography (eluent: EtOAc/hexanes) to give desired product EZ/MS: 311.3 (M+H+).

**Methyl 4-amino-3-((4-methoxy-4-methyltetrahydrofuran-3-yl)amino)benzoate:** A suspension of methyl 3-((4-methoxy-4-methyltetrahydrofuran-3-yl)amino)-4-nitrobenzoate (133 mg, 0.43 mmol), and Palladium on carbon (10% loading, 45.5 mg, 0.43 mmol) in ethanol (5.0 mL) and placed under hydrogen at 25 °C for 5 h. Next, the mixture was diluted in EtOAc and washed with brine. Then the mixture was dried over sodium sulfate, concentrated and used without further purification. EZ/MS: 281.1 (M+H+)

### Preparation of Intermediate I-1257 cis-isomer 1

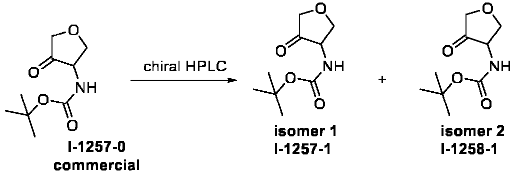

**Tert-butyl (4-hydroxy-4-methyltetrahydrofuran-3-yl)carbamate) (Isomer 1 I-1257, Isomer 2 I-1258-1):** was obtained via preparative chiral HPLC (Chiralpak IH column, Hexanes/iPrOH eluent) on racemic **I-1257-0**, giving two distinct stereoisomers **(I-1257-1** as the earlier eluting isomer, **I-1258-1** as the later eluting isomer).

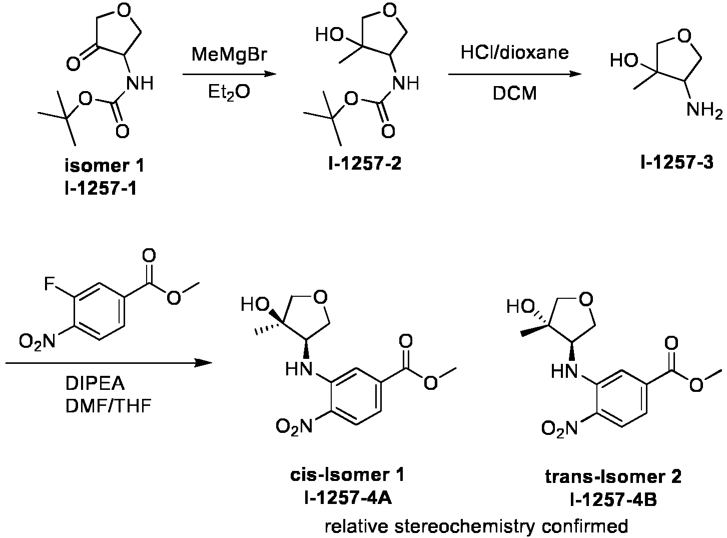

**Tert-butyl (4-hydroxy-4-methyltetrahydrofuran-3-yl)carbamate (I-1257-2):** A mixture of tert-butyl (4-oxotetrahydrofuran-3-yl)carbamate (500 mg, 2.5 mmol) **(Peak 1 I-1257-1)** in diethyl ether (212.5 mL) was cooled to 0 °C, then methylmagnesium bromide (2.48 mL, 3.0 M in diethyl ether, 7.45 mmol) was added slowly. The mixture was allowed to warm to 25 °C and allowed to stir for 16 h. Then the mixture was diluted with diethyl ether and washed with brine. Then, the mixture was dried over sodium sulfate, and concentrated to yield the title compound. 1H NMR (400 MHz, CDCl3) δ 5.02 (s, 1H), 4.68 (d, *J =* 8.1 Hz, 1H), 4.32 - 4.24 (m, 1H), 4.21 - 4.13 (m, 1H), 4.06 - 3.91 (m, 1H), 3.85 - 3.72 (m, 2H), 3.62 - 3.46 (m, 1H), 1.47 (d, *J =* 3.5 Hz, 12H), 1.38 (s, 2H).

**4-amino-3-methyltetrahydrofuran-3-ol (I-1257-3):** A suspension of tert-butyl (4-hydroxy-4-methyltetrahydrofuran-3-yl)carbamate (491 mg, 2.26 mmol) **(I-1257-2)** and hydrochloric acid (1.25 mL, 4.0M in dioxane, 5.00 mmol) in DCM (5.00 mL) was stirred overnight. The mixture was diluted in EtOAc and washed with brine, dried over sodium sulfate, and concentrated to yield the title compound. 1H NMR (400 MHz, CDCl3) δ 3.95 (ddt, J = 7.8, 3.8, 2.0 Hz, 1H), 3.77 (tq, J = 7.2, 5.3, 3.7 Hz, 2H), 3.72 - 3.60 (m, 2H), 3.55 (dd, J = 9.4, 2.0 Hz, 2H), 3.52-3.41 (m, 2H), 3.38 (t, J = 1.8 Hz, 0H), 3.33 - 3.23 (m, 2H), 3.09 (t, J = 6.2 Hz, 2H), 1.20 (t, J = 1.8 Hz, 5H), 1.17 (d, J = 2.0 Hz, 4H), 1.02 - 0.92 (m, 6H).

**Methyl 3-((4-hydroxy-4-methyltetrahydrofuran-3-yl)amino)-4-nitrobenzoate (cis-isomer Peak 1, I-1257-4A, relative stereochemistry established):** A suspension of 4-amino-3-methyltetrahydrofuran-3-ol (424 mg, 2.76 mmol) **(I-1257-3)**, methyl 3-fluoro-4-nitro-benzoate (500 mg, 2.51 mmol) and N,N-Diisopropylethylamine (2.19 mL, 1.26 mmol) in THF (4 mL) and DMF (2 mL) was stirred at 82 °C for 16 hr. Next, the mixture was purified by silica gel flash column chromatography (EtOAc/hexane) providing both cis **(I-1257-4A, Isomer 1,** the earlier eluting of two diastereomers) and trans **(I-1257-4B, Isomer 2,** the later eluting of two diastereomers) isomers. ES/MZ: 297.1 (M+H+). **Isomer 1, I-1257-4A:** 1H NMR (400 MHz, CDCl3) δ 3.95 (ddt, J = 7.8, 3.8, 2.0 Hz, 1H), 3.77 (tq, J = 7.2, 5.3, 3.7 Hz, 2H), 3.72 - 3.60 (m, 2H), 3.55 (dd, J = 9.4, 2.0 Hz, 2H), 3.52 - 3.41 (m, 2H), 3.38 (t, J = 1.8 Hz, 0H), 3.33 - 3.23 (m, 2H), 3.09 (t, J = 6.2 Hz, 2H), 1.20 (t, J = 1.8 Hz, 5H), 1.17 (d, J = 2.0 Hz, 4H), 1.02 - 0.92 (m, 6H). Two dimensional NOESY NMR identified a O17-OH to N11-NH NOE correlation and a C12-CH to C21-CH₃ NOE correlation to confirm relative stereochemistry of **I-1257-4A** as the cis diastereomer. **Isomer 2, I-1257-4B:** 1H NMR (400 MHz, CDCl3) δ 8.27 (d, J = 8.9 Hz, 1H), 8.11 (d, J = 7.7 Hz, 1H), 7.75 (d, J = 1.7 Hz, 1H), 7.33 (dd, J = 8.9, 1.7 Hz, 1H), 4.53 (dd, J = 9.6, 6.3 Hz, 1H), 4.24 (q, J = 7.0, 6.4 Hz, 1H), 3.98 (s, 3H), 3.91 (d, J = 9.8 Hz, 1H), 3.80 (d, J = 9.8 Hz, 1H), 3.73 (dd, J = 9.6, 4.6 Hz, 1H), 1.42 (s, 3H).

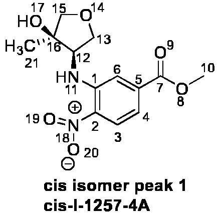

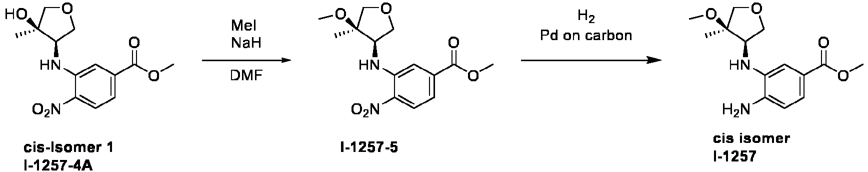

**methyl 3-((cis-4-methoxy-4-methyltetrahydrofuran-3-yl)amino)-4-nitrobenzoate (cis-isomer peak** 1, **I-1257-5**, **relative stereochemistry established):** A mixture of methyl 3-((4-hydroxy-4-methyltetrahydrofuran-3-yl)amino)-4-nitrobenzoate (144 mg, 0.49 mmol) **(cis-isomer peak 1, I-1257-4A)**, sodium hydride (20.5 mg, 0.54 mmol) and iodomethane (33 uL, 0.54 mmol) in DMF (1.00 mL) was stirred at 0 °C for 15 min then warmed to 25 °C and stirred for 16h. The mixture was diluted in EtOAc and washed with brine, Dried over sodium sulfate, concentrated, and purified by silica gel flash column chromatography (eluent: EtOAc/hexanes) to give the title compound. ES/MS: 311.3 (M+H+).

**methyl 4-amino-3-((4-methoxy-4-methyltetrahydrofuran-3-yl)amino)benzoate (cis-isomer peak 1, I-1257, relative stereochemistry established):** A mixture of methyl 3-((4-methoxy-4-methyltetrahydrofuran-3-yl)amino)-4-nitrobenzoate (133 mg, 0.43 mmol) (cis-isomer peak 1,**I-1257-5),** and 10% palladium on carbon (45.5 mg, 0.043 mmol) in ethanol (5.0 mL) and placed under hydrogen at 25 °C for 5 h. The mixture was diluted in EtOAc and washed with brine, dried over sodium sulfate, and concentrated to yield the title compound. ES/MS: 281.1 (M+H+)

### Preparation of Intermediate I-1258 cis-isomer 2:

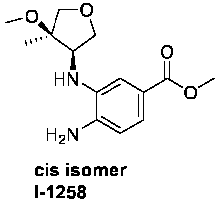

**Methyl 4-amino-3-((4-methoxy-4-methyltetrahydrofuran-3-yl)amino)benzoate (cis-isomer 2, I-1258, relative stereochemistry established):** Methyl 4-amino-3-((4-methoxy-4-methyltetrahydrofuran-3-yl)amino)benzoate **(cis-isomer 2, I-1258**, relative stereochemistry established) was prepared in a manner as described for **cis-isomer peak** 1 **I-1257**, using Intermediate **isomer 2 I-1258-1** in place of **isomer 1 I-1257-1.**

### Preparation of Intermediate I-1266

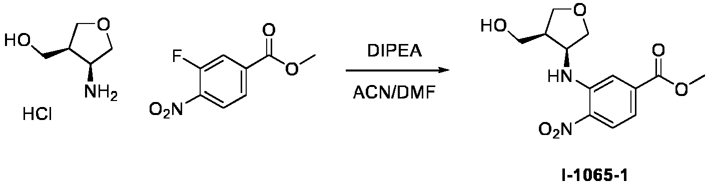

**Methyl rac-cis-4-amino-3-(((1-(fluoromethyl)cyclopropyl)methyl)amino)benzoate (I-1065-1):** To a mixture of methyl 3-fluoro-4-nitro-benzoate (120mg, 0.603 mmol), racemic cis-4-aminotetrahydrofuran-3-yl]methanol (77.7 mg, 0.66 mmol) in THF (4 mL) and DMF (2 mL), was added N,N-diisopropylethylamine (0.525 mL, 3.01 mmol). The mixture was heated at 80 °C for 18 hr. The crude mixture was diluted with EtOAc, washed with 5% LiCl and brine. The organic extract was dried over sodium sulfate and purified by silica gel column chromatography (eluent: EtOAc/hexanes) to afford the title compound. ES/MS: 297.2 (M+H⁺).

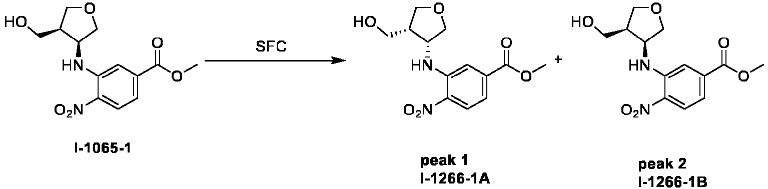

**Methyl 3-((cis-4-(hydroxymethyl)tetrahydrofuran-3-yl)amino)-4-nitrobenzoate (Peak 1-I-1266-1A, Peak 2-I-1266-1B, relative stereochemistry established):** Methyl 3-((cis-4-(hydroxymethyl)tetrahydrofuran-3-yl)amino)-4-nitrobenzoate (Peak 1-I-1266-1A, Peak 2-I-1266-1B) was obtained via preparative chiral SFC (Daicel Chiralpak AD-H column with EtOH/CO₂ eluent) on **I-1065-1,** which gave 2 distinct stereoisomers.

**Methyl 3-(((3R,4R)-4-(hydroxymethyl)tetrahydrofuran-3-yl)amino)-4-nitrobenzoate (Peak 1, 1-1266-1A):** the earlier eluting of the two stereoisomers.

**Methyl 3-(((3S,4S)-4-(hydroxymethyl)tetrahydrofuran-3-yl)amino)-4-nitrobenzoate (Peak 2, 1-1266-1B):** the later eluting of the two stereoisomers.

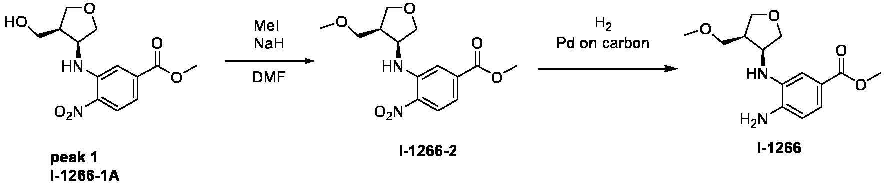

**Methyl 3-(((3S,4S)-4-(methoxymethyl)tetrahydrofuran-3-yl)amino)-4-nitrobenzoate (I-1266-2):** To a mixture of methyl 3-(((3S,4S)-4-(hydroxymethyl)tetrahydrofuran-3-yl)amino)-4-nitrobenzoate **(I-1266-1A)** (75 mg, 0.253 mmol) in DMF (4 mL) was added NaH (24.2 mg, 0.633 mmol, 60% dispersion) at 0 °C. To this mixture was added methyl iodide (35.9 mg, 0.253 mmol) and the mixture stirred for 1 hour at rt. The mixture was then split between saturated aqueous ammonium chloride and EtOAc. The aqueous layer was extracted with EtOAc five times, and then washed with brine. The organic extract was dried over sodium sulfate and purified by silica gel column chromatography (eluent: EtOAc/hexanes) to afford the title compound. ES/MS: 311.2 (M+H⁺)

**Methyl 4-amino-3-(((3S,4S)-4-(methoxymethyl)tetrahydrofuran-3-yl)amino)benzoate (I-1266, relative stereochemistry established):** A mixture of methyl 3-(((3S,4S)-4-(methoxymethyl)tetrahydrofuran-3-yl)amino)-4-nitrobenzoate **(I-1266-2)** (80 mg, 0.258 mmol) in EtOAc (5 mL) was degassed with cycles of argon then vacuum 3x. Palladium on carbon (10.0 %, 27.4 mg, 0.258 mmol) was added. The mixture was degassed with cycles of argon then vacuum and stirred at rt with a balloon of hydrogen for 24 hr. The mixture was filtered through Celite and concentrated in vacuo to give the title compound. ES/MS: 280.1 (M+H⁺).

### Preparation of Intermediate I-1267

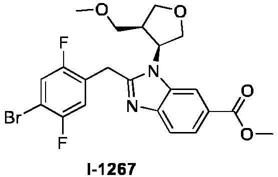

**Methyl 2-(4-bromo-2,5-difluorobenzyl)-1-((3S,4S)-4-(methoxymethyl)tetrahydrofuran-3-yl)-1H-benzo[d]imidazole-6-carboxylate:** Methyl 2-(4-bromo-2,5-difluorobenzyl)-1-((3S,4S)-4-(methoxymethyl)tetrahydrofuran-3-yl)-1H-benzo[d]imidazole-6-carboxylate was prepared in a manner as described for **-I-82**, substituting methyl 4-amino-3-(((3S,4S)-4-(methoxymethyl)tetrahydrofuran-3-yl)amino)benzoate for **I-80**.

### Preparation of Intermediate I-1268

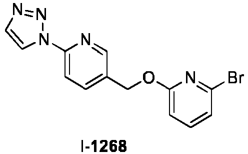

**2-bromo-6-[[6-(triazol-1-yl)-3-pyridyl]methoxy]pyridine (I-1268):** 2-bromo-6-[[6-(triazol-1-yl)-3-pyridyl]methoxy]pyridine was prepared in a manner as described for **I-84** substituting 5-(bromomethyl)-2-(triazol-1-yl)pyridine for 2-(bromomethyl)thiazole-5-carbonitrile. ES/MS: 332, 334 (M+H+).

### Preparation of Intermediate I-1269

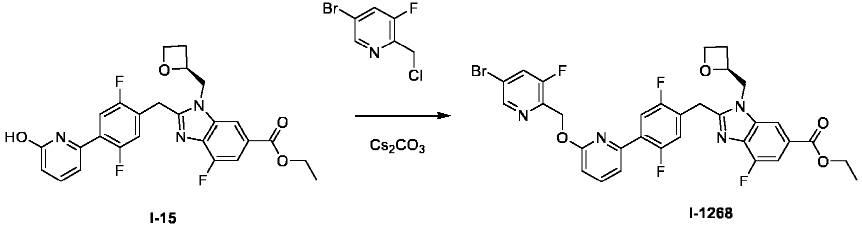

**Ethyl 2-[[4-[6-[(5-bromo-3-fluoro-2-pyridyl)methoxy]-2-pyridyl]-2,5-difluorophenyl]methyl]-7-fluoro-3-[[(2S)-oxetan-2-yl]methyl]benzimidazole-5-carboxylate (I-1269):** A suspension of ethyl 2-[[2,5-difluoro-4-(6-hydroxy-2-pyridyl)phenyl]methyl]-7-fluoro-3-[[(2S)-oxetan-2-yl]methyl]benzimidazole-5-carboxylate (300 mg, 0.603 mmol), 5-bromo-2-(chloromethyl)-3-fluoro-pyridine (162 mg, 0.724 mmol), and cesium carbonate (491 mg, 1.51 mmol) in CH₃CN was heated at 70 °C for 1 hr. Next, the mixture was filtered, concentrated, and purified by silica gel column chromatography (eluent: EtOAc/hexanes) to give desired product. ES/MS: 686.0 (M+H⁺).

### Preparation of Intermediate I-1270

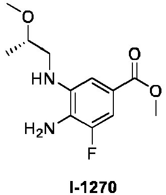

**Methyl 4-amino-3-fluoro-5-[[(2S)-2-methoxypropyl]amino]benzoate (I-1270):** Methyl 4-amino-3-fluoro-5-[[(2S)-2-methoxypropyl]amino]benzoate was prepared in a manner as described for **I-5** substituting (S)-2-methoxypropan-1-amine for (S)-oxetan-2-ylmethanamine and methyl 3,5-difluoro-4-nitrobenzoate for Ethyl 3,5-difluoro-4-nitrobenzoate. ES/MS: 257.1 (M+H⁺).

### Preparation of Intermediate I-1271

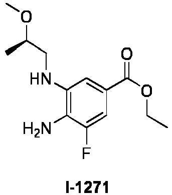

**Ethyl 4-amino-3-fluoro-5-[[(2R)-2-methoxypropyl]amino]benzoate (I-1271):** Ethyl 4-amino-3-fluoro-5-[[(2R)-2-methoxypropyl]amino]benzoate was prepared in a manner as described for **I-5** substituting (R)-2-methoxypropan-1-amine for (S)-oxetan-2-ylmethanamine. ES/MS: 271.2 (M+H⁺).

### Preparation of Intermediate I-1272

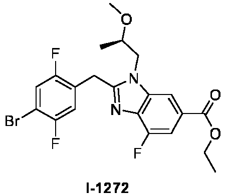

**Ethyl 2-[(4-bromo-2,5-difluoro-phenyl)methyl]-7-fluoro-3-[(2R)-2-methoxypropyl]benzimidazole-5-carboxylate (I-1272):** Ethyl 2-[(4-bromo-2,5-difluorophenyl)methyl]-7-fluoro-3-[(2R)-2-methoxypropyl]benzimidazole-5-carboxylate was prepared in a manner as described for **I-2** substituting Ethyl 4-amino-3-fluoro-5-[[(2R)-2-methoxypropyl]amino] **(I-1271)** for **I-1** and 2-(4-bromo-2,5-difluorophenyl)acetic acid for 2-(4-bromo-2-fluoro-phenyl)acetic acid. ES/MS: 485, 487 (M+H+).

### Preparation of Intermediate I-1273

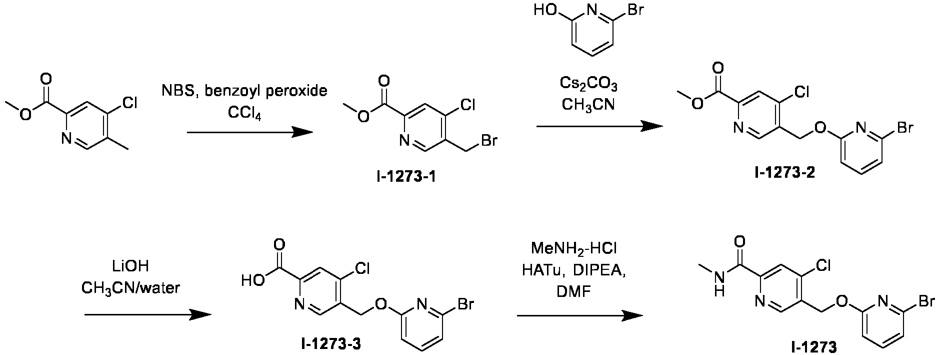

**Methyl 5-(bromomethyl)-4-chloro-pyridine-2-carboxylate (I-1273-1):** Methyl 4-chloro-5-methyl-pyridine-2-carboxylate (1.00 g, 5.39 mmol) was taken up in carbon tetrachloride (10.0 mL) and then N-Bromosuccinimide (1255 mg, 7.05 mmol) was added followed by Benzoyl peroxide (140 mg, 0.580 mmol). Next, the mixture was heated to 90° C for 45 min. Upon completion of time, the mixture was concentrated. in vacuo. The crude residue was purified by chromatography (eluent: EtOAc/hexanes) to give desired product. ES/MS: 264, 266 (M+H+).

**Methyl 5-[(6-bromo-2-pyridyl)oxymethyl]-4-chloro-pyridine-2-carboxylate (I-1273-2):** 6-Bromopyridin-2-ol (600 mg, 3.4 mmol), methyl 5-(bromomethyl)-4-chloro-pyridine-2-carboxylate (1003 mg, 3.8 mmol), and cesium carbonate (1685 mg, 5.2 mmol) were taken up in acetonitrile (12 mL) and the reaction heated to 65°C for 1 hr. Next, the mixture was filtered through Celite and concentrated in vacuo. The crude residue was purified by chromatography (eluent: EtOAc/hexanes) to give desired product. ES/MS: 357, 359 (M+H+).

**5-[(6-bromo-2-pyridyl)oxymethyl]-4-chloro-pyridine-2-carboxylic acid (I-1273-3):** Methyl 5-[(6-bromo-2-pyridyl)oxymethyl]-4-chloro-pyridine-2-carboxylate (1.17 g, 3.27 mmol) was taken up in CH₃CN (15.0 mL) and water (5.00 mL). Next, lithium hydroxide, monohydrate (408 mg, 9.72 mmol) was added to the mixture and stirred at rt for 1hr. The mixture was then diluted with EtOAc, acidified to pH~6 with 1N HCl. A precipitate formed and the precipitate was collected and washed with H₂O and Et₂O. The remaining material was extracted into EtOAc (dried over MgSO₄ and conc. in vacuo). The combined solids were used in subsequent reactions without purification. ES/MS: 343, 345 (M+H+).

**5-[(6-bromo-2-pyridyl)oxymethyl]-4-chloro-N-methyl-pyridine-2-carboxamide (I-1273):** To a solution of 5-[(6-bromo-2-pyridyl)oxymethyl]-4-chloro-pyridine-2-carboxylic acid (600 mg, 1.75 mmol), methanamine;hydrochloride (236 mg, 3.49 mmol), and o-(7-Azabenzotriazol-1-yl)-N,N,N',N'-tetramethyluronium hexafluorophosphate (976 mg, 2.57 mmol) in DMF (10 mL), N,N-Diisopropylethylamine (1.22 mL, 6.99 mmol) was added. The mixture was stirred at rt for 10 min. Next, the mixture was partitioned with EtOAc and water. The organic extract was dried over MgSO₄, filtered, and concentrated to give desired product. ES/MS: 356, 358 (M+H+).

### Preparation of Intermediate I-1274

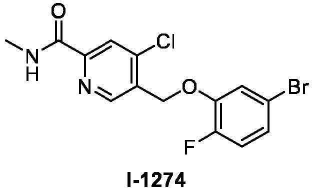

**Methyl 5-[(5-bromo-2-fluoro-phenoxy)methyl]-4-chloro-pyridine-2-carboxylate (I-1274):** Methyl 5-[(5-bromo-2-fluoro-phenoxy)methyl]-4-chloro-pyridine-2-carboxylate was prepared in a similar manner as described for I-1273 substituting 5-bromo-2-fluoro-phenol for 6-bromopyridin-2-ol in step 2. ES/MS: 374, 376 (M+H+).

### Preparation of Intermediate I-1275

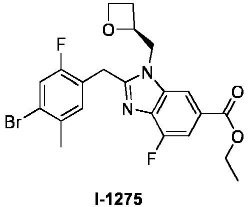

**Ethyl 2-[(4-bromo-2-fluoro-5-methyl-phenyl)methyl]-7-fluoro-3-[[(2S)-oxetan-2-yl]methyl]benzimidazole-5-carboxylate (I-1275):** Ethyl 2-[(4-bromo-2-fluoro-5-methylphenyl)methyl]-7-fluoro-3-[[(2S)-oxetan-2-yl]methyl]benzimidazole-5-carboxylate was prepared in a similar manner as described for **I-2** substituting **I-5** for **I-1** and 2-(4-bromo-2-fluoro-5-methyl-phenyl)acetic acid **I-1277** for 2-(4-bromo-2-fluoro-phenyl)acetic acid. ES/MS: 479, 481 (M+H+).

### Preparation of Intermediate I-1276

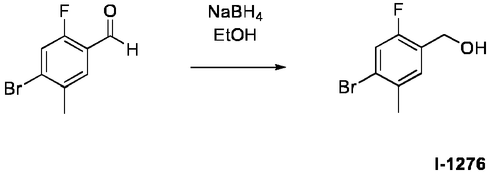

**(4-bromo-2-fluoro-5-methyl-phenyl)methanol (I-1276):** To a solution of 4-bromo-2-fluoro-5-methyl-benzaldehyde (5.0 g, 23.0 mmol) in EtOH (115 mL) at 0 ⁰C, sodium borohydride (436 mg, 11.5 mmol) was added in one portion. The mixture was placed under an inert atmosphere of argon and allowed to warm to room temperature and stirred for 2 h. The mixture was then quenched with saturated sodium NaHCO₃ (2x 30 ml), extracted with EtOAc (2x 30 ml) and dried over magnesium sulfate. The mixture was then filtered and concentrated in vacuo. The crude residue was purified by chromatography (eluent: EtOAc/hexanes) to give desired product. ¹HNMR (400 MHz, CDCl3) δ 7.29 (d, J = 7.8 Hz, 1H), 7.25 (d, J = 9.3 Hz, 1H), 4.68 (s, 2H), 2.36 (s, 3H). ¹⁹F NMR (376 MHz, Chloroform-d) δ -122.82.

### Preparation of Intermediate I-1277

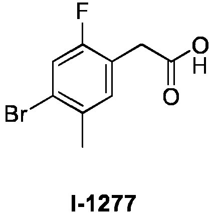

**2-(4-bromo-2-fluoro-5-methyl-phenyl)acetic acid (I-1277):** 2-(4-bromo-2-fluoro-5-methyl-phenyl)acetic acid was prepared in a manner as described for **I-1023** substituting (4-bromo-2-fluoro-5-methyl-phenyl)methanol **I-1276** for (4-bromo-2-chloro-5-methylphenyl)methanol in step 3. ¹H NMR (400 MHz, CDCl3) δ 7.28 (d, J = 9.0 Hz, 1H), 7.12 (d, J = 7.8 Hz, 1H), 3.64 (s, 2H), 2.34 (s, 3H). ¹⁹F NMR (376 MHz, CDCl3) δ -119.92.

### Preparation of Intermediate I-1278

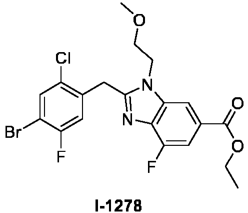

**Ethyl 2-[(4-bromo-2-chloro-5-fluoro-phenyl)methyl]-7-fluoro-3-(2-methoxyethyl)benzimidazole-5-carboxylate (I-1278):** Ethyl 2-[(4-bromo-2-chloro-5-fluorophenyl)methyl]-7-fluoro-3-(2-methoxyethyl)benzimidazole-5-carboxylate was prepared in a manner as described for **I-26** substituting ethyl 4-amino-3-fluoro-5-((2-methoxyethyl)amino)benzoate **I-1032** for ethyl 4-amino-3-fluoro-5-[[(2S)-oxetan-2-yl]methylamino]benzoate and 2-(4-bromo-2-chloro-5-fluoro-phenyl)acetic acid for 2-(4-bromo-2-chloro-5-methyl-phenyl)acetic acid. ES/MS: 488, 489 (M+H+).

### Preparation of Intermediate I-1279

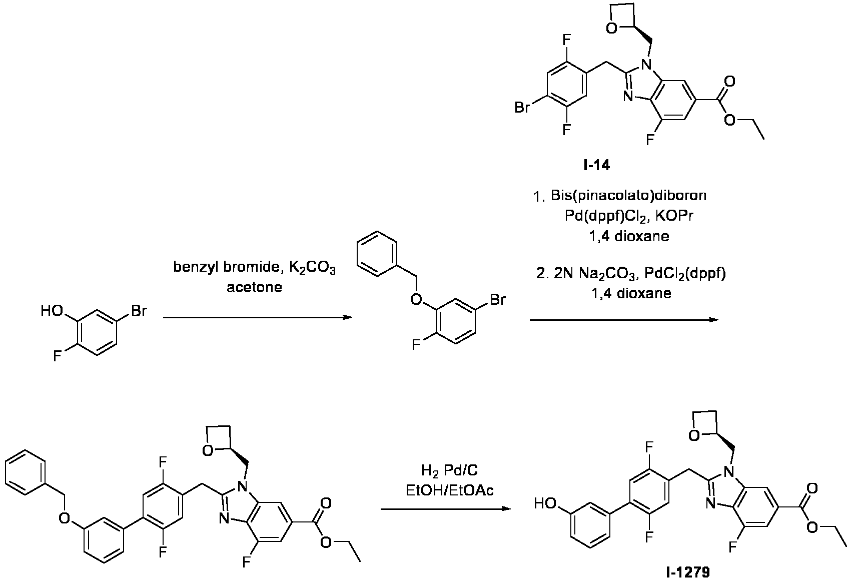

**2-benzyloxy-4-bromo-1-fluoro-benzene:** A suspension of 5-bromo-2-fluoro-phenol (5.00 g, 0.0262 mol), Benzyl bromide, reagent grade, 98% (3.74 mL, 0.0314 mol), and potassium carbonate (7.96 g, 0.0576 mol) in acetone (30 mL) was heated at 70 °C for 1 hr. The mixture was partitioned with EtOAc and water. The organic extract was dried over MgSO₄, concentrated and purified by chromatography (eluent: EtOAc/hexanes) to give desired product.

**Ethyl 2-[[4-(3-benzyloxy-4-fluoro-phenyl)-2,5-difluoro-phenyl]methyl]-7-fluoro-3-[[(2S)-**oxetan-2-yl]methyl]benzimidazole-5-carboxylate: A suspension of ethyl 2-[(4-bromo-2,5-difluoro-phenyl)methyl]-7-fluoro-3-[[(2S)-oxetan-2-yl]methyl]benzimidazole-5-carboxylate (500 mg, 1.03 mmol) **(I-14),** [1,1'-Bis(diphenylphosphino)ferrocene] dichloropalladium(II) ;PdC12(dppf) (76.7 mg, 0.103 mmol), potassium propionate (348 mg, 3.10 mmol), and Bis(pinacolato)diboron (394 mg, 1.55 mmol) was degassed, then heated at 110 °C for 2hr. Next, sodium carbonate (2000 mmol/L, 1.03 mL, 2.07 mmol), 2-benzyloxy-4-bromo-1-fluoro-benzene (320 mg, 1.14 mmol), and [1,1'-Bis(diphenylphosphino)ferrocene] dichloropalladium(II) ;PdCl₂(dppf) (38.4 mg, 0.0517 mmol) were added. Next the mixture was degassed. The mixture was then heated at 100 °C for 1 hr. Upon completion of time, the mixture was diluted with EtOAc and water. The organic extract was dried over magnesium sulfate, filtered and concentrated. The crude residue was purified by flash chromatography (eluent: EtOAc/hexanes) to yield desired product. ES/MS: 606.1 (M+H+).

**Ethyl 2-[[2,5-difluoro-4-(4-fluoro-3-hydroxy-phenyl)phenyl]methyl]-7-fluoro-3-[[(2S)-oxetan-2-yl]methyl]benzimidazole-5-carboxylate (I-1279):** A suspension of ethyl 2-[[4-(3-benzyloxy-4-fluoro-phenyl)-2,5-difluoro-phenyl]methyl]-7-fluoro-3-[[(2S)-oxetan-2-yl]methyl]benzimidazole-5-carboxylate (626 mg, 1.04 mmol) and Palladium on carbon 10 wt. % (5.00 %, 2204 mg, 1.04 mmol) was degassed by cycling the mixture between argon and vacuum 3x. Next the mixture was stirred at rt with a balloon of hydrogen for 2 hr. The mixture was then filtered through Celite and concentrated in vacuo to give desired product. ES/MS: 515.6 (M+H+)

### Preparation of Intermediate I-1280

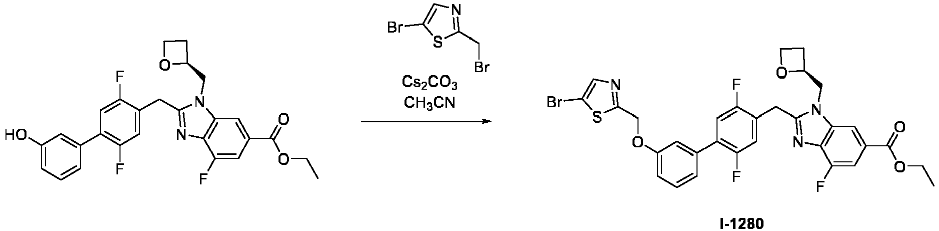

**Ethyl 2-[[4-[3-[(5-bromothiazol-2-yl)methoxy]-4-fluoro-phenyl]-2,5-difluorophenyl]methyl]-7-fluoro-3-[[(2S)-oxetan-2-yl]methyl]benzimidazole-5-carboxylate (I-1280):** A suspension of 5-bromo-2-(bromomethyl)thiazole (40.0 mg, 0.156 mmol), ethyl 2-[[2,5-difluoro-4-(4-fluoro-3-hydroxy-phenyl)phenyl]methyl]-7-fluoro-3-[[(2S)-oxetan-2-yl]methyl]benzimidazole-5-carboxylate (80.0 mg, 0.156 mmol), and cesium carbonate (152 mg, 0.467 mmol) in CH₃CN was heated at 60 °C for 1 hr. The mixture was filtered, concentrated, and purified by chromatography (eluent: EtOAc/hexanes) to give desired product. ES/MS: 692.3 (M+H+)

### Preparation of Intermediate I-1281

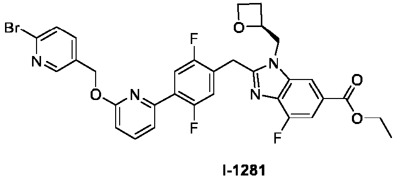

**Ethyl 2- [[4- [6- [(6-bromo-3-pyridyl)methoxy] -2-pyridyl] -2,5-difluorophenyl]methyl]-7-fluoro-3-[[(2S)-oxetan-2-yl]methyl]benzimidazole-5-carboxylate (I-1281):** Ethyl 2-[[4-[6-[(6-bromo-3-pyridyl)methoxy]-2-pyridyl]-2,5-difluoro-phenyl]methyl]-7-fluoro-3-[[(2S)-oxetan-2-yl]methyl]benzimidazole-5-carboxylate was prepared in a manner as described for Intermediate **I-1269** substituting 2-bromo-5-(bromomethyl)pyridine for 5-bromo-2-(chloromethyl)-3-fluoro-pyridine. ES/MS: 667, 669 (M+H⁺).

### Preparation of Intermediate I-1285

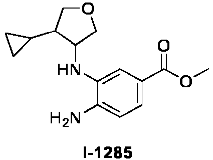

**Methyl 4-amino-3-((4-cyclopropyltetrahydrofuran-3-yl)amino)benzoate (I-1285):** Methyl 4-amino-3-((4-cyclopropyltetrahydrofuran-3-yl)amino)benzoate was prepared in a manner as described for I-69 substituting 4-cyclopropyltetrahydrofuran-3-amine hydrochloride for 2-methoxyethylamine. ES/MS: 277.2 (M+H+).

### Preparation of Intermediate I-1314

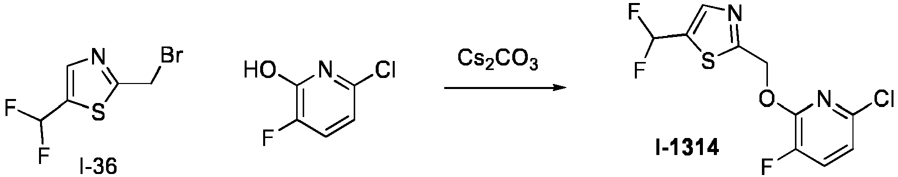

**2-(((6-chloro-3-fluoropyridin-2-yl)oxy)methyl)-5-(difluoromethyl)thiazole (I-1314):** To a vial containing 6-chloro-3-fluoro-pyridin-2-ol (20 mg, 1.0 equivalent) under argon, Cs₂CO₃ (66 mg, 1.5 equivalent) was added. Next, MeCN (1.3 mL) was added followed by the addition of 2-(bromomethyl)-5-(difluoromethyl)thiazole (**I-36**). The mixture was sealed under argon and stirred in a metal heating block at 90 °C for 90 min or until the reaction as complete as determined by LCMS. The mixture was then diluted with EtOAc, filtered, and concentrated in vacuo. Silica gel flash column chromatography (EtOAc in hexane) yielded the title compound. 1H NMR (400 MHz, Chloroform-d) δ 7.94 (t, J = 2.3 Hz, 1H), 7.38 (dd, J = 9.0, 8.2 Hz, 1H), 6.96 (dd, J = 8.2, 2.6 Hz, 1H), 6.90 (t, J = 55.3 Hz, 1H), 5.73 (s, 2H). ES/MS m/z: 294.9 (M+H⁺).

### Preparation of Intermediate I-1315

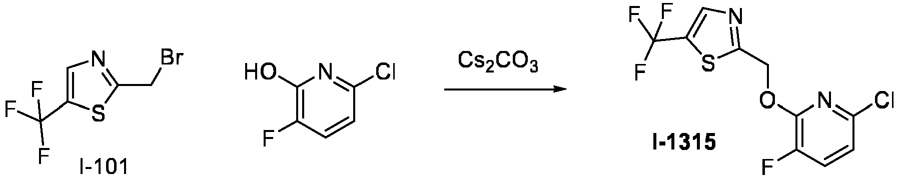

**2-(((6-chloro-3-fluoropyridin-2-yl)oxy)methyl)-5-(trifluoromethyl)thiazole (I-1315):** To 6-chloro-3-fluoro-pyridin-2-ol (20 mg, 1.0 equivalent) under argon was added Cs₂CO₃ (66 mg, 1.5 equivalent) followed by the addition of MeCN (1.3 mL), and then 2-(bromomethyl)-5-(trifluoromethyl)thiazole (**I-101**). The mixture was sealed under argon and stirred in a metal heating block at 90 °C for 30 min or until the reaction as complete as determined by LCMS. The mixture was then diluted with EtOAc, filtered, and concentrated in vacuo. Silica gel flash column chromatography (EtOAc in hexane) yielded the title compound. 1H NMR (400 MHz, Chloroform-d) δ 8.09 (d, J = 1.3 Hz, 1H), 7.40 (dd, J = 9.0, 8.2 Hz, 1H), 6.98 (dd, J = 8.2, 2.6 Hz, 1H), 5.74 (s, 2H). ES/MS m/z: 313.0 (M+H⁺).

### Preparation of Intermediate I-1316

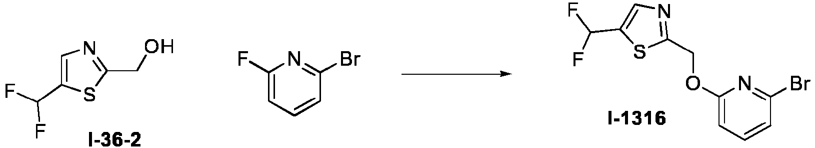

**2-(((4-bromopyrimidin-2-yl)oxy)methyl)-5-(difluoromethyl)thiazole (I-1317):** To (5-(difluoromethyl)thiazol-2-yl)methanol (**I-36-2** from preparation of **I-36,** 4.0g, 1.0 equivalent) in acetonitrile (150 mL), 2-bromo-6-fluoropyridine (4.69g, 1.1 equivalent) and cesium carbonate (11.8g, 1.5 equivalent) were added. The mixture was stirred at 60 °C for 3 hr, or until complete as determined by LCMS. The mixture was then filtered and concentrated in vacuo, then purified by silica gel flash column chromatography to yield the title compound. 1H NMR (400MHz,CDCl3) δ 7.94 (t, J = 1.2 Hz, 1H), 7.50 (t, J = 7.8 Hz, 1H),7.17 (d, J = 8 Hz, 1H), 6.90 (t, J = 56 Hz, 1H), 6.79 (d, J = 8 Hz, 1H), 5.68 (s, 2H). ES/MS m/z: 322.8 (M+H⁺).

### Preparation of Intermediate I-1317

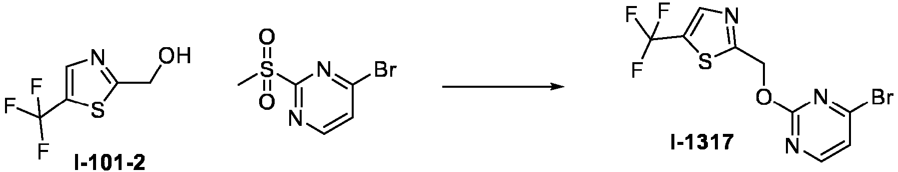

**2-(((4-bromopyrimidin-2-yl)oxy)methyl)-5-(difluoromethyl)thiazole (I-1317):** To (5-(trifluoromethyl)thiazol-2-yl)methanol (I-101-2 from preparation of Intermediate 101, 212 mg, 1.1 equivalent) in THF (4 mL) at 0 °C , potassium tert-butoxide (1 M in THF, 1.05 mL, 1.0 equivalent) was added. The mixture was stirred at 0 °C for 5 min, then it was added dropwise to a mixture of 4-bromo-2-(methylsulfonyl)pyrimidine (250 mg, 1.0 equivalent) in THF (4 mL) pre-cooled to -80 °C. The resulting mixture was then stirred at -80 °C for 90 min. Upon completion of time, the mixture was quenched with addition of saturated aqueous ammonium chloride. Next, the mixture was allowed to warm to room temperature, then partitioned between ethyl acetate and saturated aqueous ammonium chloride. The organic phase was washed with brine, then dried over magnesium sulfate, filtered, and concentrated in vacuo. Purification by silica gel flash column chromatography yielded the title compound. 1H NMR (400 MHz, Chloroform-d) δ 8.34 (d, J = 5.2 Hz, 1H), 8.08 (d, J = 1.2 Hz, 1H), 7.28 - 7.26 (m, 1H), 5.74 (s, 2H). ES/MS m/z: 339.9 (M+H⁺).

### Preparation of Intermediate I-1288

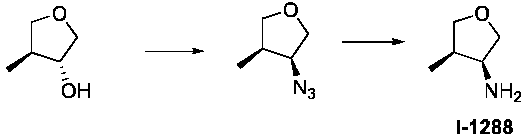

**Racemic (3S,4R)-4-methyltetrahydrofuran-3-amine (I-1288):** To a mixture of racemic (3R,4S)-4-methyltetrahydrofuran-3-ol (320 mg, 1.0 equivalent), triphenylphosphine (985 mg, 1.2 equivalent), diisopropylethylamine (0.56 mL, 1.02 equivalent) in THF (15 mL) precooled to 0 °C, diisopropyl azodicarboxylate (0.74 mL, 1.2 equivalent) was added. The mixture was then stirred at 0 °C for 15 min. Upon completion of timediphenylphosphoryl azide (0.81 mL, 1.2 equivalent) was added. The mixture was allowed to warm to 20 °C with stirring for 3 hr, then cooled back to 0 °C. Additional triphenylphosphine (1.06 g, 1.3 equivalent) as a solution in THF (6 mL) was added to the mixture, and the mixture was allowed to stir at 20 °C for 2.5 hr. Upon completion of time, water (1.5 mL) was added, and the mixture was stirred at 65 °C for 18 hr (OPERATIONAL NOTE: significant gas evolution was observed). The mixture was cooled and partitioned between ethyl acetate and a mixture of 1 N aqueous NaOH, saturated aqueous NaHCO₃ and brine; extracted aqueous phase twice more with EtOAc. The combined organic phases were dried over MgSO₄, filtered, concentrated in vacuo to yield the title compound, which was carried forward crude without further purification. ES/MS m/z: 101.9 (M+H⁺).

### Preparation of Intermediate I-1289

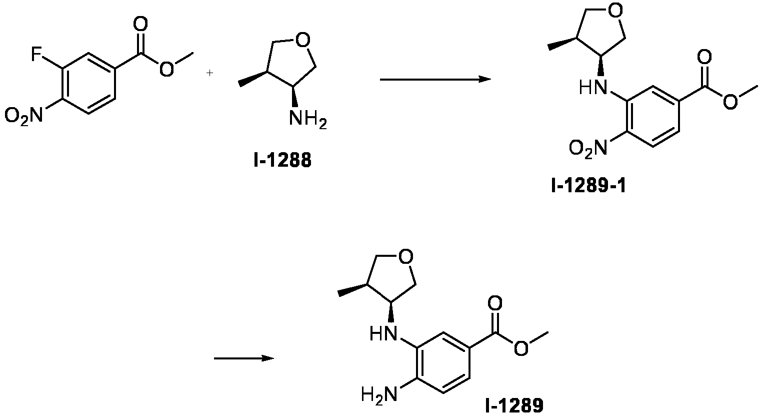

**Racemic methyl 3-[[(3R,4S)-4-methyltetrahydrofuran-3-yl]amino]-4-nitrobenzoate (I-1289-1):** To crude racemic (3R,4S)-4-methyltetrahydrofuran-3-amine (I-1288, 317 mg, 1.0 equivalent), methyl 3-fluoro-4-nitrobenzoate (687 mg, 1.1 equivalent), THF (8.8 mL), DMF (4.4 mL), and diisopropylethylamine (2.73 mL, 5.0 equivalent) were added. The mixture was stirred at 80 °C for 16 hr, or until completion as determined by LCMS. The mixture was then diluted with ethyl acetate and washed with saturated aqueous NH₄Cl, then brine. Next, the mixture was dried over magnesium sulfate, filtered and concentrated in vacuo to yield the title compound.

**Racemic methyl 4-amino-3-[[(3R,4S)-4-methyltetrahydrofuran-3-yl]amino]benzoate (I-1289):** A mixture of crude racemic methyl 3-[[(3R,4S)-4-methyltetrahydrofuran-3-yl]amino]-4-nitrobenzoate (**I-1289-1,** 878 mg, 1.0 equivalent), 10% palladium on carbon (333 mg, 0.10 equivalent) and ethanol (60 mL) was stirred under an atmosphere of hydrogen for 6 hr, or until completion as determined by LCMS. The mixture was filtered, concentrated in vacuo and purified by silica gel flash column chromatography (hexane/EtOAc) to yield the title compound. 1H NMR (400 MHz, Chloroform-d) δ 7.45 (dd, J = 8.1, 1.8 Hz, 1H), 7.34 (d, J = 1.9 Hz, 1H), 6.69 (d, J = 8.1 Hz, 1H), 4.09 - 4.02 (m, 3H), 3.85 (s, 3H), 3.68 (h, J = 3.8 Hz, 1H), 3.57 (dd, J = 8.5, 7.2 Hz, 1H), 2.66 - 2.54 (m, 1H), 1.01 (d, J = 7.0 Hz, 3H). ES/MS m/z: 251.0 (M+H⁺).

### Preparation of Intermediate I-1290, I-1291:

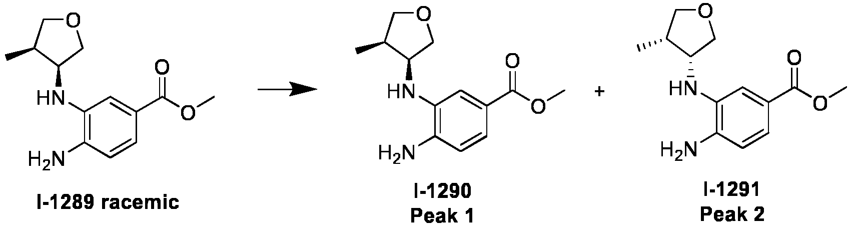

**Methyl 4-amino-3-[[(3S,4R)-4-methyltetrahydrofuran-3-yl]amino]benzoate (I-1290), Methyl 4-amino-3-[[(3R,4S)-4-methyltetrahydrofuran-3-yl]amino]benzoate (I-1291): Racemic I-1289** was purified by preparative chiral SFC (IG, 20% MeOH) to yield methyl 4-amino-3-[[(3S,4R)-4-methyltetrahydrofuran-3-yl]amino]benzoate (I-1290) as the earlier eluting isomer (Peak 1) and methyl 4-amino-3-[[(3R,4S)-4-methyltetrahydrofuran-3-yl]amino]benzoate (I-1291) as the later-eluting isomer (Peak 2).

**Methyl 4-amino-3-[[(3S,4R)-4-methyltetrahydrofuran-3-yl]amino]benzoate (I-1290):** ES/MS m/z: 251.0 (M+H⁺).

**Methyl 4-amino-3-[[(3R,4S)-4-methyltetrahydrofuran-3-yl]amino]benzoate (I-1291):** ES/MS m/z: 251.0 (M+H⁺).

### Preparation of Intermediate I-1292

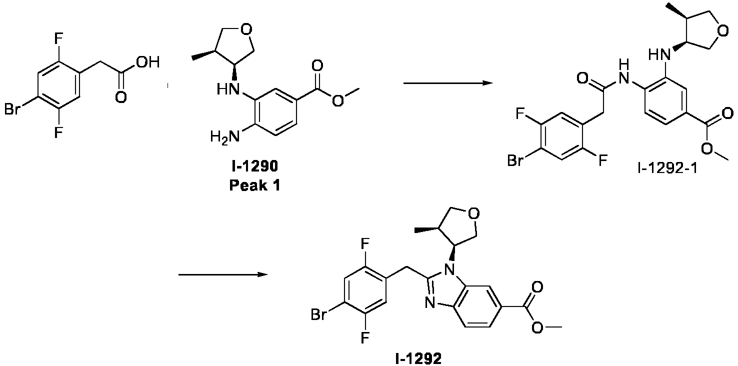

**Methyl 4-[[2-(4-bromo-2,5-difluoro-phenyl)acetyl] amino] -3- [[(3S,4R)-4-methyltetrahydrofuran-3-yl]amino]benzoate (I-1292-1):** A mixture of methyl 4-amino-3-[[(3S,4R)-4-methyltetrahydrofuran-3-yl]amino]benzoate (**I-1290,** 57 mg, 1.0 equivalent), 2-(4-bromo-2,5-difluorophenyl)acetic acid (63 mg, 1.1 equivalent), chloro-N,N,N',N'-tetramethylformamidinium hexafluorophosphate (77 mg, 1.2 equivalent), 1-methylimidazole (94 mg, 5.0 equivalent) and acetonitrile (2.3 mL) was stirred at 20 °C for 1 hr. The mixture was then diluted with EtOAc, washed with saturated aqueous NH₄Cl. Next, the mixture was saturated with aqueous NaHCO₃, then brine. The organic phase was then dried over MgSO₄, filtered, and concentrated in vacuo to yield the title compound, which was carried forward without further purification. ES/MS m/z: 483.2 (M+H⁺).

**Methyl 2- [(4-bromo-2,5-difluoro-phenyl)methyl] -3- [(3S,4R)-4-methyltetrahydrofuran-3-yl]benzimidazole-5-carboxylate (I-1292):** A mixture of methyl 4-[[2-(4-bromo-2,5-difluorophenyl)acetyl]amino]-3-[[(3S,4R)-4-methyltetrahydrofuran-3-yl]amino]benzoate (**I-1292-1,** 110 mg, 1.0 equivalent), glacial acetic acid (0.91 mL) and 1,2-dichloroethane (4.6 mL) was stirred at 80-95 °C for 48 hr, or until completion as determined by LCMS. The mixture was diluted with EtOAc. Next the mixture was quenched with saturated aqueous NaHCOs and batchwise addition of solid NaHCOs. The organic phase was washed with brine, dried over MgSO₄, filtered, and concentrated in vacuo to yield the title compound. ES/MS m/z: 465.2 (M+H⁺).

### Preparation of Intermediate I-1293

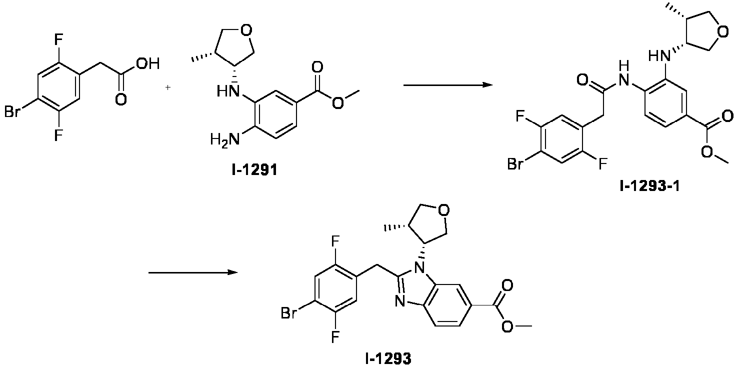

**Methyl 2-[(4-bromo-2,5-difluoro-phenyl)methyl]-3-[(3R,4S)-4-methyltetrahydrofuran-3-yl]benzimidazole-5-carboxylate (I-1293):** The title compound was prepared in a manner similar to Intermediate **I-1292,** substituting Intermediate **I-1291** in place of Intermediate **I-1290.** ES/MS m/z: 465.2 (M+H⁺).

### Preparation of Intermediate I-1306

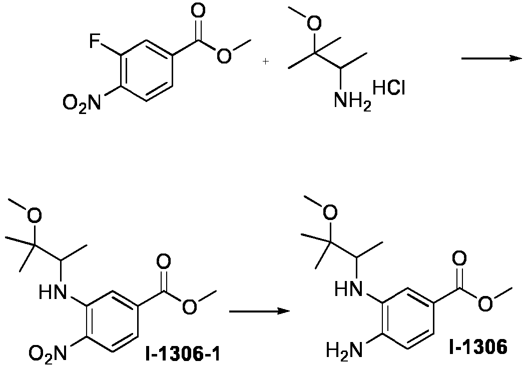

**Racemic methyl 3-[(2-methoxy-1,2-dimethyl-propyl)amino]-4-nitrobenzoate (I-1306-1):** To racemic 3-methoxy-3-methylbutan-2-amine hydrochloride (251 mg, 1.05 equivalent), methyl 3-fluoro-4-nitrobenzoate (310 mg, 1.0 equivalent), THF (2.0 mL), DMF (1.0 mL), and diisopropylethylamine (1.36 mL, 5.0 equivalent) were added. The mixture was then stirred at 80 °C for 20 hr, or until reaction completion as determined by LCMS. The mixture was diluted with ethyl acetate and washed with saturated aqueous NH₄Cl, and then brine. Next the mixture was dried over magnesium sulfate, filtered and concentrated in vacuo to yield the title compound. ES/MS m/z: 296.9 (M+H)⁺.

**Racemic methyl 4-amino-3-((3-methoxy-3-methylbutan-2-yl)amino)benzoate (I-1306):** A mixture of crude racemic methyl 3-[(2-methoxy-1,2-dimethyl-propyl)amino]-4-nitrobenzoate (**I-1306-1,** 461 mg, 1.0 equivalent), 10% palladium on carbon (83 mg, 0.10 equivalent) and ethanol (16 mL) was stirred under an atmosphere of hydrogen for 16 hr, or until reaction completion as determined by LCMS. The mixture was filtered, concentrated in vacuo and purified by silica gel flash column chromatography (hexane/EtOAc) to yield the title compound. 1H NMR (400 MHz, Methanol-d4) δ 7.34 - 7.26 (m, 2H), 6.68 (d, J = 8.0 Hz, 1H), 3.82 (s, 3H), 3.47 (q, J = 6.5 Hz, 1H), 3.25 (s, 3H), 1.27 (s, 6H), 1.16 (d, J = 6.5 Hz, 3H). ES/MS m/z: 266.9 (M+H)⁺.

### Preparation of Intermediate I-1307

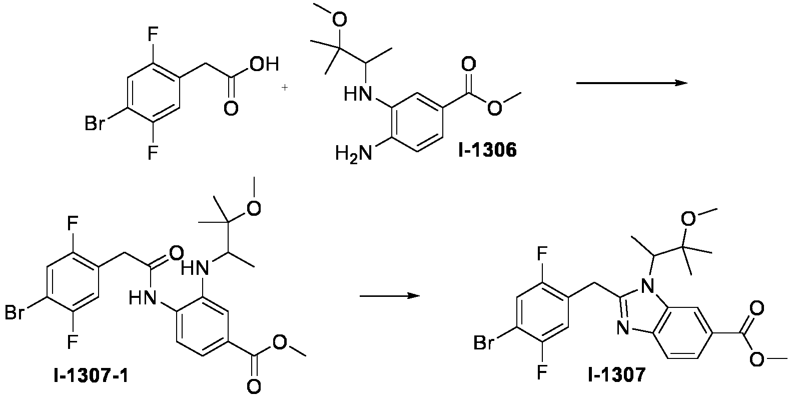

**Racemic methyl 4- [[2-(4-bromo-2,5-difluoro-phenyl)acetyl] amino] -3- [(2-methoxy-1,2-dimethyl-propyl)amino]benzoate (I-1307-1):** A mixture of racemic methyl 4-amino-3-((3-methoxy-3-methylbutan-2-yl)amino)benzoate (**I-1306,** 440 mg, 1.00 equivalent), 2-(4-bromo-2,5-difluorophenyl)acetic acid (435 mg, 1.05 equivalent), chloro-N,N,N',N'-tetramethylformamidinium hexafluorophosphate (487 mg, 1.05 equivalent), 1-methylimidazole (678 mg, 5.0 equivalent) and acetonitrile (8.1 mL) was stirred at 20 °C for 1 hr, or until reaction completion as determined by LCMS. The mixture was then diluted with EtOAc, washed with saturated aqueous NH₄Cl, then saturated aqueous NaHCO₃, and then brine. The organic phase was dried over MgSO4, filtered, and concentrated in vacuo to yield the title compound, which was carried forward without further purification. ES/MS m/z: 499.0 (M+H⁺).

**Racemic methyl 2-(4-bromo-2,5-difluorobenzyl)-1-(3-methoxy-3-methylbutan-2-yl)-1H-benzo[d]imidazole-6-carboxylate (I-1307):** A mixture of racemic methyl 4-[[2-(4-bromo-2,5-difluoro-phenyl)acetyl]amino]-3-[(2-methoxy-1,2-dimethyl-propyl)amino]benzoate (**I-1307-1,** 825 mg, 1.0 equivalent), glacial acetic acid (3.3 mL) and 1,2-dichloroethane (8.3 mL) was stirred at 80-95 °C for 96 hr, or until completion as determined by LCMS. The mixture was diluted with EtOAc, quenched with saturated aqueous NaHCOs and batchwise addition of solid NaHCOs. The organic phase was washed with brine, dried over MgSO₄, filtered, and concentrated in vacuo to yield the title compound. 1H NMR (400 MHz, Chloroform-d) δ 8.56 - 8.50 (m, 1H), 7.91 (dd, J = 8.5, 1.5 Hz, 1H), 7.69 (d, J = 8.5 Hz, 1H), 7.29 (dd, J = 8.6, 5.6 Hz, 1H), 7.03 (dd, J = 8.6, 6.3 Hz, 1H), 4.39 - 4.22 (m, 3H), 3.90 (s, 3H), 3.15 (s, 3H), 1.56 (d, J = 7.1 Hz, 3H), 1.26 (s, 3H), 0.98 (s, 3H). ES/MS m/z: 481.2 (M+H⁺).

### Preparation of Intermediate I-1310:

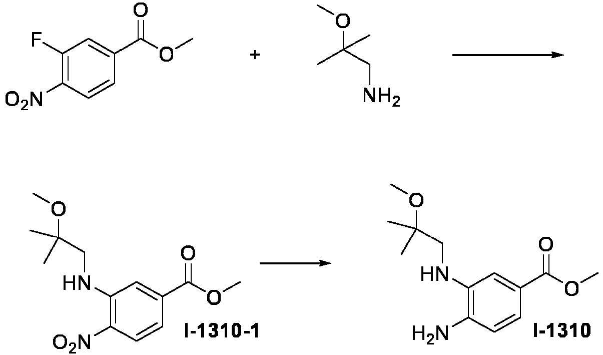

**Methyl 3-[(2-methoxy-2-methyl-propyl)amino]-4-nitrobenzoate (I-1310-1):** To 2-methoxy-2-methyl-propan-1-amine (285 mg, 1.1 equivalent), methyl 3-fluoro-4-nitrobenzoate (500 mg, 1.0 equivalent), 2-methyltetrahydrofuran (3.4 mL), DMF (1.7 mL), and diisopropylethylamine (2.2 mL, 5.0 equivalent) were added. The mixture was then stirred at 80 °C for 16 hr, or until reaction completion as determined by LCMS. Upon completion, the mixture was diluted with ethyl acetate and washed with saturated aqueous NH₄Cl, then brine. Next, the mixture was dried over magnesium sulfate, filtered and concentrated in vacuo to yield the title compound. ES/MS m/z: 282.8 (M+H)⁺.

**Methyl 4-amino-3-[(2-methoxy-2-methylpropyl)amino]benzoate (I-1310):** A mixture of crude methyl 3-[(2-methoxy-2-methyl-propyl)amino]-4-nitrobenzoate (**I-13010-1,** 555 mg, 1.0 equivalent), 10% palladium on carbon (105 mg, 0.10 equivalent) and ethanol (20 mL) was stirred under an atmosphere of hydrogen for 16 hr, or until reaction completion as determined by LCMS. The mixture was filtered, concentrated in vacuo and purified by silica gel flash column chromatography (hexane/EtOAc) to yield the title compound. 1H NMR (400 MHz, Methanol-d4) δ 7.32 (d, J = 8.0 Hz, 1H), 7.24 (d, J = 1.8 Hz, 1H), 6.68 (d, J = 8.1 Hz, 1H), 3.82 (s, 3H), 3.25 (s, 3H), 3.11 (s, 2H), 1.31 (s, 6H). ES/MS m/z: 252.9 (M+H)⁺.

### Preparation of Intermediate I-1311

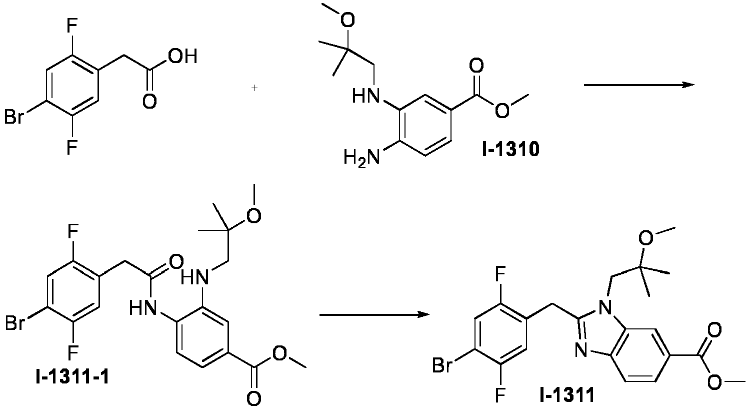

**Methyl 4-**[**[2-(4-bromo-2,5-difluorophenyl)acetyl] amino] -3- [(2-methoxy-2-methylpropyl)amino]benzoate (I-1311-1):** A mixture of methyl 4-amino-3-[(2-methoxy-2-methylpropyl)amino]benzoate (**I-1310,** 655 mg, 1.00 equivalent), 2-(4-bromo-2,5-difluorophenyl)acetic acid (684 mg, 1.05 equivalent), chloro-N,N,N',N'-tetramethylformamidinium hexafluorophosphate (765 mg, 1.05 equivalent), 1-methylimidazole (1066 mg, 5.0 equivalent) and acetonitrile (13 mL) was stirred at 20 °C for 2.5 hr, or until reaction completion as determined by LCMS. The mixture was then diluted with EtOAc, washed with saturated aqueous NH₄Cl, then saturated aqueous NaHCO₃, and then a final wash with brine. The organic phase was dried over MgSO₄, filtered, and concentrated in vacuo to yield the title compound, which was carried forward without further purification. ES/MS m/z: 485.0 (M+H⁺).

**Methyl 2-(4-bromo-2,5-difluorobenzyl)-1-(2-methoxy-2-methylpropyl)-1H-benzo[d]imidazole-6-carboxylate (I-1311):** A mixture of methyl 4-[[2-(4-bromo-2,5-difluorophenyl)acetyl]amino]-3-[(2-methoxy-2-methylpropyl)amino]benzoate (**I-1311-1,** 1260 mg, 1.0 equivalent), glacial acetic acid (5.3 mL) and 1,2-dichloroethane (13 mL) was stirred at 80 °C for 16 hr, or until completion as determined by LCMS. The mixture was diluted with EtOAc, quenched with saturated aqueous NaHCOs and batchwise addition of solid NaHCOs. The organic phase was washed with brine, dried over MgSO₄, filtered, and concentrated in vacuo to yield the title compound. 1H NMR (400 MHz, Chloroform-d) δ 8.16 (s, 1H), 8.05 (d, J = 8.6 Hz, 1H), 7.86 (d, J = 8.4 Hz, 1H), 7.35 (s, 1H), 7.30 (dd, J = 8.7, 5.5 Hz, 1H), 4.62 (s, 2H), 4.20 (s, 2H), 3.96 (s, 3H), 3.14 (s, 3H), 1.23 (s, 6H). ES/MS m/z: 467.2 (M+H⁺).

### Preparation of Intermediate I-1324

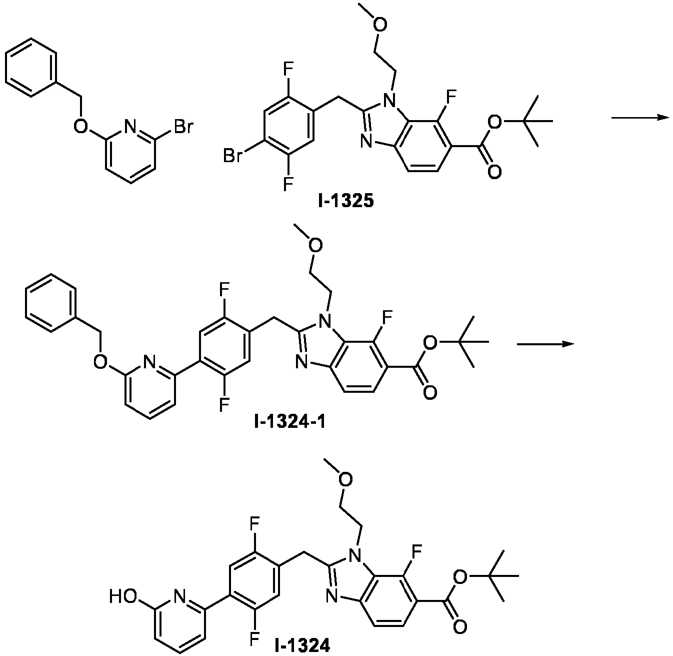

**Tert-butyl 2-(4-(6-(benzyloxy)pyridin-2-yl)-2,5-difluorobenzyl)-7-fluoro-1-(2-methoxyethyl)-1H-benzo[d]imidazole-6-carboxylate (I-1324-1):** A mixture of tert-butyl 2-(4-bromo-2,5-difluorobenzyl)-7-fluoro-1-(2-methoxyethyl)-1H-benzo[d]imidazole-6-carboxylate (49 mg, 0.965 equivalent), PdCl₂(dppf) (11.4 mg, 0.15 equivalent), potassium propionate (34 mg, 3.0 equivalent), and bis(pinacolato)diboron (34 mg, 1.3 equivalent) in 1,4-dioxane (2.0 mL) was purged with argon for 1 min. The mixture was then sealed and heated to 100 °C for 2 h. After cooling down to room temperature, 2-(benzyloxy)-6-bromopyridine (27 mg, 1.1 equivalent), PdCl₂(dppf) (5.7 mg, 0.075 equivalent), 2 M aqueous Na₂CO₃ (0.10 mL, 2.0 equivalent) were added, respectively. The resulting mixture was heated to 90 °C under argon and stirred for 2 hr, with monitoring by LCMS. The mixture was filtered and concentrated in vacuo. Purification by silica gel flash column chromatography (EtOAc/hexane gradient) yielded the title compound.

**Tert-butyl 2-(2,5-difluoro-4-(6-hydroxypyridin-2-yl)benzyl)-7-fluoro-1-(2-methoxyethyl)-1H-benzo[d]imidazole-6-carboxylate (I-1324):** A mixture of tert-butyl 2-(4-(6-(benzyloxy)pyridin-2-yl)-2,5-difluorobenzyl)-7-fluoro-1-(2-methoxyethyl)-1H-benzo[d]imidazole-6-carboxylate (I-1324-1, 40 mg, 1.0 equivalent), 10% palladium on carbon (10 mg, 0.14 equivalent), and ethanol (3.0 mL) was stirred under an atmosphere of hydrogen for 3 hr. The mixture was filtered and concentrated in vacuo to yield the title compound. ES/MS m/z: 514.7 (M+H⁺).

### Preparation of Intermediate I-1325

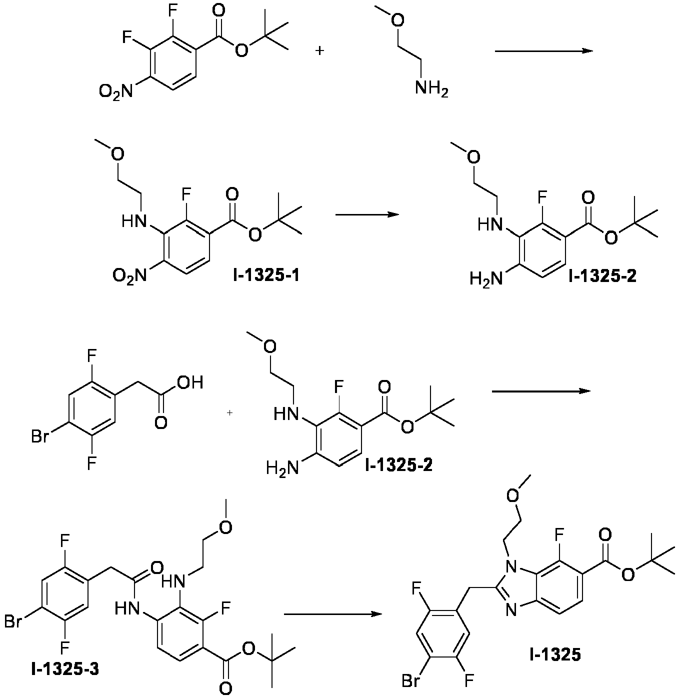

**Tert-butyl 2-fluoro-3-((2-methoxyethyl)amino)-4-nitrobenzoate (I-1325-1):** To a vial containing 2-methoxyethanamine (191 mg, 1.1 equivalent), tert-butyl 2,3-difluoro-4-nitrobenzoate (600 mg, 1.0 equivalent), THF (10 mL), and diisopropylethylamine (2.0 mL, 5.0 equivalent) were added. The mixture was stirred at 60 °C for 16 hr, with monitoring LCMS. The mixture was then diluted with ethyl acetate and washed with saturated aqueous NH₄Cl, and then brine. Next, the mixture was dried over magnesium sulfate, filtered and concentrated in vacuo to yield the title compound, which was carried forward without further purification.

**Tert-butyl 4-amino-2-fluoro-3-((2-methoxyethyl)amino)benzoate (I-1325-2):** A mixture of crude tert-butyl 2-fluoro-3-((2-methoxyethyl)amino)-4-nitrobenzoate (**I-1325-1,** 560 mg, 1.0 equivalent), 10% palladium on carbon (190 mg, 0.10 equivalent) and ethanol (15 mL) was stirred under an atmosphere of hydrogen for 4 hr, with monitoring LCMS. The mixture was filtered, concentrated in vacuo and purified by silica gel flash column chromatography (hexane/EtOAc) to yield the title compound.

**Tert-butyl 4-(2-(4-bromo-2,5-difluorophenyl)acetamido)-2-fluoro-3-((2-methoxyethyl)amino)benzoate (I-1325-3):** A mixture of tert-butyl 4-amino-2-fluoro-3-((2-methoxyethyl)amino)benzoate (**I-1325-2,** 250 mg, 1.00 equivalent), 2-(4-bromo-2,5-difluorophenyl)acetic acid (318 mg, 1.44 equivalent), chloro-N,N,N',N'-tetramethylformamidinium hexafluorophosphate (300 mg, 1.22 equivalent), 1-methylimidazole (360 mg, 5.0 equivalent) and acetonitrile (8 mL) was stirred at 20 °C for 2 hr, with monitoring by LCMS. The mixture was then diluted with EtOAc, and then washed with 1 N HCl. The organic phase was dried over MgSO₄, filtered, and concentrated in vacuo to yield the title compound, which was carried forward without further purification.

**Tert-butyl 2-(4-bromo-2,5-difluorobenzyl)-7-fluoro-1-(2-methoxyethyl)-1H-benzo[d]imidazole-6-carboxylate (I-1325):** A mixture of tert-butyl 4-(2-(4-bromo-2,5-difluorophenyl)acetamido)-2-fluoro-3-((2-methoxyethyl)amino)benzoate (I-1325-3, 331 mg, 1.0 equivalent), glacial acetic acid (0.38 mL) and 1,2-dichloroethane (5 mL) was stirred at 60 °C for 4 hr, or until completion as determined by LCMS. The mixture was diluted with EtOAc, quenched with saturated aqueous NaHCOs. The organic phase was washed with brine, dried over MgSO₄, filtered, and concentrated in vacuo to yield the title compound. 1H NMR (400 MHz, Chloroform-d) δ 7.86 (dd, J = 8.6, 6.7 Hz, 1H), 7.65 (d, J = 8.6 Hz, 1H), 7.35 (dd, J = 8.7, 5.6 Hz, 1H), 7.13 (dd, J = 8.3, 6.4 Hz, 1H), 4.62 - 4.40 (m, 4H), 3.78 (t, J = 5.1 Hz, 2H), 3.29 (s, 3H), 1.64 (s, 9H).

### Preparation of Intermediate I-1326

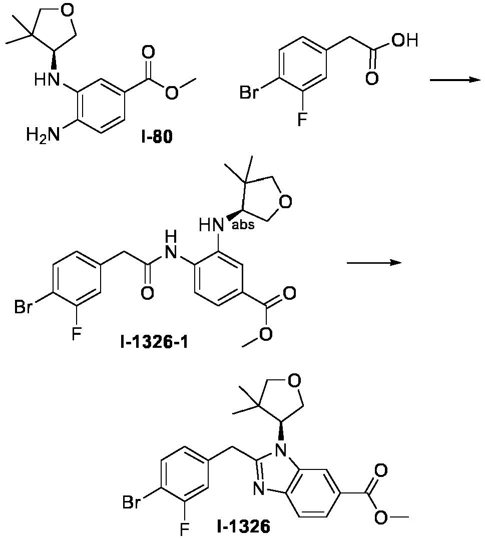

**Methyl (S)-4-(2-(4-bromo-3-fluorophenyl)acetamido)-3-((4,4-dimethyltetrahydrofuran-3-yl)amino)benzoate (I-1326-1):** A mixture of methyl (S)-4-amino-3-((4,4-dimethyltetrahydrofuran-3-yl)amino)benzoate (I-80, 200 mg, 1.0 equivalent), 2-(4-bromo-3-fluorophenyl)acetic acid (83 mg, 1.1 equivalent), chloro-N,N,N',N'-tetramethylformamidinium hexafluorophosphate (265 mg, 1.25 equivalent), 1-methylimidazole (311 mg, 5.0 equivalent) and acetonitrile (3.0 mL) was stirred at 20 °C for 2 hr, with monitoring by LCMS. The mixture was then diluted with EtOAc, then washed with 1 N HCl. The organic phase was dried over MgSO₄, filtered, and concentrated in vacuo to yield the title compound, which was carried forward without further purification.

**Methyl (S)-2-(4-bromo-3-fluorobenzyl)-1-(4,4-dimethyltetrahydrofuran-3-yl)-1H-benzo[d]imidazole-6-carboxylate (I-1326):** A mixture of methyl (S)-4-(2-(4-bromo-3-fluorophenyl)acetamido)-3-((4,4-dimethyltetrahydrofuran-3-yl)amino)benzoate (**I-1326-1,** 250 mg, 1.0 equivalent) and glacial acetic acid (0.2 mL) was stirred at 180 °C for 2 hr, or until completion as determined by LCMS. The mixture was diluted with EtOAc, quenched with saturated aqueous NaHCOs and batchwise addition of solid NaHCOs. The organic phase was washed with brine, dried over MgSO₄, filtered, and concentrated in vacuo to yield the title compound. ES/MS m/z: 462.9 (M+H⁺).

### Preparation of Intermediate I-1327

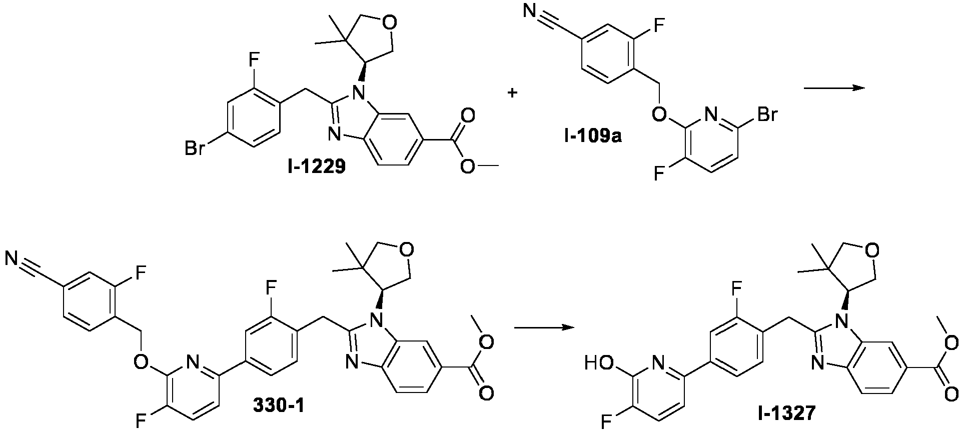

**Methyl (S)-2-(4-(6-((4-cyano-2-fluorobenzyl)oxy)-5-fluoropyridin-2-yl)-2-fluorobenzyl)-1-(4,4-dimethyltetrahydrofuran-3-yl)-1H-benzo[d]imidazole-6-carboxylate (330-1):** A mixture of methyl (S)-2-(4-bromo-2-fluorobenzyl)-1-(4,4-dimethyltetrahydrofuran-3-yl)-1H-benzo[d]imidazole-6-carboxylate (Intermediate **I-1229,** 800 mg, 1.67 mmol, 1.0 equivalent), PdCl₂(dppf) (186 mg, 0.25 mmol, 0.15 equivalent), potassium propionate (560 mg, 5.01 mmol, 3.0 equivalent), and bis(pinacolato)diboron (510 mg, 2.00 mol, 1.2 equivalent) was mixed with 1,4-dioxane (10 mL) and the resulting mixture was purged with argon for 2 min. The mixture was sealed and heated to 120 °C by microwave. The mixture was then stirred for 1 h. After cooling down to room temperature, 4-[(6-bromo-3-fluoro-2-pyridyl)oxymethyl]-3-fluoro-benzonitrile (Intermediate **I-109a,** 580 mg, 1.84 mmol, 1.1 equivalent), PdCl₂(dppf) (62 mg, 0.0834 mmol, 0.05 equivalent), 2 M aqueous Na₂CO₃ (2.0 mL, 4.17 mmol, 2.5 equivalent) were added, respectively. The resulting mixture was heated to 100 °C under argon and stirred for 3 hrs. before cooling to rt. Once cooled the mixture was filtered through a plug of Celite and MgSO₄. The filtrate was concentrated and purified by column chromatography (silica gel, EtOAc/hexane gradient) to yield the title compound. ES/MS m/z: 627.7 (M+H⁺).

**Methyl (S)-1-(4,4-dimethyltetrahydrofuran-3-yl)-2-(2-fluoro-4-(5-fluoro-6-hydroxypyridin-2-yl)benzyl)-1H-benzo[d]imidazole-6-carboxylate (I-1327):** A mixture of methyl (S)-2-(4-(6-((4-cyano-2-fluorobenzyl)oxy)-5-fluoropyridin-2-yl)-2-fluorobenzyl)-1-(4,4-dimethyltetrahydrofuran-3-yl)-1H-benzo[d]imidazole-6-carboxylate (Intermediate **330-1,** 200 mg, 1.0 equivalent) in 4M HCl solution in dioxane (8.0 mL, 10 equivalent) was heated at 80 °C overnight. The mixture was diluted with EtOAc and washed with brine, saturated aqueous NaHCO₃ and water. The organic phase was dried, concentrated in vacuo, and purified by preparative reverse-phase HPLC (CH3CN/water, 0.1%TFA) to yield the title compound. ES/MS m/z: 494.7 (M+H⁺).

### Preparation of Intermediate I-1328

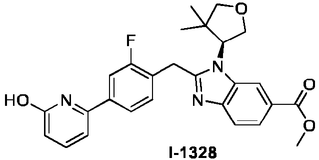

**Methyl (S)-1-(4,4-dimethyltetrahydrofuran-3-yl)-2-(2-fluoro-4-(6-hydroxypyridin-2-yl)benzyl)-1H-benzo[d]imidazole-6-carboxylate (Intermediate I-1328):** Methyl (S)-1-(4,4-dimethyltetrahydrofuran-3-yl)-2-(2-fluoro-4-(6-hydroxypyridin-2-yl)benzyl)-1H-benzo[d]imidazole-6-carboxylate was prepared in a manner similar to Intermediate 1-1327. ES/MS m/z: 476.6 (M+H⁺).

### Preparation of Intermediate I-1330

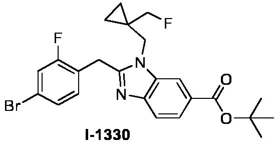

**Tert-butyl 2-(4-bromo-2-fluorobenzyl)-1-((1-(fluoromethyl)cyclopropyl)methyl)-1H-benzo[d]imidazole-6-carboxylate (Intermediate I-1330):** Tert-butyl 2-(4-bromo-2-fluorobenzyl)-1-((1-(fluoromethyl)cyclopropyl)methyl)-1H-benzo[d]imidazole-6-carboxylate was prepared in a manner described for Intermediate **I-2** using Intermediate **I-1196** and 2-(4-bromo-2-fluorophenyl)acetic acid. ES/MS m/z: 509.7 (M+H)⁺.

### Preparation of Intermediate I-1332

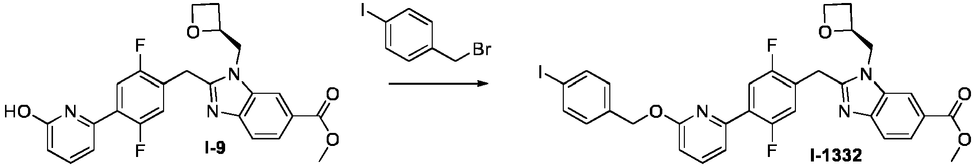

**Methyl (S)-2-(2,5-difluoro-4-(6-((4-iodobenzyl)oxy)pyridin-2-yl)benzyl)-1-(oxetan-2-ylmethyl)-1H-benzo[d]imidazole-6-carboxylate (I-1332):** A mixture of methyl (S)-2-(2,5-difluoro-4-(6-hydroxypyridin-2-yl)benzyl)-1-(oxetan-2-ylmethyl)-1H-benzo[d]imidazole-6-carboxylate (I-9,157 mg, 1.0 equivalent), 1-(bromomethyl)-4-iodobenzene (100 mg, 1.0 equivalent), cesium carbonate (150 mg, 1.4 equivalent) and acetonitrile (3.0 mL) was stirred at 50 °C for 1.5 hr or until reaction completion as determined by LCMS. The mixture was then filtered and concentrated in vacuo. Silica gel flash column chromatography (EtOAc/hexane) yielded the title compound. ES/MS m/z: 683.2 (M+H⁺).

### Preparation of Intermediate I-1333:

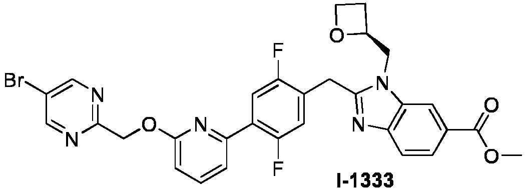

**Methyl (S)-2-(4-(6-((5-bromopyrimidin-2-yl)methoxy)pyridin-2-yl)-2,5-difluorobenzyl)-1-(oxetan-2-ylmethyl)-1H-benzo[d]imidazole-6-carboxylate (I-1333):** Methyl (S)-2-(4-(6-((5-bromopyrimidin-2-yl)methoxy)pyridin-2-yl)-2,5-difluorobenzyl)-1-(oxetan-2-ylmethyl)-1H-benzo[d]imidazole-6-carboxylate was prepared in a manner described for preparation of Intermediate **I-1332,** using Intermediate **I-9.** ES/MS m/z: 638 (M+H+).

### Preparation of Intermediate I-1334:

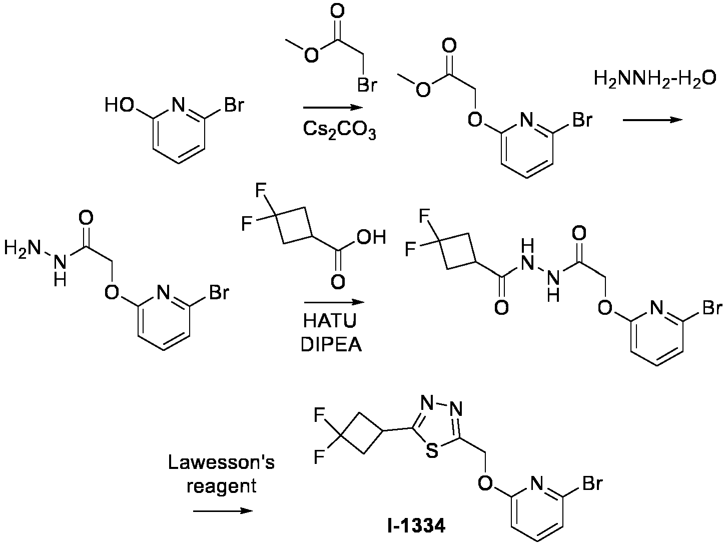

Methyl **2-[(6-bromo-2-pyridyl)oxy]acetate:** A mixture of 6-bromopyridin-2-ol (1000 mg, 1.0 equivalent), methyl 2-bromoacetate (0.65 mL, 1.2 equivalent), cesium carbonate (3.75g, 2.0 equivalent), and acetonitrile (10 mL) was stirred at 70 °C until completion as determined by LCMS. Silica gel flash chromatography yielded the title compound. ES/MS m/z: 246.3 (M+H⁺).

**2-[(6-bromo-2-pyridyl)oxy]acetohydrazide:** A mixture of methyl 2-[(6-bromo-2-pyridyl)oxy]acetate (100 mg, 1.0 equivalent), hydrazine hydrate (100 µL, 5.0 equivalent), and ethanol (2 mL) was stirred at 50 °C overnight. The mixture was partitioned between ethyl acetate and water. The organic phase was dried, filtered and concentrated in vacuo to yield the title compound. ES/MS m/z: 247.9 (M+H⁺).

**N'-[2-[(6-bromo-2-pyridyl)oxy]acetyl]-3,3-difluoro-azetidine-1-carbohydrazide:** A mixture of 3,3-difluorocyclobutanecarboxylic acid (72 mg, 1.3 equivalent), 2-[(6-bromo-2-pyridyl)oxy]acetohydrazide (100 mg, 1.0 equivalent), HATU (189 mg, 1.3 equivalent), diisopropylethylamine (0.21 mL, 3.0 equivalent), and DMF (2 mL) was stirred at 20 °C until completion as determined by LCMS. The aqueous layer was worked up and then purified by silica gel flash column chromatography which yielded the title compound. ES/MS m/z: 364.0 (M⁺).

**2-[(6-bromo-2-pyridyl)oxymethyl]-5-(3,3-difluorocyclobutyl)-1,3,4-thiadiazole (I-1334):** A mixture of N'-[2-[(6-bromo-2-pyridyl)oxy]acetyl]-3,3-difluoro-azetidine-1-carbohydrazide (60 mg, 1.0 equivalent), Lawesson's reagent (80 mg, 1.2 equivalent), and THF was stirred at 70 °C until completion as determined by LCMS. The resulting mixture was purified by silica gel flash column chromatography yielded the title compound. ES/MS m/z: 364.0 (M+H⁺).

### Preparation of Intermediate I-1335

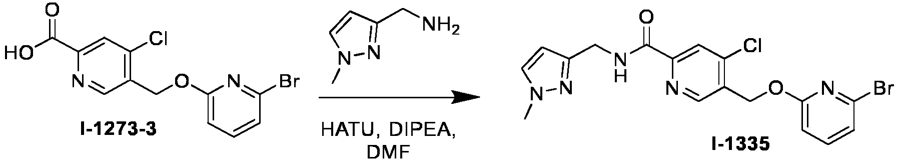

**5-(((6-bromopyridin-2-yl)oxy)methyl)-4-chloro-N-((1-methyl-1H-pyrazol-3-yl)methyl)picolinamide (I-1335):** A mixture of Intermediate 1-1273-3 (from Intermediate I-1273, 50 mg, 1.0 equivalent), (1-methylpyrazol-3-yl)methanamine (24 mg, 1.5 equivalent), HATU (83 mg, 1.5 equivalent), diisopropylethylamine (60 µL, 2.5 equivalent) in DMF (2 mL) was stirred at room temperature for 2 hr. The mixture was diluted with EtOAc, washed with 10% LiCl. Next, a mixture of saturated aqueous NaHCO₃ and brine was added. The mixture was then dried over MgSO₄, filtered, and concentrated in vacuo. Silica gel flash column chromatography (EtOAc/hexane) yielded the title compound. ES/MS m/z: 435.9 (M+H⁺).

### Preparation of Intermediate I-1336

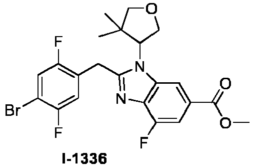

**Methyl 2-(4-bromo-2,5-difluorobenzyl)-1-(4,4-dimethyltetrahydrofuran-3-yl)-4-fluoro-1H-benzo[d]imidazole-6-carboxylate (I-1336):** Methyl 2-(4-bromo-2,5-difluorobenzyl)-1-(4,4-dimethyltetrahydrofuran-3-yl)-4-fluoro-1H-benzo[d]imidazole-6-carboxylate (I-1336) was prepared in a manner described for **I-105** substituting 4,4-dimethyltetrahydrofuran-3-amine hydrochloride for (S)-4,4-dimethyltetrahydrofuran-3-amine hydrochloride. ES/MS: 338.5 (M+Na+).

### Preparation of Intermediate I-1337

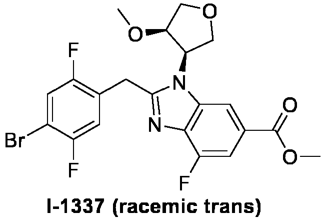

**Racemic methyl 2-(4-bromo-2,5-difluorobenzyl)-4-fluoro-1-((3R,4R)-4-methoxytetrahydrofuran-3-yl)-1H-benzo[d]imidazole-6-carboxylate (Intermediate I-1337):** Racemic methyl 2-(4-bromo-2,5-difluorobenzyl)-4-fluoro-1-((3R,4R)-4-methoxytetrahydrofuran-3-yl)-1H-benzo[d]imidazole-6-carboxylate was prepared in a manner described for preparation of Intermediate **I-1326,** using Intermediate **I-1133.** ES/MS: 499.0 (M+H⁺).

### Preparation of Intermediate I-1339

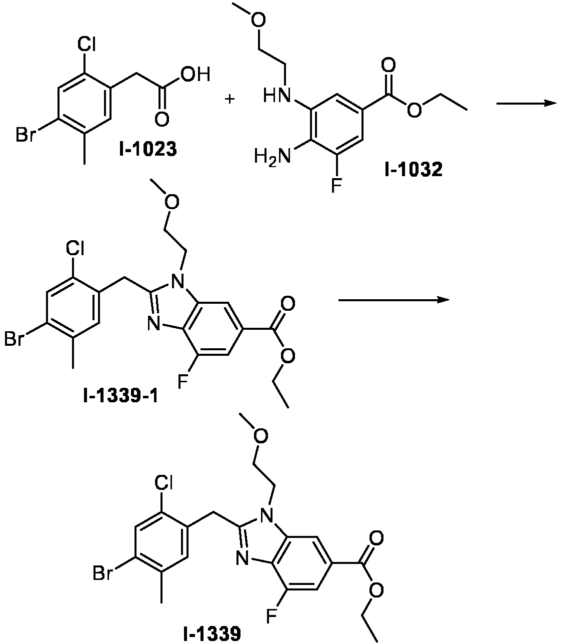

**Ethyl 2-(4-bromo-2-chloro-5-methylbenzyl)-4-fluoro-1-(2-methoxyethyl)-1H-benzo[d]imidazole-6-carboxylate (I-1339-1):** A mixture of ethyl 4-amino-3-fluoro-5-(2-methoxyethylamino)benzoate (**I-1032,** 73 mg, 1.0 equivalent), 2-(4-bromo-2-chloro-5-methylphenyl)acetic acid (**I-1023,** 90 mg, 1.2 equivalent), chloro-N,N,N',N'-tetramethylformamidinium hexafluorophosphate (100 mg, 1.25 equivalent), 1-methylimidazole (70 mg, 3.0 equivalent) and DMF (5.0 mL) was stirred at 20 °C for 5 hr, with monitoring by LCMS. The mixture was then diluted with EtOAc. Next the mixture was washed with water, followed by brine. The organic phase was dried over MgSO₄, filtered, and concentrated in vacuo to yield the title compound, which was carried forward without further purification.

**Ethyl 2-[(4-bromo-2-chloro-5-methyl-phenyl)methyl]-7-fluoro-3-(2-methoxyethyl)benzimidazole-5-carboxylate (I-1339):** A mixture of crude ethyl 2-(4-bromo-2-chloro-5-methylbenzyl)-4-fluoro-1-(2-methoxyethyl)-1H-benzo[d]imidazole-6-carboxylate **(I-1339-1,** 143 mg, 1.0 equivalent) and glacial acetic acid (5.0 mL) was stirred at reflux for 20 hr, or until completion as determined by LCMS. The mixture was concentrated in vacuo and purified by silica gel flash column chromatography (EtOAc/hexane) to yield the title compound. ES/MS m/z: 483.3 (M+H⁺).

### Preparation of Intermediate I-1343:

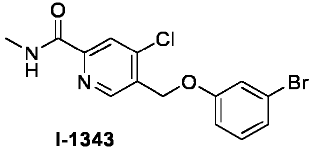

**5-[(3-bromophenoxy)methyl]-4-chloro-N-methyl-pyridine-2-carboxamide (I-1343):** 5-[(3-bromophenoxy)methyl]-4-chloro-N-methyl-pyridine-2-carboxamide was prepared in a manner as described for Intermediate **I-1273** substituting 3-bromophenol for 6-bromopyridin-2-ol in step 2. ES/MS: 355, 357 (M+H⁺).

### Preparation of Intermediate I-1344:

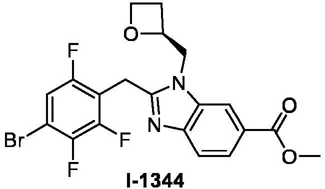

**Methyl 4-[[2-[4-[6-[(4-cyano-2-fluoro-phenyl)methoxy]-2-pyridyl] -2-fluorophenyl]acetyl]amino]-3-(2-methoxyethylamino)benzoate (I-1344):** The title compound was prepared in a manner as described for Intermediate **I-2,** using Intermediate **I-4** and 2-(4-bromo-2,3,6-trifluoro-phenyl)acetic acid. ES/MS m/z: 469.0 (M+H⁺).

### Preparation of Intermediate I-1345:

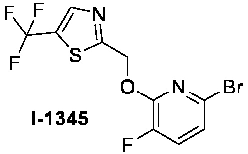

**2-(((6-bromo-3-fluoropyridin-2-yl)oxy)methyl)-5-(trifluoromethyl)thiazole:** 2-(((6-bromo-3-fluoropyridin-2-yl)oxy)methyl)-5-(trifluoromethyl)thiazole was prepared in a manner as described for **Intermediate I-1034** substituting 2-(bromomethyl)-5-(trifluoromethyl)thiazole for 6-(bromomethyl)-1-methyl-benzotriazole. ES/MS m/z: 356.6 (M+H⁺).

### Preparation of Intermediate I-1388:

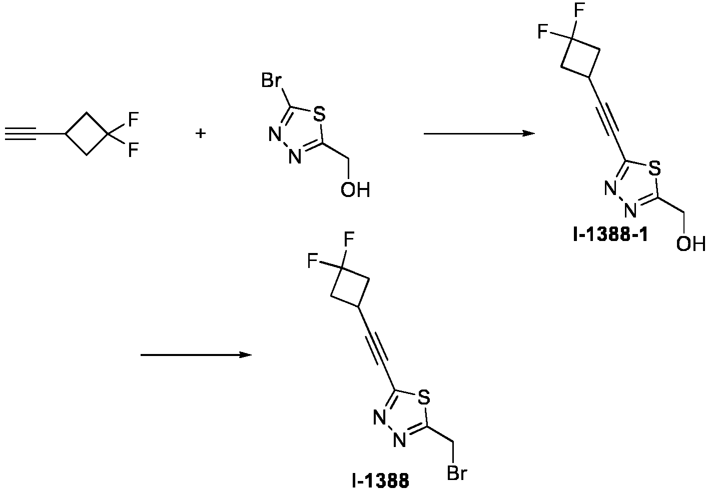

**[5- [2-(3,3-difluorocyclobutyl)ethynyl] -1,3,4-thiadiazol-2-yl] methanol (I-1388-1):** A mixture of 3-ethynyl-1,1-difluoro-cyclobutane (74.4 mg, 5.0 equivalent), (5-bromo-1,3,4-thiadiazol-2-yl)methanol (25 mg, 1.0 equivalent), bis(triphenylphosphine)palladium chloride (45 mg, 0.50 equivalent), copper(I) iodide (12.2 mg, 0.50 equivalent), diisopropylamine (0.37 mL, 20 equivalent), and THF (1.6 mL) was degassed by bubbling through argon, then heated at 105 °C for 3 hr. The mixture was diluted with water, extracted with EtOAc, and the resulting organic phase was dried over MgSO4, filtered, and concentrated in vacuo. Silica gel flash column chromatography (EtOAc/hexane) yielded the title compound. ES/MS m/z: 231.2 (M+H⁺).

**2-(bromomethyl)-5-[2-(3,3-difluorocyclobutyl)ethynyl]-1,3,4-thiadiazole (I-1388):** A mixture of [5-[2-(3,3-difluorocyclobutyl)ethynyl]-1,3,4-thiadiazol-2-yl]methanol (8.2 mg, 1.0 equivalent), triphenylphosphine (11.2 mg, 1.2 equivalent), carbon tetrabromide (14.2 mg, 1.2 equivalent) and DCM (5 mL) was stirred at 20 C for 20 hr. The mixture was concentrated and purified by silica gel flash column chromatography (EtOAc/hexane) to yield the title compound. ES/MS m/z: 293.1 (M+H⁺).

### Preparation of Intermediate I-1389

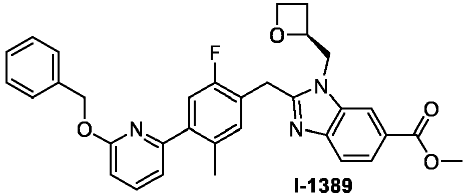

**Methyl (S)-2-(4-(6-(benzyloxy)pyridin-2-yl)-2-fluoro-5-methylbenzyl)-1-(oxetan-2-ylmethyl)-1H-benzo[d]imidazole-6-carboxylate (I-1389):** methyl (S)-2-(4-(6-(benzyloxy)pyridin-2-yl)-2-fluoro-5-methylbenzyl)-1-(oxetan-2-ylmethyl)-1H-benzo[d]imidazole-6-carboxylate was prepared in a manner similar to Intermediate **I-7,** using Intermediate **I-1276** and 2-(benzyloxy)-6-bromopyridine. ES/MS m/z: 552.2 (M+H⁺).

### Preparation of Intermediate I-1390

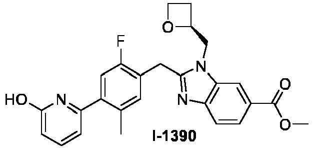

**Methyl (S)-2-(2-fluoro-4-(6-hydroxypyridin-2-yl)-5-methylbenzyl)-1-(oxetan-2-ylmethyl)-1H-benzo[d]imidazole-6-carboxylate (I-1390):** A mixture of methyl 2-[[4-(6-benzyloxy-2-pyridyl)-2-fluoro-5-methyl-phenyl]methyl]-3-[[(2S)-oxetan-2-yl]methyl]benzimidazole-5-carboxylate (Intermediate **I-1389,** 150 mg, 1.0 equivalent), 10% Pd on carbon (49.8 mg, 0.20 equivalent), ethyl acetate and ethanol was stirred under an atmosphere of hydrogen at 20 °C for 10 hr. The mixture was filtered and concentrated to yield the title compound. ES/MS m/z: 462.2 (M+H⁺).

### Preparation of Intermediate I-1391

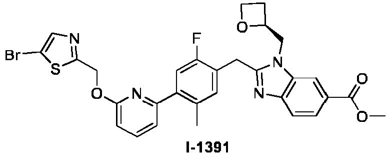

**Methyl 2-[[4-[6-[(5-bromothiazol-2-yl)methoxy]-2-pyridyl]-2-fluoro-5-methylphenyl]methyl]-3-[[(2S)-oxetan-2-yl]methyl]benzimidazole-5-carboxylate (I-1391):** A mixture of methyl (S)-2-(2-fluoro-4-(6-hydroxypyridin-2-yl)-5-methylbenzyl)-1-(oxetan-2-ylmethyl)-1H-benzo[d]imidazole-6-carboxylate (I-1390, 34 mg, 1.0 equivalent), 5-bromo-2-(bromomethyl)thiazole (19.9 mg, 1.05 equivalent), cesium carbonate (38 mg, 1.6 equivalent), and toluene was stirred at 50 C for 40 min. The mixture was filtered, concentrated and purified by silica gel flash column chromatography to yield the title compound. ES/MS m/z: 638.0 (M+H⁺).

### Preparation of Intermediate I-1392

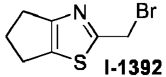

**2-(bromomethyl)-5,6-dihydro-4H-cyclopenta[d]thiazole (I-1392):** A mixture of 5,6-dihydro-4H-cyclopenta[d]thiazol-2-ylmethanol (112 mg, 1.0 equivalent), triphenylphosphine (199 mg, 1.05 equivalent), carbon tetrabromide (251 mg, 1.05 equivalent) and DCM (8 mL) was stirred at RT for 1 hr. The mixture was concentrated and purified by silica gel flash column chromatography to yield the title compound. ES/MS m/z: 218.2 (M+H⁺).

### Preparation of Intermediate I-1393

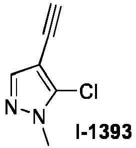

**5-chloro-4-ethynyl-1-methyl-pyrazole (I-1393):** To a mixture of 5-chloro-1-methylpyrazole-4-carbaldehyde (145 mg, 1.0 equivalent) and dimethyl (1-diazo-2-oxopropyl)phosphonate (Ohira-Bestmann reagent, 480 mg, 2.5 equivalent), and MeOH (20 mL) precooled to 0 °C was added cesium carbonate (977 mg, 3.0 equivalent). The mixture was allowed to warm to RT and stirred overnight. The mixture was concentrated and purified by silica gel flash column chromatography (EtOAc/hexane) to yield the title compound.

### Preparation of Intermediate I-1394

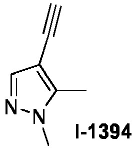

**Asdf4-ethynyl-1,5-dimethyl-pyrazole (I-1394):** Asdf4-ethynyl-1,5-dimethyl-pyrazole (I-1394) was prepared in a manner as described for Intermediate **I-1393,** using 1,5-dimethylpyrazole-4-carbaldehyde

### B. COMPOUND EXAMPLES

### Example 1: 2-[[2,5-difluoro-4-[6-[(5-methoxy-1,3,4-thiadiazol-2-yl)methoxy]-2-pyridyl]phenyl]methyl]-3-[[(2S)-oxetan-2-yl]methyl]benzimidazole-5-carboxylic acid

### Procedure 1:

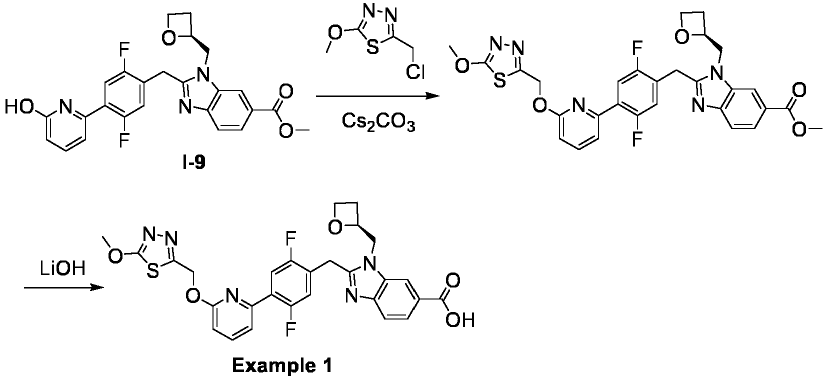

**Methyl 2- [[2,5-difluoro-4- [6- [(5-methoxy-1,3,4-thiadiazol-2-yl)methoxy] -2-pyridyl]phenyl]methyl]-3-[[(2S)-oxetan-2-yl]methyl]benzimidazole-5-carboxylate:** To a solution of methyl 2-[[2,5-difluoro-4-(6-hydroxy-2-pyridyl)phenyl]methyl]-3-[[(2S)-oxetan-2-yl]methyl]benzimidazole-5-carboxylate (60.0 mg, 0.13 mmol) and 2-(chloromethyl)-5-methoxy-1,3,4-thiadiazole (23.5 mg, 0.14 mmol) in 11 mL of acetonitrile was added Cs₂CO₃ (67 mg, 0.21 mmol). The solution was then heated to 50 °C for 30 minutes. Upon completion of the time, the solution was cooled to rt, filtered, then concentrated. The crude material was purified by normal phase chromatography 1-12 % DCM/MeOH. The product containing fractions were combined and concentrated to give the titled product. ES/MS m/z: 594.0 (M+H⁺).

**2-[[2,5-difluoro-4-[6-[(5-methoxy-1,3,4-thiadiazol-2-yl)methoxy]-2-pyridyl]phenyl]methyl]-3-[[(2S)-oxetan-2-yl]methyl]benzimidazole-5-carboxylic acid (Example 1):** To a solution of methyl 2-[[2,5-difluoro-4-[6-[(5-methoxy-1,3,4-thiadiazol-2-yl)methoxy]-2-pyridyl]phenyl]methyl]-3-[[(2S)-oxetan-2-yl]methyl]benzimidazole-5-carboxylate (36.0 mg, 0.06 mmol) in 3 mL of acetonitrile, 1 mL of water and aqueous LiOH (0.04 mL, 0.09 mmol, 2 M) were added. The solution was then stirred at rt overnight. The following day, a drop of TFA was added, then concentrated into 1 mL of DMF. The crude material was purified by reverse phase chromatography 10-58 % ACN/water with 0.1 % TFA added. The product containing fractions were combined, diluted with EtOAc, and 1 mg of sodium bicarbonate was added per mL of HPLC solvent. The aqueous layer was separated and the organic washed twice with water, then brine. The organic layer was dried over MgSO₄, filtered, and concentrated to give **Example 1.** ES/MS m/z: 580.2 (M+H⁺); ¹H NMR (400 MHz, DMSO) δ 12.78 (s, 1H), 8.26 (d, J = 1.6 Hz, 1H), 8.00 - 7.87 (m, 2H), 7.79 (dd, J = 8.4, 1.6 Hz, 1H), 7.60 (t, J = 8.2 Hz, 2H), 7.40 (dd, J = 11.6, 6.0 Hz, 1H), 6.98 (d, J = 8.2 Hz, 1H), 5.74 (s, 2H), 5.13 - 5.03 (m, 1H), 4.76 (dd, J = 15.6, 7.0 Hz, 1H), 4.63 (dd, J = 15.5, 2.8 Hz, 1H), 4.57 - 4.42 (m, 3H), 4.36 (dt, J = 9.0, 5.9 Hz, 1H), 4.10 (s, 3H), 2.72 (dq, J = 11.2, 7.7 Hz, 1H), 2.46 - 2.31 (m, 1H).

### Example 2: (S)-2-(4-(6-((5-cyanothiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyanothiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.42 (s, 1H), 8.33 - 8.27 (m, 1H), 7.98 (dd, J = 8.5, 1.5 Hz, 1H), 7.92 - 7.78 (m, 2H), 7.67 (d, J = 8.5 Hz, 1H), 7.64 - 7.58 (m, 1H), 7.20 (dd, J = 11.5, 6.0 Hz, 1H), 7.03 - 6.93 (m, 1H), 5.20 (qd, J = 7.1, 2.6 Hz, 1H), 4.73 (dd, J = 15.7, 6.9 Hz, 1H), 4.68 - 4.39 (m, 6H), 2.83 - 2.66 (m, 1H), 2.61 - 2.38 (m, 1H).

### Example 3: (S)-2-(4-(6-((5-carbamoylthiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-carbamoylthiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. ¹H NMR (400 MHz, Methanol-d4) δ 8.30 (d, J = 3.0 Hz, 2H), 7.98 (dd, J = 8.5, 1.6 Hz, 1H), 7.85 (dd, J = 9.6, 6.0 Hz, 2H), 7.66 (d, J = 8.5 Hz, 1H), 7.63 - 7.54 (m, 1H), 7.18 (dd, J = 11.6, 5.9 Hz, 1H), 6.97 (d, J = 8.1 Hz, 1H), 5.79 (s, 2H), 5.19 (d, J = 6.2 Hz, 1H), 4.80 - 4.35 (m, 7H), 2.88 - 2.69 (m, 1H), 2.50 (q, J = 9.8, 8.6 Hz, 1H).

### Example 4: (S)-2-(4-(6-((4-cyanothiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-cyanothiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.47 (s, 1H), 8.30 (d, J = 1.5 Hz, 1H), 7.98 (dd, J = 8.5, 1.5 Hz, 1H), 7.91 - 7.75 (m, 2H), 7.66 (d, J = 8.5 Hz, 1H), 7.61 (dd, J = 7.4, 1.5 Hz, 1H), 7.19 (dd, J = 11.5, 6.0 Hz, 1H), 6.96 (d, J = 8.2 Hz, 1H), 5.81 (s, 2H), 5.20 (qd, J = 7.0, 2.6 Hz, 1H), 4.73 (dd, J = 15.7, 7.0 Hz, 1H), 4.68 - 4.39 (m, 6H), 2.90 - 2.71 (m, 1H), 2.50 (ddt, J = 11.4, 9.2, 7.2 Hz, 1H).

### Example 5: (S)-2-(4-(6-((4-carbamoylthiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-carbamoylthiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.30 (d, J = 1.5 Hz, 1H), 8.21 (s, 1H), 7.98 (dd, J = 8.5, 1.5 Hz, 1H), 7.92 - 7.75 (m, 2H), 7.66 (d, J = 8.5 Hz, 1H), 7.61 (dd, J = 7.4, 1.5 Hz, 1H), 7.19 (dd, J = 11.6, 6.0 Hz, 1H), 6.95 (d, J = 8.2 Hz, 1H), 5.81 (s, 2H), 5.19 (qd, J = 7.0, 2.6 Hz, 1H), 4.72 (dd, J = 15.7, 7.0 Hz, 1H), 4.68 - 4.38 (m, 6H), 2.88 - 2.71 (m, 1H), 2.49 (ddt, J = 11.4, 9.1, 7.2 Hz, 1H).

### Example 6: (S)-2-(4-(6-((1-cyclopropyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1-cyclopropyl-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. ¹H NMR (400 MHz, Methanol-d4) δ 8.30 (d, J = 1.4 Hz, 1H), 7.98 (dd, J = 8.5, 1.5 Hz, 1H), 7.94 (dd, J = 10.8, 6.4 Hz, 1H), 7.76 (t, J = 7.9 Hz, 1H), 7.66 (d, J = 8.5 Hz, 1H), 7.62 (d, J = 2.3 Hz, 1H), 7.52 (dd, J = 7.4, 1.6 Hz, 1H), 7.18 (dd, J = 11.5, 6.0 Hz, 1H), 6.82 (d, J = 8.2 Hz, 1H), 6.33 (d, J = 2.4 Hz, 1H), 5.43 (s, 2H), 5.19 (tt, J = 7.0, 3.5 Hz, 1H), 4.78 - 4.29 (m, 7H), 3.66 - 3.52 (m, 1H), 2.95 - 2.71 (m, 1H), 2.61 - 2.39 (m, 1H), 1.17 - 0.93 (m, 4H).

### Example 7: (S)-2-(4-(6-((6,7-dihydro-4H-pyrazolo[5,1-c][1,4]oxazin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6,7-dihydro-4H-pyrazolo[5,1-c][1,4]oxazin-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.31 (d, J = 1.4 Hz, 1H), 7.99 (dd, J = 8.5, 1.5 Hz, 1H), 7.93 (dd, J = 10.8, 6.4 Hz, 1H), 7.76 (t, J = 7.9 Hz, 1H), 7.67 (d, J = 8.5 Hz, 1H), 7.53 (dd, J = 7.5, 1.6 Hz, 1H), 7.18 (dd, J = 11.5, 6.1 Hz, 1H), 6.83 (d, J = 8.2 Hz, 1H), 6.17 (s, 1H), 5.45 (s, 2H), 5.20 (dd, J = 7.4, 2.5 Hz, 1H), 4.82 (s, 2H), 4.78 - 4.39 (m, 6H), 4.17 - 4.02 (m, 4H), 2.80 (dtd, J = 11.5, 8.1, 6.1 Hz, 1H), 2.50 (ddt, J = 11.4, 9.1, 7.1 Hz, 1H).

### Example 8: (S)-2-(4-(6-((2-carbamoylthiazol-4-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-carbamoylthiazol-4-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.32 (s, 1H), 7.99 (dd, J = 8.5, 1.5 Hz, 1H), 7.88 (dd, J = 10.7, 6.4 Hz, 1H), 7.84 - 7.75 (m, 2H), 7.68 (d, J = 8.5 Hz, 1H), 7.60 - 7.52 (m, 1H), 7.19 (dd, J = 11.5, 6.1 Hz, 1H), 6.91 (d, J = 8.2 Hz, 1H), 5.64 (d, J = 0.9 Hz, 2H), 5.20 (tt, J = 7.1, 3.7 Hz, 1H), 4.80 - 4.37 (m, 7H), 2.91 - 2.71 (m, 1H), 2.59 - 2.39 (m, 1H).

### Example 9: (S)-2-(4-(6-((1-(difluoromethyl)-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1-(difluoromethyl)-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.34 (d, J = 1.4 Hz, 1H), 8.07 - 7.98 (m, 2H), 7.94 (dd, J = 10.8, 6.4 Hz, 1H), 7.78 (t, J = 7.9 Hz, 1H), 7.68 (d, J = 8.5 Hz, 1H), 7.55 (dd, J = 7.4, 1.6 Hz, 1H), 7.47 (t, J = 59.8 Hz, 1H), 7.20 (dd, J = 11.5, 6.0 Hz, 1H), 6.87 (d, J = 8.2 Hz, 1H), 6.59 (d, J = 2.7 Hz, 1H), 5.52 (s, 2H), 5.20 (qd, J = 7.1, 2.5 Hz, 1H), 4.80 - 4.30 (m, 7H), 2.81 (dtd, J = 11.4, 8.2, 6.1 Hz, 1H), 2.50 (ddt, J = 11.5, 9.2, 7.2 Hz, 1H).

### Example 10: (S)-2-(2,5-difluoro-4-(6-((1-(oxetan-3-yl)-1H-pyrazol-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((1-(oxetan-3-yl)-1H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, Methanol-d4) δ 8.31 (d, J = 1.4 Hz, 1H), 8.01 - 7.90 (m, 2H), 7.81 - 7.69 (m, 2H), 7.67 (d, J = 8.4 Hz, 1H), 7.53 (dd, J = 7.4, 1.5 Hz, 1H), 7.18 (dd, J = 11.5, 6.0 Hz, 1H), 6.84 (d, J = 8.2 Hz, 1H), 6.42 (d, J = 2.3 Hz, 1H), 5.63 - 5.53 (m, 1H), 5.51 (s, 2H), 5.19 (dt, J = 7.1, 3.5 Hz, 1H), 5.11 - 4.98 (m, 5H), 4.78 - 4.30 (m, 7H), 2.80 (ddd, J = 10.4, 5.6, 2.6 Hz, 1H), 2.50 (ddt, J = 9.2, 7.4, 1.9 Hz, 1H).

### Example 11: (S)-2-(2,5-difluoro-4-(6-(thiazol-2-ylmethoxy)pyridin-2-yl)benzyl)-1-(oxetan-2-ylmethyl)-1H-benzo[d]imidazole-6-carboxylic acid

(S)-2-(2,5-difluoro-4-(6-(thiazol-2-ylmethoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, Methanol-d4) δ 8.31 (d, J = 1.4 Hz, 1H), 7.99 (dd, J = 8.5, 1.5 Hz, 1H), 7.92 - 7.78 (m, 4H), 7.67 (d, J = 8.5 Hz, 1H), 7.62 - 7.55 (m, 2H), 7.19 (dd, J = 11.5, 6.0 Hz, 1H), 6.93 (d, J = 8.2 Hz, 1H), 5.81 (s, 2H), 5.19 (qd, J = 7.1, 2.5 Hz, 1H), 4.79 - 4.37 (m, 8H), 2.87 - 2.67 (m, 1H), 2.49 (dq, J = 11.2, 7.6 Hz, 1H).

### Example 12: (S)-2-(2,5-difluoro-4-(6-((1-(trifluoromethyl)-1H-pyrazol-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((1-(trifluoromethyl)-1H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, Methanol-d4) δ 8.31 (d, J = 1.5 Hz, 1H), 8.15 (d, J = 2.8 Hz, 1H), 7.99 (dd, J = 8.5, 1.5 Hz, 1H), 7.93 (dd, J = 10.8, 6.4 Hz, 1H), 7.79 (t, J = 7.9 Hz, 1H), 7.67 (d, J = 8.5 Hz, 1H), 7.56 (dd, J = 7.4, 1.6 Hz, 1H), 7.18 (dd, J = 11.5, 6.0 Hz, 1H), 6.88 (d, J = 8.2 Hz, 1H), 6.64 (d, J = 2.8 Hz, 1H), 5.54 (s, 2H), 5.19 (tt, J = 7.1, 3.5 Hz, 1H), 4.77 - 4.32 (m, 7H), 2.80 (dtd, J = 11.5, 8.2, 6.1 Hz, 1H), 2.50 (ddt, J = 11.5, 9.1, 7.2 Hz, 1H).

### Example 13: (S)-2-(2,5-difluoro-4-(6-((5-(trifluoromethyl)isothiazol-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-(trifluoromethyl)isothiazol-3-yl)methoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, Methanol-d4) δ 8.31 (d, J = 1.4 Hz, 1H), 7.99 (dd, J = 8.5, 1.6 Hz, 1H), 7.87 (d, J = 1.1 Hz, 1H), 7.82 (t, J = 7.9 Hz, 1H), 7.76 (dd, J = 10.8, 6.4 Hz, 1H), 7.67 (d, J = 8.5 Hz, 1H), 7.57 (dd, J = 7.4, 1.5 Hz, 1H), 7.17 (dd, J = 11.5, 6.0 Hz, 1H), 6.95 (d, J = 8.2 Hz, 1H), 5.64 (s, 2H), 5.20 (qd, J = 7.1, 2.6 Hz, 1H), 4.73 (dd, J = 15.7, 7.0 Hz, 1H), 4.68 - 4.40 (m, 6H), 2.80 (dtd, J = 11.4, 8.1, 6.1 Hz, 1H), 2.59 - 2.43 (m, 1H).

### Example 14: (S)-2-(4-(6-((5-cyanofuran-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyanofuran-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. 1H NMR (400 MHz, DMSO) δ 8.25 (s, 1H), 7.96 - 7.85 (m, 2H), 7.79 (dd, J = 8.5, 1.5 Hz, 1H), 7.66 - 7.49 (m, 3H), 7.40 (dd, J = 11.5, 6.0 Hz, 1H), 6.96 (d, J = 8.2 Hz, 1H), 6.86 (d, J = 3.6 Hz, 1H), 5.54 (s, 2H), 5.08 (d, J = 6.5 Hz, 1H), 4.76 (dd, J = 15.6, 7.1 Hz, 1H), 4.70 - 4.41 (m, 4H), 4.36 (dt, J = 9.0, 5.9 Hz, 1H), 2.82 - 2.64 (m, 1H), 2.46 - 2.29 (m, 1H).

### Example 15: (S)-2-(4-(6-((5-(difluoromethyl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(difluoromethyl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, DMSO) δ 12.77 (s, 1H), 8.26 (d, J = 1.5 Hz, 1H), 7.95 (t, J = 7.9 Hz, 1H), 7.88 (dd, J = 10.5, 6.5 Hz, 1H), 7.79 (dd, J = 8.4, 1.5 Hz, 1H), 7.66 - 7.54 (m, 3H), 7.46 - 7.35 (m, 1H), 7.04 (d, J = 8.2 Hz, 1H), 6.02 (s, 2H), 5.07 (qd, J = 7.0, 2.7 Hz, 1H), 4.76 (dd, J = 15.6, 7.0 Hz, 1H), 4.62 (dd, J = 15.6, 2.8 Hz, 1H), 4.58 - 4.40 (m, 3H), 4.35 (dt, J = 9.0, 5.9 Hz, 1H), 2.73 (ddd, J = 11.4, 8.3, 6.3 Hz, 1H), 2.39 (ddt, J = 11.2, 9.1, 7.0 Hz, 1H).

### Example 16: (S)-2-(2,5-difluoro-4-(6-((3-(methoxymethyl)-1,2,4-oxadiazol-5-yl)methoxy)pyridin-2-yl)benzyl)-1-(oxetan-2-ylmethyl)-1H-benzo [d] imidazole-6-carboxylic acid

(S)-2-(2, 5-difluoro-4-(6-((3-(methoxymethyl)-1,2,4-oxadiazol-5-yl)methoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, DMSO) δ 12.80 (s, 1H), 8.25 (d, J = 1.6 Hz, 1H), 7.98 - 7.90 (m, 1H), 7.79 (dd, J = 8.4, 1.6 Hz, 1H), 7.66 - 7.54 (m, 3H), 7.37 (dd, J = 11.6, 6.1 Hz, 1H), 7.07 (d, J = 8.2 Hz, 1H), 5.78 (s, 2H), 5.07 (qd, J = 6.9, 2.6 Hz, 1H), 4.75 (dd, J = 15.6, 7.1 Hz, 1H), 4.62 (dd, J = 15.6, 2.8 Hz, 1H), 4.52 (d, J = 17.1 Hz, 4H), 4.43 (d, J = 16.8 Hz, 1H), 4.35 (dt, J = 9.0, 5.9 Hz, 1H), 3.27 (s, 3H), 2.78 - 2.65 (m, 1H), 2.39 (ddt, J = 11.3, 9.0, 7.0 Hz, 1H).

### Example 17: (S)-2-(2,5-difluoro-4-(6-((5-methyl-1,2,4-oxadiazol-3-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-methyl-1,2,4-oxadiazol-3-yl)methoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, DMSO) δ 12.78 (s, 1H), 8.26 (d, J = 1.6 Hz, 1H), 8.00 - 7.87 (m, 2H), 7.79 (dd, J = 8.4, 1.6 Hz, 1H), 7.60 (t, J = 8.2 Hz, 2H), 7.40 (dd, J = 11.6, 6.0 Hz, 1H), 6.98 (d, J = 8.2 Hz, 1H), 5.74 (s, 2H), 5.13 - 5.03 (m, 1H), 4.76 (dd, J = 15.6, 7.0 Hz, 1H), 4.63 (dd, J = 15.5, 2.8 Hz, 1H), 4.57 - 4.42 (m, 3H), 4.36 (dt, J = 9.0, 5.9 Hz, 1H), 4.10 (s, 3H), 2.72 (dq, J = 11.2, 7.7 Hz, 1H), 2.46 - 2.31 (m, 1H).

### Example 18: (S)-2-(2,5-difluoro-4-(6-((4-(trifluoromethyl)furan-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((4-(trifluoromethyl)furan-2-yl)methoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, DMSO) δ 12.78 (s, 1H), 8.47 (p, J = 1.5 Hz, 1H), 8.26 (d, J = 1.5 Hz, 1H), 7.95 - 7.85 (m, 2H), 7.79 (dd, J = 8.4, 1.5 Hz, 1H), 7.60 (d, J = 8.4 Hz, 1H), 7.54 (dd, J = 7.5, 1.8 Hz, 1H), 7.40 (dd, J = 11.5, 6.1 Hz, 1H), 6.99 (s, 1H), 6.93 (d, J = 8.2 Hz, 1H), 5.49 (s, 2H), 5.08 (qd, J = 7.0, 2.7 Hz, 1H), 4.76 (dd, J = 15.6, 7.1 Hz, 1H), 4.63 (dd, J = 15.5, 2.7 Hz, 1H), 4.58 - 4.41 (m, 3H), 4.36 (dt, J = 8.9, 5.9 Hz, 1H), 2.79 - 2.68 (m, 1H), 2.39 (ddt, J = 11.1, 9.0, 6.9 Hz, 1H).

### Example 19: (S)-2-(4-(6-((1-(difluoromethyl)-1H-imidazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1-(difluoromethyl)-1H-imidazol-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. 1H NMR (400 MHz, DMSO) δ 12.78 (s, 1H), 8.26 (d, J = 1.5 Hz, 1H), 8.00 - 7.84 (m, 3H), 7.83 - 7.76 (m, 1H), 7.68 - 7.58 (m, 2H), 7.55 (dd, J = 7.4, 1.8 Hz, 1H), 7.39 (dd, J = 11.5, 6.1 Hz, 1H), 7.11 (d, J = 1.5 Hz, 1H), 6.93 (d, J = 8.2 Hz, 1H), 5.63 (s, 2H), 5.08 (qd, J = 7.0, 2.7 Hz, 1H), 4.76 (dd, J = 15.6, 7.1 Hz, 1H), 4.63 (dd, J = 15.6, 2.8 Hz, 1H), 4.58 - 4.41 (m, 3H), 4.36 (dt, J = 9.0, 5.9 Hz, 1H), 2.73 (dtd, J = 11.3, 8.2, 6.3 Hz, 1H), 2.46 - 2.35 (m, 1H).

### Example 20: (S)-2-(4-(6-((2-chlorothiazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-chlorothiazol-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, DMSO) δ 8.25 (d, J = 1.6 Hz, 1H), 8.01 - 7.87 (m, 2H), 7.85 (s, 1H), 7.79 (dd, J = 8.4, 1.5 Hz, 1H), 7.63 - 7.53 (m, 2H), 7.42 (dd, J = 11.6, 6.1 Hz, 1H), 6.93 (d, J = 8.3 Hz, 1H), 5.69 (s, 2H), 5.11 - 5.04 (m, 1H), 4.76 (dd, J = 15.5, 7.1 Hz, 1H), 4.63 (dd, J = 15.5, 2.8 Hz, 1H), 4.59 - 4.42 (m, 3H), 4.36 (dt, J = 8.9, 5.9 Hz, 1H), 2.78 - 2.68 (m, 1H), 2.39 (s, 1H).

### Example 21: (S)-2-(4-(6-((2-bromothiazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-bromothiazol-5-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.30 (d, J = 1.5 Hz, 1H), 7.97 (dd, J = 10.5, 6.4 Hz, 1H), 7.90 (t, J = 7.9 Hz, 1H), 7.86 - 7.80 (m, 2H), 7.63 (d, J = 8.4 Hz, 1H), 7.57 (dd, J = 7.4, 1.6 Hz, 1H), 7.44 (dd, J = 11.6, 6.0 Hz, 1H), 6.93 (d, J = 8.2 Hz, 1H), 5.70 (s, 2H), 5.08 (d, J = 6.9 Hz, 1H), 4.80 (dd, J = 15.5, 7.1 Hz, 1H), 4.66 (dd, J = 15.8, 2.7 Hz, 1H), 4.62 - 4.45 (m, 3H), 4.37 (dt, J = 9.2, 6.0 Hz, 1H), 2.78 - 2.70 (m, 1H), 2.43 - 2.37 (m, 1H).

### Example 22: (S)-2-(4-(6-((5-bromothiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-bromothiazol-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.31 (d, J = 1.5 Hz, 1H), 7.97 - 7.86 (m, 3H), 7.83 (dd, J = 8.5, 1.5 Hz, 1H), 7.64 (d, J = 8.5 Hz, 1H), 7.59 (dd, J = 7.4, 1.6 Hz, 1H), 7.42 (dd, J = 11.6, 6.1 Hz, 1H), 7.01 (d, J = 8.2 Hz, 1H), 5.75 (s, 2H), 5.08 (dt, J = 9.3, 4.6 Hz, 1H), 4.80 (dd, J = 15.6, 7.2 Hz, 1H), 4.66 (dd, J = 15.6, 2.8 Hz, 1H), 4.63 - 4.44 (m, 3H), 4.37 (dt, J = 9.1, 6.0 Hz, 1H), 2.81 - 2.65 (m, 1H), 2.44 - 2.30 (m, 1H).

### Example 23: (S)-2-(4-(6-((4-cyanothiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-cyanothiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.47 (s, 1H), 8.30 (d, J = 1.5 Hz, 1H), 7.98 (dd, J = 8.5, 1.5 Hz, 1H), 7.91 - 7.75 (m, 2H), 7.66 (d, J = 8.5 Hz, 1H), 7.61 (dd, J = 7.4, 1.5 Hz, 1H), 7.19 (dd, J = 11.5, 6.0 Hz, 1H), 6.96 (d, J = 8.2 Hz, 1H), 5.81 (s, 2H), 5.20 (qd, J = 7.0, 2.6 Hz, 1H), 4.73 (dd, J = 15.7, 7.0 Hz, 1H), 4.68 - 4.39 (m, 6H), 2.90 - 2.71 (m, 1H), 2.50 (ddt, J = 11.4, 9.2, 7.2 Hz, 1H).

### Example 24: (S)-2-(2,5-difluoro-4-(6-((5-(trifluoromethyl)isoxazol-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-(trifluoromethyl)isoxazol-3-yl)methoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.26 (d, J = 1.5 Hz, 1H), 7.92 (t, J = 7.9 Hz, 1H), 7.82 - 7.74 (m, 2H), 7.60 (dd, J = 4.8, 3.7 Hz, 2H), 7.56 (dd, J = 7.5, 1.6 Hz, 1H), 7.39 (dd, J = 11.6, 6.0 Hz, 1H), 7.00 (d, J = 8.2 Hz, 1H), 5.66 (s, 2H), 5.08 (qd, J = 7.0, 2.7 Hz, 1H), 4.76 (dd, J = 15.6, 7.0 Hz, 1H), 4.62 (dd, J = 15.6, 2.8 Hz, 1H), 4.58 - 4.40 (m, 3H), 4.36 (dt, J = 9.0, 5.9 Hz, 1H), 2.83 - 2.63 (m, 1H), 2.39 (ddt, J = 11.2, 9.0, 7.1 Hz, 1H).

### Example 25: (S)-2-(2,5-difluoro-4-(6-((5-(trifluoromethyl)-1,3,4-thiadiazol-2-yl)methoxy)pyridin-2-yl)benzyl)-1-(oxetan-2-ylmethyl)-1H-benzo [d] imidazole-6-carboxylic acid

(S)-2-(2, 5-difluoro-4-(6-((5-(trifluoromethyl)-1,3,4-thiadiazol-2-yl)methoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.25 (d, J = 1.5 Hz, 1H), 7.95 (t, J = 7.9 Hz, 1H), 7.85 (dd, J = 10.5, 6.5 Hz, 1H), 7.79 (dd, J = 8.4, 1.6 Hz, 1H), 7.60 (td, J = 5.8, 5.4, 2.9 Hz, 2H), 7.39 (dd, J = 11.5, 6.0 Hz, 1H), 7.05 (d, J = 8.3 Hz, 1H), 6.05 (s, 2H), 5.07 (d, J = 7.6 Hz, 1H), 4.75 (dd, J = 15.6, 7.0 Hz, 1H), 4.62 (dd, J = 15.6, 2.8 Hz, 1H), 4.58 - 4.41 (m, 3H), 4.35 (dt, J = 9.1, 5.9 Hz, 1H), 2.79 - 2.68 (m, 1H), 2.39 (q, J = 10.0, 8.6 Hz, 1H).

### Example 26: (S)-2-(2,5-difluoro-4-(6-(thiazolo[5,4-b]pyridin-2-ylmethoxy)pyridin-2-yl)benzyl)-1-(oxetan-2-ylmethyl)-1H-benzo[d]imidazole-6-carboxylic acid

(S)-2-(2,5-difluoro-4-(6-(thiazolo[5,4-b]pyridin-2-ylmethoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, Chloroform-d) δ 8.61 (dd, J = 4.7, 1.5 Hz, 1H), 8.32 (dd, J = 8.2, 1.5 Hz, 1H), 8.19 (d, J = 1.5 Hz, 1H), 8.08 (dd, J = 8.5, 1.5 Hz, 1H), 7.90 - 7.82 (m, 2H), 7.74 (t, J = 7.8 Hz, 1H), 7.57 (d, J = 7.4 Hz, 1H), 7.47 (dd, J = 8.2, 4.6 Hz, 1H), 7.13 (dd, J = 11.3, 6.0 Hz, 1H), 6.94 (d, J = 8.2 Hz, 1H), 5.90 (s, 2H), 5.26 - 5.12 (m, 1H), 4.65 (q, J = 7.4 Hz, 1H), 4.62 - 4.34 (m, 5H), 2.74 (dt, J = 14.7, 6.9 Hz, 1H), 2.43 (ddd, J = 15.5, 10.4, 7.2 Hz, 1H).

### Example 27: (S)-2-(4-(6-((5-bromo-4-methylthiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-bromo-4-methylthiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.25 (d, J = 1.5 Hz, 1H), 7.96 - 7.85 (m, 2H), 7.79 (dd, J = 8.4, 1.5 Hz, 1H), 7.63 - 7.56 (m, 2H), 7.39 (dd, J = 11.6, 6.0 Hz, 1H), 7.00 (d, J = 8.3 Hz, 1H), 5.70 (s, 2H), 5.07 (tt, J = 7.3, 4.1 Hz, 1H), 4.75 (dd, J = 15.6, 7.1 Hz, 1H), 4.62 (dd, J = 15.5, 2.8 Hz, 1H), 4.58 - 4.41 (m, 3H), 4.36 (dt, J = 9.0, 5.9 Hz, 1H), 2.79 - 2.68 (m, 1H), 2.40 (dt, J = 11.3, 8.0 Hz, 1H), 2.34 (s, 3H).

### Example 28: (S)-2-(2,5-difluoro-4-(6-(pyrazolo[1,5-a]pyridin-2-ylmethoxy)pyridin-2-yl)benzyl)-1-(oxetan-2-ylmethyl)-1H-benzo[d]imidazole-6-carboxylic acid

(S)-2-(2,5-difluoro-4-(6-(pyrazolo[1,5-a]pyridin-2-ylmethoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.57 (d, J = 1.4 Hz, 1H), 8.53 - 8.46 (m, 1H), 8.21 (dd, J = 8.6, 1.4 Hz, 1H), 7.99 (dd, J = 10.9, 6.3 Hz, 1H), 7.89 - 7.73 (m, 2H), 7.67 - 7.53 (m, 2H), 7.36 (dd, J = 11.3, 6.0 Hz, 1H), 7.21 (ddd, J = 8.9, 6.8, 1.1 Hz, 1H), 6.93 (d, J = 8.3 Hz, 1H), 6.88 (td, J = 6.9, 1.4 Hz, 1H), 6.65 (s, 1H), 5.68 (s, 2H), 5.26 (qd, J = 7.4, 2.4 Hz, 1H), 4.99 (dd, J = 15.5, 7.5 Hz, 1H), 4.86 - 4.76 (m, 3H), 4.76 - 4.65 (m, 1H), 4.54 (dt, J = 9.2, 6.0 Hz, 1H), 2.87 (dtd, J = 11.5, 8.2, 6.1 Hz, 1H), 2.73 - 2.50 (m, 1H).

### Example 29: (S)-2-(2,5-difluoro-4-(6-(imidazo[1,2-a]pyrimidin-2-ylmethoxy)pyridin-2-yl)benzyl)-1-(oxetan-2-ylmethyl)-1H-benzo[d]imidazole-6-carboxylic acid

(S)-2-(2,5-difluoro-4-(6-(imidazo[1,2-a]pyrimidin-2-ylmethoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, Methanol-d4) δ 9.16 (dd, J = 6.8, 1.8 Hz, 1H), 8.98 (dd, J = 4.4, 1.8 Hz, 1H), 8.52 (d, J = 1.4 Hz, 1H), 8.23 (s, 1H), 8.16 (dd, J = 8.6, 1.5 Hz, 1H), 7.98 (dd, J = 10.6, 6.3 Hz, 1H), 7.89 (t, J = 7.9 Hz, 1H), 7.76 (d, J = 8.6 Hz, 1H), 7.64 (dd, J = 7.5, 1.5 Hz, 1H), 7.56 (dd, J = 6.8, 4.3 Hz, 1H), 7.36 (dd, J = 11.2, 6.0 Hz, 1H), 7.00 (d, J = 8.2 Hz, 1H), 5.80 (s, 2H), 5.26 (tt, J = 7.4, 3.8 Hz, 1H), 5.00 - 4.92 (m, 1H), 4.84 - 4.64 (m, 4H), 4.52 (dt, J = 9.2, 5.9 Hz, 1H), 2.87 (ddd, J = 14.1, 9.7, 6.9 Hz, 1H), 2.69 - 2.46 (m, 1H).

### Example 30: (S)-2-(2,5-difluoro-4-(6-(imidazo[1,2-a]pyridin-2-ylmethoxy)pyridin-2-yl)benzyl)-1-(oxetan-2-ylmethyl)-1H-benzo[d]imidazole-6-carboxylic acid

(S)-2-(2,5-difluoro-4-(6-(imidazo[1,2-a]pyridin-2-ylmethoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.80 (dt, J = 6.8, 1.2 Hz, 1H), 8.50 (d, J = 1.3 Hz, 1H), 8.33 (s, 1H), 8.15 (dd, J = 8.6, 1.5 Hz, 1H), 8.05 - 7.84 (m, 4H), 7.75 (d, J = 8.5 Hz, 1H), 7.64 (dd, J = 7.5, 1.5 Hz, 1H), 7.51 (td, J = 6.9, 1.2 Hz, 1H), 7.36 (dd, J = 11.3, 6.0 Hz, 1H), 7.00 (d, J = 8.3 Hz, 1H), 5.81 (d, J = 0.8 Hz, 2H), 5.26 (qd, J = 7.4, 2.5 Hz, 1H), 4.99 - 4.91 (m, 1H), 4.85 - 4.61 (m, 4H), 4.51 (dt, J = 9.2, 5.9 Hz, 1H), 2.86 (dtd, J = 11.5, 8.2, 6.1 Hz, 1H), 2.56 (ddt, J = 11.6, 9.1, 7.2 Hz, 1H).

### Example 31: (S)-2-(4-(6-((6-cyanoimidazo[1,2-a]pyridin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-cyanoimidazo[1,2-a]pyridin-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 9.24 (t, J = 1.3 Hz, 1H), 8.58 (t, J = 1.0 Hz, 1H), 8.29 - 8.12 (m, 2H), 7.97 (dd, J = 10.7, 6.3 Hz, 1H), 7.86 (t, J = 7.9 Hz, 1H), 7.84 - 7.77 (m, 2H), 7.74 (dd, J = 9.4, 1.6 Hz, 1H), 7.61 (dd, J = 7.6, 1.6 Hz, 1H), 7.39 (dd, J = 11.2, 6.0 Hz, 1H), 6.97 (d, J = 8.2 Hz, 1H), 5.80 - 5.68 (m, 2H), 5.26 (td, J = 7.4, 2.4 Hz, 1H), 4.99 (dd, J = 15.5, 7.5 Hz, 1H), 4.87 - 4.76 (m, 3H), 4.76 - 4.64 (m, 1H), 4.54 (dt, J = 9.1, 6.0 Hz, 1H), 2.99 - 2.80 (m, 1H), 2.59 (ddt, J = 11.6, 9.1, 7.2 Hz, 1H).

### Example 32: (S)-2-(4-(6-((6-chloroimidazo[1,2-a]pyridin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-chloroimidazo[1,2-a]pyridin-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. ¹H NMR (400 MHz, Methanol-d4) δ 8.99 (dd, J = 1.9, 0.9 Hz, 1H), 8.52 (d, J = 1.4 Hz, 1H), 8.26 (s, 1H), 8.17 (dd, J = 8.6, 1.5 Hz, 1H), 8.04 - 7.93 (m, 2H), 7.93 - 7.83 (m, 2H), 7.76 (d, J = 8.5 Hz, 1H), 7.63 (dd, J = 7.4, 1.5 Hz, 1H), 7.37 (dd, J = 11.2, 6.0 Hz, 1H), 6.99 (d, J = 8.2 Hz, 1H), 5.83 - 5.71 (m, 2H), 5.26 (qd, J = 7.3, 2.4 Hz, 1H), 5.02 - 4.92 (m, 1H), 4.84 - 4.62 (m, 4H), 4.52 (dt, J = 9.2, 5.9 Hz, 1H), 2.87 (dtd, J = 11.5, 8.2, 6.1 Hz, 1H), 2.57 (ddt, J = 11.5, 9.0, 7.2 Hz, 1H).

### Example 33: (S)-2-(2,5-difluoro-4-(6-(imidazo[1,2-a]pyrazin-2-ylmethoxy)pyridin-2-yl)benzyl)-1-(oxetan-2-ylmethyl)-1H-benzo[d]imidazole-6-carboxylic acid

(S)-2-(2,5-difluoro-4-(6-(imidazo[1,2-a]pyrazin-2-ylmethoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, Methanol-d4) δ 9.09 (s, 1H), 8.61 (s, 1H), 8.57 (dd, J = 4.6, 1.5 Hz, 1H), 8.31 - 8.20 (m, 2H), 8.08 - 7.92 (m, 2H), 7.90 - 7.76 (m, 2H), 7.61 (dd, J = 7.5, 1.6 Hz, 1H), 7.45 - 7.36 (m, 1H), 6.98 (d, J = 8.3 Hz, 1H), 5.76 (s, 2H), 5.27 (td, J = 7.3, 2.4 Hz, 1H), 5.02 (dd, J = 15.4, 7.6 Hz, 1H), 4.85 - 4.76 (m, 2H), 4.71 (td, J = 8.2, 6.2 Hz, 1H), 4.66 - 4.59 (m, 1H), 4.56 (dt, J = 9.1, 6.0 Hz, 1H), 2.96 - 2.82 (m, 1H), 2.59 (ddt, J = 11.7, 9.2, 7.2 Hz, 1H).

### Example 34: (S)-2-(4-(6-(benzo[d]thiazol-2-ylmethoxy)pyridin-2-yl)-2,5-difluorobenzyl)-1-(oxetan-2-ylmethyl)-1H-benzo[d]imidazole-6-carboxylic acid

(S)-2-(4-(6-(benzo[d]thiazol-2-ylmethoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.46 (t, J = 0.9 Hz, 1H), 8.15 (dd, J = 8.6, 1.5 Hz, 1H), 8.00 (d, J = 8.3 Hz, 1H), 7.95 (d, J = 7.9 Hz, 1H), 7.87 (dd, J = 10.8, 6.8 Hz, 2H), 7.73 (d, J = 8.6 Hz, 1H), 7.67 - 7.59 (m, 1H), 7.51 (ddd, J = 8.3, 7.3, 1.3 Hz, 1H), 7.43 (ddd, J = 8.3, 7.2, 1.2 Hz, 1H), 7.26 (dd, J = 11.3, 6.0 Hz, 1H), 7.01 (d, J = 8.1 Hz, 1H), 5.90 (s, 2H), 5.29 - 5.16 (m, 1H), 4.88 (d, J = 7.5 Hz, 1H), 4.80 - 4.61 (m, 4H), 4.50 (dt, J = 9.1, 6.0 Hz, 1H), 2.90 - 2.76 (m, 1H), 2.66 - 2.45 (m, 1H).

### Example 35: (S)-2-(4-(6-((5-chlorobenzo[d]thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-chlorobenzo[d]thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. ¹H NMR (400 MHz, Methanol-d4) δ 8.44 (d, J = 1.4 Hz, 1H), 8.14 (dd, J = 8.6, 1.5 Hz, 1H), 7.91 (d, J = 8.6 Hz, 1H), 7.84 (dd, J = 9.7, 5.8 Hz, 2H), 7.78 (s, 2H), 7.73 (d, J = 8.5 Hz, 1H), 7.61 (d, J = 7.6 Hz, 1H), 7.41 (dd, J = 8.6, 2.1 Hz, 1H), 7.24 (dd, J = 11.3, 6.0 Hz, 1H), 7.00 (d, J = 8.2 Hz, 1H), 5.89 (s, 2H), 5.27 - 5.12 (m, 1H), 4.85 (d, J = 7.4 Hz, 1H), 4.77 - 4.57 (m, 4H), 4.50 (dt, J = 9.3, 6.0 Hz, 1H), 2.94 - 2.76 (m, 1H), 2.64 - 2.46 (m, 1H).

### Example 36: (S)-2-(2,5-difluoro-4-(6-((1-methyl-1H-pyrazol-5-yl)methoxy)pyridin-2-yl)benzyl)-1-(oxetan-2-ylmethyl)-1H-benzo[d]imidazole-6-carboxylic acid

(S)-2-(2,5-difluoro-4-(6-((1-methyl-1H-pyrazol-5-yl)methoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.27 (d, J = 1.5 Hz, 1H), 7.96 - 7.84 (m, 2H), 7.80 (dd, J = 8.4, 1.6 Hz, 1H), 7.61 (d, J = 8.4 Hz, 1H), 7.53 (dd, J = 7.4, 1.7 Hz, 1H), 7.47 - 7.35 (m, 2H), 6.93 (d, J = 8.2 Hz, 1H), 6.38 (d, J= 1.8 Hz, 1H), 5.54 (s, 2H), 5.08 (qd, J = 7.0, 2.7 Hz, 1H), 4.76 (dd, J = 15.6, 7.1 Hz, 1H), 4.63 (dd, J = 15.5, 2.8 Hz, 1H), 4.58 - 4.40 (m, 3H), 4.36 (dt, J = 9.0, 5.9 Hz, 1H), 3.87 (s, 3H), 2.72 (dddd, J = 14.2, 11.2, 6.5, 4.1 Hz, 1H), 2.40 (ddt, J = 11.2, 9.0, 7.0 Hz, 1H).

### Example 37: (S)-2-(4-(6-((5-(cyanomethyl)-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(cyanomethyl)-1-methyl-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.25 (d, J = 1.5 Hz, 1H), 7.94 (dd, J = 10.5, 6.5 Hz, 1H), 7.90 - 7.75 (m, 2H), 7.59 (d, J = 8.4 Hz, 1H), 7.50 (dd, J = 7.5, 1.7 Hz, 1H), 7.38 (dd, J = 11.5, 6.1 Hz, 1H), 6.88 (d, J = 8.3 Hz, 1H), 6.33 (s, 1H), 5.35 (s, 2H), 5.08 (qd, J = 7.1, 2.7 Hz, 1H), 4.75 (dd, J = 15.6, 7.0 Hz, 1H), 4.62 (dd, J = 15.5, 2.8 Hz, 1H), 4.58 - 4.45 (m, 2H), 4.45 (d, J = 16.9 Hz, 1H), 4.36 (dt, J = 9.0, 5.9 Hz, 1H), 4.22 (s, 2H), 3.78 (s, 3H), 2.79 - 2.67 (m, 1H), 2.46 - 2.35 (m, 1H).;1H NMR (400 MHz, DMSO-d6) δ 8.32 (d, J = 1.5 Hz, 1H), 7.97 (dd, J = 10.6, 6.3 Hz, 1H), 7.91 - 7.80 (m, 2H), 7.65 (d, J = 8.4 Hz, 1H), 7.50 (dd, J = 7.6, 1.8 Hz, 2H), 7.42 (dd, J = 11.5, 6.1 Hz, 1H), 7.02 (s, 1H), 6.88 (d, J = 8.3 Hz, 1H), 6.17 (s, 1H), 5.32 (s, 2H), 5.14 - 5.05 (m, 1H), 4.81 (dd, J = 15.5, 7.1 Hz, 1H), 4.72 - 4.63 (m, 1H), 4.64 - 4.46 (m, 3H), 4.38 (dt, J = 8.9, 5.8 Hz, 1H), 3.74 (s, 3H), 3.51 (s, 2H), 2.80 - 2.69 (m, 1H), 2.46 - 2.34 (m, 1H).

### Example 38: (S)-2-(4-(6-((5-chloro-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-chloro-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.26 (d, J = 1.5 Hz, 1H), 7.93 (dd, J = 10.6, 6.4 Hz, 1H), 7.90 - 7.76 (m, 2H), 7.60 (d, J = 8.4 Hz, 1H), 7.51 (dd, J = 7.4, 1.7 Hz, 1H), 7.39 (dd, J = 11.5, 6.1 Hz, 1H), 6.89 (d, J = 8.2 Hz, 1H), 6.48 (s, 1H), 5.34 (s, 2H), 5.08 (qd, J = 6.9, 2.6 Hz, 1H), 4.76 (dd, J = 15.6, 7.1 Hz, 1H), 4.63 (dd, J = 15.6, 2.7 Hz, 1H), 4.58 - 4.45 (m, 2H), 4.45 (d, J = 16.8 Hz, 1H), 4.36 (dt, J = 9.0, 5.9 Hz, 1H), 3.80 (s, 3H), 2.72 (dtd, J = 11.2, 8.2, 6.2 Hz, 1H), 2.40 (ddd, J = 11.2, 9.1, 5.5 Hz, 1H).

### Example 39: (S)-2-(4-(6-((5-cyclopropyl-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyclopropyl-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.31 (d, J = 1.5 Hz, 1H), 7.98 (dd, J = 8.5, 1.5 Hz, 1H), 7.92 (dd, J = 10.8, 6.4 Hz, 1H), 7.75 (t, J = 7.9 Hz, 1H), 7.67 (d, J = 8.5 Hz, 1H), 7.51 (dd, J = 7.4, 1.6 Hz, 1H), 7.18 (dd, J = 11.5, 6.0 Hz, 1H), 6.81 (d, J = 8.2 Hz, 1H), 5.98 (s, 1H), 5.35 (s, 2H), 5.20 (qd, J = 7.1, 2.6 Hz, 1H), 4.78 - 4.40 (m, 6H), 3.88 (s, 3H), 2.80 (dtd, J = 11.5, 8.2, 6.1 Hz, 1H), 2.49 (ddt, J = 11.6, 9.3, 7.2 Hz, 1H), 1.83 (tt, J = 8.4, 5.0 Hz, 1H), 1.06 - 0.93 (m, 2H), 0.72 - 0.58 (m, 2H).

### Example 40: (S)-2-(4-(6-((5-(difluoromethyl)-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(difluoromethyl)-1-methyl-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.55 (s, 1H), 8.26 (d, J = 1.5 Hz, 1H), 7.89 (t, J = 7.9 Hz, 1H), 7.83 - 7.72 (m, 2H), 7.68 (d, J = 8.0 Hz, 2H), 7.60 (d, J = 8.3 Hz, 2H), 7.51 (dd, J = 7.6, 1.8 Hz, 1H), 7.38 (dd, J = 11.5, 6.1 Hz, 1H), 6.98 (d, J = 8.2 Hz, 1H), 5.59 (s, 2H), 5.08 (qd, J = 7.0, 2.7 Hz, 1H), 4.75 (dd, J = 15.6, 7.0 Hz, 1H), 4.62 (dd, J = 15.5, 2.8 Hz, 1H), 4.57 - 4.44 (m, 3H), 4.44 - 4.30 (m, 3H), 2.72 (dtd, J = 11.3, 8.2, 6.3 Hz, 1H), 2.47 - 2.35 (m, 1H).

### Example 41: (S)-2-(4-(6-((6-((1-cyanocyclopropyl)carbamoyl)-4-methoxypyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-((1-cyanocyclopropyl)carbamoyl)-4-methoxypyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1H NMR (400 MHz, Methanol-d4) δ 8.54 (s, 1H), 8.31 (d, J = 1.5 Hz, 1H), 7.99 (dd, J = 8.5, 1.6 Hz, 1H), 7.86 - 7.75 (m, 3H), 7.67 (d, J = 8.5 Hz, 1H), 7.54 (dd, J = 7.5, 1.5 Hz, 1H), 7.18 (dd, J = 11.5, 6.0 Hz, 1H), 6.89 (d, J = 8.2 Hz, 1H), 5.59 (s, 2H), 5.19 (tt, J = 7.1, 3.5 Hz, 1H), 4.73 (dd, J = 15.7, 6.9 Hz, 1H), 4.68 - 4.39 (m, 5H), 4.07 (s, 3H), 2.80 (dtd, J = 11.4, 8.2, 6.1 Hz, 1H), 2.49 (ddt, J = 11.5, 9.2, 7.2 Hz, 1H), 1.68 - 1.52 (m, 2H), 1.44 - 1.36 (m, 2H).

### Example 42: (S)-2-(4-(6-((6-((1-cyanocyclopropyl)carbamoyl)-4-methoxypyridin-3-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((6-((1-cyanocyclopropyl)carbamoyl)-4-methoxypyridin-3-yl)methoxy)pyridin-2-yl)-2, 5-difluorobenzyl)-4-fluoro-1-(oxetan-2-ylmethyl)-1H-benzo[d]imidazole-6-carboxylic acid was prepared in a manner as described in Procedure 1. 1H NMR (400 MHz, Methanol-d4) δ 8.53 (s, 1H), 8.16 (d, J = 1.3 Hz, 1H), 7.86 - 7.70 (m, 3H), 7.65 (dd, J = 11.2, 1.2 Hz, 1H), 7.52 (dd, J = 7.5, 1.5 Hz, 1H), 7.17 (dd, J = 11.5, 6.1 Hz, 1H), 6.88 (d, J = 8.2 Hz, 1H), 5.57 (s, 2H), 5.15 (qd, J = 7.1, 2.5 Hz, 1H), 4.72 (dd, J = 15.7, 7.1 Hz, 1H), 4.66 - 4.47 (m, 5H), 4.43 (dt, J = 9.2, 6.0 Hz, 1H), 4.06 (s, 3H), 2.78 (dtd, J = 11.5, 8.2, 6.1 Hz, 1H), 2.56 - 2.33 (m, 1H), 1.76 - 1.48 (m, 2H), 1.47 - 1.32 (m, 2H).

### Example 43: (S)-2-(2,5-difluoro-4-(6-((6-(methylcarbamoyl)pyridin-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((6-(methylcarbamoyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.84 - 8.72 (m, 2H), 8.26 (d, J = 1.5 Hz, 1H), 8.14 - 8.02 (m, 2H), 7.97 - 7.86 (m, 1H), 7.86 - 7.76 (m, 2H), 7.60 (d, J = 8.4 Hz, 1H), 7.53 (dd, J = 7.5, 1.7 Hz, 1H), 7.39 (dd, J = 11.5, 6.1 Hz, 1H), 6.99 (d, J = 8.3 Hz, 1H), 5.61 (s, 2H), 5.08 (qd, J = 7.0, 2.7 Hz, 1H), 4.76 (dd, J = 15.6, 7.0 Hz, 1H), 4.63 (dd, J = 15.5, 2.8 Hz, 1H), 4.58 - 4.40 (m, 3H), 4.36 (dt, J = 9.0, 5.9 Hz, 1H), 2.82 (d, J = 4.8 Hz, 3H), 2.79 - 2.65 (m, 1H), 2.39 (ddt, J = 11.2, 9.0, 7.0 Hz, 1H).

### Example 44: (S)-2-(4-(6-((6-(dimethylcarbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-(dimethylcarbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. s1H NMR (400 MHz, DMSO-d6) δ 8.73 (d, J = 2.1 Hz, 1H), 8.25 (d, J = 1.5 Hz, 1H), 8.02 (dd, J = 8.0, 2.2 Hz, 1H), 7.89 (t, J = 7.9 Hz, 1H), 7.84 - 7.76 (m, 2H), 7.59 (dd, J = 8.2, 4.2 Hz, 2H), 7.52 (dd, J = 7.6, 1.7 Hz, 1H), 7.39 (dd, J = 11.5, 6.0 Hz, 1H), 6.98 (d, J = 8.3 Hz, 1H), 5.57 (s, 2H), 5.07 (tt, J = 7.1, 3.7 Hz, 1H), 4.75 (dd, J = 15.6, 7.0 Hz, 1H), 4.62 (dd, J = 15.6, 2.8 Hz, 1H), 4.58 - 4.40 (m, 3H), 4.36 (dt, J = 9.0, 5.9 Hz, 1H), 3.00 (s, 3H), 2.93 (s, 3H), 2.72 (dq, J = 12.1, 8.4 Hz, 1H), 2.48 - 2.30 (m, 1H).

### Example 45: (S)-2-(2,5-difluoro-4-(6-((4-methoxy-6-(methylcarbamoyl)pyridin-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((4-methoxy-6-(methylcarbamoyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 1. 1H NMR (400 MHz, DMSO-d6) δ 8.76 (q, J = 4.7 Hz, 1H), 8.55 (s, 1H), 8.26 (d, J = 1.4 Hz, 1H), 7.92 - 7.76 (m, 3H), 7.69 (s, 1H), 7.60 (d, J = 8.4 Hz, 1H), 7.52 (dd, J = 7.5, 1.7 Hz, 1H), 7.40 (dd, J = 11.5, 6.1 Hz, 1H), 6.96 (d, J = 8.3 Hz, 1H), 5.52 (s, 2H), 5.08 (qd, J = 7.0, 2.7 Hz, 1H), 4.76 (dd, J = 15.6, 7.0 Hz, 1H), 4.63 (dd, J = 15.6, 2.8 Hz, 1H), 4.58 - 4.40 (m, 3H), 4.36 (dt, J = 9.0, 5.9 Hz, 1H), 4.00 (s, 3H), 2.81 (d, J = 4.8 Hz, 3H), 2.77 - 2.66 (m, 1H), 2.40 (ddt, J = 11.2, 8.8, 6.9 Hz, 1H).

### Example 46: (S)-2-(4-(6-((4-chloro-6-(methylcarbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-(methylcarbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1. 1HNMR (400 MHz, DMSO-d6) δ 8.85 (d, J = 19.7 Hz, 2H), 8.28 (s, 1H), 8.09 (s, 1H), 7.91 (t, J = 7.9 Hz, 1H), 7.87 - 7.77 (m, 2H), 7.62 (d, J = 8.4 Hz, 1H), 7.55 (d, J = 7.3 Hz, 1H), 7.41 (dd, J = 11.5, 6.0 Hz, 1H), 7.01 (d, J = 8.3 Hz, 1H), 5.67 (s, 2H), 5.12 - 5.04 (m, 1H), 4.77 (dd, J = 15.6, 7.1 Hz, 1H), 4.64 (d, J = 14.7 Hz, 1H), 4.59 - 4.42 (m, 3H), 4.36 (dt, J = 9.0, 5.9 Hz, 1H), 2.82 (d, J = 4.7 Hz, 3H), 2.78 - 2.64 (m, 1H), 2.44 - 2.28 (m, 1H).

### Example 259: (S)-1-(4,4-dimethyltetrahydrofuran-3-yl)-2-((3'-((5-ethoxy-1,3,4-thiadiazol-2-yl)methoxy)-5-fluoro-2-methyl-[1,1'-biphenyl]-4-yl)methyl)-1H-benzo[d]imidazole-6-carboxylic acid

(S)-1-(4,4-dimethyltetrahydrofuran-3-yl)-2-((3'-((5-ethoxy-1,3,4-thiadiazol-2-yl)methoxy)-5-fluoro-2-methyl-[1,1'-biphenyl]-4-yl)methyl)-1H-benzo[d]imidazole-6-carboxylic acid was prepared in a manner as described in Procedure 1 using Intermediates I-1184 and 2-(chloromethyl)-5-ethoxy-1,3,4-thiadiazole. ES/MS m/z: 617.3 (M+H⁺). 1HNMR (400 MHz, Methanol-d4) δ 8.68 (s, 1H), 7.99 (dd, J = 8.5, 1.5 Hz, 1H), 7.69 (d, J = 8.6 Hz, 1H), 7.40 (t, J = 7.9 Hz, 1H), 7.21 (d, J = 7.6 Hz, 1H), 7.12 - 6.92 (m, 4H), 5.40 (s, 2H), 4.93 (d, J = 7.1 Hz, 1H), 4.56 (q, J = 7.4 Hz, 3H), 4.52 - 4.41 (m, 3H), 3.96 (d, J = 8.7 Hz, 1H), 3.80 (d, J = 8.7 Hz, 1H), 2.17 (s, 3H), 1.47 (t, J = 7.1 Hz, 3H), 1.33 (s, 3H), 0.65 (s, 3H).

### Example 297: (S)-2-(2,5-difluoro-4-(5-fluoro-6-((5-methyl-1,3,4-thiadiazol-2-yl)methoxy)pyridin-2-yl)benzyl)-4-fluoro-1-(oxetan-2-ylmethyl)-1H-benzo [d] imidazole-6-carboxylic acid

(S)-2-(2,5-difluoro-4-(5-fluoro-6-((5-methyl-1,3,4-thiadiazol-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 1 using Intermediate **I-1134** and 2-(chloromethyl)-5-methyl-1,3,4-thiadiazole. ES/MS m/z: 600.1 (M+H⁺). 1H NMR (400 MHz, Methanol-d4) δ 8.16 (s, 1H), 7.86 (dd, J = 10.6, 6.4 Hz, 1H), 7.75 - 7.57 (m, 3H), 7.21 (dd, J = 11.6, 6.0 Hz, 1H), 5.96 (s, 2H), 5.18 (d, J = 7.5 Hz, 1H), 4.74 (dd, J = 15.7, 7.0 Hz, 1H), 4.69 - 4.39 (m, 5H), 2.76 (s, 4H), 2.65 - 2.41 (m, 1H).

### Example 298: (S)-2-(2,5-difluoro-4-(6-((5-(methoxymethyl)-1,3,4-thiadiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(methoxymethyl)-1,3,4-thiadiazol-2-yl)methoxy)pyridin-2-yl)benzyl)-1-(oxetan-2-ylmethyl)-1H-benzo[d]imidazole-6-carboxylic acid was prepared in a manner as described in Procedure 1 using Intermediate **I-9** and 2-(chloromethyl)-5-(methoxymethyl)-1,3,4-thiadiazole. ES/MS m/z: 594.2 (M+H⁺). ¹H NMR (400 MHz, Methanol-d4) δ 8.55 (d, J = 1.4 Hz, 1H), 8.19 (dd, J = 8.6, 1.5 Hz, 1H), 7.95 (dd, J = 10.8, 6.3 Hz, 1H), 7.88 (t, J = 7.9 Hz, 1H), 7.77 (d, J = 8.6 Hz, 1H), 7.65 (dd, J = 7.5, 1.5 Hz, 1H), 7.38 (dd, J = 11.3, 6.0 Hz, 1H), 6.98 (d, J = 8.3 Hz, 1H), 5.92 (s, 2H), 5.26 (qd, J = 7.4, 2.4 Hz, 1H), 4.97 (dd, J = 15.5, 7.5 Hz, 1H), 4.86 - 4.75 (m, 4H), 4.77 - 4.65 (m, 2H), 4.54 (dt, J = 9.1, 5.9 Hz, 1H), 3.44 (s, 3H), 2.87 (dtd, J = 11.6, 8.2, 6.2 Hz, 1H), 2.58 (ddt, J = 11.5, 9.1, 7.2 Hz, 1H).

### Example 299: (S)-2-(4-(6-((5-ethyl-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-ethyl-1,3,4-thiadiazol-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1 using Intermediate **I-9** and 2-(chloromethyl)-5-ethyl-1,3,4-thiadiazole. ES/MS m/z: 578.2 (M+H⁺). ¹H NMR (400 MHz, Methanol-d4) δ 8.56 (d, J = 1.5 Hz, 1H), 8.20 (dd, J = 8.6, 1.4 Hz, 1H), 7.95 (dd, J = 10.7, 6.3 Hz, 1H), 7.87 (t, J = 7.9 Hz, 1H), 7.77 (d, J = 8.6 Hz, 1H), 7.64 (dd, J = 7.4, 1.5 Hz, 1H), 7.38 (dd, J = 11.3, 6.0 Hz, 1H), 6.97 (d, J = 8.2 Hz, 1H), 5.89 (s, 2H), 5.26 (qd, J = 7.4, 2.4 Hz, 1H), 4.97 (dd, J = 15.6, 7.5 Hz, 1H), 4.85 - 4.76 (m, 3H), 4.76 - 4.64 (m, 1H), 4.54 (dt, J = 9.1, 6.0 Hz, 1H), 3.12 (q, J = 7.6 Hz, 2H), 2.95 - 2.81 (m, 1H), 2.58 (ddt, J = 11.5, 9.1, 7.2 Hz, 1H), 1.38 (t, J = 7.6 Hz, 3H).

### Example 307: (S)-2-(4-(6-((5-cyclopropyl-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyclopropyl-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1 using Intermediate **I-9** and 2-(chloromethyl)-5-cyclopropyl-1,3,4-thiadiazole. ES/MS m/z: 590.2 (M+H⁺). 1HNMR (400 MHz, Methanol-d4) δ 8.58 (d, J = 1.3 Hz, 1H), 8.22 (dd, J = 8.6, 1.4 Hz, 1H), 7.96 (dd, J = 10.8, 6.4 Hz, 1H), 7.88 (t, J = 7.9 Hz, 1H), 7.79 (d, J = 8.6 Hz, 1H), 7.64 (dd, J = 7.5, 1.5 Hz, 1H), 7.40 (dd, J = 11.2, 6.0 Hz, 1H), 6.96 (d, J = 8.2 Hz, 1H), 5.85 (s, 2H), 5.27 (qd, J = 7.5, 2.3 Hz, 1H), 5.00 (dd, J = 15.5, 7.5 Hz, 1H), 4.86 - 4.75 (m, 3H), 4.75 - 4.63 (m, 1H), 4.55 (dt, J = 9.2, 6.0 Hz, 1H), 2.88 (dtd, J = 11.6, 8.1, 6.1 Hz, 1H), 2.59 (ddt, J = 11.7, 9.1, 7.2 Hz, 1H), 2.45 (tt, J = 8.3, 4.9 Hz, 1H), 1.33 - 1.19 (m, 2H), 1.07 (dt, J = 7.2, 4.5 Hz, 2H).

### Example 308: (S)-2-(2,5-difluoro-4-(6-((5-methyl-1,3,4-thiadiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-methyl-1,3,4-thiadiazol-2-yl)methoxy)pyridin-2-yl)benzyl)-1-(oxetan-2-ylmethyl)-1H-benzo[d]imidazole-6-carboxylic acid was prepared in a manner as described in Procedure 1 using Intermediate **I-9** and 2-(chloromethyl)-5-methyl-1,3,4-thiadiazole. ES/MS m/z: 564.2 (M+H⁺). 1HNMR (400 MHz, Methanol-d4) δ 8.60 (d, J = 1.3 Hz, 1H), 8.24 (dd, J = 8.6, 1.4 Hz, 1H), 7.98 (dd, J = 10.7, 6.3 Hz, 1H), 7.88 (t, J = 7.9 Hz, 1H), 7.80 (d, J = 8.6 Hz, 1H), 7.68 - 7.55 (m, 1H), 7.41 (dd, J = 11.2, 6.0 Hz, 1H), 6.97 (d, J = 8.2 Hz, 1H), 5.88 (s, 2H), 5.27 (dt, J = 7.4, 3.7 Hz, 1H), 5.02 (dd, J = 15.5, 7.6 Hz, 1H), 4.85 - 4.74 (m, 2H), 4.74 - 4.62 (m, 1H), 4.55 (dt, J = 9.1, 6.0 Hz, 1H), 2.95 - 2.80 (m, 1H), 2.75 (s, 3H), 2.59 (ddt, J= 11.6, 9.1, 7.2 Hz, 1H).

### Example 309: (S)-2-(4-(6-((5-(1,1-difluoroethyl)thiazol-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid

(S)-2-(4-(6-((5-(1,1-difluoroethyl)thiazol-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid was prepared in a manner as described in Procedure 32 using Intermediate **I-1127** and **I-1134**. ES/MS m/z: 468.6 (M+H⁺). 1HNMR (400 MHz, DMSO-d6) δ 8.22 - 8.10 (m, 2H), 7.91 (dd, J = 10.2, 8.2 Hz, 1H), 7.83 (dd, J = 10.5, 6.5 Hz, 1H), 7.68 -7.57 (m, 1H), 7.51 (dd, J = 11.4, 1.3 Hz, 1H), 7.41 (dd, J = 11.5, 6.0 Hz, 1H), 5.91 (s, 2H), 5.07 (d, J = 7.6 Hz, 1H), 4.79 (dd, J = 15.6, 7.1 Hz, 1H), 4.71 - 4.61 (m, 1H), 4.60 - 4.42 (m, 3H), 4.36 (dt, J = 9.0, 6.0 Hz, 1H), 2.72 (dt, J = 16.1, 7.7 Hz, 1H), 2.37 (dd, J = 18.9, 8.9 Hz, 1H), 2.13 (t, J = 18.7 Hz, 3H).

### Example 310: (S)-2-(4-(6-((5-cyano-3-fluorothiophen-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid

(S)-2-(4-(6-((5-cyano-3-fluorothiophen-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid was prepared in a manner as described in Procedure 1 using Intermediate **I-1127** and **I-1134.** ES/MS m/z: 468.6 (M+H⁺). 1HNMR (400 MHz, DMSO-d6) δ 8.16 (s, 1H), 8.03 (s, 1H), 7.91 (ddd, J = 18.4, 10.3, 7.3 Hz, 2H), 7.60 (d, J = 8.2 Hz, 1H), 7.51 (d, J = 11.4 Hz, 1H), 7.43 (dd, J = 11.5, 6.1 Hz, 1H), 5.79 (s, 2H), 5.07 (d, J = 6.5 Hz, 1H), 4.80 (dd, J = 15.6, 7.1 Hz, 1H), 4.66 (d, J = 15.4 Hz, 1H), 4.52 (q, J = 16.7 Hz, 3H), 4.43 - 4.29 (m, 1H), 2.79 - 2.68 (m, 1H), 2.37 (dd, J = 19.7, 9.7 Hz, 1H).

### Example 311: (S)-2-(4-(6-((5-chlorothiazol-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid

(S)-2-(4-(6-((5-chlorothiazol-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid was prepared in a manner as described in Procedure 1 using Intermediate **I-1134** and 5-chloro-2-(chloromethyl)thiazole. ES/MS m/z: 620.0 (M+H⁺). ¹H NMR (400 MHz, DMSO-d6) δ 8.16 (d, J = 1.3 Hz, 1H), 7.94 - 7.83 (m, 3H), 7.64 - 7.57 (m, 1H), 7.51 (dd, J = 11.4, 1.3 Hz, 1H), 7.42 (dd, J = 11.5, 6.1 Hz, 1H), 5.83 (s, 2H), 5.07 (d, J = 7.3 Hz, 1H), 4.79 (dd, J = 15.6, 7.1 Hz, 1H), 4.72 - 4.60 (m, 1H), 4.51 (q, J = 16.9 Hz, 3H), 4.36 (dt, J = 9.0, 5.9 Hz, 1H), 2.79 - 2.68 (m, 1H), 2.39 (q, J = 9.3, 8.3 Hz, 1H).

### Example 312: (S)-2-(4-(6-((5-ethoxy-1,3,4-thiadiazol-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((5-ethoxy-1,3,4-thiadiazol-2-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 1 using Intermediate **I-9** and 2-(chloromethyl)-5-ethoxy-1,3,4-thiadiazole. ES/MS m/z: 612.3 (M+H⁺). 1HNMR (400 MHz, DMSO-d6) δ 8.16 (d, J = 1.3 Hz, 1H), 8.01 -7.89 (m, 2H), 7.59 (dd, J = 7.5, 1.6 Hz, 1H), 7.51 (dd, J = 11.4, 1.3 Hz, 1H), 7.42 (dd, J = 11.5, 6.0 Hz, 1H), 6.98 (d, J = 8.3 Hz, 1H), 5.73 (s, 2H), 5.07 (tt, J = 7.0, 3.7 Hz, 1H), 4.80 (dd, J = 15.6, 7.1 Hz, 1H), 4.66 (dd, J = 15.5, 2.7 Hz, 1H), 4.61 - 4.43 (m, 5H), 4.36 (dt, J = 9.0, 6.0 Hz, 1H), 2.71 (ddt, J = 13.6, 10.2, 5.1 Hz, 1H), 2.44 - 2.33 (m, 1H), 1.36 (t, J = 7.0 Hz, 3H).

### Example 314: (S)-2-(4-(6-((5-chlorothiophen-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((5-chlorothiophen-2-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 1 using Intermediate **I-15** and 5-chloro-2-(chloromethyl)thiophene. ES/MS m/z: 600.0 (M+H⁺). ¹H NMR (400 MHz, DMSO-d6) δ 8.16 (d, J = 1.3 Hz, 1H), 7.98 (dd, J = 10.5, 6.5 Hz, 1H), 7.89 (t, J = 7.9 Hz, 1H), 7.56 (dd, J = 7.7, 1.7 Hz, 1H), 7.51 (dd, J = 11.4, 1.3 Hz, 1H), 7.43 (dd, J = 11.6, 6.0 Hz, 1H), 7.15 (d, J = 3.8 Hz, 1H), 7.04 (d, J = 3.8 Hz, 1H), 6.91 (d, J = 8.2 Hz, 1H), 5.61 (s, 2H), 5.14 - 5.02 (m, 1H), 4.80 (dd, J = 15.5, 7.1 Hz, 1H), 4.71 - 4.63 (m, 1H), 4.53 (q, J = 17.0 Hz, 3H), 4.36 (dt, J = 8.9, 6.0 Hz, 1H), 2.78 - 2.66 (m, 1H), 2.45 - 2.32 (m, 1H).

### Example 317: (S)-2-(4-(6-((5-chlorothiophen-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-chlorothiophen-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1 using Intermediate **I-9** and 5-chloro-2-(chloromethyl)thiophene. ES/MS m/z: 582.2 (M+H⁺). ¹H NMR (400 MHz, DMSO-d6) δ 8.26 (d, J = 1.5 Hz, 1H), 7.96 (dd, J = 10.5, 6.5 Hz, 1H), 7.89 (t, J = 7.9 Hz, 1H), 7.79 (dd, J = 8.4, 1.5 Hz, 1H), 7.61 (d, J = 8.4 Hz, 1H), 7.55 (dd, J = 7.7, 1.7 Hz, 1H), 7.41 (dd, J = 11.6, 6.1 Hz, 1H), 7.15 (d, J = 3.8 Hz, 1H), 7.04 (d, J = 3.8 Hz, 1H), 6.91 (d, J = 8.2 Hz, 1H), 5.61 (s, 2H), 5.13 - 5.01 (m, 1H), 4.76 (dd, J = 15.6, 7.1 Hz, 1H), 4.69 - 4.60 (m, 1H), 4.60 - 4.41 (m, 3H), 4.36 (dt, J = 9.0, 6.0 Hz, 1H), 2.80 - 2.69 (m, 1H), 2.44 - 2.32 (m, 1H).

### Example 318: (S)-2-(4-(6-((5-(1,1-difluoroethyl)thiazol-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((5-(1,1-difluoroethyl)thiazol-2-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 1 using Intermediate **I-1127** and **I-15**. ES/MS m/z: 631.0 (M+H⁺). 1HNMR (400 MHz, DMSO-d6) δ 8.22 - 8.08 (m, 2H), 7.94 (t, J = 7.9 Hz, 1H), 7.86 (dd, J = 10.5, 6.4 Hz, 1H), 7.59 (dd, J = 7.7, 1.7 Hz, 1H), 7.50 (dd, J = 11.4, 1.2 Hz, 1H), 7.41 (dd, J = 11.5, 6.1 Hz, 1H), 7.03 (d, J = 8.2 Hz, 1H), 5.82 (s, 2H), 5.07 (td, J = 8.0, 5.4 Hz, 1H), 4.78 (dd, J = 15.6, 7.1 Hz, 1H), 4.71 - 4.61 (m, 1H), 4.60 - 4.42 (m, 3H), 4.35 (dt, J = 9.0, 6.0 Hz, 1H), 2.80 - 2.69 (m, 1H), 2.39 (q, J = 10.1, 8.7 Hz, 1H), 2.12 (t, J = 18.7 Hz, 3H).

### Example 319: (S)-2-(4-(6-((5-chlorothiazol-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((5-chlorothiazol-2-yl)methoxy)pyridin-2-yl)-2, 5-difluorobenzyl)-4-fluoro-1-(oxetan-2-ylmethyl)-1H-benzo[d]imidazole-6-carboxylic acid was prepared in a manner as described in Procedure 1 using Intermediate **I-15** and 5-chloro-2-(chloromethyl)thiazole. ES/MS m/z: 601.0 (M+H⁺). ¹H NMR (400 MHz, DMSO-d6) δ 8.09 (s, 1H), 7.96 - 7.83 (m, 3H), 7.59 (dd, J = 7.6, 1.6 Hz, 1H), 7.49 (dd, J = 11.5, 1.2 Hz, 1H), 7.41 (dd, J = 11.6, 6.0 Hz, 1H), 7.01 (d, J = 8.3 Hz, 1H), 5.73 (s, 2H), 5.07 (qd, J = 7.0, 2.7 Hz, 1H), 4.76 (dd, J = 15.6, 7.0 Hz, 1H), 4.63 (dd, J = 15.5, 2.8 Hz, 1H), 4.60 - 4.41 (m, 3H), 4.35 (dt, J = 9.0, 5.9 Hz, 1H), 2.81 - 2.65 (m, 1H), 2.38 (ddt, J = 16.5, 13.3, 4.9 Hz, 1H).

### Example 324: (S)-2-(4-(6-((5-(difluoromethyl)thiazol-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((5-(difluoromethyl)thiazol-2-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 1 using Intermediate **I-15** and **I-36**. ES/MS m/z: 617.0 (M+H⁺). 1H NMR (400 MHz, DMSO-d6) δ 8.17 (t, J = 2.4 Hz, 1H), 8.05 (s, 1H), 7.94 (t, J = 7.9 Hz, 1H), 7.85 (dd, J = 10.6, 6.4 Hz, 1H), 7.65 - 7.58 (m, 1H), 7.57 - 7.23 (m, 3H), 7.04 (d, J = 8.3 Hz, 1H), 5.84 (s, 2H), 5.07 (d, J = 6.9 Hz, 1H), 4.74 (dd, J = 15.6, 7.0 Hz, 1H), 4.61 (d, J = 15.3 Hz, 1H), 4.57 - 4.41 (m, 3H), 4.35 (dt, J = 9.1, 6.0 Hz, 1H), 2.80 - 2.65 (m, 1H), 2.43 - 2.33 (m, 1H).

### Example 327: (S)-2-(2,5-difluoro-4-(6-((5-methylthiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-methylthiazol-2-yl)methoxy)pyridin-2-yl)benzyl)-1-(oxetan-2-ylmethyl)-1H-benzo[d]imidazole-6-carboxylic acid was prepared in a manner as described in Procedure 1 using Intermediate **I-9** and 2-(chloromethyl)5-methylthiazole. ES/MS m/z: 563.0 (M+H⁺). 1HNMR (400 MHz, DMSO-d6) δ 8.25 (s, 1H), 7.96 - 7.84 (m, 2H), 7.79 (dd, J = 8.4, 1.5 Hz, 1H), 7.59 (d, J = 8.4 Hz, 1H), 7.56 (d, J = 7.2 Hz, 1H), 7.49 (d, J = 1.4 Hz, 1H), 7.39 (dd, J = 11.6, 6.0 Hz, 1H), 6.97 (d, J = 8.2 Hz, 1H), 5.70 (s, 2H), 5.07 (qd, J = 7.1, 2.6 Hz, 1H), 4.75 (dd, J = 15.6, 7.0 Hz, 1H), 4.62 (dd, J = 15.6, 2.8 Hz, 1H), 4.57 - 4.41 (m, 3H), 4.36 (dt, J = 9.0, 5.9 Hz, 1H), 2.80 - 2.67 (m, 1H), 2.46 - 2.31 (m, 4H).

### Example 332: (S)-2-(4-(6-((5,6-dihydro-4H-cyclopenta[d]thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5,6-dihydro-4H-cyclopenta[d]thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 1 using Intermediate **I-9** and **I-1392.** ES/MS m/z: 589.2 (M+H⁺). 1H NMR (400 MHz, DMSO-d6) δ 8.26 (d, J = 1.5 Hz, 1H), 7.95 - 7.87 (m, 2H), 7.79 (dd, J = 8.4, 1.6 Hz, 1H), 7.61 (d, J = 8.4 Hz, 1H), 7.56 (d, J = 7.2 Hz, 1H), 7.39 (dd, J = 11.6, 6.0 Hz, 1H), 6.97 (d, J = 8.3 Hz, 1H), 5.70 (s, 2H), 5.19 - 4.93 (m, 1H), 4.76 (dd, J = 15.6, 7.1 Hz, 1H), 4.63 (dd, J = 15.6, 2.7 Hz, 1H), 4.58 - 4.41 (m, 3H), 4.36 (dt, J = 9.0, 5.9 Hz, 1H), 2.85 (t, J = 7.2 Hz, 2H), 2.80 - 2.66 (m, 3H), 2.47 - 2.29 (m, 3H).

### Example 354: (S)-2-(4-(6-((2,4-dimethylthiazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2,4-dimethylthiazol-5-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1 using Intermediate **I-9** and 5-(chloromethyl)-2,4-dimethylthiazole. ES/MS m/z: 577.2 (M+H⁺). 1H NMR (400 MHz, DMSO) δ 12.79 (s, 1H), 8.26 (d, J = 1.6 Hz, 1H), 7.95 (dd, J = 10.5, 6.4 Hz, 1H), 7.86 (t, J = 7.9 Hz, 1H), 7.80 (dd, J = 8.5, 1.5 Hz, 1H), 7.61 (d, J = 8.4 Hz, 1H), 7.52 (dd, J = 7.6, 1.8 Hz, 1H), 7.40 (dd, J = 11.5, 6.0 Hz, 1H), 6.88 (d, J = 8.3 Hz, 1H), 5.60 (s, 2H), 5.08 (qd, J = 7.0, 2.7 Hz, 1H), 4.76 (dd, J = 15.5, 7.0 Hz, 1H), 4.63 (dd, J = 15.5, 2.8 Hz, 1H), 4.52 (s, 1H), 4.59 - 4.47 (m, 1H), 4.46 (d, J = 16.8 Hz, 1H), 4.36 (dt, J = 8.9, 5.9 Hz, 1H), 2.73 (ddd, J = 11.5, 8.4, 6.2 Hz, 1H), 2.55 (s, 3H), 2.46 - 2.38 (m, 1H), 2.37 (s, 3H).

### Example 374: (S)-2-(4-(6-((6-carbamoylpyridin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-carbamoylpyridin-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 1 using Intermediate **I-9** and 2-(bromomethyl)-6-cyanopyridine. ES/MS m/z: 586.2 (M+H⁺). 1HNMR (400 MHz, Methanol-d4) δ 8.30 (d, J = 1.4 Hz, 1H), 8.04 (d, J = 7.7 Hz, 1H), 8.01 - 7.90 (m, 2H), 7.82 (t, J = 7.8 Hz, 1H), 7.74 - 7.60 (m, 3H), 7.56 (d, J = 7.4 Hz, 1H), 7.15 (dd, J = 11.5, 6.0 Hz, 1H), 6.98 (d, J = 8.2 Hz, 1H), 5.66 (s, 2H), 5.29 - 5.09 (m, 1H), 4.71 (dd, J = 15.7, 6.9 Hz, 1H), 4.67 - 4.37 (m, 5H), 2.86 - 2.63 (m, 1H), 2.49 (t, J = 9.8 Hz, 1H).

### Example 389: (S)-4-fluoro-1-(oxetan-2-ylmethyl)-2-((2,4',5-trifluoro-3'-((5-methoxy-1,3,4-thiadiazol-2-yl)methoxy)-[1,1'-biphenyl]-4-yl)methyl)-1H-benzo[d]imidazole-6-carboxylic acid

(S)-4-fluoro-1-(oxetan-2-ylmethyl)-2-((2,4',5-trifluoro-3'-((5-methoxy-1,3,4-thiadiazol-2-yl)methoxy)-[1,1'-biphenyl]-4-yl)methyl)-1H-benzo[d]imidazole-6-carboxylic acid was prepared in a manner as described in Procedure 1 using Intermediate **I-1279** and 2-(chloromethyl)-5-methoxy-1,3,4-thiadiazole. ES/MS m/z: 615.7 (M+H⁺). 1H NMR (400 MHz, DMSO-d6) δ 8.16 (d, J = 1.2 Hz, 1H), 7.60 (dd, J = 8.3, 2.0 Hz, 1H), 7.56 - 7.48 (m, 2H), 7.44 - 7.35 (m, 2H), 7.32 - 7.23 (m, 1H), 5.59 (s, 2H), 5.08 (qd, J = 7.1, 2.6 Hz, 1H), 4.80 (dd, J = 15.6, 7.1 Hz, 1H), 4.66 (dd, J = 15.5, 2.7 Hz, 1H), 4.59 - 4.42 (m, 3H), 4.36 (dt, J = 9.0, 6.0 Hz, 1H), 4.15 (s, 3H), 2.79 - 2.64 (m, 1H), 2.45 - 2.31 (m, 1H).

### Example 397: (S)-1-(4,4-dimethyltetrahydrofuran-3-yl)-2-(4-(6-((5-ethoxy-1,3,4-thiadiazol-2-yl)methoxy)-5-fluoropyridin-2-yl)-2-fluorobenzyl)-1H-benzo[d]imidazole-6-carboxylic acid

(S)-1-(4,4-dimethyltetrahydrofuran-3-yl)-2-(4-(6-((5-ethoxy-1,3,4-thiadiazol-2-yl)methoxy)-5-fluoropyridin-2-yl)-2-fluorobenzyl)-1H-benzo[d]imidazole-6-carboxylic acid was prepared in a manner as described in Procedure 1 using Intermediate **I-1327** and 2-(chloromethyl)-5-ethoxy-1,3,4-thiadiazole. ES/MS m/z: 622.8 (M+H⁺). 1H NMR (400 MHz, Methanol-d4) δ 8.89 (s, 1H), 8.18 (dd, J = 8.6, 1.4 Hz, 1H), 8.11 - 7.90 (m, 2H), 7.79 (d, J = 8.6 Hz, 1H), 7.74 - 7.62 (m, 2H), 7.56 (t, J = 8.0 Hz, 1H), 5.82 (s, 2H), 5.12 (d, J = 6.6 Hz, 1H), 4.68 (dd, J = 25.0, 2.1 Hz, 2H), 4.61 - 4.45 (m, 3H), 3.99 (d, J = 8.9 Hz, 1H), 3.83 (d, J = 8.9 Hz, 1H), 1.45 (d, J = 7.1 Hz, 3H), 1.35 (s, 3H), 0.72 (s, 3H).

### Example 401: (S)-1-(4,4-dimethyltetrahydrofuran-3-yl)-2-(2-fluoro-4-(6-((5-methoxy-1,3,4-thiadiazol-2-yl)methoxy)pyridin-2-yl)benzyl)-1H-benzo[d]imidazole-6-carboxylic acid

(S)-1-(4,4-dimethyltetrahydrofuran-3-yl)-2-(2-fluoro-4-(6-((5-methoxy-1,3,4-thiadiazol-2-yl)methoxy)pyridin-2-yl)benzyl)-1H-benzo[d]imidazole-6-carboxylic acid was prepared in a manner as described in Procedure 1 using Intermediates **I-1328** and 2-(chloromethyl)-5-ethoxy-1,3,4-thiadiazole. ES/MS m/z: 590.4 (M+H⁺). 1H NMR (400 MHz, Methanol-d4) δ 8.92 (s, 1H), 8.21 (dd, J = 8.6, 1.4 Hz, 1H), 8.11 - 7.97 (m, 2H), 7.93 - 7.76 (m, 2H), 7.72 - 7.50 (m, 2H), 6.91 (dd, J = 8.2, 5.1 Hz, 1H), 5.75 (s, 2H), 5.15 (d, J = 6.4 Hz, 1H), 4.80 - 4.71 (m, 2H), 4.64 (dd, J = 11.7, 1.4 Hz, 1H), 4.51 (dd, J = 11.7, 6.7 Hz, 1H), 4.15 (s, 3H), 4.08 - 3.93 (m, 1H), 3.83 (d, J = 8.9 Hz, 1H), 1.36 (s, 3H), 0.73 (s, 3H).

### Example 47: (S)-2-(4-(6-((5-chlorothiazol-2-yl)methoxy)pyridin-2-yl)-2,5-difluorobenzyl)-1-(oxetan-2-ylmethyl)-1H-benzo[d]imidazole-6-carboxylic acid

### Procedure 2

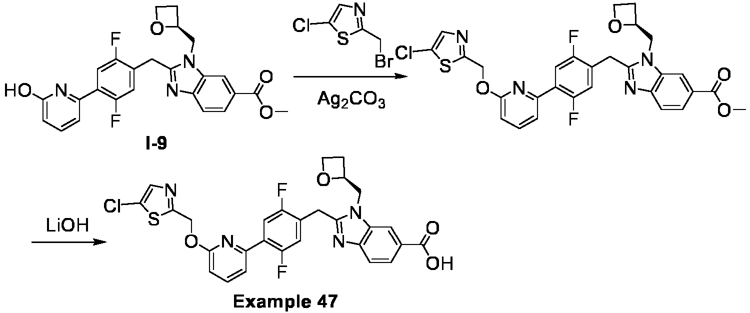

**Methyl (S)-2-(4-(6-((5-chlorothiazol-2-yl)methoxy)pyridin-2-yl)-2,5-difluorobenzyl)-1-(oxetan-2-ylmethyl)-1H-benzo[d]imidazole-6-carboxylate:** To a solution of methyl (S)-2-(2, 5-difluoro-4-(6-hydroxypyridin-2-yl)benzyl)-1-(oxetan-2-ylmethyl)-1H-benzo[d]imidazole-6-carboxylate (50 mg, 0.11 mmol) in toluene (2 mL) , 2-(bromomethyl)-5-chloro-thiazole (69 mg, 0.32 mmol) and silver carbonate (89 mg, 0.32 mmol) were added. The resulting mixture stirred at 80 °C for 16 hrs. Upon completion, the reaction mixture was filtered through celite, the filter washed with EtOAc (10 mL), and the filtrate subsequently concentrated. The crude residue was purified by silica gel chromatography (eluent: EtOAc/hexanes) to give titled product. ES/MS: 597.1 (M+H⁺).

**(S)-2-(4-(6-((5-chlorothiazol-2-yl)methoxy)pyridin-2-yl)-2,5-difluorobenzyl)-1-(oxetan-2-ylmethyl)-1H-benzo[d]imidazole-6-carboxylic acid (Example 47):** Methyl (S)-2-(4-(6-((5-chlorothiazol-2-yl)methoxy)pyridin-2-yl)-2,5-difluorobenzyl)-1-(oxetan-2-ylmethyl)-1H-benzo[d]imidazole-6-carboxylate (76 mg, 0.13 mmol) was taken up in acetonitrile (0.8 mL), to this, aqueous lithium hydroxide (1.0 M, 0.38 mL, 0.38 mmol) was added. The mixture was heated to 100 °C for 4 minutes then cooled to rt and diluted with 5% aqueous citric acid to a pH ~5. The mixture was extracted with EtOAc (2 x 5 mL) and the combined organic extracts dried over MgSO₄ and concentrated in vacuo. The material was purified by RP-HPLC (eluent: MeCN/water gradient with 0.1% TFA), which was then diluted with EtOAc (50 mL) and washed with water (5 × 20 mL). The combined organic extracts were washed with brine (15 mL), dried over MgSO₄, filtered, and concentrated to yield **Example 47** as the free base form. ES/MS: 583.3 (M+H⁺); 1HNMR (400 MHz, DMSO-d6) δ 8.26 (d, J = 1.5 Hz, 1H), 7.97 - 7.83 (m, 3H), 7.79 (dd, J = 8.4, 1.5 Hz, 1H), 7.65 - 7.55 (m, 2H), 7.40 (dd, J = 11.6, 6.0 Hz, 1H), 7.01 (d, J = 8.2 Hz, 1H), 5.73 (s, 2H), 5.07 (qd, J = 7.0, 2.7 Hz, 1H), 4.76 (dd, J = 15.6, 7.1 Hz, 1H), 4.63 (dd, J = 15.6, 2.8 Hz, 1H), 4.58 - 4.42 (m, 3H), 4.36 (dt, J = 9.0, 5.9 Hz, 1H), 2.83 - 2.64 (m, 1H), 2.47 - 2.32 (m, 1H).

### Example 48: (S)-2-(2,5-difluoro-4-(6-((1-methyl-1H-benzo[d]imidazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((1-methyl-1H-benzo[d]imidazol-2-yl)methoxy)pyridin-2-yl)benzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.31 (s, 1H), 7.98 (dd, J = 8.5, 1.5 Hz, 1H), 7.87 (t, J = 7.9 Hz, 1H), 7.76 (d, J = 7.8 Hz, 1H), 7.72 (d, J = 8.0 Hz, 1H), 7.64 (d, J = 8.5 Hz, 1H), 7.60 (dd, J = 10.4, 6.3 Hz, 1H), 7.57 (dd, J = 7.5, 1.5 Hz, 1H), 7.55 - 7.44 (m, 2H), 7.17 (dd, J = 11.3, 6.0 Hz, 1H), 7.04 (d, J = 8.2 Hz, 1H), 5.90 (s, 2H), 5.19 (qd, J = 7.2, 2.5 Hz, 1H), 4.73 (dd, J = 15.7, 6.9 Hz, 1H), 4.67 - 4.54 (m, 3H), 4.51 (s, 1H), 4.46 - 4.38 (m, 1H), 4.07 (s, 3H), 2.78 (dtd, J = 11.5, 8.1, 6.0 Hz, 1H), 2.48 (ddt, J = 11.5, 9.3, 7.1 Hz, 1H).

### Example 49: (S)-2-(4-(6-((5-chloro-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-chloro-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1H NMR (400 MHz, DMSO) δ 12.78 (s, 1H), 8.25 (s, 1H), 8.02 - 7.84 (m, 2H), 7.79 (dd, J = 8.4, 1.6 Hz, 1H), 7.65 - 7.56 (m, 2H), 7.40 (dd, J = 11.6, 6.0 Hz, 1H), 7.02 (d, J = 8.2 Hz, 1H), 5.90 (s, 2H), 5.76 (s, 1H), 5.07 (d, J = 7.1 Hz, 1H), 4.76 (dd, J = 15.5, 7.0 Hz, 1H), 4.67 - 4.59 (m, 1H), 4.58 - 4.41 (m, 3H), 4.35 (dt, J = 9.0, 5.9 Hz, 1H), 2.81 - 2.65 (m, 1H), 2.44 - 2.34 (m, 1H).

### Example 50: (S)-2-(4-(6-((2-chlorothiazol-4-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-chlorothiazol-4-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.35 (d, J = 1.5 Hz, 1H), 7.92 - 7.85 (m, 4H), 7.74 (s, 1H), 7.66 (d, J = 8.4 Hz, 1H), 7.53 (dd, J = 7.5, 1.7 Hz, 1H), 7.43 (dd, J = 11.4, 6.0 Hz, 1H), 6.96 (d, J = 8.2 Hz, 1H), 5.48 (s, 2H), 5.09 (qd, J = 7.1, 2.7 Hz, 1H), 4.83 (dd, J = 15.5, 7.2 Hz, 1H), 4.69 (dd, J = 15.5, 2.7 Hz, 1H), 4.64 - 4.54 (m, 2H), 4.52 - 4.48 (m, 1H), 4.38 (dt, J = 9.0, 6.0 Hz, 1H), 2.79 - 2.67 (m, 1H), 2.41 (ddt, J = 11.2, 8.9, 6.9 Hz, 1H).

### Example 51: (S)-2-(4-(6-((5-bromo-4-fluorothiophen-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-bromo-4-fluorothiophen-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.26 (d, J = 1.5 Hz, 1H), 7.98 - 7.86 (m, 2H), 7.80 (dd, J = 8.4, 1.6 Hz, 1H), 7.61 (d, J = 8.4 Hz, 1H), 7.56 (dd, J = 7.4, 1.6 Hz, 1H), 7.41 (dd, J = 11.6, 6.1 Hz, 1H), 7.31 - 7.21 (m, 1H), 6.93 (d, J = 8.2 Hz, 1H), 5.59 (s, 2H), 5.08 (qd, J = 7.0, 2.7 Hz, 1H), 4.76 (dd, J = 15.6, 7.0 Hz, 1H), 4.63 (dd, J = 15.6, 2.8 Hz, 1H), 4.59 - 4.42 (m, 3H), 4.36 (dt, J = 9.0, 5.9 Hz, 1H), 2.81 - 2.65 (m, 1H), 2.47 - 2.35 (m, 1H).

### Example 52: (S)-2-(4-(6-((5-cyano-3-fluorothiophen-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyano-3-fluorothiophen-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.26 (d, J = 1.5 Hz, 1H), 8.02 (s, 1H), 7.96 (dd, J = 10.6, 6.6 Hz, 1H), 7.91 (t, J = 7.9 Hz, 1H), 7.79 (dd, J = 8.4, 1.6 Hz, 1H), 7.61 (d, J = 8.5 Hz, 1H), 7.58 (dd, J = 7.5, 1.7 Hz, 1H), 7.41 (dd, J = 11.6, 6.1 Hz, 1H), 6.95 (d, J = 8.3 Hz, 1H), 5.70 (s, 2H), 5.08 (qd, J = 7.0, 2.7 Hz, 1H), 4.76 (dd, J = 15.6, 7.1 Hz, 1H), 4.63 (dd, J = 15.5, 2.8 Hz, 1H), 4.58 - 4.43 (m, 3H), 4.36 (dt, J = 9.0, 5.9 Hz, 1H), 2.72 (dtd, J = 11.2, 8.2, 6.2 Hz, 1H), 2.39 (ddt, J = 11.2, 9.0, 6.9 Hz, 1H).

### Example 53: (S)-2-(2,5-difluoro-4-(6-((4-(trifluoromethyl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((4-(trifluoromethyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.51 (d, J = 1.0 Hz, 1H), 8.25 (d, J = 1.5 Hz, 1H), 7.94 (t, J = 7.9 Hz, 1H), 7.85 (dd, J = 10.5, 6.5 Hz, 1H), 7.79 (dd, J = 8.4, 1.6 Hz, 1H), 7.59 (dd, J = 8.1, 2.5 Hz, 2H), 7.39 (dd, J = 11.6, 6.0 Hz, 1H), 7.05 (d, J = 8.3 Hz, 1H), 5.84 (s, 2H), 5.07 (qd, J = 7.1, 2.7 Hz, 1H), 4.75 (dd, J = 15.6, 7.1 Hz, 1H), 4.62 (dd, J = 15.5, 2.8 Hz, 1H), 4.57 - 4.41 (m, 3H), 4.36 (dt, J = 9.0, 5.9 Hz, 1H), 2.77 - 2.68 (m, 1H), 2.44 - 2.35 (m, 1H).

### Example 54: (S)-2-(2,5-difluoro-4-(6-((5-(trifluoromethyl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(trifluoromethyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.47 (d, J = 1.4 Hz, 1H), 8.25 (d, J = 1.5 Hz, 1H), 7.95 (t, J = 7.9 Hz, 1H), 7.87 - 7.75 (m, 2H), 7.66 - 7.53 (m, 2H), 7.39 (dd, J = 11.6, 6.0 Hz, 1H), 7.06 (d, J = 8.2 Hz, 1H), 5.88 (s, 2H), 5.07 (qd, J = 7.1, 2.7 Hz, 1H), 4.75 (dd, J = 15.6, 7.0 Hz, 1H), 4.62 (dd, J = 15.6, 2.8 Hz, 1H), 4.57 - 4.40 (m, 3H), 4.35 (dt, J = 8.9, 5.9 Hz, 1H), 2.72 (dtd, J = 10.8, 8.1, 6.1 Hz, 1H), 2.39 (ddt, J = 11.3, 9.0, 7.0 Hz, 1H).

### Example 55: (S)-2-(2,5-difluoro-4-(6-((4-(trifluoromethyl)thiophen-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((4-(trifluoromethyl)thiophen-2-yl)methoxy)pyridin-2-yl)benzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.30 - 8.18 (m, 2H), 7.95 (dd, J = 10.5, 6.5 Hz, 1H), 7.90 (t, J = 7.9 Hz, 1H), 7.80 (dd, J = 8.4, 1.5 Hz, 1H), 7.60 (d, J = 8.4 Hz, 1H), 7.56 (d, J = 8.8 Hz, 2H), 7.41 (dd, J = 11.6, 6.1 Hz, 1H), 6.94 (d, J = 8.2 Hz, 1H), 5.70 (s, 2H), 5.08 (qd, J = 7.1, 2.7 Hz, 1H), 4.76 (dd, J = 15.6, 7.1 Hz, 1H), 4.63 (dd, J = 15.6, 2.8 Hz, 1H), 4.59 - 4.41 (m, 3H), 4.36 (dt, J = 9.0, 5.9 Hz, 1H), 2.72 (dtd, J = 11.0, 8.1, 6.2 Hz, 1H), 2.48 - 2.30 (m, 1H).

### Example 56: (S)-2-(4-(6-((5-(2,2-difluoroethoxy)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(2,2-difluoroethoxy)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1H NMR (400 MHz, DMSO-d6) δ 8.26 (d, J = 1.5 Hz, 1H), 7.94 (dd, J = 10.5, 6.5 Hz, 1H), 7.88 (t, J = 7.9 Hz, 1H), 7.79 (dd, J = 8.4, 1.6 Hz, 1H), 7.60 (d, J = 8.4 Hz, 1H), 7.54 (dd, J = 7.5, 1.7 Hz, 1H), 7.40 (dd, J = 11.6, 6.1 Hz, 1H), 7.28 (s, 1H), 6.90 (d, J = 8.3 Hz, 1H), 6.29 (tt, J = 54.6, 3.3 Hz, 1H), 5.40 (s, 2H), 5.07 (qd, J = 7.0, 2.7 Hz, 1H), 4.76 (dd, J = 15.6, 7.1 Hz, 1H), 4.63 (dd, J = 15.5, 2.8 Hz, 1H), 4.57 - 4.40 (m, 3H), 4.36 (dt, J = 9.0, 5.9 Hz, 1H), 4.16 (td, J = 16.0, 3.3 Hz, 2H), 2.72 (dtd, J = 11.3, 8.3, 6.4 Hz, 1H), 2.44 - 2.28 (m, 1H).

### Example 57: (S)-2-(2,5-difluoro-4-(6-((5-(2,2,2-trifluoroethoxy)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(2,2,2-trifluoroethoxy)thiazol-2-yl)methoxy)pyridin-2-yl)benzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.26 (d, J = 1.5 Hz, 1H), 7.98 - 7.84 (m, 2H), 7.79 (dd, J = 8.4, 1.5 Hz, 1H), 7.60 (d, J = 8.4 Hz, 1H), 7.55 (dd, J = 7.5, 1.7 Hz, 1H), 7.40 (dd, J = 11.5, 6.1 Hz, 1H), 7.33 (s, 1H), 6.92 (d, J = 8.3 Hz, 1H), 5.42 (s, 2H), 5.08 (dd, J = 8.1, 5.5 Hz, 1H), 4.76 (dd, J = 15.6, 7.1 Hz, 1H), 4.71 - 4.58 (m, 3H), 4.58 - 4.41 (m, 3H), 4.36 (dt, J = 9.1, 5.9 Hz, 1H), 2.81 - 2.69 (m, 1H), 2.40 (q, J = 10.2, 8.8 Hz, 1H).

### Example 58: (S)-2-(4-(6-((5-(difluoromethyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(difluoromethyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.26 (d, J = 1.5 Hz, 1H), 8.17 (t, J = 2.4 Hz, 1H), 7.94 (t, J = 7.9 Hz, 1H), 7.84 (dd, J = 10.5, 6.5 Hz, 1H), 7.79 (dd, J = 8.4, 1.5 Hz, 1H), 7.64 - 7.57 (m, 2H), 7.57 - 7.22 (m, 2H), 7.04 (d, J = 8.3 Hz, 1H), 5.84 (s, 2H), 5.07 (qd, J = 7.0, 2.6 Hz, 1H), 4.75 (dd, J = 15.6, 7.0 Hz, 1H), 4.62 (dd, J = 15.6, 2.8 Hz, 1H), 4.56 - 4.40 (m, 3H), 4.35 (dt, J = 9.0, 5.9 Hz, 1H), 2.81 - 2.63 (m, 1H), 2.40 (ddd, J = 10.4, 8.4, 5.2 Hz, 1H).

### Example 59: (S)-2-(4-(6-((1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1,3,4-thiadiazol-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 2. 1HNMR (400 MHz, DMSO-d6) δ 9.60 (s, 1H), 8.26 (d, J = 1.5 Hz, 1H), 7.98 - 7.84 (m, 2H), 7.79 (dd, J = 8.4, 1.5 Hz, 1H), 7.60 (dd, J = 7.9, 5.4 Hz, 2H), 7.40 (dd, J = 11.6, 6.1 Hz, 1H), 7.02 (d, J = 8.2 Hz, 1H), 5.97 (s, 2H), 5.08 (qd, J = 7.1, 2.7 Hz, 1H), 4.76 (dd, J = 15.6, 7.0 Hz, 1H), 4.63 (dd, J = 15.6, 2.8 Hz, 1H), 4.57 - 4.41 (m, 3H), 4.35 (dt, J = 9.0, 5.9 Hz, 1H), 2.81 - 2.65 (m, 1H), 2.46 - 2.31 (m, 1H).

### Example 60: (S)-2-(2,5-difluoro-4-(6-((3-(trifluoromethyl)isoxazol-5-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-(trifluoromethyl)isoxazol-5-yl)methoxy)pyridin-2-yl)benzyl)-1-(oxetan-2-ylmethyl)-1H-benzo[d]imidazole-6-carboxylic acid was prepared in a manner as described in Procedure 2. 1HNMR (400 MHz, DMSO-d6) δ 8.26 (d, J = 1.5 Hz, 1H), 7.93 (t, J = 7.9 Hz, 1H), 7.83 - 7.74 (m, 2H), 7.59 (dd, J = 10.8, 8.1 Hz, 2H), 7.39 (dd, J = 11.5, 6.0 Hz, 1H), 7.30 (s, 1H), 7.01 (d, J = 8.3 Hz, 1H), 5.74 (s, 2H), 5.08 (qd, J = 7.1, 2.7 Hz, 1H), 4.76 (dd, J = 15.6, 7.0 Hz, 1H), 4.63 (dd, J = 15.6, 2.8 Hz, 1H), 4.57 - 4.40 (m, 3H), 4.36 (dt, J = 9.0, 5.9 Hz, 1H), 2.72 (ddt, J = 13.8, 10.7, 5.3 Hz, 1H), 2.47 - 2.30 (m, 1H).

### Example 61: (S)-2-(2,5-difluoro-4-(6-((5-(trifluoromethyl)furan-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(trifluoromethyl)furan-2-yl)methoxy)pyridin-2-yl)benzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.23 (d, J = 1.4 Hz, 1H), 8.04 - 7.89 (m, 2H), 7.78 (t, J = 7.9 Hz, 1H), 7.62 (d, J = 8.5 Hz, 1H), 7.55 (dd, J = 7.5, 1.6 Hz, 1H), 7.17 (dd, J = 11.5, 6.0 Hz, 1H), 6.95 (dd, J = 3.4, 1.4 Hz, 1H), 6.85 (d, J = 8.2 Hz, 1H), 6.65 (d, J = 3.4 Hz, 1H), 5.50 (s, 2H), 5.21 (qd, J = 7.0, 2.6 Hz, 1H), 4.78 - 4.32 (m, 7H), 2.80 (dtd, J = 11.5, 8.2, 6.1 Hz, 1H), 2.50 (ddt, J = 11.5, 9.0, 7.1 Hz, 1H).

### Example 62: (S)-2-(2,5-difluoro-4-(6-((1-(4-fluorophenyl)-1H-pyrazol-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((1-(4-fluorophenyl)-1H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.58 (d, J = 1.3 Hz, 1H), 8.22 (dd, J = 8.6, 1.4 Hz, 1H), 8.15 (d, J = 2.5 Hz, 1H), 8.08 (dd, J = 10.8, 6.3 Hz, 1H), 7.87 - 7.68 (m, 4H), 7.58 (dd, J = 7.5, 1.5 Hz, 1H), 7.38 (dd, J = 11.2, 6.0 Hz, 1H), 7.28 - 7.09 (m, 2H), 6.91 (d, J = 8.2 Hz, 1H), 6.60 (d, J = 2.5 Hz, 1H), 5.57 (s, 2H), 5.26 (qd, J = 7.4, 2.4 Hz, 1H), 4.99 (dd, J = 15.5, 7.5 Hz, 1H), 4.87 - 4.64 (m, 4H), 4.54 (dt, J = 9.2, 5.9 Hz, 1H), 2.98 - 2.76 (m, 1H), 2.68 - 2.41 (m, 1H).

### Example 63: (S)-2-(4-(6-((5-cyclopropyloxazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyclopropyloxazol-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.57 (d, J = 1.3 Hz, 1H), 8.21 (dd, J = 8.6, 1.4 Hz, 1H), 7.94 (dd, J = 10.8, 6.3 Hz, 1H), 7.87 - 7.69 (m, 2H), 7.60 (dd, J = 7.5, 1.5 Hz, 1H), 7.37 (dd, J = 11.3, 6.0 Hz, 1H), 6.93 (d, J = 8.2 Hz, 1H), 6.79 (s, 1H), 5.47 (s, 2H), 5.26 (qd, J = 7.4, 2.4 Hz, 1H), 4.99 (dd, J = 15.5, 7.5 Hz, 1H), 4.85 - 4.64 (m, 4H), 4.55 (dt, J = 9.3, 6.0 Hz, 1H), 2.87 (dtd, J = 14.1, 8.1, 6.7, 4.1 Hz, 1H), 2.58 (ddt, J = 9.1, 7.2, 2.0 Hz, 1H), 1.95 (td, J = 8.5, 4.2 Hz, 1H), 0.94 (dt, J = 8.5, 3.3 Hz, 2H), 0.81 - 0.65 (m, 2H) **Example 64: (S)-2-(4-(6-((1-(2,2-difluoroethyl)-1H-pyrazol-3-yl)methoxy)pyridin-2-yl)-2,5-difluorobenzyl)-1-(oxetan-2-ylmethyl)-1H-benzo[d]imidazole-6-carboxylic acid**

(S)-2-(4-(6-((1-(2,2-difluoroethyl)-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.56 (s, 1H), 8.20 (dd, J = 8.6, 1.4 Hz, 1H), 8.02 (dd, J = 10.8, 6.3 Hz, 1H), 7.87 - 7.74 (m, 2H), 7.68 (d, J = 2.3 Hz, 1H), 7.56 (dd, J = 7.5, 1.5 Hz, 1H), 7.36 (dd, J = 11.3, 6.0 Hz, 1H), 6.87 (d, J = 8.2 Hz, 1H), 6.44 (d, J = 2.3 Hz, 1H), 6.18 (t, J = 3.9 Hz, 1H), 5.47 (s, 2H), 5.26 (qd, J = 7.4, 2.4 Hz, 1H), 4.97 (dd, J = 15.5, 7.5 Hz, 1H), 4.87 - 4.76 (m, 4H), 4.64 - 4.48 (m, 4H), 2.96 - 2.77 (m, 1H), 2.67 - 2.45 (m, 1H).

### Example 65: (S)-2-(4-(6-((5,6-dihydro-4H-pyrrolo[1,2-b]pyrazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5,6-dihydro-4H-pyrrolo[1,2-b]pyrazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.60 (s, 1H), 8.23 (dd, J = 8.5, 1.3 Hz, 1H), 8.01 (dd, J = 10.8, 6.3 Hz, 1H), 7.79 (dt, J = 7.9, 3.6 Hz, 2H), 7.60 - 7.48 (m, 1H), 7.39 (dd, J = 11.2, 6.0 Hz, 1H), 6.85 (d, J = 8.2 Hz, 1H), 6.11 (s, 1H), 5.41 (s, 2H), 5.27 (dt, J = 7.3, 3.7 Hz, 1H), 5.02 (dd, J = 15.5, 7.6 Hz, 1H), 4.90 - 4.77 (m, 3H), 4.70 (td, J = 8.0, 6.4 Hz, 1H), 4.56 (dt, J = 9.3, 6.0 Hz, 1H), 4.12 (t, J = 7.2 Hz, 2H), 2.89 (q, J = 7.0 Hz, 3H), 2.61 (h, J = 7.3 Hz, 3H).

### Example 66: (S)-2-(2,5-difluoro-4-(6-((1-(2,2,2-trifluoroethyl)-1H-pyrazol-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((1-(2,2,2-trifluoroethyl)-1H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 2. 1HNMR (400 MHz, Methanol-d4) δ 8.58 (s, 1H), 8.22 (dd, J = 8.6, 1.4 Hz, 1H), 8.03 (dd, J = 10.8, 6.3 Hz, 1H), 7.90 - 7.67 (m, 3H), 7.65 - 7.47 (m, 1H), 7.38 (dd, J = 11.2, 6.0 Hz, 1H), 6.89 (d, J = 8.2 Hz, 1H), 6.49 (d, J = 2.4 Hz, 1H), 5.49 (s, 2H), 5.35 - 5.17 (m, 1H), 5.08 - 4.92 (m, 3H), 4.86 - 4.78 (m, 3H), 4.78 - 4.64 (m, 1H), 4.63 - 4.47 (m, 1H), 3.04 - 2.74 (m, 1H), 2.59 (s, 1H).

### Example 67: (S)-2-(4-(6-((5-cyclopropyl-1,3,4-oxadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyclopropyl-1,3,4-oxadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.58 (s, 1H), 8.22 (d, J = 8.5 Hz, 1H), 8.03 - 7.93 (m, 1H), 7.87 (d, J = 7.9 Hz, 1H), 7.78 (d, J = 8.6 Hz, 1H), 7.64 (d, J = 7.4 Hz, 1H), 7.38 (dd, J = 11.3, 6.0 Hz, 1H), 6.97 (d, J = 8.2 Hz, 1H), 5.63 (s, 2H), 5.27 (qd, J = 7.3, 2.3 Hz, 1H), 5.00 (dd, J = 15.5, 7.6 Hz, 1H), 4.85 - 4.65 (m, 3H), 4.55 (dt, J = 9.2, 5.9 Hz, 1H), 3.12 (q, J = 8.8, 7.4 Hz, 1H), 2.96 - 2.77 (m, 1H), 2.69 - 2.46 (m, 1H), 2.35 - 2.04 (m, 2H), 1.17 (dt, J = 7.2, 3.4 Hz, 2H), 1.07 (p, J = 4.6 Hz, 2H.

### Example 68: (S)-2-(2,5-difluoro-4-(6-((5-methoxy-1-methyl-1H-pyrazol-3-yl)methoxy)pyridin-2-yl)benzyl)-1-(oxetan-2-ylmethyl)-1H-benzo [d] imidazole-6-carboxylic acid

(S)-2-(2, 5-difluoro-4-(6-((5-methoxy-1-methyl-1 H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 2. 1HNMR (400 MHz, Methanol-d4) δ 8.65 - 8.53 (m, 1H), 8.23 (dd, J = 8.6, 1.4 Hz, 1H), 8.02 (dd, J = 10.9, 6.4 Hz, 1H), 7.88 - 7.70 (m, 2H), 7.57 (dd, J = 7.2, 1.6 Hz, 1H), 7.39 (dd, J = 11.2, 6.1 Hz, 1H), 6.94 - 6.80 (m, 1H), 5.79 (s, 1H), 5.34 (s, 2H), 5.27 (qd, J = 7.5, 2.4 Hz, 1H), 5.01 (dd, J = 15.5, 7.6 Hz, 1H), 4.87 - 4.65 (m, 4H), 4.55 (dt, J = 9.2, 6.0 Hz, 1H), 3.92 (s, 3H), 3.62 (s, 3H), 2.88 (ddt, J = 10.2, 5.5, 2.8 Hz, 1H), 2.68 - 2.47 (m, 1H).

### Example 69: (S)-2-(4-(6-((5,6-dihydro-4H-pyrrolo[1,2-b]pyrazol-2-yl)methoxy)pyridin-2-yl)-3-fluorobenzyl)-4-fluoro-1-(oxetan-2-ylmethyl)-1H-benzo[d]imidazole-6-carboxylic acid

(S)-2-(4-(6-((5,6-dihydro-4H-pyrrolo[1,2-b]pyrazol-2-yl)methoxy)pyridin-2-yl)-3-fluorobenzyl)-4-fluoro-1-(oxetan-2-ylmethyl)-1H-benzo[d]imidazole-6-carboxylic acid was prepared in a manner as described in Procedure 2. 1H NMR (400 MHz, Methanol-d4) δ 8.23 (d, J = 1.2 Hz, 1H), 8.12 (t, J = 8.2 Hz, 1H), 7.84 - 7.67 (m, 2H), 7.47 (dd, J = 7.5, 1.8 Hz, 1H), 7.35 - 7.08 (m, 2H), 6.80 (d, J = 8.2 Hz, 1H), 6.12 (s, 1H), 5.40 (s, 2H), 5.14 - 5.01 (m, 1H), 4.81 - 4.52 (m, 5H), 4.47 (d, J = 9.2 Hz, 1H), 4.11 (t, J = 7.2 Hz, 2H), 2.90 (t, J = 7.3 Hz, 2H), 2.76 (dtd, J = 11.5, 8.2, 6.0 Hz, 1H), 2.69 - 2.55 (m, 2H), 2.47 (ddt, J = 11.6, 9.2, 7.1 Hz, 1H).

### Example 70: (S)-2-(2,5-difluoro-4-(6-((4,5,6,7-tetrahydropyrazolo[1,5-a]pyridin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((4,5,6,7-tetrahydropyrazolo[1,5-a]pyridin-2-yl)methoxy)pyridin-2-yl)benzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.62 (d, J = 1.4 Hz, 1H), 8.25 (dd, J = 8.6, 1.4 Hz, 1H), 8.00 (dd, J = 10.8, 6.3 Hz, 1H), 7.85 - 7.73 (m, 2H), 7.56 (dd, J = 7.5, 1.6 Hz, 1H), 7.41 (dd, J = 11.2, 6.1 Hz, 1H), 6.86 (d, J = 8.2 Hz, 1H), 6.13 (s, 1H), 5.41 (s, 2H), 5.28 (qd, J = 7.6, 2.4 Hz, 1H), 5.04 (dd, J = 15.4, 7.6 Hz, 1H), 4.88 - 4.80 (m, 3H), 4.71 (ddd, J = 8.5, 7.4, 5.9 Hz, 1H), 4.56 (dt, J = 9.2, 6.0 Hz, 1H), 4.12 (t, J = 6.1 Hz, 2H), 2.89 (dtd, J = 11.5, 8.2, 6.1 Hz, 1H), 2.81 (t, J = 6.4 Hz, 2H), 2.60 (ddt, J = 11.6, 9.2, 7.2 Hz, 1H), 2.14 - 2.00 (m, 2H), 1.93 - 1.79 (m, 2H).

### Example 71: (S)-2-(2,5-difluoro-4-(6-((1-methyl-5-(trifluoromethyl)-1H-pyrazol-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((1-methyl-5-(trifluoromethyl)-1H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.55 (d, J = 1.4 Hz, 1H), 8.19 (dd, J = 8.6, 1.4 Hz, 1H), 8.01 (dd, J = 10.9, 6.3 Hz, 1H), 7.89 - 7.66 (m, 2H), 7.57 (dd, J = 7.5, 1.6 Hz, 1H), 7.36 (dd, J = 11.3, 6.0 Hz, 1H), 6.88 (d, J = 8.2 Hz, 1H), 6.83 (s, 1H), 5.46 (s, 2H), 5.26 (qd, J = 7.4, 2.4 Hz, 1H), 4.97 (dd, J = 15.5, 7.5 Hz, 1H), 4.87 - 4.63 (m, 4H), 4.54 (dt, J = 9.2, 6.0 Hz, 1H), 4.01 (d, J = 1.0 Hz, 3H), 2.87 (dtd, J = 11.5, 8.2, 6.1 Hz, 1H), 2.67 - 2.45 (m, 1H).

### Example 72: (S)-2-(4-(6-((1,5-dimethyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1, 5-dimethyl-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.60 (d, J = 1.4 Hz, 1H), 8.24 (dd, J = 8.6, 1.4 Hz, 1H), 8.02 (dd, J = 10.8, 6.3 Hz, 1H), 7.87 - 7.72 (m, 2H), 7.56 (dd, J = 7.5, 1.6 Hz, 1H), 7.39 (dd, J = 11.2, 6.0 Hz, 1H), 6.86 (d, J = 8.2 Hz, 1H), 6.16 (s, 1H), 5.51 (s, 1H), 5.38 (s, 2H), 5.28 (qd, J = 7.4, 2.4 Hz, 1H), 5.02 (dd, J = 15.5, 7.6 Hz, 1H), 4.87 - 4.76 (m, 2H), 4.71 (ddd, J = 8.4, 7.4, 5.9 Hz, 1H), 4.56 (dt, J = 9.1, 6.0 Hz, 1H), 3.78 (s, 3H), 2.98 - 2.77 (m, 1H), 2.69 - 2.50 (m, 1H), 2.29 (s, 3H).

### Example 73: (S)-2-(2,5-difluoro-4-(6-((1-methyl-1,4,5,6-tetrahydrocyclopenta[c]pyrazol-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((1-methyl-1,4,5,6-tetrahydrocyclopenta[c]pyrazol-3-yl)methoxy)pyridin-2-yl)benzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.58 (d, J = 1.6 Hz, 1H), 8.21 (dd, J = 8.6, 1.4 Hz, 1H), 7.96 (dd, J = 10.8, 6.3 Hz, 1H), 7.87 - 7.71 (m, 2H), 7.55 (dd, J = 7.5, 1.6 Hz, 1H), 7.38 (dd, J = 11.2, 6.0 Hz, 1H), 6.86 (d, J = 8.2 Hz, 1H), 5.39 (s, 2H), 5.26 (qd, J = 7.5, 2.4 Hz, 1H), 4.99 (dd, J = 15.5, 7.6 Hz, 1H), 4.88 - 4.64 (m, 4H), 4.55 (dt, J = 9.2, 6.0 Hz, 1H), 3.76 (s, 3H), 2.88 (dtd, J = 11.5, 8.2, 6.1 Hz, 1H), 2.72 (dd, J = 7.9, 6.3 Hz, 2H), 2.64 - 2.42 (m, 5H).

### Example 74: (S)-2-(4-(6-((5,5-difluoro-5,6-dihydro-4H-pyrrolo[1,2-b]pyrazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5,5-difluoro-5,6-dihydro-4H-pyrrolo[1,2-b]pyrazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.63 - 8.52 (m, 1H), 8.21 (dd, J = 8.6, 1.4 Hz, 1H), 8.00 (dd, J = 10.8, 6.3 Hz, 1H), 7.86 - 7.69 (m, 2H), 7.56 (dd, J = 7.5, 1.7 Hz, 1H), 7.37 (dd, J = 11.2, 6.0 Hz, 1H), 6.86 (d, J = 8.3 Hz, 1H), 6.26 (s, 1H), 5.46 (s, 2H), 5.27 (qd, J = 7.4, 2.4 Hz, 1H), 4.98 (dd, J = 15.5, 7.5 Hz, 1H), 4.87 - 4.66 (m, 4H), 4.63 - 4.46 (m, 3H), 3.53 (td, J = 13.9, 0.8 Hz, 2H), 2.88 (dtd, J = 11.6, 8.2, 6.1 Hz, 1H), 2.58 (ddt, J = 11.5, 9.2, 7.2 Hz, 1H).

### Example 75: (S)-2-(4-(6-((4',6'-dihydrospiro[cyclopropane-1,5'-pyrrolo[1,2-b]pyrazol]-2'-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4',6'-dihydrospiro[cyclopropane-1,5'-pyrrolo[1,2-b]pyrazol]-2'-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.58 (d, J = 1.3 Hz, 1H), 8.21 (dd, J = 8.6, 1.4 Hz, 1H), 8.00 (dd, J = 10.8, 6.3 Hz, 1H), 7.85 - 7.69 (m, 2H), 7.54 (dd, J = 7.5, 1.6 Hz, 1H), 7.39 (dd, J = 11.2, 6.0 Hz, 1H), 6.85 (d, J = 8.2 Hz, 1H), 6.12 (s, 1H), 5.43 (s, 2H), 5.27 (td, J = 7.3, 2.4 Hz, 1H), 5.08 - 4.98 (m, 1H), 4.92 - 4.77 (m, 3H), 4.70 (td, J = 8.1, 6.1 Hz, 1H), 4.55 (dt, J = 9.2, 6.0 Hz, 1H), 4.04 (s, 2H), 2.97 - 2.76 (m, 3H), 2.58 (ddt, J = 11.6, 9.2, 7.1 Hz, 1H), 0.95 - 0.68 (m, 4H).

### Example 76: (S)-2-(4-(6-((4-chloro-1-ethyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-1-ethyl-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.56 (d, J = 1.3 Hz, 1H), 8.19 (dd, J = 8.6, 1.4 Hz, 1H), 8.08 (dd, J = 10.8, 6.3 Hz, 1H), 7.87 - 7.67 (m, 3H), 7.55 (dd, J = 7.5, 1.6 Hz, 1H), 7.37 (dd, J = 11.2, 6.0 Hz, 1H), 6.85 (d, J = 8.2 Hz, 1H), 5.43 (s, 2H), 5.26 (qd, J = 7.4, 2.4 Hz, 1H), 5.05 - 4.96 (m, 1H), 4.90 - 4.66 (m, 4H), 4.55 (dt, J = 9.3, 6.0 Hz, 1H), 4.16 (q, J = 7.3 Hz, 2H), 2.99 - 2.76 (m, 1H), 2.70 - 2.47 (m, 1H), 1.44 (t, J = 7.3 Hz, 3H).

### Example 77: (S)-2-(4-(6-((1,4-dimethyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1,4-dimethyl-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.60 (d, J = 1.3 Hz, 1H), 8.23 (dd, J = 8.6, 1.4 Hz, 1H), 8.04 (dd, J = 10.8, 6.3 Hz, 1H), 7.91 - 7.67 (m, 2H), 7.54 (dd, J = 7.5, 1.6 Hz, 1H), 7.46 - 7.29 (m, 2H), 6.85 (d, J = 8.3 Hz, 1H), 5.41 (s, 2H), 5.27 (qd, J = 7.5, 2.4 Hz, 1H), 5.02 (dd, J = 15.5, 7.6 Hz, 1H), 4.90 - 4.76 (m, 3H), 4.71 (td, J = 8.1, 6.1 Hz, 1H), 4.56 (dt, J = 9.2, 6.0 Hz, 1H), 3.84 (s, 3H), 2.89 (dtd, J = 11.5, 8.2, 6.1 Hz, 1H), 2.59 (ddt, J = 11.6, 9.1, 7.1 Hz, 1H), 2.10 (s, 3H).

### Example 78: (S)-2-(4-(6-((4-chloro-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 2. 1H NMR (400 MHz, Methanol-d4) δ 8.54 (d, J = 1.3 Hz, 1H), 8.18 (dd, J = 8.6, 1.4 Hz, 1H), 8.08 (dd, J = 10.8, 6.3 Hz, 1H), 7.86 - 7.73 (m, 2H), 7.69 (d, J = 12.6 Hz, 2H), 7.56 (dd, J = 7.5, 1.6 Hz, 1H), 7.35 (dd, J = 11.3, 6.0 Hz, 1H), 6.85 (d, J = 8.2 Hz, 1H), 5.43 (s, 2H), 5.26 (qd, J = 7.4, 2.4 Hz, 1H), 5.00 - 4.94 (m, 1H), 4.79 - 4.65 (m, 3H), 4.61 - 4.44 (m, 3H), 3.86 (s, 3H), 2.87 (ddd, J = 10.3, 5.7, 2.7 Hz, 1H), 2.67 - 2.43 (m, 1H).

### Example 379: (S)-2-(4-(6-((3-chloro-5-((1-methylcyclopropyl)ethynyl)pyridin-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((3-chloro-5-((1-methylcyclopropyl)ethynyl)pyridin-2-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as describe in Procedure 2 using Intermediate **I-1250** and **I-15.** ES/MS m/z: 673.4 (M+H⁺). 1H NMR (400 MHz, MeOD) δ 8.39 (d, J = 1.8 Hz, 1H), 7.99 (d, J = 1.2 Hz, 1H), 7.87 (d, J = 1.8 Hz, 1H), 7.78 (t, J = 7.9 Hz, 1H), 7.74 - 7.61 (m, 2H), 7.53 (d, J = 7.4 Hz, 1H), 7.11 (dd, J = 11.5, 6.0 Hz, 1H), 6.91 (d, J = 8.2 Hz, 1H), 5.66 (s, 2H), 5.18 (d, J = 6.7 Hz, 1H), 4.73 - 4.38 (m, 7H), 2.78 (p, J = 7.5 Hz, 1H), 2.55 - 2.43 (m, 1H).

### Example 380: (S)-2-(4-(6-((3-chloro-5-((1-(fluoromethyl)cyclopropyl)ethynyl)pyridin-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((3-chloro-5-((1-(fluoromethyl)cyclopropyl)ethynyl)pyridin-2-yl)methoxy)pyridin-2-yl)-2, 5-difluorobenzyl)-4-fluoro-1-(oxetan-2-ylmethyl)-1H-benzo[d]imidazole-6-carboxylic acid was prepared in a manner as described in Procedure 2 using Intermediate **I-1251** and **I-15.** ES/MS m/z: 691.9 (M+H⁺). 1H NMR (400 MHz, MeOD) δ 8.44 (d, J = 1.8 Hz, 1H), 7.92 (d, J = 1.8 Hz, 1H), 7.79 (t, J = 7.9 Hz, 1H), 7.76 - 7.61 (m, 2H), 7.54 (d, J = 7.5 Hz, 1H), 7.13 (dd, J = 11.5, 5.9 Hz, 1H), 6.91 (d, J = 8.2 Hz, 1H), 5.67 (s, 2H), 5.17 (d, J = 7.9 Hz, 1H), 4.77 - 4.25 (m, 7H), 2.78 (t, J = 9.4 Hz, 1H), 2.48 (p, J = 8.0 Hz, 1H).

### Example 381: (S)-2-(4-(6-((3-chloro-5-((1-(difluoromethyl)cyclopropyl)ethynyl)pyridin-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((3-chloro-5-((1-(difluoromethyl)cyclopropyl)ethynyl)pyridin-2-yl)methoxy)pyridin-2-yl)-2, 5-difluorobenzyl)-4-fluoro-1-(oxetan-2-ylmethyl)-1H-benzo[d]imidazole-6-carboxylic acid was prepared in a manner as described in Procedure 2 using Intermediate **I-1252** and **I-15.** ES/MS m/z: 709.3 (M+H⁺). 1HNMR (400 MHz, DMSO) δ 8.53 (d, J = 1.8 Hz, 1H), 8.09 (d, J = 1.9 Hz, 1H), 7.96 - 7.78 (m, 2H), 7.65 (dd, J = 10.6, 6.4 Hz, 1H), 7.51 (d, J = 7.4 Hz, 1H), 7.44 (d, J = 12.1 Hz, 1H), 7.33 (dd, J = 11.6, 6.1 Hz, 1H), 6.97 (d, J = 8.3 Hz, 1H), 5.64 (s, 2H), 5.06 (s, 1H), 4.76 - 4.27 (m, 5H).

### Example 79: 2-[[2,5-difluoro-4-[6-[[5-(1-methylpyrazol-4-yl)thiazol-2-yl]methoxy]-2-pyridyl]phenyl]methyl]-3-[[(2S)-oxetan-2-yl]methyl]benzimidazole-5-carboxylic acid

### Procedure 3

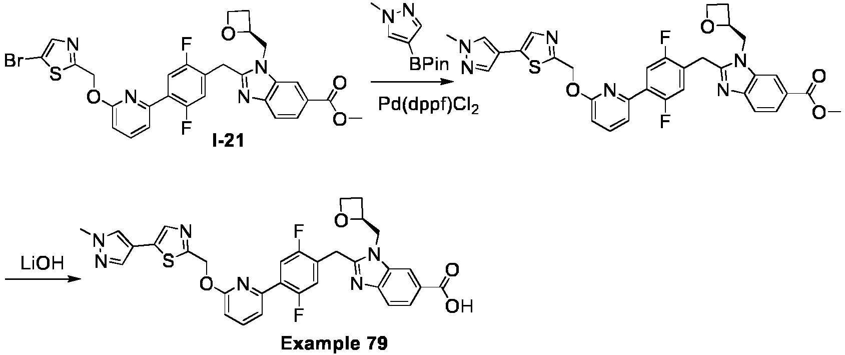

**Methyl 2-[[2,5-difluoro-4-[6-[[5-(1-methylpyrazol-4-yl)thiazol-2-yl]methoxy]-2-pyridyl]phenyl]methyl]-3-[[(2S)-oxetan-2-yl]methyl]benzimidazole-5-carboxylate:** To a suspension of methyl 2-[[4-[6-[(5-bromothiazol-2-yl)methoxy]-2-pyridyl]-2,5-difluorophenyl]methyl]-3-[[(2S)-oxetan-2-yl]methyl]benzimidazole-5-carboxylate I-21 (50 mg, 0.078 mmol), (1-methylpyrazol-4-yl)boronic acid (12 mg, 0.10 mmol), and bis(diphenylphosphino)ferrocene] dichloropalladium(II) (12 mg, 0.0016 mmol) in 1,4-dioxane (1.5 mL) was added aqueous sodium carbonate solution (0.13 mL, 0.2 mmol). The resulting solution was degassed by bubbling argon for 1 min, sealed and heated to 100 °C for 2 hrs. Upon completion of the time, the mixture was poured into water (5 mL) and extracted with EtOAc (2 x 5 mL). The organic layers were combined, washed with brine (5 mL), dried over MgSO₄, filtered, concentrated, and purified by flash chromatography (Eluent: EtOAc/hexane) to give the titled product. ES/MS: 643.2 (M+1).

### 2-[[2,5-difluoro-4-[6-[[5-(1-methylpyrazol-4-yl)thiazol-2-yl]methoxy]-2-pyridyl]phenyl]methyl]-3-[[(2S)-oxetan-2-yl]methyl]benzimidazole-5-carboxylic acid

**(Example 79):** Methyl 2-[[2,5-difluoro-4-[6-[[5-(1-methylpyrazol-4-yl)thiazol-2-yl]methoxy]-2-pyridyl]phenyl]methyl]-3-[[(2S)-oxetan-2-yl]methyl]benzimidazole-5-carboxylate (35 mg, 0.055 mmol) was taken up in acetonitrile (0.4 mL), followed by the addition of aqueous lithium hydroxide (0.3 M, 0.55 mL, 0.16 mmol). The mixture was heated to 100 °C for 4 min then cooled to rt and diluted with 5% aqueous citric acid to a pH ~5. The mixture was extracted with EtOAc (2 × 5 mL) and the combined organic extracts dried over MgSO₄ and concentrated in vacuo. The material was purified by RP-HPLC (eluent: MeCN/water gradient with 0.1% TFA), which was then diluted with EtOAc (50 mL) and washed with water (5 × 20 mL). The combined organic extracts were washed with brine (15 mL), dried over MgSO₄, filtered, and concentrated to yield **Example 79** as the free bass form. ES/MS: 629.2 (M+H⁺); 1H NMR (400 MHz, DMSO-d6) δ 8.26 (d, J = 1.4 Hz, 1H), 8.07 (s, 1H), 7.95 - 7.87 (m, 3H), 7.79 (dd, J = 8.4, 1.5 Hz, 1H), 7.75 (s, 1H), 7.63 - 7.53 (m, 2H), 7.39 (dd, J = 11.6, 6.0 Hz, 1H), 7.00 (d, J = 8.2 Hz, 1H), 5.75 (s, 2H), 5.07 (qd, J = 7.0, 2.7 Hz, 1H), 4.75 (dd, J = 15.6, 7.1 Hz, 1H), 4.62 (dd, J = 15.6, 2.8 Hz, 1H), 4.58 - 4.41 (m, 3H), 4.35 (dt, J = 9.0, 5.9 Hz, 1H), 3.83 (s, 3H), 2.71 (dtd, J = 11.2, 8.1, 6.3 Hz, 1H), 2.39 (ddt, J = 11.3, 9.1, 7.0 Hz, 1H).

### Example 80: (S)-2-(2,5-difluoro-4-(6-((5-(1-(oxetan-3-yl)-1H-pyrazol-4-yl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(1-(oxetan-3-yl)-1H-pyrazol-4-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, Methanol-d4) δ 8.31 (d, J = 1.4 Hz, 1H), 8.10 (s, 1H), 7.99 (dd, J = 8.5, 1.5 Hz, 1H), 7.91 (dd, J = 10.8, 6.4 Hz, 1H), 7.87 - 7.80 (m, 3H), 7.66 (d, J = 8.5 Hz, 1H), 7.61 (dd, J = 7.6, 1.5 Hz, 1H), 7.20 (dd, J = 11.6, 6.0 Hz, 1H), 6.94 (d, J = 8.2 Hz, 1H), 5.78 (s, 2H), 5.56 (p, J = 6.8 Hz, 1H), 5.19 (td, J = 7.4, 4.8 Hz, 1H), 5.04 (d, J = 6.9 Hz, 5H), 4.73 (dd, J = 15.6, 7.0 Hz, 1H), 4.68 - 4.40 (m, 6H), 2.89 - 2.69 (m, 1H), 2.59 - 2.39 (m, 1H).

### Example 81: (S)-2-(2,5-difluoro-4-(6-((3-(trifluoromethyl)isothiazol-5-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-(trifluoromethyl)isothiazol-5-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, Methanol-d4) δ 8.32 (d, J = 1.4 Hz, 1H), 7.99 (dd, J = 8.5, 1.6 Hz, 1H), 7.92 (dd, J = 10.6, 6.4 Hz, 1H), 7.86 (t, J = 7.9 Hz, 1H), 7.75 (s, 1H), 7.68 (d, J = 8.5 Hz, 1H), 7.62 (dd, J = 7.5, 1.5 Hz, 1H), 7.22 (dd, J = 11.5, 6.1 Hz, 1H), 6.94 (d, J = 8.2 Hz, 1H), 5.86 (s, 2H), 5.27 - 5.12 (m, 1H), 4.74 (dd, J = 15.7, 6.9 Hz, 1H), 4.69 - 4.39 (m, 5H), 2.94 - 2.71 (m, 1H), 2.60 - 2.38 (m, 1H).

### Example 82: (S)-2-(2,5-difluoro-4-(6-((1'-methyl-1'H-[1,4'-bipyrazol]-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((1'-methyl-1'H-[1,4'-bipyrazol]-3-yl)methoxy)pyridin-2-yl)benzyl)-1-(oxetan-2-ylmethyl)-1H-benzo[d]imidazole-6-carboxylic acid was prepared in a manner as described in Procedure 3. 1HNMR (400 MHz, Methanol-d4) δ 8.31 (d, J = 1.4 Hz, 1H), 8.05 - 7.94 (m, 3H), 7.91 (d, J = 2.4 Hz, 1H), 7.81 (d, J = 0.8 Hz, 1H), 7.77 (t, J = 7.9 Hz, 1H), 7.67 (d, J = 8.5 Hz, 1H), 7.54 (dd, J = 7.5, 1.5 Hz, 1H), 7.19 (dd, J = 11.5, 6.1 Hz, 1H), 6.86 (d, J = 8.2 Hz, 1H), 6.53 (d, J = 2.4 Hz, 1H), 5.53 (s, 2H), 5.20 (qd, J = 7.0, 2.5 Hz, 1H), 4.83 - 4.34 (m, 7H), 3.91 (s, 3H), 2.96 - 2.63 (m, 1H), 2.63 - 2.32 (m, 1H).

### Example 83: (S)-2-(4-(6-((1'-(difluoromethyl)-1'H-[1,4'-bipyrazol]-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1'-(difluoromethyl)-1'H-[1,4'-bipyrazol]-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, Methanol-d4) δ 8.48 (s, 1H), 8.31 (d, J = 1.4 Hz, 1H), 8.12 (s, 1H), 8.05 (d, J = 2.5 Hz, 1H), 8.02 - 7.93 (m, 2H), 7.78 (t, J = 7.9 Hz, 1H), 7.70 - 7.64 (m, 1H), 7.55 (dd, J = 7.5, 1.5 Hz, 1H), 7.44 (d, J = 59.6 Hz, 1H), 7.19 (dd, J = 11.6, 6.1 Hz, 1H), 6.86 (d, J = 8.2 Hz, 1H), 6.57 (d, J = 2.5 Hz, 1H), 5.55 (s, 2H), 5.19 (tt, J = 7.1, 3.6 Hz, 1H), 4.79 - 4.33 (m, 7H), 2.80 (dtd, J = 11.3, 8.1, 6.0 Hz, 1H), 2.49 (ddt, J = 11.5, 9.1, 7.2 Hz, 1H).

### Example 84: (S)-2-(4-(6-((5-(1-(difluoromethyl)-1H-pyrazol-4-yl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(1-(difluoromethyl)-1H-pyrazol-4-yl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.68 (s, 1H), 8.25 (d, J = 1.5 Hz, 1H), 8.18 (s, 1H), 8.09 (s, 1H), 7.98 - 7.89 (m, 2H), 7.85 - 7.79 (m, 1H), 7.74 (d, J = 44.0 Hz, 1H), 7.59 (t, J = 7.2 Hz, 2H), 7.39 (dd, J = 11.6, 6.0 Hz, 1H), 7.02 (d, J = 8.2 Hz, 1H), 5.79 (s, 2H), 5.07 (qd, J = 7.1, 2.7 Hz, 1H), 4.75 (dd, J = 15.6, 7.0 Hz, 1H), 4.62 (dd, J = 15.6, 2.8 Hz, 1H), 4.56 - 4.41 (m, 3H), 4.35 (dt, J = 9.1, 5.9 Hz, 1H), 2.71 (dtd, J = 11.3, 8.1, 6.1 Hz, 1H), 2.39 (ddt, J = 11.6, 9.3, 7.1 Hz, 1H).

### Example 85: (S)-2-(4-(6-((5-(1-(difluoromethyl)-1H-pyrazol-4-yl)thiazol-2-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((5-(1-(difluoromethyl)-1H-pyrazol-4-yl)thiazol-2-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 3. 1H NMR (400 MHz, DMSO-d6) δ 8.68 (s, 1H), 8.18 (s, 1H), 8.09 (s, 1H), 7.98 - 7.64 (m, 4H), 7.58 (dd, J = 7.5, 1.6 Hz, 1H), 7.45 (d, J = 12.0 Hz, 1H), 7.37 (dd, J = 11.6, 6.1 Hz, 1H), 7.01 (d, J = 8.3 Hz, 1H), 5.79 (s, 2H), 5.06 (dt, J = 9.5, 4.7 Hz, 1H), 4.68 (dd, J = 15.6, 6.8 Hz, 1H), 4.61 - 4.38 (m, 4H), 4.34 (dt, J = 9.1, 5.9 Hz, 1H), 2.75 - 2.64 (m, 1H), 2.41 - 2.31 (m, 1H).

### Example 86: (S)-2-(2,5-difluoro-4-(6-((3-fluoro-5-(1-methyl-1H-pyrazol-4-yl)thiophen-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((3-fluoro-5-(1-methyl-1H-pyrazol-4-yl)thiophen-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.25 (d, J = 1.5 Hz, 1H), 8.04 (s, 1H), 7.99 (dd, J = 10.6, 6.5 Hz, 1H), 7.88 (t, J = 7.9 Hz, 1H), 7.79 (dd, J = 8.4, 1.6 Hz, 1H), 7.71 (s, 1H), 7.60 (d, J = 8.4 Hz, 1H), 7.55 (dd, J = 7.5, 1.7 Hz, 1H), 7.40 (dd, J = 11.6, 6.1 Hz, 1H), 7.11 (s, 1H), 6.91 (d, J = 8.2 Hz, 1H), 5.58 (s, 2H), 5.08 (qd, J = 7.0, 2.7 Hz, 1H), 4.76 (dd, J = 15.6, 7.0 Hz, 1H), 4.63 (dd, J = 15.6, 2.8 Hz, 1H), 4.58 - 4.42 (m, 3H), 4.36 (dt, J = 9.0, 5.9 Hz, 1H), 3.82 (s, 3H), 2.72 (ddt, J = 14.4, 11.4, 7.1 Hz, 1H), 2.46 - 2.29 (m, 1H).

### Example 87: (S)-2-(2,5-difluoro-4-(6-((5-(1-methyl-1H-pyrazol-4-yl)thiazol-2-yl)methoxy)pyridin-2-yl)benzyl)-4-fluoro-1-(oxetan-2-ylmethyl)-1H-benzo [d] imidazole-6-carboxylic acid

(S)-2-(2,5-difluoro-4-(6-((5-(1-methyl-1H-pyrazol-4-yl)thiazol-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 3. 1H NMR (400 MHz, DMSO-d6) δ 8.16 (d, J = 1.3 Hz, 1H), 8.07 (s, 1H), 7.96 - 7.86 (m, 3H), 7.75 (s, 1H), 7.58 (dd, J = 7.6, 1.7 Hz, 1H), 7.51 (dd, J = 11.4, 1.3 Hz, 1H), 7.41 (dd, J = 11.6, 6.1 Hz, 1H), 7.01 (d, J = 8.2 Hz, 1H), 5.75 (s, 2H), 5.08 (dd, J = 8.2, 5.7 Hz, 1H), 4.79 (dd, J = 15.6, 7.1 Hz, 1H), 4.65 (dd, J = 15.7, 2.8 Hz, 1H), 4.58 - 4.40 (m, 3H), 4.35 (dt, J = 9.0, 5.9 Hz, 1H), 3.83 (s, 3H), 2.76 - 2.65 (m, 1H), 2.47 - 2.31 (m, 1H).

### Example 88: (S)-2-(4-(6-((5-(1-(cyclopropylmethyl)-1H-pyrazol-4-yl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(1-(cyclopropylmethyl)-1H-pyrazol-4-yl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.26 (d, J = 1.5 Hz, 1H), 8.14 (s, 1H), 7.91 (q, J = 7.3, 6.8 Hz, 3H), 7.83 - 7.73 (m, 2H), 7.59 (dd, J = 10.7, 7.3 Hz, 2H), 7.39 (dd, J = 11.6, 6.1 Hz, 1H), 7.01 (d, J = 8.3 Hz, 1H), 5.76 (s, 2H), 5.14 - 4.99 (m, 1H), 4.75 (dd, J = 15.6, 7.1 Hz, 1H), 4.62 (dd, J = 15.6, 2.8 Hz, 1H), 4.58 - 4.41 (m, 3H), 4.35 (dt, J = 9.0, 5.9 Hz, 1H), 3.95 (d, J = 7.2 Hz, 2H), 2.88 - 2.61 (m, 1H), 2.46 - 2.28 (m, 1H), 1.24 (td, J = 8.1, 7.6, 4.1 Hz, 1H), 0.58 - 0.45 (m, 2H), 0.36 (dt, J = 6.3, 4.4 Hz, 2H).

### Example 89: (S)-2-(2,5-difluoro-4-(6-((5-(1-methyl-1H-1,2,3-triazol-4-yl)thiazol-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-(1-methyl-1H-1,2,3-triazol-4-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.26 (d, J = 1.5 Hz, 1H), 8.23 (s, 1H), 8.05 (s, 1H), 7.94 (t, J = 7.9 Hz, 1H), 7.89 (dd, J = 10.5, 6.5 Hz, 1H), 7.79 (dd, J = 8.4, 1.6 Hz, 1H), 7.63 - 7.54 (m, 2H), 7.40 (dd, J = 11.5, 6.0 Hz, 1H), 7.04 (d, J = 8.2 Hz, 1H), 5.86 (s, 2H), 5.07 (d, J = 7.4 Hz, 1H), 4.76 (dd, J = 15.6, 7.0 Hz, 1H), 4.68 - 4.57 (m, 1H), 4.56 - 4.39 (m, 3H), 4.35 (dt, J = 9.0, 5.9 Hz, 1H), 4.11 (s, 3H), 2.78 - 2.62 (m, 1H), 2.40 (q, J = 10.2, 8.9 Hz, 1H).

### Example 90: (S)-2-(4-(6-((5-(1-cyclopropyl-1H-pyrazol-4-yl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(1-cyclopropyl-1H-pyrazol-4-yl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.26 (d, J = 1.5 Hz, 1H), 8.17 (s, 1H), 7.96 - 7.85 (m, 3H), 7.79 (dd, J = 8.4, 1.6 Hz, 1H), 7.74 (d, J = 0.8 Hz, 1H), 7.60 (d, J = 8.5 Hz, 1H), 7.57 (dd, J = 7.5, 1.7 Hz, 1H), 7.39 (dd, J = 11.6, 6.0 Hz, 1H), 7.00 (d, J = 8.2 Hz, 1H), 5.75 (s, 2H), 5.15 - 4.98 (m, 1H), 4.75 (dd, J = 15.6, 7.1 Hz, 1H), 4.62 (dd, J = 15.5, 2.8 Hz, 1H), 4.58 - 4.41 (m, 3H), 4.35 (dt, J = 9.0, 5.9 Hz, 1H), 3.72 (tt, J = 7.4, 3.9 Hz, 1H), 2.71 (dd, J = 16.2, 8.5 Hz, 1H), 2.40 (q, J = 10.3, 8.7 Hz, 1H), 1.05 (dq, J = 6.0, 3.9 Hz, 2H), 1.02 - 0.92 (m, 2H).

### Example 91: (S)-2-(2,5-difluoro-4-(6-((5-(1-methyl-1H-pyrazol-4-yl)-1,3,4-thiadiazol-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-(1-methyl-1H-pyrazol-4-yl)-1,3,4-thiadiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.43 (s, 1H), 8.26 (d, J = 1.5 Hz, 1H), 7.98 (d, J = 0.8 Hz, 1H), 7.97 - 7.90 (m, 2H), 7.79 (dd, J = 8.4, 1.5 Hz, 1H), 7.65 - 7.54 (m, 2H), 7.40 (dd, J = 11.6, 6.0 Hz, 1H), 7.02 (d, J = 8.2 Hz, 1H), 5.93 (s, 2H), 5.07 (d, J = 7.0 Hz, 1H), 4.75 (dd, J = 15.6, 7.1 Hz, 1H), 4.62 (d, J = 14.4 Hz, 1H), 4.57 - 4.39 (m, 3H), 4.35 (dt, J = 9.0, 6.0 Hz, 1H), 3.89 (s, 3H), 2.82 - 2.66 (m, 1H), 2.40 (q, J = 10.1, 8.6 Hz, 1H).

### Example 92: (S)-2-(4-(6-((5-(1-(difluoromethyl)-1H-pyrazol-4-yl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(1-(difluoromethyl)-1H-pyrazol-4-yl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, DMSO-d6) δ 9.03 (s, 1H), 8.38 (s, 1H), 8.25 (d, J = 1.5 Hz, 1H), 8.07 - 7.69 (m, 4H), 7.60 (td, J = 5.6, 5.2, 2.9 Hz, 2H), 7.40 (dd, J = 11.6, 6.0 Hz, 1H), 7.03 (d, J = 8.3 Hz, 1H), 5.98 (s, 2H), 5.07 (qd, J = 7.0, 2.7 Hz, 1H), 4.75 (dd, J = 15.6, 7.1 Hz, 1H), 4.62 (dd, J = 15.5, 2.8 Hz, 1H), 4.58 - 4.40 (m, 3H), 4.35 (dt, J = 9.0, 5.9 Hz, 1H), 2.78 - 2.62 (m, 1H), 2.38 (ddt, J = 17.1, 9.2, 4.3 Hz, 1H).

### Example 93: (S)-2-(2,5-difluoro-4-(6-((5-(oxazol-5-yl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(oxazol-5-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1HNMR (400 MHz, DMSO-d6) δ 8.48 (s, 1H), 8.25 (d, J = 1.5 Hz, 1H), 8.18 (s, 1H), 7.94 (t, J = 7.9 Hz, 1H), 7.88 (dd, J = 10.5, 6.5 Hz, 1H), 7.79 (dd, J = 8.4, 1.5 Hz, 1H), 7.64 - 7.55 (m, 3H), 7.39 (dd, J = 11.6, 6.0 Hz, 1H), 7.04 (d, J = 8.2 Hz, 1H), 5.83 (s, 2H), 5.08 (dd, J = 9.6, 6.8 Hz, 1H), 4.75 (dd, J = 15.6, 7.1 Hz, 1H), 4.62 (dd, J = 15.5, 2.7 Hz, 1H), 4.57 - 4.40 (m, 3H), 4.35 (dt, J = 9.0, 5.9 Hz, 1H), 2.76 - 2.68 (m, 1H), 2.40 (q, J = 10.6, 9.2 Hz, 1H).

### Example 94: (S)-2-(2,5-difluoro-4-(6-((5-(1-methyl-1H-pyrazol-5-yl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(1-methyl-1H-pyrazol-5-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1HNMR (400 MHz, DMSO-d6) δ 8.25 (d, J = 1.5 Hz, 1H), 8.10 (s, 1H), 7.99 - 7.85 (m, 2H), 7.79 (dd, J = 8.5, 1.6 Hz, 1H), 7.62 - 7.52 (m, 2H), 7.47 (d, J = 2.0 Hz, 1H), 7.39 (dd, J = 11.5, 6.0 Hz, 1H), 7.03 (d, J = 8.2 Hz, 1H), 6.54 (d, J = 2.0 Hz, 1H), 5.82 (s, 2H), 5.06 (dd, J = 8.1, 5.6 Hz, 1H), 4.75 (dd, J = 15.6, 7.0 Hz, 1H), 4.67 - 4.57 (m, 1H), 4.57 - 4.40 (m, 3H), 4.35 (dt, J = 8.9, 5.9 Hz, 1H), 3.91 (s, 3H), 2.73 (dt, J = 11.5, 7.8 Hz, 1H), 2.44 - 2.31 (m, 1H).

### Example 95: (S)-2-(2,5-difluoro-4-(6-((5-(isothiazol-4-yl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(isothiazol-4-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1HNMR (400 MHz, DMSO-d6) δ 9.29 (s, 1H), 8.97 (s, 1H), 8.26 (d, J = 5.4 Hz, 2H), 7.97 - 7.85 (m, 2H), 7.79 (dd, J = 8.4, 1.5 Hz, 1H), 7.63 - 7.55 (m, 2H), 7.39 (dd, J = 11.6, 6.0 Hz, 1H), 7.03 (d, J = 8.2 Hz, 1H), 5.81 (s, 2H), 5.07 (d, J = 6.6 Hz, 1H), 4.75 (dd, J = 15.7, 7.0 Hz, 1H), 4.62 (d, J = 14.3 Hz, 1H), 4.56 - 4.41 (m, 3H), 4.35 (dt, J = 9.1, 5.9 Hz, 1H), 2.72 (d, J = 13.0 Hz, 1H), 2.37 (dd, J = 19.4, 10.0 Hz, 1H).

### Example 96: (S)-2-(4-(6-((5-(1-cyclopropyl-1H-pyrazol-4-yl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(1-cyclopropyl-1H-pyrazol-4-yl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.52 (s, 1H), 8.25 (d, J = 1.5 Hz, 1H), 7.98 (s, 1H), 7.96 - 7.88 (m, 2H), 7.79 (dd, J = 8.4, 1.6 Hz, 1H), 7.63 - 7.52 (m, 2H), 7.39 (dd, J = 11.6, 6.1 Hz, 1H), 7.02 (d, J = 8.3 Hz, 1H), 5.93 (s, 2H), 5.07 (qd, J = 7.0, 2.6 Hz, 1H), 4.75 (dd, J = 15.6, 7.0 Hz, 1H), 4.62 (dd, J = 15.6, 2.8 Hz, 1H), 4.58 - 4.40 (m, 3H), 4.35 (dt, J = 9.0, 6.0 Hz, 1H), 3.80 (tt, J = 7.5, 3.9 Hz, 1H), 2.81 - 2.63 (m, 1H), 2.44 - 2.31 (m, 1H), 1.10 (q, J = 4.0 Hz, 2H), 1.04 - 0.94 (m, 2H).

### Example 97: (R)-2-(2,5-difluoro-4-(6-((5-(1-methyl-1H-pyrazol-4-yl)thiazol-2-yl)methoxy)pyridin-2-yl)benzyl)-1-(4,4-dimethyltetrahydrofuran-3-yl)-1H-benzo[d]imidazole-6-carboxylic acid

(R)-2-(2,5-difluoro-4-(6-((5-(1-methyl-1H-pyrazol-4-yl)thiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 3. 1H NMR (400 MHz, DMSO-d6) δ 8.50 (s, 1H), 8.07 (s, 1H), 7.97 - 7.90 (m, 2H), 7.89 (s, 1H), 7.82 (dd, J = 8.5, 1.5 Hz, 1H), 7.74 (d, J = 0.8 Hz, 1H), 7.63 (d, J = 8.5 Hz, 1H), 7.59 (dd, J = 7.5, 1.6 Hz, 1H), 7.47 (dd, J = 11.5, 6.1 Hz, 1H), 7.02 (d, J = 8.2 Hz, 1H), 5.76 (s, 2H), 5.02 (d, J = 6.7 Hz, 1H), 4.61 - 4.49 (m, 2H), 4.49 - 4.32 (m, 2H), 3.83 (s, 3H), 3.78 (d, J = 8.6 Hz, 1H), 3.72 (d, J = 8.6 Hz, 1H), 1.33 (s, 3H), 0.60 (s, 3H).

### Example 98: (S)-2-(2,5-difluoro-4-(5-fluoro-6-((5-(1-methyl-1H-pyrazol-4-yl)thiazol-2-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(5-fluoro-6-((5-(1-methyl-1H-pyrazol-4-yl)thiazol-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 3. 1H NMR (400 MHz, DMSO-d6) δ 8.15 (d, J = 1.3 Hz, 1H), 8.09 (s, 1H), 7.95 - 7.83 (m, 3H), 7.76 (s, 1H), 7.62 - 7.56 (m, 1H), 7.51 (dd, J = 11.4, 1.2 Hz, 1H), 7.41 (dd, J = 11.5, 6.1 Hz, 1H), 5.85 (s, 2H), 5.15 - 4.97 (m, 1H), 4.79 (dd, J = 15.6, 7.1 Hz, 1H), 4.65 (dd, J = 15.5, 2.7 Hz, 1H), 4.59 - 4.40 (m, 3H), 4.35 (dt, J = 9.0, 5.9 Hz, 1H), 3.84 (s, 3H), 2.81 - 2.62 (m, 1H), 2.44 - 2.29 (m, 1H).

### Example 99: (S)-2-(2,5-difluoro-4-(5-fluoro-6-((5-(1-methyl-1H-pyrazol-4-yl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(5-fluoro-6-((5-(1-methyl-1H-pyrazol-4-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.26 (d, J = 1.5 Hz, 1H), 8.09 (s, 1H), 7.92 - 7.83 (m, 3H), 7.79 (dd, J = 8.4, 1.5 Hz, 1H), 7.76 (d, J = 0.8 Hz, 1H), 7.63 - 7.55 (m, 2H), 7.40 (dd, J = 11.5, 6.1 Hz, 1H), 5.84 (s, 2H), 5.07 (qd, J = 7.0, 2.6 Hz, 1H), 4.75 (dd, J = 15.6, 7.1 Hz, 1H), 4.62 (dd, J = 15.6, 2.8 Hz, 1H), 4.56 - 4.41 (m, 3H), 4.35 (dt, J = 9.0, 5.9 Hz, 1H), 3.84 (s, 3H), 2.72 (ddt, J = 14.0, 11.3, 6.9 Hz, 1H), 2.39 (ddt, J = 11.2, 8.9, 7.0 Hz, 1H).

### Example 100: (S)-2-(2,5-difluoro-4-(6-((5-(isothiazol-5-yl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(isothiazol-5-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1HNMR (400 MHz, DMSO-d6) δ 8.58 (d, J = 1.8 Hz, 1H), 8.30 (s, 1H), 8.25 (d, J = 1.5 Hz, 1H), 7.94 (t, J = 7.9 Hz, 1H), 7.87 (dd, J = 10.5, 6.4 Hz, 1H), 7.79 (dd, J = 8.4, 1.5 Hz, 1H), 7.70 (d, J = 1.8 Hz, 1H), 7.62 - 7.55 (m, 2H), 7.39 (dd, J = 11.5, 6.0 Hz, 1H), 7.05 (d, J = 8.3 Hz, 1H), 5.84 (s, 2H), 5.07 (qd, J = 7.0, 2.7 Hz, 1H), 4.75 (dd, J = 15.6, 7.0 Hz, 1H), 4.62 (dd, J = 15.5, 2.8 Hz, 1H), 4.58 - 4.40 (m, 3H), 4.35 (dt, J = 9.0, 5.9 Hz, 1H), 2.78 - 2.64 (m, 1H), 2.39 (ddd, J = 17.9, 12.2, 8.3 Hz, 1H).

### Example 101: (S)-2-(4-(6-([5,5'-bithiazol]-2-ylmethoxy)pyridin-2-yl)-2,5-difluorobenzyl)-1-(oxetan-2-ylmethyl)-1H-benzo[d]imidazole-6-carboxylic acid

(S)-2-(4-(6-([5,5'-bithiazol]-2-ylmethoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1HNMR (400 MHz, DMSO-d6) δ 9.12 (s, 1H), 8.23 (s, 1H), 8.19 (s, 1H), 8.12 (s, 1H), 7.96 - 7.85 (m, 2H), 7.78 (dd, J = 8.4, 1.6 Hz, 1H), 7.58 (td, J = 6.3, 5.7, 3.1 Hz, 2H), 7.39 (dd, J = 11.5, 6.0 Hz, 1H), 7.03 (d, J = 8.3 Hz, 1H), 5.81 (s, 2H), 5.13 - 5.03 (m, 1H), 4.74 (dd, J = 15.6, 7.0 Hz, 1H), 4.61 (d, J = 14.6 Hz, 1H), 4.56 - 4.39 (m, 3H), 4.35 (dt, J = 8.9, 5.9 Hz, 1H), 2.79 - 2.64 (m, 1H), 2.46 - 2.34 (m, 1H).

### Example 102: (S)-2-(2,5-difluoro-4-(6-((5-(1-methyl-1H-imidazol-5-yl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(1-methyl-1H-imidazol-5-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1HNMR (400 MHz, DMSO-d6) δ 8.26 (d, J = 1.5 Hz, 1H), 8.00 (s, 1H), 7.97 - 7.85 (m, 3H), 7.79 (dd, J = 8.4, 1.5 Hz, 1H), 7.64 - 7.52 (m, 2H), 7.40 (dd, J = 11.5, 6.0 Hz, 1H), 7.25 (s, 1H), 7.03 (d, J = 8.2 Hz, 1H), 5.81 (s, 2H), 5.07 (dd, J = 7.2, 2.7 Hz, 1H), 4.76 (dd, J = 15.6, 7.1 Hz, 1H), 4.62 (dd, J = 15.5, 2.7 Hz, 1H), 4.56 - 4.40 (m, 3H), 4.35 (dt, J = 9.0, 5.9 Hz, 1H), 3.71 (s, 3H), 2.78 - 2.68 (m, 1H), 2.45 - 2.36 (m, 1H).

### Example 103: (S)-2-(2,5-difluoro-4-(6-((5-(1-methyl-1H-pyrazol-3-yl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(1-methyl-1H-pyrazol-3-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.25 (d, J = 1.5 Hz, 1H), 8.07 (s, 1H), 8.00 - 7.85 (m, 2H), 7.79 (dd, J = 8.4, 1.5 Hz, 1H), 7.75 (d, J = 2.3 Hz, 1H), 7.59 (dd, J = 8.1, 6.3 Hz, 2H), 7.39 (dd, J = 11.6, 6.0 Hz, 1H), 7.02 (d, J = 8.3 Hz, 1H), 6.66 (d, J = 2.3 Hz, 1H), 5.77 (s, 2H), 5.15 - 4.99 (m, 1H), 4.75 (dd, J = 15.6, 7.1 Hz, 1H), 4.62 (dd, J = 15.6, 2.8 Hz, 1H), 4.57 - 4.41 (m, 3H), 4.35 (dt, J = 8.9, 6.0 Hz, 1H), 3.83 (s, 3H), 2.78 - 2.68 (m, 1H), 2.44 - 2.35 (m, 1H).

### Example 104: (R)-2-(2,5-difluoro-4-(6-((4-methyl-5-(1-methyl-1H-pyrazol-4-yl)thiazol-2-yl)methoxy)pyridin-2-yl)benzyl)-1-(oxetan-2-ylmethyl)-1H-benzo [d] imidazole-6-carboxylic acid

(R)-2-(2,5-difluoro-4-(6-((4-methyl-5-(1-methyl-1H-pyrazol-4-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.25 (d, J = 1.5 Hz, 1H), 8.00 (s, 1H), 7.95 - 7.86 (m, 2H), 7.79 (dd, J = 8.4, 1.6 Hz, 1H), 7.62 - 7.53 (m, 3H), 7.38 (dd, J = 11.7, 6.0 Hz, 1H), 6.99 (d, J = 8.3 Hz, 1H), 5.70 (s, 2H), 5.07 (td, J = 7.3, 4.7 Hz, 1H), 4.75 (dd, J = 15.6, 7.1 Hz, 1H), 4.62 (dd, J = 15.6, 2.8 Hz, 1H), 4.56 - 4.41 (m, 3H), 4.35 (dt, J = 9.0, 5.9 Hz, 1H), 3.86 (s, 3H), 2.80 - 2.64 (m, 1H), 2.42 (s, 4H).

### Example 105: (S)-2-(2,5-difluoro-4-(6-((5-(pyrimidin-5-yl)thiazol-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-(pyrimidin-5-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, Methanol-d4) δ 9.11 (s, 1H), 8.99 (s, 2H), 8.42 (d, J = 1.4 Hz, 1H), 8.19 (s, 1H), 8.16 (dd, J = 8.5, 1.5 Hz, 1H), 7.91 (dd, J = 10.7, 6.3 Hz, 1H), 7.82 (t, J = 7.9 Hz, 1H), 7.75 (d, J = 8.6 Hz, 1H), 7.61 (dd, J = 7.5, 1.4 Hz, 1H), 7.26 (dd, J = 11.1, 6.0 Hz, 1H), 6.96 (d, J = 8.2 Hz, 1H), 5.82 (s, 2H), 5.22 (qd, J = 7.4, 2.4 Hz, 1H), 4.83 (dd, J = 15.6, 7.4 Hz, 1H), 4.72 - 4.60 (m, 3H), 4.51 (dt, J = 9.1, 6.0 Hz, 1H), 2.85 (dtd, J = 11.5, 8.2, 6.1 Hz, 1H), 2.62 - 2.47 (m, 1H).

### Example 106: (S)-2-(2,5-difluoro-4-(6-((5-(pyrazin-2-yl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(pyrazin-2-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, Methanol-d4) δ 9.05 (d, J = 1.5 Hz, 1H), 8.56 (dd, J = 2.6, 1.5 Hz, 1H), 8.47 (d, J = 2.6 Hz, 1H), 8.42 (d, J = 2.2 Hz, 2H), 8.15 (dd, J = 8.6, 1.5 Hz, 1H), 7.91 (dd, J = 10.7, 6.3 Hz, 1H), 7.82 (t, J = 7.9 Hz, 1H), 7.74 (d, J = 8.5 Hz, 1H), 7.61 (d, J = 7.4 Hz, 1H), 7.25 (dd, J = 11.2, 6.0 Hz, 1H), 6.97 (d, J = 8.2 Hz, 1H), 5.81 (s, 2H), 5.22 (q, J = 6.2, 5.7 Hz, 1H), 4.82 (dd, J = 15.5, 7.4 Hz, 1H), 4.74 - 4.60 (m, 4H), 4.51 (dt, J = 9.1, 6.0 Hz, 1H), 2.98 - 2.75 (m, 1H), 2.53 (dq, J = 11.3, 7.4 Hz, 1H).

### Example 107: (S)-2-(2,5-difluoro-4-(6-((5-(pyridin-2-yl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(pyridin-2-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, Methanol-d4) δ 8.53 (s, 1H), 8.50 (d, J = 5.0 Hz, 1H), 8.33 (s, 1H), 8.18 (dd, J = 8.6, 1.4 Hz, 1H), 7.96 (dd, J = 10.8, 6.3 Hz, 1H), 7.93 - 7.81 (m, 3H), 7.75 (d, J = 8.6 Hz, 1H), 7.64 (d, J = 7.3 Hz, 1H), 7.34 (dt, J = 13.0, 6.8 Hz, 2H), 7.01 (d, J = 8.3 Hz, 1H), 5.82 (s, 2H), 5.24 (d, J = 7.0 Hz, 1H), 5.01 - 4.89 (m, 1H), 4.83 - 4.71 (m, 3H), 4.67 (dd, J = 13.2, 6.5 Hz, 1H), 4.51 (dt, J = 9.2, 6.0 Hz, 1H), 2.96 - 2.77 (m, 1H), 2.71 - 2.50 (m, 1H).

### Example 108: (S)-2-(2,5-difluoro-4-(6-((5-(pyridin-3-yl)thiazol-2-yl)methoxy)pyridin-2-yl)benzyl)-1-(oxetan-2-ylmethyl)-1H-benzo[d]imidazole-6-carboxylic acid

(S)-2-(2, 5-difluoro-4-(6-((5-(pyridin-3-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3. 1H NMR (400 MHz, Methanol-d4) δ 8.92 (d, J = 2.3 Hz, 1H), 8.66 - 8.59 (m, 1H), 8.56 (d, J = 1.3 Hz, 1H), 8.29 (dt, J = 8.0, 1.8 Hz, 1H), 8.25 (s, 1H), 8.20 (dd, J = 8.6, 1.4 Hz, 1H), 7.97 (dd, J = 10.8, 6.3 Hz, 1H), 7.89 (t, J = 7.9 Hz, 1H), 7.76 (d, J = 8.6 Hz, 1H), 7.66 (td, J = 8.1, 7.5, 3.3 Hz, 2H), 7.37 (dd, J = 11.3, 6.0 Hz, 1H), 7.01 (d, J = 8.2 Hz, 1H), 5.85 (s, 2H), 5.25 (td, J = 7.2, 2.3 Hz, 1H), 4.97 (dd, J = 15.5, 7.5 Hz, 1H), 4.86 - 4.62 (m, 4H), 4.53 (dt, J = 9.2, 6.0 Hz, 1H), 2.94 - 2.78 (m, 1H), 2.67 - 2.48 (m, 1H).

### Example 109: (S)-2-(4-(6-((1',2-dimethyl-1'H,2H-[3,4'-bipyrazol]-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1',2-dimethyl-1'H,2H-[3,4'-bipyrazol]-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.24 (d, J = 1.4 Hz, 1H), 8.09 (s, 1H), 7.96 (dd, J = 10.5, 6.4 Hz, 1H), 7.85 (t, J = 7.8 Hz, 1H), 7.79 (dd, J = 8.4, 1.5 Hz, 1H), 7.74 (s, 1H), 7.59 (d, J = 8.4 Hz, 1H), 7.50 (dd, J = 7.6, 1.7 Hz, 1H), 7.38 (dd, J = 11.5, 6.1 Hz, 1H), 6.89 (d, J = 8.3 Hz, 1H), 6.44 (s, 1H), 5.36 (s, 2H), 5.08 (qd, J = 7.1, 2.6 Hz, 1H), 4.75 (dd, J = 15.6, 7.1 Hz, 1H), 4.61 (dd, J = 15.6, 2.8 Hz, 1H), 4.58 - 4.40 (m, 3H), 4.36 (dt, J = 9.0, 5.9 Hz, 1H), 3.88 (s, 3H), 3.87 (s, 3H), 2.79 - 2.67 (m, 1H), 2.39 (ddt, J = 11.2, 9.0, 6.9 Hz, 1H).

### Example 110: (S)-2-(4-(6-((1'-(difluoromethyl)-2-methyl-1'H,2H-[3,4'-bipyrazol]-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1'-(difluoromethyl)-2-methyl-1'H,2H-[3,4'-bipyrazol]-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3. 1H NMR (400 MHz, DMSO-d6) δ 8.66 (s, 1H), 8.24 - 8.14 (m, 2H), 8.04 - 7.92 (m, 1H), 7.90 - 7.75 (m, 3H), 7.59 - 7.47 (m, 2H), 7.38 (dd, J = 11.6, 6.0 Hz, 1H), 6.89 (d, J = 8.3 Hz, 1H), 6.61 (s, 1H), 5.39 (s, 2H), 5.08 (qd, J = 7.0, 2.7 Hz, 1H), 4.73 (dd, J = 15.6, 7.0 Hz, 1H), 4.61 (dd, J = 15.5, 2.8 Hz, 1H), 4.57 - 4.45 (m, 2H), 4.44 (d, J = 16.8 Hz, 1H), 4.36 (dt, J = 9.0, 5.8 Hz, 1H), 3.92 (s, 3H), 2.79 - 2.67 (m, 1H), 2.40 (dq, J = 11.1, 7.3 Hz, 1H).

### Example 331: (S)-2-(4-(6-((5-(1-(difluoromethyl)-1H-pyrazol-4-yl)thiazol-2-yl)methoxy)pyridin-2-yl)-2-fluoro-5-methylbenzyl)-1-(oxetan-2-ylmethyl)-1H-benzo[d]imidazole-6-carboxylic acid

(S)-2-(4-(6-((5-(1-(difluoromethyl)-1H-pyrazol-4-yl)thiazol-2-yl)methoxy)pyridin-2-yl)-2-fluoro-5-methylbenzyl)-1-(oxetan-2-ylmethyl)-1H-benzo[d]imidazole-6-carboxylic acid was prepared in a manner as described in Procedure 3 using Intermediate I-1391 and 1-(difluoromethyl)-4-(4,4,5,5-tetramethyl-1,3,2-dioxaborolan-2-yl)pyrazole. ES/MS m/z: 661.2 (M+H⁺). 1HNMR (400 MHz, DMSO-d6) δ 8.67 (s, 1H), 8.23 (s, 1H), 8.18 (s, 1H), 8.07 (s, 1H), 8.01 - 7.66 (m, 3H), 7.58 (d, J = 8.3 Hz, 1H), 7.35 - 7.21 (m, 3H), 6.98 (d, J = 8.2 Hz, 1H), 5.70 (s, 2H), 5.05 (d, J = 5.8 Hz, 1H), 4.72 (dd, J = 15.5, 7.1 Hz, 1H), 4.60 (d, J = 15.3 Hz, 1H), 4.54 - 4.28 (m, 4H), 2.77 - 2.69 (m, 1H), 2.40 (q, J = 10.3, 8.8 Hz, 1H), 2.26 (s, 3H).

### Example 355: (S)-2-(2,5-difluoro-4-(6-((2-(1-methyl-1H-pyrazol-4-yl)thiazol-5-yl)methoxy)pyridin-2-yl)benzyl)-1-(oxetan-2-ylmethyl)-1H-benzo [d] imidazole-6-carboxylic acid

(S)-2-(2,5-difluoro-4-(6-((2-(1-methyl-1H-pyrazol-4-yl)thiazol-5-yl)methoxy)pyridin-2-yl)benzyl)-1-(oxetan-2-ylmethyl)-1H-benzo[d]imidazole-6-carboxylic acid was prepared in a manner as described in Procedure 3 using Intermediate **I-1096** and (1-methylpyrazol-4-yl)boronic acid. ES/MS m/z: 629.2 (M+H⁺). 1H NMR (400 MHz, DMSO) δ 8.26 (d, J = 9.1 Hz, 1H), 7.98 (dd, J = 10.5, 6.4 Hz, 1H), 7.93 - 7.85 (m, 1H), 7.86 (s, 2H), 7.79 (dd, J = 8.4, 1.6 Hz, 1H), 7.58 (dd, J = 17.7, 7.8 Hz, 2H), 7.41 (dd, J = 11.6, 6.0 Hz, 1H), 6.92 (d, J = 8.3 Hz, 1H), 5.74 (d, J = 15.8 Hz, 3H), 5.08 (d, J = 7.5 Hz, 1H), 4.76 (dd, J = 15.6, 7.1 Hz, 1H), 4.63 (d, J = 13.4 Hz, 1H), 4.59 - 4.41 (m, 3H), 4.36 (dt, J = 9.0, 5.9 Hz, 1H), 3.87 (s, 3H), 2.72 (t, J = 8.9 Hz, 1H), 2.40 (q, J = 8.7 Hz, 1H), 1.36 - 1.22 (m, 3H).

### Example 382: (R)-2-(2,5-difluoro-4-(6-((5-(4-(trifluoromethyl)phenyl)thiazol-2-yl)methoxy)pyridin-2-yl)benzyl)-1-(oxetan-2-ylmethyl)-1H-benzo [d] imidazole-6-carboxylic acid

(R) -2-(2,5-difluoro-4-(6-((5-(4-(trifluoromethyl)phenyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 3 using Intermediate **I-21** and 4,4,5,5-tetramethyl-2-[4-(trifluoromethyl)phenyl]-1,3,2-dioxaborolane. ES/MS m/z: 693.0 (M+H⁺). 1H NMR (400 MHz, MeOD) δ 8.18 (d, J = 16.9 Hz, 2H), 7.96 (dd, J = 8.5, 1.5 Hz, 1H), 7.93 - 7.86 (m, 1H), 7.86 - 7.78 (m, 3H), 7.72 (d, J = 8.1 Hz, 2H), 7.61 (d, J = 7.4 Hz, 1H), 7.58 (d, J = 8.4 Hz, 1H), 7.14 (dd, J = 11.6, 6.0 Hz, 1H), 6.96 (d, J = 8.2 Hz, 1H), 5.82 (s, 2H), 5.21 (dt, J = 8.5, 4.2 Hz, 1H), 4.74 - 4.53 (m, 4H), 4.51 (d, J = 6.4 Hz, 1H), 4.48 - 4.36 (m, 1H), 2.78 (dt, J = 16.1, 7.7 Hz, 1H), 2.50 (q, J = 9.5, 8.7 Hz, 1H).

### Example 383: (S)-2-(4-(6-((5-(4-cyanophenyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(4-cyanophenyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3 using Intermediate **I-21** and 4-(4,4,5,5-tetramethyl-1,3,2-dioxaborolan-2-yl)benzonitrile. ES/MS m/z: 650.2 (M+H⁺). 1H NMR (400 MHz, MeOD) δ 8.23 (s, 1H), 8.15 (s, 1H), 7.96 (dd, J = 8.5, 1.5 Hz, 1H), 7.92 - 7.74 (m, 6H), 7.59 (dd, J = 13.8, 7.9 Hz, 2H), 7.14 (dd, J = 11.6, 6.0 Hz, 1H), 6.96 (d, J = 8.2 Hz, 1H), 5.82 (s, 2H), 5.30 - 5.10 (m, 1H), 4.77 - 4.36 (m, 7H), 2.78 (dq, J = 14.5, 7.6 Hz, 1H), 2.60 - 2.42 (m, 1H).

### Example 384: (S)-2-(4-(6-((5-(4-chlorophenyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(4-chlorophenyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 3 using Intermediate **I-21** and 2-(4-chlorophenyl)-4,4,5,5-tetramethyl-1,3,2-dioxaborolane. ES/MS m/z: 659.0 (M+H⁺). 1H NMR (400 MHz, MeOD) δ 8.06 (s, 1H), 7.98 (dd, J = 8.5, 1.5 Hz, 1H), 7.91 (dd, J = 10.8, 6.4 Hz, 1H), 7.85 (t, J = 7.9 Hz, 1H), 7.69 - 7.57 (m, 4H), 7.50 - 7.34 (m, 2H), 7.18 (dd, J = 11.6, 6.0 Hz, 1H), 6.96 (d, J = 8.2 Hz, 1H), 5.80 (s, 2H), 5.19 (td, J = 7.9, 5.3 Hz, 1H), 4.78 - 4.34 (m, 7H), 2.77 (dt, J = 11.4, 7.1 Hz, 1H), 2.49 (dq, J = 11.0, 7.3 Hz, 1H).

### Example 111: 2-[[2,5-difluoro-4-[6-[[5-[2-(1-methylpyrazol-4-yl)ethynyl]thiazol-2-yl]methoxy]-2-pyridyl]phenyl]methyl]-7-fluoro-3-[[(2S)-oxetan-2-yl]methyl]benzimidazole-5-carboxylic acid

### Procedure 4

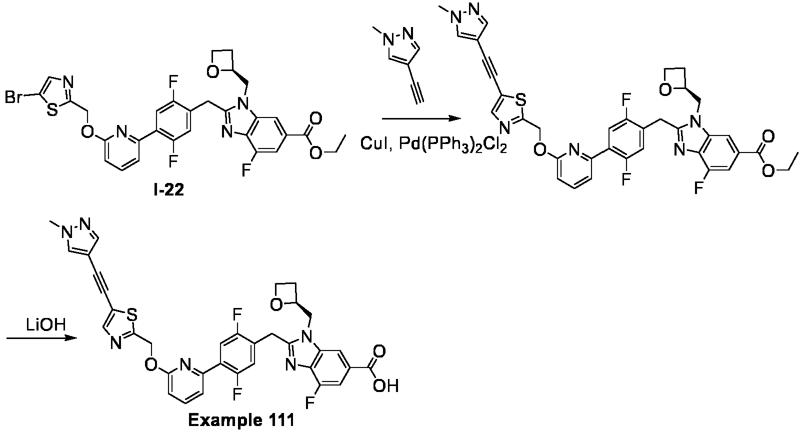

**Ethyl 2-[[2,5-difluoro-4-[6-[[5-[2-(1-methylpyrazol-4-yl)ethynyl]thiazol-2-yl] methoxy] -2-pyridyl] phenyl] methyl] -7-fluoro-3-**[[**(2S)-oxetan-2-yl] methyl] benzimidazole-5-carboxylate:** To a suspension of ethyl 2-[[4-[6-[(5-bromothiazol-2-yl)methoxy]-2-pyridyl]-2,5-difluoro-phenyl]methyl]-7-fluoro-3-[[(2S)-oxetan-2-yl]methyl]benzimidazole-5-carboxylate **I-22** (60 mg, 0.089 mmol), 4-ethynyl-1-methyl-pyrazole (66 mg, 0.62 mmol), cuprous iodide (8.5 mg, 0.0045 mmol), and bis(triphenylphosphine)palladium chloride (31 mg, 0.0045 mmol) in DMF (1.6 mL) was added diisopropylamine (0.26 mL, 1.8 mmol). The resulting solution was degassed by bubbling argon for 1 minute, sealed and heated to 100 °C for 3 hrs. Upon completion of the time, the mixture was poured into water (5 mL) and extracted with EtOAc (2 x 5 mL). The organic layers were combined, washed with brine (5 mL), dried over MgSO₄, filtered, concentrated, and purified by flash chromatography (Eluent: EtOAc/hexane) to give the desired product. ES/MS: 699.0 (M+1).

**2-[[2,5-difluoro-4-[6-[[5-[2-(1-methylpyrazol-4-yl)ethynyl]thiazol-2-yl]methoxy]-2-pyridyl]phenyl]methyl]-7-fluoro-3-[[(2S)-oxetan-2-yl]methyl]benzimidazole-5-carboxylic acid (Example 111):** Ethyl 2-[[2,5-difluoro-4-[6-[[5-[2-(1-methylpyrazol-4-yl)ethynyl]thiazol-2-yl]methoxy]-2-pyridyl]phenyl]methyl]-7-fluoro-3-[[(2S)-oxetan-2-yl]methyl]benzimidazole-5-carboxylate (43 mg, 0.062 mmol) was taken up in acetonitrile (0.8 mL) and then aqueous lithium hydroxide (1.0 M, 0.19 mL, 0.19 mmol) was added. The mixture was heated to 100 °C for 4 min then cooled to r.t. and diluted with 5% aqueous citric acid to a pH ~5. The mixture was extracted with EtOAc (2 x 5 mL) and the combined organic extracts dried over MgSO₄ and concentrated in vacuo. The material was purified by RP-HPLC (eluent: MeCN/water gradient with 0.1% TFA), which was then diluted with EtOAc (50 mL) and washed with water (5 x 20 mL). The combined organic extracts were washed with brine (15 mL), dried over MgSO₄, filtered, and concentrated to yield **Example 111** as the free base form. ES/MS: 671.0 (M+H⁺); 1H NMR (400 MHz, DMSO-d6) δ 8.15 (d, J = 1.3 Hz, 1H), 8.10 (s, 1H), 8.02 (s, 1H), 7.96 - 7.83 (m, 2H), 7.71 (s, 1H), 7.59 (dd, J = 7.5, 1.6 Hz, 1H), 7.50 (dd, J = 11.4, 1.3 Hz, 1H), 7.41 (dd, J = 11.6, 6.1 Hz, 1H), 7.02 (d, J = 8.2 Hz, 1H), 5.79 (s, 2H), 5.07 (qd, J = 7.0, 2.7 Hz, 1H), 4.79 (dd, J = 15.6, 7.1 Hz, 1H), 4.65 (dd, J = 15.5, 2.8 Hz, 1H), 4.56 - 4.45 (m, 3H), 4.35 (dt, J = 9.0, 5.9 Hz, 1H), 3.85 (s, 3H), 2.71 (dtd, J = 11.0, 8.1, 6.1 Hz, 1H), 2.38 (dddd, J = 14.4, 9.8, 8.3, 4.3 Hz, 1H).

### Example 112: (S)-2-(2,5-difluoro-4-(6-((5-((1-methyl-1H-pyrazol-4-yl)ethynyl)thiophen-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((1-methyl-1H-pyrazol-4-yl)ethynyl)thiophen-2-yl)methoxy)pyridin-2-yl)benzyl)-1-(oxetan-2-ylmethyl)-1H-benzo[d]imidazole-6-carboxylic acid was prepared in a manner as described in Procedure 4. 1H NMR (400 MHz, DMSO-d6) δ 8.26 (d, J = 1.5 Hz, 1H), 8.07 (s, 1H), 7.97 (dd, J = 10.5, 6.5 Hz, 1H), 7.89 (t, J = 7.9 Hz, 1H), 7.78 (dd, J = 8.4, 1.6 Hz, 1H), 7.68 (s, 1H), 7.63 - 7.52 (m, 2H), 7.41 (dd, J = 11.6, 6.0 Hz, 1H), 7.22 (q, J = 3.7 Hz, 2H), 6.92 (d, J = 8.3 Hz, 1H), 5.66 (s, 2H), 5.07 (qd, J = 7.1, 2.7 Hz, 1H), 4.76 (dd, J = 15.6, 7.1 Hz, 1H), 4.63 (dd, J = 15.5, 2.8 Hz, 1H), 4.57 - 4.41 (m, 3H), 4.35 (dt, J = 9.0, 5.9 Hz, 1H), 3.84 (s, 3H), 2.80 - 2.65 (m, 1H), 2.45 - 2.30 (m, 1H).

### Example 113: (S)-2-(2,5-difluoro-4-(6-((5-((3-methyloxetan-3-yl)ethynyl)thiophen-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((3-methyloxetan-3-yl)ethynyl)thiophen-2-yl)methoxy)pyridin-2-yl)benzyl)-1-(oxetan-2-ylmethyl)-1H-benzo[d]imidazole-6-carboxylic acid was prepared in a manner as described in Procedure 4. 1H NMR (400 MHz, DMSO-d6) δ 8.26 (d, J = 1.6 Hz, 1H), 7.95 (dd, J = 10.5, 6.5 Hz, 1H), 7.89 (t, J = 7.9 Hz, 1H), 7.79 (dd, J = 8.5, 1.6 Hz, 1H), 7.60 (d, J = 8.5 Hz, 1H), 7.55 (dd, J = 7.5, 1.7 Hz, 1H), 7.41 (dd, J = 11.6, 6.1 Hz, 1H), 7.19 (s, 2H), 6.92 (d, J = 8.2 Hz, 1H), 5.65 (s, 2H), 5.14 - 5.00 (m, 1H), 4.76 (dd, J = 15.6, 7.0 Hz, 1H), 4.70 (d, J = 5.5 Hz, 2H), 4.63 (dd, J = 15.6, 2.8 Hz, 1H), 4.59 - 4.43 (m, 3H), 4.40 (d, J = 5.6 Hz, 2H), 4.36 (dt, J = 9.1, 5.9 Hz, 1H), 2.78 - 2.66 (m, 1H), 2.44 - 2.32 (m, 1H), 1.60 (s, 3H).

### Example 114: (S)-2-(2,5-difluoro-4-(6-((5-((1-methyl-1H-pyrazol-4-yl)ethynyl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((1-methyl-1H-pyrazol-4-yl)ethynyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 4. 1H NMR (400 MHz, DMSO-d6) δ 8.25 (d, J = 1.5 Hz, 1H), 8.11 (s, 1H), 8.02 (s, 1H), 7.96 - 7.90 (m, 1H), 7.88 (dd, J = 10.5, 6.5 Hz, 1H), 7.78 (dd, J = 8.4, 1.5 Hz, 1H), 7.71 (s, 1H), 7.59 (dd, J = 7.9, 2.0 Hz, 2H), 7.39 (dd, J = 11.6, 6.1 Hz, 1H), 7.02 (d, J = 8.2 Hz, 1H), 5.79 (s, 2H), 5.07 (qd, J = 7.0, 2.8 Hz, 1H), 4.75 (dd, J = 15.6, 7.0 Hz, 1H), 4.62 (dd, J = 15.6, 2.8 Hz, 1H), 4.57 - 4.40 (m, 3H), 4.35 (dt, J = 9.0, 5.9 Hz, 1H), 3.85 (s, 3H), 2.71 (dtd, J = 11.1, 8.1, 6.1 Hz, 1H), 2.45 - 2.35 (m, 1H).

### Example 115: (S)-2-(2,5-difluoro-4-(6-((5-((3-methyloxetan-3-yl)ethynyl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((3-methyloxetan-3-yl)ethynyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 4. 1H NMR (400 MHz, DMSO-d6) δ 8.26 (d, J = 1.5 Hz, 1H), 7.98 (s, 1H), 7.93 (t, J = 7.9 Hz, 1H), 7.86 (dd, J = 10.5, 6.5 Hz, 1H), 7.79 (dd, J = 8.4, 1.6 Hz, 1H), 7.59 (t, J = 7.3 Hz, 2H), 7.40 (dd, J = 11.6, 6.0 Hz, 1H), 7.01 (d, J = 8.3 Hz, 1H), 5.78 (s, 2H), 5.07 (qd, J = 7.0, 2.7 Hz, 1H), 4.80 - 4.68 (m, 3H), 4.62 (dd, J = 15.6, 2.8 Hz, 1H), 4.57 - 4.39 (m, 5H), 4.35 (dt, J = 9.0, 5.9 Hz, 1H), 2.72 (dq, J = 10.7, 7.5 Hz, 1H), 2.45 - 2.32 (m, 1H), 1.61 (s, 3H).

### Example 116: (S)-2-(2,5-difluoro-4-(6-((5-(thiazol-5-ylethynyl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(thiazol-5-ylethynyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 4. 1HNMR (400 MHz, DMSO-d6) δ 9.22 (s, 1H), 8.27 (s, 1H), 8.25 (d, J = 1.5 Hz, 1H), 8.19 (s, 1H), 7.94 (t, J = 7.9 Hz, 1H), 7.87 (dd, J = 10.5, 6.4 Hz, 1H), 7.78 (dd, J = 8.4, 1.5 Hz, 1H), 7.59 (d, J = 8.3 Hz, 2H), 7.40 (dd, J = 11.6, 6.0 Hz, 1H), 7.03 (d, J = 8.3 Hz, 1H), 5.82 (s, 2H), 5.07 (qd, J = 7.1, 2.6 Hz, 1H), 4.75 (dd, J = 15.6, 7.0 Hz, 1H), 4.62 (dd, J = 15.5, 2.8 Hz, 1H), 4.58 - 4.40 (m, 3H), 4.35 (dt, J = 9.0, 5.9 Hz, 1H), 2.82 - 2.64 (m, 1H), 2.39 (ddt, J = 11.2, 9.0, 7.0 Hz, 1H).

### Example 117: (S)-2-(2,5-difluoro-4-(6-((3-fluoro-5-((1-methyl-1H-pyrazol-4-yl)ethynyl)thiophen-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-fluoro-5-((1-methyl-1H-pyrazol-4-yl)ethynyl)thiophen-2-yl)methoxy)pyridin-2-yl)benzyl)-1-(oxetan-2-ylmethyl)-1H-benzo[d]imidazole-6-carboxylic acid was prepared in a manner as described in Procedure 4. 1H NMR (400 MHz, DMSO-d6) δ 8.26 (s, 1H), 8.10 (s, 1H), 7.99 (dd, J = 10.5, 6.4 Hz, 1H), 7.89 (t, J = 7.9 Hz, 1H), 7.78 (d, J = 8.4 Hz, 1H), 7.70 (s, 1H), 7.58 (t, J = 7.3 Hz, 2H), 7.41 (dd, J = 11.7, 6.0 Hz, 1H), 7.29 (s, 1H), 6.93 (d, J = 8.3 Hz, 1H), 5.61 (s, 2H), 5.17 - 5.00 (m, 1H), 4.76 (dd, J = 15.7, 7.1 Hz, 1H), 4.63 (dd, J = 15.2, 2.7 Hz, 1H), 4.59 - 4.42 (m, 3H), 4.35 (dt, J = 9.0, 6.1 Hz, 1H), 3.85 (s, 3H), 2.71 (dd, J = 16.7, 8.2 Hz, 1H), 2.45 - 2.33 (m, 1H).

### Example 118: (S)-2-(4-(6-((5-((1-cyclopropyl-1H-pyrazol-4-yl)ethynyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((1-cyclopropyl-1H-pyrazol-4-yl)ethynyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4. 1H NMR (400 MHz, DMSO-d6) δ 8.26 (d, J = 1.5 Hz, 1H), 8.22 (s, 1H), 8.01 (s, 1H), 7.93 (t, J = 7.9 Hz, 1H), 7.88 (dd, J = 10.5, 6.4 Hz, 1H), 7.78 (dd, J = 8.4, 1.6 Hz, 1H), 7.71 (s, 1H), 7.59 (dd, J = 8.2, 2.7 Hz, 2H), 7.40 (dd, J = 11.6, 6.0 Hz, 1H), 7.02 (d, J = 8.2 Hz, 1H), 5.78 (s, 2H), 5.07 (tt, J = 6.9, 3.7 Hz, 1H), 4.75 (dd, J = 15.6, 7.0 Hz, 1H), 4.62 (dd, J = 15.5, 2.8 Hz, 1H), 4.57 - 4.41 (m, 3H), 4.35 (dt, J = 9.0, 5.9 Hz, 1H), 3.76 (tt, J = 7.4, 3.9 Hz, 1H), 2.80 - 2.65 (m, 1H), 2.43 - 2.33 (m, 1H), 1.15 - 1.02 (m, 2H), 1.02 - 0.93 (m, 2H).

### Example 316: (S)-2-(4-(6-((5-((3-cyanophenyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((3-cyanophenyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate **I-1176** and 3-ethynylbenzonitrile. ES/MS m/z: 674.0 (M+H⁺). 1H NMR (400 MHz, DMSO-d6) δ 8.21 (s, 1H), 8.17 (s, 1H), 8.09 (d, J = 1.7 Hz, 1H), 7.98 - 7.83 (m, 4H), 7.77 (dd, J = 8.3, 1.5 Hz, 1H), 7.65 (t, J = 7.9 Hz, 1H), 7.60 (d, J = 7.0 Hz, 1H), 7.54 (d, J = 8.3 Hz, 1H), 7.39 (dd, J = 11.6, 6.0 Hz, 1H), 7.04 (d, J = 8.3 Hz, 1H), 5.83 (s, 2H), 5.07 (d, J = 7.1 Hz, 1H), 4.73 (dd, J = 15.9, 7.2 Hz, 1H), 4.60 (d, J = 15.2 Hz, 1H), 4.55 - 4.40 (m, 3H), 4.40 - 4.27 (m, 1H), 2.77 - 2.70 (m, 1H), 2.42 - 2.31 (m, 1H).

### Example 320: (S)-2-(4-(6-((5-(3,3-dimethylbut-1-yn-1-yl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(3,3-dimethylbut-1-yn-1-yl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate **1176** and 3,3-dimethylbut-1-yne. ES/MS m/z: 629.2 (M+H⁺). 1H NMR (400 MHz, DMSO-d6) δ 8.20 (s, 1H), 7.92 (t, J = 7.9 Hz, 1H), 7.89 - 7.82 (m, 2H), 7.78 (dd, J = 8.4, 1.5 Hz, 1H), 7.62 - 7.50 (m, 2H), 7.38 (dd, J = 11.6, 6.0 Hz, 1H), 7.00 (d, J = 8.3 Hz, 1H), 5.75 (s, 2H), 5.07 (dd, J = 7.3, 2.7 Hz, 1H), 4.73 (dd, J = 15.6, 6.9 Hz, 1H), 4.60 (d, J = 14.7 Hz, 1H), 4.56 - 4.40 (m, 3H), 4.35 (dt, J = 9.0, 6.0 Hz, 1H), 2.78 - 2.63 (m, 1H), 2.44 - 2.30 (m, 1H), 1.26 (s, 9H).

### Example 321: (S)-2-(2,5-difluoro-4-(6-((5-((1-methylcyclopropyl)ethynyl)-1,3,4-thiadiazol-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-((1-methylcyclopropyl)ethynyl)-1,3,4-thiadiazol-2-yl)methoxy)pyridin-2-yl)benzyl)-1-(oxetan-2-ylmethyl)-1H-benzo[d]imidazole-6-carboxylic acid was prepared in a manner as described in Procedure 4 using Intermediate **I-1177** and 1-ethynyl-1-methylcyclopropane. ES/MS m/z: 628.2 (M+H⁺). 1HNMR (400 MHz, DMSO-d6) δ 8.24 (s, 1H), 7.97 - 7.85 (m, 2H), 7.79 (dd, J = 8.4, 1.5 Hz, 1H), 7.64 - 7.54 (m, 2H), 7.40 (dd, J = 11.6, 6.1 Hz, 1H), 7.01 (d, J = 8.2 Hz, 1H), 5.92 (s, 2H), 5.13 - 5.00 (m, 1H), 4.75 (dd, J = 15.6, 7.0 Hz, 1H), 4.62 (dd, J = 15.6, 2.8 Hz, 1H), 4.57 - 4.41 (m, 3H), 4.35 (dt, J = 8.9, 5.9 Hz, 1H), 2.79 - 2.68 (m, 1H), 2.45 - 2.30 (m, 1H), 1.33 (s, 3H), 1.08 (q, J = 4.2 Hz, 2H), 0.86 (q, J = 4.2 Hz, 2H).

### Example 322: 2-(2,5-difluoro-4-(6-((5-((tetrahydrofuran-3-yl)ethynyl)thiazol-2-yl)methoxy)pyridin-2-yl)benzyl)-1-(((S)-oxetan-2-yl)methyl)-1H-benzo[d]imidazole-6-carboxylic acid

2-(2, 5-difluoro-4-(6-((5-((tetrahydrofuran-3-yl)ethynyl)thiazol-2-yl)methoxy)pyridin-2-yl)benzyl)-1-(((S)-oxetan-2-yl)methyl)-1H-benzo[d]imidazole-6-carboxylic acid was prepared in a manner as described in Procedure 4 using Intermediate I**-1176** and 3-ethynyltetrahydrofuran. ES/MS m/z: 643.2 (M+H⁺). 1H NMR (400 MHz, DMSO-d6) δ 8.25 (d, J = 1.5 Hz, 1H), 7.96 - 7.88 (m, 2H), 7.85 (dd, J = 10.5, 6.5 Hz, 1H), 7.79 (dd, J = 8.4, 1.6 Hz, 1H), 7.59 (dd, J = 8.0, 6.3 Hz, 2H), 7.39 (dd, J = 11.6, 6.0 Hz, 1H), 7.01 (d, J = 8.2 Hz, 1H), 5.76 (s, 2H), 5.07 (tt, J = 7.0, 3.9 Hz, 1H), 4.75 (dd, J = 15.5, 7.0 Hz, 1H), 4.62 (dd, J = 15.6, 2.8 Hz, 1H), 4.57 - 4.40 (m, 3H), 4.35 (dt, J = 9.0, 5.9 Hz, 1H), 3.93 (t, J = 7.7 Hz, 1H), 3.80 (td, J = 8.1, 5.9 Hz, 1H), 3.77 - 3.67 (m, 1H), 3.57 (dd, J = 8.1, 6.5 Hz, 1H), 3.30 - 3.27 (m, 1H), 2.80 - 2.66 (m, 1H), 2.40 (tt, J = 8.8, 5.3 Hz, 1H), 2.23 (ddt, J = 16.1, 8.2, 6.0 Hz, 1H), 1.92 (ddd, J = 11.9, 7.6, 6.0 Hz, 1H).

### Example 323: (S)-2-(2,5-difluoro-4-(6-((5-((1-hydroxycyclobutyl)ethynyl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((1-hydroxycyclobutyl)ethynyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 4 using Intermediate I**-1176** and 1-ethynylcyclobutanol. ES/MS m/z: 643.2 (M+H⁺). 1H NMR (400 MHz, DMSO-d6) δ 8.25 (d, J = 1.5 Hz, 1H), 7.98 (s, 1H), 7.93 (t, J = 7.9 Hz, 1H), 7.86 (dd, J = 10.6, 6.5 Hz, 1H), 7.79 (dd, J = 8.4, 1.5 Hz, 1H), 7.59 (dd, J = 8.2, 2.7 Hz, 2H), 7.39 (dd, J = 11.6, 6.0 Hz, 1H), 7.02 (d, J = 8.2 Hz, 1H), 5.99 (s, 1H), 5.77 (s, 2H), 5.07 (qd, J = 7.1, 2.6 Hz, 1H), 4.75 (dd, J = 15.6, 7.0 Hz, 1H), 4.62 (dd, J = 15.6, 2.8 Hz, 1H), 4.57 - 4.41 (m, 3H), 4.35 (dt, J = 9.0, 5.9 Hz, 1H), 2.80 - 2.64 (m, 1H), 2.44 - 2.28 (m, 3H), 2.19 (qd, J = 9.2, 2.6 Hz, 2H), 1.86 - 1.63 (m, 2H).

### Example 325: (S)-2-(4-(6-((5-((4-cyanophenyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((4-cyanophenyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate **I-1176** and 4-ethynylbenzonitrile. ES/MS m/z: 674.0 (M+H⁺). 1H NMR (400 MHz, DMSO-d6) δ 8.24 (s, 1H), 8.20 (s, 1H), 7.98 - 7.89 (m, 3H), 7.86 (dd, J = 10.6, 6.5 Hz, 1H), 7.80 - 7.69 (m, 3H), 7.58 (t, J = 7.6 Hz, 2H), 7.39 (dd, J = 11.7, 6.2 Hz, 1H), 7.04 (d, J = 8.2 Hz, 1H), 5.83 (s, 2H), 5.06 (d, J = 6.2 Hz, 1H), 4.74 (dd, J = 15.6, 7.1 Hz, 1H), 4.61 (d, J = 15.1 Hz, 1H), 4.57 - 4.39 (m, 3H), 4.39 - 4.25 (m, 1H), 2.72 (d, J = 10.3 Hz, 1H), 2.44 - 2.32 (m, 1H).

### Example 326: (S)-2-(4-(6-((5-((1-(difluoromethyl)cyclopropyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((1-(difluoromethyl)cyclopropyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate I**-1176** and 1-(difluoromethyl)-1-ethynylcyclopropane. ES/MS m/z: 663.2 (M+H⁺). 1H NMR (400 MHz, DMSO-d6) δ 8.22 (s, 1H), 8.00 (s, 1H), 7.93 (t, J = 7.9 Hz, 1H), 7.85 (dd, J = 10.5, 6.5 Hz, 1H), 7.78 (dd, J = 8.4, 1.5 Hz, 1H), 7.63 - 7.53 (m, 2H), 7.38 (dd, J = 11.6, 6.1 Hz, 1H), 7.01 (d, J = 8.2 Hz, 1H), 5.99 - 5.59 (m, 3H), 5.11 - 5.01 (m, 1H), 4.74 (dd, J = 15.5, 7.0 Hz, 1H), 4.61 (d, J = 15.4 Hz, 1H), 4.57 - 4.40 (m, 3H), 4.35 (dt, J = 9.1, 6.0 Hz, 1H), 2.73 (q, J = 7.7 Hz, 1H), 2.40 (q, J = 9.4, 8.5 Hz, 1H), 1.23 (dt, J = 5.9, 2.8 Hz, 4H).

### Example 328: (S)-2-(4-(6-((5-(3-cyano-3-methylbut-1-yn-1-yl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(3-cyano-3-methylbut-1-yn-1-yl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate I**-1176** and 2,2-dimethylbut-3-ynenitrile. ES/MS m/z: 640.2 (M+H⁺). 1HNMR (400 MHz, DMSO-d6) δ 8.25 (d, J = 1.5 Hz, 1H), 8.09 (s, 1H), 7.93 (t, J = 7.9 Hz, 1H), 7.84 (dd, J = 10.5, 6.4 Hz, 1H), 7.79 (dd, J = 8.5, 1.4 Hz, 1H), 7.63 - 7.53 (m, 2H), 7.39 (dd, J = 11.6, 6.0 Hz, 1H), 7.02 (d, J = 8.2 Hz, 1H), 5.80 (s, 2H), 5.07 (qd, J = 7.0, 2.7 Hz, 1H), 4.75 (dd, J = 15.6, 7.0 Hz, 1H), 4.62 (dd, J = 15.6, 2.8 Hz, 1H), 4.58 - 4.40 (m, 3H), 4.35 (dt, J = 9.0, 5.9 Hz, 1H), 2.82 - 2.63 (m, 1H), 2.46 - 2.34 (m, 1H), 1.70 (s, 6H).;1H NMR (400 MHz, DMSO-d6) δ 8.31 (d, J = 1.4 Hz, 1H), 7.99 - 7.78 (m, 4H), 7.63 (d, J = 8.4 Hz, 1H), 7.58 (d, J = 7.3 Hz, 1H), 7.46 - 7.33 (m, 2H), 7.19 (s, 1H), 7.01 (d, J = 8.3 Hz, 1H), 5.77 (s, 2H), 5.07 (dt, J = 8.4, 4.3 Hz, 1H), 4.80 (dd, J = 15.5, 7.1 Hz, 1H), 4.66 (dd, J = 15.4, 2.8 Hz, 1H), 4.61 - 4.44 (m, 3H), 4.37 (dt, J = 9.1, 5.9 Hz, 1H), 2.72 (dq, J = 12.3, 8.9, 8.3 Hz, 1H), 2.48 - 2.34 (m, 1H), 1.39 (s, 6H).

### Example 329: (S)-2-(2,5-difluoro-4-(6-((5-((1-methylcyclopropyl)ethynyl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((1-methylcyclopropyl)ethynyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 4 using Intermediate **I-1176** and 1-ethynyl-1-methylcyclopropane. ES/MS m/z: 627.2 (M+H⁺). 1HNMR (400 MHz, DMSO-d6) δ 8.26 (d, J = 1.5 Hz, 1H), 7.92 (t, J = 7.9 Hz, 1H), 7.88 (s, 1H), 7.87 - 7.82 (m, 1H), 7.79 (dd, J = 8.4, 1.5 Hz, 1H), 7.59 (dd, J = 9.9, 7.4 Hz, 2H), 7.39 (dd, J = 11.6, 6.0 Hz, 1H), 7.00 (d, J = 8.2 Hz, 1H), 5.74 (s, 2H), 5.14 - 5.00 (m, 1H), 4.75 (dd, J = 15.6, 7.1 Hz, 1H), 4.62 (dd, J = 15.4, 2.7 Hz, 1H), 4.57 - 4.41 (m, 3H), 4.35 (dt, J = 9.0, 5.9 Hz, 1H), 2.79 - 2.68 (m, 1H), 2.45 - 2.34 (m, 1H), 1.28 (s, 3H), 0.95 (q, J = 4.0 Hz, 2H), 0.76 (q, J = 4.1 Hz, 2H).

### Example 330: (S)-2-(4-(6-((5-((3,3-difluorocyclobutyl)ethynyl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((3,3-difluorocyclobutyl)ethynyl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate **I-1177** and **I-1388.** ES/MS m/z: 664.0 (M+H⁺). 1HNMR (400 MHz, DMSO-d6) δ 8.16 (s, 1H), 7.99 - 7.83 (m, 2H), 7.77 (d, J = 8.4 Hz, 1H), 7.60 (d, J = 7.4 Hz, 1H), 7.51 (s, 1H), 7.38 (dd, J = 11.6, 6.3 Hz, 1H), 7.02 (dd, J = 8.3, 4.1 Hz, 1H), 5.95 (d, J = 4.2 Hz, 2H), 5.07 (d, J = 6.6 Hz, 1H), 4.71 (dd, J = 15.4, 7.2 Hz, 1H), 4.62 - 4.38 (m, 4H), 4.38 - 4.29 (m, 1H), 3.06 (ddd, J = 22.9, 13.3, 9.4 Hz, 2H), 2.92 - 2.65 (m, 3H), 2.43 - 2.33 (m, 1H). One signal overlapping with solvent.

### Example 333: (S)-2-(4-(6-((5-(cyclobutylethynyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(cyclobutylethynyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate I**-1176** and ethynylcyclobutane. ES/MS m/z: 627.2 (M+H⁺). 1HNMR (400 MHz, DMSO-d6) δ 8.26 (d, J = 1.5 Hz, 1H), 7.96 - 7.83 (m, 3H), 7.79 (dd, J = 8.4, 1.6 Hz, 1H), 7.64 - 7.52 (m, 2H), 7.40 (dd, J = 11.6, 6.0 Hz, 1H), 7.01 (d, J = 8.2 Hz, 1H), 5.75 (s, 2H), 5.13 - 4.99 (m, 1H), 4.75 (dd, J = 15.6, 7.1 Hz, 1H), 4.62 (dd, J = 15.5, 2.8 Hz, 1H), 4.58 - 4.41 (m, 3H), 4.35 (dt, J = 9.0, 5.9 Hz, 1H), 2.79 - 2.68 (m, 1H), 2.44 - 2.32 (m, 1H), 2.28 (qt, J = 8.4, 2.9 Hz, 2H), 2.18 - 2.04 (m, 2H), 2.00 - 1.76 (m, 2H), 1 signal overlapping with solvent.

### Example 334: (S)-2-(2,5-difluoro-4-(6-((5-(3-hydroxy-3-methylbut-1-yn-1-yl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-(3-hydroxy-3-methylbut-1-yn-1-yl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 4 using Intermediate I**-1176** and 2-methyl-2-but-3-yn-2-ol. ES/MS m/z: 631.2 (M+H⁺). 1H NMR (400 MHz, DMSO-d6) δ 8.22 (s, 1H), 7.97 - 7.89 (m, 2H), 7.86 (dd, J = 10.5, 6.4 Hz, 1H), 7.78 (dd, J = 8.4, 1.5 Hz, 1H), 7.63 - 7.51 (m, 2H), 7.39 (dd, J = 11.6, 6.1 Hz, 1H), 7.01 (d, J = 8.2 Hz, 1H), 5.77 (s, 2H), 5.58 (s, 1H), 5.07 (tt, J = 7.2, 3.6 Hz, 1H), 4.74 (dd, J = 15.6, 7.0 Hz, 1H), 4.61 (d, J = 13.4 Hz, 1H), 4.56 - 4.40 (m, 3H), 4.35 (dt, J = 9.1, 5.9 Hz, 1H), 2.81 - 2.66 (m, 1H), 2.45 - 2.31 (m, 1H), 1.43 (s, 6H).

### Example 335: (S)-2-(2,5-difluoro-4-(6-((5-((1-(trifluoromethyl)cyclopropyl)ethynyl)thiazol-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-((1-(trifluoromethyl)cyclopropyl)ethynyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 4 using Intermediate I**-1176** and 1-ethynyl-1-(trifluoromethyl)cyclopropane. ES/MS m/z: 681.0 (M+H⁺). 1H NMR (400 MHz, DMSO-d6) δ 8.25 (d, J = 1.5 Hz, 1H), 8.07 (s, 1H), 7.93 (t, J = 7.9 Hz, 1H), 7.84 (dd, J = 10.5, 6.5 Hz, 1H), 7.79 (dd, J= 8.4, 1.6 Hz, 1H), 7.62 - 7.56 (m, 2H), 7.39 (dd, J = 11.6, 6.1 Hz, 1H), 7.02 (d, J = 8.3 Hz, 1H), 5.78 (s, 2H), 5.07 (tt, J = 6.9, 4.1 Hz, 1H), 4.75 (dd, J = 15.5, 7.0 Hz, 1H), 4.62 (dd, J = 15.6, 2.8 Hz, 1H), 4.58 - 4.40 (m, 3H), 4.35 (dt, J = 9.0, 5.9 Hz, 1H), 2.80 - 2.64 (m, 1H), 2.40 (dt, J = 10.7, 7.9 Hz, 1H), 1.51 - 1.33 (m, 4H).

### Example 336: (S)-2-(4-(6-((5-((3,3-difluorocyclobutyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((3,3-difluorocyclobutyl)ethynyl)thiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediate I**-1176** and 3-ethynyl-1,1-difluorocyclobutane. ES/MS m/z: 663.1 (M+H⁺). 1H NMR (400 MHz, DMSO-d6) δ 8.25 (d, J = 1.5 Hz, 1H), 7.97 (s, 1H), 7.93 (t, J = 7.9 Hz, 1H), 7.85 (dd, J = 10.6, 6.5 Hz, 1H), 7.79 (dd, J = 8.4, 1.5 Hz, 1H), 7.64 - 7.51 (m, 2H), 7.39 (dd, J = 11.6, 6.0 Hz, 1H), 7.01 (d, J = 8.2 Hz, 1H), 5.77 (s, 2H), 5.08 (dd, J = 8.3, 5.7 Hz, 1H), 4.75 (dd, J = 15.6, 7.0 Hz, 1H), 4.69 - 4.58 (m, 1H), 4.56 - 4.41 (m, 3H), 4.35 (dt, J = 9.0, 6.0 Hz, 1H), 3.01 (dddd, J = 14.4, 13.0, 10.6, 9.0 Hz, 2H), 2.77 - 2.64 (m, 3H), 2.40 (q, J = 10.0, 8.7 Hz, 1H).

### Example 438: (S)-2-(4-(6-((6-((1H-pyrazol-4-yl)ethynyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-((1H-pyrazol-4-yl)ethynyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 4 using Intermediates **I-1203** and 4-ethynyl-1H-pyrazole. ES/MS m/z: 633.4 (M+H⁺). 1HNMR (400 MHz, Methanol-d4) δ 8.69 - 8.61 (m, 1H), 8.27 (dd, J = 1.5, 0.7 Hz, 1H), 7.99 (dd, J = 8.5, 1.5 Hz, 1H), 7.94 (dd, J = 8.1, 2.2 Hz, 1H), 7.87 (s, 2H), 7.85 - 7.78 (m, 2H), 7.76 (d, J = 8.0 Hz, 1H), 7.71 - 7.63 (m, 1H), 7.57 (dd, J = 8.0, 0.9 Hz, 1H), 7.55 - 7.49 (m, 1H), 7.14 (dd, J = 11.4, 6.0 Hz, 1H), 6.88 (dd, J = 8.3, 0.7 Hz, 1H), 5.56 (s, 2H), 5.18 (tt, J = 7.0, 3.5 Hz, 1H), 4.74 - 4.49 (m, 5H), 4.49 - 4.39 (m, 1H), 2.87 - 2.72 (m, 1H), 2.48 (ddt, J = 11.5, 9.1, 7.2 Hz, 1H).

### Example 120: (S)-2-(2,5-difluoro-4-(6-((1-phenyl-1H-pyrazol-3-yl)methoxy)pyridin-2-yl)benzyl)-1-(oxetan-2-ylmethyl)-1H-benzo[d]imidazole-6-carboxylic acid

### Procedure 5

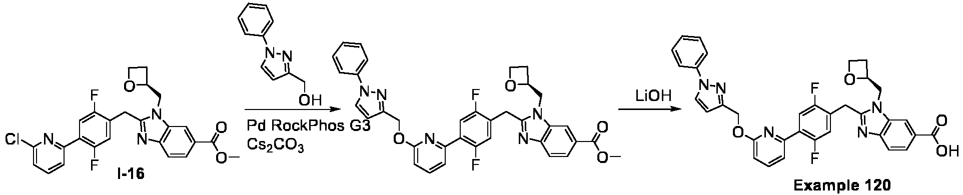

**Methyl (S)-2-(2,5-difluoro-4-(6-((1-phenyl-1H-pyrazol-3-yl)methoxy)pyridin-2-yl)benzyl)-1-(oxetan-2-ylmethyl)-1H-benzo[d]imidazole-6-carboxylate:** A suspension of methyl 2-[[4-(6-chloro-2-pyridyl)-2,5-difluoro-phenyl]methyl]-3-[[(2S)-oxetan-2-yl]methyl]benzimidazole-5-carboxylate (30.0 mg, 0.0620 mmol), (1-phenylpyrazol-3-yl)methanol (16.2 mg, 0.0930 mmol), Pd RockPhos G3 (7.00 mg, 0.00835 mmol), and cesium carbonate (60.6 mg, 0.186 mmol) in toluene (1.5 mL) was degassed with Ar for 5 min, then heated at 100 °C overnight. Following this time, the mixture was diluted with EtOAc and washed with brine. The organic extract was dried over sodium sulfate and purified by flash chromatography (eluent: EtOAc/hexanes) to give the title compound. ES/MS: 622.2 (M+H⁺).

**(S)-2-(2,5-difluoro-4-(6-((1-phenyl-1H-pyrazol-3-yl)methoxy)pyridin-2-yl)benzyl)-1-(oxetan-2-ylmethyl)-1H-benzo[d]imidazole-6-carboxylic acid (Example 120):** A suspension of methyl 2-[[2,5-difluoro-4-[6-[(1-phenylpyrazol-3-yl)methoxy]-2-pyridyl]phenyl]methyl]-3-[[(2S)-oxetan-2-yl]methyl]benzimidazole-5-carboxylate (13.0 mg, 0.0209 mmol) and lithium hydroxide, monohydrate (300 mmol/L, 0.209 mL, 0.0627 mmol) in CH₃CN (3 mL) in a 40 mL reaction vial was heated at 100 °C for 6 min. Upon completion of the time, an additional solution of lithium hydroxide, monohydrate (0.300 M, 0.209 mL, 0.06275 mmol) was added followed by heating the mixture until reaction is complete (20 min). Upon completion the reaction mixture was diluted with EtOAc and brine. Next 0.130 mL of 1M citric acid was added to the reaction mixture. The organic extract was dried over sodium sulfate, filtered and concentrated. The crude residue was purified by RP-HPLC (eluent: MeCN/H₂O). The resulting product fractions were diluted with EtOAc and neutralized with sodium bicarbonate solution. The organic extract was dried over sodium sulfate, filtered and concentrated to give **Example 120.** ES/MS: 608.2 (M+H⁺); 1H NMR (400 MHz, Methanol-d4) δ 8.33 (d, J = 1.5 Hz, 1H), 8.18 (d, J = 2.5 Hz, 1H), 8.04 - 7.96 (m, 2H), 7.82 - 7.73 (m, 3H), 7.67 (d, J = 8.5 Hz, 1H), 7.55 (dd, J = 7.5, 1.6 Hz, 1H), 7.51 - 7.38 (m, 2H), 7.36 - 7.25 (m, 1H), 7.19 (dd, J = 11.5, 6.0 Hz, 1H), 6.91 - 6.84 (m, 1H), 6.60 (d, J = 2.5 Hz, 1H), 5.58 (s, 2H), 5.19 (qd, J = 7.0, 2.6 Hz, 1H), 4.73 (dd, J = 15.7, 7.0 Hz, 1H), 4.69 - 4.47 (m, 4H), 4.44 (dt, J = 9.2, 6.0 Hz, 1H), 2.86 - 2.65 (m, 1H), 2.49 (ddt, J = 11.5, 9.2, 7.1 Hz, 1H).

### Example 121: 2-(2,5-difluoro-4-(6-((1-phenyl-1H-pyrazol-4-yl)methoxy)pyridin-2-yl)benzyl)-1-(2-methoxyethyl)-1H-benzo[d]imidazole-6-carboxylic acid

2-(2,5-difluoro-4-(6-((1-phenyl-1H-pyrazol-4-yl)methoxy)pyridin-2-yl)benzyl)-1-(2-methoxyethyl)-1H-benzo[d]imidazole-6-carboxylic acid was prepared in a manner as described in Procedure 5. 1HNMR (400 MHz, DMSO-d6) δ 8.64 (s, 1H), 8.21 (s, 1H), 7.97 - 7.76 (m, 6H), 7.60 (d, J = 8.4 Hz, 1H), 7.50 (t, J = 7.9 Hz, 3H), 7.39 (dd, J = 11.5, 6.0 Hz, 1H), 7.31 (t, J = 7.6 Hz, 1H), 6.91 (d, J = 8.3 Hz, 1H), 5.43 (s, 2H), 4.60 (t, J = 5.0 Hz, 2H), 4.44 (d, J = 10.0 Hz, 2H), 3.69 (t, J = 5.0 Hz, 2H), 3.30 (s, 2H), 3.22 (d, J = 2.8 Hz, 3H).

### Example 122: 2-(2,5-difluoro-4-(6-((1-phenyl-1H-imidazol-4-yl)methoxy)pyridin-2-yl)benzyl)-1-(2-methoxyethyl)-1H-benzo[d]imidazole-6-carboxylic acid

2-(2, 5-difluoro-4-(6-((1-phenyl-1H-imidazol-4-yl)methoxy)pyridin-2-yl)benzyl)-1-(2-methoxyethyl)-1H-benzo[d]imidazole-6-carboxylic acid was prepared in a manner as described in Procedure 5. 1HNMR (400 MHz, DMSO-d6) δ 12.78 (s, 1H), 8.31 (s, 1H), 8.20 (d, J = 1.5 Hz, 1H), 7.95 (dd, J = 10.4, 6.5 Hz, 1H), 7.91 - 7.82 (m, 2H), 7.79 (dd, J = 8.4, 1.6 Hz, 1H), 7.70 - 7.63 (m, 2H), 7.60 (d, J = 8.4 Hz, 1H), 7.56 - 7.45 (m, 3H), 7.45 - 7.26 (m, 2H), 6.91 (d, J = 8.3 Hz, 1H), 5.38 (s, 2H), 4.59 (t, J = 5.2 Hz, 2H), 4.45 (s, 2H), 3.69 (t, J = 5.1 Hz, 2H), 3.22 (s, 3H).

### Example 123: (S)-2-(2,5-difluoro-4-(6-((5-(trifluoromethyl)thiophen-2-yl)methoxy)pyridin-2-yl)benzyl)-1-(oxetan-2-ylmethyl)-1H-benzo[d]imidazole-6-carboxylic acid:

(S)-2-(2,5-difluoro-4-(6-((5-(trifluoromethyl)thiophen-2-yl)methoxy)pyridin-2-yl)benzyl)-1-(oxetan-2-ylmethyl)-1H-benzo[d]imidazole-6-carboxylic acid was prepared in a manner as described in Procedure 5. 1HNMR (400 MHz, Methanol-d4) δ 8.31 (d, J = 1.5 Hz, 1H), 8.02 - 7.90 (m, 2H), 7.81 (t, J = 7.9 Hz, 1H), 7.67 (d, J = 8.5 Hz, 1H), 7.58 (dd, J = 7.3, 1.5 Hz, 1H), 7.45 (dt, J = 3.8, 1.2 Hz, 1H), 7.25 (dd, J = 3.8, 1.3 Hz, 1H), 7.20 (dd, J = 11.5, 6.0 Hz, 1H), 6.87 (d, J = 8.2 Hz, 1H), 5.73 (s, 2H), 5.21 (qd, J = 7.1, 2.6 Hz, 1H), 4.74 (dd, J = 15.7, 6.9 Hz, 1H), 4.68 - 4.48 (m, 4H), 4.45 (dt, J = 9.2, 5.9 Hz, 1H), 2.80 (dtd, J = 11.5, 8.2, 6.1 Hz, 1H), 2.61 - 2.41 (m, 1H).

### Example 124: (S)-2-(2,5-difluoro-4-(6-((1-methyl-1H-pyrazol-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((1-methyl-1H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 5. 1H NMR (400 MHz, Methanol-d4) δ 8.32 (d, J = 1.4 Hz, 1H), 7.99 (dd, J = 8.5, 1.5 Hz, 1H), 7.95 (dd, J = 10.8, 6.4 Hz, 1H), 7.76 (t, J = 7.9 Hz, 1H), 7.68 (d, J = 8.5 Hz, 1H), 7.55 (d, J = 2.3 Hz, 1H), 7.53 (dd, J = 7.5, 1.6 Hz, 1H), 7.19 (dd, J = 11.5, 6.1 Hz, 1H), 6.83 (d, J = 8.2 Hz, 1H), 6.36 (d, J = 2.3 Hz, 1H), 5.45 (s, 2H), 5.20 (tt, J = 7.1, 3.5 Hz, 1H), 4.74 (dd, J = 15.7, 7.0 Hz, 1H), 4.69 - 4.40 (m, 5H), 3.91 (s, 2H), 2.81 (dtd, J = 11.4, 8.2, 6.1 Hz, 1H), 2.50 (ddt, J = 11.4, 9.1, 7.2 Hz, 1H).

### Example 125: (S)-2-(2,5-difluoro-4-(6-((1-(pyridin-4-yl)-1H-pyrazol-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((1-(pyridin-4-yl)-1H-pyrazol-3-yl)methoxy)pyridin-2-yl)benzyl)-1-(oxetan-2-ylmethyl)-1H-benzo[d]imidazole-6-carboxylic acid was prepared in a manner as described in Procedure 5. 1H NMR (400 MHz, Methanol-d4) δ 8.68 - 8.55 (m, 2H), 8.44 (d, J = 2.6 Hz, 1H), 8.31 (d, J = 1.4 Hz, 1H), 8.05 - 7.94 (m, 2H), 7.94 - 7.87 (m, 2H), 7.79 (t, J = 7.9 Hz, 1H), 7.67 (d, J = 8.5 Hz, 1H), 7.56 (dd, J = 7.3, 1.5 Hz, 1H), 7.18 (dd, J = 11.6, 6.1 Hz, 1H), 6.89 (d, J = 8.2 Hz, 1H), 6.69 (d, J = 2.7 Hz, 1H), 5.60 (s, 2H), 5.19 (tt, J = 7.1, 3.7 Hz, 1H), 4.76 - 4.30 (m, 6H), 2.92 - 2.69 (m, 1H), 2.59 - 2.45 (m, 1H).

### Example 126: (S)-2-(4-(6-((5-cyanothiophen-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyanothiophen-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 5. 1HNMR (400 MHz, DMSO-d6) δ 8.33 (d, J = 1.5 Hz, 1H), 7.98 - 7.88 (m, 3H), 7.85 (dd, J = 8.5, 1.5 Hz, 1H), 7.65 (d, J = 8.4 Hz, 1H), 7.57 (dd, J = 7.5, 1.6 Hz, 1H), 7.48 - 7.39 (m, 2H), 6.97 (d, J = 8.3 Hz, 1H), 5.76 (s, 2H), 5.13 - 5.04 (m, 1H), 4.82 (dd, J = 15.5, 7.2 Hz, 1H), 4.68 (dd, J = 15.6, 2.8 Hz, 1H), 4.64 - 4.47 (m, 3H), 4.38 (dt, J = 9.1, 5.9 Hz, 1H), 2.79 - 2.66 (m, 1H), 2.46 - 2.32 (m, 1H).

### Example 127: (S)-2-(4-(6-((5-carbamoylthiophen-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-carbamoylthiophen-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 5. 1HNMR (400 MHz, DMSO-d6) δ 8.32 (d, J = 1.5 Hz, 1H), 7.99 - 7.90 (m, 2H), 7.88 (d, J = 7.9 Hz, 1H), 7.84 (dd, J = 8.4, 1.5 Hz, 1H), 7.68 - 7.62 (m, 2H), 7.55 (dd, J = 7.5, 1.7 Hz, 1H), 7.43 (dd, J = 11.6, 6.1 Hz, 1H), 7.38 (s, 1H), 7.23 (d, J = 3.8 Hz, 1H), 6.93 (d, J = 8.3 Hz, 1H), 5.66 (s, 2H), 5.16 - 5.00 (m, 1H), 4.81 (dd, J = 15.6, 7.1 Hz, 1H), 4.73 - 4.60 (m, 1H), 4.60 - 4.46 (m, 3H), 4.38 (dt, J = 9.0, 5.9 Hz, 1H), 2.79 - 2.66 (m, 1H), 2.46 - 2.31 (m, 1H).

### Example 128: (S)-2-(4-(6-((5-cyanothiophen-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyanothiophen-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 5. 1H NMR (400 MHz, DMSO-d6) δ 8.39 - 8.28 (m, 1H), 8.10 (d, J = 1.4 Hz, 1H), 8.06 (d, J = 1.4 Hz, 1H), 7.90 (d, J = 7.9 Hz, 1H), 7.88 - 7.81 (m, 2H), 7.65 (t, J = 8.4 Hz, 1H), 7.53 (dd, J = 7.5, 1.7 Hz, 1H), 7.43 (dd, J = 11.4, 6.0 Hz, 1H), 6.95 (d, J = 8.3 Hz, 1H), 5.48 (s, 2H), 5.09 (qd, J = 7.0, 2.6 Hz, 1H), 4.81 (dd, J = 15.5, 7.1 Hz, 1H), 4.67 (dd, J = 15.4, 2.8 Hz, 1H), 4.64 - 4.46 (m, 3H), 4.38 (dt, J = 8.8, 5.8 Hz, 1H), 2.80 - 2.64 (m, 1H), 2.46 - 2.30 (m, 1H).

### Example 129: (S)-2-(4-(6-((5-carbamoylthiophen-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-carbamoylthiophen-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 5. 1HNMR (400 MHz, DMSO-d6) δ 8.30 (d, J = 1.5 Hz, 1H), 7.98 (s, 1H), 7.92 - 7.81 (m, 4H), 7.78 (d, J = 1.3 Hz, 1H), 7.63 (dd, J = 8.5, 5.1 Hz, 1H), 7.55 - 7.49 (m, 1H), 7.41 (dd, J = 11.5, 6.0 Hz, 2H), 6.93 (d, J = 8.2 Hz, 1H), 5.43 (s, 2H), 5.08 (d, J = 7.6 Hz, 1H), 4.80 (dd, J = 15.5, 7.0 Hz, 1H), 4.69 - 4.61 (m, 1H), 4.60 - 4.43 (m, 3H), 4.37 (dt, J = 9.0, 5.9 Hz, 1H), 2.80 - 2.69 (m, 1H), 2.45 - 2.36 (m, 1H).

### Example 130: (S)-2-(4-(6-((4-cyanothiophen-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-cyanothiophen-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 5. 1HNMR (400 MHz, DMSO-d6) δ 8.53 (d, J = 1.5 Hz, 1H), 8.30 (d, J = 1.5 Hz, 1H), 7.98 - 7.87 (m, 2H), 7.82 (dd, J = 8.5, 1.6 Hz, 1H), 7.67 - 7.60 (m, 2H), 7.56 (dd, J = 7.6, 1.7 Hz, 1H), 7.42 (dd, J = 11.6, 6.1 Hz, 1H), 6.94 (d, J = 8.2 Hz, 1H), 5.69 (s, 2H), 5.13 - 5.02 (m, 1H), 4.79 (dd, J = 15.5, 7.2 Hz, 1H), 4.65 (dd, J = 15.6, 2.8 Hz, 1H), 4.60 - 4.43 (m, 3H), 4.37 (dt, J = 9.0, 5.9 Hz, 1H), 2.79 - 2.69 (m, 1H), 2.46 - 2.33 (m, 1H).

### Example 131: (S)-2-(4-(6-((4-carbamoylthiophen-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-carbamoylthiophen-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 5. 1HNMR (400 MHz, DMSO-d6) δ 8.29 (d, J = 1.5 Hz, 1H), 8.08 (d, J = 1.5 Hz, 1H), 7.97 (dd, J = 10.5, 6.4 Hz, 1H), 7.89 (t, J = 7.8 Hz, 1H), 7.82 (dd, J = 8.4, 1.6 Hz, 1H), 7.77 (s, 1H), 7.63 (d, J = 8.5 Hz, 1H), 7.58 (d, J = 1.4 Hz, 1H), 7.57 - 7.52 (m, 1H), 7.42 (dd, J = 11.6, 6.0 Hz, 1H), 7.21 (s, 1H), 6.92 (d, J = 8.2 Hz, 1H), 5.65 (s, 2H), 5.08 (d, J = 7.2 Hz, 1H), 4.79 (dd, J = 15.4, 7.2 Hz, 1H), 4.69 - 4.61 (m, 1H), 4.61 - 4.44 (m, 3H), 4.37 (dt, J = 8.9, 5.8 Hz, 1H), 2.81 - 2.65 (m, 1H), 2.45 - 2.30 (m, 1H).

### Example 266: 2-(4-(6-((5-ethoxy-1,3,4-thiadiazol-2-yl)methoxy)pyridin-2-yl)-2,5-difluorobenzyl)-1-((1-(fluoromethyl)cyclopropyl)methyl)-1H-benzo [d] imidazole-6-carboxylic acid

2-(4-(6-((5-ethoxy-1,3,4-thiadiazol-2-yl)methoxy)pyridin-2-yl)-2, 5-difluorobenzyl)-1-((1-(fluoromethyl)cyclopropyl)methyl)-1H-benzo[d]imidazole-6-carboxylic acid was prepared in a manner as described in Procedure 5 using Intermediate **I-1187** and 2-(chloromethyl)-5-ethoxy-1,3,4-thiadiazole. ES/MS m/z: 610.3 (M+H⁺). 1H NMR (400 MHz, Methanol-d4) δ 8.57 (s, 1H), 8.19 (dd, J = 8.5, 1.4 Hz, 1H), 8.00 (dd, J = 10.7, 6.3 Hz, 1H), 7.87 (t, J = 7.9 Hz, 1H), 7.77 (d, J = 8.6 Hz, 1H), 7.65 (dd, J = 7.5, 1.6 Hz, 1H), 7.37 (dd, J = 11.3, 6.0 Hz, 1H), 6.95 (d, J = 8.3 Hz, 1H), 5.73 (s, 2H), 4.74 (d, J = 1.8 Hz, 4H), 4.53 (q, J = 7.1 Hz, 2H), 4.24 (d, J = 48.7 Hz, 2H), 1.44 (t, J = 7.1 Hz, 3H), 1.02 (t, J = 5.3 Hz, 2H), 0.90 (d, J = 5.4 Hz, 2H).

### Example 269: 2-(4-(6-((5-chlorothiazol-2-yl)methoxy)pyridin-2-yl)-2,5-difluorobenzyl)-1-((1-(fluoromethyl)cyclopropyl)methyl)-1H-benzo[d]imidazole-6-carboxylic acid

2-(4-(6-((5-chlorothiazol-2-yl)methoxy)pyridin-2-yl)-2,5-difluorobenzyl)-1-((1-(fluoromethyl)cyclopropyl)methyl)-1H-benzo[d]imidazole-6-carboxylic acid was prepared in a manner as described in Procedure 5 using Intermediates **I-1187** and **I-1188.** ES/MS m/z: 614.3 (M+H⁺). 1H NMR (400 MHz, Methanol-d4) δ 8.58 (d, J = 1.4 Hz, 1H), 8.20 (dd, J = 8.6, 1.4 Hz, 1H), 7.96 (dd, J = 10.8, 6.3 Hz, 1H), 7.88 (t, J = 7.9 Hz, 1H), 7.77 (d, J = 8.6 Hz, 1H), 7.70 - 7.58 (m, 2H), 7.37 (dd, J = 11.3, 6.0 Hz, 1H), 6.97 (d, J = 8.2 Hz, 1H), 5.72 (s, 2H), 4.75 (d, J = 1.7 Hz, 4H), 4.24 (d, J = 48.7 Hz, 2H), 1.02 (t, J = 5.2 Hz, 2H), 0.90 (d, J = 5.4 Hz, 2H).

### Example 132: (S)-2-(2,5-difluoro-4-(6-((2-fluoro-4-((1-methyl-1H-pyrazol-4-yl)ethynyl)benzyl)oxy)pyridin-2-yl)benzyl)-1-(oxetan-2-ylmethyl)-1H-benzo[d]imidazole-6-carboxylic acid

### Procedure 6

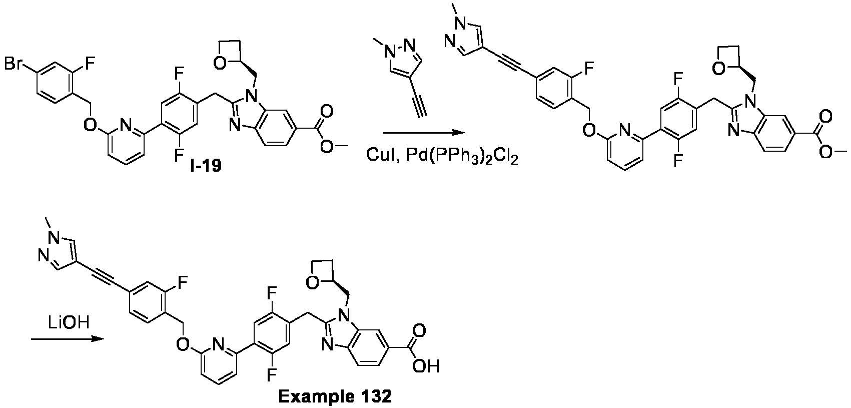

**Methyl 2-[[2,5-difluoro-4-[6-[[2-fluoro-4-[2-(1-methylpyrazol-4-yl)ethynyl]phenyl]methoxy]-2-pyridyl]phenyl]methyl]-3-[[(2S)-oxetan-2-yl]methyl]benzimidazole-5-carboxylate:** Methyl 2-[[4-[6-[(4-bromo-2-fluorophenyl)methoxy]-2-pyridyl]-2,5-difluoro-phenyl]methyl]-3-[[(2S)-oxetan-2-yl]methyl]benzimidazole-5-carboxylate (50 mg, 0.077 mmol), 4-ethynyl-1-methyl-pyrazole (82 mg, 0.77 mmol), copper iodide(3 mg, 0.16 mmol), bis(triphenylphosphine)palladium chloride (11.3 mg, 0.016 mmol) and diisopropylamine (0.11 mL, 0.8 mmol) was suspended in THF (0.5 mL). The reaction mixture was degassed by nitrogen. After which, the reaction mixture was heated to 80 °C for 1.5 hrs. Upon completion of the time, the solvent was removed, and the crude product was dissolved in 1 mL of DMF. The mixture was filtered and purified by RP-HPLC (eluent: water / MeCN 0.1% TFA) to give the desired product. ES/MS: 678.3 (M+H+).

**(S)-2-(2,5-difluoro-4-(6-((2-fluoro-4-((1-methyl-1H-pyrazol-4-yl)ethynyl)benzyl)oxy)pyridin-2-yl)benzyl)-1-(oxetan-2-ylmethyl)-1H-benzo[d]imidazole-6-carboxylic acid (Example 132):** To methyl 2-[[2,5-difluoro-4-[6-[[2-fluoro-4-[2-(1-methylpyrazol-4-yl)ethynyl]phenyl]methoxy]-2-pyridyl]phenyl]methyl]-3-[[(2S)-oxetan-2-yl]methyl]benzimidazole-5-carboxylate (30 mg, 0.044 mmol) was added 1 mL of ACN and 0.4 mL of 1 N lithium hydroxide. The mixture was heated to 80 °C for 45 min. Upon completion of the time, the filtrate was purified by RP-HPLC (eluent: water / MeCN *0.1% TFA) to give **Example 132.** ES/MS: 664.3 (M+H+). 1HNMR (400 MHz, Methanol-d4) δ 8.55 (d, J = 12.7 Hz, 1H), 8.19 (d, J = 12.9 Hz, 1H), 7.95 - 7.86 (m, 1H), 7.86 - 7.70 (m, 3H), 7.66 - 7.57 (m, 1H), 7.52 (dt, J = 12.7, 7.3 Hz, 2H), 7.36 (d, J = 11.6 Hz, 1H), 7.25 (dd, J = 16.6, 9.5 Hz, 2H), 6.98 - 6.78 (m, 1H), 5.68 - 5.45 (m, 2H), 5.23 (s, 1H), 4.95 (t, J = 10.3 Hz, 1H), 4.75 - 4.61 (m, 1H), 4.52 (d, J = 7.5 Hz, 1H), 4.01 - 3.82 (m, 3H), 3.32 (p, J = 1.7 Hz, 3H), 2.84 (s, 1H), 2.55 (s, 1H).

### Example 133: (S)-2-(2,5-difluoro-4-(6-((2-fluoro-4-((3-methyloxetan-3-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((3-methyloxetan-3-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.59 - 8.50 (m, 1H), 8.21 (dd, J = 8.6, 1.5 Hz, 1H), 7.90 (dd, J = 10.8, 6.3 Hz, 1H), 7.86 - 7.73 (m, 2H), 7.57 (dd, J = 7.3, 1.6 Hz, 1H), 7.51 (t, J = 7.8 Hz, 1H), 7.36 (dd, J = 11.2, 6.0 Hz, 1H), 7.29 - 7.14 (m, 2H), 6.92 (d, J = 8.3 Hz, 1H), 5.55 (s, 2H), 5.38 - 5.21 (m, 1H), 4.98 (dd, J = 15.6, 7.5 Hz, 1H), 4.86 (s, 1H), 4.85 - 4.63 (m, 4H), 4.63 - 4.43 (m, 3H), 2.96 - 2.82 (m, 1H), 2.58 (dq, J = 11.5, 7.3 Hz, 1H), 1.70 (s, 4H).

### Example 134: (S)-2-(2,5-difluoro-4-(6-((2-fluoro-4-((1-methyl-1H-pyrazol-3-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((1-methyl-1H-pyrazol-3-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.57 (t, J = 1.0 Hz, 1H), 8.20 (dd, J = 8.6, 1.4 Hz, 1H), 7.89 (dd, J = 10.8, 6.3 Hz, 1H), 7.82 (t, J = 7.9 Hz, 1H), 7.77 (d, J = 8.6 Hz, 1H), 7.64 (d, J = 2.3 Hz, 1H), 7.62 - 7.56 (m, 1H), 7.54 (d, J = 7.8 Hz, 1H), 7.41 - 7.27 (m, 3H), 6.97 - 6.90 (m, 1H), 6.49 (d, J = 2.3 Hz, 1H), 5.57 (s, 2H), 5.26 (tt, J = 7.4, 3.8 Hz, 1H), 4.99 (dd, J = 15.5, 7.6 Hz, 1H), 4.87-4.81 (m, 2H), 4.82 - 4.75 (m, 1H), 4.75 - 4.65 (m, 1H), 4.54 (dt, J = 9.2, 6.0 Hz, 1H), 3.92 (s, 3H), 2.96 - 2.78 (m, 1H), 2.58 (tt, J = 11.5, 7.2 Hz, 1H).

### Example 135: (S)-2-(2,5-difluoro-4-(6-((2-fluoro-4-((1-methyl-1H-pyrazol-5-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((1-methyl-1H-pyrazol-5-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.56 (d, J = 1.2 Hz, 1H), 8.20 (dd, J = 8.6, 1.4 Hz, 1H), 7.89 (dd, J = 10.8, 6.3 Hz, 1H), 7.82 (d, J = 8.1 Hz, 1H), 7.77 (d, J = 8.6 Hz, 1H), 7.64 - 7.55 (m, 2H), 7.51 (d, J = 2.1 Hz, 1H), 7.43 - 7.33 (m, 3H), 6.94 (d, J = 8.3 Hz, 1H), 6.56 (d, J = 2.1 Hz, 1H), 5.60 (s, 2H), 5.31 - 5.21 (m, 1H), 4.98 (dd, J = 15.5, 7.5 Hz, 1H), 4.86 - 4.82 (m, 2H), 4.79 (d, J = 11.6 Hz, 1H), 4.76 - 4.65 (m, 1H), 4.54 (dt, J = 9.1, 5.9 Hz, 1H), 3.99 (s, 3H), 2.97 - 2.79 (m, 1H), 2.76 - 2.47 (m, 1H).

### Example 136: (S)-2-(2,5-difluoro-4-(6-((2-fluoro-4-((2-methyloxazol-5-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((2-methyloxazol-5-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.57 (t, J = 1.0 Hz, 1H), 8.20 (dd, J = 8.6, 1.4 Hz, 1H), 7.93 - 7.73 (m, 3H), 7.59 (dd, J = 8.7, 6.8 Hz, 2H), 7.42 - 7.26 (m, 4H), 6.94 (d, J = 8.2 Hz, 1H), 5.59 (s, 2H), 5.26 (q, J = 6.5 Hz, 1H), 4.98 (dd, J = 15.5, 7.5 Hz, 1H), 4.85 - 4.82 (m, 1H), 4.82 - 4.77 (m, 1H), 4.76 - 4.61 (m, 1H), 4.54 (dt, J = 9.2, 6.0 Hz, 1H), 2.93 - 2.79 (m, 1H), 2.64 - 2.53 (m, 1H), 2.51 (s, 3H).

### Example 137: (S)-2-(2,5-difluoro-4-(6-((2-fluoro-4-(thiazol-5-ylethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-(thiazol-5-ylethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 9.03 (s, 1H), 8.49 (s, 1H), 8.19 - 8.08 (m, 2H), 7.92 - 7.78 (m, 2H), 7.75 (d, J = 8.6 Hz, 1H), 7.67 - 7.49 (m, 2H), 7.43 - 7.24 (m, 3H), 6.93 (d, J = 8.3 Hz, 1H), 5.59 (s, 2H), 5.52 (d, J = 5.8 Hz, 1H), 5.24 (d, J = 6.9 Hz, 1H), 4.81 - 4.63 (m, 5H), 4.51 (dt, J = 9.0, 5.9 Hz, 1H), 2.84 (q, J = 9.2, 8.1 Hz, 1H), 2.67 - 2.44 (m, 1H).

### Example 138: (S)-2-(2,5-difluoro-4-(6-((2-fluoro-4-(pyrimidin-5-ylethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-(pyrimidin-5-ylethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 9.14 (s, 1H), 8.95 (s, 2H), 8.54 (s, 1H), 8.18 (dd, J = 8.6, 1.4 Hz, 1H), 7.93 - 7.87 (m, 1H), 7.87 - 7.81 (m, 1H), 7.77 (d, J = 8.6 Hz, 1H), 7.71 - 7.66 (m, 1H), 7.62 - 7.56 (m, 3H), 7.46 - 7.40 (m, 2H), 7.35 (dd, J = 11.3, 6.1 Hz, 1H), 6.94 (d, J = 8.3 Hz, 1H), 5.60 (s, 2H), 5.26 (q, J = 7.5, 6.8 Hz, 1H), 4.96 (dd, J = 15.5, 7.5 Hz, 1H), 4.86 - 4.74 (m, 3H), 4.74 - 4.64 (m, 1H), 4.53 (dt, J = 9.1, 5.9 Hz, 1H), 2.87 (dt, J = 16.6, 7.9 Hz, 1H), 2.66 - 2.43 (m, 1H).

### Example 139: (S)-2-(2,5-difluoro-4-(6-((2-fluoro-4-(pyridin-3-ylethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-(pyridin-3-ylethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.81 - 8.74 (m, 1H), 8.62 - 8.54 (m, 2H), 8.20 (dd, J = 8.6, 1.4 Hz, 1H), 8.09 (dt, J = 8.0, 1.9 Hz, 1H), 7.90 (dd, J = 10.8, 6.3 Hz, 1H), 7.83 (t, J = 7.9 Hz, 1H), 7.78 (d, J = 8.6 Hz, 1H), 7.71 - 7.54 (m, 3H), 7.48 - 7.34 (m, 3H), 6.94 (d, J = 8.2 Hz, 1H), 5.60 (s, 2H), 5.34 - 5.20 (m, 1H), 4.99 (dd, J = 15.5, 7.5 Hz, 1H), 4.85 - 4.75 (m, 3H), 4.75 - 4.64 (m, 1H), 4.54 (dt, J = 9.2, 6.0 Hz, 1H), 2.94 - 2.79 (m, 1H), 2.58 (tt, J = 11.5, 7.3 Hz, 1H).

### Example 140: (S)-2-(2,5-difluoro-4-(6-((2-fluoro-4-(pyridin-4-ylethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-(pyridin-4-ylethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.77 - 8.67 (m, 2H), 8.53 (d, J = 1.3 Hz, 1H), 8.16 (dd, J = 8.6, 1.4 Hz, 1H), 7.92 - 7.79 (m, 4H), 7.76 (d, J = 8.5 Hz, 1H), 7.65 (t, J = 7.8 Hz, 1H), 7.58 (dd, J = 7.5, 1.6 Hz, 1H), 7.53 - 7.41 (m, 2H), 7.34 (dd, J = 11.3, 6.0 Hz, 1H), 6.94 (d, J = 8.2 Hz, 1H), 5.61 (s, 2H), 5.35 - 5.16 (m, 1H), 4.94 (dd, J = 15.5, 7.4 Hz, 1H), 4.86 - 4.63 (m, 4H), 4.53 (dt, J = 9.1, 6.0 Hz, 1H), 2.94 - 2.78 (m, 1H), 2.57 (ddd, J = 11.6, 9.0, 5.5 Hz, 1H).

### Example 141: (S)-2-(2,5-difluoro-4-(6-((2-fluoro-4-(thiazol-2-ylethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-(thiazol-2-ylethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.57 (d, J = 1.4 Hz, 1H), 8.20 (dd, J = 8.6, 1.4 Hz, 1H), 7.92 (d, J = 3.4 Hz, 1H), 7.90 - 7.86 (m, 1H), 7.86 - 7.79 (m, 1H), 7.79 - 7.72 (m, 2H), 7.66 - 7.53 (m, 2H), 7.48 - 7.40 (m, 2H), 7.37 (dd, J = 11.2, 6.0 Hz, 1H), 6.95 (d, J = 8.2 Hz, 1H), 5.60 (s, 2H), 5.26 (q, J = 7.4 Hz, 1H), 4.98 (dd, J = 15.5, 7.5 Hz, 1H), 4.85 - 4.76 (m, 3H), 4.76 - 4.63 (m, 1H), 4.54 (dt, J = 9.2, 6.0 Hz, 1H), 2.97 - 2.79 (m, 1H), 2.69 - 2.48 (m, 1H).

### Example 142: (S)-2-(2,5-difluoro-4-(6-((2-fluoro-4-((1-methyl-1H-imidazol-5-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((1-methyl-1H-imidazol-5-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.93 (s, 1H), 8.49 (d, J = 1.2 Hz, 1H), 8.13 (dd, J = 8.6, 1.5 Hz, 1H), 7.93 - 7.78 (m, 3H), 7.74 (d, J = 8.5 Hz, 1H), 7.65 (t, J = 7.8 Hz, 1H), 7.58 (dd, J = 7.4, 1.6 Hz, 1H), 7.51 - 7.41 (m, 2H), 7.32 (dd, J = 11.3, 6.0 Hz, 1H), 6.98 - 6.89 (m, 1H), 5.61 (s, 2H), 5.25 (qd, J = 7.3, 2.4 Hz, 1H), 4.93 (d, J = 7.4 Hz, 1H), 4.83 - 4.62 (m, 4H), 4.51 (dt, J = 9.1, 6.0 Hz, 1H), 3.99 (s, 3H), 2.99 - 2.78 (m, 1H), 2.65 - 2.44 (m, 1H).

### Example 143: (S)-2-(2,5-difluoro-4-(6-((3-fluoro-5-((1-methyl-1H-pyrazol-4-yl)ethynyl)pyridin-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-fluoro-5-((1-methyl-1H-pyrazol-4-yl)ethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.58 (d, J = 1.1 Hz, 1H), 8.46 (t, J = 1.3 Hz, 1H), 8.21 (dd, J = 8.6, 1.4 Hz, 1H), 7.91 (s, 1H), 7.90 - 7.70 (m, 4H), 7.68 (s, 1H), 7.61 - 7.54 (m, 1H), 7.36 (dd, J = 11.3, 6.1 Hz, 1H), 6.94 (d, J = 8.2 Hz, 1H), 5.64 (d, J = 1.8 Hz, 2H), 5.27 (q, J = 7.0, 6.6 Hz, 1H), 4.99 (dd, J = 15.5, 7.5 Hz, 1H), 4.86 - 4.64 (m, 4H), 4.54 (dt, J = 9.2, 5.9 Hz, 1H), 3.93 (s, 2H), 2.98 - 2.80 (m, 1H), 2.69 - 2.50 (m, 1H).

### Example 144: (S)-2-(2,5-difluoro-4-(6-((4-fluoro-6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((4-fluoro-6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.70 (d, J = 9.6 Hz, 1H), 8.60 (t, J = 1.1 Hz, 1H), 8.23 (dd, J = 8.6, 1.4 Hz, 1H), 8.00 - 7.89 (m, 2H), 7.89 - 7.76 (m, 2H), 7.75 - 7.70 (m, 1H), 7.59 (dd, J = 7.5, 1.6 Hz, 1H), 7.48 - 7.34 (m, 2H), 6.94 (d, J = 8.3 Hz, 1H), 5.63 (s, 2H), 5.36 - 5.22 (m, 1H), 5.02 (dd, J = 15.5, 7.6 Hz, 1H), 4.85 - 4.76 (m, 1H), 4.76 - 4.66 (m, 1H), 4.55 (dt, J = 9.1, 6.0 Hz, 1H), 3.98 - 3.89 (m, 5H), 2.96 - 2.81 (m, 1H), 2.59 (dq, J = 11.5, 7.4 Hz, 1H).

### Example 145: (S)-2-(2,5-difluoro-4-(6-((4-methoxy-6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((4-methoxy-6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.59 (s, 1H), 8.47 (d, J = 1.4 Hz, 1H), 8.15 - 8.05 (m, 2H), 7.91 - 7.83 (m, 2H), 7.83 - 7.79 (m, 1H), 7.79 - 7.67 (m, 2H), 7.66 - 7.55 (m, 1H), 7.33 (dd, J = 11.3, 6.0 Hz, 1H), 6.98 (d, J = 8.4 Hz, 1H), 5.60 (s, 2H), 5.25 (qd, J = 7.2, 2.5 Hz, 1H), 4.95 - 4.89 (m, 1H), 4.80 - 4.62 (m, 4H), 4.50 (dt, J = 9.2, 5.9 Hz, 1H), 4.21 (s, 3H), 3.97 (s, 3H), 2.94 - 2.78 (m, 1H), 2.56 (tt, J = 11.3, 7.2 Hz, 1H).

### Example 146: (S)-2-(2,5-difluoro-4-(6-((2-fluoro-4-((2-methylpyrimidin-5-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((2-methylpyrimidin-5-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.78 (s, 2H), 8.36 (d, J = 1.3 Hz, 1H), 8.12 (dd, J = 8.6, 1.4 Hz, 1H), 7.85 (dd, J = 10.7, 6.3 Hz, 1H), 7.79 - 7.68 (m, 2H), 7.60 - 7.50 (m, 2H), 7.43 - 7.30 (m, 2H), 7.19 (dd, J = 11.2, 6.0 Hz, 1H), 6.87 (d, J = 8.2 Hz, 1H), 5.56 (s, 2H), 5.38 (s, 1H), 5.29 - 5.16 (m, 1H), 4.76 (dd, J = 15.6, 7.3 Hz, 1H), 4.65 - 4.55 (m, 2H), 4.50 (dt, J = 9.1, 6.0 Hz, 1H), 3.37 (s, 1H), 2.93 - 2.79 (m, 1H), 2.75 (s, 3H), 2.52 (dq, J = 11.0, 7.3 Hz, 1H).

### Example 147: (S)-2-(2,5-difluoro-4-(6-((6-((1-methyl-1H-pyrazol-4-yl)ethynyl)pyridin-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.64 (d, J = 2.1 Hz, 1H), 8.24 (d, J = 1.4 Hz, 1H), 7.99 (dd, J = 8.5, 1.5 Hz, 1H), 7.94 (dd, J = 8.1, 2.2 Hz, 1H), 7.89 (s, 1H), 7.86 - 7.74 (m, 3H), 7.65 (d, J = 8.5 Hz, 1H), 7.61 - 7.48 (m, 2H), 7.14 (dd, J = 11.4, 6.0 Hz, 1H), 6.89 (d, J = 8.2 Hz, 1H), 5.56 (s, 2H), 5.19 (td, J = 7.5, 5.1 Hz, 1H), 4.75 - 4.53 (m, 4H), 4.51 (d, J = 7.1 Hz, 1H), 4.48 - 4.41 (m, 1H), 3.93 (s, 3H), 2.86 - 2.72 (m, 1H), 2.55 - 2.39 (m, 1H).

### Example 148: (S)-2-(2,5-difluoro-4-(6-((2-fluoro-4-((1-methyl-1H-imidazol-2-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((1-methyl-1H-imidazol-2-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.52 (d, J = 1.4 Hz, 1H), 8.16 (dd, J = 8.6, 1.5 Hz, 1H), 7.92 - 7.79 (m, 2H), 7.79 - 7.64 (m, 3H), 7.60 (dd, J = 5.9, 1.8 Hz, 2H), 7.58 (s, 1H), 7.56 (d, J = 1.6 Hz, 1H), 7.34 (dd, J = 11.3, 6.1 Hz, 1H), 6.95 (d, J = 8.2 Hz, 1H), 5.64 (s, 2H), 5.25 (dd, J = 7.2, 2.3 Hz, 1H), 5.00 - 4.92 (m, 1H), 4.83 - 4.64 (m, 4H), 4.52 (dt, J = 9.2, 5.9 Hz, 1H), 4.01 (s, 3H), 2.86 (dd, J = 6.8, 4.7 Hz, 1H), 2.57 (ddt, J = 9.0, 6.9, 2.0 Hz, 1H).

### Example 149: (S)-2-(4-(6-((4-((1,2-dimethyl-1H-imidazol-5-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-((1,2-dimethyl-1H-imidazol-5-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 6. 1H NMR (400 MHz, DMSO-d6) δ 8.29 (d, J = 1.5 Hz, 1H), 8.02 (s, 1H), 7.90 (t, J = 7.9 Hz, 1H), 7.86 - 7.74 (m, 2H), 7.71 - 7.58 (m, 3H), 7.53 (ddd, J = 7.8, 4.3, 1.6 Hz, 2H), 7.41 (dd, J = 11.5, 6.1 Hz, 1H), 6.98 (d, J = 8.3 Hz, 1H), 5.58 (s, 2H), 5.08 (qd, J = 7.1, 2.6 Hz, 1H), 4.79 (dd, J = 15.6, 7.1 Hz, 1H), 4.71 - 4.42 (m, 4H), 4.37 (dt, J = 9.0, 6.0 Hz, 1H), 3.77 (s, 3H), 2.80 - 2.66 (m, 1H), 2.61 (s, 3H), 2.40 (ddt, J = 11.1, 8.7, 6.9 Hz, 1H).

### Example 150: 2-(2-fluoro-4-(6-((2-fluoro-4-((2-(8-(oxetan-3-yl)-3,8-diazabicyclo[3.2.1]octan-3-yl)pyrimidin-5-yl)ethynyl)benzyl)oxy)pyridin-2-yl)benzyl)-1-(2-methoxyethyl)-1H-benzo[d]imidazole-6-carboxylic acid

2-(2-fluoro-4-(6-((2-fluoro-4-((2-(8-(oxetan-3-yl)-3,8-diazabicyclo[3.2.1]octan-3-yl)pyrimidin-5-yl)ethynyl)benzyl)oxy)pyridin-2-yl)benzyl)-1-(2-methoxyethyl)-1H-benzo[d]imidazole-6-carboxylic acid was prepared in a manner as described in Procedure 6. 1H NMR (400 MHz, DMSO-d6) δ 8.65 (s, 2H), 8.23 (d, J = 1.5 Hz, 1H), 7.97 - 7.76 (m, 4H), 7.71 - 7.56 (m, 3H), 7.50 - 7.32 (m, 3H), 6.91 (d, J = 8.2 Hz, 1H), 5.57 (s, 2H), 4.80 (q, J = 8.5, 8.0 Hz, 4H), 4.60 (q, J = 6.6, 5.1 Hz, 4H), 4.47 (s, 2H), 4.38 (s, 1H), 4.10 (s, 2H), 3.67 (t, J = 5.1 Hz, 2H), 3.49 (d, J = 14.2 Hz, 2H), 3.21 (s, 3H), 2.09 (s, 2H), 1.75 (d, J = 9.6 Hz, 2H).

### Example 151: (S)-2-(2,5-difluoro-4-(6-((2-fluoro-4-((1-methyl-1H-pyrazol-4-yl)ethynyl)benzyl)oxy)pyridin-2-yl)benzyl)-4-fluoro-1-(oxetan-2-ylmethyl)-1H-benzo[d]imidazole-6-carboxylic acid

(S)-2-(2,5-difluoro-4-(6-((2-fluoro-4-((1-methyl-1H-pyrazol-4-yl)ethynyl)benzyl)oxy)pyridin-2-yl)benzyl)-4-fluoro-1-(oxetan-2-ylmethyl)-1H-benzo[d]imidazole-6-carboxylic acid was prepared in a manner as described in Procedure 6. Multiplet Report 1H NMR (400 MHz, DMSO-d6) δ 8.16 (d, J = 1.3 Hz, 1H), 8.09 (s, 1H), 7.88 (t, J = 7.8 Hz, 1H), 7.83 (dd, J = 10.5, 6.4 Hz, 1H), 7.70 (s, 1H), 7.58 (t, J = 7.9 Hz, 1H), 7.55 - 7.48 (m, 2H), 7.45 - 7.29 (m, 3H), 6.96 (d, J = 8.2 Hz, 1H), 5.53 (s, 2H), 5.07 (qd, J = 7.0, 2.6 Hz, 1H), 4.79 (dd, J = 15.6, 7.1 Hz, 1H), 4.66 (dd, J = 15.4, 2.7 Hz, 1H), 4.60 - 4.42 (m, 3H), 4.36 (dt, J = 9.0, 5.9 Hz, 1H), 3.86 (s, 3H), 2.78 - 2.64 (m, 1H), 2.45 - 2.31 (m, 1H).

### Example 152: 2-(2,5-difluoro-4-(6-((2-fluoro-4-((2-(8-(oxetan-3-yl)-3,8-diazabicyclo [3.2.1] octan-3-yl)pyrimidin-5-yl)ethynyl)benzyl)oxy)pyridin-2-yl)benzyl)-4-fluoro-1-(((S)-oxetan-2-yl)methyl)-1H-benzo[d]imidazol e-6-carboxylic acid

2-(2,5-difluoro-4-(6-((2-fluoro-4-((2-(8-(oxetan-3-yl)-3,8-diazabicyclo[3.2.1]octan-3-yl)pyrimidin-5-yl)ethynyl)benzyl)oxy)pyridin-2-yl)benzyl)-4-fluoro-1-(((S)-oxetan-2-yl)methyl)-1H-benzo[d]imidazol e-6-carboxylic acid was prepared in a manner as described in Procedure 6. Multiplet Report 1H NMR (400 MHz, DMSO-d6) δ 8.54 (s, 2H), 8.16 (d, J = 1.2 Hz, 1H), 7.89 (t, J = 7.9 Hz, 1H), 7.83 (dd, J = 10.5, 6.4 Hz, 1H), 7.67 - 7.48 (m, 7H), 7.46 - 7.33 (m, 3H), 6.97 (d, J = 8.2 Hz, 1H), 5.54 (s, 2H), 5.07 (qd, J = 7.1, 2.7 Hz, 1H), 4.79 (dd, J = 15.6, 7.1 Hz, 1H), 4.66 (dd, J = 15.5, 2.7 Hz, 1H), 4.59 (t, J = 6.2 Hz, 2H), 4.55 - 4.43 (m, 3H), 4.43 - 4.32 (m, 3H), 4.30 (d, J = 2.4 Hz, 1H), 4.27 (d, J = 2.4 Hz, 1H), 3.63 (p, J = 5.9 Hz, 1H), 3.20 (d, J = 4.5 Hz, 2H), 3.16 - 3.07 (m, 2H), 2.82 - 2.64 (m, 1H), 2.45 - 2.32 (m, 1H), 1.90 - 1.72 (m, 2H), 1.50 - 1.38 (m, 2H).

### Example 153: (S)-4-fluoro-2-(2-fluoro-4-(6-((2-fluoro-4-((1-methyl-1H-pyrazol-4-yl)ethynyl)benzyl)oxy)pyridin-2-yl)-5-methylbenzyl)-1-(oxetan-2-ylmethyl)-1H-benzo[d]imidazole-6-carboxylic acid

(S)-4-fluoro-2-(2-fluoro-4-(6-((2-fluoro-4-((1-methyl-1H-pyrazol-4-yl)ethynyl)benzyl)oxy)pyridin-2-yl)-5-methylbenzyl)-1-(oxetan-2-ylmethyl)-1H-benzo[d]imidazole-6-carboxylic acid was prepared in a manner as described in Procedure 6. Multiplet Report 1H NMR (400 MHz, DMSO-d6) δ 8.13 (s, 1H), 8.10 (s, 1H), 7.86 (t, J = 7.8 Hz, 1H), 7.71 (s, 1H), 7.59 - 7.45 (m, 2H), 7.42 - 7.19 (m, 5H), 6.92 (d, J = 8.3 Hz, 1H), 5.45 (s, 2H), 5.06 (qd, J = 7.1, 2.6 Hz, 1H), 4.77 (dd, J = 15.6, 7.1 Hz, 1H), 4.63 (dd, J = 15.6, 2.7 Hz, 1H), 4.56 - 4.30 (m, 4H), 3.86 (s, 3H), 2.80 - 2.62 (m, 1H), 2.45 - 2.34 (m, 1H), 2.29 (s, 3H).

### Example 154: (S)-2-(2,5-difluoro-4-(6-((5-((1-methyl-1H-pyrazol-4-yl)ethynyl)pyrimidin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((1-methyl-1H-pyrazol-4-yl)ethynyl)pyrimidin-2-yl)methoxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.83 (s, 2H), 8.57 (d, J = 1.3 Hz, 1H), 8.19 (dd, J = 8.6, 1.4 Hz, 1H), 7.89 (s, 1H), 7.83 (t, J = 7.9 Hz, 1H), 7.75 (d, J = 8.6 Hz, 1H), 7.66 (s, 1H), 7.64 - 7.50 (m, 2H), 7.33 (dd, J = 11.3, 6.0 Hz, 1H), 7.01 (d, J = 8.2 Hz, 1H), 5.64 (s, 2H), 5.25 (qd, J = 7.5, 2.4 Hz, 1H), 4.98 (dd, J = 15.5, 7.6 Hz, 1H), 4.84 - 4.63 (m, 4H), 4.53 (dt, J = 9.2, 5.9 Hz, 1H), 3.92 (s, 3H), 2.92 - 2.78 (m, 1H), 2.56 (ddt, J = 9.0, 7.0, 2.0 Hz, 1H).

### Example 155: (S)-2-(2,5-difluoro-4-(6-((5-((1-methyl-1H-pyrazol-4-yl)ethynyl)pyrazin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((1-methyl-1H-pyrazol-4-yl)ethynyl)pyrazin-2-yl)methoxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.97 - 8.79 (m, 3H), 8.50 (s, 1H), 8.14 (dd, J = 8.6, 1.4 Hz, 1H), 7.96 (d, J = 1.3 Hz, 1H), 7.86 (t, J = 7.9 Hz, 1H), 7.83 - 7.66 (m, 2H), 7.60 (dd, J = 7.5, 1.4 Hz, 1H), 7.32 (dd, J = 11.3, 6.0 Hz, 1H), 7.02 (d, J = 8.2 Hz, 1H), 5.70 (s, 2H), 5.24 (dd, J = 7.3, 2.4 Hz, 1H), 4.94 (d, J = 7.4 Hz, 1H), 4.84 - 4.62 (m, 4H), 4.51 (dt, J = 9.2, 5.9 Hz, 1H), 4.01 (s, 3H), 2.94 - 2.74 (m, 1H), 2.55 (ddt, J = 9.2, 7.3, 2.0 Hz, 1H).

### Example 156: (S)-2-(2,5-difluoro-4-(6-((5-((1-methyl-1H-imidazol-5-yl)ethynyl)pyrazin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((5-((1-methyl-1H-imidazol-5-yl)ethynyl)pyrazin-2-yl)methoxy)pyridin-2-yl)benzyl)-1-(oxetan-2-ylmethyl)-1H-benzo[d]imidazole-6-carboxylic acid was prepared in a manner as described in Procedure 6. 1H NMR (400 MHz, Methanol-d4) δ 8.96 - 8.79 (m, 3H), 8.50 (s, 1H), 8.14 (dd, J = 8.6, 1.4 Hz, 1H), 7.96 (d, J = 1.3 Hz, 1H), 7.86 (t, J = 7.9 Hz, 1H), 7.83 - 7.66 (m, 2H), 7.60 (dd, J = 7.5, 1.4 Hz, 1H), 7.32 (dd, J = 11.3, 6.0 Hz, 1H), 7.02 (d, J = 8.2 Hz, 1H), 5.70 (s, 2H), 5.24 (tt, J = 7.3, 3.7 Hz, 1H), 4.83 - 4.60 (m, 5H), 4.51 (dt, J = 9.2, 5.9 Hz, 1H), 4.01 (s, 3H), 2.85 (ddd, J = 10.5, 5.8, 2.7 Hz, 1H), 2.56 (ddd, J = 11.6, 8.1, 4.6 Hz, 1H).

### Example 274: 2-(2,5-difluoro-4-(6-((3-fluoro-5-((1-methyl-1H-pyrazol-4-yl)ethynyl)pyridin-2-yl)methoxy)pyridin-2-yl)benzyl)-4-fluoro-1-((1-(fluoromethyl)cyclopropyl)methyl)-1H-benzo[d]imidazole-6-carboxylic acid

2-(2,5-difluoro-4-(6-((3-fluoro-5-((1-methyl-1H-pyrazol-4-yl)ethynyl)pyridin-2-yl)methoxy)pyridin-2-yl)benzyl)-4-fluoro-1-((1-(fluoromethyl)cyclopropyl)methyl)-1H-benzo[d]imidazole-6-carboxylic acid was prepared in a manner as described in Procedure 6 using Intermediate **1-1192** and 4-ethynyl-1-methyl-pyrazole. ES/MS m/z: 699.3 (M+H⁺). 1H NMR (400 MHz, Methanol-d4) δ 8.45 (t, J = 1.2 Hz, 1H), 8.11 (d, J = 1.2 Hz, 1H), 7.88 - 7.76 (m, 2H), 7.75 - 7.64 (m, 3H), 7.60 (d, J = 1.7 Hz, 1H), 7.53 - 7.46 (m, 1H), 7.06 (dd, J = 11.3, 6.0 Hz, 1H), 6.87 (d, J = 8.2 Hz, 1H), 5.61 (d, J = 1.8 Hz, 2H), 4.46 (d, J = 12.6 Hz, 4H), 4.18 (s, 1H), 4.06 (s, 1H), 3.93 (s, 3H), 0.84 (t, J = 4.4 Hz, 2H), 0.80 (d, J = 4.5 Hz, 2H).

### Example 277: (S)-2-(2,5-difluoro-4-(6-((2-fluoro-6-((2-methoxythiazol-5-yl)ethynyl)pyridin-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-6-((2-methoxythiazol-5-yl)ethynyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1197** and 5-ethynyl-2-methoxythiazole. ES/MS m/z: 698.7 (M+H⁺). 1H NMR (400 MHz, Methanol-d4) δ 8.29 (d, J = 1.4 Hz, 1H), 8.12 - 7.95 (m, 2H), 7.86 - 7.76 (m, 2H), 7.69 (d, J = 8.5 Hz, 1H), 7.59 - 7.51 (m, 1H), 7.48 - 7.35 (m, 2H), 7.15 (dd, J = 11.3, 6.0 Hz, 1H), 6.90 (d, J = 8.2 Hz, 1H), 5.56 (s, 2H), 5.19 (qd, J = 7.3, 2.3 Hz, 1H), 4.74 - 4.37 (m, 6H), 4.13 (s, 3H), 2.80 (dq, J = 14.1, 7.6 Hz, 1H), 2.49 (p, J = 7.9 Hz, 1H).

### Example 278: (S)-2-(2,5-difluoro-4-(6-((2-fluoro-6-((1-methyl-1H-1,2,3-triazol-5-yl)ethynyl)pyridin-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-6-((1-methyl-1H-1,2,3-triazol-5-yl)ethynyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1197** and 5-ethynyl-1-methyltriazole. ES/MS m/z: 666.6 (M+H⁺). 1H NMR (400 MHz, Methanol-d4) δ 8.29 (s, 1H), 8.12 (t, J = 8.5 Hz, 1H), 8.05 - 7.95 (m, 2H), 7.84 - 7.74 (m, 2H), 7.69 (d, J = 8.5 Hz, 1H), 7.64 - 7.51 (m, 2H), 7.15 (dd, J = 11.2, 6.0 Hz, 1H), 6.91 (d, J = 8.3 Hz, 1H), 5.59 (s, 2H), 5.19 (d, J = 6.4 Hz, 1H), 4.75 - 4.41 (m, 6H), 4.21 (s, 3H), 2.81 (dd, J = 19.2, 8.9 Hz, 1H), 2.54 - 2.44 (m, 1H).

### Example 279: 2-(2,5-difluoro-4-(6-((2-fluoro-4-((1-methyl-1H-1,2,3-triazol-5-yl)ethynyl)benzyl)oxy)pyridin-2-yl)benzyl)-1-(4,4-dimethyltetrahydrofuran-3-yl)-1H-benzo[d]imidazole-6-carboxylic acid

2-(2,5-difluoro-4-(6-((2-fluoro-4-((1-methyl-1H-1,2,3-triazol-5-yl)ethynyl)benzyl)oxy)pyridin-2-yl)benzyl)-1-(4,4-dimethyltetrahydrofuran-3-yl)-1H-benzo[d]imidazole-6-carboxylic acid was prepared in a manner as described in Procedure 6 using Intermediates **1-1200** and 5-ethynyl-1-methyltriazole. ES/MS m/z: 693.6 (M+H⁺). 1H NMR (400 MHz, Methanol-d4) δ 8.88 (s, 1H), 8.16 (dd, J = 8.6, 1.4 Hz, 1H), 7.95 (s, 1H), 7.90 (dd, J = 10.8, 6.3 Hz, 1H), 7.84 (t, J = 7.9 Hz, 1H), 7.77 (d, J = 8.6 Hz, 1H), 7.68 - 7.55 (m, 2H), 7.49 - 7.32 (m, 3H), 6.95 (d, J = 8.2 Hz, 1H), 5.61 (s, 2H), 5.13 (d, J = 6.6 Hz, 1H), 4.77 - 4.60 (m, 3H), 4.52 (dd, J = 11.6, 6.7 Hz, 1H), 4.19 (s, 3H), 4.00 (d, J = 8.9 Hz, 1H), 3.84 (d, J = 8.9 Hz, 1H), 1.41 (s, 3H), 0.76 (s, 3H).

### Example 284: (S)-2-(2,5-difluoro-4-(6-((3-fluoro-5-((4-fluorophenyl)ethynyl)pyridin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((3-fluoro-5-((4-fluorophenyl)ethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1204** and 1-ethynyl-4-fluorobenzene. ES/MS m/z: 679.8 (M+H⁺). 1H NMR (400 MHz, Methanol-d4) δ 8.50 (s, 1H), 8.20 (s, 1H), 7.99 (d, J = 8.5 Hz, 1H), 7.79 (dd, J = 10.8, 6.3 Hz, 1H), 7.69 (dt, J = 16.1, 8.4 Hz, 3H), 7.59 - 7.44 (m, 3H), 7.08 (dt, J = 14.2, 7.2 Hz, 3H), 6.86 (d, J = 8.2 Hz, 1H), 5.62 (s, 2H), 5.16 (q, J = 7.5, 6.8 Hz, 1H), 4.61 - 4.51 (m, 3H), 4.45 (dt, J = 15.1, 7.4 Hz, 3H), 2.76 (dq, J = 13.9, 7.6 Hz, 1H), 2.45 (t, J = 9.5 Hz, 1H).

### Example 285: (S)-2-(2,5-difluoro-4-(6-((3-fluoro-5-(isothiazol-4-ylethynyl)pyridin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((3-fluoro-5-(isothiazol-4-ylethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1204** and 4-ethynylisothiazole. ES/MS m/z: 668.7 (M+H⁺). 1H NMR (400 MHz, Methanol-d4) δ 9.02 (s, 1H), 8.61 (s, 1H), 8.53 (s, 1H), 8.20 (s, 1H), 7.99 (d, J = 8.5 Hz, 1H), 7.78 (dd, J = 10.9, 6.4 Hz, 1H), 7.75 - 7.65 (m, 3H), 7.51 (d, J = 7.6 Hz, 1H), 7.06 (dd, J = 11.4, 6.0 Hz, 1H), 6.87 (d, J = 8.1 Hz, 1H), 5.64 (s, 2H), 5.17 (d, J = 7.0 Hz, 1H), 4.60 - 4.52 (m, 3H), 4.45 (dt, J = 15.4, 7.8 Hz, 3H), 2.77 (t, J = 9.1 Hz, 1H), 2.46 (p, J = 8.8 Hz, 1H).

### Example 286: (S)-2-(4-(6-((5-((1H-pyrazol-4-yl)ethynyl)-3-fluoropyridin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((1H-pyrazol-4-yl)ethynyl)-3-fluoropyridin-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **1-1204** and 4-ethynyl-1H-pyrazole. ES/MS m/z: 651.7 (M+H⁺). 1H NMR (400 MHz, Methanol-d4) δ 8.47 (t, J = 1.3 Hz, 1H), 8.20 (dd, J = 1.4, 0.7 Hz, 1H), 7.99 (dd, J = 8.5, 1.5 Hz, 1H), 7.86 - 7.75 (m, 3H), 7.70 (t, J = 8.0 Hz, 2H), 7.61 (dd, J = 10.0, 1.7 Hz, 1H), 7.52 - 7.46 (m, 1H), 7.06 (dd, J = 11.3, 6.0 Hz, 1H), 6.86 (d, J = 8.2 Hz, 1H), 5.61 (d, J = 1.9 Hz, 2H), 5.17 (qd, J = 7.0, 2.5 Hz, 1H), 4.60 - 4.51 (m, 3H), 4.51 - 4.39 (m, 3H), 2.86 - 2.62 (m, 1H), 2.56 - 2.34 (m, 1H).

### Example 287: (S)-2-(4-(6-((4-((1-(difluoromethyl)-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-((1-(difluoromethyl)-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **I-52** and 1-(difluoromethyl)-4-iodopyrazole. ES/MS m/z: 700.4 (M+H⁺). 1H NMR (400 MHz, Methanol-d4) δ 8.17 (d, J = 9.3 Hz, 2H), 8.00 (d, J = 8.5 Hz, 1H), 7.83 (d, J = 15.5 Hz, 2H), 7.70 (q, J = 7.9 Hz, 2H), 7.50 (d, J = 7.2 Hz, 2H), 7.43 - 7.15 (m, 3H), 7.06 (dd, J = 11.3, 6.0 Hz, 1H), 6.83 (d, J = 8.2 Hz, 1H), 5.54 (s, 2H), 5.30 - 5.10 (m, 1H), 4.60 - 4.51 (m, 3H), 4.51 - 4.37 (m, 3H), 2.77 (t, J = 9.3 Hz, 1H), 2.47 (h, J = 8.3, 7.8 Hz, 1H).

### Example 288: (S)-2-(2,5-difluoro-4-(6-((3-fluoro-5-((1-methyl-1H-1,2,3-triazol-5-yl)ethynyl)pyridin-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-fluoro-5-((1-methyl-1H-1,2,3-triazol-5-yl)ethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1204** and 5-ethynyl-1-methyltriazole. ES/MS m/z: 666.3 (M+H⁺). 1H NMR (400 MHz, Methanol-d4) δ 8.61 - 8.56 (m, 1H), 8.22 (d, J = 1.4 Hz, 1H), 8.02 - 7.89 (m, 2H), 7.88 - 7.71 (m, 3H), 7.68 (s, 1H), 7.51 (dd, J = 7.5, 1.4 Hz, 1H), 7.08 (dd, J = 11.4, 6.0 Hz, 1H), 6.88 (d, J = 8.2 Hz, 1H), 5.66 (d, J = 1.8 Hz, 2H), 5.18 (qd, J = 6.9, 2.5 Hz, 1H), 4.71 - 4.57 (m, 2H), 4.56 - 4.40 (m, 4H), 4.19 (s, 3H), 2.78 (dtd, J = 11.5, 8.2, 6.1 Hz, 1H), 2.47 (ddt, J = 11.3, 8.9, 7.2 Hz, 1H).

### Example 289: (S)-2-(2,5-difluoro-4-(6-((3-methyl-5-((1-methyl-1H-pyrazol-4-yl)ethynyl)pyridin-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-methyl-5-((1-methyl-1H-pyrazol-4-yl)ethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1205** and 4-ethynyl-1-methylpyrazole. ES/MS m/z: 661.2 (M+H⁺). 1H NMR (400 MHz, Methanol-d4) δ 8.44 (s, 1H), 8.23 (s, 1H), 7.99 (d, J = 8.3 Hz, 1H), 7.79 (s, 1H), 7.78 - 7.66 (m, 4H), 7.64 (s, 1H), 7.50 (dd, J = 7.6, 1.5 Hz, 1H), 7.10 (dd, J = 11.3, 5.9 Hz, 1H), 6.87 (d, J = 8.2 Hz, 1H), 5.56 (s, 2H), 5.18 (qd, J = 7.1, 2.5 Hz, 1H), 4.72 - 4.58 (m, 2H), 4.58 - 4.39 (m, 4H), 3.92 (s, 3H), 2.77 (dtd, J = 11.4, 8.1, 6.1 Hz, 1H), 2.47 (s, 4H).

### Example 291: (S)-2-(2,5-difluoro-4-(6-((3-fluoro-5-((2-methylthiazol-4-yl)ethynyl)pyridin-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-fluoro-5-((2-methylthiazol-4-yl)ethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1204** and 4-ethynyl-2-methylthiazole. ES/MS m/z: 682.1 (M+H⁺). 1H NMR (400 MHz, Methanol-d4) δ 8.60 - 8.50 (m, 2H), 8.20 (dd, J = 8.6, 1.4 Hz, 1H), 7.91 - 7.74 (m, 5H), 7.63 - 7.54 (m, 1H), 7.36 (dd, J = 11.3, 6.0 Hz, 1H), 6.95 (d, J = 8.3 Hz, 1H), 5.66 (d, J = 1.8 Hz, 2H), 5.26 (q, J = 7.0 Hz, 1H), 4.98 (dd, J = 15.5, 7.5 Hz, 1H), 4.86 - 4.76 (m, 3H), 4.70 (dd, J = 15.5, 8.8 Hz, 1H), 4.54 (dt, J = 9.0, 5.9 Hz, 1H), 2.95 - 2.80 (m, 1H), 2.74 (s, 3H), 2.65 - 2.51 (m, 1H).

### Example 292: (S)-2-(2,5-difluoro-4-(6-((3-fluoro-5-(thiazol-4-ylethynyl)pyridin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((3-fluoro-5-(thiazol-4-ylethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1204** and 4-ethynylthiazole. ES/MS m/z: 668.0 (M+H⁺). 1H NMR (400 MHz, Methanol-d4) δ 8.99 (s, 1H), 8.56 (s, 1H), 8.20 (s, 1H), 7.99 (s, 1H), 7.87 (s, 1H), 7.76-7.56 (m, 4H), 7.52 (s, 1H), 7.07 (s, 1H), 6.88 (s, 1H), 5.64 (s, 2H), 5.18 (s, 1H), 4.75-4.47 (m, 6H), 2.92 - 2.67 (m, 1H), 2.47 (s, 1H).

### Example 293: (S)-2-(2,5-difluoro-4-(6-((3-fluoro-5-((1-methyl-1H-imidazol-5-yl)ethynyl)pyridin-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-fluoro-5-((1-methyl-1H-imidazol-5-yl)ethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1204** and 5-ethynyl-1-methylthiazole. ES/MS m/z: 665.2 (M+H⁺). 1H NMR (400 MHz, Methanol-d4) δ 8.52 (s, 1H), 8.17 (s, 1H), 8.00 (dd, J = 8.5, 1.5 Hz, 1H), 7.78 (dd, J = 10.8, 6.3 Hz, 1H), 7.75 - 7.57 (m, 5H), 7.33 (s, 1H), 7.04 (dd, J = 11.3, 6.0 Hz, 1H), 6.86 (d, J = 8.2 Hz, 1H), 5.63 (d, J = 1.9 Hz, 2H), 5.16 (dt, J = 9.0, 4.6 Hz, 1H), 4.64 (q, J = 7.4 Hz, 1H), 4.57 (d, J = 6.0 Hz, 1H), 4.47 - 4.38 (m, 3H), 3.77 (s, 3H), 2.77 (dq, J = 14.5, 7.5 Hz, 1H), 2.46 (d, J = 9.2 Hz, 1H).

### Example 294: (S)-2-(2,5-difluoro-4-(6-((3-fluoro-5-((2-methoxythiazol-5-yl)ethynyl)pyridin-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((3-fluoro-5-((2-methoxythiazol-5-yl)ethynyl)pyridin-2-yl)methoxy)pyridin-2-yl)benzyl)-1-(oxetan-2-ylmethyl)-1H-benzo[d]imidazole-6-carboxylic acid was prepared in a manner as described in procedure 6 using Intermediate **1-1204** and 5-ethynyl-2-methoxythiazole. ES/MS m/z: 698.0 (M+H⁺). 1H NMR (400 MHz, Methanol-d4) δ 8.48 (s, 1H), 8.20 (d, J = 1.4 Hz, 1H), 8.00 (dd, J = 8.5, 1.5 Hz, 1H), 7.77 (dd, J = 10.8, 6.3 Hz, 1H), 7.74 - 7.67 (m, 2H), 7.64 (dd, J = 9.8, 1.7 Hz, 1H), 7.51 (d, J = 7.4 Hz, 1H), 7.39 (s, 1H), 7.06 (dd, J = 11.3, 6.0 Hz, 1H), 6.86 (d, J = 8.2 Hz, 1H), 5.62 (d, J = 1.9 Hz, 2H), 5.37 (s, 1H), 5.24 - 5.07 (m, 1H), 4.57 (dd, J = 15.9, 8.9 Hz, 2H), 4.52 - 4.39 (m, 3H), 4.11 (s, 3H), 2.77 (dq, J = 15.2, 7.8 Hz, 1H), 2.46 (dt, J = 18.6, 8.1 Hz, 1H).

### Example 295: (S)-2-(2,5-difluoro-4-(5-fluoro-6-((6-((1-methyl-1H-pyrazol-4-yl)ethynyl)pyridin-3-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(5-fluoro-6-((6-((1-methyl-1H-pyrazol-4-yl)ethynyl)pyridin-3-yl)methoxy)pyridin-2-yl)benzyl)-4-fluoro-1-(oxetan-2-ylmethyl)-1H-benzo[d]imidazole-6-carboxylic acid was prepared in a manner as described in Procedure 6 using Intermediate **1-1208** and 4-ethynyl-1-methylpyrazole. ES/MS m/z: 683.2 (M+H⁺). 1H NMR (400 MHz, Methanol-d4) δ 8.65 (s, 1H), 8.00 (s, 1H), 7.91 (d, J = 8.0 Hz, 1H), 7.84 - 7.61 (m, 3H), 7.49 (d, J = 6.3 Hz, 2H), 7.09 (dd, J = 11.8, 5.9 Hz, 1H), 5.59 (s, 2H), 5.14 (d, J = 7.2 Hz, 1H), 4.57 - 4.38 (m, 6H), 3.92 (s, 3H), 2.77 (t, J = 9.4 Hz, 1H), 2.44 (s, 1H).

### Example 296: (S)-2-(2,5-difluoro-4-(5-fluoro-6-((3-fluoro-5-((1-methyl-1H-pyrazol-4-yl)ethynyl)pyridin-2-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(5-fluoro-6-((3-fluoro-5-((1-methyl-1H-pyrazol-4-yl)ethynyl)pyridin-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 6 using Intermediate **1-1209** and 4-ethynyl-1-methylpyrazole. ES/MS m/z: 701.2 (M+H⁺). 1H NMR (400 MHz, Methanol-d4) δ 8.48 (s, 1H), 8.03 (s, 1H), 7.78 (d, J = 26.4 Hz, 3H), 7.72-7.55 (m, 2H), 7.51 (d, J = 7.6 Hz, 2H), 7.09 (s, 1H), 5.71 (s, 2H), 5.16 (s, 1H), 4.78-4.49 (m, J = 14.8 Hz, 3H), 4.47 (d, J = 15.9 Hz, 3H), 3.94 (s, 3H), 2.77 (d, J = 10.5 Hz, 1H), 2.58 - 2.38 (m, 1H).

### Example 300: (S)-2-(4-(6-((4-chloro-6-((1-methyl-1H-pyrazol-4-yl)ethynyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((1-methyl-1H-pyrazol-4-yl)ethynyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **1-24** and 4-ethynyl-1-methylpyrazole. ES/MS m/z: 681.0 (M+H⁺). 1HNMR (400 MHz, Methanol-d4) δ 8.66 (s, 1H), 8.52 (d, J = 1.3 Hz, 1H), 8.16 (dd, J = 8.6, 1.5 Hz, 1H), 7.96 (s, 1H), 7.93 - 7.79 (m, 2H), 7.76 (d, J = 8.6 Hz, 1H), 7.72 (s, 1H), 7.67 (dd, J = 12.5, 7.4 Hz, 1H), 7.59 (d, J = 7.6 Hz, 1H), 7.35 (dd, J = 11.3, 6.1 Hz, 1H), 6.97 (d, J = 8.2 Hz, 1H), 5.67 (s, 2H), 5.25 (q, J = 6.4, 5.8 Hz, 1H), 4.94 (dd, J = 15.6, 7.5 Hz, 1H), 4.85 - 4.63 (m, 4H), 4.52 (dt, J = 9.2, 5.9 Hz, 1H), 3.94 (s, 3H), 2.86 (dt, J = 19.4, 7.8 Hz, 1H), 2.66 - 2.46 (m, 1H).

### Example 301: (S)-2-(2,5-difluoro-4-(6-((2-fluoro-4-((1-methyl-1H-1,2,3-triazol-4-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((1-methyl-1H-1,2,3-triazol-4-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6 using Intermediate **I-52** and 4-bromo-1-methyltriazole. ES/MS m/z: 665.2 (M+H⁺). 1H NMR (400 MHz, Methanol-d4) δ 8.58 (d, J = 1.3 Hz, 1H), 8.25 - 8.16 (m, 2H), 7.96 - 7.73 (m, 3H), 7.65 (m, 2H), 7.46 - 7.30 (m, 3H), 6.94 (d, J = 8.2 Hz, 1H), 5.58 (s, 2H), 5.27 (q, J = 6.8 Hz, 1H), 5.00 (dd, J = 15.5, 7.6 Hz, 1H), 4.84 - 4.58 (m, 3H), 4.55 (dt, J = 9.2, 6.0 Hz, 1H), 4.15 (s, 3H), 2.96 - 2.80 (m, 1H), 2.65 - 2.47 (m, 1H).

### Example 302: (S)-2-(4-(6-((3-chloro-5-((1-methyl-1H-pyrazol-4-yl)ethynyl)pyridin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((3-chloro-5-((1-methyl-1H-pyrazol-4-yl)ethynyl)pyridin-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **1-1210** and 4-ethynyl-1-methylpyrazole. ES/MS m/z: 681.0 (M+H⁺). 1HNMR (400 MHz, Methanol-d4) δ 8.56 (d, J = 1.4 Hz, 1H), 8.52 (d, J = 1.8 Hz, 1H), 8.19 (dd, J = 8.6, 1.4 Hz, 1H), 7.99 (d, J = 1.8 Hz, 1H), 7.90 (s, 1H), 7.88 - 7.73 (m, 3H), 7.64 (d, J = 1.6 Hz, 2H), 7.34 (dd, J = 11.3, 6.0 Hz, 1H), 6.96 (d, J = 8.3 Hz, 1H), 5.69 (s, 2H), 5.25 (dd, J = 8.1, 5.9 Hz, 1H), 4.97 (dd, J = 15.5, 7.5 Hz, 1H), 4.85 - 4.75 (m, 3H), 4.75 - 4.63 (m, 1H), 4.53 (dt, J = 9.1, 5.9 Hz, 1H), 3.93 (s, 3H), 2.93 - 2.78 (m, 1H), 2.68 - 2.51 (m, 1H).

### Example 303: (S)-2-(2,5-difluoro-4-(6-((3-fluoro-5-(thiazol-5-ylethynyl)pyridin-2-yl)methoxy)pyridin-2-yl)benzyl)-1-(oxetan-2-ylmethyl)-1H-benzo [d] imidazole-6-carboxylic acid

(S)-2-(2,5-difluoro-4-(6-((3-fluoro-5-(thiazol-5-ylethynyl)pyridin-2-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1204** and 5-ethynylthiazole. ES/MS m/z: 668.2 (M+H⁺). 1H NMR (400 MHz, Methanol-d4) δ 9.07 (s, 1H), 8.56 (s, 1H), 8.30 (s, 1H), 8.18 (s, 1H), 7.98 (dd, J = 8.5, 1.5 Hz, 1H), 7.86 (dd, J = 10.1, 1.6 Hz, 1H), 7.84 - 7.72 (m, 2H), 7.66 (d, J = 8.5 Hz, 1H), 7.55 (d, J = 7.4 Hz, 1H), 7.17 (dd, J = 11.5, 6.0 Hz, 1H), 6.91 (d, J = 8.2 Hz, 1H), 5.67 (d, J = 1.7 Hz, 2H), 5.20 (d, J = 8.0 Hz, 1H), 4.77 - 4.51 (m, 5H), 4.51 - 4.35 (m, 1H), 2.79 (dt, J = 16.5, 7.6 Hz, 1H), 2.50 (dd, J = 10.5, 8.1 Hz, 1H).

### Example 304: (S)-2-(2,5-difluoro-4-(6-((2-methoxy-4-((1-methyl-1H-pyrazol-4-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-methoxy-4-((1-methyl-1H-pyrazol-4-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6 using Intermediate **1-1211** and 4-ethynyl-1-methylpyrazole. ES/MS m/z: 676.2 (M+H⁺). 1H NMR (400 MHz, Methanol-d4) δ 8.54 (s, 1H), 8.17 (dd, J = 8.6, 1.4 Hz, 1H), 7.89 (dd, J = 10.9, 6.3 Hz, 1H), 7.86 - 7.72 (m, 3H), 7.63 (s, 1H), 7.56 (d, J = 7.6 Hz, 1H), 7.39 (d, J = 7.8 Hz, 1H), 7.33 (dd, J = 11.3, 6.0 Hz, 1H), 7.10 (d, J = 1.4 Hz, 1H), 7.05 (dd, J = 7.7, 1.4 Hz, 1H), 6.91 (d, J = 8.2 Hz, 1H), 5.52 (s, 2H), 5.25 (q, J = 6.6 Hz, 1H), 4.95 (dd, J = 15.6, 7.5 Hz, 1H), 4.86 - 4.65 (m, 4H), 4.53 (dt, J = 9.0, 5.9 Hz, 1H), 3.92 (s, 6H), 2.86 (dt, J = 16.4, 7.8 Hz, 1H), 2.70 - 2.48 (m, 1H).

### Example 305: (S)-2-(2,5-difluoro-4-(6-((4-((2-methylpyrimidin-5-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((4-((2-methylpyrimidin-5-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6 using Intermediate **1-1212** and 5-ethynyl-2-methylpyrimidine. ES/MS m/z: 658.2 (M+H⁺). 1H NMR (400 MHz, Methanol-d4) δ 8.82 (s, 2H), 8.56 (s, 1H), 8.20 (dd, J = 8.6, 1.4 Hz, 1H), 7.90 - 7.73 (m, 3H), 7.62 - 7.49 (m, 6H), 7.36 (dd, J = 11.2, 6.0 Hz, 1H), 6.95 (d, J = 8.3 Hz, 1H), 5.54 (s, 2H), 5.26 (q, J = 6.7 Hz, 1H), 4.98 (dd, J = 15.5, 7.5 Hz, 1H), 4.86 - 4.75 (m, 3H), 4.70 (dd, J = 14.7, 8.1 Hz, 1H), 4.54 (dt, J = 9.1, 6.0 Hz, 1H), 2.87 (dt, J = 19.7, 8.1 Hz, 1H), 2.73 (s, 3H), 2.65 - 2.53 (m, 1H).

### Example 306: (S)-2-(2,5-difluoro-4-(6-((4-((1-methyl-1H-pyrazol-4-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((4-((1-methyl-1H-pyrazol-4-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6 using Intermediate **1-1212** and 4-ethynyl-1-methylpyrazole. ES/MS m/z: 646.2 (M+H⁺). 1H NMR (400 MHz, Methanol-d4) δ 8.63 - 8.57 (m, 1H), 8.23 (dd, J = 8.6, 1.4 Hz, 1H), 7.89 (dd, J = 10.8, 6.3 Hz, 1H), 7.86 - 7.75 (m, 3H), 7.74 - 7.52 (m, 6H), 7.39 (dd, J = 11.2, 6.0 Hz, 1H), 6.93 (d, J = 8.2 Hz, 1H), 5.51 (s, 2H), 5.27 (qd, J = 7.6, 2.4 Hz, 1H), 5.01 (dd, J = 15.5, 7.6 Hz, 1H), 4.85 - 4.75 (m, 2H), 4.70 (td, J = 7.8, 6.0 Hz, 1H), 4.55 (dt, J = 9.2, 6.0 Hz, 1H), 3.92 (s, 3H), 2.97 - 2.82 (m, 1H), 2.70 - 2.52 (m, 1H).

### Example 356: (S)-2-(2,5-difluoro-4-(6-((2-fluoro-4-((2-oxo-1,2-dihydropyridin-4-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((2-oxo-1,2-dihydropyridin-4-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6 using Intermediate **I-52** and 4-bromo-1H-pyridin-2-one. ES/MS m/z: 677.3 (M+H⁺). 1HNMR (400 MHz, DMSO-d6) δ 8.27 (d, J = 1.5 Hz, 1H), 7.88 (t, J = 7.8 Hz, 1H), 7.84 - 7.76 (m, 2H), 7.68 - 7.58 (m, 2H), 7.58 - 7.49 (m, 2H), 7.47 - 7.44 (m, 1H), 7.41 (td, J = 11.6, 5.3 Hz, 2H), 6.97 (d, J = 8.3 Hz, 1H), 6.51 (d, J = 1.6 Hz, 1H), 6.24 (d, J = 6.8 Hz, 1H), 5.55 (s, 2H), 5.07 (d, J = 6.3 Hz, 1H), 4.77 (dd, J = 15.6, 7.1 Hz, 1H), 4.63 (d, J = 14.6 Hz, 1H), 4.58 - 4.42 (m, 3H), 4.36 (dt, J = 9.1, 5.9 Hz, 1H), 2.77 - 2.68 (m, 1H), 2.39 (t, J = 9.9 Hz, 1H).

### Example 357: (S)-2-(2,5-difluoro-4-(6-((2-fluoro-4-((1-methyl-2-oxo-1,2-dihydropyridin-4-yl)ethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-4-((1-methyl-2-oxo-1,2-dihydropyridin-4-yl)ethynyl)benzyl)oxy)pyridin-2-yl)benzyl)-1-(oxetan-2-ylmethyl)-1H-benzo[d]imidazole-6-carboxylic acid was prepared in a manner as described in Procedure 6 using Intermediate **I-52** and 4-bromo-1methylpyridin-2-one. ES/MS m/z: 691.2 (M+H⁺). 1H NMR (400 MHz, DMSO-d6) δ 8.28 (s, 1H), 7.88 (t, J = 7.9 Hz, 1H), 7.81 (t, J = 3.2 Hz, 1H), 7.80 - 7.77 (m, 1H), 7.75 (d, J = 6.9 Hz, 1H), 7.67 - 7.60 (m, 2H), 7.55 - 7.50 (m, 2H), 7.45 (dd, J = 7.8, 1.5 Hz, 1H), 7.39 (dd, J = 11.5, 6.1 Hz, 1H), 6.97 (d, J = 8.3 Hz, 1H), 6.57 (d, J = 1.8 Hz, 1H), 6.30 (dd, J = 7.0, 1.9 Hz, 1H), 5.55 (s, 2H), 5.07 (d, J = 7.0 Hz, 1H), 4.77 (dd, J = 15.6, 7.1 Hz, 1H), 4.71 - 4.59 (m, 1H), 4.59 - 4.42 (m, 3H), 4.42 - 4.29 (m, 1H), 3.43 (s, 3H), 2.72 (t, J = 9.1 Hz, 1H), 2.45 - 2.29 (m, 1H).

### Example 358: (S)-2-(4-(6-((6-chloro-5-((1-methyl-1H-pyrazol-4-yl)ethynyl)pyridin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-chloro-5-((1-methyl-1H-pyrazol-4-yl)ethynyl)pyridin-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **I-1047** and 4-ethynyl-1-methylpyrazole. ES/MS m/z: 681.1 (M+H⁺). 1H NMR (400 MHz, DMSO-d6) δ 8.26 (d, J = 1.6 Hz, 1H), 8.15 (s, 1H), 8.05 (d, J = 7.9 Hz, 1H), 7.91 (t, J = 7.9 Hz, 1H), 7.79 (dd, J = 8.5, 1.5 Hz, 1H), 7.73 (s, 1H), 7.67 (dd, J = 10.5, 6.4 Hz, 1H), 7.60 (d, J = 8.5 Hz, 1H), 7.52 (dd, J = 7.7, 4.7 Hz, 2H), 7.36 (dd, J = 11.5, 6.1 Hz, 1H), 7.04 (d, J = 8.2 Hz, 1H), 5.54 (s, 2H), 5.06 (d, J = 6.6 Hz, 1H), 4.75 (dd, J = 15.5, 7.1 Hz, 1H), 4.62 (d, J = 15.4 Hz, 1H), 4.57 - 4.40 (m, 3H), 4.35 (dt, J = 9.0, 5.9 Hz, 1H), 3.86 (s, 3H), 2.72 (d, J = 9.7 Hz, 1H), 2.39 (q, J = 10.1, 9.0 Hz, 1H).

### Example 359: (S)-2-(4-(6-((4-((5-carbamoyl-1-methyl-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-((5-carbamoyl-1-methyl-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **I-1043** and **I-1048.** ES/MS m/z: 707.3 (M+H⁺). 1H NMR (400 MHz, DMSO-d6) δ 8.26 (d, J = 1.5 Hz, 1H), 7.99 (s, 1H), 7.88 (t, J = 7.9 Hz, 1H), 7.84 - 7.78 (m, 2H), 7.77 (s, 1H), 7.69 (s, 1H), 7.63 - 7.58 (m, 2H), 7.56 - 7.49 (m, 1H), 7.44 (dd, J = 10.7, 1.5 Hz, 1H), 7.41 - 7.35 (m, 2H), 6.96 (d, J = 8.3 Hz, 1H), 5.54 (s, 2H), 5.07 (d, J = 7.0 Hz, 1H), 4.76 (dd, J = 15.6, 7.2 Hz, 1H), 4.63 (d, J = 15.1 Hz, 1H), 4.56 - 4.41 (m, 3H), 4.35 (dt, J = 9.1, 6.0 Hz, 1H), 3.99 (s, 3H), 2.77 - 2.68 (m, 1H), 2.39 (t, J = 10.0 Hz, 1H).

### Example 360: (S)-2-(2,5-difluoro-4-(6-((2-methoxy-6-((1-methyl-1H-pyrazol-4-yl)ethynyl)pyridin-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((2-methoxy-6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **I-1046** and 4-ethynyl-1-methylpyrazole. ES/MS m/z: 677.2 (M+H⁺). 1H NMR (400 MHz, DMSO-d6) δ 8.28 (d, J = 1.5 Hz, 1H), 8.15 (s, 1H), 7.88 (t, J = 7.9 Hz, 1H), 7.84 - 7.73 (m, 4H), 7.61 (d, J = 8.4 Hz, 1H), 7.51 (dd, J = 7.5, 1.7 Hz, 1H), 7.39 (dd, J = 11.5, 6.1 Hz, 1H), 7.19 (d, J = 7.4 Hz, 1H), 6.98 (d, J = 8.2 Hz, 1H), 5.44 (s, 2H), 5.12 - 5.02 (m, 1H), 4.77 (dd, J = 15.5, 7.1 Hz, 1H), 4.71 - 4.55 (m, 1H), 4.55 - 4.41 (m, 3H), 4.36 (dt, J = 8.9, 5.9 Hz, 1H), 3.94 (s, 3H), 3.86 (s, 3H), 2.72 (dd, J = 12.2, 5.9 Hz, 1H), 2.39 (t, J = 9.9 Hz, 1H).

### Example 361: (S)-2-(4-(6-((4-((5-chloro-1-methyl-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-((5-chloro-1-methyl-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediates **I-1043** and **I-1393.** ES/MS m/z: 698.3 (M+H⁺). 1H NMR (400 MHz, DMSO-d6) δ 8.28 (d, J = 1.5 Hz, 1H), 7.88 (t, J = 7.9 Hz, 1H), 7.85 (s, 1H), 7.84 - 7.77 (m, 2H), 7.60 (dd, J = 9.6, 8.2 Hz, 2H), 7.52 (dd, J = 7.6, 1.7 Hz, 1H), 7.46 - 7.33 (m, 3H), 6.96 (d, J = 8.3 Hz, 1H), 5.54 (s, 2H), 5.08 (dd, J = 9.3, 6.6 Hz, 1H), 4.77 (dd, J = 15.6, 7.1 Hz, 1H), 4.64 (dd, J = 15.5, 2.7 Hz, 1H), 4.60 - 4.43 (m, 3H), 4.36 (dt, J = 9.1, 6.0 Hz, 1H), 3.84 (s, 3H), 2.72 (dt, J = 9.9, 7.0 Hz, 1H), 2.39 (ddd, J = 17.3, 8.9, 7.1 Hz, 1H).

### Example 362: (S)-2-(2,5-difluoro-4-(6-((2-fluoro-6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((2-fluoro-6-((1-methyl-1H-pyrazol-4-yl)ethynyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 6 using Intermediate **1-1045** and 4-ethynyl-1-methylpyrazole. ES/MS m/z: 665.2 (M+H⁺). 1H NMR (400 MHz, DMSO-d6) δ 8.27 (d, J = 1.5 Hz, 1H), 8.19 (s, 1H), 8.11 (dd, J = 9.8, 7.6 Hz, 1H), 7.90 (t, J = 7.9 Hz, 1H), 7.87 - 7.74 (m, 3H), 7.61 (d, J = 8.4 Hz, 1H), 7.54 (dt, J = 7.5, 1.8 Hz, 2H), 7.40 (dd, J = 11.5, 6.1 Hz, 1H), 6.99 (d, J = 8.2 Hz, 1H), 5.54 (s, 2H), 5.07 (d, J = 7.6 Hz, 1H), 4.77 (dd, J = 15.6, 7.0 Hz, 1H), 4.68 - 4.60 (m, 1H), 4.59 - 4.40 (m, 3H), 4.36 (dt, J = 9.0, 6.0 Hz, 1H), 3.88 (s, 3H), 2.73 (q, J = 8.1 Hz, 1H), 2.41 (q, J = 9.9, 8.9 Hz, 1H).

### Example 363: (S)-2-(4-(6-((2-chloro-6-((1-methyl-1H-pyrazol-4-yl)ethynyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-chloro-6-((1-methyl-1H-pyrazol-4-yl)ethynyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **I-1044** and 4-ethynyl-1-methylpyrazole. ES/MS m/z: 681.2 (M+H⁺). 1H NMR (400 MHz, DMSO-d6) δ 8.27 (d, J = 1.5 Hz, 1H), 8.20 (s, 1H), 8.02 (d, J = 7.8 Hz, 1H), 7.92 (t, J = 7.9 Hz, 1H), 7.83 - 7.74 (m, 3H), 7.67 - 7.59 (m, 3H), 7.59 - 7.52 (m, 1H), 7.39 (dd, J = 11.5, 6.0 Hz, 1H), 7.03 (d, J = 8.2 Hz, 1H), 5.57 (s, 2H), 5.13 - 5.03 (m, 1H), 4.77 (dd, J = 15.6, 7.1 Hz, 1H), 4.63 (d, J = 14.6 Hz, 1H), 4.56 - 4.42 (m, 3H), 4.36 (dt, J = 9.0, 5.9 Hz, 1H), 3.88 (s, 3H), 2.72 (dq, J = 13.0, 6.7, 5.9 Hz, 1H), 2.43 - 2.31 (m, 1H).

### Example 364: (S)-2-(4-(6-((4-((1,5-dimethyl-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-((1,5-dimethyl-1H-pyrazol-4-yl)ethynyl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 6 using Intermediate **I-1043** and **I-1394.** ES/MS m/z: 678.2 (M+H⁺). 1HNMR (400 MHz, DMSO-d6) δ 8.26 (s, 1H), 7.88 (t, J = 7.9 Hz, 1H), 7.84 - 7.76 (m, 2H), 7.62 - 7.54 (m, 4H), 7.51 (d, J = 7.3 Hz, 1H), 7.38 (dd, J = 11.3, 6.3 Hz, 2H), 7.33 (d, J = 7.9 Hz, 1H), 6.96 (d, J = 8.3 Hz, 1H), 5.52 (s, 2H), 5.06 (dt, J = 9.6, 4.8 Hz, 1H), 4.75 (dd, J = 15.5, 7.1 Hz, 1H), 4.62 (dd, J = 15.6, 2.8 Hz, 1H), 4.57 - 4.40 (m, 3H), 4.36 (dd, J = 9.0, 5.9 Hz, 1H), 3.75 (s, 3H), 2.76 - 2.69 (m, 1H), 2.39 (s, 1H), 2.35 (s, 3H).

### Example 390: (S)-2-(2,5-difluoro-4-(6-((3-fluoro-5-(thiazol-5-ylethynyl)pyridin-2-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(6-((3-fluoro-5-(thiazol-5-ylethynyl)pyridin-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 6 using Intermediate **I-1269** and 5-ethynylthiazole. ES/MS m/z: 686.0 (M+H⁺). 1HNMR (400 MHz, DMSO-d6) δ 9.24 (d, J = 0.7 Hz, 1H), 8.63 (t, J = 1.4 Hz, 1H), 8.30 (d, J = 0.7 Hz, 1H), 8.15 (d, J = 1.3 Hz, 1H), 8.09 (dd, J = 10.4, 1.7 Hz, 1H), 7.88 (t, J = 7.9 Hz, 1H), 7.73 (dd, J = 10.6, 6.5 Hz, 1H), 7.60 - 7.45 (m, 2H), 7.39 (dd, J = 11.6, 6.1 Hz, 1H), 6.97 (d, J = 8.2 Hz, 1H), 5.64 (d, J = 1.8 Hz, 2H), 5.17 - 4.98 (m, 1H), 4.79 (dd, J = 15.6, 7.1 Hz, 1H), 4.65 (dd, J = 15.5, 2.7 Hz, 1H), 4.59 - 4.42 (m, 3H), 4.35 (dt, J = 9.0, 5.9 Hz, 1H), 2.71 (ddd, J = 16.8, 9.5, 4.9 Hz, 1H), 2.45 - 2.26 (m, 1H).

### Example 391: (S)-2-(2,5-difluoro-4-(6-((6-((1-methyl-1H-pyrazol-4-yl)ethynyl)pyridin-3-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(6-((6-((1-methyl-1H-pyrazol-4-yl)ethynyl)pyridin-3-yl)methoxy)pyridin-2-yl)benzyl)-4-fluoro-1-(oxetan-2-ylmethyl)-1H-benzo[d]imidazole-6-carboxylic acid was prepared in a manner as described in Procedure 6 using Intermediate **I-1281** and 4-ethynyl-1-methylpyrazole. ES/MS m/z: 665.2 (M+H⁺). 1H NMR (400 MHz, DMSO-d6) δ 8.71 (d, J = 2.2 Hz, 1H), 8.25 - 8.08 (m, 2H), 7.98 - 7.80 (m, 3H), 7.75 (s, 1H), 7.69 - 7.48 (m, 6H), 7.41 (dd, J = 11.4, 6.1 Hz, 1H), 6.98 (d, J = 8.2 Hz, 1H), 5.54 (s, 2H), 5.14 - 5.02 (m, 1H), 4.80 (dd, J = 15.6, 7.1 Hz, 1H), 4.66 (dd, J = 15.5, 2.7 Hz, 1H), 4.61 - 4.43 (m, 3H), 4.36 (dt, J = 9.0, 5.9 Hz, 1H), 3.87 (s, 3H), 2.83 - 2.62 (m, 1H), 2.45 - 2.31 (m, 1H).

### Example 392: (S)-2-(2,5-difluoro-4-(6-((3-fluoro-5-((1-methyl-1H-pyrazol-4-yl)ethynyl)pyridin-2-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(6-((3-fluoro-5-((1-methyl-1H-pyrazol-4-yl)ethynyl)pyridin-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 6 using Intermediate **I-1269** and 4-ethynyl-1-methylpyrazole. ES/MS m/z: 683.2 (M+H⁺). 1H NMR (400 MHz, DMSO-d6) δ 8.53 (t, J = 1.4 Hz, 1H), 8.22 - 8.07 (m, 2H), 7.94 (dd, J = 10.5, 1.7 Hz, 1H), 7.88 (t, J = 7.9 Hz, 1H), 7.80 - 7.68 (m, 2H), 7.55 - 7.45 (m, 2H), 7.39 (dd, J = 11.6, 6.1 Hz, 1H), 6.96 (d, J = 8.3 Hz, 1H), 5.61 (d, J = 1.8 Hz, 2H), 5.07 (qd, J = 7.0, 2.6 Hz, 1H), 4.79 (dd, J = 15.6, 7.1 Hz, 1H), 4.65 (dd, J = 15.6, 2.7 Hz, 1H), 4.60 - 4.42 (m, 3H), 4.36 (dt, J = 9.0, 5.9 Hz, 1H), 3.87 (s, 3H), 2.76 - 2.61 (m, 1H), 2.38 (ddt, J = 11.1, 8.7, 7.0 Hz, 1H).

### Example 395: (S)-2-(2,5-difluoro-4-(6-((2-fluoro-4-((1-methyl-1H-imidazol-5-yl)ethynyl)benzyl)oxy)pyridin-2-yl)benzyl)-4-fluoro-1-(oxetan-2-ylmethyl)-1H-benzo[d]imidazole-6-carboxylic acid

(S)-2-(2,5-difluoro-4-(6-((2-fluoro-4-((1-methyl-1H-imidazol-5-yl)ethynyl)benzyl)oxy)pyridin-2-yl)benzyl)-4-fluoro-1-(oxetan-2-ylmethyl)-1H-benzo[d]imidazole-6-carboxylic acid was prepared in a manner as described in Procedure 6 using Intermediate 1-20 and 5-ethynyl-1-methyl-1H-imidazole. ES/MS m/z: 682.2 (M+H⁺). 1H NMR (400 MHz, DMSO-d6) δ 8.16 (d, J = 1.3 Hz, 1H), 7.94 - 7.77 (m, 3H), 7.62 (t, J = 7.8 Hz, 1H), 7.60 - 7.46 (m, 3H), 7.46 - 7.37 (m, 2H), 7.35 (s, 1H), 6.97 (d, J = 8.2 Hz, 1H), 5.55 (s, 2H), 5.07 (d, J = 7.0 Hz, 1H), 4.80 (dd, J = 15.6, 7.1 Hz, 1H), 4.71 - 4.62 (m, 1H), 4.60 - 4.43 (m, 3H), 4.36 (dt, J = 9.0, 6.0 Hz, 1H), 3.72 (s, 3H), 2.72 (dd, J = 12.3, 6.2 Hz, 1H), 2.40 (q, J = 9.8, 8.5 Hz, 1H).

### Example 404: (S)-2-(4-(6-((5-((4-cyanophenyl)ethynyl)pyrimidin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((4-cyanophenyl)ethynyl)pyrimidin-2-yl)methoxy)pyridin-2-yl)-2,5-difluorobenzyl)-1-(oxetan-2-ylmethyl)-1H-benzo[d]imidazole-6-carboxylic acid was prepared in a manner described in Procedure 6 using Intermediates **I-1333** and 4-ethynylbenzonitrile. ES/MS m/z: 669.4 (M+H⁺). 1H NMR (400 MHz, Methanol-d4) δ 8.94 (d, J = 1.0 Hz, 2H), 8.54 (s, 1H), 8.16 (dd, J = 8.6, 1.5 Hz, 1H), 7.91 - 7.76 (m, 3H), 7.76 - 7.63 (m, 3H), 7.63 - 7.49 (m, 2H), 7.31 (dd, J = 11.3, 6.0 Hz, 1H), 7.02 (d, J = 8.2 Hz, 1H), 5.67 (s, 2H), 5.24 (qd, J = 7.4, 2.3 Hz, 1H), 5.02 - 4.93 (m, 1H), 4.82 - 4.65 (m, 4H), 4.51 (dt, J = 9.4, 6.0 Hz, 1H), 2.98 - 2.75 (m, 1H), 2.62 - 2.42 (m, 1H).

### Example 405: (S)-2-(2,5-difluoro-4-(6-((4-(pyrimidin-5-ylethynyl)benzyl)oxy)pyridin-2-yl)benzyl)-1-(oxetan-2-ylmethyl)-1H-benzo[d]imidazole-6-carboxylic acid

(S)-2-(2,5-difluoro-4-(6-((4-(pyrimidin-5-ylethynyl)benzyl)oxy)pyridin-2-yl)benzyl)-1-(oxetan-2-ylmethyl)-1H-benzo[d]imidazole-6-carboxylic acid was prepared in a manner as described in Procedure 6 using Intermediates **I-1332** and 5-ethynylpyrimidine. ES/MS m/z: 644.2 (M+H⁺). 1H NMR (400 MHz, Methanol-d4) δ 9.12 (s, 1H), 8.93 (s, 2H), 8.58 (d, J = 1.3 Hz, 1H), 8.21 (dd, J = 8.6, 1.4 Hz, 1H), 7.93 - 7.74 (m, 4H), 7.67 - 7.52 (m, 4H), 7.37 (dd, J = 11.2, 6.0 Hz, 1H), 6.95 (d, J = 8.2 Hz, 1H), 5.55 (s, 2H), 5.33 - 5.19 (m, 1H), 4.99 (dd, J = 15.5, 7.5 Hz, 1H), 4.85 - 4.66 (m, 4H), 4.54 (dt, J = 9.2, 6.0 Hz, 1H), 2.96 - 2.77 (m, 1H), 2.58 (ddt, J = 9.0, 7.1, 2.0 Hz, 1H).

### Example 157: 2-[[4-[6-[[4-(2-cyclopropylthiazol-4-yl)-2-fluoro-phenyl]methoxy]-2-pyridyl]-2,5-difluoro-phenyl]methyl]-3-[[(2S)-oxetan-2-yl]methyl]benzimidazole-5-carboxylic acid

### Procedure 7

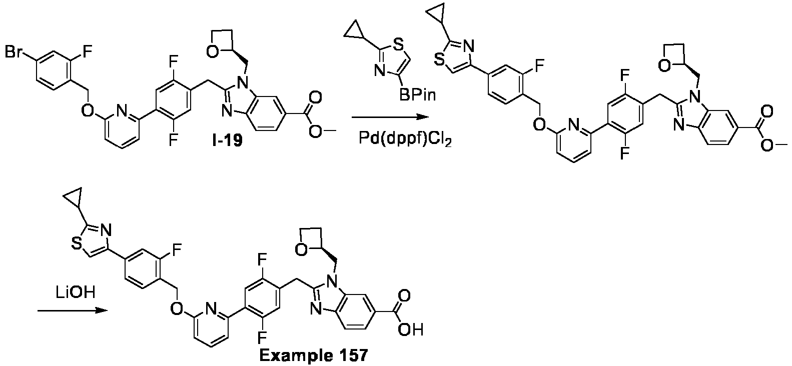

**Methyl 2-[[4-[6-[[4-(2-cyclopropylthiazol-4-yl)-2-fluoro-phenyl]methoxy]-2-pyridyl]-2,5-difluoro-phenyl]methyl]-3-[[(2S)-oxetan-2-yl]methyl]benzimidazole-5-carboxylate:** Methyl 2-[[4-[6-[(4-bromo-2-fluoro-phenyl)methoxy]-2-pyridyl]-2,5-difluorophenyl]methyl]-3-[[(2S)-oxetan-2-yl]methyl]benzimidazole-5-carboxylate (50 mg, 0.077 mmol), 2-cyclopropyl-4-(4,4,5,5-tetramethyl-1,3,2-dioxaborolan-2-yl)thiazole (38.5 mg, 0.15 mmol), 2 N of sodium carbonate (0.08 mL, 0.16 mmol) and (1,1'-bis(diphenylphosphino)ferrocene)-dichloropalladium(II) (5.7 mg, 0.008 mmole) was suspended in dioxane (0.5 mL). The reaction mixture was degassed by nitrogen. After which, the reaction mixture was heated to 120 °C in the microwave reactor for 30 min. Upon completion the solvent was removed, and the crude residue was dissolved in 1 mL of DMF. The mixture was then filtered and purified by RP-HPLC (eluent: water / MeCN 0.1% TFA) to give the desired product. ES/MS: 697.3 (M+H⁺).

**2-[[4-[6-[[4-(2-cyclopropylthiazol-4-yl)-2-fluoro-phenyl]methoxy]-2-pyridyl]-2,5-difluoro-phenyl]methyl]-3-[[(2S)-oxetan-2-yl]methyl]benzimidazole-5-carboxylic acid (Example 158):** 1 mL of ACN and 0.3 mL of 1 N lithium hydroxide was added to methyl 2-[[4-[6-[[4-(2-cyclopropylthiazol-4-yl)-2-fluoro-phenyl]methoxy]-2-pyridyl]-2,5-difluorophenyl]methyl]-3-[[(2S)-oxetan-2-yl]methyl]benzimidazole-5-carboxylate (20.0 mg, 0.029 mmol). The mixture was heated to 80 °C for 30 min. Upon completion the filtrate was purified by RP-HPLC (eluent: water / MeCN *0.1% TFA) to give **Example 158.** ES/MS: 683.2 (M+H+). 1H NMR (400 MHz, Methanol-d4) δ 8.53 (d, J = 1.1 Hz, 1H), 8.18 (dd, J = 8.6, 1.4 Hz, 1H), 7.94 (dd, J = 10.8, 6.3 Hz, 1H), 7.81 (t, J = 7.9 Hz, 1H), 7.76 (d, J = 8.6 Hz, 1H), 7.72 - 7.64 (m, 2H), 7.61 (s, 1H), 7.56 (ddd, J = 7.9, 5.0, 3.4 Hz, 2H), 7.34 (dd, J = 11.3, 6.0 Hz, 1H), 6.92 (d, J = 8.2 Hz, 1H), 5.57 (s, 2H), 5.31 - 5.17 (m, 1H), 4.95 (dd, J = 15.6, 7.5 Hz, 1H), 4.85 - 4.73 (m, 3H), 4.73 - 4.64 (m, 1H), 4.54 - 4.44 (m, 1H), 2.94 - 2.78 (m, 1H), 2.68 - 2.48 (m, 1H), 2.48 - 2.31 (m, 1H), 1.19 (dt, J = 8.2, 3.3 Hz, 2H), 1.14 - 1.04 (m, 2H).

### Example 159: (S)-2-(4-(6-((4-(1-((1-cyanocyclopropyl)methyl)-1H-pyrazol-4-yl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-(1-((1-cyanocyclopropyl)methyl)-1H-pyrazol-4-yl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 7. 1H NMR (400 MHz, Methanol-d4) δ 8.52 (d, J = 1.1 Hz, 1H), 8.16 (td, J = 5.1, 4.6, 1.5 Hz, 2H), 8.01 - 7.85 (m, 2H), 7.81 (t, J = 7.9 Hz, 1H), 7.75 (d, J = 8.6 Hz, 1H), 7.61 - 7.47 (m, 2H), 7.47 - 7.36 (m, 2H), 7.33 (dd, J = 11.3, 6.0 Hz, 1H), 6.90 (d, J = 8.3 Hz, 1H), 5.55 (s, 2H), 5.33 - 5.18 (m, 1H), 4.97 - 4.90 (m, 1H), 4.82 - 4.63 (m, 4H), 4.52 (dt, J = 9.2, 6.0 Hz, 1H), 4.31 (s, 2H), 2.93 - 2.75 (m, 1H), 2.67 - 2.46 (m, 1H), 1.38 (dd, J = 7.4, 5.3 Hz, 2H), 1.32 (dd, J = 7.4, 5.3 Hz, 2H).

### Example 160: (S)-2-(4-(6-((4-(2-cyclopropyloxazol-5-yl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-(2-cyclopropyloxazol-5-yl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 7. 1H NMR (400 MHz, Methanol-d4) δ 8.54 (d, J = 1.4 Hz, 1H), 8.19 (dd, J = 8.6, 1.4 Hz, 1H), 7.91 (dd, J = 10.8, 6.3 Hz, 1H), 7.81 (t, J = 7.9 Hz, 1H), 7.76 (d, J = 8.5 Hz, 1H), 7.67 - 7.53 (m, 2H), 7.53 - 7.38 (m, 3H), 7.35 (dd, J = 11.3, 6.1 Hz, 1H), 6.92 (d, J = 8.3 Hz, 1H), 5.57 (s, 2H), 5.33 - 5.18 (m, 1H), 4.96 (dd, J = 15.5, 7.5 Hz, 1H), 4.83 - 4.73 (m, 3H), 4.73 - 4.64 (m, 1H), 4.53 (dt, J = 9.2, 5.9 Hz, 1H), 2.95 - 2.80 (m, 1H), 2.57 (dq, J = 11.6, 7.4 Hz, 1H), 2.18 (tt, J = 8.2, 5.2 Hz, 1H), 1.22 - 1.05 (m, 4H).

### Example 161: (S)-2-(4-(6-((6-(1-cyclopropyl-1H-pyrazol-4-yl)-4-fluoropyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-(1-cyclopropyl-1H-pyrazol-4-yl)-4-fluoropyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 7. Multiplet Report 1H NMR (400 MHz, DMSO-d6) δ 8.68 (d, J = 10.5 Hz, 1H), 8.44 (s, 1H), 8.26 (d, J = 1.5 Hz, 1H), 8.04 (s, 1H), 7.96 - 7.83 (m, 2H), 7.79 (dd, J = 8.4, 1.5 Hz, 1H), 7.68 (d, J = 11.4 Hz, 1H), 7.61 (d, J = 8.4 Hz, 1H), 7.53 (dd, J = 7.5, 1.7 Hz, 1H), 7.40 (dd, J = 11.5, 6.1 Hz, 1H), 6.94 (d, J = 8.2 Hz, 1H), 5.54 (s, 2H), 5.08 (qd, J = 7.1, 2.7 Hz, 1H), 4.76 (dd, J = 15.6, 7.1 Hz, 1H), 4.63 (dd, J = 15.6, 2.8 Hz, 1H), 4.59 - 4.40 (m, 3H), 4.36 (dt, J = 9.0, 5.9 Hz, 1H), 3.78 (tt, J = 7.5, 3.9 Hz, 1H), 2.82 - 2.63 (m, 1H), 2.40 (ddt, J = 11.3, 9.1, 7.0 Hz, 1H), 1.15 - 1.04 (m, 2H), 1.03 - 0.94 (m, 2H).

### Example 162: (S)-2-(2,5-difluoro-4-(6-((4-fluoro-6-(1-(oxetan-3-yl)-1H-pyrazol-4-yl)pyridin-3-yl)methoxy)pyridin-2-yl)benzyl)-1-(oxetan-2-ylmethyl)-1H-benzo[d]imidazole-6-carboxylic acid

(S)-2-(2,5-difluoro-4-(6-((4-fluoro-6-(1-(oxetan-3-yl)-1H-pyrazol-4-yl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 7. Multiplet Report 1H NMR (400 MHz, DMSO-d6) δ 8.71 (d, J = 10.4 Hz, 1H), 8.56 (s, 1H), 8.26 (d, J = 1.5 Hz, 1H), 8.22 (s, 1H), 7.96 - 7.84 (m, 2H), 7.79 (dd, J = 8.5, 1.5 Hz, 1H), 7.73 (d, J = 11.3 Hz, 1H), 7.61 (d, J = 8.4 Hz, 1H), 7.53 (dd, J = 7.5, 1.7 Hz, 1H), 7.40 (dd, J = 11.5, 6.1 Hz, 1H), 6.94 (d, J = 8.2 Hz, 1H), 5.62 (p, J = 6.9 Hz, 1H), 5.55 (s, 2H), 5.08 (qd, J = 7.0, 2.7 Hz, 1H), 5.01 - 4.87 (m, 4H), 4.76 (dd, J = 15.6, 7.1 Hz, 1H), 4.63 (dd, J = 15.5, 2.8 Hz, 1H), 4.58 - 4.41 (m, 3H), 4.36 (dt, J = 9.0, 5.9 Hz, 1H), 2.81 - 2.65 (m, 1H), 2.45 - 2.34 (m, 1H).

### Example 163: (S)-2-(4-(6-((4-chloro-6-(1-cyclopropyl-1H-pyrazol-4-yl)pyridin-3-yl)methoxy)pyridin-2-yl)-2-fluoro-5-methylbenzyl)-4-fluoro-1-(oxetan-2-ylmethyl)-1H-benzo[d]imidazole-6-carboxylic acid

(S)-2-(4-(6-((4-chloro-6-(1-cyclopropyl-1H-pyrazol-4-yl)pyridin-3-yl)methoxy)pyridin-2-yl)-2-fluoro-5-methylbenzyl)-4-fluoro-1-(oxetan-2-ylmethyl)-1H-benzo[d]imidazole-6-carboxylic acid was prepared in a manner as described in Procedure 7. Multiplet Report 1H NMR (400 MHz, DMSO-d6) δ 8.63 (s, 1H), 8.46 (s, 1H), 8.15 (d, J = 1.3 Hz, 1H), 8.06 (s, 1H), 7.92 (s, 1H), 7.86 (t, J = 7.8 Hz, 1H), 7.58 - 7.41 (m, 1H), 7.37 - 7.20 (m, 3H), 6.92 (d, J = 8.3 Hz, 1H), 5.47 (s, 2H), 5.06 (qd, J = 7.1, 2.6 Hz, 1H), 4.78 (dd, J = 15.6, 7.1 Hz, 1H), 4.64 (dd, J = 15.6, 2.7 Hz, 1H), 4.56 - 4.29 (m, 4H), 3.78 (tt, J = 7.5, 3.9 Hz, 1H), 2.80 - 2.65 (m, 1H), 2.47 - 2.34 (m, 1H), 2.33 (s, 3H), 1.12 - 0.93 (m, 4H).

### Example 387: (S)-2-((3'-((5-(1-(difluoromethyl)-1H-pyrazol-4-yl)thiazol-2-yl)methoxy)-2,4',5-trifluoro-[1,1'-biphenyl]-4-yl)methyl)-4-fluoro-1-(oxetan-2-ylmethyl)-1H-benzo[d]imidazole-6-carboxylic acid

(S)-2-((3'-((5-(1-(difluoromethyl)-1H-pyrazol-4-yl)thiazol-2-yl)methoxy)-2,4',5-trifluoro-[1,1'-biphenyl]-4-yl)methyl)-4-fluoro-1-(oxetan-2-ylmethyl)-1H-benzo[d]imidazole-6-carboxylic acid was prepared in a manner as described in Procedure 7 using Intermediates **I-1280** and 1-(difluoromethyl)-4-(4,4,5,5-tetramethyl-1,3,2-dioxaborolan-2-yl)pyrazole. ES/MS m/z: 700.6 (M+H⁺). 1HNMR (400 MHz, DMSO-d6) δ 8.74 (s, 1H), 8.23 (s, 1H), 8.15 (d, J = 1.2 Hz, 1H), 8.12 (s, 1H), 7.84 (t, J = 59.1 Hz, 1H), 7.60 (dd, J = 8.2, 2.0 Hz, 1H), 7.57 - 7.48 (m, 2H), 7.46 - 7.34 (m, 2H), 7.31 - 7.20 (m, 1H), 5.63 (s, 2H), 5.11 - 5.02 (m, 1H), 4.79 (dd, J = 15.6, 7.1 Hz, 1H), 4.66 (dd, J = 15.5, 2.7 Hz, 1H), 4.59 - 4.42 (m, 3H), 4.41 - 4.32 (m, 1H), 2.72 (ddt, J = 15.8, 12.4, 6.0 Hz, 1H), 2.47 - 2.31 (m, 1H).

### Example 164: (S)-2-(2,5-difluoro-4-(6-((2-fluoro-4-(1-(oxetan-3-yl)-1H-pyrazol-4-yl)benzyl)oxy)pyridin-2-yl)benzyl)-1-(oxetan-2-ylmethyl)-1H-benzo[d]imidazole-6-carboxylic acid

### Procedure 8

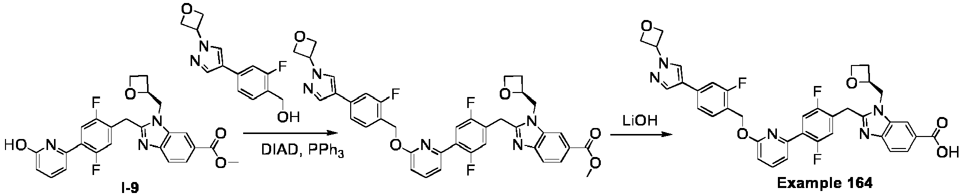

**Methyl (S)-2-(2,5-difluoro-4-(6-((2-fluoro-4-(1-(oxetan-3-yl)-1H-pyrazol-4-yl)benzyl)oxy)pyridin-2-yl)benzyl)-1-(oxetan-2-ylmethyl)-1H-benzo[d]imidazole-6-carboxylate:** To a solution of methyl 2-[[2,5-difluoro-4-(6-oxo-1H-pyridin-2-yl)phenyl]methyl]-3-[[(2S)-oxetan-2-yl]methyl]benzimidazole-5-carboxylate (50.0 mg, 0.107 mmol), [2-fluoro-4-[1-(oxetan-3-yl)pyrazol-4-yl]phenyl]methanol (35.4 mg, 0.143 mmol), and triphenylphosphine (84.5 mg, 0.322 mmol) in THF (4 mL) at 0 °C, was added dropwise a solution of diisopropyl azodicarboxylate (1000 mmol/L, 0.322 mL, 0.322 mmol). Next, the solution was gradually warmed to rt and stirred for 2 hr. Upon completion of the time, the mixture was diluted with EtOAc and washed with brine. The organic extract was dried over sodium sulfate and purified by flash chromatography (eluent: EtOAc/hexanes) to give the title compound. ES/MS: 696.2 (M+H⁺).

**(S)-2-(2,5-difluoro-4-(6-((2-fluoro-4-(1-(oxetan-3-yl)-1H-pyrazol-4-yl)benzyl)oxy)pyridin-2-yl)benzyl)-1-(oxetan-2-ylmethyl)-1H-benzo[d]imidazole-6-carboxylic acid (Example 164):** A suspension of methyl 2-[[2,5-difluoro-4-[6-[[2-fluoro-4-[1-(oxetan-3-yl)pyrazol-4-yl]phenyl]methoxy]-2-pyridyl]phenyl]methyl]-3-[[(2S)-oxetan-2-yl]methyl]benzimidazole-5-carboxylate (42.4 mg, 0.0609 mmol) and lithium hydroxide, monohydrate (300 mmol/L, 0.609 mL, 0.183 mmol) in CH₃CN (3 mL) in a 40 mL reaction vial was heated at 100 °C for 20 min. Upon completion of the time, 0.180 mL of 1M citric acid was added to the solution. The organic extract was dried over sodium sulfate, filtered and concentrated. Next, the crude product was purified by RP-HPLC (eluent: MeCN/H₂O). The resulting product fractions were diluted with EtOAc and neutralized with sodium bicarbonate solution. The organic extract was dried over sodium sulfate, filtered and concentrated to give
**Example 164.** ES/MS: 682.2 (M+H⁺); 1H NMR (400 MHz, Methanol-d4) δ 8.49 (d, J = 1.4 Hz, 1H), 8.20 (d, J = 0.8 Hz, 1H), 8.15 - 8.08 (m, 1H), 7.99 (s, 1H), 7.93 (dd, J = 10.7, 6.3 Hz, 1H), 7.80 (t, J = 7.9 Hz, 1H), 7.74 (d, J = 8.5 Hz, 1H), 7.58 - 7.46 (m, 2H), 7.46 - 7.34 (m, 2H), 7.31 (dd, J = 11.3, 6.1 Hz, 1H), 6.90 (d, J = 8.2 Hz, 1H), 5.63 - 5.57 (m, 1H), 5.55 (s, 2H), 5.24 (qd, J = 7.3, 2.4 Hz, 1H), 5.08 (s, 2H), 5.06 (s, 2H), 4.83 - 4.63 (m, 5H), 4.51 (dt, J = 9.1, 6.0 Hz, 1H), 2.94 - 2.75 (m, 1H), 2.63 - 2.44 (m, 1H).

### Example 165: (S)-2-(4-(6-((2-carbamoylthiazol-5-yl)methoxy)pyridin-2-yl)-2,5-difluorobenzyl)-1-(oxetan-2-ylmethyl)-1H-benzo[d]imidazole-6-carboxylic acid

### Procedure 9

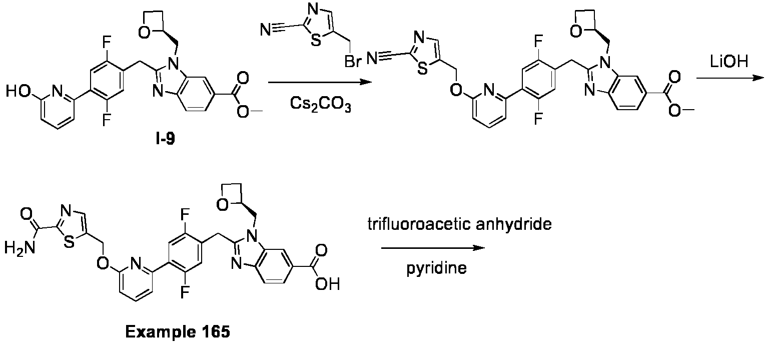

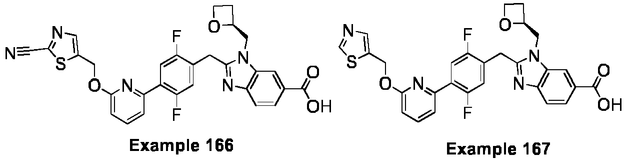

**Methyl (S)-2-(4-(6-((2-cyanothiazol-5-yl)methoxy)pyridin-2-yl)-2,5-difluorobenzyl)-1-(oxetan-2-ylmethyl)-1H-benzo[d]imidazole-6-carboxylate:** A suspension of 5-(bromomethyl)thiazole-2-carbonitrile (96 mg, 0.47 mmol), methyl 2-[[2,5-difluoro-4-(6-hydroxy-2-pyridyl)phenyl]methyl]-3-[[(2S)-oxetan-2-yl]methyl]benzimidazole-5-carboxylate (165 mg, 0.35 mmol), and cesium carbonate (185 mg, 0.57 mmol) in CH₃CN (3 mL) was heated at 60 °C for 1 hr. Upon completion of the time, the mixture was diluted with EtOAc and washed with brine. The organic extract was dried over sodium sulfate and purified by flash chromatography (eluent: EtOAc/hexanes) to give the title compound. ES/MS: 588.0 (M+H⁺).

**(S)-2-(4-(6-((2-carbamoylthiazol-5-yl)methoxy)pyridin-2-yl)-2,5-difluorobenzyl)-1-(oxetan-2-ylmethyl)-1H-benzo[d]imidazole-6-carboxylic acid (Example 165):** A suspension of methyl 2-[[4-[6-[(2-cyanothiazol-5-yl)methoxy]-2-pyridyl]-2,5-difluoro-phenyl]methyl]-3-[[(2S)-oxetan-2-yl]methyl]benzimidazole-5-carboxylate (121 mg, 0.206 mmol) and lithium hydroxide, monohydrate (300 mmol/L, 2.06 mL, 0.617 mmol) in CH₃CN (5 mL) in a 40 mL reaction vial was heated at 100 °C until full conversion of nitrile to amide. Upon conversion 0.617 mL of 1M citric acid was added. The organic extract was dried over sodium sulfate, filtered and concentrated and then purified by RP-HPLC (eluent: MeCN/H₂O). The resulting product fractions were diluted with EtOAc and neutralized with sodium bicarbonate solution. The organic extract was dried over sodium sulfate, filtered and concentrated to give **Example 165.** ES/MS: 592.2 (M+H⁺); 1H NMR (400 MHz, Methanol-d4) δ 8.32 (d, J = 1.5 Hz, 1H), 8.02 - 7.94 (m, 4H), 7.85 - 7.73 (m, 1H), 7.68 (d, J = 8.5 Hz, 1H), 7.59 (dd, J = 7.2, 1.5 Hz, 1H), 7.21 (dd, J = 11.5, 6.0 Hz, 1H), 6.87 (d, J = 8.2 Hz, 1H), 5.79 (d, J = 0.8 Hz, 2H), 5.29 - 5.16 (m, 1H), 4.74 (dd, J = 15.7, 7.0 Hz, 1H), 4.69 - 4.41 (m, 6H), 2.88 - 2.67 (m, 1H), 2.59 - 2.41 (m, 1H).

### Examples 166 and 167:

**(S)-2-(4-(6-((2-cyanothiazol-5-yl)methoxy)pyridin-2-yl)-2,5-difluorobenzyl)-1-(oxetan-2-ylmethyl)-1H-benzo[d]imidazole-6-carboxylic acid (Example 166) and (S)-2-(2,5-difluoro-4-(6-(thiazol-5-ylmethoxy)pyridin-2-yl)benzyl)-1-(oxetan-2-ylmethyl)-1H-benzo[d]imidazole-6-carboxylic acid (Example 167):** To a suspension of 2-[[4-[6-[(2-carbamoylthiazol-5-yl)methoxy]-2-pyridyl]-2,5-difluoro-phenyl]methyl]-3-[[(2S)-oxetan-2-yl]methyl]benzimidazole-5-carboxylic acid (78.2 mg, 0.132 mmol) in THF (3 mL) at 0 °C, was added pyridine (0.0539 mL, 0.667 mmol). Next, a solution of trifluoroacetic anhydride (TFAA-0.0372 mL, 0.267 mmol) in THF (1.0 mL) was slowly added. Next, aliquots of Trifluoroacetic anhydride were continuously added until complete conversion of amide to nitrile. Upon conversion the mixture was carefully neutralized with 2M sodium carbonate solution (650 uL). Once neutralized the solution was partitioned with EtOAc and water. The organic extract was dried over sodium sulfate, filtered and concentrated. Purified by RP-HPLC (eluent: MeCN/H₂O). The resulting product fractions were diluted with EtOAc and neutralized with sodium bicarbonate solution. The organic extract was dried over sodium sulfate, filtered and concentrated to give title products.

**(S)-2-(4-(6-((2-cyanothiazol-5-yl)methoxy)pyridin-2-yl)-2,5-difluorobenzyl)-1-(oxetan-2-ylmethyl)-1H-benzo[d]imidazole-6-carboxylic acid (Example 166):ES/MS:** 574.1 (M+H⁺); 1H NMR (400 MHz, Methanol-d4) δ 8.31 (d, J = 1.4 Hz, 1H), 8.19 (s, 1H), 7.99 (dd, J = 8.5, 1.5 Hz, 1H), 7.94 (dd, J = 10.6, 6.4 Hz, 1H), 7.84 (t, J = 7.9 Hz, 1H), 7.68 (d, J = 8.5 Hz, 1H), 7.61 (dd, J = 7.4, 1.5 Hz, 1H), 7.23 (dd, J = 11.5, 6.1 Hz, 1H), 6.89 (d, J = 8.2 Hz, 1H), 5.84 (d, J = 0.8 Hz, 2H), 5.21 (qd, J = 7.1, 2.6 Hz, 1H), 4.75 (dd, J = 15.7, 7.0 Hz, 1H), 4.69 - 4.48 (m, 5H), 4.45 (dt, J = 9.2, 5.9 Hz, 1H), 2.81 (dtd, J = 11.5, 8.2, 6.0 Hz, 1H), 2.62 - 2.42 (m, 1H).

**(S)-2-(2,5-difluoro-4-(6-(thiazol-5-ylmethoxy)pyridin-2-yl)benzyl)-1-(oxetan-2-ylmethyl)-1H-benzo[d]imidazole-6-carboxylic acid (Example 167):**ES/MS: 549.1 (M+H⁺); 1H NMR (400 MHz, Methanol-d4) δ 8.97 (d, J = 0.8 Hz, 1H), 8.31 (d, J = 1.5 Hz, 1H), 8.01 - 7.95 (m, 4H), 7.85 - 7.75 (m, 1H), 7.67 (d, J = 8.5 Hz, 1H), 7.57 (dd, J = 7.3, 1.6 Hz, 1H), 7.21 (dd, J = 11.5, 6.1 Hz, 1H), 6.84 (d, J = 8.2 Hz, 1H), 5.78 (d, J = 0.8 Hz, 2H), 5.20 (qd, J = 7.1, 2.5 Hz, 1H), 4.74 (dd, J = 15.7, 6.9 Hz, 1H), 4.69 - 4.48 (m, 5H), 4.45 (dt, J = 9.2, 5.9 Hz, 1H), 2.80 (dtd, J = 11.5, 8.1, 6.0 Hz, 1H), 2.50 (ddt, J = 11.4, 9.2, 7.2 Hz, 1H).

### Example 168: (S)-2-(4-(6-((2-cyanooxazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-cyanooxazol-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 9. 1H NMR (400 MHz, Methanol-d4) δ 8.32 (d, J = 1.5 Hz, 1H), 7.99 (dd, J = 8.5, 1.5 Hz, 1H), 7.94 (dd, J = 10.7, 6.3 Hz, 1H), 7.83 (t, J = 7.9 Hz, 1H), 7.68 (d, J = 8.5 Hz, 1H), 7.59 (dd, J = 7.4, 1.5 Hz, 1H), 7.51 (s, 1H), 7.30 - 7.15 (m, 1H), 6.89 (d, J = 8.2 Hz, 1H), 5.63 (d, J = 0.7 Hz, 2H), 5.21 (tt, J = 7.2, 3.6 Hz, 1H), 4.74 (dd, J = 15.7, 6.9 Hz, 1H), 4.68 - 4.37 (m, 7H), 2.81 (dtd, J = 11.4, 8.2, 6.1 Hz, 1H), 2.50 (ddt, J = 11.5, 9.2, 7.1 Hz, 1H).

### Example 169: (S)-2-(2,5-difluoro-4-(6-(oxazol-5-ylmethoxy)pyridin-2-yl)benzyl)-1-(oxetan-2-ylmethyl)-1H-benzo[d]imidazole-6-carboxylic acid

(S)-2-(2,5-difluoro-4-(6-(oxazol-5-ylmethoxy)pyridin-2-yl)benzyl)-1-(oxetan-2-ylmethyl)-1H-benzo[d]imidazole-6-carboxylic acid was prepared in a manner as described in Procedure 9. 1H NMR (400 MHz, Methanol-d4) δ 8.31 (d, J = 1.4 Hz, 1H), 8.23 (s, 1H), 8.06 - 7.90 (m, 2H), 7.79 (t, J = 7.9 Hz, 1H), 7.67 (d, J = 8.5 Hz, 1H), 7.56 (dd, J = 7.4, 1.5 Hz, 1H), 7.25 (s, 1H), 7.20 (dd, J = 11.5, 6.1 Hz, 1H), 6.84 (d, J = 8.2 Hz, 1H), 5.56 (s, 2H), 5.20 (qd, J = 7.0, 2.6 Hz, 1H), 4.74 (dd, J = 15.7, 6.9 Hz, 1H), 4.69 - 4.40 (m, 6H), 2.80 (dtd, J = 11.4, 8.1, 6.1 Hz, 1H), 2.50 (ddt, J = 11.4, 9.2, 7.2 Hz, 1H).

### Example 170: (S)-2-(4-(6-((2-cyanothiazol-4-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-cyanothiazol-4-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 9. 1H NMR (400 MHz, Methanol-d4) δ 8.31 (d, J = 1.4 Hz, 1H), 8.04 (d, J = 0.9 Hz, 1H), 7.98 (dd, J = 8.5, 1.5 Hz, 1H), 7.84 (dd, J = 10.3, 3.9 Hz, 1H), 7.79 (d, J = 7.9 Hz, 1H), 7.67 (d, J = 8.5 Hz, 1H), 7.56 (dd, J = 7.5, 1.5 Hz, 1H), 7.19 (dd, J = 11.5, 6.0 Hz, 1H), 6.91 (d, J = 8.2 Hz, 1H), 5.67 (d, J = 0.8 Hz, 2H), 5.20 (qd, J = 7.1, 2.5 Hz, 1H), 4.73 (dd, J = 15.7, 7.0 Hz, 1H), 4.69 - 4.39 (m, 6H), 2.80 (dtd, J = 11.4, 8.2, 6.1 Hz, 1H), 2.50 (ddt, J = 11.5, 9.1, 7.2 Hz, 1H).

### Example 171: (S)-2-(4-(6-((5-carbamoylfuran-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-carbamoylfuran-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 9. 1H NMR (400 MHz, DMSO) δ 8.26 (d, J = 1.5 Hz, 1H), 7.99 - 7.84 (m, 2H), 7.80 (dd, J = 8.5, 1.6 Hz, 2H), 7.61 (d, J = 8.4 Hz, 1H), 7.54 (dd, J = 7.5, 1.8 Hz, 1H), 7.40 (dd, J = 11.5, 5.9 Hz, 2H), 7.11 (d, J = 3.4 Hz, 1H), 6.94 (d, J = 8.2 Hz, 1H), 6.69 (d, J = 3.4 Hz, 1H), 5.47 (s, 2H), 5.08 (qd, J = 7.0, 2.7 Hz, 1H), 4.76 (dd, J = 15.6, 7.1 Hz, 1H), 4.69 - 4.24 (m, 5H), 2.87 - 2.62 (m, 1H), 2.45 - 2.29 (m, 1H).

### Example 172: (S)-2-(4-(6-((4-carbamoyl-2,5-difluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-carbamoyl-2,5-difluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 9. 1H NMR (400 MHz, Methanol-d4) δ 8.56 (t, J = 1.0 Hz, 1H), 8.20 (dd, J = 8.6, 1.4 Hz, 1H), 7.93 - 7.80 (m, 2H), 7.77 (d, J = 8.5 Hz, 1H), 7.66 - 7.53 (m, 2H), 7.38 (ddd, J = 16.7, 11.0, 5.8 Hz, 2H), 6.97 (d, J = 8.4 Hz, 1H), 5.59 (s, 2H), 5.35 - 5.18 (m, 1H), 4.97 (dd, J = 15.5, 7.5 Hz, 1H), 4.84 - 4.75 (m, 3H), 4.74 - 4.63 (m, 1H), 4.53 (dt, J = 9.2, 6.0 Hz, 1H), 2.94 - 2.80 (m, 1H), 2.57 (dq, J = 11.5, 7.4 Hz, 1H).

### Example 173: (S)-2-(4-(6-((6-carbamoylpyridin-3-yl)methoxy)pyridin-2-yl)-3-fluorobenzyl)-1-(oxetan-2-ylmethyl)-1H-benzo[d]imidazole-6-carboxylic acid

(S)-2-(4-(6-((6-carbamoylpyridin-3-yl)methoxy)pyridin-2-yl)-3-fluorobenzyl)-1-(oxetan-2-ylmethyl)-1H-benzo[d]imidazole-6-carboxylic acid was prepared in a manner as described in Procedure 9. 1H NMR (400 MHz, DMSO-d6) δ 12.03 (s, 1H), 8.75 (dd, J = 2.0, 1.0 Hz, 1H), 8.32 - 8.17 (m, 1H), 8.17 - 8.01 (m, 3H), 7.96 (t, J = 8.3 Hz, 1H), 7.91 - 7.76 (m, 2H), 7.64 (d, J = 8.6 Hz, 2H), 7.46 (dd, J = 7.5, 1.8 Hz, 1H), 7.41 - 7.23 (m, 2H), 6.94 (d, J = 8.2 Hz, 1H), 5.58 (s, 2H), 5.06 - 4.96 (m, 1H), 4.84 (t, J = 2.6 Hz, 1H), 4.71 (dd, J = 15.5, 7.2 Hz, 1H), 4.53 - 4.42 (m, 2H), 4.38 - 4.29 (m, 1H), 4.00 (ddd, J = 12.1, 7.5, 5.1 Hz, 1H), 2.82 - 2.64 (m, 1H), 2.44 - 2.29 (m, 1H).

### Example 174: 2-(4-(6-((6-carbamoylpyridin-3-yl)methoxy)pyridin-2-yl)-2,5-difluorobenzyl)-1-(2-methoxyethyl)-1H-benzo[d]imidazole-6-carboxylic acid

2-(4-(6-((6-carbamoylpyridin-3-yl)methoxy)pyridin-2-yl)-2, 5-difluorobenzyl)-1-(2-methoxyethyl)-1H-benzo[d]imidazole-6-carboxylic acid was prepared in a manner as described in Procedure 9. 1H NMR (400 MHz, Methanol-d4) δ 8.76 (dd, J = 2.0, 0.9 Hz, 1H), 8.56 (s, 1H), 8.22 (dd, J = 8.6, 1.4 Hz, 1H), 8.17 - 7.99 (m, 2H), 7.93 - 7.72 (m, 3H), 7.58 (dd, J = 7.5, 1.6 Hz, 1H), 7.36 (dd, J = 11.2, 6.0 Hz, 1H), 6.96 (d, J = 8.2 Hz, 1H), 5.63 (s, 2H), 4.86 - 4.70 (m, 4H), 3.84 (t, J = 4.9 Hz, 2H), 3.33 (s,, 3H), 2.67 (s, 3H).

### Example 175 and 176: (S)-2-(4-(6-((6-((cyanomethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid (Example 175) and (S)-2-(4-(6-((6-((2-amino-2-oxoethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid & (S)-2-(4-(6-((6-((cyanomethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

### Procedure 10

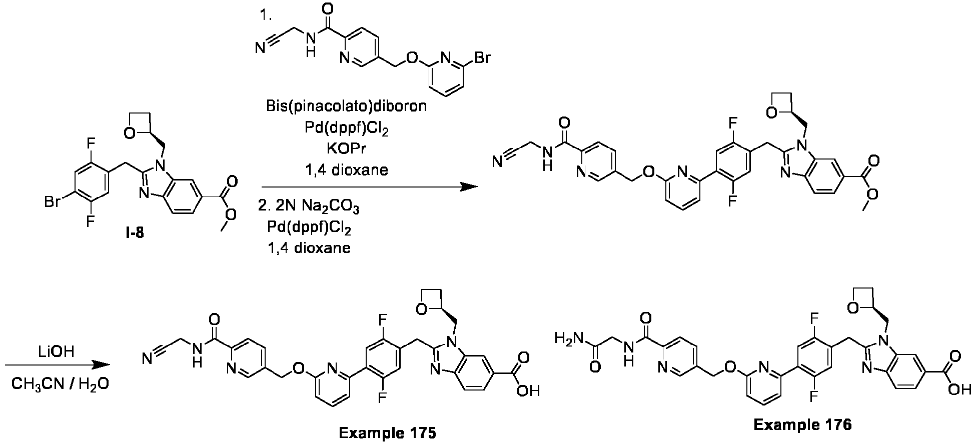

**Methyl (S)-2-(4-(6-((6-((cyanomethyl)carbamoyl)pyridin-3-yl)methoxy)pyridin-2-yl)-2,5-difluorobenzyl)-1-(oxetan-2-ylmethyl)-1H-benzo[d]imidazole-6-carboxylate:** In a 8 mL reaction vial, a suspension of methyl 2-[(4-bromo-2,5-difluoro-phenyl)methyl]-3-[[(2S)-oxetan-2-yl]methyl]benzimidazole-5-carboxylate (80.0 mg, 0.177 mmol), Bis(pinacolato)diboron (54.9 mg, 0.216 mmol), [1,1'-Bis(diphenylphosphino)ferrocene] dichloropalladium(II); PdCl₂(dppf) (19.7 mg, 0.0266 mmol), and potassium propionate (59.7 mg, 0.532 mmol) in dioxane (1.5 mL) was degassed with Ar for 5 min. Upon completion of the time, the mixture was heated at 110 °C thermally for 1 hr. Following this time LCMS shows complete conversion of bromide. The mixture was cooled to rt. Sodium carbonate (2.00 M, 0.233 mL, 0.467 mmol) was added and the reaction mixture was stirred at rt for 5 min. Upon completion of this time [1,1'-Bis(diphenylphosphino)ferrocene] dichloropalladium(II); PdCl₂(dppf) (13.1 mg, 0.0176 mmol) and a solution of 5-[(6-bromo-2-pyridyl)oxymethyl]-N-(cyanomethyl)pyridine-2-carboxamide (85.1 mg, 0.245 mmol) in dioxane (1.5 mL) was added to the reaction mixture. The reaction mixture was degassed for 5 min with argon, then heated at 90 °C for 2 hr. Next, the reaction mixture was diluted with EtOAc and washed with brine and saturated sodium bicarbonate solution. The organic extract was dried over sodium sulfate, filtered and concentrated. The crude residue was purified by flash chromatography (eluent: EtOAc/hexanes) to yield the desired compound. ES/MS: 639.2.

### (S)-2-(4-(6-((6-((cyanomethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid (Example 175) and (S)-2-(4-(6-((6-((2-amino-2-oxoethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

**(Example 176):** A suspension of methyl 2-[[4-[6-[[6-(cyanomethylcarbamoyl)-3-pyridyl]methoxy]-2-pyridyl]-2,5-difluoro-phenyl]methyl]-3-[[(2S)-oxetan-2-yl]methyl]benzimidazole-5-carboxylate (86.4 mg, 0.135 mmol) and lithium hydroxide, monohydrate (300 mmol/L, 1.35 mL, 0.406 mmol) in CH₃CN (5 mL) in a 40 mL reaction vial was heated at 100 °C for 5 min. Upon completion of the time more LiOH (0.406 mL) was added and heating was resumed for 25 min. Following this time, the solution was diluted with EtOAc and brine. Once the solution was diluted, 0.406 mL 1M citric acid was added. The organic extract was dried over sodium sulfate, filtered and concentrated. Purified by RP-HPLC (eluent: MeCN/H₂O). The resulting product fractions were diluted with EtOAc and neutralized with sodium bicarbonate solution. The organic extract was dried over sodium sulfate, filtered and concentrated to give title products.

**(S)-2-(4-(6-((6-((cyanomethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid (Example 175):** ES/MS: 625.2 (M+H⁺); 1H NMR (400 MHz, Methanol-d4) δ 8.79 (d, J = 2.0 Hz, 1H), 8.31 (d, J = 1.4 Hz, 1H), 8.16 - 8.02 (m, 2H), 7.99 (dd, J = 8.5, 1.6 Hz, 1H), 7.85 - 7.70 (m, 2H), 7.67 (d, J = 8.5 Hz, 1H), 7.54 (dd, J = 7.5, 1.5 Hz, 1H), 7.19 (dd, J = 11.5, 6.1 Hz, 1H), 6.92 (d, J = 8.2 Hz, 1H), 5.63 (s, 2H), 5.19 (qd, J = 7.1, 2.5 Hz, 1H), 4.78 - 4.40 (m, 7H), 4.37 (s, 2H), 2.79 (dtd, J = 11.4, 8.2, 6.1 Hz, 1H), 2.49 (ddt, J = 11.5, 9.2, 7.2 Hz, 1H).

**(S)-2-(4-(6-((6-((2-amino-2-oxoethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid (Example 176):** ES/MS: 643.2 (M+H⁺); 1H NMR (400 MHz, Methanol-d4) δ 8.80 (t, J = 1.4 Hz, 1H), 8.32 (d, J = 1.4 Hz, 1H), 8.11 (qd, J = 8.0, 1.5 Hz, 2H), 7.99 (dd, J = 8.5, 1.5 Hz, 1H), 7.85 - 7.71 (m, 2H), 7.67 (d, J = 8.5 Hz, 1H), 7.55 (dd, J = 7.4, 1.5 Hz, 1H), 7.19 (dd, J = 11.5, 6.0 Hz, 1H), 6.93 (d, J = 8.2 Hz, 1H), 5.65 (s, 2H), 5.29 - 5.14 (m, 1H), 4.77 - 4.48 (m, 6H), 4.45 (dt, J = 9.1, 5.9 Hz, 1H), 4.10 (s, 2H), 2.86 - 2.67 (m, 1H), 2.58 - 2.40 (m, 1H).

### Example 177: (S)-2-(2,5-difluoro-4-(6-((1-methyl-1H-imidazol-4-yl)methoxy)pyridin-2-yl)benzyl)-1-(oxetan-2-ylmethyl)-1H-benzo[d]imidazole-6-carboxylic acid

(S)-2-(2,5-difluoro-4-(6-((1-methyl-1H-imidazol-4-yl)methoxy)pyridin-2-yl)benzyl)-1-(oxetan-2-ylmethyl)-1H-benzo[d]imidazole-6-carboxylic acid was prepared in a manner as described in Procedure 10. 1H NMR (400 MHz, CDCl3) δ 8.16 (d, J = 1.5 Hz, 1H), 8.07 (dd, J = 8.5, 1.5 Hz, 1H), 7.98 (dd, J= 10.7, 6.3 Hz, 1H), 7.81 (d, J = 8.5 Hz, 1H), 7.67 - 7.56 (m, 2H), 7.50 - 7.38 (m, 1H), 7.12 (dd, J = 11.3, 6.0 Hz, 1H), 7.01 (d, J = 1.4 Hz, 1H), 6.78 (dd, J = 8.2, 0.7 Hz, 1H), 5.48 (s, 2H), 5.13 (qd, J = 6.8, 2.8 Hz, 1H), 4.66 - 4.27 (m, 6H), 3.71 (s, 3H), 2.69 (dtd, J = 11.5, 8.1, 6.0 Hz, 1H), 2.37 (ddt, J = 11.5, 9.0, 7.2 Hz, 1H).

### Example 178: (S)-2-(2,5-difluoro-4-(6-((1-methyl-1H-imidazol-5-yl)methoxy)pyridin-2-yl)benzyl)-1-(oxetan-2-ylmethyl)-1H-benzo[d]imidazole-6-carboxylic acid

(S)-2-(2,5-difluoro-4-(6-((1-methyl-1H-imidazol-5-yl)methoxy)pyridin-2-yl)benzyl)-1-(oxetan-2-ylmethyl)-1H-benzo[d]imidazole-6-carboxylic acid was prepared in a manner as described in Procedure 10. 1H NMR (400 MHz, CDCl3) δ 8.18 (d, J = 1.5 Hz, 1H), 8.09 (dd, J = 8.5, 1.5 Hz, 1H), 7.91 (dd, J= 10.5, 6.2 Hz, 1H), 7.84 (d, J = 8.5 Hz, 1H), 7.72 (s, 1H), 7.66 (t, J = 7.9 Hz, 1H), 7.53 - 7.44 (m, 1H), 7.31 - 7.22 (m, 2H), 7.17 (dd, J = 11.3, 6.0 Hz, 1H), 6.74 (d, J = 8.2 Hz, 1H), 5.48 (s, 2H), 5.18 (qd, J = 6.9, 2.7 Hz, 1H), 4.72 - 4.57 (m, 2H), 4.57 - 4.35 (m, 4H), 2.89 - 2.66 (m, 1H), 2.43 (ddt, J = 11.3, 8.9, 7.2 Hz, 1H).

### Example 179: (S)-2-(4-(6-((1,2-dimethyl-1H-imidazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1,2-dimethyl-1H-imidazol-5-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 10. 1H NMR (400 MHz, CDCl3) δ 8.18 (d, J = 1.5 Hz, 1H), 8.09 (dd, J = 8.4, 1.5 Hz, 1H), 7.90 (dd, J = 10.5, 6.3 Hz, 1H), 7.82 (d, J = 8.5 Hz, 1H), 7.65 (t, J = 7.9 Hz, 1H), 7.49 (dd, J = 7.6, 1.4 Hz, 1H), 7.17 (d, J = 6.3 Hz, 2H), 6.73 (d, J = 8.1 Hz, 1H), 5.43 (s, 2H), 5.17 (qd, J = 6.8, 2.8 Hz, 1H), 4.68 - 4.56 (m, 2H), 4.56 - 4.34 (m, 4H), 3.63 (s, 3H), 2.81 - 2.64 (m, 1H), 2.52 (s, 3H), 2.47 - 2.35 (m, 1H).

### Example 180: (S)-2-(4-(6-((1-(difluoromethyl)-2-methyl-1H-imidazol-4-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1-(difluoromethyl)-2-methyl-1H-imidazol-4-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 10. 1H NMR (400 MHz, DMSO) δ 12.78 (s, 1H), 8.26 (d, J = 1.6 Hz, 1H), 7.98 - 7.89 (m, 1H), 7.85 (t, J = 7.9 Hz, 1H), 7.82 - 7.72 (m, 2H), 7.61 (d, J = 8.6 Hz, 1H), 7.50 (d, J = 6.0 Hz, 2H), 7.39 (dd, J = 11.5, 6.0 Hz, 1H), 6.88 (d, J = 8.3 Hz, 1H), 5.28 (s, 2H), 5.08 (qd, J = 7.1, 2.6 Hz, 1H), 4.76 (dd, J = 15.6, 7.0 Hz, 1H), 4.63 (dd, J = 15.5, 2.7 Hz, 1H), 4.58 - 4.42 (m, 3H), 4.36 (dt, J = 9.0, 5.9 Hz, 1H), 2.80 - 2.65 (m, 1H), 2.49 - 2.28 (m, 1H).

### Example 181: (S)-2-(4-(6-((1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1H-pyrazol-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, DMSO-d6) δ 8.25 (d, J = 1.5 Hz, 1H), 7.92 (dd, J = 10.5, 6.5 Hz, 1H), 7.86 (t, J = 7.9 Hz, 1H), 7.79 (dd, J = 8.4, 1.6 Hz, 1H), 7.69 - 7.62 (m, 1H), 7.60 (d, J = 8.4 Hz, 1H), 7.51 (dd, J = 7.5, 1.7 Hz, 1H), 7.39 (dd, J = 11.6, 6.1 Hz, 1H), 6.89 (d, J = 8.2 Hz, 1H), 6.34 (d, J = 2.1 Hz, 1H), 5.44 (s, 2H), 5.08 (qd, J = 6.9, 2.7 Hz, 1H), 4.75 (dd, J = 15.5, 7.0 Hz, 1H), 4.62 (dd, J = 15.6, 2.8 Hz, 1H), 4.58 - 4.41 (m, 3H), 4.36 (dt, J = 9.0, 5.9 Hz, 1H), 2.72 (tdd, J = 9.8, 8.0, 4.1 Hz, 1H), 2.40 (ddt, J = 11.1, 8.8, 6.9 Hz, 1H).

### Example 182: (R)-2-(4-(6-((5-cyanothiazol-2-yl)methoxy)pyridin-2-yl)-2,5-difluorobenzyl)-1-(4,4-dimethyltetrahydrofuran-3-yl)-1H-benzo[d]imidazole-6-carboxylic acid

(R)-2-(4-(6-((5-cyanothiazol-2-yl)methoxy)pyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 10. 1H NMR (400 MHz, DMSO-d6) δ 8.69 (s, 1H), 8.48 (s, 1H), 7.96 (t, J = 7.8 Hz, 1H), 7.92 - 7.76 (m, 2H), 7.66 - 7.58 (m, 2H), 7.46 (dd, J = 11.3, 6.2 Hz, 1H), 7.07 (d, J = 8.3 Hz, 1H), 5.89 (s, 2H), 5.01 (d, J = 6.7 Hz, 1H), 4.58 - 4.48 (m, 2H), 4.49 - 4.31 (m, 2H), 3.82 - 3.72 (m, 2H), 1.33 (s, 3H), 0.60 (s, 3H).

### Example 183: (S)-2-(4-(6-((6-((1-cyanocyclopropyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-((1-cyanocyclopropyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.77 (d, J = 2.0 Hz, 1H), 8.31 (d, J = 1.4 Hz, 1H), 8.18 - 8.02 (m, 2H), 7.99 (dd, J = 8.5, 1.5 Hz, 1H), 7.87 - 7.71 (m, 2H), 7.67 (d, J = 8.5 Hz, 1H), 7.54 (dd, J = 7.5, 1.5 Hz, 1H), 7.19 (dd, J = 11.4, 6.0 Hz, 1H), 6.92 (d, J = 8.2 Hz, 1H), 5.63 (s, 2H), 5.19 (tt, J = 7.1, 3.5 Hz, 1H), 4.80 - 4.36 (m, 7H), 2.80 (dtd, J = 10.8, 7.9, 5.8 Hz, 1H), 2.59 - 2.41 (m, 1H), 1.70 - 1.52 (m, 2H), 1.44 - 1.31 (m, 2H).

### Example 184: (S)-2-(4-(6-((4-chloro-6-((1-cyanocyclopropyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((1-cyanocyclopropyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.77 (s, 1H), 8.31 (d, J = 1.4 Hz, 1H), 8.17 (s, 1H), 7.98 (dd, J = 8.5, 1.5 Hz, 1H), 7.82 (t, J = 7.9 Hz, 1H), 7.76 (dd, J = 10.7, 6.4 Hz, 1H), 7.66 (d, J = 8.5 Hz, 1H), 7.56 (dd, J = 7.5, 1.5 Hz, 1H), 7.18 (dd, J = 11.5, 6.0 Hz, 1H), 6.94 (d, J = 8.3 Hz, 1H), 5.72 (s, 2H), 5.18 (tt, J = 7.2, 3.7 Hz, 1H), 4.73 (dd, J = 15.7, 7.0 Hz, 1H), 4.68 - 4.38 (m, 5H), 2.91 - 2.65 (m, 1H), 2.56 - 2.36 (m, 1H), 1.67 - 1.54 (m, 2H), 1.46 - 1.35 (m, 2H).

### Example 185: (S)-2-(4-(6-((4-chloro-6-((1-cyanocyclopropyl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((4-chloro-6-((1-cyanocyclopropyl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 10. 1H NMR (400 MHz, Methanol-d4) δ 8.76 (s, 1H), 8.29 - 8.03 (m, 2H), 7.81 (t, J = 7.9 Hz, 1H), 7.75 (dd, J = 10.7, 6.3 Hz, 1H), 7.65 (dd, J = 11.2, 1.2 Hz, 1H), 7.55 (dd, J = 7.5, 1.5 Hz, 1H), 7.18 (dd, J = 11.5, 6.0 Hz, 1H), 6.93 (d, J = 8.2 Hz, 1H), 5.70 (s, 2H), 5.15 (qd, J = 7.1, 2.5 Hz, 1H), 4.72 (dd, J = 15.7, 7.1 Hz, 1H), 4.67 - 4.46 (m, 4H), 4.43 (dt, J = 9.2, 6.0 Hz, 1H), 2.78 (dtd, J = 11.4, 8.2, 6.1 Hz, 1H), 2.47 (ddt, J= 11.5, 9.1, 7.2 Hz, 1H), 1.66 - 1.51 (m, 2H), 1.44 - 1.34 (m, 2H).

### Example 186: (S)-2-(4-(6-((4-chloro-6-((1-(difluoromethyl)cyclopropyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((1-(difluoromethyl)cyclopropyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.77 (s, 1H), 8.31 (d, J = 1.4 Hz, 1H), 8.14 (s, 1H), 7.98 (dd, J = 8.5, 1.5 Hz, 1H), 7.82 (t, J = 7.9 Hz, 1H), 7.77 (dd, J = 10.7, 6.4 Hz, 1H), 7.66 (d, J = 8.5 Hz, 1H), 7.56 (dd, J = 7.4, 1.5 Hz, 1H), 7.18 (dd, J = 11.4, 6.0 Hz, 1H), 6.94 (d, J = 8.2 Hz, 1H), 5.99 (t, J = 57.3 Hz, 1H), 5.71 (s, 2H), 5.19 (qd, J = 7.1, 2.6 Hz, 1H), 4.72 (dd, J = 15.7, 7.0 Hz, 1H), 4.68 - 4.33 (m, 5H), 2.79 (dtd, J = 11.4, 8.1, 6.1 Hz, 1H), 2.49 (ddt, J = 11.5, 9.1, 7.2 Hz, 1H), 1.23 - 1.15 (m, 2H), 1.05 (p, J = 2.5 Hz, 2H).

### Example 187: (S)-2-(4-(6-((4-chloro-6-((3-cyanooxetan-3-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((3-cyanooxetan-3-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.82 (s, 1H), 8.31 (d, J = 1.4 Hz, 1H), 8.17 (s, 1H), 7.98 (dd, J = 8.5, 1.5 Hz, 1H), 7.83 (t, J = 7.9 Hz, 1H), 7.77 (dd, J = 10.7, 6.3 Hz, 1H), 7.67 (d, J = 8.5 Hz, 1H), 7.56 (dd, J = 7.4, 1.5 Hz, 1H), 7.19 (dd, J = 11.5, 6.0 Hz, 1H), 6.96 (d, J = 8.2 Hz, 1H), 5.73 (s, 2H), 5.19 (qd, J = 7.1, 2.5 Hz, 1H), 5.03 (d, J = 7.3 Hz, 2H), 4.90 (dd, J = 8.8, 1.9 Hz, 2H), 4.73 (dd, J = 15.7, 7.0 Hz, 1H), 4.68 - 4.35 (m, 6H), 2.97 - 2.68 (m, 1H), 2.49 (ddt, J = 11.5, 9.1, 7.1 Hz, 1H).

### Example 188: (S)-2-(4-(6-((4-chloro-6-((3-cyanooxetan-3-yl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((4-chloro-6-((3-cyanooxetan-3-yl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 10. 1H NMR (400 MHz, Methanol-d4) δ 8.82 (s, 1H), 8.17 (s, 1H), 8.16 (d, J = 1.3 Hz, 1H), 7.83 (t, J = 7.9 Hz, 1H), 7.77 (dd, J = 10.7, 6.3 Hz, 1H), 7.65 (dd, J = 11.1, 1.2 Hz, 1H), 7.56 (dd, J = 7.4, 1.5 Hz, 1H), 7.19 (dd, J = 11.5, 6.1 Hz, 1H), 6.96 (d, J = 8.2 Hz, 1H), 5.73 (s, 2H), 5.16 (tt, J = 7.6, 3.8 Hz, 1H), 5.03 (d, J = 7.3 Hz, 2H), 4.93 - 4.89 (m, 2H), 4.73 (dd, J = 15.7, 7.1 Hz, 1H), 4.68 - 4.34 (m, 6H), 2.91 - 2.66 (m, 1H), 2.57 - 2.37 (m, 1H).

### Example 189: (S)-2-(4-(6-((4-chloro-6-((1-cyanocyclobutyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((1-cyanocyclobutyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.80 (s, 1H), 8.32 (d, J = 1.4 Hz, 1H), 8.15 (s, 1H), 7.99 (dd, J = 8.5, 1.5 Hz, 1H), 7.83 (t, J = 7.9 Hz, 1H), 7.77 (dd, J = 10.7, 6.3 Hz, 1H), 7.67 (d, J = 8.5 Hz, 1H), 7.57 (dd, J = 7.4, 1.5 Hz, 1H), 7.19 (dd, J = 11.5, 6.0 Hz, 1H), 6.96 (d, J = 8.2 Hz, 1H), 5.73 (s, 2H), 5.19 (qd, J = 7.0, 2.5 Hz, 1H), 4.74 (dd, J = 15.7, 7.0 Hz, 1H), 4.68 - 4.32 (m, 6H), 2.92 - 2.70 (m, 3H), 2.70 - 2.41 (m, 3H), 2.21 (dt, J = 11.6, 8.6 Hz, 1H), 2.16 - 2.04 (m, 1H).

### Example 190: (S)-2-(4-(6-((4-chloro-6-((1-cyanocyclobutyl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((4-chloro-6-((1-cyanocyclobutyl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 10. 1H NMR (400 MHz, Methanol-d4) δ 8.78 (s, 1H), 8.15 (d, J = 1.2 Hz, 1H), 8.13 (s, 1H), 7.81 (t, J = 7.9 Hz, 1H), 7.75 (dd, J = 10.7, 6.3 Hz, 1H), 7.63 (d, J = 1.2 Hz, 1H), 7.54 (dd, J = 7.5, 1.5 Hz, 1H), 7.18 (dd, J = 11.5, 6.1 Hz, 1H), 6.94 (d, J = 8.2 Hz, 1H), 5.70 (s, 2H), 5.15 (qd, J = 7.2, 2.5 Hz, 1H), 4.72 (dd, J = 15.7, 7.1 Hz, 1H), 4.67 - 4.35 (m, 6H), 2.79 (ddt, J = 11.2, 8.6, 4.7 Hz, 3H), 2.65 - 2.52 (m, 2H), 2.52 - 2.38 (m, 1H), 2.32 - 2.15 (m, 1H), 2.10 (tdd, J = 9.4, 6.9, 4.7 Hz, 1H).

### Example 191: (S)-2-(4-(6-((4-chloro-6-((2,2,2-trifluoroethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((2,2,2-trifluoroethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, DMSO-d6) δ 12.75 (s, 1H), 9.43 (t, J = 6.6 Hz, 1H), 8.87 (s, 1H), 8.25 (d, J = 1.5 Hz, 1H), 8.14 (s, 1H), 7.90 (t, J = 7.9 Hz, 1H), 7.84 - 7.74 (m, 2H), 7.60 (d, J = 8.4 Hz, 1H), 7.54 (dd, J = 7.5, 1.6 Hz, 1H), 7.39 (dd, J = 11.5, 6.0 Hz, 1H), 7.00 (d, J = 8.3 Hz, 1H), 5.68 (s, 2H), 5.07 (qd, J = 7.1, 2.7 Hz, 1H), 4.75 (dd, J = 15.6, 7.1 Hz, 1H), 4.62 (dd, J = 15.6, 2.8 Hz, 1H), 4.57 - 4.40 (m, 3H), 4.35 (dt, J = 9.0, 5.9 Hz, 1H), 4.07 (qd, J = 9.6, 6.5 Hz, 2H).

### Example 192: (S)-2-(4-(6-((4-chloro-6-((2,2-difluoroethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((2,2-difluoroethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.78 (s, 1H), 8.31 (s, 1H), 8.15 (s, 1H), 8.02 - 7.94 (m, 1H), 7.88 - 7.71 (m, 2H), 7.66 (d, J = 8.4 Hz, 1H), 7.55 (d, J = 7.1 Hz, 1H), 7.18 (dd, J = 11.5, 6.0 Hz, 1H), 6.94 (d, J = 8.2 Hz, 1H), 6.20 - 5.80 (m, 1H), 5.71 (s, 2H), 5.18 (d, J = 7.6 Hz, 1H), 4.66 - 4.55 (m, 2H), 4.55 - 4.37 (m, 2H), 3.84 - 3.67 (m, 2H), 3.03 - 2.71 (m, 1H), 2.48 (s, 1H).

### Example 193: (S)-2-(4-(6-((4-chloro-6-(((1-methyl-1H-pyrazol-4-yl)methyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-(((1-methyl-1H-pyrazol-4-yl)methyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.74 (s, 1H), 8.35 (d, J = 1.4 Hz, 1H), 8.14 (s, 1H), 8.01 (dd, J = 8.5, 1.5 Hz, 1H), 7.88 - 7.73 (m, 2H), 7.67 (d, J = 8.5 Hz, 1H), 7.55 (d, J = 7.9 Hz, 2H), 7.45 (s, 1H), 7.20 (dd, J = 11.4, 6.0 Hz, 1H), 6.93 (d, J = 8.2 Hz, 1H), 5.69 (s, 2H), 5.18 (td, J = 7.7, 7.3, 5.2 Hz, 1H), 4.76 (dd, J = 15.7, 7.1 Hz, 1H), 4.62 (dt, J = 15.4, 3.6 Hz, 3H), 4.53 (d, J = 16.8 Hz, 1H), 4.43 (d, J = 4.6 Hz, 3H), 3.82 (s, 3H), 2.89 - 2.38 (m, 2H).

### Example 194: (S)-2-(4-(6-((4-chloro-6-((1-methyl-1H-pyrazol-4-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((1-methyl-1H-pyrazol-4-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, DMSO-d6) δ 11.00 (s, 1H), 8.88 (s, 1H), 8.26 (s, 1H), 8.17 (s, 1H), 8.07 (s, 1H), 7.91 (t, J = 7.9 Hz, 1H), 7.85 - 7.76 (m, 2H), 7.71 (s, 1H), 7.60 (d, J = 8.4 Hz, 1H), 7.55 (d, J = 7.3 Hz, 1H), 7.40 (dd, J = 11.5, 6.0 Hz, 1H), 7.01 (d, J = 8.2 Hz, 1H), 5.70 (s, 2H), 5.10 - 5.01 (m, 1H), 4.75 (dd, J = 15.7, 7.0 Hz, 1H), 4.66 - 4.57 (m, 1H), 4.57 - 4.40 (m, 3H), 4.35 (dt, J = 8.8, 5.9 Hz, 1H), 3.82 (s, 3H), 2.69 - 2.65 (m, 1H), 2.41 - 2.29 (m, 1H).

### Example 195: (S)-2-(4-(6-((4-chloro-6-(((1-methyl-1H-pyrazol-5-yl)methyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-(((1-methyl-1H-pyrazol-5-yl)methyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. H NMR (400 MHz, DMSO-d6) δ 12.74 (s, 1H), 9.39 (t, J = 6.2 Hz, 1H), 8.83 (s, 1H), 8.25 (d, J = 1.5 Hz, 1H), 8.10 (s, 1H), 7.90 (t, J = 7.9 Hz, 1H), 7.84 - 7.76 (m, 2H), 7.59 (d, J = 8.4 Hz, 1H), 7.54 (dd, J = 7.6, 1.7 Hz, 1H), 7.39 (dd, J = 11.5, 6.1 Hz, 1H), 7.26 (d, J = 1.8 Hz, 1H), 6.99 (d, J = 8.3 Hz, 1H), 6.13 (d, J = 1.8 Hz, 1H), 5.67 (s, 2H), 5.07 (qd, J = 6.9, 2.6 Hz, 1H), 4.75 (dd, J = 15.6, 7.1 Hz, 1H), 4.62 (dd, J = 15.5, 2.7 Hz, 1H), 4.55 - 4.47 (m, 4H), 4.47 - 4.40 (m, 1H), 4.35 (dt, J = 9.0, 5.9 Hz, 1H), 3.82 (s, 3H), 2.75 - 2.66 (m, 1H), 2.43 - 2.28 (m, 1H).

### Example 196: (S)-2-(4-(6-((4-chloro-6-((1-methyl-1H-pyrazol-5-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((1-methyl-1H-pyrazol-5-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, DMSO-d6) δ 10.74 (s, 1H), 8.91 (s, 1H), 8.25 (d, J = 1.5 Hz, 1H), 8.20 (s, 1H), 7.91 (t, J = 7.9 Hz, 1H), 7.86 - 7.74 (m, 2H), 7.59 (d, J = 8.4 Hz, 1H), 7.55 (dd, J = 7.5, 1.6 Hz, 1H), 7.44 - 7.33 (m, 2H), 7.02 (d, J = 8.3 Hz, 1H), 6.25 (d, J = 2.0 Hz, 1H), 5.72 (s, 2H), 5.07 (d, J = 7.3 Hz, 1H), 4.75 (dd, J = 15.5, 7.0 Hz, 1H), 4.62 (dd, J = 15.6, 2.8 Hz, 1H), 4.56 - 4.39 (m, 3H), 4.35 (dt, J = 8.9, 5.9 Hz, 1H), 3.68 (s, 3H), 2.79 - 2.62 (m, 1H), 2.44 - 2.30 (m, 1H).

### Example 197: (S)-2-(4-(6-((6-((1-cyanocyclopropyl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((6-((1-cyanocyclopropyl)carbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 10. Multiplet Report 1H NMR (400 MHz, DMSO-d6) δ 9.73 (s, 1H), 8.79 (d, J = 1.9 Hz, 1H), 8.22 - 8.03 (m, 3H), 7.90 (t, J = 7.9 Hz, 1H), 7.83 (dd, J = 10.4, 6.4 Hz, 1H), 7.57 - 7.47 (m, 2H), 7.41 (dd, J = 11.5, 6.1 Hz, 1H), 7.00 (d, J = 8.3 Hz, 1H), 5.63 (s, 2H), 5.08 (qd, J = 7.1, 2.6 Hz, 1H), 4.80 (dd, J = 15.6, 7.1 Hz, 1H), 4.66 (dd, J = 15.6, 2.7 Hz, 1H), 4.60 - 4.42 (m, 3H), 4.36 (dt, J = 9.0, 5.9 Hz, 1H), 2.72 (dtd, J = 11.4, 8.3, 6.4 Hz, 1H), 2.39 (ddt, J = 11.3, 9.1, 7.1 Hz, 1H), 1.62 - 1.48 (m, 2H), 1.45 - 1.27 (m, 2H).

### Example 198: (S)-2-(4-(6-((4-chloro-6-(methylcarbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((4-chloro-6-(methylcarbamoyl)pyridin-3-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 10. Multiplet Report 1H NMR (400 MHz, DMSO-d6) δ 8.86 (q, J = 4.8 Hz, 1H), 8.82 (s, 1H), 8.16 (d, J = 1.3 Hz, 1H), 8.08 (s, 1H), 7.91 (t, J = 7.9 Hz, 1H), 7.83 (dd, J = 10.4, 6.4 Hz, 1H), 7.60 - 7.45 (m, 2H), 7.41 (dd, J = 11.5, 6.1 Hz, 1H), 7.00 (d, J = 8.2 Hz, 1H), 5.67 (s, 2H), 5.07 (qd, J = 7.1, 2.6 Hz, 1H), 4.80 (dd, J = 15.6, 7.1 Hz, 1H), 4.66 (dd, J = 15.5, 2.7 Hz, 1H), 4.60 - 4.41 (m, 3H), 4.36 (dt, J = 9.0, 5.9 Hz, 1H), 2.82 (d, J = 4.8 Hz, 3H), 2.78 - 2.63 (m, 1H), 2.45 - 2.31 (m, 1H).

### Example 199: (S)-2-(4-(6-((4-chloro-6-((2-cyanopropan-2-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-((2-cyanopropan-2-yl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, DMSO-d6) δ 9.03 (s, 1H), 8.84 (s, 1H), 8.25 (d, J = 1.5 Hz, 1H), 8.14 (s, 1H), 7.91 (t, J = 7.9 Hz, 1H), 7.85 - 7.72 (m, 2H), 7.66 - 7.52 (m, 2H), 7.39 (dd, J = 11.5, 6.1 Hz, 1H), 7.01 (d, J = 8.2 Hz, 1H), 5.70 (s, 2H), 5.07 (qd, J = 7.1, 2.7 Hz, 1H), 4.75 (dd, J = 15.6, 7.0 Hz, 1H), 4.62 (dd, J = 15.5, 2.8 Hz, 1H), 4.58 - 4.40 (m, 3H), 4.36 (dt, J = 9.0, 5.9 Hz, 1H), 2.72 (dtd, J = 11.3, 8.2, 6.3 Hz, 1H), 2.44 - 2.25 (m, 1H), 1.72 (s, 6H).

### Example 200: 2-(4-(6-((4-chloro-6-(((R)-1-cyanoethyl)carbamoyl)pyridin-3-yl)methoxy)pyridin-2-yl)-2,5-difluorobenzyl)-1-(((S)-oxetan-2-yl)methyl)-1H-benzo[d]imidazole-6-carboxylic acid

2-(4-(6-((4-chloro-6-(((R)-1-cyanoethyl)carbamoyl)pyridin-3-yl)methoxy)pyridin-2-yl)-2,5-difluorobenzyl)-1-(((S)-oxetan-2-yl)methyl)-1H-benzo[d]imidazole-6-carboxylic acid was prepared in a manner as described in Procedure 10. 1H NMR (400 MHz, DMSO-d6) δ 9.65 (d, J = 8.0 Hz, 1H), 8.86 (s, 1H), 8.25 (d, J = 1.5 Hz, 1H), 8.14 (s, 1H), 7.92 (d, J = 7.9 Hz, 1H), 7.89 - 7.74 (m, 2H), 7.72 - 7.50 (m, 2H), 7.40 (dd, J = 11.5, 6.0 Hz, 1H), 7.01 (d, J = 8.2 Hz, 1H), 5.69 (s, 2H), 5.17 - 4.94 (m, 2H), 4.75 (dd, J = 15.6, 7.0 Hz, 1H), 4.62 (dd, J = 15.5, 2.8 Hz, 1H), 4.58 - 4.41 (m, 3H), 4.36 (dt, J = 9.1, 5.9 Hz, 1H), 2.86 - 2.65 (m, 1H), 2.39 (ddt, J = 11.2, 9.0, 6.9 Hz, 1H), 1.55 (d, J = 7.2 Hz, 3H).

### Example 201: 2-(4-(6-((4-chloro-6-(((S)-1-cyanoethyl)carbamoyl)pyridin-3-yl)methoxy)pyridin-2-yl)-2,5-difluorobenzyl)-1-(((S)-oxetan-2-yl)methyl)-1H-benzo[d]imidazole-6-carboxylic acid

2-(4-(6-((4-chloro-6-(((S)-1-cyanoethyl)carbamoyl)pyridin-3-yl)methoxy)pyridin-2-yl)-2,5-difluorobenzyl)-1-(((S)-oxetan-2-yl)methyl)-1H-benzo[d]imidazole-6-carboxylic acid was prepared in a manner as described in Procedure 10. 1H NMR (400 MHz, DMSO-d6) δ 9.65 (d, J = 8.0 Hz, 1H), 8.87 (s, 1H), 8.23 (s, 1H), 8.14 (s, 1H), 7.91 (t, J = 7.9 Hz, 1H), 7.88 - 7.75 (m, 2H), 7.56 (t, J = 7.3 Hz, 2H), 7.39 (dd, J = 11.5, 6.0 Hz, 1H), 7.01 (d, J = 8.2 Hz, 1H), 5.69 (s, 2H), 5.20 - 4.95 (m, 2H), 4.74 (dd, J = 15.6, 7.0 Hz, 1H), 4.61 (dd, J = 15.6, 2.8 Hz, 1H), 4.58 - 4.40 (m, 3H), 4.36 (dt, J = 9.1, 5.9 Hz, 1H), 2.84 - 2.65 (m, 1H), 2.47 - 2.27 (m, 1H), 1.55 (d, J = 7.2 Hz, 3H).

### Example 202: (R)-2-(4-(6-((6-((1-cyanocyclopropyl)carbamoyl)pyridin-3-yl)methoxy)pyridin-2-yl)-2,5-difluorobenzyl)-1-(4,4-dimethyltetrahydrofuran-3-yl)-1H-benzo [d] imidazole-6-carboxylic acid

(R)-2-(4-(6-((6-((1-cyanocyclopropyl)carbamoyl)pyridin-3-yl)methoxy)pyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 10. 1H NMR (400 MHz, DMSO-d6) δ 9.73 (s, 1H), 8.79 (d, J = 1.9 Hz, 1H), 8.51 (s, 1H), 8.17 - 8.03 (m, 2H), 7.95 - 7.79 (m, 3H), 7.64 (d, J = 8.4 Hz, 1H), 7.55 (dd, J = 7.6, 1.5 Hz, 1H), 7.48 (dd, J = 11.2, 6.3 Hz, 1H), 7.01 (d, J = 8.3 Hz, 1H), 5.64 (s, 2H), 5.04 (d, J = 6.6 Hz, 1H), 4.60 - 4.50 (m, 2H), 4.49 - 4.36 (m, 2H), 3.83 - 3.70 (m, 2H), 1.58 - 1.50 (m, 2H), 1.33 (d, J = 4.1 Hz, 5H), 0.61 (s, 3H).

### Example 203: (S)-2-(4-(2-((4-carbamoyl-2-fluorobenzyl)oxy)pyrimidin-4-yl)-2,5-difluorobenzyl)-1-(oxetan-2-ylmethyl)-1H-benzo[d]imidazole-6-carboxylic acid

(S)-2-(4-(2-((4-carbamoyl-2-fluorobenzyl)oxy)pyrimidin-4-yl)-2,5-difluorobenzyl)-1-(oxetan-2-ylmethyl)-1H-benzo[d]imidazole-6-carboxylic acid was prepared in a manner as described in Procedure 10. 1H NMR (400 MHz, Methanol-d4) δ 8.72 (d, J = 5.2 Hz, 1H), 8.53 (dd, J = 1.5, 0.7 Hz, 1H), 8.18 (dd, J = 8.6, 1.4 Hz, 1H), 8.04 (dd, J = 10.4, 6.1 Hz, 1H), 7.82 - 7.62 (m, 5H), 7.43 (dd, J = 11.3, 5.9 Hz, 1H), 5.69 (s, 2H), 5.27 (tt, J = 7.5, 3.8 Hz, 1H), 5.01 - 4.92 (m, 1H), 4.85 - 4.64 (m, 4H), 4.52 (dt, J = 9.1, 6.0 Hz, 1H), 2.87 (dtd, J = 11.5, 8.2, 6.2 Hz, 1H), 2.57 (ddt, J = 11.6, 9.1, 7.2 Hz, 1H), 1.96 (s, 1H).

### Example 254: (S)-2-(4-(6-((5-chlorothiazol-2-yl)methoxy)pyridin-2-yl)-2,5-difluorobenzyl)-1-(4,4-dimethyltetrahydrofuran-3-yl)-1H-benzo[d]imidazole-6-carboxylic acid

(S)-2-(4-(6-((5-chlorothiazol-2-yl)methoxy)pyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 10 using Intermediates I**-1223** and **I-82.** ES/MS m/z: 611.1 (M+H⁺). 1H NMR (400 MHz, Methanol-d4) δ 8.89 (s, 1H), 8.17 (dd, J = 8.6, 1.4 Hz, 1H), 7.98 (dd, J = 10.8, 6.3 Hz, 1H), 7.88 (t, J = 7.9 Hz, 1H), 7.78 (d, J = 8.6 Hz, 1H), 7.70 - 7.59 (m, 2H), 7.41 (dd, J = 11.2, 6.1 Hz, 1H), 6.97 (d, J = 8.3 Hz, 1H), 5.72 (s, 2H), 5.13 (d, J = 6.5 Hz, 1H), 4.79 - 4.59 (m, 3H), 4.53 (dd, J = 11.6, 6.7 Hz, 1H), 4.00 (d, J = 8.9 Hz, 1H), 3.84 (d, J = 8.9 Hz, 1H), 1.41 (s, 3H), 0.75 (s, 3H).

### Example 255: (S)-2-(4-(6-((5-chlorothiazol-2-yl)methoxy)-5-fluoropyridin-2-yl)-2,5-difluorobenzyl)-1-(4,4-dimethyltetrahydrofuran-3-yl)-1H-benzo [d] imidazole-6-carboxylic acid

(S)-2-(4-(6-((5-chlorothiazol-2-yl)methoxy)-5-fluoropyridin-2-yl)-2, 5-difluorobenzyl)-1-(4,4-dimethyltetrahydrofuran-3-yl)-1H-benzo[d]imidazole-6-carboxylic acid was prepared in a manner as described in Procedure 10 using Intermediates **I-1224** and **I-82.** ES/MS m/z: 629.1 (M+H⁺). 1H NMR (400 MHz, Methanol-d4) δ 8.85 (s, 1H), 8.13 (dd, J = 8.6, 1.4 Hz, 1H), 7.93 (dd, J = 10.8, 6.4 Hz, 1H), 7.76 (d, J = 8.5 Hz, 1H), 7.73 - 7.61 (m, 3H), 7.38 (dd, J = 11.3, 6.0 Hz, 1H), 5.80 (s, 2H), 5.10 (d, J = 6.5 Hz, 1H), 4.74 - 4.56 (m, 3H), 4.52 (dd, J = 11.5, 6.8 Hz, 1H), 3.99 (d, J = 8.9 Hz, 1H), 3.84 (d, J = 8.9 Hz, 1H), 1.40 (s, 3H), 0.74 (s, 3H).

### Example 256: 2-(4-(6-((5-ethoxy-1,3,4-thiadiazol-2-yl)methoxy)-5-fluoropyridin-2-yl)-2,5-difluorobenzyl)-1-((1-(fluoromethyl)cyclopropyl)methyl)-1H-benzo [d] imidazole-6-carboxylic acid

2-(4-(6-((5-ethoxy-1,3,4-thiadiazol-2-yl)methoxy)-5-fluoropyridin-2-yl)-2,5-difluorobenzyl)-1-((1-(fluoromethyl)cyclopropyl)methyl)-1H-benzo[d]imidazole-6-carboxylic acid was prepared in a manner as described in Procedure 10 using Intermediates **I-1139** and **I-1180.** ES/MS m/z: 628.4 (M+H⁺). 1H NMR (400 MHz, DMSO-d6) δ 8.24 (d, J = 1.5 Hz, 1H), 7.90 (ddd, J = 10.0, 7.5, 2.3 Hz, 2H), 7.79 (dd, J = 8.4, 1.5 Hz, 1H), 7.61 (d, J = 8.5 Hz, 2H), 7.43 (dd, J = 11.6, 6.1 Hz, 1H), 5.83 (s, 2H), 4.67 - 4.37 (m, 6H), 4.24 (s, 1H), 4.11 (d, J = 6.3 Hz, 1H), 1.37 (t, J = 7.0 Hz, 3H), 0.90 - 0.78 (m, 2H), 0.73 (d, J = 5.0 Hz, 2H).

### Example 257: (S)-2-(4-(6-((5-chlorothiazol-2-yl)methoxy)pyridin-2-yl)-2-fluoro-5-methylbenzyl)-1-(4,4-dimethyltetrahydrofuran-3-yl)-1H-benzo[d]imidazole-6-carboxylic acid

(S)-2-(4-(6-((5-chlorothiazol-2-yl)methoxy)pyridin-2-yl)-2-fluoro-5-methylbenzyl)-1-(4,4-dimethyltetrahydrofuran-3-yl)-1H-benzo[d]imidazole-6-carboxylic acid was prepared in a manner as described in Procedure 10 using Intermediates **I-1223** and **I-1076.** ES/MS m/z: 607.5 (M+H⁺). 1H NMR (400 MHz, Methanol-d4) δ 8.93 (s, 1H), 8.22 (dd, J = 8.6, 1.4 Hz, 1H), 7.88 (dd, J = 8.3, 7.4 Hz, 1H), 7.82 (d, J = 8.6 Hz, 1H), 7.65 (s, 1H), 7.42 (d, J = 7.6 Hz, 1H), 7.35 (d, J = 10.8 Hz, 1H), 7.23 (d, J = 7.3 Hz, 1H), 6.96 (d, J = 8.3 Hz, 1H), 5.64 (s, 2H), 5.16 (d, J = 6.4 Hz, 1H), 4.79 - 4.61 (m, 3H), 4.52 (dd, J = 11.7, 6.6 Hz, 1H), 4.01 (d, J = 9.0 Hz, 1H), 3.84 (d, J = 8.9 Hz, 1H), 2.38 (s, 3H), 1.37 (s, 3H), 0.73 (s, 3H).

### Example 258: (S)-1-(4,4-dimethyltetrahydrofuran-3-yl)-2-(2-fluoro-4-(5-fluoro-6-((5-methoxy-1,3,4-thiadiazol-2-yl)methoxy)pyridin-2-yl)-5-methylbenzyl)-1H-benzo[d]imidazole-6-carboxylic acid

(S)-1-(4,4-dimethyltetrahydrofuran-3-yl)-2-(2-fluoro-4-(5-fluoro-6-((5-methoxy-1,3,4-thiadiazol-2-yl)methoxy)pyridin-2-yl)-5-methylbenzyl)-1H-benzo[d]imidazole-6-carboxylic acid was prepared in a manner as described in Procedure 10 using Intermediates **I-1244** and **I-1076.** ES/MS m/z: 622.3 (M+H⁺). 1H NMR (400 MHz, Methanol-d4) δ 8.93 (s, 1H), 8.22 (dd, J = 8.6, 1.4 Hz, 1H), 7.88 (dd, J = 8.3, 7.4 Hz, 1H), 7.82 (d, J = 8.6 Hz, 1H), 7.65 (s, 1H), 7.42 (d, J = 7.6 Hz, 1H), 7.35 (d, J = 10.8 Hz, 1H), 7.23 (d, J = 7.3 Hz, 1H), 6.96 (d, J = 8.3 Hz, 1H), 5.64 (s, 2H), 5.16 (d, J = 6.4 Hz, 1H), 4.79 - 4.61 (m, 3H), 4.52 (dd, J = 11.7, 6.6 Hz, 1H), 4.01 (d, J = 9.0 Hz, 1H), 3.84 (d, J = 8.9 Hz, 1H), 2.38 (s, 3H), 1.37 (s, 3H), 0.73 (s, 3H).

### Example 260: (S)-1-(4,4-dimethyltetrahydrofuran-3-yl)-2-((3'-((5-ethoxy-1,3,4-thiadiazol-2-yl)methoxy)-4',5-difluoro-2-methyl-[1,1'-biphenyl]-4-yl)methyl)-1H-benzo[d]imidazole-6-carboxylic acid

(S)-1-(4,4-dimethyltetrahydrofuran-3-yl)-2-((3'-((5-ethoxy-1,3,4-thiadiazol-2-yl)methoxy)-4',5-difluoro-2-methyl-[1,1'-biphenyl]-4-yl)methyl)-1H-benzo[d]imidazole-6-carboxylic acid was prepared in a manner as described in Procedure 10 using Intermediates **I-1183** and **I-1076.** ES/MS m/z: 635.2 (M+H⁺). 1H NMR (400 MHz, Methanol-d4) δ 8.69 (s, 1H), 7.99 (dd, J = 8.5, 1.5 Hz, 1H), 7.69 (d, J = 8.5 Hz, 1H), 7.29 - 7.15 (m, 3H), 7.04 (d, J = 10.5 Hz, 1H), 6.98 (ddd, J = 8.3, 4.3, 2.1 Hz, 1H), 5.45 (s, 2H), 4.94 (s, 1H), 4.64 - 4.52 (m, 3H), 4.52 - 4.40 (m, 3H), 3.96 (d, J = 8.7 Hz, 1H), 3.80 (d, J = 8.8 Hz, 1H), 2.16 (s, 3H), 1.47 (t, J = 7.1 Hz, 3H), 1.34 (s, 3H), 0.66 (s, 3H).

### Example 261: (S)-1-(4,4-dimethyltetrahydrofuran-3-yl)-2-(2-fluoro-4-(6-((5-methoxy-1,3,4-thiadiazol-2-yl)methoxy)pyridin-2-yl)-5-methylbenzyl)-1H-benzo[d]imidazole-6-carboxylic acid

(S)-1-(4,4-dimethyltetrahydrofuran-3-yl)-2-(2-fluoro-4-(6-((5-methoxy-1,3,4-thiadiazol-2-yl)methoxy)pyridin-2-yl)-5-methylbenzyl)-1H-benzo[d]imidazole-6-carboxylic acid was prepared in a manner as described in Procedure 10 using Intermediates **I-1090** and **I-1076.** ES/MS m/z: 604.4 (M+H⁺). 1H NMR (400 MHz, Methanol-d4) δ 8.67 (s, 1H), 7.99 (dd, J = 8.5, 1.5 Hz, 1H), 7.85 (t, J = 7.8 Hz, 1H), 7.70 (d, J = 8.5 Hz, 1H), 7.31 (d, J = 10.7 Hz, 1H), 7.22 (dd, J = 9.3, 7.5 Hz, 2H), 6.90 (d, J = 8.3 Hz, 1H), 5.62 (s, 2H), 4.52 - 4.42 (m, 3H), 4.14 (s, 3H), 3.97 (d, J = 8.7 Hz, 1H), 3.79 (d, J = 8.7 Hz, 1H), 2.34 (s, 3H), 1.32 (s, 3H), 0.64 (s, 3H).

### Example 262: (S)-1-(4,4-dimethyltetrahydrofuran-3-yl)-2-(4-(6-((5-ethoxy-1,3,4-thiadiazol-2-yl)methoxy)-5-fluoropyridin-2-yl)-2-fluoro-5-methylbenzyl)-1H-benzo [d]imidazole-6-carboxylic acid

(S)-1-(4,4-dimethyltetrahydrofuran-3-yl)-2-(4-(6-((5-ethoxy-1,3,4-thiadiazol-2-yl)methoxy)-5-fluoropyridin-2-yl)-2-fluoro-5-methylbenzyl)-1H-benzo[d]imidazole-6-carboxylic acid was prepared in a manner as described in Procedure 10 using Intermediates **I-1139** and **I-1076.** ES/MS m/z: 636.3 (M+H⁺). 1H NMR (400 MHz, Methanol-d4) δ 8.68 (s, 1H), 7.99 (dd, J = 8.5, 1.5 Hz, 1H), 7.79 - 7.60 (m, 2H), 7.30 (d, J = 10.6 Hz, 1H), 7.27 - 7.18 (m, 2H), 5.69 (s, 2H), 4.93 (s, 1H), 4.67 - 4.49 (m, 3H), 4.49 - 4.35 (m, 3H), 3.96 (d, J = 8.8 Hz, 1H), 3.79 (d, J = 8.8 Hz, 1H), 2.33 (s, 3H), 1.44 (t, J = 7.1 Hz, 3H), 1.32 (s, 3H), 0.64 (s, 3H).

### Example 263: (S)-1-(4,4-dimethyltetrahydrofuran-3-yl)-2-(4-(6-((5-ethoxy-1,3,4-thiadiazol-2-yl)methoxy)pyridin-2-yl)-2,3,6-trifluorobenzyl)-1H-benzo[d]imidazole-6-carboxylic acid

(S)-1-(4,4-dimethyltetrahydrofuran-3-yl)-2-(4-(6-((5-ethoxy-1,3,4-thiadiazol-2-yl)methoxy)pyridin-2-yl)-2,3,6-trifluorobenzyl)-1H-benzo[d]imidazole-6-carboxylic acid was prepared in a manner as described in Procedure 10 using Intermediates **I-1145** and **I-1231.** ES/MS m/z: 640.4 (M+H⁺). 1H NMR (400 MHz, Methanol-d4) δ 8.69 (s, 1H), 7.95 (dd, J = 8.5, 1.5 Hz, 1H), 7.91 (t, J = 7.9 Hz, 1H), 7.81 (ddd, J = 10.6, 5.7, 2.1 Hz, 1H), 7.67 (dd, J = 7.6, 1.6 Hz, 1H), 7.61 (d, J = 8.5 Hz, 1H), 6.97 (d, J = 8.3 Hz, 1H), 5.73 (s, 2H), 5.09 (d, J = 6.7 Hz, 1H), 4.71 (dd, J = 11.1, 1.6 Hz, 1H), 4.67 - 4.56 (m, 2H), 4.56 - 4.50 (m, 2H), 4.43 (d, J = 17.0 Hz, 1H), 3.97 (d, J = 8.7 Hz, 1H), 3.84 (d, J = 8.7 Hz, 1H), 1.54 - 1.39 (m, 6H), 0.77 (s, 3H).

### Example 264: (S)-1-(4,4-dimethyltetrahydrofuran-3-yl)-2-(4-(6-((5-ethoxy-1,3,4-thiadiazol-2-yl)methoxy)pyridin-2-yl)-2-fluoro-5-methylbenzyl)-1H-benzo[d] imidazole-6-carboxylic acid

(S)-1-(4,4-dimethyltetrahydrofuran-3-yl)-2-(4-(6-((5-ethoxy-1,3,4-thiadiazol-2-yl)methoxy)pyridin-2-yl)-2-fluoro-5-methylbenzyl)-1H-benzo[d]imidazole-6-carboxylic acid was prepared in a manner as described in Procedure 10 using Intermediates **I-1145** and **I-1076.** ES/MS m/z: 618.3 (M+H⁺). 1HNMR (400 MHz, Methanol-d4) δ 8.68 (s, 1H), 7.99 (dd, J = 8.6, 1.4 Hz, 1H), 7.85 (t, J = 7.8 Hz, 1H), 7.70 (d, J = 8.5 Hz, 1H), 7.31 (d, J = 10.6 Hz, 1H), 7.22 (t, J = 7.9 Hz, 2H), 6.89 (d, J = 8.3 Hz, 1H), 5.61 (s, 2H), 4.92 (d, J = 7.9 Hz, 1H), 4.64 - 4.40 (m, 6H), 3.97 (d, J = 8.8 Hz, 1H), 3.79 (d, J = 8.8 Hz, 1H), 2.34 (s, 3H), 1.44 (t, J = 7.1 Hz, 3H), 1.32 (s, 3H), 0.64 (s, 3H).

### Example 265: (S)-1-(4,4-dimethyltetrahydrofuran-3-yl)-2-(4-(6-((5-ethoxy-1,3,4-thiadiazol-2-yl)methoxy)-5-fluoropyridin-2-yl)-2,3,6-trifluorobenzyl)-1H-benzo[d]imidazole-6-carboxylic acid

(S)-1-(4,4-dimethyltetrahydrofuran-3-yl)-2-(4-(6-((5-ethoxy-1,3,4-thiadiazol-2-yl)methoxy)-5-fluoropyridin-2-yl)-2,3,6-trifluorobenzyl)-1H-benzo[d]imidazole-6-carboxylic acid was prepared in a manner as described in Procedure 10 using Intermediates **I-1139** and **I-1185.** ES/MS m/z: 658.6 (M+H⁺). 1H NMR (400 MHz, Methanol-d4) δ 8.67 (s, 1H), 7.95 (dd, J = 8.6, 1.5 Hz, 1H), 7.82 - 7.64 (m, 3H), 7.59 (d, J = 8.5 Hz, 1H), 5.82 (s, 2H), 5.08 (d, J = 6.8 Hz, 1H), 4.72 (d, J = 11.0 Hz, 1H), 4.68 - 4.51 (m, 4H), 4.42 (d, J = 17.1 Hz, 1H), 3.99 (d, J = 8.7 Hz, 1H), 3.84 (d, J = 8.7 Hz, 1H), 1.50 - 1.40 (m, 6H), 0.77 (s, 3H).

### Example 270: (S)-1-(4,4-dimethyltetrahydrofuran-3-yl)-2-(2,3,6-trifluoro-4-(5-fluoro-6-((5-methoxy-1,3,4-thiadiazol-2-yl)methoxy)pyridin-2-yl)benzyl)-1H-benzo[d]imidazole-6-carboxylic acid

(S)-1-(4,4-dimethyltetrahydrofuran-3-yl)-2-(2,3,6-trifluoro-4-(5-fluoro-6-((5-methoxy-1,3,4-thiadiazol-2-yl)methoxy)pyridin-2-yl)benzyl)-1H-benzo[d]imidazole-6-carboxylic acid was prepared in a manner as described in Procedure 10 using Intermediates **I-1244** and **I-1231.** ES/MS m/z: 644.2 (M+H⁺). 1H NMR (400 MHz, Methanol-d4) δ 8.83 (s, 1H), 8.09 (dd, J = 8.5, 1.5 Hz, 1H), 7.88 - 7.79 (m, 1H), 7.79 - 7.66 (m, 3H), 5.82 (s, 2H), 5.16 (d, J = 6.6 Hz, 1H), 4.82 - 4.66 (m, 2H), 4.66 - 4.47 (m, 2H), 4.18 (s, 3H), 3.99 (d, J = 8.7 Hz, 1H), 3.86 (d, J = 8.8 Hz, 1H), 1.48 (s, 3H), 0.81 (s, 3H).

### Example 273: (S)-1-(4,4-dimethyltetrahydrofuran-3-yl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid

(S)-1-(4,4-dimethyltetrahydrofuran-3-yl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid was prepared in a manner as described in Procedure 10 using Intermediates **I-1090** and **I-1231.** ES/MS m/z: 626.9 (M+H⁺). 1H NMR (400 MHz, Methanol-d4) δ 8.85 (s, 1H), 8.11 (dd, J = 8.6, 1.4 Hz, 1H), 7.98 - 7.82 (m, 2H), 7.76 - 7.63 (m, 2H), 6.99 (d, J = 8.2 Hz, 1H), 5.74 (s, 2H), 5.17 (d, J = 6.5 Hz, 1H), 4.84 - 4.64 (m, 3H), 4.57 (dd, J = 11.6, 6.8 Hz, 1H), 4.16 (s, 3H), 4.00 (d, J = 8.8 Hz, 1H), 3.87 (d, J = 8.8 Hz, 1H), 1.48 (s, 3H), 0.81 (s, 3H).

### Example 281A: (S)-1-(4,4-dimethyltetrahydrofuran-3-yl)-2-(2,3,6-trifluoro-4-(6-((5-methyl-1,3,4-thiadiazol-2-yl)methoxy)pyridin-2-yl)benzyl)-1H-benzo[d]imidazole-6-carboxylic acid

(S)-1-(4,4-dimethyltetrahydrofuran-3-yl)-2-(2,3,6-trifluoro-4-(6-((5-methyl-1,3,4-thiadiazol-2-yl)methoxy)pyridin-2-yl)benzyl)-1H-benzo[d]imidazole-6-carboxylic acid was prepared in a manner as described in Procedure 10 using Intermediates **I-1207** and **I-1185,** then performing preparative chiral SFC (Daicel Chiralpak AD-H column, EtOH/CO₂ eluent) on the resulting racemic product; **Example 281A** was the later-eluting of two stereoisomers. ES/MS m/z: 610.5 (M+H⁺). 1HNMR (400 MHz, Methanol-d4) δ 8.83 (s, 1H), 8.09 (dd, J = 8.5, 1.4 Hz, 1H), 7.92 (t, J = 7.9 Hz, 1H), 7.83 (ddd, J = 10.6, 5.6, 2.1 Hz, 1H), 7.74 - 7.64 (m, 2H), 7.01 (d, J = 8.2 Hz, 1H), 5.89 (s, 2H), 5.17 (d, J = 6.5 Hz, 1H), 4.78 (d, J = 17.4 Hz, 1H), 4.75 - 4.67 (m, 1H), 4.64 - 4.52 (m, 2H), 3.99 (d, J = 8.9 Hz, 1H), 3.87 (d, J = 8.8 Hz, 1H), 2.75 (s, 3H), 1.48 (s, 3H), 0.81 (s, 3H).

### Example 281B: (R)-1-(4,4-dimethyltetrahydrofuran-3-yl)-2-(2,3,6-trifluoro-4-(6-((5-methyl-1,3,4-thiadiazol-2-yl)methoxy)pyridin-2-yl)benzyl)-1H-benzo [d] imidazole-6-carboxylic acid

(R)-1-(4,4-dimethyltetrahydrofuran-3-yl)-2-(2,3,6-trifluoro-4-(6-((5-methyl-1,3,4-thiadiazol-2-yl)methoxy)pyridin-2-yl)benzyl)-1H-benzo[d]imidazole-6-carboxylic acid was prepared in a manner as described in Procedure 10 using Intermediates **I-1207** and **I-1185,** then performing preparative chiral SFC (Daicel Chiralpak AD-H column, EtOH/CO₂ eluent) on the resulting racemic product; **Example 281B** was the earlier-eluting of two stereoisomers. ES/MS m/z: 610.3 (M+H⁺). 1HNMR (400 MHz, Methanol-d4) δ 8.84 (s, 1H), 8.10 (dd, J = 8.6, 1.4 Hz, 1H), 7.96 - 7.78 (m, 2H), 7.74 - 7.64 (m, 2H), 7.04 - 6.91 (m, 1H), 5.89 (s, 2H), 5.17 (d, J = 6.5 Hz, 1H), 4.78 (d, J = 17.4 Hz, 1H), 4.71 (d, J = 11.3 Hz, 1H), 4.65 - 4.52 (m, 2H), 4.00 (d, J = 8.8 Hz, 1H), 3.87 (d, J = 8.8 Hz, 1H), 2.75 (s, 3H), 1.48 (s, 3H), 0.81 (s, 3H).

### Example 282: 1-(4,4-dimethyltetrahydrofuran-3-yl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid

1-(4,4-dimethyltetrahydrofuran-3-yl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid was prepared in a manner as described in Procedure 10 using Intermediates 1-1090 and 1-1185. ES/MS m/z: 626.7 (M+H⁺). 1HNMR (400 MHz, Methanol-d4) δ 8.79 (s, 1H), 8.05 (dd, J = 8.5, 1.5 Hz, 1H), 7.97 - 7.83 (m, 2H), 7.68 (d, J = 8.6 Hz, 2H), 6.98 (d, J = 8.2 Hz, 1H), 5.51 (s, 2H), 5.14 (d, J = 6.5 Hz, 1H), 4.77 - 4.66 (m, 2H), 4.66 - 4.51 (m, 2H), 4.06 (s, 1H), 3.99 (d, J = 8.8 Hz, 1H), 3.86 (d, J = 8.8 Hz, 1H), 1.48 (d, J = 2.3 Hz, 3H), 0.80 (s, 3H).

### Example 283: (S)-2-(2,5-difluoro-4-(6-((5-methyl-1,3,4-thiadiazol-2-yl)methoxy)pyridin-2-yl)benzyl)-1-(4,4-dimethyltetrahydrofuran-3-yl)-4-fluoro-1H-benzo[d]imidazole-6-carboxylic acid

(S)-2-(2, 5-difluoro-4-(6-((5-methyl-1,3,4-thiadiazol-2-yl)methoxy)pyridin-2-yl)benzyl)-1-(4,4-dimethyltetrahydrofuran-3-yl)-4-fluoro-1H-benzo[d]imidazole-6-carboxylic acid was prepared in a manner as described in Procedure 10 using Intermediates **I-1207** and **I-105.** ES/MS m/z: 610.4 (M+H⁺). 1H NMR (400 MHz, Methanol-d4) δ 8.57 (s, 1H), 7.94 (dd, J = 10.8, 6.4 Hz, 1H), 7.85 (t, J = 7.9 Hz, 1H), 7.69 (dd, J = 11.0, 1.2 Hz, 1H), 7.62 (dd, J = 7.5, 1.5 Hz, 1H), 7.26 (dd, J = 11.4, 6.0 Hz, 1H), 6.94 (d, J = 8.2 Hz, 1H), 5.88 (s, 2H), 4.98 (d, J = 6.6 Hz, 1H), 4.65 - 4.43 (m, 4H), 3.94 (d, J = 8.8 Hz, 1H), 3.80 (d, J = 8.8 Hz, 1H), 2.74 (s, 3H), 1.36 (s, 3H), 0.68 (s, 3H).

### Example 290: (S)-2-(2,5-difluoro-4-(6-((5-methyl-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid

(S)-2-(2, 5-difluoro-4-(6-((5-methyl-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 10 using Intermediate **I-1207** and **I-82.** ES/MS m/z: 592.2 (M+H⁺). 1H NMR (400 MHz, Methanol-d4) δ 8.87 (s, 1H), 8.15 (dd, J = 8.6, 1.4 Hz, 1H), 7.98 (dd, J = 10.8, 6.3 Hz, 1H), 7.88 (t, J = 7.9 Hz, 1H), 7.77 (d, J = 8.5 Hz, 1H), 7.65 (dd, J = 7.6, 1.5 Hz, 1H), 7.39 (dd, J = 11.3, 6.0 Hz, 1H), 6.97 (d, J = 8.3 Hz, 1H), 5.88 (s, 2H), 5.12 (d, J = 6.6 Hz, 1H), 4.80 - 4.59 (m, 3H), 4.52 (dd, J = 11.5, 6.8 Hz, 1H), 4.00 (d, J = 8.9 Hz, 1H), 3.84 (d, J = 8.9 Hz, 1H), 2.75 (s, 3H), 1.41 (s, 3H), 0.75 (s, 3H).

### Example 313: (S)-2-(2,5-difluoro-4-(5-fluoro-6-((5-(trifluoromethyl)thiazol-2-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(5-fluoro-6-((5-(trifluoromethyl)thiazol-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 10 using Intermediate **I-1130** and **I-14.** ES/MS m/z: 653.0 (M+H⁺). 1HNMR (400 MHz, DMSO-d6) δ 8.49 (t, J = 1.3 Hz, 1H), 8.16 (d, J = 1.3 Hz, 1H), 7.93 (dd, J = 10.2, 8.3 Hz, 1H), 7.79 (dd, J = 10.5, 6.5 Hz, 1H), 7.66 - 7.56 (m, 1H), 7.51 (dd, J = 11.4, 1.3 Hz, 1H), 7.41 (dd, J = 11.6, 6.1 Hz, 1H), 5.97 (s, 2H), 5.07 (d, J = 7.0 Hz, 1H), 4.79 (dd, J = 15.6, 7.1 Hz, 1H), 4.65 (d, J = 14.5 Hz, 1H), 4.59 - 4.42 (m, 3H), 4.35 (dt, J = 8.9, 5.9 Hz, 1H), 2.71 (dd, J = 16.8, 8.9 Hz, 1H), 2.37 (dd, J = 19.2, 8.8 Hz, 1H).

### Example 315: 2-(2-chloro-5-fluoro-4-(6-((5-methoxy-1,3,4-thiadiazol-2-yl)methoxy)pyridin-2-yl)benzyl)-4-fluoro-1-(2-methoxyethyl)-1H-benzo[d]imidazole-6-carboxylic acid

2-(2-chloro-5-fluoro-4-(6-((5-methoxy-1,3,4-thiadiazol-2-yl)methoxy)pyridin-2-yl)benzyl)-4-fluoro-1-(2-methoxyethyl)-1H-benzo[d]imidazole-6-carboxylic acid was prepared in a manner as described in Procedure 10 using Intermediate **I-1090** and **I-1278.** ES/MS m/z: 602.0 (M+H⁺). 1H NMR (400 MHz, DMSO-d6) δ 8.16 (d, J = 7.2 Hz, 1H), 8.11 (d, J = 1.3 Hz, 1H), 7.93 (t, J = 7.9 Hz, 1H), 7.58 (dd, J = 7.5, 1.7 Hz, 1H), 7.51 (dd, J = 11.4, 1.3 Hz, 1H), 7.45 (d, J = 11.8 Hz, 1H), 7.00 (d, J = 8.3 Hz, 1H), 5.73 (s, 2H), 4.63 (t, J = 5.1 Hz, 2H), 4.54 (s, 2H), 4.11 (s, 3H), 3.70 (t, J = 5.0 Hz, 2H), 3.22 (s, 3H).

### Example 337: (S)-2-(4-(6-((5-(1,1-difluoroethyl)thiazol-2-yl)methoxy)-5-fluoropyridin-2-yl)-2-fluoro-5-methylbenzyl)-1-(4,4-dimethyltetrahydrofuran-3-yl)-1H-benzo[d]imidazole-6-carboxylic acid

(S)-2-(4-(6-((5-(1,1-difluoroethyl)thiazol-2-yl)methoxy)-5-fluoropyridin-2-yl)-2-fluoro-5-methylbenzyl)-1-(4,4-dimethyltetrahydrofuran-3-yl)-1H-benzo[d]imidazole-6-carboxylic acid was prepared in a manner as describe in Procedure 10 using Intermediate **I-1076** and **I-1078.** ES/MS m/z: 655.0 (M+H⁺). 1H NMR (400 MHz, DMSO) δ 8.48 (s, 1H), 8.17 (t, J = 1.8 Hz, 1H), 7.89 (dd, J = 10.4, 8.1 Hz, 1H), 7.81 (dd, J = 8.4, 1.5 Hz, 1H), 7.63 (d, J = 8.5 Hz, 1H), 7.37 - 7.27 (m, 3H), 5.81 (s, 2H), 5.00 (d, J = 6.6 Hz, 1H), 4.56 - 4.38 (m, 3H), 4.33 (d, J = 16.8 Hz, 1H), 3.92 (s, 26H), 3.78 (d, J = 8.6 Hz, 1H), 3.73 (d, J = 8.6 Hz, 1H), 2.24 (s, 3H), 2.17 (s, 1H), 2.13 (s, 2H), 2.08 (s, 1H), 1.31 (s, 3H), 0.59 (s, 3H).

### Example 338: (S)-2-(4-(6-((5-(1,1-difluoroethyl)thiazol-2-yl)methoxy)-5-fluoropyridin-2-yl)-2,5-difluorobenzyl)-1-(4,4-dimethyltetrahydrofuran-3-yl)-4-fluoro-1H-benzo[d]imidazole-6-carboxylic acid

(S)-2-(4-(6-((5-(1,1-difluoroethyl)thiazol-2-yl)methoxy)-5-fluoropyridin-2-yl)-2,5-difluorobenzyl)-1-(4,4-dimethyltetrahydrofuran-3-yl)-4-fluoro-1H-benzo[d]imidazole-6-carboxylic acid was prepared in a manner as described in Procedure 10 using Intermediate **I-108** and **I-1078.** ES/MS m/z: 677.0 (M+H⁺). 1H NMR (400 MHz, DMSO) δ 8.35 (s, 1H), 8.19 (d, J = 1.8 Hz, 1H), 7.96 - 7.81 (m, 2H), 7.62 (dd, J = 7.4, 2.7 Hz, 1H), 7.56 - 7.43 (m, 2H), 5.92 (s, 2H), 5.03 (d, J = 6.5 Hz, 1H), 4.59 - 4.48 (m, 2H), 4.47 - 4.35 (m, 2H), 3.91 (s, 33H), 3.74 (q, J = 8.7 Hz, 2H), 2.25 - 2.01 (m, 3H), 1.33 (s, 3H), 0.61 (s, 3H).

### Example 339: (S)-2-(2,5-difluoro-4-(5-fluoro-6-((5-methoxy-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid

(S)-2-(2, 5-difluoro-4-(5-fluoro-6-((5-methoxy-1,3,4-thiadiazol-2-yl)methoxy)pyri din-2-yl)benzyl)-1-(4,4-dimethyltetrahydrofuran-3-yl)-1H-benzo[d]imidazole-6-carboxylic acid was prepared in a manner as described in Procedure 10 using Intermediate **I-82** and **I-1089.** ES/MS m/z: 625.6 (M+H⁺). 1H NMR (400 MHz, DMSO) δ 12.78 (s, 1H), 8.48 (s, 1H), 7.98 - 7.86 (m, 2H), 7.79 (dd, J = 8.4, 1.5 Hz, 1H), 7.62 (dd, J = 8.2, 3.2 Hz, 2H), 7.47 (dd, J = 11.5, 6.1 Hz, 1H), 5.84 (s, 2H), 5.01 (d, J = 6.6 Hz, 1H), 4.57 - 4.48 (m, 2H), 4.44 (dd, J = 11.1, 6.8 Hz, 1H), 4.38 (d, J = 16.9 Hz, 1H), 4.12 (s, 3H), 3.81 - 3.70 (m, 2H), 1.33 (s, 3H), 0.60 (s, 3H).

### Example 340: (S)-2-(4-(6-((5-(1,1-difluoroethyl)thiazol-2-yl)methoxy)-5-fluoropyridin-2-yl)-2,5-difluorobenzyl)-1-(4,4-dimethyltetrahydrofuran-3-yl)-1H-benzo[d]imidazole-6-carboxylic acid

(S)-2-(4-(6-((5-(1,1-difluoroethyl)thiazol-2-yl)methoxy)-5-fluoropyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 10 using Intermediate **I-82** and **I-1078.** ES/MS m/z: 658.6 (M+H⁺). 1H NMR (400 MHz, DMSO) δ 12.79 (s, 1H), 8.48 (s, 1H), 8.19 (t, J = 1.8 Hz, 1H), 7.96 - 7.76 (m, 3H), 7.62 (dd, J = 8.4, 2.9 Hz, 2H), 7.46 (dd, J = 11.4, 6.2 Hz, 1H), 5.91 (s, 2H), 5.01 (d, J = 6.6 Hz, 1H), 4.57 - 4.48 (m, 2H), 4.48 - 4.33 (m, 2H), 3.81 - 3.70 (m, 2H), 2.18 (s, 1H), 2.13 (s, 2H), 2.08 (s, 1H), 1.33 (s, 3H), 0.60 (s, 3H).

### Example 341: 2-(4-(6-((5-(difluoromethyl)thiazol-2-yl)methoxy)pyridin-2-yl)-2,5-difluorobenzyl)-1-(4,4-dimethyltetrahydrofuran-3-yl)-4-fluoro-1H-benzo[d]imidazole-6-carboxylic acid

2-(4-(6-((5-(difluoromethyl)thiazol-2-yl)methoxy)pyridin-2-yl)-2,5-difluorobenzyl)-1-(4,4-dimethyltetrahydrofuran-3-yl)-4-fluoro-1H-benzo[d]imidazole-6-carboxylic acid was prepared in a manner as described in Procedure 10 using Intermediate **I-108** and **I-1336.** ES/MS m/z: 644.6 (M+H⁺). 1H NMR (400 MHz, DMSO) δ 8.35 (s, 1H), 8.17 (t, J = 2.4 Hz, 1H), 7.99 - 7.84 (m, 2H), 7.61 (dd, J = 7.6, 1.6 Hz, 1H), 7.56 - 7.38 (m, 3H), 7.05 (d, J = 8.2 Hz, 1H), 5.84 (s, 2H), 5.03 (d, J = 6.6 Hz, 1H), 4.59 - 4.48 (m, 2H), 4.47 - 4.35 (m, 2H), 3.75 (q, J = 8.7 Hz, 2H), 1.33 (s, 3H), 0.61 (s, 3H).

### Example 342: (S)-2-(2,5-difluoro-4-(6-((5-methoxy-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid

(S)-2-(2, 5-difluoro-4-(6-((5-methoxy-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 10 using Intermediate **I-82** and **I-1090.** ES/MS m/z: 608.0 (M+H⁺). 1H NMR (400 MHz, DMSO) δ 12.78 (s, 1H), 8.47 (s, 1H), 8.01 - 7.89 (m, 2H), 7.79 (dd, J = 8.4, 1.5 Hz, 1H), 7.65 - 7.57 (m, 2H), 7.46 (dd, J = 11.5, 6.1 Hz, 1H), 6.99 (d, J = 8.2 Hz, 1H), 5.74 (s, 2H), 5.01 (d, J = 6.7 Hz, 1H), 4.57 - 4.48 (m, 2H), 4.48 - 4.34 (m, 2H), 4.10 (s, 3H), 3.82 - 3.70 (m, 2H), 1.33 (s, 3H), 1.36 - 1.22 (m, 1H), 0.60 (s, 3H).

### Example 343: (S)-2-(2,5-difluoro-4-(6-((5-((1-methylcyclopropyl)methoxy)-1,3,4-thiadiazol-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((5-((1-methylcyclopropyl)methoxy)-1,3,4-thiadiazol-2-yl)methoxy)pyridin-2-yl)benzyl)-1-(oxetan-2-ylmethyl)-1H-benzo[d]imidazole-6-carboxylic acid was prepared in a manner as described in Procedure 10 using Intermediate **I-1085** and **I-8.** ES/MS m/z: 634.3 (M+H⁺). 1H NMR (400 MHz, DMSO) δ 12.78 (s, 2H), 8.26 (d, J = 1.6 Hz, 1H), 7.92 (dd, J = 9.5, 6.1 Hz, 2H), 7.79 (dd, J = 8.4, 1.6 Hz, 1H), 7.64 - 7.55 (m, 2H), 7.40 (dd, J = 11.6, 6.1 Hz, 1H), 6.98 (d, J = 8.2 Hz, 1H), 5.50 (s, 2H), 5.07 (tt, J = 7.1, 3.7 Hz, 1H), 4.76 (dd, J = 15.5, 7.0 Hz, 1H), 4.63 (dd, J = 15.5, 2.8 Hz, 1H), 4.58 - 4.41 (m, 3H), 4.36 (dt, J = 9.0, 5.9 Hz, 1H), 3.30 (s, 2H), 2.72 (s, 1H), 2.40 (ddd, J = 11.4, 8.9, 5.6 Hz, 1H), 1.24 (s, 1H).

### Example 344: (S)-2-(4-(6-((5-cyclobutoxy-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyclobutoxy-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10 using Intermediate **I-1084** and **I-8.** ES/MS m/z: 620.2 (M+H⁺). 1H NMR (400 MHz, DMSO) δ 12.78 (s, 1H), 8.26 (d, J = 1.5 Hz, 1H), 7.97 - 7.88 (m, 2H), 7.79 (dd, J = 8.4, 1.6 Hz, 1H), 7.64 - 7.55 (m, 2H), 7.40 (dd, J = 11.6, 6.0 Hz, 1H), 6.98 (d, J = 8.2 Hz, 1H), 5.72 (s, 2H), 5.19 - 5.04 (m, 2H), 4.76 (dd, J = 15.6, 7.1 Hz, 1H), 4.63 (dd, J = 15.5, 2.8 Hz, 1H), 4.58 - 4.41 (m, 3H), 4.36 (dt, J = 9.0, 5.9 Hz, 1H), 3.30 (s, 1H), 2.73 (dt, J = 11.6, 7.7 Hz, 1H), 2.40 (ddt, J = 9.4, 7.3, 5.0 Hz, 3H), 2.22 - 2.07 (m, 2H), 1.78 (q, J = 10.3 Hz, 1H), 1.68 - 1.52 (m, 1H), 1.24 (s, 1H).

### Example 345: (S)-2-(2,5-difluoro-4-(6-((5-methoxy-1,3,4-thiadiazol-2-yl)methoxy)pyridin-2-yl)benzyl)-1-(4,4-dimethyltetrahydrofuran-3-yl)-4-fluoro-1H-benzo[d]imidazole-6-carboxylic acid

(S)-2-(2, 5-difluoro-4-(6-((5-methoxy-1,3,4-thiadiazol-2-yl)methoxy)pyridin-2-yl)benzyl)-1-(4,4-dimethyltetrahydrofuran-3-yl)-4-fluoro-1H-benzo[d]imidazole-6-carboxylic acid was prepared in a manner as described in Procedure 10 using Intermediate **I-108** and **I-1090.** ES/MS m/z: 626.2 (M+H⁺). 1H NMR (400 MHz, DMSO) δ 8.36 (s, 1H), 8.05 - 7.88 (m, 2H), 7.61 (dd, J = 7.6, 1.7 Hz, 1H), 7.50 (ddd, J = 17.4, 11.3, 3.7 Hz, 2H), 6.99 (dd, J = 8.3, 2.1 Hz, 1H), 5.74 (s, 2H), 5.03 (d, J = 6.6 Hz, 1H), 4.60 - 4.49 (m, 2H), 4.48 - 4.37 (m, 2H), 4.10 (s, 2H), 3.75 (q, J = 8.7 Hz, 2H), 1.33 (s, 3H), 0.60 (s, 3H).

### Example 346: (S)-2-(2,5-difluoro-4-(5-fluoro-6-((5-methoxy-1,3,4-thiadiazol-2-yl)methoxy)pyridin-2-yl)benzyl)-1-(4,4-dimethyltetrahydrofuran-3-yl)-4-fluoro-1H-benzo[d]imidazole-6-carboxylic acid

(S)-2-(2, 5-difluoro-4-(5-fluoro-6-((5-methoxy-1,3,4-thiadiazol-2-yl)methoxy)pyri din-2-yl)benzyl)-1-(4,4-dimethyltetrahydrofuran-3-yl)-4-fluoro-1H-benzo[d]imidazole-6-carboxylic acid was prepared in a manner as described in Procedure 10 using Intermediate **1-108** and **I-1089.** ES/MS m/z: 644.0 (M+H⁺). 1H NMR (400 MHz, DMSO) δ 13.11 (s, 1H), 8.36 (s, 1H), 7.99 - 7.86 (m, 2H), 7.63 (dt, J = 7.6, 1.7 Hz, 1H), 7.56 - 7.44 (m, 2H), 5.84 (s, 2H), 5.03 (d, J = 6.6 Hz, 1H), 4.59 - 4.49 (m, 2H), 4.48 - 4.36 (m, 2H), 4.11 (s, 3H), 1.33 (s, 3H), 0.61 (s, 3H). **Example 347: (S)-2-(4-(6-((5-(3-(difluoromethyl)cyclobutoxy)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid**

(S)-2-(4-(6-((5-(3-(difluoromethyl)cyclobutoxy)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10 using Intermediate **I-1080** and **I-8.** ES/MS m/z: 670.0 (M+H⁺). 1H NMR (400 MHz, DMSO) δ 12.77 (s, 1H), 8.26 (d, J = 1.6 Hz, 1H), 7.98 - 7.88 (m, 2H), 7.79 (dd, J = 8.4, 1.6 Hz, 1H), 7.64 - 7.55 (m, 2H), 7.41 (dd, J = 11.6, 6.0 Hz, 1H), 6.98 (d, J = 8.3 Hz, 1H), 6.29 - 5.86 (m, 1H), 5.73 (s, 2H), 5.20 - 5.05 (m, 1H), 4.76 (dd, J = 15.5, 7.0 Hz, 1H), 4.63 (dd, J = 15.5, 2.7 Hz, 1H), 4.58 - 4.41 (m, 3H), 4.36 (dt, J = 8.9, 5.9 Hz, 1H), 2.79 - 2.68 (m, 1H), 2.55 (dd, J = 7.2, 2.4 Hz, 1H), 2.39 (s, 1H), 2.20 - 2.11 (m, 2H).

### Example 348: (S)-2-(4-(6-((5-((1-acetylazetidin-3-yl)oxy)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-((1-acetylazetidin-3-yl)oxy)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10 using Intermediate **I-1081** and **I-8.** ES/MS m/z: 663.2 (M+H⁺). 1H NMR (400 MHz, DMSO) δ 12.78 (s, 1H), 8.27 (s, 1H), 7.99 - 7.89 (m, 2H), 7.80 (dd, J = 8.5, 1.5 Hz, 1H), 7.60 (t, J = 7.5 Hz, 2H), 7.41 (dd, J = 11.5, 5.9 Hz, 1H), 6.99 (d, J = 8.3 Hz, 1H), 5.75 (s, 2H), 5.45 (dt, J = 6.5, 3.0 Hz, 1H), 5.08 (d, J = 7.4 Hz, 1H), 4.76 (dd, J = 15.5, 7.1 Hz, 1H), 4.63 (d, J = 13.7 Hz, 1H), 4.59 - 4.41 (m, 4H), 4.36 (dt, J = 8.8, 5.9 Hz, 1H), 4.23 (t, J = 9.4 Hz, 2H), 3.92 - 3.85 (m, 1H), 2.72 (s, 1H), 2.39 (s, 1H), 1.77 (s, 3H), 1.24 (s, 1H), 0.94 (d, J = 6.7 Hz, 1H).

### Example 349: (S)-2-(4-(6-((2-ethoxy-4-methylthiazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-ethoxy-4-methylthiazol-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10 using Intermediate **I-1093** and **I-8.** ES/MS m/z: 607.2 (M+H⁺). 1H NMR (400 MHz, DMSO) δ 12.79 (s, 1H), 8.26 (d, J = 1.5 Hz, 1H), 8.24 (s, 0H), 7.95 (dd, J = 10.5, 6.4 Hz, 1H), 7.86 (t, J = 7.9 Hz, 1H), 7.79 (ddd, J = 8.4, 4.2, 1.5 Hz, 1H), 7.60 (dd, J = 8.4, 5.8 Hz, 1H), 7.52 (dd, J = 7.6, 1.8 Hz, 1H), 7.40 (dd, J = 11.5, 6.1 Hz, 1H), 7.33 - 7.15 (m, 1H), 6.88 (d, J = 8.3 Hz, 1H), 5.51 (s, 2H), 5.08 (dd, J = 8.4, 5.8 Hz, 2H), 4.74 (td, J = 15.6, 7.1 Hz, 1H), 4.68 - 4.55 (m, 2H), 4.55 - 4.47 (m, 3H), 4.46 - 4.38 (m, 1H), 4.34 (q, J = 7.1 Hz, 3H), 2.72 (dt, J = 16.1, 7.5 Hz, 1H), 2.38 (td, J = 15.1, 6.4 Hz, 1H), 2.26 (s, 3H), 1.31 (t, J = 7.0 Hz, 3H).

### Example 350: (S)-2-(2,5-difluoro-4-(6-((5-(2,2,2-trifluoroethoxy)-1,3,4-thiadiazol-2-yl)methoxy)pyridin-2-yl)benzyl)-1-(oxetan-2-ylmethyl)-1H-benzo [d] imidazole-6-carboxylic acid

(S)-2-(2, 5-difluoro-4-(6-((5-(2,2,2-trifluoroethoxy)-1,3,4-thiadiazol-2-yl)methoxy)pyridin-2-yl)benzyl)-1-(oxetan-2-ylmethyl)-1H-benzo[d]imidazole-6-carboxylic acid was prepared in a manner as described in Procedure 10 using Intermediate **I-1082** and **I-8.** ES/MS m/z: 648.2 (M+H⁺). 1H NMR (400 MHz, DMSO) δ 12.80 (s, 1H), 8.25 (d, J = 1.5 Hz, 1H), 8.00 - 7.89 (m, 2H), 7.79 (dd, J = 8.4, 1.5 Hz, 1H), 7.63 - 7.56 (m, 2H), 7.40 (dd, J = 11.6, 6.0 Hz, 1H), 7.00 (d, J = 8.2 Hz, 1H), 5.78 (s, 2H), 5.24 (q, J = 8.7 Hz, 2H), 5.07 (d, J = 5.2 Hz, 1H), 4.76 (dd, J = 15.6, 7.1 Hz, 1H), 4.62 (dd, J = 15.5, 2.7 Hz, 1H), 4.58 - 4.41 (m, 3H), 4.35 (dt, J = 8.9, 5.9 Hz, 1H), 2.82 - 2.60 (m, 1H), 2.40 (q, J = 9.0 Hz, 1H).

### Example 351: (S)-2-(2,5-difluoro-4-(6-((5-methoxy-1,3,4-thiadiazol-2-yl)methoxy)pyridin-2-yl)benzyl)-4-fluoro-1-(oxetan-2-ylmethyl)-1H-benzo[d]imidazole-6-carboxylic acid

(S)-2-(2,5-difluoro-4-(6-((5-methoxy-1,3,4-thiadiazol-2-yl)methoxy)pyridin-2-yl)benzyl)-4-fluoro-1-(oxetan-2-ylmethyl)-1H-benzo[d]imidazole-6-carboxylic acid was prepared in a manner as described in Procedure 10 using Intermediate **I-1090** and **I-14.** ES/MS m/z: 598.2 (M+H⁺). 1H NMR (400 MHz, DMSO) δ 8.11 (s, 1H), 8.03 - 7.87 (m, 2H), 7.59 (d, J = 7.4 Hz, 1H), 7.50 (d, J = 11.5 Hz, 1H), 7.41 (dd, J = 11.5, 6.0 Hz, 1H), 6.99 (d, J = 8.2 Hz, 1H), 5.74 (s, 2H), 5.07 (d, J = 5.8 Hz, 2H), 4.76 (s, 1H), 4.70 - 4.42 (m, 4H), 4.41 - 4.29 (m, 1H), 4.10 (s, 3H), 2.68 (s, 1H), 2.34 (d, J = 1.9 Hz, 1H).

### Example 352: (S)-2-(4-(6-((5-(2,2-difluoroethoxy)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(2,2-difluoroethoxy)-1,3,4-thiadiazol-2-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 10 using Intermediate **I-1083** and **I-8.** ES/MS m/z: 630.2 (M+H⁺). 1H NMR (400 MHz, DMSO-d6) δ 12.76 (s, 1H), 8.25 (d, J = 1.5 Hz, 1H), 8.00 - 7.88 (m, 2H), 7.78 (dd, J = 8.4, 1.5 Hz, 1H), 7.62 - 7.56 (m, 2H), 7.40 (dd, J = 11.6, 6.0 Hz, 1H), 6.98 (d, J = 8.3 Hz, 1H), 6.42 (tt, J = 53.8, 3.1 Hz, 1H), 5.75 (s, 2H), 5.11 - 5.02 (m, 1H), 4.85 - 4.71 (m, 3H), 4.62 (dd, J = 15.5, 2.7 Hz, 1H), 4.58 - 4.41 (m, 3H), 4.40 - 4.30 (m, 1H), 2.76 - 2.65 (m, 1H), 2.42 - 2.30 (m, 1H).

### Example 353: (S)-2-(4-(6-((4-chloro-2-ethoxythiazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-2-ethoxythiazol-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10 using Intermediate **I-1094** and **I-8.** ES/MS m/z: 627.0 (M+H⁺). 1H NMR (400 MHz, DMSO) δ 12.79 (s, 1H), 8.27 (dd, J = 7.2, 1.5 Hz, 1H), 8.01 - 7.74 (m, 2H), 7.65 - 7.52 (m, 2H), 7.41 (dd, J = 11.6, 6.0 Hz, 1H), 7.34 - 7.14 (m, 1H), 6.92 (d, J = 8.2 Hz, 1H), 5.53 (s, 1H), 5.06 (dqd, J = 13.5, 7.0, 2.6 Hz, 1H), 4.75 (td, J = 15.3, 7.1 Hz, 1H), 4.68 - 4.55 (m, 1H), 4.55 - 4.29 (m, 5H), 2.78 - 2.67 (m, 1H), 2.39 (dq, J = 10.8, 7.5 Hz, 1H), 1.32 (t, J = 7.0 Hz, 2H).

### Example 365: (S)-2-(4-(6-((4-chloro-6-(methylcarbamoyl)pyridin-3-yl)methoxy)pyridin-2-yl)-2,5-difluorobenzyl)-1-(4,4-dimethyltetrahydrofuran-3-yl)-4-fluoro-1H-benzo[d]imidazole-6-carboxylic acid

(S)-2-(4-(6-((4-chloro-6-(methylcarbamoyl)pyridin-3-yl)methoxy)pyridin-2-yl)-2,5-difluorobenzyl)-1-(4,4-dimethyltetrahydrofuran-3-yl)-4-fluoro-1H-benzo[d]imidazole-6-carboxylic acid was prepared in a manner as described in Procedure 10 using Intermediate **I-1273** and **I-108.** ES/MS m/z: 680.2 (M+H⁺). 1HNMR (400 MHz, DMSO-d6) δ 8.85 (d, J = 5.0 Hz, 1H), 8.81 (s, 1H), 8.35 (s, 1H), 8.07 (s, 1H), 7.91 (t, J = 7.9 Hz, 1H), 7.84 (dd, J = 10.4, 6.4 Hz, 1H), 7.61 - 7.54 (m, 1H), 7.54 - 7.49 (m, 1H), 7.47 (dd, J = 11.4, 6.1 Hz, 1H), 7.01 (d, J = 8.3 Hz, 1H), 5.67 (s, 2H), 5.03 (d, J = 6.7 Hz, 1H), 4.58 - 4.48 (m, 2H), 4.46 - 4.34 (m, 2H), 3.74 (q, J = 8.7 Hz, 1H), 2.81 (d, J = 4.8 Hz, 3H), 1.33 (s, 3H), 0.60 (s, 3H).

### Example 366: (S)-2-(4-(6-((4-chloro-6-(((1-methyl-1H-pyrazol-3-yl)methyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((4-chloro-6-(((1-methyl-1H-pyrazol-3-yl)methyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 10 using Intermediate **I-1059** and **I-8.** ES/MS m/z: 714.3 (M+H⁺). 1H NMR (400 MHz, DMSO-d6) δ 9.08 (t, J = 6.1 Hz, 1H), 8.82 (s, 1H), 8.24 (s, 1H), 8.10 (s, 1H), 7.98 - 7.86 (m, 1H), 7.82 - 7.73 (m, 2H), 7.64 - 7.48 (m, 3H), 7.38 (dd, J = 11.5, 6.0 Hz, 1H), 7.00 (d, J = 8.2 Hz, 1H), 6.11 (d, J = 2.2 Hz, 1H), 5.67 (s, 2H), 5.06 (d, J = 4.6 Hz, 1H), 4.74 (dd, J = 15.6, 6.9 Hz, 1H), 4.67 - 4.53 (m, 1H), 4.53 - 4.45 (m, 2H), 4.42 (d, J = 6.1 Hz, 2H), 4.35 (dd, J = 9.1, 5.9 Hz, 1H), 3.76 (s, 3H), 2.72 (d, J = 10.4 Hz, 1H), 2.44 - 2.32 (m, 1H), 1.23 (s, 2H).

### Example 376: (S)-1-(oxetan-2-ylmethyl)-2-(2,3,6-trifluoro-4-(6-((4-(trifluoromethyl)thiazol-2-yl)methoxy)pyridin-2-yl)benzyl)-1H-benzo[d]imidazole-6-carboxylic acid

(S)-1-(oxetan-2-ylmethyl)-2-(2,3,6-trifluoro-4-(6-((4-(trifluoromethyl)thiazol-2-yl)methoxy)pyridin-2-yl)benzyl)-1H-benzo[d]imidazole-6-carboxylic acid was prepared in a manner as described in Procedure 10 using Intermediate **I-1097** and **I-1344.** ES/MS m/z: 653.2 (M+H⁺). 1H NMR (400 MHz, DMSO) δ 8.51 (d, J = 1.2 Hz, 1H), 7.99 (t, J = 7.9 Hz, 1H), 7.77 (d, J = 1.9 Hz, 1H), 7.65 (d, J = 7.2 Hz, 1H), 7.51 (d, J = 25.6 Hz, 1H), 7.10 (d, J = 8.3 Hz, 1H), 5.85 (s, 2H), 5.13 (s, 1H), 4.88 - 4.23 (m, 5H), 2.74 (s, 1H), 2.38 (s, 1H)

### Example 377: (S)-4-fluoro-1-(oxetan-2-ylmethyl)-2-(2,3,6-trifluoro-4-(2-((4-(trifluoromethyl)thiazol-2-yl)methoxy)pyrimidin-4-yl)benzyl)-1H-benzo[d]imidazole-6-carboxylic acid

(S)-4-fluoro-1-(oxetan-2-ylmethyl)-2-(2,3,6-trifluoro-4-(2-((4-(trifluoromethyl)thiazol-2-yl)methoxy)pyrimidin-4-yl)benzyl)-1H-benzo[d]imidazole-6-carboxylic acid was prepared in a manner as described in Procedure 10 using Intermediate **I-1245** and **I-1235.** ES/MS m/z: 654.1 (M+H⁺). 1H NMR (400 MHz, DMSO) δ 8.88 (d, J = 5.2 Hz, 1H), 8.50 (s, 1H), 8.08 (s, 1H), 7.86 (d, J = 8.5 Hz, 1H), 7.76 (d, J = 5.0 Hz, 1H), 7.49 (d, J = 11.6 Hz, 1H), 5.91 (s, 2H), 5.13 (d, J = 6.0 Hz, 1H), 4.99 - 4.25 (m, 5H), 2.71 (d, J = 27.8 Hz, 1H), 2.42 - 2.25 (m, 1H).

### Example 378: (S)-2-(4-(6-((5-(1,1-difluoroethyl)thiazol-2-yl)methoxy)-5-fluoropyridin-2-yl)-2,3,6-trifluorobenzyl)-4-fluoro-1-(oxetan-2-ylmethyl)-1H-benzo[d]imidazole-6-carboxylic acid

(S)-2-(4-(6-((5-(1,1-difluoroethyl)thiazol-2-yl)methoxy)-5-fluoropyridin-2-yl)-2,3,6-trifluorobenzyl)-4-fluoro-1-(oxetan-2-ylmethyl)-1H-benzo[d]imidazole-6-carboxylic acid was prepared in a manner as described in Procedure 10 using Intermediate **I-1078** and **I-1235.** ES/MS m/z: 667.1 (M+H⁺). 1H NMR (400 MHz, DMSO) δ 8.19 (s, 1H), 8.08 (s, 1H), 8.02 - 7.91 (m, 1H), 7.83 - 7.60 (m, 1H), 7.49 (d, J = 10.6 Hz, 1H), 5.92 (s, 2H), 5.12 (s, 1H), 4.95 - 4.21 (m, 5H), 2.55 (s, 1H), 2.33 (s, 1H), 2.13 (t, J = 18.7 Hz, 3H).

### Example 398: 2-(4-(6-((4-chloro-6-(methylcarbamoyl)pyridin-3-yl)methoxy)pyridin-2-yl)-2,3,6-trifluorobenzyl)-4-fluoro-1-((1-(fluoromethyl)cyclopropyl)methyl)-1H-benzo[d]imidazole-6-carboxylic acid

2-(4-(6-((4-chloro-6-(methylcarbamoyl)pyridin-3-yl)methoxy)pyridin-2-yl)-2,3,6-trifluorobenzyl)-4-fluoro-1-((1-(fluoromethyl)cyclopropyl)methyl)-1H-benzo[d]imidazole-6-carboxylic acid was prepared in a manner as described in Procedure 10 using Intermediates **I-1273** and **I-1201.** ES/MS m/z: 686.8 (M+H⁺). 1H NMR (400 MHz, Methanol-d4) δ 8.77 (s, 1H), 8.19 (d, J = 1.2 Hz, 1H), 8.14 (s, 1H), 7.88 (t, J = 7.9 Hz, 1H), 7.75 - 7.50 (m, 3H), 7.00 (d, J = 8.3 Hz, 1H), 5.72 (s, 2H), 4.63 (s, 2H), 4.56 (s, 2H), 4.26 (s, 1H), 4.14 (s, 1H), 3.79 - 3.62 (m, 2H), 2.96 (s, 3H), 1.04 - 0.92 (m, 2H), 0.86 (d, J = 4.8 Hz, 2H).

### Example 399: (S)-2-(4-(6-((4-chloro-6-(methylcarbamoyl)pyridin-3-yl)methoxy)pyridin-2-yl)-2-fluorobenzyl)-1-(4,4-dimethyltetrahydrofuran-3-yl)-1H-benzo[d]imidazole-6-carboxylic acid

(S)-2-(4-(6-((4-chloro-6-(methylcarbamoyl)pyridin-3-yl)methoxy)pyridin-2-yl)-2-fluorobenzyl)-1-(4,4-dimethyltetrahydrofuran-3-yl)-1H-benzo[d]imidazole-6-carboxylic acid was prepared in a manner as described in Procedure 10 using Intermediates **I-1273** and **I-1229.** ES/MS m/z: 645.5 (M+H⁺). 1HNMR (400 MHz, Methanol-d4) δ 8.88 (s, 1H), 8.75 (s, 1H), 8.32 - 8.10 (m, 2H), 8.04 - 7.91 (m, 2H), 7.90 - 7.71 (m, 2H), 7.59 (d, J = 7.5 Hz, 1H), 7.52 (t, J = 8.0 Hz, 1H), 6.95 (d, J = 8.2 Hz, 1H), 5.74 (s, 2H), 5.51 (s, 1H), 5.10 (d, J = 6.5 Hz, 1H), 4.78 - 4.58 (m, 3H), 4.49 (dd, J = 11.6, 6.7 Hz, 1H), 3.99 (d, J = 8.9 Hz, 1H), 3.82 (d, J = 8.9 Hz, 1H), 2.96 (s, 3H), 1.35 (s, 3H), 0.71 (s, 3H).

### Example 400: (S)-1-(4,4-dimethyltetrahydrofuran-3-yl)-2-(3-fluoro-4-(6-((5-methoxy-1,3,4-thiadiazol-2-yl)methoxy)pyridin-2-yl)benzyl)-1H-benzo[d]imidazole-6-carboxylic acid

(S)-1-(4,4-dimethyltetrahydrofuran-3-yl)-2-(3-fluoro-4-(6-((5-methoxy-1,3,4-thiadiazol-2-yl)methoxy)pyridin-2-yl)benzyl)-1H-benzo[d]imidazole-6-carboxylic acid was prepared in a manner as described in Procedure 10 using Intermediates **I-1090** and **I-1326.** ES/MS m/z: 590.7 (M+H⁺). 1H NMR (400 MHz, Methanol-d4) δ 8.87 (s, 1H), 8.29 - 8.12 (m, 2H), 7.95 - 7.75 (m, 2H), 7.59 (dd, J = 7.4, 1.7 Hz, 1H), 7.46 - 7.25 (m, 2H), 6.90 (d, J = 8.2 Hz, 1H), 5.70 (s, 2H), 5.07 (d, J = 6.6 Hz, 1H), 4.80 - 4.55 (m, 3H), 4.46 (dd, J = 11.5, 6.8 Hz, 1H), 4.14 (s, 3H), 3.99 (d, J = 8.9 Hz, 1H), 3.80 (d, J = 8.9 Hz, 1H), 3.77 - 3.68 (m, 1H), 1.31 (s, 3H), 0.66 (s, 3H).

### Example 406: (S)-2-(4-(6-((5-(difluoromethyl)thiazol-2-yl)methoxy)-5-fluoropyridin-2-yl)-2,5-difluorobenzyl)-1-(oxetan-2-ylmethyl)-1H-benzo[d]imidazole-6-carboxylic acid

(S)-2-(4-(6-((5-(difluoromethyl)thiazol-2-yl)methoxy)-5-fluoropyridin-2-yl)-2,5-difluorobenzyl)-1-(oxetan-2-ylmethyl)-1H-benzo[d]imidazole-6-carboxylic acid was prepared in a manner as described in Procedure 10 using Intermediates **I-8** and **I-1314.** ES/MS m/z: 617.2 (M+H⁺). 1H NMR (400 MHz, Acetonitrile-d3) δ 8.22 (s, 1H), 8.02 (t, J = 2.3 Hz, 1H), 7.87 (dd, J = 8.4, 1.5 Hz, 1H), 7.80 (dd, J = 10.7, 6.5 Hz, 1H), 7.68 - 7.56 (m, 3H), 7.19 (dd, J = 11.1, 5.7 Hz, 1H), 7.07 (t, J = 54.8 Hz, 1H), 5.86 (s, 2H), 5.16 - 5.05 (m, 1H), 4.58 (tt, J = 14.4, 7.0 Hz, 2H), 4.49 (d, J = 2.8 Hz, 1H), 4.44 (d, J = 11.3 Hz, 2H), 4.36 (dt, J = 9.0, 5.7 Hz, 1H), 2.78 - 2.64 (m, 1H), 2.47 - 2.34 (m, 1H).

### Example 407: (S)-2-(4-(6-((5-(difluoromethyl)thiazol-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid

(S)-2-(4-(6-((5-(difluoromethyl)thiazol-2-yl)methoxy)-5-fluoropyridin-2-yl)-2,5-difluorobenzyl)-4-fluoro-1-(oxetan-2-ylmethyl)-1H-benzo[d]imidazole-6-carboxylic acid was prepared in a manner as described in Procedure 10 using Intermediates **I-108** and **I-1314.** ES/MS m/z: 635.1 (M+H⁺). 1H NMR (400 MHz, Acetonitrile-d3) δ 8.11 (d, J = 1.3 Hz, 1H), 8.05 (t, J = 2.4 Hz, 1H), 7.84 (dd, J = 10.7, 6.5 Hz, 1H), 7.70 - 7.55 (m, 3H), 7.29 - 7.21 (m, 1H), 7.10 (t, J = 54.9 Hz, 1H), 5.89 (s, 2H), 5.14 (ddd, J = 14.5, 7.0, 2.6 Hz, 1H), 4.70 - 4.51 (m, 3H), 4.48 (d, J = 10.6 Hz, 2H), 4.40 (dt, J = 9.2, 5.8 Hz, 1H), 2.82 - 2.72 (m, 1H), 2.44 (ddd, J = 16.2, 9.1, 4.0 Hz, 1H).

### Example 408: (S)-2-(2,5-difluoro-4-(2-((5-(trifluoromethyl)thiazol-2-yl)methoxy)pyrimidin-4-yl)benzyl)-4-fluoro-1-(oxetan-2-ylmethyl)-1H-benzo[d]imidazole-6-carboxylic acid

(S)-2-(2, 5-difluoro-4-(2-((5-(trifluoromethyl)thiazol-2-yl)methoxy)pyrimidin-4-yl)benzyl)-4-fluoro-1-(oxetan-2-ylmethyl)-1H-benzo[d]imidazole-6-carboxylic acid was prepared in a manner as described in Procedure 10 using Intermediates **I-108** and **I-1317.** ES/MS m/z: 636.2 (M+H⁺). 1HNMR (400 MHz, Acetonitrile-d3) δ 8.72 (d, J = 5.2 Hz, 1H), 8.24 (s, 1H), 8.12 (d, J = 1.2 Hz, 1H), 7.99 (dd, J = 10.4, 6.3 Hz, 1H), 7.69 (d, J = 5.3 Hz, 1H), 7.60 (d, J = 11.4 Hz, 1H), 7.31 (dd, J = 11.6, 5.9 Hz, 1H), 5.85 (s, 2H), 5.14 (d, J = 7.1 Hz, 2H), 4.74 - 4.56 (m, 3H), 4.56 - 4.48 (m, 2H), 4.45 - 4.36 (m, 1H), 2.82 - 2.71 (m, 1H), 2.47 - 2.39 (m, 1H).

### Example 409: 2-(2,5-difluoro-4-(5-fluoro-6-((5-(trifluoromethyl)thiazol-2-yl)methoxy)pyridin-2-yl)benzyl)-1-((3S,4R)-4-methyltetrahydrofuran-3-yl)-1H-benzo[d]imidazole-6-carboxylic acid

2-(2,5-difluoro-4-(5-fluoro-6-((5-(trifluoromethyl)thiazol-2-yl)methoxy)pyridin-2-yl)benzyl)-1-((3S,4R)-4-methyltetrahydrofuran-3-yl)-1H-benzo[d]imidazole-6-carboxylic acid was prepared in a manner as described in Procedure 10 using Intermediates **I-1292** and **I-1315**. ES/MS m/z: 649.2 (M+H⁺). 1H NMR (400 MHz, Acetonitrile-d3) δ 8.57 (s, 1H), 8.20 (d, J = 1.4 Hz, 1H), 7.86 (dd, J = 8.5, 1.5 Hz, 1H), 7.80 (dd, J = 10.8, 6.5 Hz, 1H), 7.69 - 7.56 (m, 3H), 7.21 (dd, J = 11.6, 6.1 Hz, 1H), 5.88 (s, 2H), 5.24 (t, J = 7.2 Hz, 1H), 4.55 (dd, J = 10.9, 1.4 Hz, 1H), 4.37 (s, 2H), 4.23 (dd, J = 11.0, 6.7 Hz, 1H), 4.13 (t, J = 8.5 Hz, 1H), 3.67 (t, J = 8.4 Hz, 1H), 2.88 (hept, J = 7.1 Hz, 1H), 0.57 (d, J = 7.0 Hz, 3H). Example 410: 2-(2,5-difluoro-4-(5-fluoro-6-((5-methoxy-1,3,4-thiadiazol-2-yl)methoxy)pyridin-2-yl)benzyl)-4-fluoro-1-((1-(fluoromethyl)cyclopropyl)methyl)-1H-benzo[d]imidazole-6-carboxylic acid

### Example 410: 2-(2,5-difluoro-4-(5-fluoro-6-((5-methoxy-1,3,4-thiadiazol-2-yl)methoxy)pyridin-2-yl)benzyl)-4-fluoro-1-((1-(fluoromethyl)cyclopropyl)methyl)-1H-benzo[d]imidazole-6-carboxylic acid

2-(2,5-difluoro-4-(5-fluoro-6-((5-methoxy-1,3,4-thiadiazol-2-yl)methoxy)pyridin-2-yl)benzyl)-4-fluoro-1-((1-(fluoromethyl)cyclopropyl)methyl)-1H-benzo[d]imidazole-6-carboxylic acid was prepared in a manner as described in Procedure 10 using Intermediates **I-1190** and **I-1089.** ES/MS m/z: 632.2 (M+H⁺). 1H NMR (400 MHz, Acetonitrile-d3) δ 8.09 (d, J = 1.2 Hz, 1H), 7.89 (dd, J = 10.8, 6.5 Hz, 1H), 7.66 - 7.53 (m, 3H), 7.23 (dd, J = 11.6, 6.1 Hz, 1H), 5.76 (s, 2H), 4.44 (s, 2H), 4.39 (s, 2H), 4.20 (s, 1H), 4.12 (s, 3H), 4.08 (s, 1H), 0.85 - 0.79 (m, 2H), 0.79 - 0.72 (m, 2H).

### Example 411: 2-(2,5-difluoro-4-(6-((5-methoxy-1,3,4-thiadiazol-2-yl)methoxy)pyridin-2-yl)benzyl)-4-fluoro-1-((1-(fluoromethyl)cyclopropyl)methyl)-1H-benzo [d] imidazole-6-carboxylic acid

2-(2,5-difluoro-4-(6-((5-methoxy-1,3,4-thiadiazol-2-yl)methoxy)pyridin-2-yl)benzyl)-4-fluoro-1-((1-(fluoromethyl)cyclopropyl)methyl)-1H-benzo[d]imidazole-6-carboxylic acid was prepared in a manner as described in Procedure 10, using Intermediates **I-1190** and **I-1090**. ES/MS m/z: 614.2 (M+H⁺). 1H NMR (400 MHz, Methanol-d4) δ 8.11 (d, J = 1.2 Hz, 1H), 7.90 (dd, J = 10.7, 6.4 Hz, 1H), 7.81 (t, J = 7.9 Hz, 1H), 7.66 - 7.56 (m, 2H), 7.15 (dd, J = 11.5, 6.1 Hz, 1H), 6.88 (d, J = 8.2 Hz, 1H), 5.70 (s, 2H), 4.50 (s, 2H), 4.49 (s, 2H), 4.20 (s, 1H), 4.13 (s, 3H), 4.08 (s, 1H), 0.89 - 0.84 (m, 2H), 0.77 (q, J = 4.8 Hz, 2H).

### Example 412: 2-(2,5-difluoro-4-(6-((5-(trifluoromethyl)thiazol-2-yl)methoxy)pyridin-2-yl)benzyl)-4-fluoro-1-((1-(fluoromethyl)cyclopropyl)methyl)-1H-benzo [d] imidazole-6-carboxylic acid

2-(2,5-difluoro-4-(6-((5-(trifluoromethyl)thiazol-2-yl)methoxy)pyridin-2-yl)benzyl)-4-fluoro-1-((1-(fluoromethyl)cyclopropyl)methyl)-1H-benzo[d]imidazole-6-carboxylic acid was prepared in a manner as described in Procedure 10 using Intermediates **I-1190** and **I-107**. ES/MS m/z: 651.1 (M+H⁺). 1H NMR (400 MHz, Acetonitrile-d3) δ 8.18 (d, J = 1.3 Hz, 1H), 8.09 (d, J = 1.3 Hz, 1H), 7.88 - 7.79 (m, 2H), 7.64 - 7.52 (m, 2H), 7.22 (dd, J = 11.6, 6.1 Hz, 1H), 6.95 (dd, J = 8.3, 0.7 Hz, 1H), 5.83 - 5.77 (m, 2H), 4.44 (s, 2H), 4.39 (s, 2H), 4.20 (s, 1H), 4.08 (s, 1H), 0.85 - 0.79 (m, 2H), 0.78 - 0.73 (m, 2H).

### Example 413: 2-(4-(6-((5-(difluoromethyl)thiazol-2-yl)methoxy)pyridin-2-yl)-2,5-difluorobenzyl)-4-fluoro-1-((1-(fluoromethyl)cyclopropyl)methyl)-1H-benzo [d] imidazole-6-carboxylic acid

2-(4-(6-((5-(difluoromethyl)thiazol-2-yl)methoxy)pyridin-2-yl)-2,5-difluorobenzyl)-4-fluoro-1-((1-(fluoromethyl)cyclopropyl)methyl)-1H-benzo[d]imidazole-6-carboxylic acid was prepared in a manner as described in Procedure 10 using Intermediates **I-1190** and **I-1316**. ES/MS m/z: 663.0 (M+H⁺). 1HNMR (400 MHz, Acetonitrile-d3) δ 8.11 (d, J = 1.3 Hz, 1H), 8.03 (t, J = 2.4 Hz, 1H), 7.92 - 7.83 (m, 2H), 7.65 - 7.57 (m, 2H), 7.29 - 6.90 (m, 3H), 5.82 (s, 2H), 4.47 (s, 2H), 4.42 (s, 2H), 4.23 (s, 1H), 4.11 (s, 1H), 0.85 (d, J = 5.0 Hz, 2H), 0.80 - 0.74 (m, 2H).

### Example 414: (S)-2-(2,5-difluoro-4-(6-((5-(trifluoromethyl)thiazol-2-yl)methoxy)pyridin-2-yl)benzyl)-1-(4,4-dimethyltetrahydrofuran-3-yl)-1H-benzo[d]imidazole-6-carboxylic acid

(S)-2-(2,5-difluoro-4-(6-((5-(trifluoromethyl)thiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 10 using Intermediates **I-82** and **I-107**. ES/MS m/z: 645.2 (M+H⁺). 1HNMR (400 MHz, Acetonitrile-d3) δ 8.55 (s, 1H), 8.18 (d, J = 1.4 Hz, 1H), 7.90 - 7.79 (m, 3H), 7.59 (d, J = 8.3 Hz, 2H), 7.23 (dd, J = 11.6, 6.1 Hz, 1H), 6.94 (d, J= 8.3 Hz, 1H), 5.81 (s, 2H), 4.84 (d, J = 6.8 Hz, 1H), 4.56 (dd, J = 11.2, 1.6 Hz, 1H), 4.42 (dd, J = 11.1, 6.9 Hz, 1H), 4.36 (d, J = 5.9 Hz, 2H), 3.85 (d, J = 8.7 Hz, 1H), 3.74 (d, J = 8.7 Hz, 1H), 1.34 (s, 3H), 0.65 (s, 3H).

### Example 415: (S)-2-(2,5-difluoro-4-(5-fluoro-6-((5-(trifluoromethyl)thiazol-2-yl)methoxy)pyridin-2-yl)benzyl)-1-(4,4-dimethyltetrahydrofuran-3-yl)-1H-benzo[d]imidazole-6-carboxylic acid

(S)-2-(2,5-difluoro-4-(5-fluoro-6-((5-(trifluoromethyl)thiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 10 using Intermediates **I-82** and **I-1315**. ES/MS m/z: 663.2 (M+H⁺). 1H NMR (400 MHz, Acetonitrile-d3) δ 8.55 (s, 1H), 8.21 (d, J = 1.4 Hz, 1H), 7.86 (dd, J = 8.5, 1.6 Hz, 1H), 7.81 (dd, J = 10.7, 6.5 Hz, 1H), 7.69 - 7.56 (m, 3H), 7.23 (dd, J = 11.6, 6.1 Hz, 1H), 5.88 (s, 2H), 4.84 (d, J = 6.6 Hz, 1H), 4.57 (dd, J = 11.2, 1.6 Hz, 1H), 4.43 (dd, J = 11.3, 7.0 Hz, 1H), 4.35 (d, J = 6.3 Hz, 2H), 3.86 (d, J = 8.7 Hz, 1H), 3.75 (d, J = 8.7 Hz, 1H), 1.34 (s, 3H), 0.65 (s, 3H).

### Example 416: (S)-2-(4-(6-((5-(difluoromethyl)thiazol-2-yl)methoxy)-5-fluoropyridin-2-yl)-2,5-difluorobenzyl)-1-(4,4-dimethyltetrahydrofuran-3-yl)-1H-benzo [d] imidazole-6-carboxylic acid

(S)-2-(4-(6-((5-(difluoromethyl)thiazol-2-yl)methoxy)-5-fluoropyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 10 using Intermediates **I**-**82** and **I-1314**. ES/MS m/z: 645.2 (M+H⁺). 1H NMR (400 MHz, Acetonitrile-d3) δ 8.55 (s, 1H), 8.02 (t, J = 2.4 Hz, 1H), 7.86 (dd, J = 8.5, 1.5 Hz, 1H), 7.82 (dd, J = 10.7, 6.5 Hz, 1H), 7.68 - 7.54 (m, 3H), 7.22 (dd, J = 12.0, 5.7 Hz, 1H), 7.08 (t, J = 54.9 Hz, 1H), 5.86 (s, 2H), 4.84 (d, J = 6.7 Hz, 1H), 4.57 (dd, J = 11.2, 1.6 Hz, 1H), 4.43 (dd, J = 11.3, 7.0 Hz, 1H), 4.35 (d, J = 6.2 Hz, 2H), 3.86 (d, J = 8.7 Hz, 1H), 3.75 (d, J = 8.7 Hz, 1H), 1.34 (s, 3H), 0.65 (s, 3H).

### Example 417: 2-(4-(6-((4-chloro-6-(methylcarbamoyl)pyridin-3-yl)methoxy)pyridin-2-yl)-2,5-difluorobenzyl)-4-fluoro-1-((1-(fluoromethyl)cyclopropyl)methyl)-1H-benzo[d]imidazole-6-carboxylic acid

2-(4-(6-((4-chloro-6-(methylcarbamoyl)pyridin-3-yl)methoxy)pyridin-2-yl)-2,5-difluorobenzyl)-4-fluoro-1-((1-(fluoromethyl)cyclopropyl)methyl)-1H-benzo[d]imidazole-6-carboxylic acid was prepared in a manner as described in Procedure 10 using Intermediates **I-1190** and **I-1273**. ES/MS m/z: 668.2 (M+H⁺). 1HNMR (400 MHz, DMSO-d6) δ 13.10 (s, 1H), 8.86 (q, J = 4.8 Hz, 1H), 8.81 (s, 1H), 8.13 (d, J = 1.3 Hz, 1H), 8.07 (s, 1H), 7.94 - 7.87 (m, 1H), 7.83 (dd, J = 10.4, 6.4 Hz, 1H), 7.55 (dd, J = 7.5, 1.7 Hz, 1H), 7.51 (dd, J = 11.3, 1.2 Hz, 1H), 7.43 (dd, J = 11.5, 6.1 Hz, 1H), 7.00 (d, J = 8.2 Hz, 1H), 5.66 (s, 2H), 4.57 (s, 2H), 4.46 (s, 2H), 4.24 (s, 1H), 4.11 (s, 1H), 2.81 (d, J = 4.9 Hz, 3H), 0.90 - 0.81 (m, 2H), 0.76 - 0.68 (m, 2H).

### Example 204 and 205: 2-[[4-[6-[(5-carbamoyl-4-fluoro-2-thienyl)methoxy]-2-pyridyl]-2,5-difluoro-phenyl]methyl]-3-[[(2S)-oxetan-2-yl]methyl]benzimidazole-5-carboxylic acid (Example 204) & 2-[[4-[6-[(5-cyano-4-fluoro-2-thienyl)methoxy]-2-pyridyl]-2,5-difluorophenyl]methyl]-3-[[(2S)-oxetan-2-yl]methyl]benzimidazole-5-carboxylic acid

### Procedure 11:

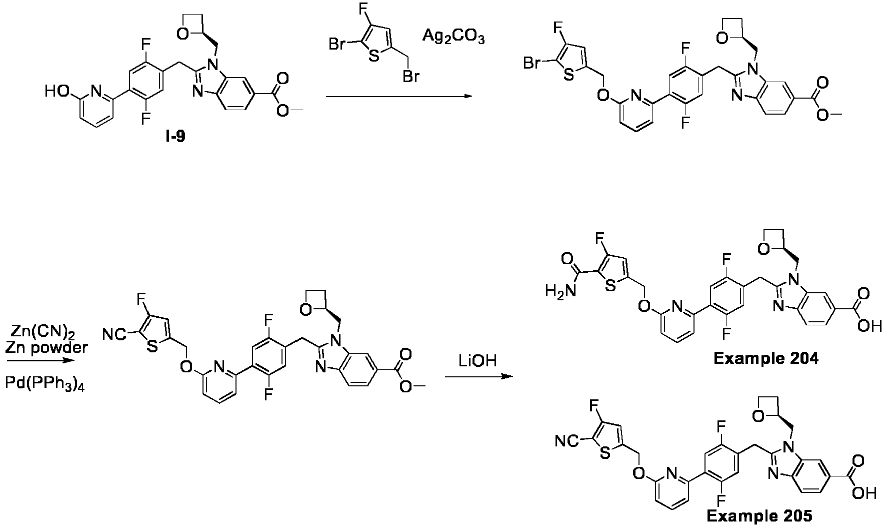

**Methyl 2-[[4-[6- [(5-bromo-4-fluoro-2-thienyl)methoxy] -2-pyridyl] -2,5-difluorophenyl]methyl]-3-[[(2S)-oxetan-2-yl]methyl]benzimidazole-5-carboxylate:** To a solution of methyl (S)-2-(2,5-difluoro-4-(6-hydroxypyridin-2-yl)benzyl)-1-(oxetan-2-ylmethyl)-1H-benzo[d]imidazole-6-carboxylate (150 mg, 0.32 mmol) in toluene (5 mL) was added 2-bromo-5-(bromomethyl)-3-fluoro-thiophene (106 mg, 0.39 mmol) and silver carbonate (270 mg, 0.97 mmol) and the resultant mixture stirred at 90 °C for 2 hours. Upon completion the reaction mixture was filtered through celite, the filter washed with EtOAc (10 mL), and the filtrate subsequently concentrated. The crude residue was purified by silica gel chromatography (eluent: EtOAc/hexanes) to give desired product. ES/MS: 660.0 (M+H⁺).

**Methyl 2-[[4-[6- [(5-cyano-4-fluoro-2-thienyl)methoxy] -2-pyridyl] -2,5-difluorophenyl]methyl]-3-[[(2S)-oxetan-2-yl]methyl]benzimidazole-5-carboxylate:** Methyl 2-[[4-[6-[(5-bromo-4-fluoro-2-thienyl)methoxy]-2-pyridyl]-2,5-difluoro-phenyl]methyl]-3-[[(2S)-oxetan-2-yl]methyl]benzimidazole-5-carboxylate (60 mg, 0.09 mmol), zinc cyanide (16 mg, 0.14 mmol), zinc powder (0.3 mg, 0.005 mmol), and Pd(PPh₃)₄ (42 mg, 0.037 mmol) in DMF (1.5 mL) was degassed by bubbling argon for 1 min, sealed and heated to 100 °C for 20 hours. Upon completion the mixture was poured into water (5 mL) and extracted with EtOAc (2 x 5 mL). The organic layers were combined, washed with brine (5 mL), dried over MgSO₄, filtered, concentrated, and purified by flash chromatography (Eluent: EtOAc/hexane) to give the desired product. ES/MS: 605.1 (M+1).

**2-[[4-[6-[(5-carbamoyl-4-fluoro-2-thienyl)methoxy]-2-pyridyl]-2,5-difluorophenyl]methyl]-3-[[(2S)-oxetan-2-yl]methyl]benzimidazole-5-carboxylic acid_(Example 204) and 2-[[4-[6-[(5-cyano-4-fluoro-2-thienyl)methoxy]-2-pyridyl]-2,5-difluorophenyl]methyl]-3-[[(2S)-oxetan-2-yl]methyl]benzimidazole-5-carboxylic acid (Example 205):** Methyl 2-[[4-[6-[(5-cyano-4-fluoro-2-thienyl)methoxy]-2-pyridyl]-2,5-difluorophenyl]methyl]-3-[[(2S)-oxetan-2-yl]methyl]benzimidazole-5-carboxylate (7.0 mg, 0.012 mmol) was taken up in acetonitrile (0.4 mL) and aqueous lithium hydroxide (0.3 M, 0.12 mL, 0.035 mmol) was added. The mixture was heated to 100 °C for 4 minutes then cooled to r.t. and diluted with 5% aqueous citric acid to a pH ~5. The mixture was extracted with EtOAc (2 x 5 mL) and the combined organic extracts dried over MgSO₄ and concentrated in vacuo. The material was purified by RP-HPLC (eluent: MeCN/water gradient with 0.1% TFA), which was then diluted with EtOAc (50 mL) and washed with water (5 x 20 mL). The combined organic extracts were washed with brine (15 mL), dried over MgSO₄, filtered, and concentrated to yield the products.

**2-[[4-[6-[(5-carbamoyl-4-fluoro-2-thienyl)methoxy]-2-pyridyl]-2,5-difluorophenyl]methyl]-3-[[(2S)-oxetan-2-yl]methyl]benzimidazole-5-carboxylic acid_(Example 204):** ES/MS: 609.0 (M+H⁺). 1H NMR (400 MHz, DMSO-d6) δ 8.25 (d, J = 1.5 Hz, 1H), 7.97 - 7.87 (m, 2H), 7.83 - 7.76 (m, 1H), 7.67 (s, 1H), 7.63 - 7.53 (m, 2H), 7.40 (dd, J = 11.6, 6.0 Hz, 1H), 7.29 (s, 1H), 7.24 (s, 1H), 6.96 (d, J = 8.3 Hz, 1H), 5.63 (s, 2H), 5.07 (d, J = 6.9 Hz, 1H), 4.75 (dd, J = 15.6, 7.0 Hz, 1H), 4.62 (d, J = 14.3 Hz, 1H), 4.58 - 4.41 (m, 3H), 4.36 (dt, J = 8.9, 5.9 Hz, 1H), 2.78 - 2.69 (m, 1H), 2.41 (d, J = 9.4 Hz, 1H).

**2-[[4-[6-[(5-cyano-4-fluoro-2-thienyl)methoxy]-2-pyridyl]-2,5-difluorophenyl]methyl]-3-[[(2S)-oxetan-2-yl]methyl]benzimidazole-5-carboxylic acid (Example 205):** ES/MS: 591.1 (M+H⁺). 1HNMR (400 MHz, Methanol-d4) δ 8.31 (d, J = 1.4 Hz, 1H), 7.99 (dd, J = 8.5, 1.5 Hz, 1H), 7.91 (dd, J = 10.7, 6.4 Hz, 1H), 7.84 (t, J = 7.9 Hz, 1H), 7.67 (d, J = 8.5 Hz, 1H), 7.60 (dd, J = 7.6, 1.6 Hz, 1H), 7.25 - 7.16 (m, 2H), 6.91 (d, J = 8.4 Hz, 1H), 5.69 (s, 2H), 5.20 (qd, J = 7.2, 2.7 Hz, 1H), 4.74 (dd, J = 15.7, 6.9 Hz, 1H), 4.69 - 4.56 (m, 3H), 4.51 (d, J = 16.7 Hz, 1H), 4.44 (dt, J = 9.2, 6.0 Hz, 1H), 2.88 - 2.71 (m, 1H), 2.56 - 2.43 (m, 1H).

### Example 206: (S)-2-(4-(6-((5-carbamoyl-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-carbamoyl-1-methyl-1H-pyrazol-3-yl)methoxy)pyridin-2-yl)-2, 5-difluorobenzyl)-1-(oxetan-2-ylmethyl)-1H-benzo[d]imidazole-6-carboxylic acid was prepared in a manner as described in Procedure 11. 1H NMR (400 MHz, DMSO-d6) δ 8.26 (d, J = 1.5 Hz, 1H), 7.99 - 7.90 (m, 2H), 7.86 (t, J = 7.9 Hz, 1H), 7.79 (dd, J = 8.4, 1.5 Hz, 1H), 7.61 (d, J = 8.4 Hz, 1H), 7.51 (dd, J = 7.5, 1.7 Hz, 1H), 7.48 (s, 1H), 7.38 (dd, J = 11.5, 6.1 Hz, 1H), 6.93 (s, 1H), 6.89 (d, J = 8.3 Hz, 1H), 5.39 (s, 2H), 5.08 (qd, J = 7.0, 2.7 Hz, 1H), 4.76 (dd, J = 15.6, 7.0 Hz, 1H), 4.63 (dd, J = 15.5, 2.8 Hz, 1H), 4.59 - 4.40 (m, 3H), 4.36 (dt, J = 9.0, 5.9 Hz, 1H), 4.05 (s, 3H), 2.72 (dtd, J = 11.1, 8.2, 6.4 Hz, 1H), 2.39 (ddt, J = 11.2, 8.9, 7.0 Hz, 1H).

### Example 207: (S)-2-(4-(6-((5-cyano-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-cyano-1-methyl-1H-pyrazol-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 11. 1H NMR (400 MHz, DMSO-d6) δ 8.25 (d, J = 1.5 Hz, 1H), 7.95 - 7.83 (m, 2H), 7.79 (dd, J = 8.4, 1.6 Hz, 1H), 7.60 (d, J = 8.5 Hz, 1H), 7.52 (dd, J = 7.5, 1.7 Hz, 1H), 7.39 (dd, J = 11.6, 6.0 Hz, 1H), 7.21 (s, 1H), 6.90 (d, J = 8.2 Hz, 1H), 5.44 (s, 2H), 5.08 (qd, J = 7.1, 2.7 Hz, 1H), 4.75 (dd, J = 15.6, 7.0 Hz, 1H), 4.62 (dd, J = 15.6, 2.8 Hz, 1H), 4.57 - 4.41 (m, 3H), 4.36 (dt, J = 9.0, 5.9 Hz, 1H), 4.02 (s, 3H), 2.79 - 2.62 (m, 1H), 2.40 (ddt, J = 11.3, 9.1, 7.0 Hz, 1H).

### Example 208: (S)-2-(4-(6-((6-carbamoyl-4-fluoropyridin-3-yl)methoxy)-5-fluoropyridin-2-yl)-2,5-difluorobenzyl)-4-fluoro-1-(oxetan-2-ylmethyl)-1H-benzo[d]imidazole-6-carboxylic acid

(S)-2-(4-(6-((6-carbamoyl-4-fluoropyridin-3-yl)methoxy)-5-fluoropyridin-2-yl)-2,5-difluorobenzyl)-4-fluoro-1-(oxetan-2-ylmethyl)-1H-benzo[d]imidazole-6-carboxylic acid was prepared in a manner as described in Procedure 11. 1H NMR (400 MHz, MeOD) δ 8.84 (d, J = 9.3 Hz, 1H), 8.18 (s, 1H), 7.95 - 7.84 (m, 1H), 7.81 (t, J = 8.6 Hz, 1H), 7.73 - 7.46 (m, 3H), 7.20 (t, J = 9.1 Hz, 1H), 5.76 (s, J = 2.5 Hz, 2H), 5.17 (d, J = 7.6 Hz, 1H), 4.74 (dd, J = 15.9, 7.0 Hz, 1H), 4.69 - 4.57 (m, 2H), 4.52 (d, J = 16.2 Hz, 1H), 4.45 (d, J = 7.4 Hz, 1H), 2.78 (d, J = 9.4 Hz, 1H), 2.49 (t, J = 9.2 Hz, 1H).

### Example 209: (S)-2-(4-(6-((5-carbamoylpyrazin-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-carbamoylpyrazin-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 11. 1H NMR (400 MHz, DMSO-d6) δ 9.17 (d, J = 1.4 Hz, 1H), 8.83 (d, J = 1.4 Hz, 1H), 8.31 (d, J = 1.5 Hz, 1H), 8.24 (s, 1H), 7.92 (t, J = 7.9 Hz, 1H), 7.87 - 7.80 (m, 2H), 7.71 - 7.60 (m, 2H), 7.54 (dd, J = 7.6, 1.6 Hz, 1H), 7.39 (dd, J = 11.5, 6.1 Hz, 1H), 7.06 (d, J = 8.3 Hz, 1H), 5.70 (s, 2H), 5.16 - 4.98 (m, 1H), 4.80 (dd, J = 15.5, 7.2 Hz, 1H), 4.70 - 4.60 (m, 1H), 4.58 - 4.42 (m, 3H), 4.37 (dt, J = 9.0, 5.9 Hz, 1H), 2.79 - 2.62 (m, 1H), 2.44 - 2.33 (m, 1H).

### Example 210: 2-[[4-[6-[(1-cyclopropyltriazol-4-yl)methoxy]-2-pyridyl]-2,5-difluorophenyl]methyl]-3-[[(2S)-oxetan-2-yl]methyl]benzimidazole-5-carboxylic acid

### Procedure 12

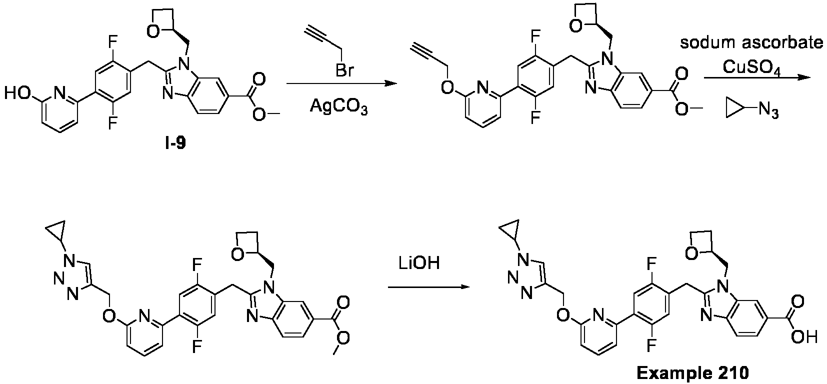

**Methyl 2-[[2,5-difluoro-4-(6-prop-2-ynoxy-2-pyridyl)phenyl]methyl]-3-[[(2S)-oxetan-2-yl]methyl]benzimidazole-5-carboxylate:** To a solution of methyl 2-[[2,5-difluoro-4-(6-hydroxy-2-pyridyl)phenyl]methyl]-3-[[(2S)-oxetan-2-yl]methyl]benzimidazole-5-carboxylate (100 mg, 0.21 mmol) in toluene (2 mL) was added 3-bromoprop-1-yne (35 mg, 0.23 mmol) and silver carbonate (117 mg, 0.42 mmol) and the resultant mixture stirred at 80 °C for 16 hrs. Following this time, the reaction mixture was filtered through celite, the filter washed with EtOAc (10 mL), and the filtrate subsequently concentrated. The crude residue was purified by silica gel chromatography (eluent: EtOAc/hexanes) to give desired product. ES/MS: 504.2 (M+H⁺).

**Methyl 2-[[4-[6-[(1-cyclopropyltriazol-4-yl)methoxy]-2-pyridyl]-2,5-difluorophenyl]methyl]-3-[[(2S)-oxetan-2-yl]methyl]benzimidazole-5-carboxylate:** To a solution of methyl 2-[[2,5-difluoro-4-(6-prop-2-ynoxy-2-pyridyl)phenyl]methyl]-3-[[(2S)-oxetan-2-yl]methyl]benzimidazole-5-carboxylate (66.0 mg, 0.13 mmol) in tert-butanol (1 mL) was added Copper(II) sulfate pentahydrate (6.55 mg, 0.02 mmol), L-Ascorbic acid sodium salt ( 5.19 mg, 0.02 mmol), and azidocyclopropane (33.5 mg, 0.26 mmol). The reaction mixture was then stirred at 40 °C for 16 hrs. Following this time, the reaction mixture wad diluted with EtOAc and ammonium chloride, the organic layer was washed with bicarb, dried over MgSO₄, and the filtrate subsequently concentrated. The crude residue was purified by silica gel chromatography (eluent: EtOAc/hexanes) to give desired product. ES/MS: 587.2 (M+H⁺).

**2-[[4-[6-[(1-cyclopropyltriazol-4-yl)methoxy]-2-pyridyl]-2,5-difluorophenyl]methyl]-3-[[(2S)-oxetan-2-yl]methyl]benzimidazole-5-carboxylic acid (Example 210):** To a solution of methyl 2-[[4-[6-[(1-cyclopropyltriazol-4-yl)methoxy]-2-pyridyl]-2,5-difluoro-phenyl]methyl]-3-[[(2S)-oxetan-2-yl]methyl]benzimidazole-5-carboxylate (98.0 mg, 0.06 mmol) in 3 mL of acetonitrile and 1 mL of water was added aqueous LiOH (0.04 mL, 0.17 mmol, 2 M). The solution was then stirred at rt overnight. The following day, a drop of TFA was added, then concentrated into 1 mL of DMF. The crude material was purified by reverse phase chromatography 10-58 % ACN/water with 0.1 % TFA added. The product containing fractions were combined, diluted with EtOAc, and 1 mg of sodium bicarbonate was added per mL of HPLC solvent. The aqueous layer was separated and the organic washed twice with water, then brine. The organic layer was dried over MgSO₄, filtered, and concentrated to give 26.0 mg of 2-[[4-[6-[(1-cyclopropyltriazol-4-yl)methoxy]-2-pyridyl]-2,5-difluoro-phenyl]methyl]-3-[[(2S)-oxetan-2-yl]methyl]benzimidazole-5-carboxylic acid. **Example 210.** ES/MS m/z: 573.2 (M+H⁺); 1H NMR (400 MHz, DMSO) δ 12.78 (s, 1H), 8.25 (d, J = 4.5 Hz, 2H), 7.94 (dd, J = 10.4, 6.4 Hz, 1H), 7.87 (t, J = 7.9 Hz, 1H), 7.79 (dd, J = 8.4, 1.6 Hz, 1H), 7.60 (d, J = 8.4 Hz, 1H), 7.55 - 7.47 (m, 1H), 7.39 (dd, J = 11.5, 6.1 Hz, 1H), 6.89 (d, J = 8.2 Hz, 1H), 5.76 (s, 2H), 5.48 (s, 2H), 5.08 (d, J = 5.5 Hz, 1H), 4.76 (dd, J = 15.6, 7.1 Hz, 1H), 4.63 (d, J = 14.5 Hz, 1H), 4.57 - 4.41 (m, 3H), 4.36 (dt, J = 9.0, 6.0 Hz, 1H), 3.99 (tt, J = 7.3, 3.9 Hz, 1H), 3.30 (s, 2H), 2.80 - 2.69 (m, 1H), 2.46 - 2.35 (m, 1H), 1.21 - 1.13 (m, 2H), 1.14 - 1.04 (m, 2H).

### Example 211: 2-[[4-[6-[[6-(3-cyclopropyl-1,2,4-triazol-1-yl)-3-pyridyl]methoxy]-2-pyridyl]-2,5-difluoro-phenyl]methyl]-3-[[(2S)-oxetan-2-yl]methyl]benzimidazole-5-carboxylic acid

### Procedure 13

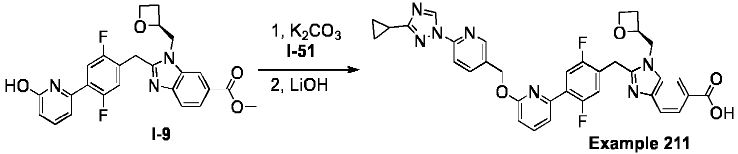

**2-[[4-[6-[[6-(3-cyclopropyl-1,2,4-triazol-1-yl)-3-pyridyl]methoxy]-2-pyridyl]-2,5-difluoro-phenyl]methyl]-3-[[(2S)-oxetan-2-yl]methyl]benzimidazole-5-carboxylic acid (Example 211):** To a solution of methyl 2-[[2,5-difluoro-4-(6-hydroxy-2-pyridyl)phenyl]methyl]-3-[[(2S)-oxetan-2-yl]methyl]benzimidazole-5-carboxylate (47 mg, 0.1 mmol) was dissolved in 1 mL of DMF, [6-(3-cyclopropyl-1,2,4-triazol-1-yl)-3-pyridyl]methyl 4-methylbenzenesulfonate **(I-51)** (37.4 mg, 0.1 mmol) and potassium carbonate (70 mg, 0.5 mmol) was added to the solution. It was stirred for 2 hrs. Following this time, the solution was diluted with EtOAc (10 mL) and washed with water. The organic extracts were dried over sodium sulfate, filtered and the filtrate was concentrated in vacuo. To the crude product was added 1 mL of ACN and 0.3 mL of 2 N lithium hydroxide. The mixture was heated to 80 °C for 30 min., followed by the addition of 0.3 mL of acetic acid to the mixture. The mixture was filtered and purified by RP-HPLC (eluent: water / MeCN 0.1% TFA) to give **Example 211.** ES/MS: 650.3 (M+H⁺). 1HNMR (400 MHz, Methanol-d4) δ 9.14 (s, 1H), 8.63 (d, J = 2.2 Hz, 1H), 8.51 (d, J = 1.3 Hz, 1H), 8.15 (td, J = 8.7, 1.8 Hz, 2H), 7.98 - 7.78 (m, 3H), 7.76 (d, J = 8.6 Hz, 1H), 7.57 (d, J = 7.1 Hz, 1H), 7.34 (dd, J = 11.3, 6.0 Hz, 1H), 6.94 (d, J = 8.2 Hz, 1H), 5.61 (s, 2H), 5.25 (q, J = 7.1 Hz, 1H), 5.00 - 4.91 (m, 1H), 4.84 - 4.64 (m, 4H), 4.52 (dt, J = 9.1, 5.9 Hz, 1H), 2.95 - 2.75 (m, 1H), 2.65 - 2.43 (m, 1H), 2.14 (ddd, J = 13.3, 8.3, 5.1 Hz, 1H), 1.15 - 0.97 (m, 4H).

### Example 212: (S)-2-(4-(6-((6-(4-cyclopropyl-1H-1,2,3-triazol-1-yl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-(4-cyclopropyl-1H-1,2,3-triazol-1-yl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 13. 1H NMR (400 MHz, Methanol-d4) δ 8.66 (d, J = 2.1 Hz, 1H), 8.55 (d, J = 1.4 Hz, 1H), 8.45 (s, 1H), 8.24 - 8.04 (m, 3H), 7.90 (dd, J = 10.7, 6.3 Hz, 1H), 7.82 (t, J = 7.9 Hz, 1H), 7.76 (d, J = 8.6 Hz, 1H), 7.56 (dd, J = 7.5, 1.6 Hz, 1H), 7.35 (dd, J = 11.2, 6.0 Hz, 1H), 6.94 (d, J = 8.2 Hz, 1H), 5.61 (s, 2H), 5.36 - 5.16 (m, 1H), 5.05 - 4.91 (m, 1H), 4.82 - 4.74 (m, 3H), 4.68 (ddd, J = 13.2, 9.3, 5.3 Hz, 1H), 4.52 (dt, J = 9.1, 5.9 Hz, 1H), 2.85 (dq, J = 11.5, 8.0 Hz, 1H), 2.56 (tt, J = 11.0, 7.2 Hz, 1H), 2.06 (ddd, J = 13.4, 8.7, 5.0 Hz, 1H), 1.09 - 0.96 (m, 2H), 0.94 - 0.78 (m, **2H) Example 213: 2-[[4-[6-[[6-(4-cyclopropyltriazol-1-yl)-3-pyridyl]methoxy]-2**-**pyridyl]-2,5-difluoro-phenyl]methyl]-3-[[(2S)-oxetan-2-yl]methyl]benzimidazole-5-carboxylic acid**

### Procedure 14

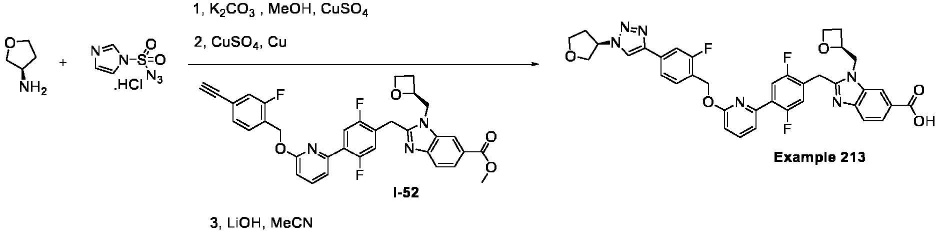

**2-[[4-[6-[[6-(4-cyclopropyltriazol-1-yl)-3-pyridyl]methoxy]**-**2-pyridyl]-2,5-difluorophenyl]methyl]-3-[[(2S)-oxetan-2-yl]methyl]benzimidazole-5-carboxylic acid (Example 213):** To a suspension of (3R)-tetrahydrofuran-3-amine (12 mg, 0.14 mmol), 1H-imidazole-1-sulfonyl azide hydrochloride (36 mg, 0.17 mmol), potassium carbonate (38 mg, 0.28 mmol), and cupric sulfate pentahydrate (3.5 mg, 0.014 mmol) in 1 mL of MeOH, was stirred for overnight. Acetic acid (33 mg, 0.55 mmol) was added to the suspension. Methyl 2-[[4-[6-[(4-ethynyl-2-fluoro-phenyl)methoxy]-2-pyridyl]-2,5-difluoro-phenyl]methyl]-3-[[(2S)-oxetan-2-yl]methyl]benzimidazole-5-carboxylate (60 mg, 0.1 mmol), copper sulfate (2 mg in 0.1 mL of water) and copper power (20 mg) were added to the mixture which was then stirred overnight. The next day the reaction mixture was filtered with celite and washed with EtOAc. The organic layer was dried over sodium sulfate, filtered and the filtrate was concentrated in vacuo. To the crude product was added 1 mL of ACN and 0.3 mL of 2 N lithium hydroxide. The mixture was heated to 80 °C for 30 min. 0.3 mL of acetic acid was added to the mixture. The mixture was filtered and purified by RP-HPLC (eluent: water / MeCN 0.1% TFA) to give **Example 213**.ES/MS: 697.2 (M+H+): 1H NMR (400 MHz, Methanol-d4) δ 8.60 (t, J = 1.0 Hz, 1H), 8.42 (s, 1H), 8.24 (dd, J = 8.6, 1.4 Hz, 1H), 7.94 (dd, J = 10.8, 6.3 Hz, 1H), 7.87 - 7.74 (m, 2H), 7.74 - 7.54 (m, 5H), 7.39 (dd, J = 11.2, 6.0 Hz, 1H), 6.93 (d, J = 8.3 Hz, 1H), 5.58 (s, 2H), 5.40 (ddt, J = 8.2, 5.6, 2.9 Hz, 1H), 5.27 (qd, J = 7.5, 2.4 Hz, 1H), 5.02 (dd, J = 15.5, 7.6 Hz, 1H), 4.90 (s, 1H), 4.85 - 4.74 (m, 1H), 4.74 - 4.62 (m, 1H), 4.55 (dt, J = 9.1, 6.0 Hz, 1H), 4.20 (q, J = 7.8 Hz, 1H), 4.16 - 4.07 (m, 2H), 3.97 (td, J = 8.6, 5.5 Hz, 1H), 2.94 - 2.82 (m, 1H), 2.76 - 2.49 (m, 2H), 2.43 (dddd, J = 13.6, 8.1, 5.6, 2.9 Hz, 1H).

### Example 214: (S)-2-(4-(6-((4-(1-cyclopropyl-1H-1,2,3-triazol-4-yl)-2-fluorobenzyl)oxy)pyridin-2-yl)-2,5-difluorobenzyl)-1-(oxetan-2-ylmethyl)-1H-benzo [d] imidazole-6-carboxylic acid

(S)-2-(4-(6-((4-(1-cyclopropyl-1H-1,2,3-triazol-4-yl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 14. 1H NMR (400 MHz, Methanol-d4) δ 8.58 (t, J = 1.0 Hz, 1H), 8.42 (s, 1H), 8.22 (dd, J = 8.6, 1.5 Hz, 1H), 7.94 (dd, J = 10.8, 6.3 Hz, 1H), 7.82 (t, J = 7.9 Hz, 1H), 7.78 (d, J = 8.6 Hz, 1H), 7.66 - 7.61 (m, 2H), 7.61 - 7.54 (m, 2H), 7.37 (dd, J = 11.2, 6.1 Hz, 1H), 6.99 - 6.82 (m, 1H), 5.59 (s, 2H), 5.27 (qd, J = 7.4, 2.4 Hz, 1H), 4.99 (dd, J = 15.5, 7.5 Hz, 1H), 4.86 - 4.73 (m, 3H), 4.73 - 4.66 (m, 1H), 4.54 (dt, J = 9.2, 6.0 Hz, 1H), 3.98 (tt, J = 7.4, 3.7 Hz, 1H), 2.97 - 2.78 (m, 1H), 2.72 - 2.47 (m, 1H), 1.36 - 1.27 (m, 2H), 1.27 - 1.19 (m, 2H).;1H NMR (400 MHz, Chloroform-d) δ 7.89 - 7.76 (m, 2H), 7.46 - 7.32 (m, 2H), 7.25 (d, 1H), 7.21 (dd, J = 7.9, 1.5 Hz, 1H), 7.10 (dd, J = 10.3, 1.4 Hz, 1H), 5.20 - 4.98 (m, 2H), 2.47 (s, 3H), 0.27 (s, 10H).

### Example 215: (S)-2-(2,5-difluoro-4-(6-((2-fluoro-4-(1-(oxetan-3-yl)-1H-1,2,3-triazol-4-yl)benzyl)oxy)pyridin-2-yl)benzyl)-1-(oxetan-2-ylmethyl)-1H-benzo [d] imidazole-6-carboxylic acid

(S)-2-(2,5-difluoro-4-(6-((2-fluoro-4-(1-(oxetan-3 -yl)-1H-1,2,3-triazol-4-yl)benzyl)oxy)pyridin-2-yl)benzyl)-1-(oxetan-2-ylmethyl)-1H-benzo[d]imidazole-6-carboxylic acid was prepared in a manner as described in Procedure 14. 1H NMR (400 MHz, Methanol-d4) δ 8.63 (s, 1H), 8.56 (d, J = 1.4 Hz, 1H), 8.20 (dd, J = 8.7, 1.4 Hz, 1H), 7.94 (dd, J = 10.8, 6.3 Hz, 1H), 7.82 (t, J = 7.9 Hz, 1H), 7.77 (d, J = 8.6 Hz, 1H), 7.72 - 7.60 (m, 3H), 7.57 (dd, J = 7.5, 1.6 Hz, 1H), 7.36 (dd, J = 11.2, 6.0 Hz, 1H), 6.93 (d, J = 8.3 Hz, 1H), 5.89 (tt, J = 7.5, 5.9 Hz, 1H), 5.59 (s, 2H), 5.33 - 5.21 (m, 1H), 5.22 - 5.12 (m, 2H), 5.12 - 5.03 (m, 2H), 4.97 (dd, J = 15.5, 7.5 Hz, 1H), 4.84 - 4.73 (m, 3H), 4.73 - 4.62 (m, 1H), 4.53 (dt, J = 9.1, 6.0 Hz, 1H), 2.87 (dq, J = 11.5, 8.0 Hz, 1H), 2.67 - 2.45 (m, 1H).

### Example 216: (S)-2-(4-(6-((4-(1-(bicyclo[1.1.1]pentan-1-yl)-1H-1,2,3-triazol-4-yl)-2-fluorobenzyl)oxy)pyridin-2-yl)-2,5-difluorobenzyl)-1-(oxetan-2-ylmethyl)-1H-benzo[d]imidazole-6-carboxylic acid

(S)-2-(4-(6-((4-(1-(bicyclo[1.1.1]pentan-1-yl)-1H-1,2,3-triazol-4-yl)-2-fluorobenzyl)oxy)pyridin-2-yl)-2,5-difluorobenzyl)-1-(oxetan-2-ylmethyl)-1H-benzo[d]imidazole-6-carboxylic acid was prepared in a manner as described in Procedure 14. 1H NMR (400 MHz, Methanol-d4) δ 8.59 (d, J = 1.4 Hz, 1H), 8.45 (s, 1H), 8.23 (dd, J = 8.6, 1.4 Hz, 1H), 7.94 (dd, J = 10.8, 6.3 Hz, 1H), 7.88 - 7.75 (m, 2H), 7.74 - 7.50 (m, 5H), 7.38 (dd, J = 11.2, 6.0 Hz, 1H), 6.93 (d, J = 8.2 Hz, 1H), 5.59 (s, 2H), 5.27 (qd, J = 7.5, 2.4 Hz, 1H), 5.00 (dd, J = 15.5, 7.5 Hz, 1H), 4.87 - 4.75 (m, 2H), 4.75 - 4.64 (m, 1H), 4.55 (dt, J = 9.2, 6.0 Hz, 1H), 2.88 (dtd, J = 11.5, 8.2, 6.1 Hz, 1H), 2.76 (s, 1H), 2.58 (ddt, J = 11.7, 10.0, 7.2 Hz, 1H).

### Example 217: 2-(2,5-difluoro-4-(6-((2-fluoro-4-(1-((S)-tetrahydrofuran-3-yl)-1H-1,2,3-triazol-4-yl)benzyl)oxy)pyridin-2-yl)benzyl)-1-(((S)-oxetan-2-yl)methyl)-1H-benzo[d]imidazole-6-carboxylic acid

2-(2,5-difluoro-4-(6-((2-fluoro-4-(1-((S)-tetrahydrofuran-3-yl)-1H-1,2,3-triazol-4-yl)benzyl)oxy)pyridin-2-yl)benzyl)-1-(((S)-oxetan-2-yl)methyl)-1H-benzo[d]imidazole-6-carboxylic acid was prepared in a manner as described in Procedure 14. 1H NMR (400 MHz, Methanol-d4) δ 8.57 (t, J = 1.0 Hz, 1H), 8.42 (s, 1H), 8.21 (dd, J = 8.6, 1.5 Hz, 1H), 7.93 (dd, J = 10.8, 6.3 Hz, 1H), 7.86 - 7.73 (m, 2H), 7.72 - 7.52 (m, 5H), 7.37 (dd, J = 11.2, 6.0 Hz, 1H), 6.93 (d, J = 8.3 Hz, 1H), 5.59 (s, 2H), 5.40 (ddt, J = 8.1, 5.5, 2.9 Hz, 1H), 5.33 - 5.19 (m, 1H), 4.99 (dd, J = 15.5, 7.5 Hz, 1H), 4.85 - 4.75 (m, 3H), 4.75 - 4.62 (m, 1H), 4.54 (dt, J = 9.2, 6.0 Hz, 1H), 4.20 (q, J = 7.6 Hz, 1H), 4.17 - 4.04 (m, 2H), 3.97 (td, J = 8.6, 5.5 Hz, 1H), 2.87 (dq, J = 11.5, 7.9 Hz, 1H), 2.70 - 2.52 (m, 2H), 2.43 (dtd, J = 14.6, 9.5, 8.9, 4.7 Hz, 1H).

### Example 218: (S)-2-(2,5-difluoro-4-(6-((2-fluoro-4-(1-(tetrahydro-2H-pyran-4-yl)-1H-1,2,3-triazol-4-yl)benzyl)oxy)pyridin-2-yl)benzyl)-1-(oxetan-2-ylmethyl)-1H-benzo [d] imidazole-6-carboxylic acid

(S)-2-(2,5-difluoro-4-(6-((2-fluoro-4-(1-(tetrahydro-2H-pyran-4-yl)-1H-1,2,3-triazol-4-yl)benzyl)oxy)pyridin-2-yl)benzyl)-1-(oxetan-2-ylmethyl)-1H-benzo[d]imidazole-6-carboxylic acid was prepared in a manner as described in Procedure 14. 1H NMR (400 MHz, Methanol-d4) δ 8.58 (d, J = 1.5 Hz, 1H), 8.49 (s, 1H), 8.21 (dd, J = 8.6, 1.4 Hz, 1H), 7.94 (dd, J = 10.8, 6.3 Hz, 1H), 7.89 - 7.73 (m, 2H), 7.72 - 7.61 (m, 3H), 7.61 - 7.55 (m, 1H), 7.37 (dd, J = 11.2, 6.0 Hz, 1H), 6.93 (d, J = 8.2 Hz, 1H), 5.59 (s, 2H), 5.32 - 5.18 (m, 1H), 4.99 (dd, J = 15.5, 7.5 Hz, 1H), 4.86 - 4.77 (m, 3H), 4.77 - 4.64 (m, 1H), 4.54 (dt, J = 9.2, 6.0 Hz, 1H), 4.12 (dt, J = 11.5, 3.3 Hz, 2H), 3.65 (ddd, J = 11.9, 8.4, 6.3 Hz, 2H), 2.97 - 2.78 (m, 1H), 2.74 - 2.49 (m, 1H), 2.25 - 2.11 (m, 4H).

### Example 219: 2-(4-(6-((4-(1-((1s,3s)-3-((tert-butoxycarbonyl)amino)cyclobutyl)-1H-1,2,3-triazol-4-yl)-2-fluorobenzyl)oxy)pyridin-2-yl)-2,5-difluorobenzyl)-1-(((S)-oxetan-2-yl)methyl)-1H-benzo[d]imidazole-6-carboxylic acid

2-(4-(6-((4-(1-((1s,3s)-3-((tert-butoxycarbonyl)amino)cyclobutyl)-1H-1,2,3-triazol-4-yl)-2-fluorobenzyl)oxy)pyridin-2-yl)-2, 5-difluorobenzyl)-1-(((S)-oxetan-2-yl)methyl)-1H-benzo[d]imidazole-6-carboxylic acid was prepared in a manner as described in Procedure 14. 1H NMR (400 MHz, Methanol-d4) δ 8.53 (s, 1H), 8.45 (s, 1H), 8.18 (d, J = 8.7 Hz, 1H), 7.93 (dd, J = 10.8, 6.3 Hz, 1H), 7.78 (dd, J = 19.3, 8.3 Hz, 2H), 7.68 - 7.60 (m, 2H), 7.58 (t, J = 7.4 Hz, 1H), 7.34 (dd, J = 11.3, 5.9 Hz, 1H), 6.92 (d, J = 8.3 Hz, 1H), 5.59 (s, 2H), 5.25 (d, J = 7.4 Hz, 1H), 4.99 - 4.89 (m, 3H), 4.84 - 4.64 (m, 4H), 4.52 (dt, J = 9.2, 6.0 Hz, 1H), 4.02 (d, J = 8.7 Hz, 1H), 3.07 - 2.94 (m, 2H), 2.86 (dt, J = 17.0, 7.8 Hz, 1H), 2.71 - 2.44 (m, 2H), 1.47 (s, 9H).

### Example 220: (S)-2-(2,5-difluoro-4-(6-((2-fluoro-4-(1-(1-methylazetidin-3-yl)-1H-1,2,3-triazol-4-yl)benzyl)oxy)pyridin-2-yl)benzyl)-1-(oxetan-2-ylmethyl)-1H-benzo [d] imidazole-6-carboxylic acid

(S)-2-(2,5-difluoro-4-(6-((2-fluoro-4-(1-(1-methylazetidin-3-yl)-1H-1,2,3-triazol-4-yl)benzyl)oxy)pyridin-2-yl)benzyl)-1-(oxetan-2-ylmethyl)-1H-benzo[d]imidazole-6-carboxylic acid was prepared in a manner as described in Procedure 14. 1H NMR (400 MHz, Methanol-d4) δ 8.49 (d, J = 6.2 Hz, 2H), 8.14 (dd, J = 8.5, 1.5 Hz, 1H), 7.94 (dd, J = 10.8, 6.3 Hz, 1H), 7.82 (t, J = 7.9 Hz, 1H), 7.75 (d, J = 8.5 Hz, 1H), 7.72 - 7.62 (m, 3H), 7.57 (d, J = 7.4 Hz, 1H), 7.32 (dd, J = 11.3, 6.0 Hz, 1H), 6.92 (d, J = 8.2 Hz, 1H), 5.68 (p, J = 7.0 Hz, 1H), 5.60 (s, 2H), 5.25 (q, J = 7.0 Hz, 1H), 5.04 - 4.89 (m, 1H), 4.86 (s, 1H), 4.83 - 4.60 (m, 4H), 4.51 (dt, J = 9.3, 5.9 Hz, 1H), 3.59 - 3.40 (m, 1H), 3.37 (s, 1H), 3.21 - 3.09 (m, 3H), 2.84 (dd, J = 16.3, 9.1 Hz, 1H), 2.63 - 2.46 (m, 1H), 2.05 (s, 1H).

### Example 221: 2-[[2,5-difluoro-4-[6-[[6-(3-methyl-3-methylsulfonyl-but-1-ynyl)-3-pyridyl]methoxy]-2-pyridyl]phenyl]methyl]-3-(2-methoxyethyl)benzimidazole-5-carboxylic acid

### Procedure 15

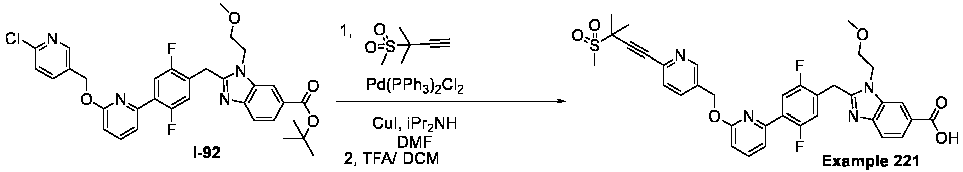

**2-[[2,5-difluoro-4-[6-[[6-(3-methyl-3-methylsulfonyl-but-1-ynyl)-3-pyridyl]methoxy]-2-pyridyl]phenyl]methyl]-3-(2-methoxyethyl)benzimidazole-5-carboxylic acid (Example 221):** Tert-butyl 2-[[4-[6-[(6-chloro-3-pyridyl)methoxy]-2-pyridyl]-2,5-difluorophenyl]methyl]-3-(2-methoxyethyl)benzimidazole-5-carboxylate (**I-92**) (50 mg, 0.08 mmol), 3-methyl-3-methylsulfonyl-but-1-yne (35 mg, 0.24 mmol), copper iodide (3 mg, 0.16 mmol), bis(triphenylphosphine)palladium chloride (11.3 mg, 0.016 mmole) and diisopropylamine (0.11 mL, 0.8 mmol) was suspended in DMF (0.5 mL). The reaction mixture was degassed by nitrogen. After which, the mixture was heated to 80 °C for 2 hrs. Upon completion of the time, the mixture was filter with celite and washed with EtOAc. The organic layer was washed with water and brine. The organic layer was filtered, and the filtrate was concentrated in vacuo. Next, 1 mL of DCM and 0.4 mL of TFA was added to the crude product. The mixture was then stirred for 3 hrs. The solvent was removed and remaining crude product was dissolved in 1 mL of DMF. The resulting mixture was then filtered and purified by RP-HPLC (eluent: water / MeCN 0.1% TFA) to give **Example 221.** ES/MS: 675.5 (M+H+): 1H NMR (400 MHz, Methanol-d4) δ 8.64 (s, 1H), 8.14 (d, J = 1.4 Hz, 1H), 8.01 (dd, J = 8.5, 1.5 Hz, 1H), 7.84 (dd, J = 8.1, 2.2 Hz, 1H), 7.78 - 7.73 (m, 1H), 7.73 - 7.62 (m, 2H), 7.48 (dd, J = 14.3, 7.7 Hz, 2H), 7.09 - 7.01 (m, 1H), 6.80 (d, J = 8.2 Hz, 1H), 5.49 (s, 2H), 4.43 (s, 2H), 4.40 (t, J = 5.2 Hz, 2H), 3.68 (t, J = 5.0 Hz, 2H), 3.24 (s, 3H), 3.11 (s, 3H), 1.74 (s, 6H).

### Example 222: (S)-2-(4-(2-((6-carbamoylpyridin-3-yl)methoxy)pyrimidin-4-yl)-2,5-difluorobenzyl)-1-(oxetan-2-ylmethyl)-1H-benzo[d]imidazole-6-carboxylic acid

### Procedure 16

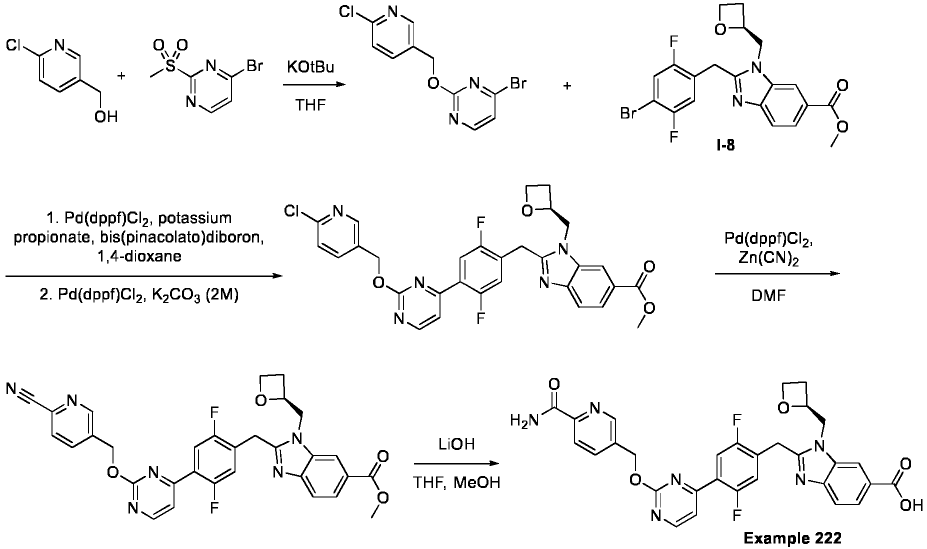

**4-bromo-2-((6-chloropyridin-3-yl)methoxy)pyrimidine:** Potassium tert-butoxide (1M in THF, 2.11 mL, 2.11 mmol) was added dropwise to a solution of (6-chloropyridin-3-yl)methanol (333 mg, 2.32 mmol) in THF (5 mL) at 0 °C and the solution was stirred for 15 min. Next, the resulting solution was added dropwise to a solution of 4-bromo-2-(methylsulfonyl)pyrimidine (500 mg, 2.11 mmol) in THF (5 mL) at -78 °C. The resulting solution was stirred for 1 hr. Following this time, the mixture was warmed to 0 °C, and water was added dropwise. The mixture was warmed to rt and diluted with EtOAc, and saturated NH₄Cl. The phases were separated, and the organic phase was dried over Na₂SO₄, filtered, concentrated, and purified by flash chromatography (EtOAc/hexanes) to give title product.

**Methyl (S)-2-(4-(2-((6-chloropyridin-3-yl)methoxy)pyrimidin-4-yl)-2,5-difluorobenzyl)-1-(oxetan-2-ylmethyl)-1H-benzo[d]imidazole-6-carboxylate:** The title compound was prepared in a manner as described for Intermediate **I-7**, using methyl (S)-2-(4-bromo-2,5-difluorobenzyl)-1-(oxetan-2-ylmethyl)-1H-benzo[d]imidazole-6-carboxylate in place of methyl 2-(4-bromo-2,5-difluorophenyl)acetate, and 4-bromo-2-((6-chloropyridin-3-yl)methoxy)pyrimidine in place of 4-[(6-bromo-2-pyridyl)oxymethyl]-3-fluoro-benzonitrile.

**Methyl (S)-2-(4-(2-((6-cyanopyridin-3-yl)methoxy)pyrimidin-4-yl)-2,5-difluorobenzyl)-1-(oxetan-2-ylmethyl)-1H-benzo[d]imidazole-6-carboxylate:** Methyl (S)-2-(4-(2-((6-chloropyridin-3-yl)methoxy)pyrimidin-4-yl)-2, 5-difluorobenzyl)-1-(oxetan-2-ylmethyl)-1H-benzo[d]imidazole-6-carboxylate (50 mg, 0.0845 mmol), Zn(CN)₂ (11.9 mg, 0.101 mmol), and Pd(dppf)Cl₂ (69.1 mg, 0.0845 mmol) were taken up in DMF (1 mL), and degassed with bubbling argon for 60 seconds, and heated at 100 °C overnight. The next day the reaction mixture was cooled, diluted with EtOAc, filtered through celite, concentrated, and partitioned between EtOAc and water. Aqueous layer was extracted an additional time with EtOAc, then combined organic layers were washed twice with water and once with brine, concentrated, and purified by flash chromatography (EtOAc/hexanes) to give title product.

**(S)-2-(4-(2-((6-carbamoylpyridin-3-yl)methoxy)pyrimidin-4-yl)-2,5-difluorobenzyl)-1-(oxetan-2-ylmethyl)-1H-benzo[d]imidazole-6-carboxylic acid (Example 222):** LiOH (2M in H₂O, 0.23 mL, 0.461 mmol) was added to a solution of methyl (S)-2-(4-(2-((6-cyanopyridin-3-yl)methoxy)pyrimidin-4-yl)-2,5-difluorobenzyl)-1-(oxetan-2-ylmethyl)-1H-benzo[d]imidazole-6-carboxylate (26.8 mg, 0.0461 mmol) in THF (0.5 mL) and MeOH (0.5 mL). The mixture was stirred at 40 °C for 30 min. Following this time, the mixture was concentrated, taken up in DMF (1.5 mL), acidified with AcOH (0.1 mL), diluted with water (0.5 mL), purified by RP-HPLC (eluent: water / MeCN 0.1% TFA) to give **Example 222.** ES/MS: 587.2 (M+H⁺); ¹H NMR (400 MHz, DMSO) δ 8.86 - 8.75 (m, 2H), 8.27 (d, J = 1.5 Hz, 1H), 8.17 - 8.03 (m, 3H), 7.93 (dd, J = 10.2, 6.2 Hz, 1H), 7.80 (dd, J = 8.5, 1.6 Hz, 1H), 7.69 - 7.57 (m, 3H), 7.49 (dd, J = 11.5, 5.9 Hz, 1H), 5.63 (s, 2H), 5.18 - 5.01 (m, 1H), 4.77 (dd, J = 15.6, 7.1 Hz, 1H), 4.70 - 4.43 (m, 4H), 4.35 (dt, J = 9.1, 6.0 Hz, 1H), 2.79 - 2.68 (m, 1H), 2.44 - 2.32 (m, 1H).

**Example 223: (S)-2-(4-(2-((6-carbamoyl-2-(difluoromethoxy)pyridin-3-yl)methoxy)pyrimidin-4-yl)-2,5-difluorobenzyl)-1-(oxetan-2-ylmethyl)-1H-benzo[d]imidazole-6-carboxylic acid**(S)-2-(4-(2-((6-carbamoyl-2-(difluoromethoxy)pyridin-3-yl)methoxy)pyrimidin-4-yl)-2,5-difluorobenzyl)-1-(oxetan-2-ylmethyl)-1H-benzo[d]imidazole-6-carboxylic acid was prepared in a manner as described in Procedure 16. 1H NMR (400 MHz, DMSO) δ 8.76 (d, J = 5.1 Hz, 1H), 8.45 - 7.98 (m, 4H), 7.85 (dd, J = 10.2, 6.2 Hz, 1H), 7.80 - 7.74 (m, 2H), 7.64 - 7.55 (m, 2H), 7.46 (dd, J = 11.5, 5.8 Hz, 1H), 7.29 (d, J = 8.6 Hz, 1H), 5.95 (s, 2H), 5.13 - 4.99 (m, 1H), 4.75 (dd, J = 15.6, 7.1 Hz, 1H), 4.66 - 4.58 (m, 1H), 4.58 - 4.41 (m, 3H), 4.34 (dt, J = 9.0, 5.9 Hz, 1H), 2.76 - 2.68 (m, 1H), 2.41 - 2.35 (m, 1H).

### Example 224: 2-[[2,5-difluoro-4-[6-[[5-(1-methylpyrazol-4-yl)thiazol-2-yl]methoxy]-2-pyridyl]phenyl]methyl]-3-[(2R)-2-methoxypropyl]benzimidazole-5-carboxylic acid

### Procedure 17

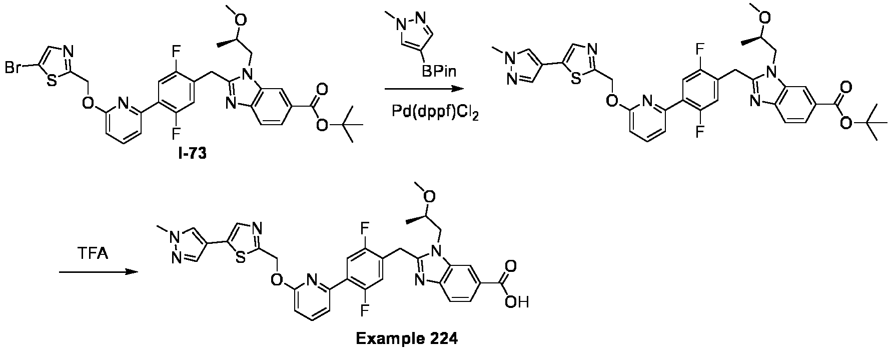

**Tert-butyl 2-[[2,5-difluoro-4-[6-[[5-(1-methylpyrazol-4-yl)thiazol-2-yl]methoxy]-2-pyridyl]phenyl]methyl]-3-[(2R)-2-methoxypropyl]benzimidazole-5-carboxylate:** To a solution of tert-butyl 2-[[4-[6-[(5-bromothiazol-2-yl)methoxy]-2-pyridyl]-2,5-difluorophenyl]methyl]-3-[(2R)-2-methoxypropyl]benzimidazole-5-carboxylate (**I-73**) (60 mg, 0.088 mmol) in 1,4-dioxane (2 mL), 1-methyl-4-(4,4,5,5-tetramethyl-1,3,2-dioxaborolan-2-yl)pyrazole (36 mg, 0.18 mmol), Pd(dppf)Cl₂ (6.5 mg, 0.009 mmol) and aqueous sodium carbonate (2.0M, 88 uL, 0.18 mmol) were added. The reaction mixture was bubbled with argon for 1 min, then the reaction vessel sealed and heated to 100 °C for 1 hr. Upon completion of this time, the reaction mixture was diluted with EtOAc (25 mL), washed with water (5 mL) and brine (5 mL). The organic layer was dried over MgSO₄, filtered, concentrated and purified by silica gel column chromatography (eluent: EtOAc/hexanes) to provide desired product. ES/MS: 686.8 (M+H⁺).

**2-[[2,5-difluoro-4-[6-[[5-(1-methylpyrazol-4-yl)thiazol-2-yl]methoxy]-2-pyridyl]phenyl]methyl]-3-[(2R)-2-methoxypropyl]benzimidazole-5-carboxylic acid (Example 224):** To a solution of tert-butyl 2-[[2,5-difluoro-4-[6-[[5-(1-methylpyrazol-4-yl)thiazol-2-yl]methoxy]-2-pyridyl]phenyl]methyl]-3-[(2R)-2-methoxypropyl]benzimidazole-5-carboxylate (43 mg, 0.063 mmol) in DCM (2 mL), 0.25 mL TFA was added and the resulting solution stirred at 40 °C for 1 hr. The reaction mixture was diluted with EtOAc (30 mL), washed with water (3 x 5 mL), concentrated and purified by RP-HPLC (eluent: water / MeCN 0.1% TFA). The combined fractions were then frozen and placed on a lyophilizer to provide **Example 224**. ES/MS: 631.1 (M+H⁺). 1HNMR (400 MHz, DMSO-d6) δ 8.33 (d, J = 1.4 Hz, 1H), 8.07 (s, 1H), 8.00 - 7.84 (m, 4H), 7.74 (d, J = 0.8 Hz, 1H), 7.66 (d, J = 8.5 Hz, 1H), 7.58 (dd, J = 7.5, 1.6 Hz, 1H), 7.43 (dd, J = 11.6, 6.1 Hz, 1H), 7.02 (d, J = 8.3 Hz, 1H), 5.75 (s, 2H), 4.58 (dd, J = 15.3, 3.2 Hz, 1H), 4.54 (s, 2H), 4.42 (dd, J = 15.1, 8.9 Hz, 1H), 3.84 (s, 3H), 3.71 (ddd, J = 9.2, 6.2, 3.1 Hz, 1H), 3.08 (s, 3H), 1.24 (d, J = 6.1 Hz, 3H).

### Example 225: 2-(4-(6-((4-(1-cyclopropyl-1H-pyrazol-4-yl)-2-fluorobenzyl)oxy)pyridin-2-yl)-2,5-difluorobenzyl)-1-(2-methoxyethyl)-1H-benzo[d]imidazole-6-carboxylic acid

2-(4-(6-((4-(1-cyclopropyl-1H-pyrazol-4-yl)-2-fluorobenzyl)oxy)pyridin-2-yl)-2,5-difluorobenzyl)-1-(2-methoxyethyl)-1H-benzo[d]imidazole-6-carboxylic acid was prepared in a manner as described in Procedure 17. 1H NMR (400 MHz, Methanol-d4) δ 8.25 (d, J = 1.4 Hz, 1H), 8.11 (s, 1H), 7.99 (dd, J = 8.5, 1.6 Hz, 1H), 7.93 - 7.83 (m, 2H), 7.78 (t, J = 7.9 Hz, 1H), 7.65 (d, J = 8.4 Hz, 1H), 7.53 (t, J = 7.8 Hz, 2H), 7.43 - 7.32 (m, 2H), 7.14 (dd, J = 11.6, 6.1 Hz, 1H), 6.87 (d, J = 8.2 Hz, 1H), 5.55 (s, 2H), 4.57 (t, J = 5.1 Hz, 2H), 4.50 (s, 2H), 3.75 (t, J = 5.0 Hz, 2H), 3.73 - 3.63 (m, 1H), 1.20 - 1.01 (m, 4H), 1.01 - 0.83 (m, 1H).

### Example 226: 2-(2,5-difluoro-4-(6-((2-fluoro-4-(1-(tetrahydro-2H-pyran-4-yl)-1H-pyrazol-4-yl)benzyl)oxy)pyridin-2-yl)benzyl)-1-(2-methoxyethyl)-1H-benzo[d]imidazole-6-carboxylic acid

2-(2,5-difluoro-4-(6-((2-fluoro-4-(1-(tetrahydro-2H-pyran-4-yl)-1H-pyrazol-4-yl)benzyl)oxy)pyridin-2-yl)benzyl)-1-(2-methoxyethyl)-1H-benzo[d]imidazole-6-carboxylic acid was prepared in a manner as described in Procedure 17. ¹H NMR (400 MHz, DMSO-*d*₆) δ 8.36 (s, 1H), 8.25 (d, 1H), 7.97 (s, 1H), 7.93 - 7.80 (m, 3H), 7.70 - 7.57 (m, 2H), 7.61 - 7.48 (m, 6H), 7.49 - 7.36 (m, 2H), 6.94 (d, 1H), 5.49 (s, 2H), 4.63 (t, 2H), 4.49 (s, 2H), 4.45 - 4.29 (m, 1H), 3.97 (d, 5H), 3.69 (t, 2H), 3.48 (td, 2H), 2.12 - 1.88 (m, 6H).

### Example 227: 2-(2,5-difluoro-4-(6-((2-fluoro-4-(1-(2-morpholinoethyl)-1H-pyrazol-4-yl)benzyl)oxy)pyridin-2-yl)benzyl)-1-(2-methoxyethyl)-1H-benzo[d]imidazole-6-carboxylic acid

2-(2,5-difluoro-4-(6-((2-fluoro-4-(1-(2-morpholinoethyl)-1H-pyrazol-4-yl)benzyl)oxy)pyridin-2-yl)benzyl)-1-(2-methoxyethyl)-1H-benzo[d]imidazole-6-carboxylic acid was prepared in a manner as described in Procedure 17. ¹H NMR (400 MHz, DMSO-*d*₆) δ 8.35 (s, 1H), 8.23 (d, 1H), 8.10 (s, 1H), 7.92 - 7.82 (m, 2H), 7.81 (dd, 1H), 7.64 - 7.49 (m, 4H), 7.50 - 7.35 (m, 2H), 6.94 (d, 1H), 5.51 (s, 2H), 4.59 (dt, 4H), 4.46 (s, 2H), 3.69 (t, 2H), 3.57 (s, 0H), 3.22 (s, 3H).

### Example 228: 2-(2,5-difluoro-4-(6-((2-fluoro-4-(6-(pyrrolidin-1-yl)pyridin-3-yl)benzyl)oxy)pyridin-2-yl)benzyl)-1-(2-methoxyethyl)-1H-benzo[d]imidazole-6-carboxylic acid

2-(2, 5-difluoro-4-(6-((2-fluoro-4-(6-(pyrrolidin-1-yl)pyridin-3-yl)benzyl)oxy)pyridin-2-yl)benzyl)-1-(2-methoxyethyl)-1H-benzo[d]imidazole-6-carboxylic acid was prepared in a manner as described in Procedure 17. 1H NMR (400 MHz, DMSO-*d*₆) δ 8.34 (s, 1H), 8.23 (d, 1H), 7.93 - 7.77 (m, 3H), 7.70 - 7.49 (m, 5H), 7.40 (dd, 1H), 6.95 (d, 1H), 5.56 (s, 2H), 4.61 (t, 2H), 4.46 (s, 2H), 3.69 (t, 3H), 3.22 (s, 3H), 2.48 (d, 5H), 2.02 (d, 1H), 2.02 (s, 4H).

### Example 229: 2-(2,5-difluoro-4-(6-((6-(1-(piperidin-4-yl)-1H-pyrazol-4-yl)pyridin-3-yl)methoxy)pyridin-2-yl)benzyl)-1-(2-methoxyethyl)-1H-benzo[d]imidazole-6-carboxylic acid

2-(2,5-difluoro-4-(6-((6-(1-(piperidin-4-yl)-1H-pyrazol-4-yl)pyridin-3-yl)methoxy)pyridin-2-yl)benzyl)-1-(2-methoxyethyl)-1H-benzo[d]imidazole-6-carboxylic acid was prepared in a manner as described in Procedure 17. 1H NMR (400 MHz, Acetonitrile-d3) δ 8.34 (d, 1H), 8.07 - 7.95 (m, 2H), 7.96 - 7.84 (m, 2H), 7.85 - 7.71 (m, 2H), 7.59 - 7.50 (m, 2H), 7.42 - 7.32 (m, 2H), 7.27 (dd, 1H), 6.88 (d, 1H), 5.55 (s, 2H), 4.57 (d, 4H), 4.54 - 4.45 (m, 1H), 3.74 (t, 2H), 3.55 (d, 2H), 3.42 (s, 19H), 2.36 - 2.25 (m, 4H).**Example 230: 2-[[2,5-difluoro-4-[6-[(1-methylthieno[2,3-c]pyrazol-5-yl)methoxy]**-**2-pyridyl]phenyl]methyl]-3-[[(2S)-oxetan-2-yl]methyl]benzimidazole-5-carboxylic acid**

### Procedure 18

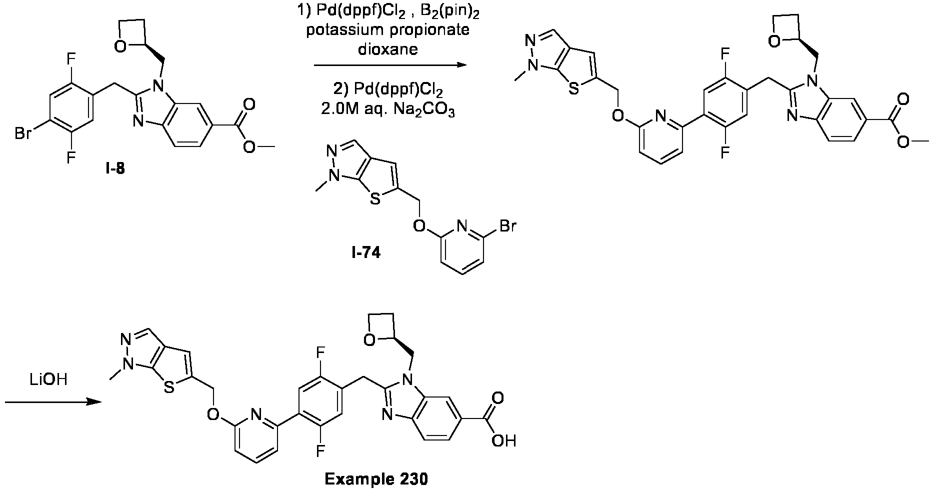

**Methyl 2-[[2,5-difluoro-4-[6-[(1-methylthieno [2,3-c] pyrazol-5-yl)methoxy]-2-pyridyl]phenyl]methyl]-3-[[(2S)-oxetan-2-yl]methyl]benzimidazole-5-carboxylate:** In a 8 mL reaction vial, a suspension of methyl 2-[(4-bromo-2,5-difluoro-phenyl)methyl]-3-[[(2S)-oxetan-2-yl]methyl]benzimidazole-5-carboxylate (**I-8**) (110 mg, 0.24 mmol), Bis(pinacolato)diboron (93 mg, 0.37 mmol), [1,1'-Bis(diphenylphosphino)ferrocene] dichloropalladium(II); PdCl₂(dppf) (18 mg, 0.024 mmol), and potassium propionate (82 mg, 0.73 mmol) in dioxane (2 mL) was degassed with Ar for 5 min. Upon completion the mixture was heated at 110 °C thermally for 1 hr. LCMS shows complete conversion of bromide. Following this time, the mixture was cooled to rt. Sodium carbonate (2.00 M, 0.24 mL, 0.49 mmol) was added to the solution and the solution was stirred at rt for 5 min. Next, [1,1'-Bis(diphenylphosphino)ferrocene] dichloropalladium(II); PdCl₂(dppf) (9.0 mg, 0.012 mmol) and 5-[(6-bromo-2-pyridyl)oxymethyl]-1-methyl-thieno[2,3-c]pyrazole (**I-74**) (87 mg, 0.27 mmol) was added to the reaction mixture. The reaction mixture was degassed for 5 min with Ar, then heated thermally at 90 °C for 2 hrs. Upon completion the reaction mixture was diluted with EtOAc and washed with brine and saturated sodium bicarbonate solution. The organic extract was dried over sodium sulfate, filtered and concentrated. The crude residue was purified by flash chromatography (eluent: EtOAc/hexanes) to yield desired product. ES/MS: 616.8 (M+H⁺).

**2-[[2,5-difluoro-4-[6-[(1-methylthieno[2,3-c]pyrazol-5-yl)methoxy]-2-pyridyl]phenyl]methyl]-3-[[(2S)-oxetan-2-yl]methyl]benzimidazole-5-carboxylic acid (Example 230):** A suspension of methyl 2-[[2,5-difluoro-4-[6-[(1-methylthieno[2,3-c]pyrazol-5-yl)methoxy]-2-pyridyl]phenyl]methyl]-3-[[(2S)-oxetan-2-yl]methyl]benzimidazole-5-carboxylate (86 mg, 0.14 mmol) and aqueous lithium hydroxide (0.5 mL, 10 mg, 0.42 mmol) in CH₃CN (3 mL) was heated at 100 °C for 10 min. 5% aqueous citric acid was added (3 mL), the reaction mixture was diluted with EtOAc (25 mL), washed with water (5 mL) and brine (5 mL). The organic layer was dried over MgSO₄, filtered, concentrated and purified by silica gel column chromatography (eluent: MeOH/DCM) to provide **Example 230.** ES/MS: 602.2 (M+H⁺). 1HNMR (400 MHz, DMSO-d6) δ 12.78 (s, 1H), 8.26 (d, J = 1.5 Hz, 1H), 7.99 (dd, J = 10.5, 6.4 Hz, 1H), 7.88 (t, J = 7.9 Hz, 1H), 7.80 (dd, J = 8.4, 1.5 Hz, 1H), 7.71 (s, 1H), 7.61 (d, J = 8.5 Hz, 1H), 7.55 (dd, J = 7.4, 1.6 Hz, 1H), 7.41 (dd, J = 11.6, 6.0 Hz, 1H), 7.22 (s, 1H), 6.91 (d, J = 8.2 Hz, 1H), 5.67 (s, 2H), 5.08 (qd, J = 7.1, 2.7 Hz, 1H), 4.76 (dd, J = 15.6, 7.1 Hz, 1H), 4.63 (dd, J = 15.5, 2.8 Hz, 1H), 4.59 - 4.42 (m, 3H), 4.36 (dt, J = 9.0, 5.9 Hz, 1H), 3.89 (s, 3H), 2.80 - 2.64 (m, 1H), 2.46 - 2.31 (m, 1H).

### Example 275: 2-(2,5-difluoro-4-(6-((5-methoxy-1,3,4-thiadiazol-2-yl)methoxy)pyridin-2-yl)benzyl)-1-((1-(difluoromethyl)cyclopropyl)methyl)-4-fluoro-1H-benzo [d] imidazole-6-carboxylic acid

2-(2, 5-difluoro-4-(6-((5-methoxy-1,3,4-thiadiazol-2-yl)methoxy)pyridin-2-yl)benzyl)-1-((1-(difluoromethyl)cyclopropyl)methyl)-4-fluoro-1H-benzo[d]imidazole-6-carboxylic acid was prepared in a manner as described in Procedure 18 using Intermediate **I-1090** and **I-1194.** ES/MS m/z: 632.4 (M+H⁺). 1H NMR (400 MHz, Methanol-d4) δ 8.19 (d, J = 1.3 Hz, 1H), 8.00 - 7.91 (m, 1H), 7.84 (td, J = 7.8, 3.7 Hz, 1H), 7.69 (dd, J = 11.1, 1.2 Hz, 1H), 7.61 (dd, J = 7.5, 1.5 Hz, 1H), 7.29 - 7.18 (m, 1H), 6.91 (d, J = 8.2 Hz, 1H), 5.72 (s, 2H), 5.61 - 5.40 (m, 1H), 4.71 (s, 2H), 4.54 (s, 2H), 4.15 (s, 3H), 0.99 (t, J = 3.5 Hz, 2H), 0.88 (hept, J = 4.2, 3.6 Hz, 2H).

### Example 280: 4-fluoro-1-((1-(fluoromethyl)cyclopropyl)methyl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid

4-fluoro-1-((1-(fluoromethyl)cyclopropyl)methyl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid was prepared in a manner as described in Procedure 18 using Intermediates **I-1090** and **I-1201.** ES/MS m/z: 632.3 (M+H⁺). 1HNMR (400 MHz, Methanol-d4) δ 8.11 (d, J = 1.2 Hz, 1H), 7.83 (t, J = 7.9 Hz, 1H), 7.79 - 7.70 (m, 1H), 7.65 - 7.56 (m, 2H), 6.91 (d, J = 8.2 Hz, 1H), 5.70 (s, 2H), 4.54 (d, J = 19.4 Hz, 4H), 4.24 (s, 1H), 4.15 (s, 4H), 0.90 (dd, J = 8.1, 3.6 Hz, 2H), 0.85 (d, J = 4.8 Hz, 2H).

### Example 367: (S)-4-chloro-2-(2,5-difluoro-4-(5-fluoro-6-((5-methoxy-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid

(S)-4-chloro-2-(2,5-difluoro-4-(5-fluoro-6-((5-methoxy-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 18 using Intermediate **I-1244** and **I-1037**. ES/MS m/z: 660.0 (M+H⁺). 1H NMR (400 MHz, Methanol-d4) δ 8.65 (s, 1H), 7.99 (d, J = 1.3 Hz, 1H), 7.94 - 7.83 (m, 1H), 7.75 - 7.55 (m, 2H), 7.23 (dd, J = 11.5, 6.0 Hz, 1H), 5.80 (d, J = 1.1 Hz, 2H), 4.92 (d, J = 6.8 Hz, 1H), 4.69 - 4.35 (m, 4H), 4.17 (s, 3H), 3.93 (d, J = 8.8 Hz, 1H), 3.78 (d, J = 8.8 Hz, 1H), 1.32 (s, 3H), 0.62 (s, 3H).

### Example 369: (S)-2-(2-chloro-4-(5-fluoro-6-((5-(trifluoromethyl)thiazol-2-yl)methoxy)pyridin-2-yl)-5-methylbenzyl)-1-(oxetan-2-ylmethyl)-1H-benzo [d] imidazole-6-carboxylic acid

(S)-2-(2-chloro-4-(5-fluoro-6-((5-(trifluoromethyl)thiazol-2-yl)methoxy)pyridin-2-yl)-5-methylbenzyl)-1-(oxetan-2-ylmethyl)-1H-benzo[d]imidazole-6-carboxylic acid was prepared in a manner as described in Procedure 18 using Intermediate **I-102** and **I-1024**. ES/MS m/z: 647.1 (M+H⁺). 1H NMR (400 MHz, Methanol-d4) δ 8.31 (d, J = 1.4 Hz, 1H), 8.23 (q, J = 1.2 Hz, 1H), 7.98 (dd, J = 8.5, 1.5 Hz, 1H), 7.72 - 7.62 (m, 2H), 7.49 (s, 1H), 7.22 (dd, J = 8.1, 2.8 Hz, 1H), 7.19 (s, 1H), 5.83 (s, 2H), 5.17 (qd, J = 7.1, 2.5 Hz, 1H), 4.60 - 4.36 (m, 3H), 3.00 - 2.67 (m, 1H), 2.63 - 2.45 (m, 1H), 2.25 (s, 3H).

### Example 370: (S)-2-(2,5-difluoro-4-(5-fluoro-6-((5-(trifluoromethyl)thiazol-2-yl)methoxy)pyridin-2-yl)benzyl)-1-(oxetan-2-ylmethyl)-1H-benzo [d] imidazole-6-carboxylic acid

(S)-2-(2,5-difluoro-4-(5-fluoro-6-((5-(trifluoromethyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 18 using Intermediate **I-102** and **I-8**. ES/MS m/z: 634.6 (M+H⁺). 1HNMR (400 MHz, Methanol-d4) δ 8.34 - 8.22 (m, 2H), 7.99 (dd, J = 8.5, 1.5 Hz, 1H), 7.81 (dd, J = 10.7, 6.4 Hz, 1H), 7.75 - 7.59 (m, 3H), 7.20 (dd, J = 11.6, 5.9 Hz, 1H), 5.93 (s, 2H), 5.20 (d, J = 8.9 Hz, 1H), 4.73 (dd, J = 15.7, 7.0 Hz, 1H), 4.70 - 4.50 (m, 3H), 4.49 - 4.34 (m, 1H), 2.80 (s, 1H), 2.50 (t, J = 9.3 Hz, 1H).

### Example 371: (S)-2-(2-chloro-4-(5-fluoro-6-((5-(trifluoromethyl)thiazol-2-yl)methoxy)pyridin-2-yl)-5-methylbenzyl)-1-(4,4-dimethyltetrahydrofuran-3-yl)-1H-benzo[d]imidazole-6-carboxylic acid

(S)-2-(2-chloro-4-(5-fluoro-6-((5-(trifluoromethyl)thiazol-2-yl)methoxy)pyridin-2-yl)-5-methylbenzyl)-1-(4,4-dimethyltetrahydrofuran-3-yl)-1H-benzo[d]imidazole-6-carboxylic acid was prepared in a manner as described in Procedure 18 using Intermediate **I-102** and **I-1030**. ES/MS m/z: 674.4 (M+H⁺). 1H NMR (400 MHz, DMSO-d6) δ 8.53 - 8.45 (m, 2H), 7.91 (dd, J = 10.3, 8.1 Hz, 1H), 7.80 (dd, J = 8.5, 1.4 Hz, 1H), 7.62 (d, J = 8.4 Hz, 1H), 7.49 (s, 1H), 7.39 - 7.31 (m, 2H), 5.87 (s, 2H), 5.00 (d, J = 6.7 Hz, 1H), 4.59 - 4.46 (m, 2H), 4.46 - 4.32 (m, 2H), 3.80 (d, J = 8.6 Hz, 1H), 3.72 (d, J = 8.6 Hz, 1H), 2.22 (s, 3H), 1.32 (s, 3H), 0.65 (s, 3H).

### Example 373: (S)-2-(2,5-difluoro-4-(6-((4-(methylcarbamoyl)thiazol-2-yl)methoxy)pyridin-2-yl)benzyl)-1-(oxetan-2-ylmethyl)-1H-benzo[d]imidazole-6-carboxylic acid

(S)-2-(2,5-difluoro-4-(6-((4-(methylcarbamoyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 18 using Intermediate **I**-**1017** and **I**-**8**. ES/MS m/z: 606.2 (M+H⁺). 1H NMR (400 MHz, Methanol-d4) δ 8.53 (s, 1H), 8.31 (d, J = 1.5 Hz, 1H), 8.16 (d, J = 1.0 Hz, 1H), 7.99 (dt, J = 8.5, 1.3 Hz, 1H), 7.84 (dd, J = 10.0, 5.8 Hz, 2H), 7.67 (d, J = 8.5 Hz, 1H), 7.60 (d, J = 7.5 Hz, 1H), 7.19 (dd, J = 11.5, 6.0 Hz, 1H), 6.95 (d, J = 8.1 Hz, 1H), 5.80 (s, 1H), 5.29 - 5.11 (m, 1H), 4.73 (dd, J = 15.7, 6.9 Hz, 1H), 4.68 - 4.33 (m, 6H), 2.93 (d, J = 4.7 Hz, 3H), 2.85 - 2.69 (m, 1H), 2.60 - 2.40 (m, 1H).

### Example 375: (S)-2-(4-(6-((6-((2-cyanoethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-((2-cyanoethyl)carbamoyl)pyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 18 using Intermediates **I-1010** and **I-8.** ES/MS m/z: 639.2 (M+H⁺). 1H NMR (400 MHz, Methanol-d4) δ 8.79 (d, J = 1.8 Hz, 1H), 8.31 (s, 1H), 8.19 - 8.03 (m, 2H), 7.99 (dd, J = 8.5, 1.5 Hz, 1H), 7.85 - 7.74 (m, 2H), 7.67 (d, J = 8.5 Hz, 1H), 7.54 (d, J = 7.3 Hz, 1H), 7.18 (dd, J = 11.5, 6.0 Hz, 1H), 6.93 (d, J = 8.2 Hz, 1H), 5.64 (s, 2H), 5.26 - 5.05 (m, 1H), 4.73 (dd, J = 15.7, 6.9 Hz, 1H), 4.69 - 4.34 (m, 5H), 3.69 (t, J = 6.7 Hz, 2H), 2.80 (t, J = 6.7 Hz, 3H), 2.57 - 2.42 (m, 1H).

### Example 385: (S)-2-(4-(6-((5-(3,3-difluorocyclobutyl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid

(S)-2-(4-(6-((5-(3,3-difluorocyclobutyl)-1,3,4-thiadiazol-2-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 18 using Intermediates **I-1334** and **I-8**. ES/MS m/z: 640.1 (M+H⁺). 1HNMR (400 MHz, DMSO-d6) δ 8.26 (d, J = 1.5 Hz, 1H), 8.01 - 7.86 (m, 2H), 7.79 (dd, J = 8.4, 1.5 Hz, 1H), 7.60 (dd, J = 7.7, 5.4 Hz, 2H), 7.40 (dd, J = 11.6, 6.1 Hz, 1H), 7.00 (d, J = 8.2 Hz, 1H), 5.91 (s, 2H), 5.08 (qd, J = 7.1, 2.6 Hz, 1H), 4.76 (dd, J = 15.6, 7.1 Hz, 1H), 4.63 (dd, J = 15.6, 2.8 Hz, 1H), 4.57 - 4.40 (m, 3H), 4.36 (dt, J = 9.0, 5.9 Hz, 1H), 4.02 - 3.89 (m, 1H), 3.23 - 3.05 (m, 2H), 3.03 - 2.88 (m, 2H), 2.72 (dtd, J = 11.3, 8.3, 6.4 Hz, 1H), 2.39 (ddt, J = 11.2, 8.8, 7.0 Hz, 1H).

### Example 386: 2-(2-chloro-4-(6-((4-chloro-6-(methylcarbamoyl)pyridin-3-yl)methoxy)pyridin-2-yl)-5-fluorobenzyl)-4-fluoro-1-(2-methoxyethyl)-1H-benzo[d]imidazole-6-carboxylic acid

2-(2-chloro-4-(6-((4-chloro-6-(methylcarbamoyl)pyridin-3-yl)methoxy)pyridin-2-yl)-5-fluorobenzyl)-4-fluoro-1-(2-methoxyethyl)-1H-benzo[d]imidazole-6-carboxylic acid was prepared in a manner as described in Procedure 18 using Intermediates **I-1273** and **I-1278**. ES/MS m/z: 656.7 (M+H⁺). 1H NMR (400 MHz, DMSO-d6) δ 8.85 (d, J = 5.0 Hz, 1H), 8.80 (s, 1H), 8.11 - 8.06 (m, 2H), 7.98 (d, J = 7.2 Hz, 1H), 7.91 (t, J = 7.9 Hz, 1H), 7.59 - 7.48 (m, 2H), 7.44 (d, J = 11.8 Hz, 1H), 7.02 (d, J = 8.3 Hz, 1H), 5.67 (s, 2H), 4.63 (t, J = 5.2 Hz, 2H), 4.52 (s, 2H), 3.70 (t, J = 5.0 Hz, 2H), 3.22 (s, 3H), 2.81 (d, J = 4.8 Hz, 3H).

### Example 388: (S)-2-((3'-((4-chloro-6-(methylcarbamoyl)pyridin-3-yl)methoxy)-2,4',5-trifluoro-[1,1'-biphenyl]-4-yl)methyl)-4-fluoro-1-(oxetan-2-ylmethyl)-1H-benzo[d]imidazole-6-carboxylic acid

(S)-2-((3'-((4-chloro-6-(methylcarbamoyl)pyridin-3-yl)methoxy)-2,4',5-trifluoro-[1,1'-biphenyl]-4-yl)methyl)-4-fluoro-1-(oxetan-2-ylmethyl)-1H-benzo[d]imidazole-6-carboxylic acid was prepared in a manner as described in Procedure 18 using Intermediates **I-1274** and **I-14**. ES/MS m/z: 669.8 (M+H⁺). 1H NMR (400 MHz, DMSO-d6) δ 8.91 (q, J = 4.8 Hz, 1H), 8.85 (s, 1H), 8.16 (d, J = 1.3 Hz, 1H), 8.10 (s, 1H), 7.69 - 7.58 (m, 1H), 7.58 - 7.47 (m, 2H), 7.46 - 7.34 (m, 2H), 7.32 - 7.22 (m, 1H), 5.46 (s, 2H), 5.08 (qd, J = 7.1, 2.6 Hz, 1H), 4.80 (dd, J = 15.6, 7.1 Hz, 1H), 4.66 (dd, J = 15.6, 2.7 Hz, 1H), 4.61 - 4.41 (m, 3H), 4.36 (dt, J = 8.9, 5.9 Hz, 1H), 2.83 (d, J = 4.8 Hz, 3H), 2.79 - 2.67 (m, 1H), 2.45 - 2.33 (m, 1H).

### Example 393: (R)-2-(4-(6-((4-chloro-6-(methylcarbamoyl)pyridin-3-yl)methoxy)pyridin-2-yl)-2,5-difluorobenzyl)-4-fluoro-1-(2-methoxypropyl)-1H-benzo[d]imidazole-6-carboxylic acid

(R)-2-(4-(6-((4-chloro-6-(methylcarbamoyl)pyridin-3-yl)methoxy)pyridin-2-yl)-2,5-difluorobenzyl)-4-fluoro-1-(2-methoxypropyl)-1H-benzo[d]imidazole-6-carboxylic acid was prepared in a manner as described I Procedure 18 using Intermediate **I-1273** and **I-1272.** ES/MS m/z: 654.2 (M+H⁺). 1HNMR (400 MHz, DMSO-d6) δ 8.86 (q, J = 4.9 Hz, 1H), 8.82 (s, 1H), 8.12 (d, J = 1.2 Hz, 1H), 8.08 (s, 1H), 7.91 (t, J = 7.9 Hz, 1H), 7.83 (dd, J = 10.4, 6.4 Hz, 1H), 7.62 - 7.46 (m, 2H), 7.40 (dd, J = 11.5, 6.0 Hz, 1H), 7.00 (d, J = 8.3 Hz, 1H), 5.67 (s, 2H), 4.54 (dd, J = 15.2, 3.1 Hz, 1H), 4.47 (s, 2H), 4.37 (dd, J = 15.2, 8.8 Hz, 1H), 3.68 (ddd, J = 9.2, 6.2, 3.1 Hz, 1H), 3.08 (s, 3H), 2.81 (d, J = 4.7 Hz, 3H), 1.23 (d, J = 6.1 Hz, 3H).

### Example 394: (S)-2-(4-(6-((4-chloro-6-(methylcarbamoyl)pyridin-3-yl)methoxy)pyridin-2-yl)-2-fluoro-5-methylbenzyl)-4-fluoro-1-(oxetan-2-ylmethyl)-1H-benzo [d] imidazole-6-carboxylic acid

(S)-2-(4-(6-((4-chloro-6-(methylcarbamoyl)pyridin-3-yl)methoxy)pyridin-2-yl)-2-fluoro-5-methylbenzyl)-4-fluoro-1-(oxetan-2-ylmethyl)-1H-benzo[d]imidazole-6-carboxylic acid was prepared in a manner as described in Procedure 18 using Intermediate **I-1273** and **I-1275**. ES/MS m/z: 648.2 (M+H⁺). 1H NMR (400 MHz, DMSO-d6) δ 8.86 (q, J = 4.8 Hz, 1H), 8.77 (s, 1H), 8.15 (d, J = 1.2 Hz, 1H), 8.07 (s, 1H), 7.88 (t, J = 7.8 Hz, 1H), 7.50 (dd, J = 11.4, 1.3 Hz, 1H), 7.37 - 7.23 (m, 3H), 6.97 (d, J = 8.3 Hz, 1H), 5.58 (s, 2H), 5.06 (qd, J = 7.0, 2.6 Hz, 1H), 4.78 (dd, J = 15.6, 7.1 Hz, 1H), 4.64 (dd, J = 15.6, 2.7 Hz, 1H), 4.55 - 4.31 (m, 4H), 2.82 (d, J = 4.8 Hz, 3H), 2.76 - 2.64 (m, 1H), 2.38 (ddt, J = 11.5, 9.1, 7.1 Hz, 1H), 2.28 (s, 3H).

### Example 418: (S)-2-(4-(2-((5-chlorothiophen-2-yl)methoxy)pyrimidin-4-yl)-2,5-difluorobenzyl)-1-(4,4-dimethyltetrahydrofuran-3-yl)-1H-benzo [d] imidazole-6-carboxylic acid

(S)-2-(4-(2-((5-chlorothiophen-2-yl)methoxy)pyrimidin-4-yl)-2,5-difluorobenzyl)-1-(4,4-dimethyltetrahydrofuran-3-yl)-1H-benzo[d]imidazole-6-carboxylic acid was prepared in a manner as described in Procedure 18 using Intermediates **I-1137** and **I-22**. ES/MS m/z: 611.0 (M+H⁺). 1HNMR (400 MHz, DMSO-d6) δ 8.80 (d, J = 5.2 Hz, 1H), 8.48 (s, 1H), 8.07 - 8.00 (m, 1H), 7.80 (dd, J = 8.5, 1.4 Hz, 1H), 7.66 (dd, J = 5.3, 1.8 Hz, 1H), 7.61 (d, J = 8.4 Hz, 1H), 7.56 (dd, J = 11.7, 6.0 Hz, 1H), 7.19 (d, J = 3.8 Hz, 1H), 7.07 (d, J = 3.8 Hz, 1H), 5.63 (s, 2H), 5.02 (d, J = 6.6 Hz, 1H), 4.57 - 4.52 (m, 2H), 4.49 - 4.39 (m, 2H), 3.78 (d, J = 8.7 Hz, 1H), 3.73 (d, J = 8.6 Hz, 1H), 1.34 (s, 3H), 0.61 (s, 3H).

### Example 420: (S)-2-(2-chloro-4-(6-((5-ethoxy-1,3,4-thiadiazol-2-yl)methoxy)-5-fluoropyridin-2-yl)-5-methylbenzyl)-1-(4,4-dimethyltetrahydrofuran-3-yl)-1H-benzo[d]imidazole-6-carboxylic acid

(S)-2-(2-chloro-4-(6-((5-ethoxy-1,3,4-thiadiazol-2-yl)methoxy)-5-fluoropyridin-2-yl)-5-methylbenzyl)-1-(4,4-dimethyltetrahydrofuran-3-yl)-1H-benzo[d]imidazole-6-carboxylic acid was prepared in a manner as described in Procedure 18 using Intermediates **I-1139** and **I-22**. ES/MS m/z: 652.0 (M+H⁺). 1HNMR (400 MHz, DMSO-d6) δ 8.49 (s, 1H), 7.88 (dd, J = 10.4, 8.2 Hz, 1H), 7.80 (dd, J = 8.4, 1.5 Hz, 1H), 7.63 (d, J = 8.5 Hz, 1H), 7.58 (s, 1H), 7.35 (d, J = 7.9 Hz, 2H), 5.71 (s, 2H), 4.99 (d, J = 6.8 Hz, 1H), 4.64 - 4.46 (m, 4H), 4.46 - 4.34 (m, 2H), 3.80 (d, J = 8.6 Hz, 1H), 3.72 (d, J = 8.6 Hz, 1H), 2.32 (s, 3H), 1.37 (t, J = 7.0 Hz, 3H), 1.32 (s, 3H), 0.64 (s, 3H).

### Example 421: (S)-2-(2-chloro-4-(6-((5-ethoxy-1,3,4-thiadiazol-2-yl)methoxy)-5-fluoropyridin-2-yl)-5-methylbenzyl)-1-(4,4-dimethyltetrahydrofuran-3-yl)-4-fluoro-1H-benzo[d]imidazole-6-carboxylic acid

(S)-2-(2-chloro-4-(6-((5-ethoxy-1,3,4-thiadiazol-2-yl)methoxy)-5-fluoropyridin-2-yl)-5-methylbenzyl)-1-(4,4-dimethyltetrahydrofuran-3-yl)-4-fluoro-1H-benzo[d]imidazole-6-carboxylic acid was prepared in a manner as described in Procedure 18 using Intermediates **I-1139** and **I-1031**. ES/MS m/z: 670.0 (M+H⁺). 1H NMR (400 MHz, DMSO-d6) δ 8.36 (s, 1H), 7.88 (dd, J = 10.4, 8.1 Hz, 1H), 7.59 (s, 1H), 7.52 (d, J = 11.2 Hz, 1H), 7.41 - 7.32 (m, 2H), 5.72 (s, 2H), 5.01 (d, J = 6.6 Hz, 1H), 4.62 - 4.45 (m, 4H), 4.45 - 4.35 (m, 2H), 3.79 (d, J = 8.7 Hz, 1H), 3.72 (d, J = 8.7 Hz, 1H), 2.33 (s, 3H), 1.37 (t, J = 7.0 Hz, 3H), 1.32 (s, 3H), 0.64 (s, 3H).

### Example 422: (S)-1-(4,4-dimethyltetrahydrofuran-3-yl)-2-(4-(6-((5-ethoxy-1,3,4-thiadiazol-2-yl)methoxy)-5-fluoropyridin-2-yl)-2-fluoro-5-methylbenzyl)-4-fluoro-1H-benzo [d] imidazole-6-carboxylic acid

(S)-1-(4,4-dimethyltetrahydrofuran-3-yl)-2-(4-(6-((5-ethoxy-1,3,4-thiadiazol-2-yl)methoxy)-5-fluoropyridin-2-yl)-2-fluoro-5-methylbenzyl)-4-fluoro-1H-benzo[d]imidazole-6-carboxylic acid was prepared in a manner as described in Procedure 18 using Intermediates **I-1139** and I**-1075**. ES/MS m/z: 654.0 (M+H⁺). 1HNMR (400 MHz, DMSO-d6) δ 8.35 (s, 1H), 7.88 (dd, J = 10.4, 8.1 Hz, 1H), 7.52 (d, J = 11.2 Hz, 1H), 7.41 - 7.28 (m, 3H), 5.72 (s, 2H), 5.02 (d, J = 6.6 Hz, 1H), 4.60 - 4.28 (m, 6H), 3.83 - 3.69 (m, 2H), 2.33 (s, 3H), 1.37 (t, J = 7.0 Hz, 3H), 1.30 (s, 3H), 0.59 (s, 3H).

### Example 423: (S)-1-(4,4-dimethyltetrahydrofuran-3-yl)-2-(4-(6-((5-ethoxy-1,3,4-thiadiazol-2-yl)methoxy)-5-fluoropyridin-2-yl)-2,5-difluorobenzyl)-4-fluoro-1H-benzo[d]imidazole-6-carboxylic acid

(S)-1-(4,4-dimethyltetrahydrofuran-3-yl)-2-(4-(6-((5-ethoxy-1,3,4-thiadiazol-2-yl)methoxy)-5-fluoropyridin-2-yl)-2, 5-difluorobenzyl)-4-fluoro-1H-benzo[d]imidazole-6-carboxylic acid was prepared in a manner as described in Procedure 18 using Intermediates **I-1139** and **I-105**. ES/MS m/z: 658.6 (M+H⁺). 1HNMR (400 MHz, DMSO-d6) δ 8.35 (s, 1H), 7.92 (ddd, J = 15.5, 10.3, 7.4 Hz, 2H), 7.65 - 7.59 (m, 1H), 7.52 (d, J = 11.6 Hz, 1H), 7.50 - 7.44 (m, 1H), 5.83 (s, 2H), 5.03 (d, J = 6.6 Hz, 1H), 4.59 - 4.33 (m, 6H), 3.75 (q, J = 8.7 Hz, 2H), 1.36 (t, J = 7.1 Hz, 3H), 1.33 (s, 3H), 0.60 (s, 3H).

### Example 424: (S)-1-(4,4-dimethyltetrahydrofuran-3-yl)-2-(4-(6-((5-ethoxy-1,3,4-thiadiazol-2-yl)methoxy)-5-fluoropyridin-2-yl)-2,5-difluorobenzyl)-1H-benzo[d]imidazole-6-carboxylic acid

(S)-1-(4,4-dimethyltetrahydrofuran-3-yl)-2-(4-(6-((5-ethoxy-1,3,4-thiadiazol-2-yl)methoxy)-5-fluoropyridin-2-yl)-2,5-difluorobenzyl)-1H-benzo[d]imidazole-6-carboxylic acid was prepared in a manner as described in Procedure 18 using Intermediates **I-1139** and **I-82**. ES/MS m/z: 640.6 (M+H⁺). 1HNMR (400 MHz, DMSO-d6) δ 8.49 (s, 1H), 7.99 - 7.87 (m, 2H), 7.81 (dd, J = 8.5, 1.5 Hz, 1H), 7.62 (dd, J = 9.4, 4.7 Hz, 2H), 7.48 (dd, J = 11.4, 6.2 Hz, 1H), 5.83 (s, 2H), 5.02 (d, J = 6.6 Hz, 1H), 4.57 - 4.37 (m, 6H), 3.78 (d, J = 8.7 Hz, 1H), 3.73 (d, J = 8.6 Hz, 1H), 1.37 (t, J = 7.0 Hz, 3H), 1.33 (s, 3H), 0.60 (s, 3H).

### Example 425: (S)-2-(4-(6-((5-(1,1-difluoroethyl)thiazol-2-yl)methoxy)pyridin-2-yl)-2,5-difluorobenzyl)-1-(4,4-dimethyltetrahydrofuran-3-yl)-1H-benzo[d]imidazole-6-carboxylic acid

(S)-2-(4-(6-((5-(1,1-difluoroethyl)thiazol-2-yl)methoxy)pyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 18 using Intermediates **I-1173** and **I-82**. ES/MS m/z: 640.6 (M+H⁺). 1HNMR (400 MHz, DMSO-d6) δ 8.49 (s, 1H), 8.17 (t, J = 1.8 Hz, 1H), 7.94 (t, J = 7.9 Hz, 1H), 7.87 (dd, J = 10.5, 6.4 Hz, 1H), 7.81 (dd, J = 8.4, 1.5 Hz, 1H), 7.66 - 7.55 (m, 2H), 7.46 (dd, J = 11.5, 6.1 Hz, 1H), 7.04 (d, J = 8.2 Hz, 1H), 5.82 (s, 2H), 5.01 (d, J = 6.6 Hz, 1H), 4.60 - 4.47 (m, 2H), 4.47 - 4.34 (m, 2H), 3.78 (d, J = 8.7 Hz, 1H), 3.73 (d, J = 8.6 Hz, 1H), 2.12 (t, J = 18.7 Hz, 3H), 1.33 (s, 3H), 0.60 (s, 3H).

### Example 426: (S)-2-(2-chloro-4-(6-((5-(1,1-difluoroethyl)thiazol-2-yl)methoxy)-5-fluoropyridin-2-yl)-5-methylbenzyl)-1-(4,4-dimethyltetrahydrofuran-3-yl)-4-fluoro-1H-benzo[d]imidazole-6-carboxylic acid

(S)-2-(2-chloro-4-(6-((5-(1,1-difluoroethyl)thiazol-2-yl)methoxy)-5-fluoropyridin-2-yl)-5-methylbenzyl)-1-(4,4-dimethyltetrahydrofuran-3-yl)-4-fluoro-1H-benzo[d]imidazole-6-carboxylic acid was prepared in a manner as described in Procedure 18 using Intermediates **I-1078** and **I-1031.** ES/MS m/z: 689.6 (M+H⁺). 1HNMR (400 MHz, DMSO-d6) δ 8.36 (s, 1H), 8.17 (d, J = 1.9 Hz, 1H), 7.89 (dd, J = 10.3, 8.0 Hz, 1H), 7.51 (d, J = 10.6 Hz, 2H), 7.41 - 7.27 (m, 2H), 5.82 (s, 2H), 5.01 (d, J = 6.7 Hz, 1H), 4.54 (dd, J = 19.7, 13.9 Hz, 2H), 4.46 - 4.30 (m, 2H), 3.79 (d, J = 8.6 Hz, 1H), 3.72 (d, J = 8.7 Hz, 1H), 2.25 (s, 3H), 2.13 (t, J = 18.7 Hz, 3H), 1.33 (s, 3H), 0.65 (s, 3H).

### Example 427: (S)-2-(2-chloro-4-(6-((5-(1,1-difluoroethyl)thiazol-2-yl)methoxy)-5-fluoropyridin-2-yl)-5-methylbenzyl)-1-(4,4-dimethyltetrahydrofuran-3-yl)-1H-benzo[d]imidazole-6-carboxylic acid

(S)-2-(2-chloro-4-(6-((5-(1,1-difluoroethyl)thiazol-2-yl)methoxy)-5-fluoropyridin-2-yl)-5-methylbenzyl)-1-(4,4-dimethyltetrahydrofuran-3-yl)-1H-benzo[d]imidazole-6-carboxylic acid was prepared in a manner as described in Procedure 18 using Intermediates **I-1078** and **I-1030.** ES/MS m/z: 671.6 (M+H⁺). 1HNMR (400 MHz, DMSO-d6) δ 8.49 (s, 1H), 8.17 (t, J = 1.8 Hz, 1H), 7.90 (dd, J = 10.4, 8.2 Hz, 1H), 7.80 (dd, J = 8.4, 1.4 Hz, 1H), 7.62 (d, J = 8.5 Hz, 1H), 7.52 (s, 1H), 7.35 (d, J = 5.6 Hz, 2H), 5.81 (s, 2H), 5.05 - 4.92 (m, 1H), 4.64 - 4.47 (m, 2H), 4.46 - 4.32 (m, 2H), 3.80 (d, J = 8.7 Hz, 1H), 3.72 (d, J = 8.6 Hz, 1H), 2.24 (s, 3H), 2.13 (t, J = 18.7 Hz, 3H), 1.33 (s, 3H), 0.65 (s, 3H).

### Example 428: (S)-2-(2-chloro-4-(6-((5-(1,1-difluoroethyl)thiazol-2-yl)methoxy)pyridin-2-yl)-5-methylbenzyl)-1-(4,4-dimethyltetrahydrofuran-3-yl)-1H-benzo[d]imidazole-6-carboxylic acid

(S)-2-(2-chloro-4-(6-((5-(1,1-difluoroethyl)thiazol-2-yl)methoxy)pyridin-2-yl)-5-methylbenzyl)-1-(4,4-dimethyltetrahydrofuran-3-yl)-1H-benzo[d]imidazole-6-carboxylic acid was prepared in a manner as described in Procedure 18 using Intermediates **I-1173** and **I-1030.** ES/MS m/z: 654.6 (M+H⁺). 1HNMR (400 MHz, DMSO-d6) δ 8.49 (s, 1H), 8.21 - 8.12 (m, 1H), 7.95 - 7.88 (m, 1H), 7.80 (dd, J = 8.5, 1.5 Hz, 1H), 7.63 (d, J = 8.4 Hz, 1H), 7.51 (s, 1H), 7.34 (s, 1H), 7.31 (d, J = 7.4 Hz, 1H), 7.01 (d, J = 8.2 Hz, 1H), 5.72 (s, 2H), 5.00 (d, J = 6.7 Hz, 1H), 4.60 - 4.49 (m, 2H), 4.48 - 4.34 (m, 2H), 3.80 (d, J = 8.6 Hz, 1H), 3.72 (d, J = 8.6 Hz, 1H), 2.24 (s, 3H), 2.12 (t, J = 18.6 Hz, 3H), 1.33 (s, 3H), 0.65 (s, 3H).

### Example 429: (S)-1-(4,4-dimethyltetrahydrofuran-3-yl)-4-fluoro-2-(2-fluoro-4-(5-fluoro-6-((5-methoxy-1,3,4-thiadiazol-2-yl)methoxy)pyridin-2-yl)benzyl)-1H-benzo [d] imidazole-6-carboxylic acid

(S)-1-(4,4-dimethyltetrahydrofuran-3-yl)-4-fluoro-2-(2-fluoro-4-(5-fluoro-6-((5-methoxy-1,3,4-thiadiazol-2-yl)methoxy)pyridin-2-yl)benzyl)-1H-benzo[d]imidazole-6-carboxylic acid was prepared in a manner as described in Procedure 18 using Intermediates **I-1244** and **I-1230**. ES/MS m/z: 625.6 (M+H⁺). 1HNMR (400 MHz, DMSO-d6) δ 8.35 (s, 1H), 8.07 - 7.93 (m, 2H), 7.88 (dd, J = 10.2, 8.2 Hz, 1H), 7.78 (dd, J = 8.3, 2.7 Hz, 1H), 7.56 - 7.47 (m, 2H), 5.85 (s, 2H), 5.01 (d, J = 6.6 Hz, 1H), 4.51 (dd, J = 14.1, 8.0 Hz, 2H), 4.47 - 4.39 (m, 2H), 4.11 (s, 3H), 3.83 - 3.70 (m, 2H), 1.30 (s, 3H), 0.58 (s, 3H).

### Example 430: (S)-1-(4,4-dimethyltetrahydrofuran-3-yl)-2-(4-(6-((5-ethoxy-1,3,4-thiadiazol-2-yl)methoxy)pyridin-2-yl)-2,5-difluorobenzyl)-1H-benzo[d]imidazole-6-carboxylic acid

(S)-1-(4,4-dimethyltetrahydrofuran-3-yl)-2-(4-(6-((5-ethoxy-1,3,4-thiadiazol-2-yl)methoxy)pyridin-2-yl)-2,5-difluorobenzyl)-1H-benzo[d]imidazole-6-carboxylic acid was prepared in a manner as described in Procedure 18 using Intermediates **I-1145** and **I-82**. ES/MS m/z: 621.6 (M+H⁺). 1HNMR (400 MHz, DMSO-d6) δ 8.50 (s, 1H), 8.00 - 7.88 (m, 2H), 7.82 (dd, J = 8.4, 1.4 Hz, 1H), 7.64 (d, J = 8.5 Hz, 1H), 7.60 (dd, J = 7.6, 1.6 Hz, 1H), 7.48 (dd, J = 11.5, 6.1 Hz, 1H), 6.99 (d, J = 8.3 Hz, 1H), 5.73 (s, 2H), 5.02 (d, J = 6.7 Hz, 1H), 4.64 - 4.38 (m, 6H), 3.85 - 3.66 (m, 2H), 1.36 (t, J = 7.1 Hz, 3H), 1.33 (s, 3H), 0.60 (s, 3H).

### Example 431: (S)-2-(4-(6-((5-cyclopropyl-1,3,4-thiadiazol-2-yl)methoxy)pyridin-2-yl)-2,5-difluorobenzyl)-1-(4,4-dimethyltetrahydrofuran-3-yl)-1H-benzo[d]imidazole-6-carboxylic acid

(S)-2-(4-(6-((5-cyclopropyl-1,3,4-thiadiazol-2-yl)methoxy)pyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 18 using Intermediates **I-1146** and **I-82**. ES/MS m/z: 617.6 (M+H⁺). 1HNMR (400 MHz, DMSO-d6) δ 8.50 (s, 1H), 7.97 - 7.89 (m, 2H), 7.82 (dd, J = 8.5, 1.4 Hz, 1H), 7.64 (d, J = 8.5 Hz, 1H), 7.62 - 7.58 (m, 1H), 7.48 (dd, J = 11.5, 6.1 Hz, 1H), 7.00 (d, J = 8.3 Hz, 1H), 5.85 (s, 2H), 5.03 (d, J = 6.7 Hz, 1H), 4.57 (d, J = 7.4 Hz, 1H), 4.53 (s, 1H), 4.48 - 4.35 (m, 2H), 3.83 - 3.66 (m, 2H), 2.50 - 2.46 (m, 1H), 1.33 (s, 3H), 1.23 - 1.13 (m, 2H), 1.03 - 0.94 (m, 2H), 0.61 (s, 3H).

### Example 432: (S)-2-(4-(6-((5-(difluoromethyl)-1,3,4-thiadiazol-2-yl)methoxy)pyridin-2-yl)-2,5-difluorobenzyl)-1-(4,4-dimethyltetrahydrofuran-3-yl)-1H-benzo[d]imidazole-6-carboxylic acid

(S)-2-(4-(6-((5-(difluoromethyl)-1,3,4-thiadiazol-2-yl)methoxy)pyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 18 using Intermediates **I-1147** and **I-82**. ES/MS m/z: 627.6 (M+H⁺). 1HNMR (400 MHz, DMSO-d6) δ 8.50 (s, 1H), 8.01 - 7.88 (m, 2H), 7.82 (d, J = 8.5 Hz, 1H), 7.73 - 7.34 (m, 4H), 7.05 (d, J = 8.3 Hz, 1H), 6.02 (s, 2H), 5.02 (d, J = 6.6 Hz, 1H), 4.56 (d, J = 7.9 Hz, 1H), 4.53 (s, 1H), 4.49 - 4.37 (m, 2H), 3.83 - 3.68 (m, 2H), 1.33 (s, 3H), 0.60 (s, 3H).

### Example 433: (S)-2-(2,5-difluoro-4-(6-((5-(trifluoromethyl)-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid

(S)-2-(2,5-difluoro-4-(6-((5-(trifluoromethyl)-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 18 using Intermediates **I-1148** and **I-82**. ES/MS m/z: 645.6 (M+H⁺). 1HNMR (400 MHz, DMSO-d6) δ 8.49 (s, 1H), 7.97 (t, J = 7.9 Hz, 1H), 7.88 (dd, J = 10.4, 6.5 Hz, 1H), 7.81 (dd, J = 8.4, 1.5 Hz, 1H), 7.62 (dd, J = 7.5, 3.4 Hz, 2H), 7.47 (dd, J = 11.4, 6.1 Hz, 1H), 7.07 (d, J = 8.3 Hz, 1H), 6.06 (s, 2H), 5.01 (d, J = 6.6 Hz, 1H), 4.59 - 4.50 (m, 2H), 4.48 - 4.36 (m, 2H), 3.81 - 3.71 (m, 2H), 1.33 (s, 3H), 0.60 (s, 3H).

### Example 434: (S)-2-(2-chloro-5-fluoro-4-(6-((5-methoxy-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid

(S)-2-(2-chloro-5-fluoro-4-(6-((5-methoxy-1,3,4-thiadiazol-2-yl)methoxy)pyridin-2-yl)benzyl)-1-(4,4-dimethyltetrahydrofuran-3-yl)-1H-benzo[d]imidazole-6-carboxylic acid was prepared in a manner as described in Procedure 18 using Intermediates **I-1090** and **I-1132.** ES/MS m/z: 624.0 (M+H⁺). 1HNMR (400 MHz, DMSO-d6) δ 8.50 (s, 1H), 8.18 (d, J = 7.1 Hz, 1H), 7.94 (t, J = 7.9 Hz, 1H), 7.80 (dd, J = 8.4, 1.5 Hz, 1H), 7.64 - 7.56 (m, 2H), 7.51 (d, J = 11.8 Hz, 1H), 7.01 (d, J = 8.3 Hz, 1H), 5.73 (s, 2H), 5.01 (d, J = 6.7 Hz, 1H), 4.63 (d, J = 17.0 Hz, 1H), 4.54 (d, J = 11.0 Hz, 1H), 4.51 - 4.39 (m, 2H), 4.11 (s, 3H), 3.80 (d, J = 8.6 Hz, 1H), 3.73 (d, J = 8.6 Hz, 1H), 1.35 (s, 3H), 0.65 (s, 3H).

### Example 435: (S)-2-(4-(6-((2-(ethoxycarbonyl)isoindolin-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((2-(ethoxycarbonyl)isoindolin-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 18 using Intermediates **I-1131** and **I-8.** ES/MS m/z: 655.2 (M+H⁺). 1HNMR (400 MHz, DMSO-d6) δ 8.26 (d, J = 1.5 Hz, 1H), 7.99 - 7.75 (m, 3H), 7.64 - 7.56 (m, 1H), 7.53 - 7.30 (m, 5H), 6.96 (dd, J = 19.7, 8.3 Hz, 1H), 5.78 - 5.33 (m, 2H), 5.08 (d, J = 6.8 Hz, 1H), 4.76 (dd, J = 15.6, 7.1 Hz, 1H), 4.63 (d, J = 11.3 Hz, 5H), 4.59 - 4.42 (m, 3H), 4.36 (dt, J = 9.0, 5.9 Hz, 1H), 4.17 - 4.04 (m, 2H), 2.78 - 2.69 (m, 1H), 2.41 (q, J = 9.8, 8.7 Hz, 1H), 1.23 (td, J = 7.1, 2.9 Hz, 3H).

### Example 436: (S)-1-(4,4-dimethyltetrahydrofuran-3-yl)-4-fluoro-2-(2-fluoro-4-(5-fluoro-6-((5-(trifluoromethyl)thiazol-2-yl)methoxy)pyridin-2-yl)-5-methylbenzyl)-1H-benzo[d]imidazole-6-carboxylic acid

(S)-1-(4,4-dimethyltetrahydrofuran-3-yl)-4-fluoro-2-(2-fluoro-4-(5-fluoro-6-((5-(trifluoromethyl)thiazol-2-yl)methoxy)pyridin-2-yl)-5-methylbenzyl)-1H-benzo[d]imidazole-6-carboxylic acid was prepared in a manner as described in Procedure 18 using Intermediates **I-1130** and **I-1075**. ES/MS m/z: 677.1 (M+H⁺). 1HNMR (400 MHz, DMSO-d6) δ 8.47 (d, J = 1.4 Hz, 1H), 8.35 (s, 1H), 7.90 (dd, J = 10.4, 8.1 Hz, 1H), 7.52 (dd, J = 11.2, 1.2 Hz, 1H), 7.35 (dd, J = 8.2, 2.9 Hz, 1H), 7.30 (dd, J = 9.2, 6.6 Hz, 2H), 5.87 (s, 2H), 5.01 (d, J = 6.6 Hz, 1H), 4.54 - 4.37 (m, 3H), 4.34 (d, J = 16.9 Hz, 1H), 3.80 - 3.74 (m, 1H), 3.72 (d, J = 8.6 Hz, 1H), 2.22 (s, 3H), 1.30 (s, 3H), 0.59 (s, 3H).

### Example 437: 2-(2,5-difluoro-4-(6-((5-(trifluoromethyl)thiazol-2-yl)methoxy)pyridin-2-yl)benzyl)-1-(4,4-dimethyltetrahydrofuran-3-yl)-4-fluoro-1H-benzo[d]imidazole-6-carboxylic acid

2-(2,5-difluoro-4-(6-((5-(trifluoromethyl)thiazol-2-yl)methoxy)pyridin-2-yl)benzyl)-1-(4,4-dimethyltetrahydrofuran-3-yl)-4-fluoro-1H-benzo[d]imidazole-6-carboxylic acid was prepared in a manner as described in Procedure 18 using Intermediates **I-107** and **I-1336**. ES/MS m/z: 663.0 (M+H⁺). 1HNMR (400 MHz, DMSO-d6) δ 8.47 (d, J = 1.4 Hz, 1H), 8.35 (s, 1H), 7.96 (t, J = 7.9 Hz, 1H), 7.85 (dd, J = 10.5, 6.4 Hz, 1H), 7.61 (dd, J = 7.5, 1.5 Hz, 1H), 7.52 (dd, J = 11.2, 1.2 Hz, 1H), 7.47 (dd, J = 11.5, 6.1 Hz, 1H), 7.06 (d, J = 8.3 Hz, 1H), 5.88 (s, 2H), 5.03 (d, J = 6.6 Hz, 1H), 4.60 - 4.49 (m, 2H), 4.47 - 4.36 (m, 2H), 3.74 (q, J = 8.7 Hz, 2H), 1.33 (s, 3H), 0.60 (s, 3H).

### Example 439: (S)-2-(2,6-difluoro-4-(6-((5-methoxy-1,3,4-thiadiazol-2-yl)methoxy)pyridin-2-yl)benzyl)-1-(4,4-dimethyltetrahydrofuran-3-yl)-4-fluoro-1H-benzo[d]imidazole-6-carboxylic acid

(S)-2-(2,6-difluoro-4-(6-((5-methoxy-1,3,4-thiadiazol-2-yl)methoxy)pyridin-2-yl)benzyl)-1-(4,4-dimethyltetrahydrofuran-3-yl)-4-fluoro-1H-benzo[d]imidazole-6-carboxylic acid was prepared in a manner as described in Procedure 18 using Intermediates **I-1234** and **I-1090.** ES/MS m/z: 626.2 (M+H⁺). 1H NMR (400 MHz, DMSO) δ 8.23 - 8.08 (m, 2H), 7.94 (dd, J = 16.6, 8.5 Hz, 4H), 7.83 (d, J = 7.3 Hz, 1H), 7.45 (d, J = 11.6 Hz, 1H), 7.08 - 6.88 (m, 1H), 5.77 (d, J = 7.9 Hz, 3H), 5.06 (s, 1H), 4.59 - 4.37 (m, 3H), 4.10 (d, J = 1.4 Hz, 5H), 3.98 - 3.62 (m, 2H), 1.36 (s, 3H), 0.65 (s, 3H).

### Example 440: (S)-2-(4-(6-((4-cyano-2-fluorobenzyl)oxy)pyridin-2-yl)-2-fluorobenzyl)-1-(4,4-dimethyltetrahydrofuran-3-yl)-4-fluoro-1H-benzo[d]imidazole-6-carboxylic acid

(S)-2-(4-(6-((4-cyano-2-fluorobenzyl)oxy)pyridin-2-yl)-2-fluorobenzyl)-1-(4,4-dimethyltetrahydrofuran-3-yl)-4-fluoro-1H-benzo[d]imidazole-6-carboxylic acid was prepared in a manner as described in Procedure 18 using Intermediates **I-1230** and **I-3.** ES/MS m/z: 613.6 (M+H⁺). 1H NMR (400 MHz, DMSO) δ 8.35 (s, 1H), 7.99 - 7.81 (m, 4H), 7.81 - 7.62 (m, 3H), 7.58 - 7.38 (m, 2H), 6.94 (d, J = 8.2 Hz, 1H), 5.63 (s, 2H), 5.00 (d, J = 6.5 Hz, 1H), 4.61 - 4.37 (m, 4H), 1.30 (s, 3H), 0.58 (s, 3H).

### Example 441: (S)-2-(2-chloro-4-(5-fluoro-6-((5-methoxy-1,3,4-thiadiazol-2-yl)methoxy)pyridin-2-yl)-5-methylbenzyl)-1-(4,4-dimethyltetrahydrofuran-3-yl)-1H-benzo[d]imidazole-6-carboxylic acid

(S)-2-(2-chloro-4-(5-fluoro-6-((5-methoxy-1,3,4-thiadiazol-2-yl)methoxy)pyridin-2-yl)-5-methylbenzyl)-1-(4,4-dimethyltetrahydrofuran-3-yl)-1H-benzo[d]imidazole-6-carboxylic acid was prepared in a manner as described in Procedure 18 using Intermediates **I-1030** and **I-1244**. ES/MS m/z: 638.4 (M+H⁺). 1H NMR (400 MHz, DMSO) δ 8.32 (s, 1H), 7.93 - 7.81 (m, 1H), 7.74 (d, J = 8.4 Hz, 1H), 7.57 (s, 1H), 7.39 - 7.24 (m, 1H), 5.72 (s, 2H), 4.90 (s, 1H), 4.62 - 4.23 (m, 4H), 4.11 (s, 3H), 3.83 (d, J = 8.5 Hz, 1H), 3.70 (d, J = 8.5 Hz, 1H), 1.29 (s, 3H), 0.64 (s, 3H).

### Example 442: (S)-2-(2-chloro-4-(6-((5-methoxy-1,3,4-thiadiazol-2-yl)methoxy)pyridin-2-yl)-5-methylbenzyl)-1-(4,4-dimethyltetrahydrofuran-3-yl)-1H-benzo[d]imidazole-6-carboxylic acid

(S)-2-(2-chloro-4-(6-((5-methoxy-1,3,4-thiadiazol-2-yl)methoxy)pyridin-2-yl)-5-methylbenzyl)-1-(4,4-dimethyltetrahydrofuran-3-yl)-1H-benzo[d]imidazole-6-carboxylic acid was prepared in a manner as described in Procedure 18 using Intermediates **I-1030** and **I-1090**. ES/MS m/z: 620.4 (M+H⁺). 1H NMR (400 MHz, DMSO) δ 8.53 (s, 1H), 7.95 - 7.80 (m, 2H), 7.66 (d, J = 8.5 Hz, 1H), 7.63 - 7.56 (m, 1H), 7.39 (d, J = 7.6 Hz, 1H), 7.31 (dd, J = 7.4, 3.1 Hz, 1H), 6.96 (dd, J = 8.3, 2.9 Hz, 1H), 5.63 (s, 2H), 5.03 (d, J = 6.7 Hz, 1H), 4.72 - 4.37 (m, 4H), 4.24 (d, J = 19.6 Hz, 1H), 4.10 (s, 2H), 3.85 - 3.69 (m, 2H), 2.34 (s, 3H), 1.32 (d, J = 2.3 Hz, 3H), 0.65 (d, J = 2.0 Hz, 3H).

### Example 443: (S)-2-(2-chloro-4-(5-fluoro-6-((5-methoxy-1,3,4-thiadiazol-2-yl)methoxy)pyridin-2-yl)-5-methylbenzyl)-4-fluoro-1-(oxetan-2-ylmethyl)-1H-benzo[d]imidazole-6-carboxylic acid

(S)-2-(2-chloro-4-(5-fluoro-6-((5-methoxy-1,3,4-thiadiazol-2-yl)methoxy)pyridin-2-yl)-5-methylbenzyl)-4-fluoro-1-(oxetan-2-ylmethyl)-1H-benzo[d]imidazole-6-carboxylic acid was prepared in a manner as described in Procedure 18, starting with Intermediates **I-1031** and **I-1244.** 1H NMR (400 MHz, MeOD) δ 8.08 (d, J = 1.2 Hz, 1H), 7.74 - 7.61 (m, 2H), 7.55 (s, 1H), 7.21 (dd, J = 8.1, 2.9 Hz, 1H), 7.15 (s, 1H), 5.70 (s, 2H), 5.12 (dd, J = 7.3, 2.6 Hz, 1H), 4.73 - 4.39 (m, 7H), 4.16 (s, 3H), 2.87 - 2.64 (m, 1H), 2.48 (ddt, J = 11.5, 9.1, 7.1 Hz, 1H), 2.30 (s, 3H). ES/MS m/z: 628.2 (M+H⁺).

### Example 444: (S)-2-((3'-((4-chloro-6-(methylcarbamoyl)pyridin-3-yl)methoxy)-2,5-difluoro-[1,1'-biphenyl]-4-yl)methyl)-1-(oxetan-2-ylmethyl)-1H-benzo[d]imidazole-6-carboxylic acid

(S)-2-((3'-((4-chloro-6-(methylcarbamoyl)pyridin-3-yl)methoxy)-2,5-difluoro-[1,1'-biphenyl]-4-yl)methyl)-1-(oxetan-2-ylmethyl)-1H-benzo[d]imidazole-6-carboxylic acid was prepared in a manner as described in Procedure 18, starting with Intermediates **I-1343** and **I-8**. 1H NMR (400 MHz, Methanol-d4) δ 8.80 (s, 1H), 8.59 (dd, J = 1.5, 0.7 Hz, 1H), 8.23 (dd, J = 8.6, 1.4 Hz, 1H), 8.16 (s, 1H), 7.79 (dd, J = 8.6, 0.6 Hz, 1H), 7.53 - 7.42 (m, 2H), 7.38 (dd, J = 10.3, 6.2 Hz, 1H), 7.25 (dt, J = 7.5, 1.6 Hz, 2H), 7.16 (ddd, J = 8.3, 2.4, 1.1 Hz, 1H), 5.37 (s, 2H), 5.27 (qd, J = 7.5, 2.5 Hz, 1H), 5.01 (dd, J = 15.5, 7.6 Hz, 2H), 4.87 - 4.65 (m, 3H), 4.55 (dt, J = 9.2, 6.0 Hz, 1H), 2.98 (s, 3H), 2.88 (dtd, J = 11.5, 8.2, 6.1 Hz, 1H), 2.59 (ddt, J = 11.5, 9.1, 7.1 Hz, 1H). ES/MS m/z: 633.2 (M+H⁺).

### Example 445: (S)-4-chloro-2-(2,5-difluoro-4-(6-((5-(trifluoromethyl)thiazol-2-yl)methoxy)pyridin-2-yl)benzyl)-1-(oxetan-2-ylmethyl)-1H-benzo [d] imidazole-6-carboxylic acid

(S)-4-chloro-2-(2,5-difluoro-4-(6-((5-(trifluoromethyl)thiazol-2-yl)methoxy)pyridin-2-yl)benzyl)-1-(oxetan-2-ylmethyl)-1H-benzo[d]imidazole-6-carboxylic acid was prepared in a manner as described in Procedure 18, starting with Intermediates **I-1150** and **I-107.** 1H NMR (400 MHz, DMSO-d6) δ 8.54 - 8.42 (m, 1H), 8.27 (d, J = 1.4 Hz, 1H), 7.95 (t, J = 7.9 Hz, 1H), 7.84 (dd, J = 10.5, 6.4 Hz, 1H), 7.79 (d, J = 1.3 Hz, 1H), 7.60 (d, J = 7.4 Hz, 1H), 7.40 (dd, J = 11.5, 6.0 Hz, 1H), 7.06 (d, J = 8.3 Hz, 1H), 5.88 (s, 2H), 5.05 (d, J = 7.2 Hz, 1H), 4.79 (dd, J = 15.5, 7.1 Hz, 1H), 4.66 (d, J = 15.6 Hz, 1H), 4.57 (d, J = 16.8 Hz, 1H), 4.54 - 4.44 (m, 2H), 4.41 - 4.28 (m, 1H), 2.72 (dd, J = 18.2, 9.2 Hz, 1H), 2.44 - 2.28 (m, 1H). ES/MS m/z: 651.0 (M+H⁺).

### Example 446: (S)-1-(oxetan-2-ylmethyl)-2-(2,3,6-trifluoro-4-(5-fluoro-6-((5-(trifluoromethyl)thiazol-2-yl)methoxy)pyridin-2-yl)benzyl)-1H-benzo[d]imidazole-6-carboxylic acid

(S)-1-(oxetan-2-ylmethyl)-2-(2,3,6-trifluoro-4-(5-fluoro-6-((5-(trifluoromethyl)thiazol-2-yl)methoxy)pyridin-2-yl)benzyl)-1H-benzo[d]imidazole-6-carboxylic acid was prepared in a manner as described in Procedure 18, starting with Intermediates **I-1345** and **I-1344.** 1H NMR (400 MHz, MeOD) δ 8.23 (dd, J = 23.7, 1.4 Hz, 2H), 7.95 (dd, J = 8.4, 1.5 Hz, 1H), 7.75 (dd, J = 9.6, 8.2 Hz, 1H), 7.70 - 7.60 (m, 2H), 7.54 (d, J = 8.4 Hz, 1H), 5.93 (s, 2H), 5.29 (d, J = 7.1 Hz, 1H), 4.82 - 4.40 (m, 6H), 2.94 - 2.74 (m, 1H), 2.55 (q, J = 9.7, 8.5 Hz, 1H). ES/MS m/z: 653.12 (M+H⁺).

### Example 447: (S)-4-fluoro-1-(oxetan-2-ylmethyl)-2-(2,3,6-trifluoro-4-(5-fluoro-6-((5-(trifluoromethyl)thiazol-2-yl)methoxy)pyridin-2-yl)benzyl)-1H-benzo[d]imidazole-6-carboxylic acid

(S)-4-fluoro-1-(oxetan-2-ylmethyl)-2-(2,3,6-trifluoro-4-(5-fluoro-6-((5-(trifluoromethyl)thiazol-2-yl)methoxy)pyridin-2-yl)benzyl)-1H-benzo[d]imidazole-6-carboxylic acid was prepared in a manner as described in Procedure 18, starting with Intermediates **I-1345** and **I-1235.** 1H NMR (400 MHz, MeOD) δ 8.25 (s, 1H), 8.05 (s, 1H), 7.78 - 7.69 (m, 1H), 7.69 - 7.54 (m, 3H), 5.92 (s, 2H), 5.26 (tt, J = 7.0, 3.5 Hz, 1H), 4.80 - 4.61 (m, 6H), 4.55 (d, J = 17.0 Hz, 1H), 4.51 - 4.36 (m, 1H), 2.90 - 2.77 (m, 1H), 2.59 - 2.44 (m, 1H). ES/MS m/z: 671.1 (M+H⁺).

### Example 448: (S)-1-(oxetan-2-ylmethyl)-2-(2,3,6-trifluoro-4-(6-((5-(trifluoromethyl)thiazol-2-yl)methoxy)pyridin-2-yl)benzyl)-1H-benzo[d]imidazole-6-carboxylic acid

(S)-1-(oxetan-2-ylmethyl)-2-(2,3,6-trifluoro-4-(6-((5-(trifluoromethyl)thiazol-2-yl)methoxy)pyridin-2-yl)benzyl)-1H-benzo[d]imidazole-6-carboxylic acid was prepared in a manner as described in Procedure 18, starting with Intermediates **I-107** and **I-1344.** 1H NMR (400 MHz, MeOD) δ 8.27 - 8.13 (m, 2H), 7.99 - 7.84 (m, 2H), 7.73 - 7.61 (m, 2H), 7.53 (d, J = 8.4 Hz, 1H), 7.03 (d, J = 8.2 Hz, 1H), 5.85 (s, 2H), 5.28 (tt, J = 6.7, 4.2 Hz, 1H), 4.81 - 4.38 (m, 6H), 2.83 (ddt, J = 16.0, 14.1, 8.1 Hz, 1H), 2.61 - 2.45 (m, 1H). ES/MS m/z: 635.1 (M+H⁺).

### Example 449: (S)-4-fluoro-1-(oxetan-2-ylmethyl)-2-(2,3,6-trifluoro-4-(6-((5-(trifluoromethyl)thiazol-2-yl)methoxy)pyridin-2-yl)benzyl)-1H-benzo[d]imidazole-6-carboxylic acid

(S)-4-fluoro-1-(oxetan-2-ylmethyl)-2-(2,3,6-trifluoro-4-(6-((5-(trifluoromethyl)thiazol-2-yl)methoxy)pyridin-2-yl)benzyl)-1H-benzo[d]imidazole-6-carboxylic acid was prepared in a manner as described in Procedure 18, starting with Intermediates **I-107** and **I-1235.** 1H NMR (400 MHz, MeOD) δ 8.23 (d, J = 1.4 Hz, 1H), 7.97 (d, J = 1.3 Hz, 1H), 7.92 (t, J = 7.9 Hz, 1H), 7.69 - 7.58 (m, 3H), 7.03 (d, J = 8.3 Hz, 1H), 5.86 (s, 2H), 5.28 (q, J = 7.1, 6.3 Hz, 1H), 4.81 - 4.50 (m, 5H), 2.82 (q, J = 8.4, 7.4 Hz, 1H), 2.53 (dt, J = 17.7, 7.8 Hz, 1H). ES/MS m/z: 653.0 (M+H⁺).

### Example 231: 2-[[2,5-difluoro-4-[6-[[2-fluoro-4-[2-(oxetan-3-yl)ethynyl]phenyl]methoxy]-2-pyridyl]phenyl]methyl]-3-(2-methoxyethyl)benzimidazole-5-carboxylic acid

### Procedure 19

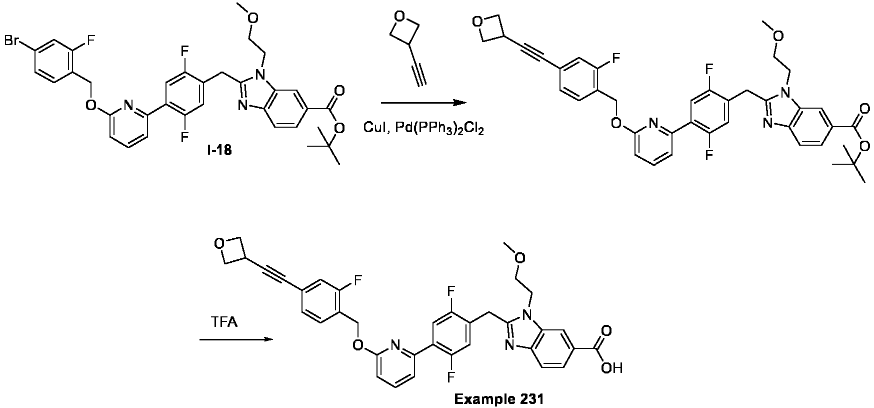

**Tert-butyl 2-**[**[2,5-difluoro-4-[6-**[**[2-fluoro-4-[2-(oxetan-3-yl)ethynyl]phenyl]methoxy]-2-pyridyl]phenyl]methyl]-3-(2-methoxyethyl)benzimidazole-5-carboxylate:** To a suspension of tert-butyl 2-[[4-[6-[(4-bromo-2-fluoro-phenyl)methoxy]-2-pyridyl]-2,5-difluoro-phenyl]methyl]-3-(2-methoxyethyl)benzimidazole-5-carboxylate **I-18** (40 mg, 0.059 mmol), 3-ethynyloxetane (16 mg, 0.19 mmol), cuprous iodide (5.3 mg, 0.028 mmol), and bis(triphenylphosphine)palladium chloride (10 mg, 0.015 mmol) in DMF (1.6 mL) was added diisopropylamine (0.20 mL, 1.2 mmol). The resulting solution was degassed by bubbling argon for 1 min, sealed and heated to 100 °C for 3 hours. Upon completion the mixture was poured into water (5 mL) and extracted with EtOAc (2 x 5 mL). The organic layers were combined, washed with brine (5 mL), dried over MgSO₄, filtered, concentrated, and purified by flash chromatography (Eluent: EtOAc/hexane) to give the desired product. ES/MS: 684.2 (M+1).

**2-[[2,5-difluoro-4-[6-[[2-fluoro-4-[2-(oxetan-3-yl)ethynyl]phenyl]methoxy]-2-pyridyl]phenyl]methyl]-3-(2-methoxyethyl)benzimidazole-5-carboxylic acid (Example 231):** To a solution of tert-butyl 2-[[2,5-difluoro-4-[6-[[2-fluoro-4-[2-(oxetan-3-yl)ethynyl]phenyl]methoxy]-2-pyridyl]phenyl]methyl]-3-(2-methoxyethyl)benzimidazole-5-carboxylate (44 mg, 0.064 mmol) in DCM (2 mL), 0.25 mL TFA was added and the resulting solution was stirred at 40 °C for 1 hr. The reaction mixture was then concentrated directly and purified by RP-HPLC (eluent: water / MeCN 0.1% TFA). The combined fractions were then frozen and placed on a lyophilizer to provide **Example 231.** ES/MS: 628.3 (M+H⁺). 1H NMR (400 MHz, DMSO-d6) δ 8.30 (d, J = 1.5 Hz, 1H), 7.94 - 7.77 (m, 3H), 7.66 (d, J = 8.5 Hz, 1H), 7.56 (t, J = 7.8 Hz, 1H), 7.51 (dd, J = 7.6, 1.7 Hz, 1H), 7.42 (dd, J = 11.5, 6.1 Hz, 1H), 7.39 - 7.27 (m, 2H), 6.96 (d, J = 8.2 Hz, 1H), 5.52 (s, 2H), 4.80 (dd, J = 8.5, 5.4 Hz, 2H), 4.66 (t, J = 5.1 Hz, 2H), 4.61 (dd, J = 7.1, 5.4 Hz, 2H), 4.52 (s, 2H), 4.16 (tt, J = 8.5, 7.1 Hz, 1H), 3.70 (t, J = 5.0 Hz, 2H), 3.22 (s, 3H).

### Example 232: 2-(4-(6-((4-(azetidin-3-ylethynyl)-2-fluorobenzyl)oxy)pyridin-2-yl)-2,5-difluorobenzyl)-1-(2-methoxyethyl)-1H-benzo[d]imidazole-6-carboxylic acid

2-(4-(6-((4-(azetidin-3-ylethynyl)-2-fluorobenzyl)oxy)pyridin-2-yl)-2,5-difluorobenzyl)-1-(2-methoxyethyl)-1H-benzo[d]imidazole-6-carboxylic acid was prepared in a manner as described in Procedure 19. Multiplet Report 1H NMR (400 MHz, DMSO-d6) δ 8.25 (s, 1H), 7.89 (t, J = 8.0 Hz, 1H), 7.82 (d, J = 9.0 Hz, 2H), 7.68 - 7.56 (m, 2H), 7.52 (d, J = 7.6 Hz, 1H), 7.37 (td, J = 17.7, 6.9 Hz, 3H), 6.96 (d, J = 8.3 Hz, 1H), 5.53 (s, 2H), 4.61 (d, J = 5.4 Hz, 2H), 4.47 (s, 2H), 4.23 (t, J = 7.3 Hz, 2H), 4.10 - 3.92 (m, 3H), 3.69 (q, J = 4.4, 3.8 Hz, 2H), 3.22 (t, J = 1.9 Hz, 3H).

### Example 233: 2-(2,5-difluoro-4-(6-((2-fluoro-4-((1-methyl-1H-imidazol-5-yl)ethynyl)benzyl)oxy)pyridin-2-yl)benzyl)-1-(2-methoxyethyl)-1H-benzo[d]imidazole-6-carboxylic acid

2-(2,5-difluoro-4-(6-((2-fluoro-4-((1-methyl-1H-imidazol-5-yl)ethynyl)benzyl)oxy)pyridin-2-yl)benzyl)-1-(2-methoxyethyl)-1H-benzo[d]imidazole-6-carboxylic acid was prepared in a manner as described in Procedure 19. Multiplet Report 1HNMR (400 MHz, DMSO-d6) δ 8.75 (s, 1H), 8.24 (d, J = 1.5 Hz, 1H), 8.01 - 7.85 (m, 2H), 7.85 - 7.75 (m, 2H), 7.73 - 7.56 (m, 3H), 7.51 (ddd, J = 14.7, 7.7, 1.7 Hz, 2H), 7.40 (dd, J = 11.5, 6.1 Hz, 1H), 6.98 (d, J = 8.3 Hz, 1H), 5.57 (s, 2H), 4.61 (t, J = 5.1 Hz, 2H), 4.47 (s, 2H), 3.85 (s, 3H), 3.69 (t, J = 5.0 Hz, 2H), 3.21 (s, 3H).

### Example 234: 2-[[2,5-difluoro-4-[6-[[2-fluoro-4-[2-(1-methylazetidin-3-yl)ethynyl]phenyl]methoxy]-2-pyridyl]phenyl]methyl]-3-(2-methoxyethyl)benzimidazole-5-carboxylic acid

### Procedure 20

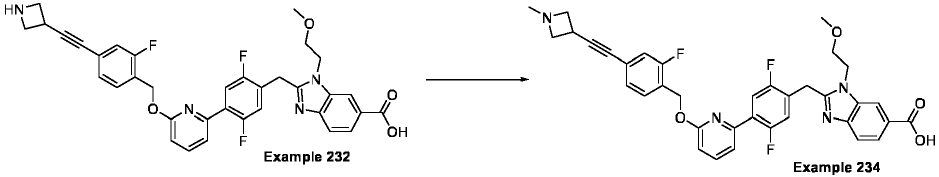

**2-[[2,5-difluoro-4-[6-[[2-fluoro-4-[2-(1-methylazetidin-3-yl)ethynyl]phenyl]methoxy]-2-pyridyl]phenyl]methyl]-3-(2-methoxyethyl)benzimidazole-5-carboxylic acid (Example 234):** To a solution of 2-[[4-[6-[[4-[2-(azetidin-3-yl)ethynyl]-2-fluoro-phenyl]methoxy]-2-pyridyl]-2, 5-difluoro-phenyl]methyl]-3-(2-methoxyethyl)benzimidazole-5-carboxylic acid (**Example 232**) (20 mg, 0.032 mmol) in MeOH (2 mL), 37% aqueous formaldehyde (0.5 mL) was added. The resultant solution was stirred at rt for 1 hr at which point sodium triacetoxyborohydride (34 mg, 0.16 mmol) was added as a single portion. The resultant mixture was stirred at rt for 1 hr, then concentrated directly and purified by RP-HPLC (eluent: water / MeCN 0.1% TFA). The combined fractions were then frozen and placed on a lyophilizer to provide **Example 234.** ES/MS: 641.3 (M+H⁺). 1H NMR (400 MHz, DMSO-d6) δ 7.68 (s, 1H), 7.36 (d, J = 8.8 Hz, 1H), 7.19 - 6.85 (m, 3H), 6.76 (d, J = 8.6 Hz, 2H), 6.48 (q, J = 12.6 Hz, 3H), 6.10 (d, J = 8.4 Hz, 1H), 4.75 (s, 2H), 3.95 (s, 2H), 3.89 (s, 2H), 3.78 (s, 1H), 3.55 (s, 3H), 3.36 (s, 1H), 3.17 (s, 1H), 3.01 (s, 2H), 2.19 (s, 3H).

### Example 235: 2-[[2,5-difluoro-4-[6-[[2-fluoro-4-[2-(1-methoxycarbonylazetidin-3-yl)ethynyl]phenyl]methoxy]-2-pyridyl]phenyl]methyl]-3-(2-methoxyethyl)benzimidazole-5-carboxylic acid

### Procedure 21

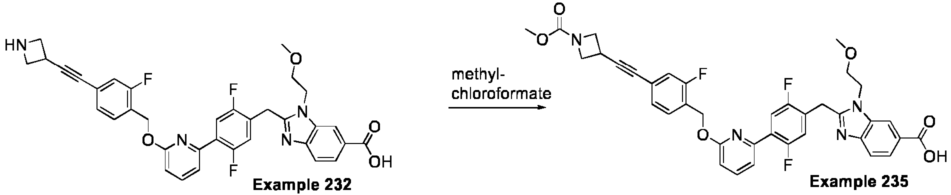

**2-[[2,5-difluoro-4-[6-[[2-fluoro-4-[2-(1-methoxycarbonylazetidin-3-yl)ethynyl]phenyl]methoxy]-2-pyridyl]phenyl]methyl]-3-(2-methoxyethyl)benzimidazole-5-carboxylic acid (Example 235):** To a solution of 2-[[4-[6-[[4-[2-(azetidin-3-yl)ethynyl]-2-fluoro-phenyl]methoxy]-2-pyridyl]-2, 5-difluoro-phenyl]methyl]-3-(2-methoxyethyl)benzimidazole-5-carboxylic acid (**Example 232**) (20 mg, 0.032 mmol) in DCM (2 mL), methyl chloroformate (10 mg, 0.096 mmol) and diisopropylethylamine (30 uL, 0.17 mmol) were added. The mixture was then stirred for 1 hr at rt, after which, aqueous lithium hydroxide was added (0.5 mL, 10 mg, 0.42 mmol) and the resultant mixture heated to 50 °C for 10 min. Upon completion 5% aqueous citric acid was added (3 mL), the reaction mixture was diluted with EtOAc (25 mL), washed with water (5 mL) and brine (5 mL). The organic layer was dried over MgSO₄, filtered, concentrated and purified by RP-HPLC (eluent: water / MeCN 0.1% TFA). The combined fractions were then frozen and placed on a lyophilizer to provide **Example 235.** ES/MS: 685.3 (M+H⁺). 1H NMR (400 MHz, Methanol-d4) δ 8.55 (t, J = 1.0 Hz, 1H), 8.22 (dd, J = 8.6, 1.4 Hz, 1H), 7.89 (dd, J = 10.8, 6.3 Hz, 1H), 7.85 - 7.73 (m, 2H), 7.57 (dd, J = 7.5, 1.6 Hz, 1H), 7.50 (t, J = 7.8 Hz, 1H), 7.35 (dd, J = 11.2, 6.1 Hz, 1H), 7.29 - 7.15 (m, 2H), 6.92 (d, J = 8.2 Hz, 1H), 5.55 (s, 2H), 4.81 (t, J = 5.0 Hz, 2H), 4.75 (s, 2H), 4.31 (t, J = 8.4 Hz, 2H), 4.01 (t, J = 7.1 Hz, 2H), 3.88 - 3.81 (m, 2H), 3.75 - 3.61 (m, 4H), 3.32 (s, 3H).

### Example 236: 2-[[2,5-difluoro-4-[6-[[4-fluoro-6-[1-(oxetan-3-yl)triazol-4-yl]-3-pyridyl]methoxy]-2-pyridyl]phenyl]methyl]-3-[[(2S)-oxetan-2-yl]methyl]benzimidazole-5-carboxylic acid

### Procedure 22

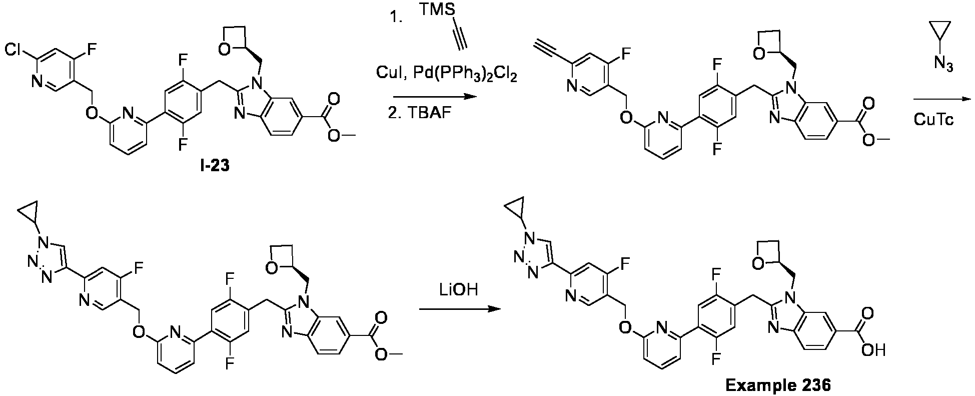

**Methyl 2-[[4-[6-[(6-ethynyl-4-fluoro-3-pyridyl)methoxy]-2-pyridyl]-2,5-difluorophenyl]methyl]-3-[[(2S)-oxetan-2-yl]methyl]benzimidazole-5-carboxylate:** To a solution of methyl 2-[[4-[6-[(6-chloro-4-fluoro-3-pyridyl)methoxy]-2-pyridyl]-2,5-difluoro-phenyl]methyl]-3-[[(2S)-oxetan-2-yl]methyl]benzimidazole-5-carboxylate (**I-23**) (162 mg, 0.27 mmol) in DMF (2 mL), cuprous iodide (2.5 mg, 0.013 mmol), trans-dichlorobis(triphenylphosphine)palladium (9.4 mg, 0.013 mmol), trimethylsilyl acetylene (45 uL, 0.32 mmol) and triethylamine (0.74 mL, 5.3 mmol) were added. The resulting solution was degassed by bubbling argon for 1 min, sealed and heated to 100 °C for 3 hrs. Upon completion the mixture was poured into water (5 mL) and extracted with EtOAc (2 x 5 mL). The organic layers were combined, washed with brine (5 mL), dried over MgSO₄, filtered, concentrated, and the crude residue was dissolved in THF (2 mL) and TBAF (1.0M in THF, 0.35 mL, 0.35 mmol) was added. After stirring at rt for 10 min the reaction mixture was diluted with EtOAc (25 mL), washed with water (5 mL) and brine (5 mL). The organic layer was dried over MgSO₄, filtered, concentrated and purified by flash chromatography (Eluent: EtOAc/hexane) to give the desired product. ES/MS: 599.4 (M+H+).

**Methyl 2-[[2,5-difluoro-4-[6-[[4-fluoro-6-[1-(oxetan-3-yl)triazol-4-yl]-3-pyridyl]methoxy]-2-pyridyl]phenyl]methyl]-3-[[(2S)-oxetan-2-yl]methyl]benzimidazole-5-carboxylate:** Methyl 2-[[4-[6-[(6-ethynyl-4-fluoro-3-pyridyl)methoxy]-2-pyridyl]-2,5-difluorophenyl]methyl]-3-[[(2S)-oxetan-2-yl]methyl]benzimidazole-5-carboxylate (20 mg, 0.033 mmol), 3-azidooxetane (3.3 mg, 0.033 mmol), THF (2 mL) and Copper(I) thiophene-2-carboxylate (0.6 mg, 0.003 mmol) were combined in a 4 mL vial and stirred at rt for 30 min. Upon completion the reaction mixture was concentrated directly and purified by flash chromatography (Eluent: EtOAc/hexane) to give the desired product. ES/MS: 698.6 (M+H+).

**2-[[2,5-difluoro-4-[6-[[4-fluoro-6-[1-(oxetan-3-yl)triazol-4-yl]-3-pyridyl]methoxy]-2-pyridyl]phenyl]methyl]-3-[[(2S)-oxetan-2-yl]methyl]benzimidazole-5-carboxylic acid (Example 236):** A suspension of methyl 2-[[2,5-difluoro-4-[6-[[4-fluoro-6-[1-(oxetan-3-yl)triazol-4-yl]-3-pyridyl]methoxy]-2-pyridyl]phenyl]methyl]-3-[[(2S)-oxetan-2-yl]methyl]benzimidazole-5-carboxylate (14 mg, 0.020 mmol) and aqueous lithium hydroxide (0.1 mL, 2 mg, 0.084 mmol) in CH₃CN (2 mL) was heated at 100 °C for 10 min. Upon completion 5% aqueous citric acid was added (3 mL), the reaction mixture was diluted with EtOAc (25 mL), washed with water (5 mL) and brine (5 mL). The organic layer was dried over MgSO₄, filtered, concentrated and purified by RP-HPLC (eluent: MeCN/H₂O). The resulting product fractions were diluted with EtOAc and neutralized with sodium bicarbonate solution. The organic extract was dried over sodium sulfate, filtered and concentrated to give **Example 236.** ES/MS: 684.3 (M+H⁺). 1H NMR (400 MHz, DMSO-d6) δ 8.94 (s, 1H), 8.84 (d, J = 10.0 Hz, 1H), 8.31 (d, J = 1.5 Hz, 1H), 7.97 - 7.87 (m, 3H), 7.83 (dd, J = 8.4, 1.5 Hz, 1H), 7.64 (d, J = 8.5 Hz, 1H), 7.54 (dd, J = 7.6, 1.7 Hz, 1H), 7.43 (dd, J = 11.5, 6.0 Hz, 1H), 6.97 (d, J = 8.2 Hz, 1H), 5.92 (tt, J = 7.6, 6.2 Hz, 1H), 5.61 (s, 2H), 5.20 - 4.88 (m, 5H), 4.81 (dd, J = 15.6, 7.1 Hz, 1H), 4.67 (dd, J = 15.4, 2.7 Hz, 1H), 4.63 - 4.46 (m, 3H), 4.38 (dt, J = 8.9, 5.9 Hz, 1H), 2.82 - 2.70 (m, 1H), 2.41 (dq, J = 11.2, 7.4 Hz, 1H).

### Example 237: 2-[[2,5-difluoro-4-[6-[[2-fluoro-4-[1-(3-pyridylmethyl)pyrazol-4-yl]phenyl]methoxy]-2-pyridyl]phenyl]methyl]-3-(2-methoxyethyl)benzimidazole-5-carboxylic acid

### Procedure 23

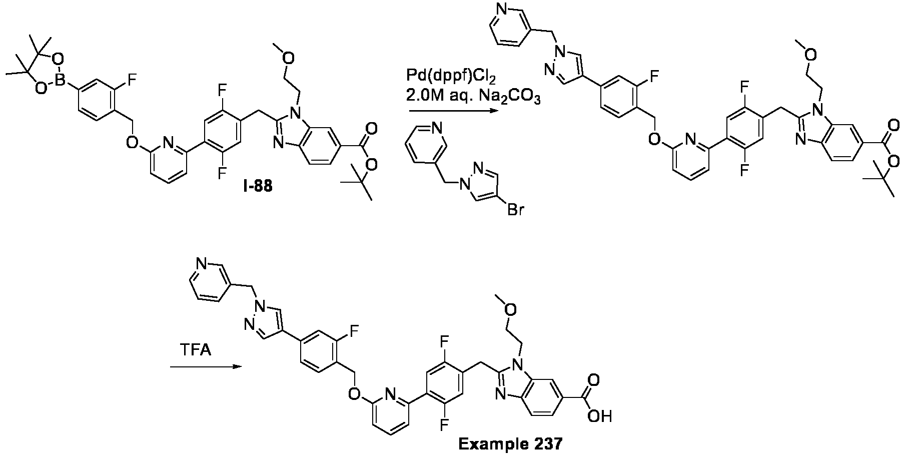

**Tert-butyl 2-[[2,5-difluoro-4-[6-[[2-fluoro-4-[1-(3-pyridylmethyl)pyrazol-4-yl]phenyl]methoxy]-2-pyridyl]phenyl]methyl]-3-(2-methoxyethyl)benzimidazole-5-carboxylate:** Tert-butyl 2-[[2,5-difluoro-4-[6-[[2-fluoro-4-(4,4,5,5-tetramethyl-1,3,2-dioxaborolan-2-yl)phenyl]methoxy]-2-pyridyl]phenyl]methyl]-3-(2-methoxyethyl)benzimidazole-5-carboxylate (**I-88**) (23 mg, 0.032 mmol), 3-[(4-bromopyrazol-1-yl)methyl]pyridine (7.5 mg, 0.032 mmol), 1,1'-Bis(diphenylphosphino)ferrocene-palladium(II)dichloride dichloromethane complex (2.6 mg, 0.0032 mmol), sodium carbonate (6.7 mg, 0.063 mmol) 1,4-dioxane (1 mL), and water (0.5 mL) were combined is a reaction vial, degassed via bubbling argon for 1 min, and then heated to 100 °C for 10 min in a microwave reactor. Upon completion the reaction mixture was concentrated directly, diluted with 9:1 acetonitrile:water, passed through a C18 SPE column, concentrated and carried forward without further purification.

**2-[[2,5-difluoro-4-[6-[[2-fluoro-4-[1-(3-pyridylmethyl)pyrazol-4-yl]phenyl]methoxy]-2-pyridyl]phenyl]methyl]-3-(2-methoxyethyl)benzimidazole-5-carboxylic acid (Example 237):** To a solution of tert-butyl 2-[[2,5-difluoro-4-[6-[[2-fluoro-4-[1-(3-pyridylmethyl)pyrazol-4-yl]phenyl]methoxy]-2-pyridyl]phenyl]methyl]-3-(2-methoxyethyl)benzimidazole-5-carboxylate (24 mg, 0.031 mmol) in DCM (2 mL) was added 0.25 mL TFA and the resulting solution stirred at 40 °C for 1 hr. The reaction mixture was concentrated directly and purified by RP-HPLC (eluent: water / MeCN 0.1% TFA). The combined fractions were then frozen and placed on a lyophilizer to provide Example 237. ES/MS: 705.2 (M+H⁺). 1H NMR (400 MHz, Acetonitrile-d3) δ 8.68 (s, 2H), 8.34 (s, 1H), 8.08 - 7.98 (m, 3H), 7.90 (q, 2H), 7.78 (dt, 2H), 7.69 (s, 2H), 7.54 (t, 2H), 7.41 - 7.32 (m, 2H), 7.27 (dd, 1H), 6.88 (d, 1H), 5.55 (s, 2H), 5.47 (s, 2H), 4.57 (d, 4H), 3.74 (t, 2H), 3.25 (s, 3H).

### Example 238: 2-[[4-[6-[[1-(cyanomethyl)pyrazol-3-yl]methoxy]-2-pyridyl]-2,5-difluorophenyl]methyl]-3-[[(2S)-oxetan-2-yl]methyl]benzimidazole-5-carboxylic acid

### Procedure 24

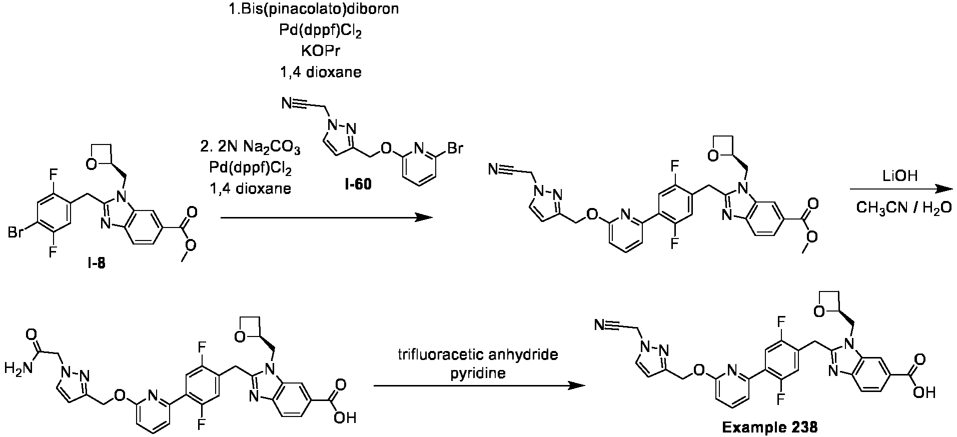

**Methyl 2-[[4-[6-[[1-(cyanomethyl)pyrazol-3-yl]methoxy]-2-pyridyl]-2,5-difluorophenyl]methyl]-3-[[(2S)-oxetan-2-yl]methyl]benzimidazole-5-carboxylate:** In a 8 mL reaction vial, a suspension of methyl 2-[(4-bromo-2,5-difluoro-phenyl)methyl]-3-[[(2S)-oxetan-2-yl]methyl]benzimidazole-5-carboxylate (40.0 mg, 0.0886 mmol), Bis(pinacolato)diboron (27.5 mg, 0.108 mmol), [1,1'-Bis(diphenylphosphino)ferrocene] dichloropalladium(II); PdCl₂(dppf) (9.86 mg, 0.0133 mmol), and potassium propionate (29.8 mg, 0.266 mmol) in dioxane (1.5 mL) was degassed with Ar for 5 min. The mixture was heated at 110 °C thermally for 1 hr. LCMS shows complete conversion of bromide. Upon completion the mixture was cooled to rt. Next, sodium carbonate (2.00 M, 0.1 mL, 0.2 mmol) was added to the reaction mixture and the mixture was stirred at rt for 5 min. Upon completion of time, [1,1'-Bis(diphenylphosphino)ferrocene] dichloropalladium(II) ;PdCl₂(dppf) (6.54 mg, 0.00881 mmol)and a solution of 2-[3-[(6-bromo-2-pyridyl)oxymethyl]pyrazol-1-yl]acetonitrile (I-60) (27.5 mg, 0.0938 mmol) in dioxane (1.5 mL) was added. Then the reaction mixture was degassed for 5 min with Ar, then heated thermally at 90 °C for 2 hr. The reaction mixture was diluted with EtOAc and washed with brine and saturated sodium bicarbonate solution. The organic extract was dried over sodium sulfate, filtered and concentrated. The crude residue was purified by flash chromatography (eluent: EtOAc/hexanes) to yield desired product. ES/MS: 585.2 (M+H⁺).

**2-[[4-[6-[[1-(2-amino-2-oxo-ethyl)pyrazol-3-yl]methoxy]-2-pyridyl]-2,5-difluorophenyl]methyl]-3-[[(2S)-oxetan-2-yl]methyl]benzimidazole-5-carboxylic acid:** A suspension of methyl 2-[[4-[6-[[1-(cyanomethyl)pyrazol-3-yl]methoxy]-2-pyridyl]-2,5-difluorophenyl]methyl]-3-[[(2S)-oxetan-2-yl]methyl]benzimidazole-5-carboxylate (35.3 mg, 0.0604 mmol) and lithium hydroxide, monohydrate (0.3 M, 0.604 mL, 0.181 mmol) in CH₃CN (3 mL) in a 40 ml reaction vial was heated at 90 °C until completion (1. 5 hr). Next, the mixture was diluted with EtOAc and brine. Next,0.360 mL 1M citric acid was added to the mixture. The organic extract was dried over sodium sulfate, filtered and concentrated. Carried onto next step below without purification.ES/MS: 589 (M+H⁺).

**2-[[4-[6-[[1-(cyanomethyl)pyrazol-3-yl]methoxy]-2-pyridyl]-2,5-difluorophenyl]methyl]-3-[[(2S)-oxetan-2-yl]methyl]benzimidazole-5-carboxylic acid (Example 238):** To a solution of 2-[[4-[6-[[1-(2-amino-2-oxo-ethyl)pyrazol-3-yl]methoxy]-2-pyridyl]-2,5-difluoro-phenyl]methyl]-3-[[(2S)-oxetan-2-yl]methyl]benzimidazole-5-carboxylic acid (35.1 mg, 0.0596 mmol) in THF (4 mL) at 0 °C, was added pyridine (0.0243 mL, 0.301 mmol). Next, a solution of trifluoroacetic anhydride (1.00 mmol/L in THF, 121 mL, 0.121 mmol) was slowly added. The resulting solution was gradually warmed to rt. After 1 hr, an additional portion of trifluoroacetic anhydride solution (1.00 mmol/L in THF, 121 mL, 0.121 mmol) was added. After 30 min, the mixture was carefully quenched with 2M sodium carbonate solution (0.120 mL). The mixture was partitioned with EtOAc and brine. The organic extract was dried over sodium sulfate, filtered and concentrated. Purified by RP-HPLC (eluent: MeCN/H₂O). The resulting product fractions were diluted with EtOAc and neutralized with sodium bicarbonate solution. The organic extract was dried over sodium sulfate, filtered and concentrated to give **Example 238.** ES/MS: 571.2 (M+H⁺); 1HNMR (400 MHz, Methanol-d4) δ 8.32 (d, J = 1.5 Hz, 2H), 8.06 - 7.90 (m, 4H), 7.76 (ddd, J = 15.7, 7.8, 2.5 Hz, 3H), 7.71 - 7.65 (m, 2H), 7.54 (dt, J = 7.5, 2.2 Hz, 2H), 7.18 (dd, J = 11.5, 6.0 Hz, 2H), 6.84 (dd, J = 9.0, 5.3 Hz, 2H), 6.47 (d, J = 2.4 Hz, 1H), 6.41 (t, J = 2.7 Hz, 1H), 5.48 (d, J = 5.0 Hz, 3H), 5.32 (s, 2H), 5.20 (qd, J = 7.0, 2.5 Hz, 2H), 4.79 - 4.36 (m, 14H), 2.80 (dtd, J = 11.4, 8.2, 6.1 Hz, 2H), 2.50 (ddt, J = 11.5, 9.1, 7.1 Hz, 2H).

### Example 239: (S)-2-(4-(6-((1-(cyanomethyl)-1H-pyrazol-5-yl)methoxy)pyridin-2-yl)-2,5-difluorobenzyl)-1-(oxetan-2-ylmethyl)-1H-benzo[d]imidazole-6-carboxylic acid

(S)-2-(4-(6-((1-(cyanomethyl)-1H-pyrazol-5-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 24. 1H NMR (400 MHz, Methanol-d4) δ 8.31 (d, J = 1.4 Hz, 1H), 7.99 (dd, J = 8.5, 1.5 Hz, 1H), 7.94 (dd, J = 10.6, 6.4 Hz, 1H), 7.81 (t, J = 7.9 Hz, 1H), 7.67 (d, J= 8.5 Hz, 1H), 7.60 - 7.52 (m, 2H), 7.20 (dd, J = 11.4, 6.0 Hz, 1H), 6.91 (d, J = 8.1 Hz, 1H), 6.52 (d, J = 1.9 Hz, 1H), 5.63 (s, 2H), 5.41 (s, 2H), 5.20 (qd, J = 7.1, 2.5 Hz, 1H), 4.73 (dd, J = 15.7, 7.0 Hz, 1H), 4.69 - 4.38 (m, 5H), 2.92 - 2.69 (m, 1H), 2.58 - 2.35 (m, 1H).

### Example 240: 2-[[4-[6-[(6-carbamoyl-2-fluoro-3-pyridyl)methoxy]-2-pyridyl]-2,5-difluorophenyl]methyl]-3-[[(2S)-oxetan-2-yl]methyl]benzimidazole-5-carboxylic acid

### Procedure 25

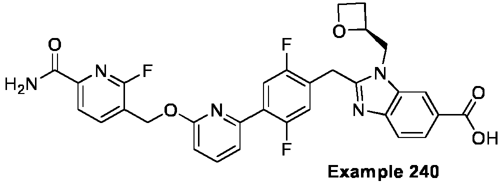

**2-[[4-[6-[(6-carbamoyl-2-fluoro-3-pyridyl)methoxy]-2-pyridyl]-2,5-difluorophenyl]methyl]-3-[[(2S)-oxetan-2-yl]methyl]benzimidazole-5-carboxylic acid (Example 240):** The title compound was prepared in a manner as described in Procedure 11 substituting 3-(bromomethyl)-6-chloro-2-fluoropyridine for 2-bromo-5-(bromomethyl)-3-fluoro-thiophene. ES/MS: 604.2 (M+H⁺); 1H NMR (400 MHz, DMSO-d6) δ 12.75 (s, 1H), 8.31 - 8.23 (m, 2H), 8.06 (s, 1H), 7.99 (dd, J = 7.6, 1.5 Hz, 1H), 7.94 - 7.86 (m, 1H), 7.82 - 7.76 (m, 2H), 7.76 - 7.69 (m, 1H), 7.61 (d, J = 8.4 Hz, 1H), 7.54 (dd, J = 7.5, 1.7 Hz, 1H), 7.45 - 7.31 (m, 1H), 7.00 (d, J = 8.2 Hz, 1H), 5.60 (s, 2H), 5.08 (qd, J = 7.0, 2.7 Hz, 1H), 4.76 (dd, J = 15.6, 7.1 Hz, 1H), 4.62 (dd, J = 15.5, 2.8 Hz, 1H), 4.58 - 4.41 (m, 3H), 4.36 (dt, J = 9.1, 5.9 Hz, 1H), 2.79 - 2.65 (m, 1H), 2.39 (ddt, J = 11.2, 8.9, 6.9 Hz, 1H).

### Example 241: (S)-2-(4-(6-((6-carbamoyl-4-fluoropyridin-3-yl)methoxy)pyridin-2-yl)-2,5-difluorobenzyl)-4-fluoro-1-(oxetan-2-ylmethyl)-1H-benzo[d]imidazole-6-carboxylic acid

(S)-2-(4-(6-((6-carbamoyl-4-fluoropyridin-3-yl)methoxy)pyridin-2-yl)-2,5-difluorobenzyl)-4-fluoro-1-(oxetan-2-ylmethyl)-1H-benzo[d]imidazole-6-carboxylic acid was prepared in a manner as described in Procedure 25. 1H NMR (400 MHz, DMSO-d6) δ 8.84 (d, J = 9.5 Hz, 1H), 8.21 (s, 1H), 8.12 (s, 1H), 7.88 (td, J = 10.1, 6.4 Hz, 3H), 7.80 (s, 1H), 7.52 (dd, J = 17.5, 9.4 Hz, 2H), 7.41 (dd, J = 11.5, 6.1 Hz, 1H), 6.97 (d, J = 8.3 Hz, 1H), 5.65 (s, 2H), 5.08 (d, J = 7.3 Hz, 1H), 4.78 (dd, J = 15.5, 7.2 Hz, 1H), 4.64 (d, J = 15.3 Hz, 1H), 4.51 (q, J = 17.1 Hz, 3H), 4.43 - 4.28 (m, 1H), 2.71 (d, J = 23.9 Hz, 1H), 2.47 - 2.29 (m, 1H).

### Example 242: (S)-2-(4-(6-((6-carbamoyl-4-methoxypyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-carbamoyl-4-methoxypyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 25. 1HNMR (400 MHz, DMSO-d6) δ 8.55 (s, 1H), 8.24 (d, J = 1.5 Hz, 1H), 8.11 (d, J = 2.9 Hz, 1H), 7.96 - 7.75 (m, 4H), 7.71 (s, 1H), 7.66 (d, J = 2.9 Hz, 1H), 7.59 (d, J = 8.4 Hz, 1H), 7.52 (dd, J = 7.6, 1.7 Hz, 1H), 7.39 (dd, J = 11.5, 6.1 Hz, 1H), 6.96 (d, J = 8.2 Hz, 1H), 5.53 (s, 2H), 5.08 (qd, J = 7.0, 2.6 Hz, 1H), 4.75 (dd, J = 15.5, 7.0 Hz, 1H), 4.62 (dd, J = 15.5, 2.8 Hz, 1H), 4.57 - 4.41 (m, 3H), 4.36 (dt, J = 9.0, 5.9 Hz, 1H), 4.00 (s, 3H), 2.72 (ddt, J = 14.2, 10.7, 5.3 Hz, 1H), 2.39 (ddt, J = 17.6, 14.2, 7.1 Hz, 1H).

### Example 243: 2-[[2,5-difluoro-4-[6-[[4-fluoro-6-(methylcarbamoyl)-3-pyridyl]methoxy]-2-pyridyl]phenyl]methyl]-3-[[(2S)-oxetan-2-yl]methyl]benzimidazole-5-carboxylic acid

### Procedure 26

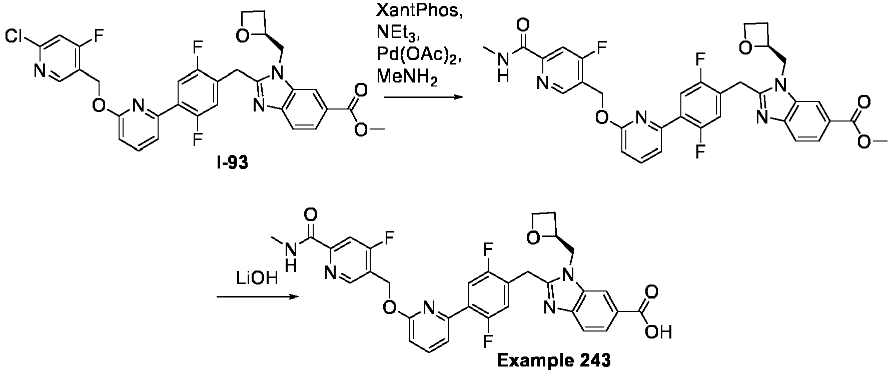

**Methyl 2-[[2,5-difluoro-4-[6-[[4-fluoro-6-(methylcarbamoyl)-3-pyridyl]methoxy]-2-pyridyl]phenyl]methyl]-3-[[(2S)-oxetan-2-yl]methyl]benzimidazole-5-carboxylate:** Methyl 2-[[4-[6-[(6-bromo-4-fluoro-3-pyridyl)methoxy]-2-pyridyl]-2,5-difluoro-phenyl]methyl]-3-[[(2S)-oxetan-2-yl]methyl]benzimidazole-5-carboxylate (30 mg, 0.046 mmol), XantPhos (5.3 mg, 0.0092 mmol), palladium acetate (2.1 mg, 0.0092 mmol) and triethylamine (0.256 mL, 1.84 mmol) were combined in DMF (1.00 mL) and CO, then bubbled through for 5 min. Upon completion methylamine (2.0M, 0.230 mL, 0.459 mmol) was then added and the reaction vessel sealed and heated to 110 °C under balloon CO atmosphere for 20 min. Upon completion the reaction mixture was directly loaded onto silica and purified by flash chromatography (eluent: EtOAc/hexanes) to yield desired product. ES/MS: 632.2 (M+H⁺).

**2-[[2,5-difluoro-4-[6-[[4-fluoro-6-(methylcarbamoyl)-3-pyridyl]methoxy]-2-pyridyl]phenyl]methyl]-3-[[(2S)-oxetan-2-yl]methyl]benzimidazole-5-carboxylic acid (Example 243):** Methyl 2-[[2,5-difluoro-4-[6-[[4-fluoro-6-(methylcarbamoyl)-3-pyridyl]methoxy]-2-pyridyl]phenyl]methyl]-3-[[(2S)-oxetan-2-yl]methyl]benzimidazole-5-carboxylate was converted to the title compound in a manner as described for step 2 of Procedure 1. To yield **Example 243.** ES/MS: 618.2 (M+H⁺); 1H NMR (400 MHz, DMSO-d6) δ 8.90 - 8.81 (m, 2H), 8.25 (d, J = 1.6 Hz, 1H), 7.94 - 7.82 (m, 3H), 7.79 (dd, J = 8.4, 1.5 Hz, 1H), 7.63 - 7.50 (m, 2H), 7.40 (dd, J = 11.5, 6.1 Hz, 1H), 6.97 (d, J = 8.2 Hz, 1H), 5.65 (s, 2H), 5.13 - 5.03 (m, 1H), 4.76 (dd, J = 15.6, 7.1 Hz, 1H), 4.63 (dd, J = 15.6, 2.8 Hz, 1H), 4.58 - 4.41 (m, 3H), 4.36 (dt, J = 9.0, 5.9 Hz, 1H), 2.82 (d, J = 4.8 Hz, 3H), 2.79 - 2.68 (m, 1H), 2.46 - 2.35 (m, 1H).

### Example 244: (S)-2-(4-(6-((6-(cyclopentylcarbamoyl)-4-fluoropyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-(cyclopentylcarbamoyl)-4-fluoropyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 26. 1HNMR (400 MHz, DMSO-d6) δ 12.75 (s, 1H), 8.84 (d, J = 9.4 Hz, 1H), 8.63 (d, J = 8.0 Hz, 1H), 8.25 (d, J = 1.5 Hz, 1H), 7.94 - 7.76 (m, 4H), 7.63 - 7.50 (m, 2H), 7.40 (dd, J = 11.5, 6.1 Hz, 1H), 6.97 (d, J = 8.2 Hz, 1H), 5.65 (s, 2H), 5.08 (qd, J = 7.0, 2.7 Hz, 1H), 4.76 (dd, J = 15.5, 7.0 Hz, 1H), 4.63 (dd, J = 15.6, 2.8 Hz, 1H), 4.58 - 4.45 (m, 2H), 4.45 (d, J = 16.8 Hz, 1H), 4.36 (dt, J = 9.0, 5.9 Hz, 1H), 4.22 (p, J = 7.2 Hz, 1H), 2.79 - 2.67 (m, 1H), 2.40 (ddt, J = 11.2, 9.0, 7.0 Hz, 1H), 1.94 - 1.82 (m, 2H), 1.69 (s, 1H), 1.69 - 1.49 (m, 4H).

### Example 245: (S)-2-(4-(6-((6-(cyclopropylcarbamoyl)-4-fluoropyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-(cyclopropylcarbamoyl)-4-fluoropyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 26. Multiplet Report 1H NMR (400 MHz, DMSO-d6) δ 8.87 - 8.78 (m, 2H), 8.24 (d, J = 1.5 Hz, 1H), 7.93 - 7.81 (m, 3H), 7.79 (dd, J = 8.4, 1.6 Hz, 1H), 7.62 - 7.50 (m, 2H), 7.40 (dd, J = 11.6, 6.1 Hz, 1H), 6.97 (d, J = 8.3 Hz, 1H), 5.65 (s, 2H), 5.08 (tt, J = 9.5, 4.6 Hz, 1H), 4.75 (dd, J = 15.6, 7.0 Hz, 1H), 4.62 (dd, J = 15.6, 2.8 Hz, 1H), 4.58 - 4.45 (m, 2H), 4.45 (d, J = 16.8 Hz, 1H), 4.36 (dt, J = 9.0, 6.0 Hz, 1H), 2.91 (td, J = 6.7, 2.5 Hz, 1H), 2.79 - 2.68 (m, 1H), 2.46 - 2.35 (m, 1H), 0.68 (dq, J = 5.2, 2.1 Hz, 4H).

### Example 246: (S)-2-(4-(6-((6-(cyclobutylcarbamoyl)-4-fluoropyridin-3-yl)methoxy)pyridin-2-yl)-2,5-difluorobenzyl)-1-(oxetan-2-ylmethyl)-1H-benzo[d]imidazole-6-carboxylic acid

(S)-2-(4-(6-((6-(cyclobutylcarbamoyl)-4-fluoropyridin-3-yl)methoxy)pyridin-2-yl)-2,5-difluorobenzyl)-1-(oxetan-2-ylmethyl)-1H-benzo[d]imidazole-6-carboxylic acid was prepared in a manner as described in Procedure 26. 1H NMR (400 MHz, DMSO-d6) δ 9.03 (d, J = 8.5 Hz, 1H), 8.85 (d, J = 9.4 Hz, 1H), 8.24 (s, 1H), 7.94 - 7.75 (m, 4H), 7.56 (dd, J = 16.9, 7.4 Hz, 2H), 7.40 (dd, J = 11.5, 6.0 Hz, 1H), 6.97 (d, J = 8.2 Hz, 1H), 5.66 (s, 2H), 5.12 - 5.05 (m, 1H), 4.75 (dd, J = 15.7, 7.0 Hz, 1H), 4.66 - 4.31 (m, 5H), 3.30 (s, 2H), 2.79 - 2.65 (m, 1H), 2.40 (t, J = 9.7 Hz, 1H), 2.17 (q, J = 8.0 Hz, 4H), 1.65 (p, J = 8.8, 8.1 Hz, 2H).

### Example 247: (S)-2-(2,5-difluoro-4-(6-((4-fluoro-6-(oxetan-3-ylcarbamoyl)pyridin-3-yl)methoxy)pyridin-2-yl)benzyl)-1-(oxetan-2-ylmethyl)-1H-benzo [d] imidazole-6-carboxylic acid

(S)-2-(2,5-difluoro-4-(6-((4-fluoro-6-(oxetan-3-ylcarbamoyl)pyridin-3-yl)methoxy)pyridin-2-yl)benzyl)-1-(oxetan-2-ylmethyl)-1H-benzo[d]imidazole-6-carboxylic acid was prepared in a manner as described in Procedure 26. 1H NMR (400 MHz, DMSO-d6) δ 9.58 (d, J = 6.9 Hz, 1H), 8.89 (d, J = 9.5 Hz, 1H), 8.27 - 8.21 (m, 1H), 7.94 - 7.82 (m, 3H), 7.79 (dd, J = 8.4, 1.6 Hz, 1H), 7.56 (dd, J = 17.1, 7.6 Hz, 2H), 7.40 (dd, J = 11.5, 6.0 Hz, 1H), 6.98 (d, J = 8.3 Hz, 1H), 5.67 (s, 2H), 5.04 (tt, J = 14.1, 6.5 Hz, 2H), 4.81 - 4.69 (m, 2H), 4.74 - 4.53 (m, 4H), 4.56 - 4.31 (m, 4H), 2.73 (dd, J = 12.2, 5.9 Hz, 1H), 2.45 - 2.35 (m, 1H).

### Example 248: 2-[[4-[6-[(5-carbamoyl-2-pyridyl)methoxy]-2-pyridyl]-2,5-difluorophenyl]methyl]-3-[[(2S)-oxetan-2-yl]methyl]benzimidazole-5-carboxylic acid

### Procedure 27

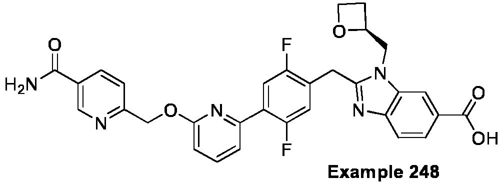

**2-[[4-[6-[(5-carbamoyl-2-pyridyl)methoxy]**-**2-pyridyl]-2,5-difluoro-phenyl]methyl]-3-[[(2S)-oxetan-2-yl]methyl]benzimidazole-5-carboxylic acid (Example 248):** The title compound was prepared in a manner as described in Procedure 9 substituting 6-(bromomethyl)nicotinonitrile for 5-(bromomethyl)thiazole-2-carbonitrile, silver carbonate for cesium carbonate, and toluene for acetonitrile. ES/MS: 586.2 (M+H⁺); 1H NMR (400 MHz, DMSO-d6) δ 9.02 (d, J = 2.2 Hz, 1H), 8.30 (d, J = 1.5 Hz, 1H), 8.23 (dd, J = 8.2, 2.2 Hz, 1H), 8.14 (s, 1H), 7.91 (t, J = 7.9 Hz, 1H), 7.83 (dd, J = 8.5, 1.5 Hz, 1H), 7.70 - 7.49 (m, 4H), 7.38 (dd, J = 11.6, 6.0 Hz, 1H), 7.04 (d, J = 8.2 Hz, 1H), 5.60 (s, 2H), 5.07 (d, J = 5.7 Hz, 1H), 4.79 (dd, J = 15.5, 7.2 Hz, 1H), 4.70 - 4.42 (m, 3H), 4.36 (dt, J = 9.0, 5.9 Hz, 1H), 2.72 - 2.60 (m, 1H), 2.44 - 2.33 (m, 1H).

### Example 249: 2-[[4-[6-[[4-(cyclopropylcarbamoyl)phenyl]methoxy]-2-pyridyl]-2,5-difluoro-phenyl]methyl]-3-(2-methoxyethyl)benzimidazole-5-carboxylic acid

### Procedure 28

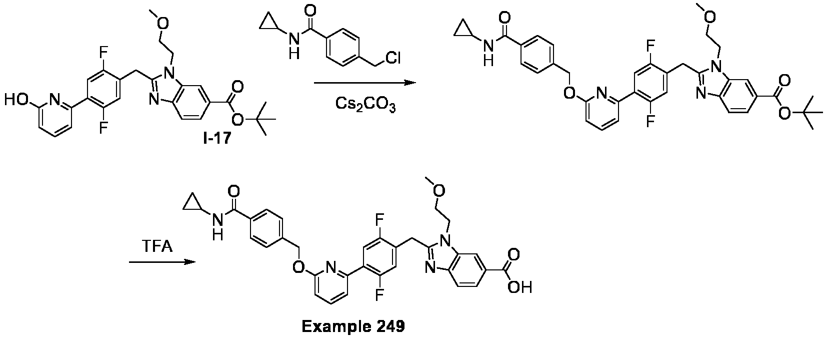

**Tert-butyl 2-[[4-[6-[[4-(cyclopropylcarbamoyl)phenyl]methoxy]-2-pyridyl]-2,5-difluoro-phenyl]methyl]-3-(2-methoxyethyl)benzimidazole-5-carboxylate:** To a solution of tert-butyl 2-[[2,5-difluoro-4-(6-hydroxy-2-pyridyl)phenyl]methyl]-3-(2-methoxyethyl)benzimidazole-5-carboxylate (45 mg, 0.091 mmol) and 4-(chloromethyl)-N-cyclopropyl-benzamide (20 mg, 0.095 mmol) in 3 mL of acetonitrile , Cs₂CO₃ (62 mg, 0.19 mmol) was added. The solution was then heated to 50 °C for 30 min. Upon completion the solution was cooled to rt, filtered, then concentrated. The crude material was purified by silica gel column chromatography (eluent: EtOAc/hexanes) to provide desired product.

**2-[[4-[6-[[4-(cyclopropylcarbamoyl)phenyl]methoxy]-2-pyridyl]-2,5-difluorophenyl]methyl]-3-(2-methoxyethyl)benzimidazole-5-carboxylic acid (Example 249):** To a solution of tert-butyl 2-[[4-[6-[[4-(cyclopropylcarbamoyl)phenyl]methoxy]-2-pyridyl]-2,5-difluoro-phenyl]methyl]-3-(2-methoxyethyl)benzimidazole-5-carboxylate (18 mg, 0.027 mmol) in DCM (2.5 mL) was added 0.5 mL TFA and the resulting solution stirred at rt for 1 hour. Upon completion the reaction mixture was diluted with EtOAc (30 mL), washed with water (3 x 5 mL), concentrated and purified by RP-HPLC (eluent: water / MeCN 0.1% TFA). The combined fractions were then frozen and placed on a lyophilizer to provide **Example 249.** ES/MS: 613.3 (M+H⁺). 1H NMR (400 MHz, Methanol-d4) δ 8.61 (t, J = 1.0 Hz, 1H), 8.27 (dd, J = 8.6, 1.4 Hz, 1H), 7.92 - 7.69 (m, 5H), 7.61 - 7.50 (m, 3H), 7.39 (dd, J = 11.1, 6.1 Hz, 1H), 6.94 (d, J = 8.2 Hz, 1H), 5.55 (s, 2H), 4.88 - 4.77 (m, 4H), 4.17 (ddd, J = 12.2, 9.0, 3.7 Hz, 1H), 3.91 - 3.78 (m, 2H), 3.67 (m, 6H), 3.35 - 3.26 (m, 3H).

### Example 250: 2-(4-(6-((6-(3-cyclopropyl-1H-1,2,4-triazol-1-yl)pyridin-3-yl)methoxy)pyridin-2-yl)-2,5-difluorobenzyl)-1-(2-methoxyethyl)-1H-benzo[d]imidazole-6-carboxylic acid

2-(4-(6-((6-(3 -cyclopropyl-1H-1,2,4-triazol-1-yl)pyridin-3-yl)methoxy)pyridin-2-yl)-2,5-difluorobenzyl)-1-(2-methoxyethyl)-1H-benzo[d]imidazole-6-carboxylic acid was prepared in a manner as described in Procedure 28. 1HNMR (400 MHz, Methanol-d4) δ 9.14 (s, 1H), 8.62 (d, J = 2.2 Hz, 1H), 8.53 (t, J = 1.0 Hz, 1H), 8.20 (dd, J = 8.6, 1.4 Hz, 1H), 8.12 (d, J = 2.3 Hz, 1H), 7.97 - 7.80 (m, 3H), 7.80 - 7.69 (m, 1H), 7.65 - 7.52 (m, 1H), 7.35 (dd, J = 11.2, 6.0 Hz, 1H), 7.02 - 6.84 (m, 1H), 5.60 (s, 2H), 4.80 (t, J = 5.0 Hz, 2H), 4.74 (s, 2H), 3.83 (t, J = 4.9 Hz, 2H), 2.14 (ddd, J = 13.2, 8.3, 5.0 Hz, 1H), 1.14 - 0.95 (m, 4H).

### Example 251: 2-[[4-[6-[[4-[2-(1-acetylazetidin-3-yl)ethynyl]-2-fluoro-phenyl]methoxy]-2-pyridyl]-2,5-difluoro-phenyl]methyl]-3-(2-methoxyethyl)benzimidazole-5-carboxylic acid

### Procedure 29

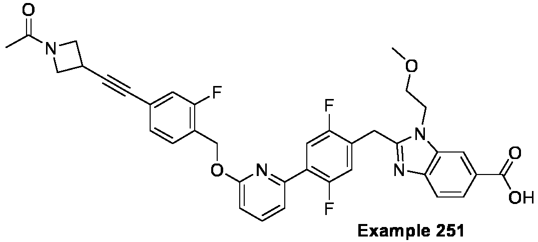

**2-[[4-[6-[[4-[2-(1-acetylazetidin-3-yl)ethynyl]-2-fluoro-phenyl]methoxy]-2-pyridyl] - 2,5-difluoro-phenyl]methyl]-3-(2-methoxyethyl)benzimidazole-5-carboxylic acid (Example 251):** The title compound was prepared in a manner as described in Procedure 21 substituting acetic anhydride for methyl chloroformate.ES/MS: 669.3 (M+H⁺); 1H NMR (400 MHz, DMSO-d6) δ 8.32 (d, J = 30.4 Hz, 1H), 7.97 - 7.76 (m, 3H), 7.68 (dd, J = 23.4, 8.5 Hz, 1H), 7.61 - 7.45 (m, 2H), 7.36 (td, J = 21.4, 20.2, 7.5 Hz, 3H), 6.96 (d, J = 8.5 Hz, 1H), 5.52 (s, 2H), 4.65 (d, J = 5.4 Hz, 3H), 4.50 (d, J = 8.6 Hz, 2H), 4.43 (t, J = 8.7 Hz, 1H), 4.16 (q, J = 6.8, 4.6 Hz, 3H), 3.82 (t, J = 7.3 Hz, 1H), 3.70 (d, J = 5.7 Hz, 4H), 3.22 (d, J = 3.2 Hz, 3H), 1.77 (d, J = 2.5 Hz, 3H).

### Example 252: 2-[[2,5-difluoro-4-[5-fluoro-6-[[5-(trifluoromethyl)thiazol-2-yl]methoxy]-2-pyridyl]phenyl]methyl]-3-[(3S)-4,4-dimethyltetrahydrofuran-3-yl]-7-fluoro-benzimidazole-5-carboxylic acid

### Procedure 30

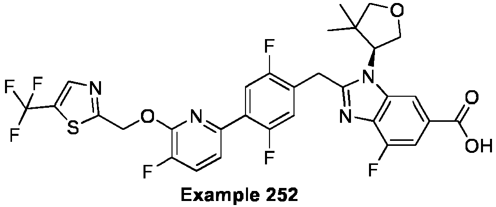

**2-[[2,5-difluoro-4-[5-fluoro-6-[[5-(trifluoromethyl)thiazol-2-yl]methoxy]-2-pyridyl]phenyl]methyl]-3-[(3S)-4,4-dimethyltetrahydrofuran-3-yl]-7-fluoro-benzimidazole-5-carboxylic acid (Example 252):** To a mixture of methyl 2-[[2,5-difluoro-4-[5-fluoro-6-[[5-(trifluoromethyl)thiazol-2-yl]methoxy]-2-pyridyl]phenyl]methyl]-3-[(3S)-4,4-dimethyltetrahydrofuran-3-yl]-7-fluoro-benzimidazole-5-carboxylate (**Intermediate I-106**, 55 mg, 0.0792 mmol) in acetonitrile (3.00 mL) and 2 N lithium hydroxide (0.12 mL, 0.24 mmol) was added and the resulting mixture stirred at 105 °C for 4 minutes. To the mixture was added 0.5 mL of acetic acid. Solvent was removed in vacuo and the resulting residue was purified by reverse-phase preparative HPLC (eluent: water / MeCN, 0.1% TFA) to yield **Example 252**. 1H NMR (400 MHz, DMSO) δ 13.08 (s, 1H), 8.49 (d, J = 1.3 Hz, 1H), 8.35 (s, 1H), 7.93 (dd, J = 10.2, 8.3 Hz, 1H), 7.82 (dd, J = 10.2, 6.7 Hz, 1H), 7.66 - 7.59 (m, 1H), 7.56 - 7.43 (m, 2H), 5.98 (s, 2H), 5.03 (d, J = 6.6 Hz, 1H), 4.59 - 4.48 (m, 2H), 4.47 - 4.35 (m, 2H), 3.84 (s, 14H), 3.74 (q, J = 8.7 Hz, 2H), 1.33 (s, 3H), 0.60 (s, 3H). ES/MS m/z: 680.4 (M+H)⁺.

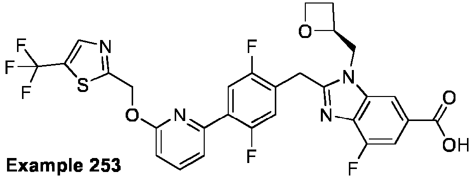

**Example 253: (S)-2-(2,5-difluoro-4-(6-((5-(trifluoromethyl)thiazol-2-yl)methoxy)pyridin-2-yl)benzyl)-4-fluoro-1-(oxetan-2-ylmethyl)-1H-benzo[d]imidazole-6-carboxylic acid (Example 253): Example 253** was prepared in a manner as described in Procedure 30, with the following modifications: LiOH (1.0 M in H2O, 10.4 mL, 4.0 mmol) was added to a solution of ethyl (S)-2-(2,5-difluoro-4-(6-((5-(trifluoromethyl)thiazol-2-yl)methoxy)pyridin-2-yl)benzyl)-4-fluoro-1-(oxetan-2-ylmethyl)-1H-benzo[d]imidazole-6-carboxylate (Intermediate **I-109**, 1.72 g, 2.60 mmol) in acetonitrile (30 mL), and the resulting solution was heated to reflux for 12 min. Reaction was quenched with 1 mL acetic acid, and diluted with EtOAc (100 mL). The organic extracts were washed with water (4 x 50 mL), dried over MgSO₄, filtered, concentrated in vacuo. The crude material was purified by reverse phase chromatography (MeCN/water gradient with 0.1 % TFA added). The combined fractions were diluted with EtOAc (100 mL), washed with water (5 x 80 mL), dried over MgSO₄, filtered, and concentrated to afford **Example 253.** 1H NMR (400 MHz, DMSO-d6) δ 8.47 (d, J = 1.4 Hz, 1H), 8.15 (d, J = 1.3 Hz, 1H), 7.95 (t, J = 7.9 Hz, 1H), 7.82 (dd, J = 10.5, 6.4 Hz, 1H), 7.59 (dd, J = 7.5, 1.6 Hz, 1H), 7.51 (dd, J = 11.4, 1.3 Hz, 1H), 7.40 (dd, J = 11.5, 6.1 Hz, 1H), 7.05 (d, J = 8.2 Hz, 1H), 5.88 (s, 2H), 5.07 (qd, J = 7.1, 2.6 Hz, 1H), 4.79 (dd, J = 15.6, 7.1 Hz, 1H), 4.65 (dd, J = 15.5, 2/.7 Hz, 1H), 4.60 - 4.41 (m, 3H), 4.35 (dt, J = 9.0, 5.9 Hz, 1H), 2.79 - 2.65 (m, 1H), 2.38 (ddt, J = 11.3, 9.0, 7.0 Hz, 1H). ES/MS m/z: 634.6 (M+H)⁺.

### Example 267: 2-[[4-[2-[(5-cyano-3-fluoro-2-thienyl)methoxy]pyrimidin-4-yl]-2,5-difluorophenyl]methyl]-3-[(3S)-4,4-dimethyltetrahydrofuran-3-yl]benzimidazole-5-carboxylic acid

### Procedure 31

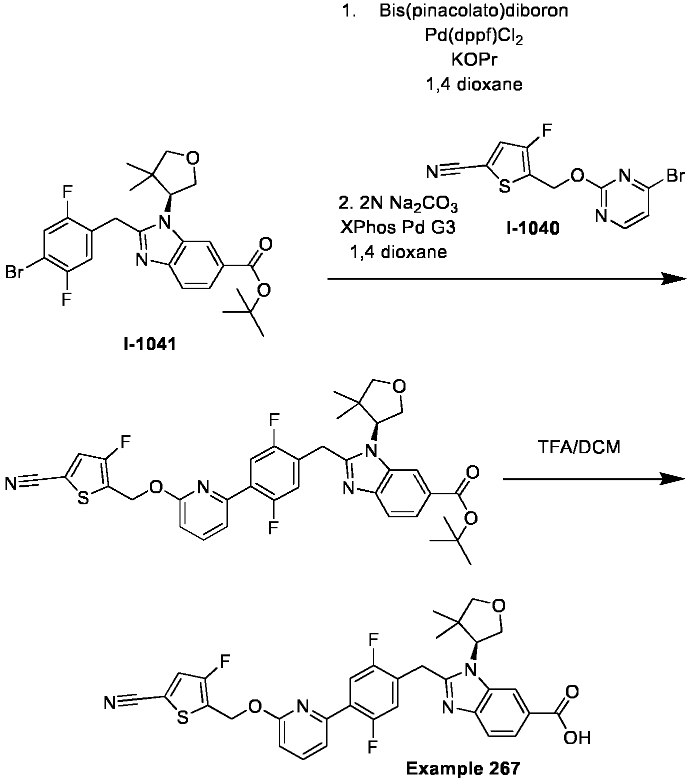

**tert-butyl 2-[[4-[2-[(5-cyano-3-fluoro-2-thienyl)methoxy]pyrimidin-4-yl]-2,5-difluoro-phenyl]methyl]-3-[(3S)-4,4-dimethyltetrahydrofuran-3-yl]benzimidazole-5-carboxylate:** In a 8 mL reaction vial, a suspension of tert-butyl 2-[(4-bromo-2,5-difluorophenyl)methyl]-3-[(3S)-4,4-dimethyltetrahydrofuran-3-yl]benzimidazole-5-carboxylate (39.0 mg, 0.0748 mmol), Bis(pinacolato)diboron (23.2 mg, 0.0913 mmol), [1,1'-Bis(diphenylphosphino)ferrocene] dichloropalladium(II) ;PdCl₂(dppf) (8.32 mg, 0.0112 mmol), and potassium propionate (25.2 mg, 0.224 mmol) in dioxane (1.5 mL) was degassed with Ar for 5 min .Next, the mixture was heated at 110 °C thermally for 45 min. LCMS shows complete conversion of bromide. Upon completion, the mixture was cooled to rt. Sodium carbonate (2.00 M, 0.084 mL, 0.169 mmol) was added and the mixture was stirred at rt for 5 min. Next, XPhos Pd G3 (6.33 mg, 0.00748 mmol) and 5-[(4-bromopyrimidin-2-yl)oxymethyl]-4-fluoro-thiophene-2-carbonitrile (23.5 mg, 0.0748 mmol) were added. The mixture was then degassed for 5 min with Ar, and then heated thermally at 90 °C for 90 min. The mixture was diluted with EtOAc and washed with brine and saturated sodium bicarbonate solution. The organic extract was dried over sodium sulfate, filtered and concentrated. The crude residue was purified by flash chromatography (eluent: EtOAc/hexanes) to yield desired product. ES/MS: 676.0 (M+H⁺); **2-[[4-[2-[(5-cyano-3-fluoro-2-thienyl)methoxy]pyrimidin-4-yl]-2,5-difluoro-phenyl]methyl]-3-[(3S)-4,4-dimethyltetrahydrofuran-3-yl]benzimidazole-5-carboxylic acid (Example 267):** To a solution of tert-butyl 2-[[4-[2-[(5-cyano-3-fluoro-2-thienyl)methoxy]pyrimidin-4-yl]-2,5-difhioro-phenyl]methyl]-3-[(3S)-4,4-dimethyltetrahydrofuran-3-yl]benzimidazole-5-carboxylate (23.6 mg, 0.0349 mmol) in DCM (3 mL), was added 2,2,2-trifluoroacetic acid (0.106 mL, 1.40 mmol). The mixture was stirred at 40 °C until completion. The reaction was concentrated and purified by RP-HPLC (eluent: MeCN/H₂O) to give title product. ES/MS: 611.0 (M+H⁺); 1H NMR (400 MHz, DMSO-d6) δ 8.82 (d, J = 5.2 Hz, 1H), 8.49 (s, 1H), 8.02 (d, J = 11.6 Hz, 2H), 7.80 (dd, J = 8.4, 1.4 Hz, 1H), 7.69 (dd, J = 5.2, 1.7 Hz, 1H), 7.62 (d, J = 8.5 Hz, 1H), 7.56 (dd, J = 11.5, 5.9 Hz, 1H), 5.71 (s, 2H), 5.02 (d, J = 6.6 Hz, 1H), 4.65 - 4.51 (m, 2H), 4.50 - 4.29 (m, 2H), 3.80 - 3.63 (m, 2H), 1.34 (s, 3H), 0.61 (s, 3H).

### Example 268: 2-(2,5-difluoro-4-(5-fluoro-6-((5-methoxy-1,3,4-thiadiazol-2-yl)methoxy)pyridin-2-yl)benzyl)-1-((1-(fluoromethyl)cyclopropyl)methyl)-1H-benzo[d]imidazole-6-carboxylic acid

2-(2,5-difluoro-4-(5-fluoro-6-((5-methoxy-1,3,4-thiadiazol-2-yl)methoxy)pyridin-2-yl)benzyl)-1-((1-(fluoromethyl)cyclopropyl)methyl)-1H-benzo[d]imidazole-6-carboxylic acid was prepared in a manner as described in for Procedure 31 using Intermediates **I-1244** and **I-1188.** ES/MS m/z: 614.3 (M+H⁺). 1HNMR (400 MHz, Methanol-d4) δ 8.57 (d, J = 1.3 Hz, 1H), 8.20 (dd, J = 8.6, 1.4 Hz, 1H), 7.96 (dd, J = 10.7, 6.3 Hz, 1H), 7.77 (d, J = 8.6 Hz, 1H), 7.75 - 7.60 (m, 2H), 7.38 (dd, J = 11.3, 6.0 Hz, 1H), 5.81 (s, 2H), 4.74 (s, 4H), 4.32 (d, J = 11.8 Hz, 1H), 4.18 (d, J = 2.6 Hz, 4H), 1.10 - 1.00 (m, 2H), 0.90 (d, J = 5.0 Hz, 2H).

### Example 271: 1-((1-(fluoromethyl)cyclopropyl)methyl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid

1-((1-(fluoromethyl)cyclopropyl)methyl)-2-(2,3,6-trifluoro-4-(6-((5-methoxy-1,3,4-thiadiazol-2-yl)methoxy)pyridin-2-yl)benzyl)-1H-benzo[d]imidazole-6-carboxylic acid was prepared in a manner as described in Procedure 31 using Intermediates **I-1090** and **I-1188.** ES/MS m/z: 614.2 (M+H⁺). 1H NMR (400 MHz, Methanol-d4) δ 8.50 (s, 1H), 8.11 (dd, J = 8.6, 1.5 Hz, 1H), 7.91 (t, J = 7.9 Hz, 1H), 7.88 - 7.79 (m, 1H), 7.69 (dd, J = 12.6, 8.3 Hz, 2H), 6.99 (d, J = 8.2 Hz, 1H), 5.74 (s, 2H), 4.73 (s, 4H), 4.30 (s, 1H), 4.17 (d, J = 5.5 Hz, 4H), 1.09 - 1.00 (m, 2H), 0.91 (d, J = 5.4 Hz, 2H).

### Example 272: 2-(2,5-difluoro-4-(6-((5-methoxy-1,3,4-thiadiazol-2-yl)methoxy)pyridin-2-yl)benzyl)-1-((1-(fluoromethyl)cyclopropyl)methyl)-1H-benzo[d]imidazole-6-carboxylic acid

2-(2,5-difluoro-4-(6-((5-methoxy-1,3,4-thiadiazol-2-yl)methoxy)pyridin-2-yl)benzyl)-1-((1-(fluoromethyl)cyclopropyl)methyl)-1H-benzo[d]imidazole-6-carboxylic acid was prepared in a manner as described in Procedure 31 using Intermediates **I-1090** and **I-1180.** ES/MS m/z: 596.2 (M+H⁺). 1H NMR (400 MHz, Methanol-d4) δ 8.57 (s, 1H), 8.19 (dd, J = 8.6, 1.4 Hz, 1H), 8.07 - 7.96 (m, 1H), 7.87 (dd, J = 9.2, 6.6 Hz, 1H), 7.77 (d, J = 8.6 Hz, 1H), 7.65 (dd, J = 7.6, 1.6 Hz, 1H), 7.38 (dd, J = 11.3, 6.1 Hz, 1H), 6.95 (d, J = 8.2 Hz, 1H), 5.73 (s, 2H), 4.74 (d, J = 1.8 Hz, 4H), 4.30 (s, 1H), 4.18 (s, 1H), 4.16 (s, 3H), 1.09 - 0.99 (m, 2H), 0.94 - 0.77 (m, 2H).

### Example 276: 2-(2,5-difluoro-4-(6-((5-methoxy-1,3,4-thiadiazol-2-yl)methoxy)pyridin-2-yl)benzyl)-1-((1-(difluoromethyl)cyclopropyl)methyl)-1H-benzo[d]imidazole-6-carboxylic acid

2-(2, 5-difluoro-4-(6-((5-methoxy-1,3,4-thiadiazol-2-yl)methoxy)pyridin-2-yl)benzyl)-1-((1-(difluoromethyl)cyclopropyl)methyl)-1H-benzo[d]imidazole-6-carboxylic acid was prepared in a manner as described in Procedure 31 using Intermediates **I-1090** and **I-1196.** ES/MS m/z: 614.6 (M+H⁺). 1H NMR (400 MHz, Methanol-d4) δ 8.51 (s, 1H), 8.17 (d, J = 1.5 Hz, 1H), 8.06 - 7.97 (m, 1H), 7.87 (dd, J = 9.3, 6.5 Hz, 1H), 7.76 (s, 1H), 7.64 (dd, J = 7.4, 1.6 Hz, 1H), 7.35 (dd, J = 11.3, 6.0 Hz, 1H), 6.94 (dd, J = 8.3, 5.2 Hz, 1H), 5.80 - 5.41 (m, 4H), 4.71 (s, 2H), 4.16 (s, 3H), 1.15 - 0.99 (m, 5H).

**Example 368: 1-((1-(fluoromethyl)cyclopropyl)methyl)-2-(2,3,6-trifluoro-4-(5-fluoro-6-((5-methoxy-1,3,4-thiadiazol-2-yl)methoxy)pyridin-2-yl)benzyl)-1H-benzo[d]imidazole-6-carboxylic acid**
1-((1-(fluoromethyl)cyclopropyl)methyl)-2-(2,3,6-trifluoro-4-(5-fluoro-6-((5-methoxy-1,3,4-thiadiazol-2-yl)methoxy)pyridin-2-yl)benzyl)-1H-benzo[d]imidazole-6-carboxylic acid was prepared in a manner as described in Procedure 31 using Intermediate I-**1244** and **I-1180**. ES/MS m/z: 632.7 (M+H⁺). 1HNMR (400 MHz, Methanol-d4) δ 8.52 (d, J = 1.4 Hz, 1H), 8.14 (dd, J = 8.6, 1.5 Hz, 1H), 7.87 - 7.80 (m, 0H), 7.80 - 7.66 (m, 3H), 5.82 (s, 2H), 4.75 (d, J = 2.8 Hz, 4H), 4.30 (s, 1H), 4.18 (d, J = 2.0 Hz, 3H), 1.10 - 1.01 (m, 2H), 0.93 - 0.86 (m, 2H).

### Example 396: 2-(4-(6-((5-ethoxy-1,3,4-thiadiazol-2-yl)methoxy)pyridin-2-yl)-2,5-difluorobenzyl)-7-fluoro-1-(2-methoxyethyl)-1H-benzo[d]imidazole-6-carboxylic acid

2-(4-(6-((5-ethoxy-1,3,4-thiadiazol-2-yl)methoxy)pyridin-2-yl)-2,5-difluorobenzyl)-7-fluoro-1-(2-methoxyethyl)-1H-benzo[d]imidazole-6-carboxylic acid was prepared in a manner as described in Procedure 31 using Intermediate **I-1145** and **I-1325**. ES/MS m/z: 600.6 (M+H⁺). 1H NMR (400 MHz, Methanol-d4) δ 8.05 - 7.75 (m, 3H), 7.62 (dd, J = 7.5, 1.5 Hz, 1H), 7.49 (d, J = 8.6 Hz, 1H), 7.27 (dd, J = 11.4, 6.0 Hz, 1H), 6.93 (d, J = 8.2 Hz, 1H), 5.72 (s, 2H), 4.73 (t, J = 5.0 Hz, 2H), 4.64 - 4.49 (m, 4H), 3.84 (t, J = 4.9 Hz, 2H), 3.32 (dt, J = 3.3, 1.7 Hz, 3H), 1.44 (t, J = 7.1 Hz, 3H).

### Example 402: 2-(4-(6-((4-chloro-6-(methylcarbamoyl)pyridin-3-yl)methoxy)pyridin-2-yl)-2-fluorobenzyl)-1-((1-(fluoromethyl)cyclopropyl)methyl)-1H-benzo [d] imidazole-6-carboxylic acid

2-(4-(6-((4-chloro-6-(methylcarbamoyl)pyridin-3-yl)methoxy)pyridin-2-yl)-2-fluorobenzyl)-1-((1-(fluoromethyl)cyclopropyl)methyl)-1H-benzo[d]imidazole-6-carboxylic acid was prepared in a manner as described in Procedure 31 using Intermediates **I-1273** and **I-1329.** ES/MS m/z: 632.4 (M+H⁺). 1HNMR (400 MHz, Methanol-d4) δ 8.76 (s, 1H), 8.59 (t, J = 1.0 Hz, 1H), 8.21 (dd, J = 8.6, 1.4 Hz, 1H), 8.13 (s, 1H), 8.00 - 7.90 (m, 2H), 7.90 - 7.82 (m, 1H), 7.82 - 7.71 (m, 1H), 7.59 (d, J = 7.4 Hz, 1H), 7.51 (t, J = 8.1 Hz, 1H), 6.95 (d, J = 8.2 Hz, 1H), 5.74 (s, 2H), 4.75 (d, J = 5.1 Hz, 4H), 4.30 (s, 1H), 4.18 (s, 1H), 2.96 (s, 3H), 1.11 - 0.96 (m, 2H), 0.96 - 0.74 (m, 2H).

### Example 419: (S)-2-(4-(6-((5-cyano-3-fluorothiophen-2-yl)methoxy)pyridin-2-yl)-2,5-difluorobenzyl)-1-(4,4-dimethyltetrahydrofuran-3-yl)-1H-benzo[d]imidazole-6-carboxylic acid

(S)-2-(4-(6-((5-cyano-3-fluorothiophen-2-yl)methoxy)pyridin-2-yl)-2,5-difluorobenzyl)-1-(4,4-dimethyltetrahydrofuran-3-yl)-1H-benzo[d]imidazole-6-carboxylic acid was prepared in a manner as described in Procedure 31 using Intermediates **I-1138** and **I-22**. ES/MS m/z: 619.0 (M+H⁺). 1H NMR (400 MHz, DMSO-d6) δ 8.50 (s, 1H), 8.03 - 7.96 (m, 2H), 7.92 (t, J = 7.9 Hz, 1H), 7.82 (dd, J = 8.4, 1.5 Hz, 1H), 7.64 (d, J = 8.5 Hz, 1H), 7.59 (dd, J = 7.5, 1.6 Hz, 1H), 7.48 (dd, J = 11.5, 6.1 Hz, 1H), 6.96 (d, J = 8.2 Hz, 1H), 5.70 (s, 2H), 5.02 (d, J = 6.7 Hz, 1H), 4.60 - 4.50 (m, 2H), 4.48 - 4.37 (m, 2H), 3.79 (d, J = 8.7 Hz, 1H), 3.73 (d, J = 8.6 Hz, 1H), 1.33 (s, 3H), 0.60 (s, 3H).

### Example 450: 2-(4-(6-((4-chloro-6-(methylcarbamoyl)pyridin-3-yl)methoxy)pyridin-2-yl)-2,3,6-trifluorobenzyl)-1-((1-(fluoromethyl)cyclopropyl)methyl)-1H-benzo[d]imidazole-6-carboxylic acid

2-(4-(6-((4-chloro-6-(methylcarbamoyl)pyridin-3-yl)methoxy)pyridin-2-yl)-2,3,6-trifluorobenzyl)-1-((1-(fluoromethyl)cyclopropyl)methyl)-1H-benzo[d]imidazole-6-carboxylic acid was prepared in a manner as described in Procedure 31, starting with Intermediates **I-1188** and **I-1273.** 1HNMR (400 MHz, Methanol-d4) δ 8.75 (s, 1H), 8.51 (d, J = 1.4 Hz, 1H), 8.12 (d, J = 7.1 Hz, 2H), 7.89 (dd, J = 8.3, 7.4 Hz, 1H), 7.85 - 7.64 (m, 2H), 7.61 (dd, J = 7.4, 1.6 Hz, 1H), 7.02 (d, J = 8.2 Hz, 1H), 5.71 (s, 2H), 4.74 (s, 4H), 4.30 (s, 1H), 4.18 (s, 1H), 2.96 (s, 3H), 1.02 (t, J = 5.2 Hz, 2H), 0.91 (d, J = 5.3 Hz, 2H). ES/MS m/z: 668.2 (M+H⁺).

### Example 451: 2-((3'-((4-chloro-6-(methylcarbamoyl)pyridin-3-yl)methoxy)-2,3,5-trifluoro-[1,1'-biphenyl]-4-yl)methyl)-1-((1-(fluoromethyl)cyclopropyl)methyl)-1H-

2-((3'-((4-chloro-6-(methylcarbamoyl)pyridin-3-yl)methoxy)-2,3,5-trifluoro-[1,1'-biphenyl]-4-yl)methyl)-1-((1-(fluoromethyl)cyclopropyl)methyl)-1H-benzo[d]imidazole-6-carboxylic acid was prepared in a manner as described in Procedure 31, starting with Intermediates **I-1343** and **I-1273.** 1H NMR (400 MHz, Methanol-d4) δ 8.80 (s, 1H), 8.56 (d, J = 1.4 Hz, 1H), 8.24 - 8.09 (m, 2H), 7.74 (d, J = 8.6 Hz, 1H), 7.50 (t, J = 8.0 Hz, 1H), 7.37 - 7.25 (m, 3H), 7.20 (ddd, J = 8.3, 2.6, 0.9 Hz, 1H), 5.38 (s, 2H), 4.79 (d, J = 8.3 Hz, 4H), 4.31 (s, 1H), 4.19 (s, 1H), 2.98 (s, 3H), 1.06 (dt, J = 6.4, 4.9 Hz, 2H), 1.00 - 0.85 (m, 2H). ES/MS m/z: 667.2 (M+H⁺).

### Example 372: 2-[[4-[6-[(5-cyanothiazol-2-yl)methoxy]-2-pyridyl]-2,5-difluorophenyl]methyl]-7-fluoro-3-[[(2S)-oxetan-2-yl]methyl]benzimidazole-5-carboxylic acid

### Procedure 32

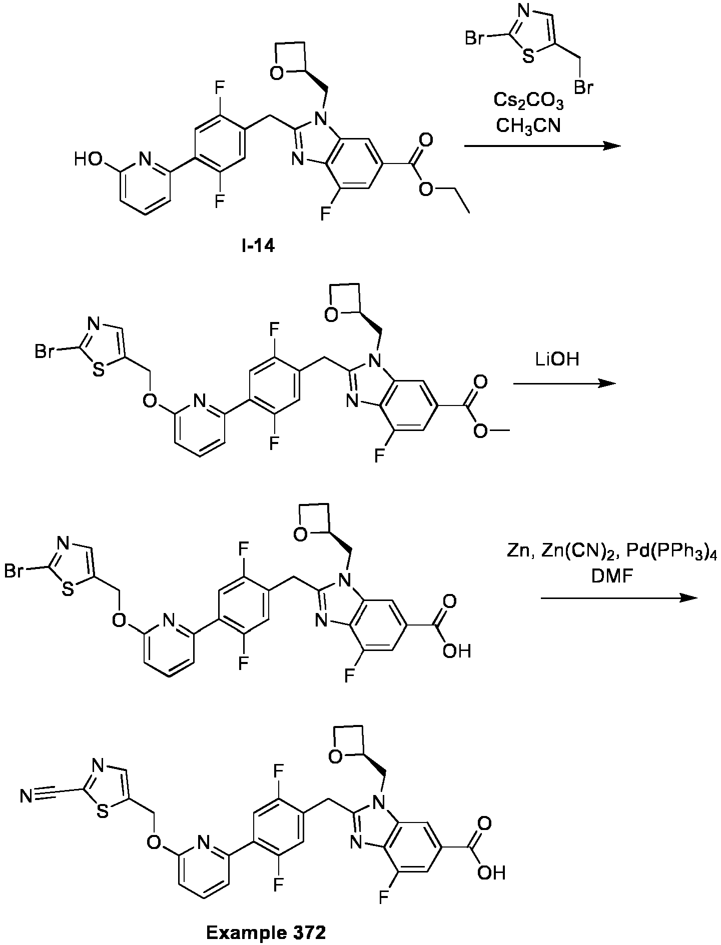

**Ethyl 2-[[4-[6-[(5-bromothiazol-2-yl)methoxy]-2-pyridyl]-2,5-difluorophenyl]methyl]-7-fluoro-3-[[(2S)-oxetan-2-yl]methyl]benzimidazole-5-carboxylate:** A suspension of 5-bromo-2-(bromomethyl)thiazole (57 mg, 0.22 mmol), ethyl 2-[[2,5-difluoro-4-(6-hydroxy-2-pyridyl)phenyl]methyl]-7-fluoro-3-[[(2S)-oxetan-2-yl]methyl]benzimidazole-5-carboxylate (100 mg, 0.20 mmol), and cesium carbonate (106 mg, 0.32 mmol) in CH₃CN (2 mL) was heated at 60 °C for 50 min. Next, the mixture was diluted with EtOAc and washed with brine. The mixture was dried over sodium sulfate, concentrated, and purified by chromatography (eluent: EtOAc/hexanes) to give desired product. ES/MS: 674.6 (M+H⁺); 1H NMR (400 MHz, Chloroform-d) δ 7.94 (d, J = 1.2 Hz, 1H), 7.91 - 7.81 (m, 1H), 7.77 - 7.65 (m, 3H), 7.57 (d, J = 7.6 Hz, 1H), 7.13 (dd, J = 11.4, 6.0 Hz, 1H), 6.86 (d, J = 8.2 Hz, 1H), 5.73 (d, J = 1.0 Hz, 2H), 5.14 (qd, J = 6.9, 2.5 Hz, 1H), 4.78 - 4.58 (m, 1H), 4.58 - 4.27 (m, 6H), 2.75 (ddt, J = 14.1, 11.6, 6.9 Hz, 1H), 2.50 - 2.29 (m, 1H).

**2-[[4-[6-[(5-bromothiazol-2-yl)methoxy]-2-pyridyl]-2,5-difluoro-phenyl]methyl]-7-fluoro-3-[[(2S)-oxetan-2-yl]methyl]benzimidazole-5-carboxylic acid:** A solution of ethyl 2-[[4-[6-[(5-bromothiazol-2-yl)methoxy]-2-pyridyl]-2,5-difluoro-phenyl]methyl]-7-fluoro-3-[[(2S)-oxetan-2-yl]methyl]benzimidazole-5-carboxylate (53.8 mg, 0.0799 mmol) and Lithium hydroxide, monohydrate (300 mmol/L, 0.799 mL, 0.240 mmol) in CH₃CN (3 mL) in a 40 ml reaction vial was heated at 100 °C for 30 min. The mixture was then diluted with EtOAc and brine Next, 0.200 mL 1M citric acid was added to the mixture and the organic extract was dried over sodium sulfate, concentrated to give desired product, which was carried onto next step without further purification. ES/MS: 645, 647 (M+H⁺).

**2-[[4-[6-[(5-cyanothiazol-2-yl)methoxy]-2-pyridyl]-2,5-difluoro-phenyl]methyl]-7-fluoro-3-[[(2S)-oxetan-2-yl]methyl]benzimidazole-5-carboxylic acid (Example 372):** In a 40 ml reaction vial, a suspension of 2-[[4-[6-[(5-bromothiazol-2-yl)methoxy]-2-pyridyl]-2,5-difluorophenyl]methyl]-3-[[(2S)-oxetan-2-yl]methyl]benzimidazole-5-carboxylic acid (50.8 mg, 0.0810 mmol), tetrakis(triphenylphosphine)palladium(O) (46.8 mg, 0.0405 mmol), Zinc cyanide (19.0 mg, 0.162 mmol) and Zinc powder (2.50 mg, 0.0382 mmol) in DMF (3 mL) was degassed with argon for 3 min, then heated at 100 °C for 4 hr. Upon completion of time, the mixture was diluted with EtOAc, washed with 5% LiCl and brine. The organic extract was dried over sodium sulfate, filtered and concentrated. Purified by RP-HPLC (eluent: MeCN/H₂O). The resulting product fractions were diluted with EtOAc and neutralized with sodium bicarbonate solution. The organic extract was dried over sodium sulfate, filtered and concentrated to give title product. ES/MS: 592 (M+H⁺); 1HNMR (400 MHz, Methanol-d4) δ 8.42 (s, 1H), 8.16 (d, J = 1.2 Hz, 1H), 7.93 - 7.75 (m, 2H), 7.72 - 7.55 (m, 2H), 7.19 (dd, J = 11.5, 6.0 Hz, 1H), 6.98 (d, J = 8.2 Hz, 1H), 5.86 (s, 2H), 5.26 - 5.05 (m, 1H), 4.73 (dd, J = 15.7, 7.0 Hz, 1H), 4.68 - 4.34 (m, 6H), 2.92 - 2.70 (m, 1H), 2.48 (dd, J = 11.4, 8.8 Hz, 1H).

### Example 403: (S)-2-(4-(6-((4-((2-chloropyrimidin-5-yl)ethynyl)benzyl)oxy)pyridin-2-yl)-2,5-difluorobenzyl)-1-(oxetan-2-ylmethyl)-1H-benzo[d]imidazole-6-carboxylic acid

### Procedure 33

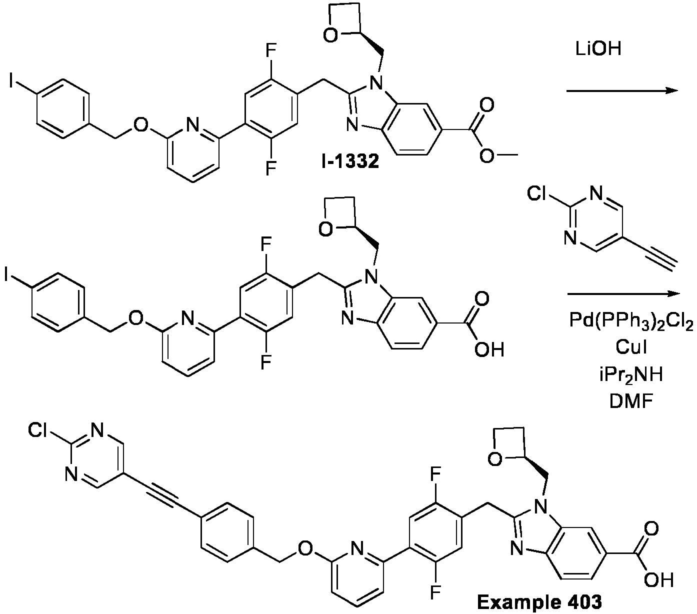

**(S)-2-(2,5-difluoro-4-(6-((4-iodobenzyl)oxy)pyridin-2-yl)benzyl)-1-(oxetan-2-ylmethyl)-1H-benzo[d]imidazole-6-carboxylic acid:** A mixture of methyl (S)-2-(2,5-difluoro-4-(6-((4-iodobenzyl)oxy)pyridin-2-yl)benzyl)-1-(oxetan-2-ylmethyl)-1H-benzo[d]imidazole-6-carboxylate (I-1332, 30 mg, 1.0 equivalent), lithium hydroxide monohydrate (1.1 mg, 1.0 equivalent), acetonitrile (1.5 mL), and water (0.5 mL) was stirred at 80 °C for 1 hr. Next, the mixture was concentrated in vacuo to yield the title compound, which was carried forward without further purification.

**(S)-2-(4-(6-((4-((2-chloropyrimidin-5-yl)ethynyl)benzyl)oxy)pyridin-2-yl)-2,5-difluorobenzyl)-1-(oxetan-2-ylmethyl)-1H-benzo[d]imidazole-6-carboxylic acid (Example 403):** A mixture of crude (S)-2-(2,5-difluoro-4-(6-((4-iodobenzyl)oxy)pyridin-2-yl)benzyl)-1-(oxetan-2-ylmethyl)-1H-benzo[d]imidazole-6-carboxylic acid (20 mg, 1.0 equivalent), 2-chloro-5-ethynyl-pyrimidine (20 mg, 5.0 equivalent), Bis(triphenylphosphine)palladium Chloride (4.2 mg, 0.20 equivalent), copper(I) iodide (1.1 mg, 0.20 equivalent), diisopropylamine (40 µL, 10 equivalent), and DMF (1.0 mL) was stirred at 90 °C for 1 hr. upon completion, the mixture was purified by reverse-phase preparative HPLC (acetonitrile/water, 0.1% TFA) to yield (S)-2-(4-(6-((4-((2-chloropyrimidin-5-yl)ethynyl)benzyl)oxy)pyridin-2-yl)-2, 5-difluorobenzyl)-1-(oxetan-2-ylmethyl)-1H-benzo[d]imidazole-6-carboxylic acid **(Example 403).** 1H NMR (400 MHz, Chloroform-d) δ 8.75 (s, 2H), 8.21 (d, J = 1.6 Hz, 1H), 8.14 - 8.04 (m, 1H), 7.92 (d, J = 8.6 Hz, 1H), 7.83 (dd, J = 10.8, 6.2 Hz, 1H), 7.80 - 7.69 (m, 1H), 7.65 - 7.57 (m, 1H), 7.56 - 7.47 (m, 2H), 7.28 - 7.19 (m, 1H), 6.88 - 6.80 (m, 1H), 5.50 (s, 2H), 5.41 (s, 1H), 5.19 (q, J = 5.1, 2.6 Hz, 1H), 5.08 (t, J = 2.8 Hz, 1H), 4.78 - 4.55 (m, 2H), 4.48 (dt, J = 10.7, 6.5 Hz, 2H), 3.82 - 3.72 (m, 2H), 3.72 - 3.65 (m, 1H), 2.81 (dd, J = 11.7, 6.8 Hz, 1H), 2.55 - 2.36 (m, 1H). ES/MS m/z: 678.3 (M+H⁺).

### Comparative Example 1 (CE-1)

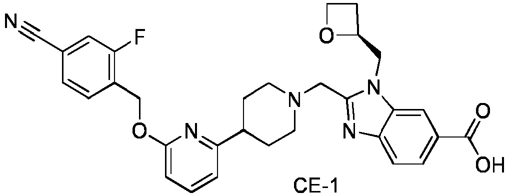

**(S)-2-((4-(6-((4-cyano-2-fluorobenzyl)oxy)pyridin-2-yl)piperidin-1-yl)methyl)-1-(oxetan-2-ylmethyl)-1H-benzo[d]imidazole-6-carboxylic acid:** (S)-2-((4-(6-((4-cyano-2-fluorobenzyl)oxy)pyridin-2-yl)piperidin-1-yl)methyl)-1-(oxetan-2-ylmethyl)-1H-benzo[d]imidazole-6-carboxylic acid was prepared in a manner as described in Patent Application WO2018/109607 (see Example 4A-01) and references therein.

### Comparative Example 2 (CE-2)

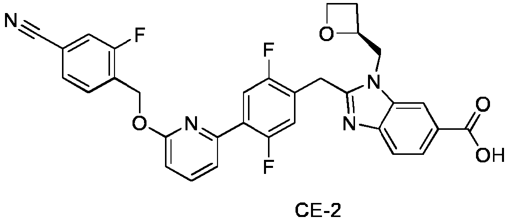

**(S)-2-(4-(6-((4-cyano-2-fluorobenzyl)oxy)pyridin-2-yl)-2,5-difluorobenzyl)-1-(oxetan-2-ylmethyl)-1H-benzo[d]imidazole-6-carboxylic acid (Comparative Example 2):** (S)-2-(4-(6-((4-cyano-2-fluorobenzyl)oxy)pyridin-2-yl)-2, 5-difluorobenzyl)-1-(oxetan-2-ylmethyl)-1H-benzo[d]imidazole-6-carboxylic acid was prepared in a manner as described in Patent Application WO2021/081207 (see Example 444) and references therein.

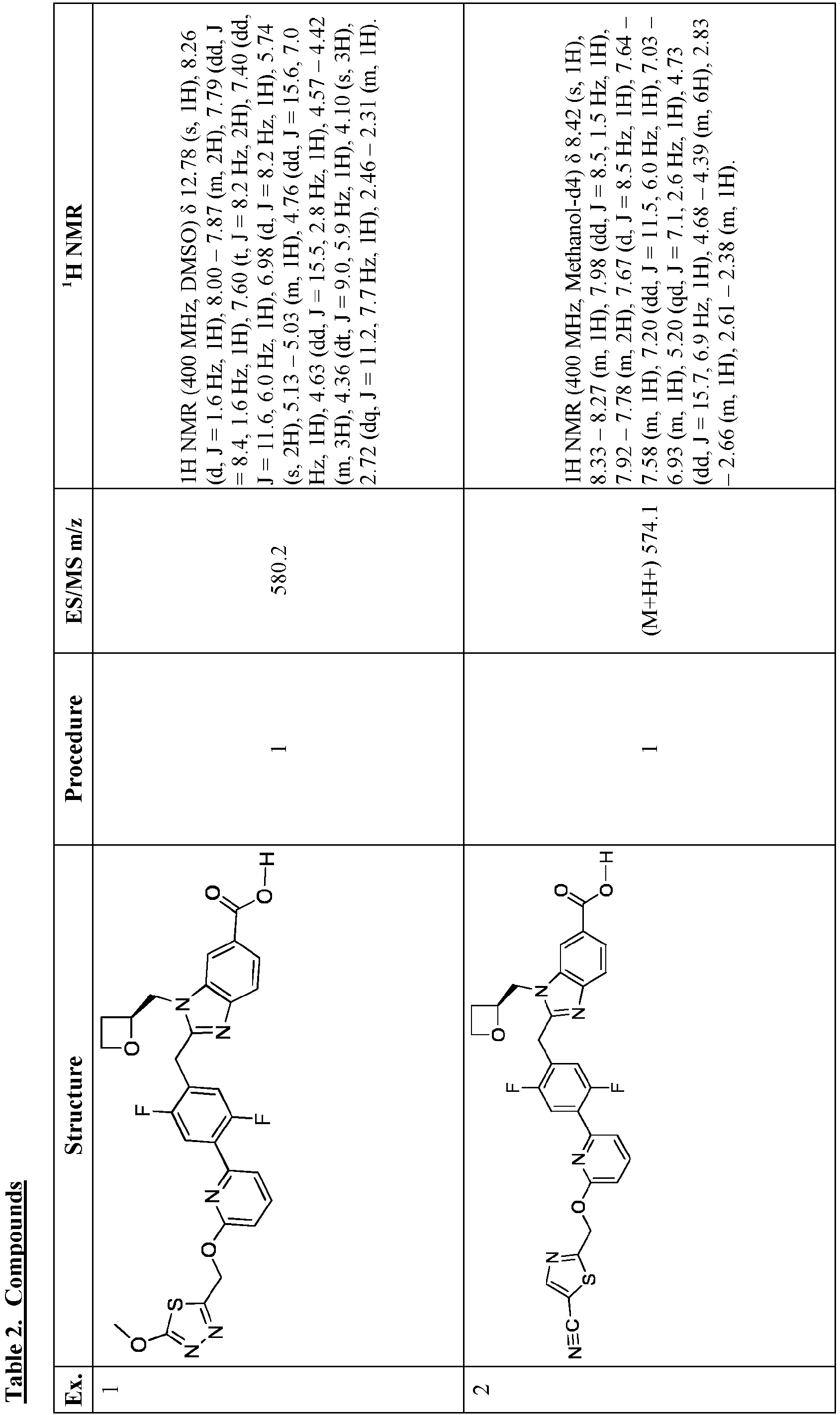

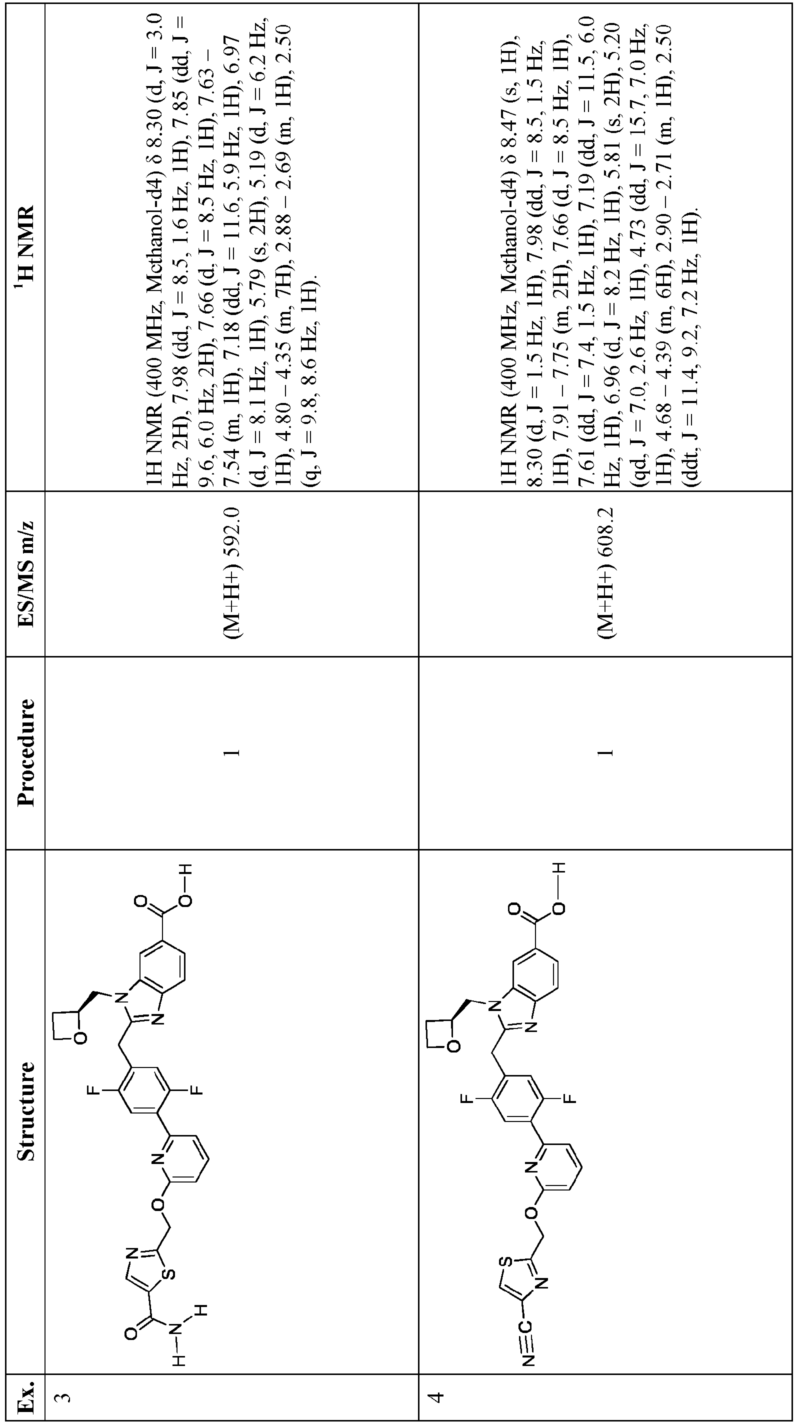

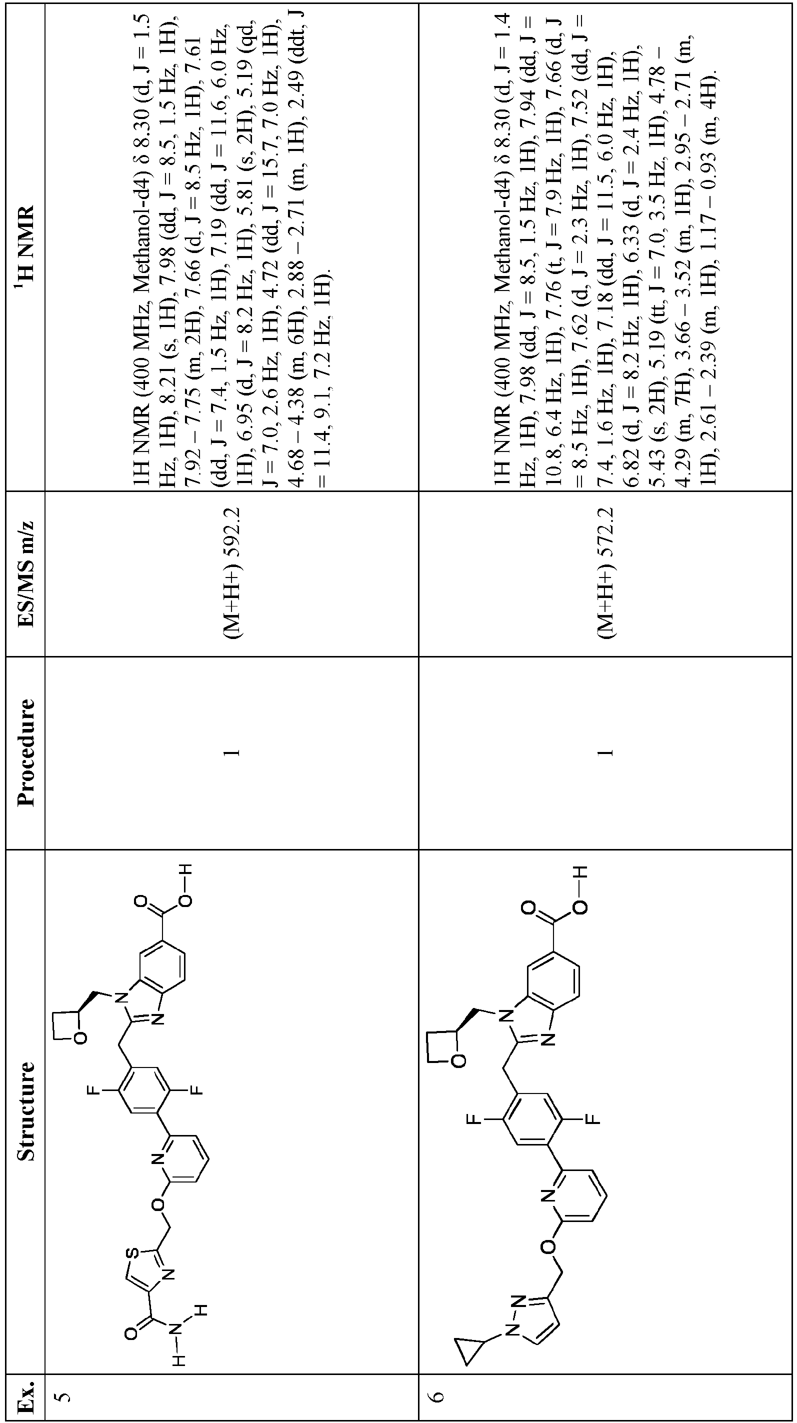

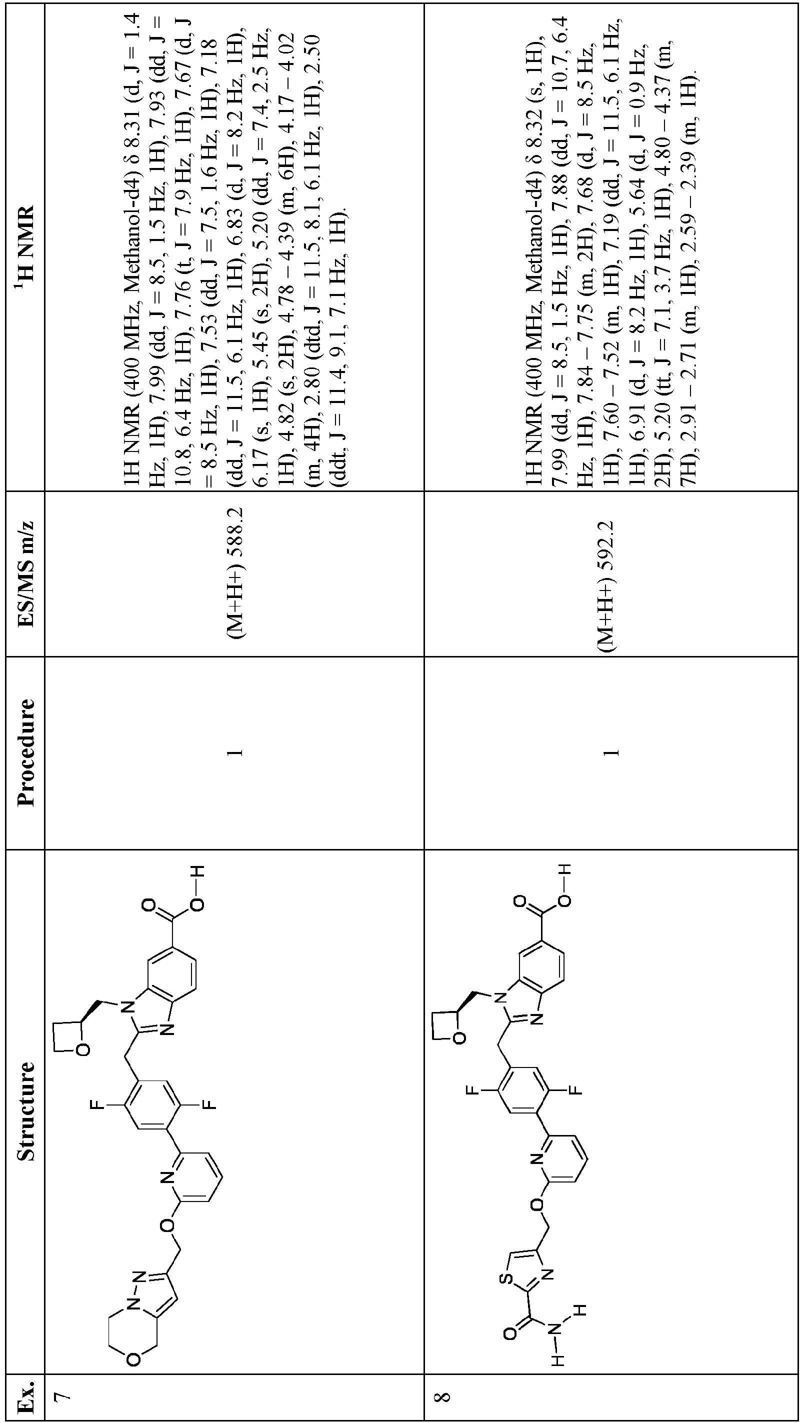

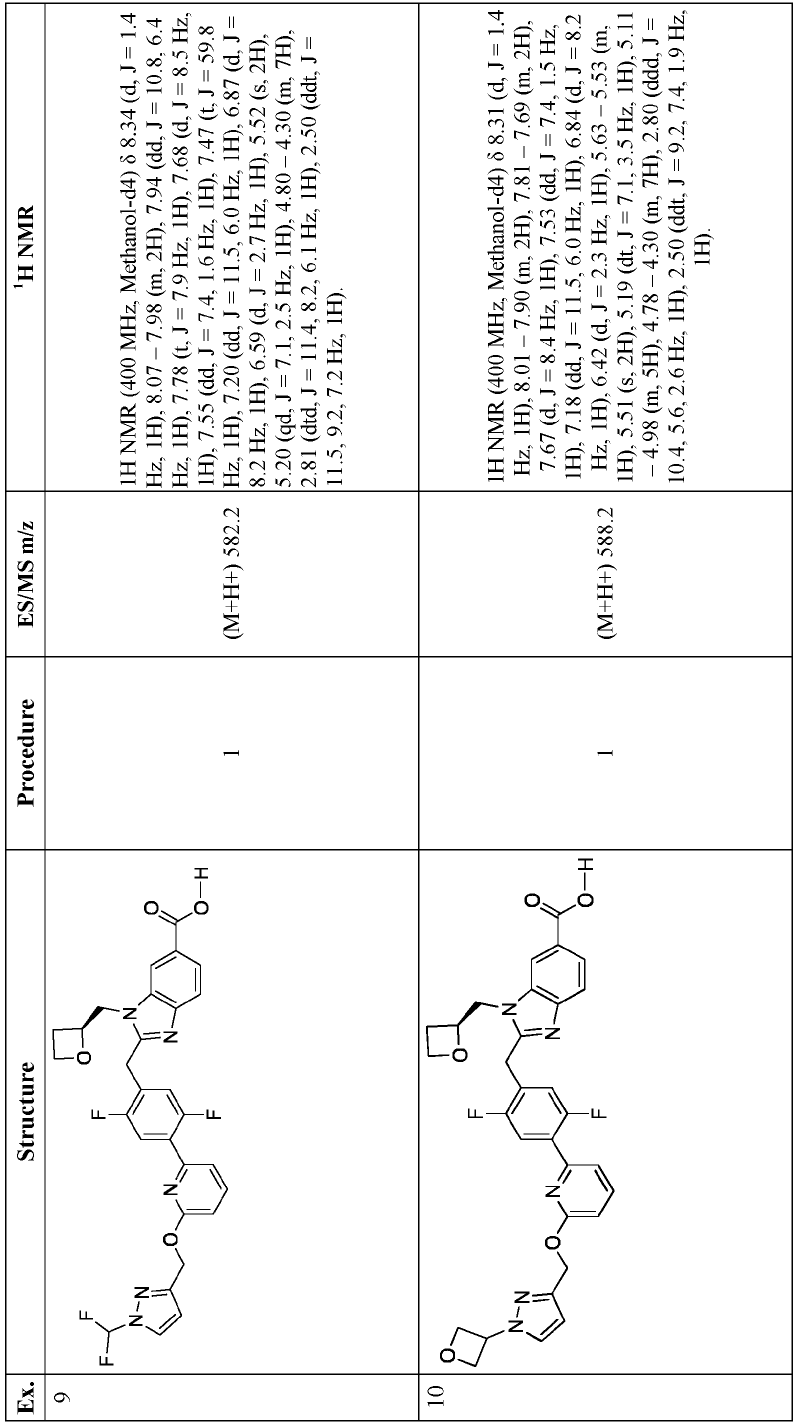

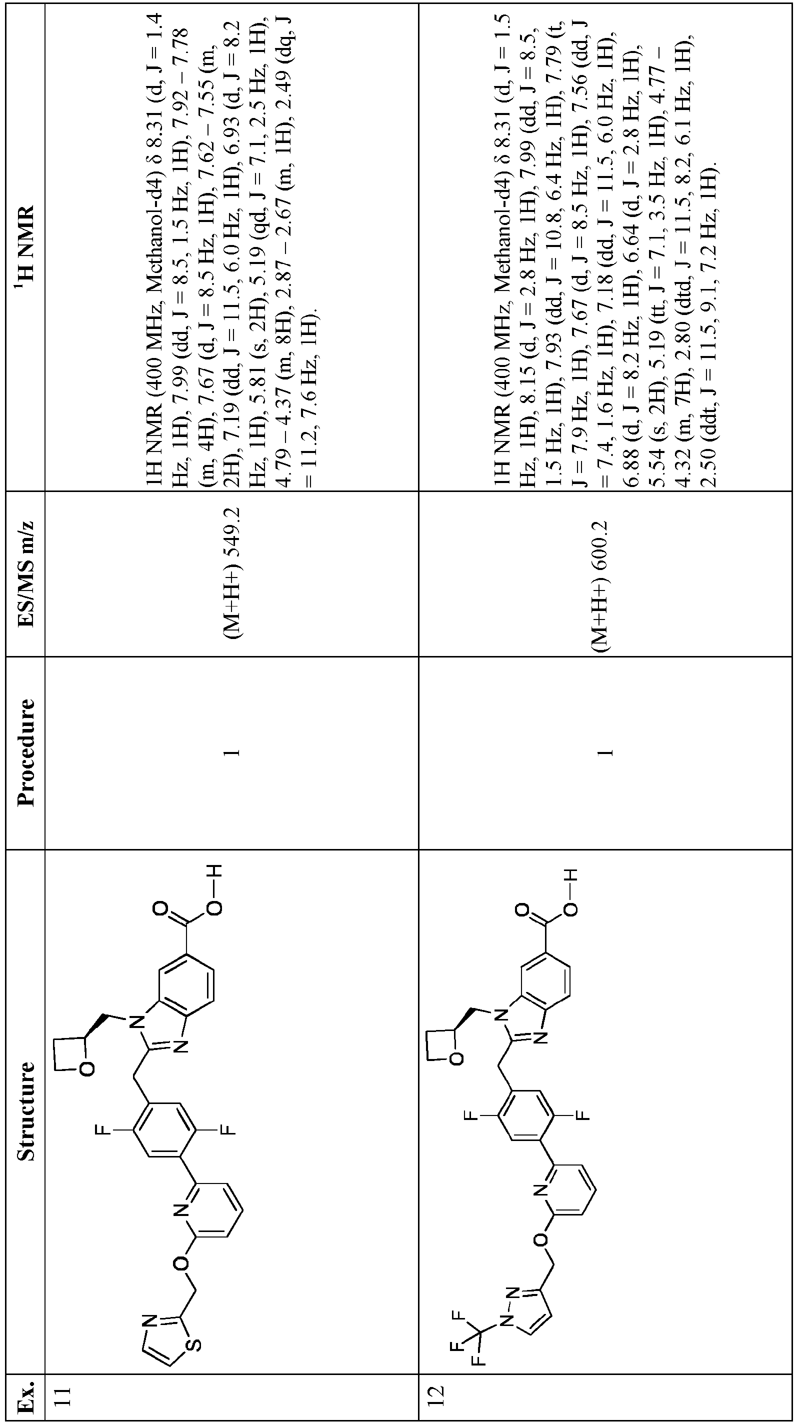

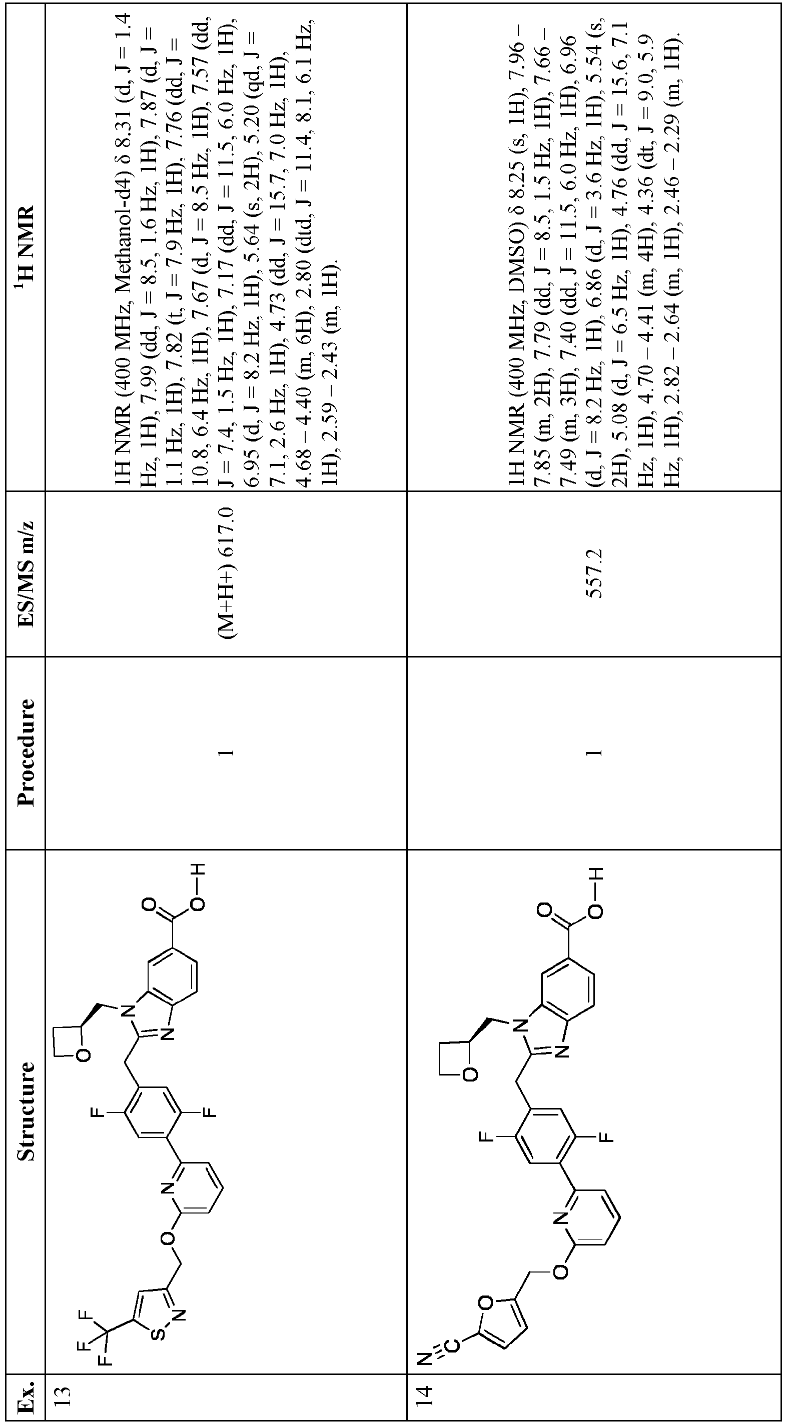

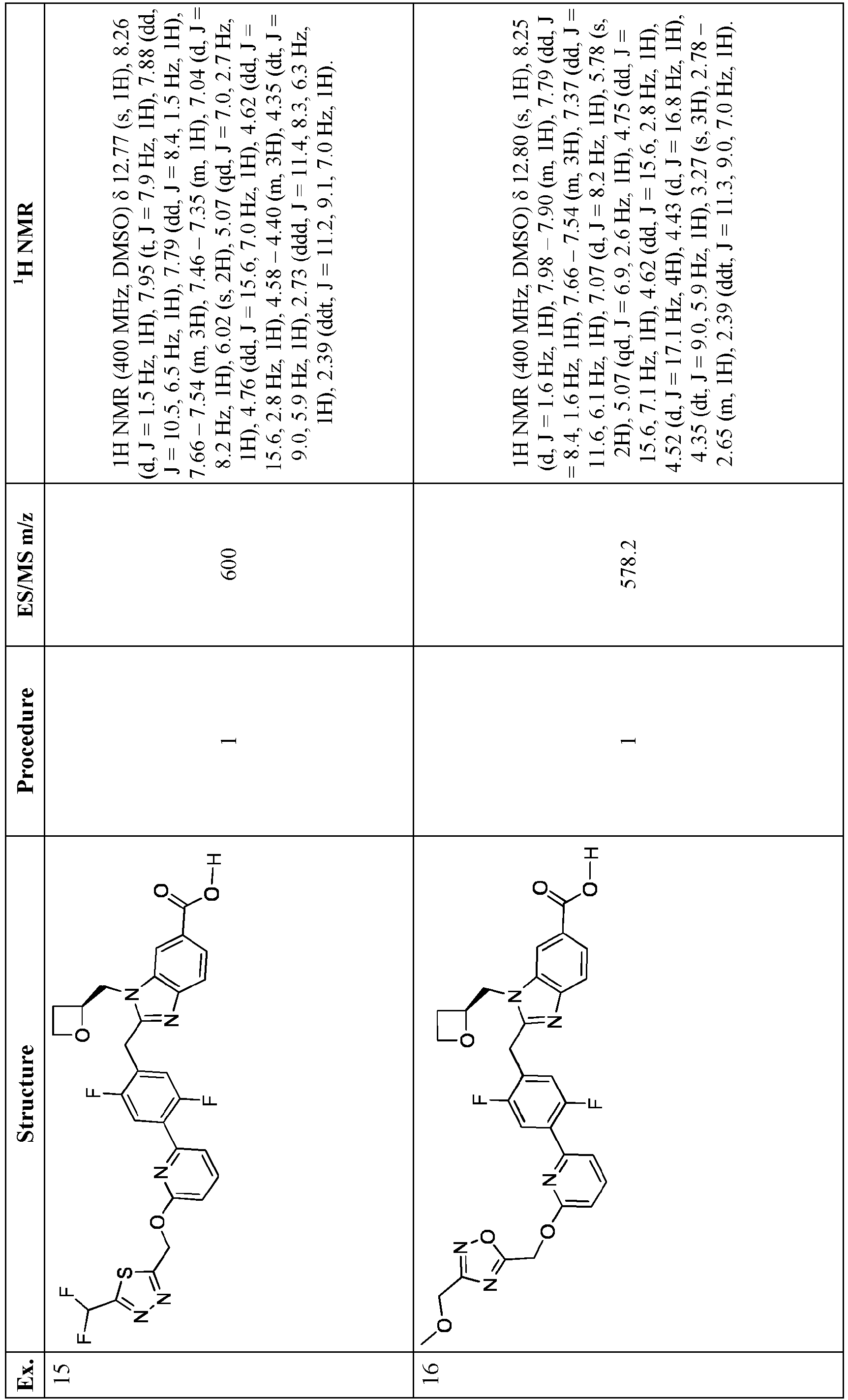

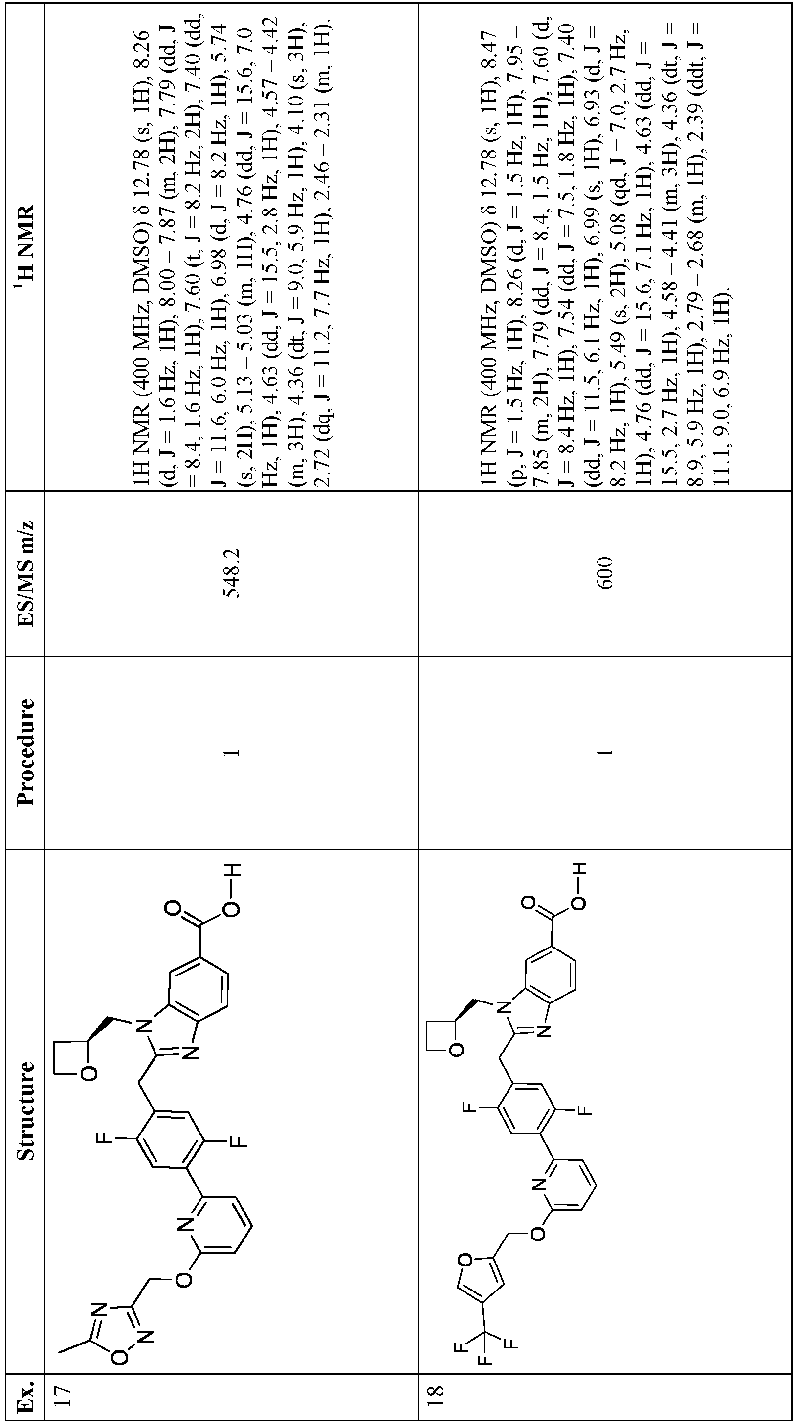

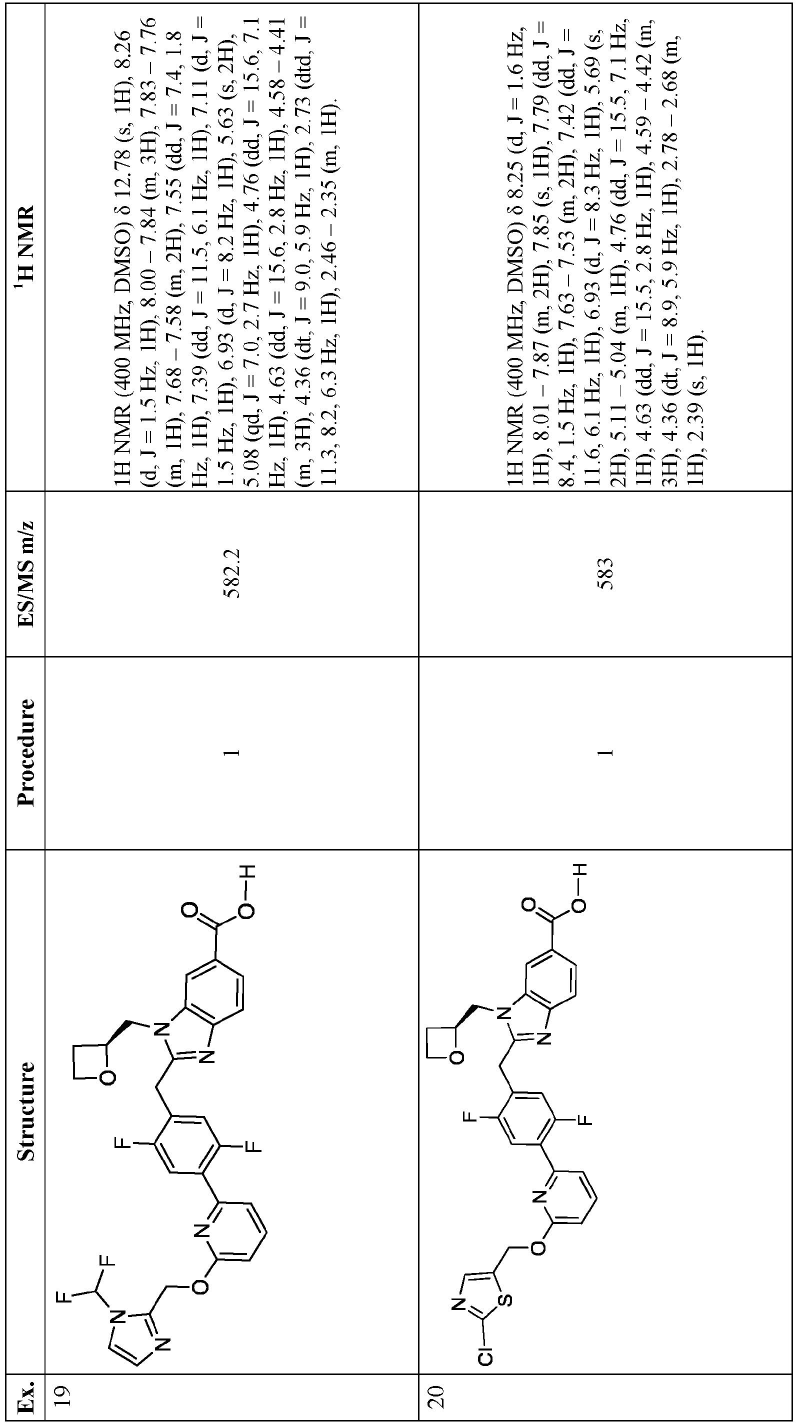

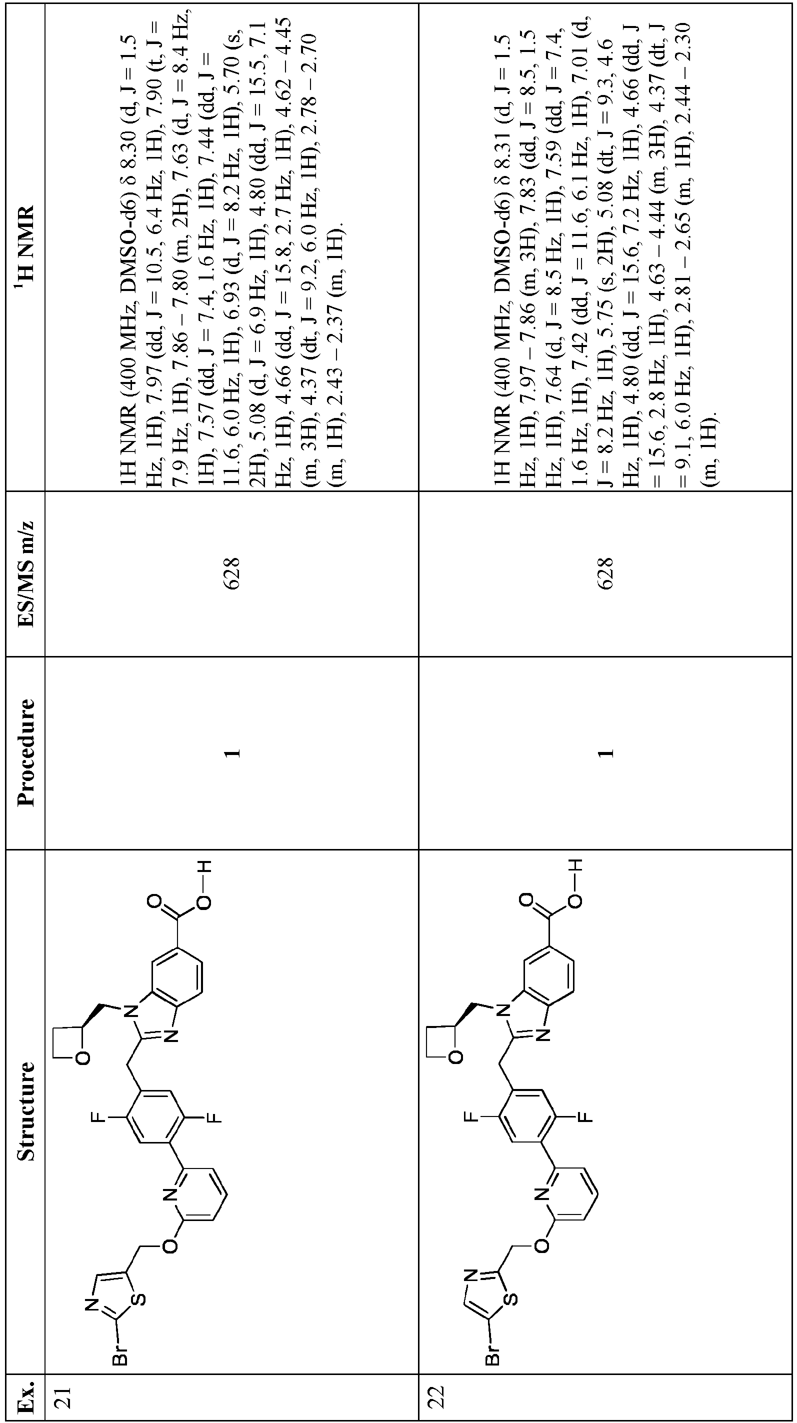

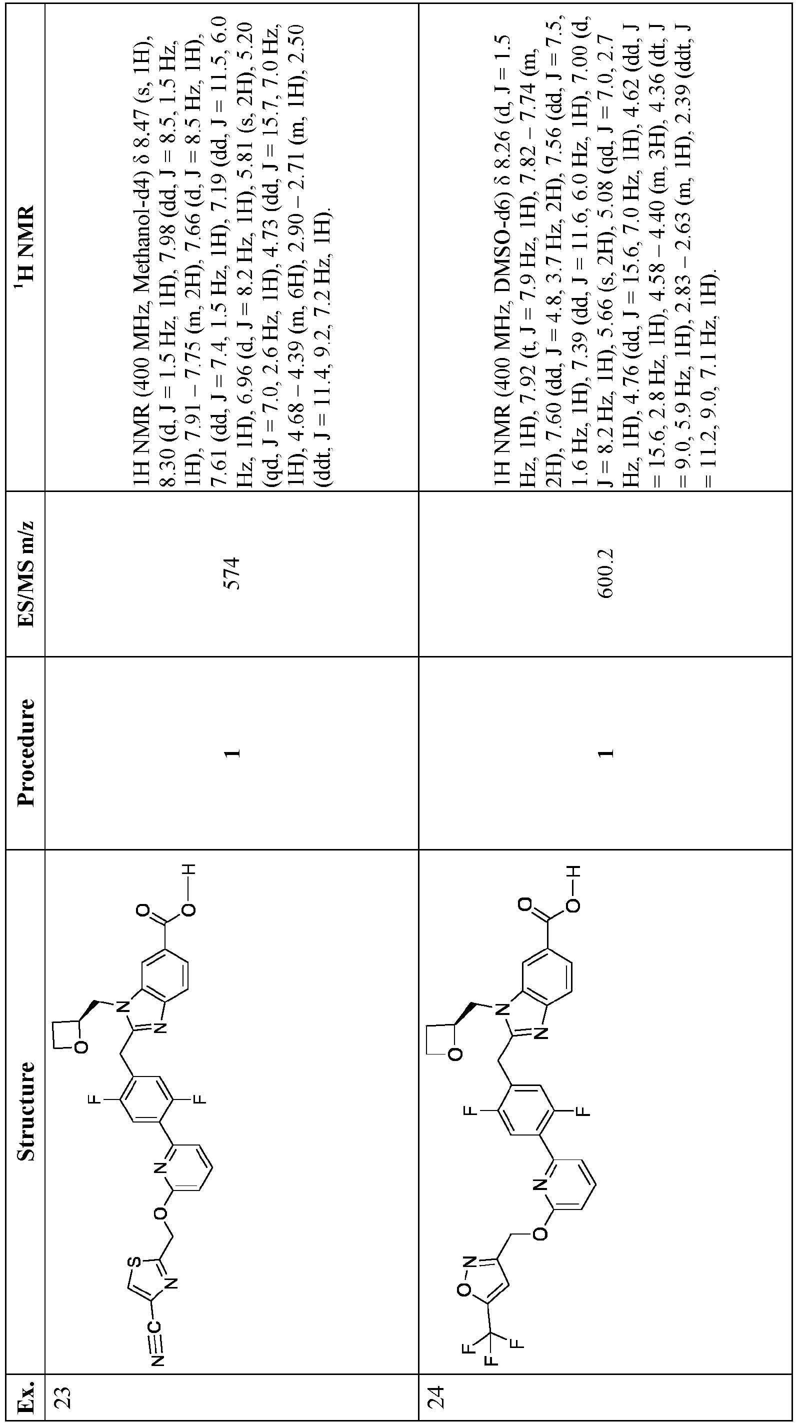

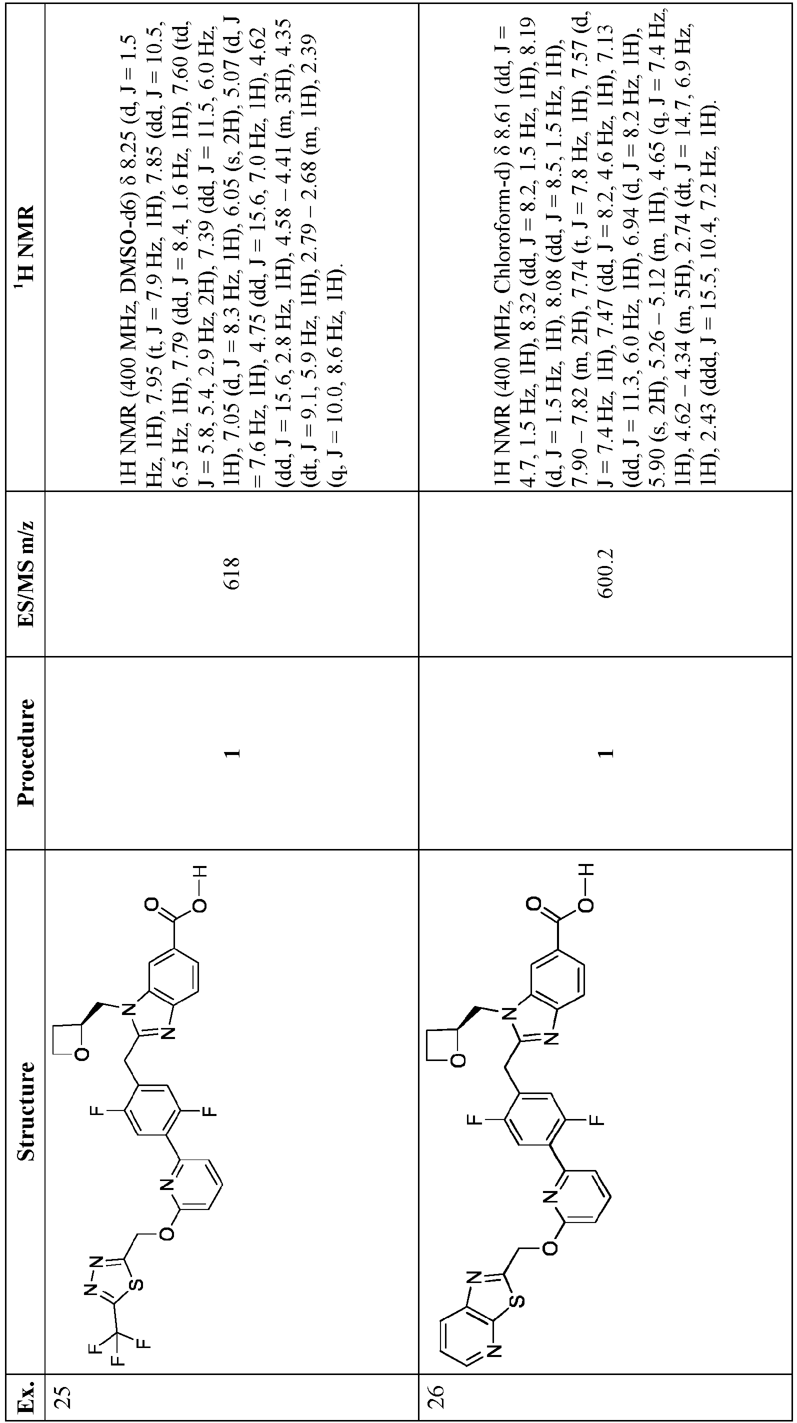

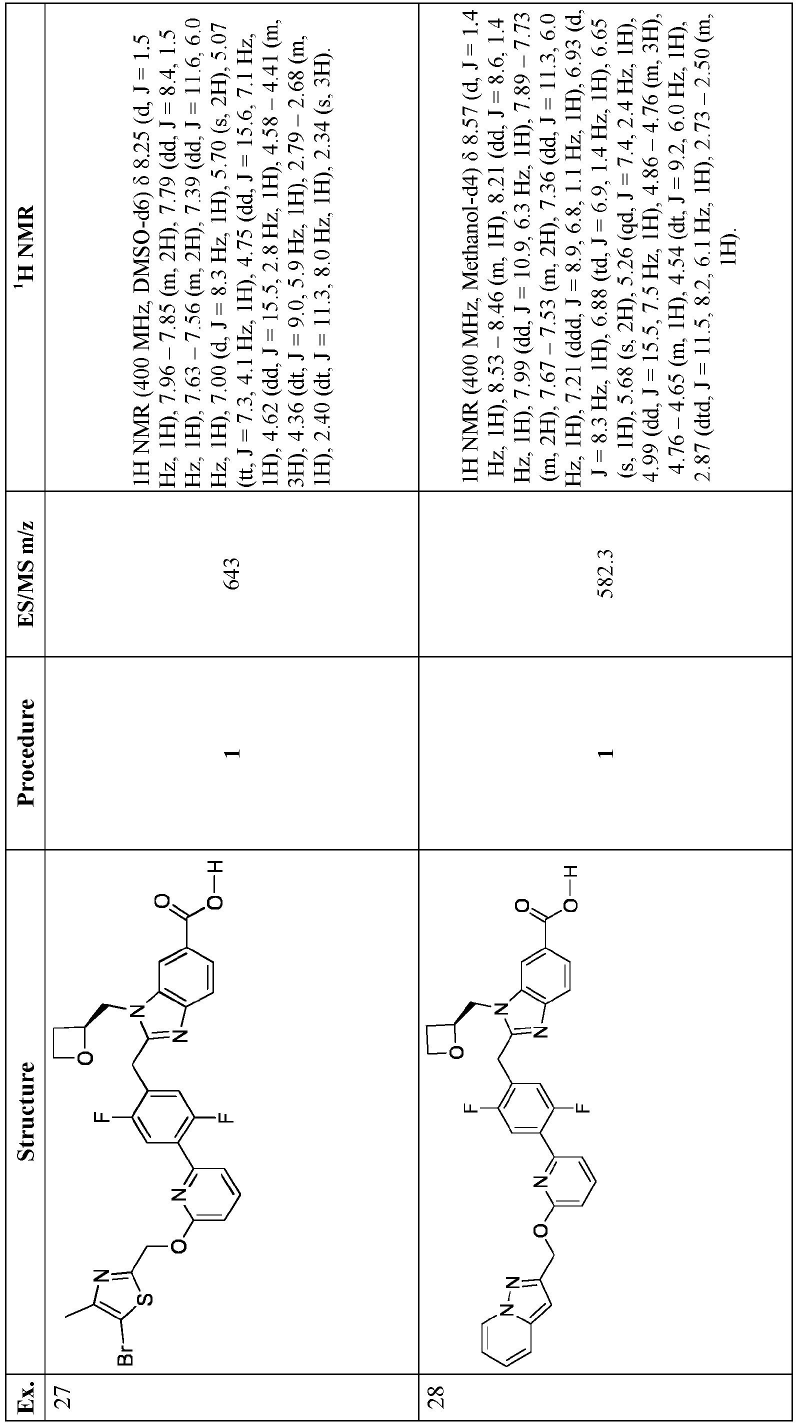

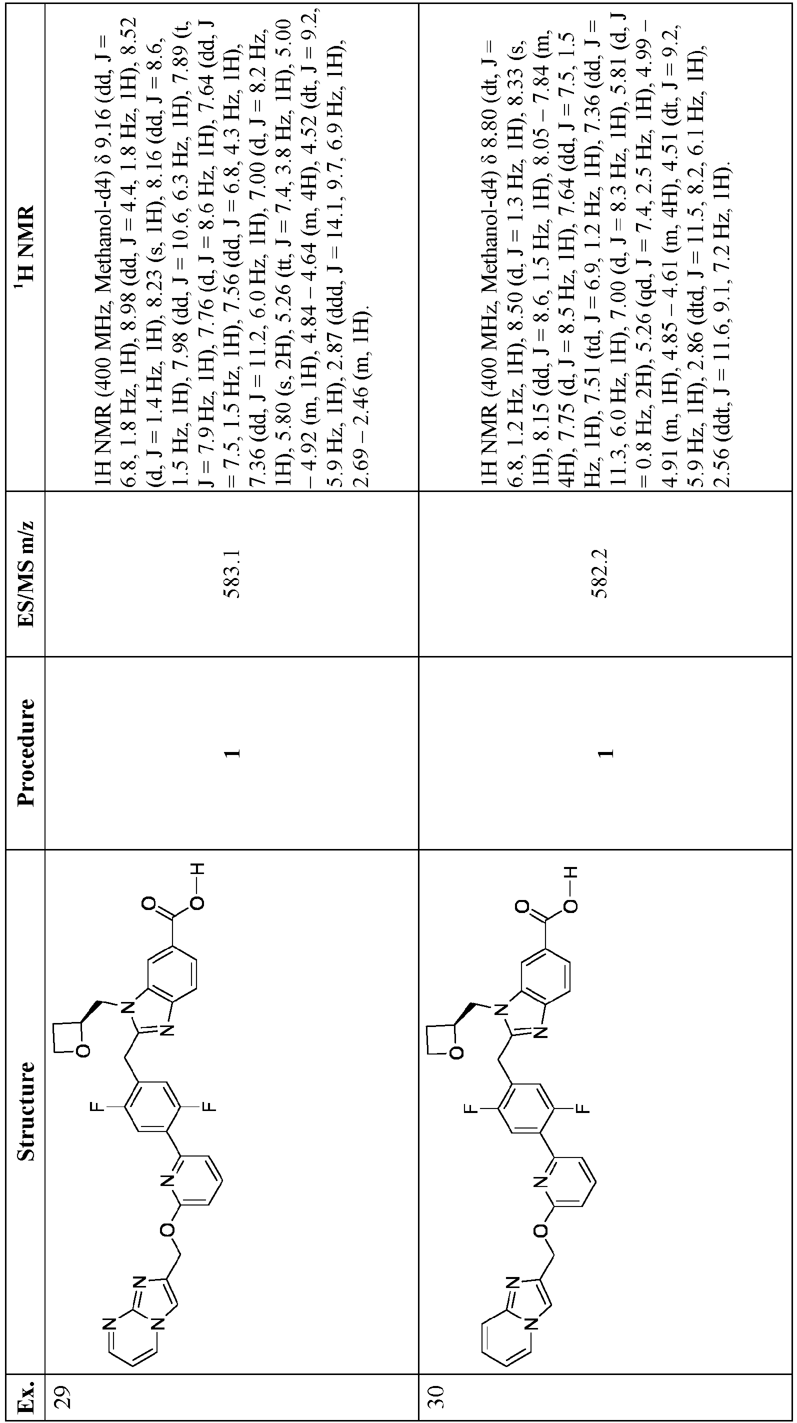

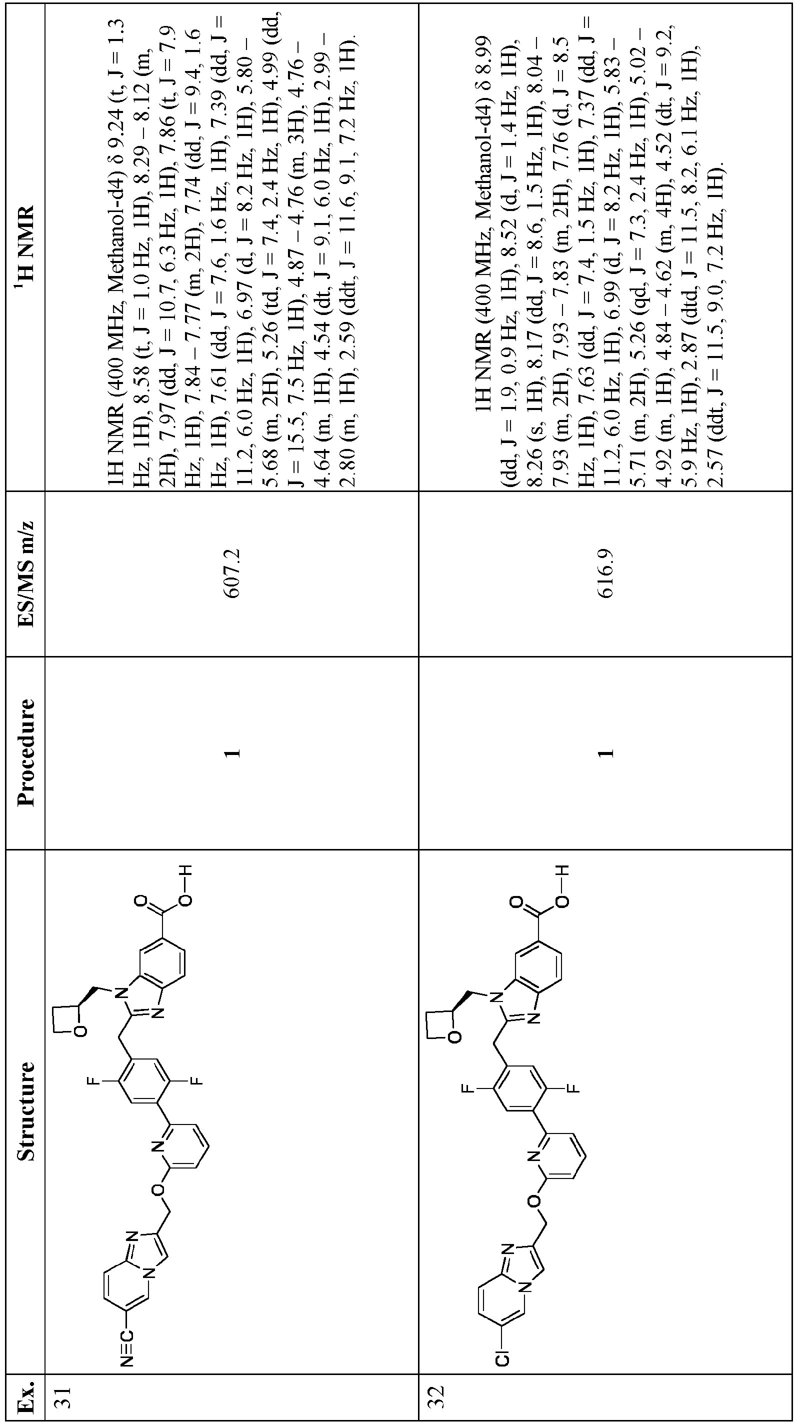

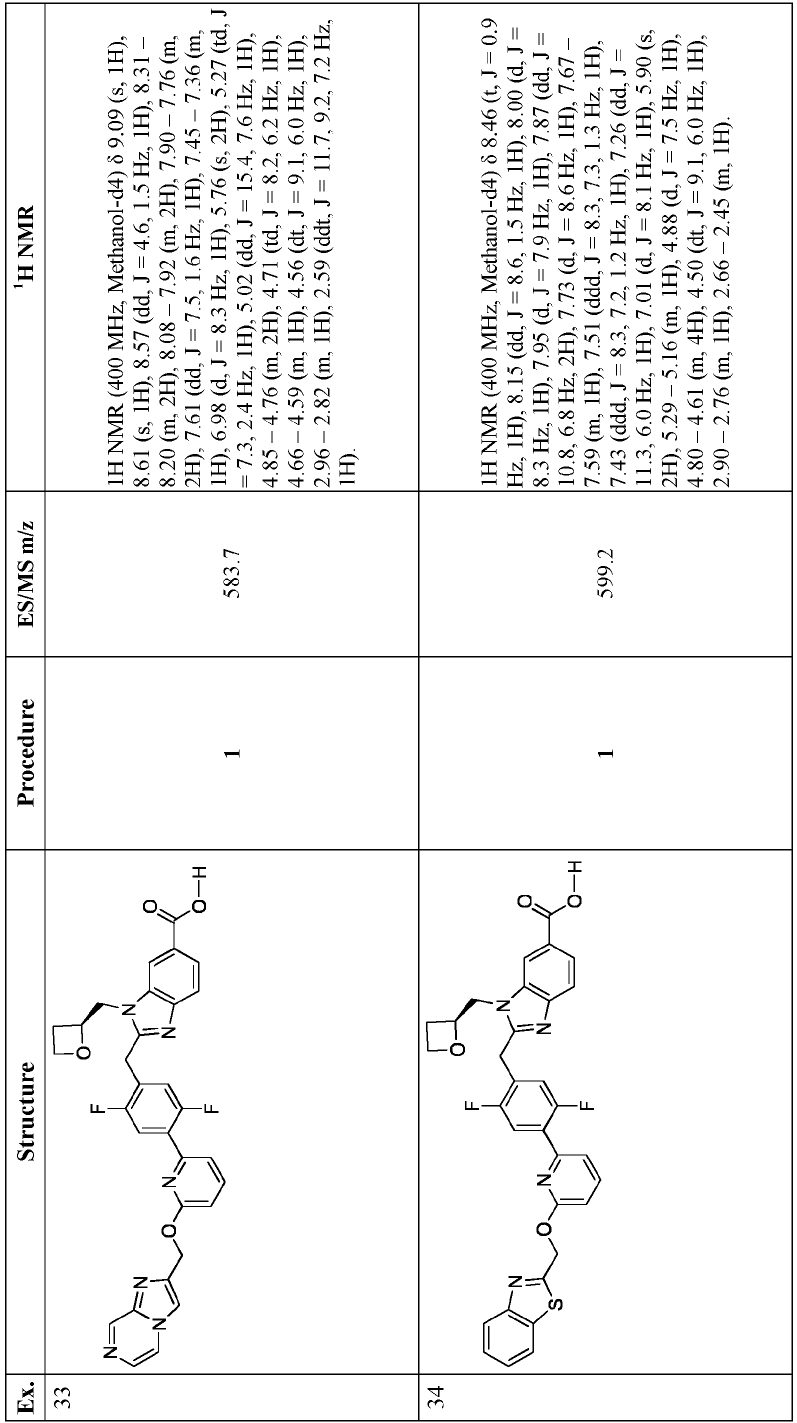

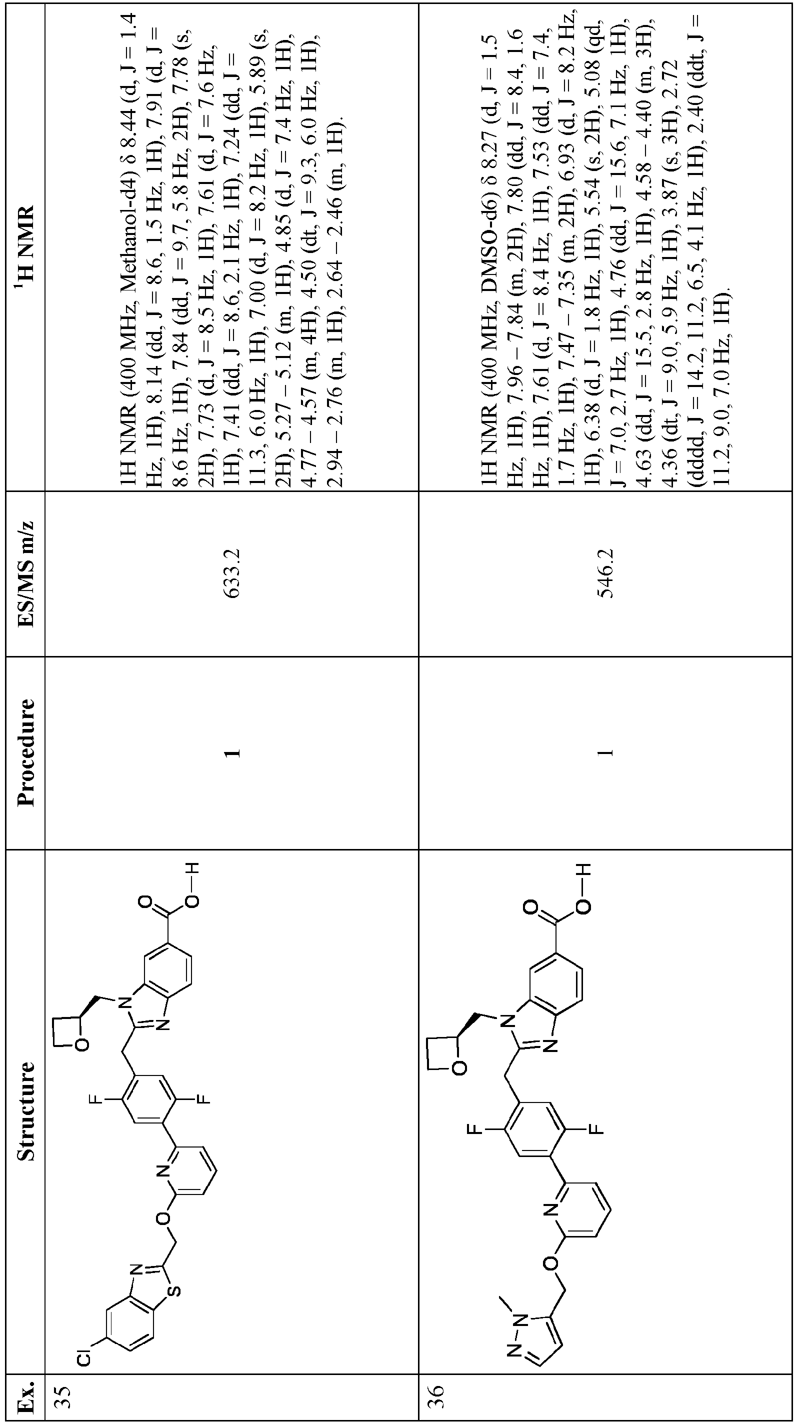

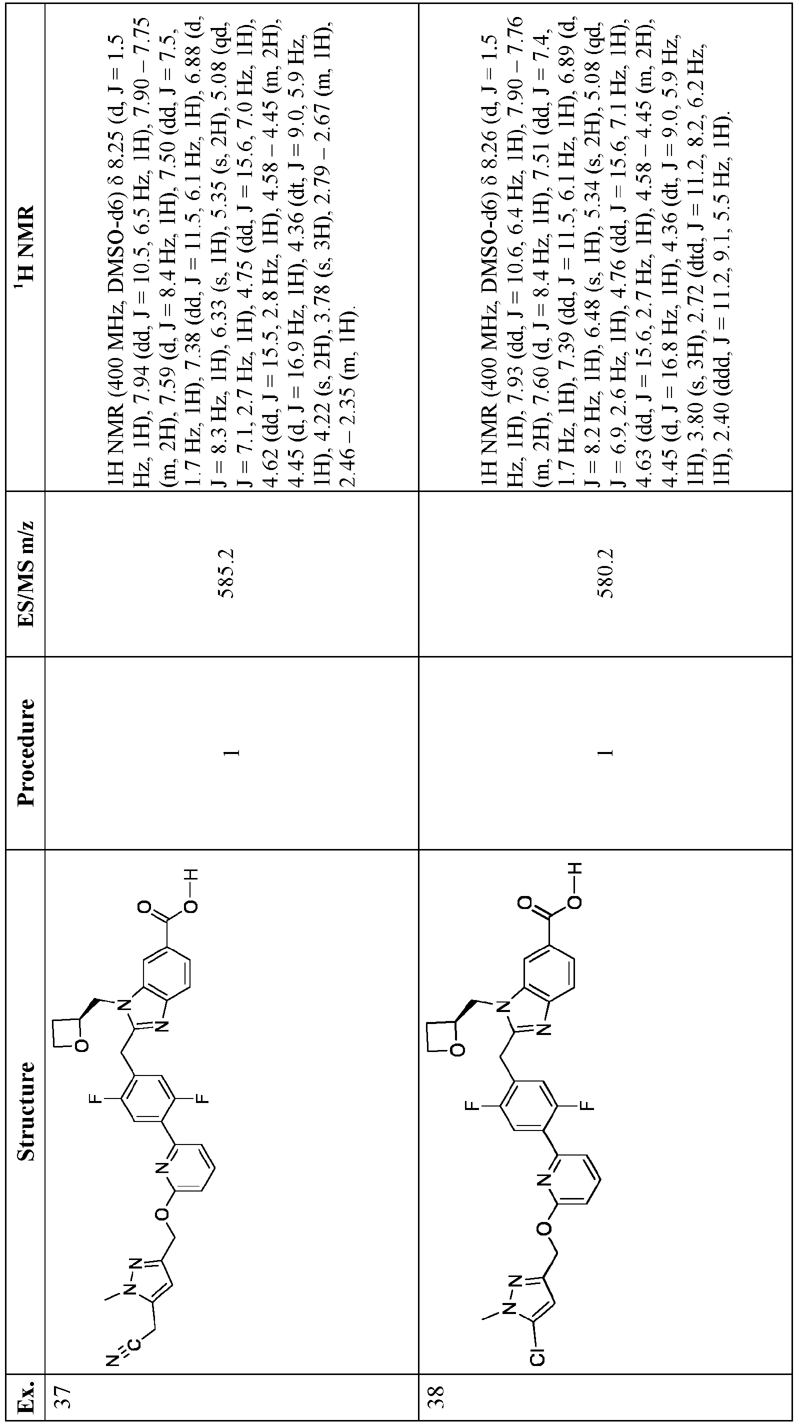

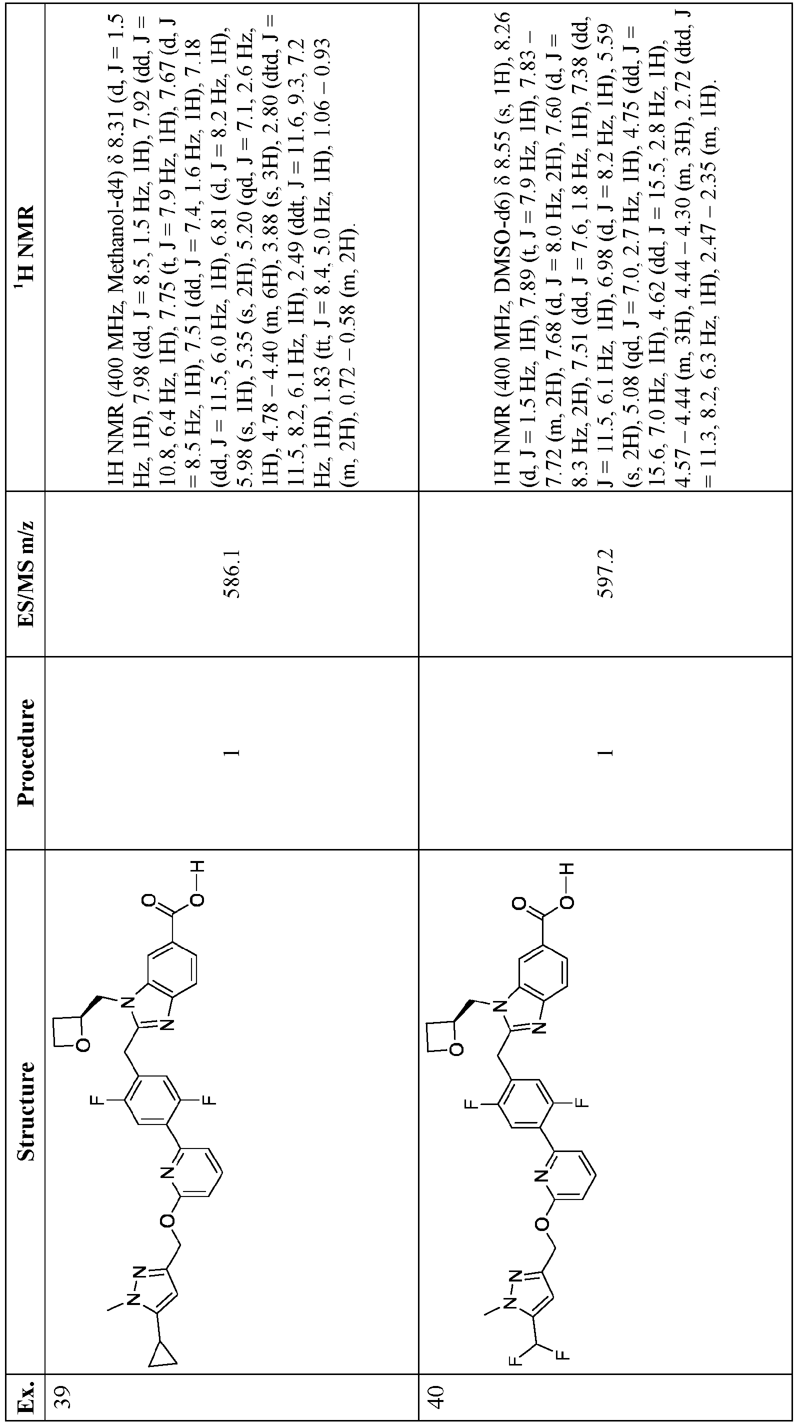

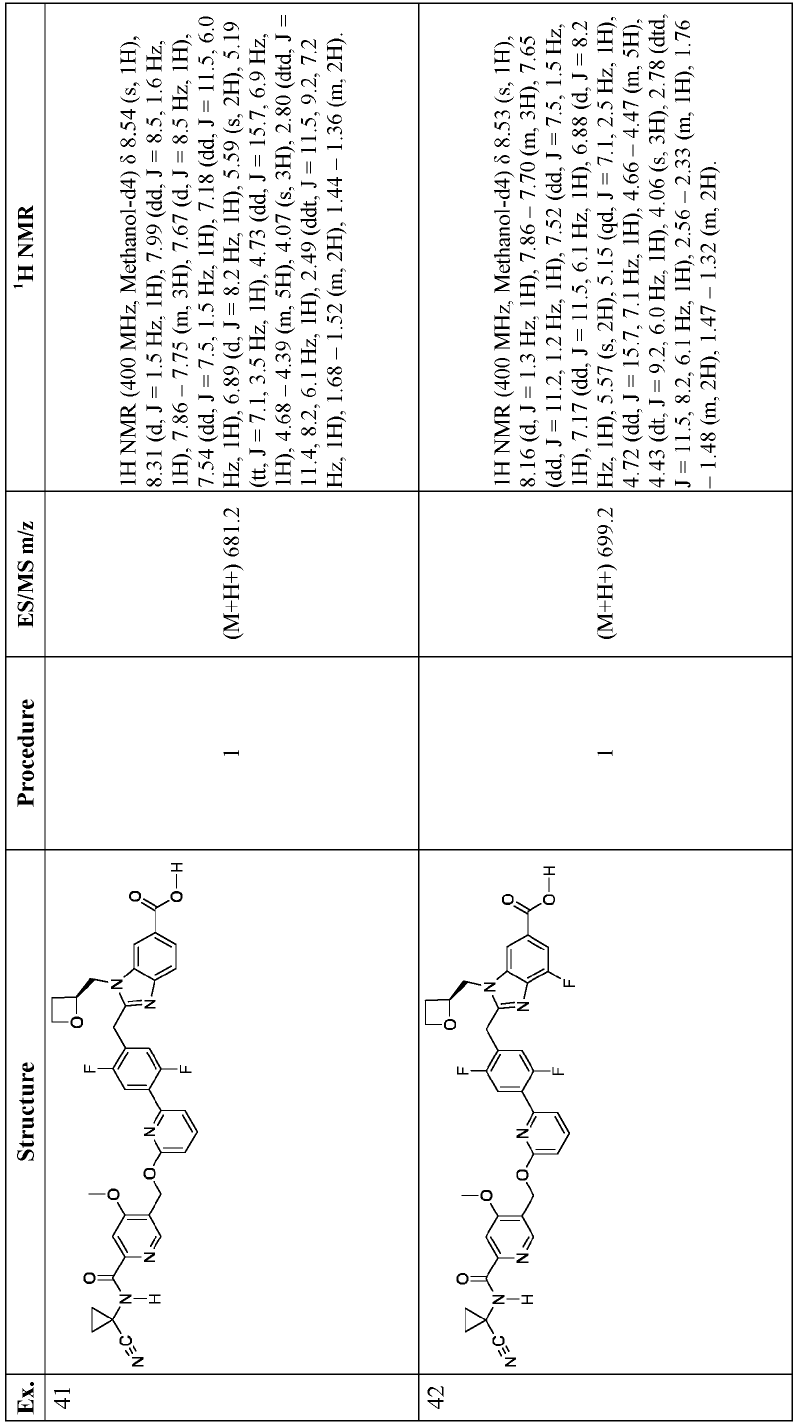

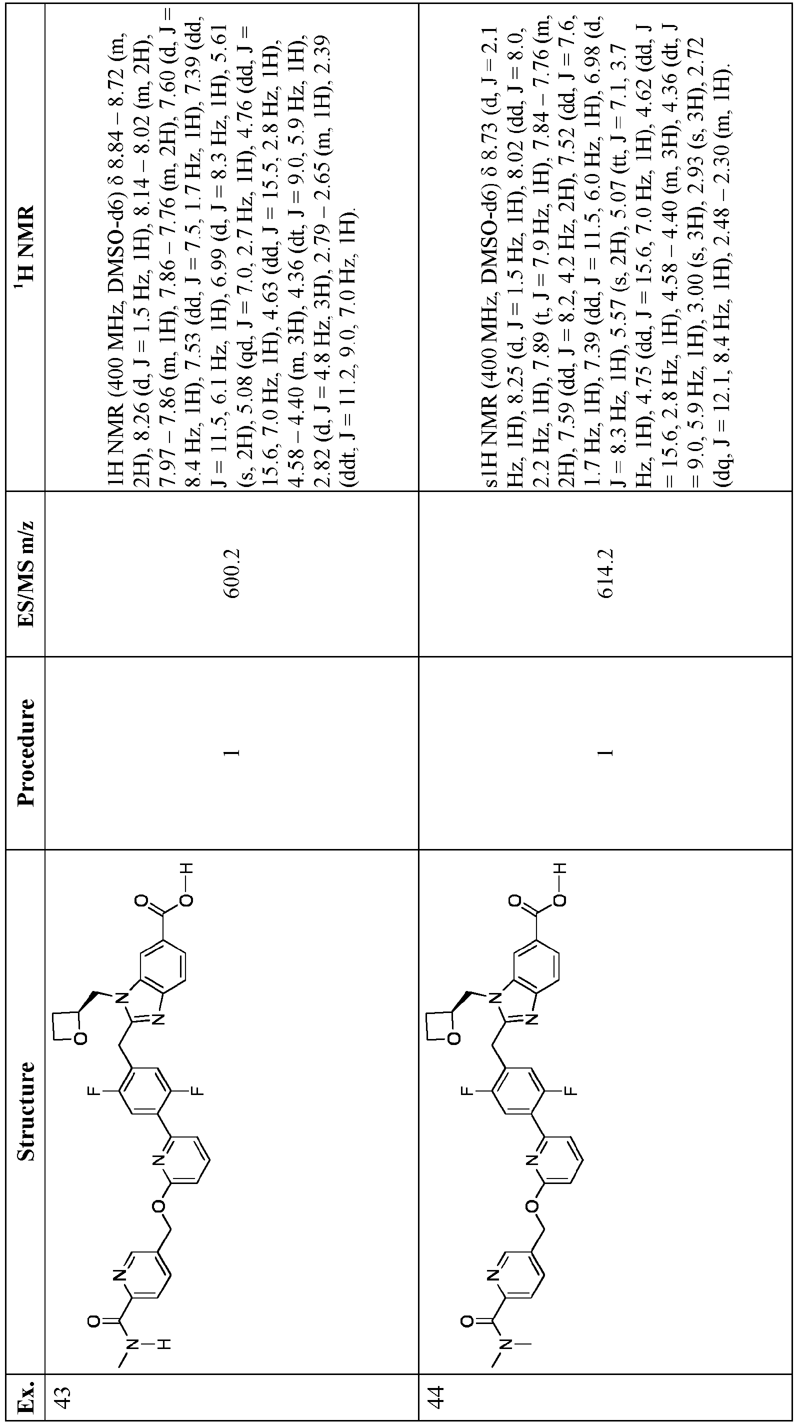

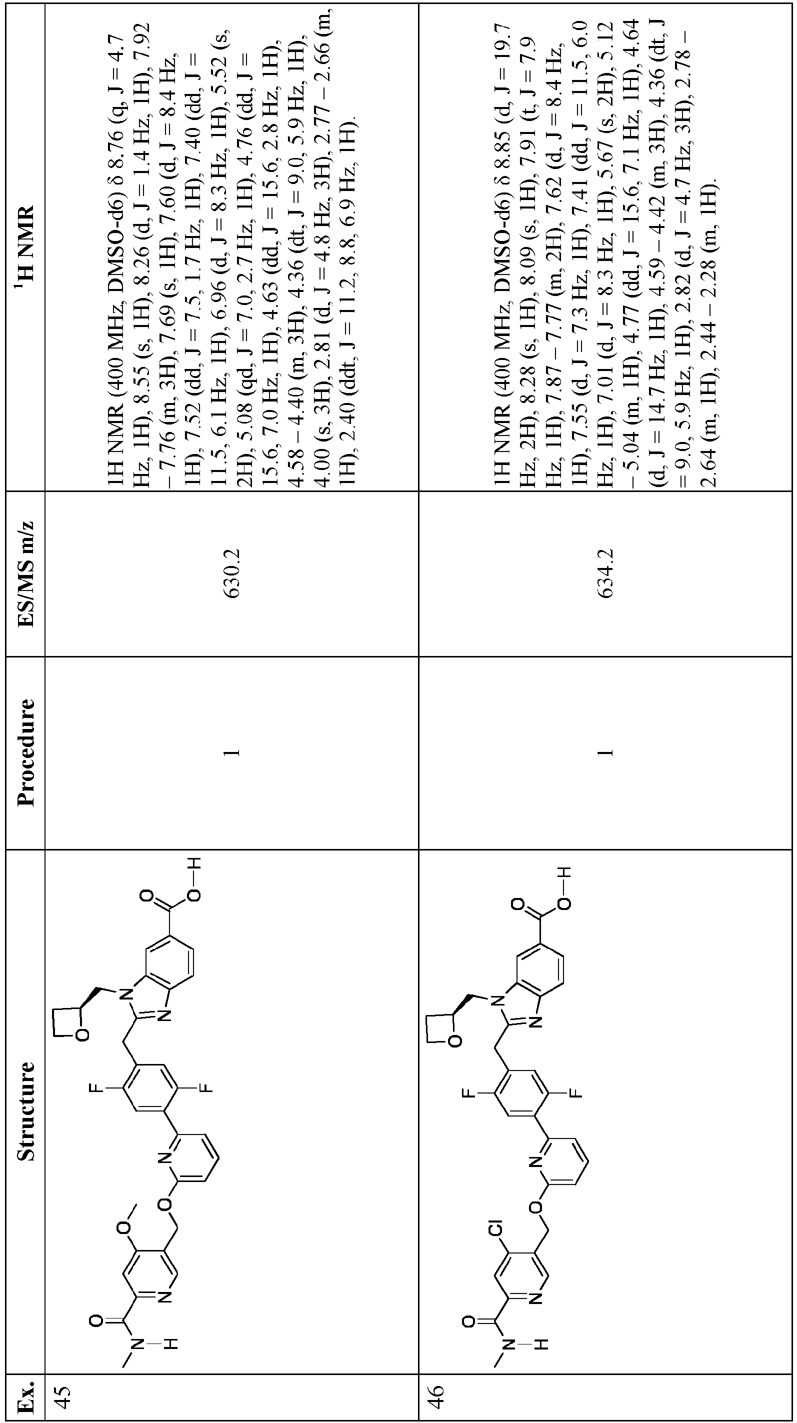

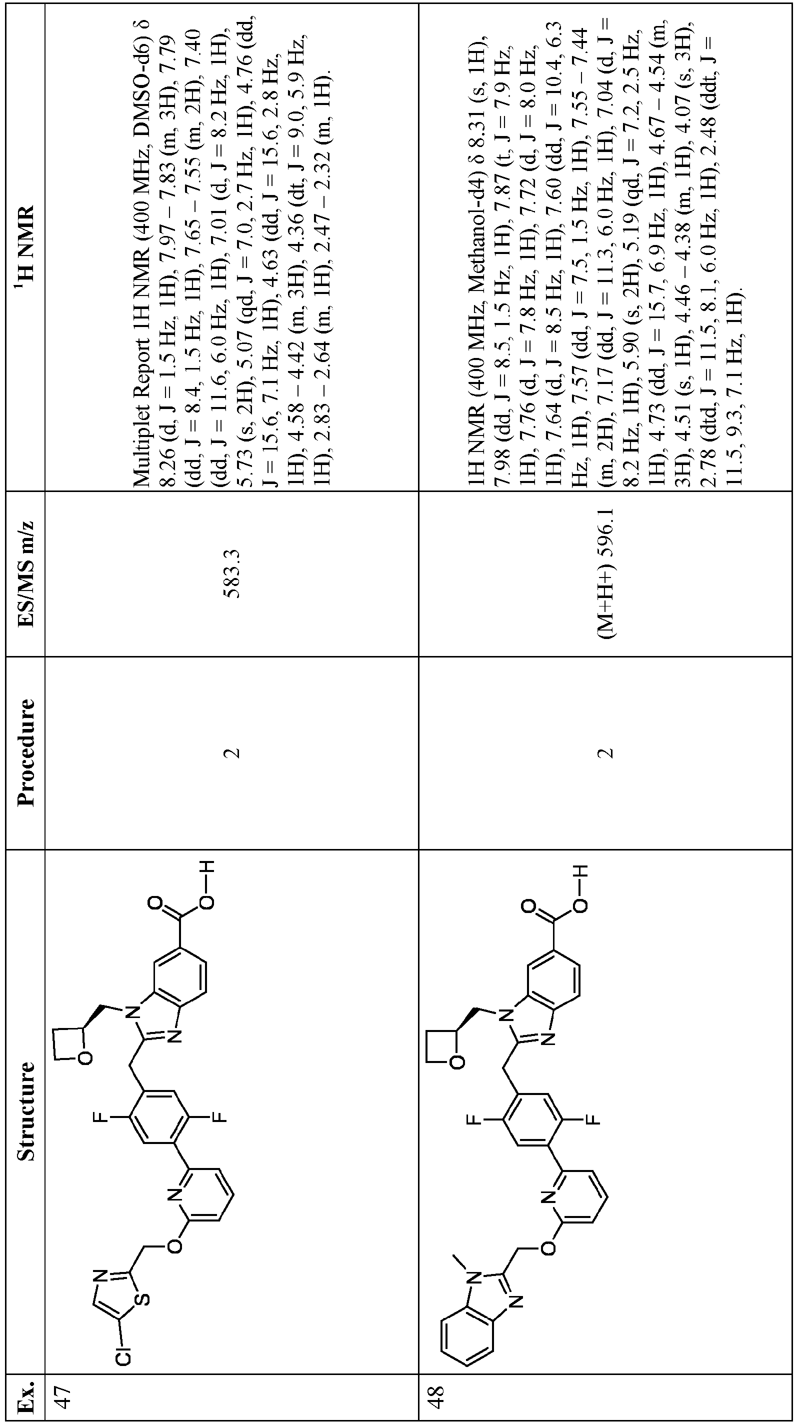

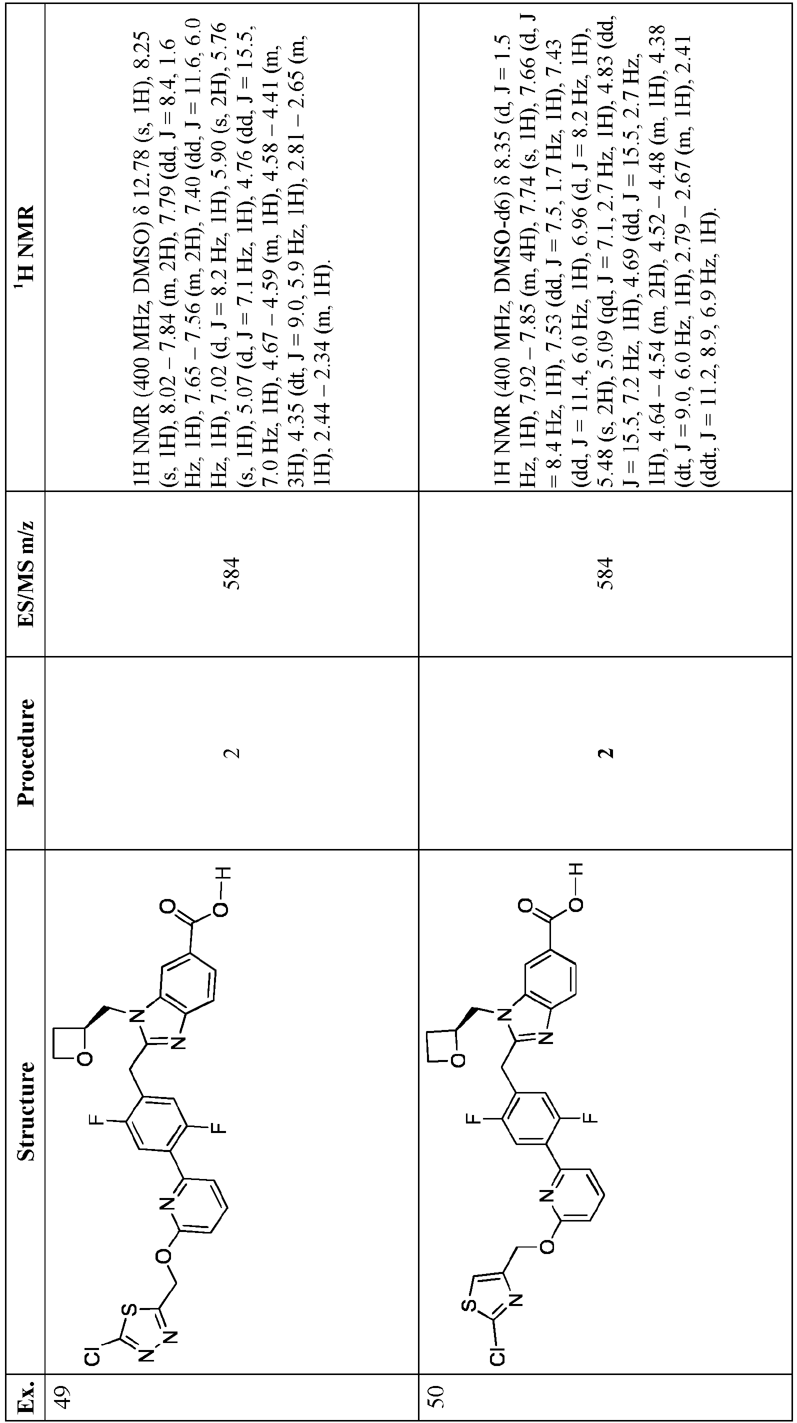

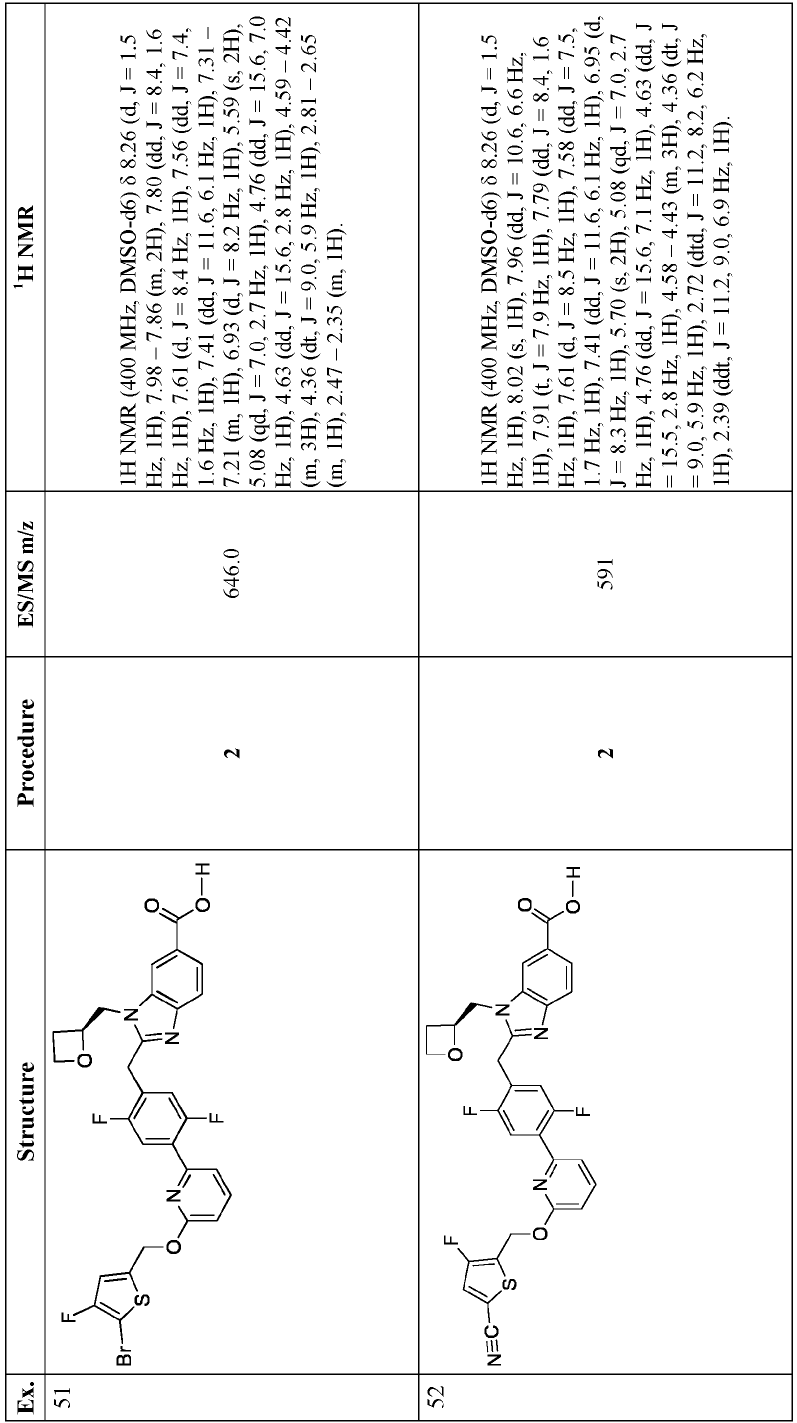

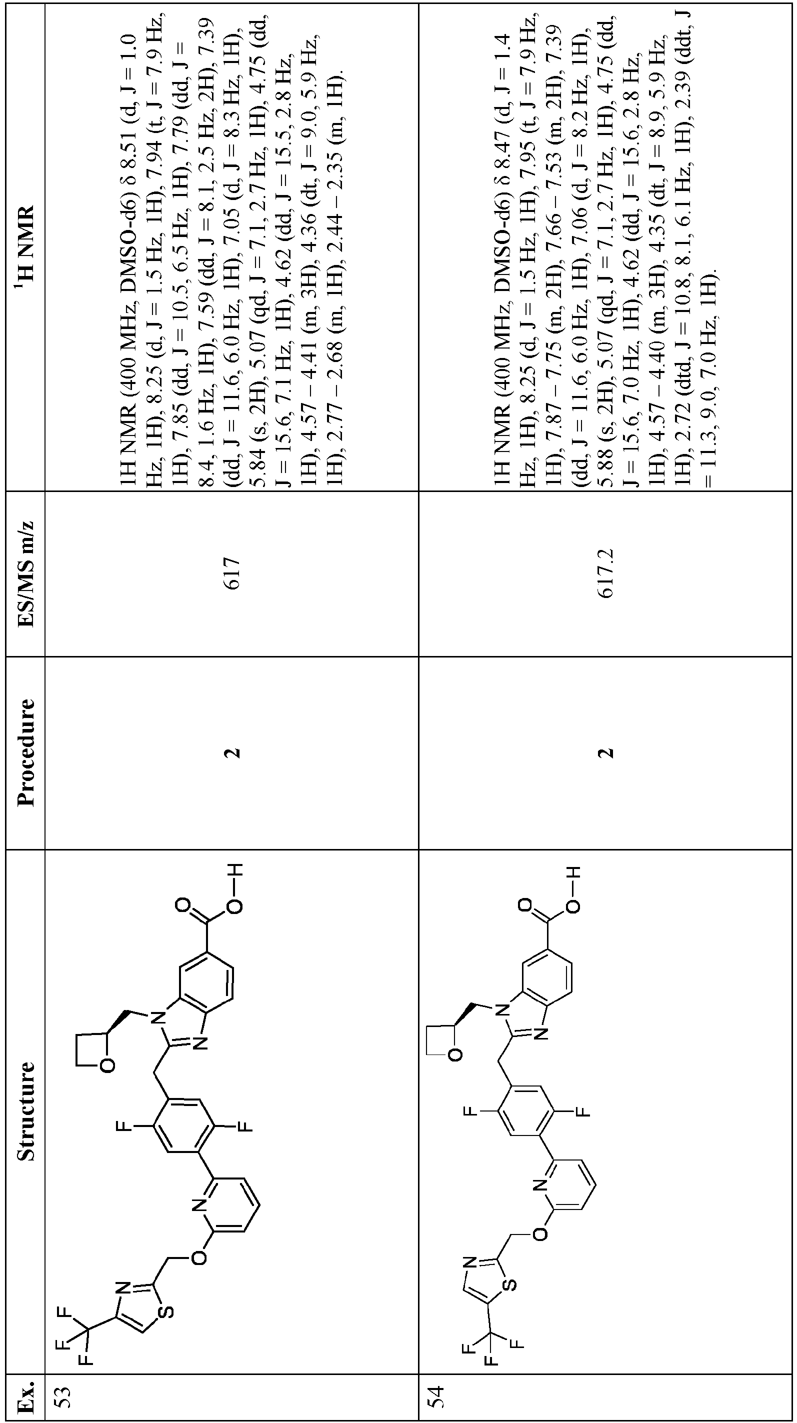

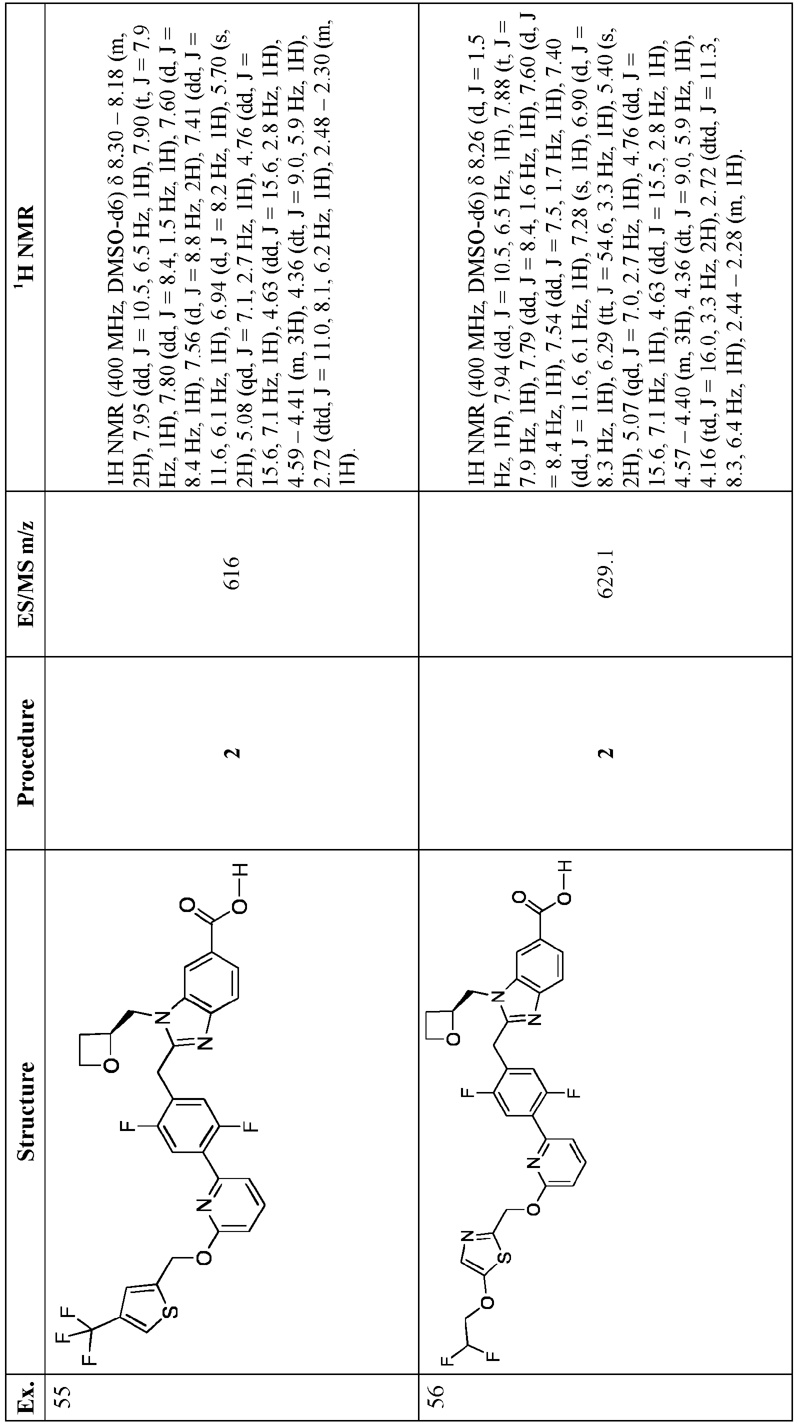

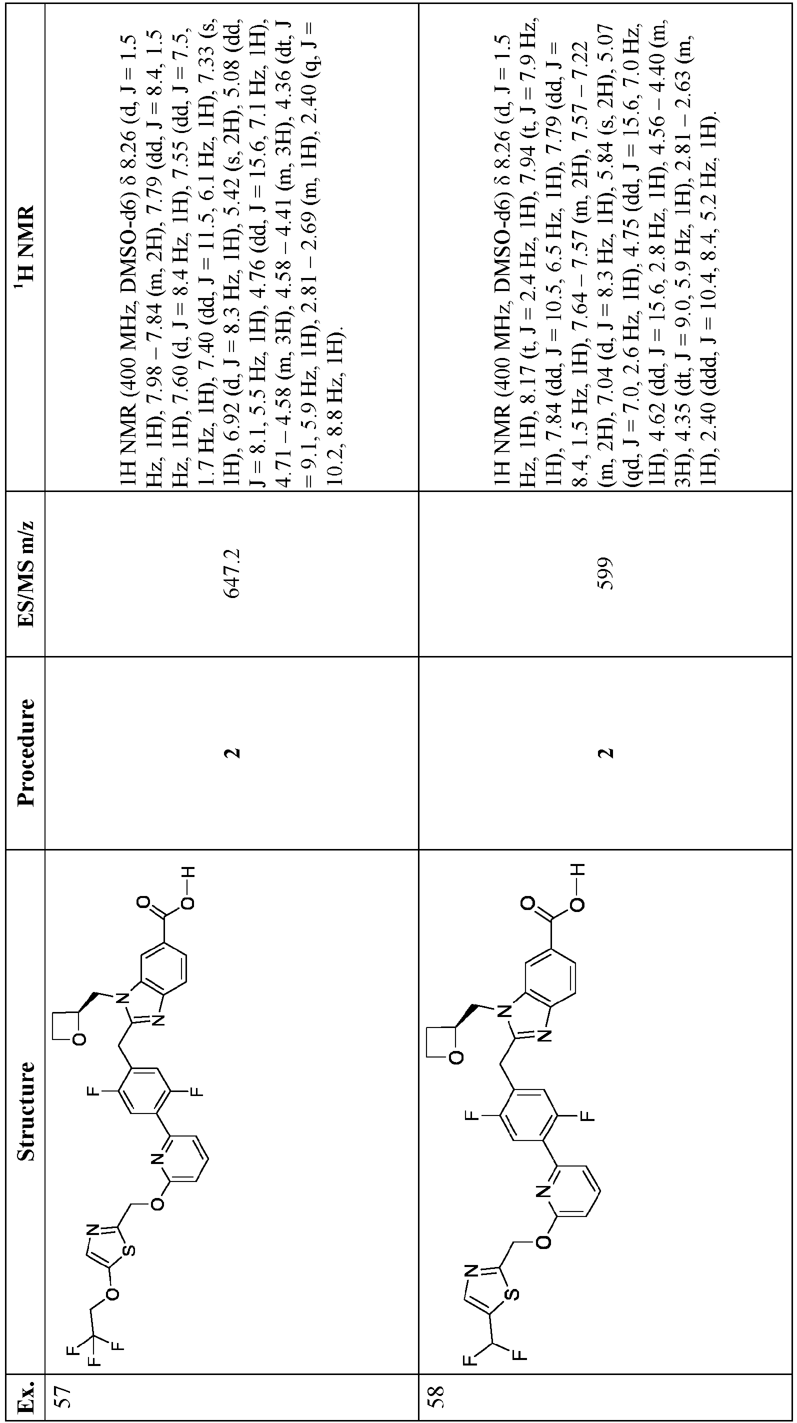

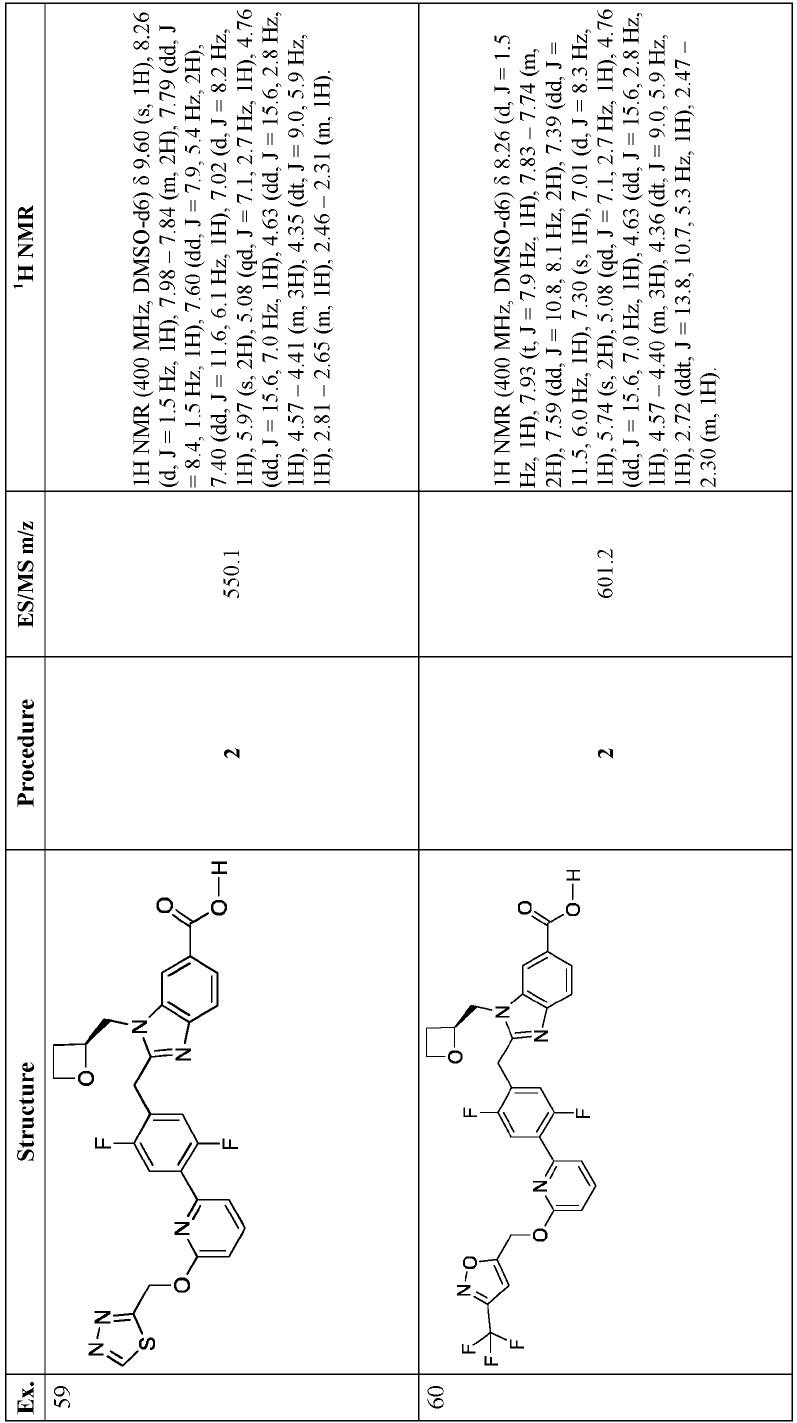

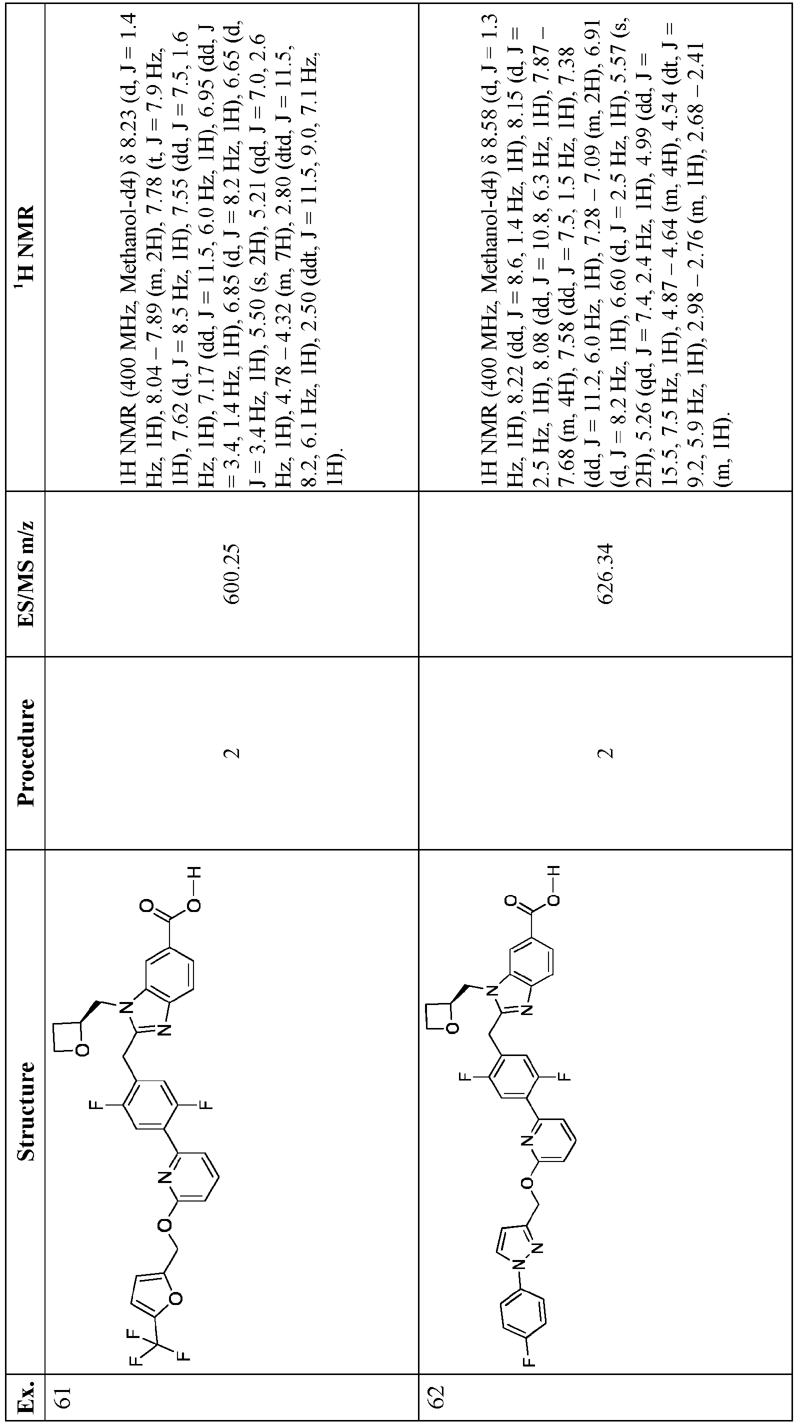

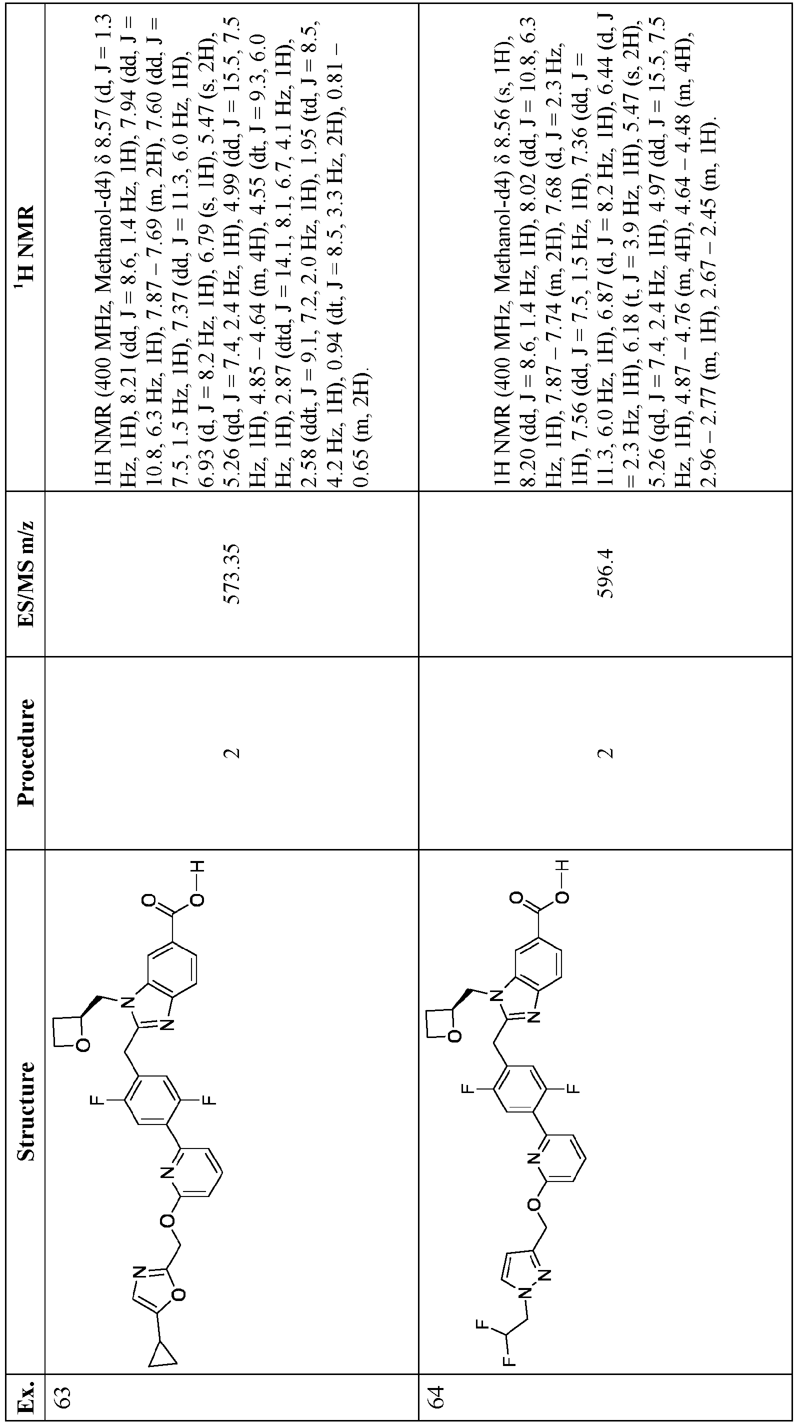

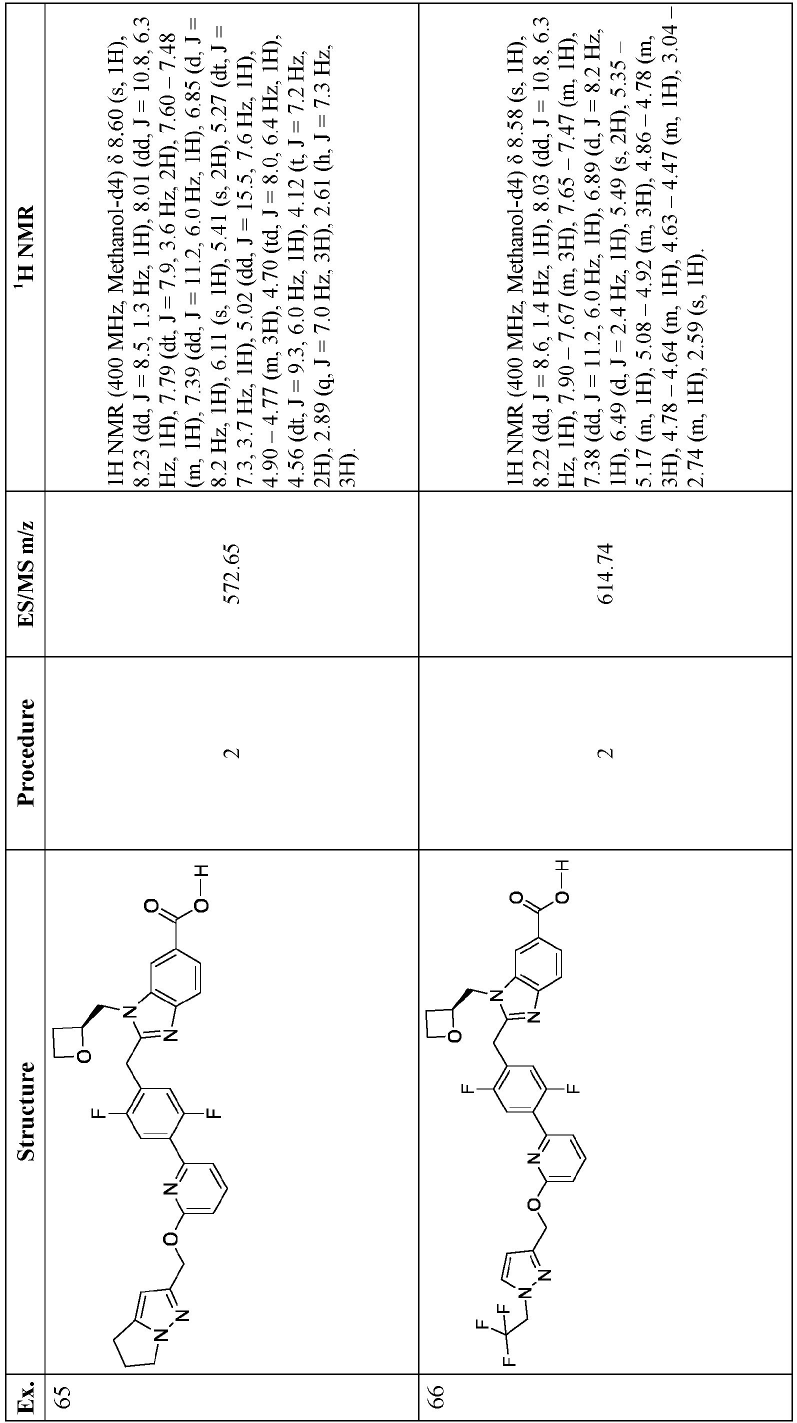

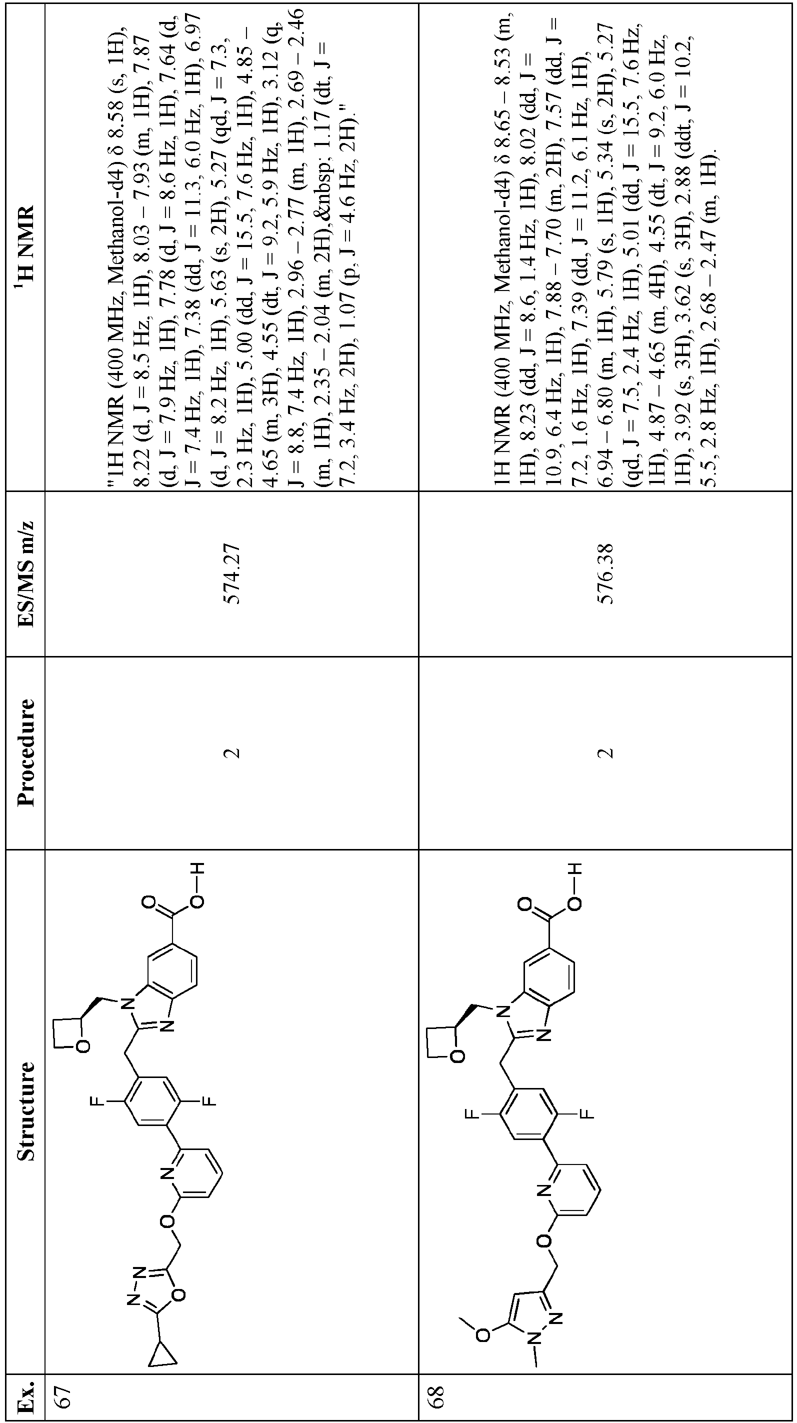

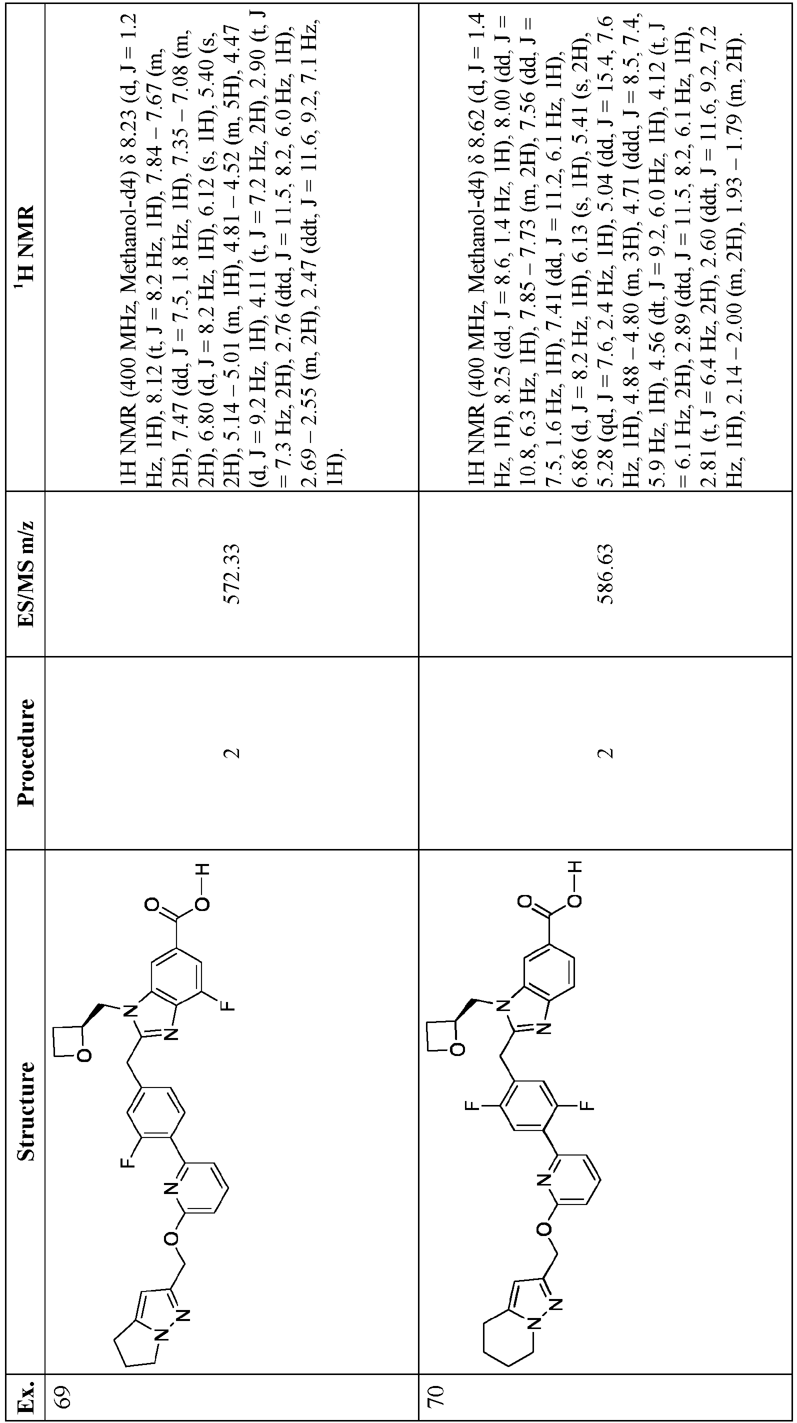

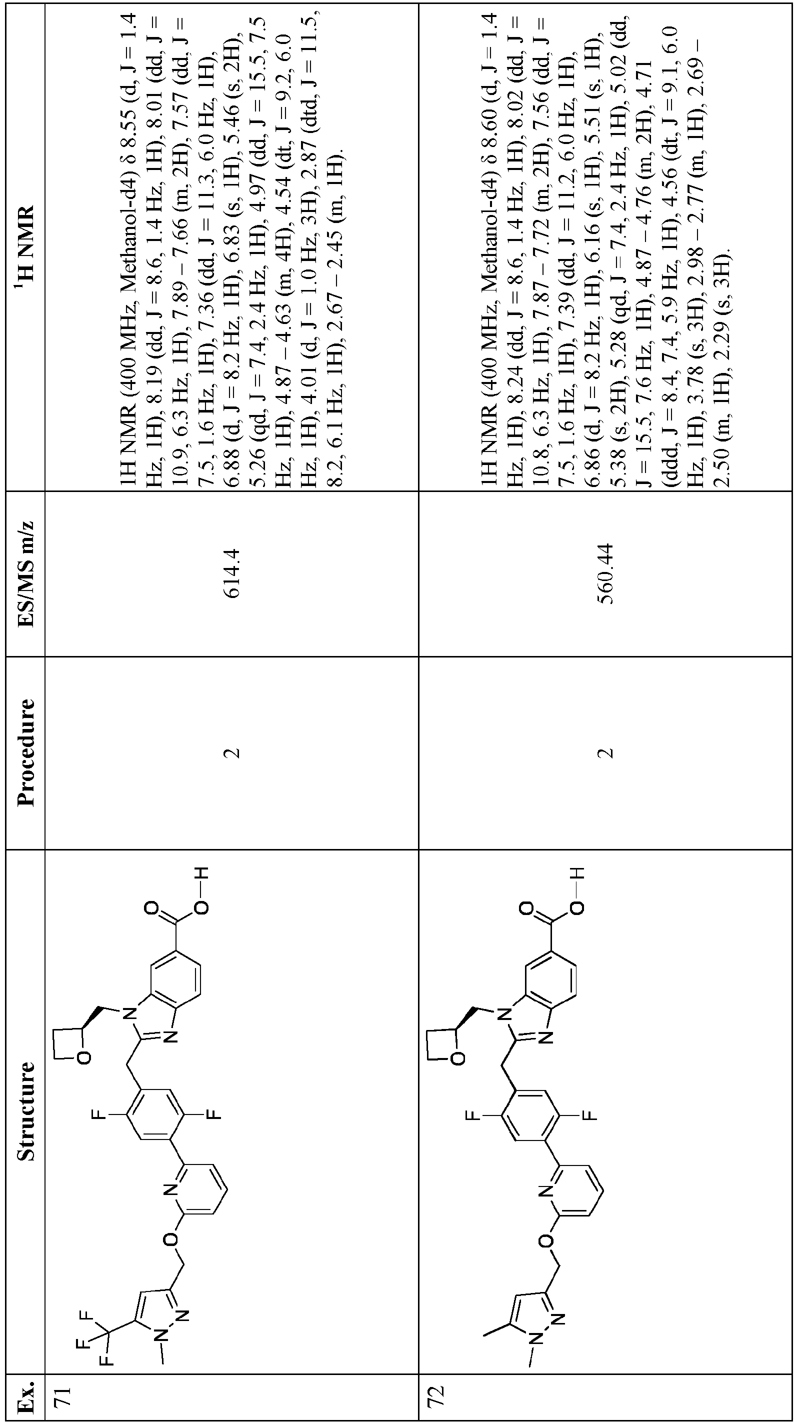

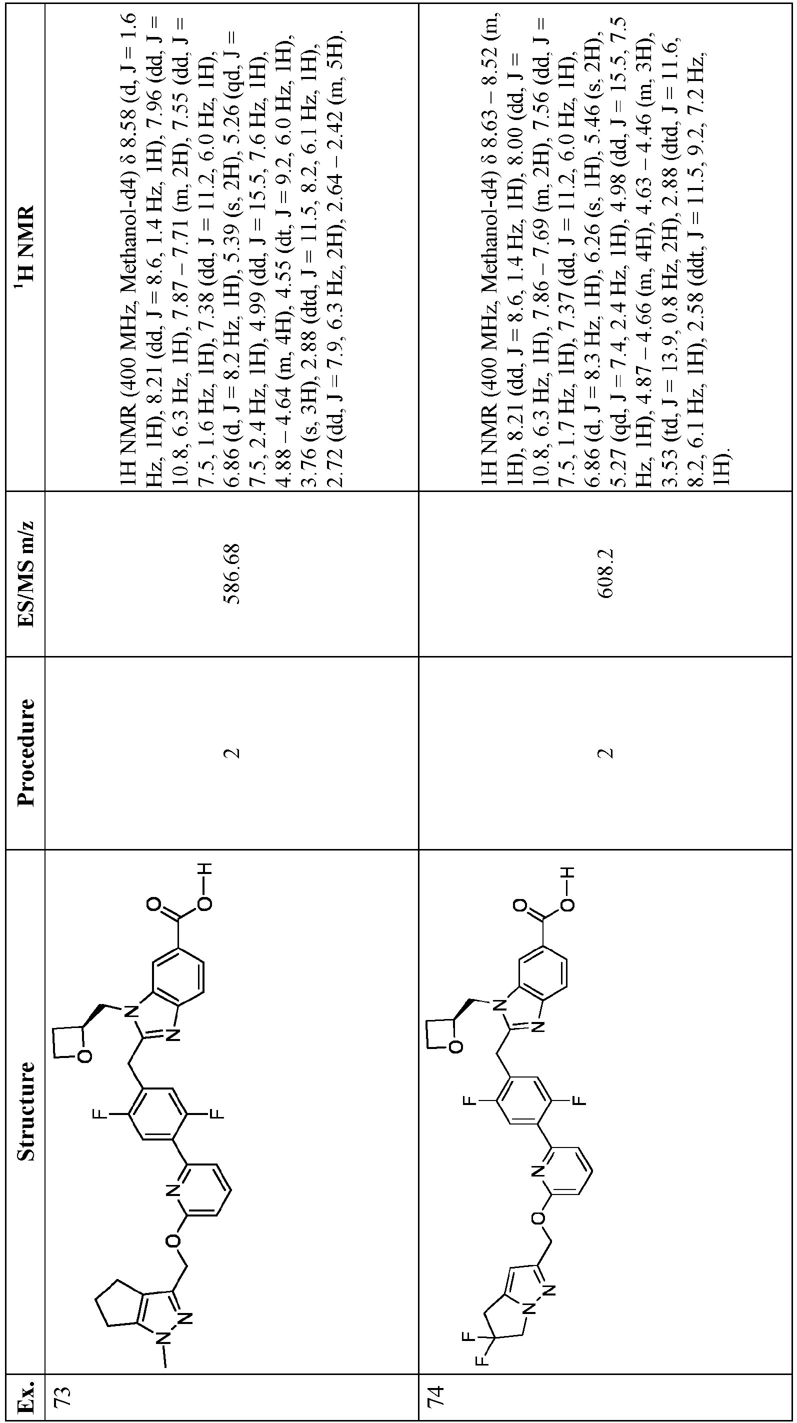

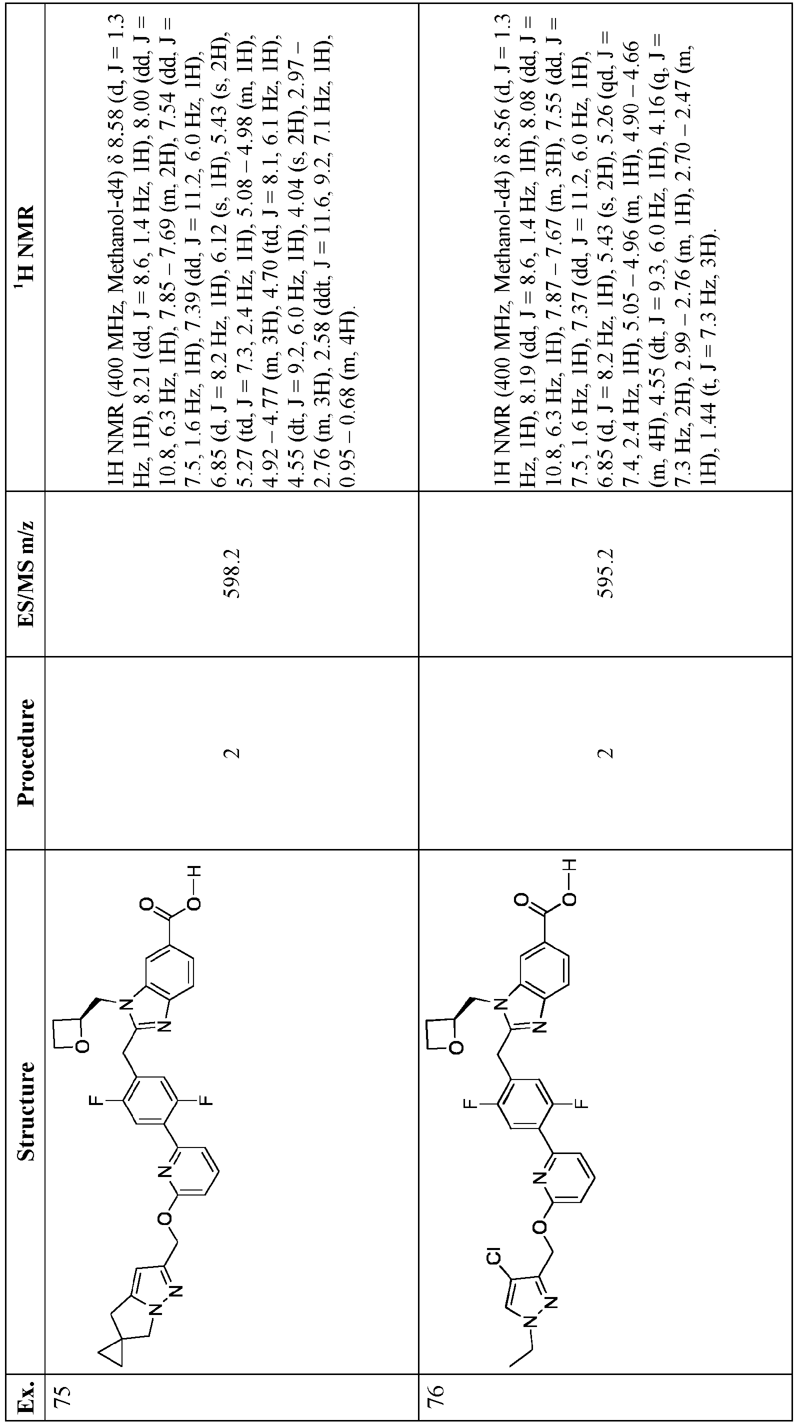

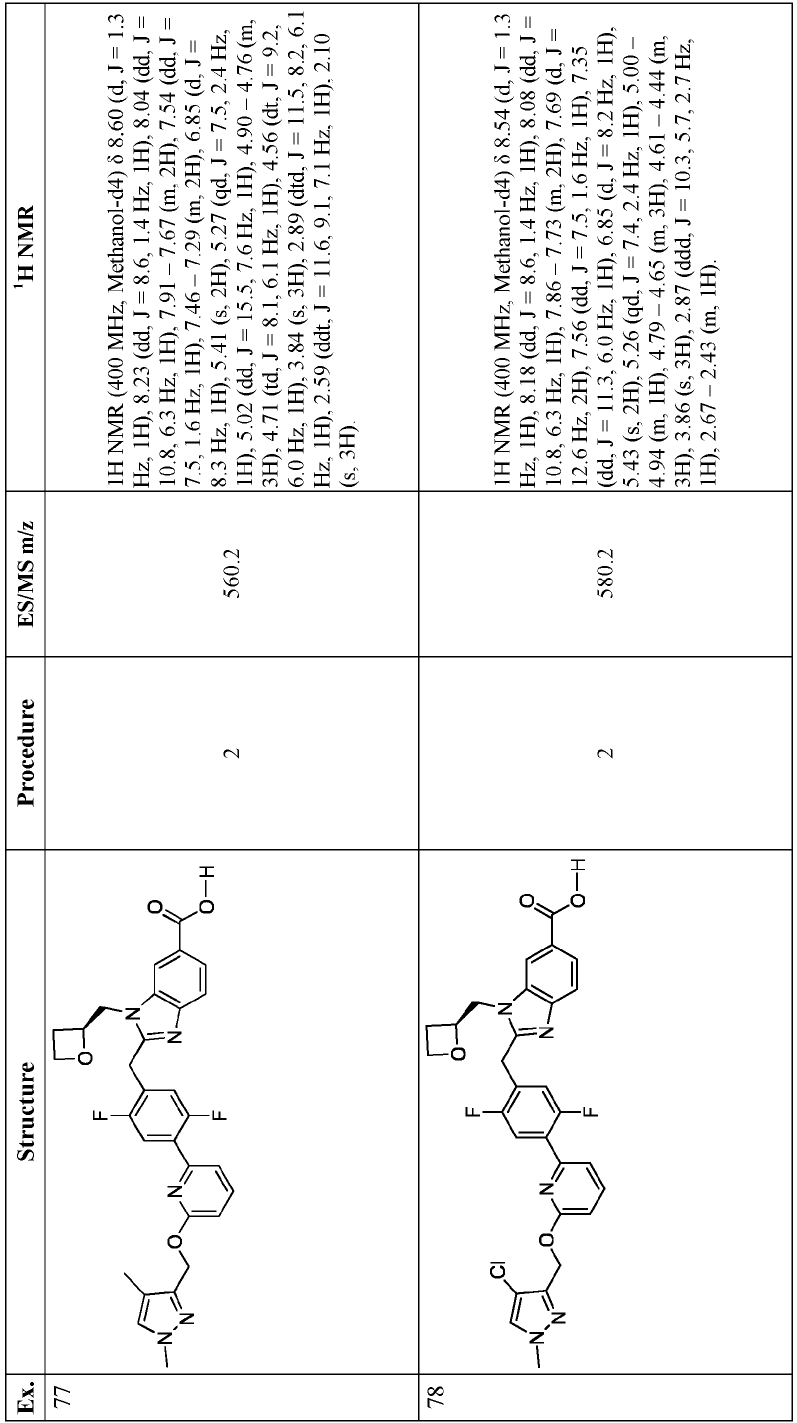

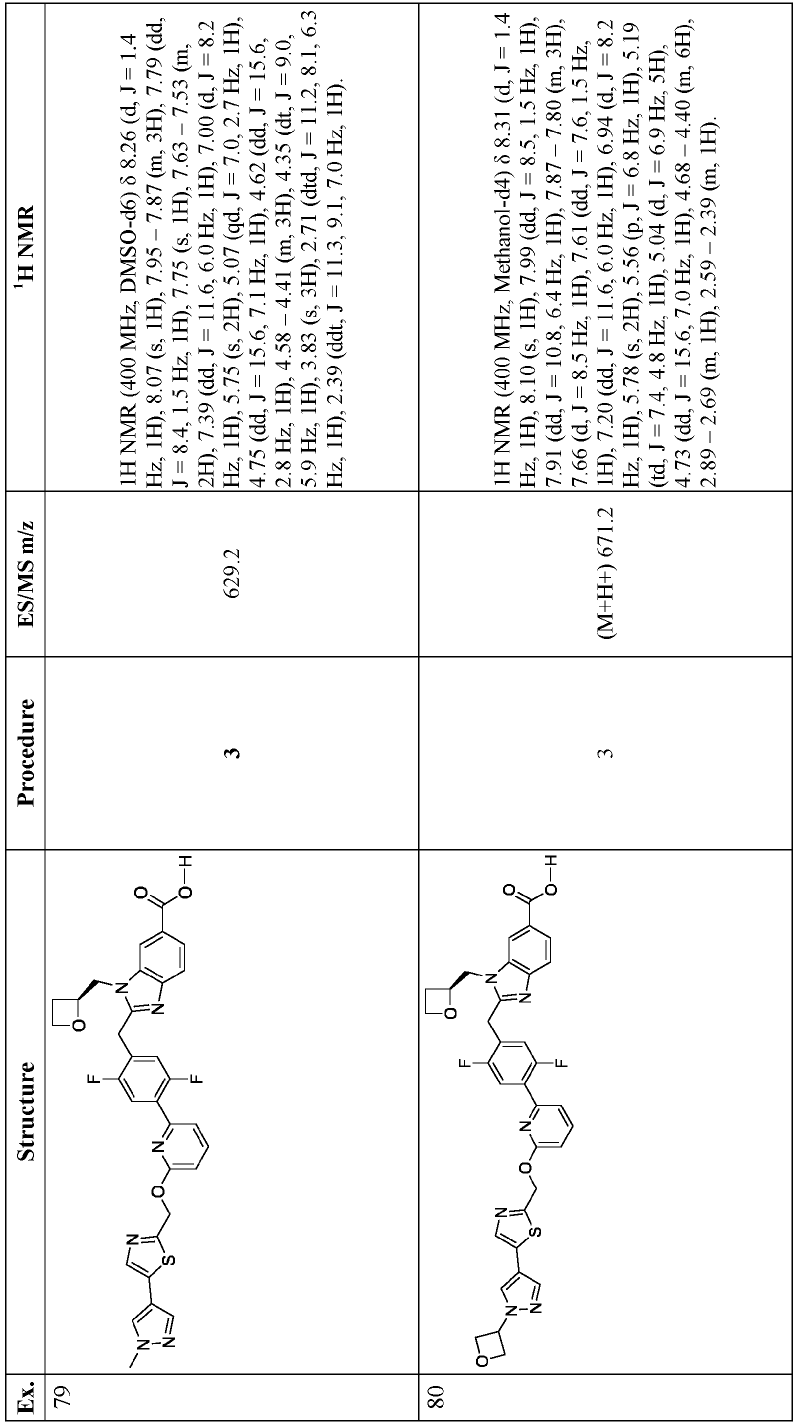

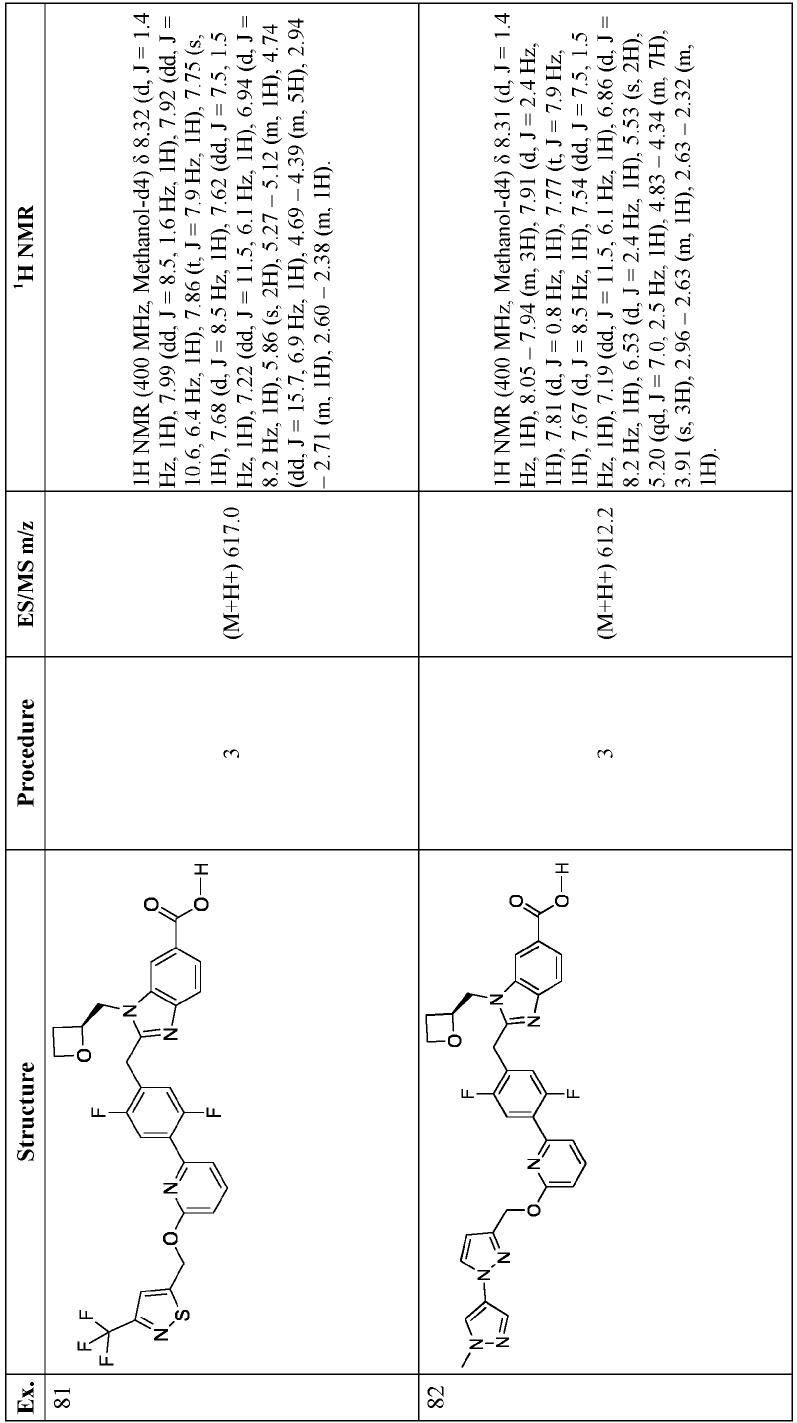

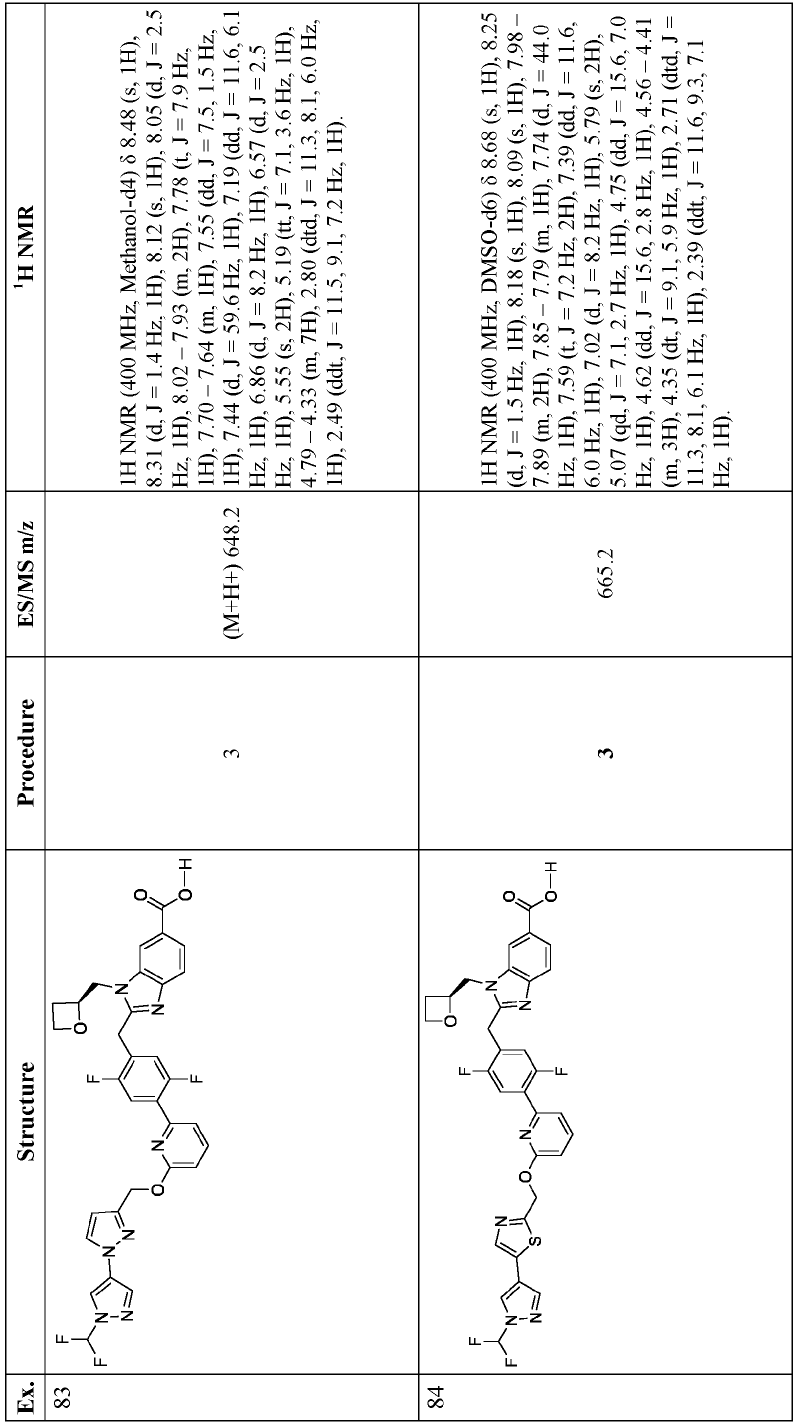

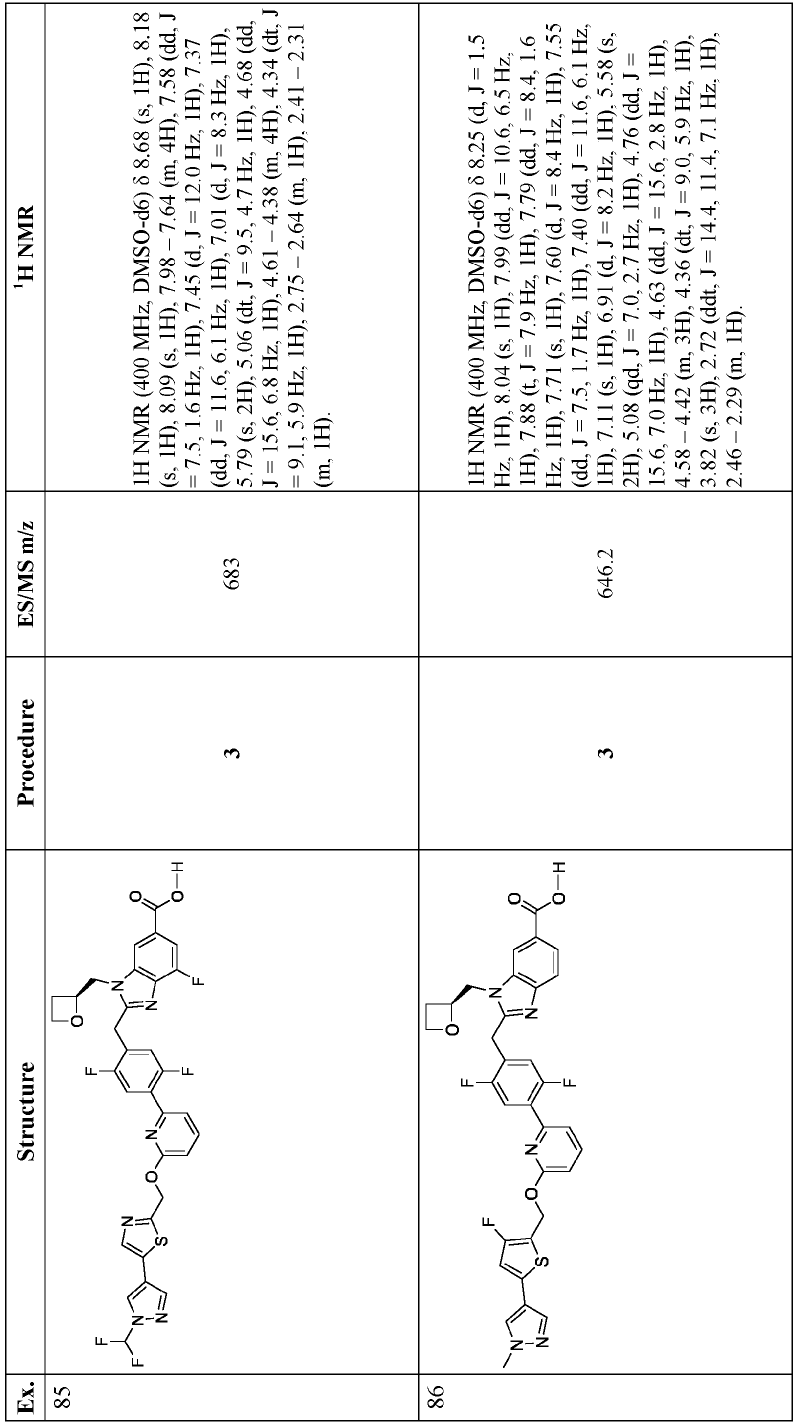

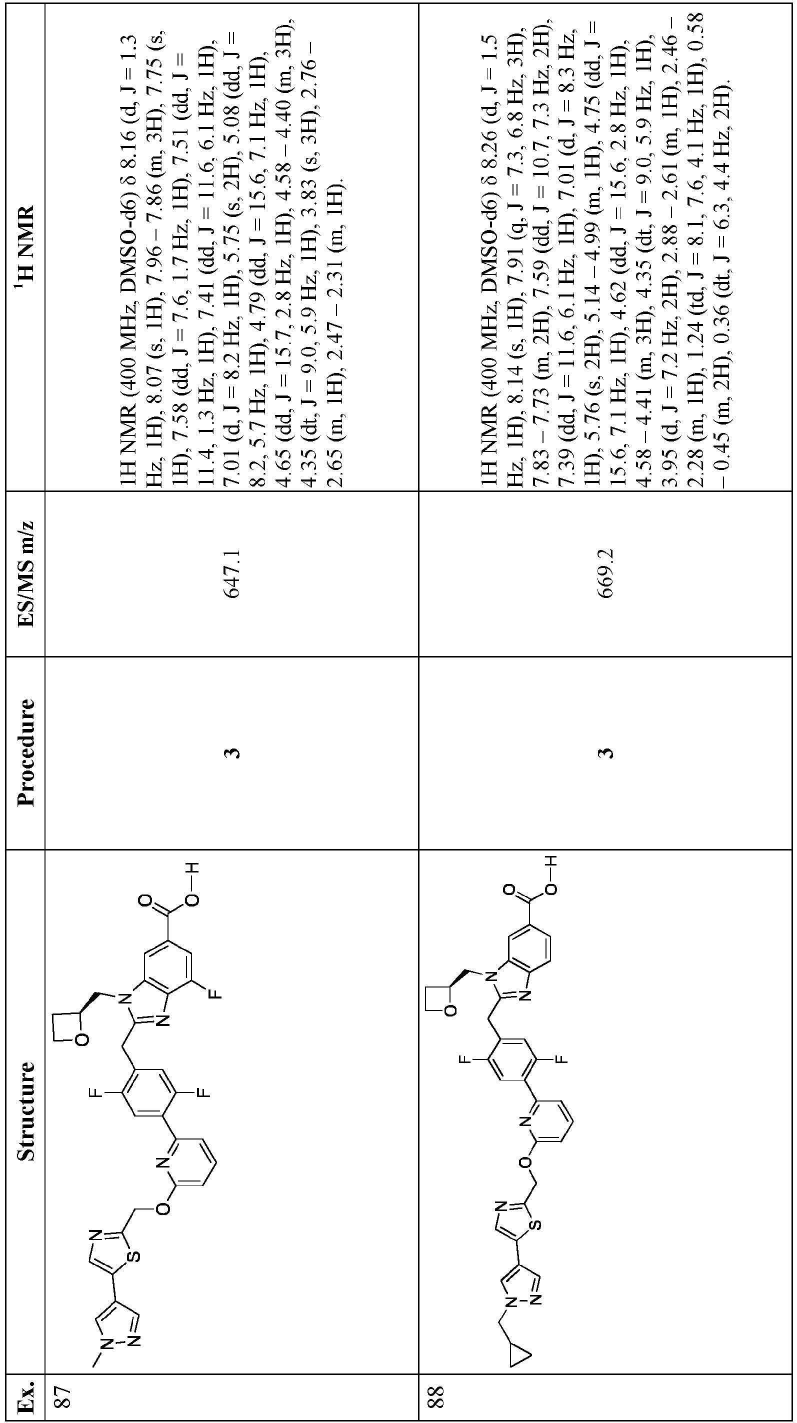

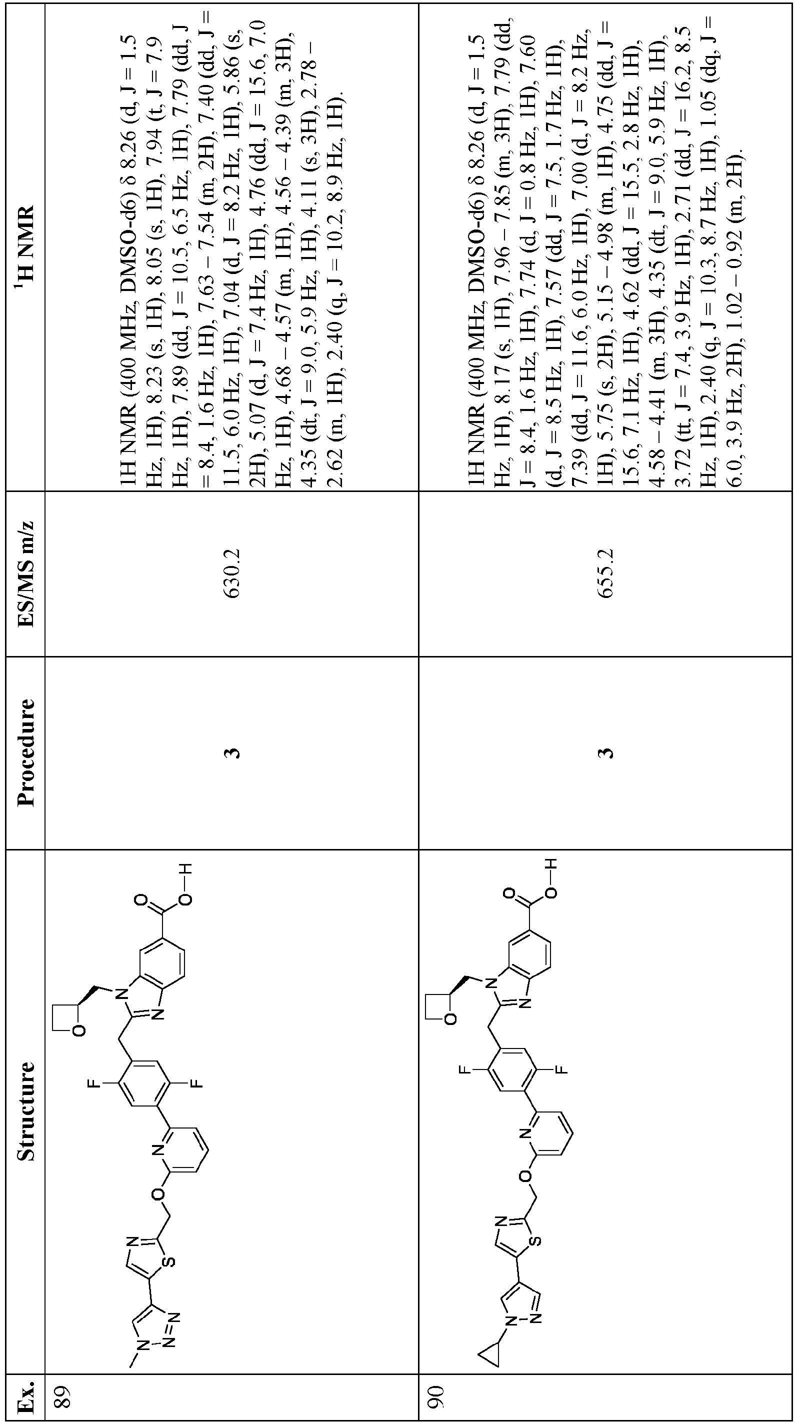

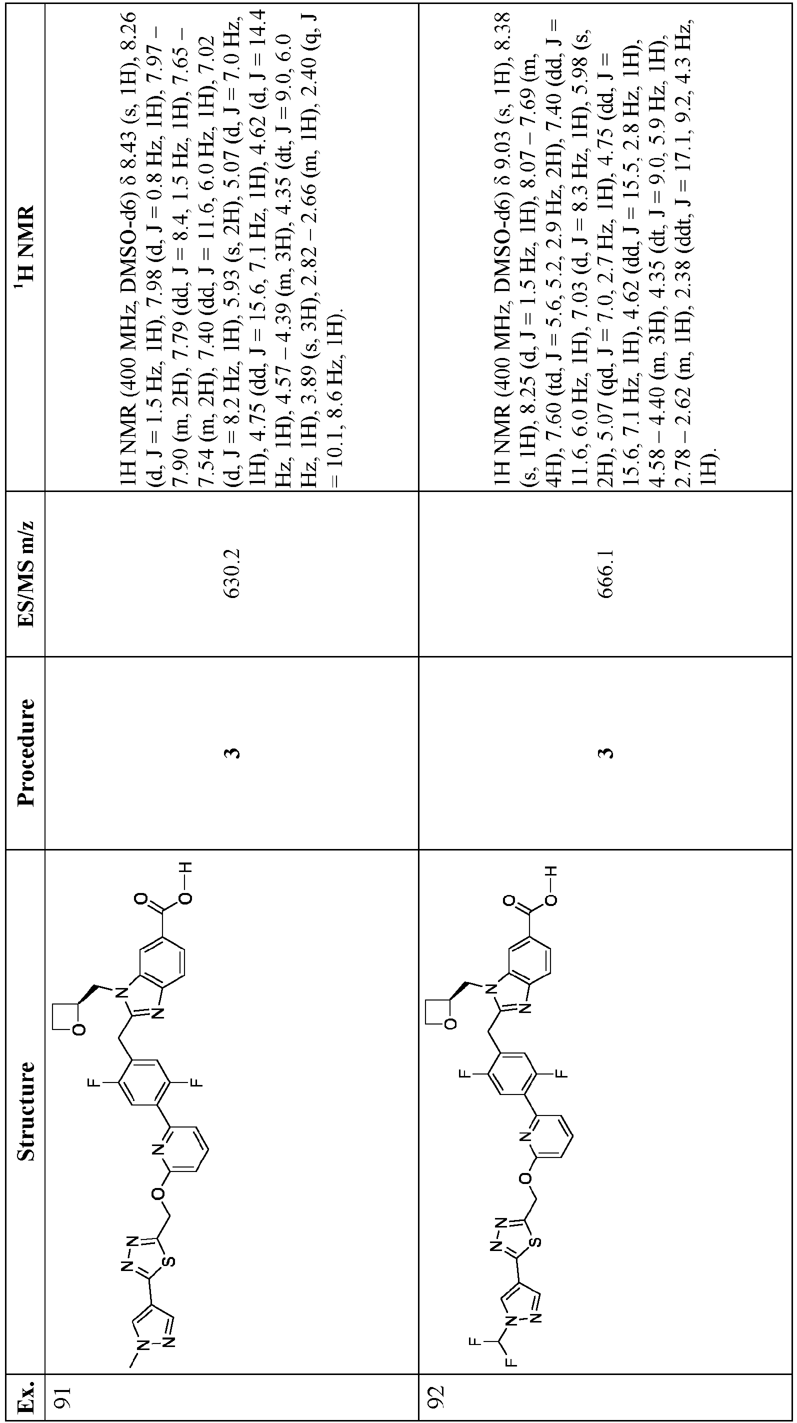

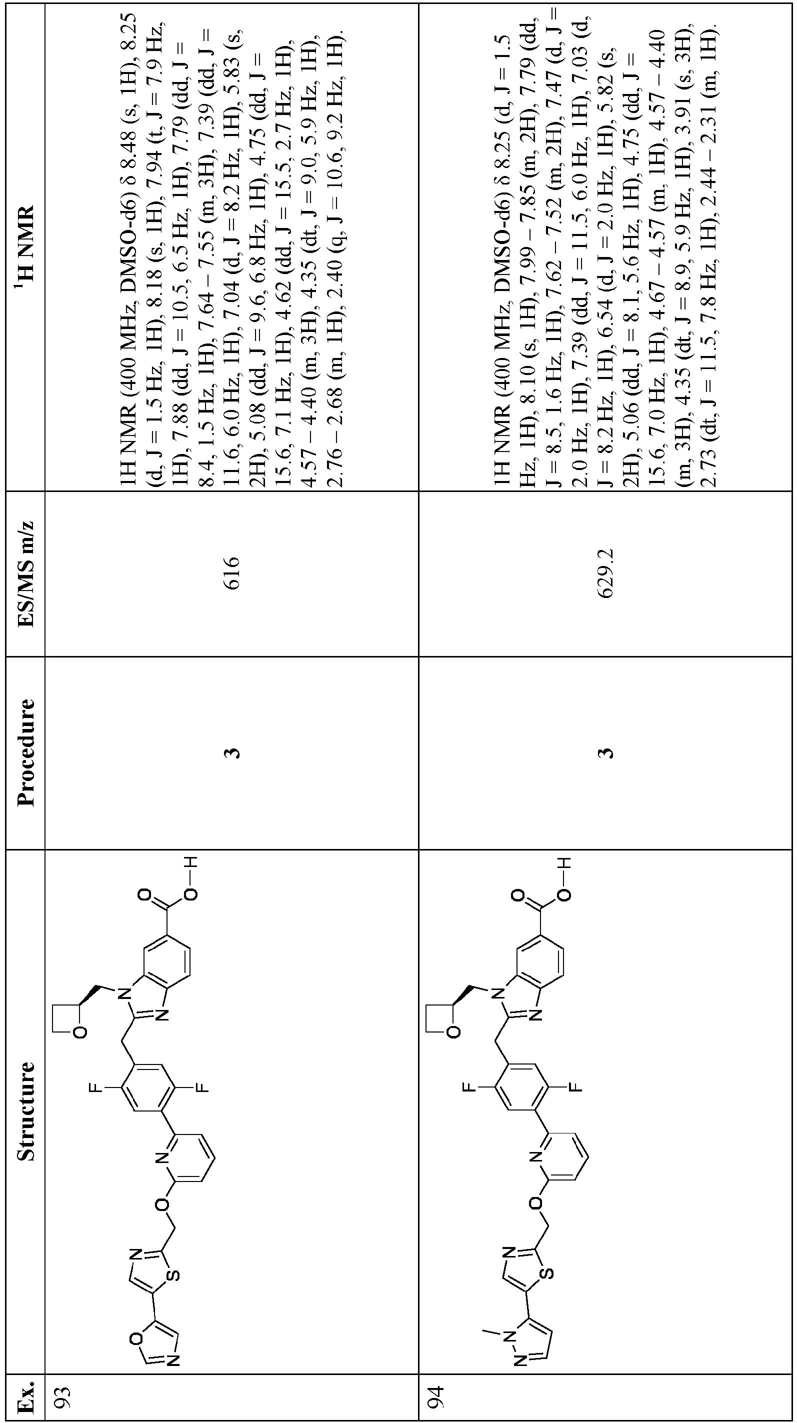

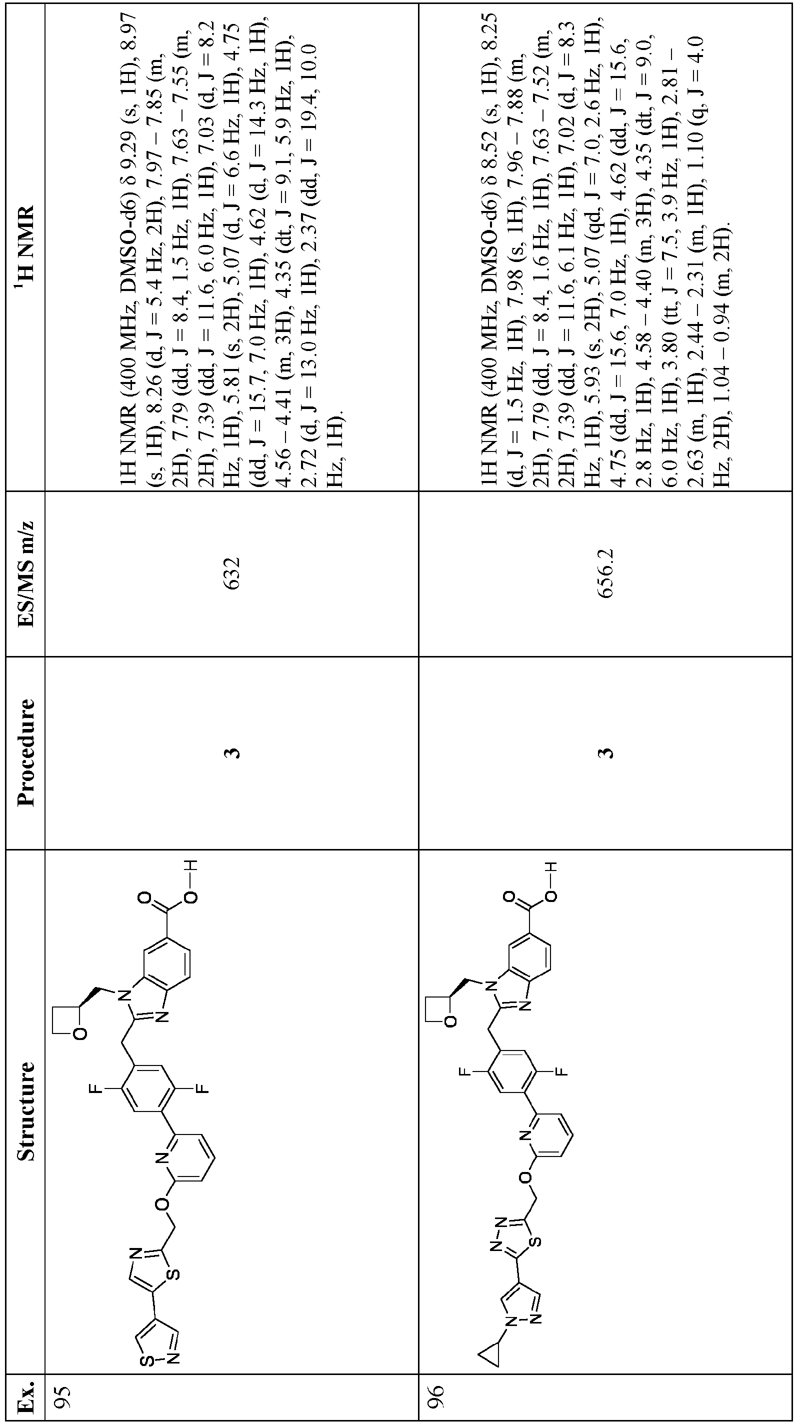

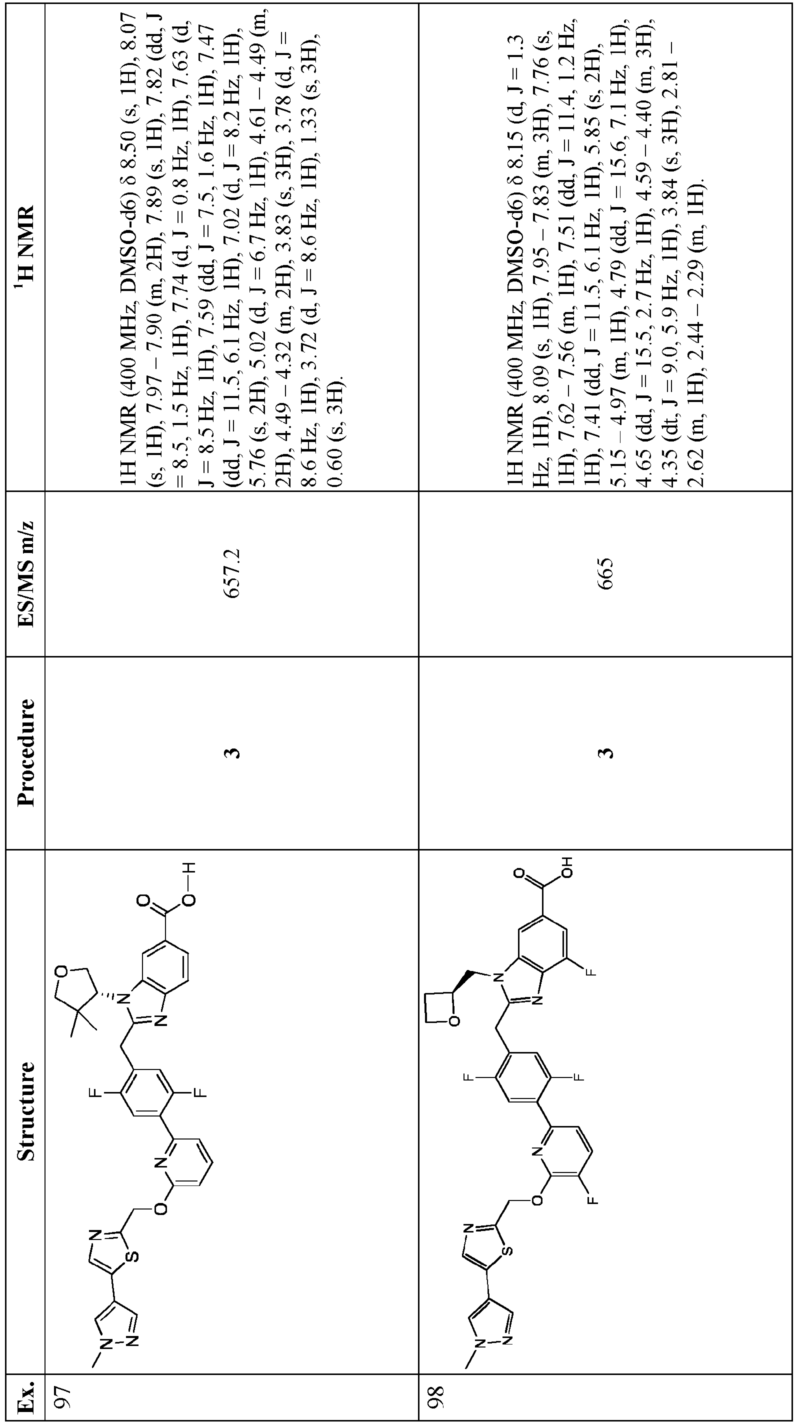

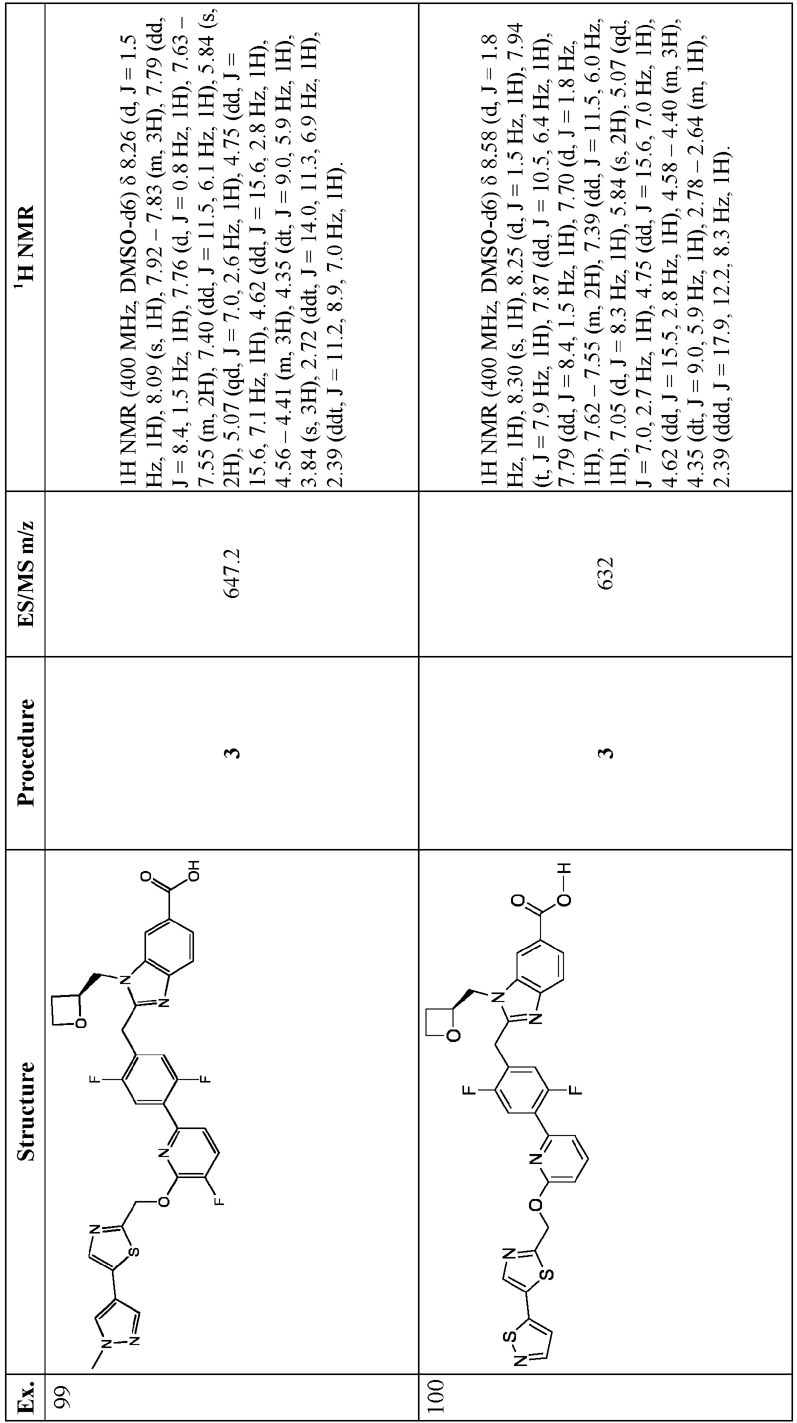

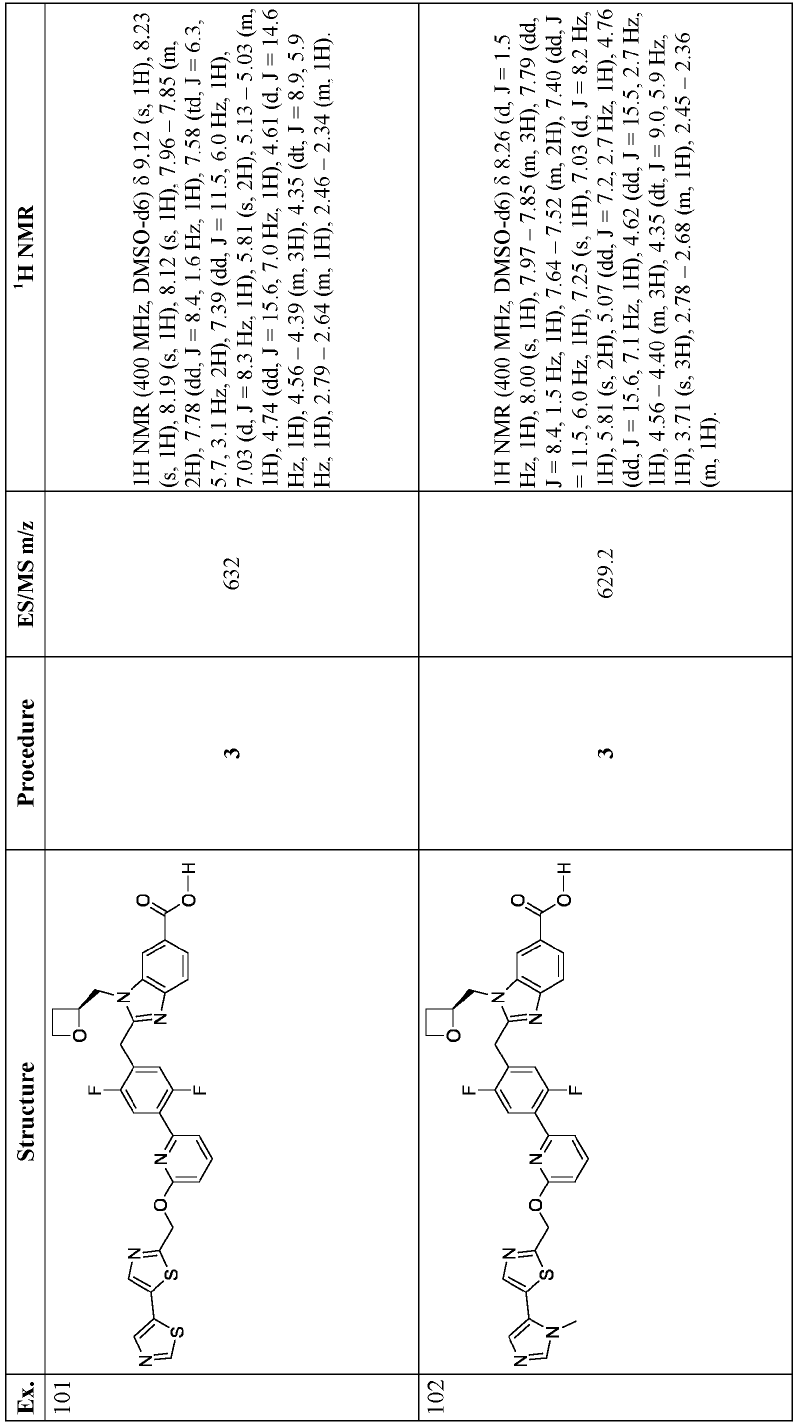

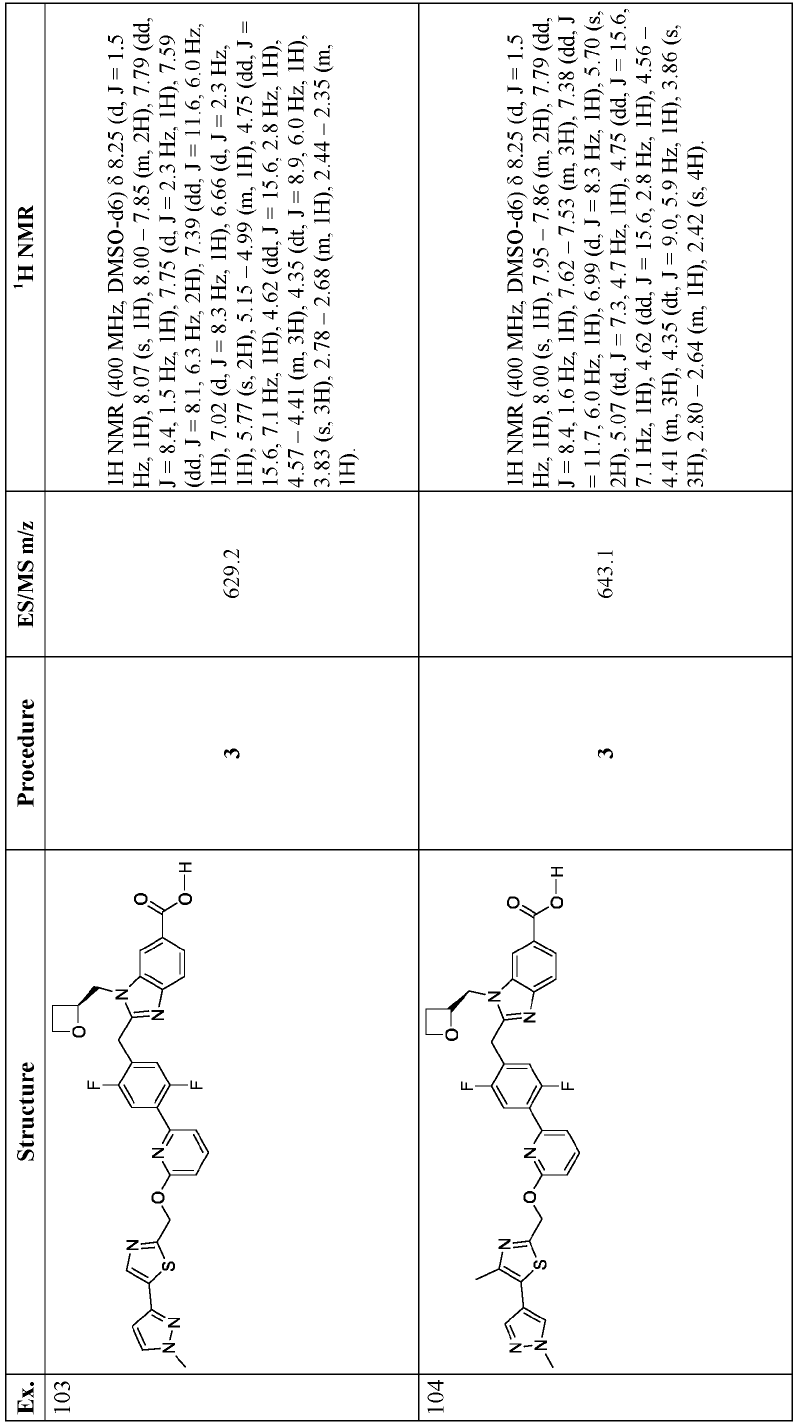

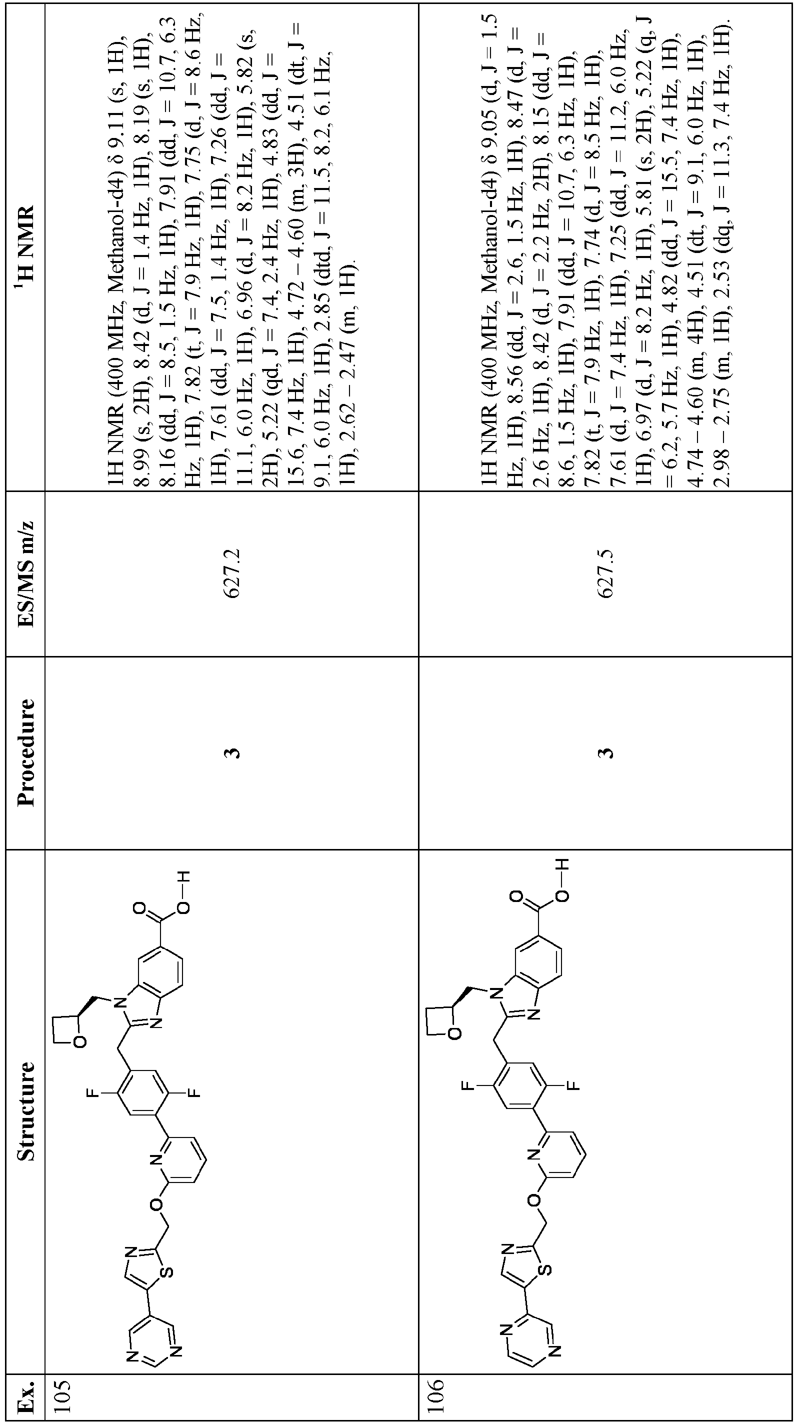

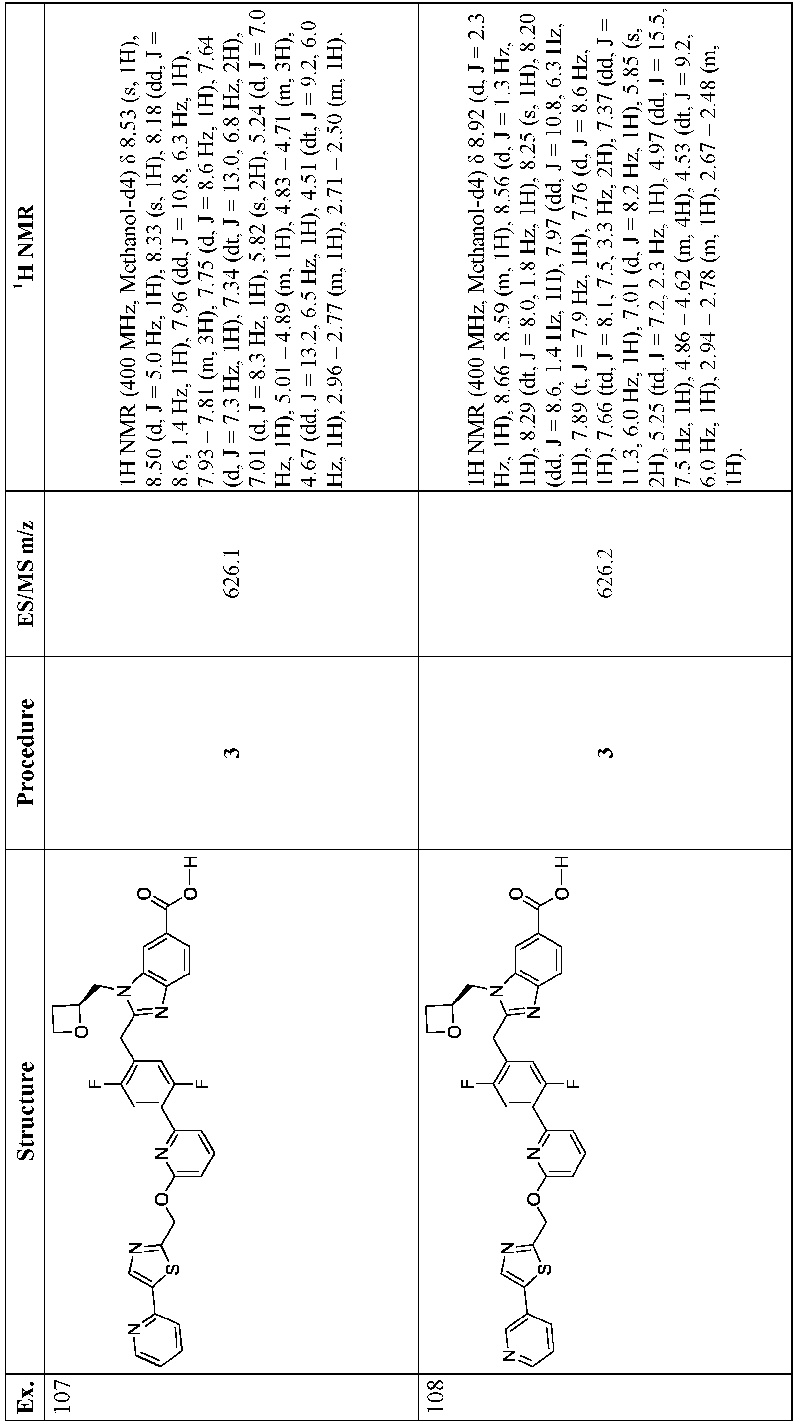

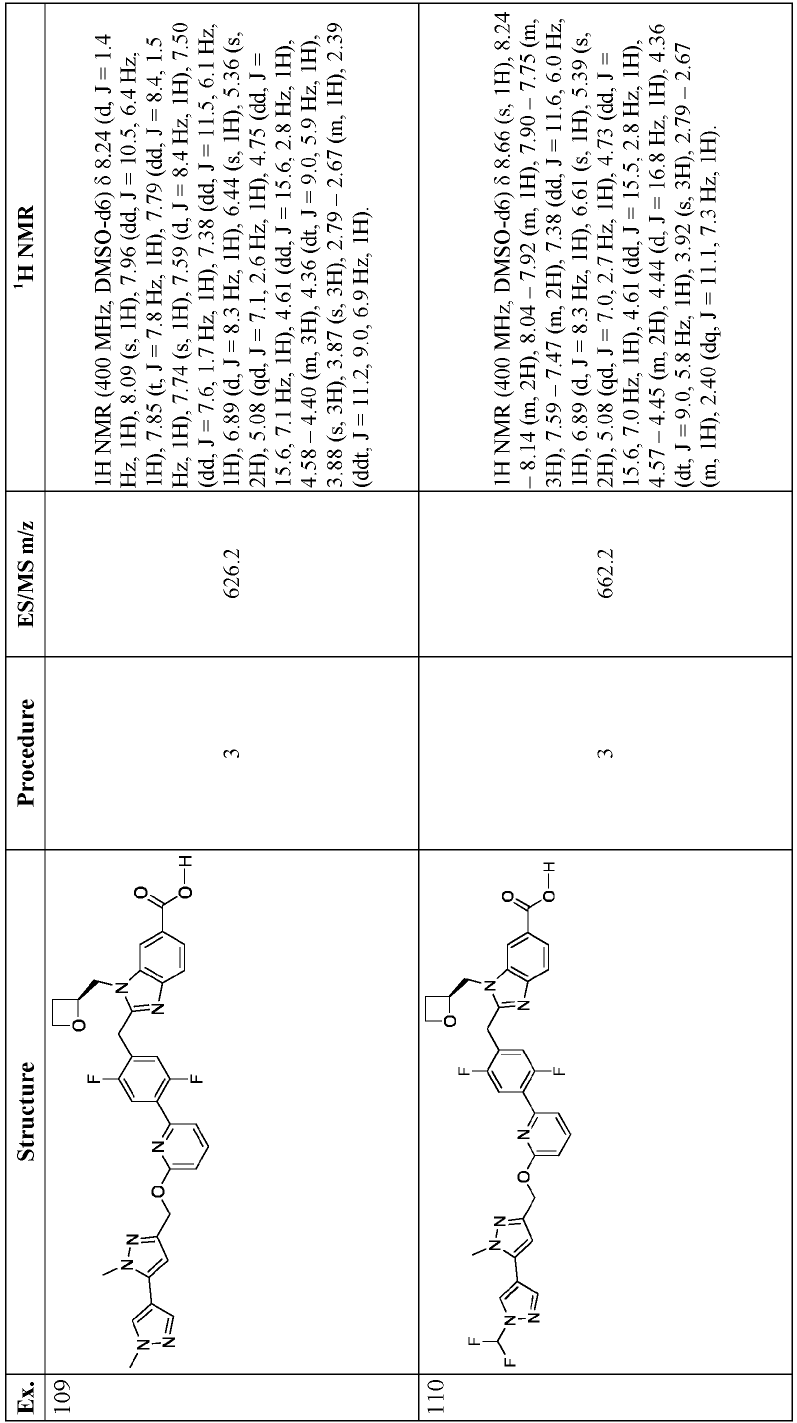

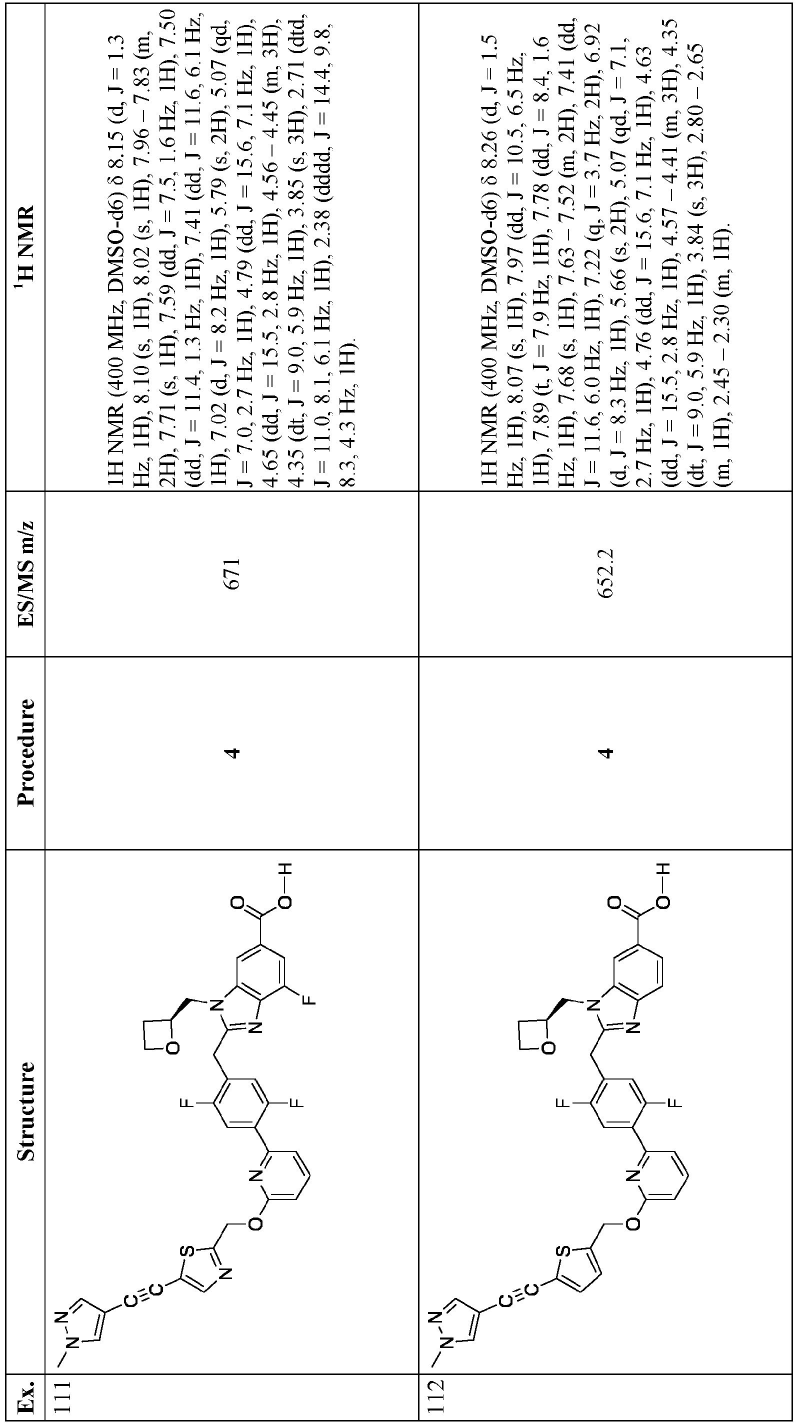

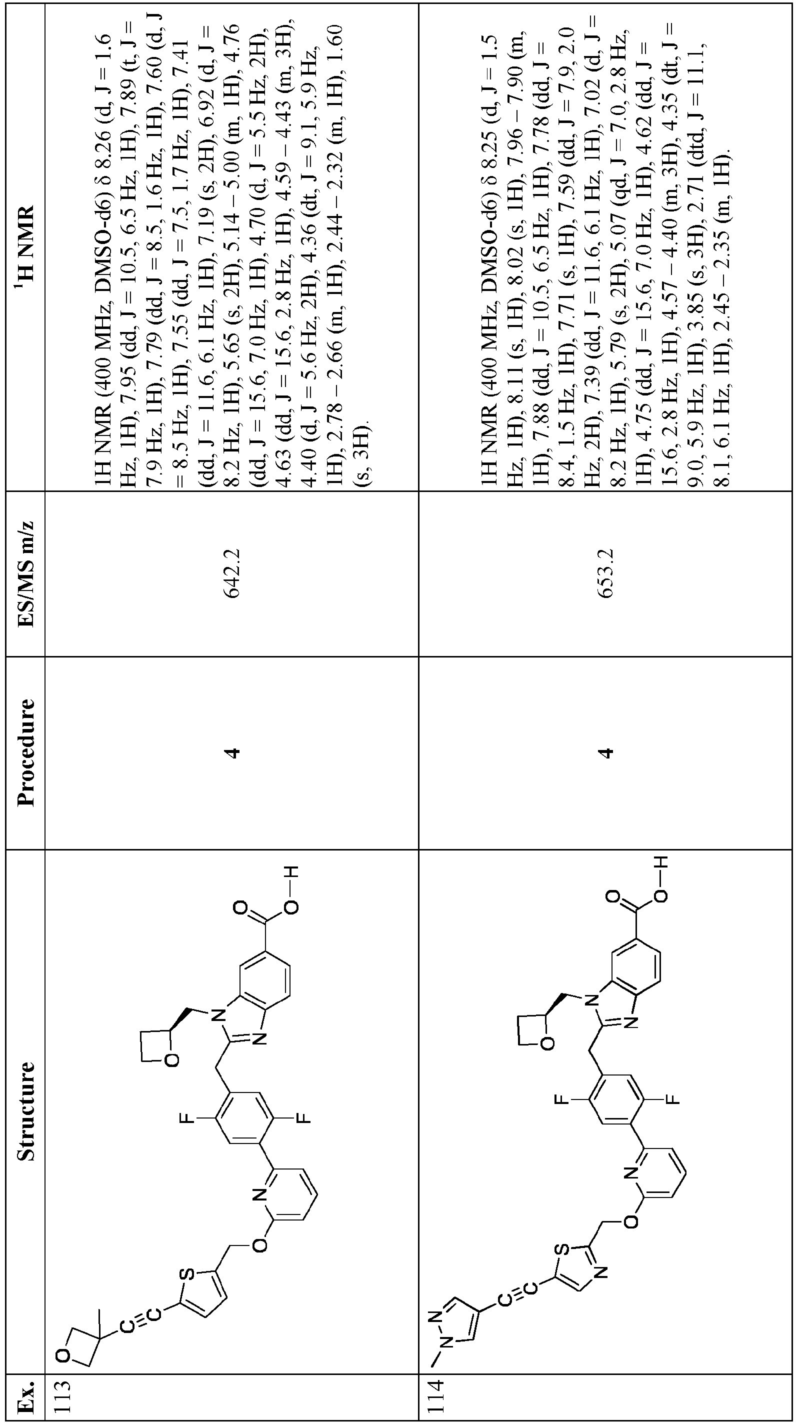

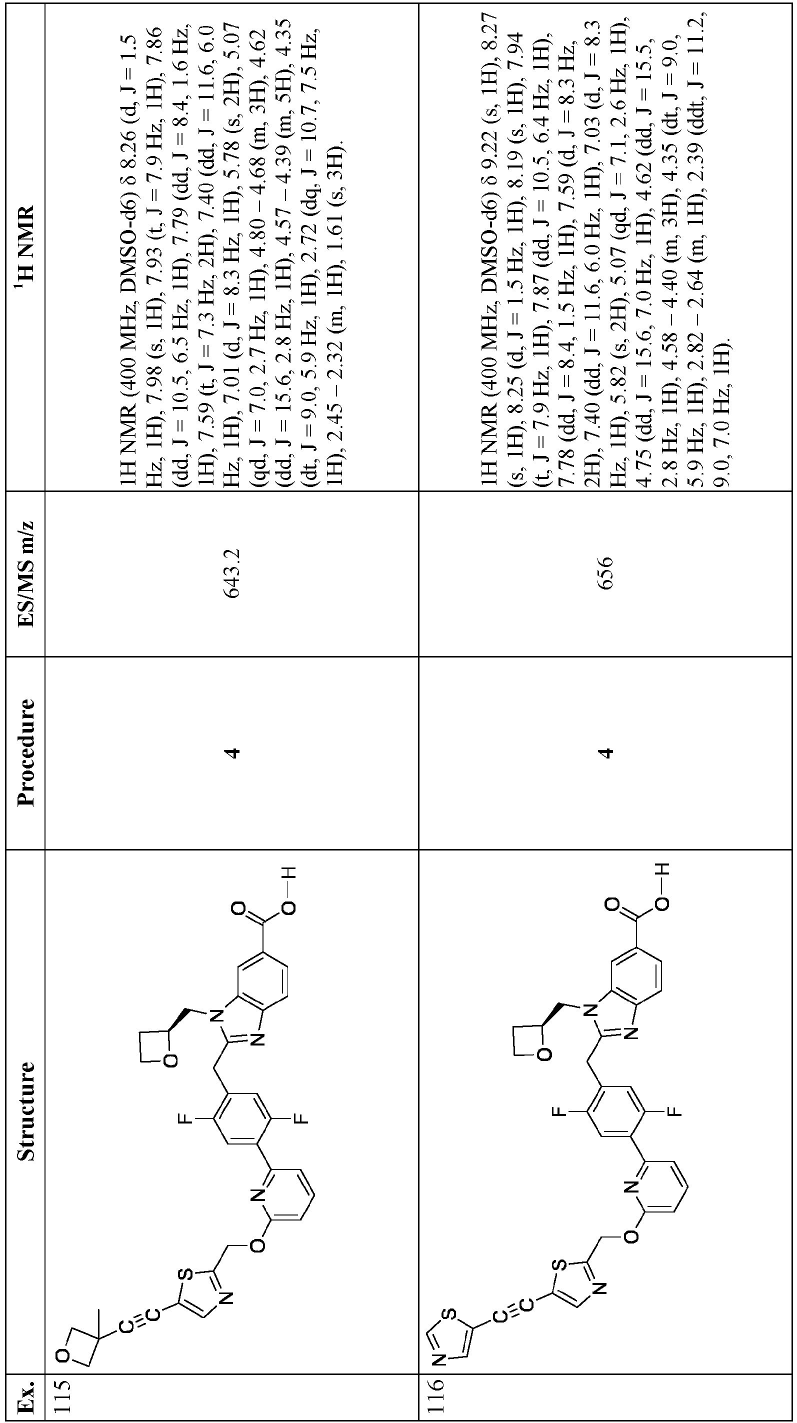

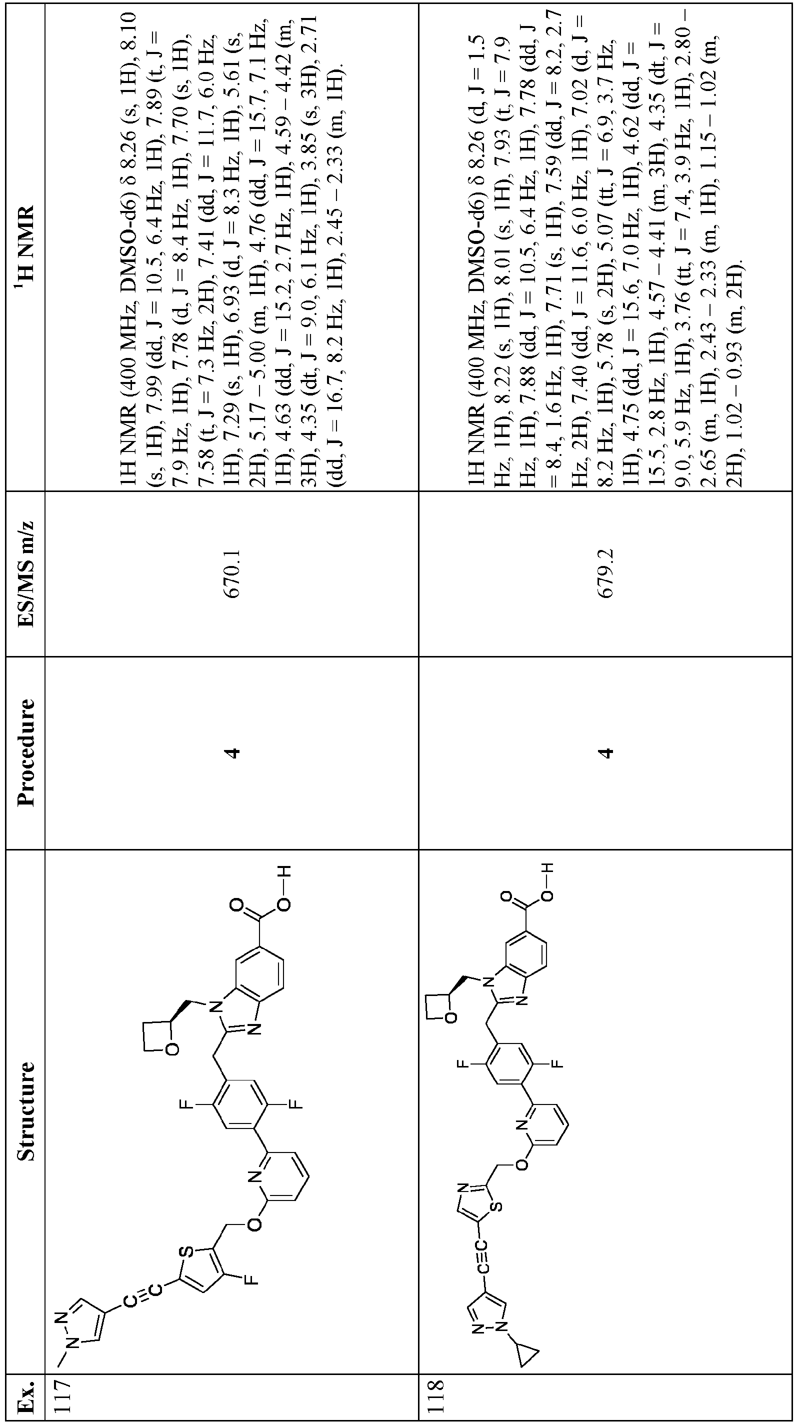

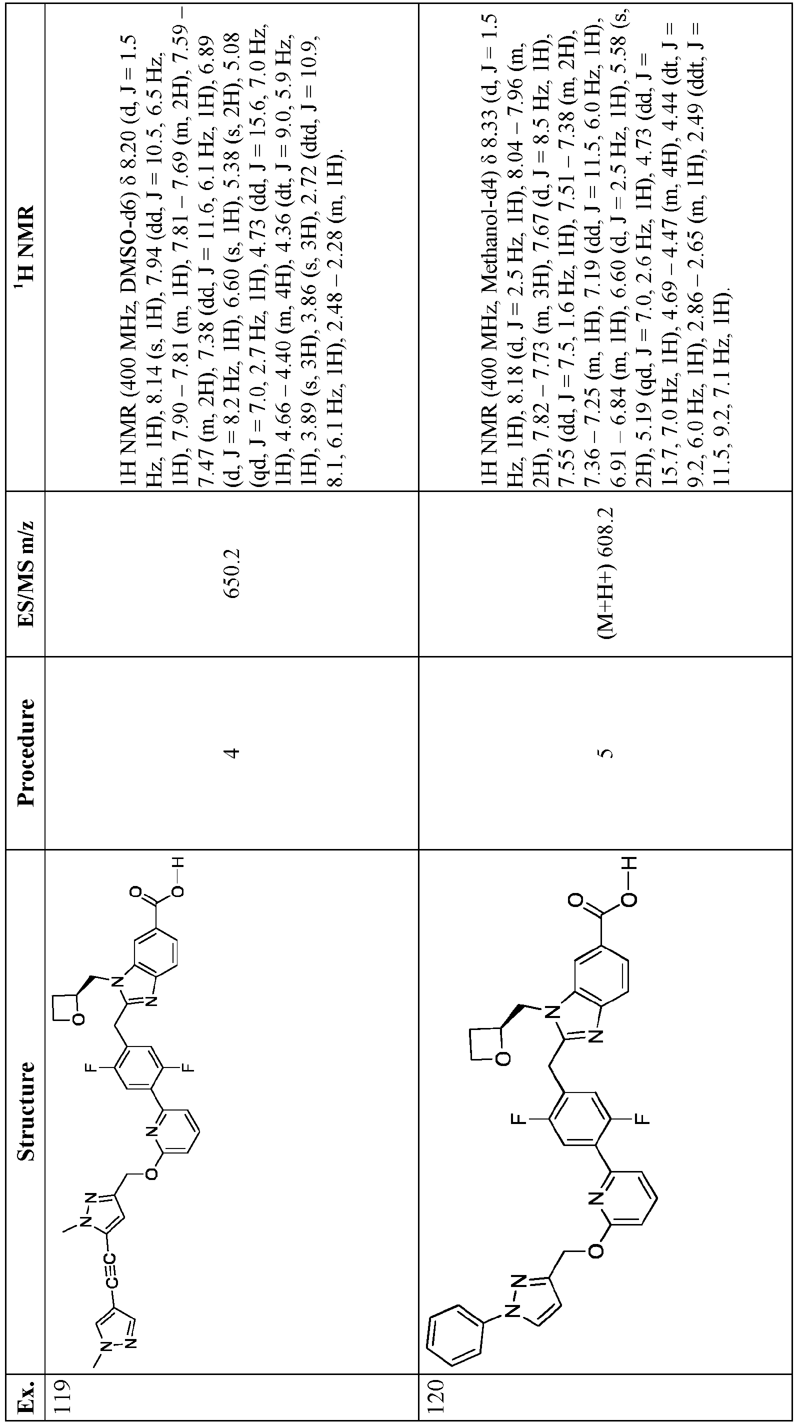

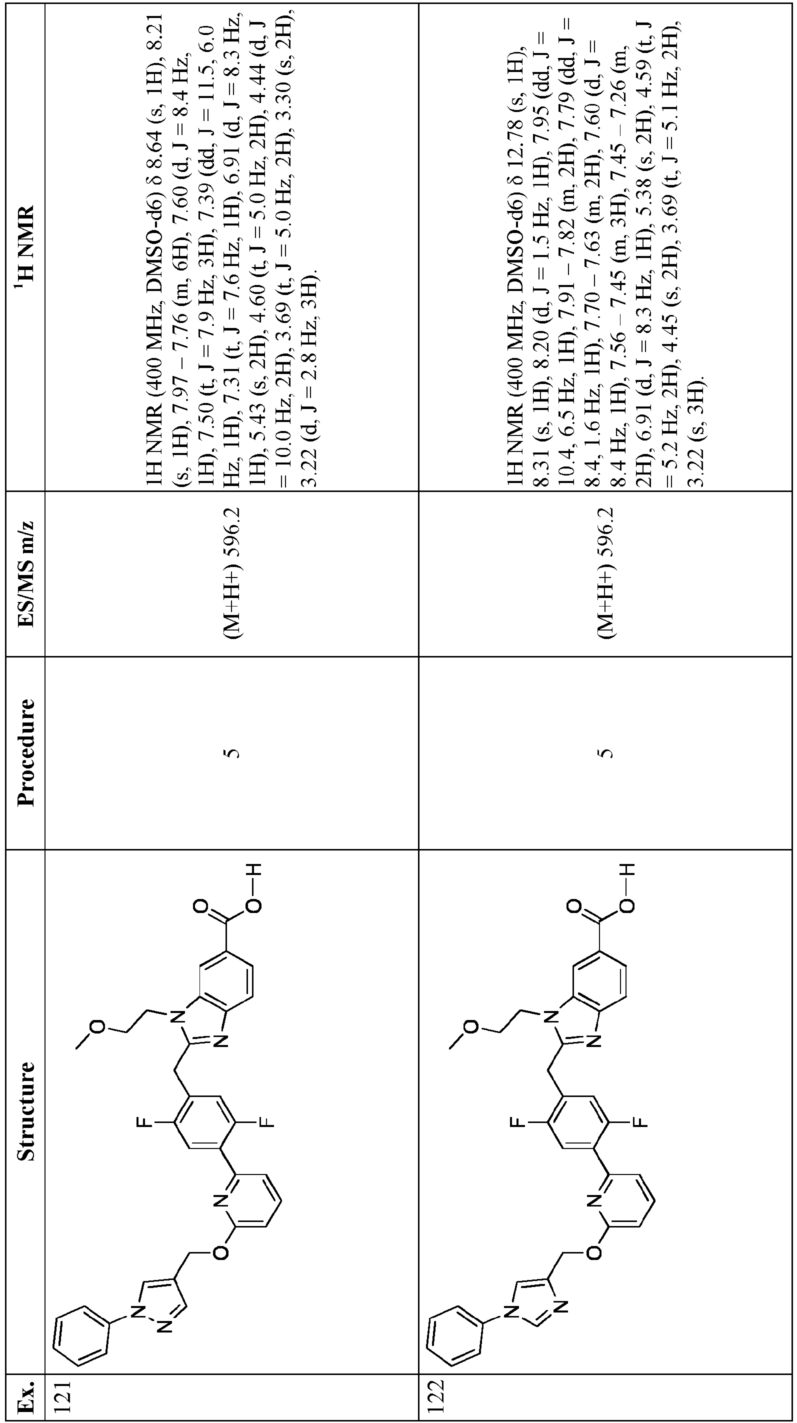

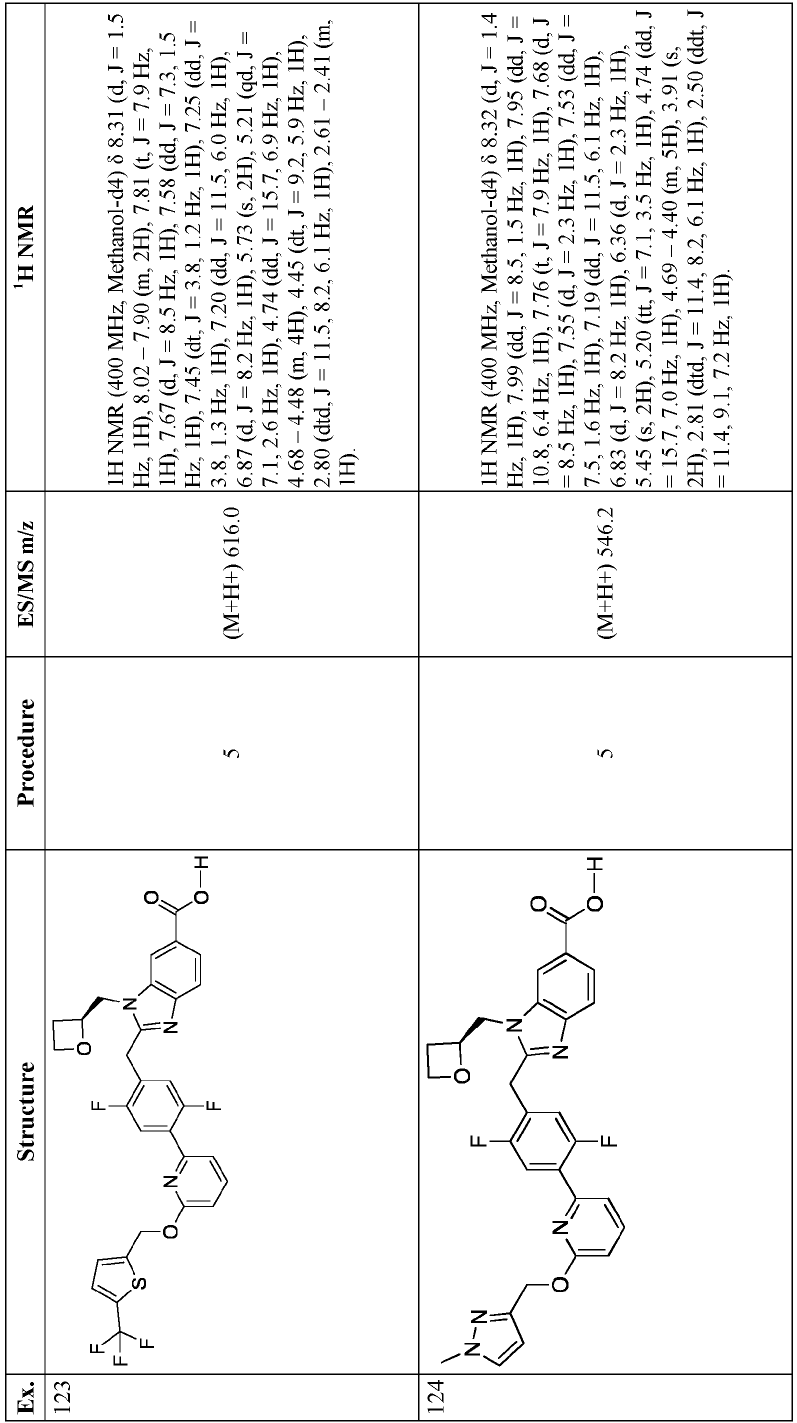

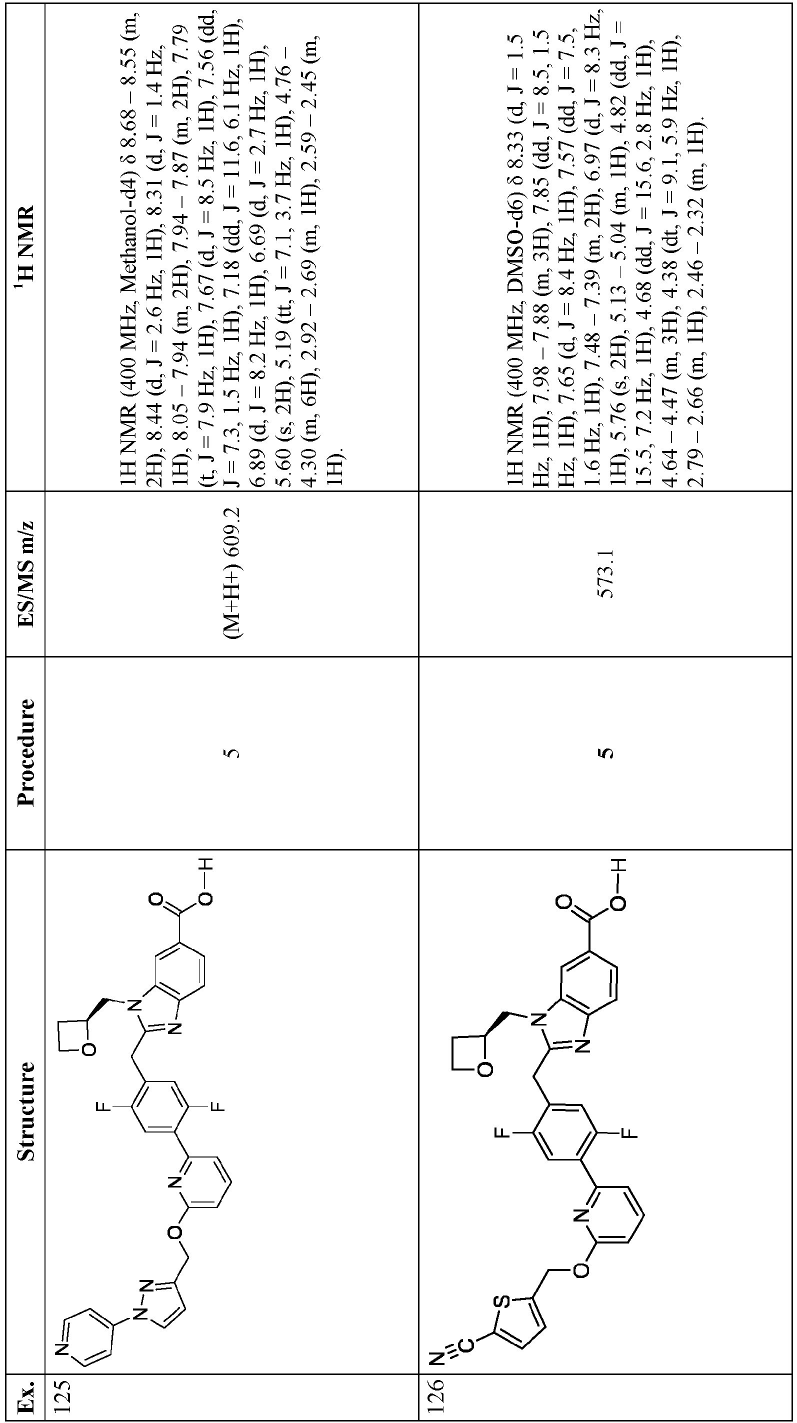

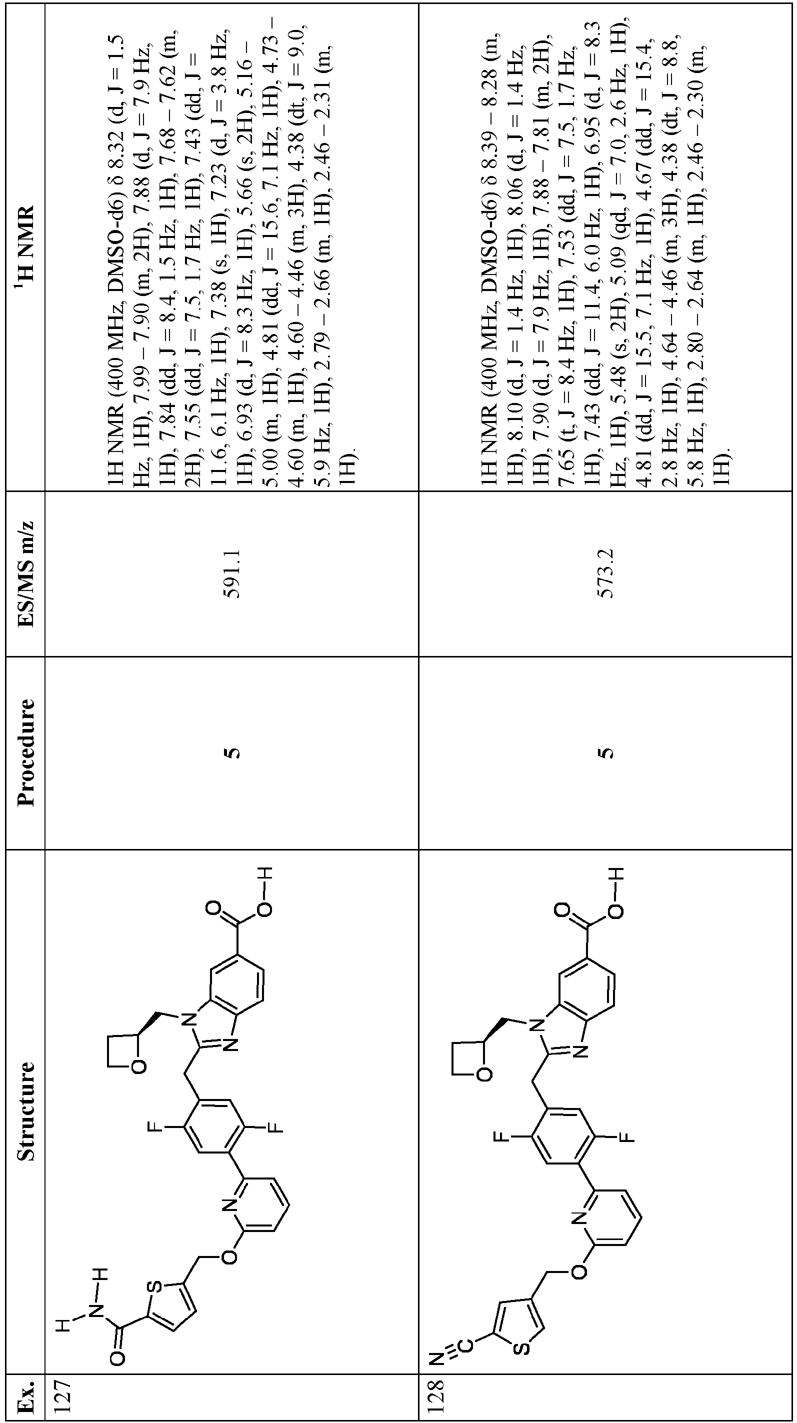

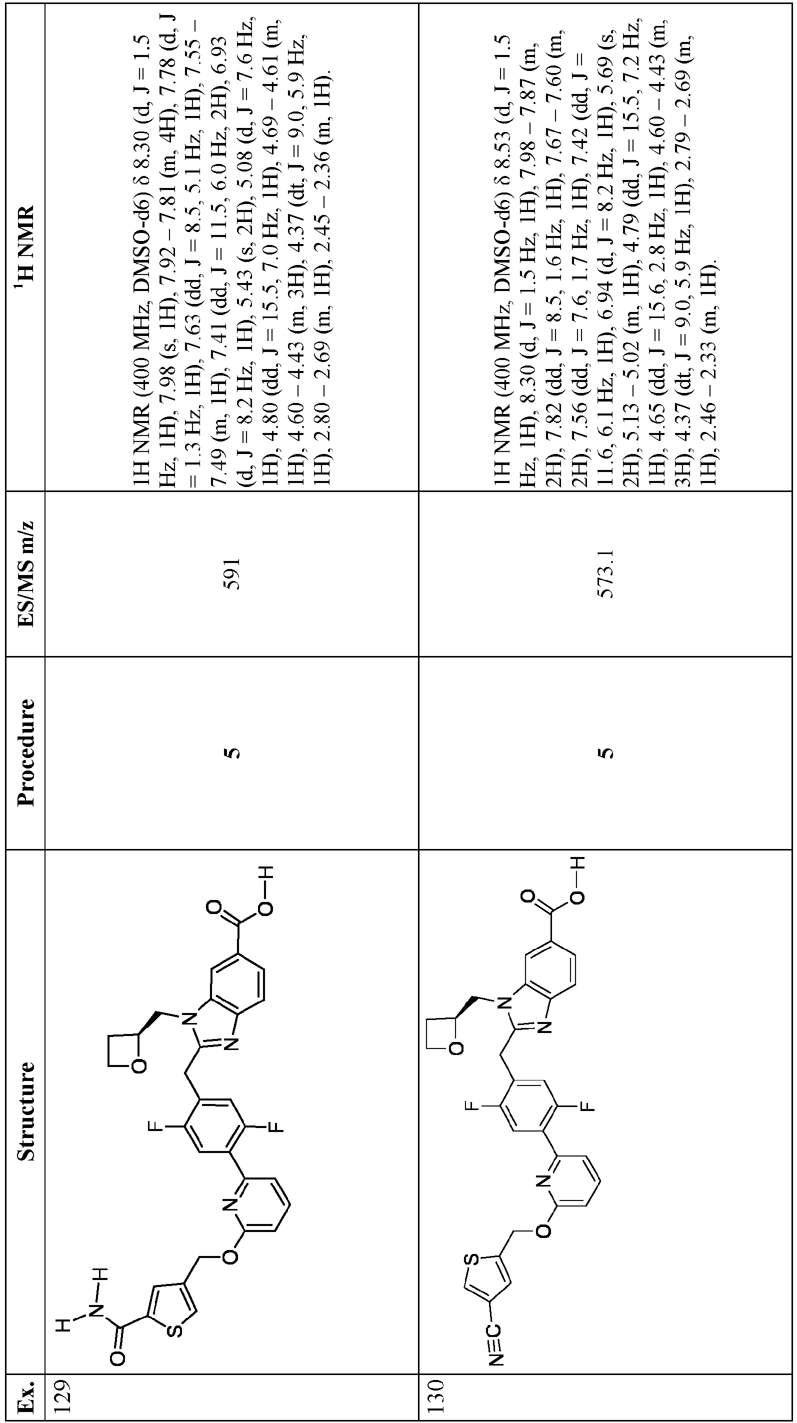

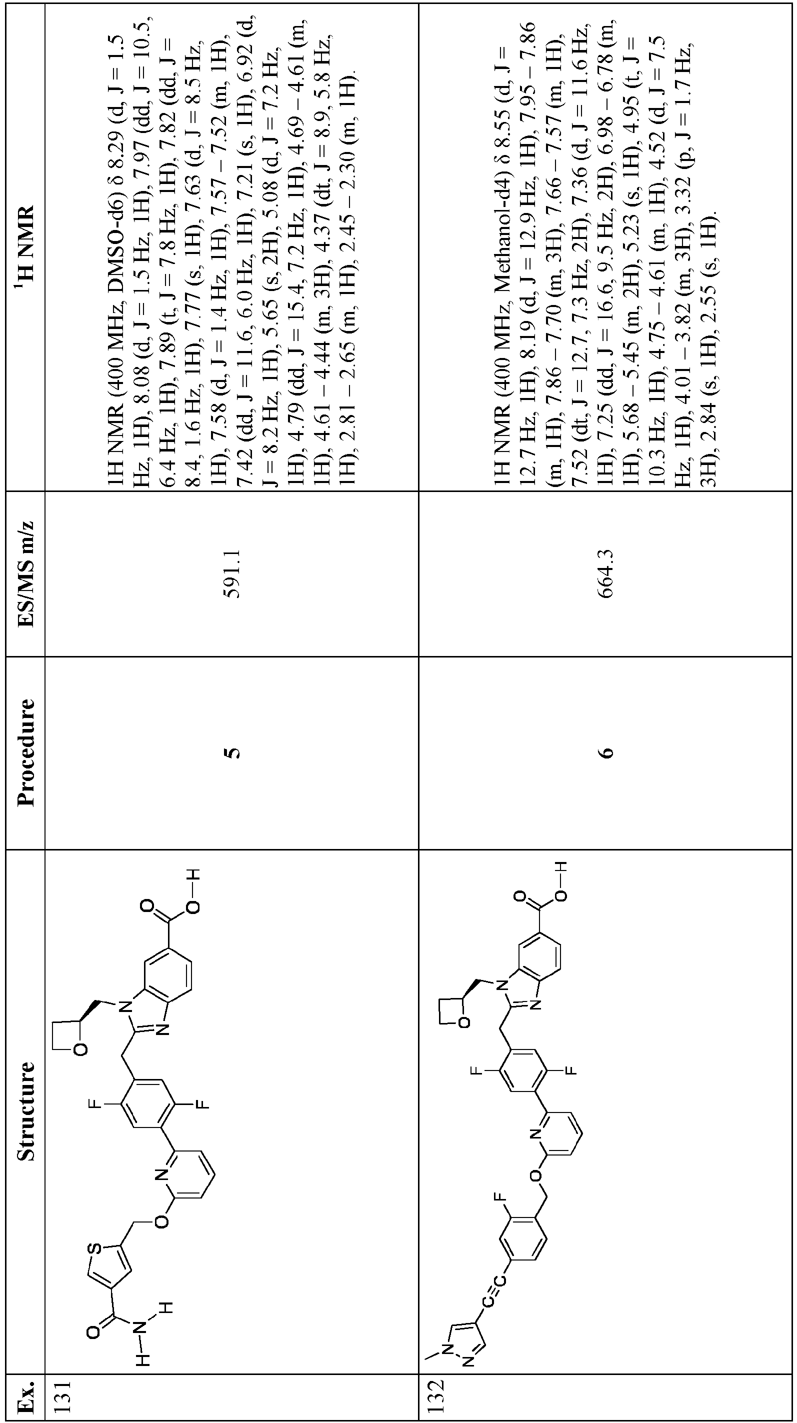

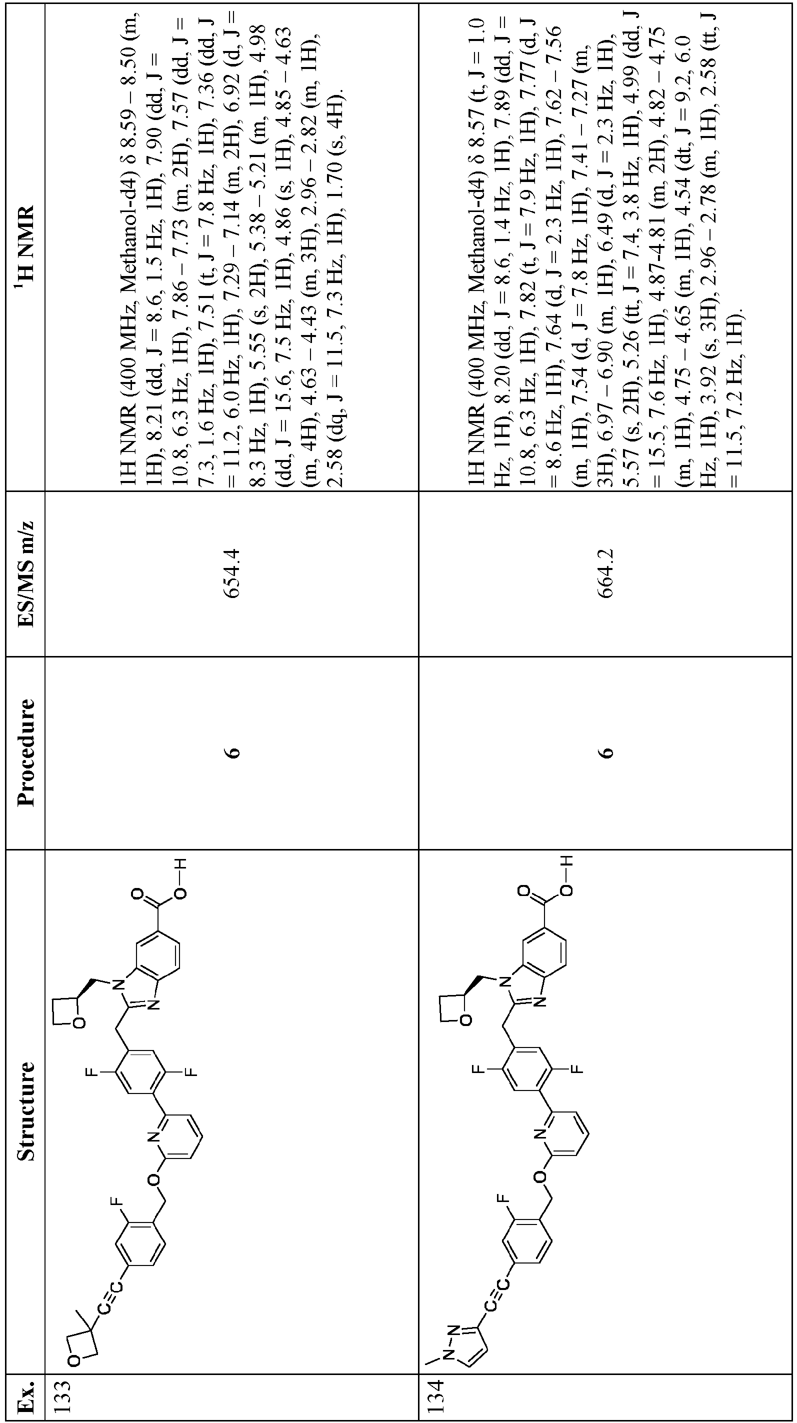

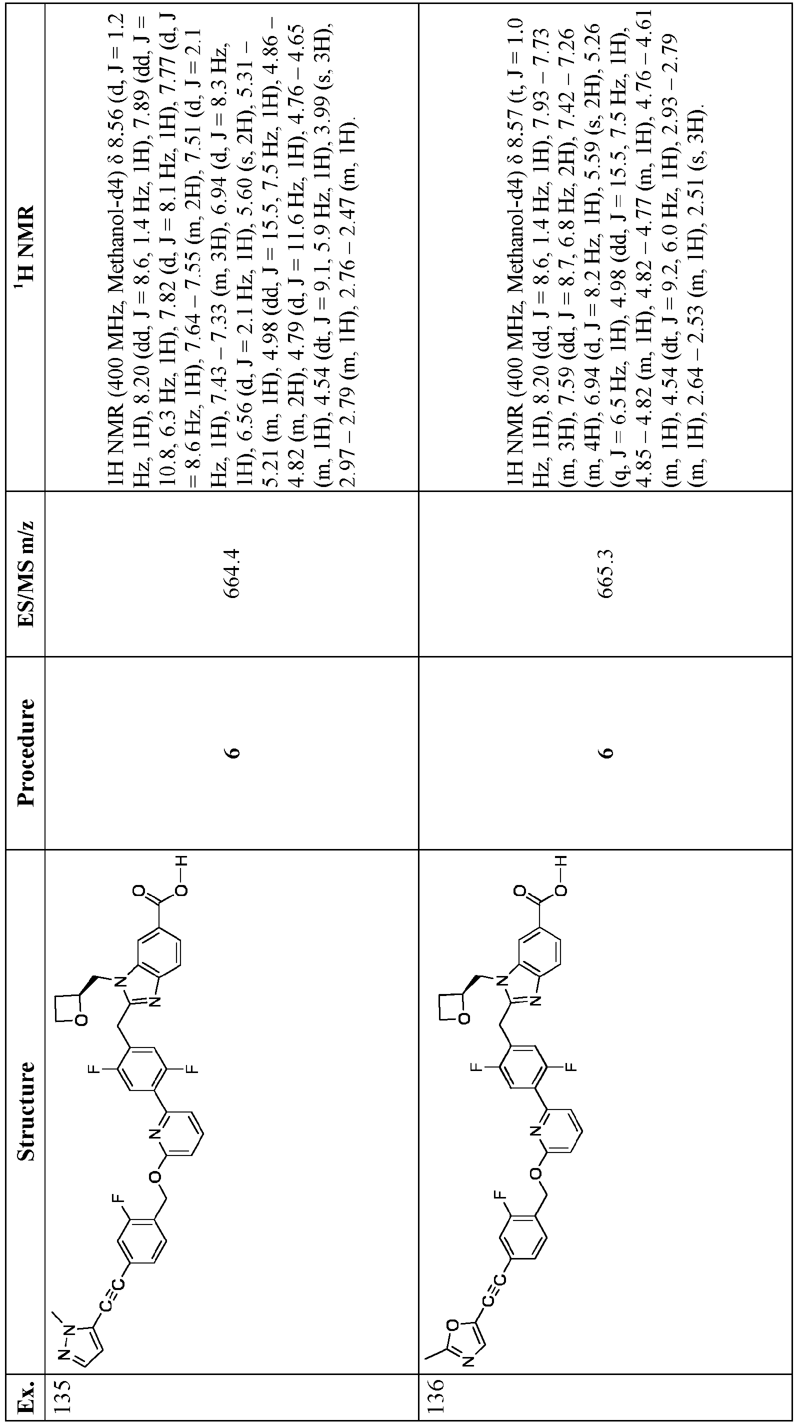

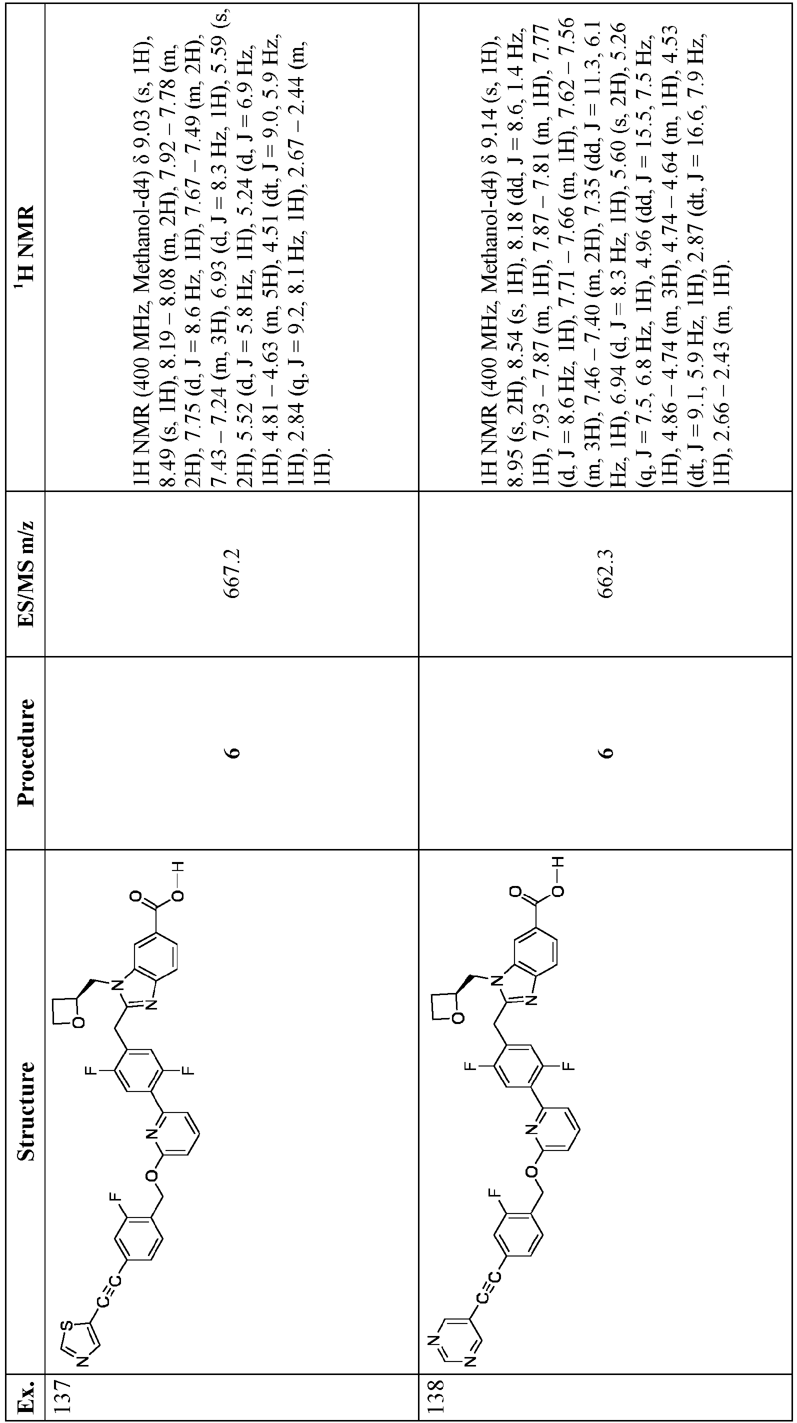

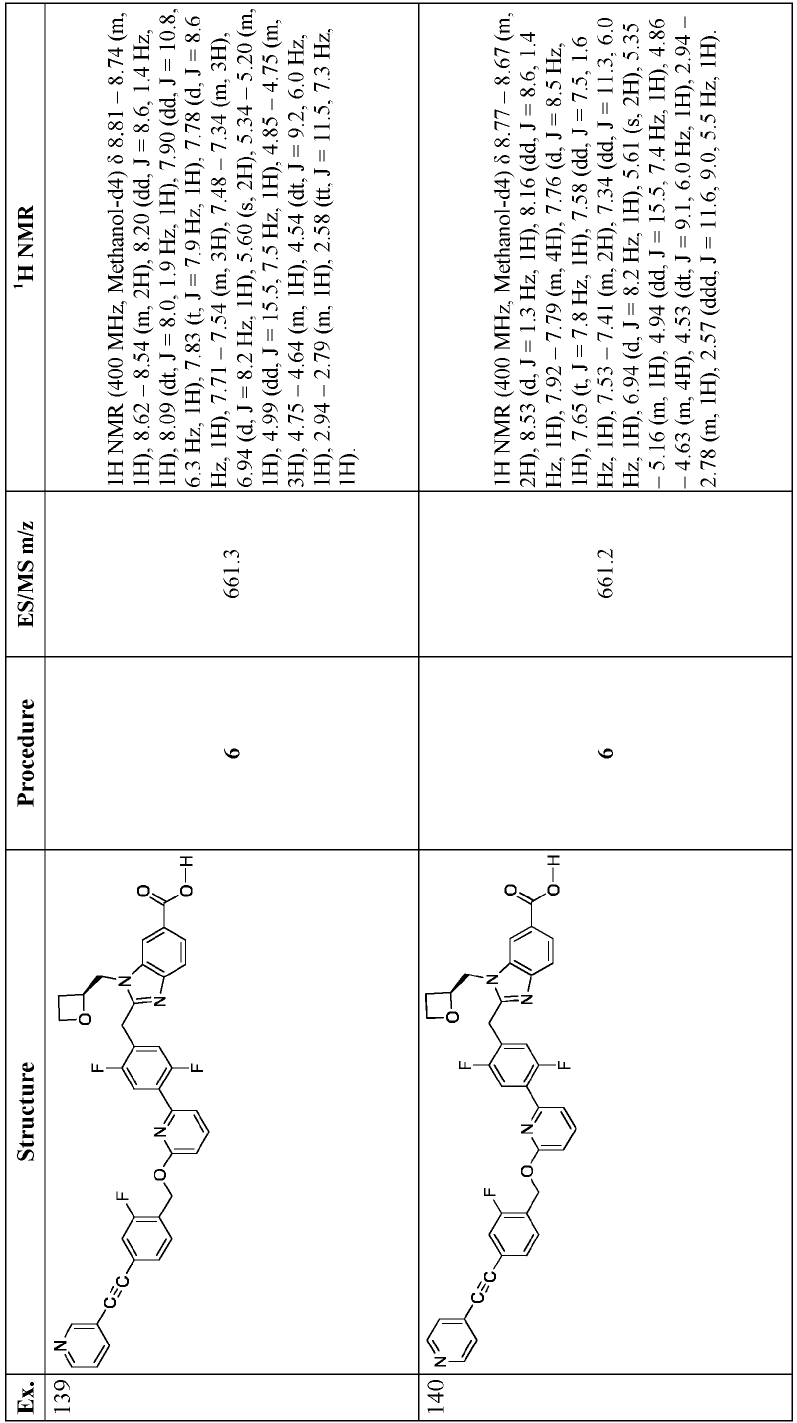

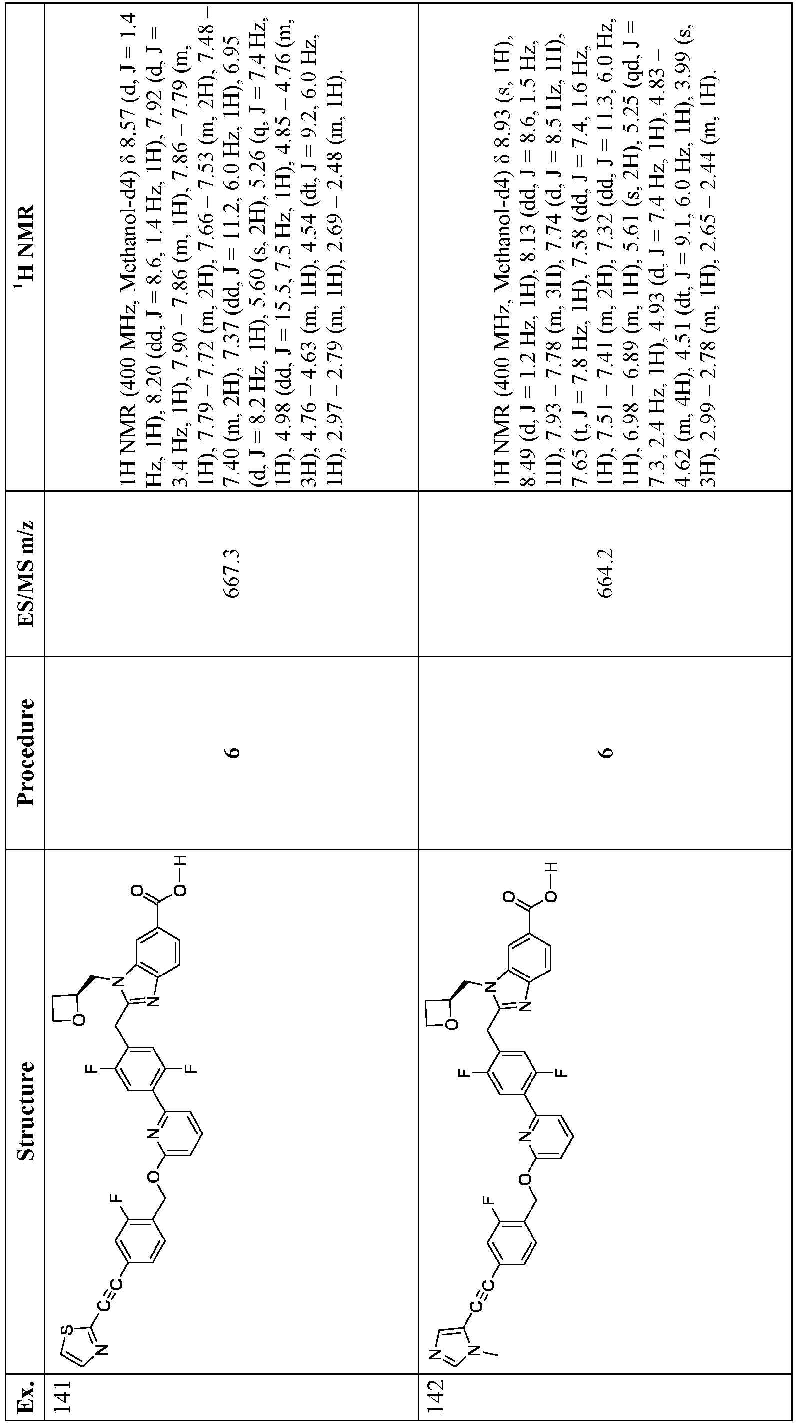

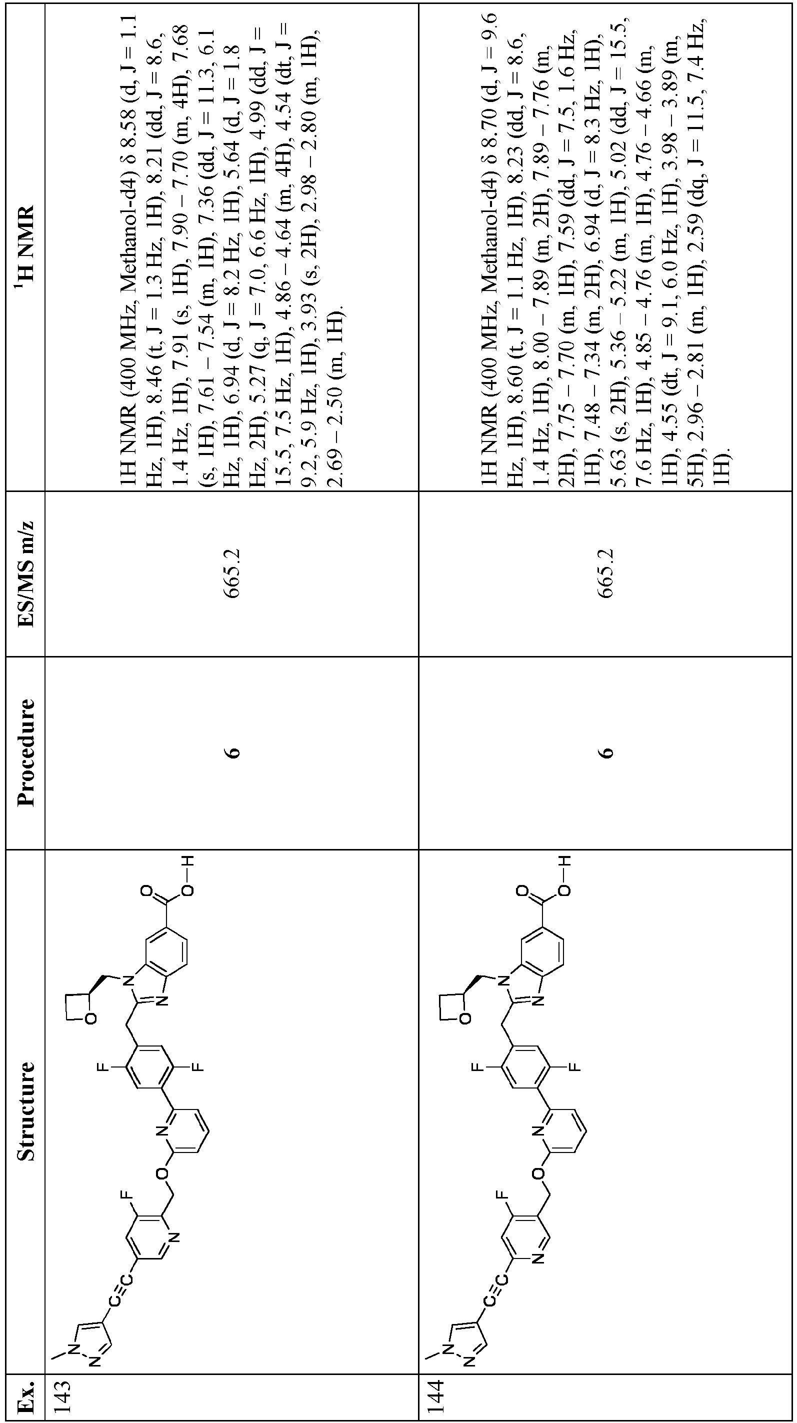

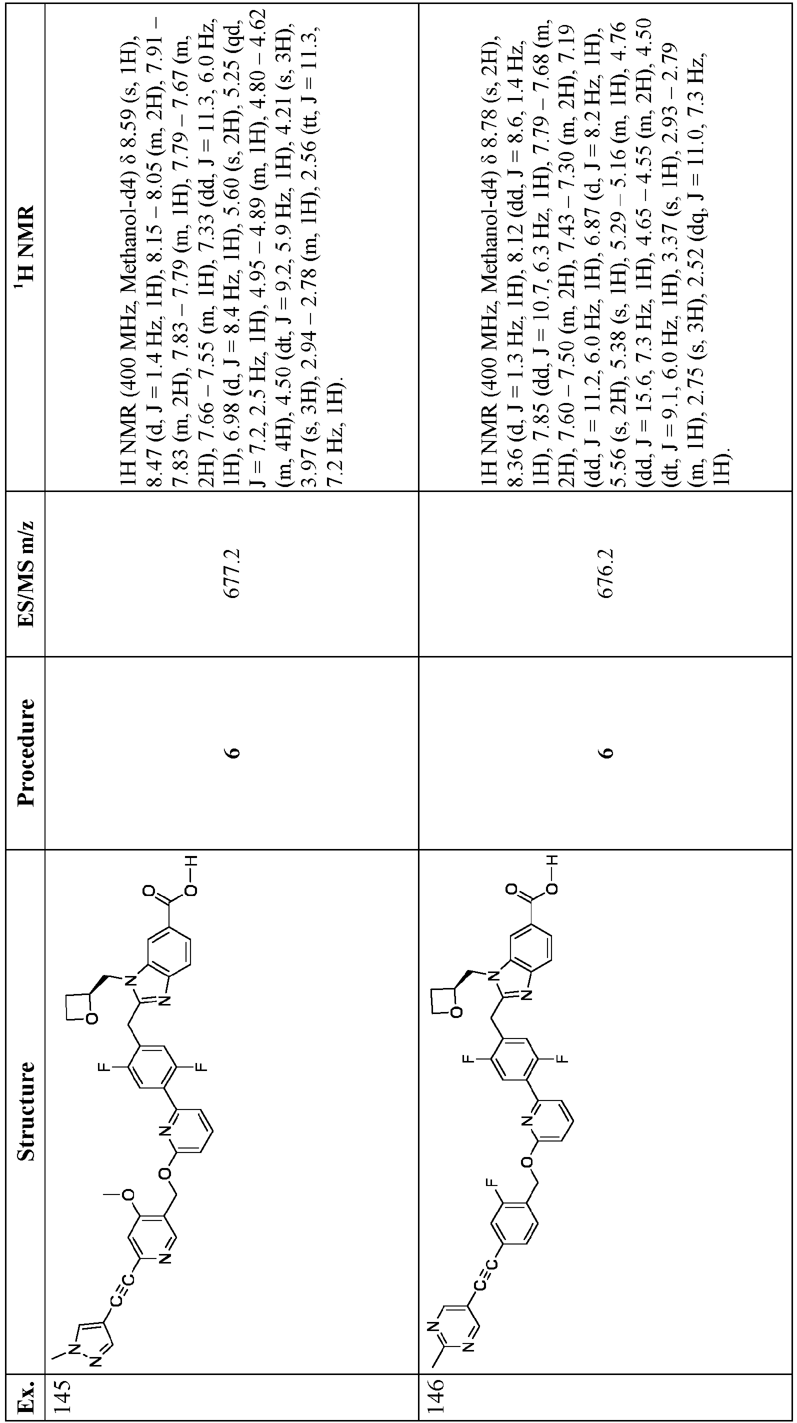

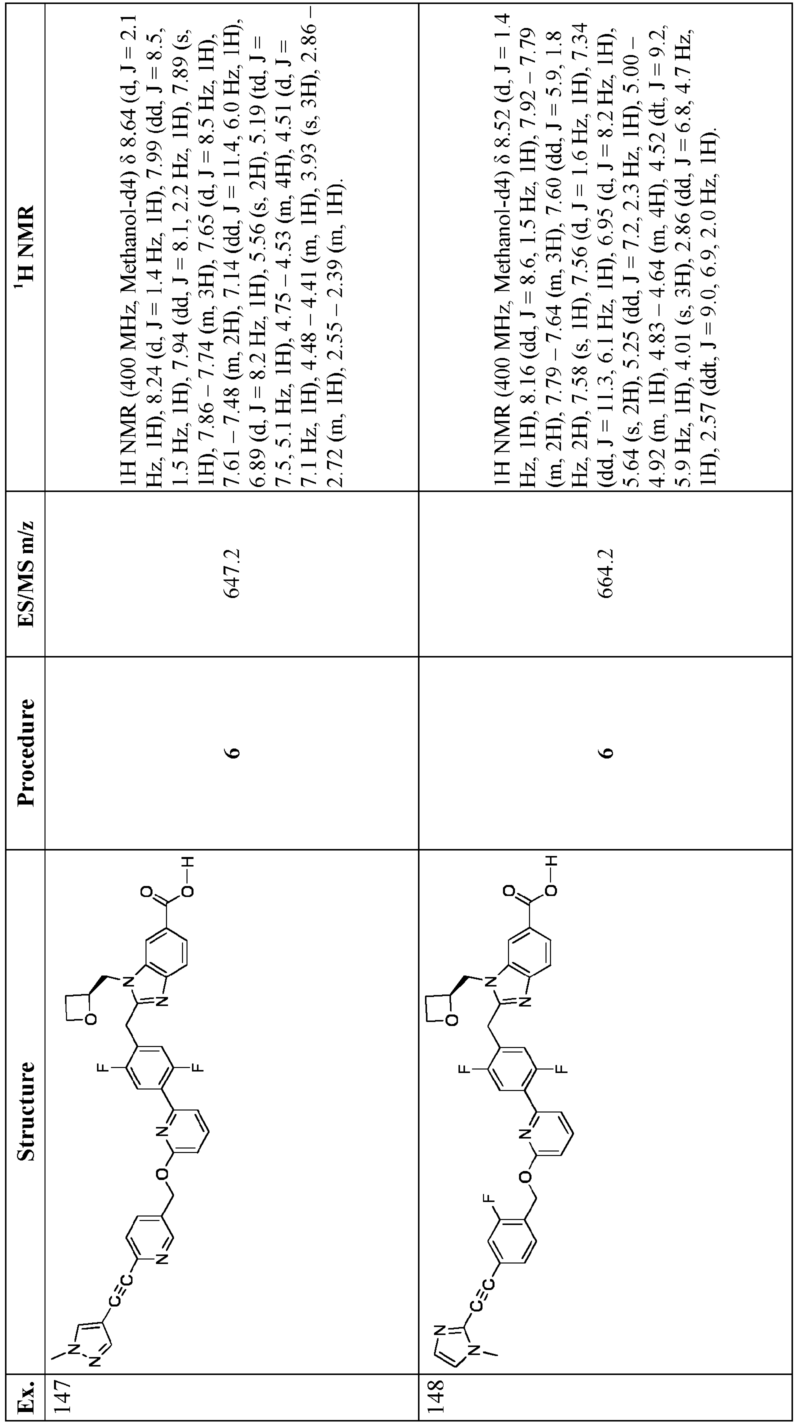

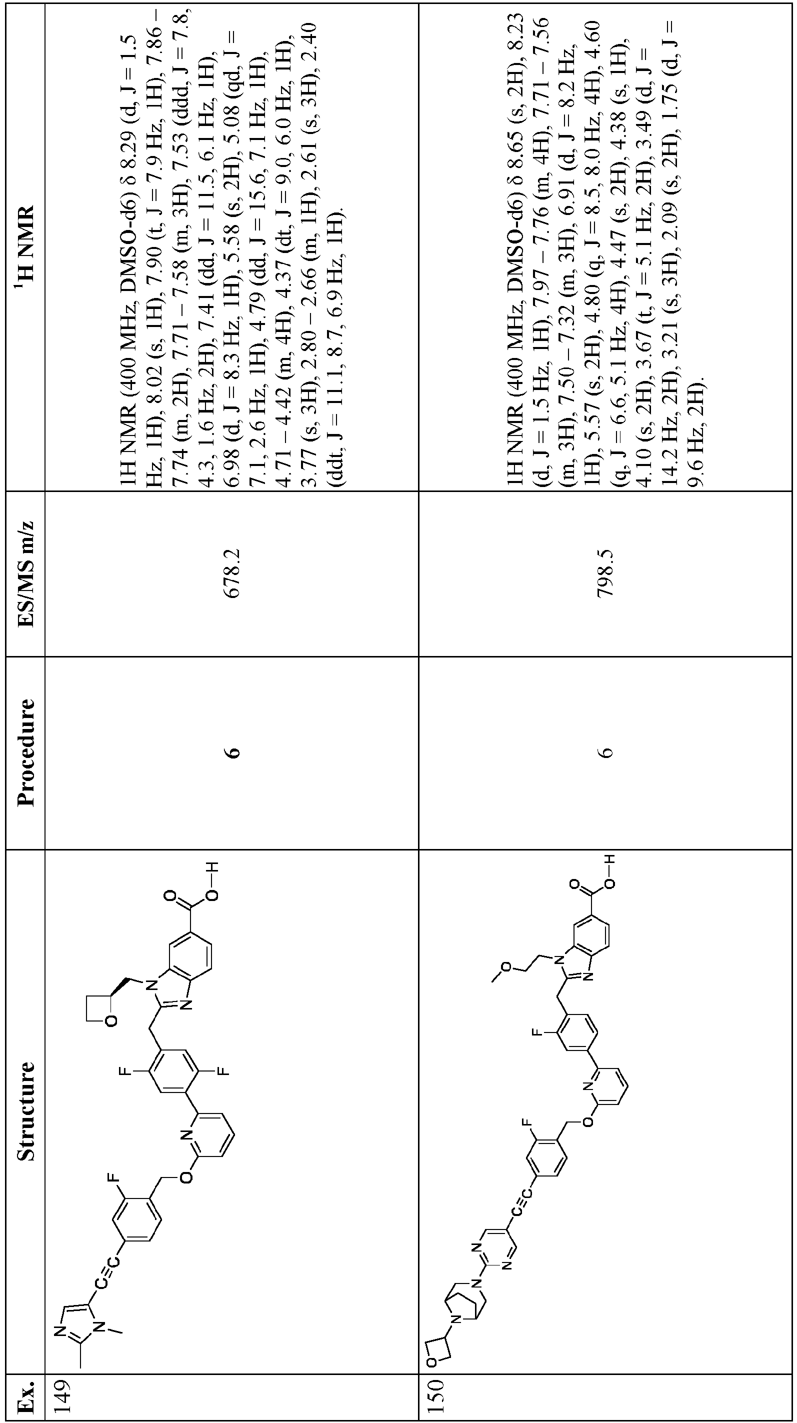

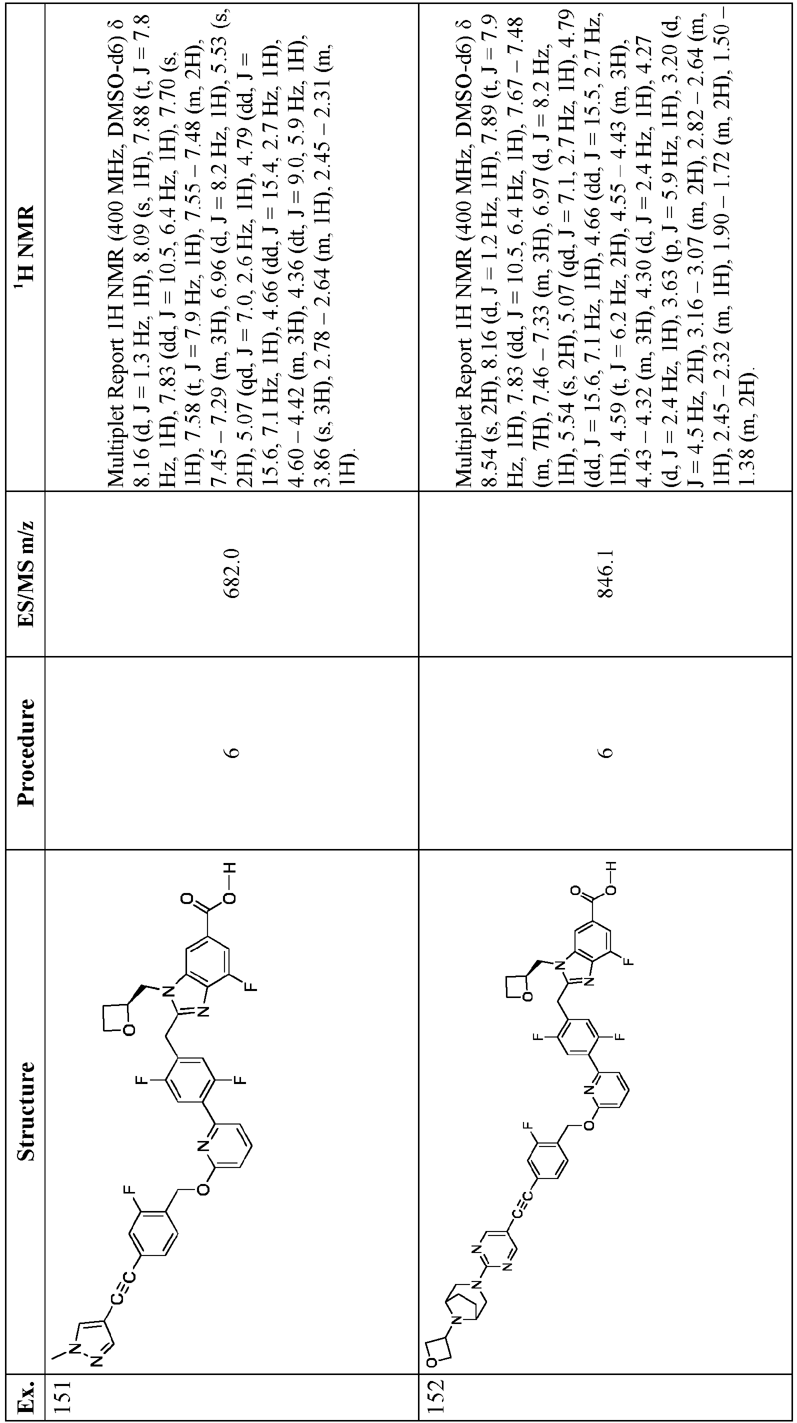

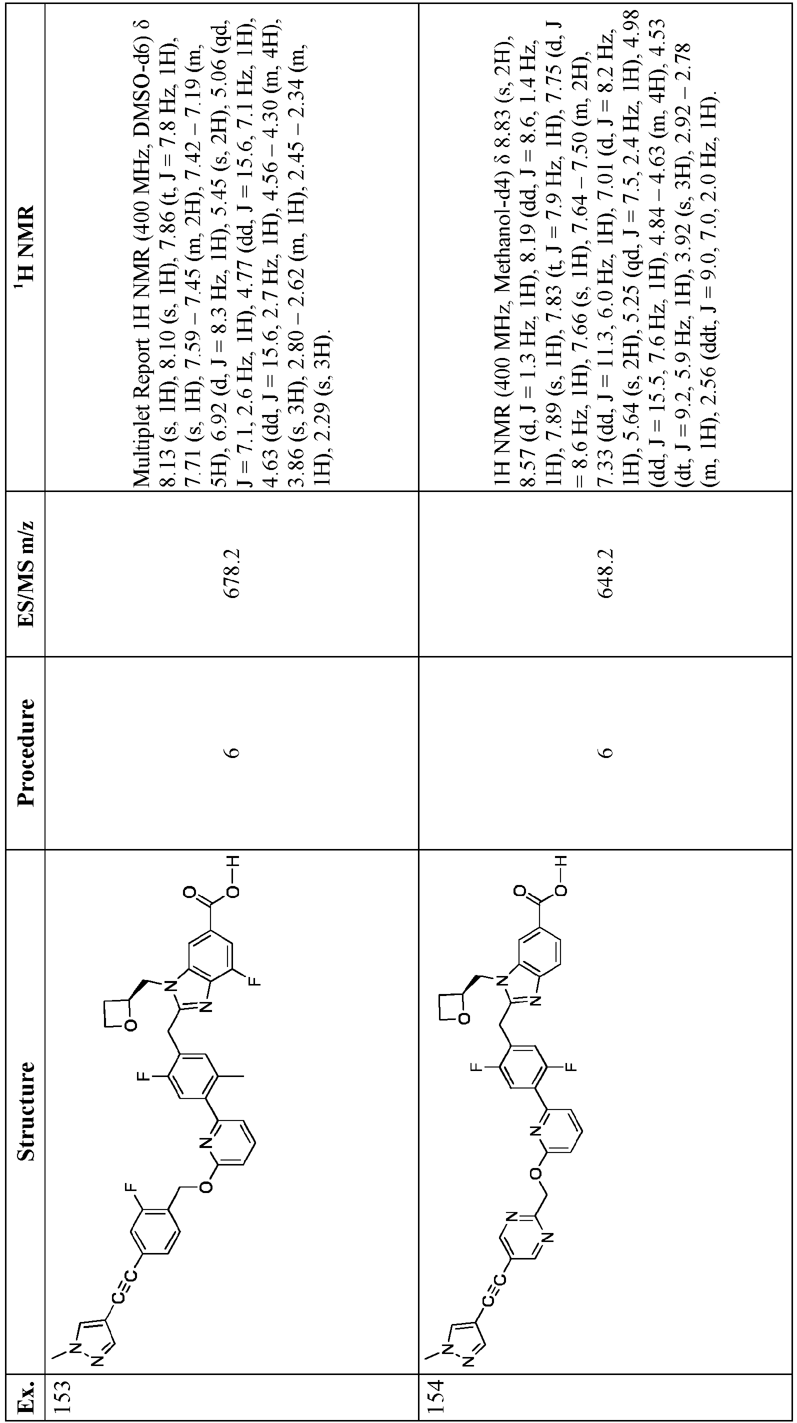

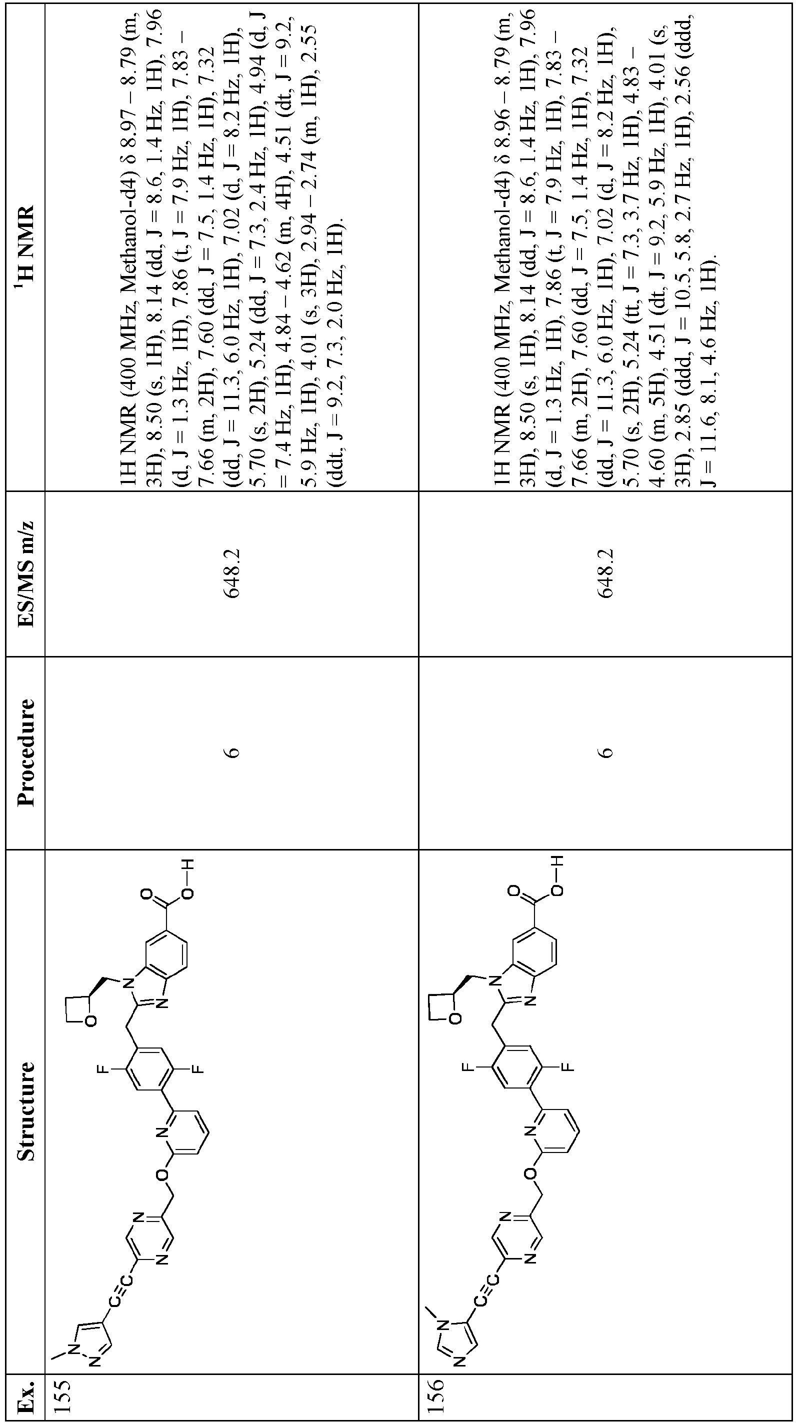

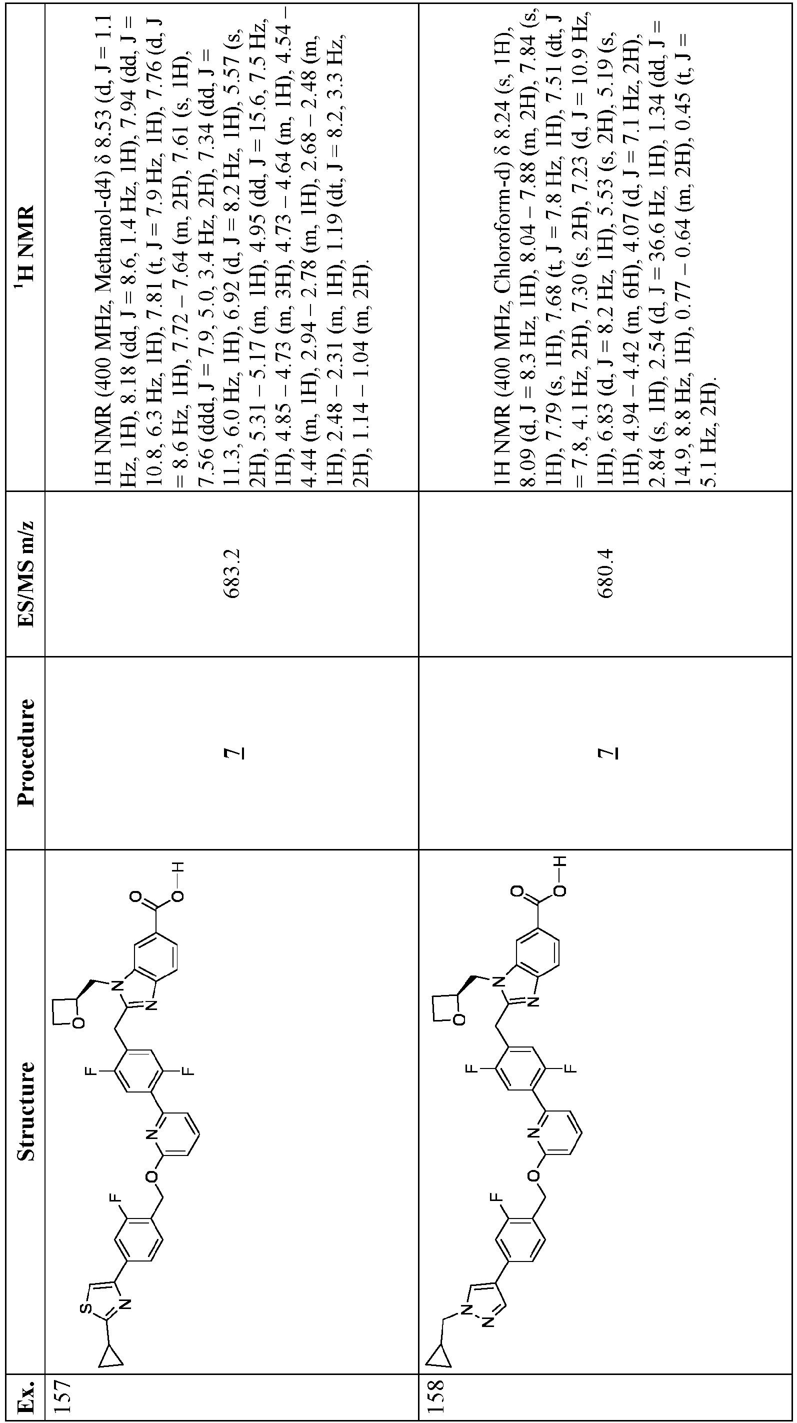

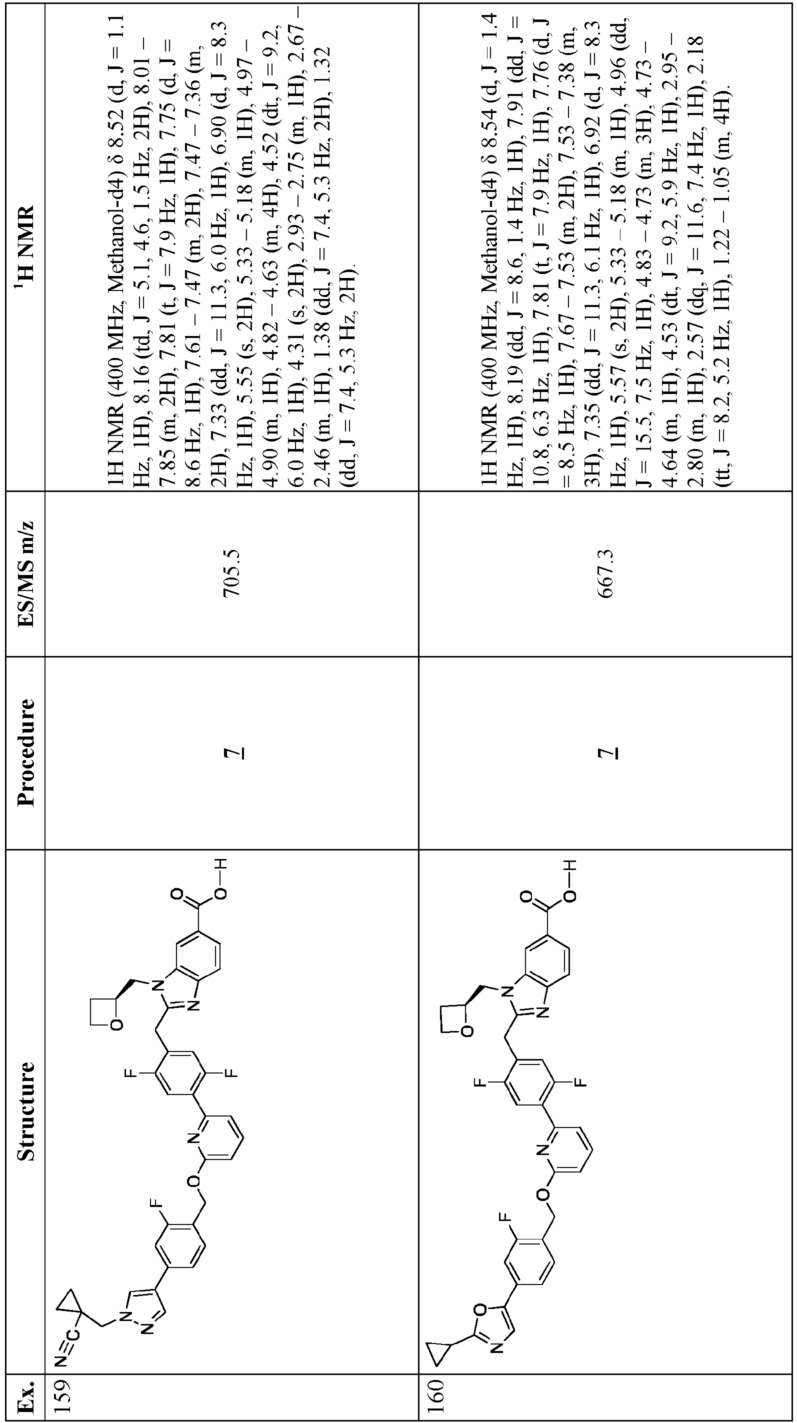

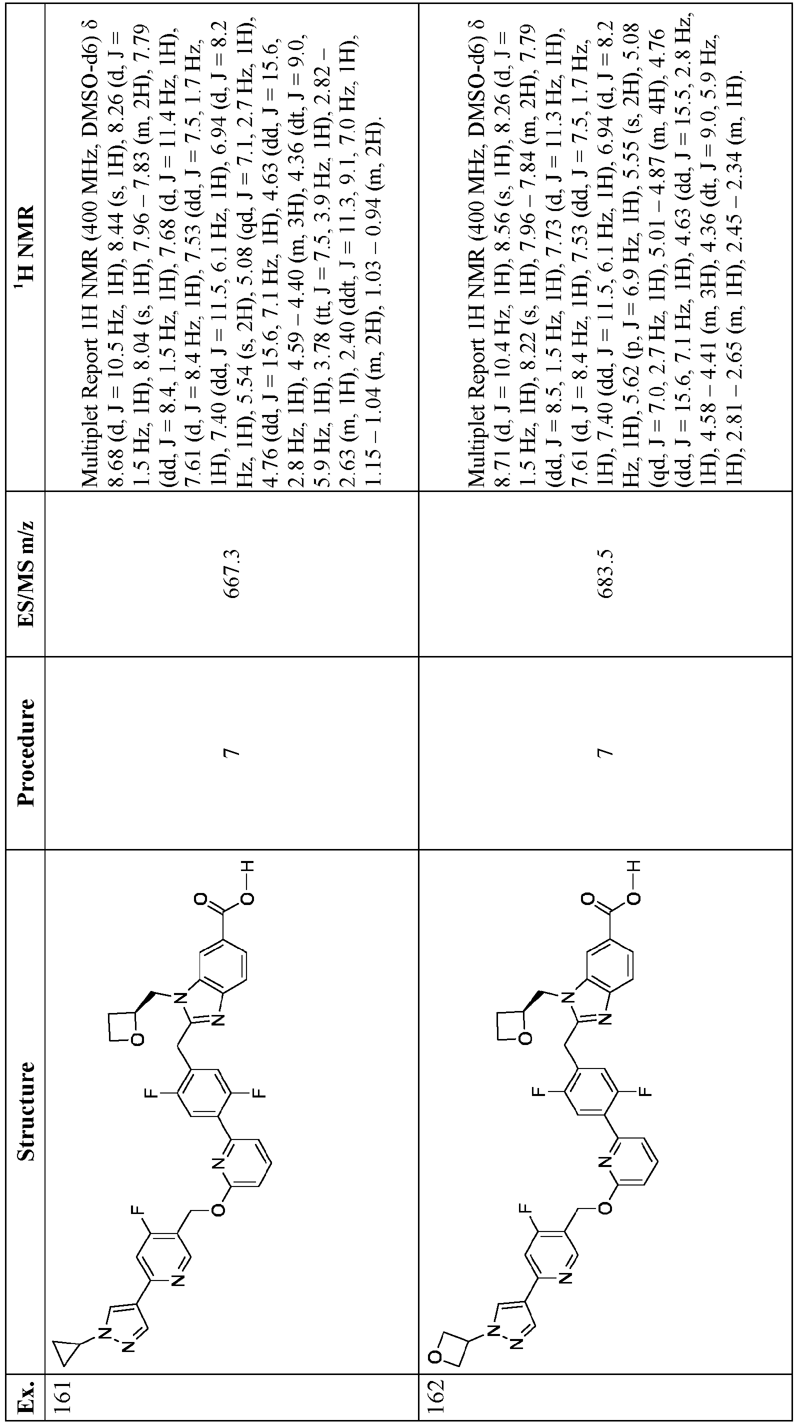

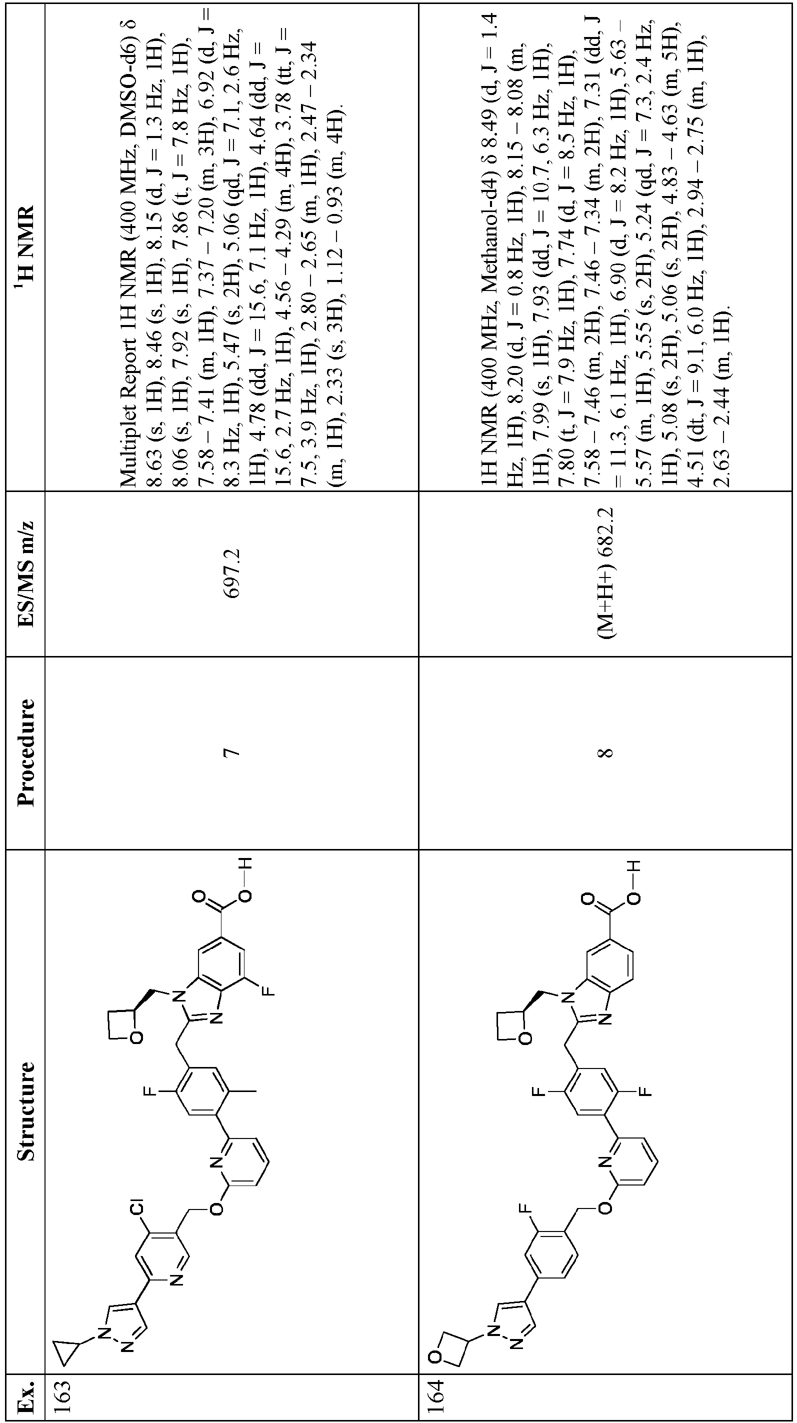

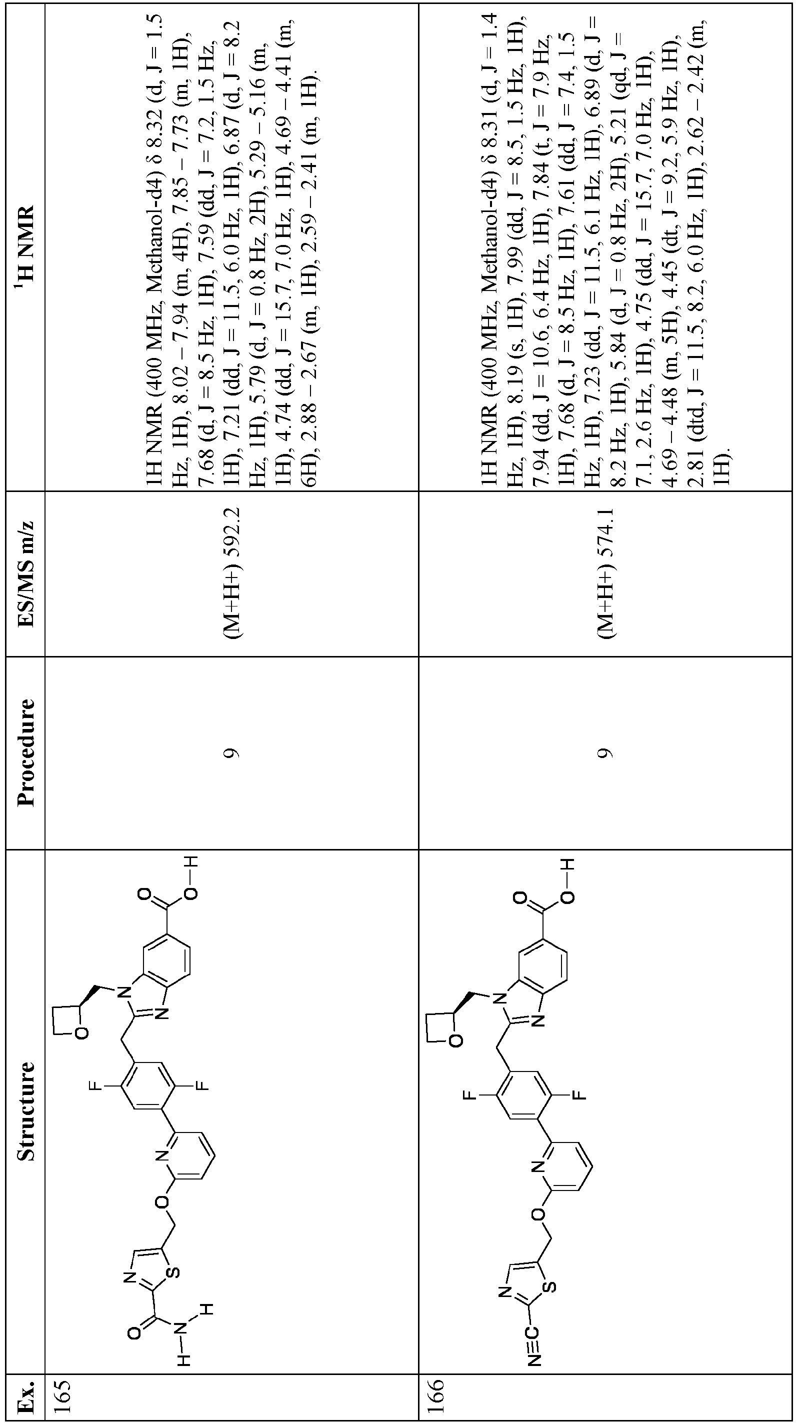

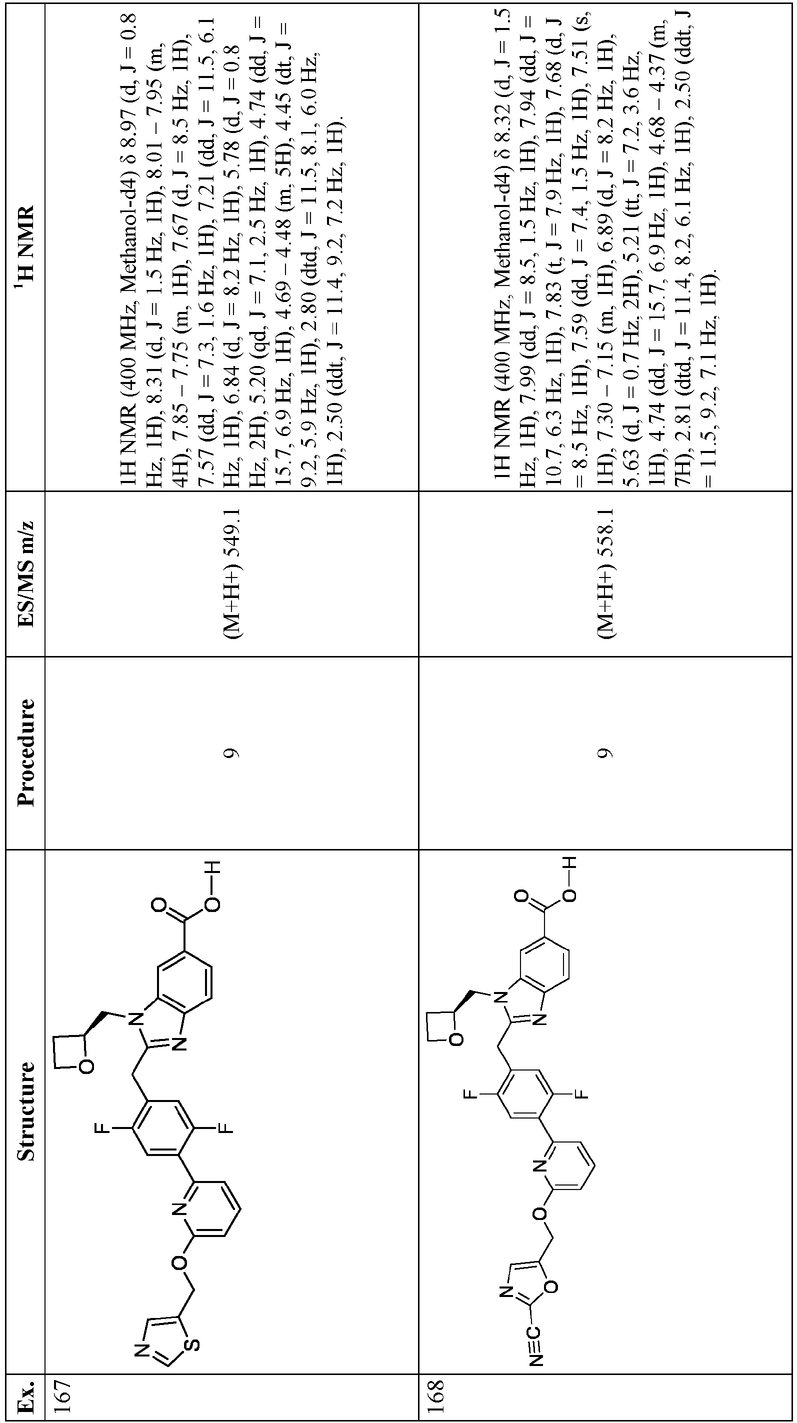

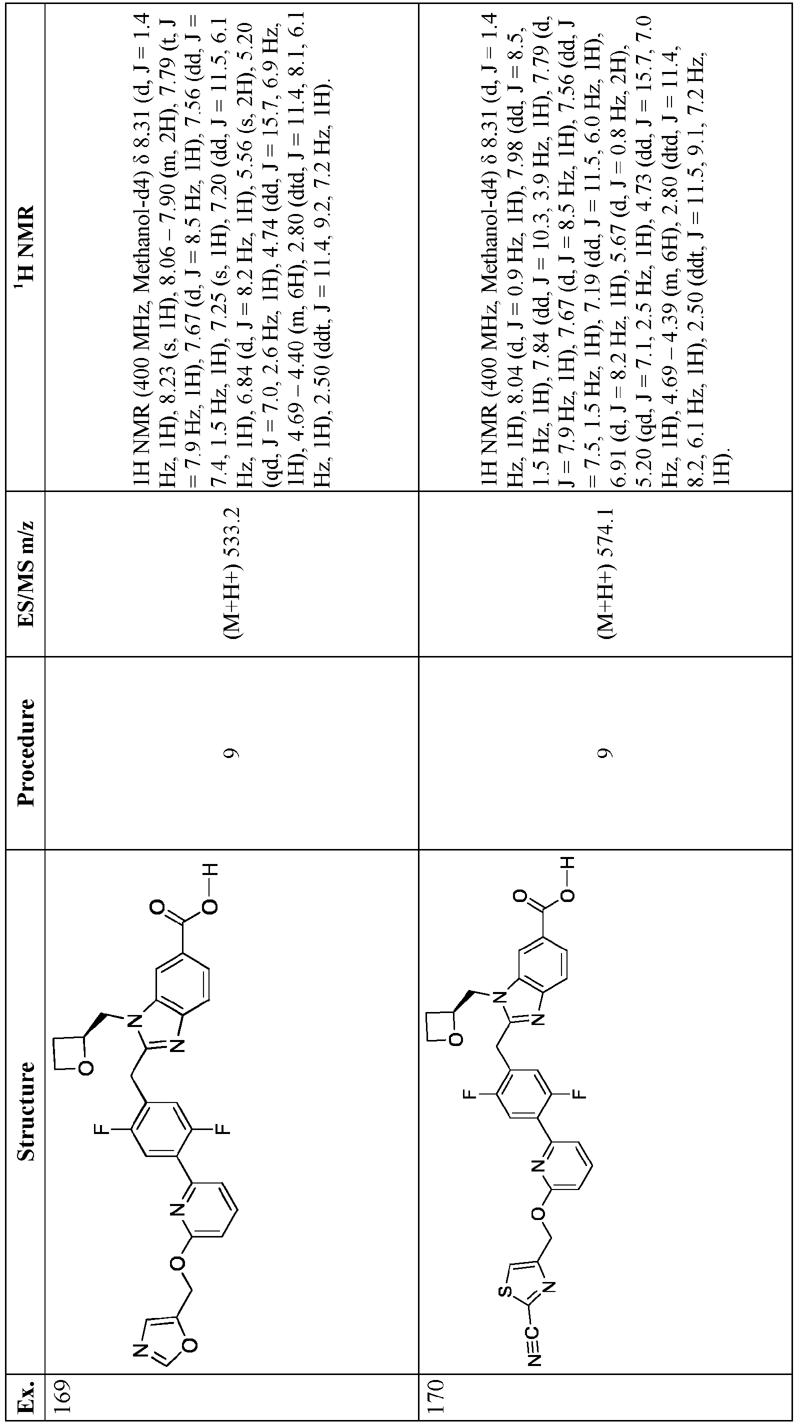

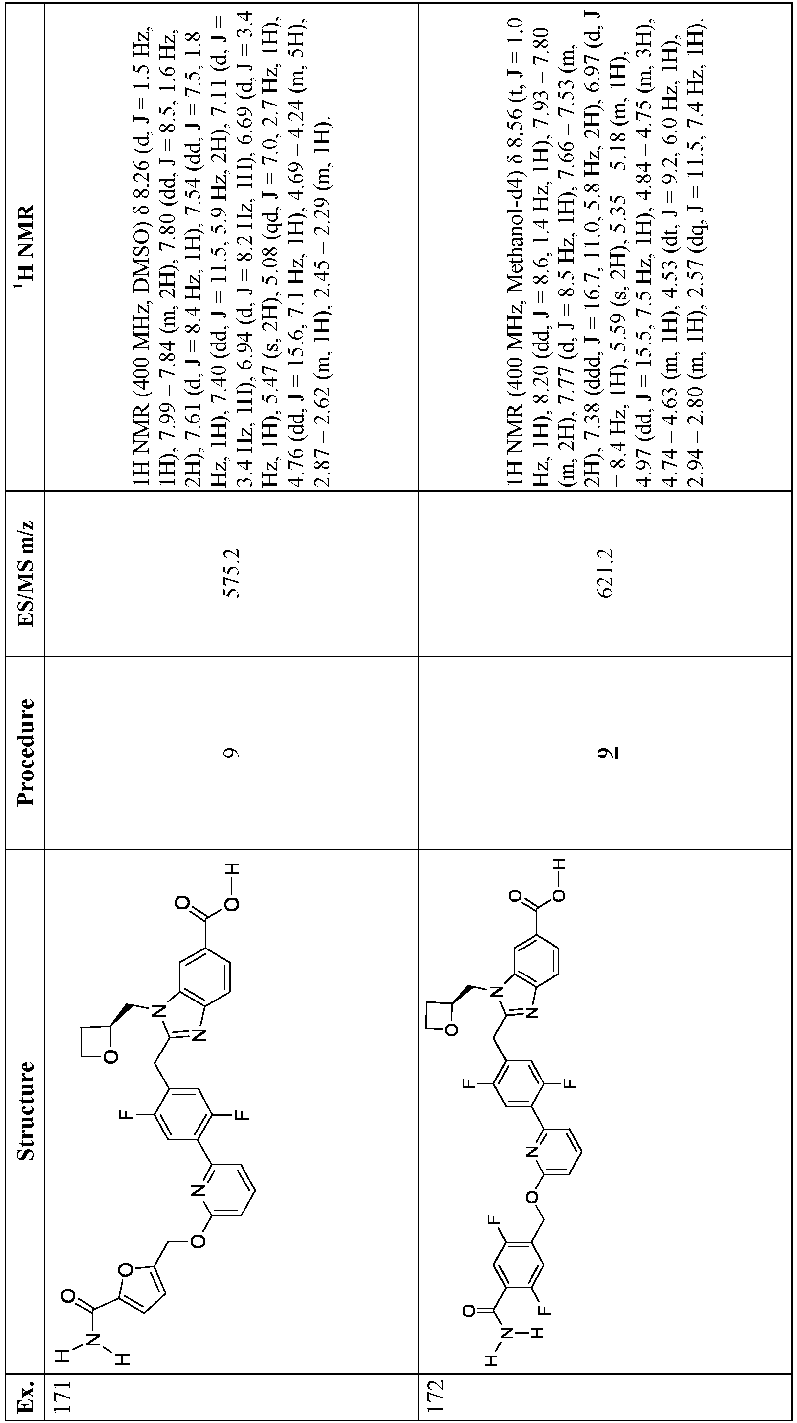

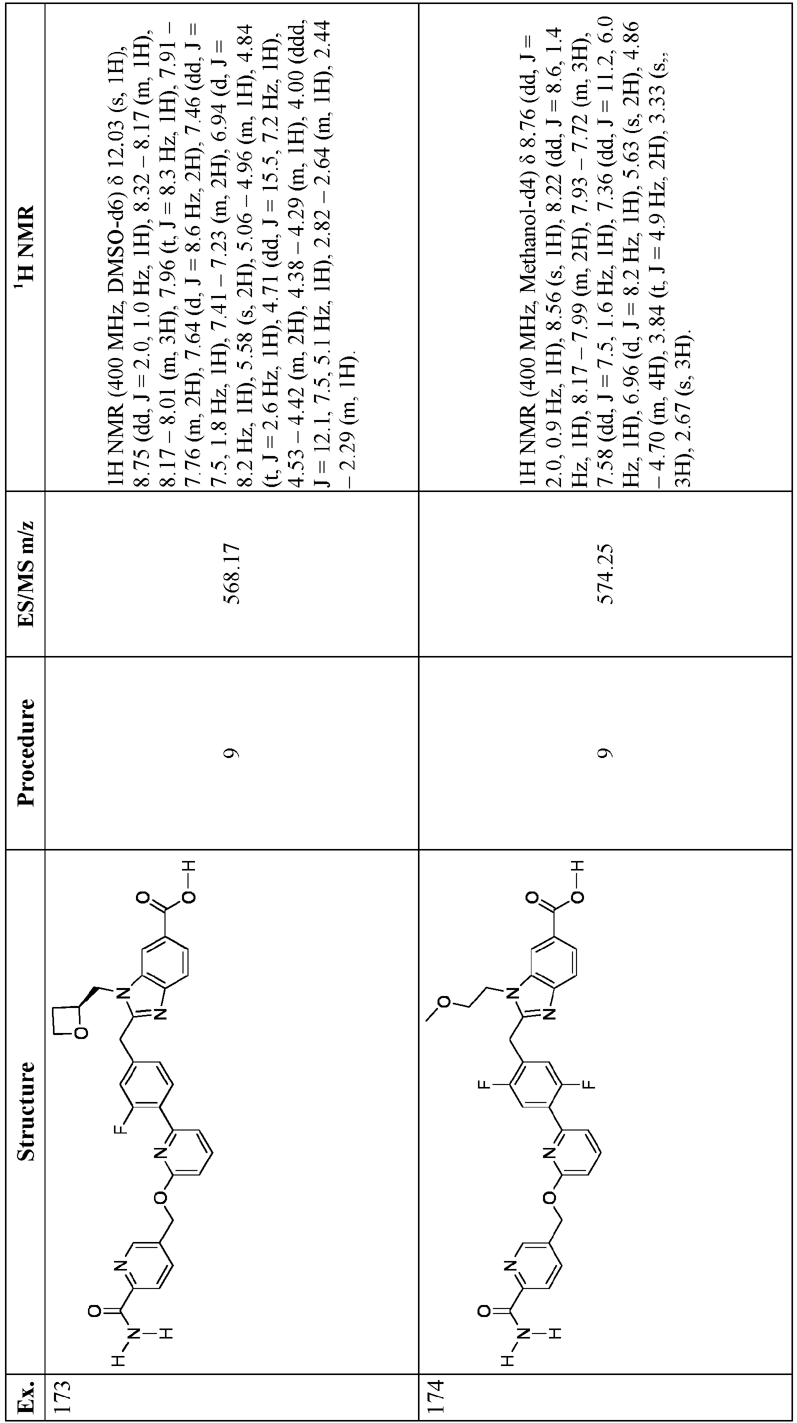

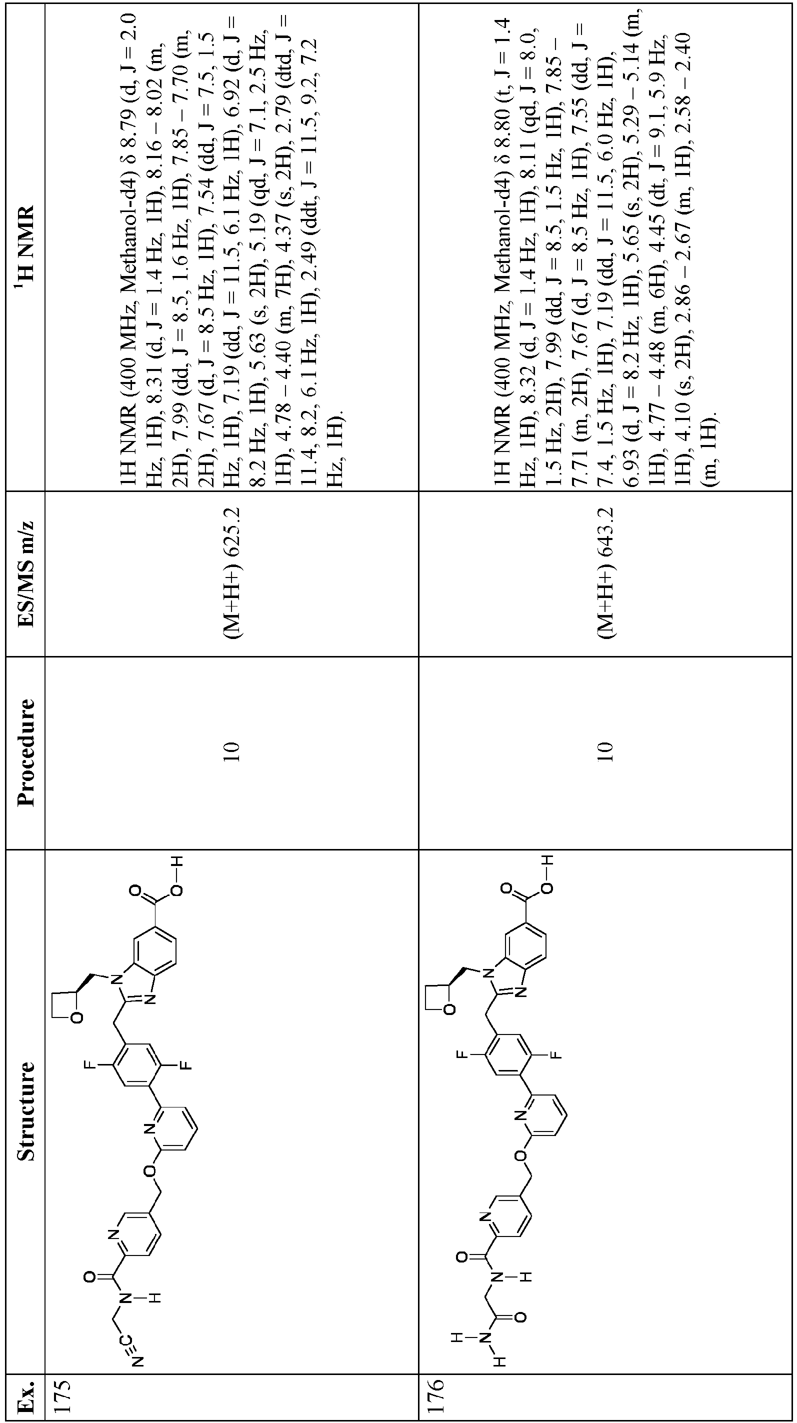

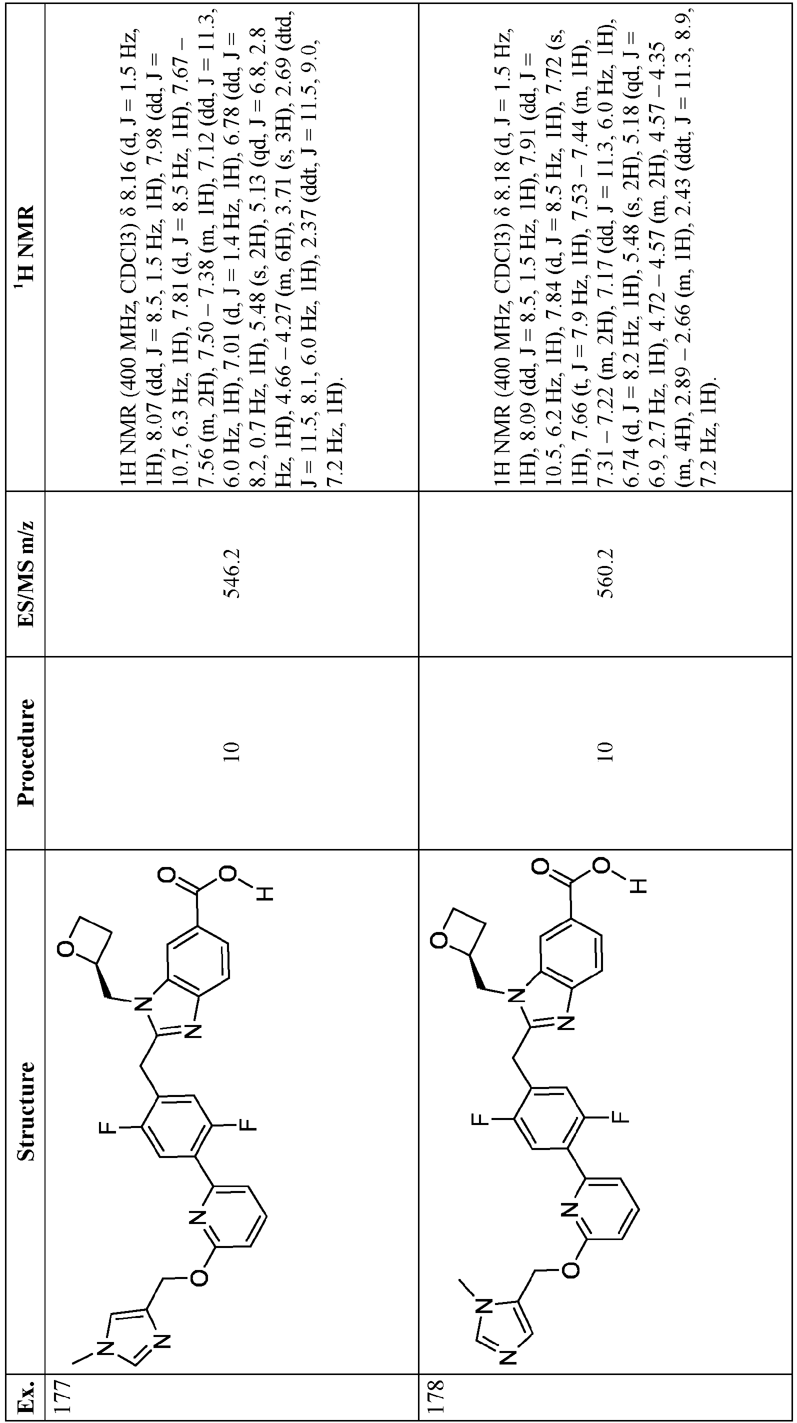

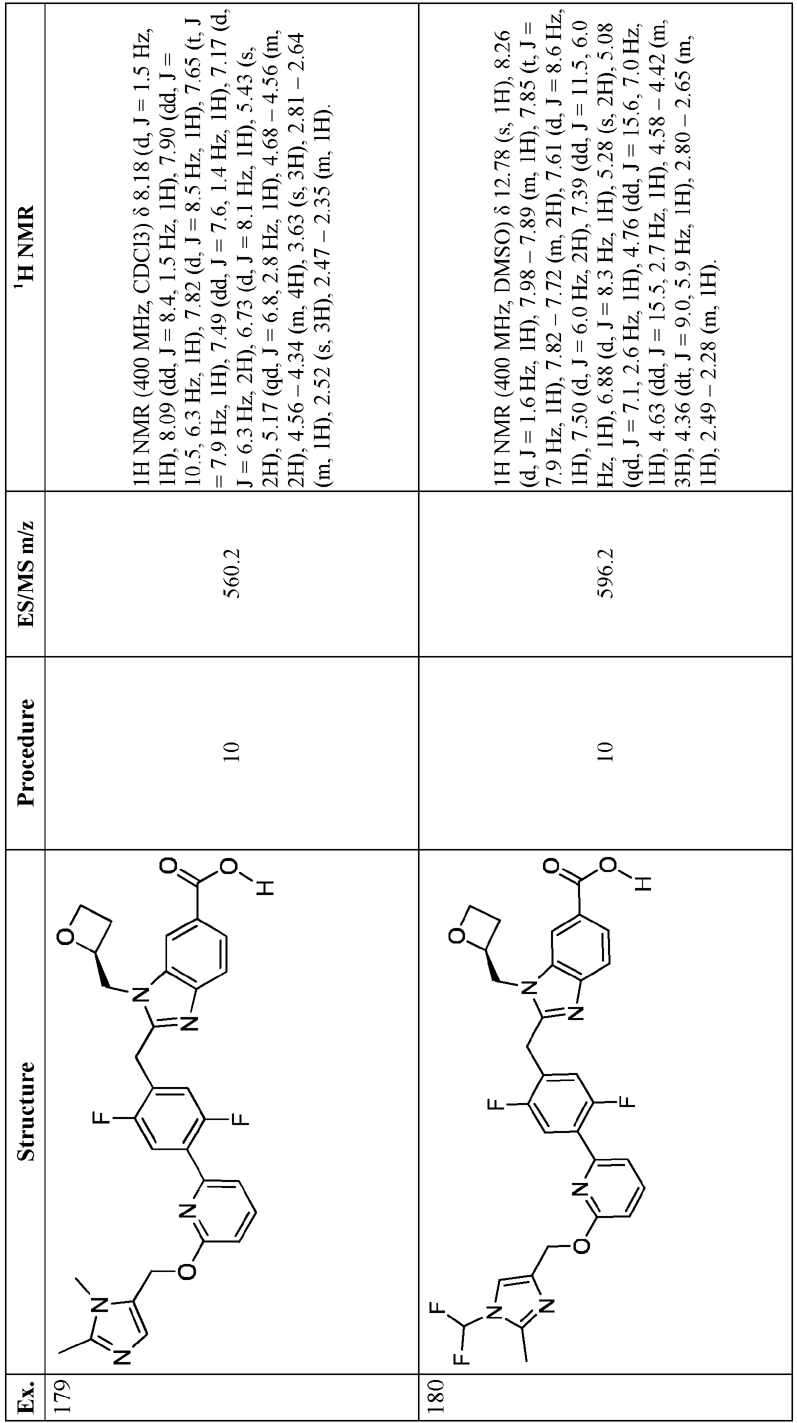

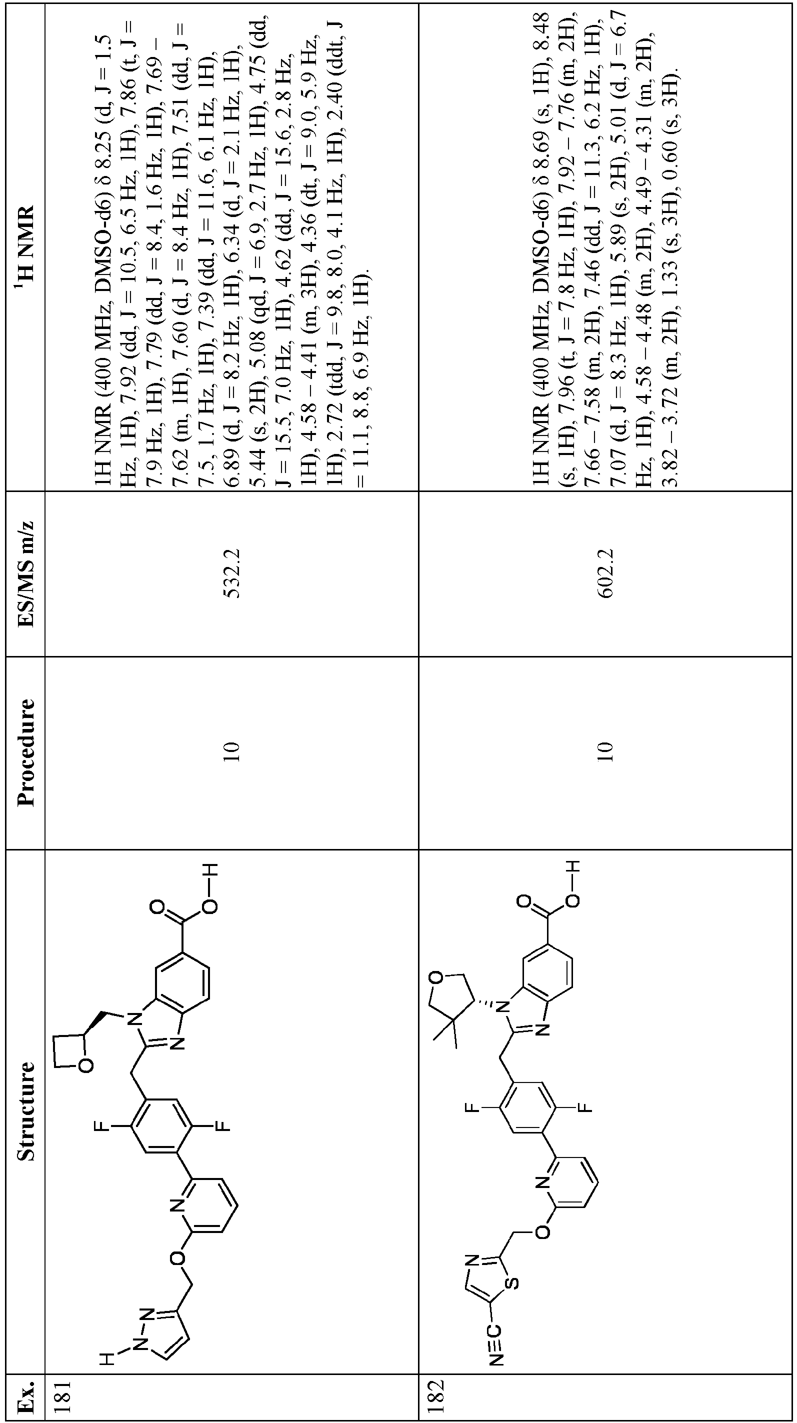

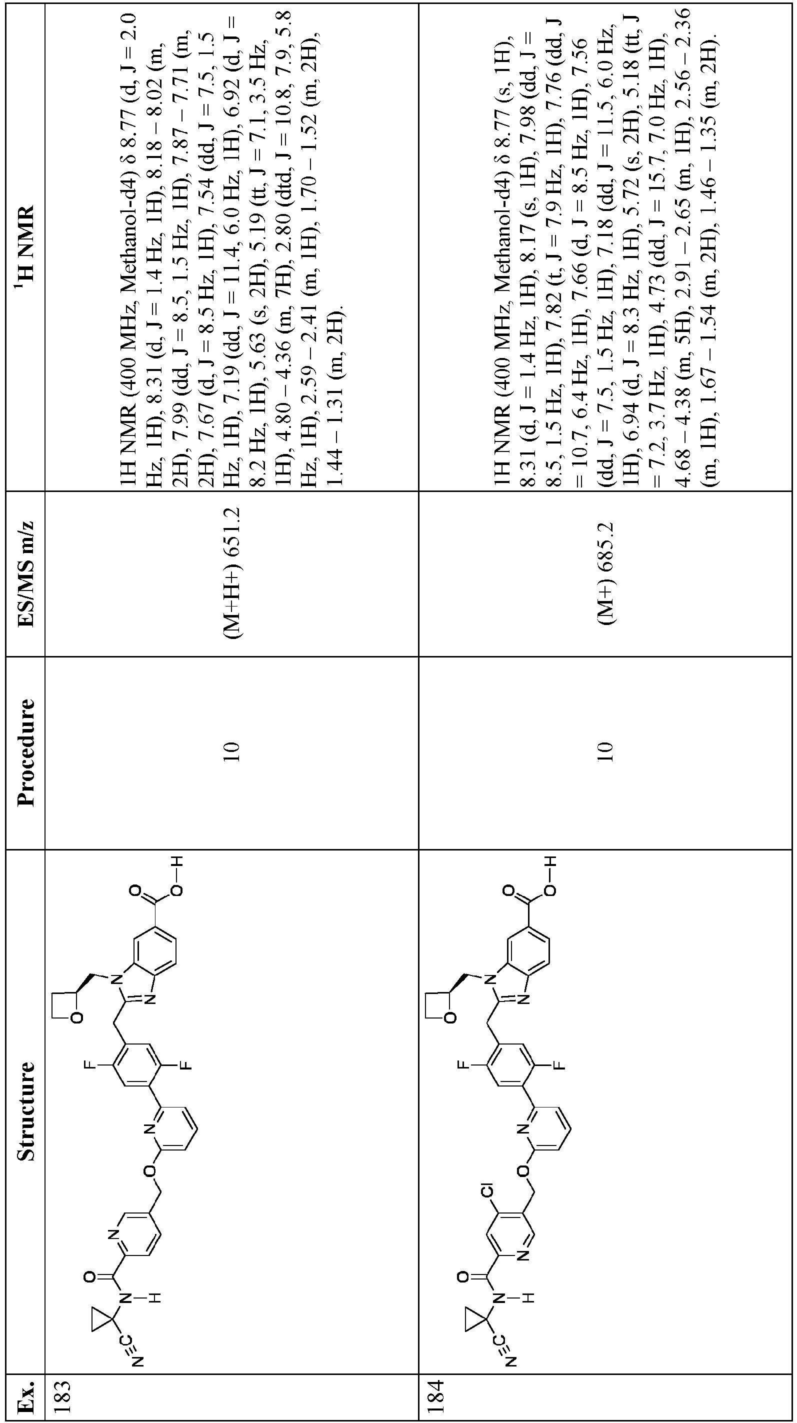

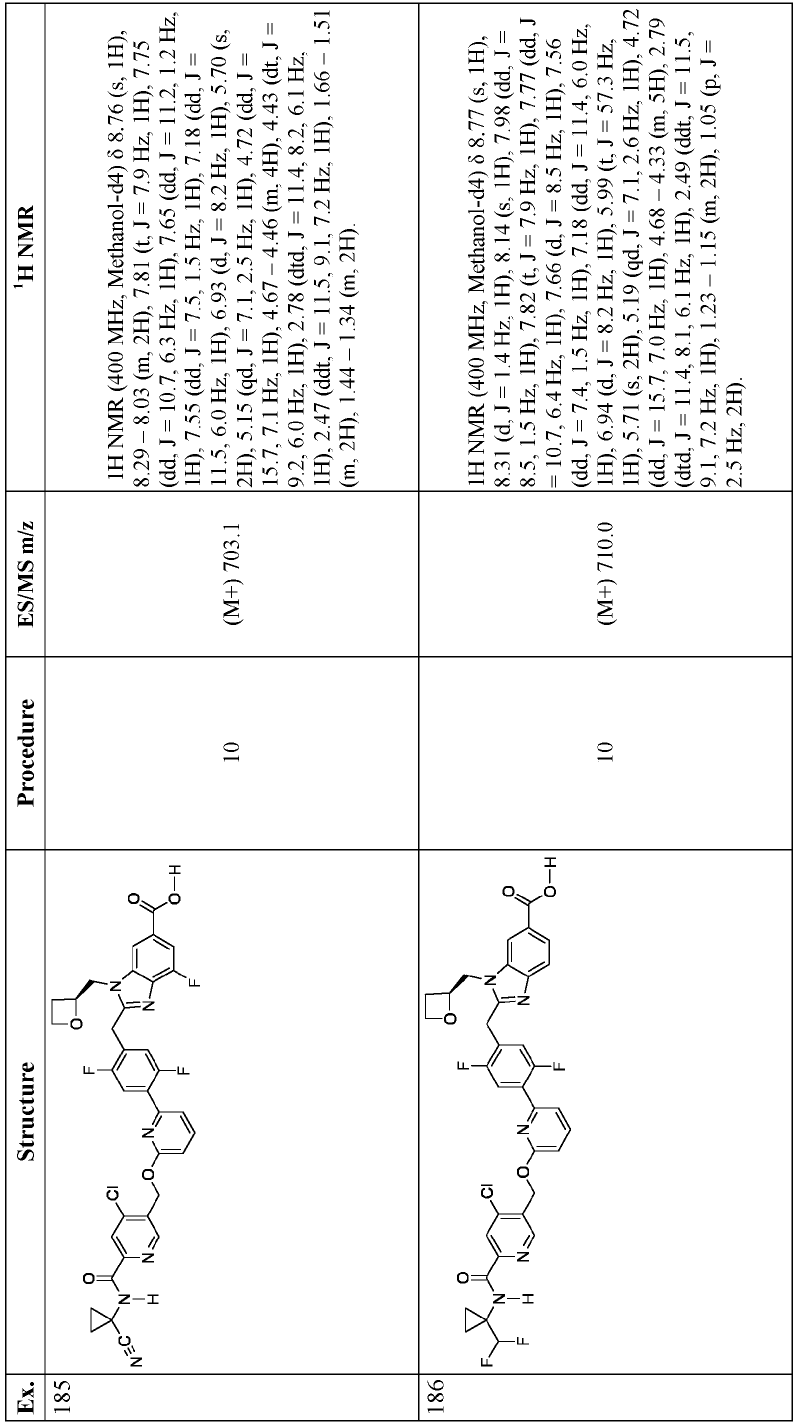

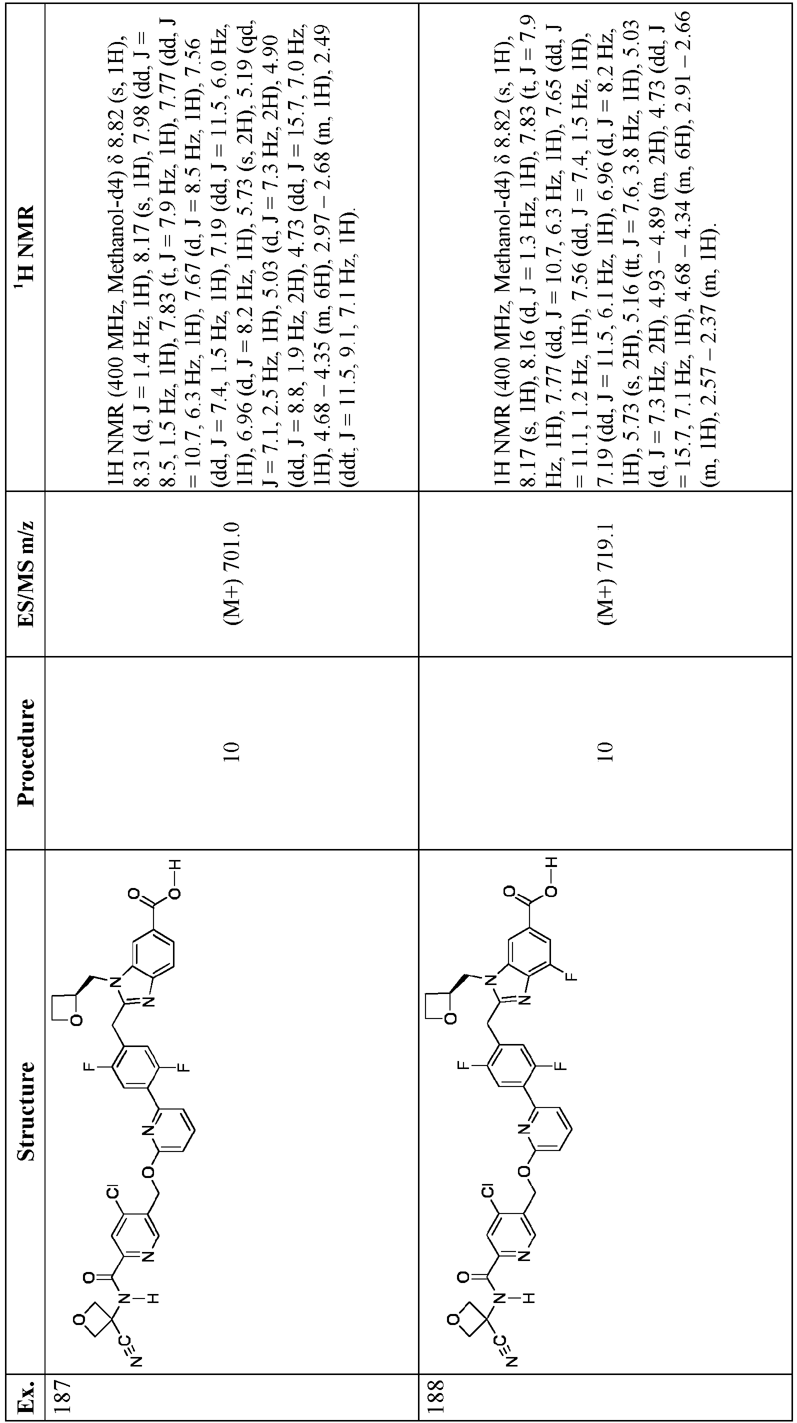

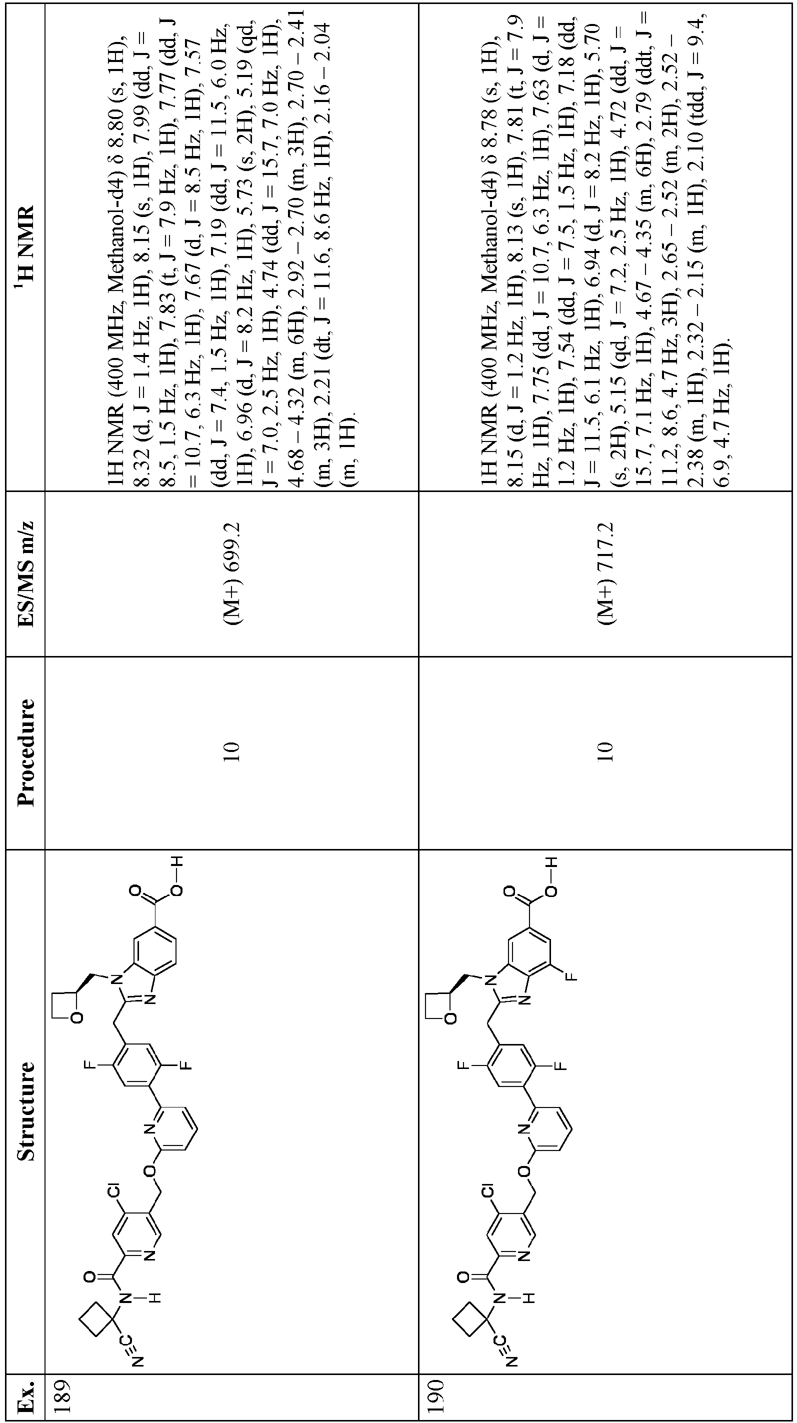

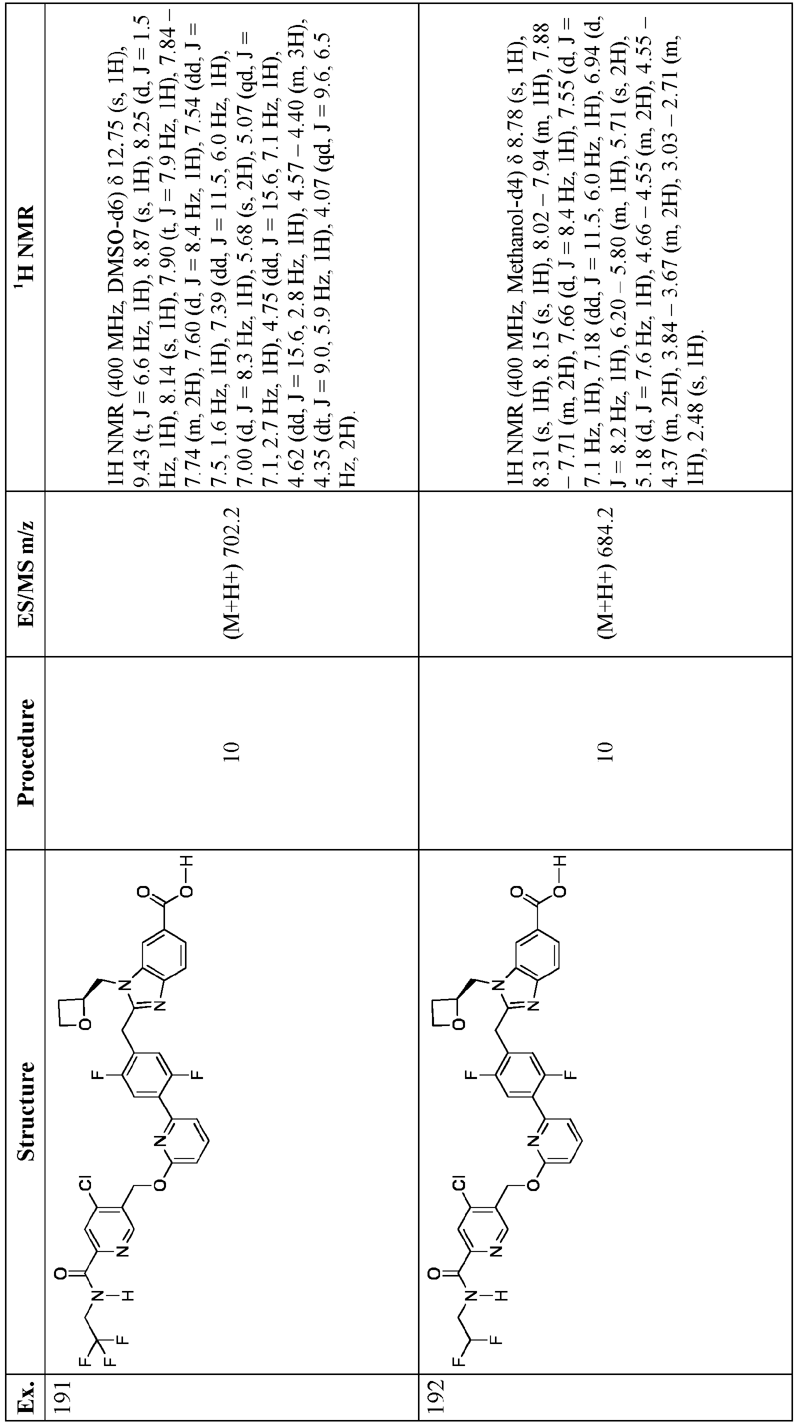

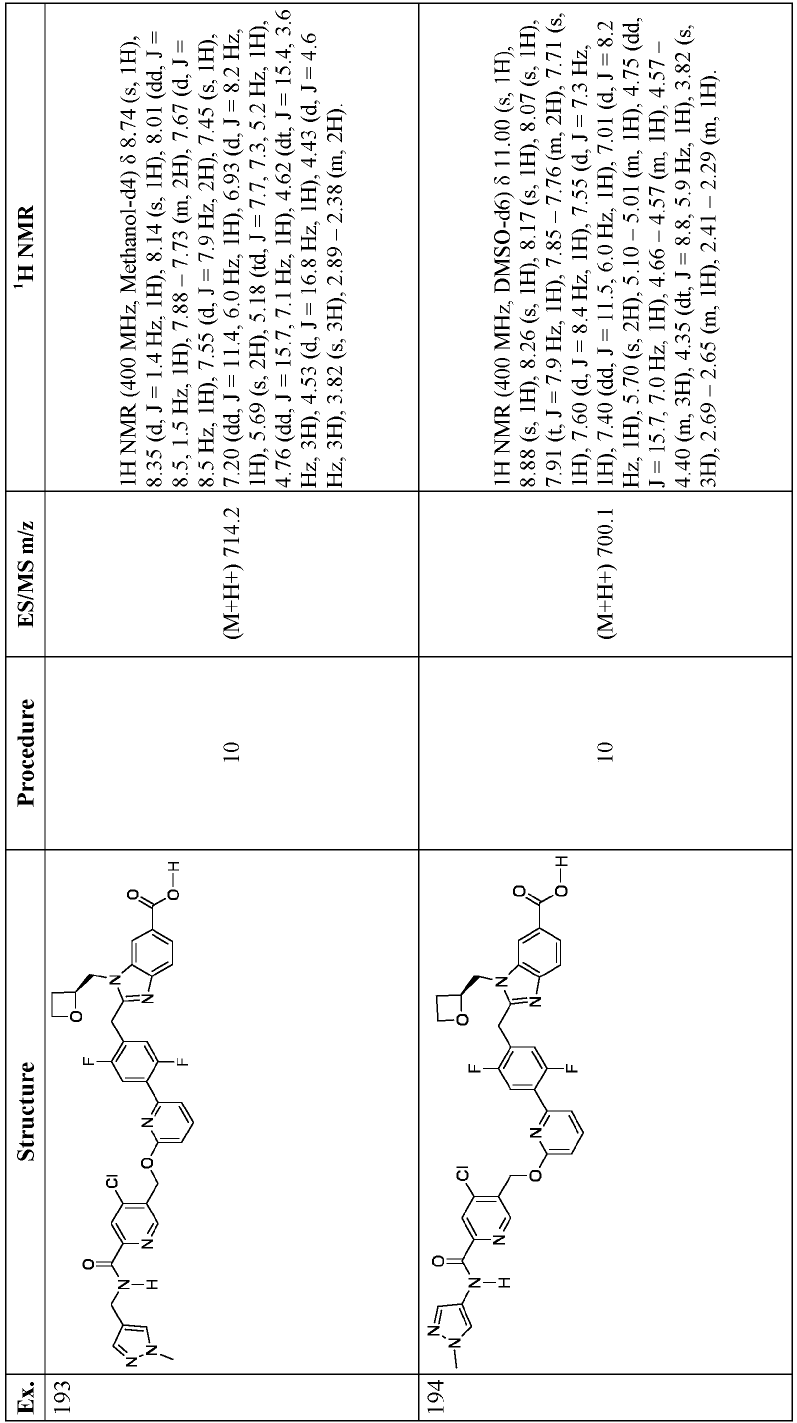

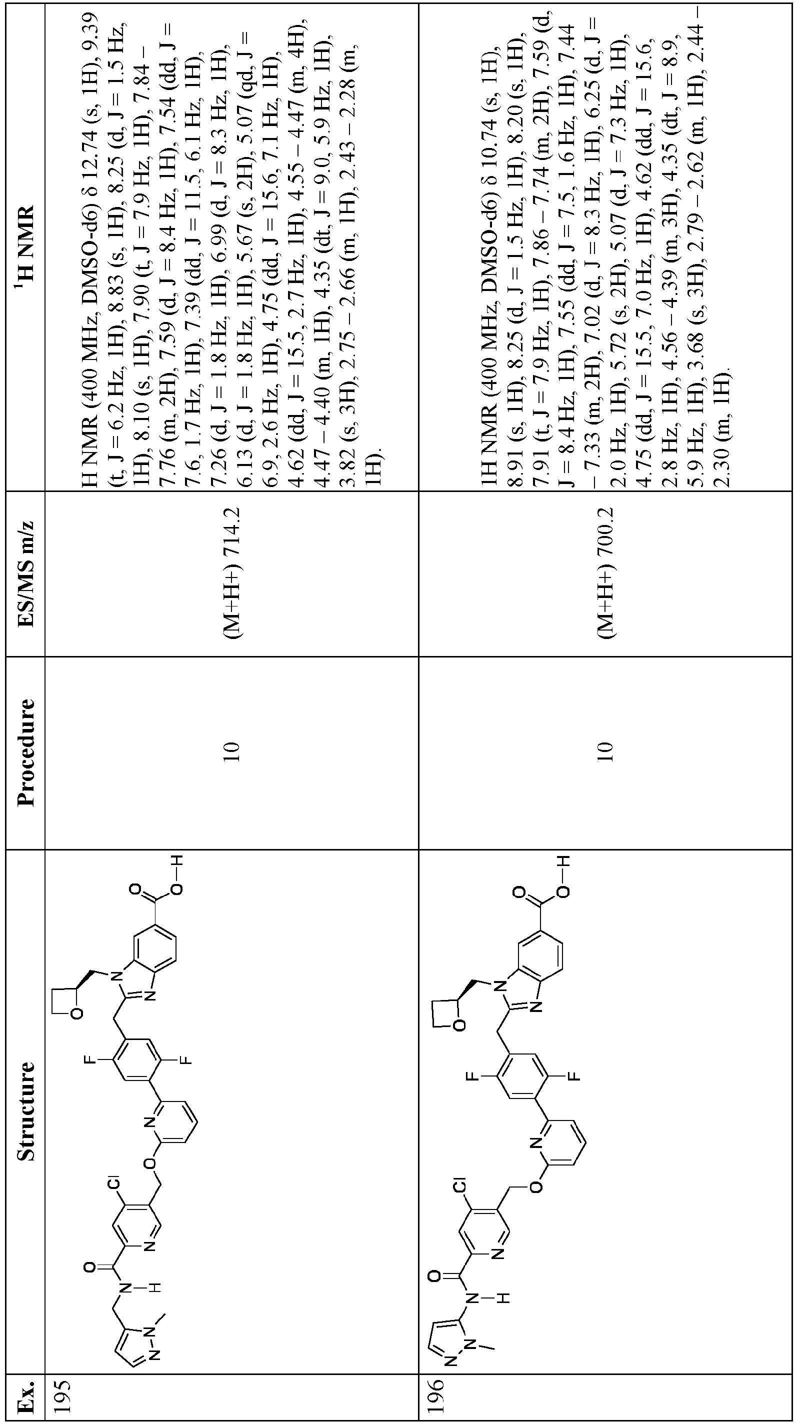

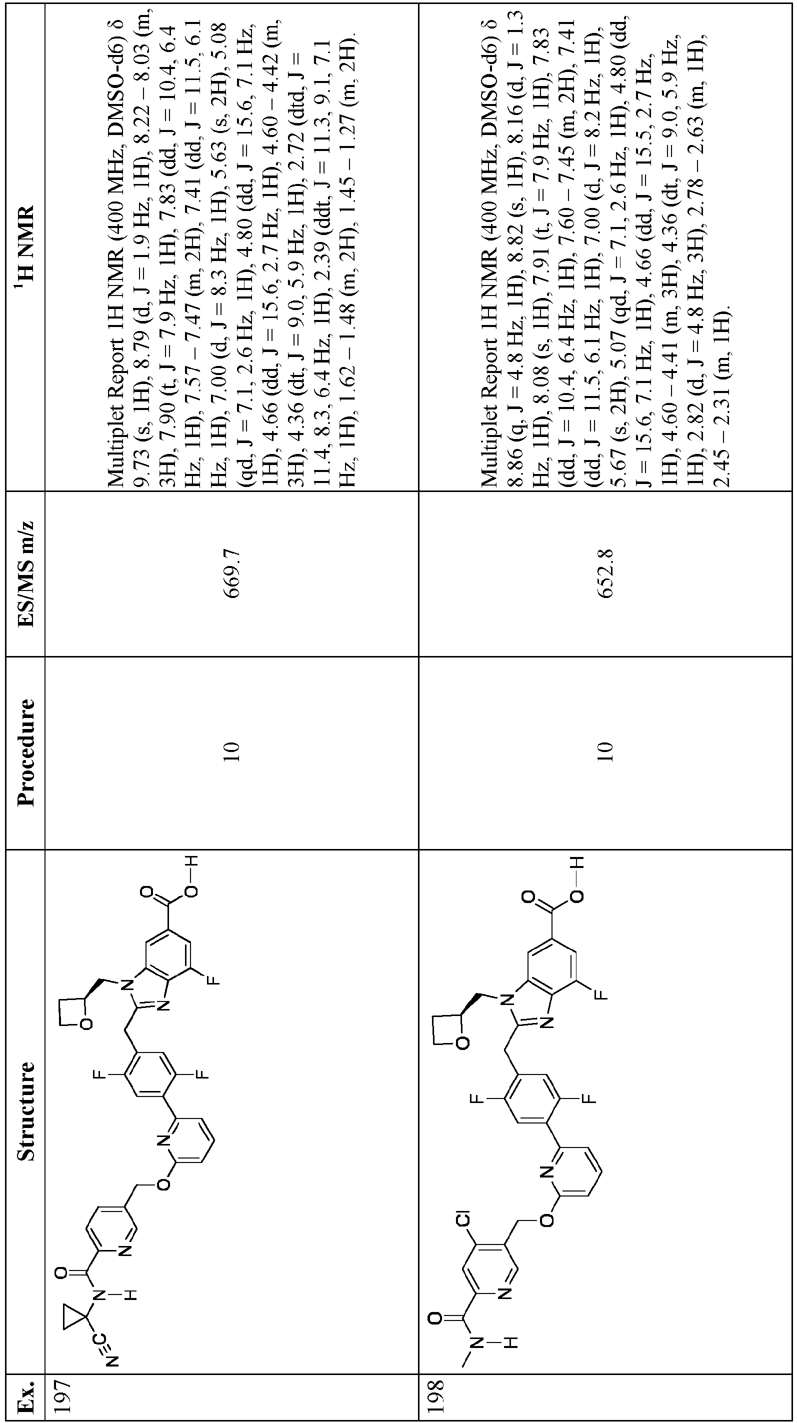

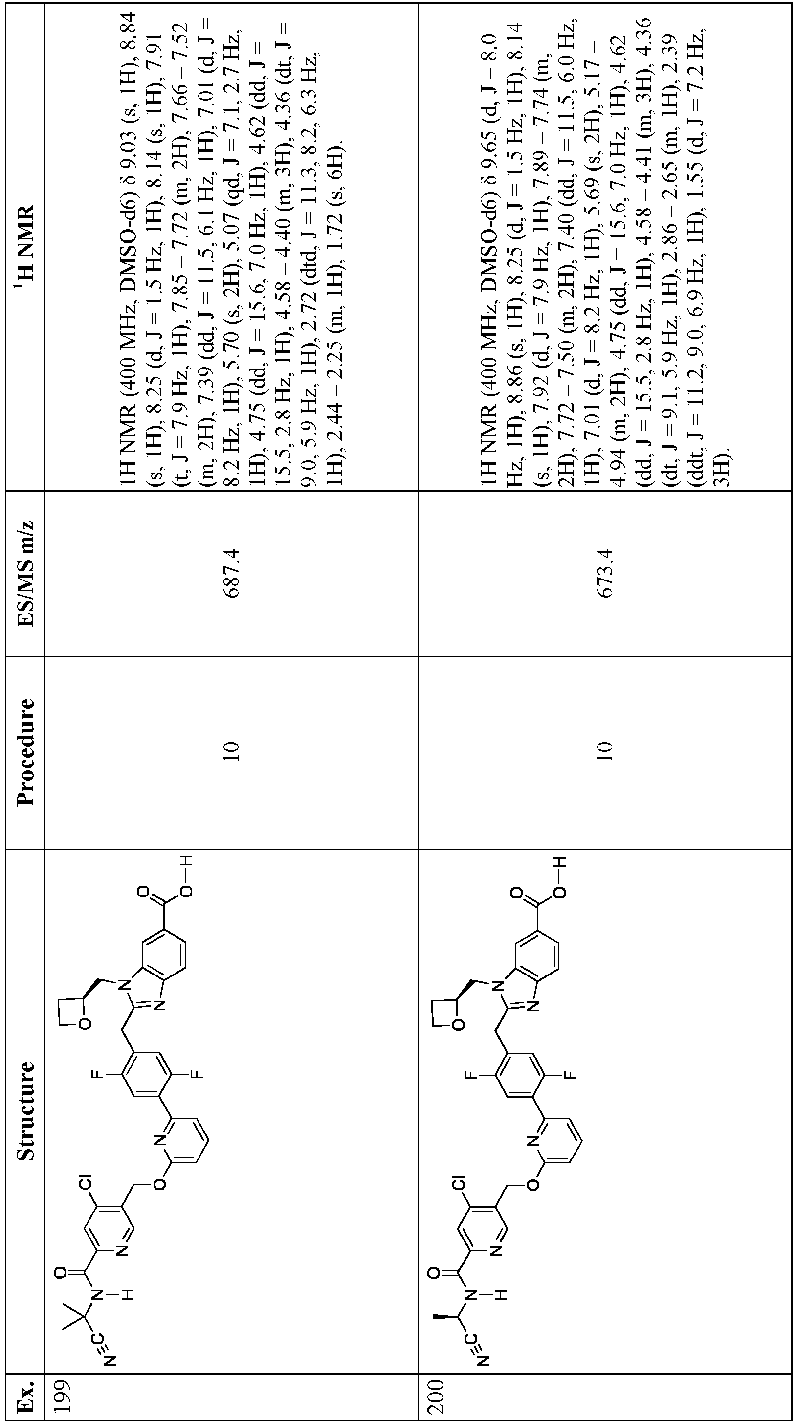

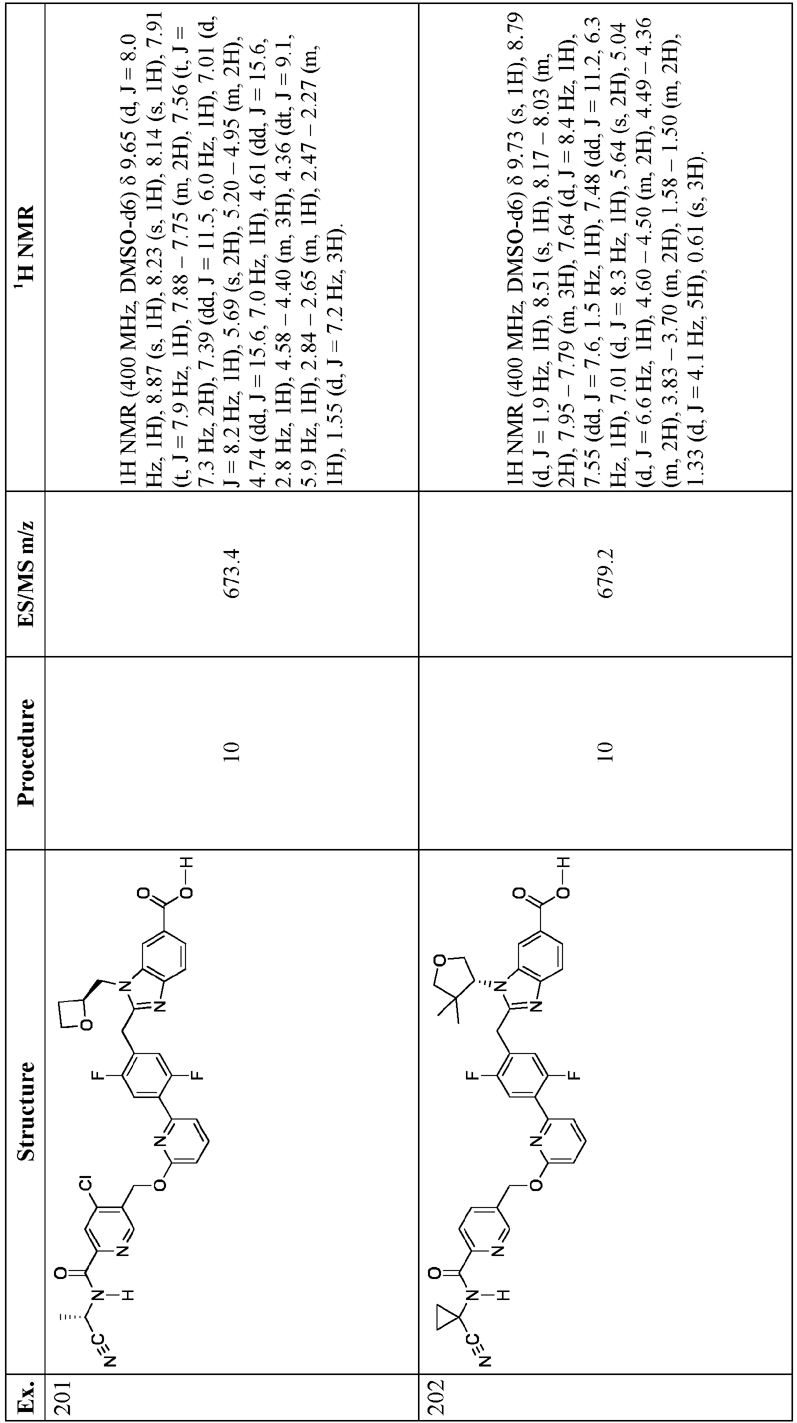

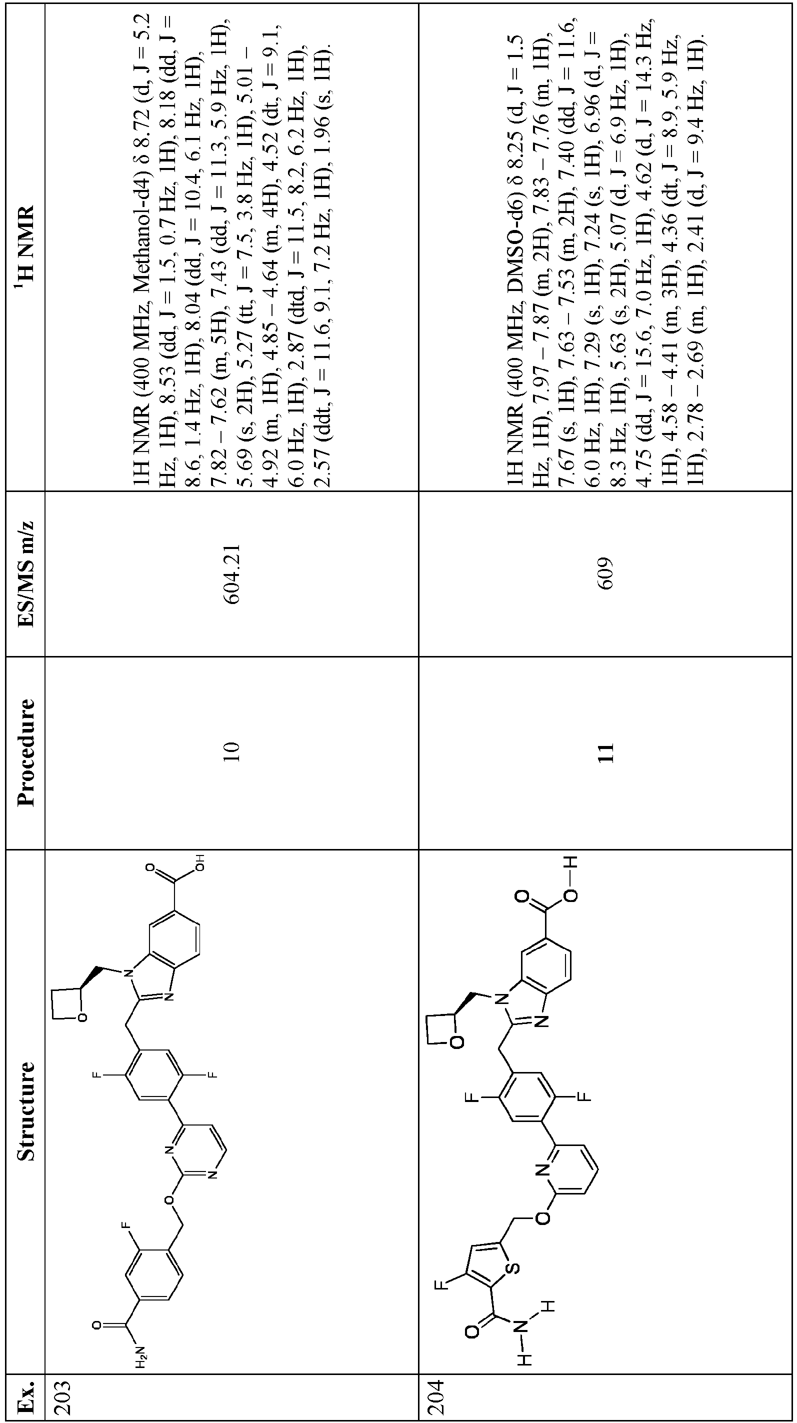

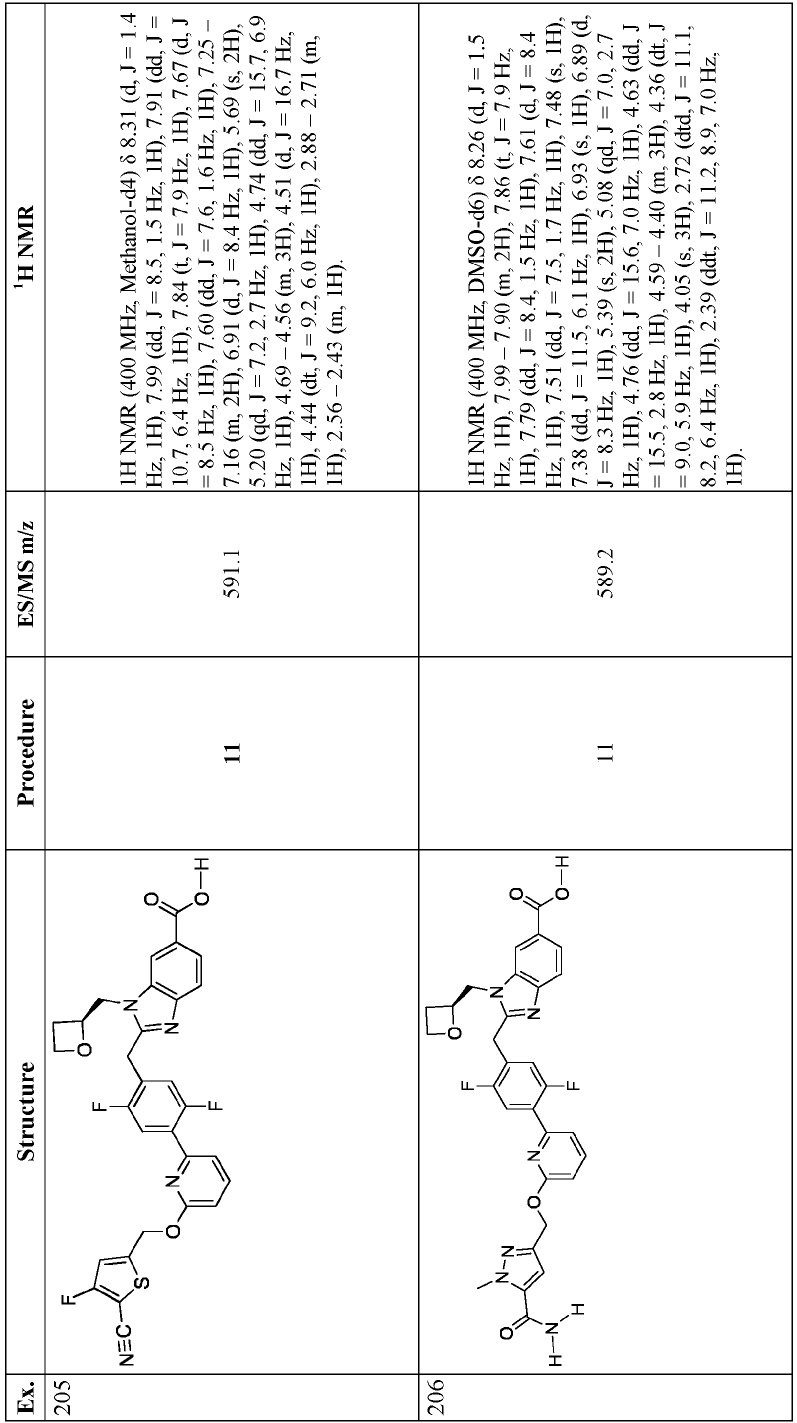

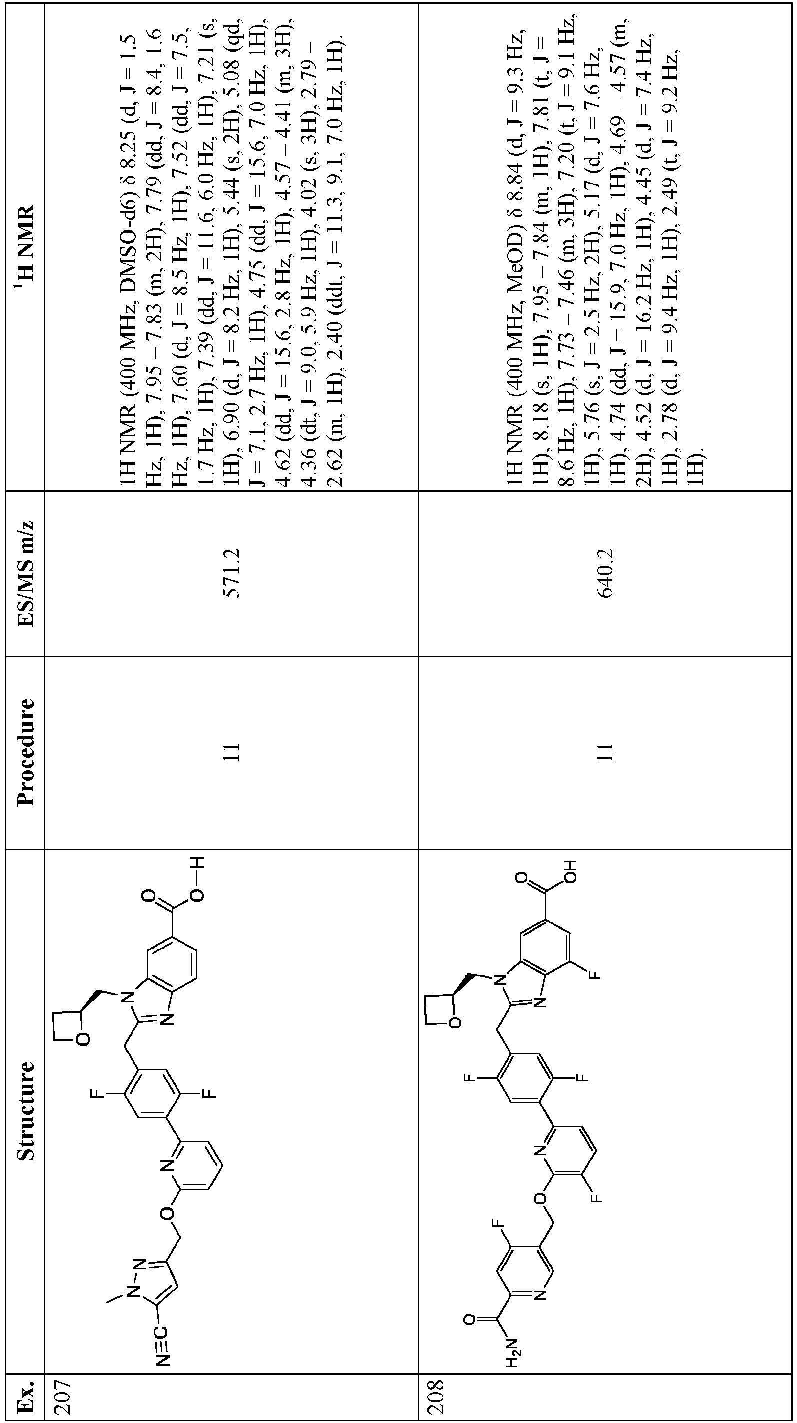

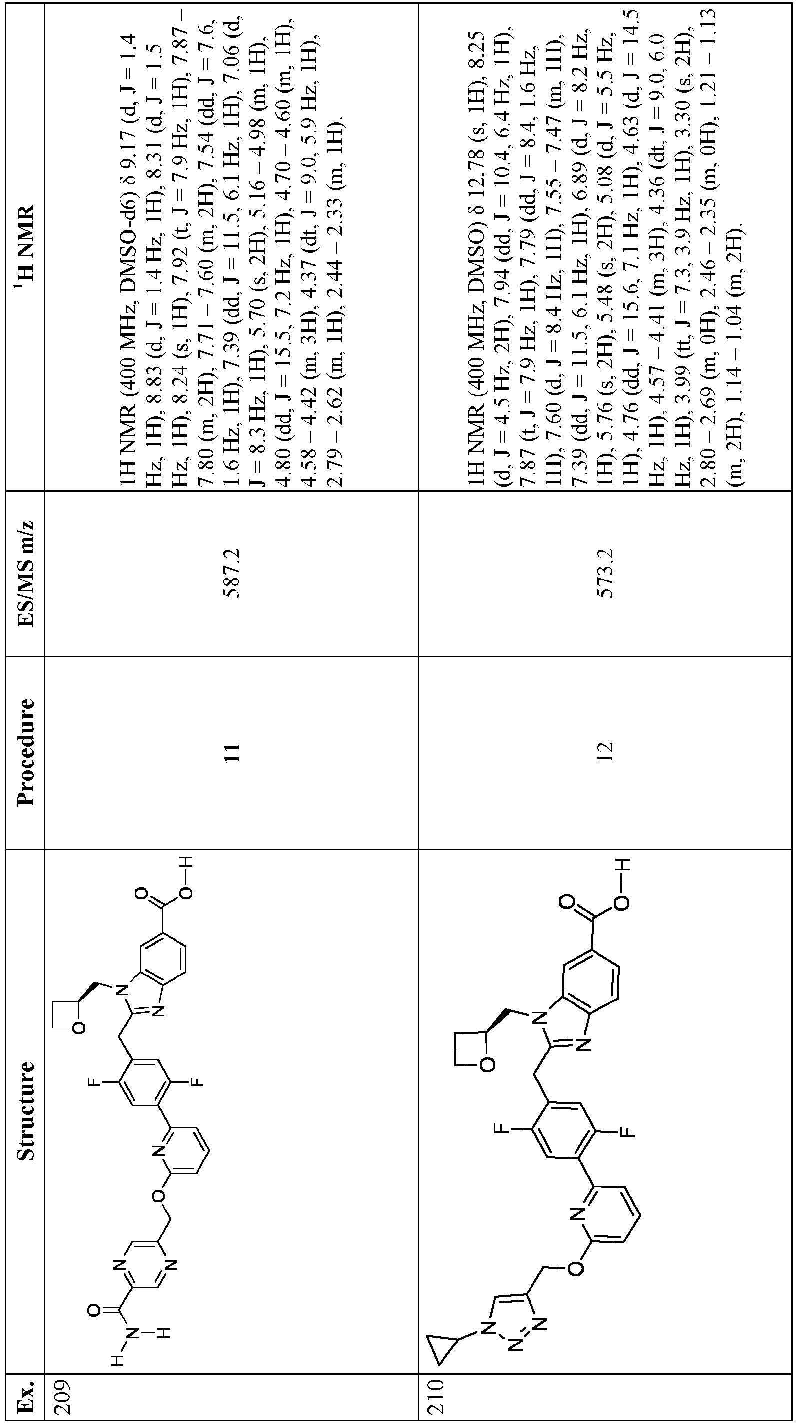

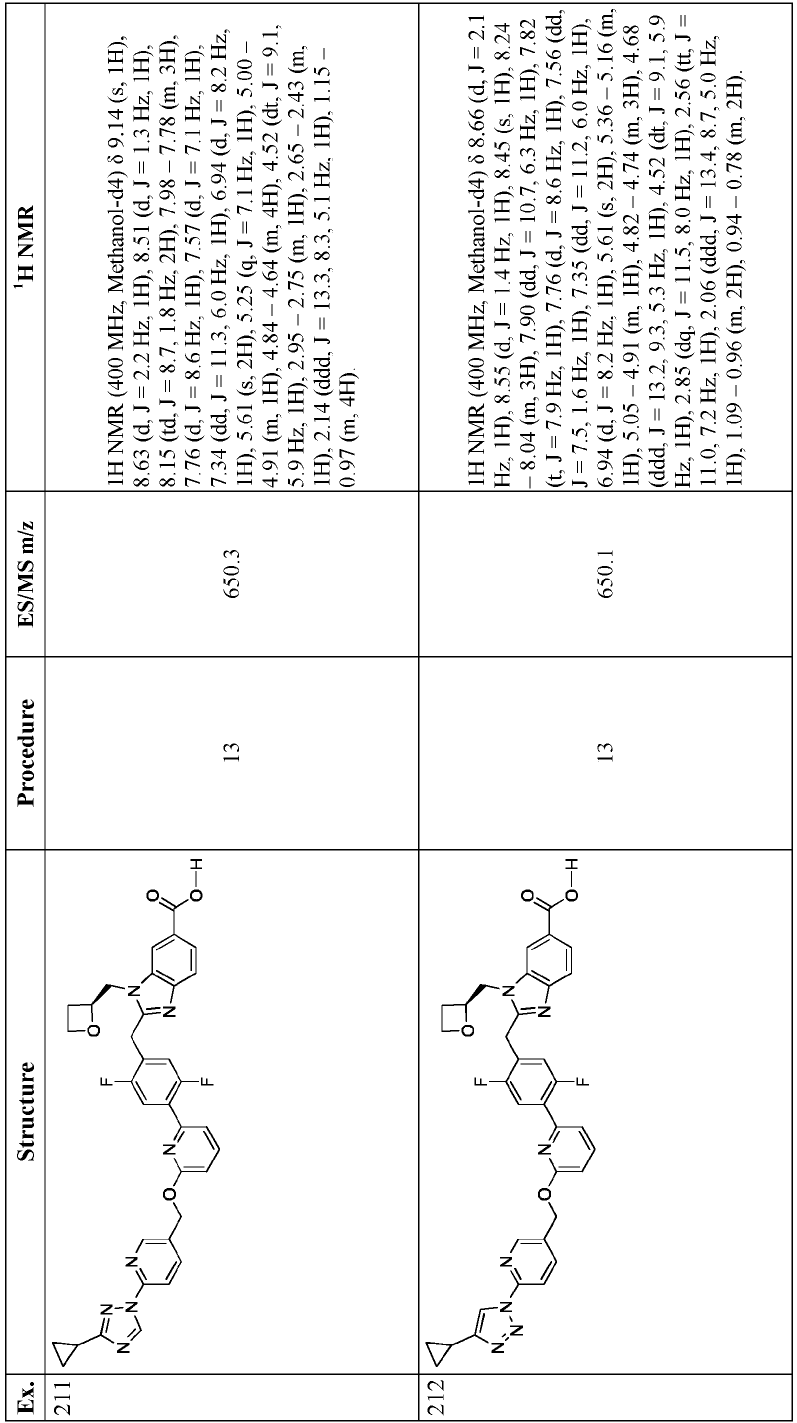

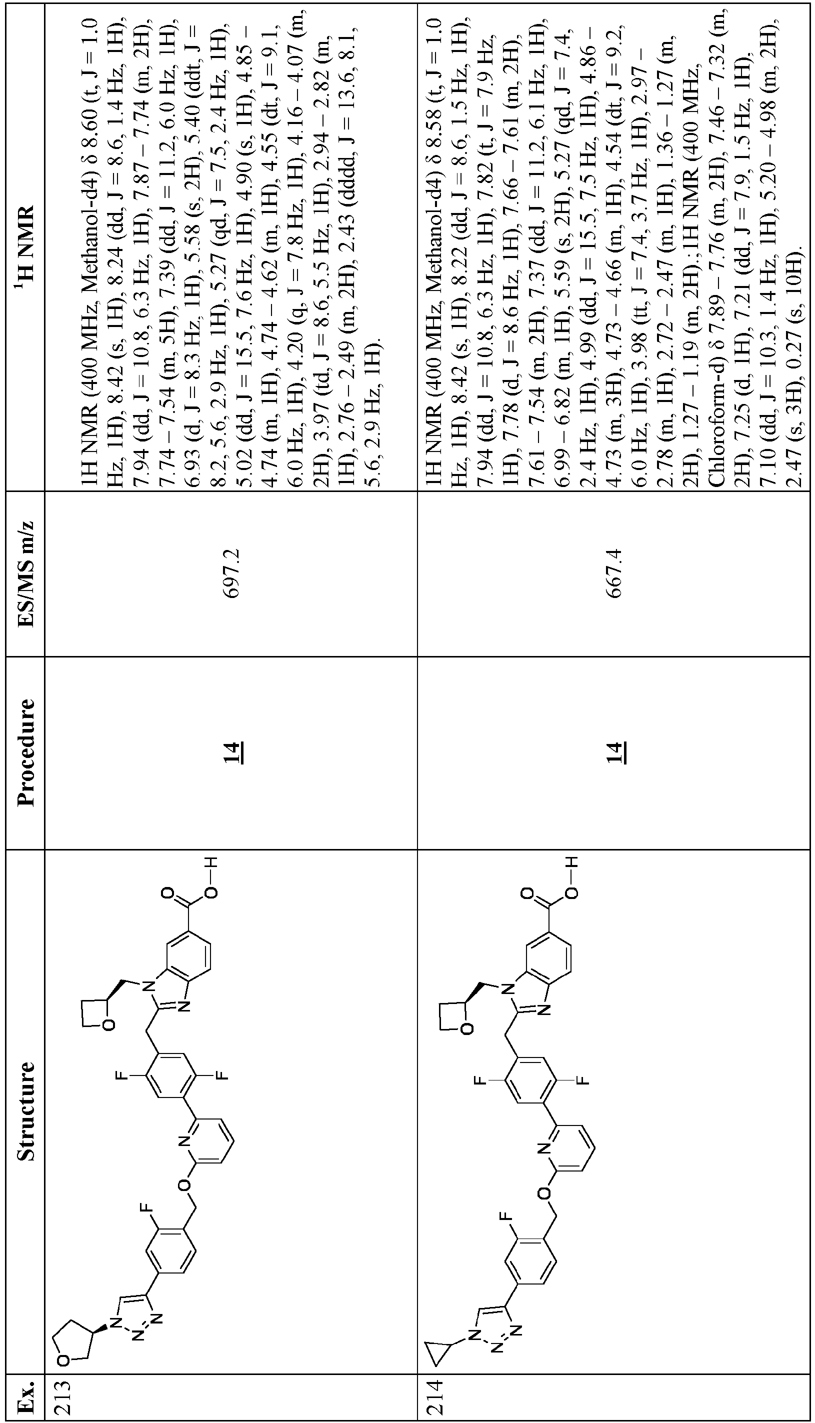

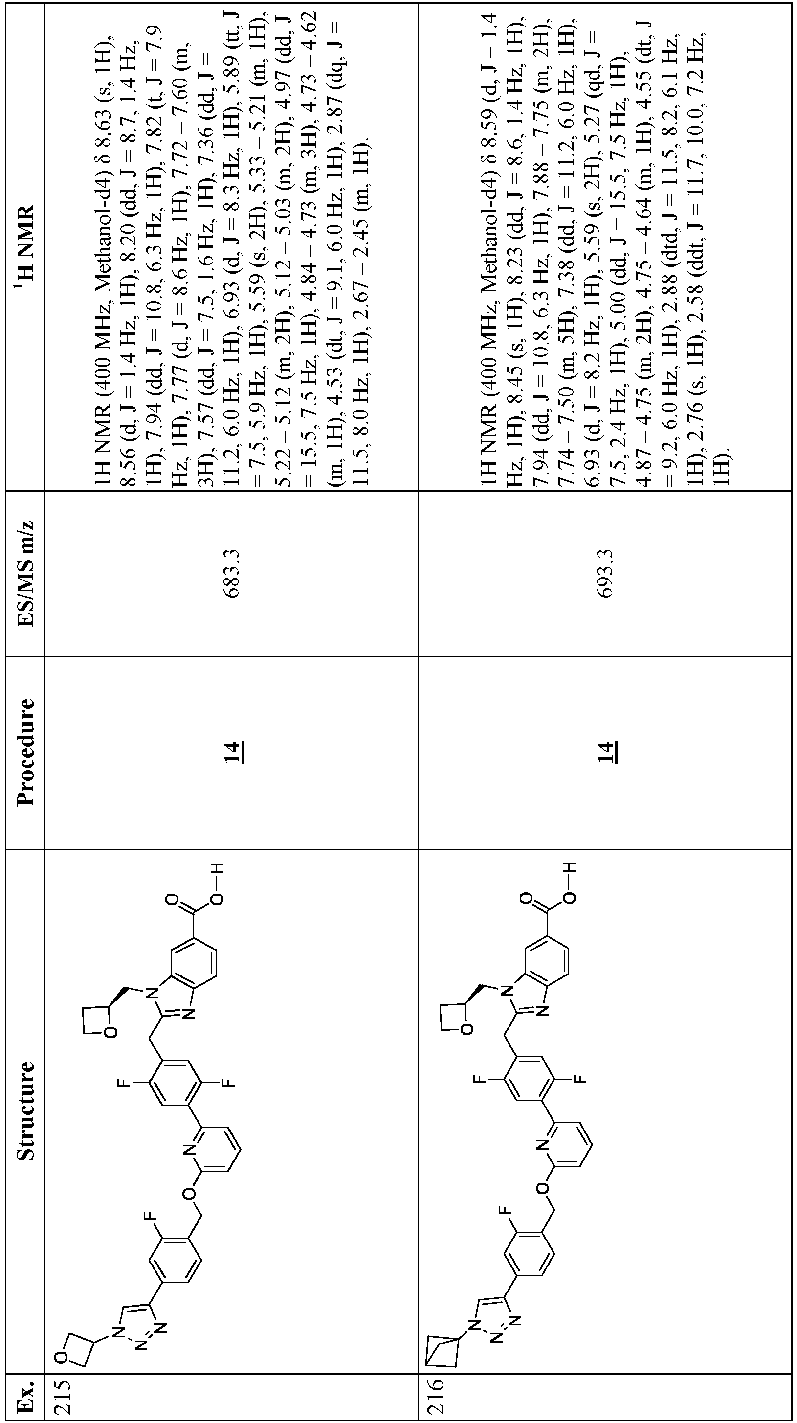

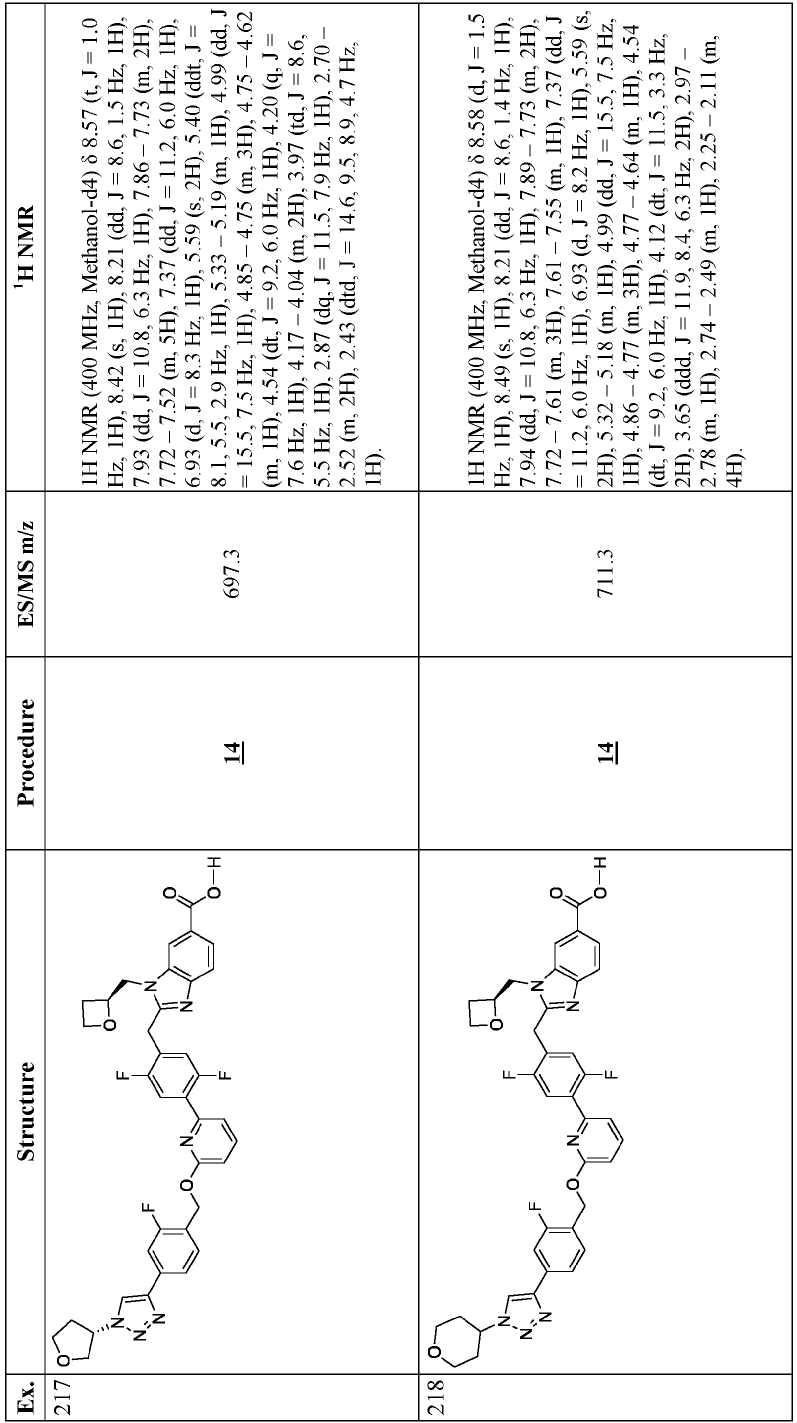

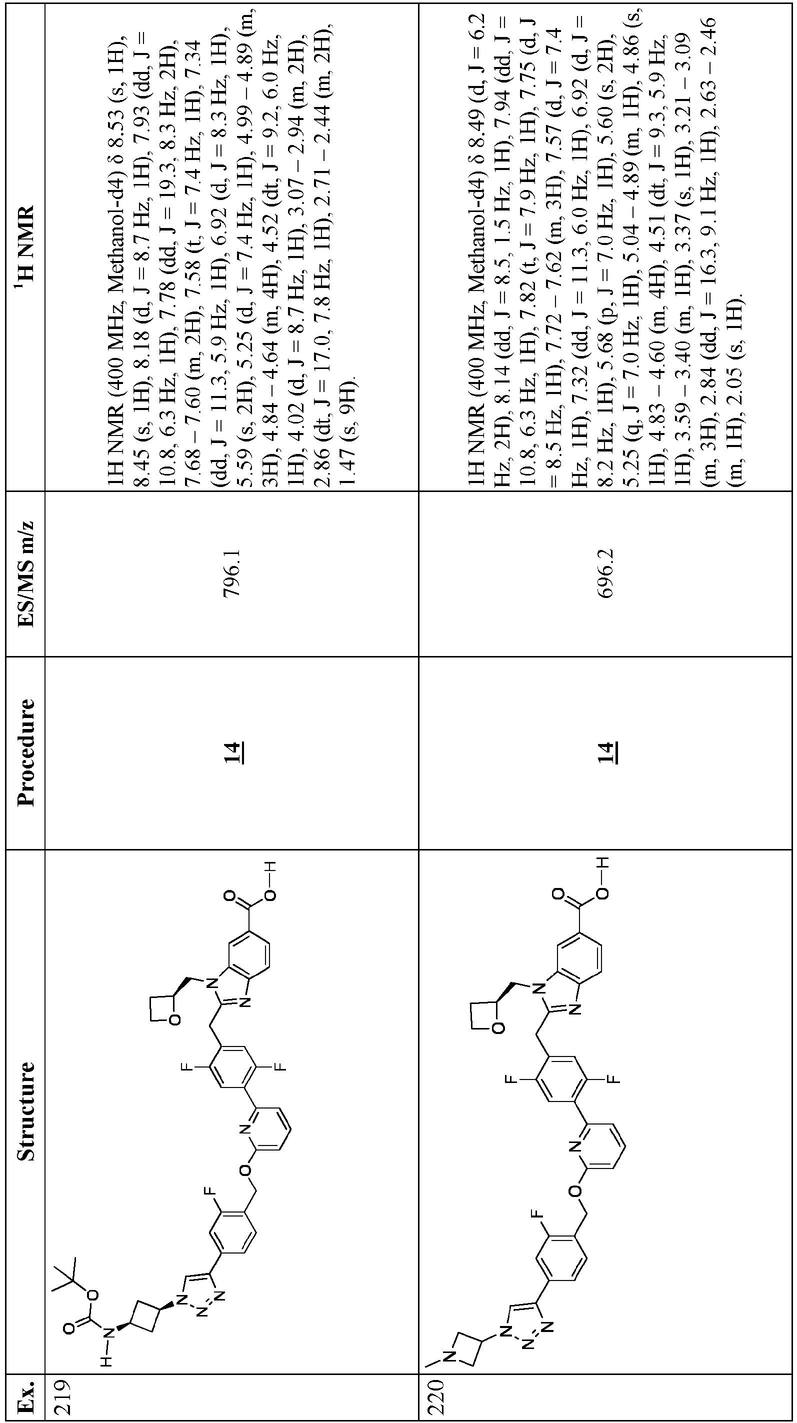

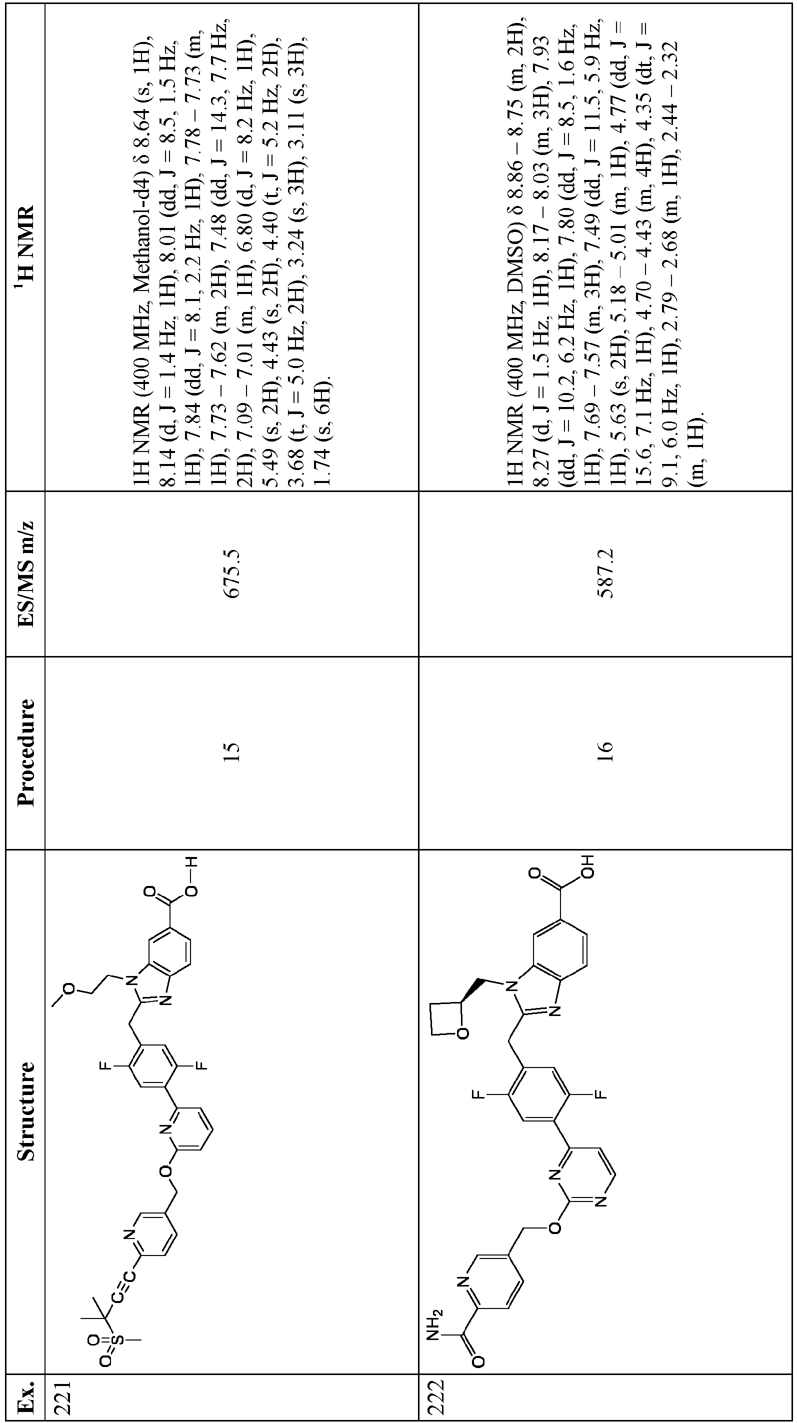

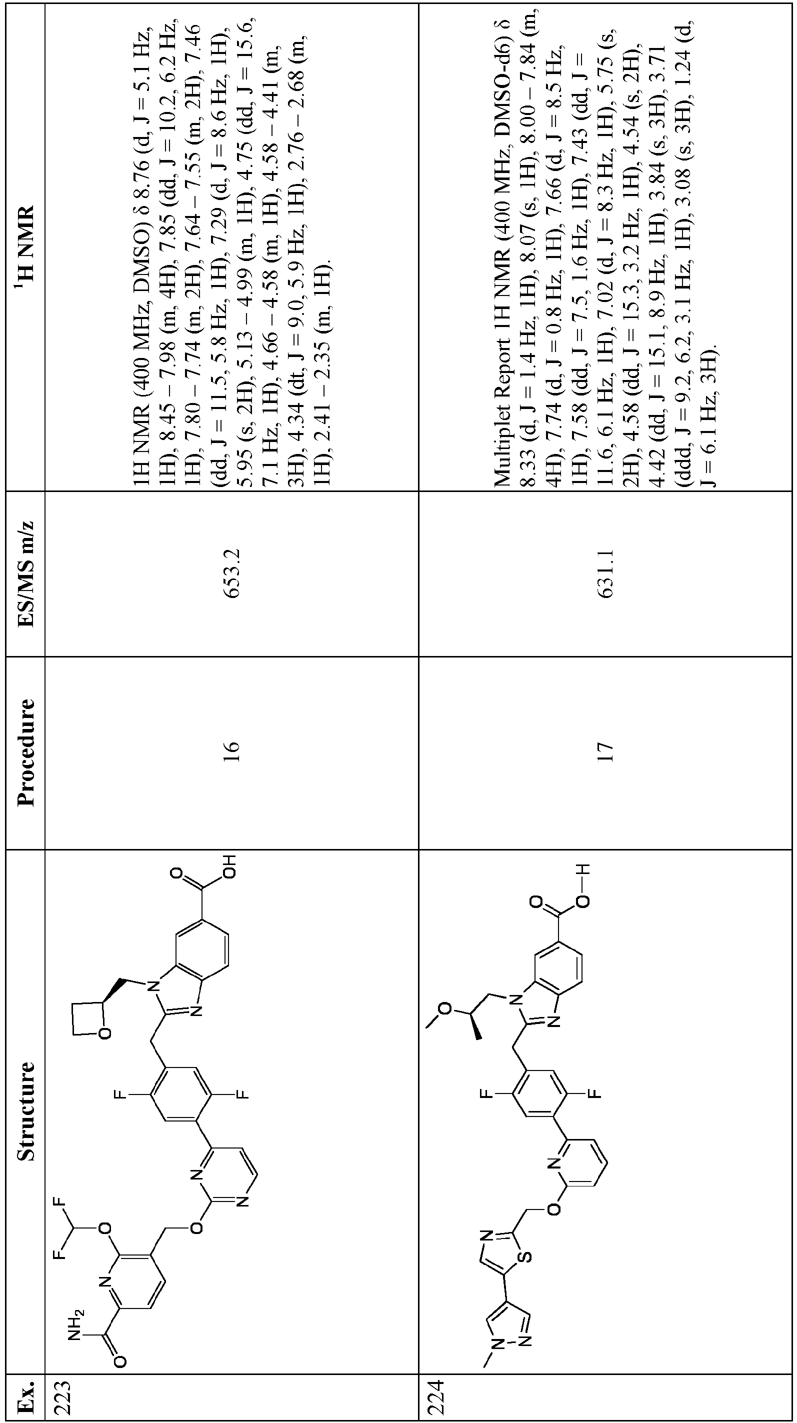

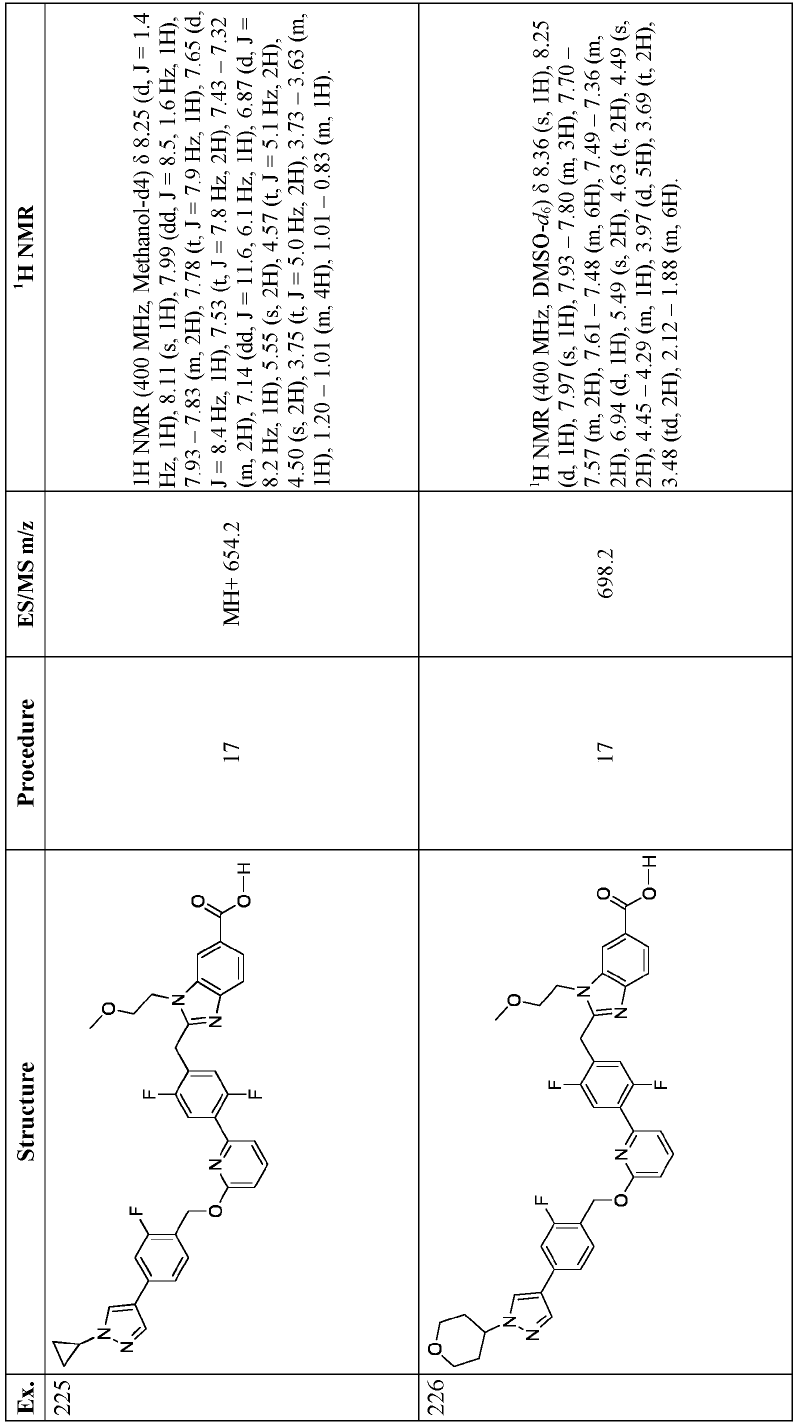

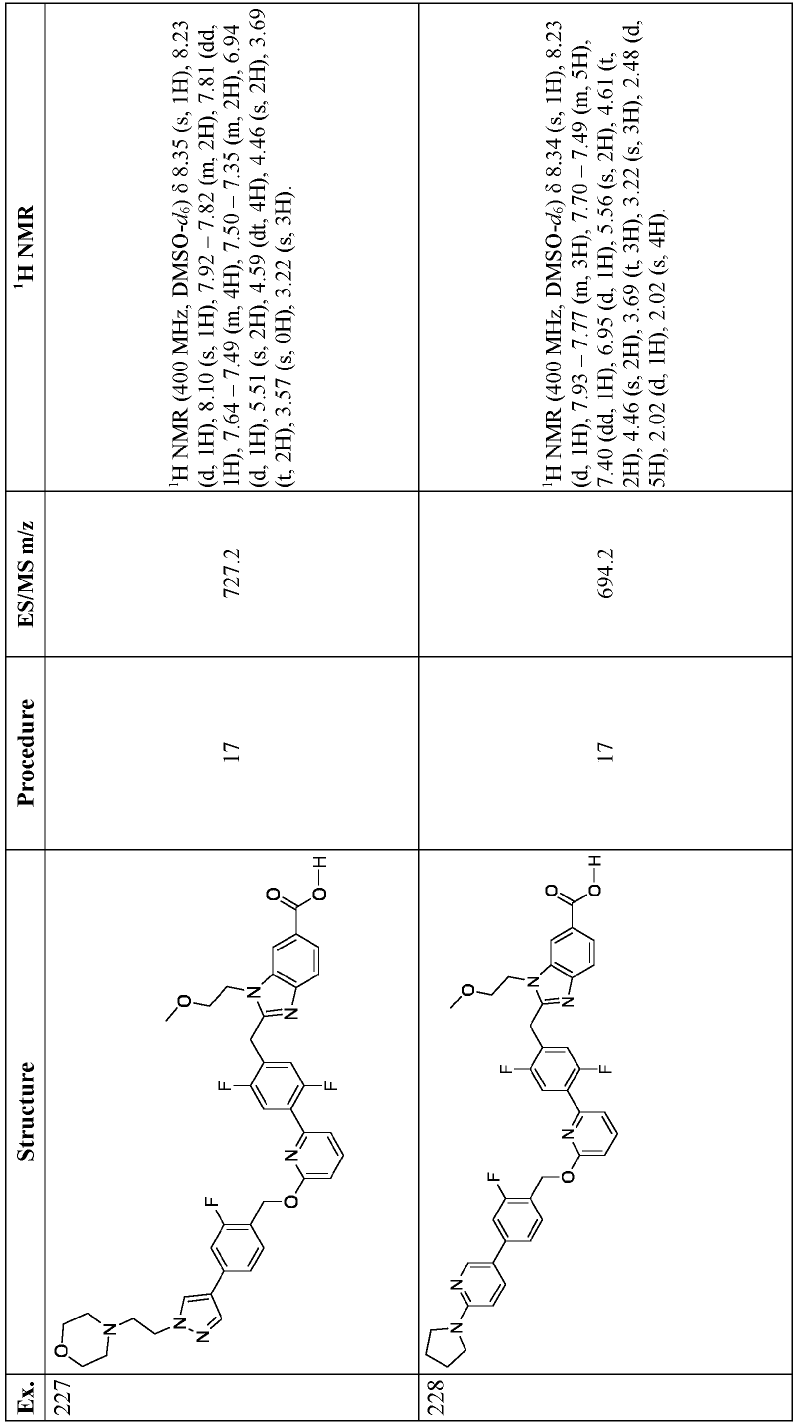

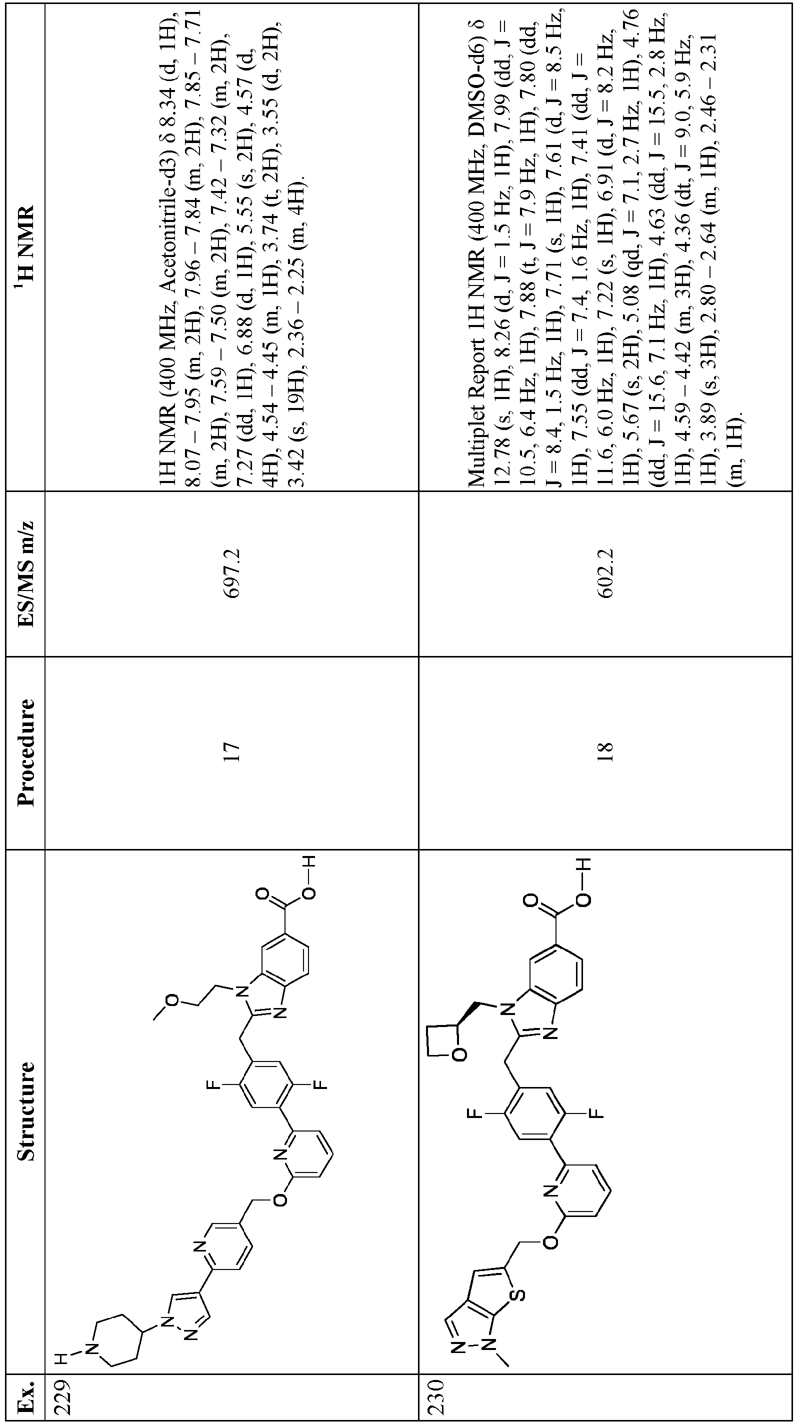

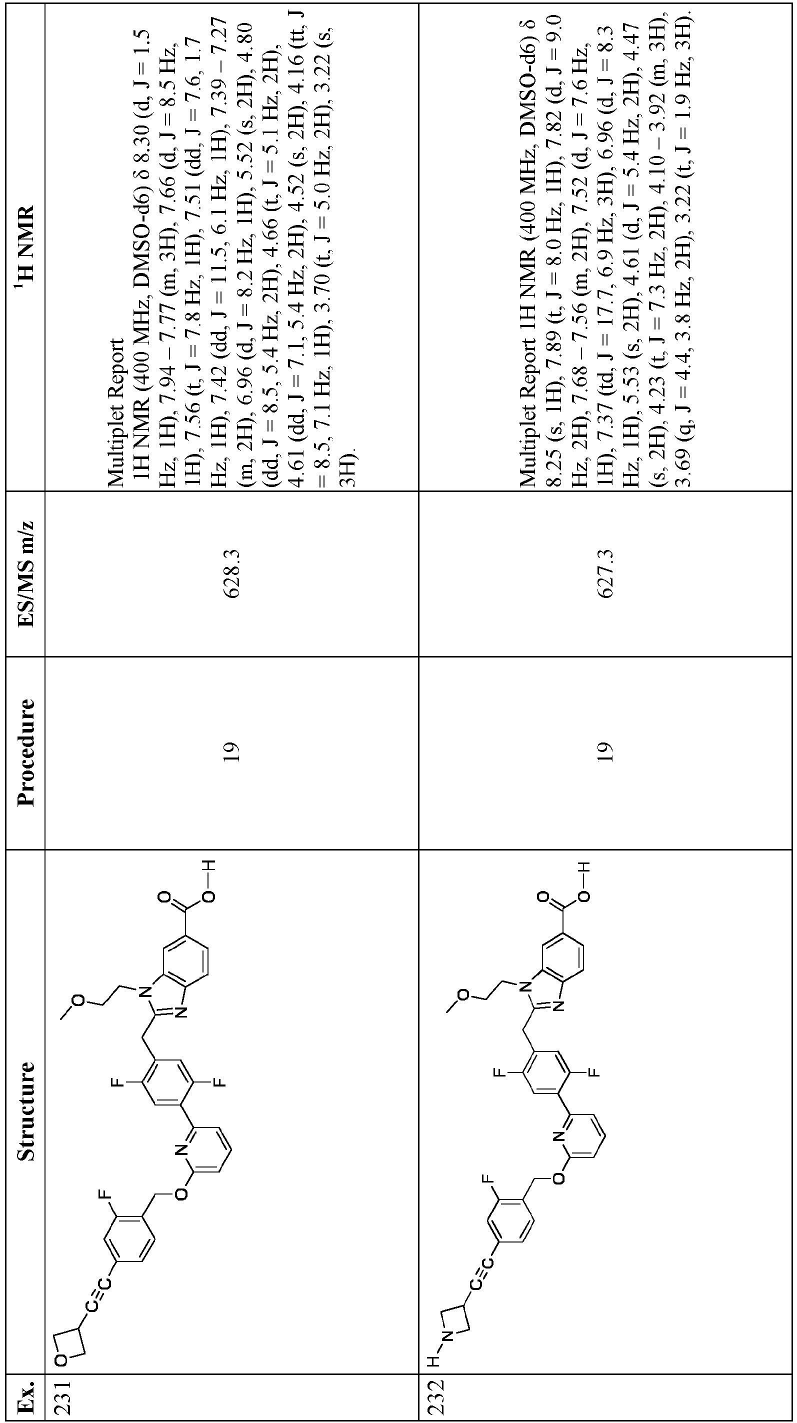

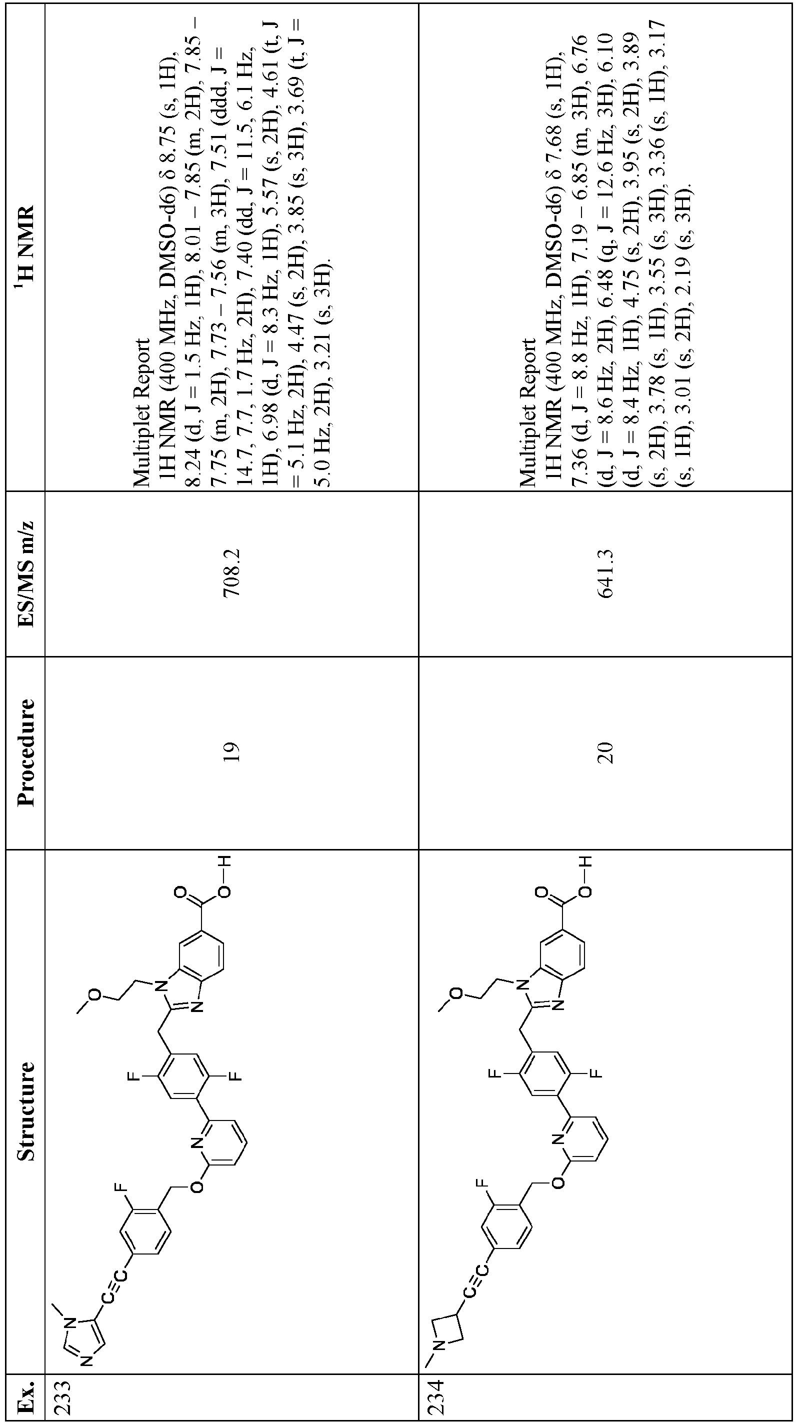

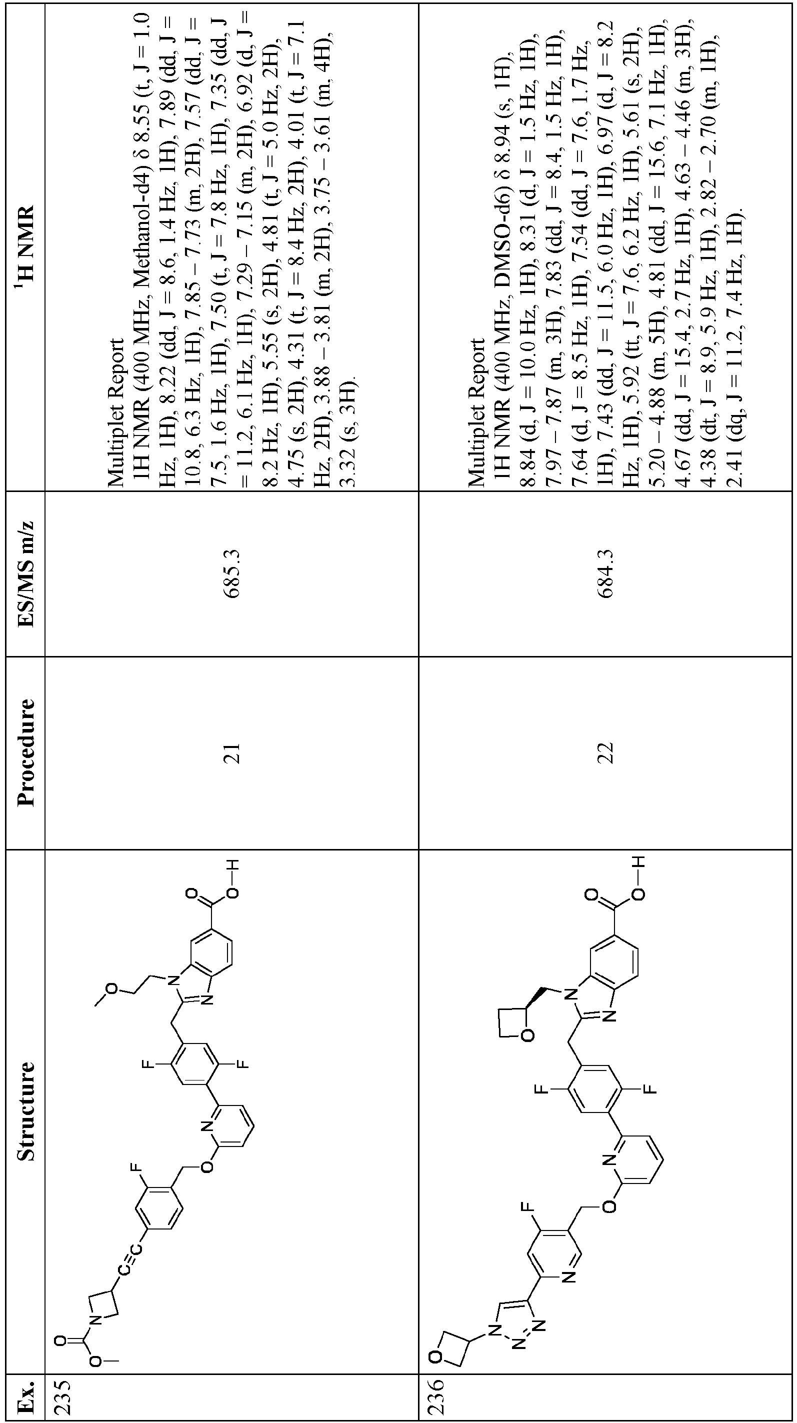

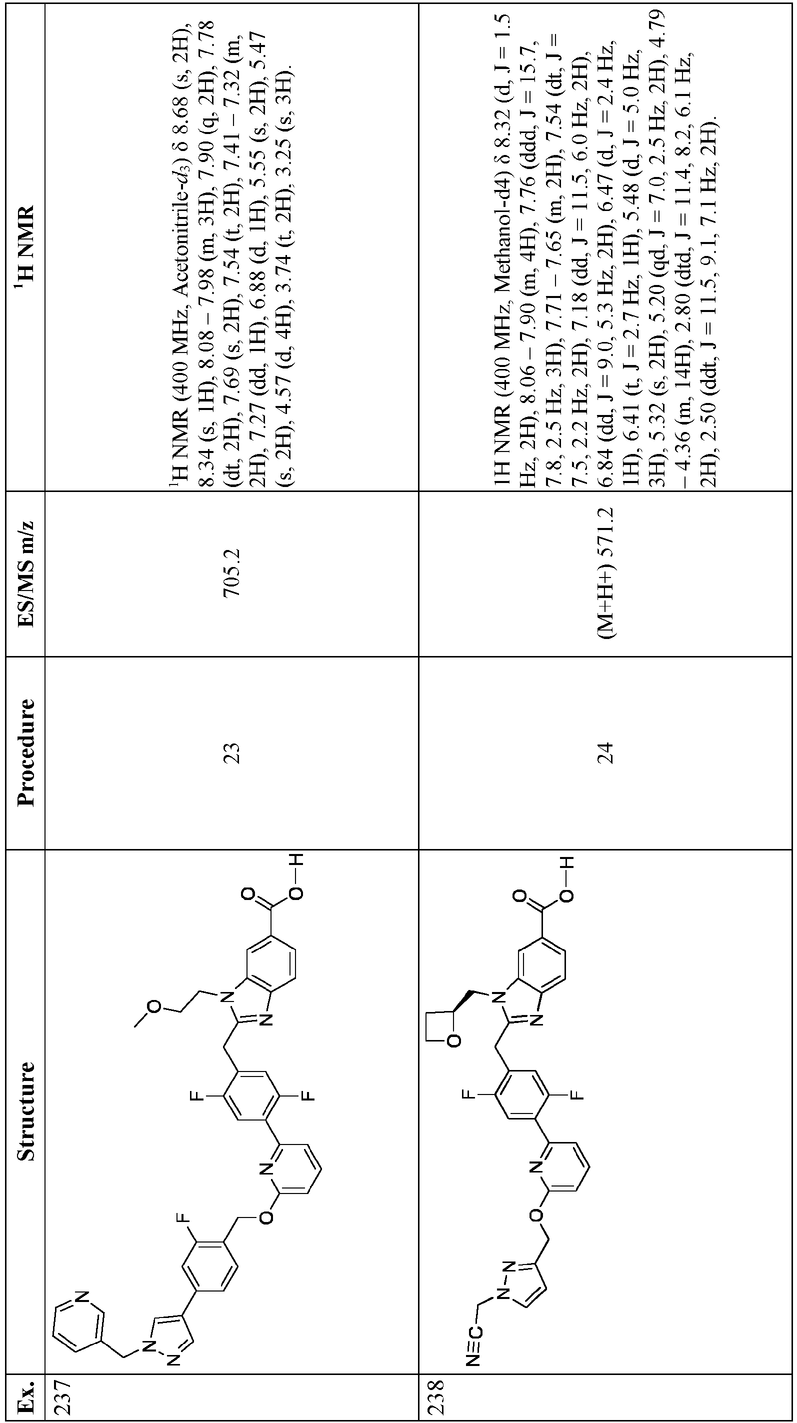

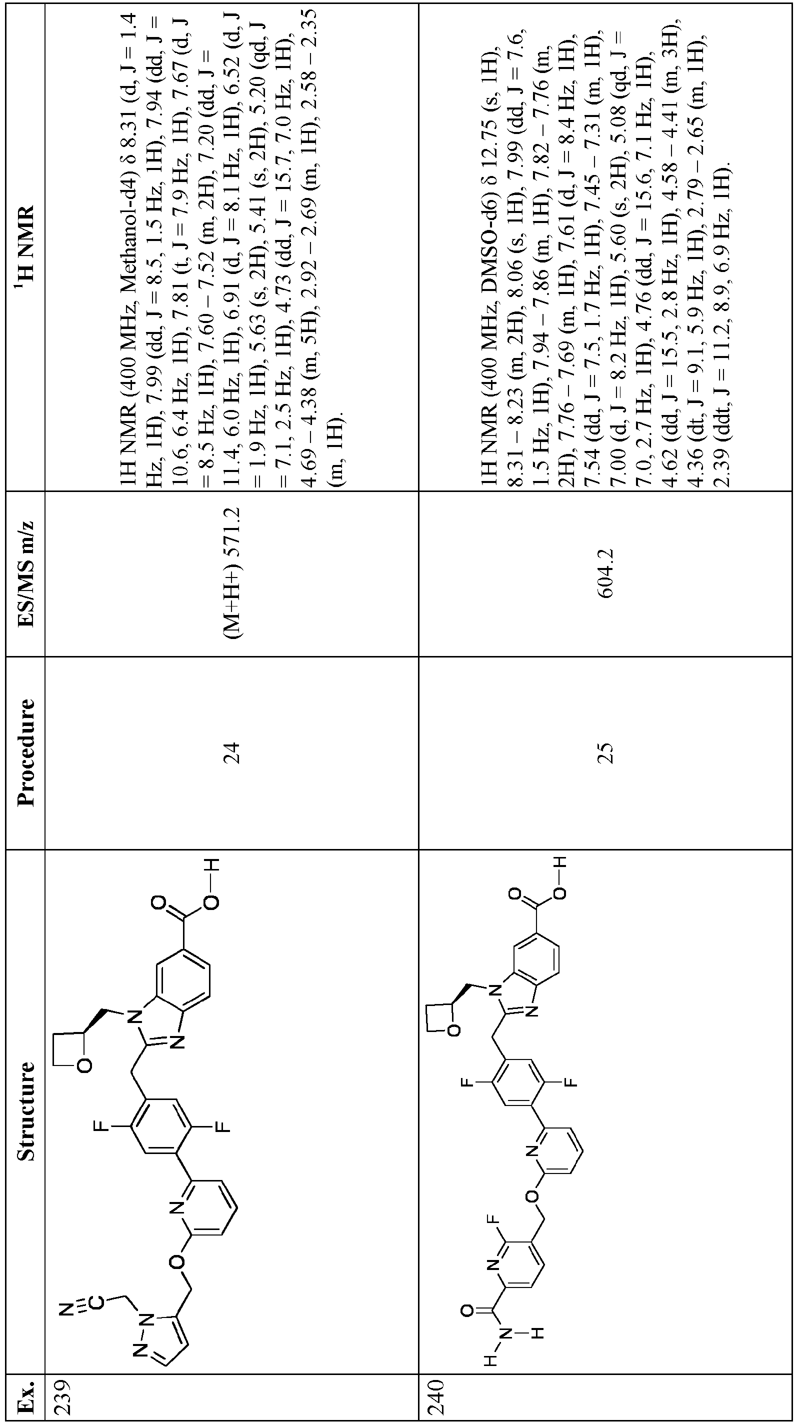

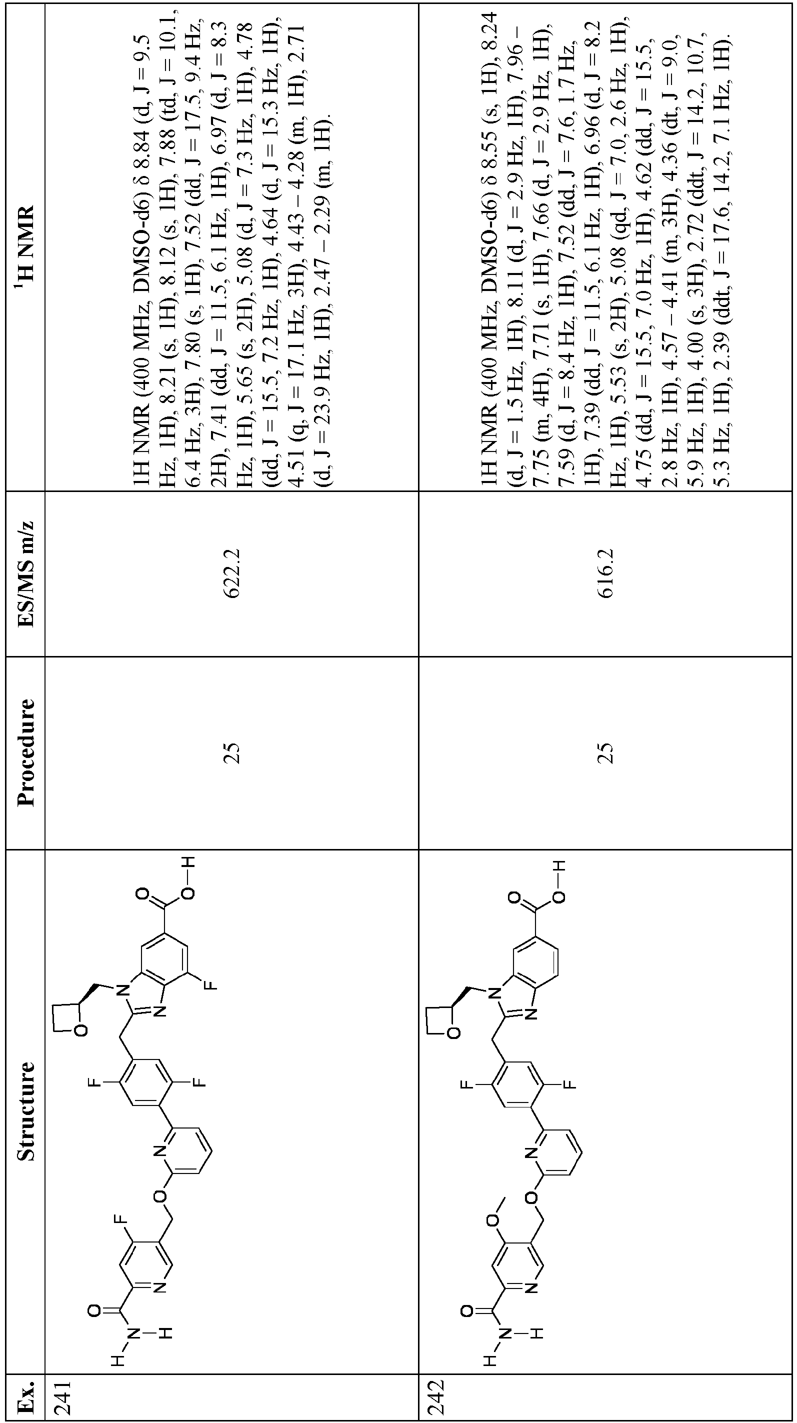

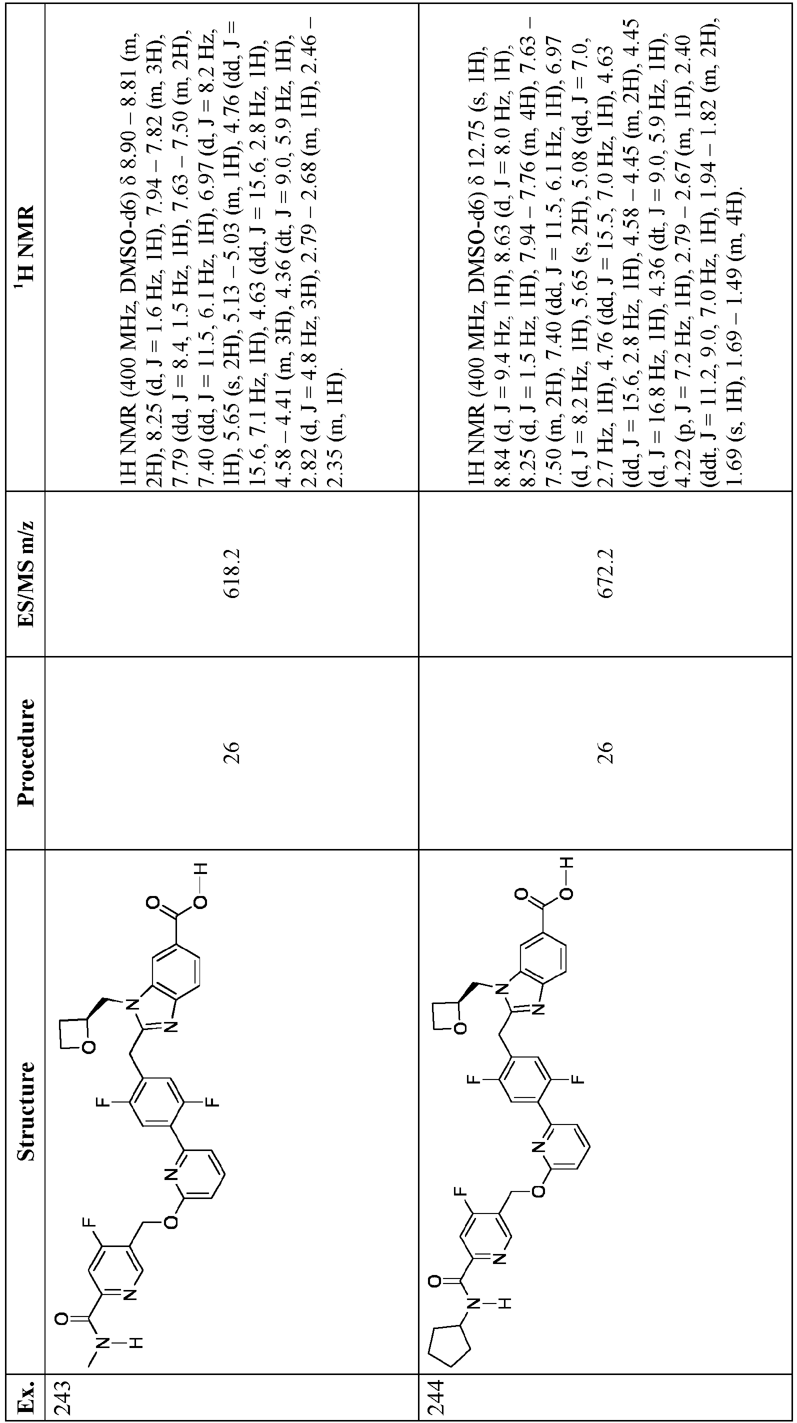

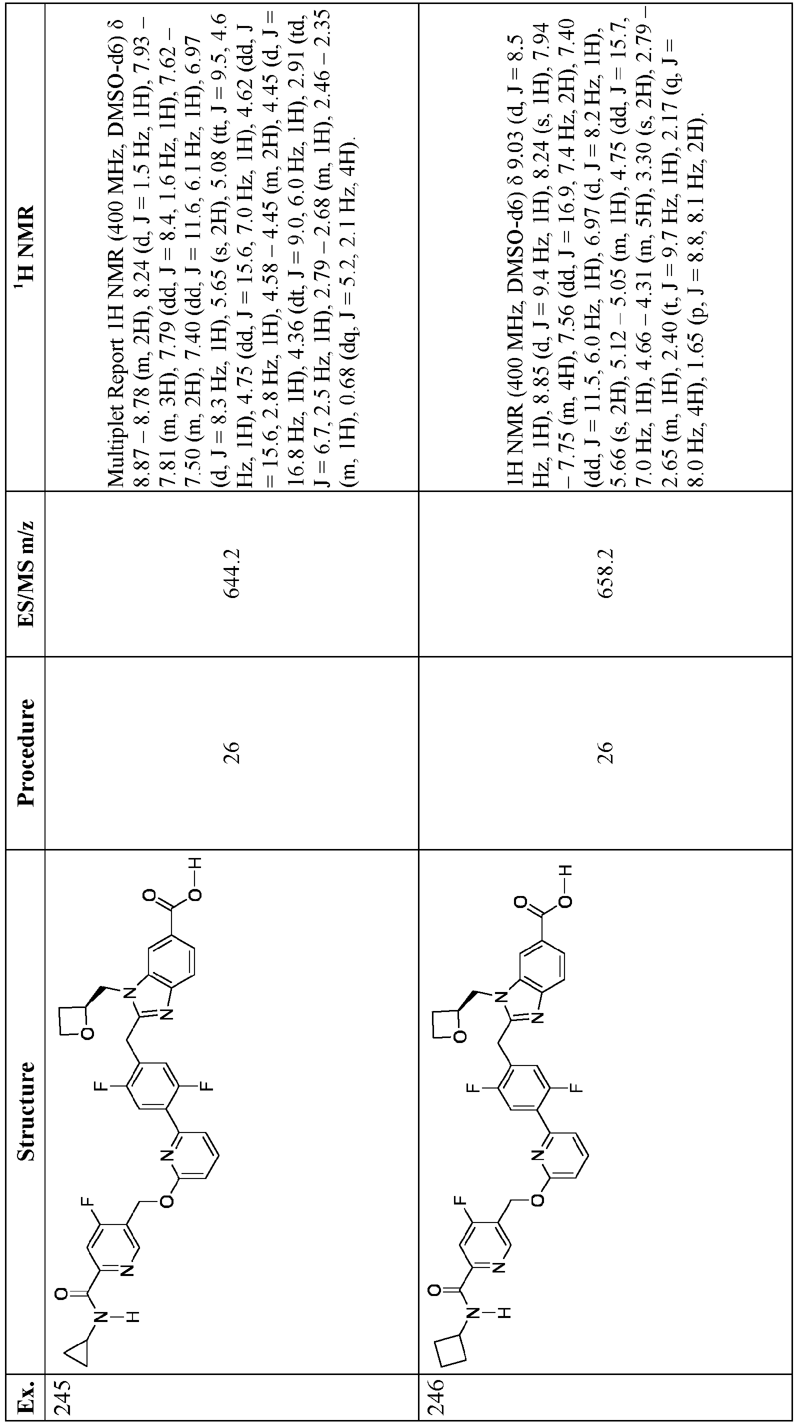

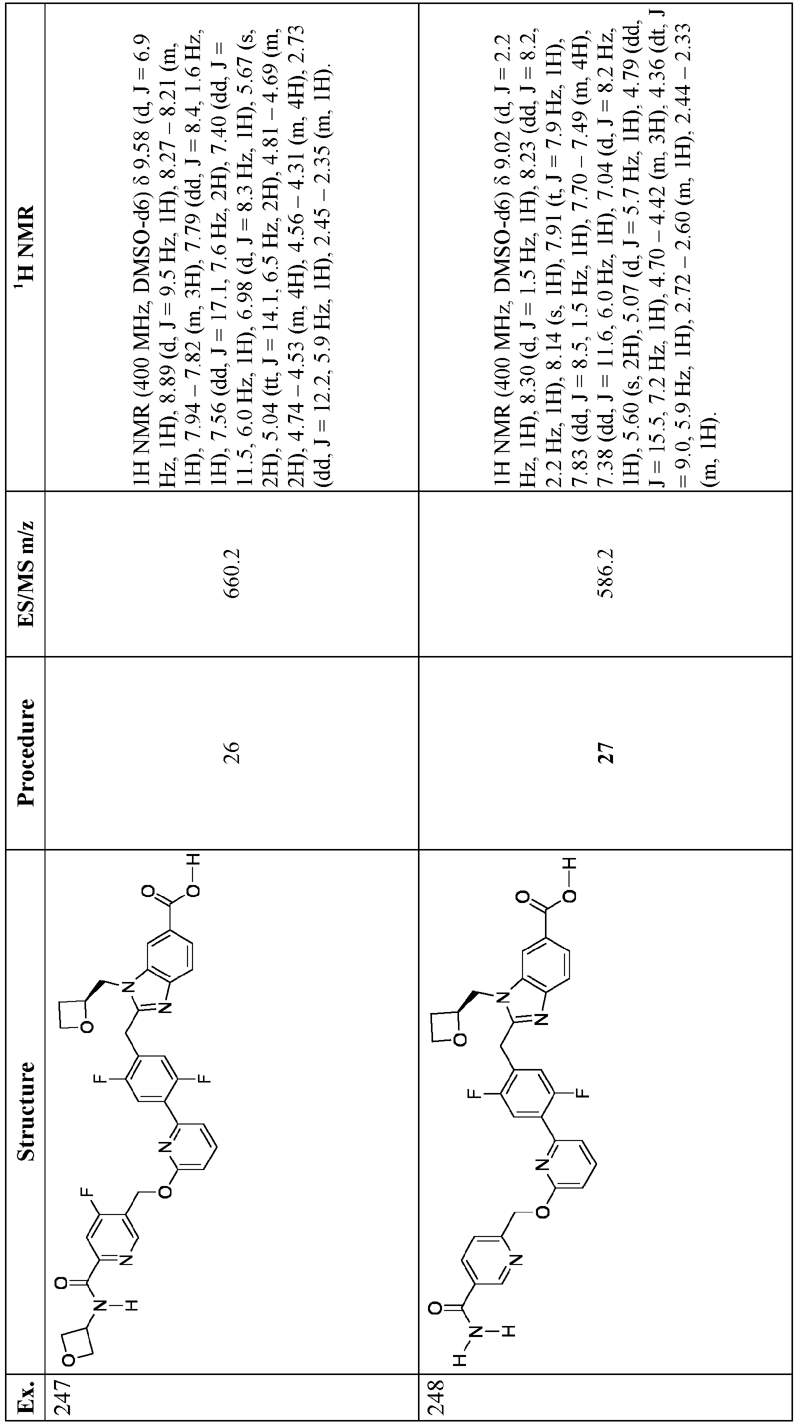

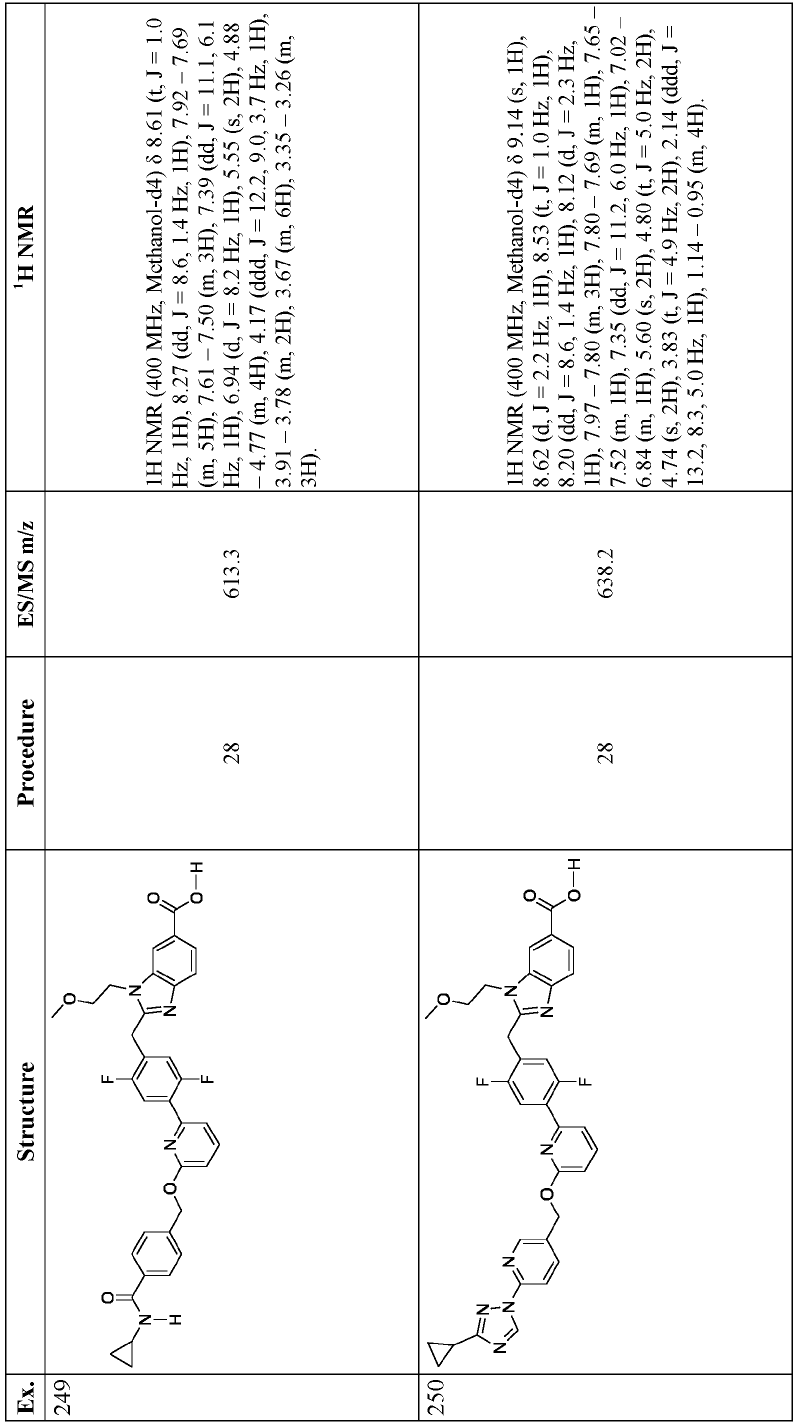

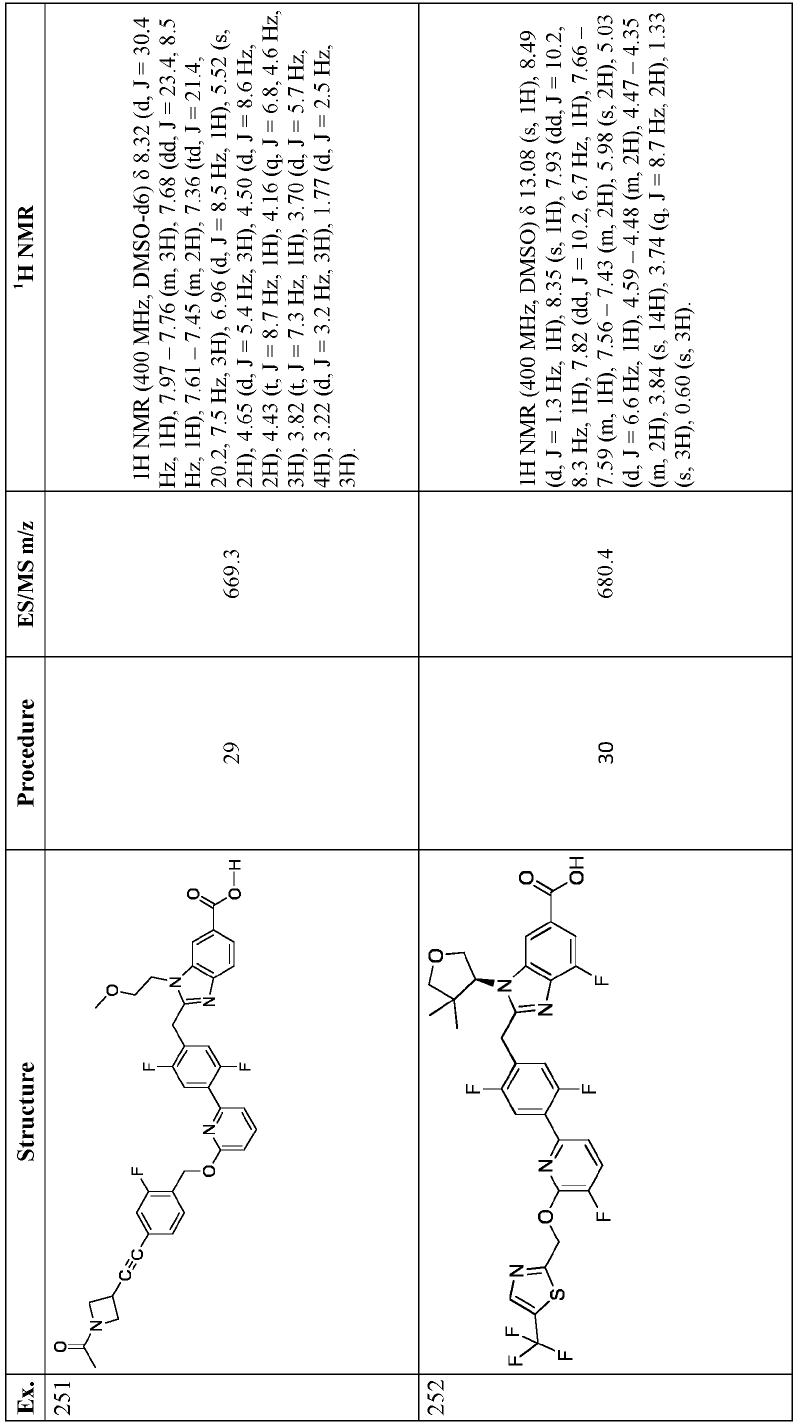

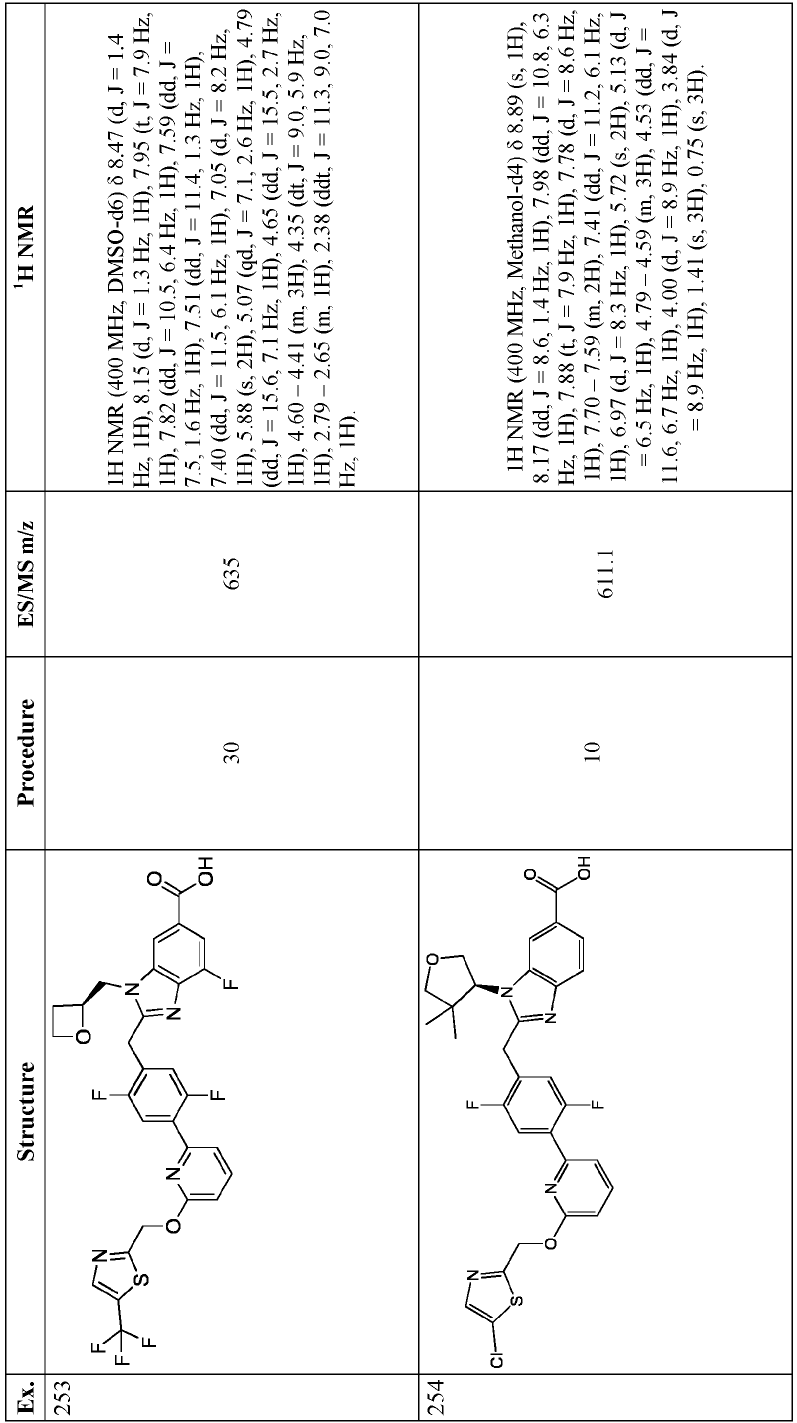

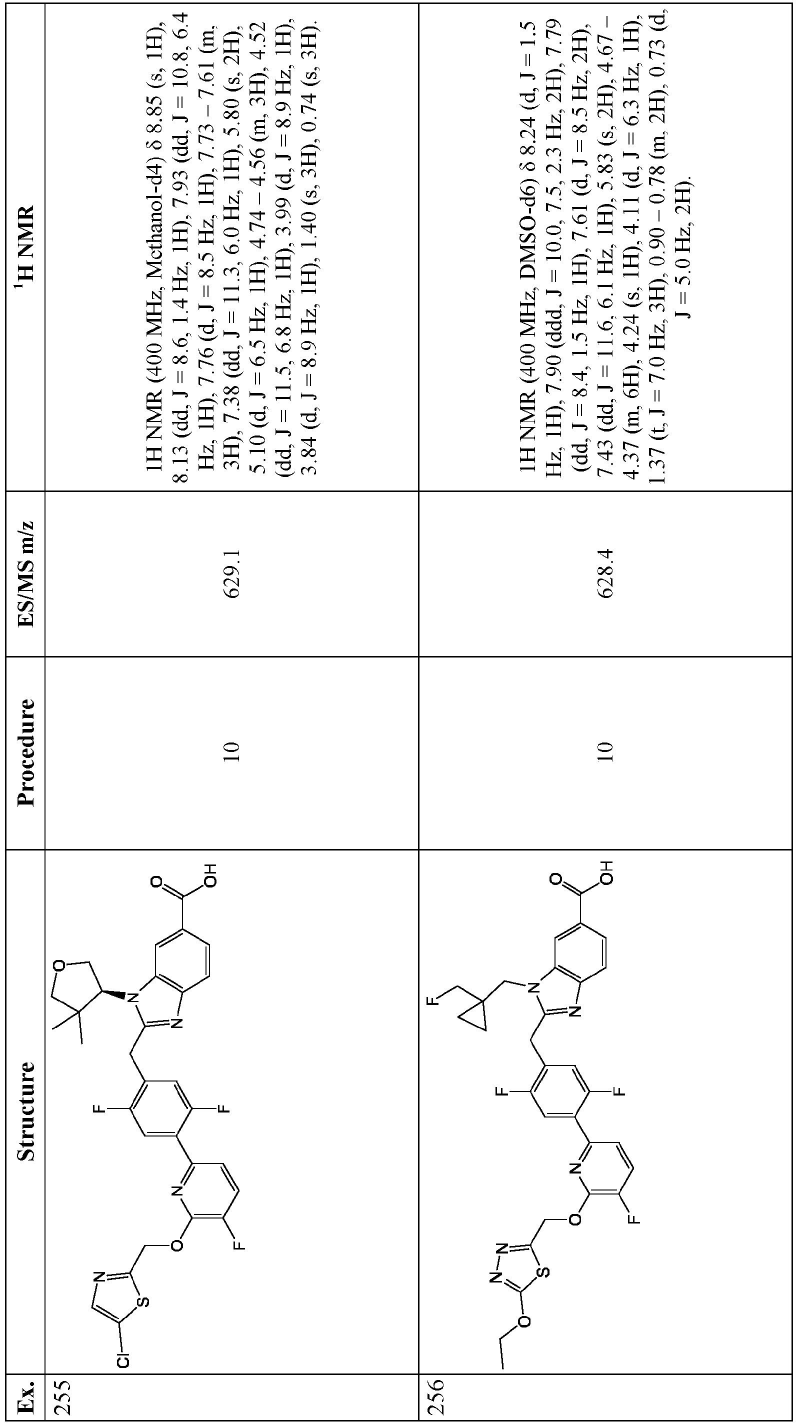

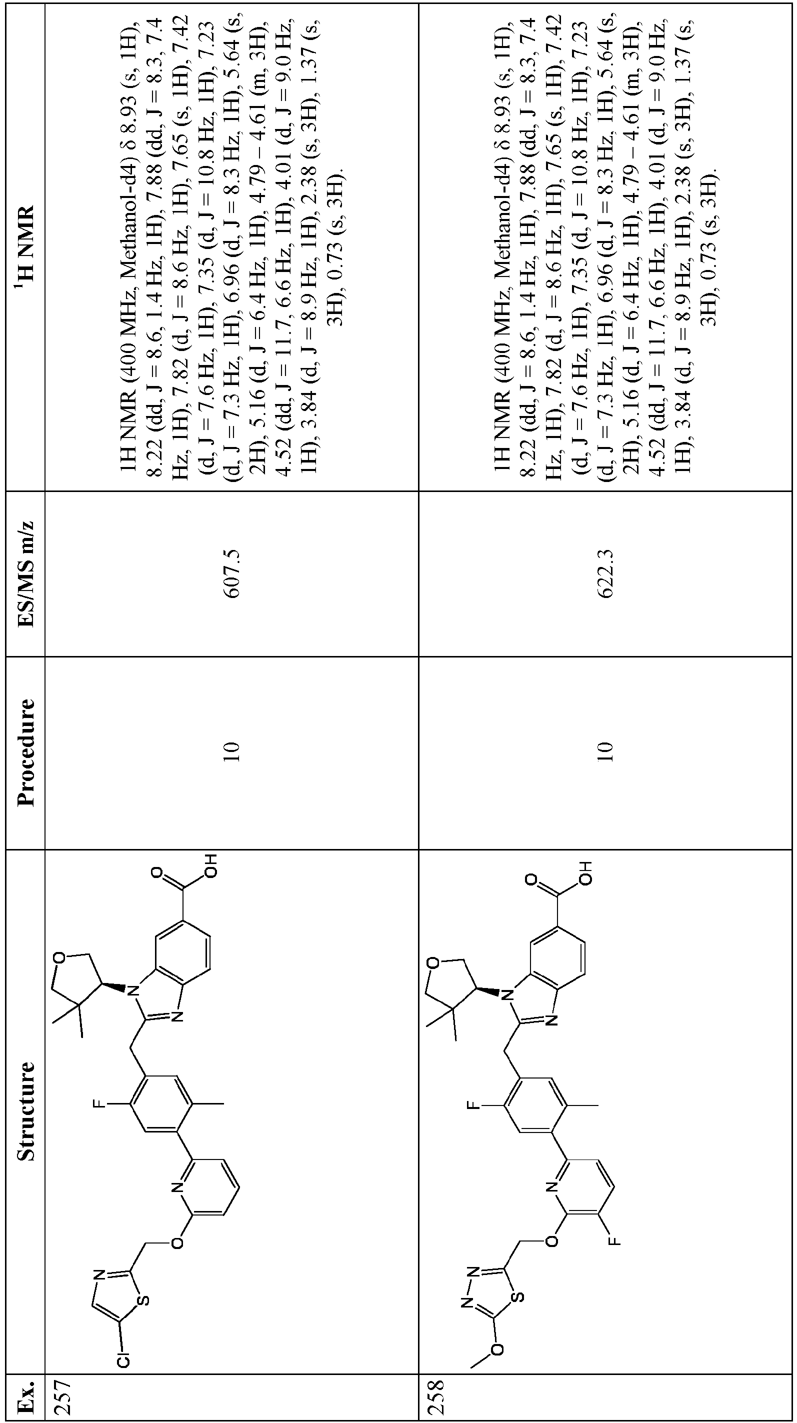

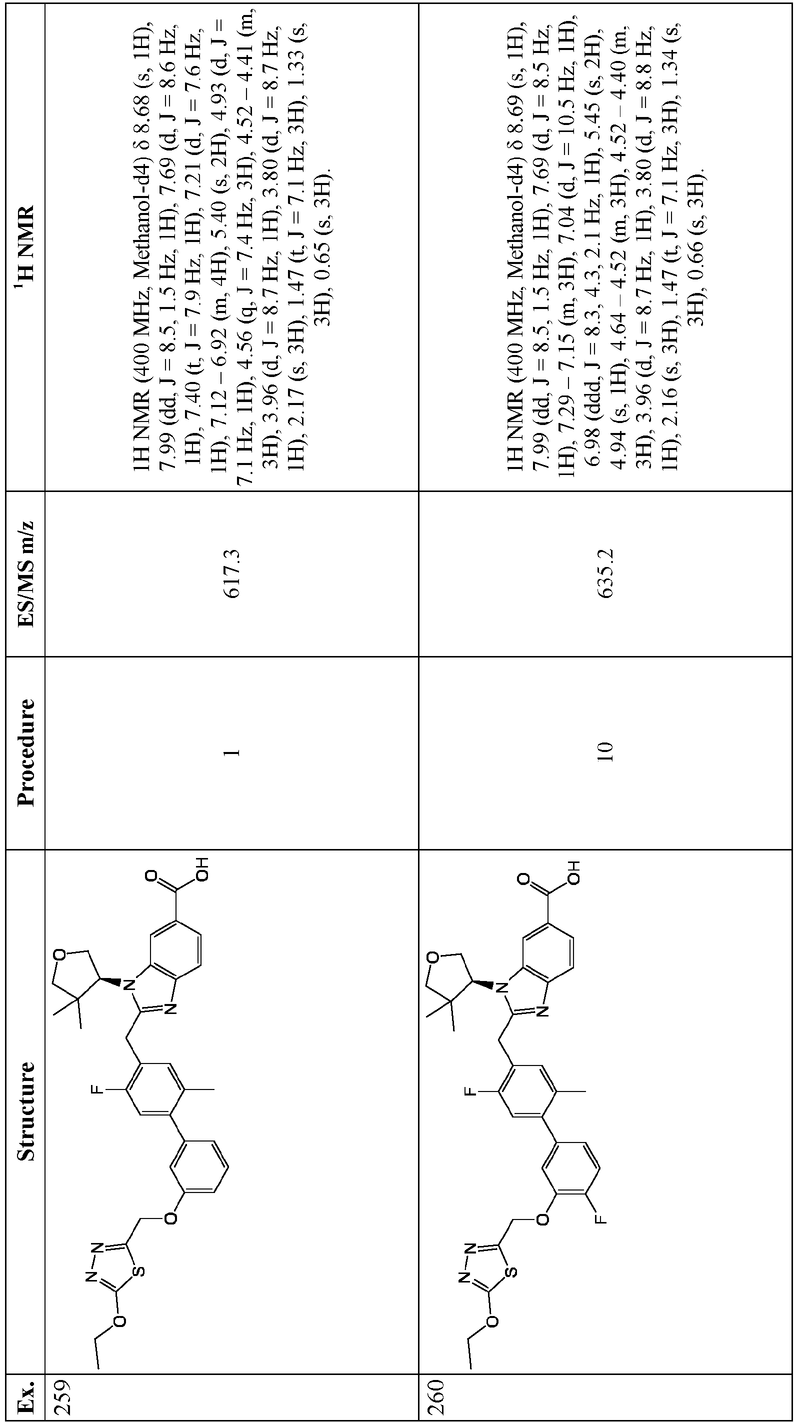

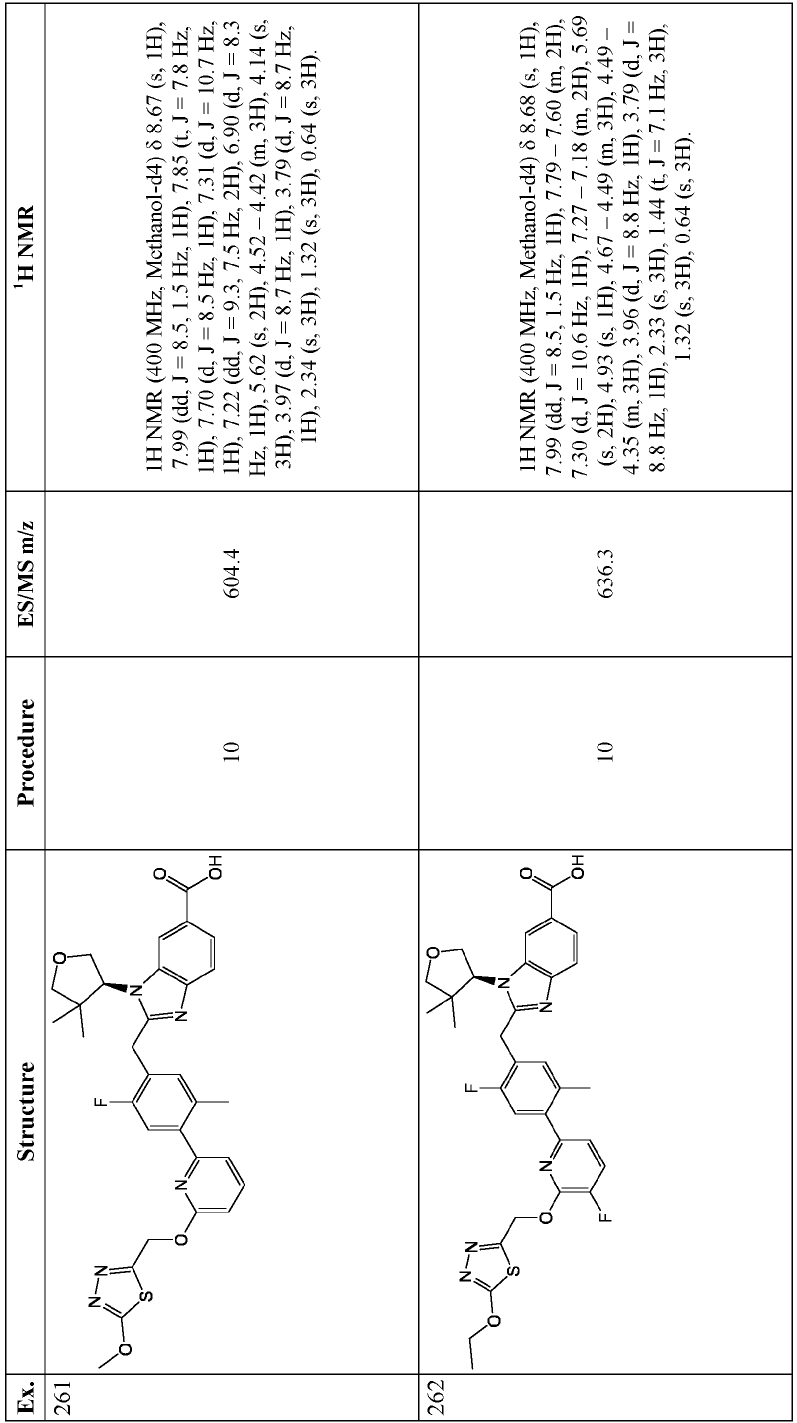

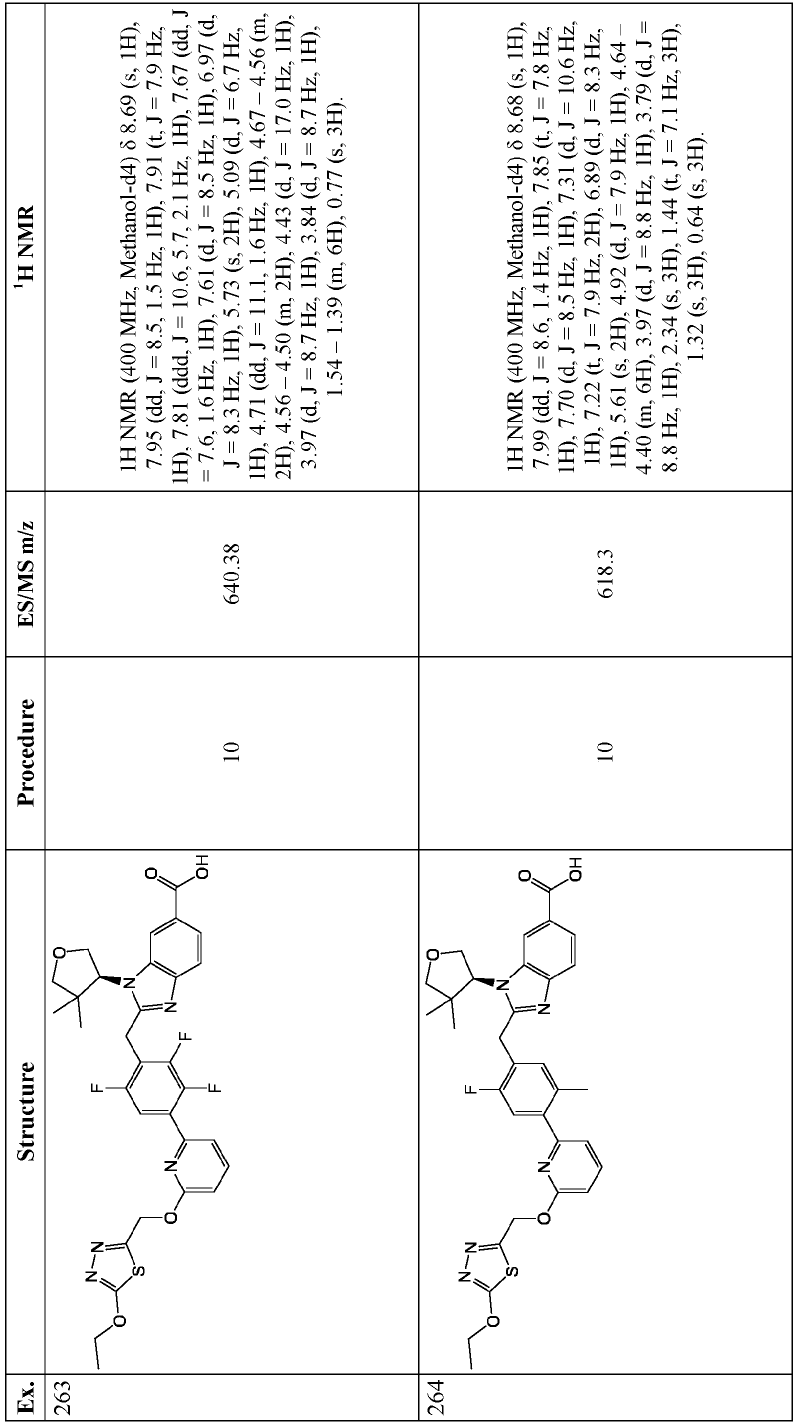

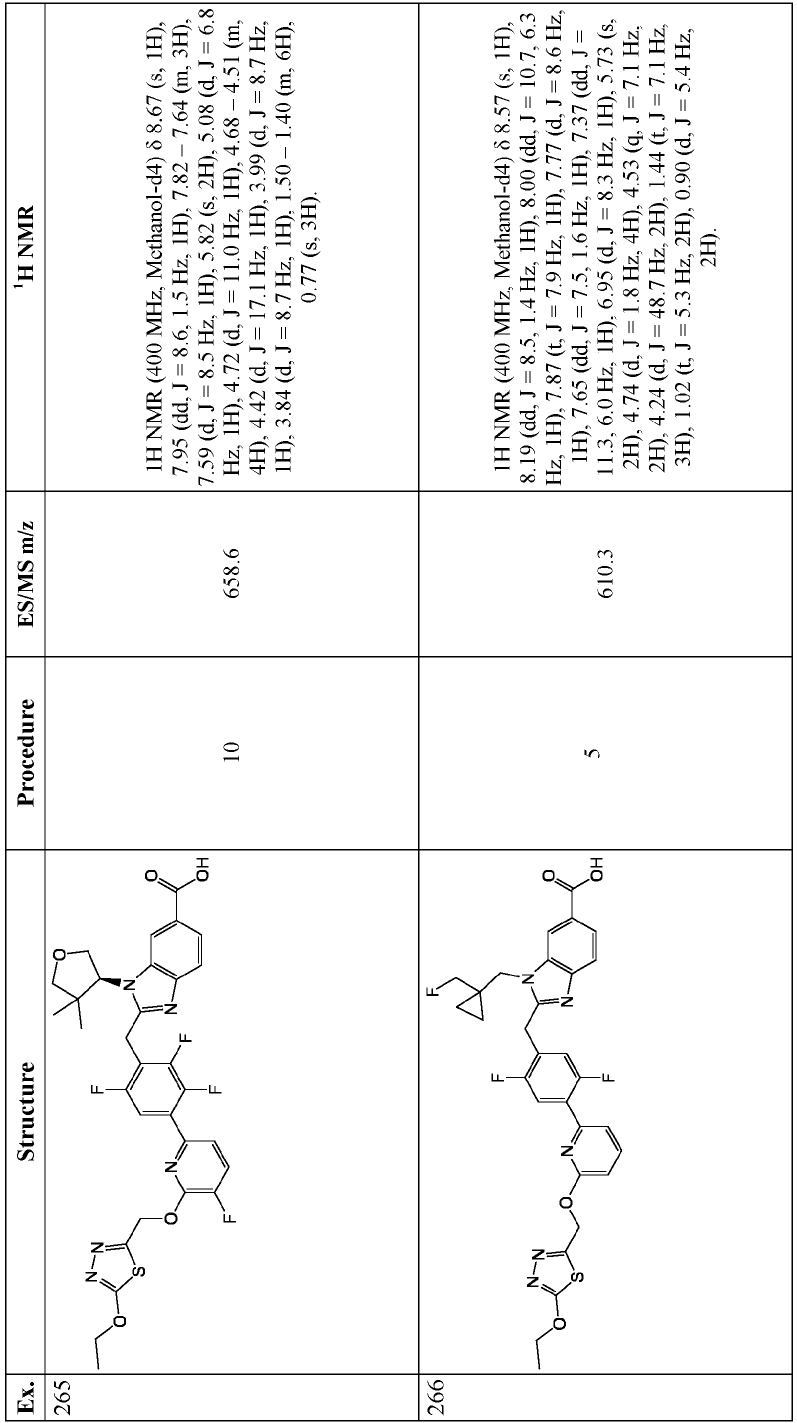

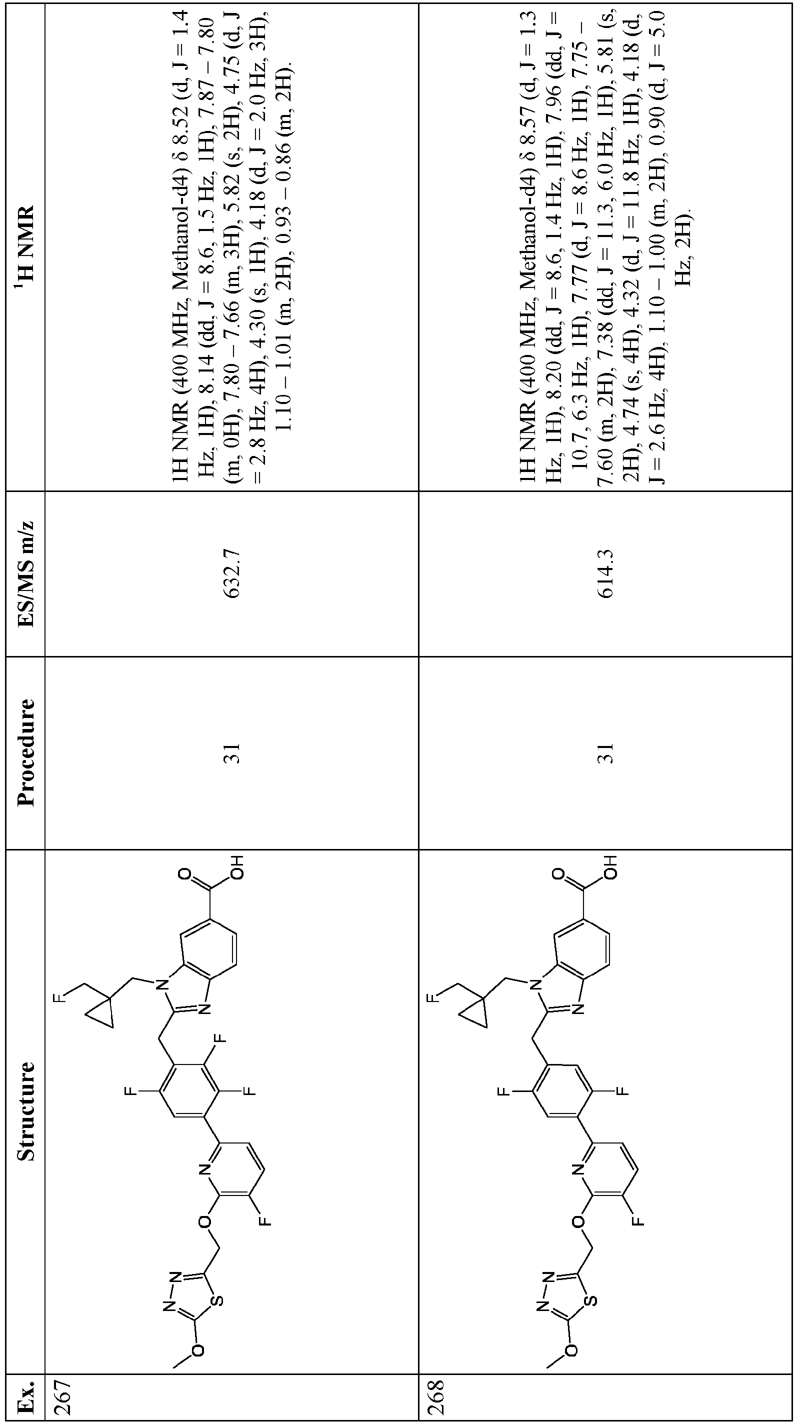

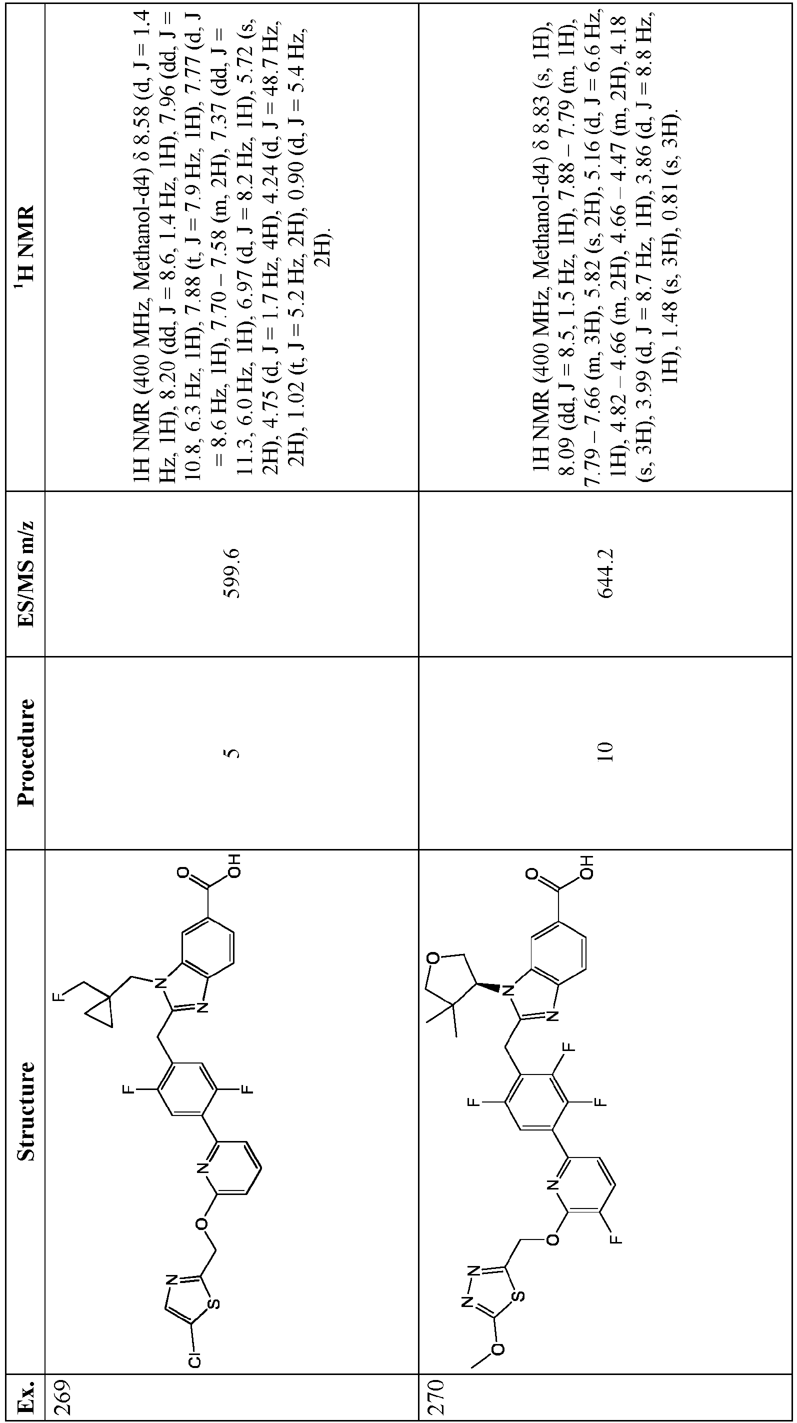

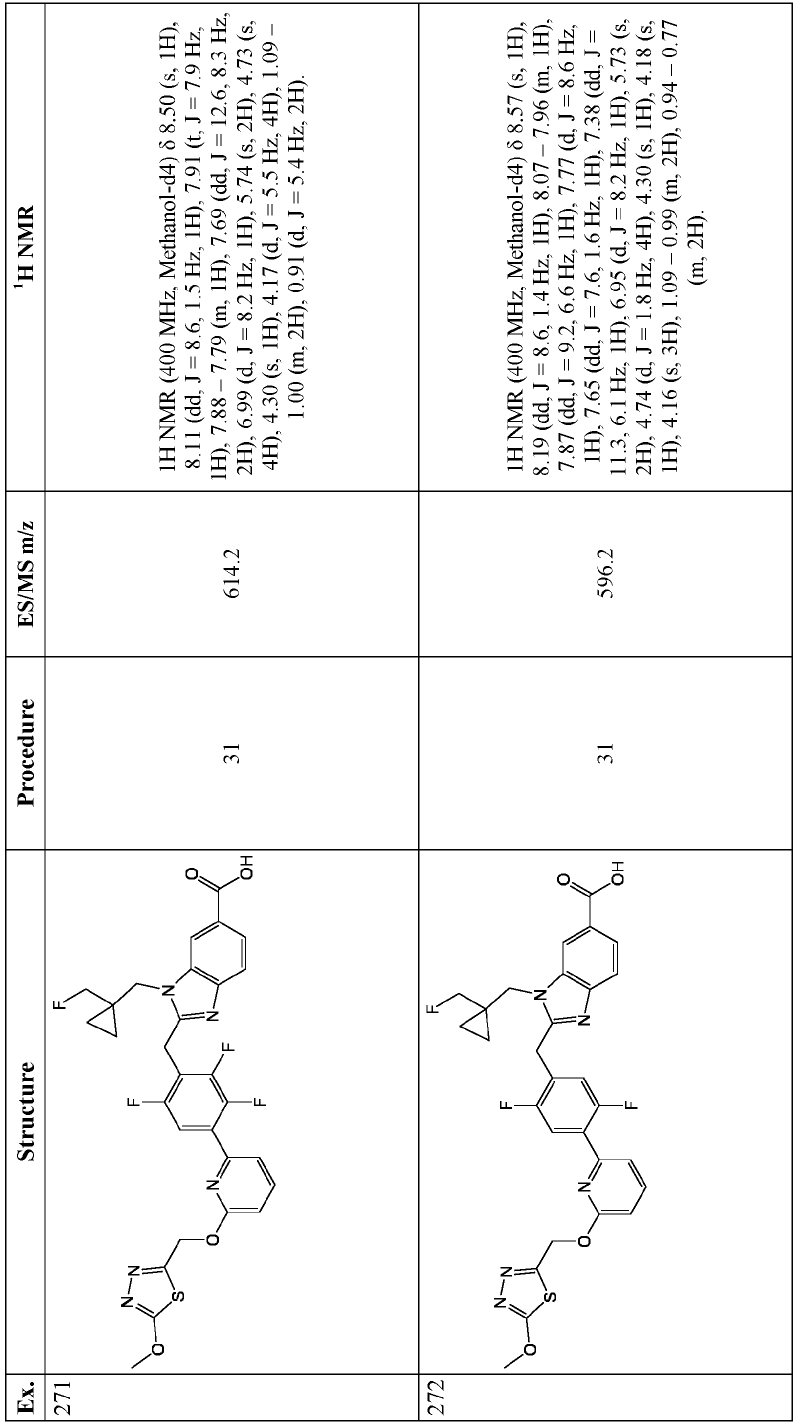

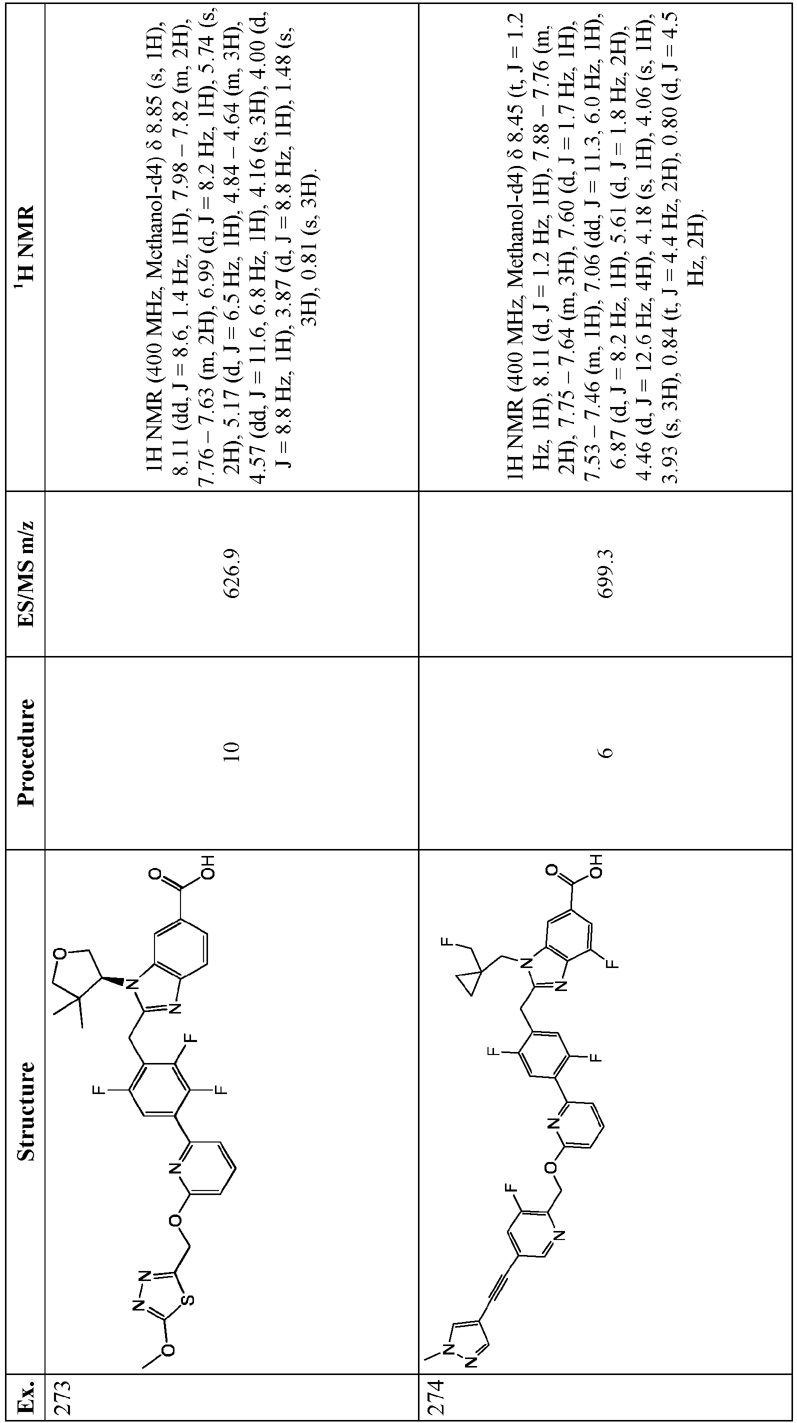

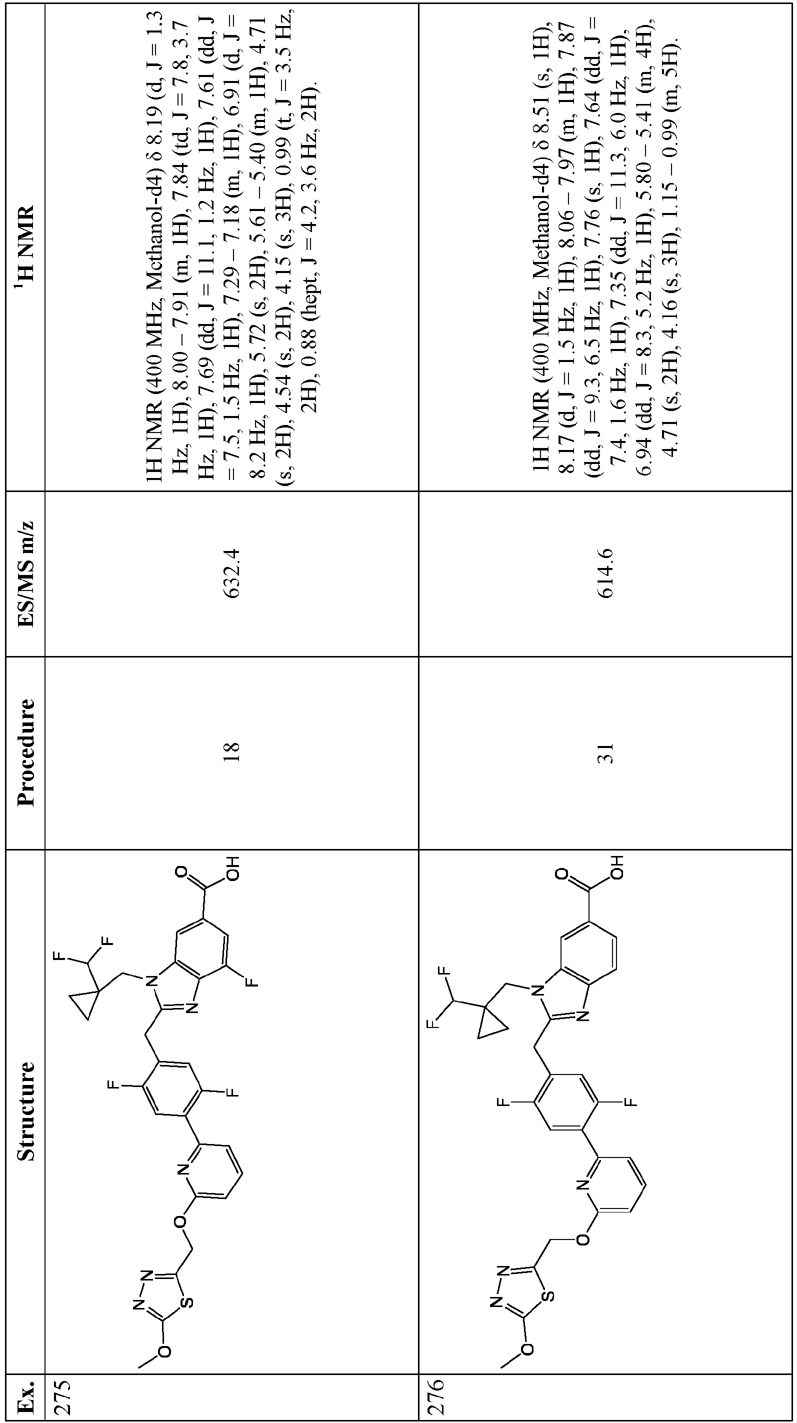

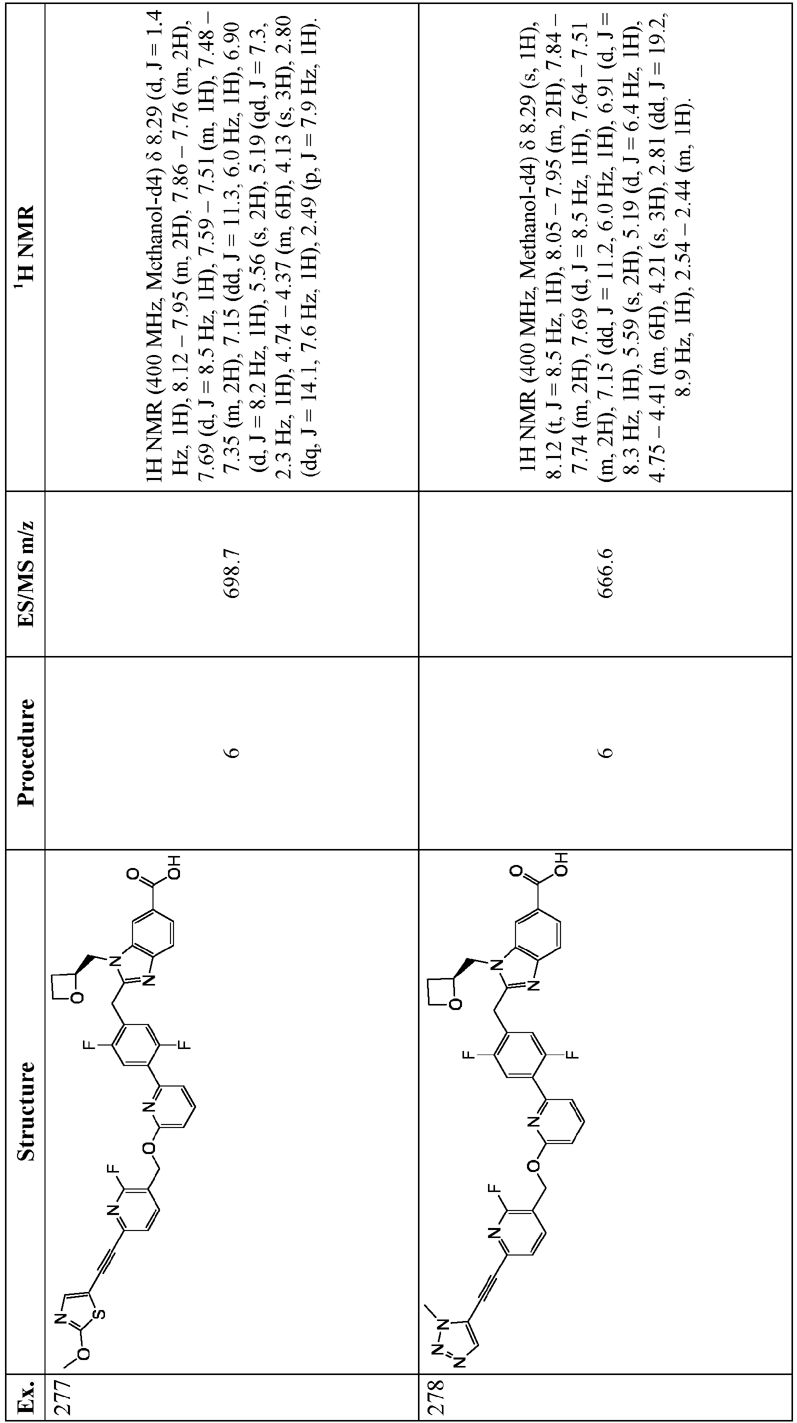

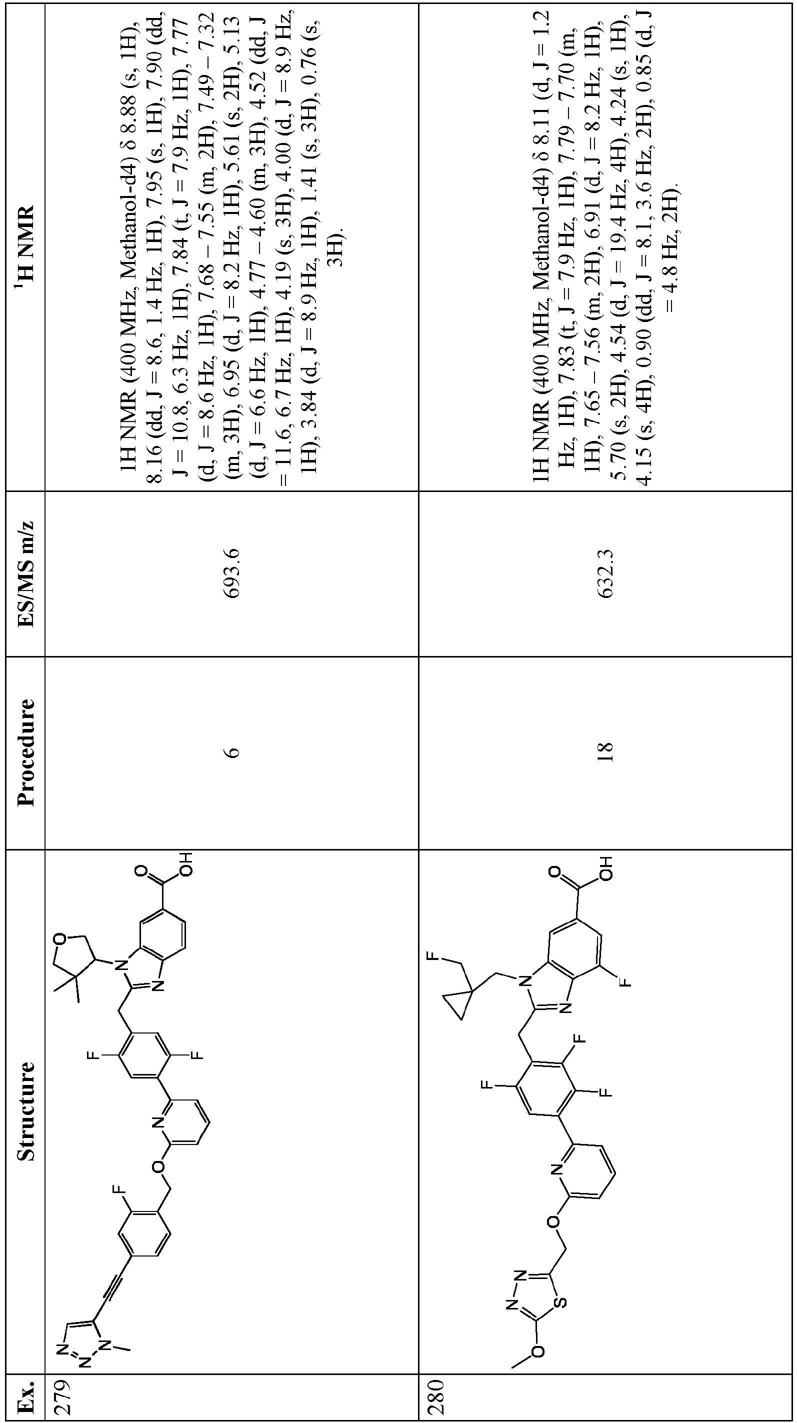

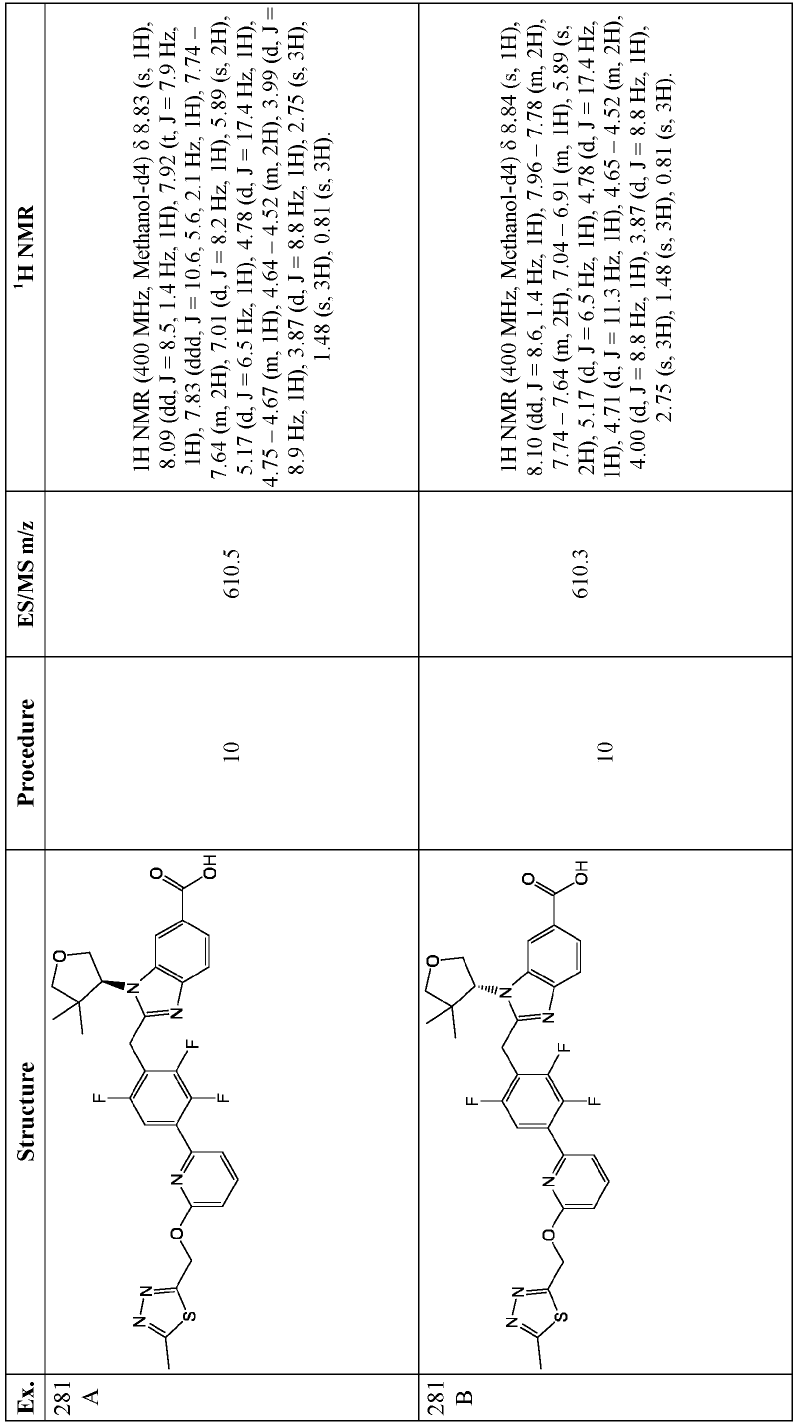

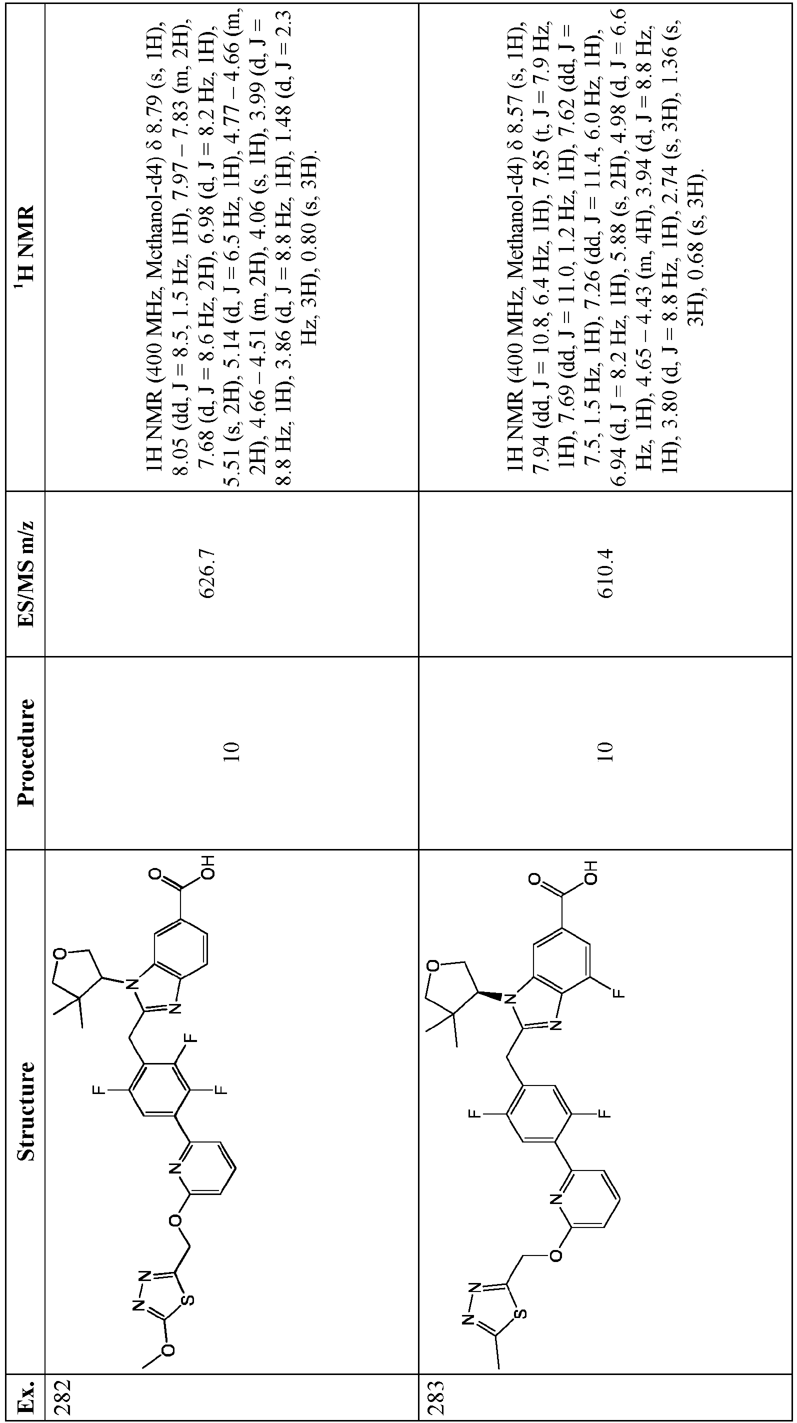

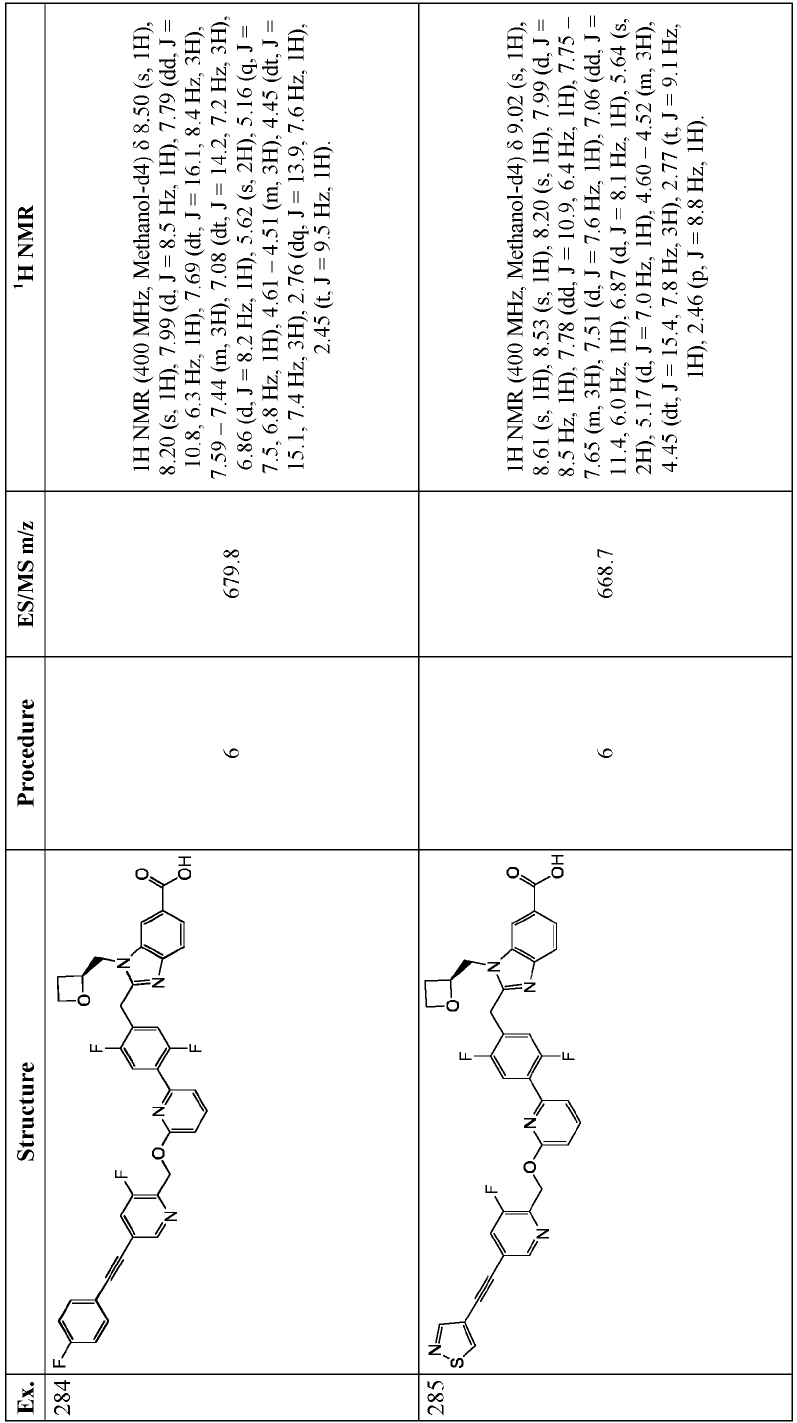

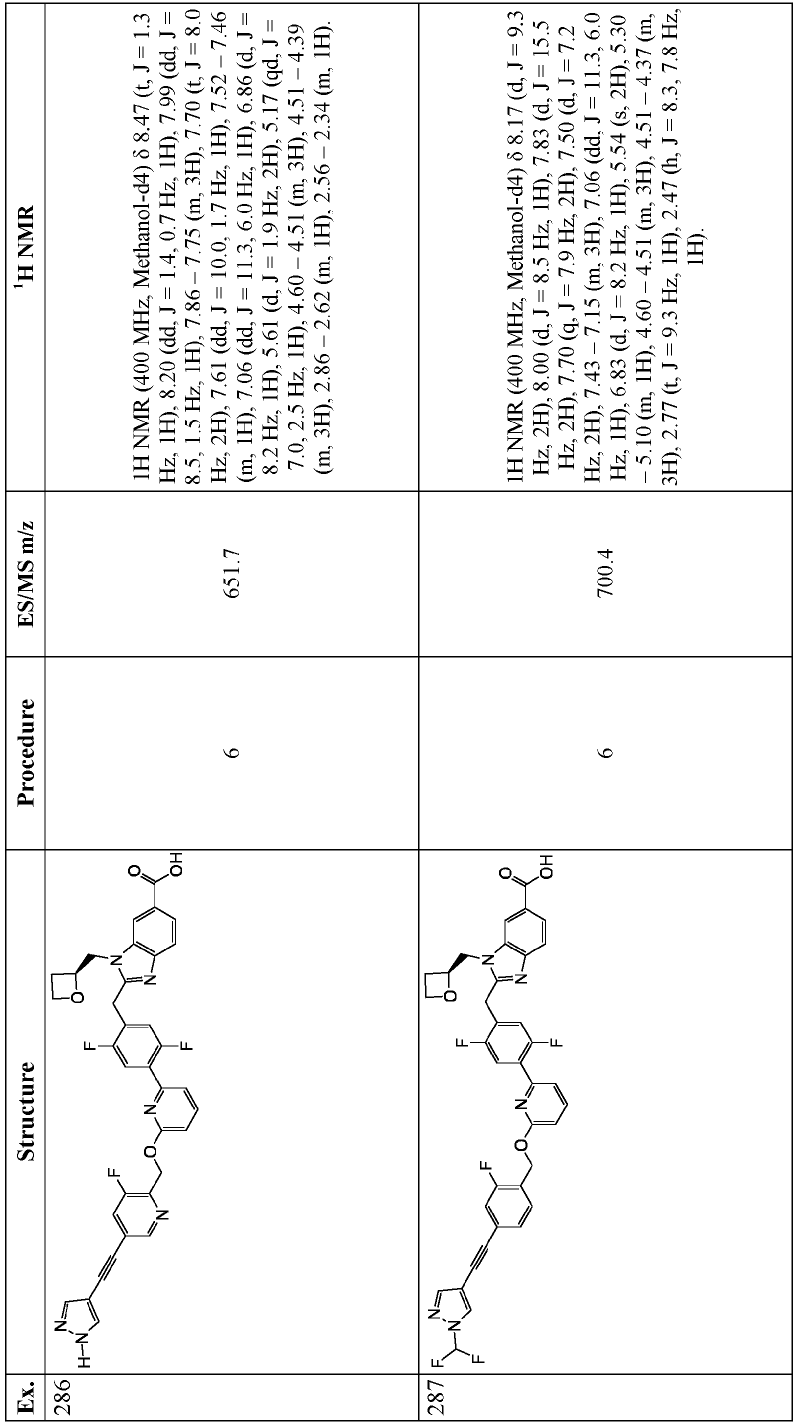

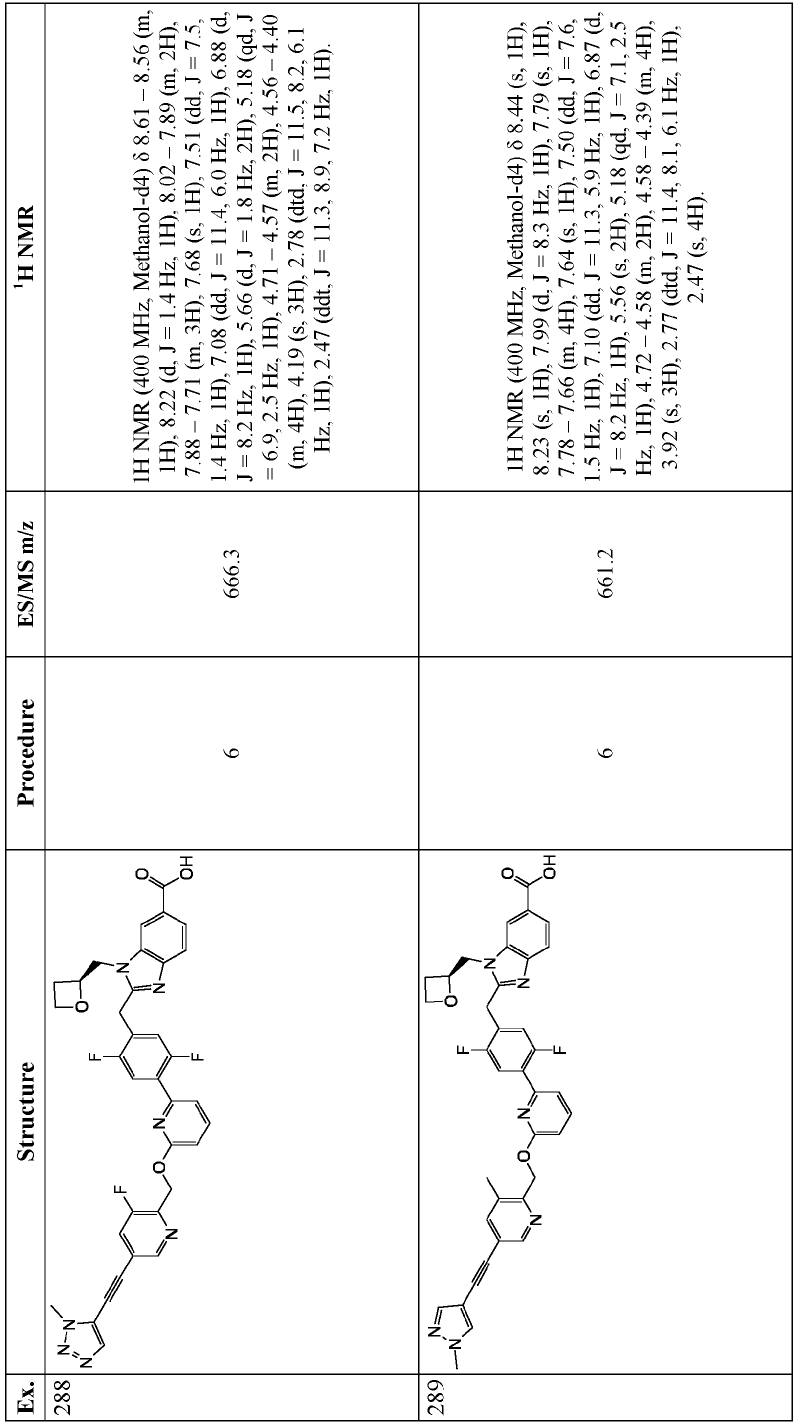

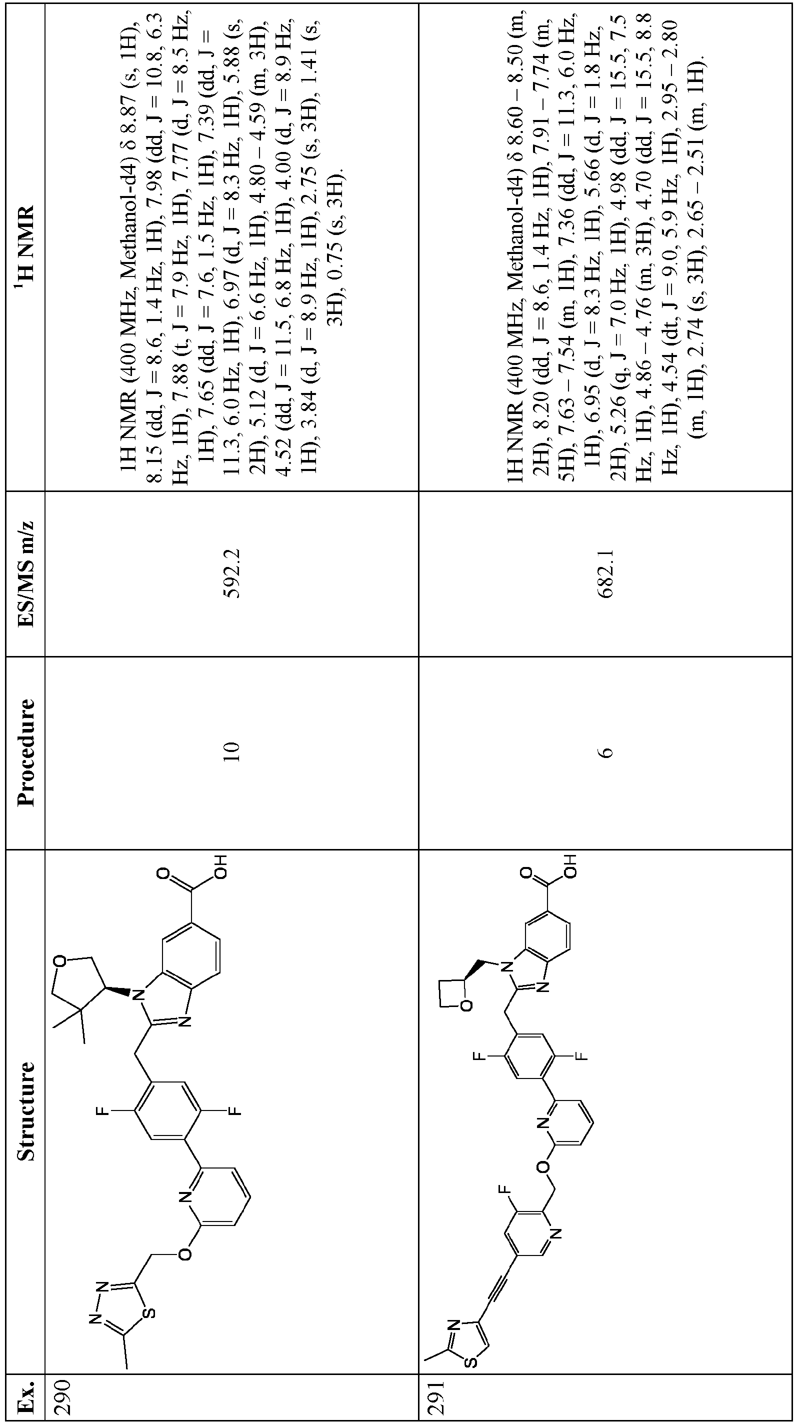

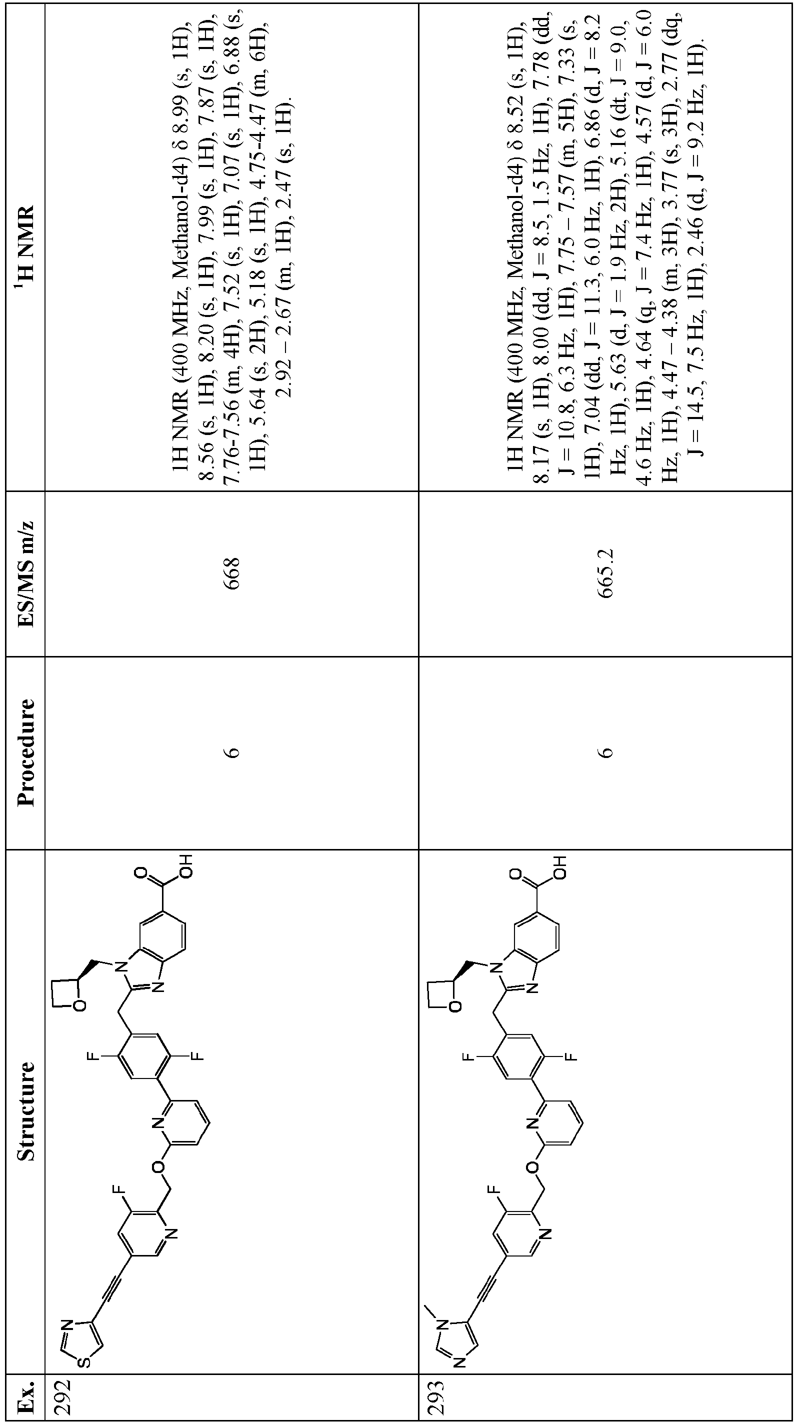

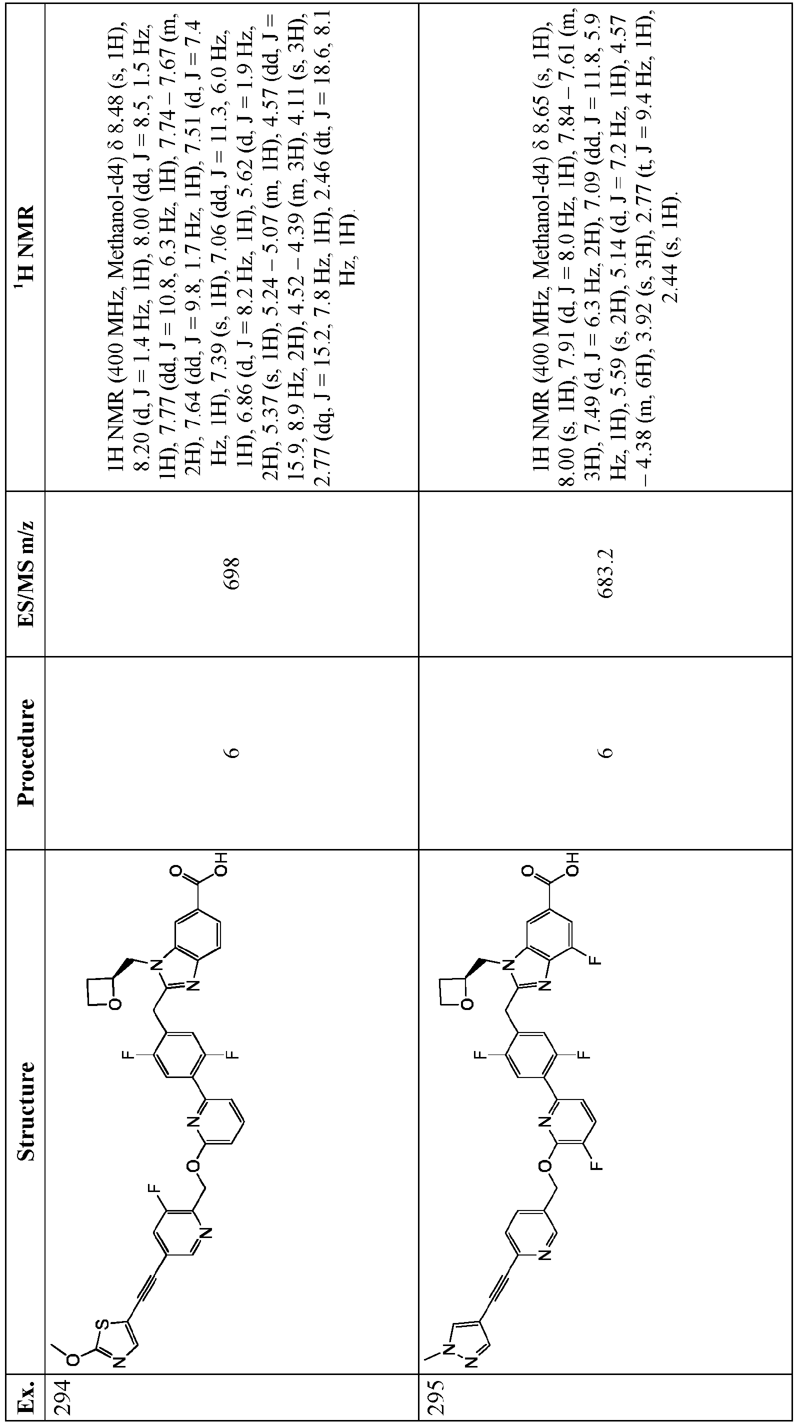

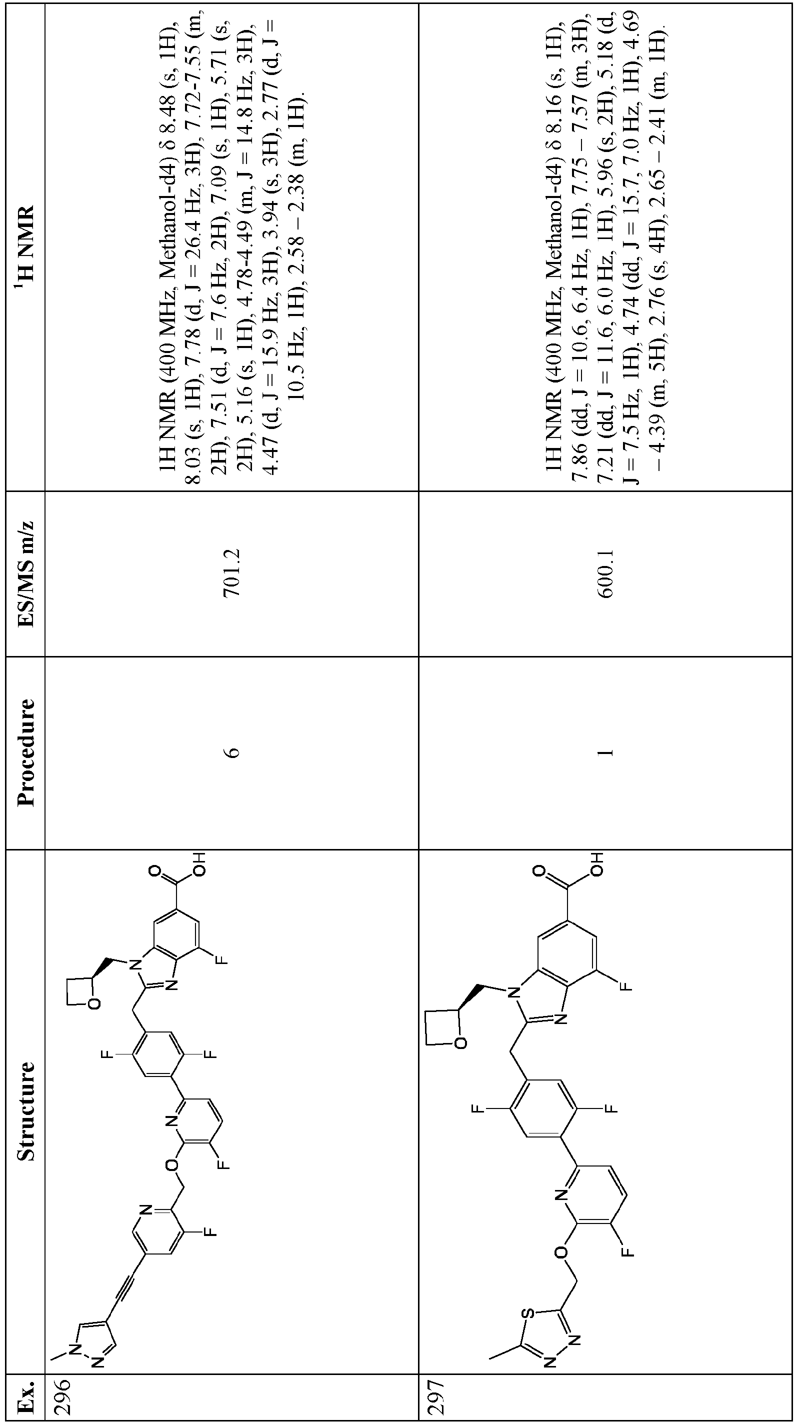

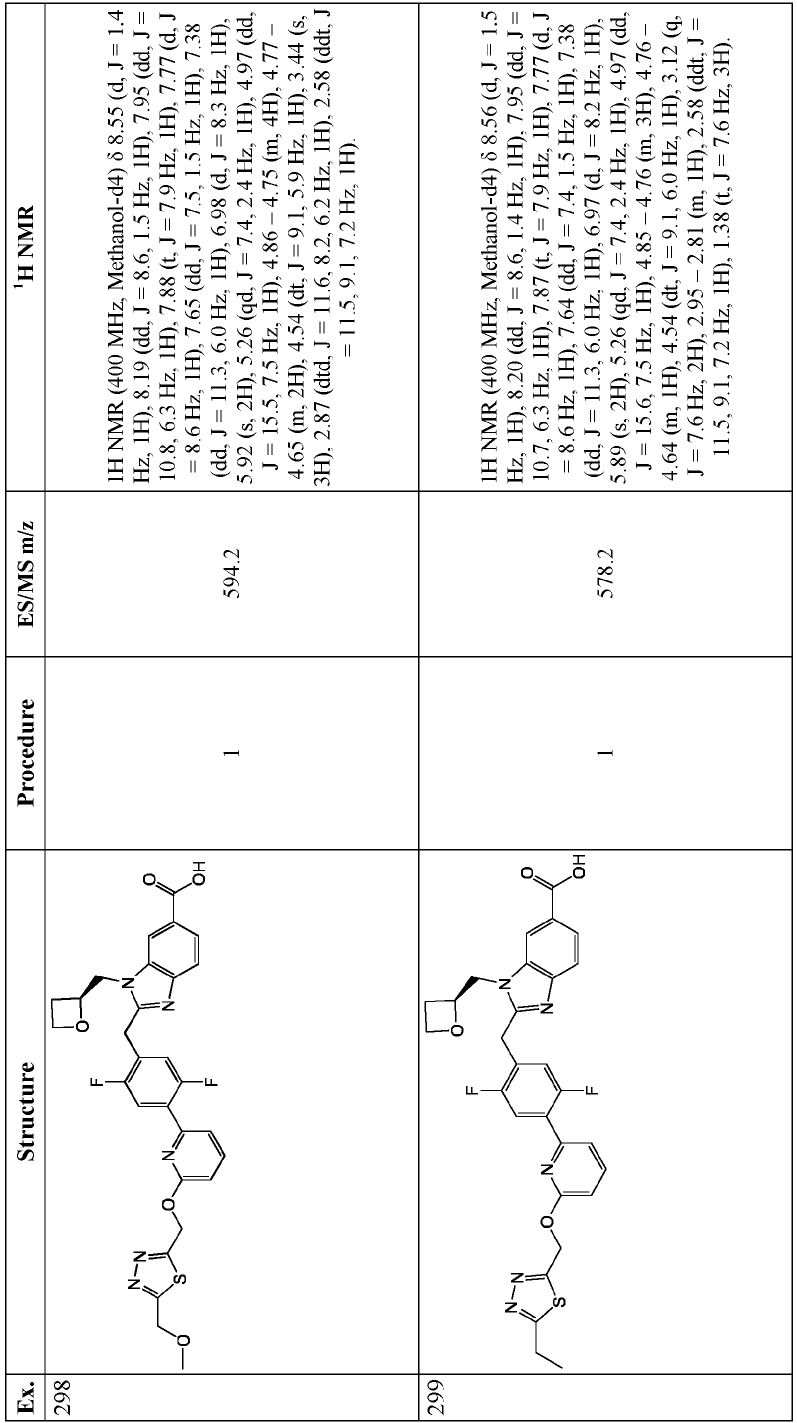

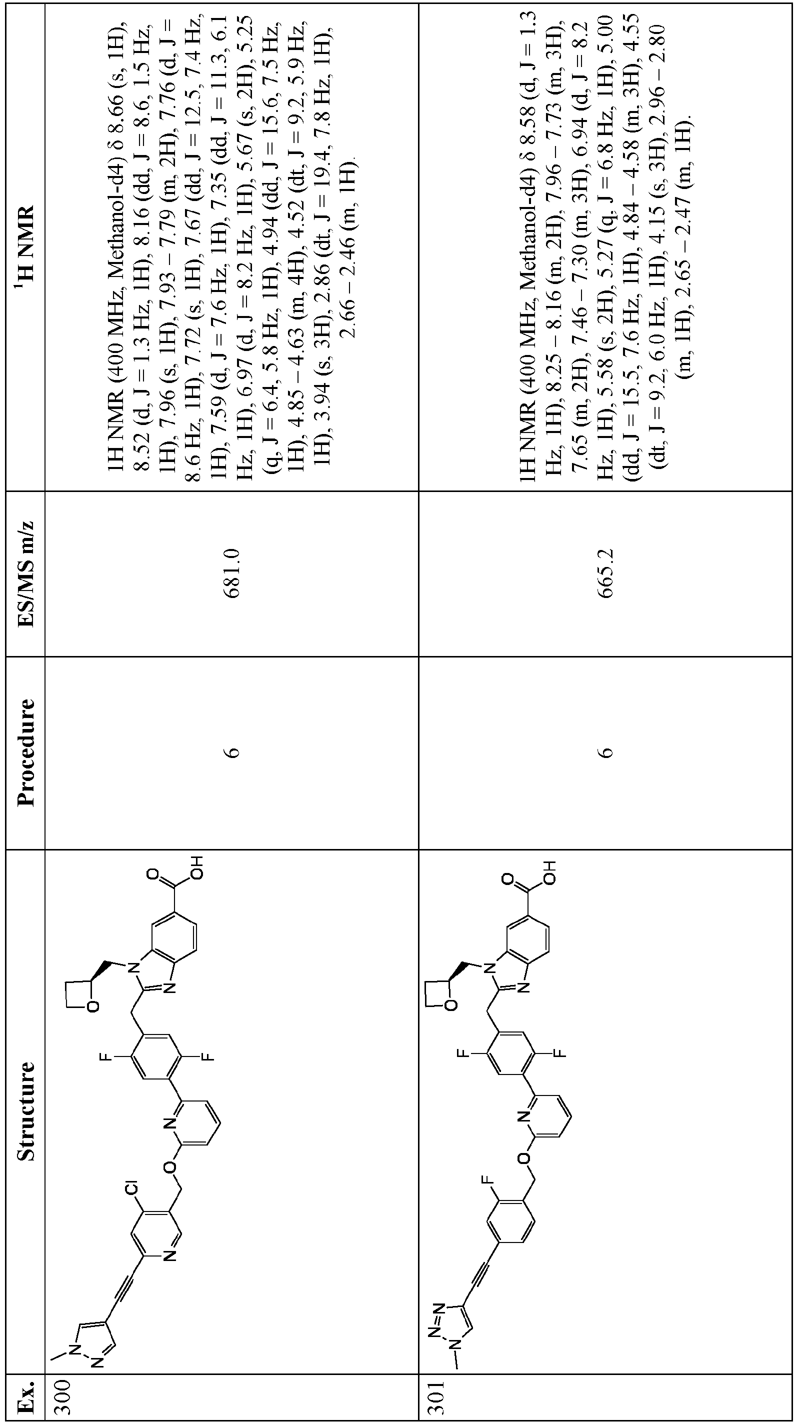

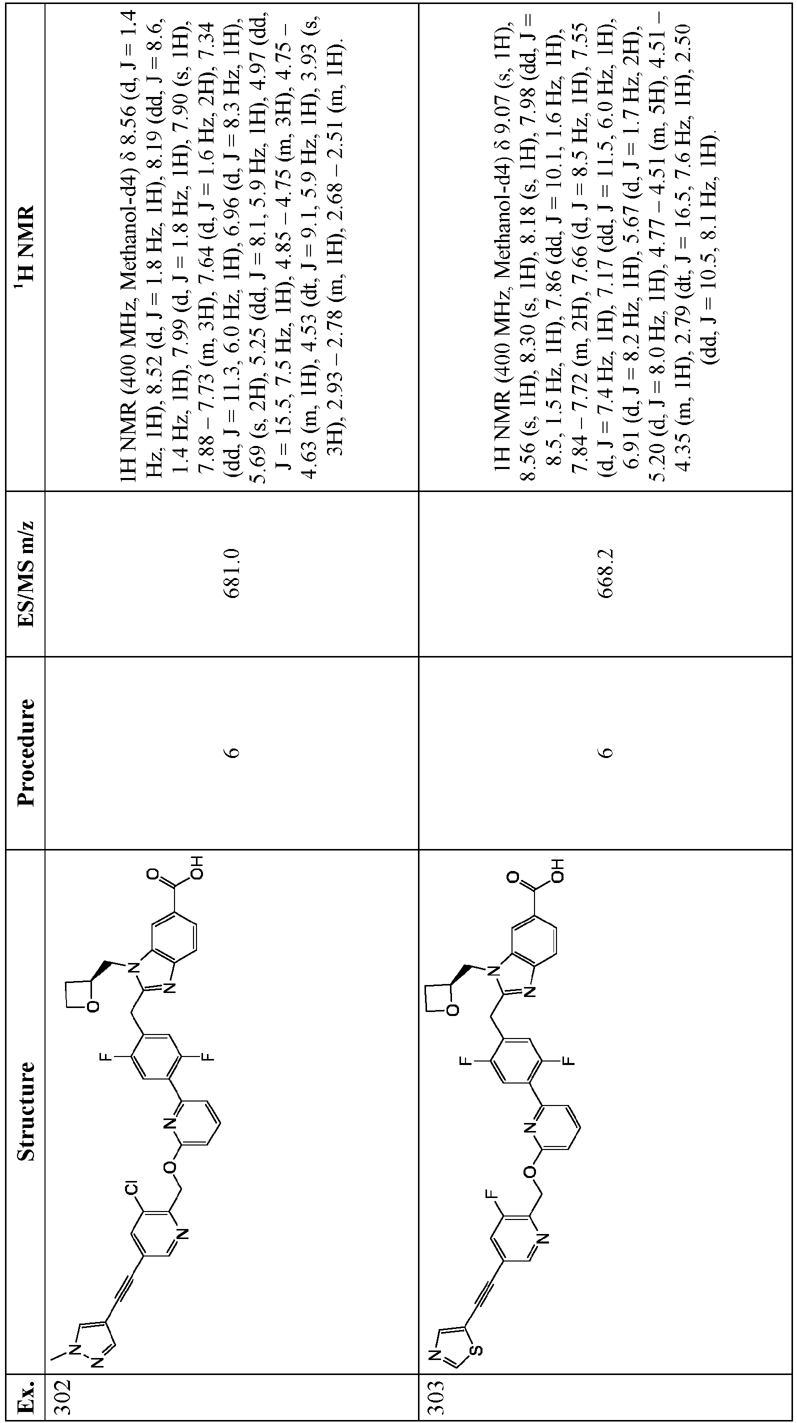

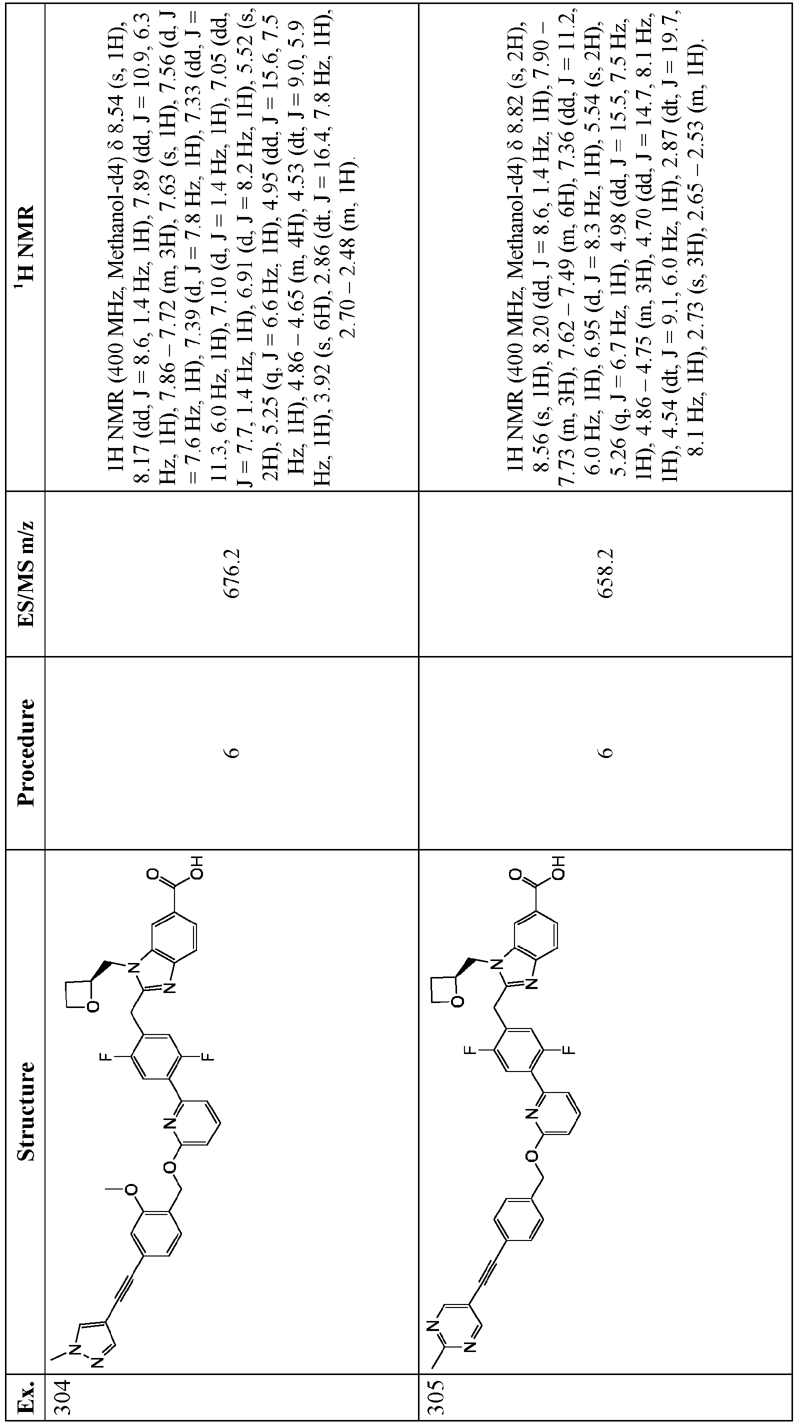

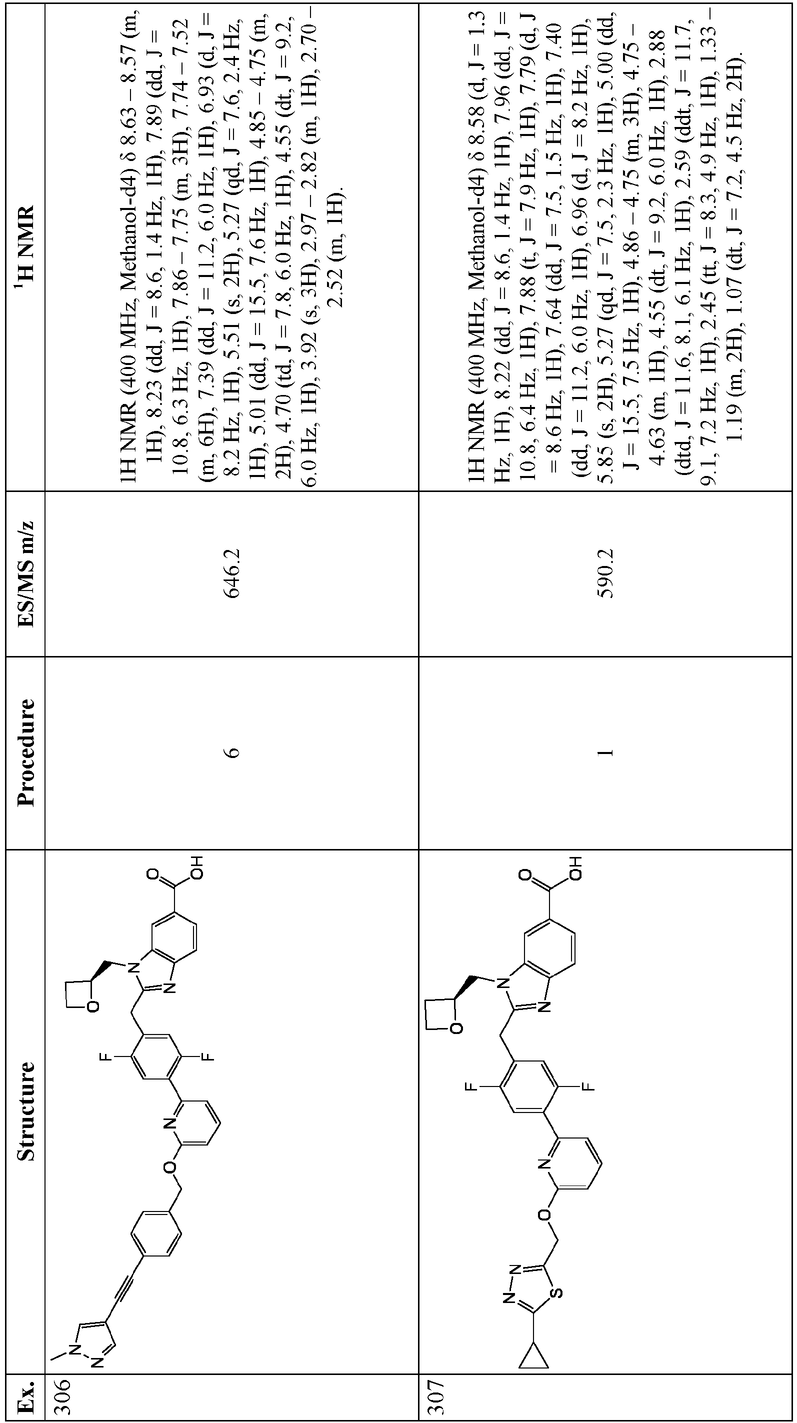

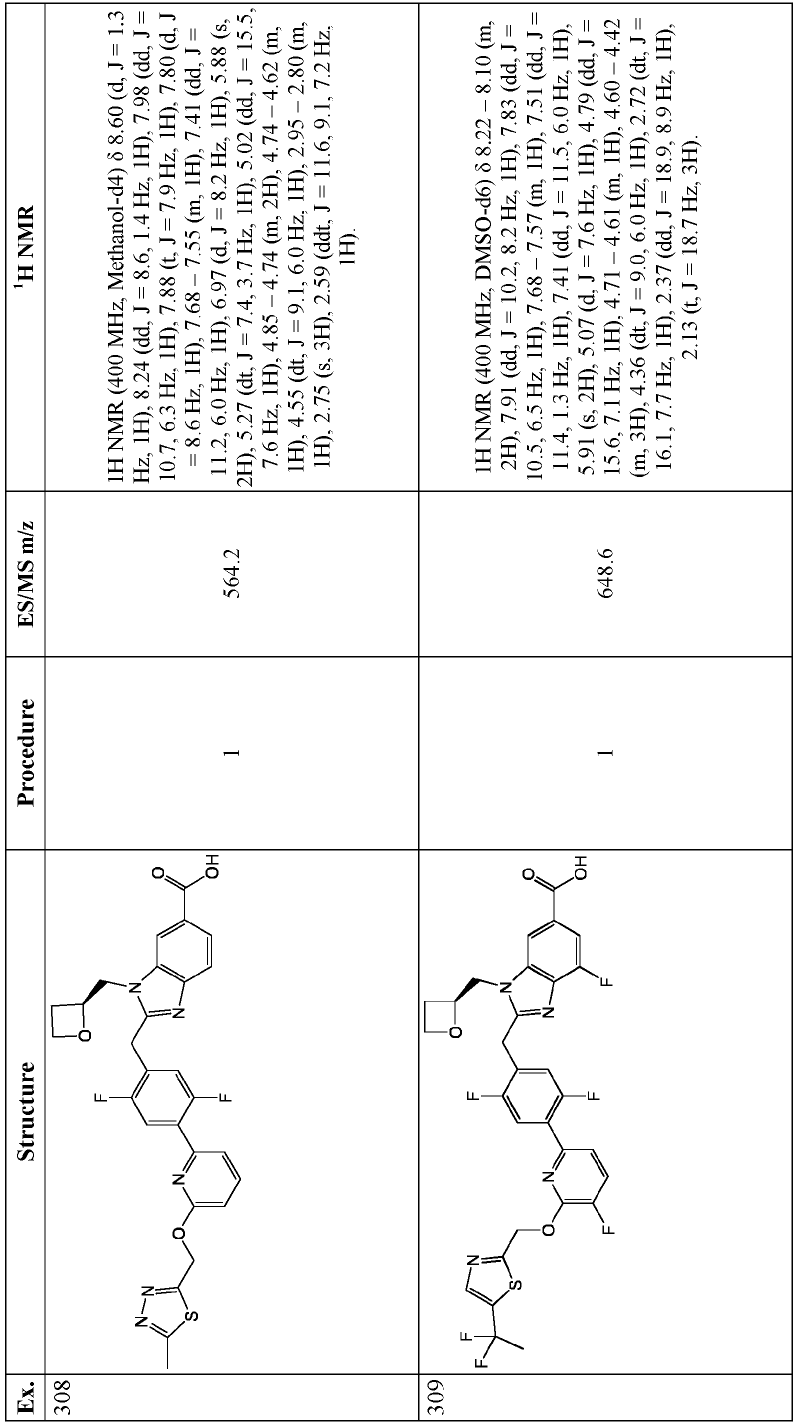

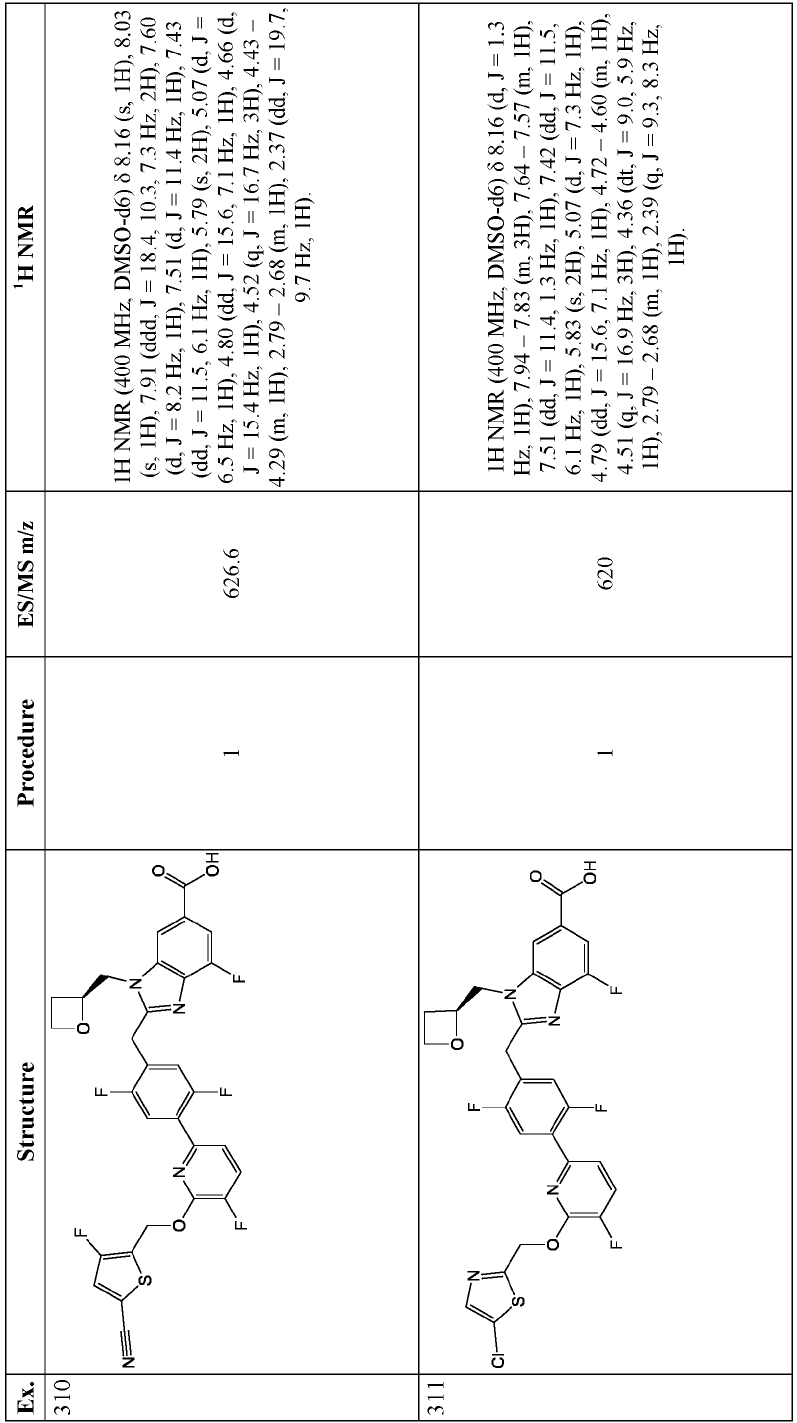

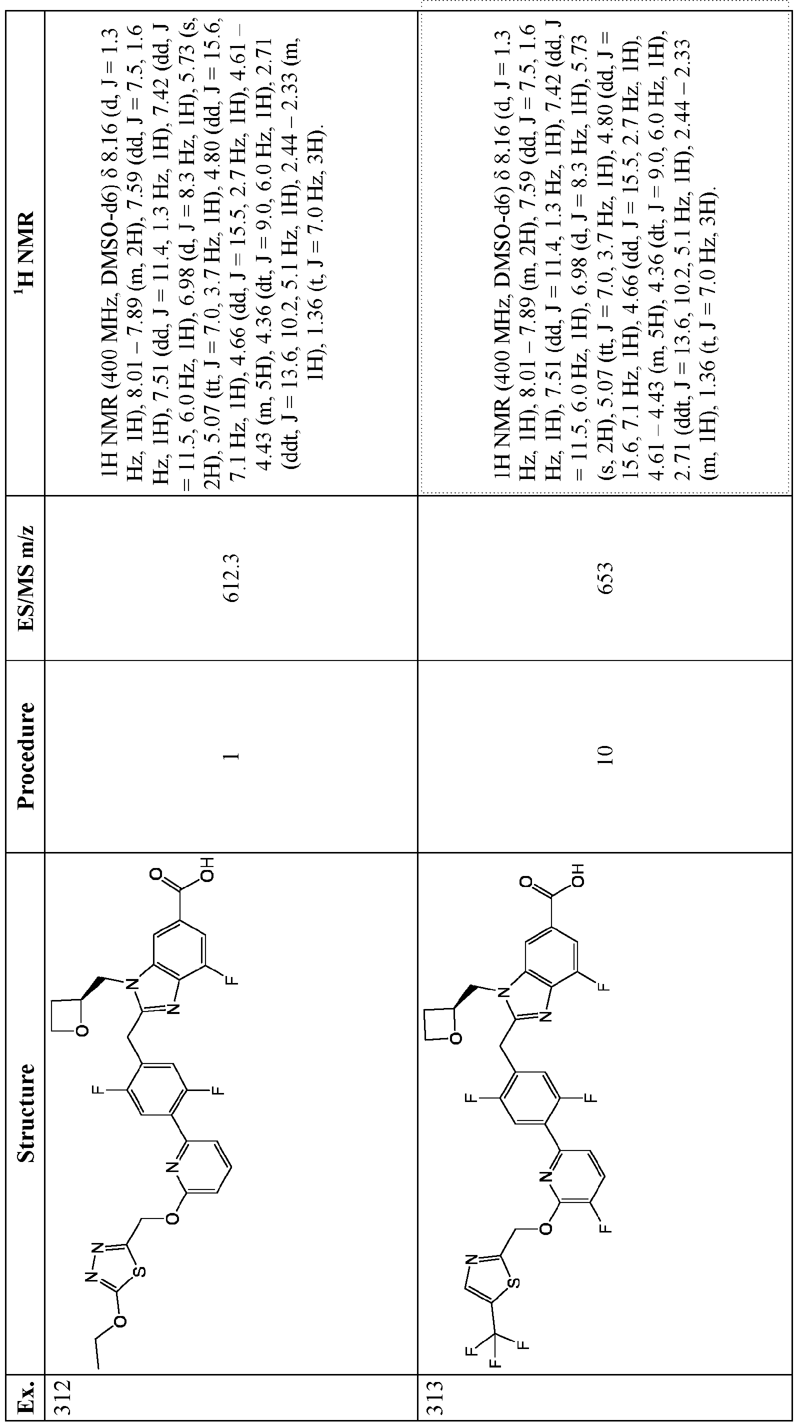

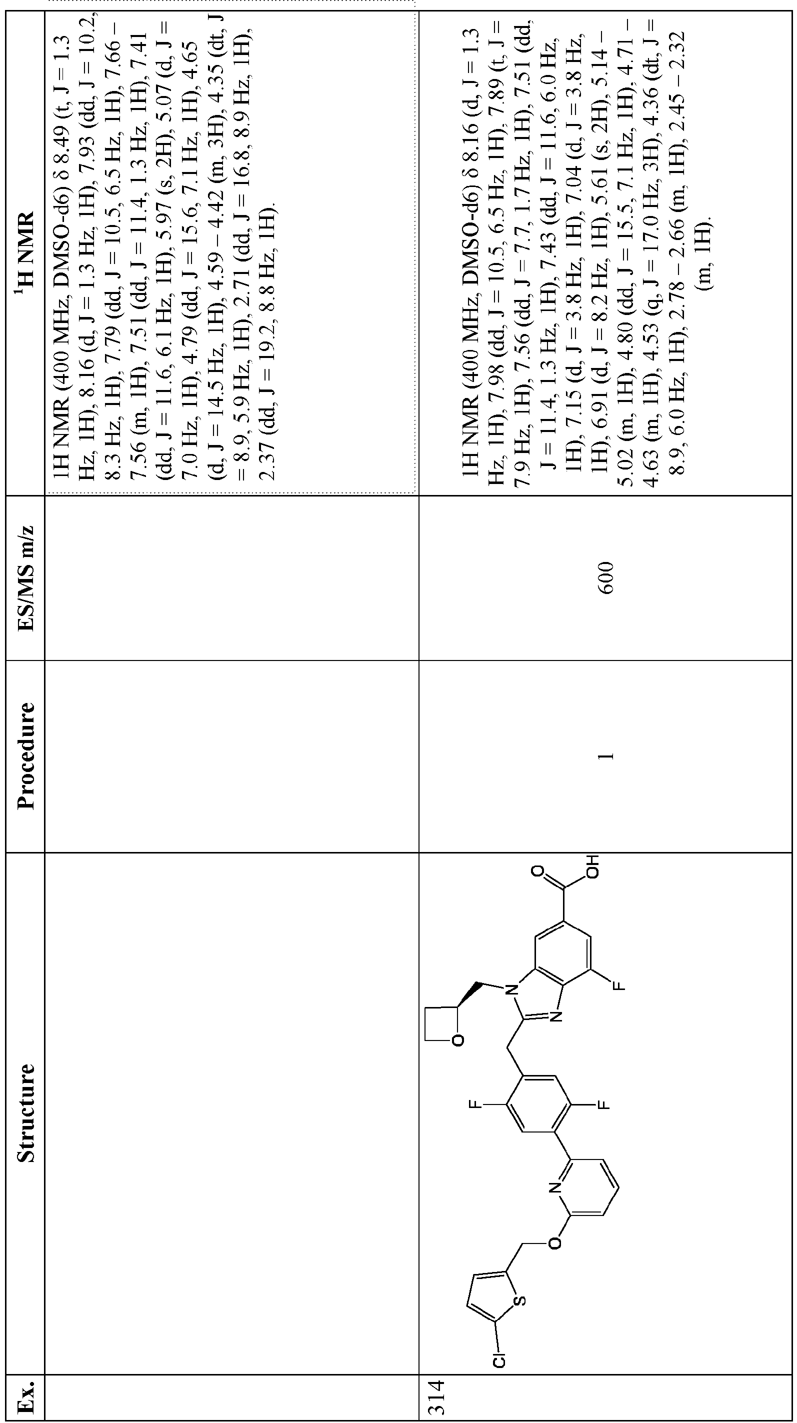

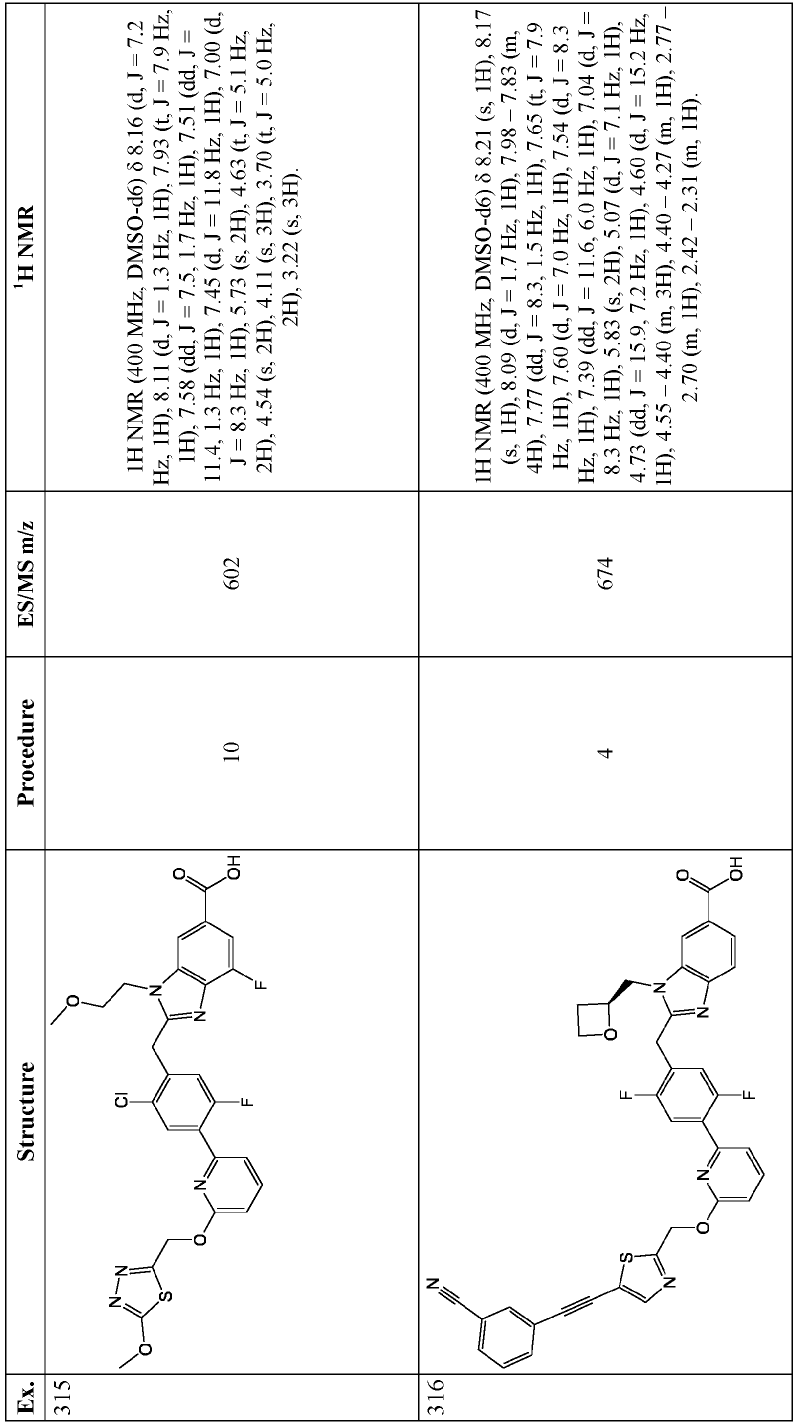

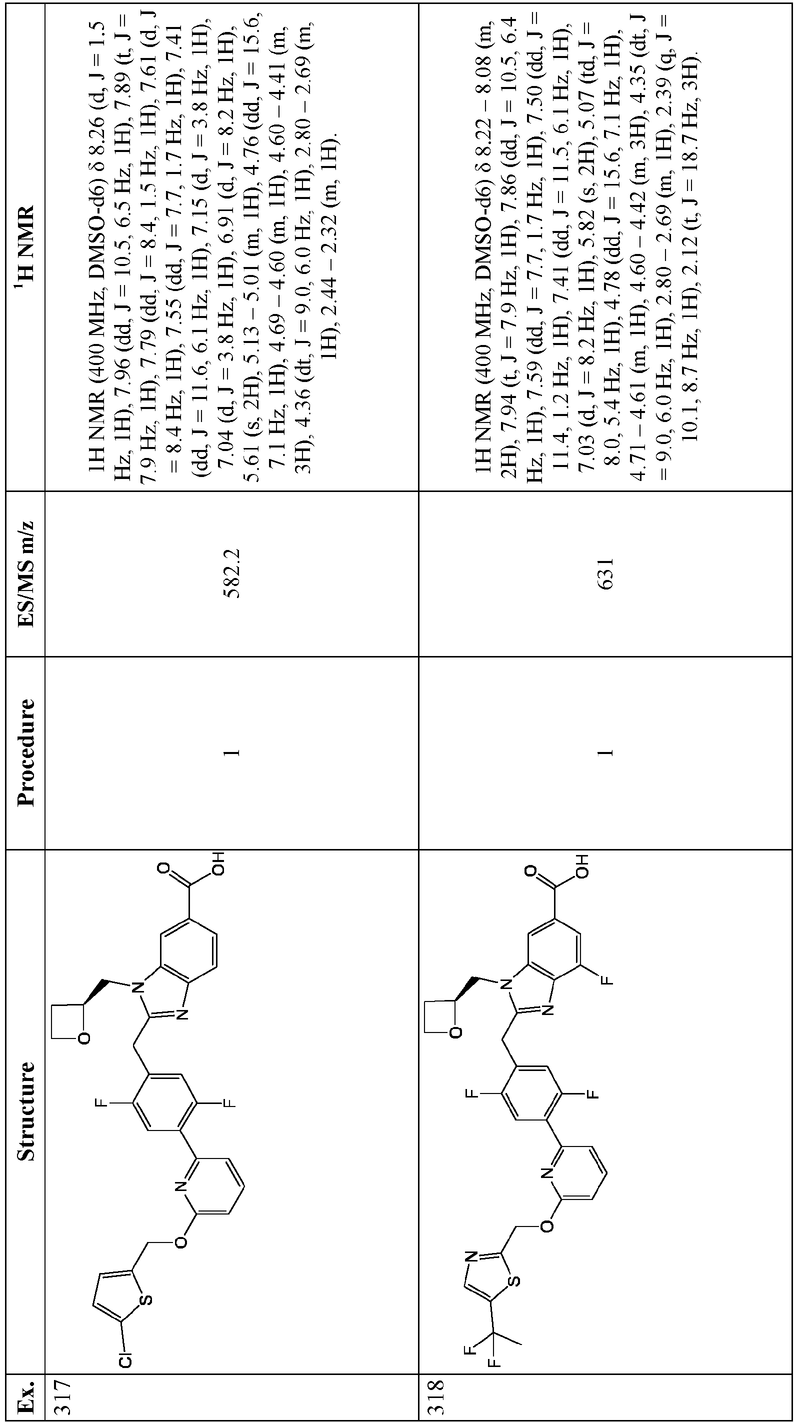

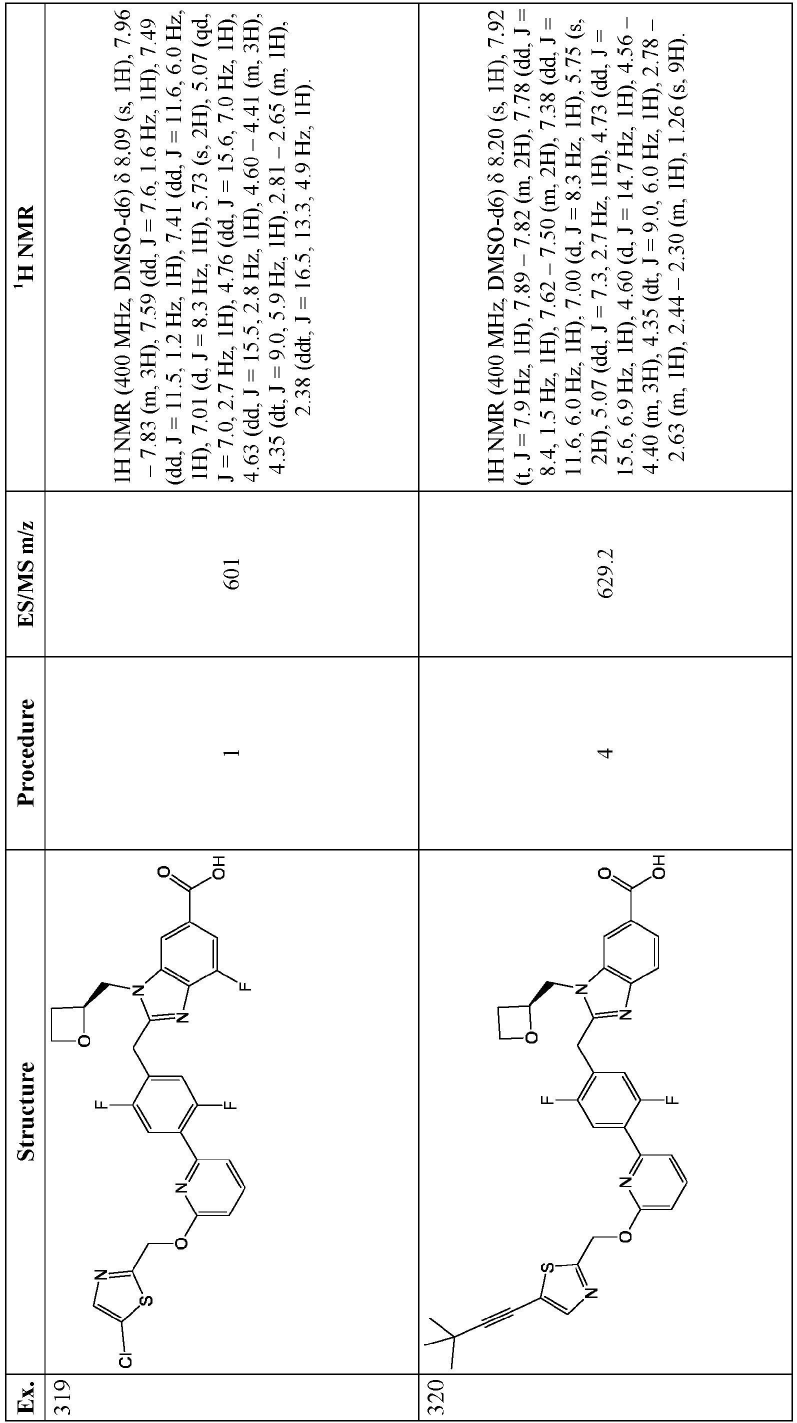

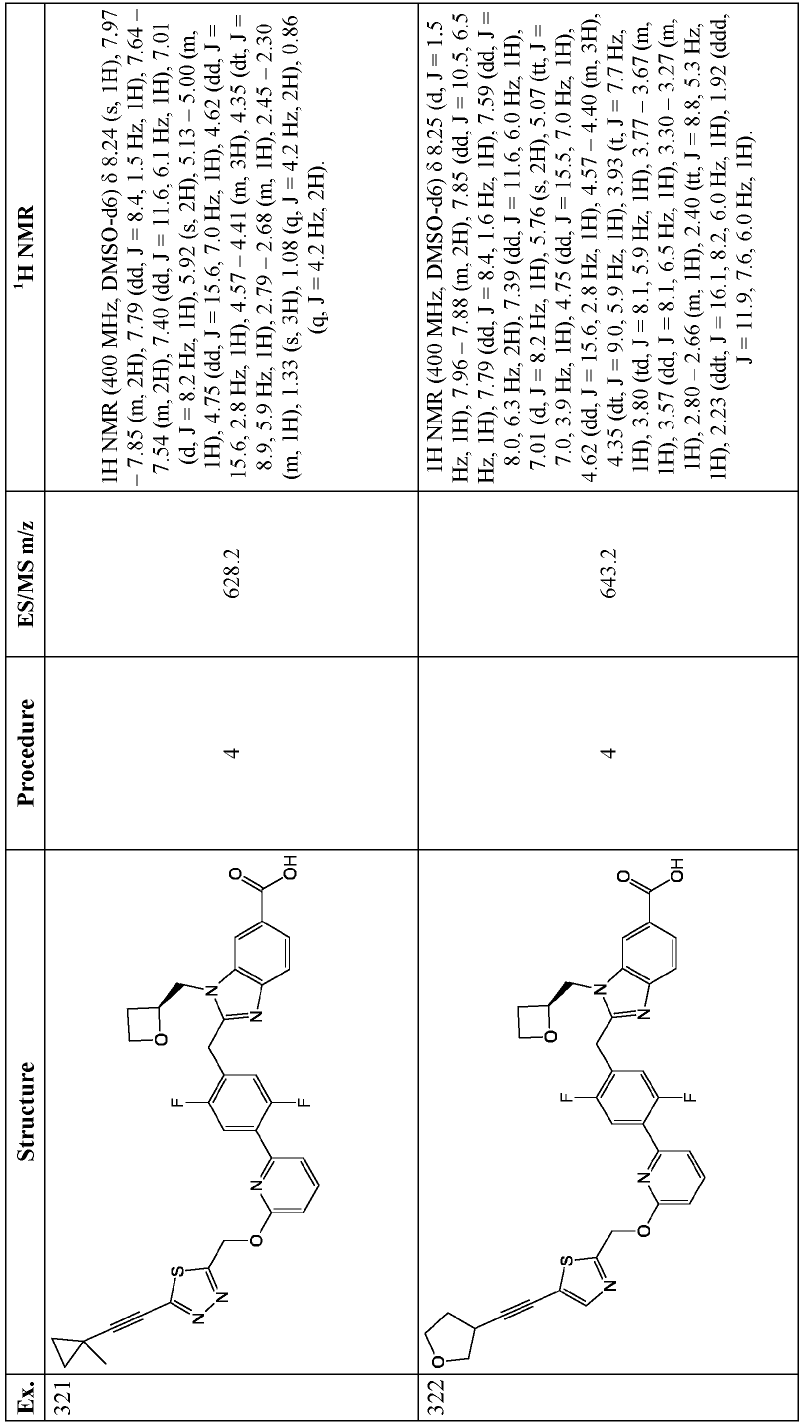

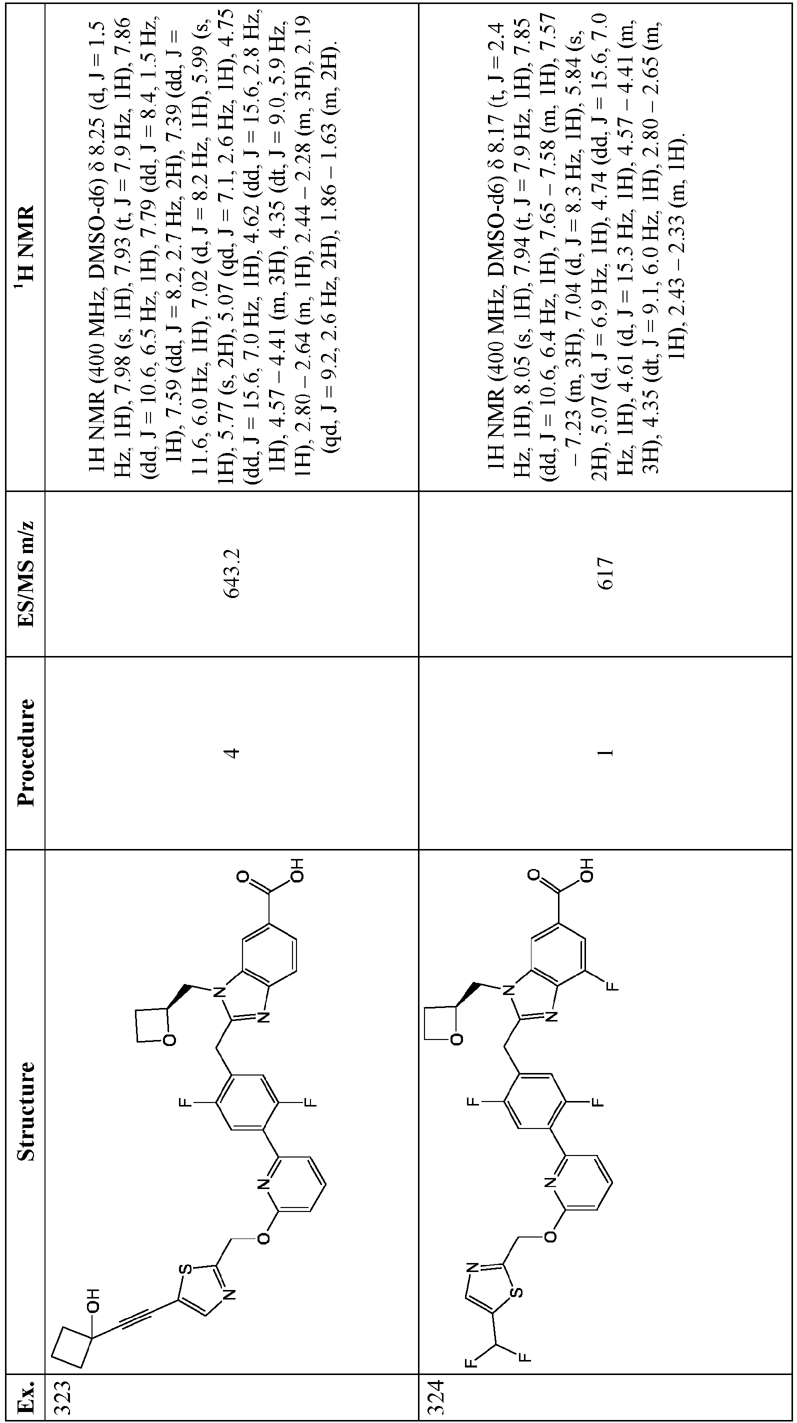

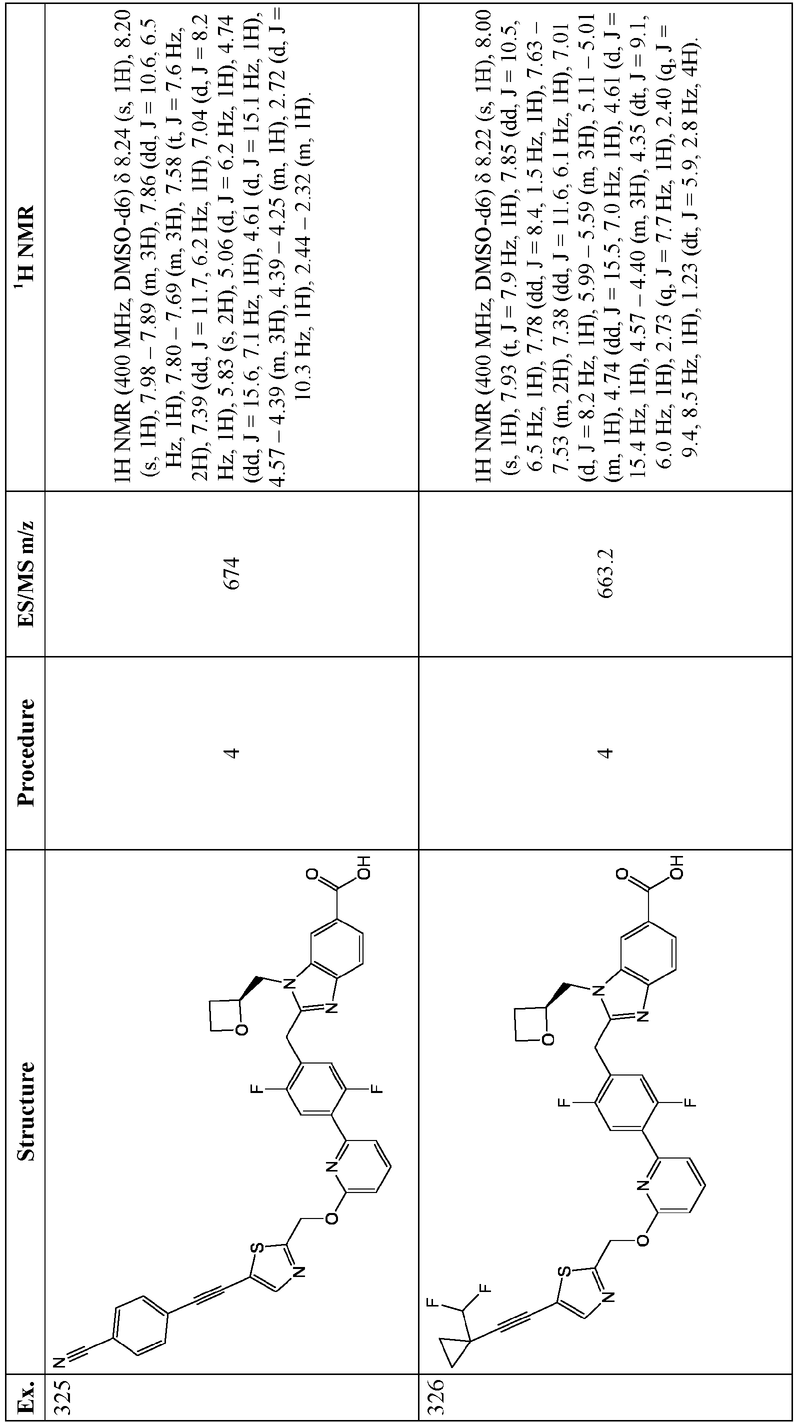

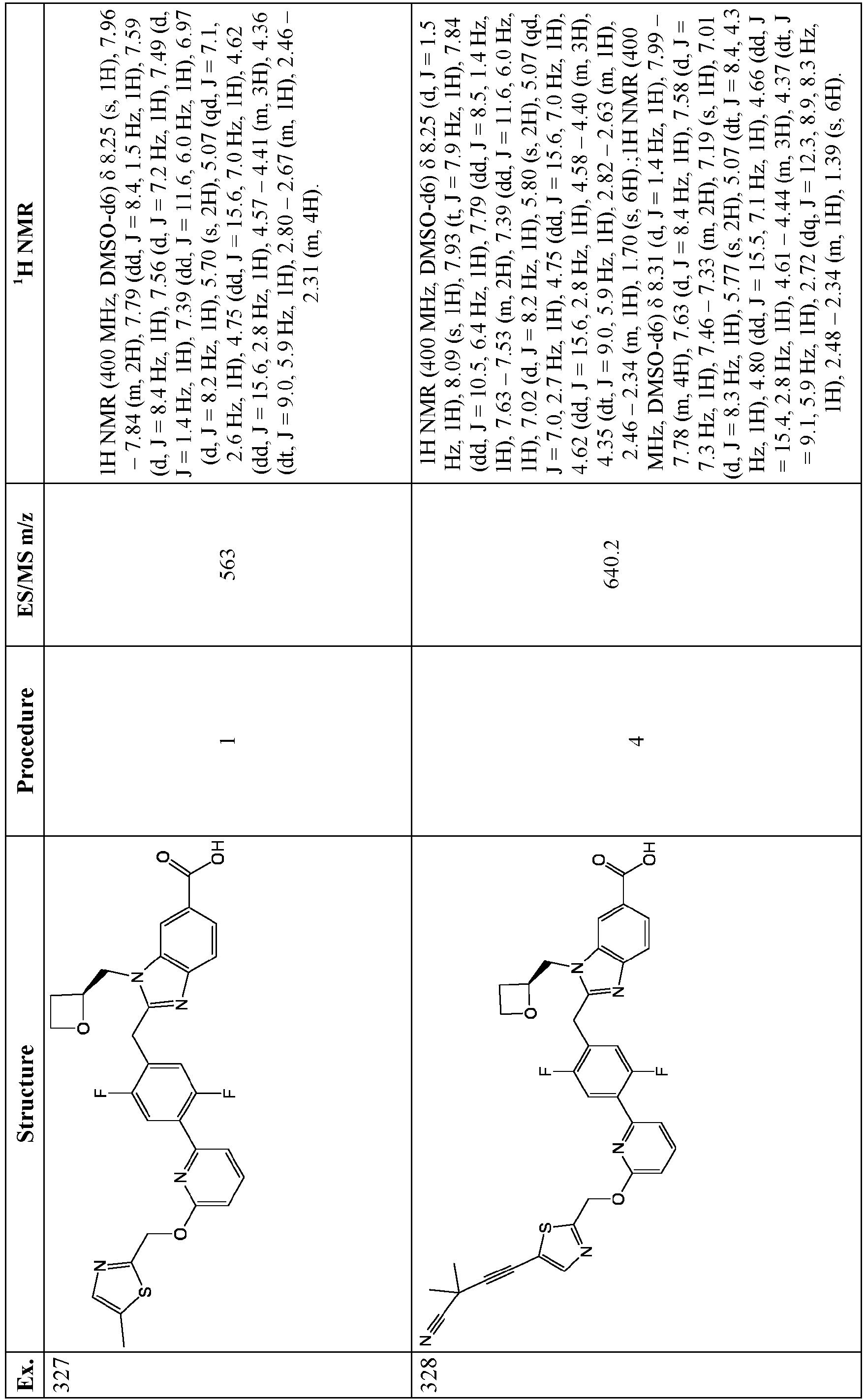

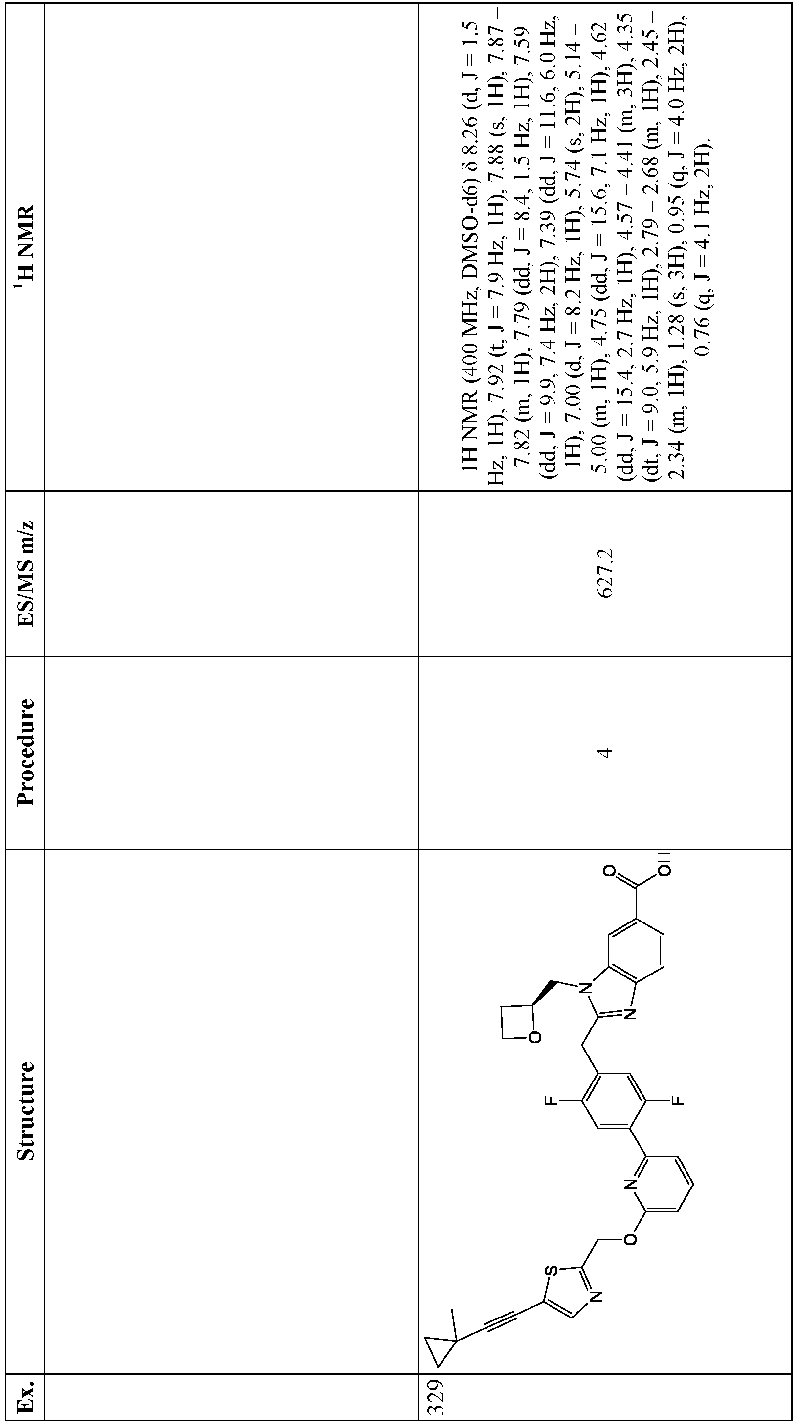

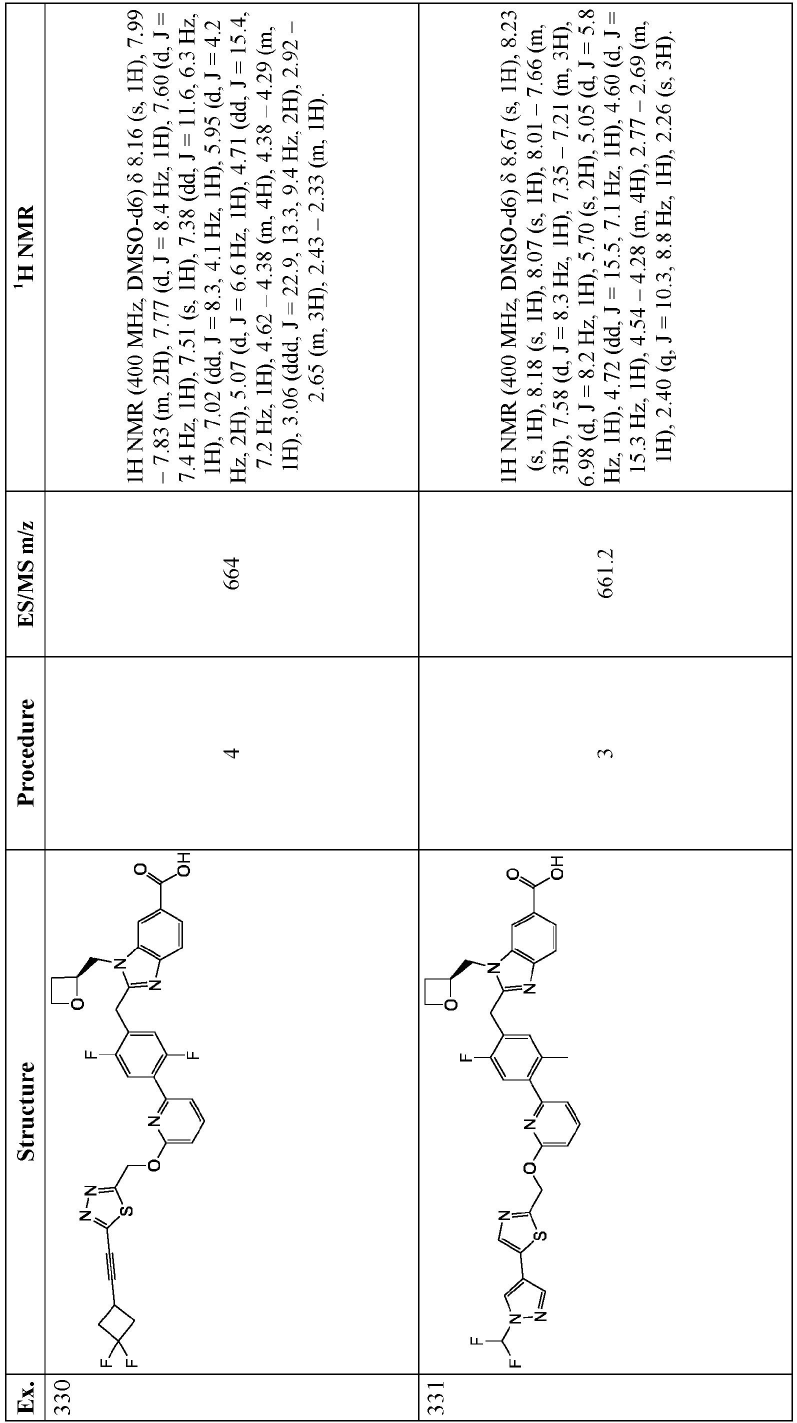

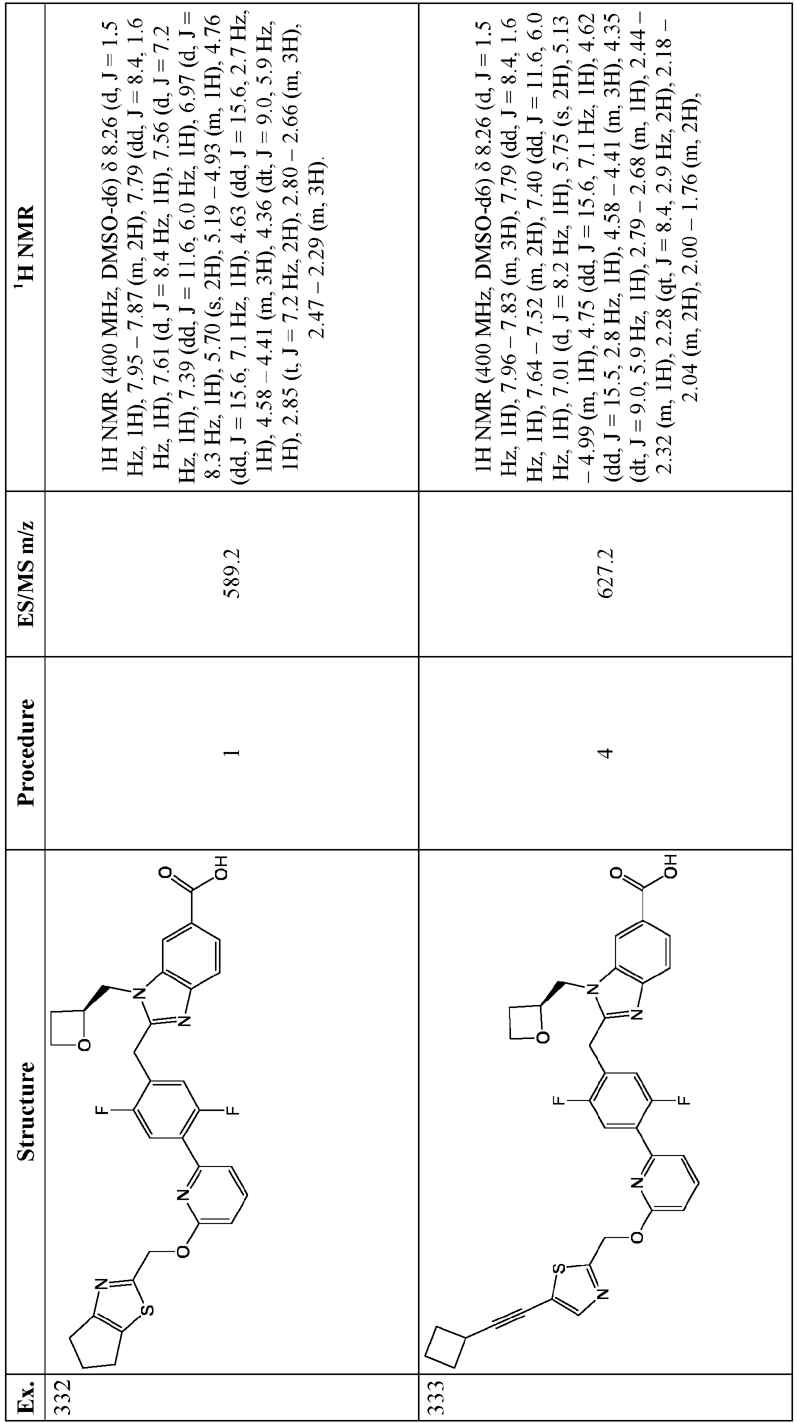

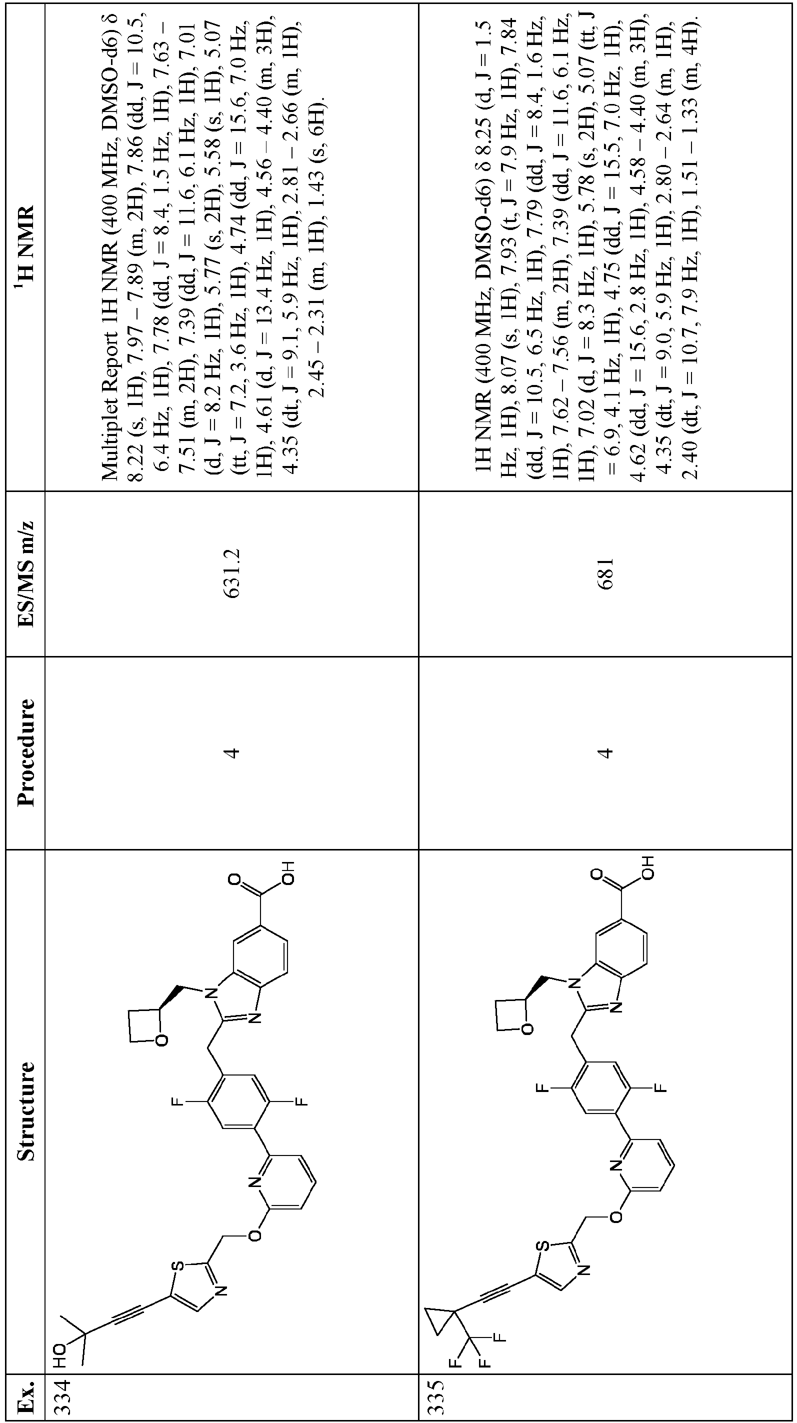

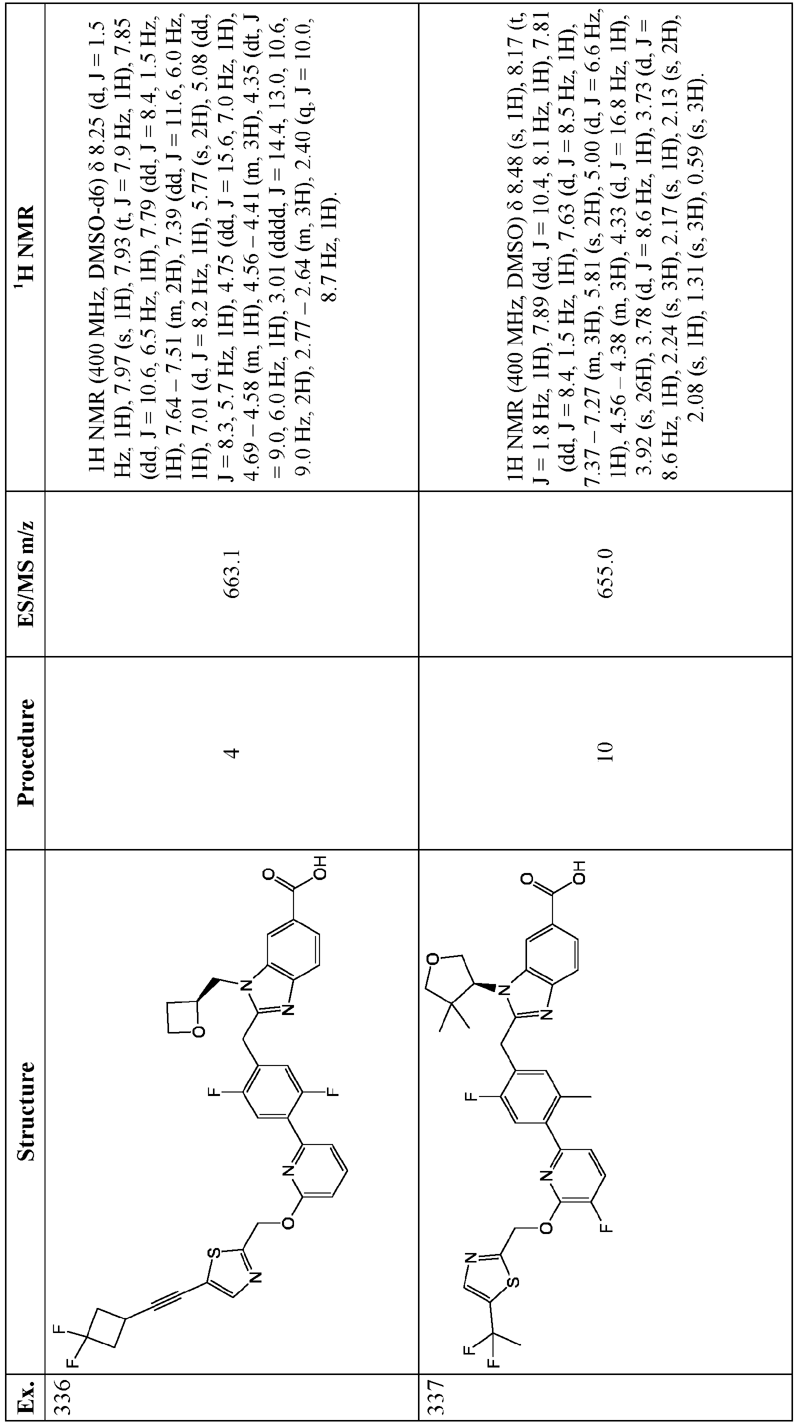

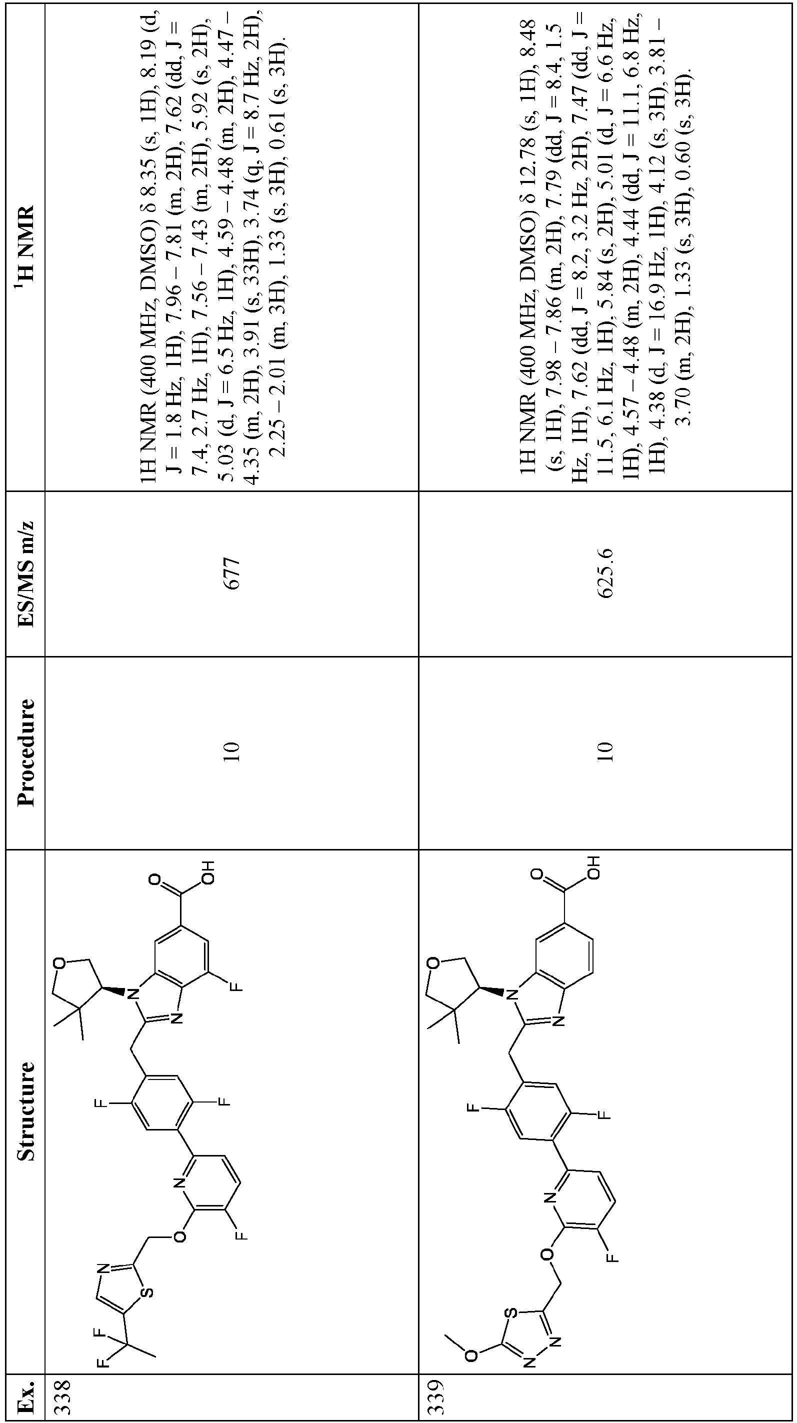

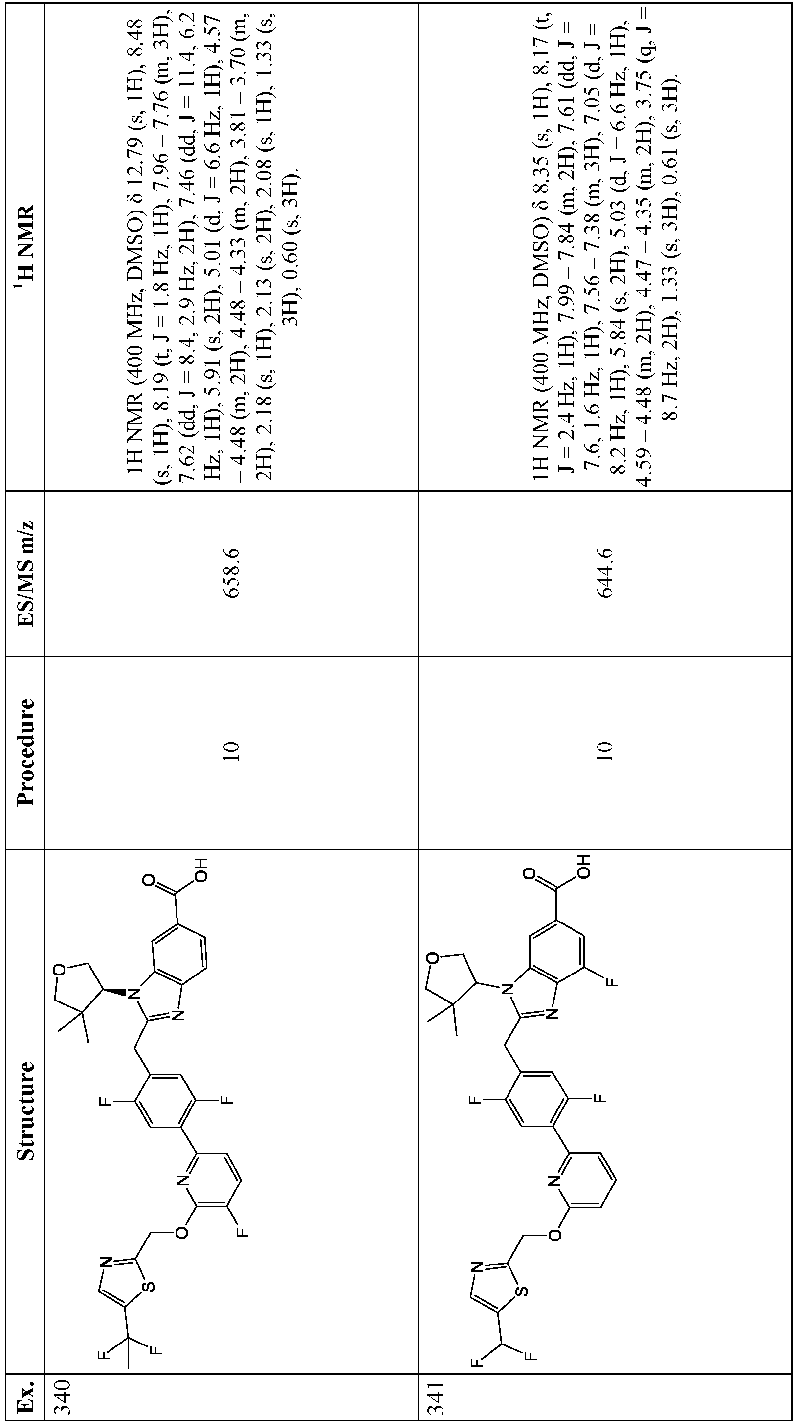

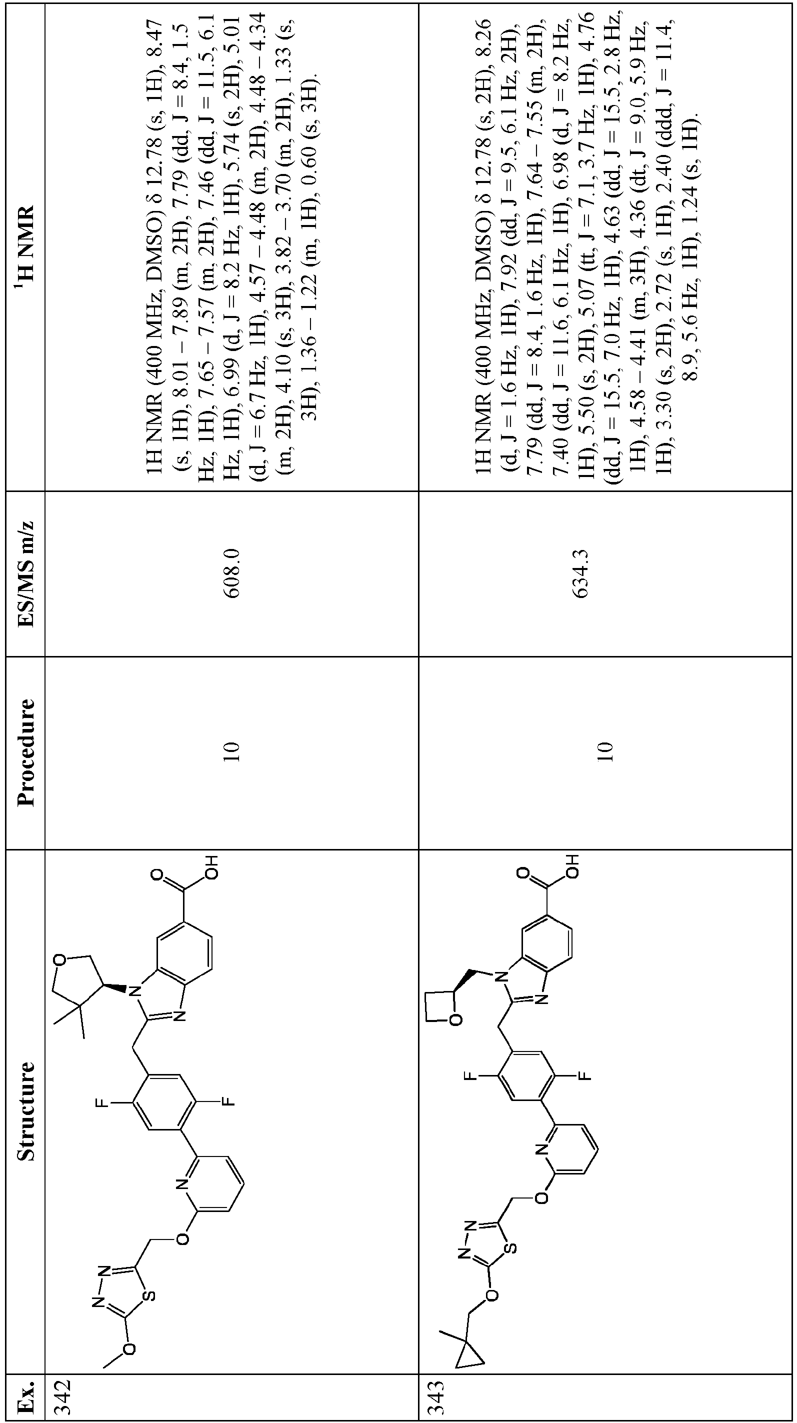

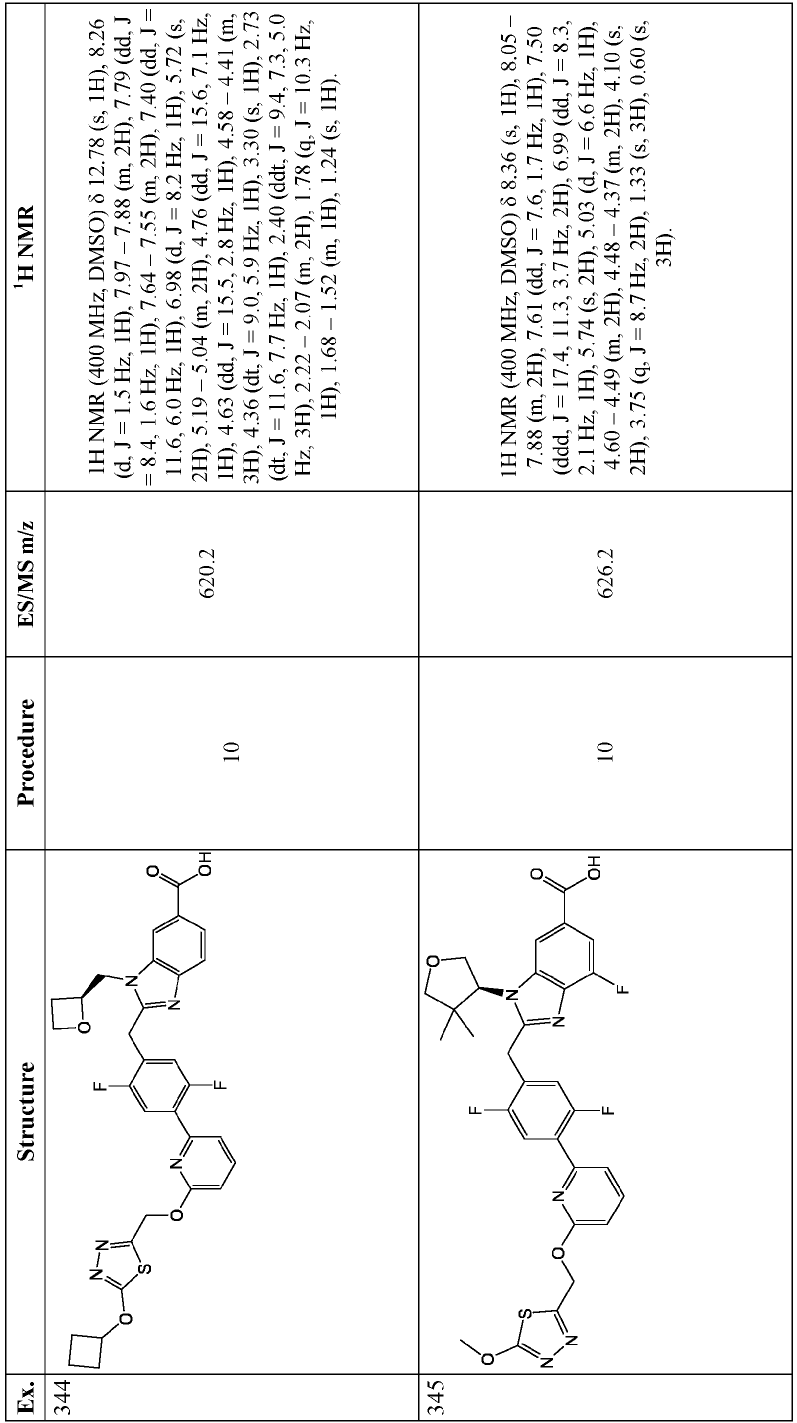

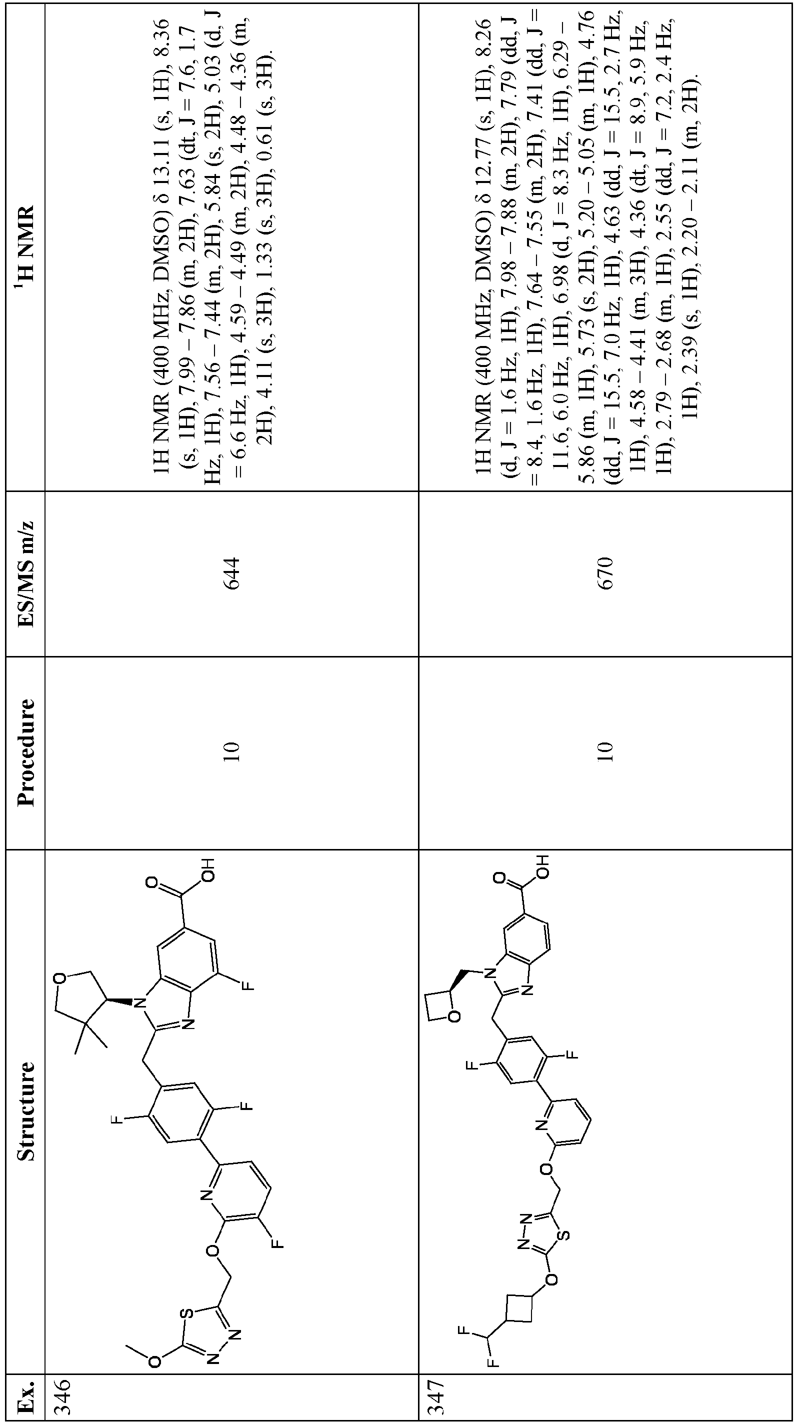

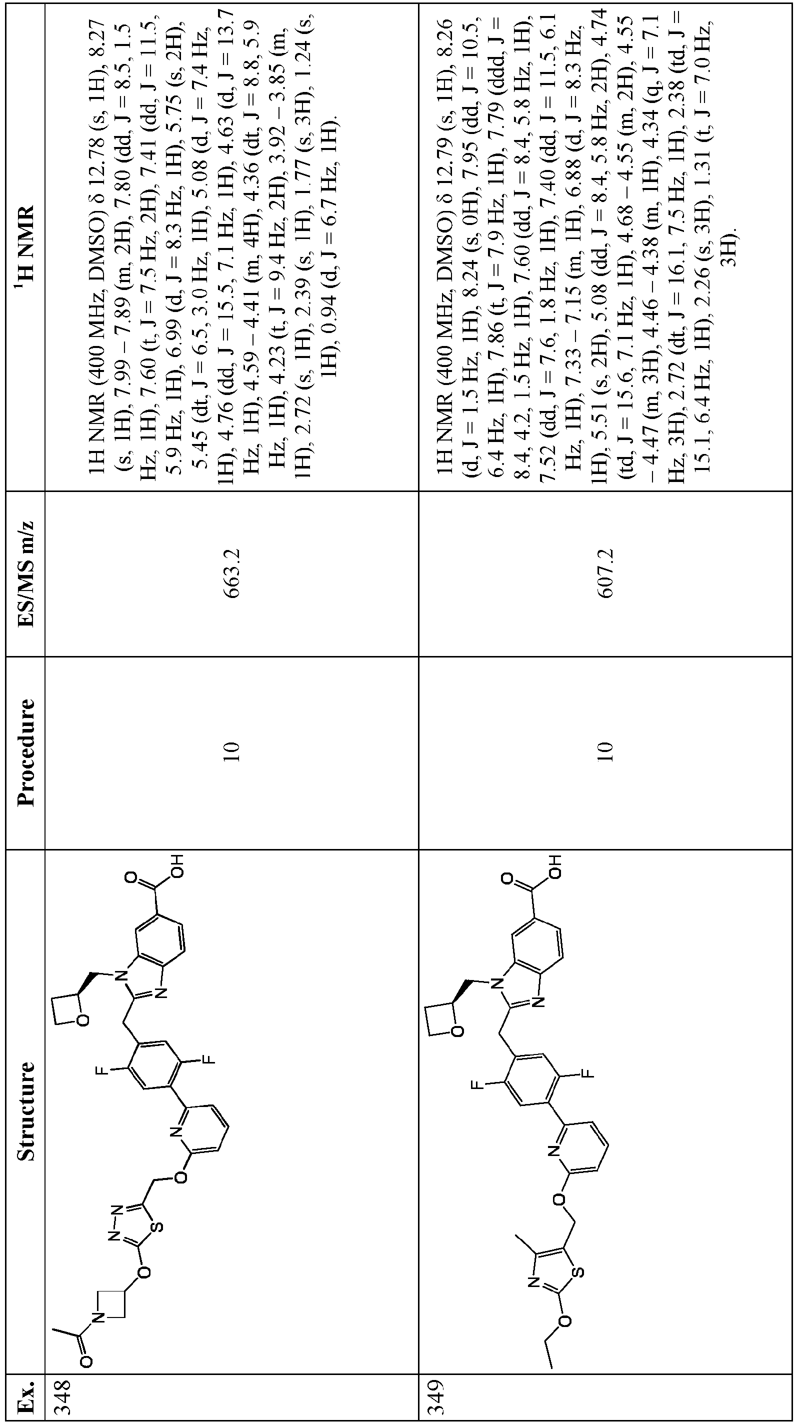

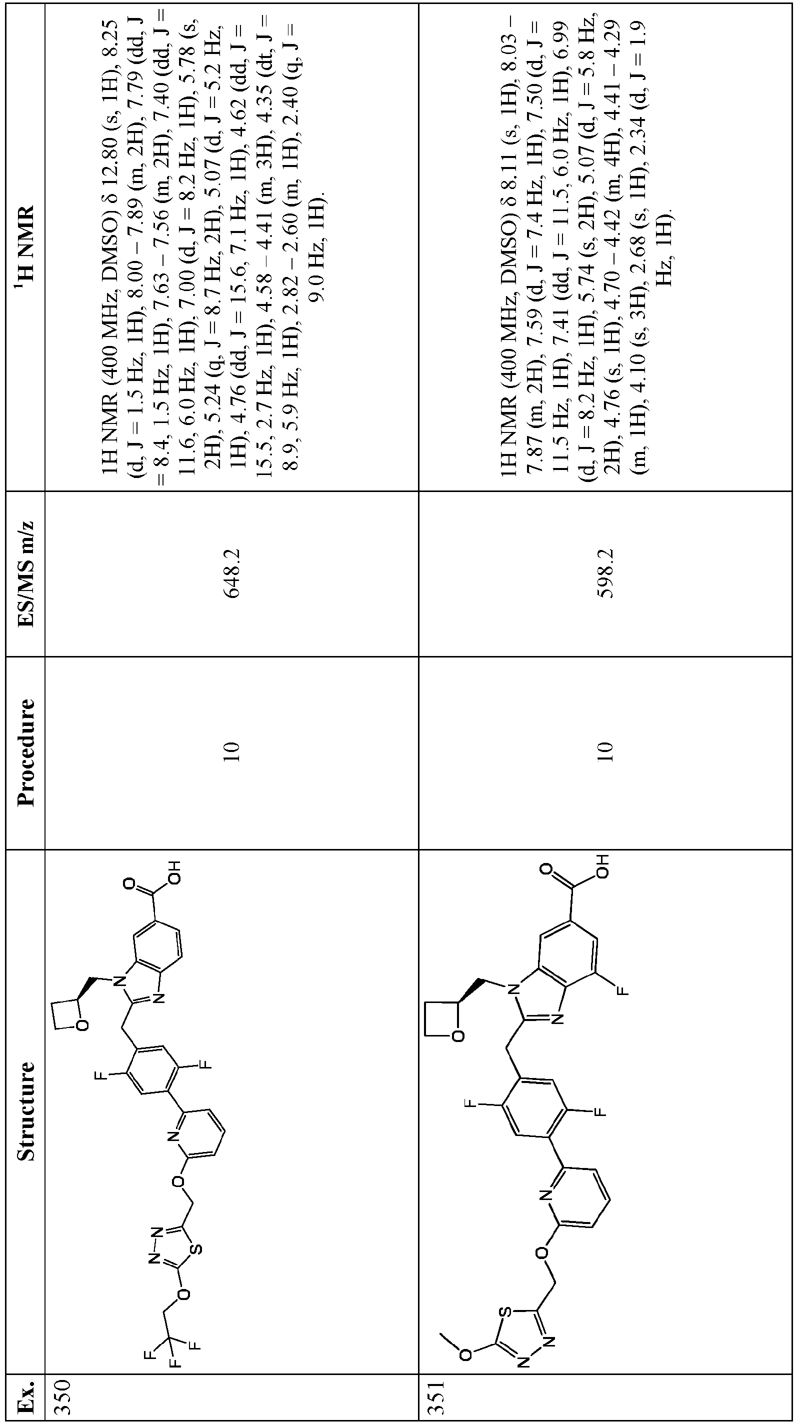

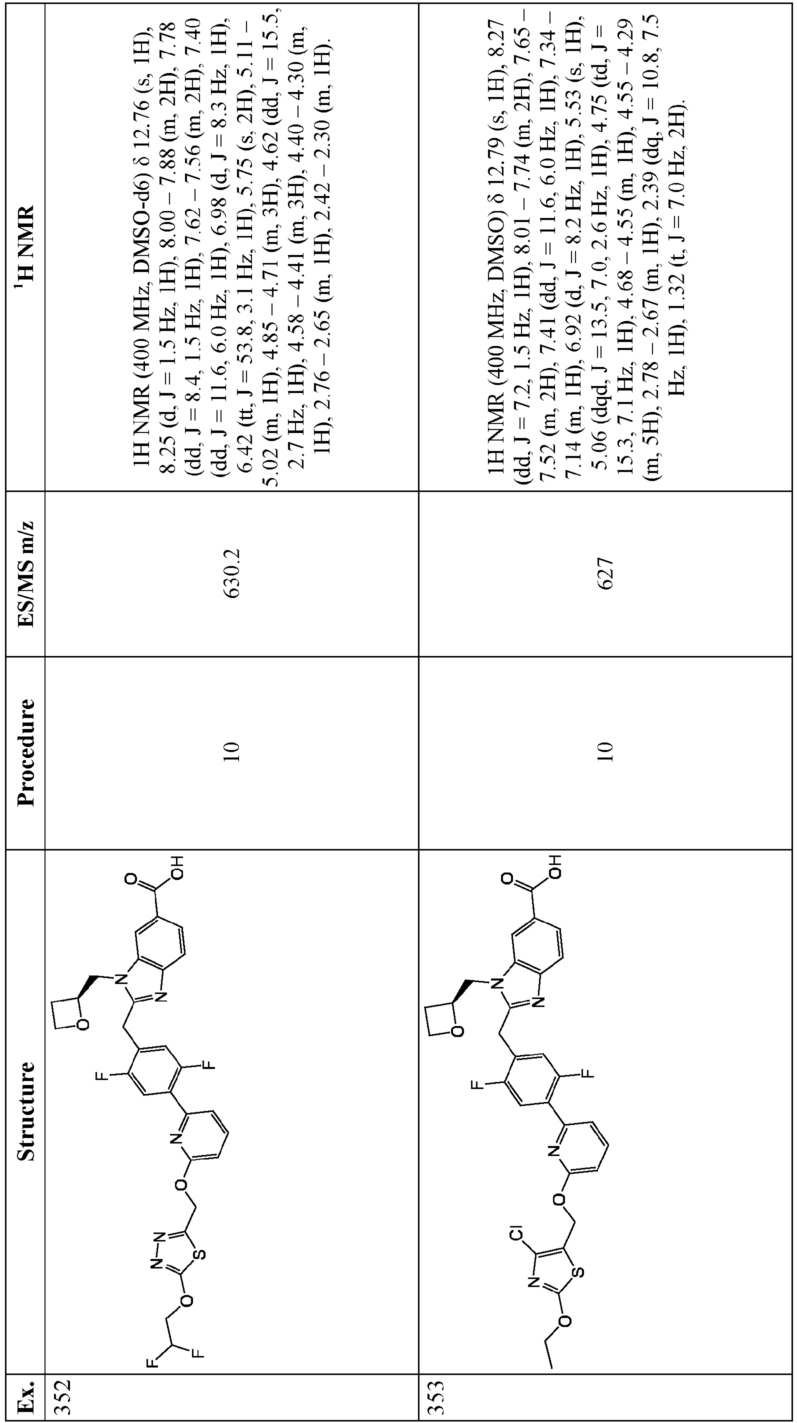

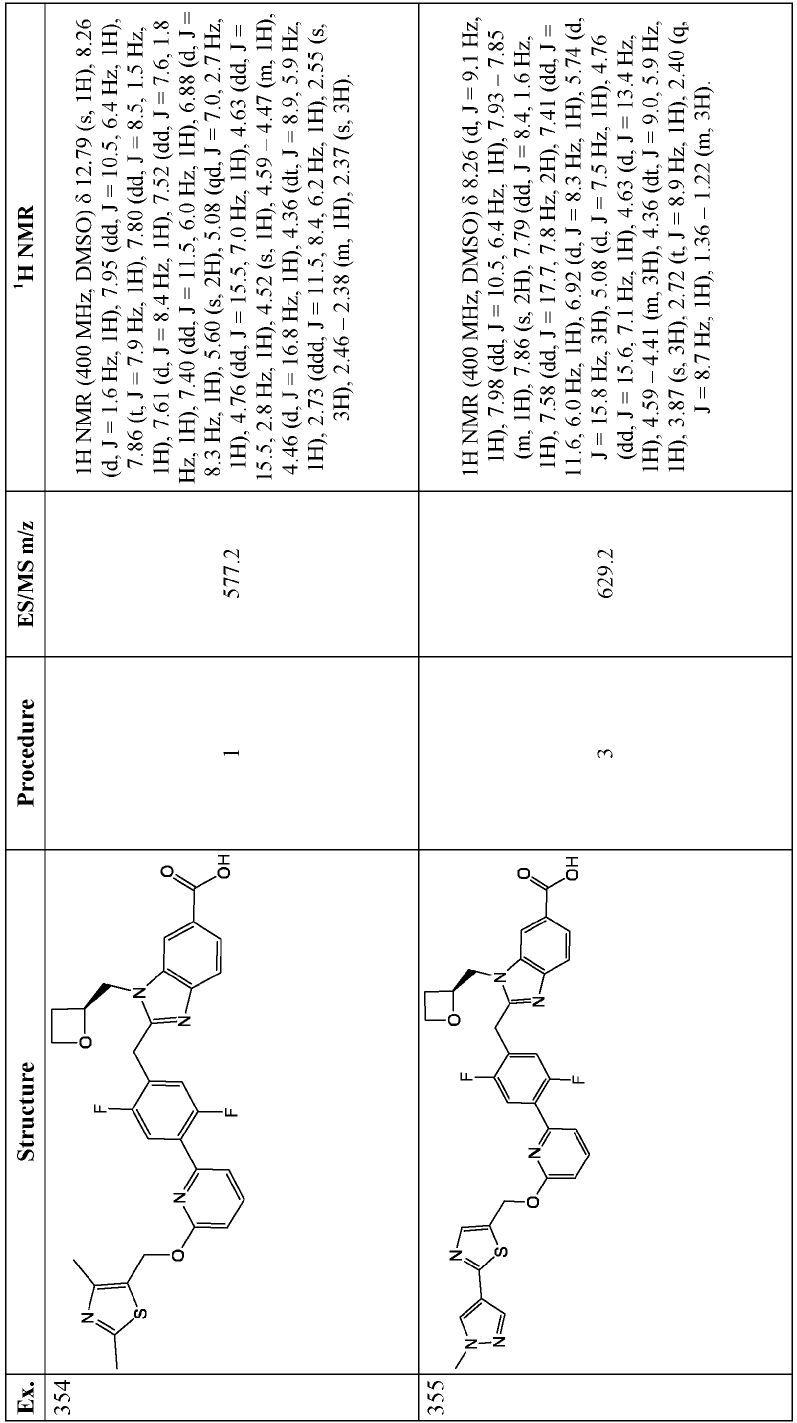

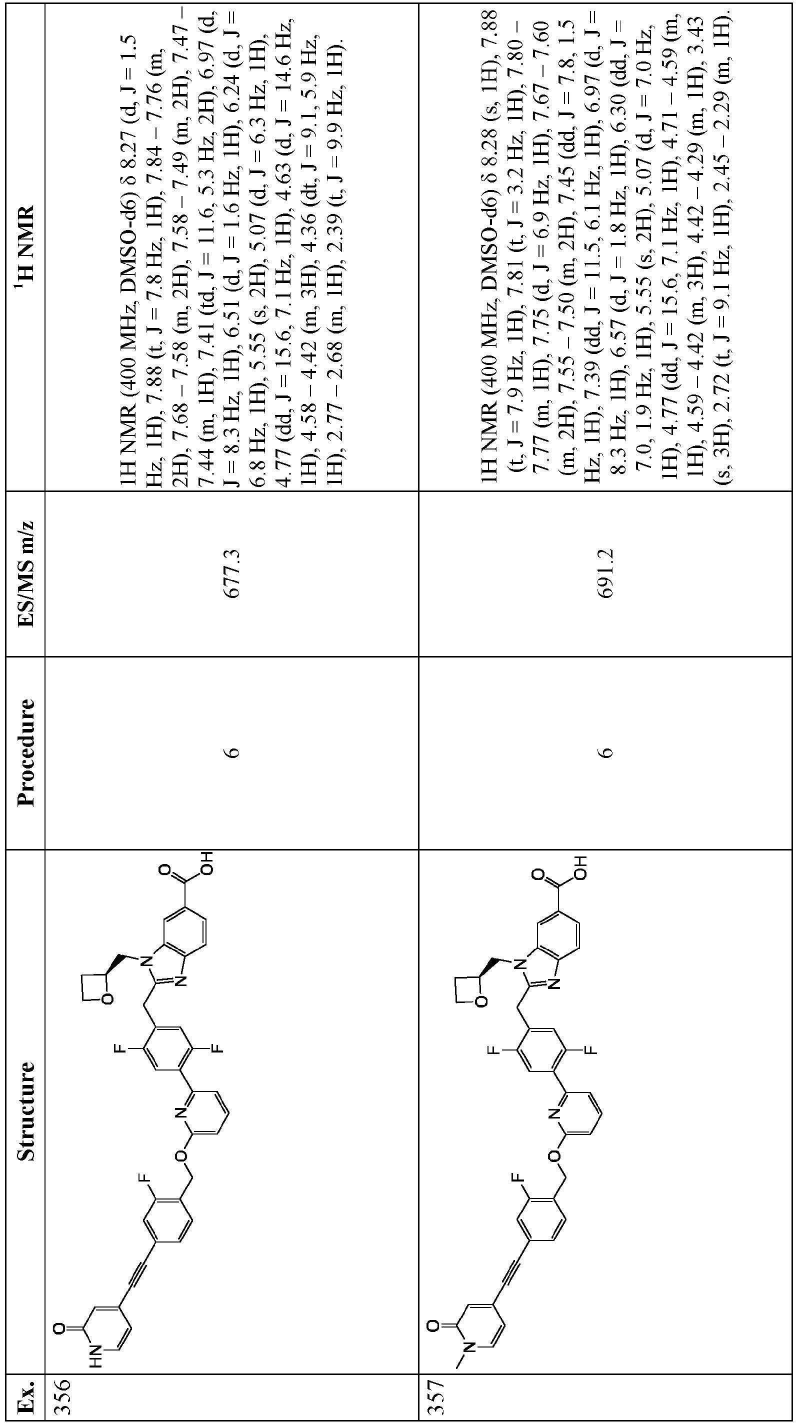

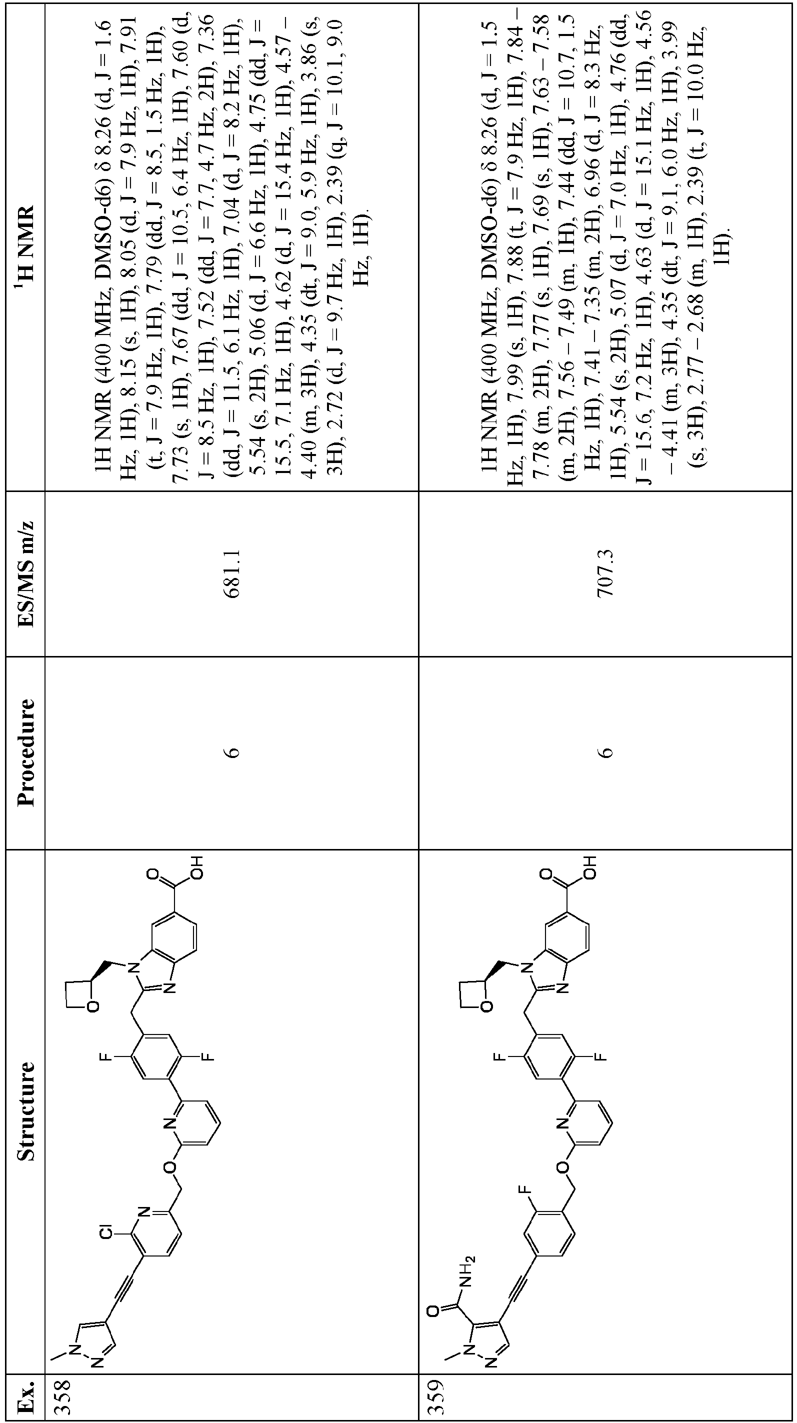

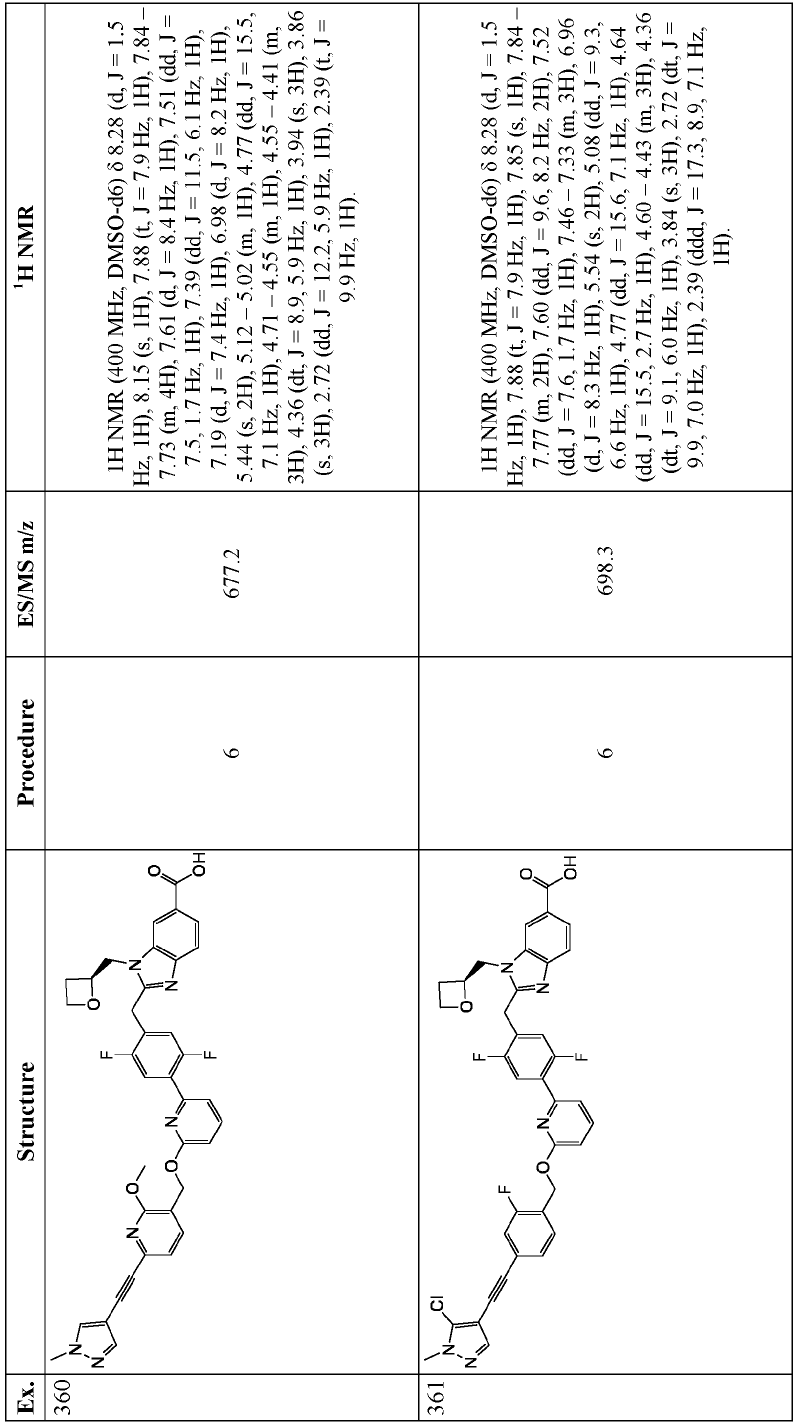

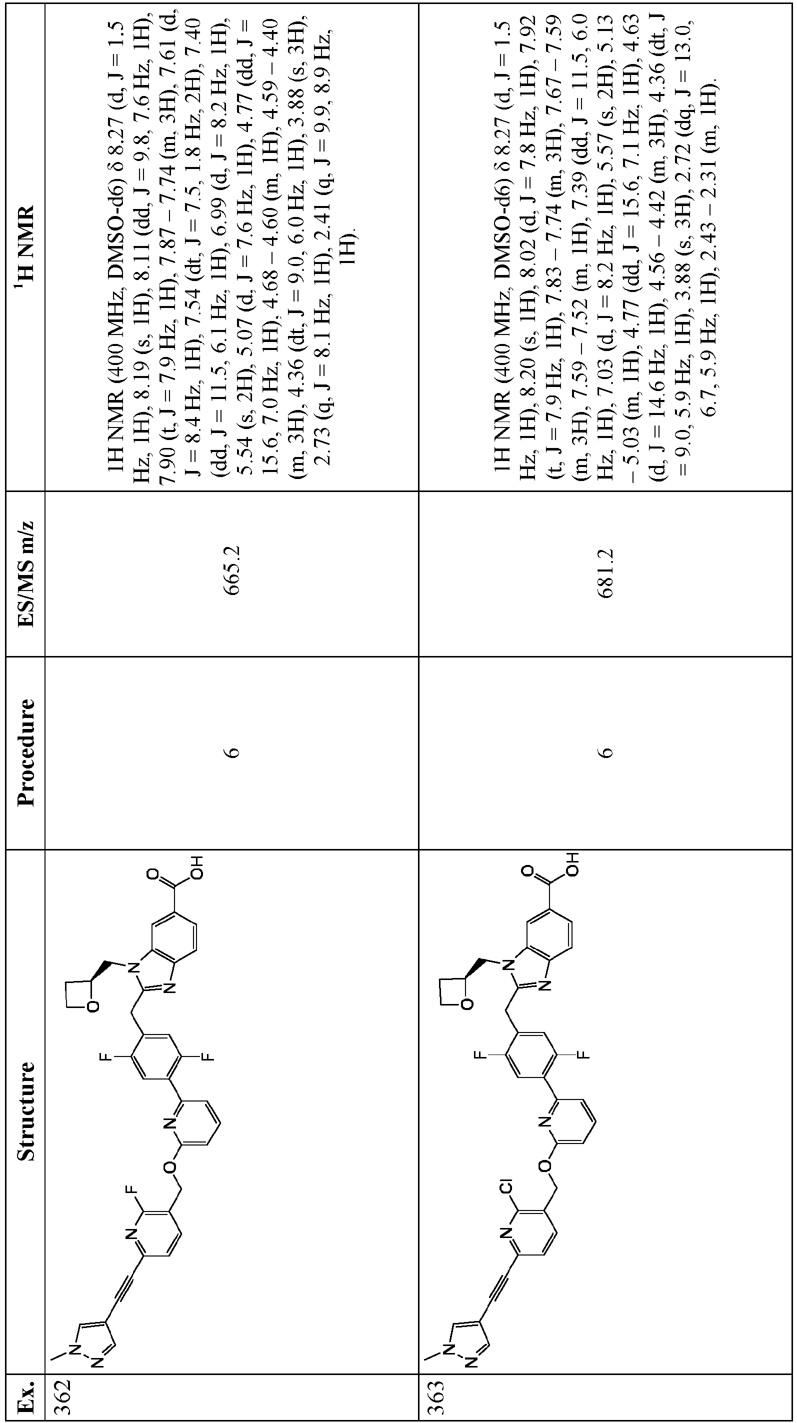

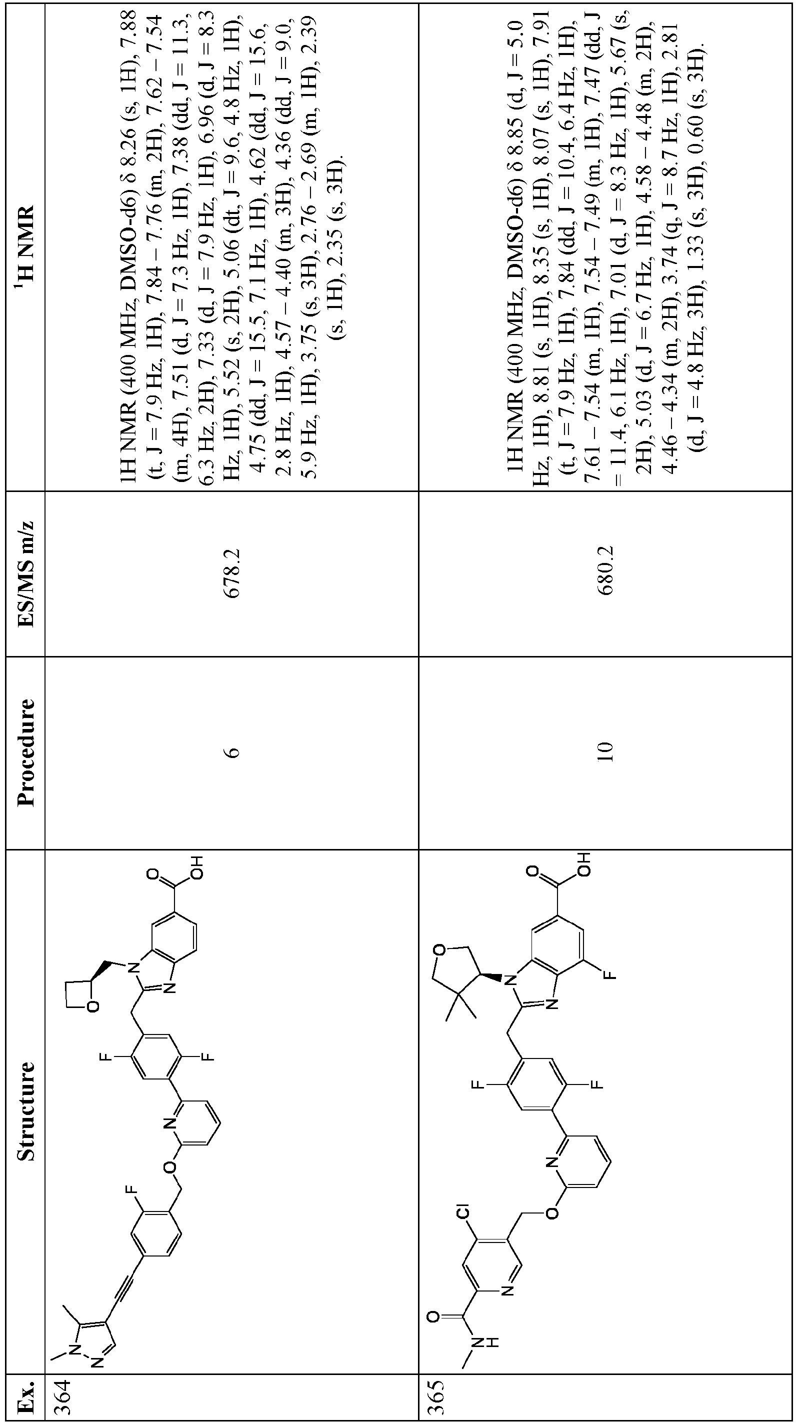

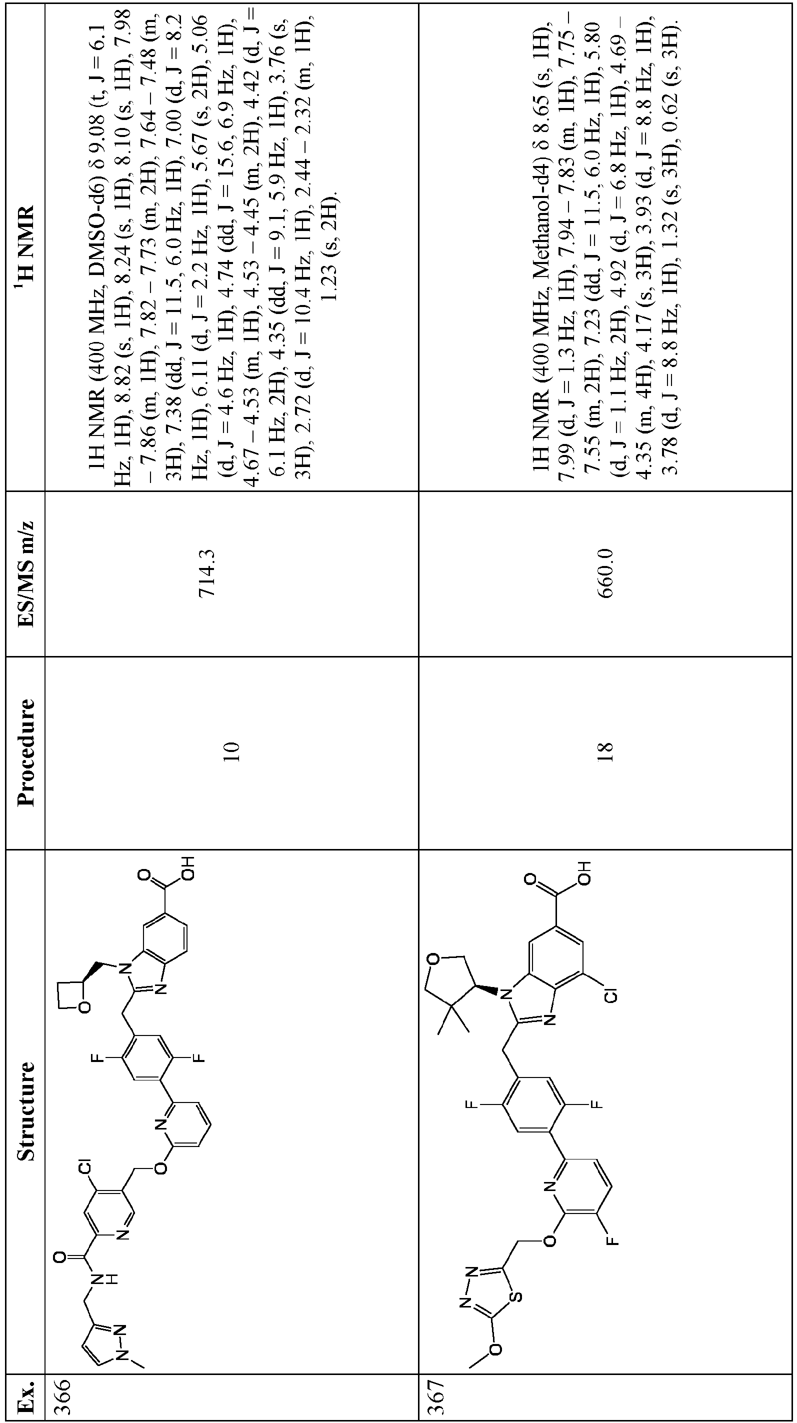

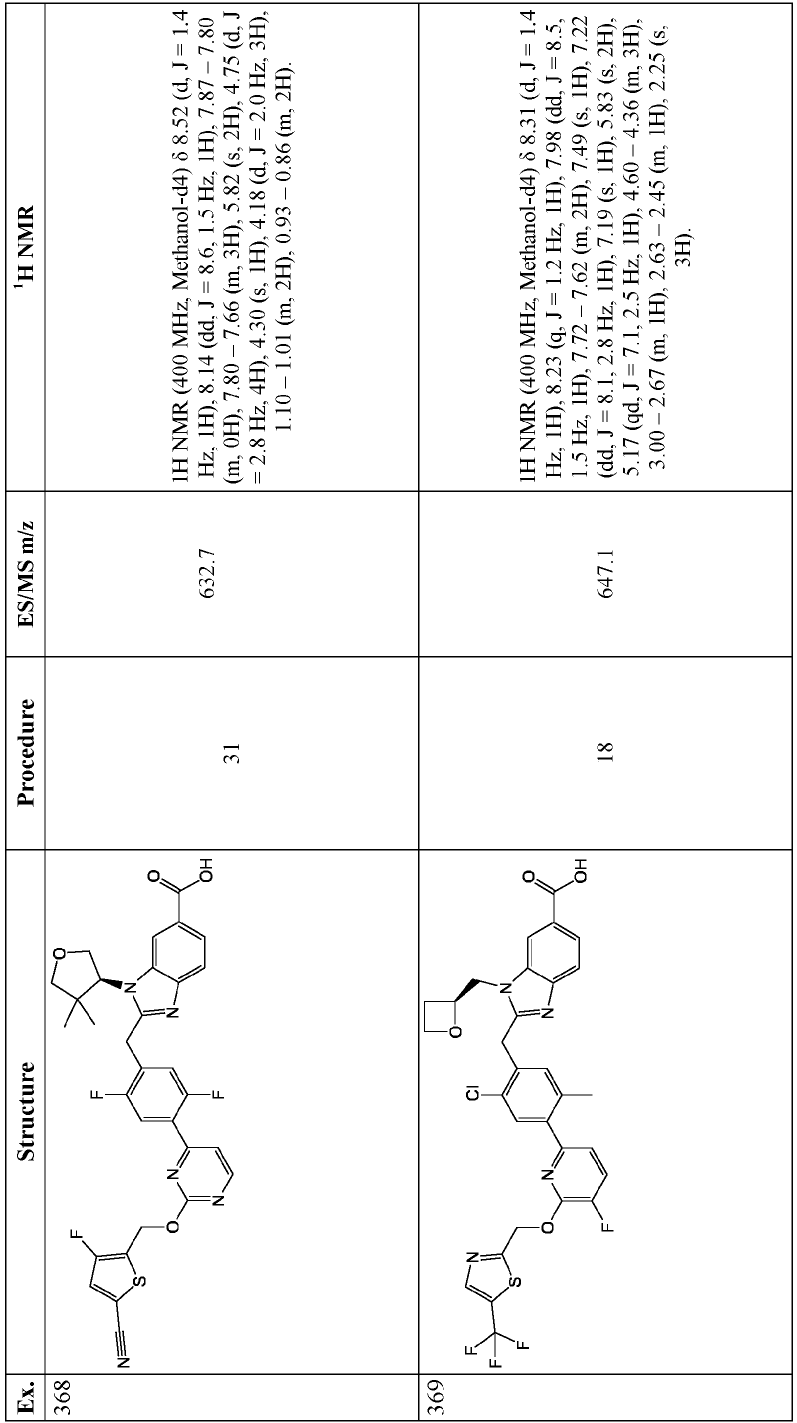

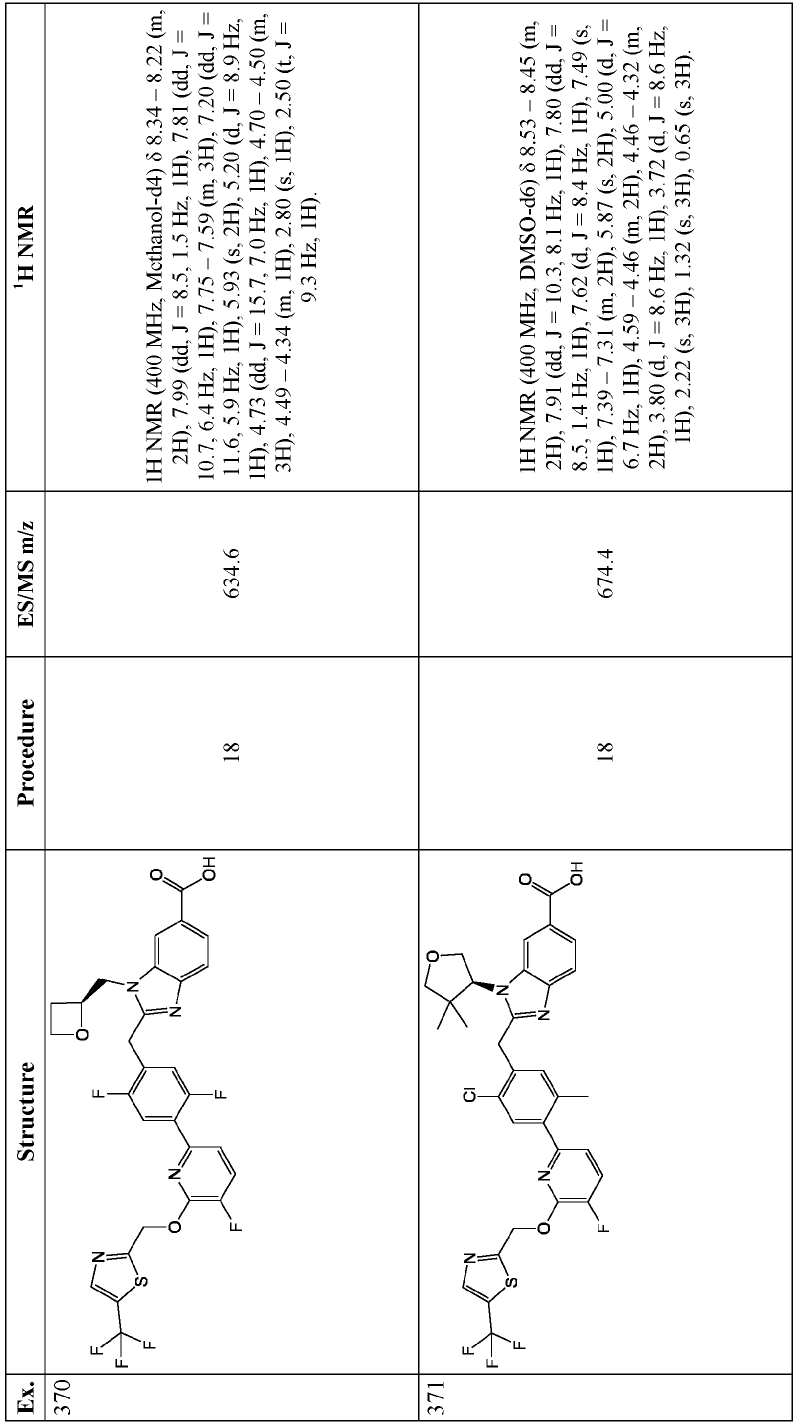

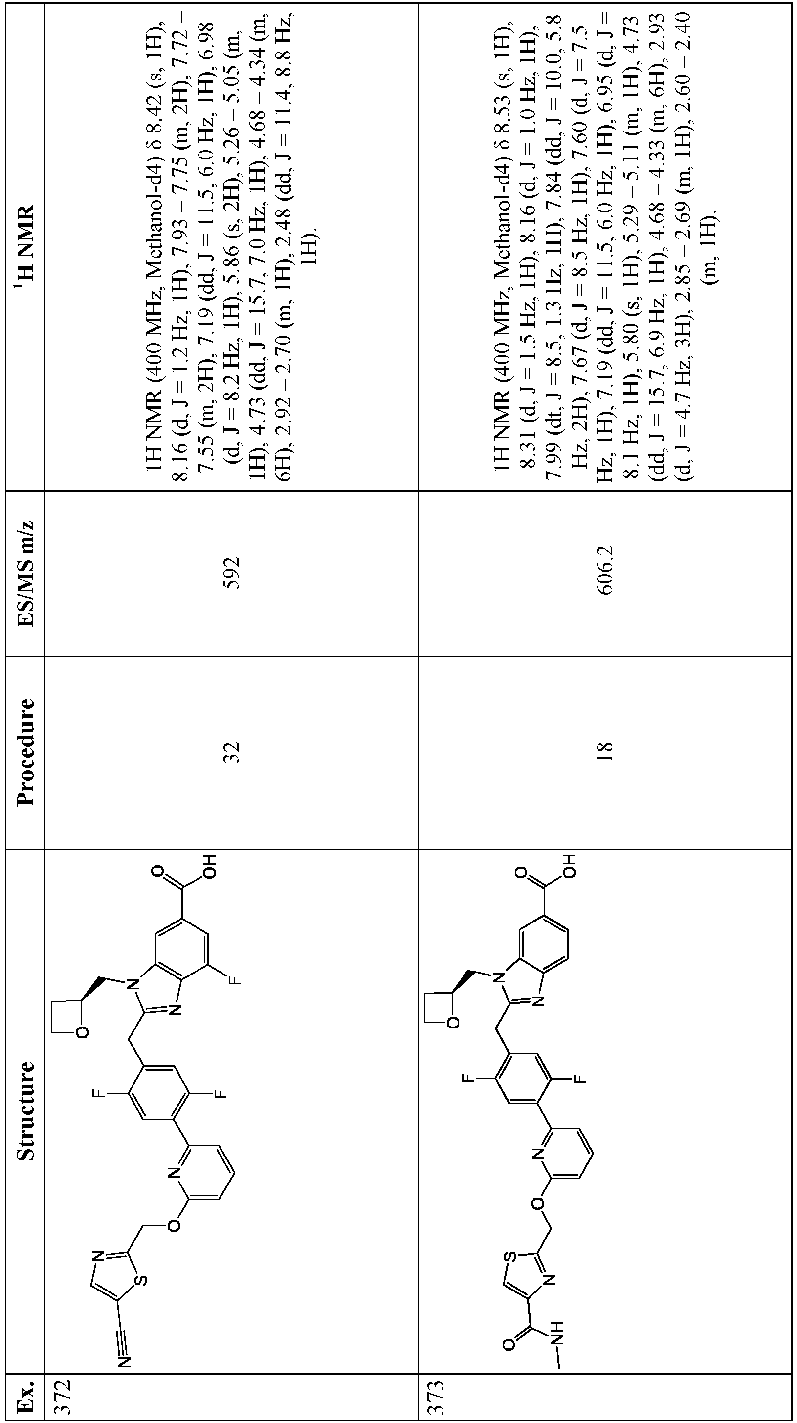

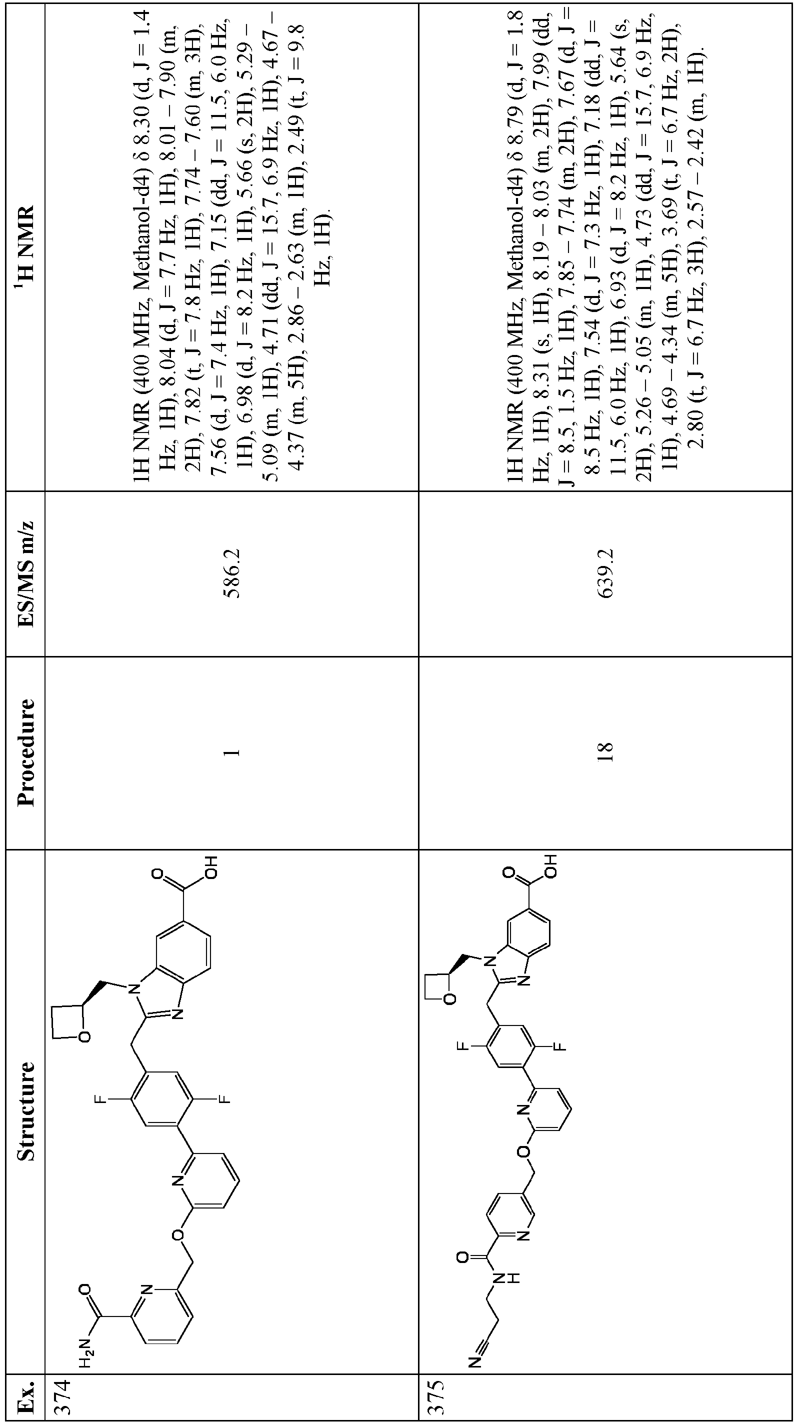

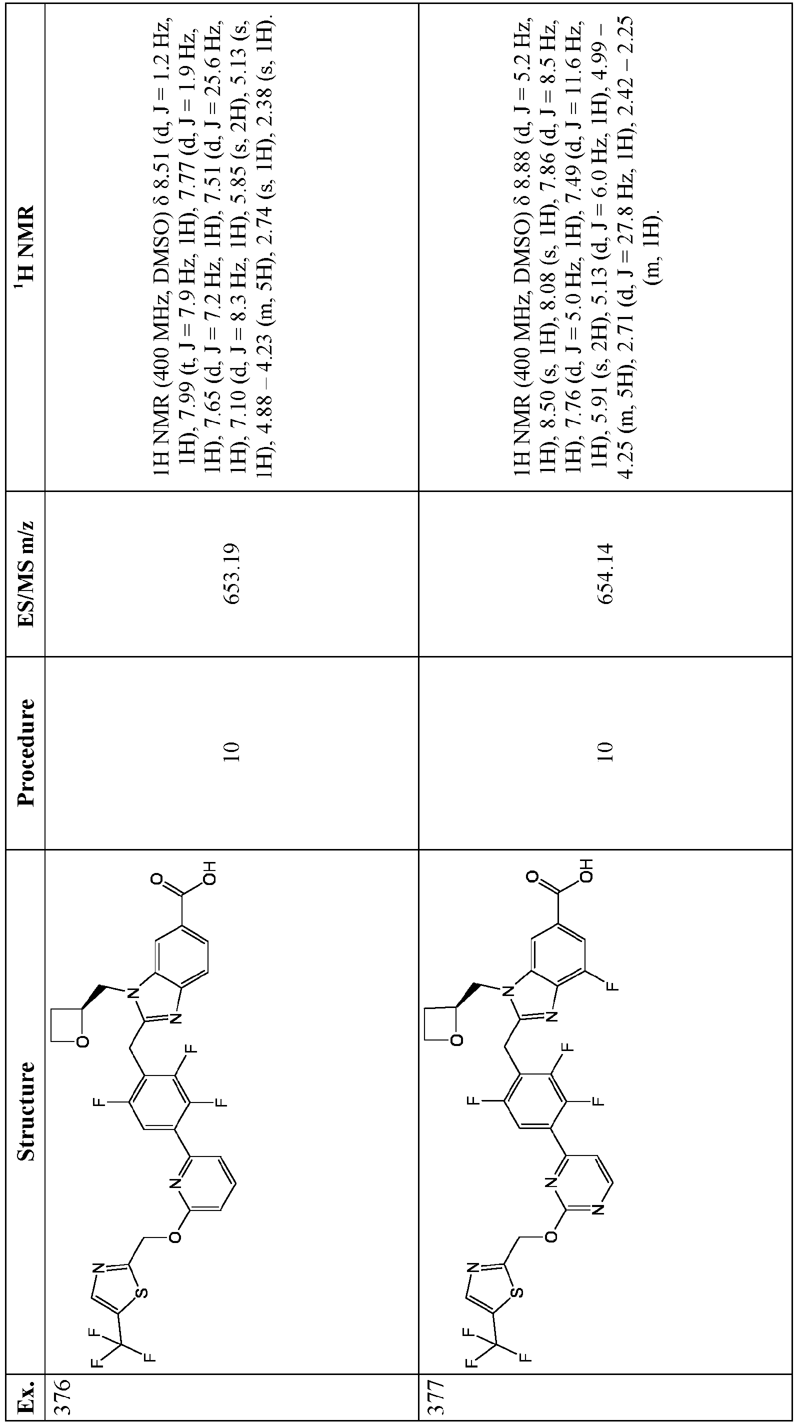

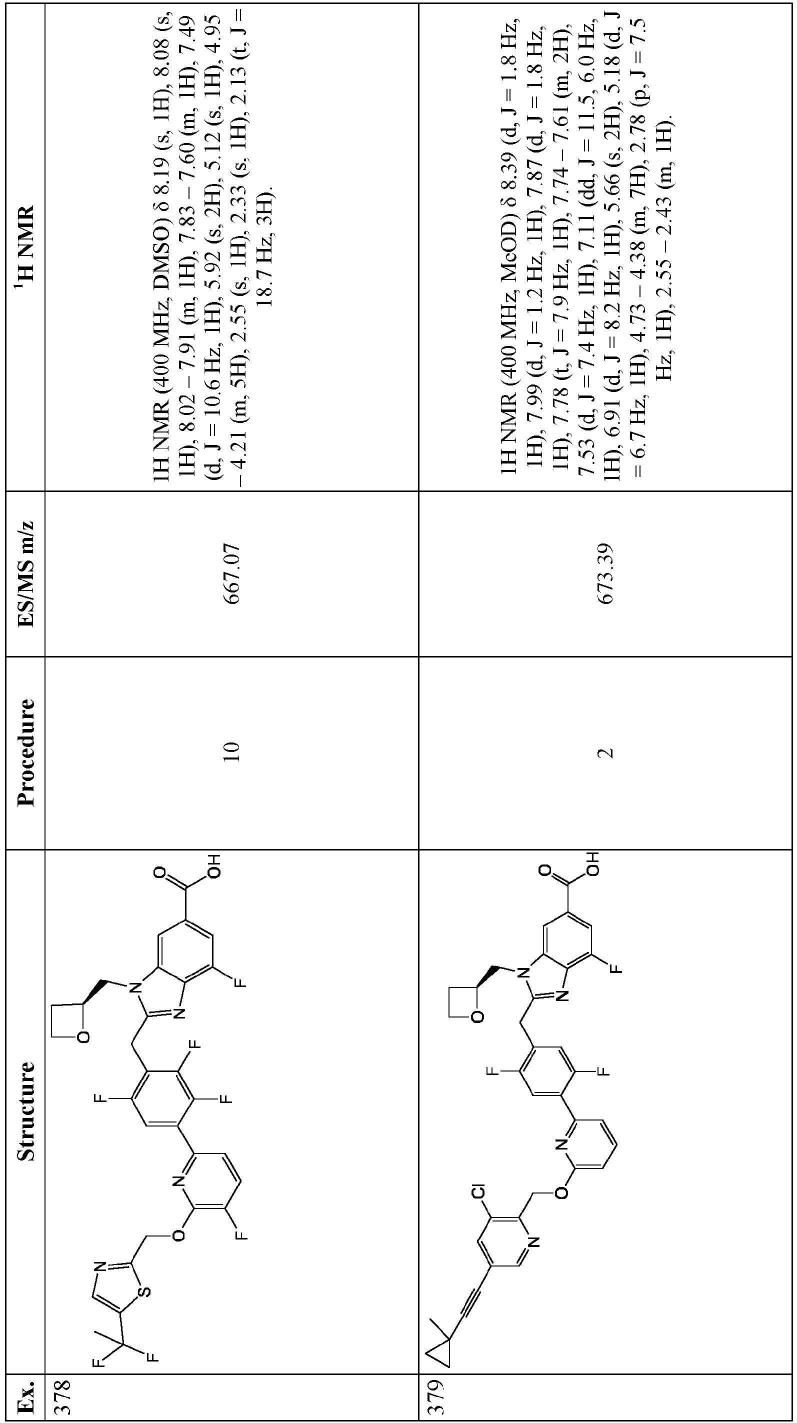

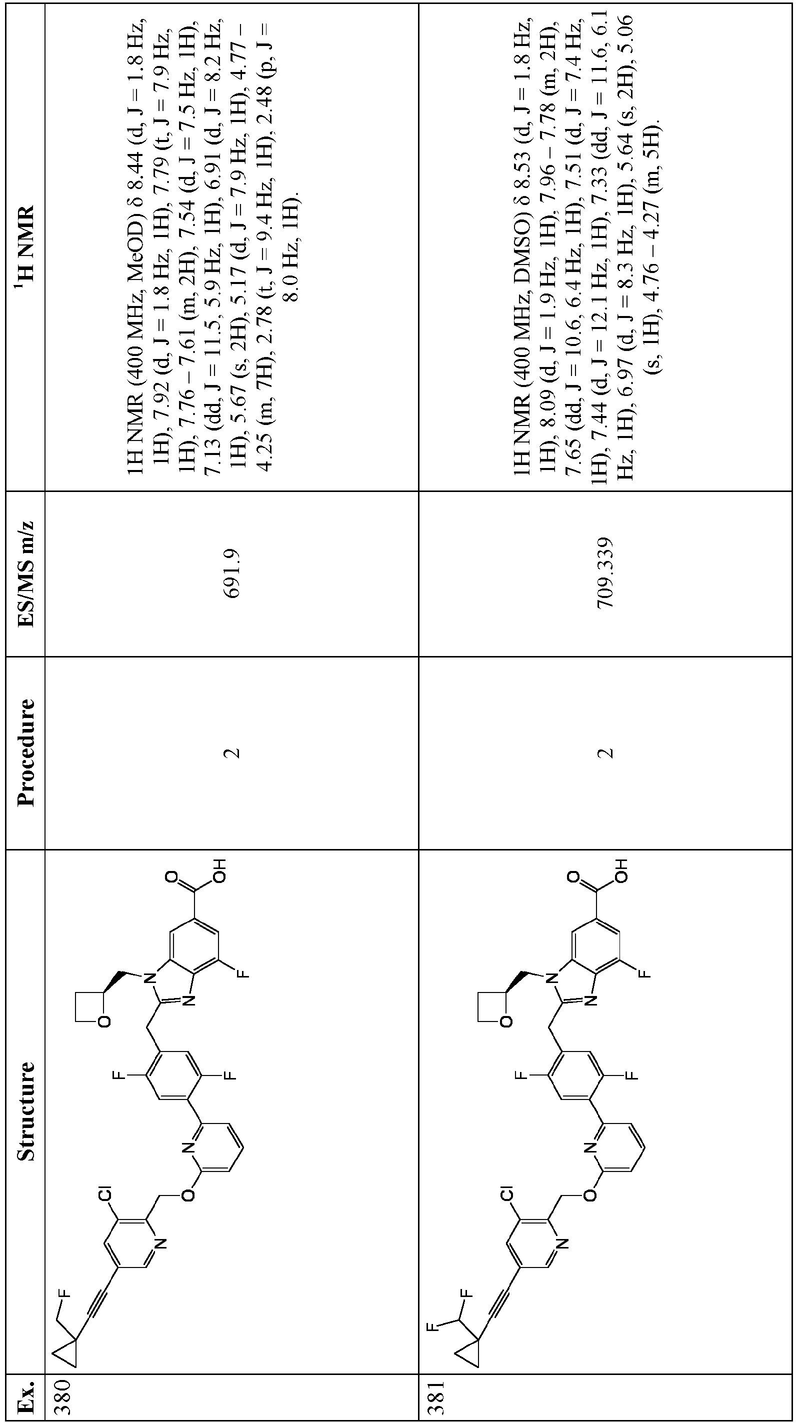

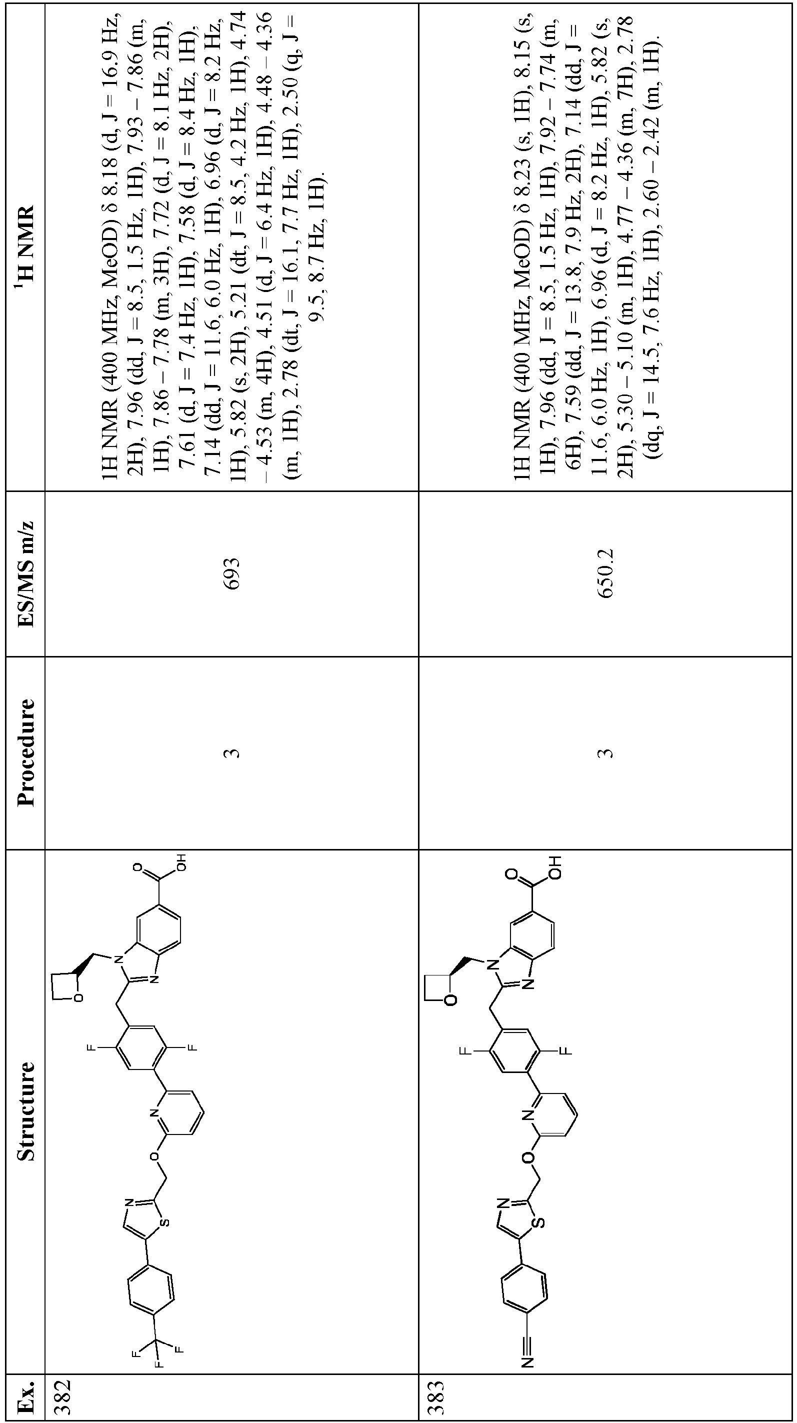

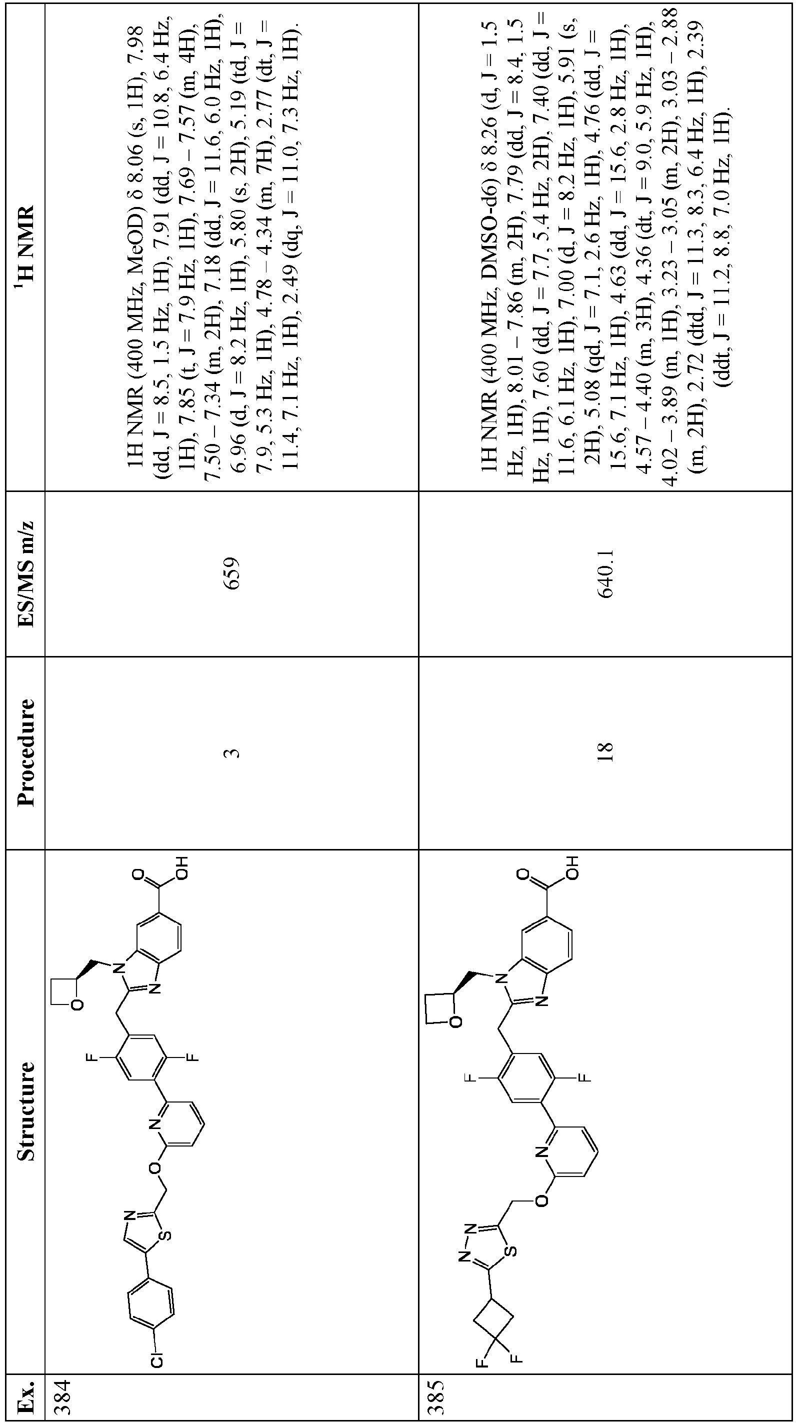

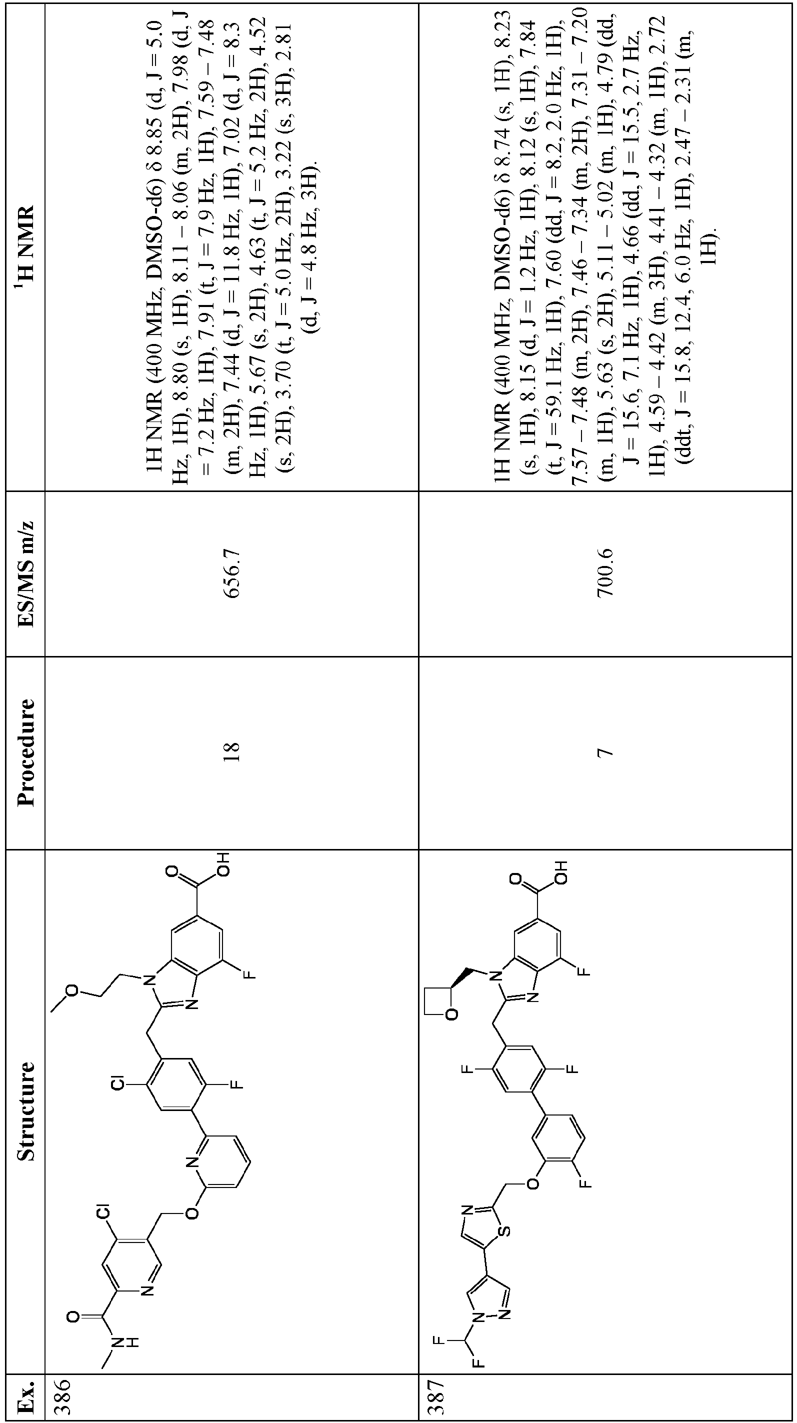

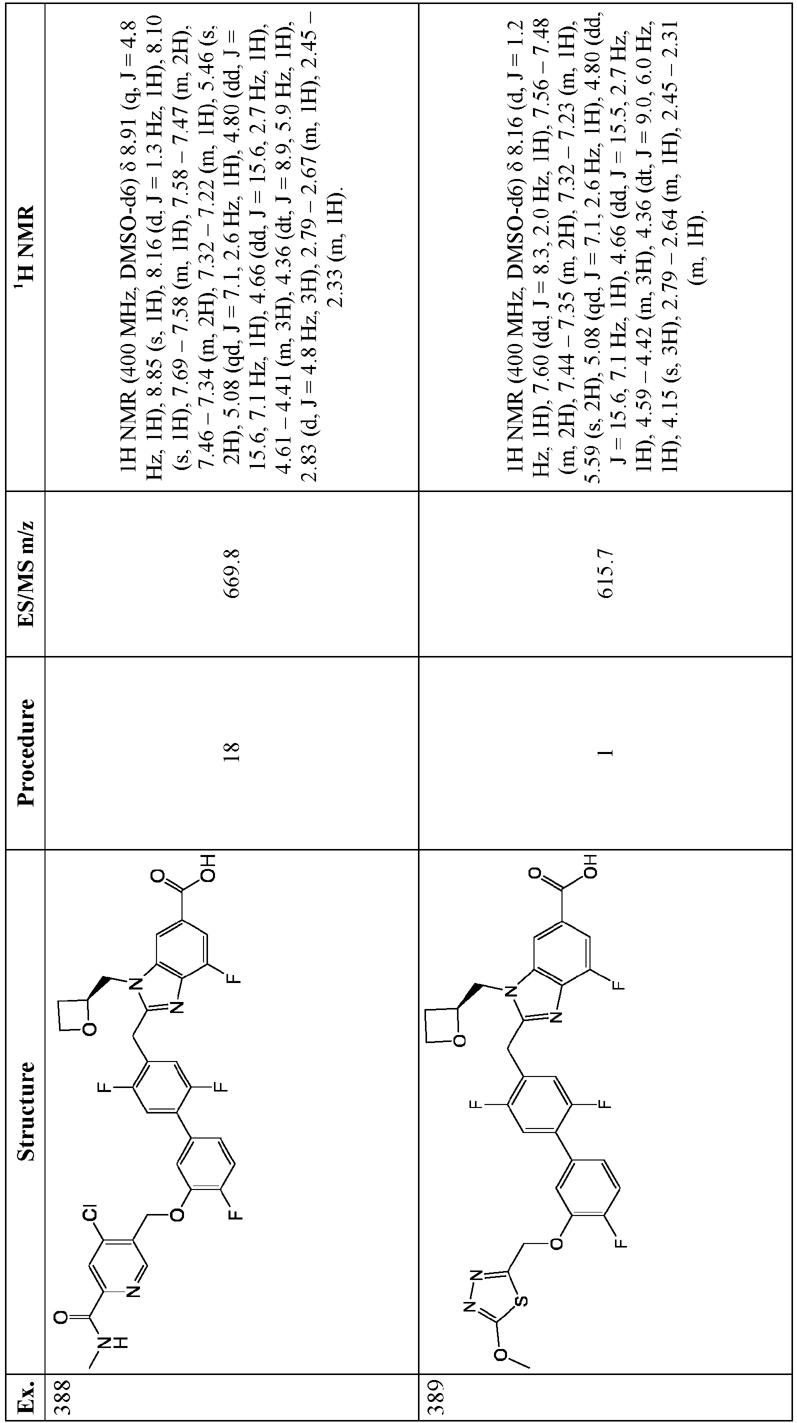

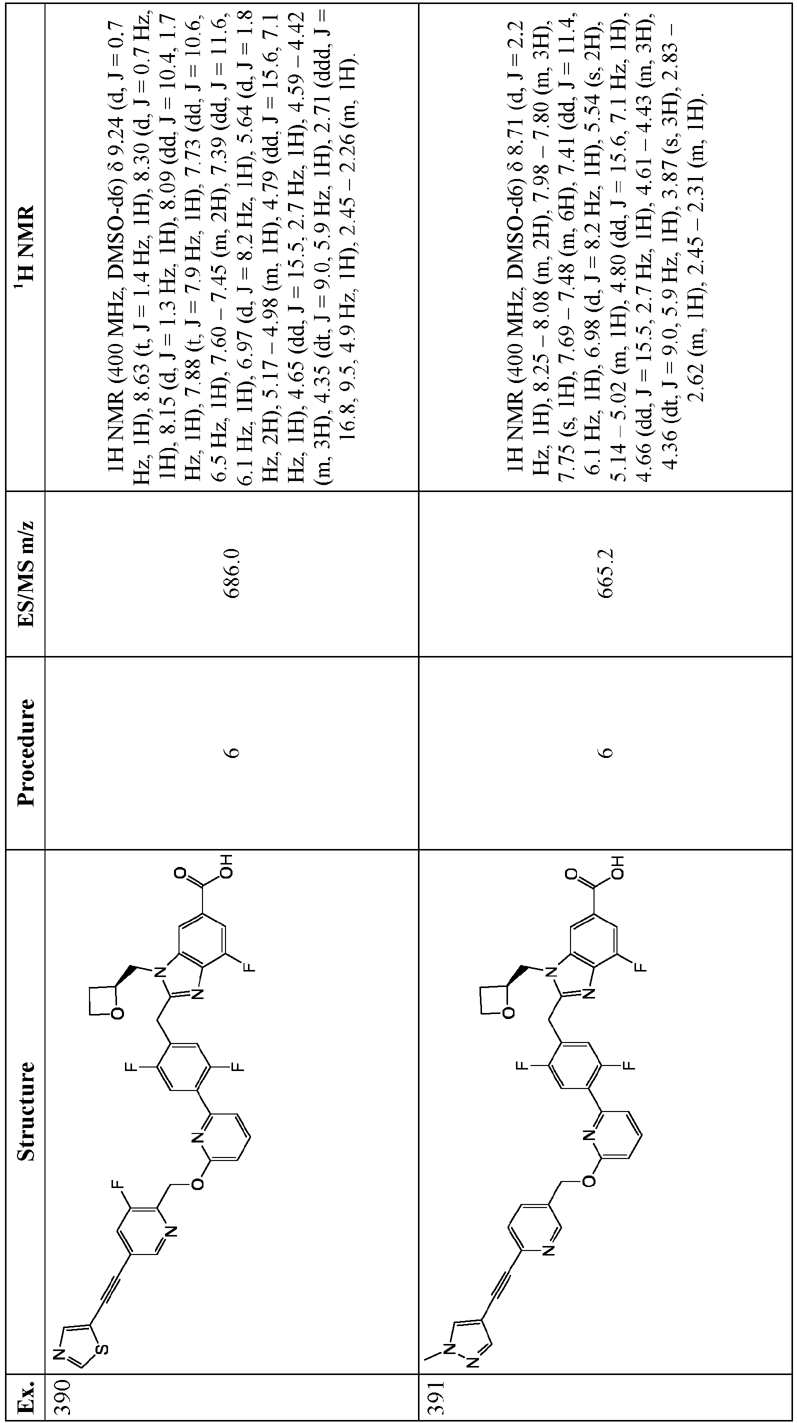

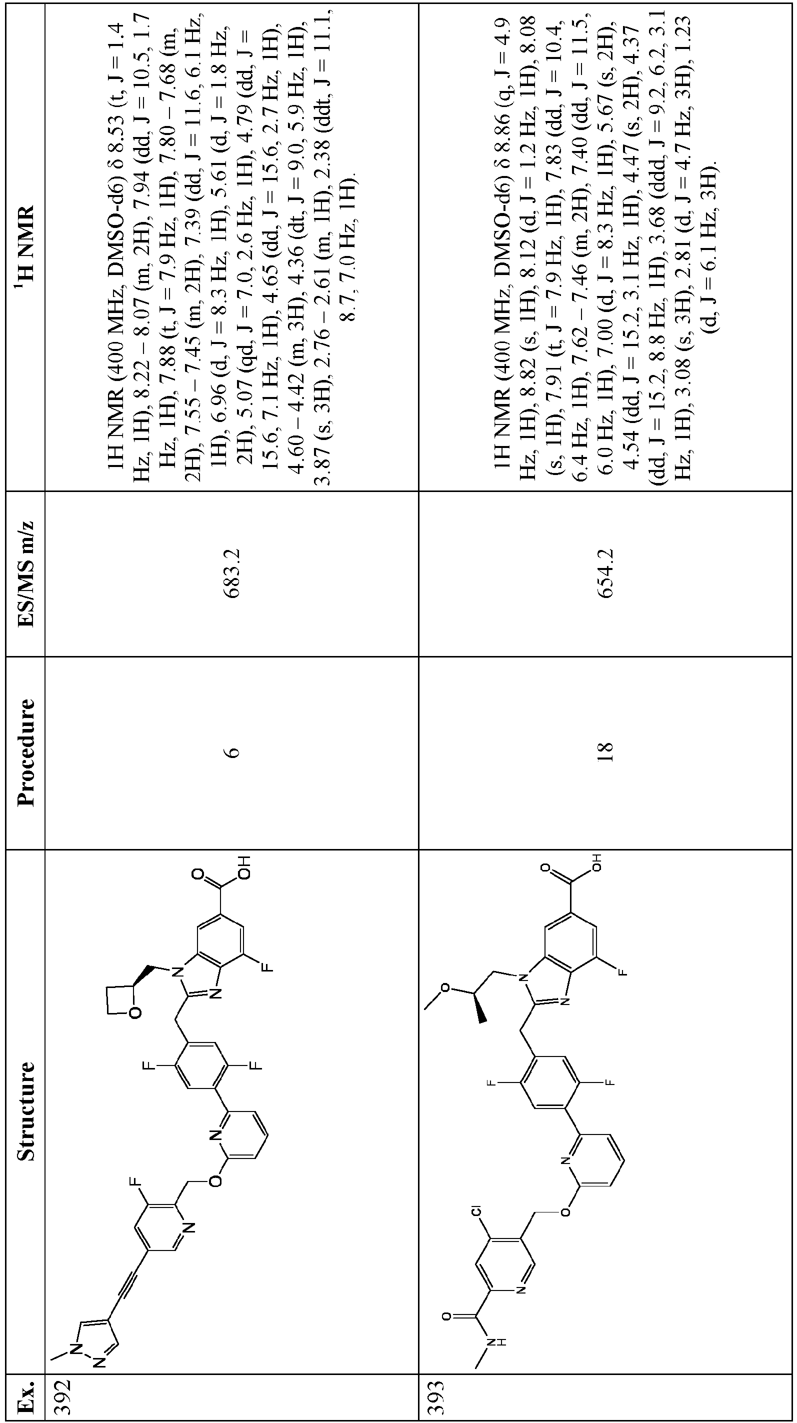

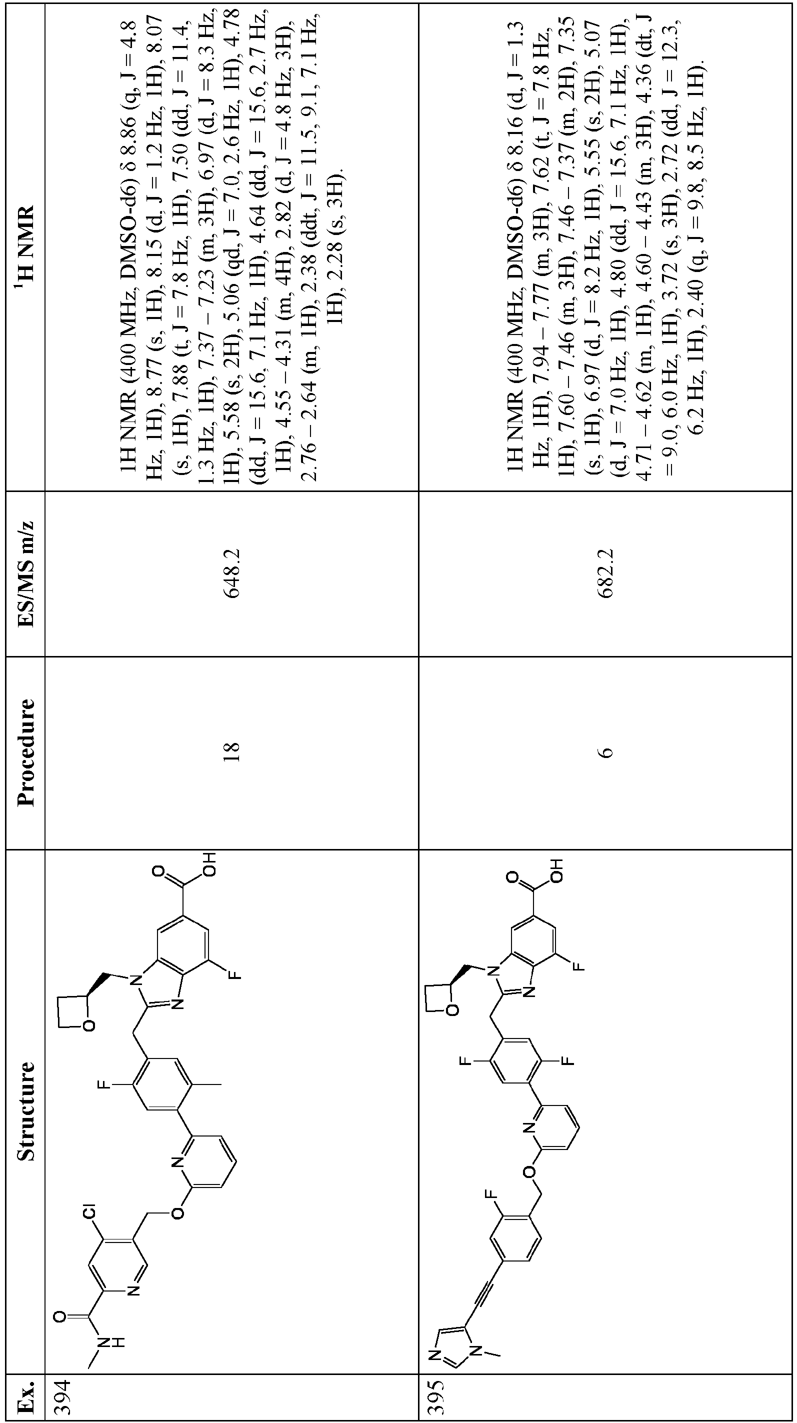

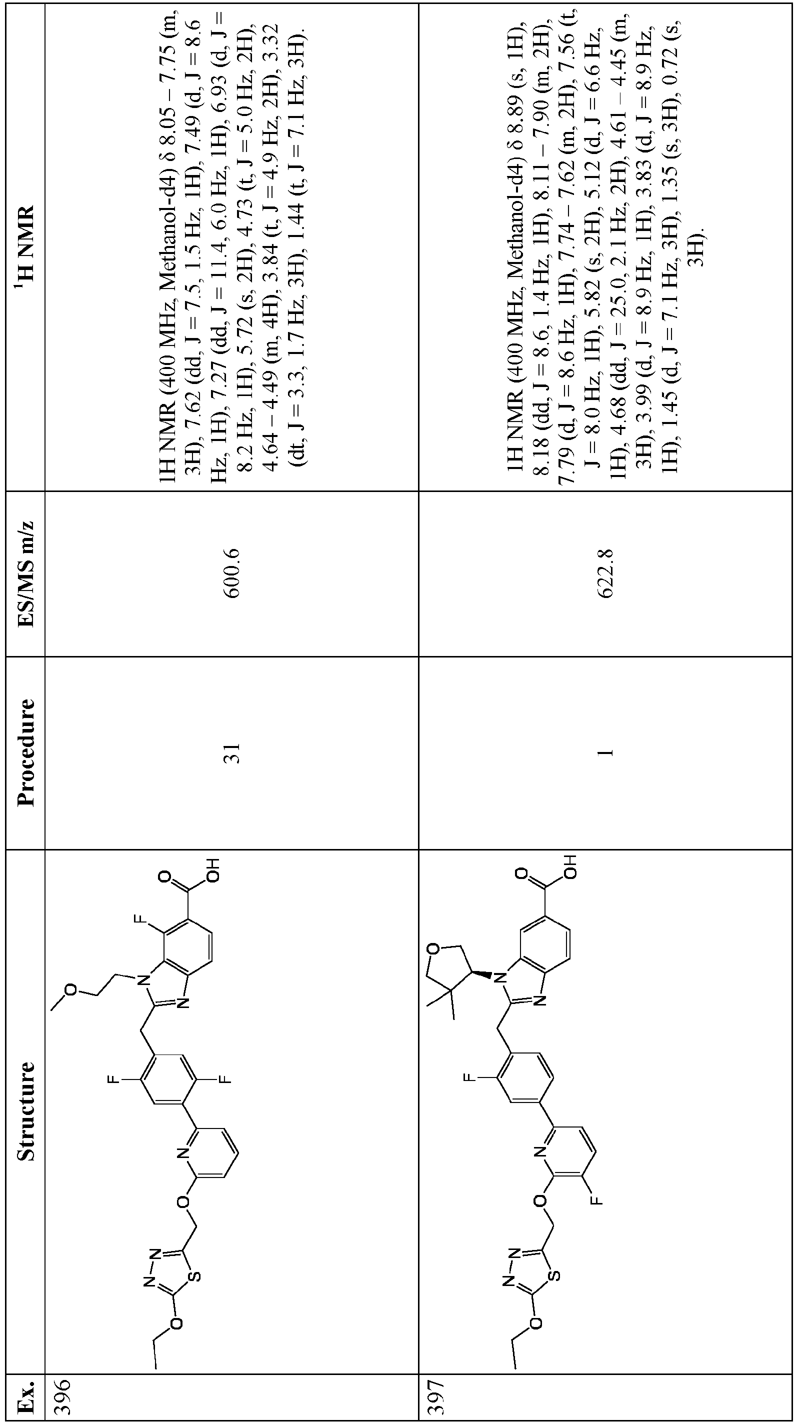

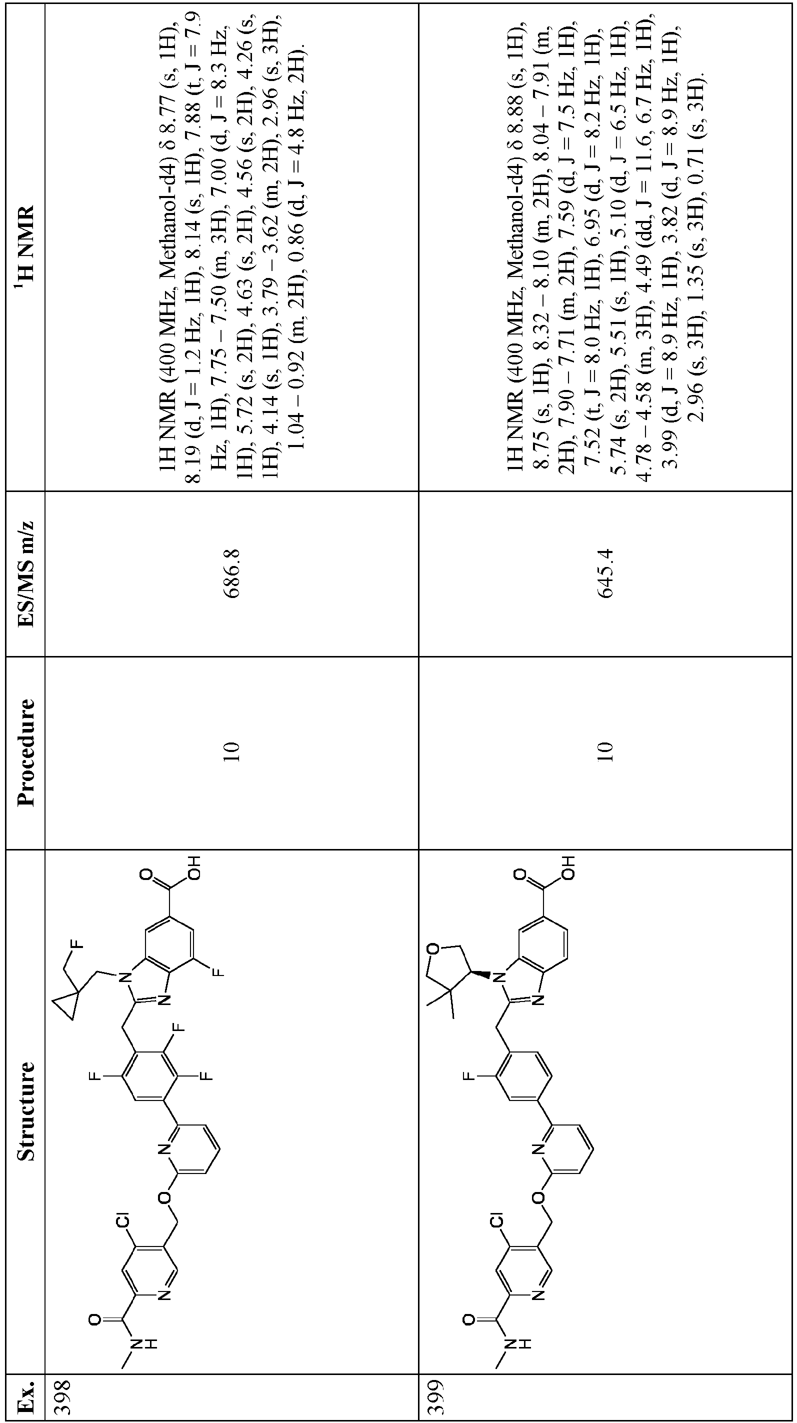

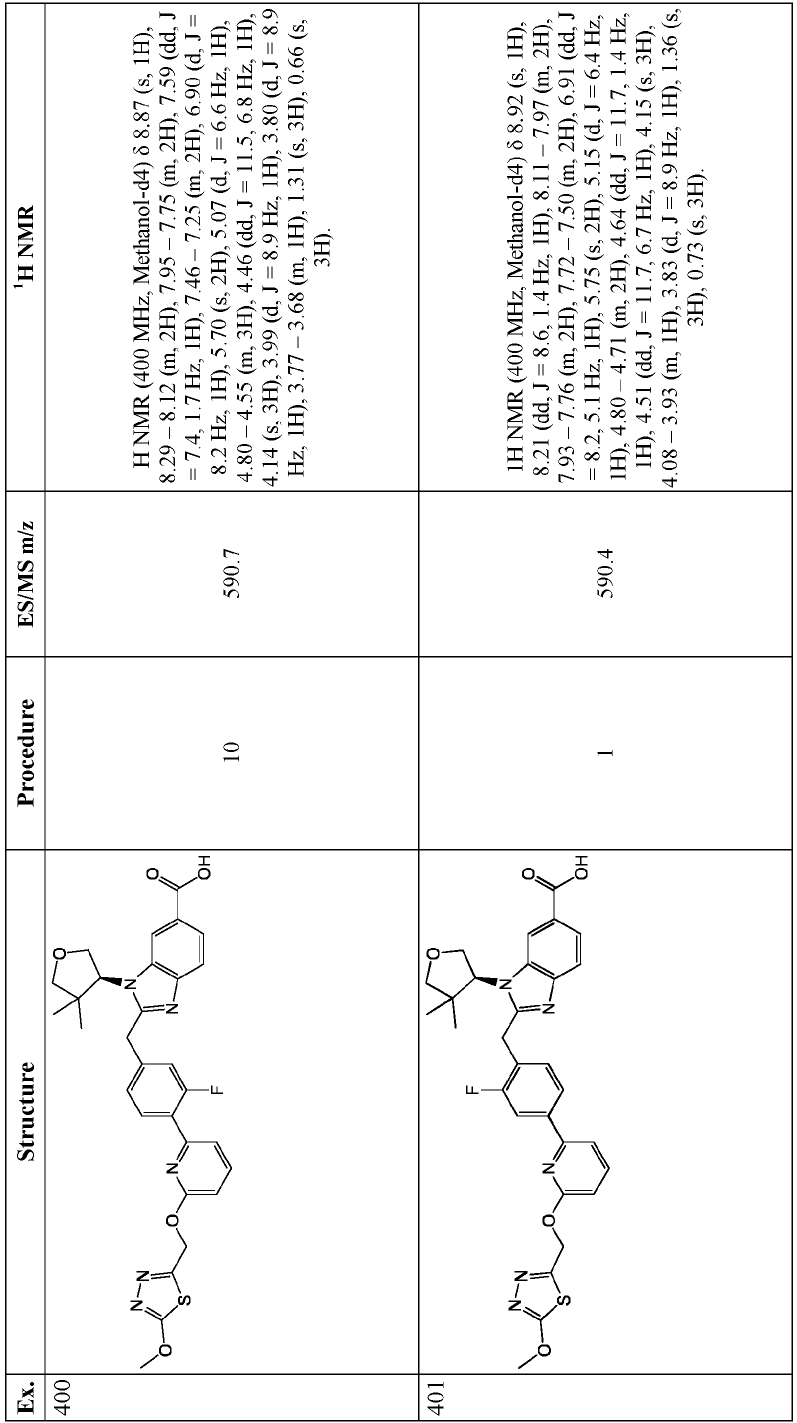

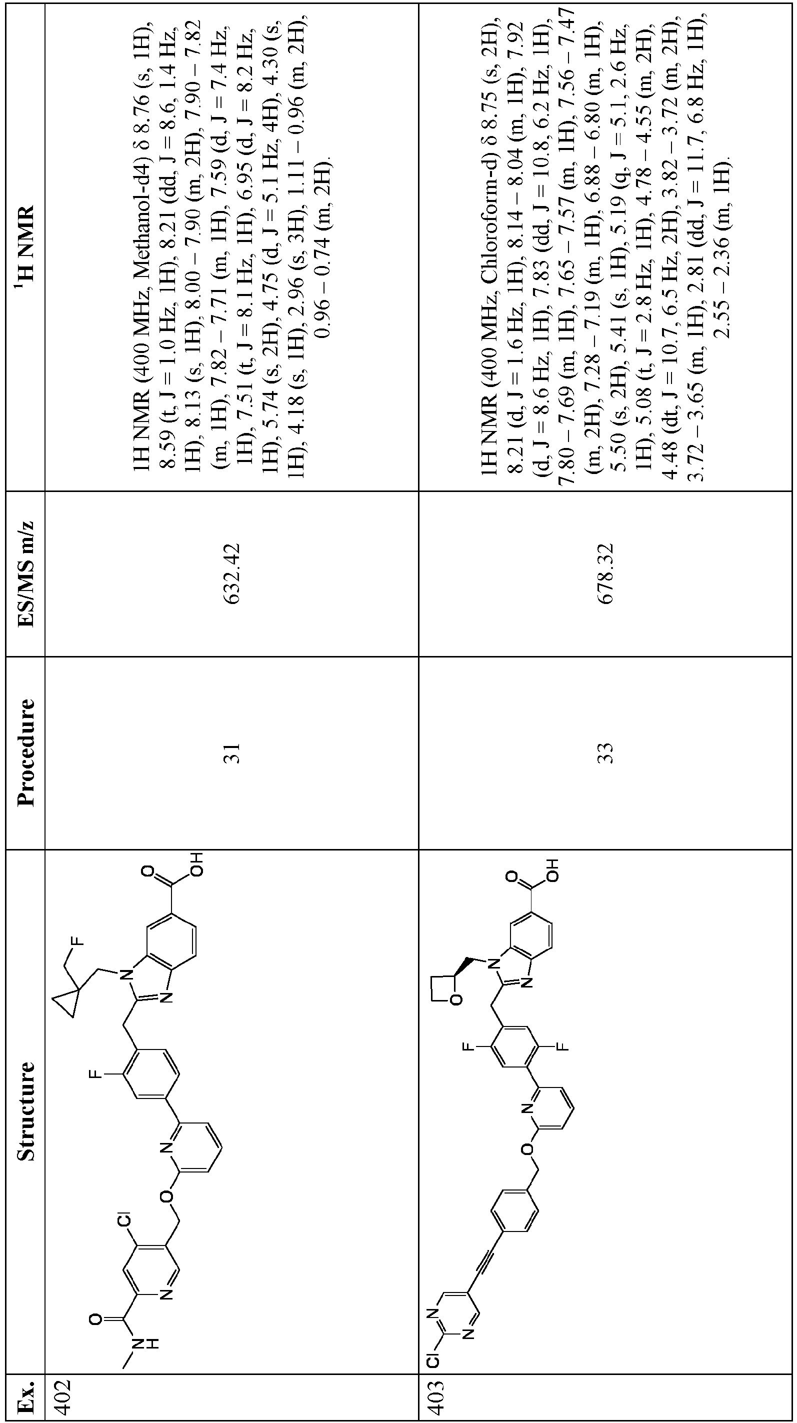

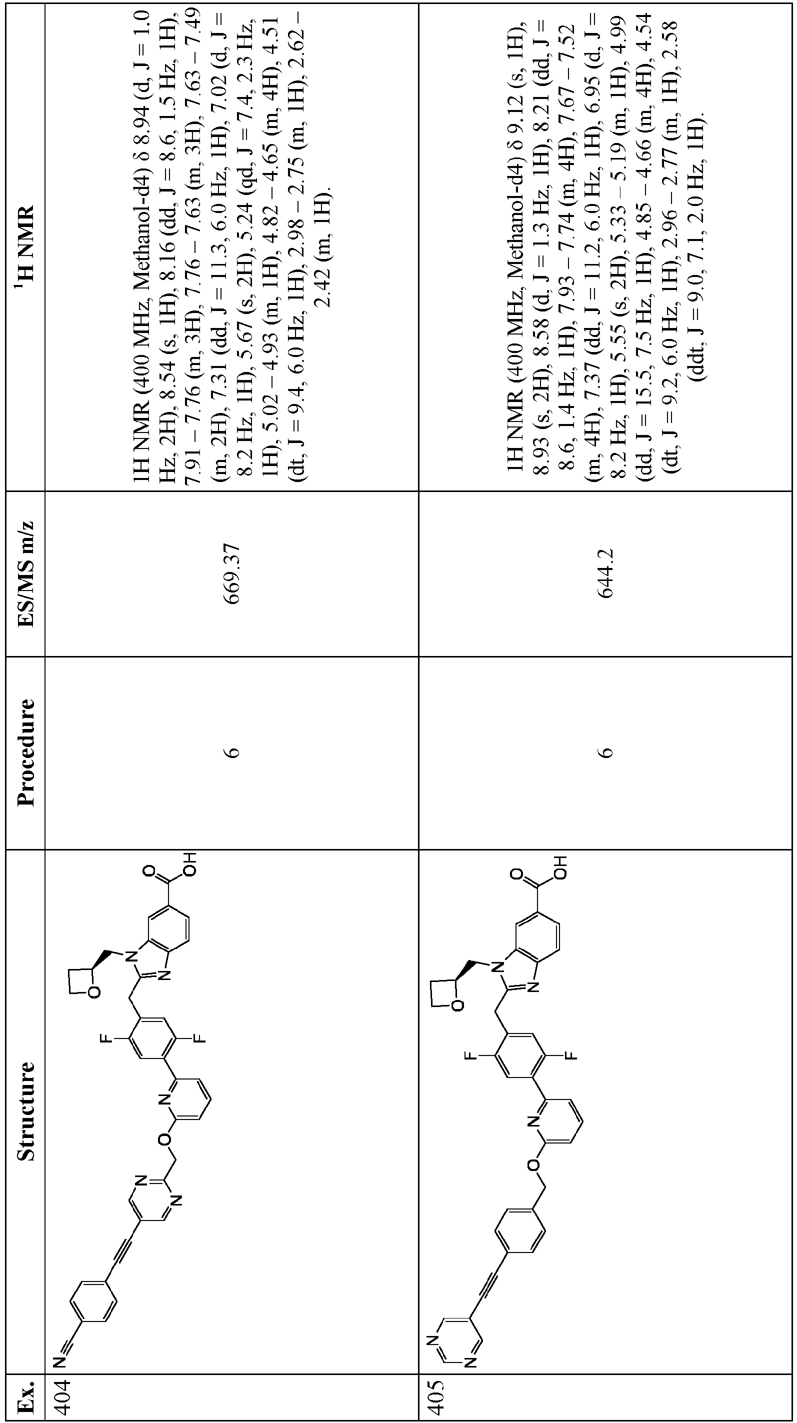

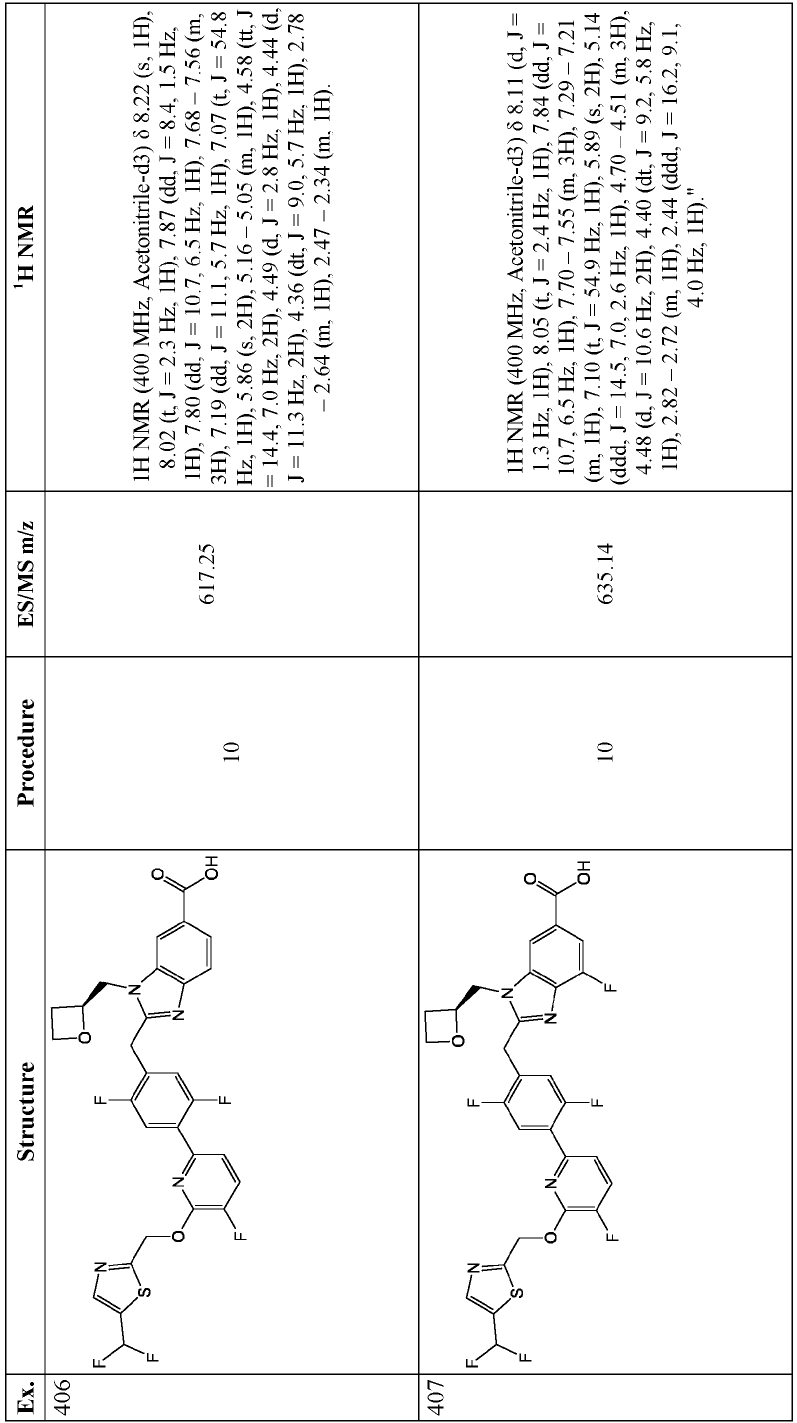

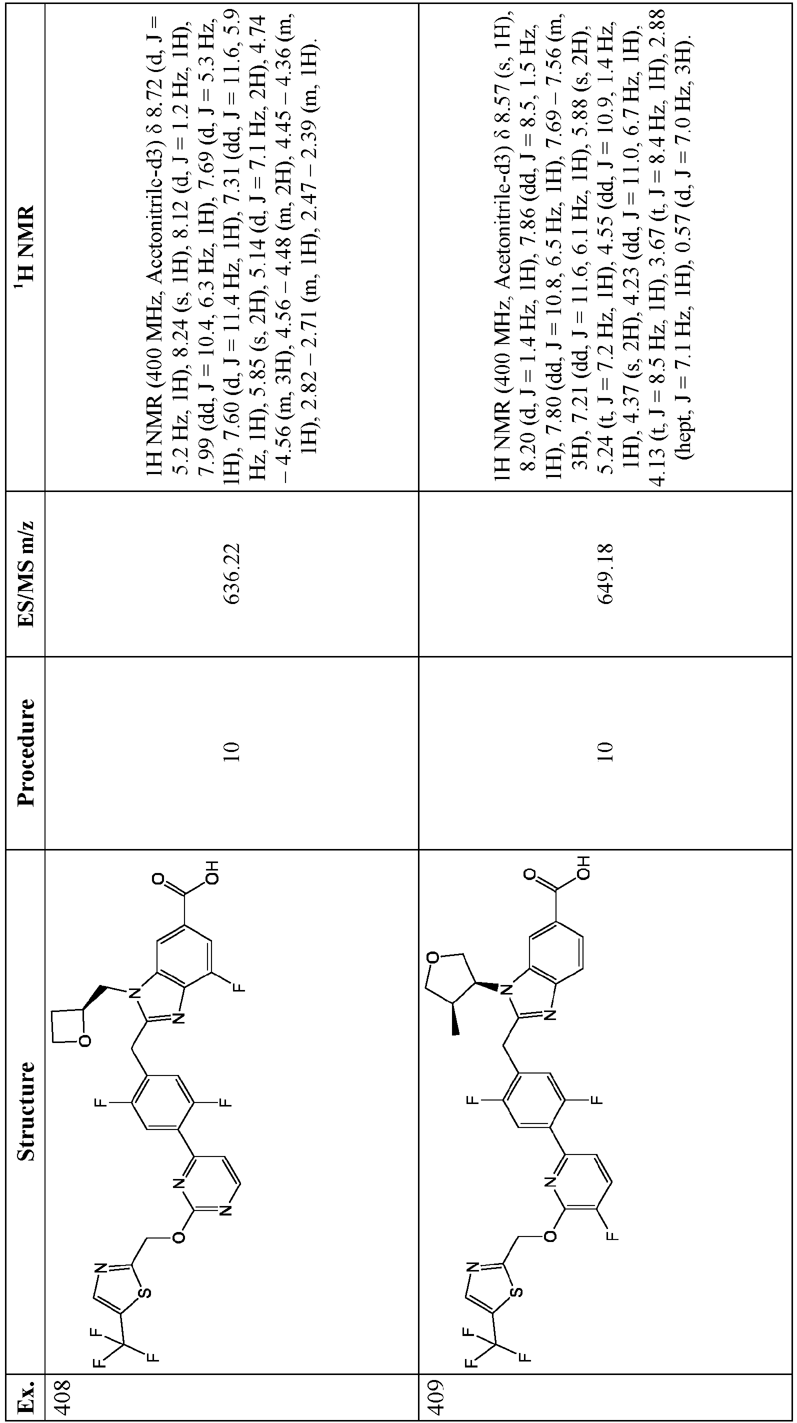

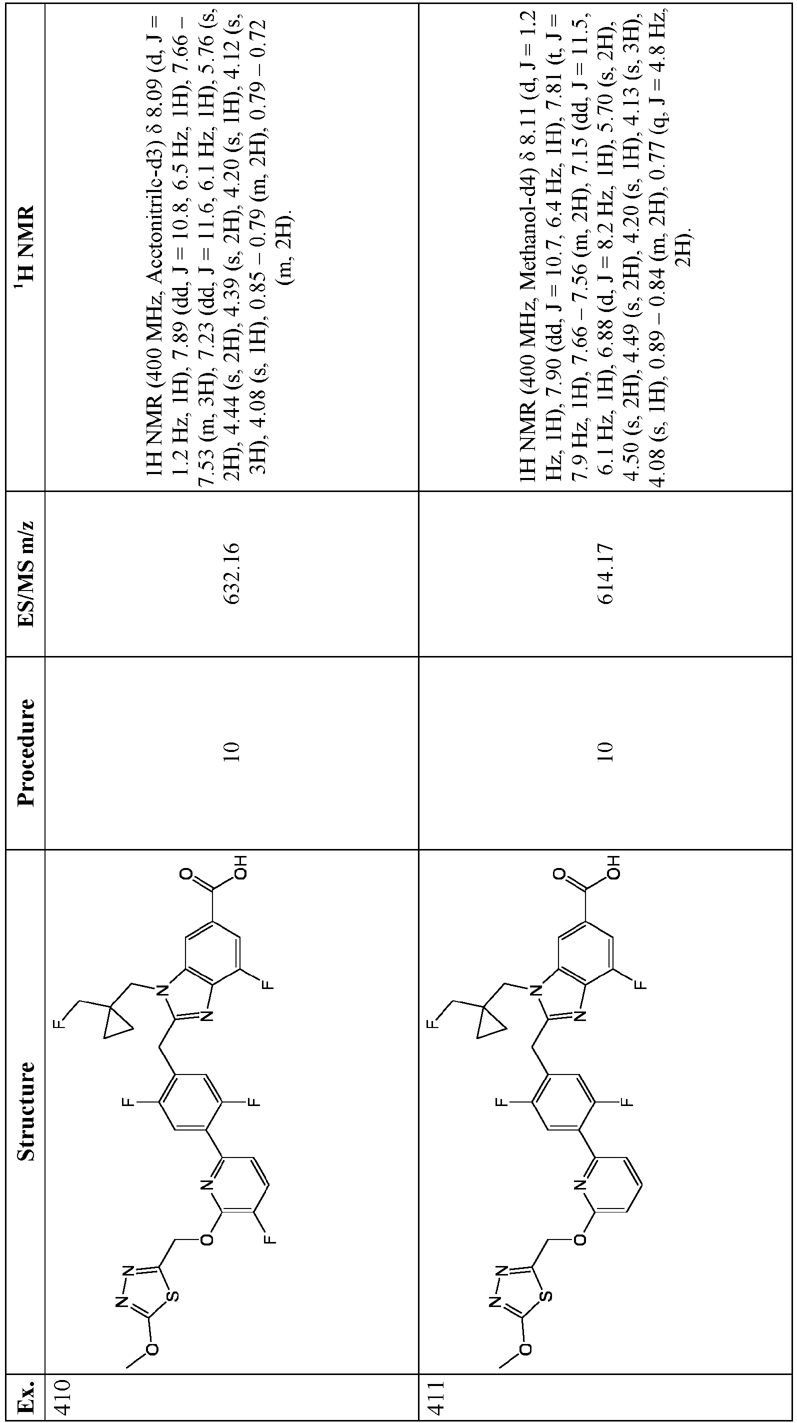

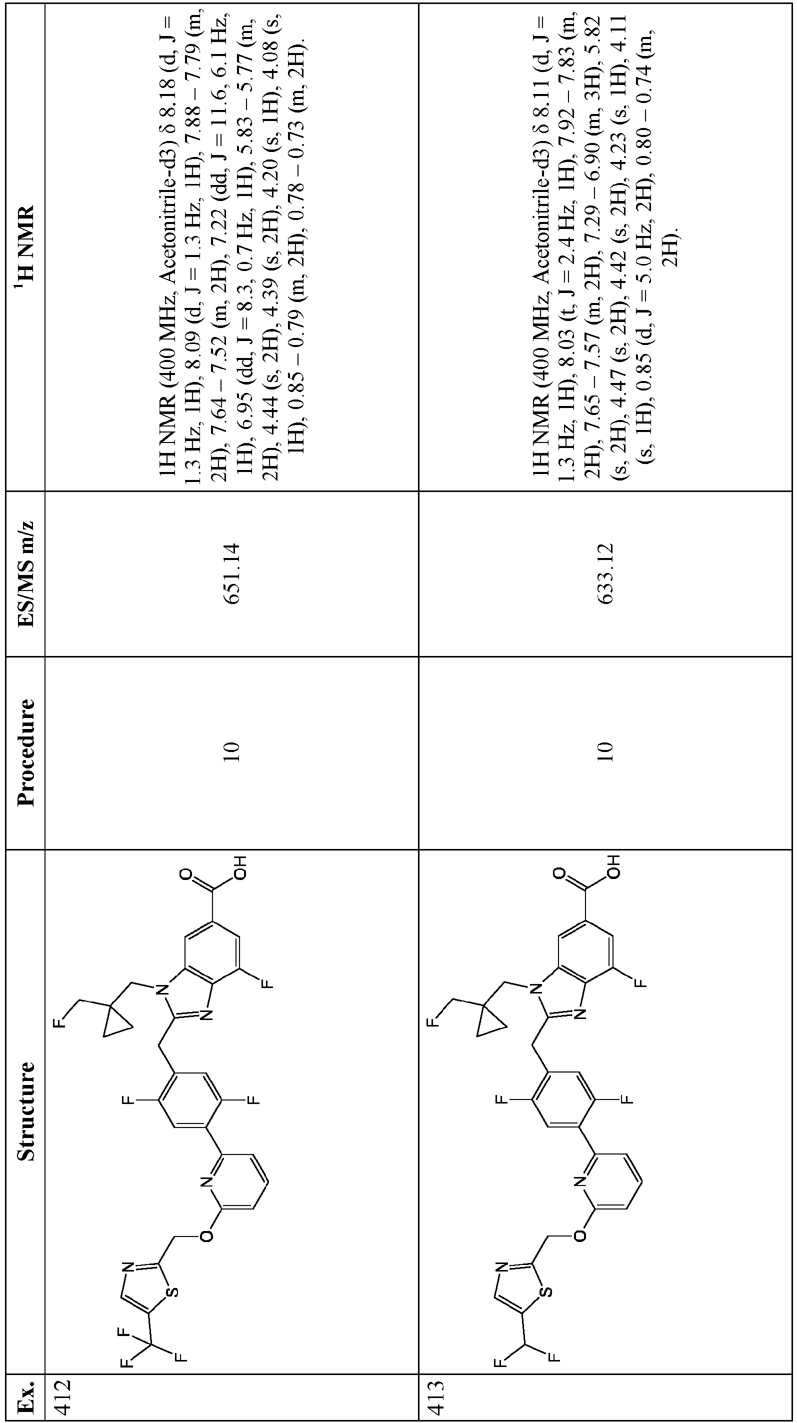

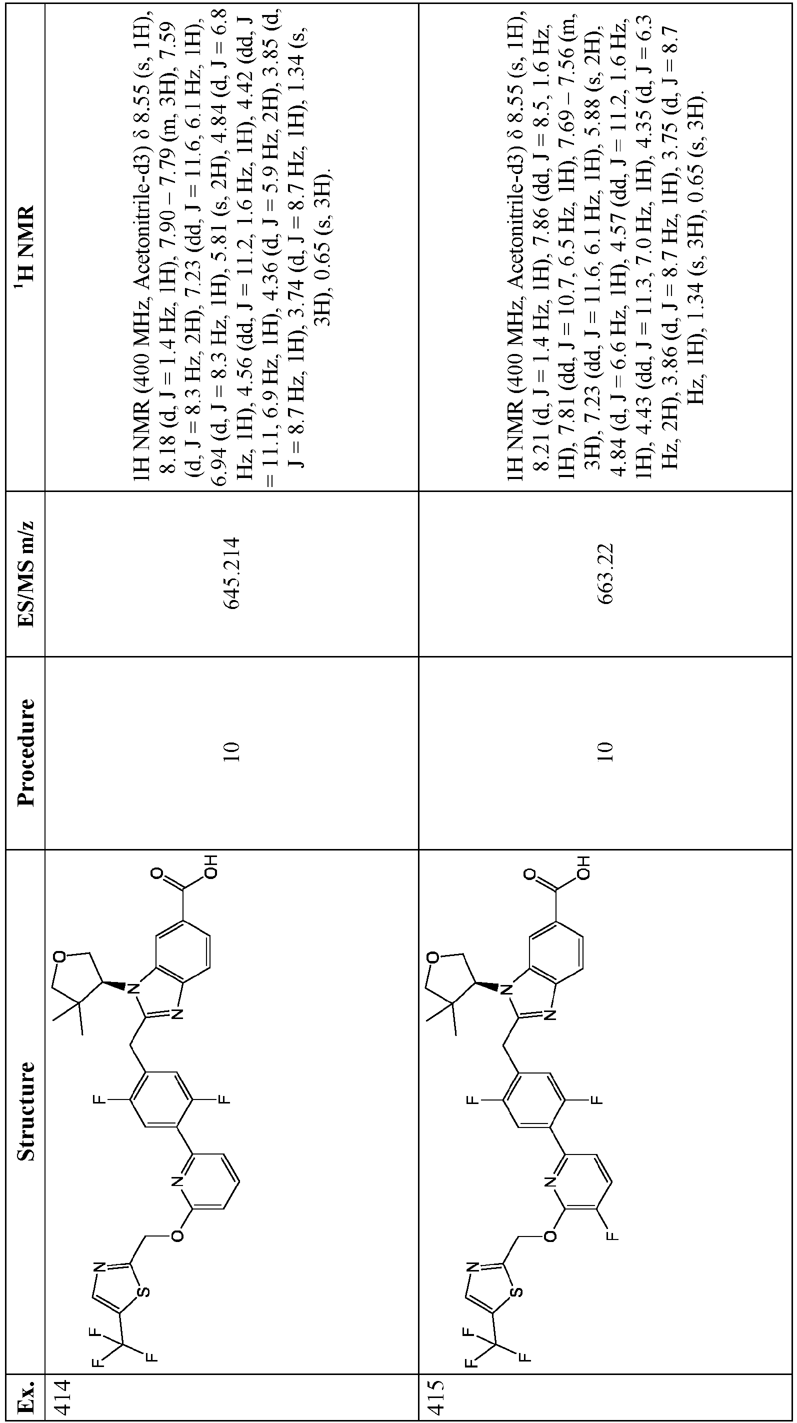

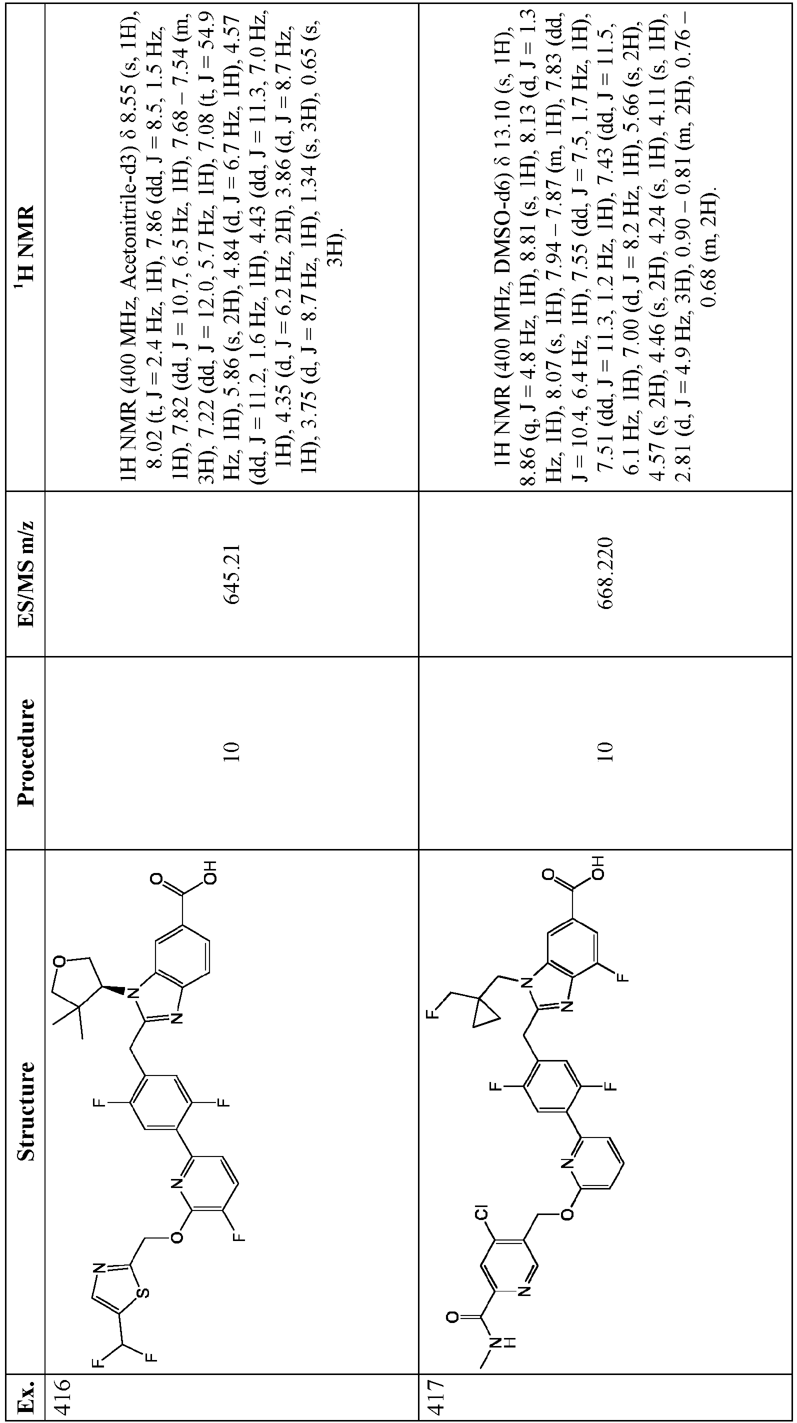

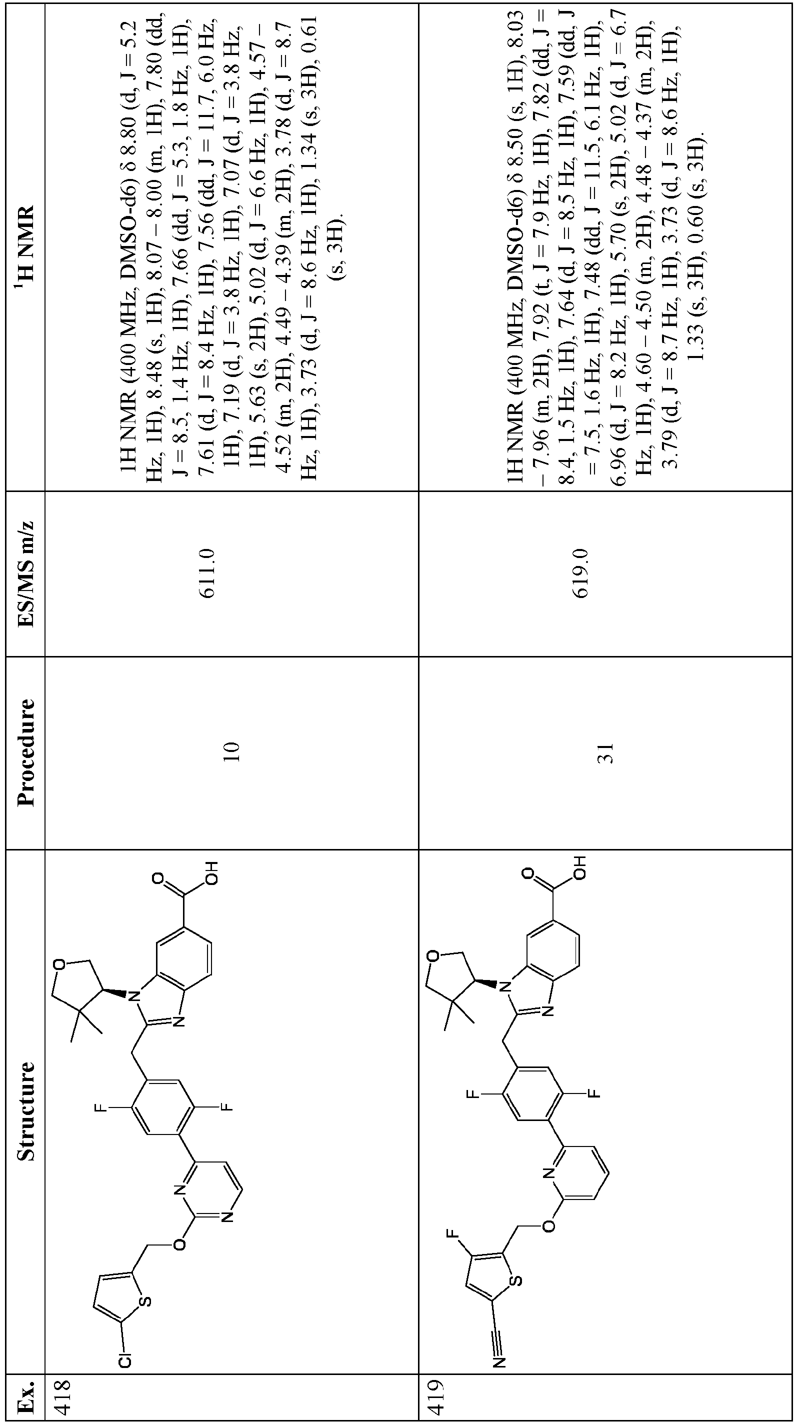

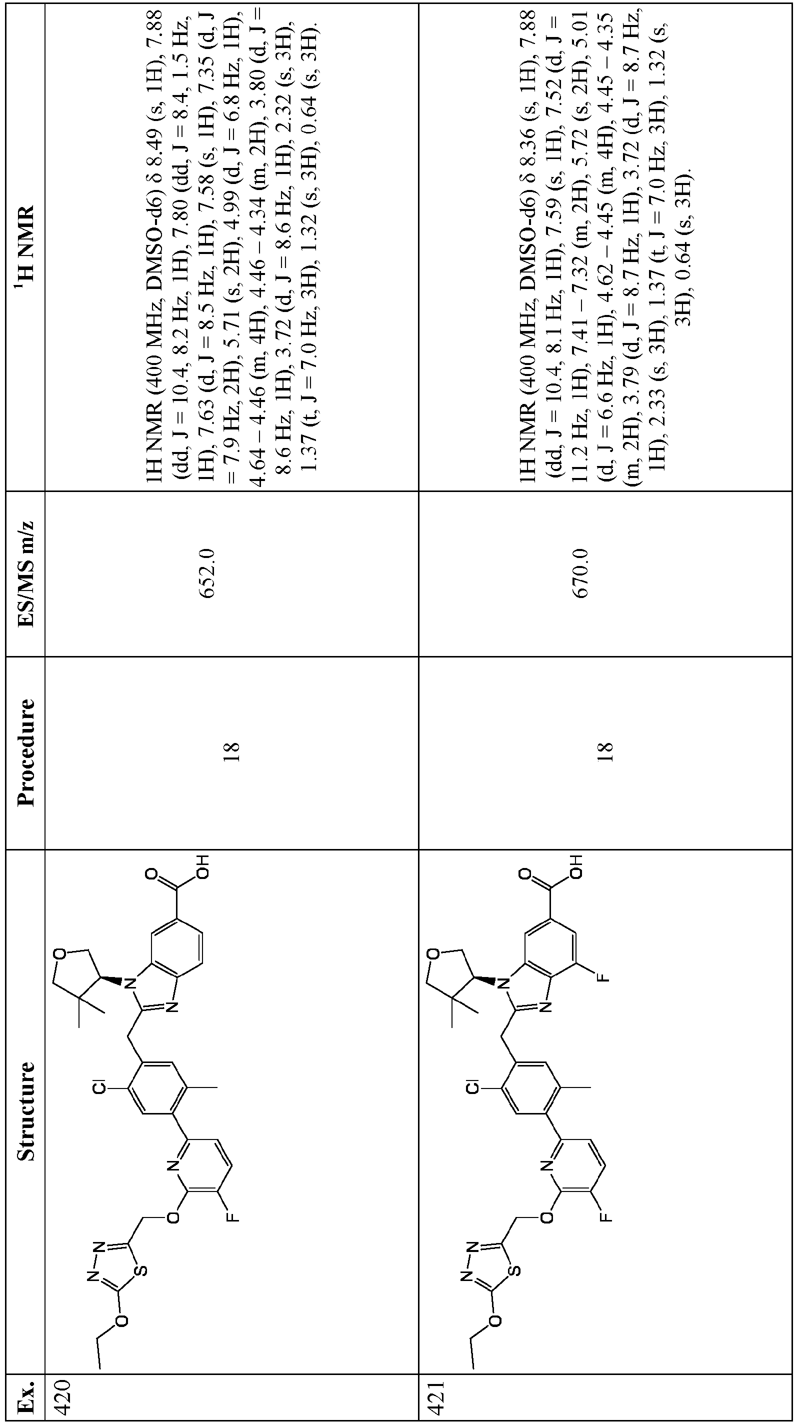

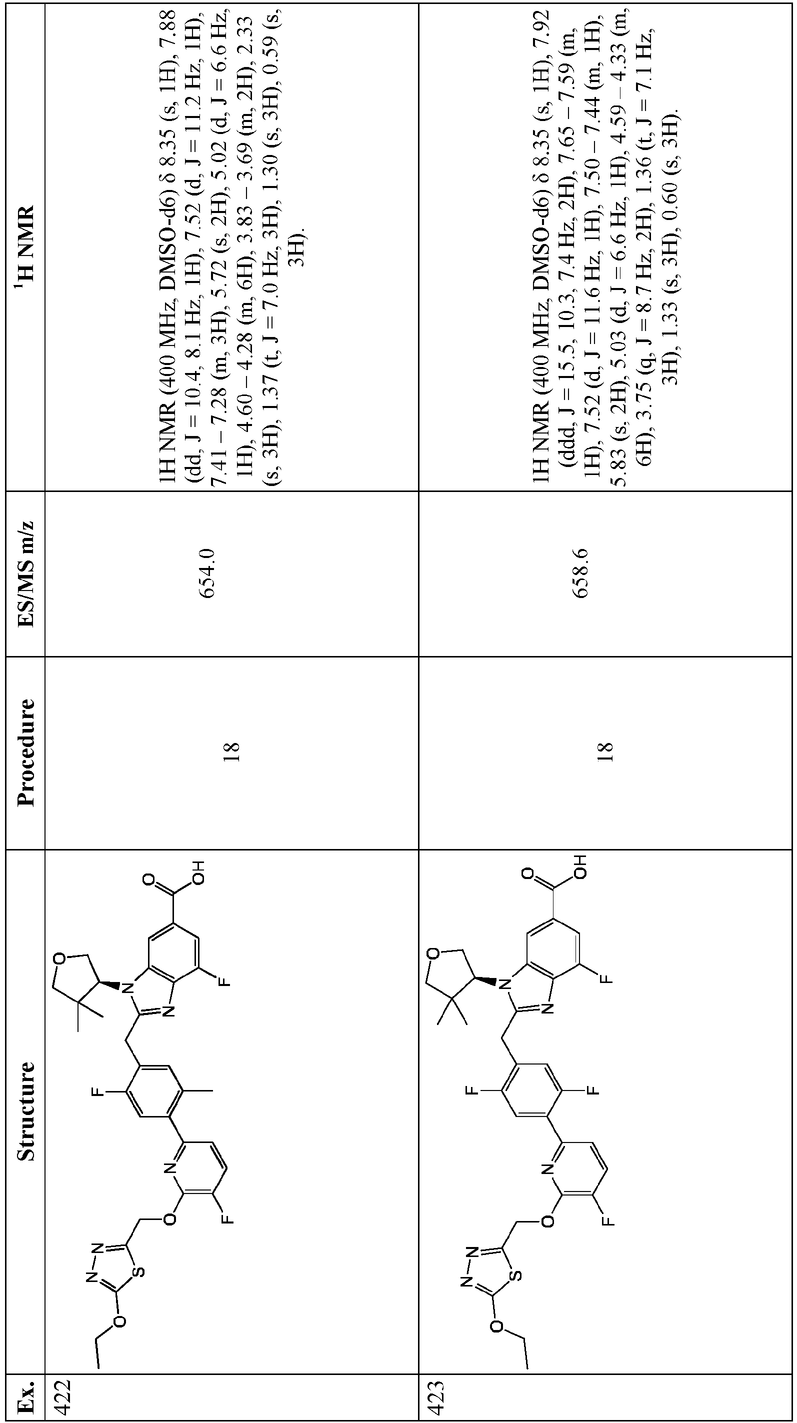

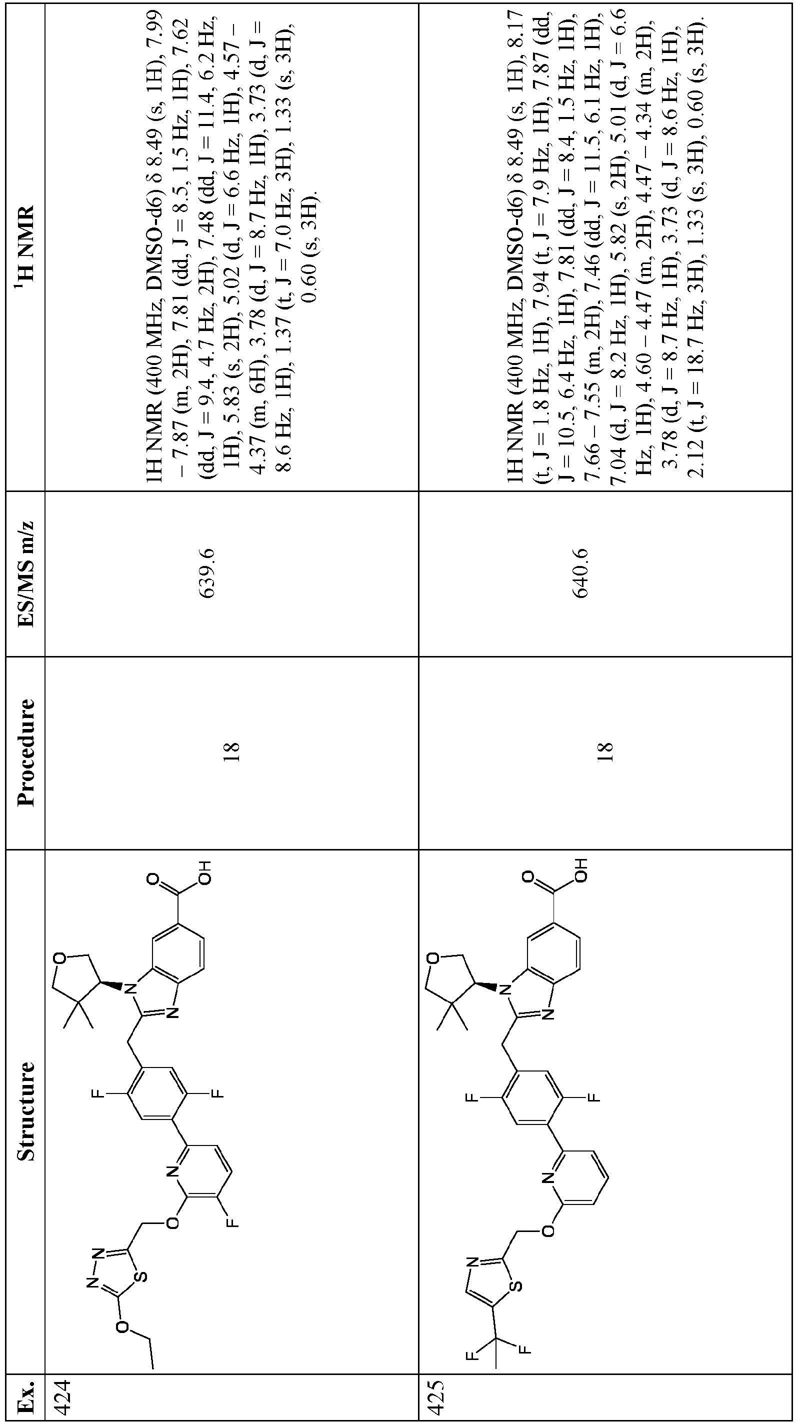

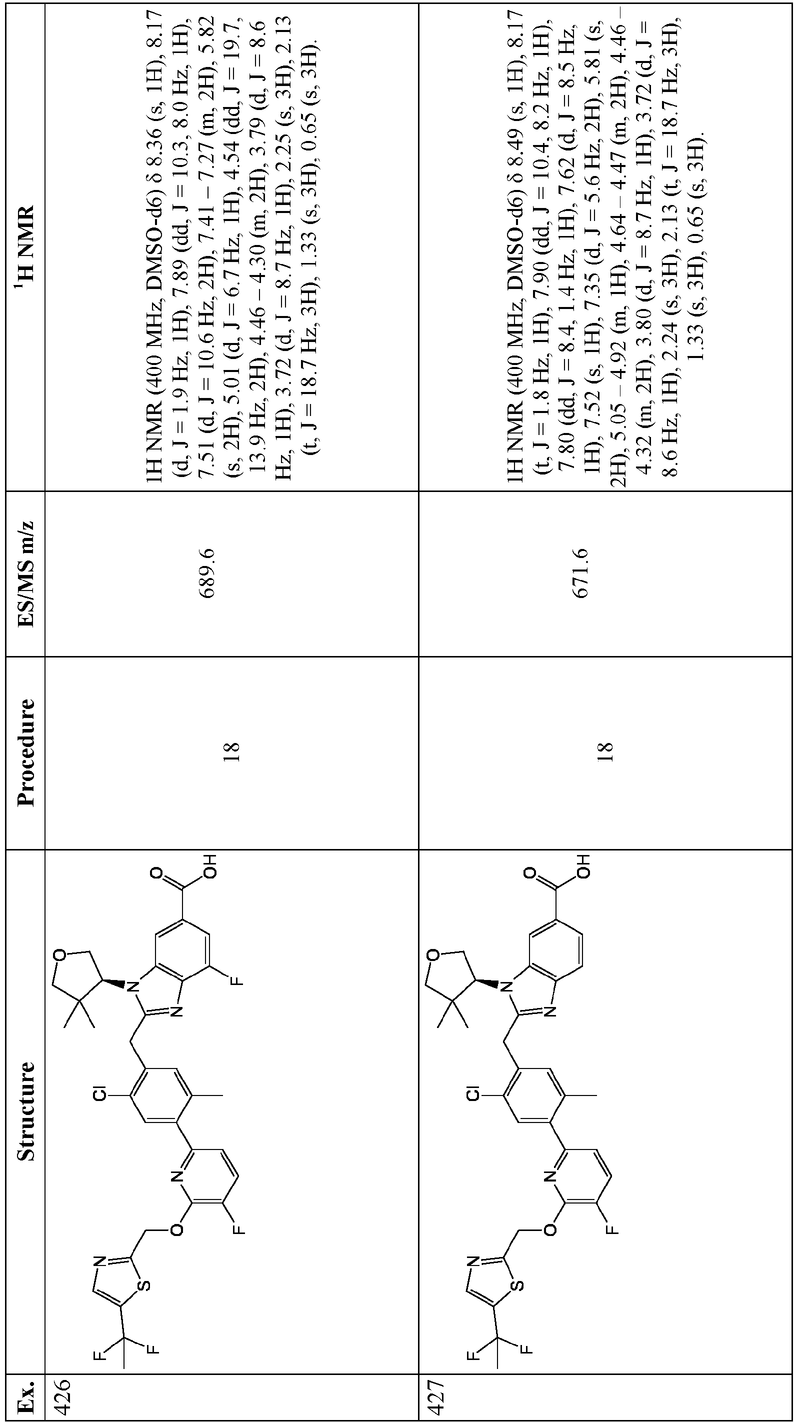

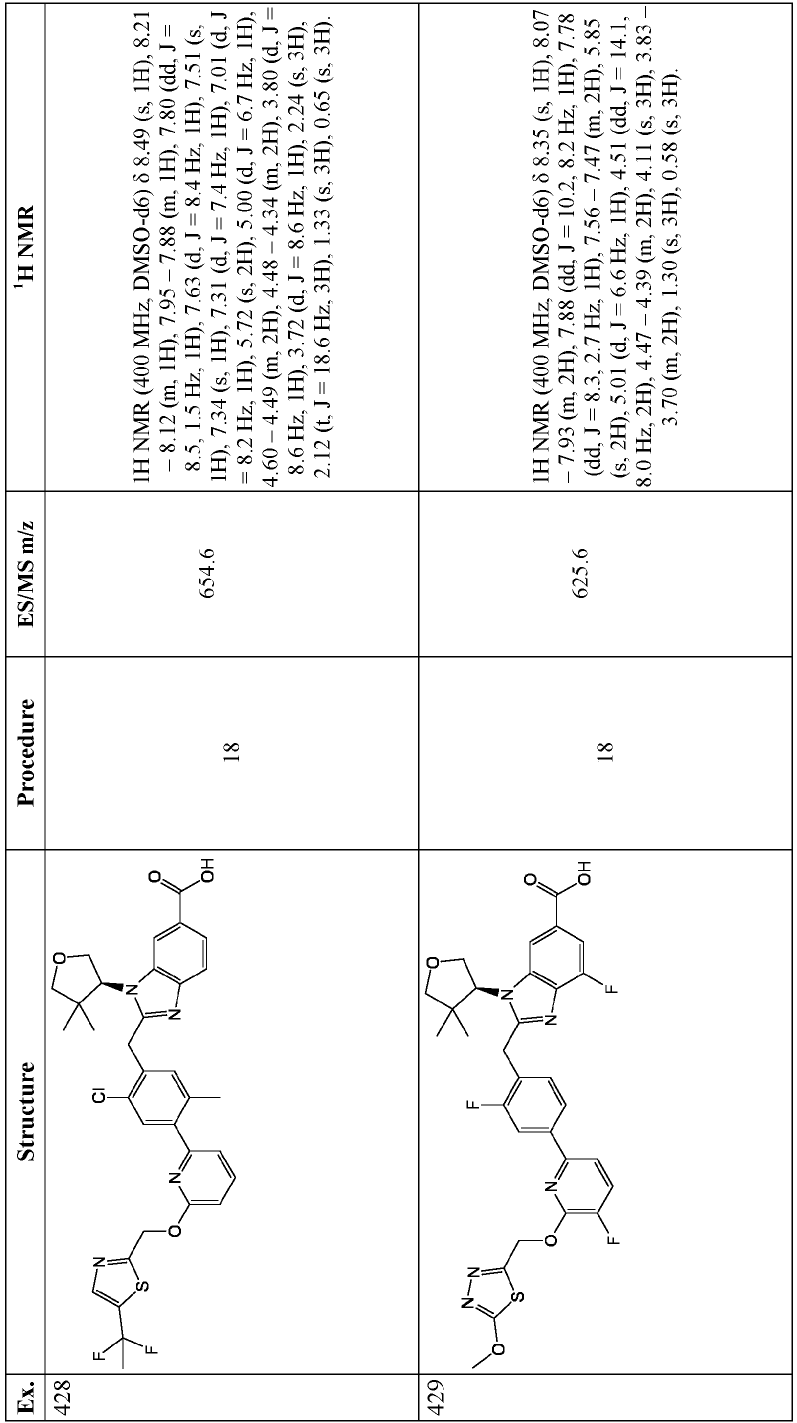

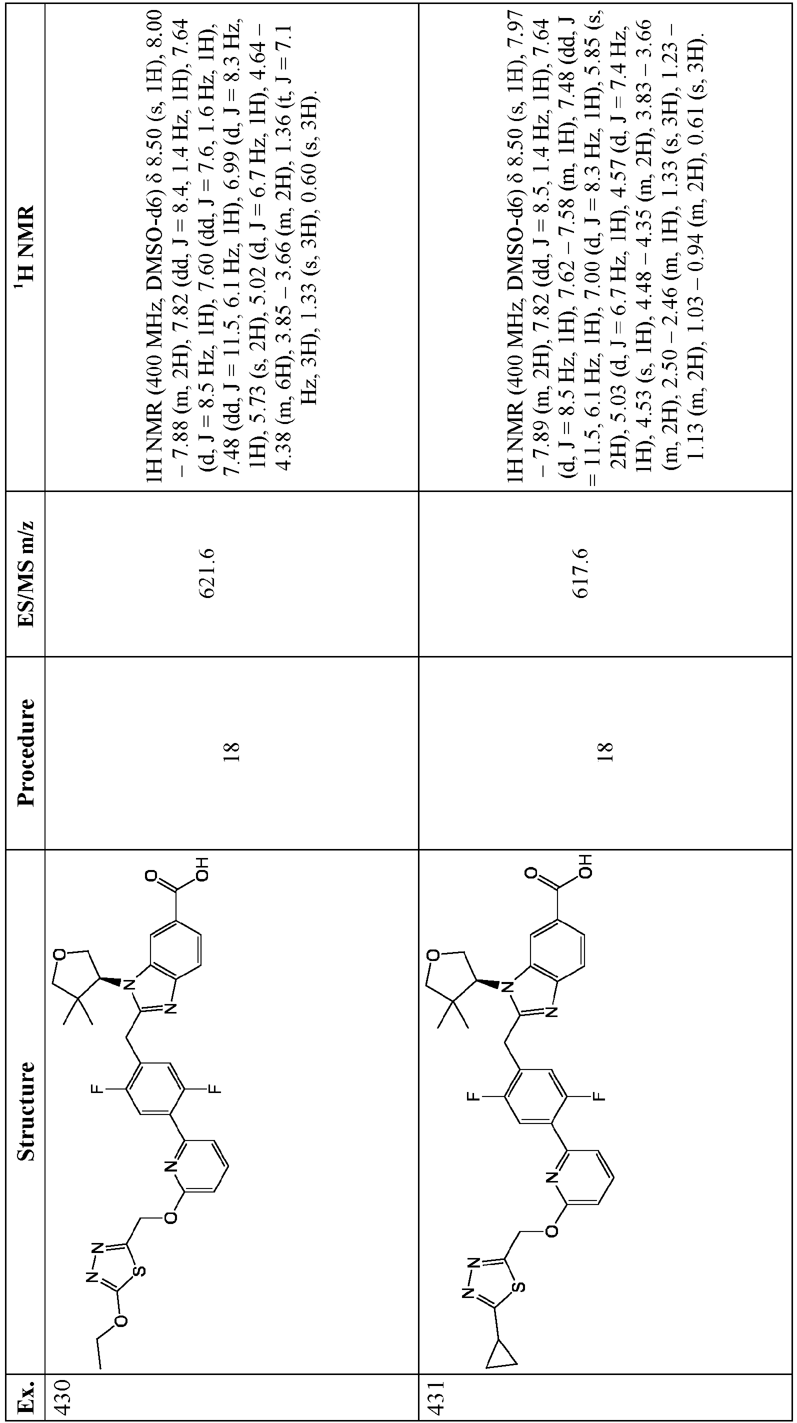

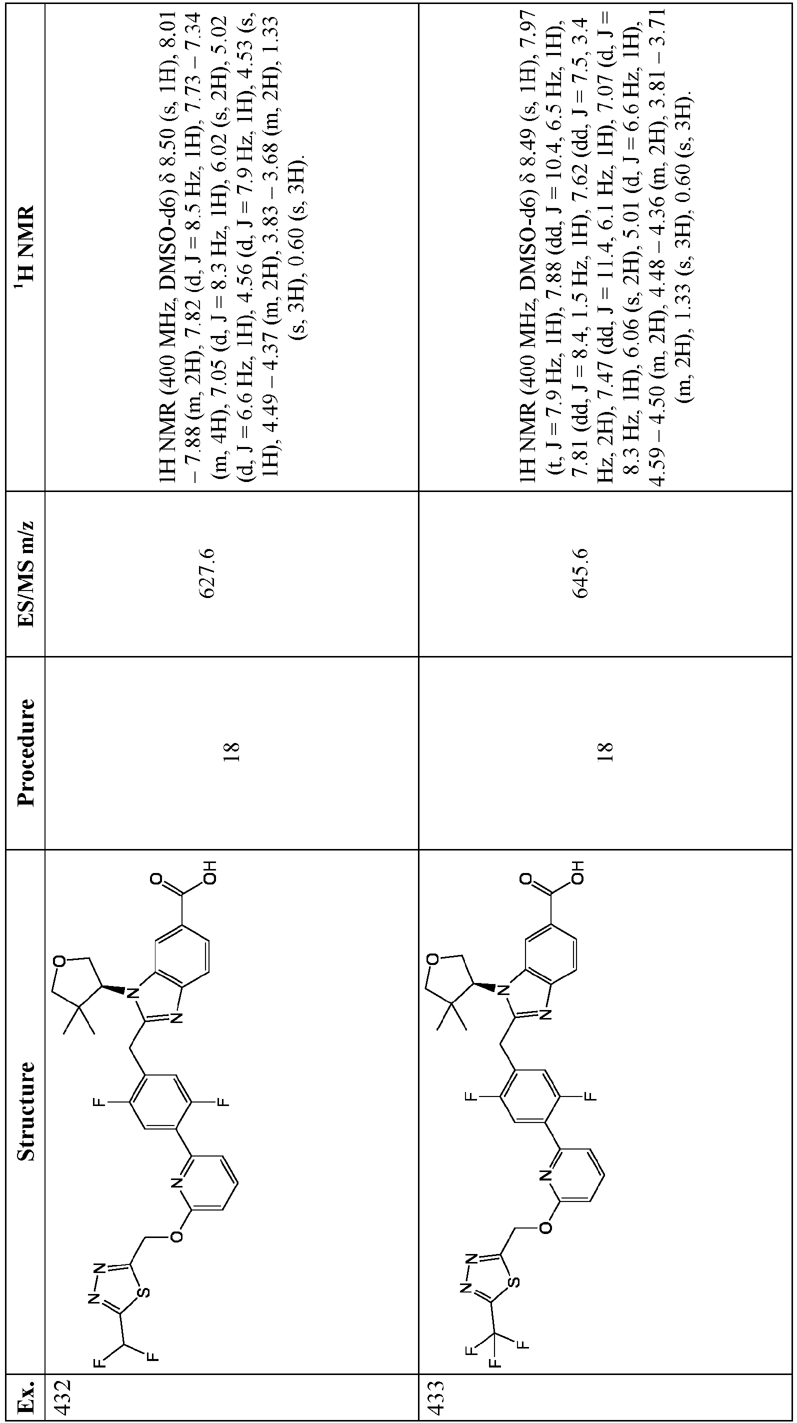

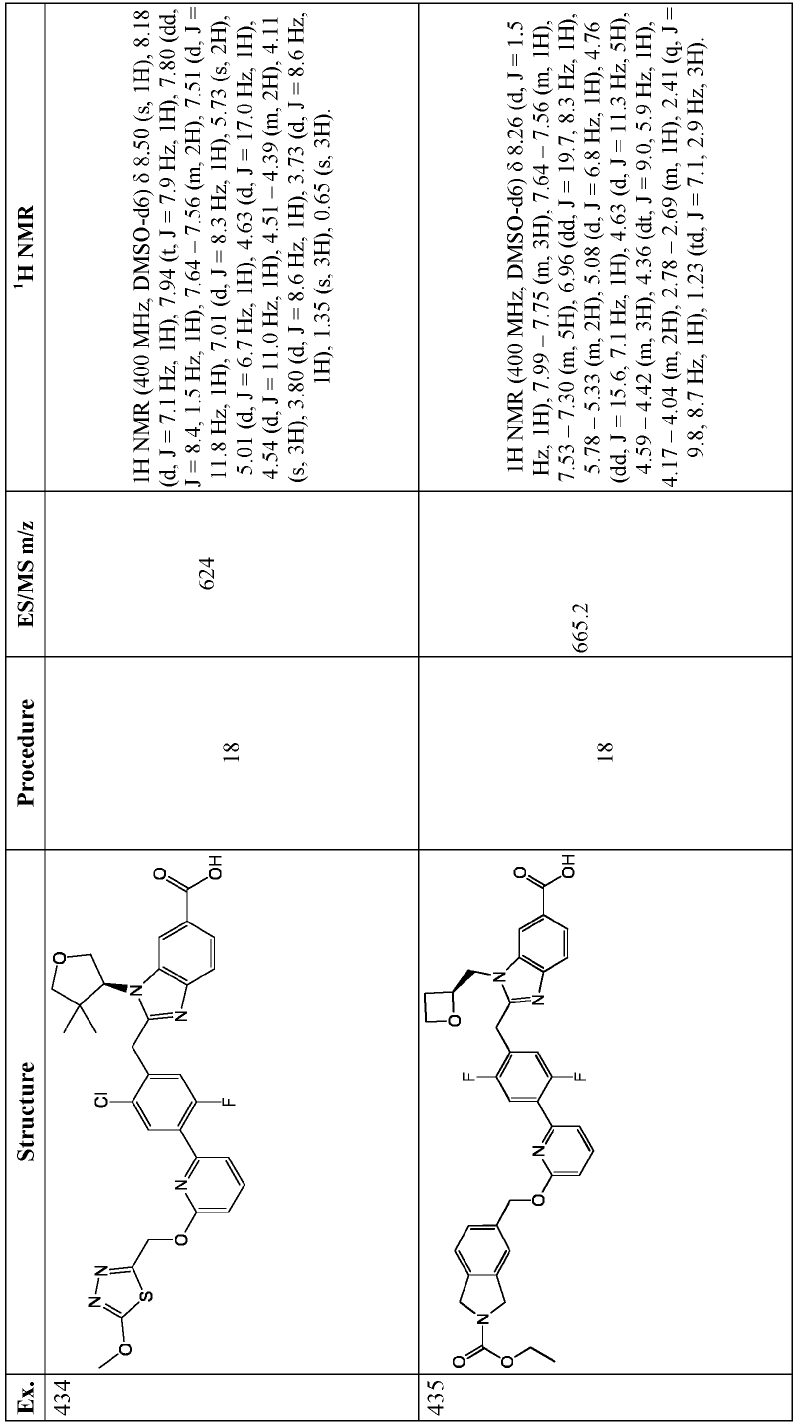

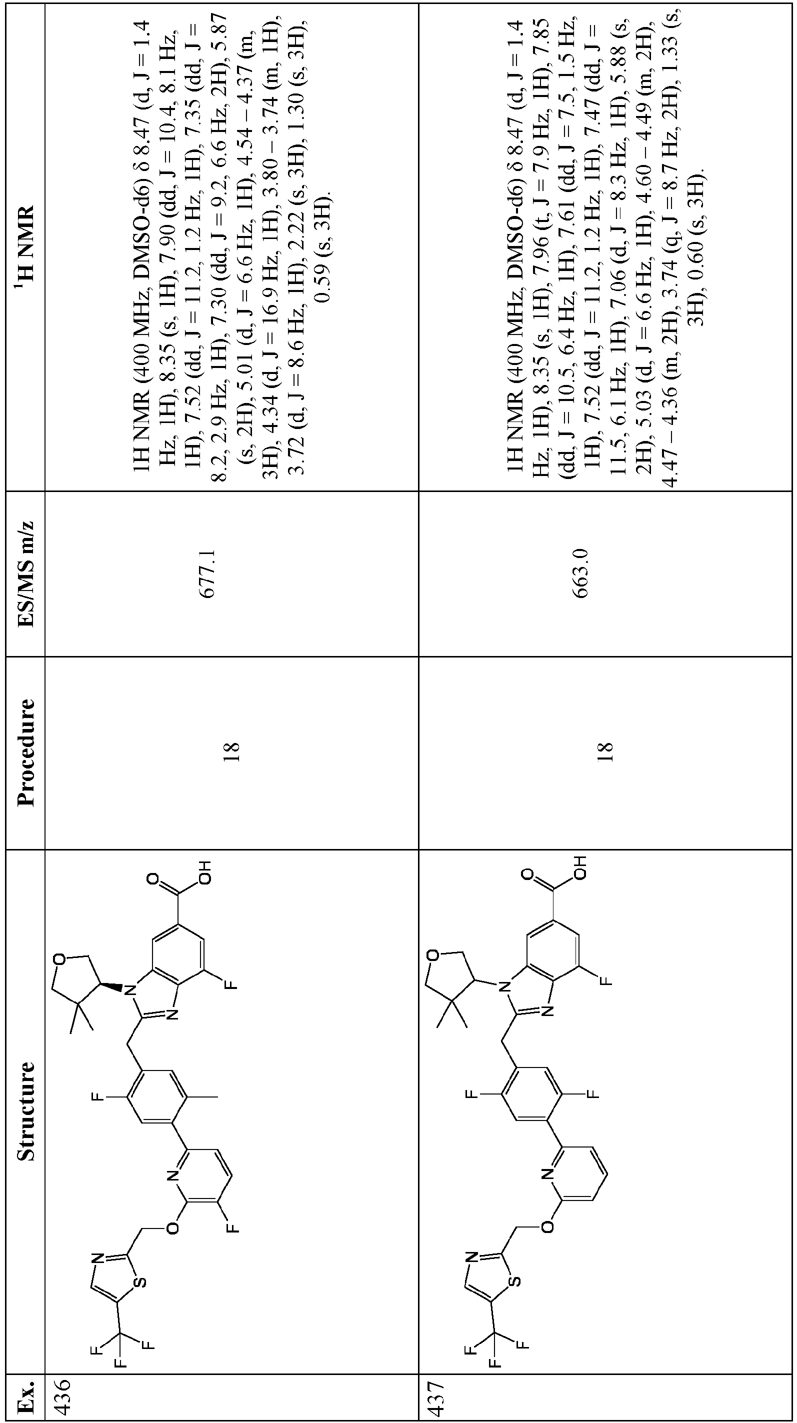

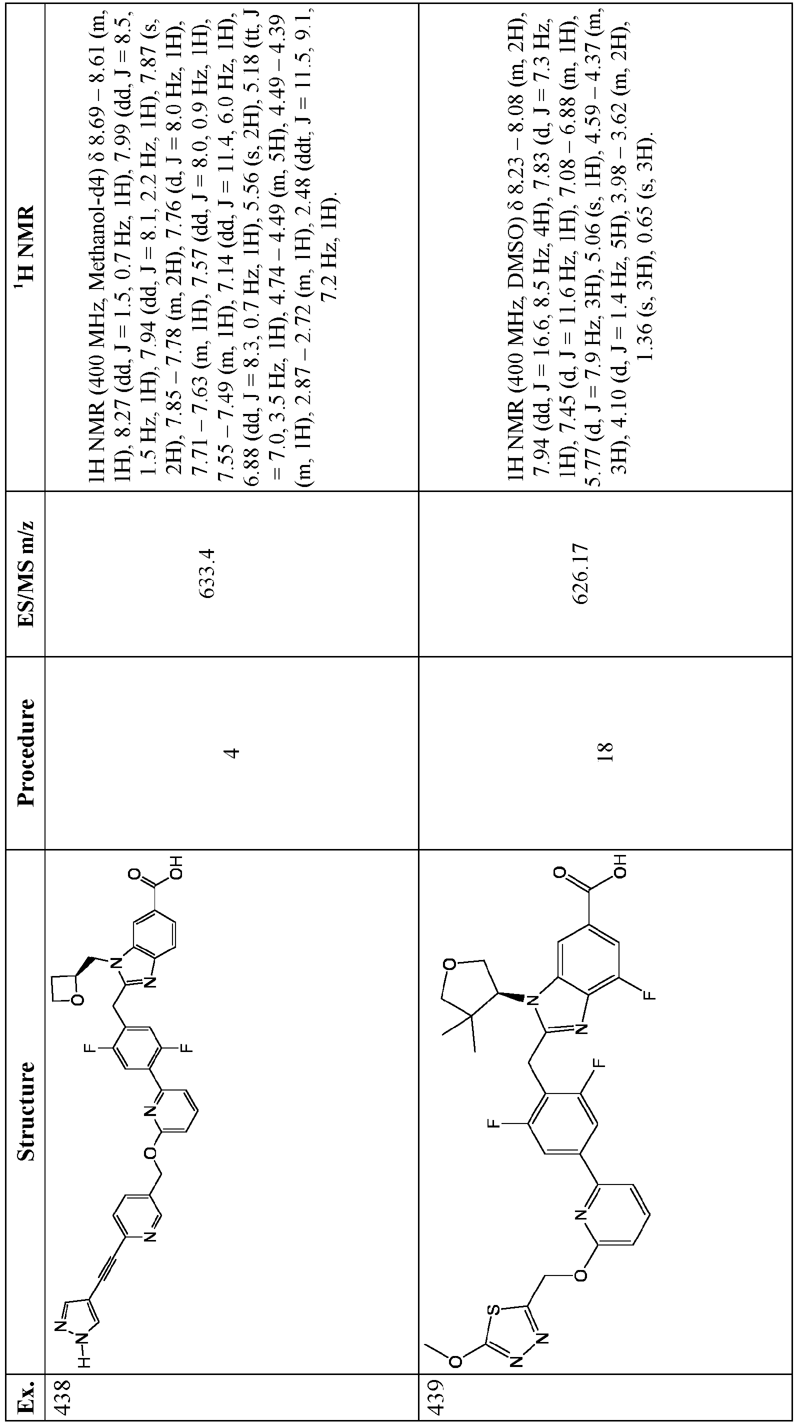

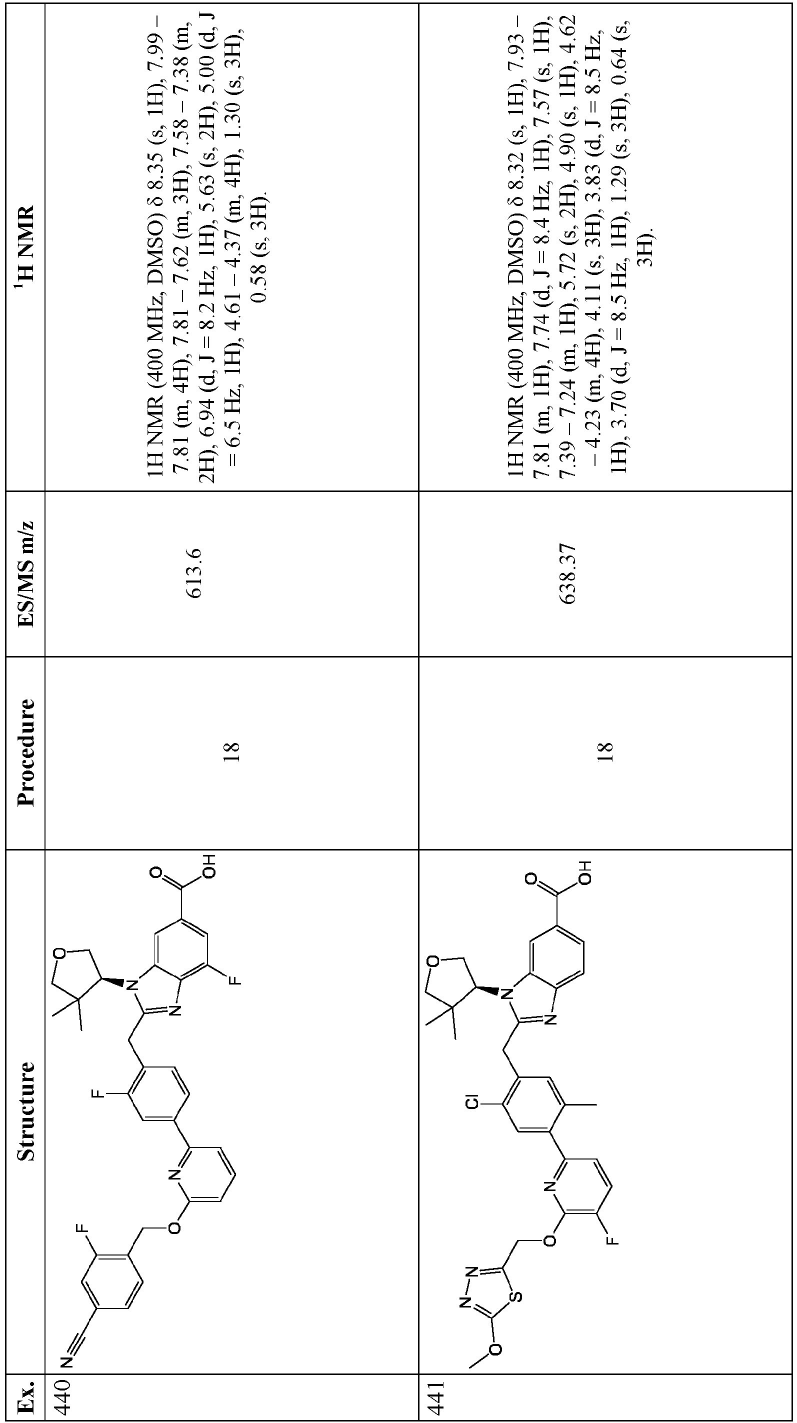

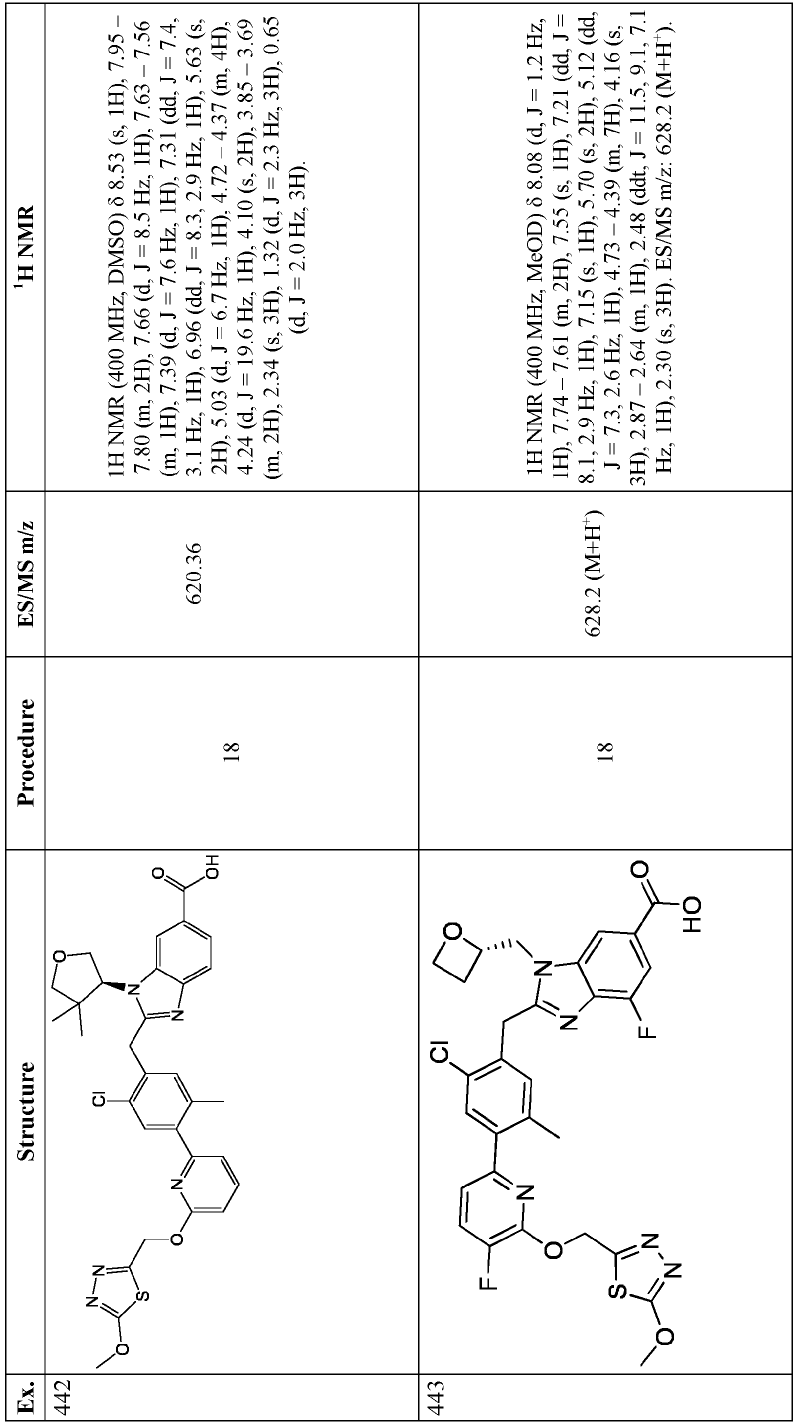

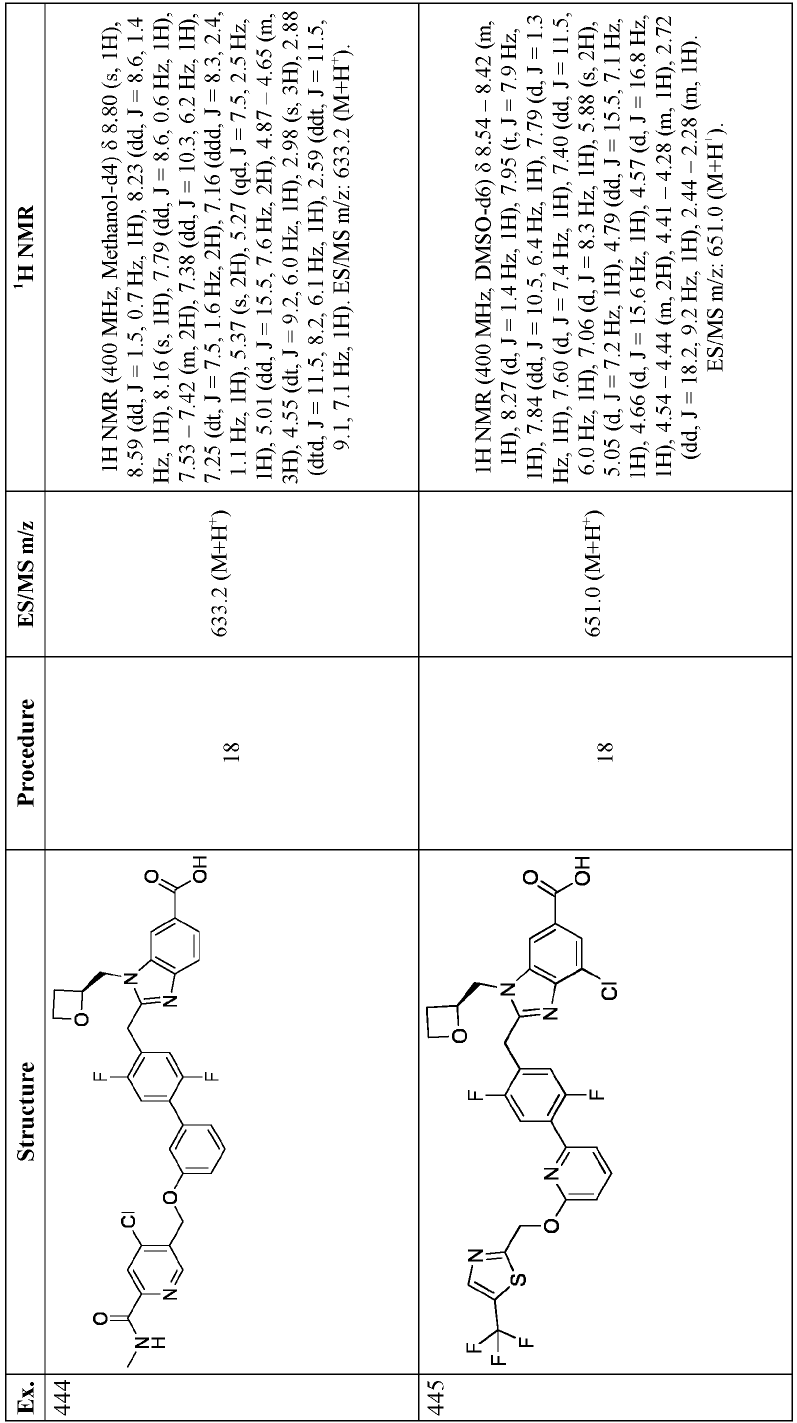

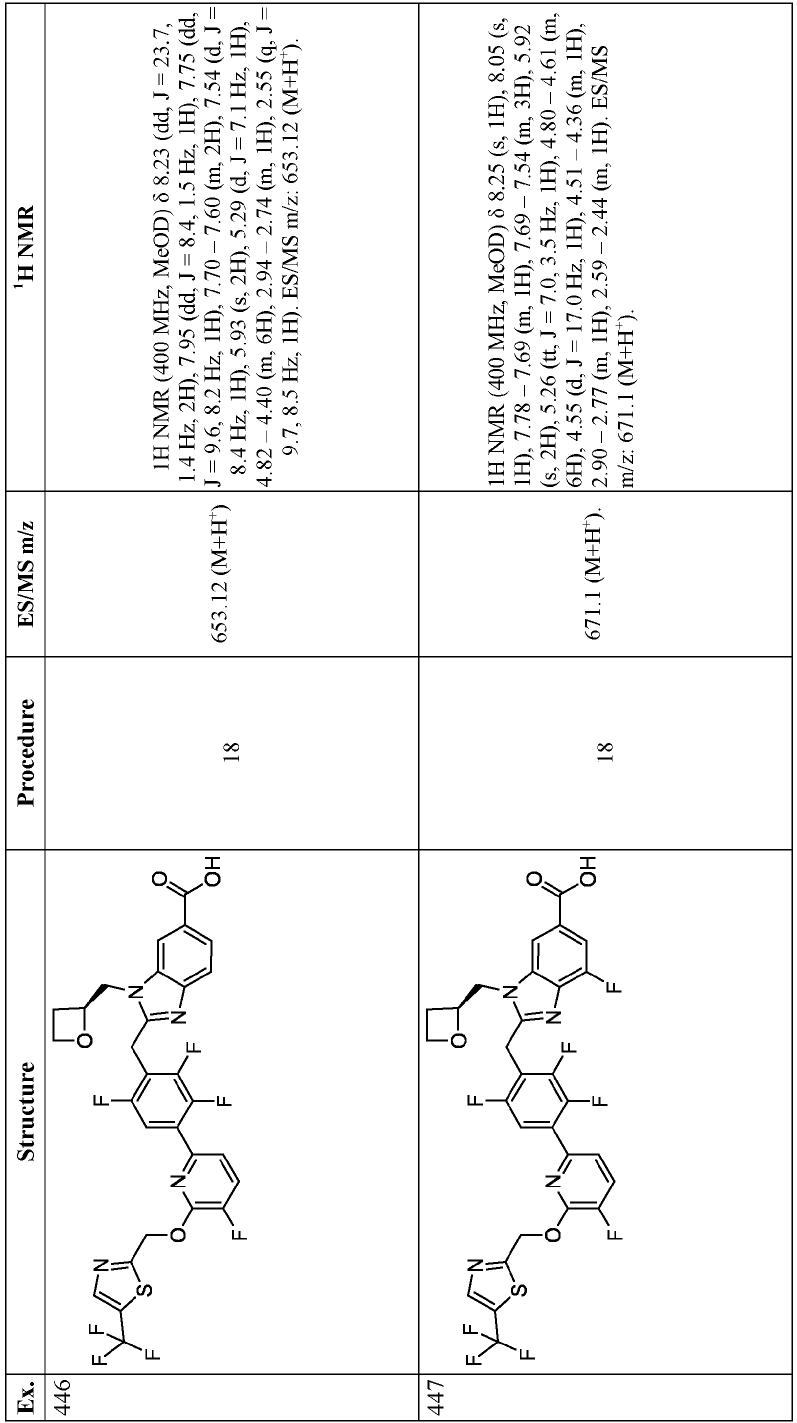

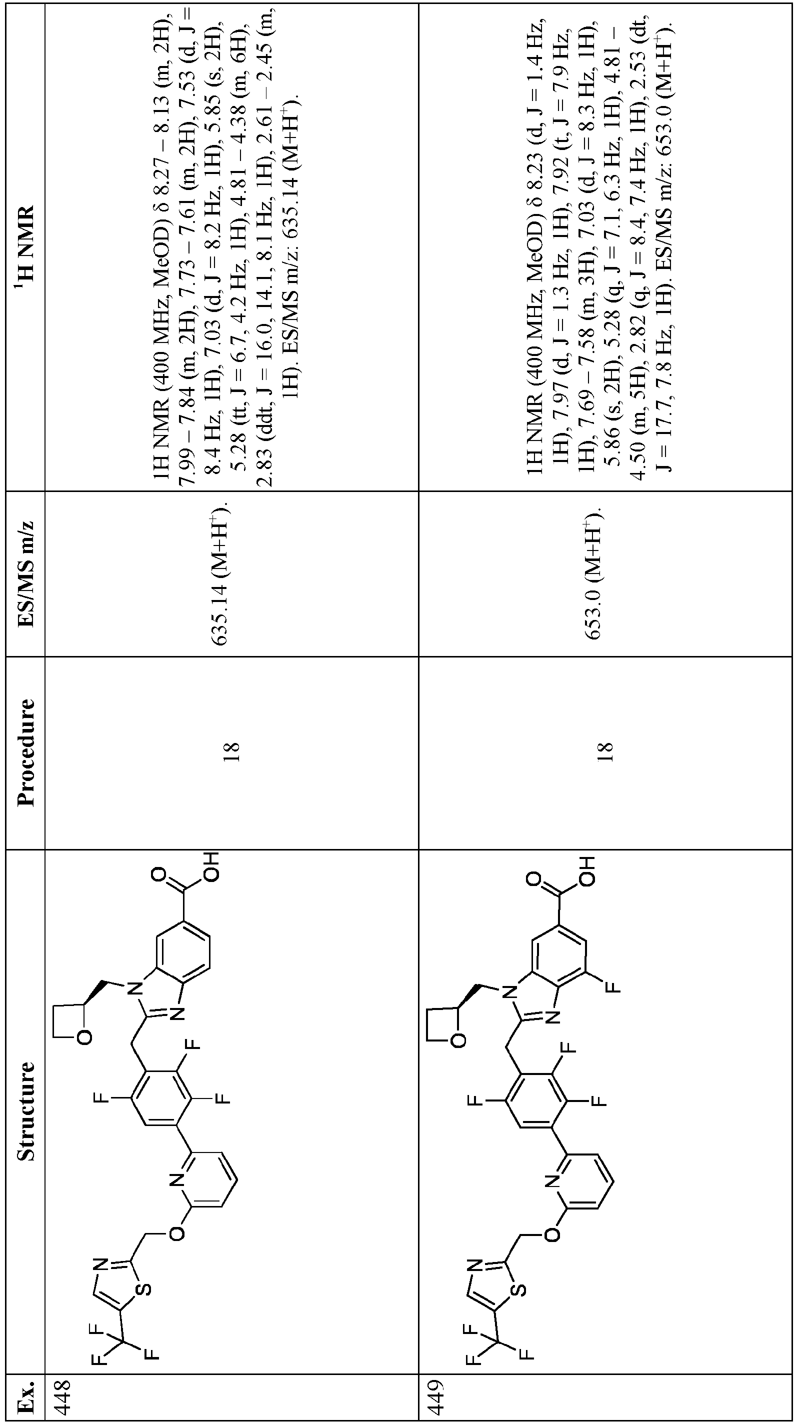

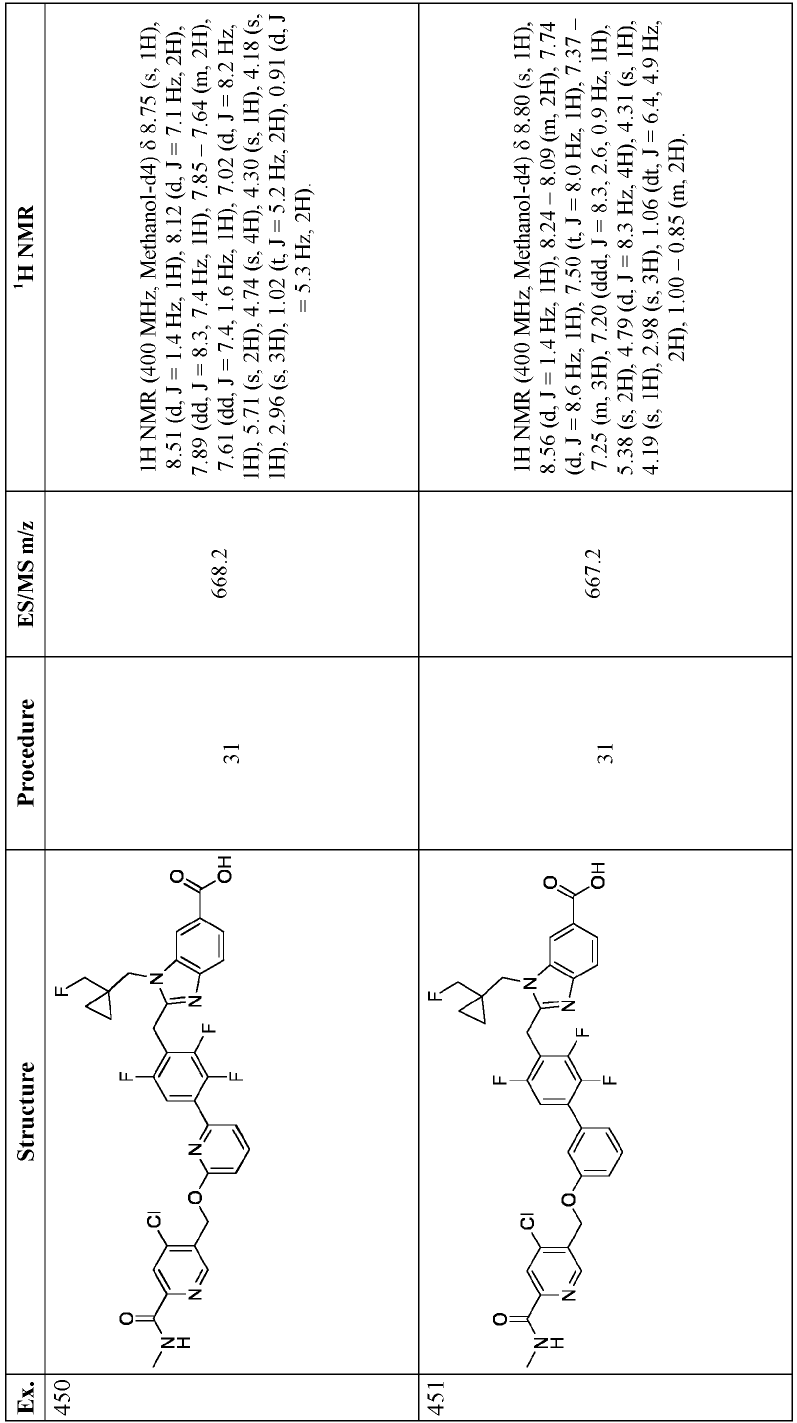

### C. BIOLOGICAL DATA

### GLP-1R ACTIVATION - cAMP Assay 1

GLP-1R activation by compounds of the present disclosure was quantified by measuring cAMP increase in CHO cells stably expressing GLP-1R (MultiSpan product # C1267-1a). The cells were harvested and plated in growth medium (DMEM/F-12 (Corning product # 10-090-CV) supplemented with 10% FBS (HyClone product # SH30071-03), penicillin/streptomycin (Corning product # 30-002CI) and 10µg/ml puromycin (Gibco product # A11138-03)) at 1,000 cells/well in a 384-well plate (Greiner product # 781080). The cells were then incubated overnight at 37 °C, 5% CO₂. The next day, the medium was removed and the cells were washed with DPBS (Corning product # 21-031-CM) before adding the assay medium (HBSS, Corning product # 21-023-CV) with 20mM Hepes (Gibco product # 15630-080) and 0. 1% BSA (Rockland Immunochemicals product # BSA-1000)). Following the medium change, the cells were incubated for 1 hour at 37 °C, 5% CO₂. The tested GLP-1 compound was added to the cells in a 10-point dose response followed by a 30-minute incubation at 37 °C, 5% CO₂. cAMP concentration increase was then detected using Cisbio's cAMP Gs Dynamic Kit (product # 62AM4PEC) according to the manufacturer's protocol. The response was plotted against the log of the agonist concentration and fitted to a sigmoidal equation to determine the EC₅₀.

### GLP-1R ACTIVATION - cAMP Assay 2

GLP-1R activation by small molecule agonists was quantified by measuring cAMP increase in CHO cells stably expressing GLP-1R (MultiSpan product # C1267-1a). 50nL of agonists were pre-spotted in a 10-point dose response onto 384-well plate (Corning product # CL3826) using the Labcyte Echo System. The cells were harvested and plated in assay buffer (HBSS (Corning product # 21-023-CV) with 20mM Hepes (Gibco product # 15630-080) and 0.1% BSA (Rockland Immunochemicals product # BSA-1000) or 100% Human Plasma (Innovative Research product #50-643-396) at 1,000 cells/well, 10µL/well, onto the pre-spotted plates. The cells are then incubated for 30 minutes at 37⁰C, 5% CO₂. cAMP concentration increase was then detected using Cisbio's cAMP Gs Dynamic Kit (product # 62AM4PEC) according to the manufacturer's protocol. The response was plotted against the log of the agonist concentration and fitted to a sigmoidal equation to determine the EC₅₀.

Compounds were run on one of the two GLP-1R Activation cAMP assays and the result are listed in Table 3. below.

**Table 3. Activity**

| **Example No.** | **GLP-1R Activation - Assay EC50 (nm)** | **Example No.** | **GLP-1R Activation - Assay EC50 (nm)** |
|---|---|---|---|
| 1 | 0.27 | 228 | 26.00 |
| 2 | 0.11 | 229 | 17.87 |
| 3 | 3.15 | 230 | 0.33 |
| 4 | 1.43 | 231 | 1.51 |
| 5 | 0.50 | 232 | 5.65 |
| 6 | 3.09 | 233 | 1.20 |
| 7 | 3.71 | 234 | 5.90 |
| 8 | 0.92 | 235 | 1.10 |
| 9 | 0.95 | 236 | 0.22 |
| 10 | 7.55 | 237 | 3.68 |
| 11 | 0.60 | 238 | 8.61 |
| 12 | 1.16 | 239 | 446.57 |
| 13 | 0.54 | 240 | 1.13 |
| 14 | 5.68 | 241 | 0.12 |
| 15 | 0.96 | 242 | 0.02 |
| 16 | 8.86 | 243 | 0.17 |
| 17 | 7.00 | 244 | 2.28 |
| 18 | 0.32 | 245 | 0.10 |
| 19 | 23.24 | 246 | 0.57 |
| 20 | 0.33 | 247 | 0.25 |
| 21 | 0.41 | 248 | 5.61 |
| 22 | 0.17 | 249 | 36.08 |
| 23 | 1.43 | 250 | 22.60 |
| 24 | 1.54 | 251 | 0.89 |
| 25 | 0.53 | 252 | 0.075 |
| 26 | 0.23 | 253 | 0.026 |
| 27 | 0.08 | 253 | 0.12 |
| 28 | 0.44 | 254 | 0.374 |
| 29 | 13.75 | 255 | 0.366 |
| 30 | 1.99 | 256 | 0.443 |
| 31 | 1.32 | 257 | 0.583 |
| 32 | 0.79 | 258 | 0.483 |
| 33 | 1.75 | 259 | 0.577 |
| 34 | 0.20 | 260 | 0.254 |
| 35 | 0.22 | 261 | 0.044 |
| 36 | 5.28 | 262 | 0.134 |
| 37 | 8.46 | 263 | 0.131 |
| 38 | 0.60 | 265 | 0.152 |
| 39 | 0.86 | 266 | 0.367 |
| 40 | 0.83 | 267 | 0.401 |
| 41 | 0.17 | 268 | 0.762 |
| 42 | 0.07 | 269 | 0.748 |
| 43 | 0.30 | 270 | 0.191 |
| 44 | 75.29 | 271 | 0.897 |
| 46 | 0.14 | 272 | 1.899 |
| 47 | 0.09 | 273 | 0.144 |
| 48 | 2.33 | 274 | 0.196 |
| 49 | 0.65 | 275 | 0.372 |
| 50 | 0.67 | 276 | 1.313 |
| 51 | 0.21 | 277 | 0.312 |
| 52 | 0.13 | 278 | 0.617 |
| 53 | 0.38 | 279 | 0.955 |
| 54 | 0.09 | 280 | 0.067 |
| 55 | 0.44 | 281A | 0.27 |
| 56 | 7.19 | 281B | 20.799 |
| 57 | 0.73 | 282 | 0.311 |
| 58 | 0.14 | 283 | 0.109 |
| 59 | 2.38 | 284 | 0.183 |
| 60 | 1.44 | 285 | 0.126 |
| 61 | 0.96 | 286 | 0.042 |
| 62 | 1.98 | 287 | 0.185 |
| 63 | 0.89 | 288 | 0.067 |
| 64 | 6.38 | 289 | 0.262 |
| 65 | 0.19 | 290 | 0.155 |
| 66 | 6.65 | 291 | 0.689 |
| 67 | 6.52 | 292 | 0.499 |
| 68 | 5.37 | 293 | 0.184 |
| 69 | 2.59 | 294 | 0.182 |
| 70 | 1.93 | 295 | 0.202 |
| 71 | 0.29 | 296 | 0.036 |
| 72 | 0.82 | 297 | 0.508 |
| 73 | 1.10 | 298 | 0.466 |
| 74 | 0.25 | 299 | 2.63 |
| 75 | 0.61 | 300 | 0.823 |
| 76 | 4.23 | 301 | 0.341 |
| 77 | 6.32 | 302 | 0.567 |
| 78 | 9.40 | 303 | 0.252 |
| 79 | 0.06 | 304 | 0.524 |
| 80 | 0.19 | 305 | 0.305 |
| 81 | 0.81 | 306 | 0.621 |
| 82 | 5.53 | 307 | 0.59 |
| 83 | 3.51 | 308 | 0.683 |
| 84 | 0.07 | 309 | 0.084 |
| 85 | 0.05 | 310 | 0.038 |
| 86 | 0.14 | 311 | 0.03 |
| 87 | 0.08 | 312 | 0.044 |
| 88 | 0.44 | 313 | 0.032 |
| 89 | 1.86 | 314 | 0.094 |
| 90 | 0.21 | 315 | 0.764 |
| 91 | 0.12 | 316 | 0.129 |
| 92 | 0.07 | 317 | 0.125 |
| 93 | 0.06 | 318 | 0.028 |
| 94 | 2.87 | 319 | 0.034 |
| 95 | 0.08 | 320 | 0.537 |
| 96 | 0.13 | 321 | 0.246 |
| 97 | 0.11 | 322 | 0.349 |
| 98 | 0.03 | 323 | 0.281 |
| 99 | 0.14 | 324 | 0.123 |
| 100 | 0.09 | 325 | 0.582 |
| 101 | 0.14 | 326 | 0.451 |
| 102 | 13.55 | 327 | 0.381 |
| 103 | 0.23 | 328 | 0.342 |
| 104 | 0.07 | 329 | 0.226 |
| 105 | 0.14 | 330 | 0.431 |
| 106 | 0.14 | 331 | 0.58 |
| 107 | 0.31 | 332 | 0.109 |
| 108 | 0.18 | 333 | 0.205 |
| 109 | 0.62 | 334 | 0.354 |
| 110 | 0.95 | 335 | 0.202 |
| 111 | 0.05 | 336 | 0.261 |
| 112 | 0.13 | 337 | 1.2 |
| 113 | 0.16 | 338 | 0.218 |
| 114 | 0.11 | 339 | 0.043 |
| 115 | 0.07 | 340 | 0.058 |
| 116 | 0.15 | 341 | 0.772 |
| 117 | 0.28 | 342 | 0.039 |
| 118 | 0.44 | 343 | 0.459 |
| 119 | 0.39 | 344 | 0.055 |
| 120 | 2.84 | 345 | 0.027 |
| 121 | 729.57 | 346 | 0.027 |
| 122 | 695.38 | 347 | 0.271 |
| 123 | 2.65 | 348 | 1.868 |
| 124 | 0.81 | 349 | 0.323 |
| 125 | 3.64 | 350 | 0.209 |
| 126 | 0.31 | 351 | 0.182 |
| 127 | 3.72 | 352 | 0.52 |
| 128 | 1.95 | 353 | 0.188 |
| 129 | 3.17 | 354 | 0.622 |
| 130 | 0.57 | 355 | 0.355 |
| 131 | 3.38 | 356 | 0.099 |
| 132 | 0.17 | 357 | 0.508 |
| 133 | 0.30 | 358 | 0.243 |
| 134 | 0.60 | 359 | 0.051 |
| 135 | 0.34 | 360 | 0.174 |
| 136 | 0.25 | 361 | 0.62 |
| 137 | 0.24 | 362 | 0.33 |
| 138 | 0.28 | 363 | 0.295 |
| 139 | 0.28 | 364 | 0.929 |
| 140 | 0.40 | 365 | 0.164 |
| 141 | 0.33 | 366 | 0.56 |
| 142 | 0.25 | 367 | 0.235 |
| 143 | 0.14 | 368 | 0.712 |
| 144 | 0.99 | 369 | 0.683 |
| 145 | 0.25 | 370 | 0.28 |
| 146 | 0.09 | 371 | 1.766 |
| 147 | 0.60 | 372 | 0.024 |
| 148 | 0.51 | 373 | 0.079 |
| 149 | 0.29 | 374 | 0.478 |
| 150 | 10.02 | 375 | 0.372 |
| 151 | 0.10 | 376 | 0.153 |
| 152 | 0.68 | 377 | 0.481 |
| 153 | 0.36 | 378 | 0.068 |
| 154 | 0.52 | 379 | 0.303 |
| 155 | 0.83 | 380 | 0.078 |
| 156 | 1.19 | 381 | 0.301 |
| 157 | 3.07 | 382 | 1.271 |
| 158 | 0.20 | 383 | 0.6 |
| 159 | 0.18 | 384 | 0.563 |
| 160 | 0.20 | 385 | 0.11 |
| 161 | 0.08 | 386 | 0.121 |
| 162 | 0.16 | 387 | 0.095 |
| 163 | 0.70 | 388 | 0.186 |
| 164 | 0.10 | 389 | 0.257 |
| 165 | 2.00 | 390 | 0.235 |
| 166 | 0.62 | 391 | 0.305 |
| 167 | 0.87 | 392 | 0.206 |
| 168 | 21.31 | 393 | 0.777 |
| 169 | 11.82 | 394 | 0.62 |
| 170 | 8.29 | 395 | 0.197 |
| 171 | 2.94 | 396 | 0.678 |
| 172 | 2.57 | 397 | 0.497 |
| 173 | 23.24 | 398 | 0.017 |
| 174 | 40.62 | 399 | 0.01 |
| 175 | 0.11 | 400 | 0.412 |
| 176 | 2.19 | 401 | 0.487 |
| 177 | 34.57 | 402 | 0.208 |
| 178 | 23.59 | 403 | 0.096 |
| 179 | 4.59 | 404 | 0.689 |
| 180 | 28.04 | 405 | 0.363 |
| 181 | 5.99 | 406 | 0.385 |
| 182 | 0.19 | 407 | 0.239 |
| 183 | 0.26 | 408 | 0.381 |
| 184 | 0.12 | 409 | 0.695 |
| 185 | 0.08 | 410 | 0.551 |
| 186 | 0.91 | 411 | 0.315 |
| 187 | 0.12 | 412 | 0.099 |
| 188 | 0.05 | 413 | 0.36 |
| 189 | 0.30 | 414 | 0.318 |
| 190 | 0.20 | 415 | 0.189 |
| 191 | 0.69 | 416 | 0.336 |
| 192 | 0.32 | 417 | 0.26 |
| 193 | 0.22 | 418 | 0.384 |
| 194 | 0.17 | 419 | 0.096 |
| 195 | 0.11 | 420 | 0.178 |
| 196 | 0.29 | 421 | 0.087 |
| 197 | 0.08 | 422 | 0.066 |
| 198 | 0.04 | 423 | 0.023 |
| 199 | 0.53 | 424 | 0.027 |
| 200 | 0.24 | 425 | 0.535 |
| 201 | 0.39 | 426 | 0.931 |
| 202 | 0.58 | 427 | 1.418 |
| 203 | 1.10 | 428 | 3.628 |
| 204 | 5.57 | 429 | 0.024 |
| 205 | 0.25 | 430 | 0.039 |
| 206 | 43.28 | 431 | 0.195 |
| 207 | 0.46 | 432 | 0.093 |
| 208 | 0.02 | 433 | 0.413 |
| 209 | 0.14 | 434 | 0.087 |
| 210 | 15.65 | 435 | 0.427 |
| 211 | 0.51 | 436 | 0.367 |
| 212 | 0.10 | 437 | 0.105 |
| 213 | 0.48 | 438 | 0.335 |
| 214 | 0.08 | 439 | 0.397 |
| 215 | 0.18 | 440 | 0.041 |
| 216 | 0.72 | 441 | 0.475 |
| 217 | 0.27 | 442 | 0.669 |
| 218 | 0.80 | 443 | 3.449 |
| 219 | 0.85 | 444 | 0.433 |
| 220 | 2.08 | 445 | 0.101 |
| 221 | 1072.83 | 446 | 0.170 |
| 222 | 3.59 | 447 | 0.020 |
| 223 | 0.51 | 448 | 0.118 |
| 224 | 1.11 | 449 | 0.081 |
| 225 | 0.70 | 450 | 0.050 |
| 226 | 8.36 | 451 | 0.611 |
| 227 | 3.45 | | |

### PHAMACOKINETIC (PK) STUDIES

### Test Articles:

Examples 54 and 253 were tested along with comparative examples CE-1 and CE-2.

### DOSE FORMULATION PREPARATION

Compounds CE-2, Example 54 or Example 253 were combined with a mixture of 10% NMP (v:v) and 90% PEG300 (v:v), and the mixture was stirred until a homogeneous solution was obtained (1 mg/mL final concentration of test article).

Compound CE-1 was combined with a mixture of 15% 2-hydroxypropyl-β-cyclodextrin (w:v) and 85% Mili-Q water (v:v), and the mixture was stirred until a homogeneous solution was obtained (0.5 mg/mL final concentration of test article).

### STUDY DESIGN

Young adult non-naive Cynomolgus monkeys with weight about 2.5-5kg (n = 1-3/test article/route)were fasted overnight and then dosed with either CE-1 (at 1 mg test article per kg animal weight), CE-2 (at 2 mg test article per kg animal weight), Example 54 (at 2 mg test article per kg animal weight), or Example 253 (at 2 mg test article per kg animal weight). Food was supplied approximately 4 hours post dose and animals were provided with free access to water all the time. Animals were restrained at designated time points for blood sampling. Approximately 500 µL of blood samples was taken via cephalic or saphenous vein into EDTA-K2 tubes. For oral administration **(PO):** blood samples were generally collected pre- dose, and at 15 min, 30 min, 1 hr, 2 hr, 4 hr, 8 hr, 12 hr, and 24 hr time points after dosing. Blood was maintained on wet ice, in chilled cryoracks, or at approximately 5°C prior to centrifugation to obtain plasma. Centrifugation was conducted within 1 hour of collection. Plasma (approximately 200 µL) was placed into a micronic tubes containing 4 µL of formic acid (the final concentration of formic acid in plasma was approximately 2%) and samples were vortex mixed. Samples were maintained on dry ice prior to storage at approximately -70°C.

### Bioanalytical Analysis

LC-MS/MS assays were utilized to quantify the concentration of the test articles in plasma as follows.

### Sample Preparation

50 µL plasma sample was mixed with 350 µL of ACN combined with an internal standard. The mixture was then vortexed for about 1-5 min, and centrifuged at about 4,000-5,800 rpm for about 10-15 mm. An aliquot of 1 µL supernatant was used for LC-MS/MS analysis.

### LC-MS/MS Method

MS conditions: A Sciex API 6500 equipped with electrospray ionization in the positive ion selected reaction monitoring mode was used for detection.
HPLC conditions
Mobile Phase A: H₂O-0.1% Formic Acid
Mobile Phase B: ACN-0.1% Formic Acid

| **Time (min)** | **Mobile Phase B (%)** |
|---|---|
| **0.10** | 35 |
| **0.55** | 50 |
| **1.10** | 95 |
| **1.22** | 95 |
| **1.26** | 35 |
| **1.50** | stop |

Column: Phenomenex Kinetex XBCl8 (2.l x 50 mm, 2.6 µm) with a flow rate of 1.20 mL/min
Column temperature: 60 °C
Retention time: approximately between 0.85 to 1.05
min

### Pharmacokinetics (PK) Parameters

Pharmacokinetics parameters, including area under the curve (AUC₀₋₂₄), maximum plasma concentration (Cₘₐₓ), time to reach maximum plasma concentration (Tₘₐₓ), oral bioavailability (F%), *etc.,* were calculated using Dotmatics software in non-compartmental model.

The results of the pharmacokinetics studies are presented in FIG. 1 and Table 4. As is apparent in FIG. 1, CE-1 demonstrates low exposures (<10nM plasma concentrations) over a period of 12h after a single oral dose of 1 mg/kg that subsequently drop to below the detection limit. In comparison, CE-2 achieves disproportionately higher exposures 2 mg/kg at a 2-fold higher dose than CE-1 and has sustained exposures over 24h after a single oral dose. However, the oral bioavailability for CE-1 and CE-2 is low, 2.5% and 27.4%, respectively.

In contrast, as illustrated in FIG. 1, the plasma concentrations of orally - administered Example 54 and Example 253 at the same dose level as CE-2 (*i.e.,* 2 mg/kg were higher than CE-1 (134-298-fold higher AUC₀₋₂₄ despite only 2-fold higher dose), and CE-2 (2-5-fold higher AUC₀₋₂₄ for the same dose) over 24 hours after oral dosing. Further, the oral bioavailability of Example 54 and Example 253 were higher than that of CE-1 and CE-2. These data show that compounds disclosed herein can provide prolonged exposures and/or higher oral bioavailability at least within 24h following a single oral dose administration of compound.

**Table 4. In vivo Monkey Oral PK Profiles**

| **Compound No.** | **Oral dose** (Arbitrary unit) | **Cmax** (Arbitrary unit) | **Tmax** (hr) | **AUC₀₋₂₄** (Arbitrary units) | **F%** |
|---|---|---|---|---|---|
| Example 54 | 2 mg/kg | 1270 ± 628 | 4.7 ± 1.2 | 14200 ± 7690 | 89.0 ± 33.0 |
| Example | 2 mg/kg | 582 ± 296 | 5.3 ± 2.3 | 6390 ± 2360 | 55.7 ± 20.6 |
| 253 Compound CE-1 | 1 mg/kg | 4.5 ± 0.8 | 2.2 ± 1.8 | 47.7 ± 13.3 | 2.5 ± 0.2 |
| Compound CE-2 | 2 mg/kg | 224 ± 130 | 4.0 ± 2.0 | 2810 ± 1330 | 27.4 ± 12.9 |

Although the foregoing has been described in some detail by way of illustration and Example for purposes of clarity of understanding, one of skill in the art will appreciate that certain changes and modifications may be practiced within the scope of the claims. In addition, each reference provided herein is incorporated by reference in its entirety to the same extent as if each reference was individually incorporated by reference. Where a conflict exists between the instant application and a reference provided herein, the instant application shall dominate.

The present description further includes the subject matter disclosed in the following clauses 1 to 55:

### CLAUSES:

1. A compound of Formula **(I):** 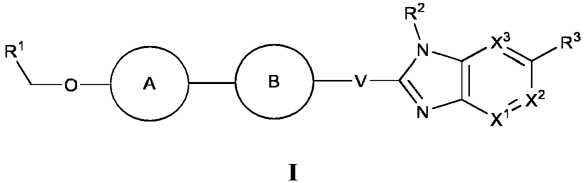 or a pharmaceutically acceptable salt thereof, wherein
   R¹ is
      i) 5-membered heteroaryl, optionally substituted with one to four R⁴;
      ii) 5-membered heteroaryl fused to a heteroaryl or aryl to form a heteroaryl ring system, wherein the heteroaryl ring system is optionally substituted with one to four R⁴;
      iii) 5-membered heteroaryl substituted with two R⁴ groups attached to adjacent ring atoms and optionally substituted with one or two additional R⁴, wherein the two R⁴ groups attached to adjacent ring atoms are combined with the atoms to which they are attached to form a C₅₋₁₀ cycloalkyl or heterocyclyl, which is each optionally substituted with one to four R⁶;
      iv) 6-membered heteroaryl or phenyl substituted with C₂-alkynyl, wherein the 6-membered heteroaryl or phenyl is optionally substituted with one to three R⁴, wherein the C₂-alkynyl is substituted with -C(CH₃)₂SO₂CH₃, aryl, heteroaryl, or heterocyclyl, wherein the aryl, heteroaryl or heterocyclyl is optionally substituted with one to four R⁵;
      v) 6-membered heteroaryl or phenyl substituted with -C(O)NR^{a}R^{b}, wherein the heteroaryl or phenyl is optionally substituted with one to four R⁴; or
      vi) 6-membered heteroaryl or phenyl, wherein the 6-membered heteroaryl or phenyl is substituted with heteroaryl, C₃₋₁₀ cycloalkyl, or heterocycle and optionally substituted with one to three R⁴, wherein the heteroaryl, C₃₋₁₀ cycloalkyl, or heterocycle is each substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl, wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶;
   X¹, X², and X³ are each independently -C(H)=, or -C(R⁸)=; ring A is 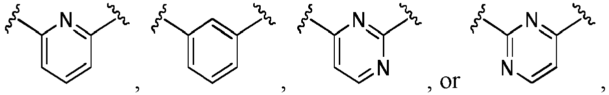 each optionally substituted with one to three Z^{1a} groups, each independently C₁₋₆ alkyl, C₁₋₆ haloalkyl, C₁₋₆ alkoxy, C₁₋₆ haloalkoxy, halogen, -OH, or -CN;
   ring B is C₆₋₁₀ aryl or heteroaryl, which is each optionally substituted with one to four R⁴; V is -C(O)-, -O-, -N(R^{6a})-, or -C(R^{6b})(R^{6c})-;
   R² is H, C₁₋₆ alkyl, C₂₋₆ alkynyl, C₁₋₆ haloalkyl, C₃₋₁₀ cycloalkyl, or heterocyclyl,
      wherein the alkyl, alkynyl, cycloalkyl, or heterocyclyl is each optionally substituted with one to four Z¹, wherein each Z¹ is independently C₁₋₆ alkyl, C₁₋₆ alkoxy, C₁₋₆ hydroxyalkyl, C₂₋₆ alkoxyalkyl, halogen, C₁₋₆ haloalkyl, C₁₋₆ haloalkoxy, -OH, -CN, C₁₋₆ alkyl-CN, -O-C₃₋₆ cycloalkyl, heterocyclyl, C₃₋₁₀ cycloalkyl, or heteroaryl optionally substituted with 1 to 4 groups each independently C₁₋₆ alkyl, C₁₋₆ alkoxy, halogen, -CN, C₁₋₆ haloalkyl, or C₁₋₆ haloalkoxy;
   R³ is -C(O)OR^{3a};
   R^{3a} is H, C₁₋₄ alkyl-N(R^{9a})(R^{9b}), -C₁₋₄ alkyl-N(R^{9a})C(O)-O-C₁₋₄ alkyl-OP(O)(OR^{9c})₂, C₁₋₄ alkyl-C(O)N(R^{9a})(R^{9b}), -C₁₋₄ alkyl-O-C(O)-C₁₋₄ alkyl, -C₁₋₄ alkyl-O-C(O)-O-C₁₋₄alkyl, -C₁₋₄ alkyl-O-C(O)-C₁₋₄ alkyl-N(R^{9a})(R^{9b}), -C₁₋₄ alkyl-O-C(O)-C₁₋₄ alkyl-OP(O)(OR^{9c})₂, -CH₂CH(N(R^{9a})₂)C(O)OR^{9b}, -P(O)(OR^{9c})₂, -OP(O)(OR^{9c})₂, - CH₂P(O)(OR^{9c})₂, -CH₂OP(O)(OR^{9c})₂, -OCH₂P(O)(OR^{9c})₂, C(O)OCH₂P(O)(OR^{9c})₂, -P(O)(R^{9c})(OR^{9d}), -OP(O)(R^{9c})(OR^{9d}), - CH₂P(O)(R^{9c})(OR^{9d}), -OCH₂P(O)(R^{9c})(OR^{9d}), -C(O)OCH₂P(O)(R^{9c})(OR^{9d}), - P(O)(N(R^{9c})₂)₂, -OP(O)(N(R^{9c})₂)₂, -CH₂P(O)(N(R^{9c})₂)₂, -OCH₂P(O)(N(R^{9c})₂)₂, - C(O)OCH₂P(O)(N(R^{9c})₂)₂, -P(O)(N(R^{9c})₂)(OR^{9d}), -OP(O)(N(R^{9c})₂)(OR^{9d}), - CH₂P(O)(N(R^{9c})₂)(OR^{9d}), -OCH₂P(O)(N(R^{9c})₂)(OR^{9d}), - C(O)OCH₂P(O)(N(R^{9c})₂)(OR^{9d}), -P(O)(R^{9c})(N(R^{9d})₂), -OP(O)(R^{9c})(N(R^{9d})₂), - CH₂P(O)(R^{9c})(N(R^{9d})₂), -OCH₂P(O)(R^{9c})(N(R^{9d})₂), - C(O)OCH₂P(O)(R^{9c})(N(R^{9d})₂), or C₁₋₆ alkyl-heterocyclyl,
      wherein the alkyl or heterocyclyl is each optionally substituted with one to four halogens;
   each R⁴ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₅ cycloalkyl, heterocyclyl, C₆₋₁₀ aryl, heteroaryl, oxo, -NO₂, -N₃, -CN, -OR^{10a}, -C(O)R^{10a}, -C(O)O-R^{10a}, -C(O)-N(R^{10a})(R^{10b}), -N(R^{10a})(R^{10b}), -N(R^{10a})₂(R^{10b})⁺, -N(R^{10a})C(O)-R^{10b}, -N(R^{10a})C(O)OR^{10b}, -N(R^{10a})C(O)N(R^{10b})(R^{10c}), -N(R^{10a})S(O)₂(R^{10b}), -N R^{10a}S(O)₂N(R^{10b})(R^{10c}), -NR^{10a}S(O)₂O(R^{10b}), -OC(O)R^{10a}, -OC(O)O R^{10a}, -OC(O)-N(R^{10a})(R^{10b}), -S-R^{10a}, -S(O) R^{10a}, -S(O)(NH)R^{10a}, - S(O)₂R^{10a}, -S(O)₂N(R^{10a})(R^{10b}), -S(O)(NR^{10a}) R^{10b}, or -Si(R^{10a})₃;
      wherein each alkyl, haloalkyl, alkenyl, alkynyl, cycloalkyl, heterocyclyl, aryl, or heteroaryl is optionally substituted with one to four R⁵;
   each R⁵ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, C₃₋₁₅ cycloalkyl, halogen, C₃₋₁₅ cycloalkyl, heterocyclyl, C₆₋₁₀ aryl, heteroaryl, oxo, -NO₂, -N₃, -CN, -O- R^{10a}, -C(O)-R^{10a}, - C(O)OR^{10a}, -C(O)N(R^{10a})(R^{10b}), - N(R^{10a})(R^{10b}), -N(R^{10a})C(O)R^{10b}, -N(R^{10a})C(O)OR^{10b}, -N(R^{10a})C(O)N(R^{10b})(R^{10c}), -N(R^{10a})S(O)₂(R^{10b}), -NR^{10a}S(O)₂N(R^{10b})(R^{10c}), -NR^{10a}S(O)₂O(R^{10b}), -OC(O)R^{10a}, -OC(O)OR^{10a}, -OC(O)-N(R^{10a})(R^{10b}), -S-R^{10a}, -S(O)R^{10a}, -S(O)(NH)R^{10a}, -S(O)₂R^{10a}, -S(O)₂N(R^{10a})(R^{10b}), -S(O)(NR^{10a})R^{10b}, or -Si(R^{10a})₃,
      wherein each alkyl, haloalkyl, alkenyl, alkynyl, cycloalkyl, heterocyclyl, aryl, or heteroaryl is optionally substituted with one to four R⁶;
   each R⁶ and R⁸ is independently C₁₋₉ alkyl, C₁₋₈ haloalkyl, C₂₋₆ alkenyl, C₂₋₆ alkynyl, halogen, C₃₋₁₅ cycloalkyl, heterocyclyl, C₆₋₁₀ aryl, heteroaryl, oxo, -OH, -CN, - NO₂, -NH₂, -N₃, -SH, -O(C₁₋₉ alkyl), -O(C₁₋₈ haloalkyl), -O(C₂₋₆ alkenyl), -O(C₂₋₆ alkynyl), -O(C₃₋₁₅ cycloalkyl), -O(heterocyclyl), -O(C₆₋₁₀ aryl), - O(heteroaryl), -NH(C₁₋₉ alkyl), -NH(C₁₋₈ haloalkyl), -NH(C₂₋₆ alkenyl), -NH(C₂₋₆ alkynyl), -NH(C₃₋₁₅ cycloalkyl), -NH(heterocyclyl), -NH(C₆₋₁₀ aryl), - NH(heteroaryl), -N(C₁₋₉ alkyl)₂, -N(C₁₋₈ haloalkyl)₂, -N(C₂₋₆ alkenyl)₂, -N(C₂₋₆ alkynyl)₂, -N(C₃₋₁₅ cycloalkyl)₂, -N(heterocyclyl)₂, -N(C₆₋₁₀ aryl)₂, - N(heteroaryl)₂, -N(C₁₋₉ alkyl)(C₁₋₈ haloalkyl), -N(C₁₋₉ alkyl)(C₂₋₆ alkenyl), -N(C₁₋₉ alkyl)(C₂₋₆ alkynyl), -N(C₁₋₉ alkyl)(C₃₋₁₅ cycloalkyl), -N(C₁₋₉ alkyl)(heterocyclyl), -N(C₁₋₉ alkyl)(C₆₋₁₀ aryl), -N(C₁₋₉ alkyl)(heteroaryl), -C(O)(C₁₋₉ alkyl), -C(O)(C₁₋₈ haloalkyl), -C(O)(C₂₋₆ alkenyl), - C(O)(C₂₋₆ alkynyl), -C(O)(C₃₋₁₅ cycloalkyl), -C(O)(heterocyclyl), -C(O)(C₆₋₁₀ aryl), -C(O)(heteroaryl), -C(O)O(C₁₋₉ alkyl), -C(O)O(C₁₋₈ haloalkyl), -C(O)O(C₂₋₆ alkenyl), -C(O)O(C₂₋₆ alkynyl), -C(O)O(C₃₋₁₅ cycloalkyl), - C(O)O(heterocyclyl), -C(O)O(C₆₋₁₀ aryl), -C(O)O(heteroaryl), -C(O)NH₂, -C(O)NH(C₁₋₉ alkyl), -C(O)NH(C₁₋₈ haloalkyl), -C(O)NH(C₂₋₆ alkenyl), -C(O)NH(C₂₋₆ alkynyl), -C(O)NH(C₃₋₁₅ cycloalkyl), -C(O)NH(heterocyclyl), -C(O)NH(C₆₋₁₀ aryl), -C(O)NH(heteroaryl), -C(O)N(C₁₋₉ alkyl)₂, -C(O)N(C₁₋₈ haloalkyl)₂, -C(O)N(C₂₋₆ alkenyl)₂, -C(O)N(C₂₋₆ alkynyl)₂, -C(O)N(C₃₋₁₅ cycloalkyl)₂, -C(O)N(heterocyclyl)₂, -C(O)N(C₆₋₁₀ aryl)₂, -C(O)N(heteroaryl)₂, -NHC(O)(C₁₋₉ alkyl), -NHC(O)(C₁₋₈ haloalkyl), -NHC(O)(C₂₋₆ alkenyl), -NHC(O)(C₂₋₆ alkynyl), -NHC(O)(C₃₋₁₅ cycloalkyl), -NHC(O)(heterocyclyl), -NHC(O)(C₆₋₁₀ aryl), -NHC(O)(heteroaryl), -NHC(O)O(C₁₋₉ alkyl), -NHC(O)O(C₁₋₈ haloalkyl), -NHC(O)O(C₂₋₆ alkenyl), -NHC(O)O(C₂₋₆ alkynyl), -NHC(O)O(C₃₋₁₅ cycloalkyl), -NHC(O)O(heterocyclyl),-NHC(O)O(C₆₋₁₀ aryl), -NHC(O)O(heteroaryl), -NHC(O)NH(C₁₋₉ alkyl), -NHC(O)NH(C₁₋₈ haloalkyl), -NHC(O)NH(C₂₋₆ alkenyl), -NHC(O)NH(C₂₋₆ alkynyl), -NHC(O)NH(C₃₋₁₅ cycloalkyl), -NHC(O)NH(heterocyclyl), -NHC(O)NH(C₆₋₁₀ aryl), -NHC(O)NH(heteroaryl), -NHS(O)(C₁₋₉ alkyl), -N(C₁₋₉ alkyl)(S(O)(C₁₋₉ alkyl), - S(C₁₋₉ alkyl), -S(C₁₋₈ haloalkyl), -S(C₂₋₆ alkenyl), -S(C₂₋₆ alkynyl), -S(C₃₋₁₅ cycloalkyl), -S(heterocyclyl), -S(C₆₋₁₀ aryl), -S(heteroaryl), -S(O)N(C₁₋₉ alkyl)₂, -S(O)(C₁₋₉ alkyl), -S(O)(C₁₋₈ haloalkyl), -S(O)(C₂₋₆ alkenyl), -S(O)(C₂₋₆ alkynyl), -S(O)(C₃₋₁₅ cycloalkyl), -S(O)(heterocyclyl), -S(O)(C₆₋₁₀ aryl), -S(O)(heteroaryl), -S(O)₂(C₁₋₉ alkyl), -S(O)₂(C₁₋₈ haloalkyl), - S(O)₂(C₂₋₆ alkenyl), -S(O)₂(C₂₋₆ alkynyl), -S(O)₂(C₃₋₁₅ cycloalkyl), -S(O)₂(heterocyclyl), -S(O)₂(C₆₋₁₀ aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
      wherein each alkyl, cycloalkyl, heterocyclyl, aryl, or heteroaryl is optionally substituted with 1 to 3 C₁₋₉ alkyl, C₁₋₈ haloalkyl, halogen, -OH, -NH₂, CO₂H,-O(C₁₋₉ alkyl), -O(C₁₋₈ haloalkyl), -O(C₃₋₁₅ cycloalkyl), - O(heterocyclyl), -O(aryl), -O(heteroaryl), -NH(C₁₋₉ alkyl), -NH(C₁₋₈ haloalkyl), -NH(C₃₋₁₅ cycloalkyl), -NH(heterocyclyl), -NH(aryl), - NH(heteroaryl), -N(C₁₋₉ alkyl)₂, -N(C₃₋₁₅ cycloalkyl)₂, -NHC(O)(C₁₋₈ haloalkyl), -NHC(O)(C₃₋₁₅ cycloalkyl), -NHC(O)(heterocyclyl), - NHC(O)(aryl), -NHC(O)(heteroaryl), -NHC(O)O(C₁₋₉ alkyl), -NHC(O)O(C₁₋₈ haloalkyl), -NHC(O)O(C₂₋₆ alkynyl), -NHC(O)O(C₃₋₁₅ cycloalkyl), -NHC(O)O(heterocyclyl), - NHC(O)O(aryl), -NHC(O)O(heteroaryl), -NHC(O)NH(C₁₋₉ alkyl), S(O)₂(C₁₋₉ alkyl), -S(O)₂(C₁₋₈ haloalkyl), -S(O)₂(C₃₋₁₅ cycloalkyl), - S(O)₂(heterocyclyl), -S(O)₂(aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
      wherein the alkyl or heterocyclyl is each optionally substituted with one to four halogens;
   R^{6a} is H, C₁₋₆ alkyl, C₃₋₁₀ cycloalkyl, heterocyclyl, -S(O)₂R^{6a1}, or -S(O)₂N(R^{6a1})(NR^{6a2}), wherein the cycloalkyl or heterocyclyl is each optionally substituted with C₁₋₆ alkyl, F, or -CN;
   each R^{6b} and R^{6c} is independently H, C₁₋₆ alkyl, C₁₋₆ haloalkyl, C₂₋₆ alkoxyalkyl, halogen, C₃₋₁₀ cycloalkyl, heterocyclyl, -C₁₋₆ alkyl-N(R^{9a})(R^{9b}), -CN, -OR^{6c1}, or - N(R^{6c2})(R^{6c3}),
      wherein the alkyl, cycloalkyl, or heterocyclyl is each optionally substituted with one to four R^{6b1}; or
   R^{6b} and R^{6c} are combined with the atom to which they are attached to form a C₃₋₁₀ cycloalkyl or heterocyclyl, which is each optionally substituted with one to four R^{6b1}; or
   R^{6a} or R^{6c} is combined with one R⁴ group and the atoms to which they are attached to form a C₅₋₁₀ cycloalkyl or heterocyclyl, which is each optionally substituted with one to four R¹⁰;
   each R^{6b1} and R¹⁰ is independently C₁₋₆ alkyl, C₁₋₆ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₀ cycloalkyl, heterocyclyl, C₆₋₁₀ aryl, heteroaryl, oxo, -OH, -CN, CO₂R^{3e}, -NO₂, or - C(O)N(R^{2a})(R^{2b}),
      wherein the heterocyclyl or heteroaryl is optionally substituted with C₁₋₆ alkyl, C₁₋₆ haloalkyl, or C₁₋₆ haloalkoxy;
   each R^{6a1}, R^{6a2}, R^{6c1}, R^{6c2}, and R^{6c3} is independently H, C₁₋₆ alkyl or C₃₋₁₀ cycloalkyl;
   each R^{9a} and R^{9b} is independently H, C₁₋₆ alkyl, or C₁₋₆ haloalkyl, or R^{9a} and R^{9b} together form a 6-membered heterocyclyl;
   each R^{9c}, R^{9d}, R^{10a}, R^{10b}, and R^{10c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl,
      wherein the alkyl, alkenyl, alkynyl, cycloalkyl, heterocyclyl, aryl, or heteroaryl is each optionally substituted with one to four R⁶;
   R^{a} and R^{b} are independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶;
   optionally R^{a} and R^{b} are combined with the atom to which they are attached to form a 5- or 6-membered heterocyclyl, which is optionally substituted with one to four R⁶;
   each R^{2a}, R^{2b}, R^{12a}, R^{12b}, and R^{12c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl
      wherein the alkyl, alkenyl, alkynyl, cycloalkyl, heterocyclyl, aryl, or heteroaryl is each optionally substituted with one to four R⁶;
   each R^{3e} is independently H, C₁₋₆ alkyl, C₁₋₆ haloalkyl, -C₁₋₄ alkyl-N(R^{9a})(R^{9b}), -C₁₋₄ alkyl-C(O)N(R^{9a})(R^{9b}), -C₁₋₄ alkyl-O-C(O)-C₁₋₄ alkyl, -C₁₋₄ alkyl-O-C(O)-O-C₁₋₄alkyl, -C₁₋₄ alkyl-O-C(O)-C₁₋₄ alkyl-N(R^{9a})(R^{9b}), -C₁₋₄ alkyl-C₃₋₈ cycloalkyl, -C₁₋₄ alkyl-heterocyclyl, C₃₋₁₀ cycloalkyl, heterocyclyl, C₆₋₁₀ aryl, heteroaryl, - P(O)(OR^{9c})₂, -CH2P(O)(OR^{9c})₂, -OCH₂P(O)(OR^{9c})₂, -C(O)OCH₂P(O)(OR^{9c})₂, - P(O)(R^{9c})(OR^{9d}), -OP(O)(R^{9c})(OR^{9d}), -CH₂P(O)(R^{9c})(OR^{9d}), - C(O)OCH₂P(O)(R^{9c})(OR^{9d}), -P(O)(N(R^{9c})₂)₂, -CH₂P(O)(N(R^{9c})₂)₂, - C(O)OCH₂P(O)(N(R^{9c})₂)₂, -P(O)(N(R^{9c})₂)(OR^{9d}), -CH₂P(O)(N(R^{9c})₂)(OR^{9d}), - C(O)OCH₂P(O)(N(R^{9c})₂)(OR^{9d}), -P(O)(R^{9c})(N(R^{9d})₂), -CH₂P(O)(R^{9c})(N(R^{9d} )₂), or -C(O)OCH₂P(O)(R^{9c})(N(R^{9d})₂);
      wherein the alkyl, alkenyl, cycloalkyl, heterocyclyl, aryl, or heteroaryl is each optionally substituted with one to four R⁶; wherein each heteroaryl has 5 to 12 ring members and has one to four heteroatoms each independently N, O, or S;

   wherein each heterocyclyl has three to twelve ring members and has one to four heteroatoms, each independently N, O, or S; and
   wherein each heteroaryl has five to twelve ring members and one to four heteroatoms, each independently N, O, or S.
2. The compound of clause 1, or a pharmaceutically acceptable salt thereof, wherein R¹ is
   i) 5-membered heteroaryl, optionally substituted with one to four R⁴;
   ii) 5-membered heteroaryl fused to a heteroaryl or aryl to form a heteroaryl ring system,
      wherein the heteroaryl ring system is optionally substituted with one to four R⁴; or
   iii) 5-membered heteroaryl substituted with two R⁴ groups attached to adjacent ring atoms and optionally substituted with one or two additional R⁴,
      wherein the two R⁴ groups attached to adjacent ring atoms are combined with the atoms to which they are attached to form a C₅₋₁₀ cycloalkyl or heterocyclyl, which is each optionally substituted with one to four R⁶.
3. The compound of clause 1 or 2, or a pharmaceutically acceptable salt thereof, wherein R¹ is
   pyrrolyl, pyrazolyl, thienyl, indolyl, imidazolyl, oxazolyl, isoxazolyl, thiazolyl, furyl, oxadiazolyl, thiadiazolyl, benzothiazolyl, benzoxazolyl, indazolyl, benzofuranyl, benzimidazolyl, pyrrolo[2,3-*b*]pyridinyl, triazolyl, tetrazolyl, thiazolo[5,4-*b*]pyridinyl, pyrazolo[1,5-*a*]pyridinyl, imidazo[1,2-*a*]pyrimidinyl, imidazo[1,2-*a*]pyridinyl, imidazo[1,2-*a*]pyrazinyl, or 1λ²-thieno[3,2-*d*]pyrazolyl, optionally substituted with one to four R⁴; or
   5-membered heteroaryl substituted with two to four R⁴,
      wherein two R⁴ groups attached to adjacent ring atoms are combined with the atoms to which they are attached to form a C₅₋₆ cycloalkyl or heterocyclyl, which is each optionally substituted with one to four R⁶.
4. The compound of any one of clauses 1-3, or a pharmaceutically acceptable salt thereof, wherein
   R¹ is
   pyrazolyl, thienyl, imidazolyl, oxazolyl, isoxazolyl, thiazolyl, furyl, oxadiazolyl, thiadiazolyl, benzimidazolyl, triazolyl, thiazolo[5,4-b]pyridinyl, pyrazolo[1,5-*a*]pyridinyl, imidazo[1,2-*a*]pyrimidinyl, imidazo[1,2-*a*]pyridinyl, imidazo[1,2-*a*]pyrazinyl, or 1λ²-thieno[3,2-*d*]pyrazolyl, optionally substituted with one to four R⁴; or
   pyrazolyl substituted with two to four R⁴,
      wherein two R⁴ groups attached to adjacent pyrazolyl atoms are combined with the atoms to which they are attached to form a C₅₋₆ cycloalkyl or heterocyclyl, which is each optionally substituted with one to four R⁶.
5. The compound of any one of clauses 1-4, or a pharmaceutically acceptable salt thereof, wherein
   R¹ is 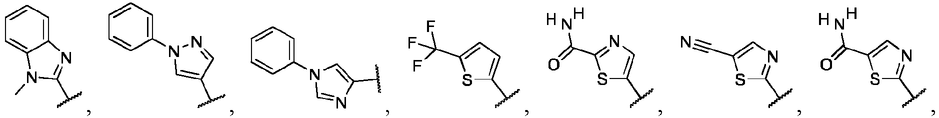 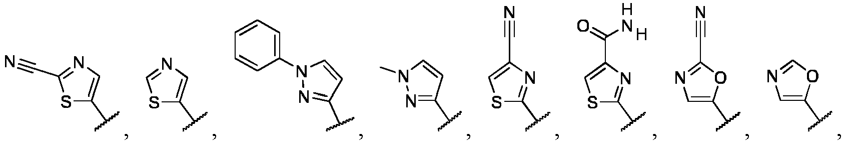 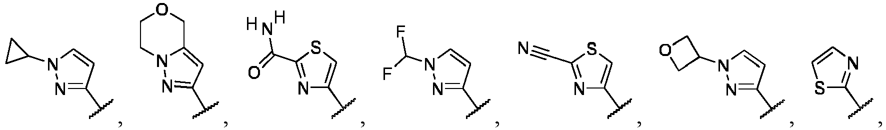 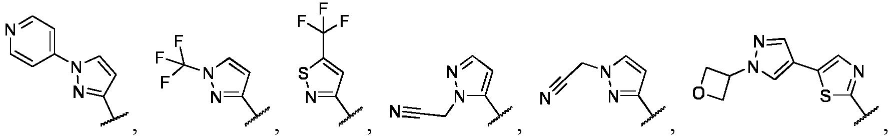 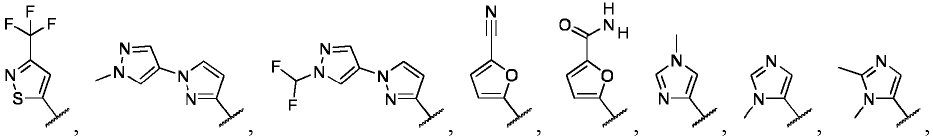 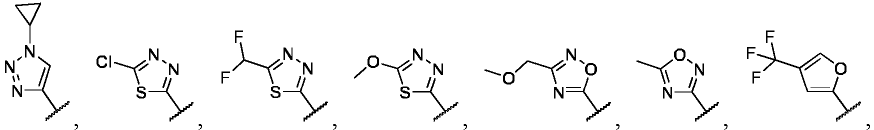 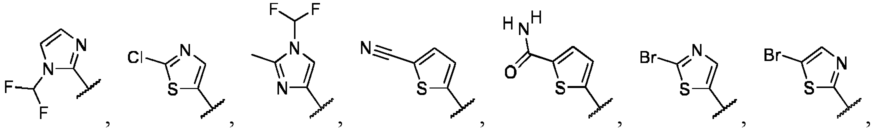 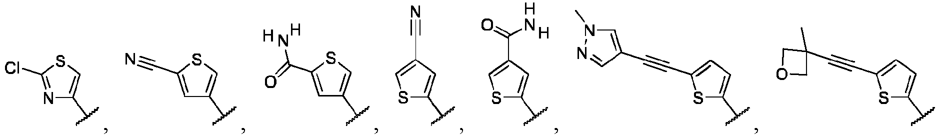 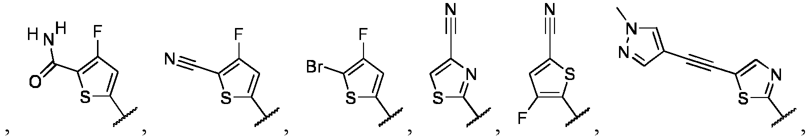 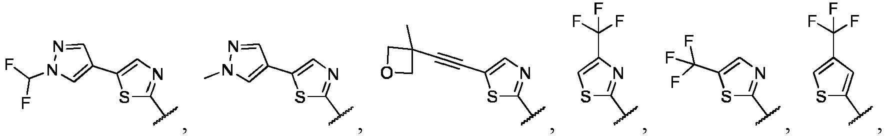 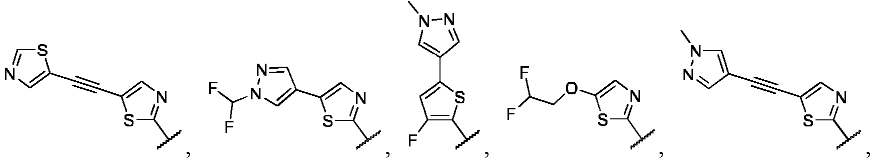 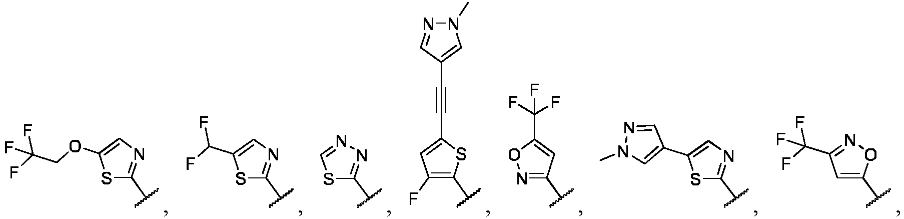 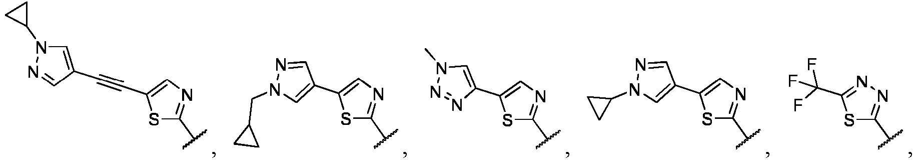 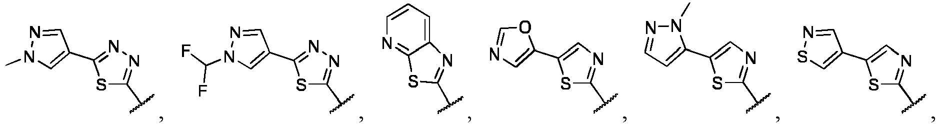 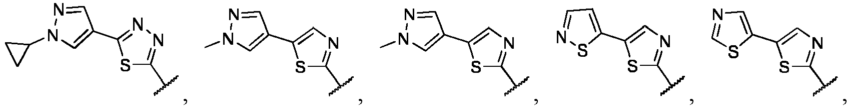 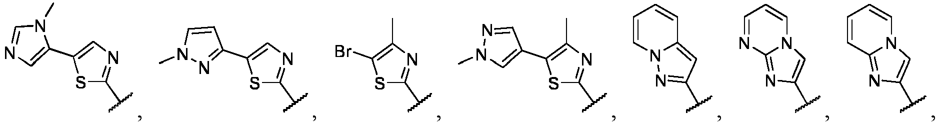 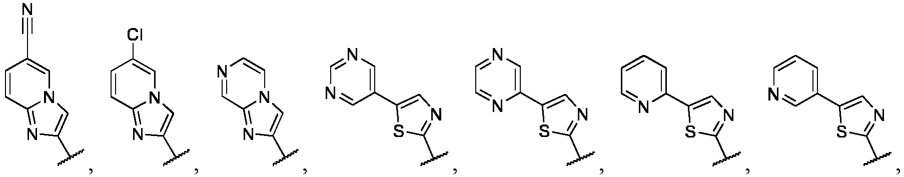 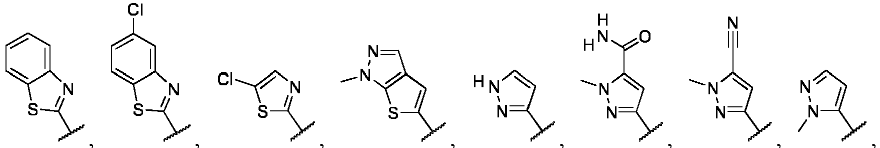 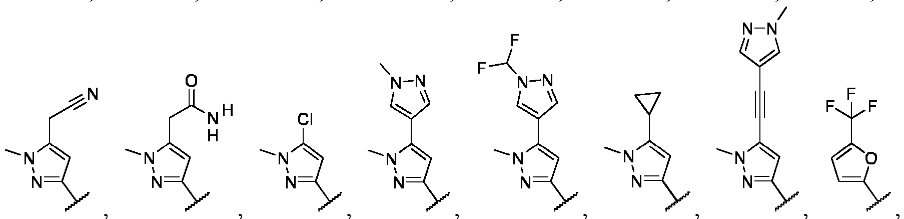 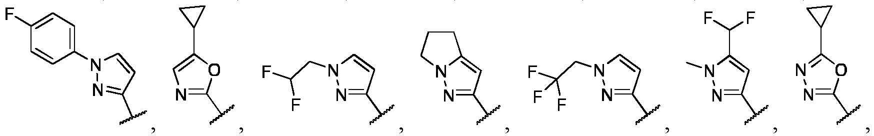 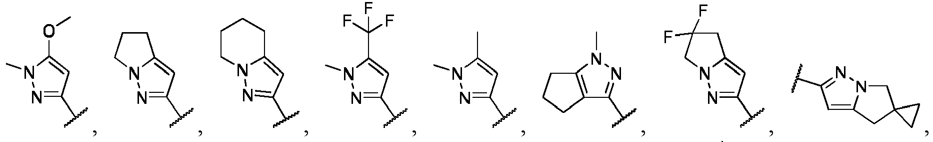 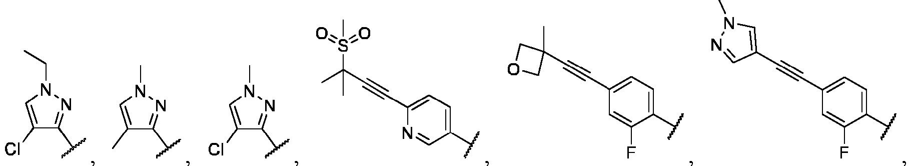 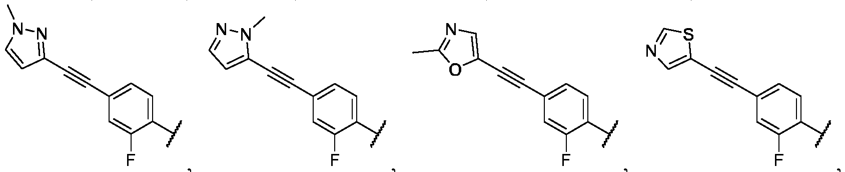 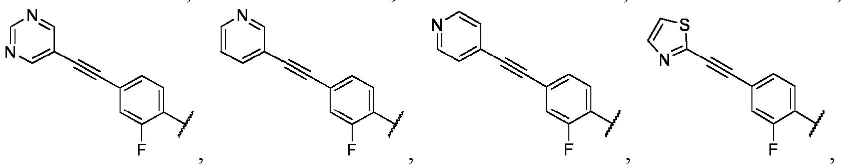 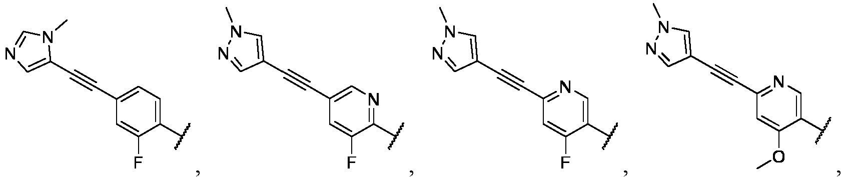 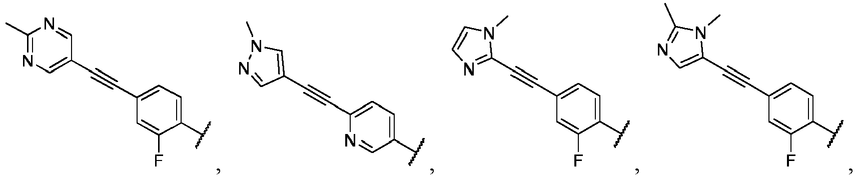 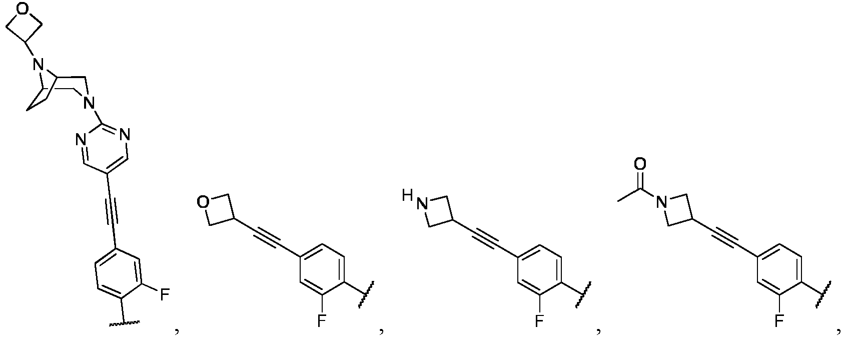 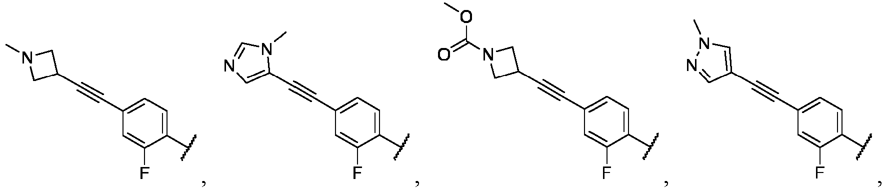 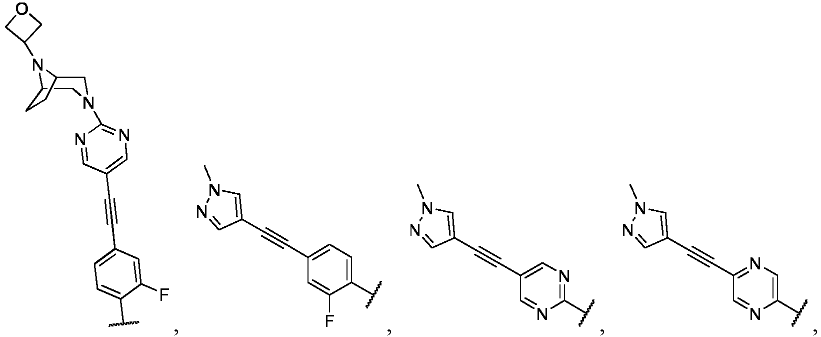 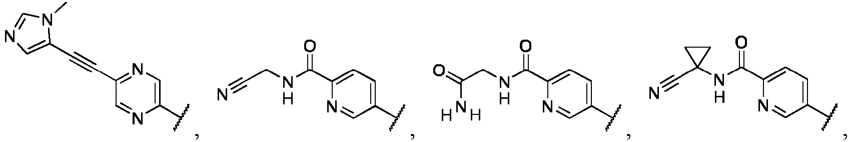 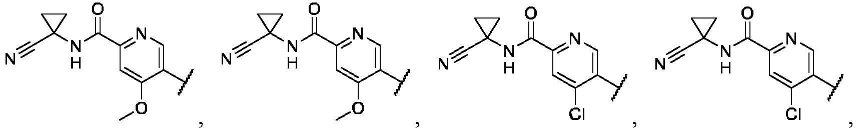 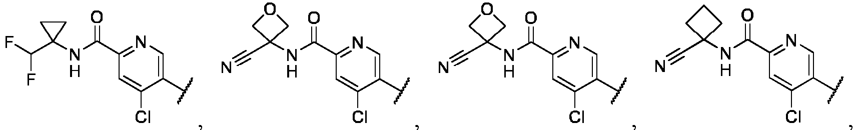 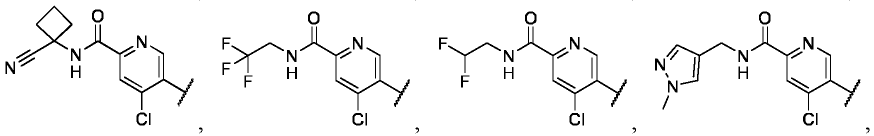 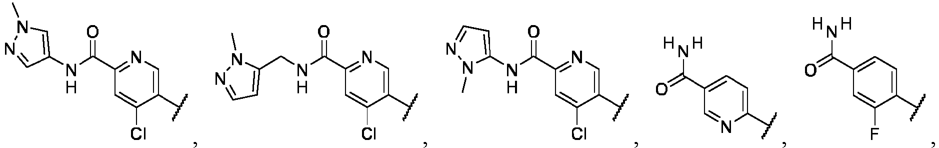 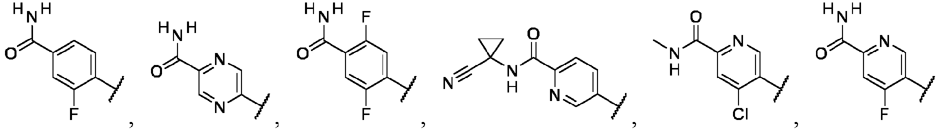 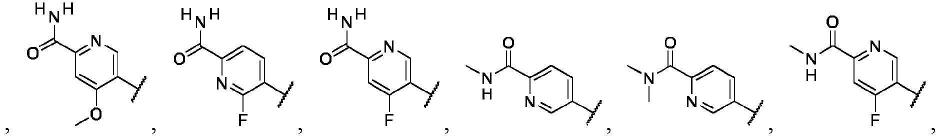 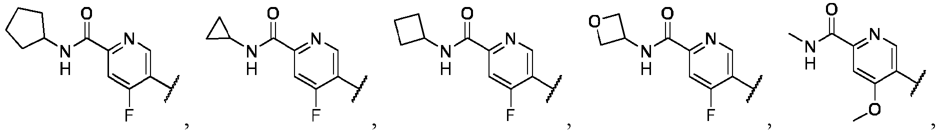 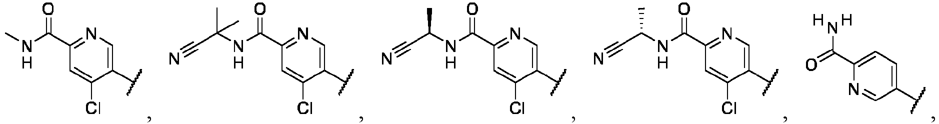 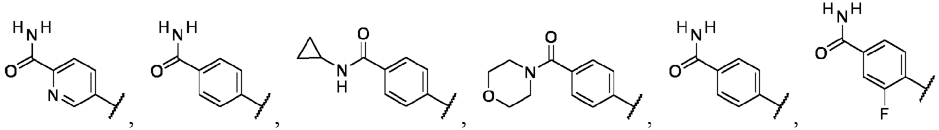 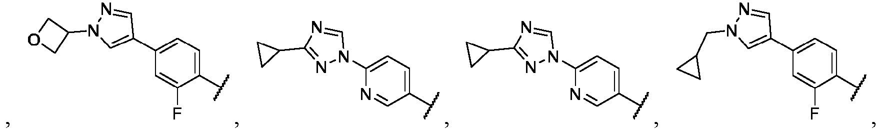 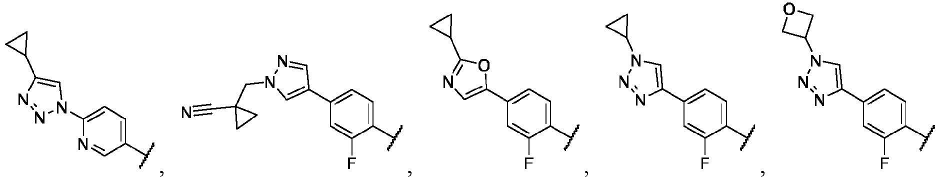 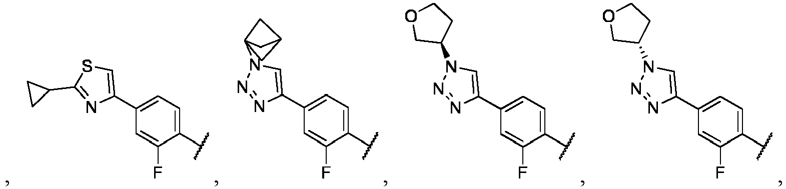 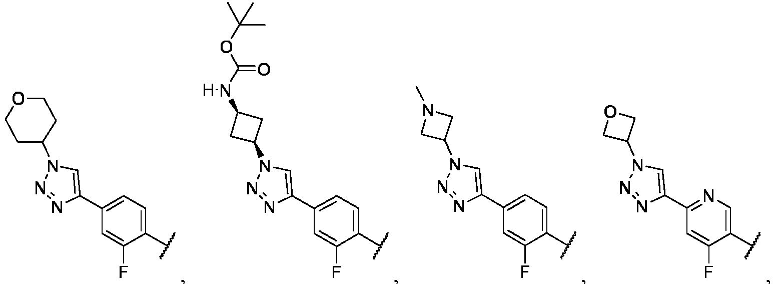 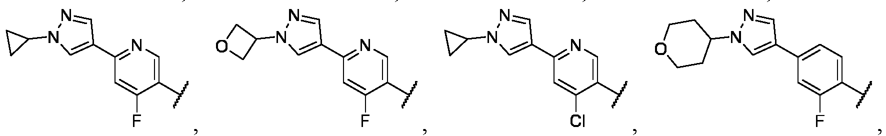 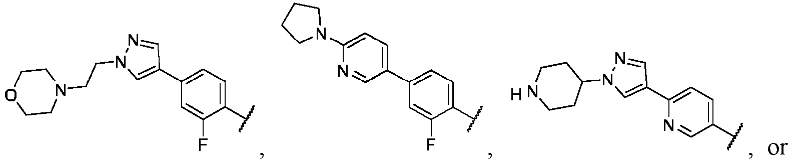 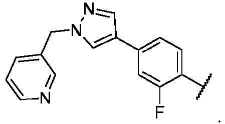
6. The compound of clause 1, or a pharmaceutically acceptable salt thereof, wherein
   R¹ is 6-membered heteroaryl or phenyl substituted with C₂-alkynyl and optionally substituted with one to three R⁴,
   wherein the C₂-alkynyl is substituted with -C(CH₃)₂SO₂CH₃, aryl, heteroaryl, or heterocyclyl,
   wherein the aryl, heteroaryl or heterocyclyl is optionally substituted with one to four R⁵.
7. The compound of clause 6, or a pharmaceutically acceptable salt thereof, wherein
   R¹ is pyridyl, pyrazinyl, pyrimidinyl, pyridazinyl, quinolyl, isoquinolyl, quinoxalyl, quinazolyl, thianaphthenyl, quinazolinyl-4(3H)-one, or phenyl, substituted with C₂-alkynyl and optionally substituted with one to three R⁴,
   wherein the C₂-alkynyl is substituted with -C(CH₃)₂SO₂CH₃, aryl, heteroaryl, or heterocyclyl,
   wherein the aryl, heteroaryl or heterocyclyl is optionally substituted with one to four R⁵.
8. The compound of clause 6 or 7, or a pharmaceutically acceptable salt thereof, wherein
   R¹ is pyridyl, pyrazinyl, pyrimidinyl, or phenyl, substituted with C₂-alkynyl and optionally substituted with one to three R⁴,
   wherein the C₂-alkynyl is substituted with -C(CH₃)₂SO₂CH₃, aryl, heteroaryl, or heterocyclyl,
   wherein the aryl, heteroaryl or heterocyclyl is optionally substituted with one to four R⁵.
9. The compound of any one of clauses 6-8, or a pharmaceutically acceptable salt thereof, wherein
   R¹ is pyridyl, pyrazinyl, pyrimidinyl, or phenyl, substituted with C₂-alkynyl and optionally substituted with one R⁴,
   wherein the C₂-alkynyl is substituted with -C(CH₃)₂SO₂CH₃, aryl, heteroaryl, or heterocyclyl,
   wherein the aryl, heteroaryl or heterocyclyl is optionally substituted with one to four R⁵.
10. The compound of any one of clauses 1 and 6-9, or a pharmaceutically acceptable salt thereof, wherein
   R¹ is 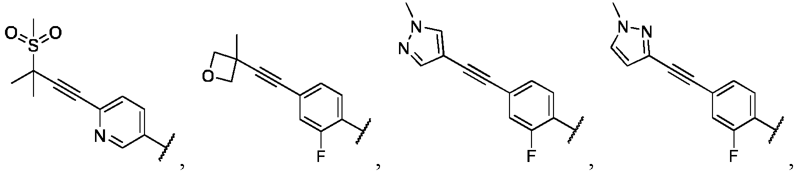 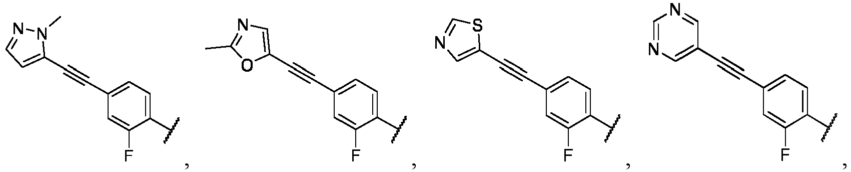 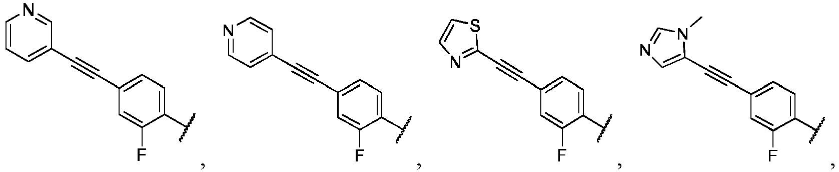 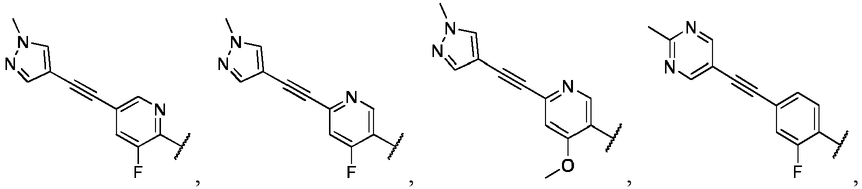 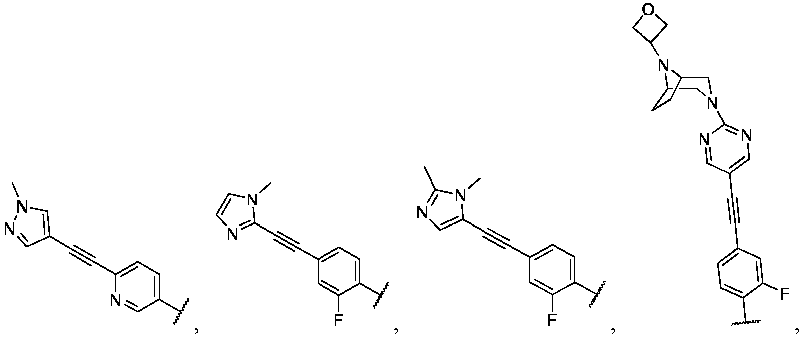 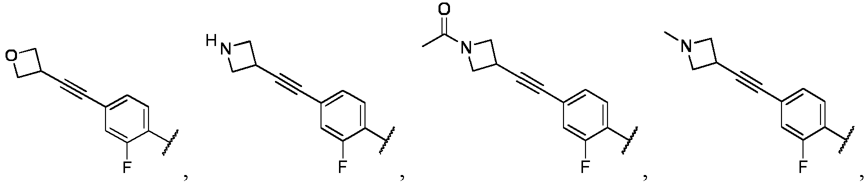 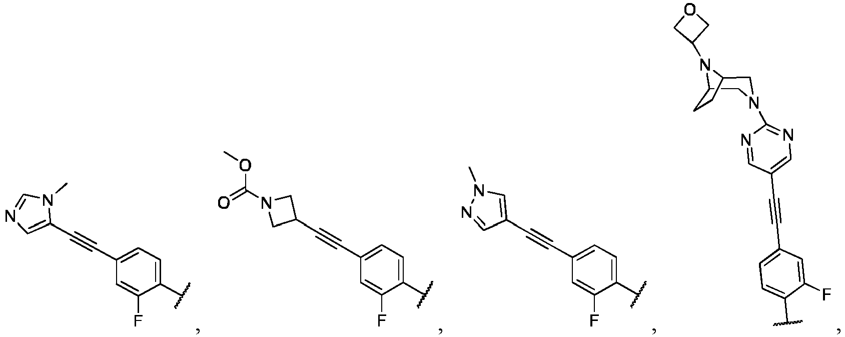 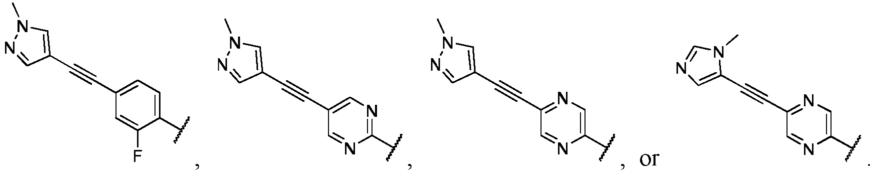
11. The compound of clause 1, or a pharmaceutically acceptable salt thereof, wherein
   R¹ is 6-membered heteroaryl or phenyl substituted with -C(O)NR^{a}R^{b},
   wherein the heteroaryl or phenyl is optionally substituted with one to four R⁴.
12. The compound of clause 11, or a pharmaceutically acceptable salt thereof, wherein
   R¹ is
   pyridyl, pyrazinyl, pyrimidinyl, pyridazinyl, quinolyl, isoquinolyl, quinoxalyl, quinazolyl, thianaphthenyl, quinazolinyl-4(3H)-one; or
   phenyl substituted with -C(O)NR^{a}R^{b}, wherein the heteroaryl or phenyl is optionally substituted with one to four R⁴.
13. The compound of clause 11 or 12, or a pharmaceutically acceptable salt thereof, wherein
   R¹ is
   pyridyl, pyrazinyl, or phenyl substituted with -C(O)NR^{a}R^{b},
   wherein the heteroaryl or phenyl is optionally substituted with one to four R⁴.
14. The compound of any one of clauses 11-13, or a pharmaceutically acceptable salt thereof, wherein
   R¹ is pyridyl, pyrazinyl, or phenyl substituted with -C(O)NR^{a}R^{b},
   wherein the pyridyl, pyrazinyl, or phenyl is optionally substituted with one R⁴.
15. The compound of any one of clauses 11-14, or a pharmaceutically acceptable salt thereof, wherein
   R^{a} and R^{b} are independently H, C₁₋₆ alkyl, C₂₋₆ alkenyl, C₂₋₆ alkynyl, C₃₋₆ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶; or
   optionally R^{a} and R^{b} are combined with the nitrogen atom to which they are attached to form a 5- or 6-membered heterocyclyl, wherein the heterocyclyl is optionally substituted with one to four R⁶.
16. The compound of any one of clauses 11-15, or a pharmaceutically acceptable salt thereof, wherein
   R^{a} and R^{b} are independently H, C₁₋₄ alkyl, C₃₋₆ cycloalkyl, heterocyclyl, or heteroaryl wherein the alkyl, cycloalkyl, heterocyclyl, or heteroaryl is each optionally substituted with one to four R⁶; or
   optionally R^{a} and R^{b} are combined with the nitrogen atom to which they are attached to form a 5- or 6-membered heterocyclyl,
   wherein the heterocyclyl is optionally substituted with one to four R⁶.
17. The compound of any one of clauses 1 and 11-16, or a pharmaceutically acceptable salt thereof, wherein
   R¹ is: 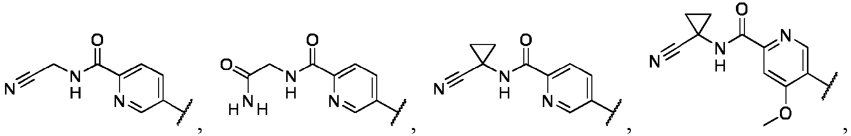 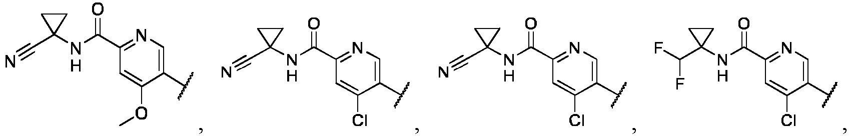 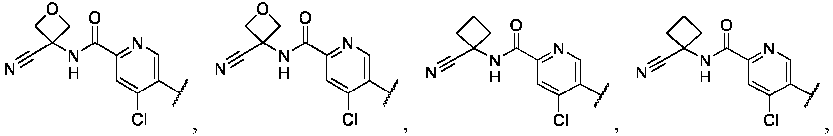 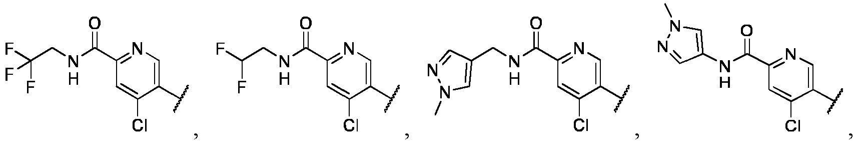 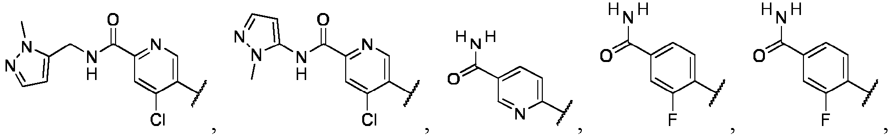 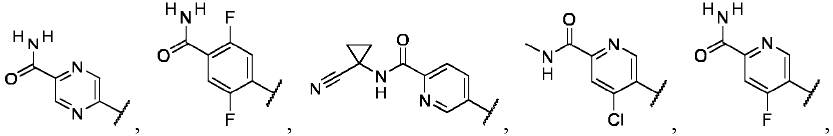 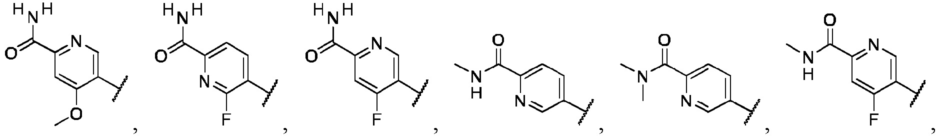 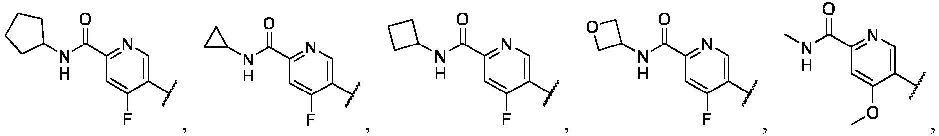 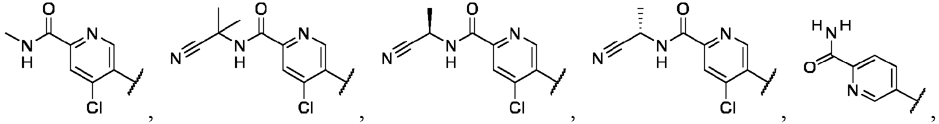 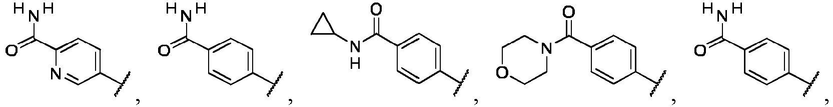 or 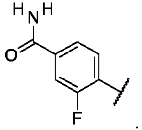
18. The compound of clause 1, or a pharmaceutically acceptable salt thereof, wherein
   R¹ is 6-membered heteroaryl or phenyl, each substituted with heteroaryl and optionally substituted with one to three R⁴,
   wherein the heteroaryl is substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl,
   wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶.
19. The compound of clause 18 or a pharmaceutically acceptable salt thereof, wherein
   R¹ is pyridyl, pyrazinyl, pyrimidinyl, pyridazinyl, quinolyl, isoquinolyl, quinoxalyl, quinazolyl,
   thianaphthenyl, quinazolinyl-4(3H)-one, or phenyl, each substituted with heteroaryl and optionally substituted with one to three R⁴,
   wherein the heteroaryl is substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl,
   wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶.
20. The compound of clause 18 or 19, or a pharmaceutically acceptable salt thereof, wherein
   R¹ is pyridyl or phenyl, each substituted with heteroaryl and optionally substituted with one to three R⁴,
   wherein the heteroaryl is substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl,
   wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶.
21. The compound of any one of clauses 18-20, or a pharmaceutically acceptable salt thereof, wherein
   R¹ is pyridyl or phenyl, each substituted with heteroaryl and optionally substituted with one to three R⁴,
   wherein the heteroaryl is substituted with C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl,
   wherein the C₃₋₁₀ cycloalkyl, heterocyclyl, C₁₋₆ alkyl-C₃₋₁₀ cycloalkyl, C₁₋₆ alkyl-heterocyclyl, or C₁₋₆ alkyl-heteroaryl is optionally substituted with one to four R⁶.
22. The compound of any one of clauses 1 and 18-21, or a pharmaceutically acceptable salt thereof, wherein
   R¹ is: 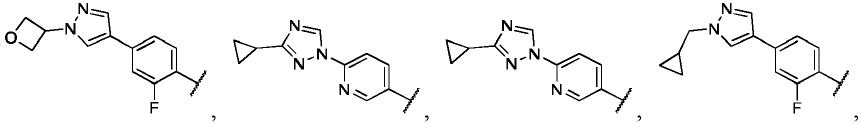 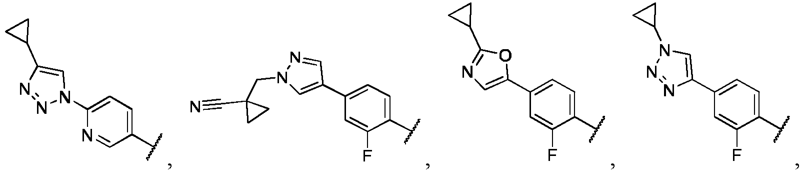 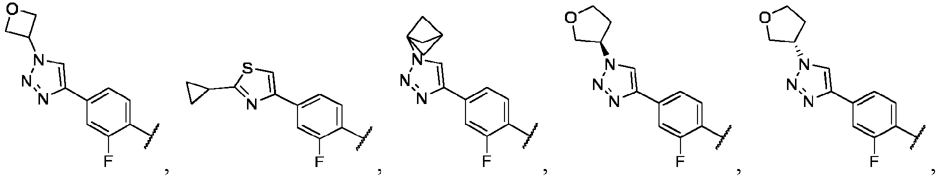 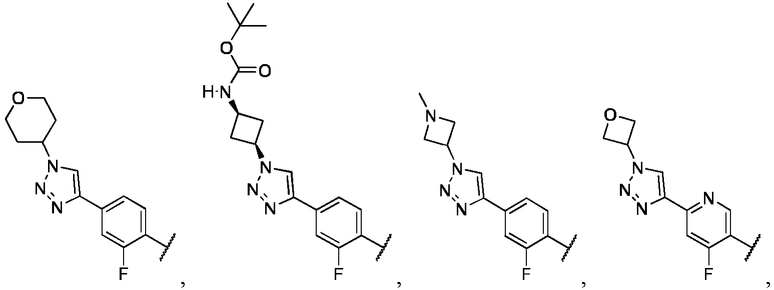 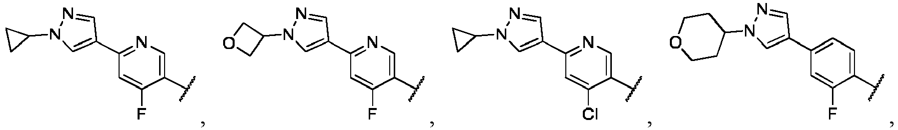 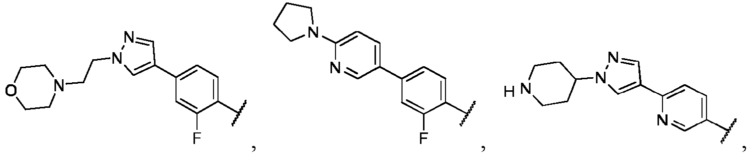 or 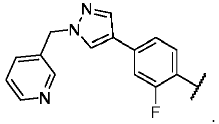
23. The compound of clause any one of clauses 1-22, or a pharmaceutically acceptable salt thereof, wherein
   ring B is C₆₋₁₀ aryl, optionally substituted with one to four R⁴.
24. The compound of any one of clauses 1-23, or a pharmaceutically acceptable salt thereof, wherein
   each R⁴ is independently C₁₋₉ alkyl or halogen.
25. The compound of any one of clauses 1-24, or a pharmaceutically acceptable salt thereof, wherein
   each R⁴ is independently methyl or F.
26. The compound of any one of clauses 1-25, or a pharmaceutically acceptable salt thereof, wherein
   ring B is substituted with two R⁴.
27. The compound of clause 26, or a pharmaceutically acceptable salt thereof, wherein each R⁴ is F.
28. The compound of clause any one of clauses 1-27, or a pharmaceutically acceptable salt thereof, wherein
   V is -C(R^{6b})(R^{6c})-.
29. The compound of clause any one of clauses 1-28, or a pharmaceutically acceptable salt thereof, wherein
   V is -CH₂-.
30. The compound of any one of clauses 1-29, or a pharmaceutically acceptable salt thereof, wherein
   ring A is 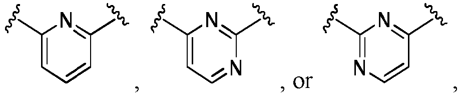 each optionally substituted with one halogen.
31. The compound of any one of clauses 1-30, or a pharmaceutically acceptable salt thereof, wherein
   two of X¹, X², and X³ is -CH= and one is -C(F)=.
32. The compound of any one of clauses 1-31, or a pharmaceutically acceptable salt thereof, wherein
   R² is C₁₋₆ alkyl or heterocyclyl, wherein the alkyl or heterocyclyl is each optionally substituted with one to four Z¹,
   wherein each Z¹ is independently C₁₋₆ alkyl, C₁₋₆ alkoxy, C₁₋₆ hydroxyalkyl, C₂₋₆ alkoxyalkyl, halogen, C₁₋₆ haloalkyl, C₁₋₆ haloalkoxy, -OH, -CN, C₁₋₆ alkyl-CN, - O-C₃₋₆ cycloalkyl, heterocyclyl, or heteroaryl optionally substituted with 1 to 4 groups each independently C₁₋₆ alkyl, C₁₋₆ alkoxy, halogen, -CN, C₁₋₆ haloalkyl, or C₁₋₆ haloalkoxy.
33. The compound of any one of clauses 1-32, or a pharmaceutically acceptable salt thereof, wherein
   R² is C₁₋₆ alkyl or heterocyclyl, wherein the alkyl or heterocyclyl is each optionally substituted with one to four Z¹,
   wherein each Z¹ is independently C₁₋₆ alkyl, C₁₋₆ alkoxy, or heterocyclyl.
34. The compound of any one of clauses 1-33, or a pharmaceutically acceptable salt thereof, wherein
   R² is 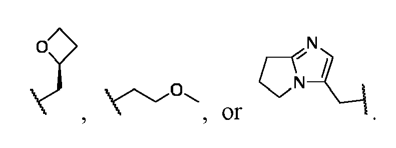
35. The compound of any one of clauses 1-34, or a pharmaceutically acceptable salt thereof, wherein
   R³ is -C(O)OH.
36. A compound of Formula (II) 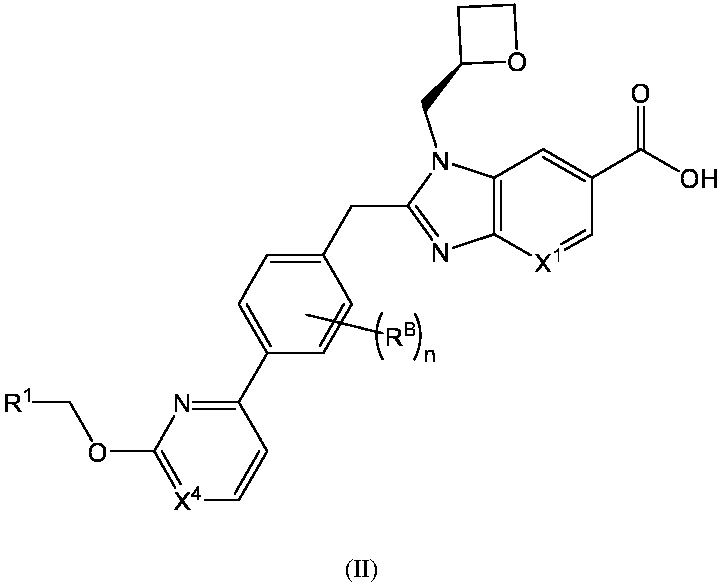 or a pharmaceutically acceptable salt thereof, wherein
   R¹ is thiazolyl optionally substituted with R⁴;
   X¹ is -C(H)= or -C(R⁸)=;
   X⁴ is -CH=, -C(R⁸)= or N;
   each R^{B} is independently C₁₋₉ alkyl, C₁₋₈ haloalkyl, or halogen;
   each R⁸ is independently halogen; and
   n is 0, 1, 2, or 3.
37. The compound of any of the preceding clauses, or a pharmaceutically acceptable salt thereof, wherein
   R¹ is 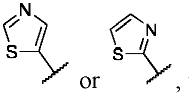 which is each substituted with C₁₋₈ haloalkyl.
38. The compound of any of the preceding clauses, or a pharmaceutically acceptable salt thereof, wherein R¹ is 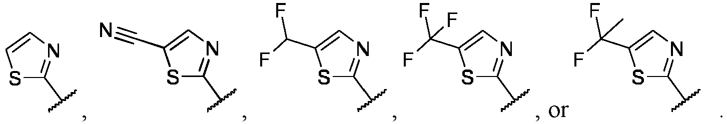
39. The compound of any of the preceding clauses, or a pharmaceutically acceptable salt thereof, wherein R⁸ is F or Cl.
40. The compound of any of the preceding clauses, or a pharmaceutically acceptable salt thereof, having the structure of a compound in Table 2.
41. A pharmaceutical composition comprising a pharmaceutically effective amount of a compound of any one of clauses 1-40, or a pharmaceutically acceptable salt thereof, and a pharmaceutically acceptable carrier or excipient.
42. The pharmaceutical composition of clause 41 further comprising one or more additional therapeutic agents.
43. The pharmaceutical composition of clause 41 for use in treating a glucagon-like peptide 1 receptor (GLP-1R) mediated disease or condition.
44. A method of treating GLP-1R mediated disease or condition comprising administering to a subject in need thereof a pharmaceutically effective amount of a compound of any one of clauses 1-40, or a pharmaceutically acceptable salt thereof.
45. The method of clause 44, wherein the disease or condition comprises a liver disease.
46. The method of clause 45, wherein the disease or condition comprises liver fibrosis, non-alcoholic fatty liver disease (NAFLD), non-alcoholic steatohepatitis (NASH), liver cirrhosis, compensated liver fibrosis, decompensated liver fibrosis, hepatocellular carcinoma, Primary Biliary Cirrhosis (PBC), or Primary Sclerosing Choleangitis (PSC).
47. The method of clause 46, wherein the disease or condition comprises non-alcoholic fatty liver disease (NAFLD).
48. The method of clause 46, wherein the disease or condition comprises non-alcoholic steatohepatitis (NASH).
49. The method of clause 44, wherein the disease or condition comprises a metabolic disease.
50. The method of clause 44, wherein the disease or condition comprises type 1 diabetes, type 2 diabetes, pre-diabetes, idiopathic type 1 diabetes, latent autoimmune diabetes, maturity onset diabetes of the young, early onset diabetes, malnutrition-related diabetes, gestational diabetes, hyperglycemia, insulin resistance, hepatic insulin resistance, impaired glucose tolerance, diabetic neuropathy, diabetic nephropathy, kidney disease, diabetic retinopathy, adipocyte dysfunction, visceral adipose deposition, obesity, eating disorders, sleep apnea, weight gain, sugar craving, dyslipidemia, hyperinsulinemia, congestive heart failure, myocardial infarction, stroke, hemorrhagic stroke, ischemic stroke, traumatic brain injury, pulmonary hypertension, restenosis after angioplasty, intermittent claudication, post-prandial lipemia, metabolic acidosis, ketosis, arthritis, left ventricular hypertrophy, Parkinson's Disease, peripheral arterial disease, macular degeneration, cataract, glomerulosclerosis, chronic renal failure, metabolic syndrome, angina pectoris, premenstrual syndrome, thrombosis, atherosclerosis, impaired glucose metabolism, vascular restenosis, dementia, or Alzheimer's disease.
51. The method of any one of clauses 44 to 50, wherein the compound or pharmaceutically acceptable salt thereof is administered in combination with an additional therapeutic agent.
52. The pharmaceutical composition of clause 42 or the method of clause 51, wherein the additional therapeutic agent comprises an anti-obesity agent including but not limited to peptide YY or an analogue thereof, a neuropeptide Y receptor type 2 (NPYR2) agonist, a NPYR1 agonist, an NPYR5 antagonist, a cannabinoid receptor type 1 (CB1 R) antagonist, a lipase inhibitor (e.g., orlistat), a human proislet peptide (HIP), a melanocortin receptor 4 agonist (MC4R)(e.g., setmelanotide), a melanin concentrating hormone receptor 1 antagonist, a farnesoid X receptor (FXR) agonist (e.g. obeticholic acid), apoptotic signal-regulating kinase (ASK-1) inhibitor, zonisamide, phentermine (alone or in combination with topiramate), a norepinephrine/dopamine reuptake inhibitor (e.g., buproprion), an opioid receptor antagonist (*e.g.*, naltrexone), a combination of norepinephrine/dopamine reuptake inhibitor and opioid receptor antagonist (*e*.*g*., a combination of bupropion and naltrexone), a GDF-15 analog, sibutramine, a cholecystokinin agonist, amylin and analogues thereof (*e*.*g*., pramlintide), leptin and analogues thereof (*e.g.,* metroleptin), a serotonergic agent (*e.g.,* lorcaserin), a methionine aminopeptidase 2 (MetAP2) inhibitor (*e*.*g*., beloranib or ZGN-1061), phendimetrazine, diethylpropion, benzphetamine, an SGLT2 inhibitor (*e.g*., empagliflozin, canagliflozin, dapagliflozin, ipragliflozin, tofogliflozin, sergliflozin etabonate, remogliflozin etabonate, or ertugliflozin), an SGLTL1 inhibitor, a dual SGLT2/SGLT1 inhibitor, a fibroblast growth factor receptor (FGFR) modulator, an AMP-activated protein kinase (AMPK) activator, biotin, a MAS receptor modulator, or a glucagon receptor agonist (alone or in combination with another GLP-1 R agonist, *e.g*., liraglutide, exenatide, dulaglutide, albiglutide, lixisenatide, or semaglutide), a peroxisome proliferator-activated receptor alpha (PPARα) agonist, fish oil, an acetyl-coA carboxylase (ACC) inhibitor, a TGFβ antagonist, GFRAL agonist, and/or a pharmaceutically acceptable salt thereof.
53. A method for manufacturing a medicament for treating a GLP-1R mediated disease or condition in a human in need thereof, characterized in that a compound of any one of clauses 1-40, or a pharmaceutically acceptable salt thereof, is used.
54. Use of the compound of any one of clauses 1-40, or a pharmaceutically acceptable salt thereof, for the manufacture of a medicament for treating a GLP-1R mediated disease or condition.
55. The compound of any one of clauses 1-40 or a pharmaceutically acceptable salt thereof, for use in treating a GLP-1R mediated disease or condition in a human in need thereof.

## Claims

1. A compound of Formula (I): 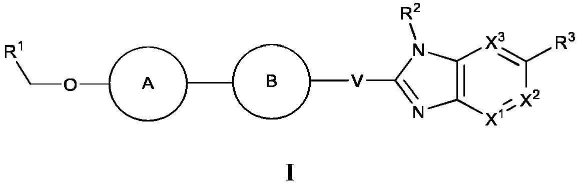 or a pharmaceutically acceptable salt thereof, wherein
R¹ is pyrrolyl, pyrazolyl, thienyl, indolyl, imidazolyl, oxazolyl, isoxazolyl, thiazolyl, furyl, oxadiazolyl, thiadiazolyl, triazolyl, or tetrazolyl, optionally substituted with one to four R⁴;
X¹, X², and X³ are each independently -C(H)=, or -C(R⁸)=; ring A is 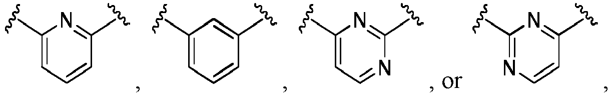
each optionally substituted with one to three Z^{1a} groups, each independently C₁₋₆ alkyl, C₁₋₆ haloalkyl, C₁₋₆ alkoxy, C₁₋₆ haloalkoxy, halogen, -OH, or -CN;
ring B is phenyl, which is each optionally substituted with one to four R⁴;
V is -CH₂-;
R² is C₁₋₆ alkyl or heterocyclyl, wherein the alkyl or heterocyclyl is each optionally substituted with one to four Z¹,
each Z¹ is independently C₁₋₆ alkyl, C₁₋₆ alkoxy, C₁₋₆ hydroxyalkyl, C₂₋₆ alkoxyalkyl, halogen, C₁₋₆ haloalkyl, C₁₋₆ haloalkoxy, -OH, -CN, C₁₋₆ alkyl-CN, -O-C₃₋₆ cycloalkyl, heterocyclyl, or heteroaryl optionally substituted with 1 to 4 groups each independently C₁₋₆ alkyl, C₁₋₆ alkoxy, halogen, -CN, C₁₋₆ haloalkyl, or C₁₋₆ haloalkoxy;
R³ is -C(O)OH;
each R⁴ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₅ cycloalkyl, heterocyclyl, C₆₋₁₀ aryl, heteroaryl, oxo, -NO₂, -N3, -CN, -OR^{10a}, -C(O)R^{10a}, -C(O)O-R^{10a}, -C(O)-N(R^{10a})(R^{10b}), -N(R^{10a})(R^{10b}), -N(R^{10a})₂(R^{10b})⁺, -N(R^{10a})C(O)- R^{10b}, -N(R^{10a})C(O)OR^{10b}, -N(R^{10a})C(O)N(R^{10b})(R^{10c}), -N(R^{10a})S(O)2(R^{10b}), -N R^{10a}S(O)₂N(R^{10b})(R^{10c}), -N^{R10a}S(O)₂O(R^{10b}), -OC(O)R^{10a}, -OC(O)O R^{10a}, -OC(O)-N(R^{10a})(R^{10b}), -S-R^{10a}, -S(O) R^{10a}, -S(O)(NH)R^{10a}, -S(O)₂R^{10a}, -S(O)₂N(R^{10a})(R^{10b}), -S(O)(NR^{10a}) R^{10b}, or -Si(R^{10a})₃;
wherein each alkyl, haloalkyl, alkenyl, alkynyl, cycloalkyl, heterocyclyl, aryl, or heteroaryl is optionally substituted with one to four R⁵;
each R⁵ is independently C₁₋₉ alkyl, C₁₋₈ haloalkyl, C₁₋₆ alkoxy, C₁₋₆ haloalkoxy, C₂₋₆ alkoxyalkyl, C₂₋₆ alkenyl, C₂₋₆ alkynyl, C₃₋₁₅ cycloalkyl, halogen, C₃₋₁₅ cycloalkyl, heterocyclyl, C₆₋₁₀ aryl, heteroaryl, oxo, -NO₂, -N₃, -CN, -O- R^{10a}, -C(O)-R^{10a}, - C(O)OR^{10a}, -C(O)N(R^{10a})(R^{10b}), -N(R^{10a})(R^{10b}), -N(R^{10a})C(O)R^{10b}, -N(R^{10a})C(O)OR^{10b}, -N(R^{10a})C(O)N(R^{10b})(R^{10c}), -N(R^{10a})S(O)₂(R^{10b}), - NR^{10a}S(O)₂N(R^{10b})(R^{10c}), -NR^{10a}S(O)₂O(R^{10b}), -OC(O)R^{10a}, -OC(O)OR^{10a}, -OC(O)-N(R^{10a})(R^{10b}), -S-R^{10a}, -S(O)R^{10a}, -S(O)(NH)R^{10a}, -S(O)₂R^{10a}, -S(O)₂N(R^{10a})(R^{10b}), -S(O)(NR^{10a})R^{10b}, or -Si(R^{10a})₃,
wherein each alkyl, haloalkyl, alkenyl, alkynyl, cycloalkyl, heterocyclyl, aryl, or heteroaryl is optionally substituted with one to four R⁶;
each R⁶ and R⁸ is independently C₁₋₉ alkyl, C₁₋₈ haloalkyl, C₂₋₆ alkenyl, C₂₋₆ alkynyl, halogen, C₃₋₁₅ cycloalkyl, heterocyclyl, C₆₋₁₀ aryl, heteroaryl, oxo, -OH, -CN, - NO₂, -NH₂, -N₃, -SH, -O(C₁₋₉ alkyl), -O(C₁₋₈ haloalkyl), -O(C₂₋₆ alkenyl), -O(C₂₋₆ alkynyl), -O(C₃₋₁₅ cycloalkyl), -O(heterocyclyl), -O(C₆₋₁₀ aryl), -O(heteroaryl), -NH(C₁₋₉ alkyl), -NH(C₁₋₈ haloalkyl), -NH(C₂₋₆ alkenyl), -NH(C₂₋₆ alkynyl), -NH(C₃₋₁₅ cycloalkyl), -NH(heterocyclyl), -NH(C₆₋₁₀ aryl), -NH(heteroaryl), -N(C₁₋₉ alkyl)₂, -N(C₁₋₈ haloalkyl)₂, -N(C₂₋₆ alkenyl)₂, -N(C₂₋₆ alkynyl)₂, -N(C₃₋₁₅ cycloalkyl)₂, -N(heterocyclyl)₂, -N(C₆₋₁₀ aryl)₂, -N(heteroaryl)₂, -N(C₁₋₉ alkyl)(C₁₋₈ haloalkyl), -N(C₁₋₉ alkyl)(C₂₋₆ alkenyl), -N(C₁₋₉ alkyl)(C₂₋₆ alkynyl), -N(C₁₋₉ alkyl)(C₃₋₁₅ cycloalkyl), -N(C₁₋₉ alkyl)(heterocyclyl), -N(C₁₋₉ alkyl)(C₆₋₁₀ aryl), - N(C₁₋₉ alkyl)(heteroaryl), -C(O)(C₁₋₉ alkyl), -C(O)(C₁₋₈ haloalkyl), -C(O)(C₂₋₆ alkenyl), -C(O)(C₂₋₆ alkynyl), -C(O)(C₃₋₁₅ cycloalkyl), -C(O)(heterocyclyl), - C(O)(C₆₋₁₀ aryl), -C(O)(heteroaryl), -C(O)O(C₁₋₉ alkyl), -C(O)O(C₁₋₈ haloalkyl), -C(O)O(C₂₋₆ alkenyl), -C(O)O(C₂₋₆ alkynyl), -C(O)O(C₃₋₁₅ cycloalkyl), - C(O)O(heterocyclyl), -C(O)O(C₆₋₁₀ aryl), -C(O)O(heteroaryl), -C(O)NH₂, -C(O)NH(C₁₋₉ alkyl), -C(O)NH(C₁₋₈ haloalkyl), -C(O)NH(C₂₋₆ alkenyl), -C(O)NH(C₂₋₆ alkynyl), -C(O)NH(C₃₋₁₅ cycloalkyl), -C(O)NH(heterocyclyl), -C(O)NH(C₆₋₁₀ aryl), -C(O)NH(heteroaryl), -C(O)N(C₁₋₉ alkyl)₂, -C(O)N(C₁₋₈ haloalkyl)₂, -C(O)N(C₂₋₆ alkenyl)₂, -C(O)N(C₂₋₆ alkynyl)₂, -C(O)N(C₃₋₁₅ cycloalkyl)₂, -C(O)N(heterocyclyl)₂, -C(O)N(C₆₋₁₀ aryl)₂, -C(O)N(heteroaryl)₂, -NHC(O)(C₁₋₉ alkyl), -NHC(O)(C₁₋₈ haloalkyl), -NHC(O)(C₂₋₆ alkenyl), -NHC(O)(C₂₋₆ alkynyl), -NHC(O)(C₃₋₁₅ cycloalkyl), -NHC(O)(heterocyclyl), -NHC(O)(C₆₋₁₀ aryl), -NHC(O)(heteroaryl), -NHC(O)O(C₁₋₉ alkyl), -NHC(O)O(C₁₋₈ haloalkyl), -NHC(O)O(C₂₋₆ alkenyl), -NHC(O)O(C₂₋₆ alkynyl), -NHC(O)O(C₃₋₁₅ cycloalkyl), -NHC(O)O(heterocyclyl),-NHC(O)O(C₆₋₁₀ aryl), -NHC(O)O(heteroaryl), -NHC(O)NH(C₁₋₉ alkyl), -NHC(O)NH(C₁₋₈ haloalkyl), -NHC(O)NH(C₂₋₆ alkenyl), -NHC(O)NH(C₂₋₆ alkynyl), -NHC(O)NH(C₃₋₁₅ cycloalkyl), -NHC(O)NH(heterocyclyl), -NHC(O)NH(C₆₋₁₀ aryl), -NHC(O)NH(heteroaryl), -NHS(O)(C₁₋₉ alkyl), -N(C₁₋₉ alkyl)(S(O)(C₁₋₉ alkyl), - S(C₁₋₉ alkyl), -S(C₁₋₈ haloalkyl), -S(C₂₋₆ alkenyl), -S(C₂₋₆ alkynyl), -S(C₃₋₁₅ cycloalkyl), -S(heterocyclyl), -S(C₆₋₁₀ aryl), -S(heteroaryl), -S(O)N(C₁₋₉ alkyl)₂, -S(O)(C₁₋₉ alkyl), -S(O)(C₁₋₈ haloalkyl), -S(O)(C₂₋₆ alkenyl), -S(O)(C₂₋₆ alkynyl), -S(O)(C₃₋₁₅ cycloalkyl), -S(O)(heterocyclyl), -S(O)(C₆₋₁₀ aryl), -S(O)(heteroaryl), -S(O)₂(C₁₋₉ alkyl), -S(O)₂(C₁₋₈ haloalkyl), - S(O)₂(C₂₋₆ alkenyl), -S(O)₂(C₂₋₆ alkynyl), -S(O)₂(C₃₋₁₅ cycloalkyl), -S(O)₂(heterocyclyl), -S(O)₂(C₆₋₁₀ aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
wherein each alkyl, cycloalkyl, heterocyclyl, aryl, or heteroaryl is optionally substituted with 1 to 3 C₁₋₉ alkyl, C₁₋₈ haloalkyl, halogen, -OH, -NH₂, CO₂H,-O(C₁₋₉ alkyl), -O(C₁₋₈ haloalkyl), -O(C₃₋₁₅ cycloalkyl), - O(heterocyclyl), -O(aryl), -O(heteroaryl), -NH(C₁₋₉ alkyl), -NH(C₁₋₈ haloalkyl), -NH(C₃₋₁₅ cycloalkyl), -NH(heterocyclyl), -NH(aryl), - NH(heteroaryl), -N(C₁₋₉ alkyl)₂, -N(C₃₋₁₅ cycloalkyl)₂, -NHC(O)(C₁₋₈ haloalkyl), -NHC(O)(C₃₋₁₅ cycloalkyl), -NHC(O)(heterocyclyl), - NHC(O)(aryl), -NHC(O)(heteroaryl), -NHC(O)O(C₁₋₉ alkyl), -NHC(O)O(C₁₋₈ haloalkyl), -NHC(O)O(C₂₋₆ alkynyl), -NHC(O)O(C₃₋₁₅ cycloalkyl), -NHC(O)O(heterocyclyl), - NHC(O)O(aryl), -NHC(O)O(heteroaryl), -NHC(O)NH(C₁₋₉ alkyl), S(O)₂(C₁₋₉ alkyl), -S(O)₂(C₁₋₈ haloalkyl), -S(O)₂(C₃₋₁₅ cycloalkyl), - S(O)₂(heterocyclyl), -S(O)₂(aryl), -S(O)₂(heteroaryl), -S(O)(NH)(C₁₋₉ alkyl), -S(O)₂NH(C₁₋₉ alkyl), or -S(O)₂N(C₁₋₉ alkyl)₂,
wherein the alkyl or heterocyclyl is each optionally substituted with one to four halogens;
R^{6a} is H, C₁₋₆ alkyl, C₃₋₁₀ cycloalkyl, heterocyclyl, -S(O)₂R^{6a1}, or -S(O)₂N(R^{6a1})(NR^{6a2}), wherein the cycloalkyl or heterocyclyl is each optionally substituted with C₁₋₆ alkyl, F, or -CN;
each R^{6b} and R^{6c} is independently H, C₁₋₆ alkyl, C₁₋₆ haloalkyl, C₂₋₆ alkoxyalkyl, halogen, C₃₋₁₀ cycloalkyl, heterocyclyl, -C₁₋₆ alkyl-N(R^{9a})(R^{9b}), -CN, -OR^{6c1}, or - N(R^{6c2})(R^{6c3}),
wherein the alkyl, cycloalkyl, or heterocyclyl is each optionally substituted with one to four R^{6b1}; or
R^{6b} and R^{6c} are combined with the atom to which they are attached to form a C₃₋₁₀ cycloalkyl or heterocyclyl, which is each optionally substituted with one to four R^{6b1}; or
R^{6a} or R^{6c} is combined with one R⁴ group and the atoms to which they are attached to form a C₅₋₁₀ cycloalkyl or heterocyclyl, which is each optionally substituted with one to four R¹⁰;
each R^{6b1} and R¹⁰ is independently C₁₋₆ alkyl, C₁₋₆ haloalkyl, C₁₋₆ alkoxy, C₁₋₆ haloalkoxy, C₂₋₆ alkoxyalkyl, C₂₋₆ alkenyl, C₂₋₆ alkynyl, halogen, C₃₋₁₀ cycloalkyl, heterocyclyl, C₆₋₁₀ aryl, heteroaryl, oxo, -OH, -CN, CO₂R^{3e}, -NO₂, or - C(O)N(R^{2a})(R^{2b}),
wherein the heterocyclyl or heteroaryl is optionally substituted with C₁₋₆ alkyl, C₁₋₆ haloalkyl, or C₁₋₆ haloalkoxy;
each R^{6a1}, R^{6a2}, R^{6c1}, R^{6e2}, and R^{6c3} is independently H, C₁₋₆ alkyl or C₃₋₁₀ cycloalkyl;
each R^{9a} and R^{9b} is independently H, C₁₋₆ alkyl, or C₁₋₆ haloalkyl, or R^{9a} and R^{9b} together form a 6-membered heterocyclyl;
each R^{9c}, R^{9d}, R^{10a}, R^{10b}, and R^{10c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl,
wherein the alkyl, alkenyl, alkynyl, cycloalkyl, heterocyclyl, aryl, or heteroaryl is each optionally substituted with one to four R⁶;
R^{a} and R^{b} are independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl, wherein the alkyl, alkenyl, alkynyl, cycloalkyl, heterocyclyl, aryl, or heteroaryl is each optionally substituted with one to four R⁶;
optionally R^{a} and R^{b} are combined with the atom to which they are attached to form a 5- or 6-membered heterocyclyl, which is optionally substituted with one to four R⁶;
each R^{2a}, R^{2b}, R^{12a}, R^{12b}, and R^{12c} is independently H, C₁₋₉ alkyl, C₂₋₆ alkenyl, C₂₋₆ alkynyl, C₃₋₁₅ cycloalkyl, heterocyclyl, C₆₋₁₀ aryl, or heteroaryl
wherein the alkyl, alkenyl, alkynyl, cycloalkyl, heterocyclyl, aryl, or heteroaryl is each optionally substituted with one to four R⁶;
each R^{3e} is independently H, C₁₋₆ alkyl, C₁₋₆ haloalkyl, -C₁₋₄ alkyl-N(R^{9a})(R^{9b}), -C₁₋₄ alkyl-C(O)N(R^{9a})(R^{9b}), -C₁₋₄ alkyl-O-C(O)-C₁₋₄ alkyl, -C₁₋₄ alkyl-O-C(O)-O-C₁₋₄alkyl, -C₁₋₄ alkyl-O-C(O)-C₁₋₄ alkyl-N(R^{9a})(R^{9b}), -C₁₋₄ alkyl-C₃₋₈ cycloalkyl, -C₁₋₄ alkyl-heterocyclyl, C₃₋₁₀ cycloalkyl, heterocyclyl, C₆₋₁₀ aryl, heteroaryl, - P(O)(OR^{9c})₂, -CH2P(O)(OR^{9c})₂, -OCH₂P(O)(OR^{9c})₂, -C(O)OCH₂P(O)(OR^{9c})₂, - P(O)(R^{9c})(OR^{9d}), -OP(O)(R^{9c})(OR^{9d}), -CH₂P(O)(R^{9c})(OR^{9d}), - C(O)OCH₂P(O)(R^{9c})(OR^{9d}), -P(O)(N(R^{9c})₂)₂, -CH₂P(O)(N(R^{9c})₂)₂, - C(O)OCH₂P(O)(N(R^{9C})₂)₂, -P(O)(N(R^{9c})₂)(OR^{9d}), -CH₂P(O)(N(R^{9c})₂)(OR^{9d}), - C(O)OCH₂P(O)(N(R^{9c})₂)(OR^{9d}), -P(O)(R^{9c})(N(R^{9d})₂), -CH₂P(O)(R^{9c})(N(R^{9d})₂), or -C(O)OCH₂P(O)(R^{9c})(N(R^{9d})₂);
wherein the alkyl, alkenyl, cycloalkyl, heterocyclyl, aryl, or heteroaryl is each optionally substituted with one to four R⁶; wherein each heteroaryl has 5 to 12 ring members and has one to four heteroatoms each independently N, O, or S;
wherein each heterocyclyl has three to twelve ring members and has one to four heteroatoms, each independently N, O, or S; and
wherein each heteroaryl has five to twelve ring members and one to four heteroatoms, each independently N, O, or S.

2. The compound of claim 1, or a pharmaceutically acceptable salt thereof, wherein
R¹ is

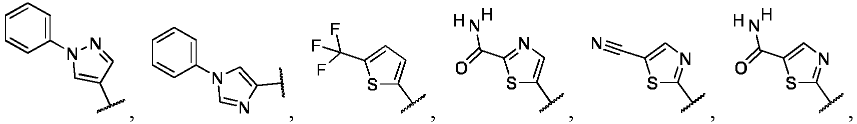

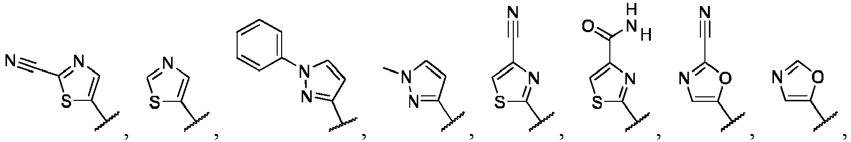

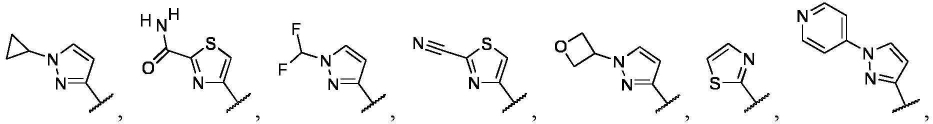

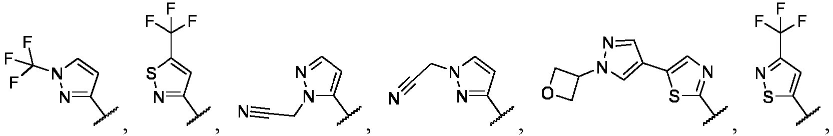

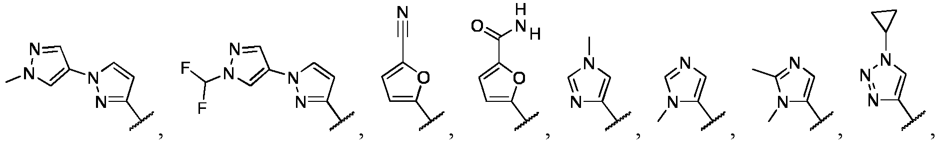

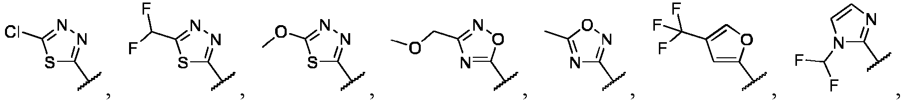

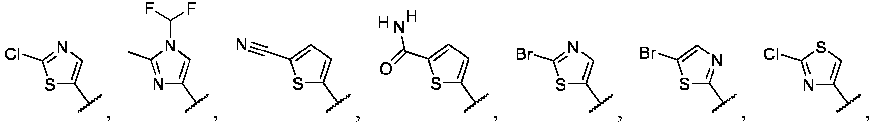

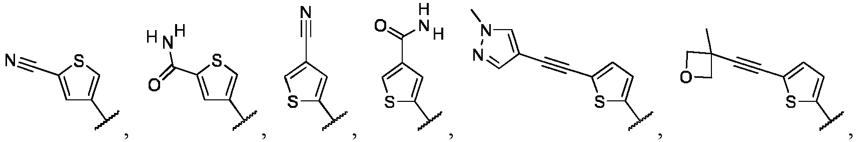

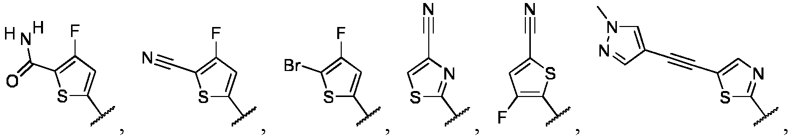

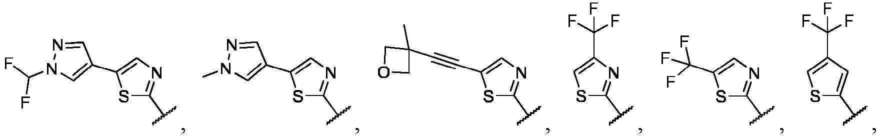

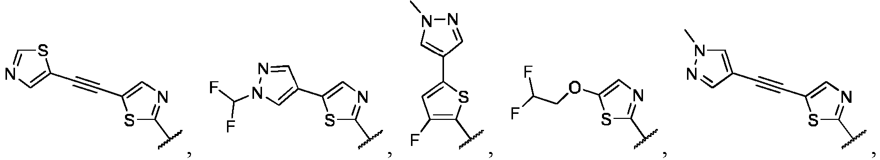

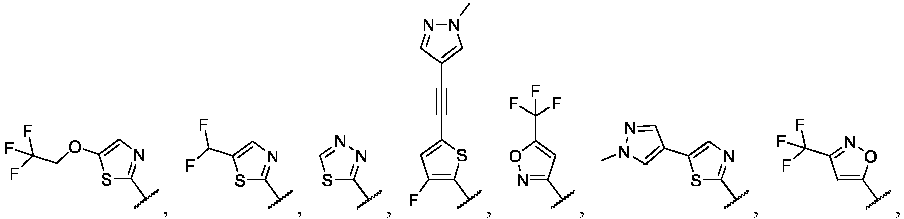

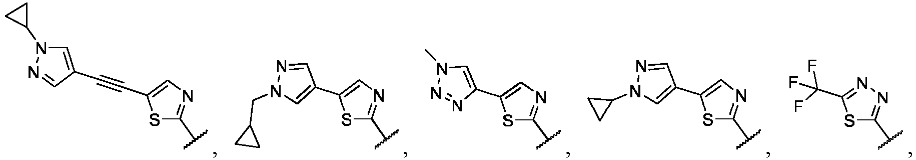

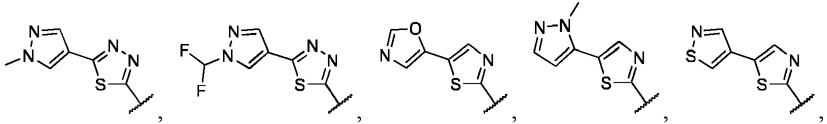

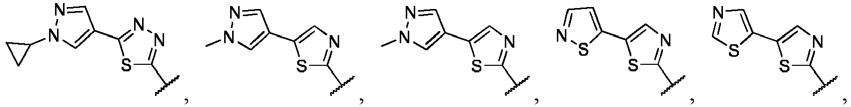

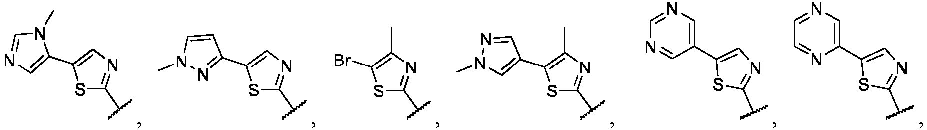

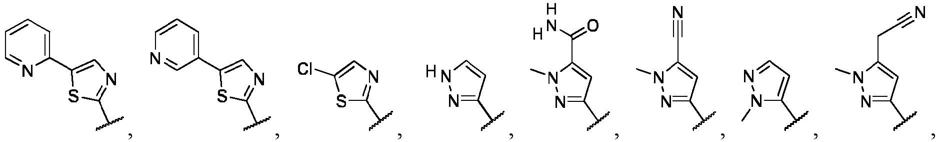

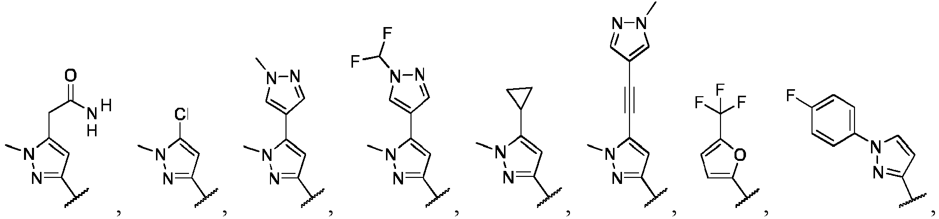

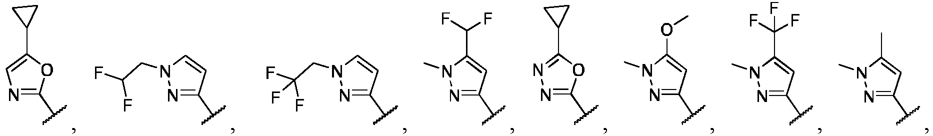

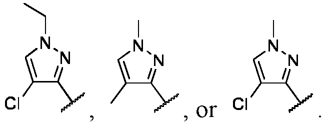

3. The compound of any one of claims 1-2, or a pharmaceutically acceptable salt thereof, wherein
each R⁴ is independently C₁₋₉ alkyl or halogen.

4. The compound of any one of claims 1-3, or a pharmaceutically acceptable salt thereof, wherein
each R⁴ is independently methyl or F.

5. The compound of any one of claims 1-4, or a pharmaceutically acceptable salt thereof, wherein
ring B is substituted with two R⁴.

6. The compound of claim 5, or a pharmaceutically acceptable salt thereof, wherein each R⁴ is F.

7. The compound of claim any one of claims 1-6, or a pharmaceutically acceptable salt thereof, wherein
ring A is 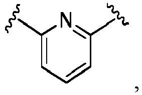 optionally substituted with one halogen.

8. The compound of any one of claims 1-7, or a pharmaceutically acceptable salt thereof, wherein
R² is C₁₋₆ alkyl or heterocyclyl, wherein the alkyl or heterocyclyl is each optionally substituted with one to four Z¹,
wherein each Z¹ is independently C₁₋₆ alkyl, C₁₋₆ alkoxy, or heterocyclyl.

9. The compound of any one of claims 1-8, or a pharmaceutically acceptable salt thereof, wherein
R² is

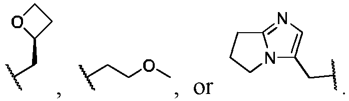

10. The compound of any one of claims 1-9, or a pharmaceutically acceptable salt thereof, wherein R⁸ is F or Cl.

11. The compound of any one of claims 1-10, or a pharmaceutically acceptable salt thereof, wherein the compound has the structure: 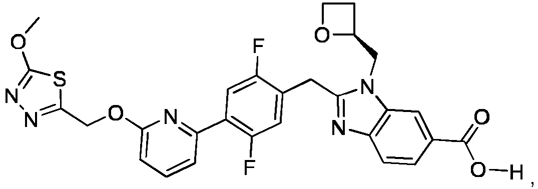 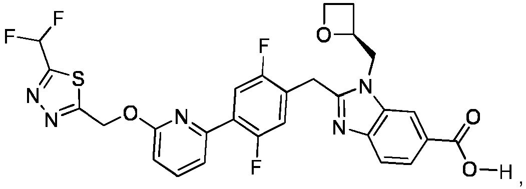 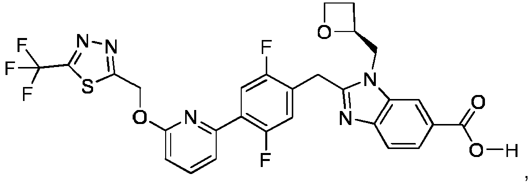 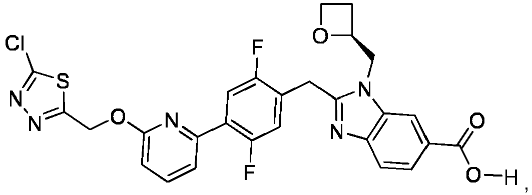 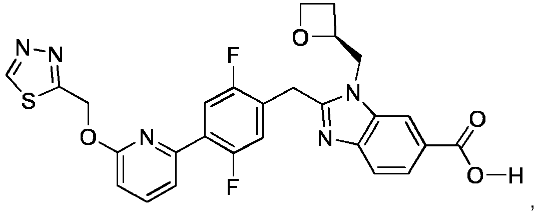 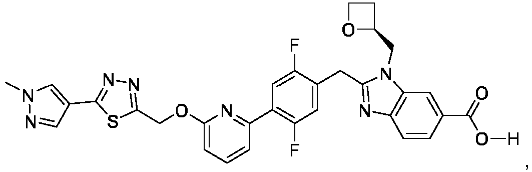 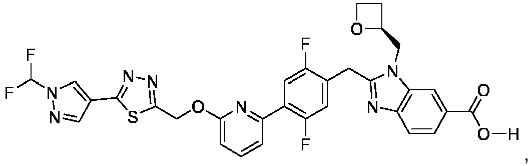 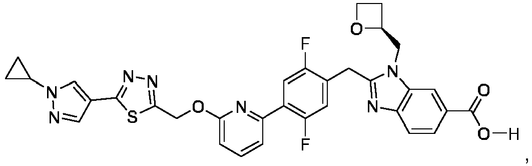 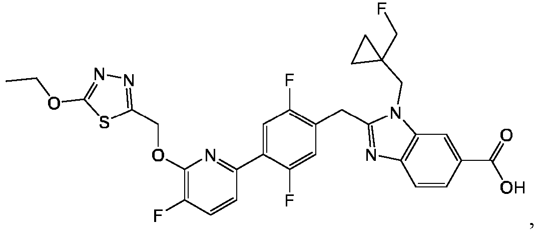 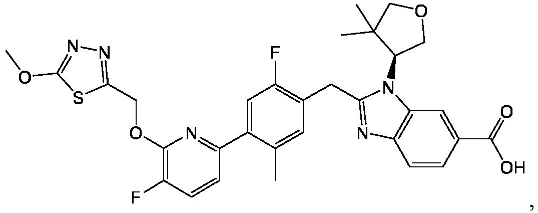 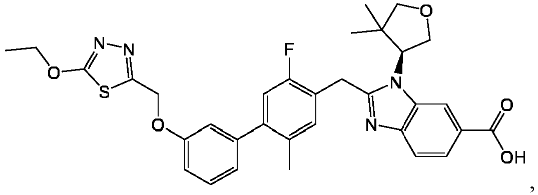 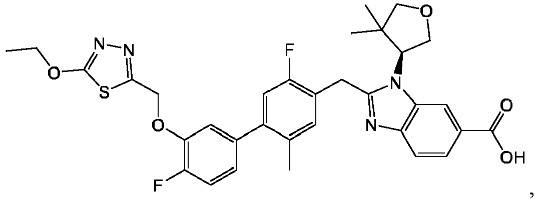 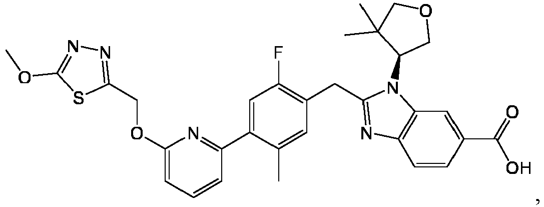 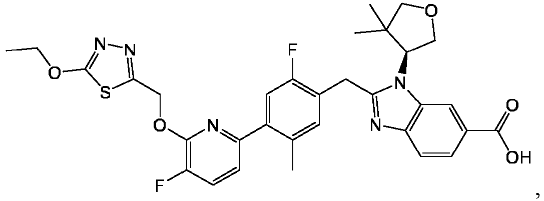 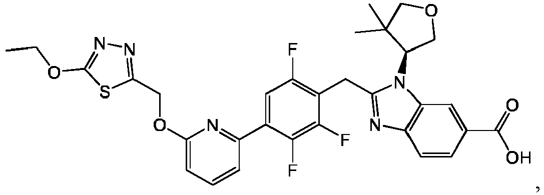 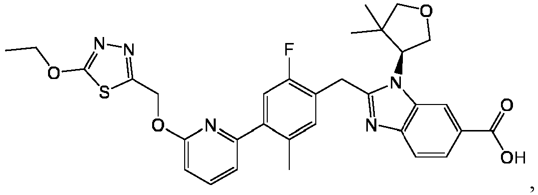 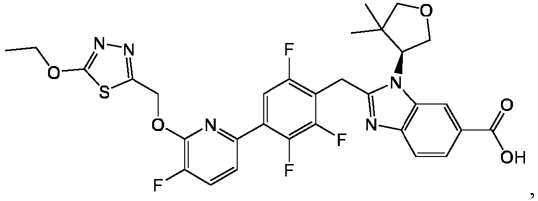 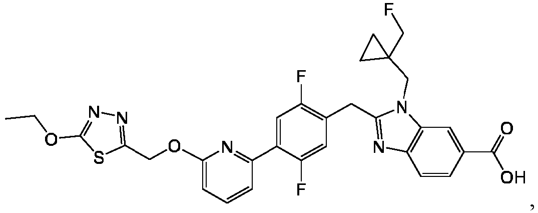 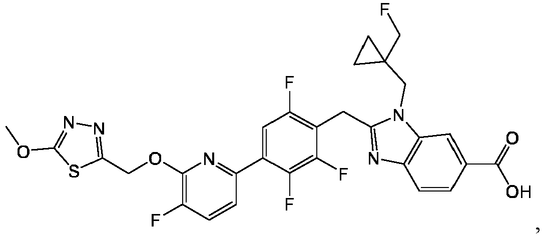 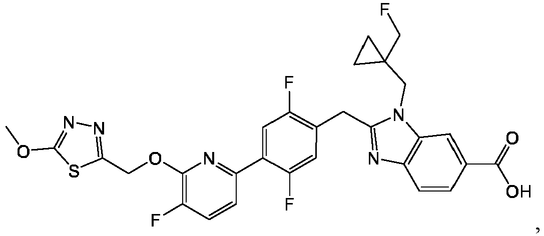 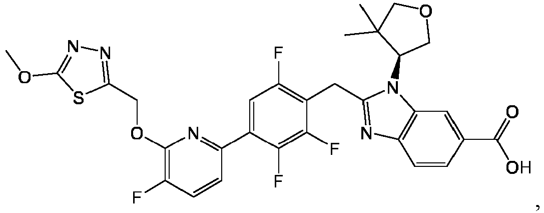 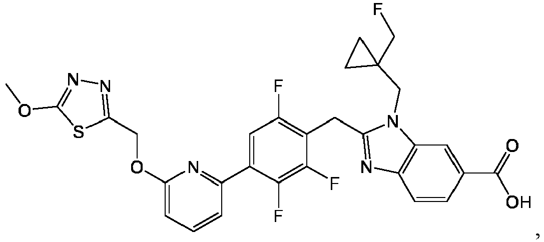 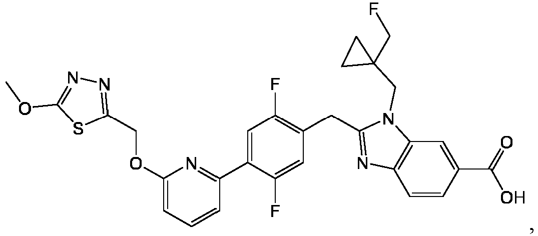 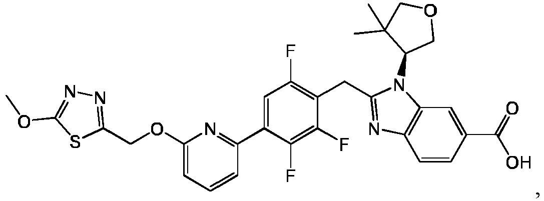 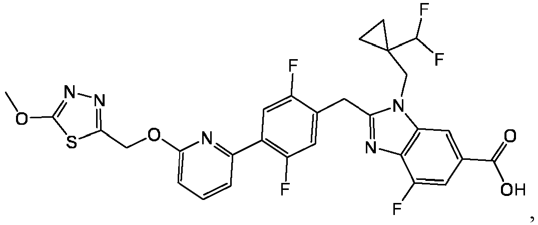 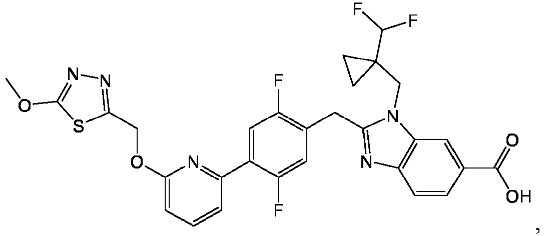 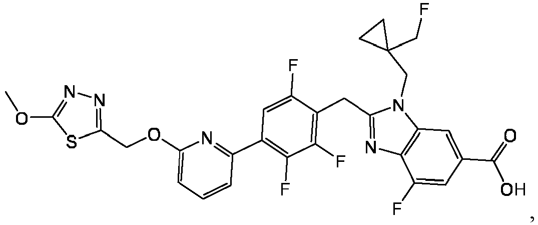 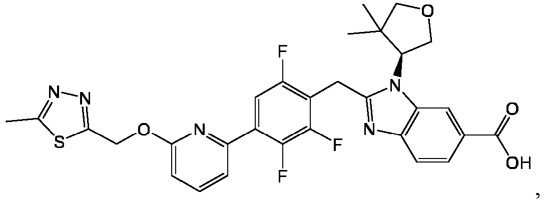 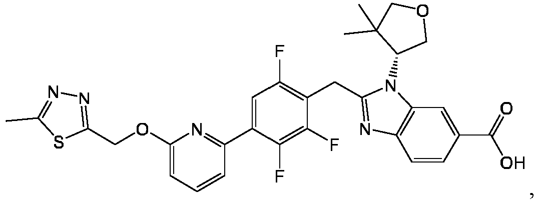 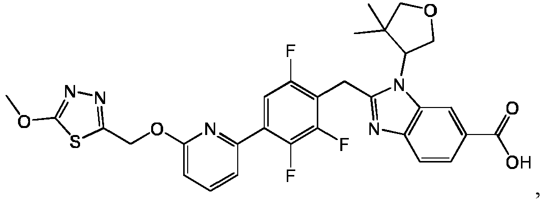 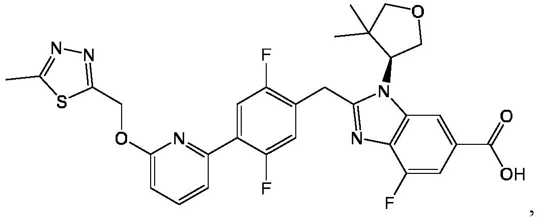 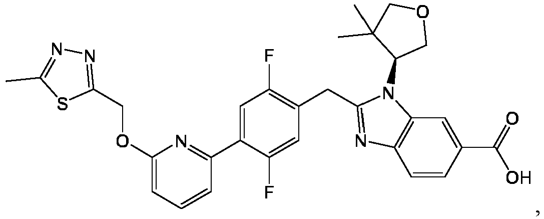 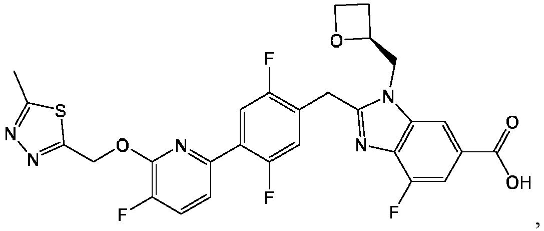 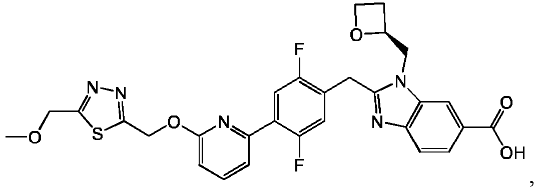 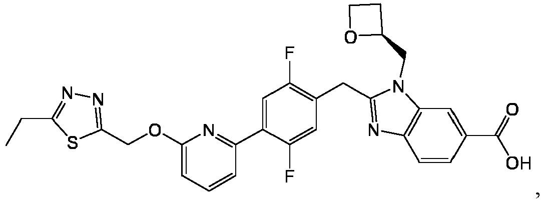 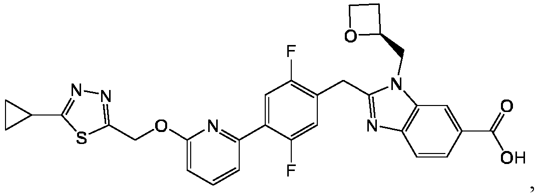 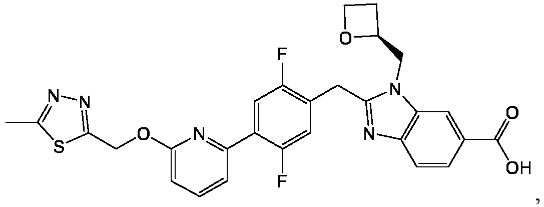 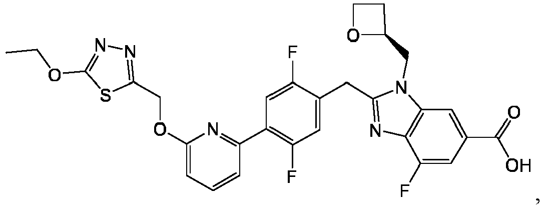 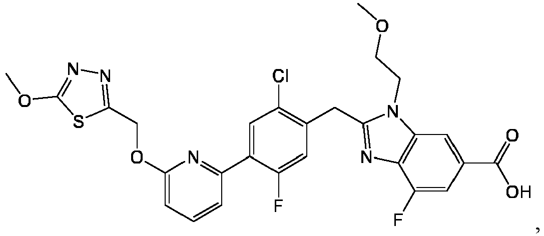 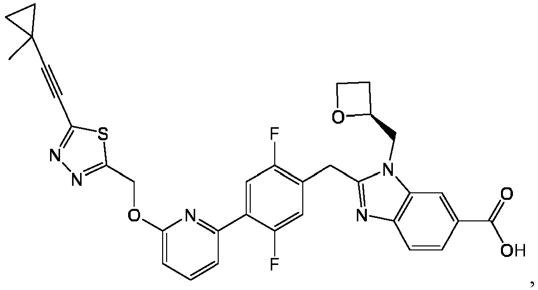 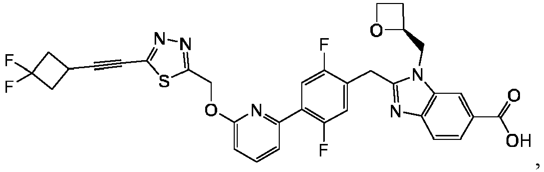 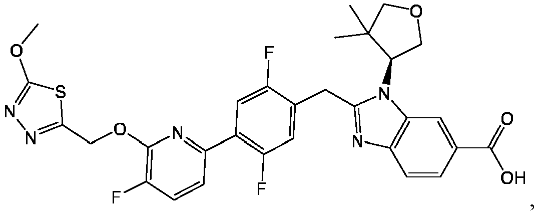 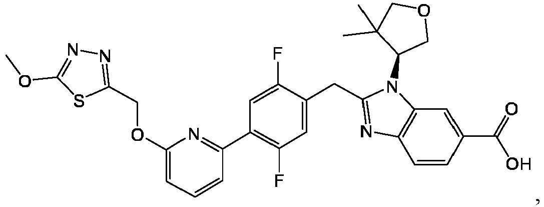 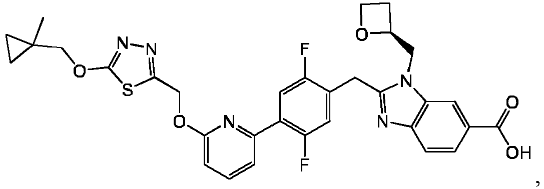 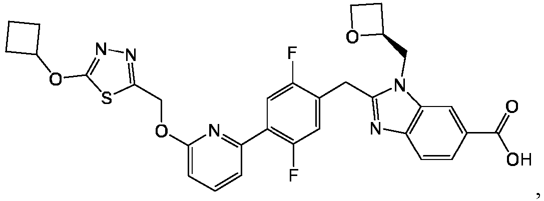 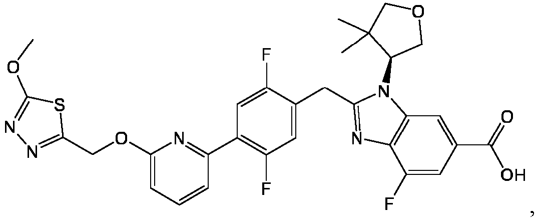 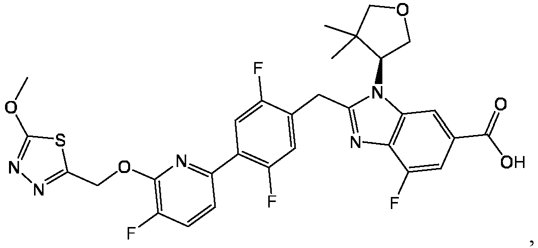 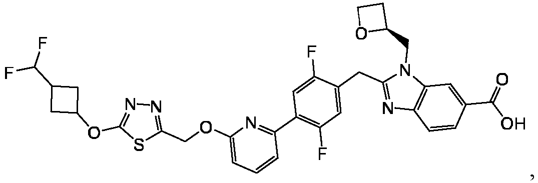 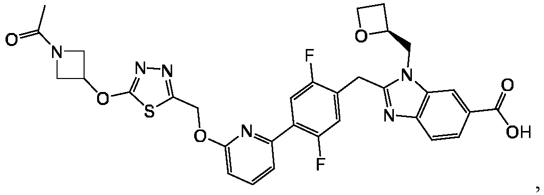 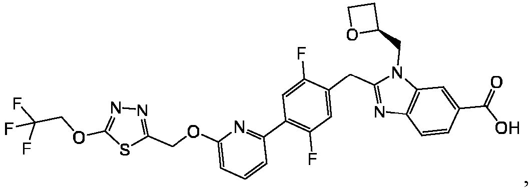 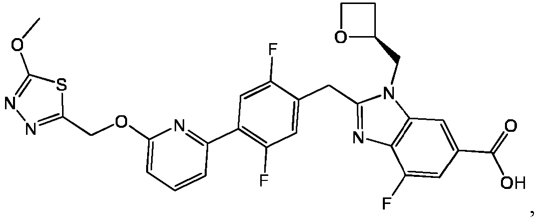 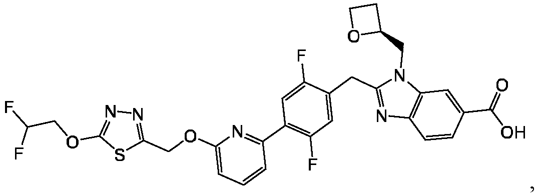 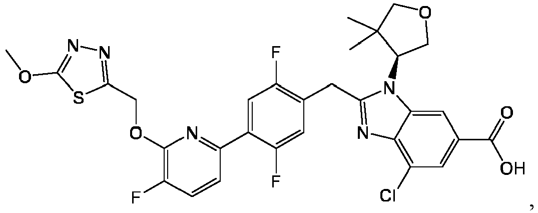 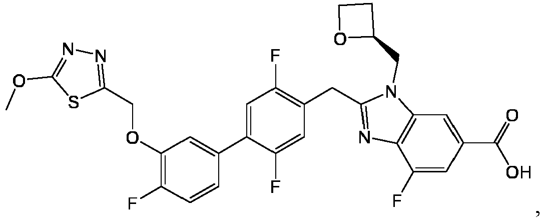 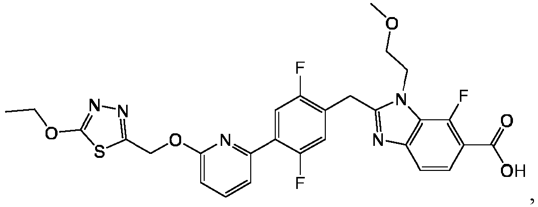 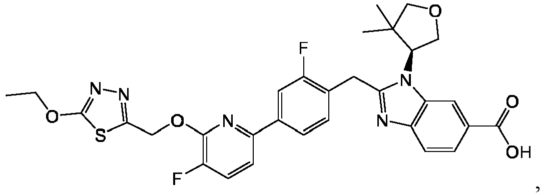 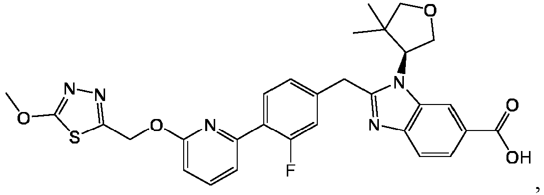 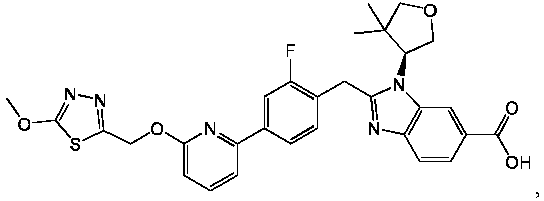 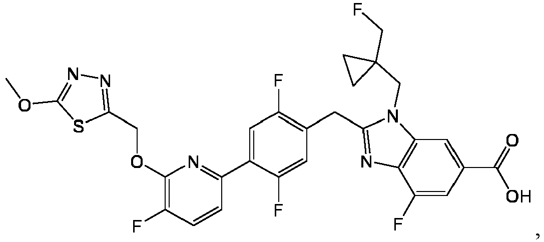 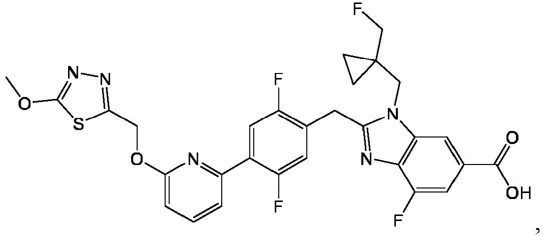 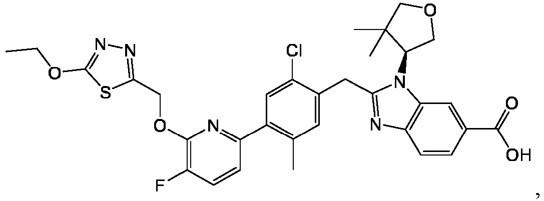 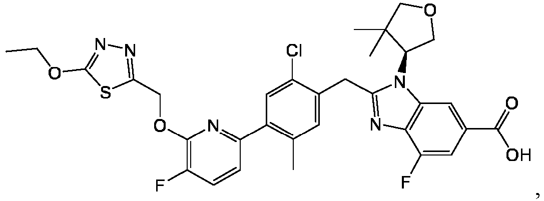 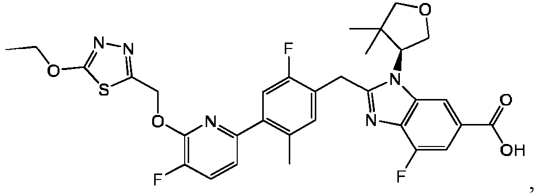 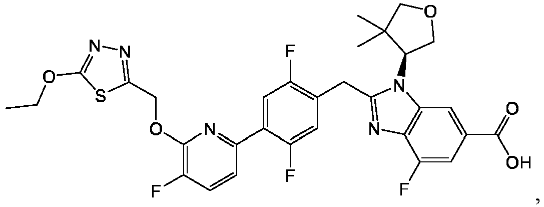 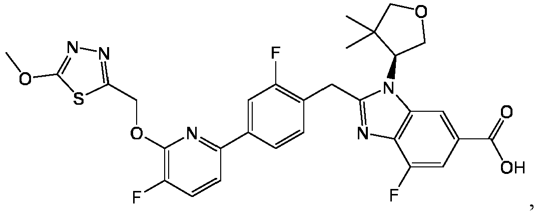 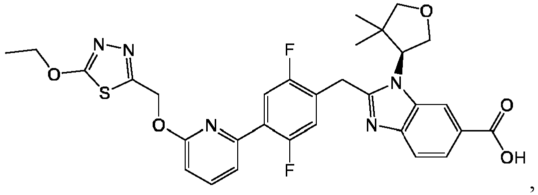 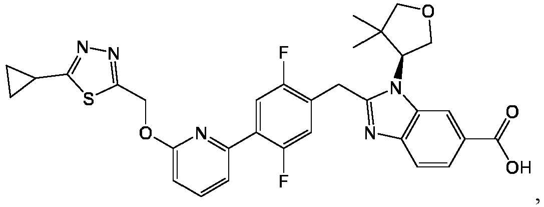 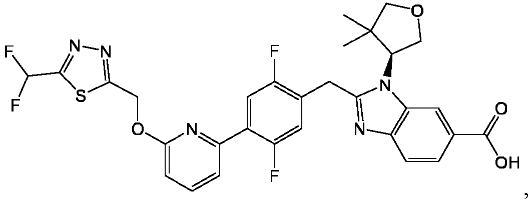 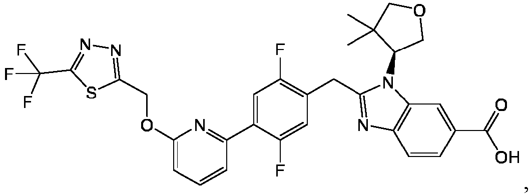 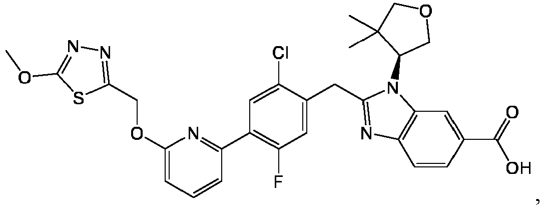 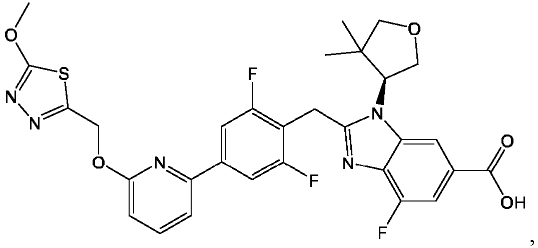 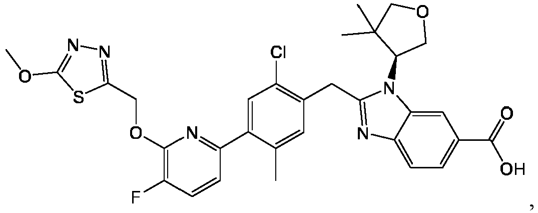 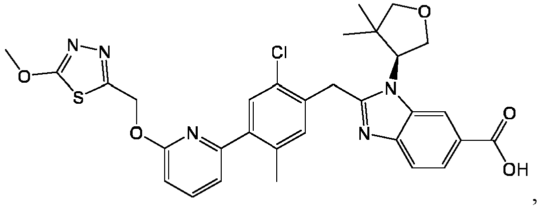 or

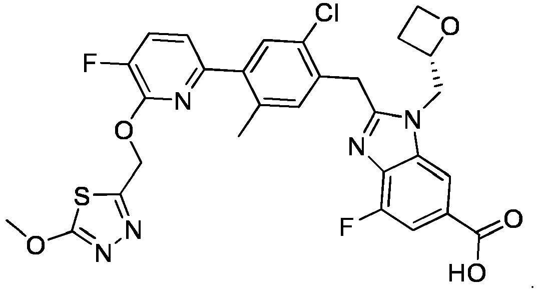

12. A pharmaceutical composition comprising a pharmaceutically effective amount of a compound of any one of claims 1-11, or a pharmaceutically acceptable salt thereof, and a pharmaceutically acceptable carrier or excipient.

13. A compound of any one of claims 1-11, or a pharmaceutically acceptable salt thereof, for use in method of treating GLP-1R mediated disease or condition in a subject in need thereof, wherein the GLP-1R mediated disease or condition is a liver disease or a metabolic disease.

14. The compound for use of claim 13, wherein the disease or condition comprises a liver disease, optionally wherein the disease or condition comprises liver fibrosis, non-alcoholic fatty liver disease (NAFLD), non-alcoholic steatohepatitis (NASH), liver cirrhosis, compensated liver fibrosis, decompensated liver fibrosis, hepatocellular carcinoma, Primary Biliary Cirrhosis (PBC), or Primary Sclerosing Choleangitis (PSC), optionally wherein the disease or condition is non-alcoholic fatty liver disease (NAFLD) or non-alcoholic steatohepatitis (NASH).

15. The compound for use of claim 13, wherein the disease or condition comprises a metabolic disease, optionally wherein the disease or condition comprises type 1 diabetes, type 2 diabetes, pre-diabetes, idiopathic type 1 diabetes, latent autoimmune diabetes, maturity onset diabetes of the young, early onset diabetes, malnutrition-related diabetes, gestational diabetes, hyperglycemia, insulin resistance, hepatic insulin resistance, impaired glucose tolerance, diabetic neuropathy, diabetic nephropathy, kidney disease, diabetic retinopathy, adipocyte dysfunction, visceral adipose deposition, obesity, eating disorders, sleep apnea, weight gain, sugar craving, dyslipidemia, hyperinsulinemia, congestive heart failure, myocardial infarction, stroke, hemorrhagic stroke, ischemic stroke, traumatic brain injury, pulmonary hypertension, restenosis after angioplasty, intermittent claudication, post-prandial lipemia, metabolic acidosis, ketosis, arthritis, left ventricular hypertrophy, Parkinson's Disease, peripheral arterial disease, macular degeneration, cataract, glomerulosclerosis, chronic renal failure, metabolic syndrome, angina pectoris, premenstrual syndrome, thrombosis, atherosclerosis, impaired glucose metabolism, vascular restenosis, dementia, or Alzheimer's disease.
